# EUROPEAN PATENT APPLICATION

(11) **EP 4 592 295 A1**
(43) Date of publication of application: **30.07.2025**
(21) Application number: 23867655.5
(22) Date of filing: 25.09.2023
(51) Int. Cl.: C07D 498/14, C07D 498/22, C07D 519/00, C07D 471/14, A61K 31/553, A61K 31/519, A61K 31/4985, A61K 31/5377, A61K 31/506, A61P 35/00

(54) **PYRIMIDINE-FUSED RING COMPOUND, AND PREPARATION METHOD THEREFOR AND USE THEREOF**

(30) Priority: 23.09.2022 CN 202211164578; 12.10.2022 CN 202211246740; 19.10.2022 CN 202211279547; 02.11.2022 CN 202211362218; 16.11.2022 CN 202211435120; 20.09.2023 CN 202311221724
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: JIANG, Tao, Shanghai 201203 (CN); ZHOU, Fusheng, Shanghai 201203 (CN); LIANG, Tao, Shanghai 201203 (CN); LIN, Chonglan, Shanghai 201203 (CN); CAI, Lijian, Shanghai 201203 (CN); LIU, Zhubo, Shanghai 201203 (CN); XU, Xiaoming, Shanghai 201203 (CN); MA, Kai, Shanghai 201203 (CN); ZHANG, Leitao, Shanghai 201203 (CN); LI, Zhen, Shanghai 201203 (CN); LAN, Jiong, Shanghai 201203 (CN)
(74) Representative: Heller, Benjamin Henry
(86) International application number: PCT/CN2023/121172
(87) International publication number: WO 2024/061370

(57) **Abstract**

The present invention discloses a pyrimidine-fused ring compound having an inhibitory effect on KRAS gene mutation, or a pharmaceutically acceptable salt, a stereoisomer, or a solvate thereof. Further, the present invention also discloses a pharmaceutical composition comprising the compound, and use thereof in the preparation of drugs against cancers.

## Description

### Technical Field

The present invention belongs to the field of medicines, and specifically to a class of pyrimidine-fused ring compounds and a preparation method and use thereof.

### Background

KRAS is a 21 kD member of the Ras family of GTPase proteins and is an essential component for cellular signaling. The role of KRAS in malignant tumors and mutations in various types of tumors (e.g., G12C mutation, G12D mutation, and G12V mutation, etc.) are widely known, and thus KRAS has become a very attractive target for cancer treatment in the pharmaceutical industry. Compounds that inhibit KRAS activity are highly desirable for researchers in this field and are under intensive research. At present, breakthroughs are made for KRAS G12C in this field. For example, the KRAS G12C inhibitors of AMG and MIRATI have shown sufficient safety and efficacy. However, there is still constant interest and effort in developing KRAS inhibitors, especially inhibitors that activate KRAS mutants, particularly KRAS G12D.

### Summary of the Invention

The present invention provides a class of novel pyrimidine-fused ring compounds, which can be used as KRAS G12D inhibitors and have the advantages of high activity, good selectivity and low toxicity and side effects. The present invention also provides a class of novel pyrimidine-fused ring compounds, which will serve as KRAS G12D inhibitors with better bioavailability and higher AUC.

A first aspect of the present invention provides a compound represented by Formula (A) or a pharmaceutically acceptable salt, stereoisomer or solvate thereof: 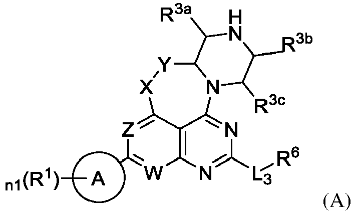 where in the formula,
X is O, S or NR¹¹, in which R¹¹ is selected from H, C1-C6 alkyl, C1-C6 deuterated alkyl and cycloalkyl;
Y is CR¹²R¹³, CR¹⁴R¹⁵CR¹⁶R¹⁷, C(O) or C(O)CR¹⁸R¹⁹, where
R¹² and R¹³ are each independently selected from H, halogen, hydroxyl, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, -C1-C4 alkyl-NR¹²¹R¹²²; or R¹² and R¹³, together with the carbon atom to which they are attached, form a cycloalkyl group;
R¹⁴ and R¹⁵ are each independently selected from H, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, -C1-C4 alkyl-NR¹²¹R¹²²; or R¹⁴ and R¹⁵, together with the carbon atom to which they are attached, form a cycloalkyl group;
R¹⁶ and R¹⁷ are each independently selected from H, halogen, hydroxyl, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, -C1-C4 alkyl-NR¹²¹R¹²²; and
R¹⁸ and R¹⁹ are each independently selected from H, C1-C3 alkyl and halogen, in which
R¹²¹ and R¹²² are each independently selected from H and C1-C3 alkyl; or R¹²¹ and R¹²², together with the nitrogen atom to which they are attached, form a 3- to 6-membered nitrogen-containing heterocyclic group, and
the cycloalkyl, the heterocyclyl, the aryl, the heteroaryl, and the 3- to 6-membered nitrogen-containing heterocyclic group are each independently optionally substituted with a group selected from the group consisting of halogen, hydroxyl, nitro, cyano, C1-C3 alkyl, C1-C3 deuterated alkyl, C1-C3 haloalkyl, C1-C3 alkoxy, and C1-C3 haloalkoxy;
W is N or CR²⁰, where R²⁰ is H, cyano, C1-C6 alkyl, halogen, C1-C6 haloalkyl, C1-C6 alkoxy, cycloalkyl, cycloalkyl-O-, heterocyclyl or heterocyclyl-O-, in which the cycloalkyl and heterocyclyl are each independently optionally substituted with halo;
ring A (ring A refers to 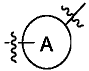 herein) is selected from the group consisting of: aryl and heteroaryl;
R¹ is a substituent at any position on ring A; n1 is 0, 1, 2, 3, 4 or 5;
each R¹ is independently selected from C1-C6 alkyl, halogen, hydroxyl, cyano, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl,-S-C1-C6 haloalkyl, C1-C6 hydroxyalkyl, -CH₂C(O)NR²⁷R²⁸, -C2-C6 alkynyl-NR²⁹R³⁰, C2-C6 deuterated alkynyl, (C1-C6 alkoxy) C1-C6 haloalkyl- or cycloalkyl, where the cycloalkyl is optionally substituted with halo or C1-C6 alkyl, and where
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl, or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxy, cyano, heterocyclyl, NR²⁵¹R²⁵², C(O)NR²⁵³R²⁵⁴ or SO₂C1-C6 alkyl, where R²⁵¹, R²⁵², R²⁵³ and R²⁵⁴ are each independently H or C1-C6 alkyl; or R²⁵¹ and R²⁵², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy; or R²⁵³ and R²⁵⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy; and
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
Z is N or CR², where R² is H, cyano, halogen, C1-C6 alkyl, C1-C6 alkoxy, C1-C6 haloalkyl, C1-C6 haloalkoxy, C1-C6 deuterated alkoxy, NR³¹R³², C2-C4 alkynyl or CH₂OR³³, where R³¹ and R³² are each independently hydrogen or C1-C6 alkyl; or R³¹ and R³², together with the nitrogen atom to which they are attached, form a 3- to 6-membered nitrogen-containing heterocyclic group; and R³³ is hydrogen or C1-C6 alkyl;
R^{3a}, R^{3b}, and R^{3c} are each independently hydrogen, halogen, hydroxyl, cyano, C1-C3 alkyl, C1-C3 deuterated alkyl, or C1-C3 haloalkyl; or
R^{3a} and R^{3b} are linked to form -CHR^{3a1}- or -CHR^{3a2}CHR^{3a3}-; and R^{3c} is hydrogen, halogen, hydroxyl, cyano, C1-C3 alkyl, C1-C3 deuterated alkyl or C1-C3 haloalkyl, where R^{3a1}, R^{3a2}, and R^{3a3} are each independently hydrogen, halogen, hydroxyl, cyano, C1-C3 alkyl, C1-C3 deuterated alkyl or C1-C3 haloalkyl; or
R^{3a} and R^{3c} are linked to form -CHR^{3a1}- or -CHR^{3a2}CHR^{3a3}-; and R^{3b} is hydrogen, halogen, hydroxyl, cyano, C1-C3 alkyl, C1-C3 deuterated alkyl or C1-C3 haloalkyl, where R^{3a1}, R^{3a2}, and R^{3a3} are each independently hydrogen, halogen, hydroxyl, cyano, C1-C3 alkyl, C1-C3 deuterated alkyl or C1-C3 haloalkyl;
L₃ is a bond, O or NR³⁴;
R⁶ is -L-6 to 10-membered fused heterocyclyl, -L-7 to 11-membered spiro heterocyclyl, -L-spiro-substituted 6 to 10-membered fused heterocyclyl, -L-spiro-substituted 7 to 11-membered spiro heterocyclyl, -L-parallel-substituted 6 to 10-membered fused heterocyclyl, -L-parallel-substituted 7 to 11-membered spiro heterocyclyl, or -L-spiro- and parallel-substituted 6 to 10-membered fused heterocyclyl;
the spiro-substituted 6- to 10-membered fused heterocyclyl refers to a spiro-substituted 6- to 10-membered fused heterocyclic group formed when 2 hydrogen atoms on the same carbon atom in any 1 or 2 carbon atoms in a 6- to 10-membered fused heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the spiro-substituted 7- to 11-membered spiro heterocyclyl refers to a spiro-substituted 7- to 11-membered spiro heterocyclic group formed when 2 hydrogen atoms on the same carbon atom in any 1 or 2 carbon atoms in a 7- to 11-membered spiro heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the parallel-substituted 6- to 10-membered fused heterocyclyl refers to a parallel-substituted 6- to 10-membered fused heterocyclic group formed when 1 hydrogen atom on each carbon atom in the same pair of carbon atoms in any 1 or 2 pairs of adjacent carbon atoms in a 6- to 10-membered fused heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 6- to 10-membered fused heterocyclyl refers to a parallel-substituted 6- to 10-membered fused heterocyclic group formed when hydrogen atoms on any 2 non-adjacent carbon atoms in a 6- to 10-membered fused heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the parallel-substituted 7- to 11-membered spiro heterocyclyl refers to a parallel-substituted 7- to 11-membered spiro heterocyclic group formed when 1 hydrogen atom on each carbon atom in the same pair of carbon atoms in any 1 or 2 pairs of adjacent carbon atoms in a 7- to 11-membered spiro heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 7- to 11-membered spiro heterocyclyl refers to a parallel-substituted 7- to 11-membered spiro heterocyclic group formed when hydrogen atoms on any two non-adjacent carbon atoms in a 7- to 11-membered spiro heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the spiro- and parallel-substituted 6- to 10-membered fused heterocyclyl refers to a spiro- and parallel-substituted 6- to 10-membered fused heterocyclic group formed when 1 hydrogen atom on each carbon atom in any 1 pair of adjacent carbon atoms in a 6- to 10-membered fused heterocyclic group is replaced by -(CH₂)ₘ₄-,-(CH₂)ₘ₅O(CH₂)ₘ₆-, or -(CH₂)ₘ₇NRⁿ(CH₂)ₘ₈ to form a parallel-substitution, and 2 hydrogen atoms on another carbon atom in the 6- to 10-membered fused heterocyclic group are both replaced by -(CH₂)ₘ₄-, -(CH₂)ₘ₅O(CH₂)ₘ₆-, or -(CH₂)ₘ₇NRⁿ(CH₂)ₘ₈ to form a spiro-substitution;
the 6- to 10-membered fused heterocyclyl and the 7- to 11-membered spiro heterocyclyl each independently have 1, 2, 3 or 4 heteroatoms selected from N(R^{m}), S, S(=O), S(=O)₂, O as ring atoms;
the spiro-substituted 6- to 10-membered fused heterocyclyl, the spiro-substituted 7- to 11-membered spiro heterocyclyl, the parallel-substituted 6- to 10-membered fused heterocyclyl, the parallel-substituted 7- to 11-membered spiro heterocyclyl, and the spiro- and parallel-substituted 6- to 10-membered fused heterocyclyl each independently have 1, 2, 3, 4 or 5 heteroatoms selected from N(R^{m}), S, S(=O), S(=O)₂, and O as ring atoms;
Rⁿ is hydrogen, C1-C4 alkyl, deuterated C1-C4 alkyl, C1-C4 haloalkyl, C3-C20 cycloalkyl, or a 3-20 membered heterocyclyl;
R^{m} is absent, hydrogen, C1-C4 alkyl, deuterated C1-C4 alkyl, C1-C4 haloalkyl, C3-C20 cycloalkyl or 3-20 membered heterocyclyl;
where the 6- to 10-membered fused heterocyclyl and the 7- to 11-membered spiro heterocyclyl are each independently saturated or partially unsaturated; and when the 6- to 10-membered fused heterocyclyl and the 7- to 11-membered spiro heterocyclyl are partially unsaturated, the ring has 1 or 2 double bonds;
2 hydrogen atoms on the same carbon atom in any 1 or 2 carbon atoms in the 6- to 10-membered fused heterocyclyl (the 6- to 10-membered fused heterocyclyl refers to a -L-6- to 10-membered fused heterocyclyl, a - L-spiro-substituted 6- to 10-membered fused heterocyclyl, a -L-parallel-substituted 6- to 10-membered fused heterocyclyl, or a -L-spiro- and parallel-substituted 6- to 10-membered fused heterocyclyl) are optionally both replaced by =CR^{2a}R^{2b}; and the 6- to 10-membered fused heterocyclyl is optionally further substituted with one or more R^{6a};
2 hydrogen atoms on the same carbon atom in any 1 or 2 carbon atoms in the 7- to 11-membered spiro heterocyclyl (the 7- to 11-membered spiro heterocyclyl refers to a 7- to 11-membered spiro heterocyclyl in a -L-7- to 11-membered spiro heterocyclyl, a -L-spiro-substituted 7- to 11-membered spiro heterocyclyl or a -L-parallel-substituted 7- to 11-membered spiro heterocyclyl) are optionally both replaced by =CR^{2a}R^{2b}; and the 7- to 11-membered spiro heterocyclyl is optionally further substituted with one or more R^{6a};
where R^{2a} and R^{2b} are each independently hydrogen, halogen, cyano, C1-C4 alkyl, C1-C4 haloalkyl, C6-C10 aryl or a 5-membered or 6-membered heteroaryl, in which the C6-C10 aryl or the 5-membered or 6-membered heteroaryl is optionally substituted with 1, 2 or 3 groups selected from halogen, cyano, C1-C4 alkyl, C1-C4 alkoxy, C1-C4 haloalkyl or C1-C4 haloalkoxy; or each pair of R^{2a} and R^{2b} independently forms, together with the carbon atoms to which they are attached, a C3-C6 monocyclic cycloalkyl or a 3- to 6-membered monocyclic heterocyclic group, in which the C3-C6 monocyclic cycloalkyl or the 3- to 6-membered monocyclic heterocyclic group is optionally substituted with 1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl;
R^{6a} is each independently halogen, hydroxyl, C1-C6 hydroxylalkyl, C1-C6 alkyl, C2-C6 alkenyl, C2-C6 haloalkenyl, C2-C6 alkynyl, C2-C6 haloalkynyl, C1-C6 haloalkyl, C1-C6 alkoxy, C1-C6 haloalkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl-C1-C3 alkyl-, t-butyldimethylsilyloxyCH₂-, -N(R^{6b})₂, (C1-C3 alkoxy)C1-C3 alkyl, (C1-C3 alkyl)C(O)-, oxo, (C1-C3 haloalkyl)C(O)-, -SO₂F, (C1-C3 alkoxy)C1-C3 alkoxy, -CH₂OC(O)N(R^{6b})₂,-CH₂NHC(O)OC1-C6 alkyl, -CH₂NHC(O)N(R^{6b})₂, -CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂, -OC(O)NH(C1-C3 alkyl)O(C1-C3 alkyl),-OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂heterocyclyl, where the phenyl involved in the above groups is optionally substituted with -C(O)H or OH; the heterocyclyl in-C1-C3 alkyl-heterocyclyl is optionally substituted with oxo; Q is a bond or O; R^{6b} is each independently hydrogen, C1-C6 alkyl, C1-C6 deuterated alkyl or C1-C6 haloalkyl; or two R^{6b} each independently form, together with the nitrogen atom to which they are attached, a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, trifluoromethoxy; and the C3-C6 cycloalkyl is optionally substituted with 1 or 2 C1-C6 alkyl;
each m4 is 2, 3 or 4;
m5, m6, m7, and m8 are each independently 0, 1, 2, or 3; m5 and m6 are not both 0; m7 and m8 are not both 0;
   or
R⁶ is hydrogen, -N(R³⁴)₂, heterocyclyl, cycloalkyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-aryl, -L-COOH or -L-C(O)OC1-C6 alkyl, where the heterocyclyl, the cycloalkyl, the aryl in the -L-NR³⁴C(O)-aryl, the heterocyclyl in the -L-heterocyclyl, and the cycloalkyl in the -L-cycloalkyl are each optionally substituted with one or more R³⁵ or optionally two hydrogen atoms on the same carbon atom are both substituted with -CH₂CH₂- or -CH₂CH₂CH₂- to form a cyclopropyl or cyclobutyl group; and the aryl in the -L-aryl and the heteroaryl in the -L-heteroaryl are each optionally substituted with one or more R³⁶,
where L is each independently a bond, C1-C4 alkylene or heteroaryl, in which 1 or 2 hydrogen atom(s) on any carbon atom(s) in the C1-C4 alkylene is/are optionally each independently substituted with deuterium, C1-C6 alkyl, C1-C6 haloalkyl, C2-C4 alkenylene or C2-C4 haloalkenylene; or 2 hydrogen atoms on any carbon atom in the C1-C4 alkylene are optionally both replaced by -(CH₂)ₘ₃-, -(CH₂)ₘ₁-O-(CH₂)ₘ₂-, or -(CH₂)ₘ₁-NR⁹-(CH₂)ₘ₂-, to form a cyclic substituent; m3 is 1 or 2; m1 is each independently 0, 1, 2 or 3; m2 is each independently 0, 1, 2 or 3; and m1 and m2 are not both 0;
R⁹ is hydrogen, C1-C6 alkyl, C1-C6 deuterated alkyl, C1-C6 haloalkyl, C1-C6 alkyl-hydroxyl, C1-C6 alkyl-cyano, C1-C6 alkoxy, C1-C6 alkyl-C1-C6 alkoxy, C3-C6 monocyclic cycloalkyl, C3-C6 deuterated cycloalkyl or C3-C6 halocycloalkyl;
R³⁵ is each independently halogen, hydroxyl, C1-C3 alkyl, C2-C4 alkenylene, C2-C4 halo alkenylene, C1-C3 haloalkyl, C1-C3 alkoxy, C3-C6 cycloalkyl, -C1-C3 alkyl-C1-C3 alkoxy, -C1-C3 alkyl-hydroxyl, heterocyclyl (for example, a 3- to 6-membered heterocyclyl), cyano, -phenyl, -phenyl-SO₂F, -O-phenyl, -O-phenyl-SO₂F, - NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂,-CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂,-OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂,-OC(O)NH(C1-C3 alkyl)O(C1-C3 alkyl)phenyl, -OC(O)heterocyclyl (for example, -OC(O)-3- to 6-membered heterocyclyl) or -CH₂heterocyclyl (for example, -CH₂-3- to 6-membered heterocyclyl), where the phenyl in the-NHC(O)phenyl or -OC(O)NH(C1-C3 alkyl)O(C1-C3 alkyl)phenyl is optionally substituted with-C(O)H or OH; the heterocyclyl in the -CH₂heterocyclyl is optionally substituted with oxo; and the hydrogen atom on the -C1-C3 alkyl- is substituted with 1 or 2 groups selected from halogen, deuterium, C1-C3 alkyl, and C1-C3 haloalkyl;
R³⁶ is each independently halogen, hydroxyl, HC(O)-, C1-C4 alkyl, C1-C4 alkoxy, C1-C4 haloalkyl, C1-C4 hydroxylalkyl or -N(R³⁴)₂; and
R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy.

In one embodiment, R^{6a} is each independently halogen, hydroxyl, C1-C6 hydroxylalkyl, C1-C6 alkyl, C2-C6 alkenyl, C2-C6 haloalkenyl, C2-C6 alkynyl, C2-C6 haloalkynyl, C1-C6 haloalkyl, C1-C6 alkoxy, C1-C6 haloalkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl-C1-C3 alkyl-, t-butyldimethylsilyloxyCH₂-, -N(R^{6b})₂, (C1-C3 alkoxy)C1-C3 alkyl, (C1-C3 alkyl)C(O)-, oxo, (C1-C3 haloalkyl)C(O)-, -SO₂F, (C1-C3 alkoxy)C1-C3 alkoxy,-CH₂OC(O)N(R⁶⁶)₂, -CH₂NHC(O)OC1-C6 alkyl, -CH₂NHC(O)N(R^{6b})₂, -CH₂NHC(O)C1-C6 alkyl,-CH₂(pyrazolyl), -CH₂NHSO₂C1-C6 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or-CH₂heterocyclyl, where the phenyl involved in the above groups is optionally substituted with-C(O)H or OH; the heterocyclyl in -C1-C3 alkyl-heterocyclyl is optionally substituted with oxo; Q is a bond or O; R^{6b} is each independently hydrogen, C1-C6 alkyl, C1-C6 deuterated alkyl or C1-C6 haloalkyl; or two R^{6b} each independently form, together with the nitrogen atom to which they are attached, a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and the C3-C6 cycloalkyl is optionally substituted with 1 or 2 C1-C6 alkyl.

In one embodiment, R^{6a} is each independently halogen, hydroxyl, C1-C3 hydroxylalkyl, C1-C3 alkyl, C2-C4 alkenyl, haloC2-C4 alkenyl, C2-C4 alkynyl, haloC2-C4 alkynyl, C1-C3 haloalkyl, C1-C3 alkoxy, C1-C3 haloalkoxy, C3-C6 cycloalkyl, cyano, -Q-phenyl, -Q-phenyl-SO₂F, -NHC(O)phenyl, -NHC(O)phenyl-SO₂F, C1-C3 alkyl substituted pyrazolyl, aryl-C1-C3 alkyl-, t-butyldimethylsilyloxyCH₂-, -N(R^{6b})₂, (C1-C3 alkoxy)C1-C3 alkyl, (C1-C3 alkyl)C(O)-, oxo, (C1-C3 haloalkyl)C(O)-, -SO₂F, (C1-C3 alkoxy)C1-C3 alkoxy,-CH₂OC(O)N(R^{6b})₂, -CH₂NHC(O)OC1-C3 alkyl, -CH₂NHC(O)N(R^{6b})₂, -CH₂NHC(O)C1-C3 alkyl,-CH₂(pyrazolyl), -CH₂NHSO₂C1-C3 alkyl, -C1-C3 alkyl-OC(O)heterocyclyl, -OC(O)N(R^{6b})₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl, -OC(O) C1-C3 alkyl, -OC(O)C1-C3 haloalkyl, -C1-C3 alkyl-OC(O)C1-C3 alkyl, -C1-C3 alkyl-OC(O)C1-C3 haloalkyl, -OC(O)phenyl, -C1-C3 alkyl-OC(O)phenyl or-CH₂heterocyclyl, where the phenyl involved in the above groups is optionally substituted with-C(O)H or OH; the heterocyclyl in the -C1-C3 alkyl-heterocyclyl is optionally substituted with oxo; Q is a bond or O; R^{6b} is each independently hydrogen, C1-C3 alkyl, C1-C3 deuterated alkyl or C1-C3 haloalkyl; or two R^{6b} each independently form, together with the nitrogen atom to which they are attached, a 3- to 6-membered nitrogen containing heterocyclic group, where the 3- to 6-membered nitrogen containing heterocyclic groupis optionally substituted with 1 or 2 groups selected from halogen, methyl, trifluoromethyl, methoxy, and trifluoromethoxy; and the C3-C6 cycloalkyl is optionally substituted with 1 or 2 C1-C6 alkyl.

In one embodiment, R^{6a} is each independently halogen, hydroxyl, C1-C3 alkyl, C2-C4 alkenyl, C2-C4 haloalkenyl, C2-C4 alkynyl, C1-C3 haloalkyl, C3-C6 cycloalkyl, cyano, -N(R^{6b})₂, where R^{6b} is each independently hydrogen, C1-C3 alkyl, C1-C3 deuterated alkyl or C1-C3 haloalkyl; or two R^{6b} each independently form, together with the nitrogen atom to which they are attached, a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and the C3-C6 cycloalkyl is optionally substituted with 1 or 2 methyl.

In one embodiment, R^{6a} is each independently halogen, hydroxyl, methyl, ethyl, propyl, ethenyl, haloethenyl (for example, monofluoroethenyl, and difluoroethenyl), ethynyl, propynyl, halomethyl, haloethyl, cyclopropyl, cyano, or -N(R^{6b})₂, where R^{6b} is each independently hydrogen or methyl; and the cyclopropyl is optionally substituted with 1 or 2 methyl.

In some embodiments, the compound is represented by Formula (I): 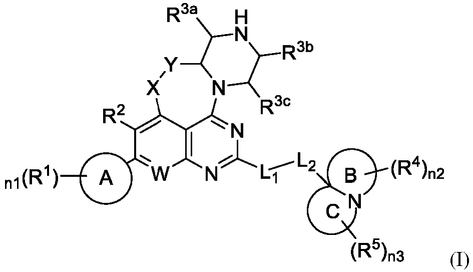 where in the formula,
X, Y, W, ring A, R¹, n1, and R² are each as defined above;
R^{3a}, R^{3b}, and R^{3c} are each independently hydrogen, halogen, hydroxyl, cyano, C1-C3 alkyl, C1-C3 deuterated alkyl or C1-C3 haloalkyl;
L₁ is a bond, O or NR³⁴;
L₂ is C1-C4 alkylene or heteroaryl, where 1 or 2 hydrogen atom(s) on any carbon atom(s) in the C1-C4 alkylene is/are optionally each independently substituted with deuterium, C1-C6 alkyl or C1-C6 haloalkyl; or 2 hydrogen atoms on any carbon atom in the C1-C4 alkylene are optionally both replaced by -(CH₂)ₘ₃-, -(CH₂)ₘ₁-O-(CH₂)ₘ₂-, or -(CH₂)ₘ₁-N-(CH₂)ₘ₂-, to form a cyclic substituent; m3 is 1 or 2; m1 is each independently 0, 1, 2 or 3; m2 is each independently 0, 1, 2 or 3; and m1 and m2 are not both 0;
ring B is a 3- to 6-membered nitrogen-containing heterocyclic group;
ring C is a 3- to 6-membered nitrogen containing heterocyclic group;
R⁴ is a substituent at any position on ring B; n2 is 0, 1, 2 or 3;
R⁵ is a substituent at any position on ring C; n3 is 0, 1, 2 or 3;
R⁴ and R⁵ are defined as follows:
   (a) each R⁴ and each R⁵ are each independently halogen, hydroxyl, C1-C3 hydroxylalkyl, C1-C3 alkyl, C1-C3 haloalkyl, C1-C3 alkoxy, C2-C6 alkenyl, C2-C6 haloalkenyl, C2-C4 alkenylene, C2-C4 halo alkenylene, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂,-CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl,-OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the-CH₂heterocyclyl is optionally substituted with oxo; or
   (b) 1 R⁴ is -CH₂CH₂- or -CH₂CH₂CH₂-, and forms, together with the carbon atom to which it is attached, cyclopropyl or cyclobutyl; the remaining R⁴ and each R⁵ is each independently halogen, hydroxyl, C1-C3 hydroxylalkyl, C1-C3 alkyl, C1-C3 haloalkyl, C1-C3 alkoxy, C2-C6 alkenyl, C2-C6 haloalkenyl, C2-C4 alkenylene, C2-C4 halo alkenylene, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl,-CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo; or
   (*c*) 1 R⁵ is -CH₂CH₂- or -CH₂CH₂CH₂-, and forms, together with the carbon atom to which it is attached, cyclopropyl or cyclobutyl; the remaining R⁵ and each R⁴ are each independently halogen, hydroxyl, C1-C3 hydroxylalkyl, C1-C3 alkyl, C1-C3 haloalkyl, C1-C3 alkoxy, C2-C6 alkenyl, C2-C6 haloalkenyl, C2-C4 alkenylene, C2-C4 halo alkenylene, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl,-CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo; or
   (d) 1 R⁴ is -CH₂CH₂- or -CH₂CH₂CH₂-, and forms, together with the carbon atom to which it is attached, cyclopropyl or cyclobutyl; 1 R⁵ is -CH₂CH₂- or -CH₂CH₂CH₂-, and forms, together with the carbon atom to which it is attached, cyclopropyl or cyclobutyl; the remaining R⁴ and the remaining R⁵ are each independently halogen, hydroxyl, C1-C3 hydroxylalkyl, C1-C3 alkyl, C1-C3 haloalkyl, C2-C4 alkenylene, C2-C4 halo alkenylene, C1-C3 alkoxy, C2-C6 alkenyl, C2-C6 haloalkenyl, cyano, -phenyl, -phenyl-SO₂F, -O-phenyl, -O-phenyl-SO₂F, - NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂,-CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂,-OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂,-OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo;
R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic groupis optionally substituted with 1 or 2 groups selected from halogen, methyl, trifluoromethyl, methoxy, and trifluoromethoxy.

In some embodiments, the compound is represented by Formula (I): 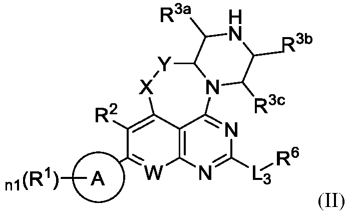
where in the formula, X, Y, W, ring A, R¹, n1, R², L₃, and R⁶ are each as defined above;
R^{3a} and R^{3b} are linked to form -CHR^{3a1}- or -CHR^{3a2}CHR^{3a3}-; and R^{3c} is hydrogen, halogen, hydroxyl, cyano, C1-C3 alkyl, C1-C3 deuterated alkyl or C1-C3 haloalkyl, where R^{3a1}, R^{3a2}, and R^{3a3} are each independently hydrogen, halogen, hydroxyl, cyano, C1-C3 alkyl, C1-C3 deuterated alkyl or C1-C3 haloalkyl; or
R^{3a} and R^{3c} are linked to form -CHR^{3a1}- or -CHR^{3a2}CHR^{3a3}-; and R^{3b} is hydrogen, halogen, hydroxyl, cyano, C1-C3 alkyl, C1-C3 deuterated alkyl or C1-C3 haloalkyl, where R^{3a1}, R^{3a2}, and R^{3a3} are each independently hydrogen, halogen, hydroxyl, cyano, C1-C3 alkyl, C1-C3 deuterated alkyl or C1-C3 haloalkyl.

In some embodiments, the compound is represented by Formula (II-1) or Formula (II-2): 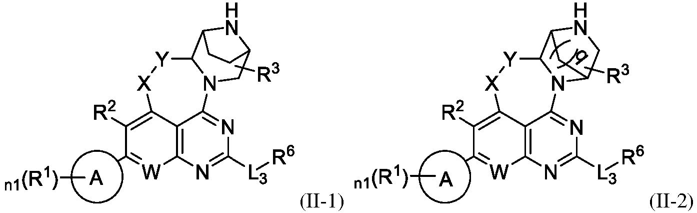 where in each formula, q is 1 or 2; R³ is substituent at any position on a bridged ring (the bridged ring bearing R³ herein is generally a bridged ring as shown in 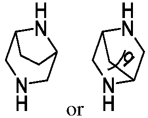 ) and is each independently hydrogen, halogen, hydroxyl, cyano, C1-C3 alkyl, C1-C3 deuterated alkyl or C1-C3 haloalkyl; and R¹, n1, ring A, R², X, Y, W, L₃, and R⁶ are each as defined above.

In some embodiments, the compound is represented by Formula (III): 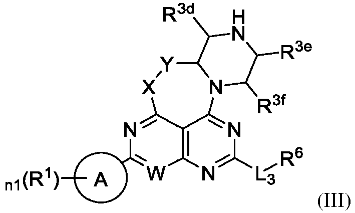
where in the formula, X, Y, W, ring A, R¹, n1, L₃, and R⁶ are each as defined above;
R^{3d}, R^{3e}, and R^{3f} are each independently hydrogen, halogen, hydroxyl, cyano, C1-C3 alkyl, C1-C3 deuterated alkyl or C1-C3 haloalkyl; or
R^{3d} and R^{3e} are linked to form -CHR^{3d1}- or -CHR^{3d2}CHR^{3d3}-; and R^{3f} is hydrogen, halogen, hydroxyl, cyano, C1-C3 alkyl, C1-C3 deuterated alkyl or C1-C3 haloalkyl, where R^{3d1}, R^{3d2}, and R^{3d3} are each independently hydrogen, halogen, hydroxyl, cyano, C1-C3 alkyl, C1-C3 deuterated alkyl or C1-C3; or
R^{3d} and R^{3f} are linked to form -CHR^{3f1}- or -CHR^{3f2}CHR^{3f3}-; and R^{3e} is hydrogen, halogen, hydroxyl, cyano, C1-C3 alkyl, C1-C3 deuterated alkyl or C1-C3 haloalkyl, where R^{3f1}, R^{3f2}, and R^{3f3} are each independently hydrogen, halogen, hydroxyl, cyano, C1-C3 alkyl, C1-C3 deuterated alkyl or C1-C3 haloalkyl.

In one embodiment, the present invention provides a compound of Formula (I) or a pharmaceutically acceptable salt, stereoisomer or solvate thereof: 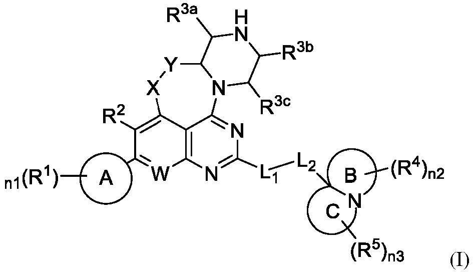
where in the formula, X is O or NR¹¹, in which R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, cyano, C1-C3 alkyl, C1-C3 deuterated alkyl, and C1-C3 haloalkyl; and R¹⁶, R¹⁷, R¹⁸, and R¹⁹ are each independently selected from H, C1-C3 alkyl and halo;
W is N or CR²⁰, where R²⁰ is H, cyano, C1-C6 alkyl, halogen, C1-C6 haloalkyl, C1-C6 alkoxy, cycloalkyl, cycloalkyl-O-, heterocyclyl or heterocyclyl-O-, in which the cycloalkyl and the heterocyclyl are each independently substituted with halo;
ring A is selected from the group consisting of aryl and heteroaryl;
R¹ is a substituent at any position on ring A; and R¹ is each independently selected from C1-C6 alkyl, halogen, hydroxyl, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyl alkynyl, C1-C6cyano alkyl, triazolyl, -S-C1-C6 haloalkyl, C1-C6 hydroxylalkyl, -CH₂C(O)NR²⁷R²⁸, -C2-C6 alkynylNR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, in which
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxyl, cyano, heterocyclyl, NR²⁵¹R²⁵², C(O)NR²⁵³R²⁵⁴ or SO₂C1-C6 alkyl, where R²⁵¹, R²⁵², R²⁵³, and R²⁵⁴ are each independently H or C1-C6 alkyl; R²⁵¹ and R²⁵², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and R²⁵³ and R²⁵⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
n1 is 0, 1, 2, or 3;
R² is H, cyano, halogen, C1-C6 alkyl, C1-C6 alkoxy, C1-C6 haloalkyl, C1-C6 haloalkoxy, C1-C6 deuterated alkoxy, NR³¹R³², C2-C4 alkynyl or CH₂OR³³, where R³¹, R³², and R³³ are each independently hydrogen or C1-C6 alkyl;
R^{3a}, R^{3b}, and R^{3c} are each independently hydrogen, halogen, hydroxyl, cyano, C1-C3 alkyl, C1-C3 deuterated alkyl or C1-C3 haloalkyl;
L₁ is a bond, O or NR³⁴;
L₂ is C1-C4 alkylene or heteroaryl, where 1 or 2 hydrogen atom(s) on any carbon atom(s) in the C1-C4 alkylene is/are optionally each independently substituted with deuterium, C1-C6 alkyl or C1-C6 haloalkyl; or 2 hydrogen atoms on any carbon atom in the C1-C4 alkylene are optionally both replaced by -(CH₂)ₘ₃-, -(CH₂)ₘ₁-O-(CH₂)ₘ₂-, or -(CH₂)ₘ₁-N-(CH₂)ₘ₂-, to form a cyclic substituent; m3 is 1 or 2; m1 is each independently 0, 1, 2 or 3; m2 is each independently 0, 1, 2 or 3; and m1 and m2 are not both 0;
ring B is a 3- to 6-membered nitrogen-containing heterocyclic group;
ring C is a 3- to 6-membered nitrogen containing heterocyclic group;
R⁴ is a substituent at any position on ring B; n2 is 0, 1, 2 or 3;
R⁵ is a substituent at any position on ring C; n3 is 0, 1, 2 or 3;
R⁴ and R⁵ are defined as follows:
   (a) each R⁴ and each R⁵ are each independently halogen, hydroxyl, C1-C3 hydroxylalkyl, C1-C3 alkyl, C1-C3 haloalkyl, C1-C3 alkoxy, cyano, -phenyl, -phenyl-SO₂F, -O-phenyl, -O-phenyl-SO₂F, -NHC(O)phenyl, - NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂, -CH₂NHC(O)C1-C6 alkyl,-CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo; or
   (b) 1 R⁴ is -CH₂CH₂- or -CH₂CH₂CH₂-, and forms, together with the carbon atom to which it is attached, cyclopropyl or cyclobutyl; the remaining R⁴ and each R⁵ is each independently halogen, hydroxyl, C1-C3 hydroxylalkyl, C1-C3 alkyl, C1-C3 haloalkyl, C1-C3 alkoxy, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂,-CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂,-OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂,-OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo; or
   (c) 1 R⁵ is -CH₂CH₂- or -CH₂CH₂CH₂-, and forms, together with the carbon atom to which it is attached, cyclopropyl or cyclobutyl; the remaining R⁵ and each R⁴ are each independently halogen, hydroxyl, C1-C3 hydroxylalkyl, C1-C3 alkyl, C1-C3 haloalkyl, C1-C3 alkoxy, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl, -CH₂NHC(O)N(R³⁴)₂,-CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂,-OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂,-OC(O)NH(C1-C3 alkyl)O(C1-C3 alkyl)phenyl, -OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the -CH₂heterocyclyl is optionally substituted with oxo; or
   (d) 1 R⁴ is -CH₂CH₂- or -CH₂CH₂CH₂-, and forms, together with the carbon atom to which it is attached, cyclopropyl or cyclobutyl; 1 R⁵ is -CH₂CH₂- or -CH₂CH₂CH₂-, and forms, together with the carbon atom to which it is attached, cyclopropyl or cyclobutyl; the remaining R⁴ and the remaining R⁵ are each independently halogen, hydroxyl, C1-C3 hydroxylalkyl, C1-C3 alkyl, C1-C3 haloalkyl, C1-C3 alkoxy, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, -CH₂NHC(O)OC1-C6 alkyl,-CH₂NHC(O)N(R³⁴)₂, -CH₂NHC(O)C1-C6 alkyl, -CH₂(pyrazolyl), -CH₂NHSO₂C1-C6 alkyl,-CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl,-OC(O)heterocyclyl or -CH₂heterocyclyl, where the phenyl in the -NHC(O)phenyl or -OC(O)NH(C1-C3 alkyl)O(C1-C3 alkyl)phenyl is optionally substituted with-C(O)H or OH; and the heterocyclyl in the-CH₂heterocyclyl is optionally substituted with oxo; and
R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy.

In some embodiments, the compound is represented by Formula (I-1) or Formula (I-2): 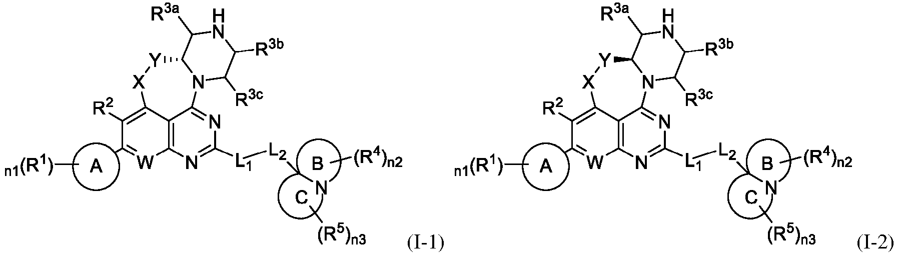 where in each formula, X, Y, W, R¹, R², R^{3a}, R^{3b}, R^{3c}, R⁴, R⁵, n1, n2, n3, L₁, L₂, ring A, ring B, and ring C are each as defined in Formula (I).

In one embodiment, the present invention provides a compound of Formula (II-1) or Formula (II-2) or a pharmaceutically acceptable salt, stereoisomer or solvate thereof: 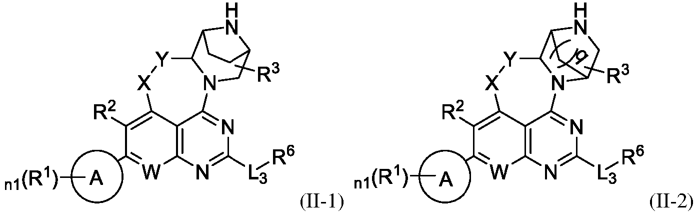 where in each formula,
q is 1 or 2;
X is O or NR¹¹, in which R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, cyano, C1-C3 alkyl, C1-C3 deuterated alkyl, and C1-C3 haloalkyl; and R¹⁶, R¹⁷, R¹⁸, and R¹⁹ are each independently selected from H, C1-C3 alkyl and halo;
W is N or CR²⁰, where R²⁰ is H, cyano, C1-C6 alkyl, halogen, C1-C6 haloalkyl, C1-C6 alkoxy, cycloalkyl, cycloalkyl-O-, heterocyclyl or heterocyclyl-O-, in which the cycloalkyl and the heterocyclyl are each independently substituted with halo;
ring A is selected from the group consisting of aryl and heteroaryl;
R¹ is a substituent at any position on ring A; and R¹ is each independently selected from C1-C6 alkyl, halogen, hydroxyl, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyl alkynyl, C1-C6cyano alkyl, triazolyl, -S-C1-C6 haloalkyl, C1-C6 hydroxylalkyl, -CH₂C(O)NR²⁷R²⁸, -C2-C6 alkynylNR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, in which
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxyl, cyano, heterocyclyl, NR²⁵¹R²⁵², C(O)NR²⁵³R²⁵⁴ or SO₂C1-C6 alkyl, where R²⁵¹, R²⁵², R²⁵³, and R²⁵⁴ are each independently H or C1-C6 alkyl; R²⁵¹ and R²⁵², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and R²⁵³ and R²⁵⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
n1 is 0, 1, 2, or 3;
R² is H, cyano, halogen, C1-C6 alkyl, C1-C6 alkoxy, C1-C6 haloalkyl, C1-C6 haloalkoxy, C1-C6 deuterated alkoxy, NR³¹R³², C2-C4 alkynyl or CH₂OR³³, where R³¹, R³², and R³³ are each independently hydrogen or C1-C6 alkyl;
R³ is a substitutent at any position on a bridged ring and is hydrogen, halogen, hydroxyl, cyano, C1-C3 alkyl, C1-C3 deuterated alkyl or C1-C3 haloalkyl;
L₃ is a bond, O or NR³⁴;
R⁶ is -L-6 to 10-membered fused heterocyclyl, -L-7 to 11-membered spiro heterocyclyl, -L-spiro-substituted 6 to 10-membered fused heterocyclyl, -L-spiro-substituted 7 to 11-membered spiro heterocyclyl, -L-parallel-substituted 6 to 10-membered fused heterocyclyl, -L-parallel-substituted 7 to 11-membered spiro heterocyclyl, or -L-spiro- and parallel-substituted 6 to 10-membered fused heterocyclyl;
the spiro-substituted 6- to 10-membered fused heterocyclyl refers to a spiro-substituted 6- to 10-membered fused heterocyclic group formed when 2 hydrogen atoms on the same carbon atom in any 1 or 2 carbon atoms in a 6- to 10-membered fused heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the spiro-substituted 7- to 11-membered spiro heterocyclyl refers to a spiro-substituted 7- to 11-membered spiro heterocyclic group formed when 2 hydrogen atoms on the same carbon atom in any 1 or 2 carbon atoms in a 7- to 11-membered spiro heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the parallel-substituted 6- to 10-membered fused heterocyclyl refers to a parallel-substituted 6- to 10-membered fused heterocyclic group formed when 1 hydrogen atom on each carbon atom in the same pair of carbon atoms in any 1 or 2 pairs of adjacent carbon atoms in a 6- to 10-membered fused heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 6- to 10-membered fused heterocyclyl refers to a parallel-substituted 6- to 10-membered fused heterocyclic group formed when hydrogen atoms on any 2 non-adjacent carbon atoms in a 6- to 10-membered fused heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the parallel-substituted 7- to 11-membered spiro heterocyclyl refers to a parallel-substituted 7- to 11-membered spiro heterocyclic group formed when 1 hydrogen atom on each carbon atom in the same pair of carbon atoms in any 1 or 2 pairs of adjacent carbon atoms in a 7- to 11-membered spiro heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 7- to 11-membered spiro heterocyclyl refers to a parallel-substituted 7- to 11-membered spiro heterocyclic group formed when hydrogen atoms on any two non-adjacent carbon atoms in a 7- to 11-membered spiro heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the spiro- and parallel-substituted 6- to 10-membered fused heterocyclyl refers to a spiro- and parallel-substituted 6- to 10-membered fused heterocyclic group formed when 1 hydrogen atom on each carbon atom in any 1 pair of adjacent carbon atoms in a 6- to 10-membered fused heterocyclic group is replaced by -(CH₂)ₘ₄-,-(CH₂)ₘ₅O(CH₂)ₘ₆-, or -(CH₂)ₘ₇NRⁿ(CH₂)ₘ₈ to form a parallel-substitution, and 2 hydrogen atoms on another carbon atom in the 6- to 10-membered fused heterocyclic group are both replaced by -(CH₂)ₘ₄-, -(CH₂)ₘ₅O(CH₂)ₘ₆-, or -(CH₂)ₘ₇NRⁿ(CH₂)ₘ₈ to form a spiro-substitution;
the 6- to 10-membered fused heterocyclyl or the 7- to 11-membered spiro heterocyclyl have 1, 2, 3 or 4 heteroatoms selected from N(R^{m}), S, S(=O), S(=O)₂, O as ring atoms;
the spiro-substituted 6- to 10-membered fused heterocyclyl, the spiro-substituted 7- to 11-membered spiro heterocyclyl, the parallel-substituted 6- to 10-membered fused heterocyclyl, the parallel-substituted 7- to 11-membered spiro heterocyclyl, or the spiro- and parallel-substituted 6- to 10-membered fused heterocyclyl each independently has 1, 2, 3, 4 or 5 heteroatoms selected from N(R^{m}), S, S(=O), S(=O)₂, and O as ring atoms;
Rⁿ is hydrogen, C1-C4 alkyl, deuterated C1-C4 alkyl, C1-C4 haloalkyl, C3-C20 cycloalkyl, or a 3-20 membered heterocyclyl;
R^{m} is absent, hydrogen, C1-C4 alkyl, deuterated C1-C4 alkyl, C1-C4 haloalkyl, C3-C20 cycloalkyl or 3-20 membered heterocyclyl;
where the 6- to 10-membered fused heterocyclyl or the 7- to 11-membered spiro heterocyclyl is saturated or partially unsaturated, and when the 6- to 10-membered fused heterocyclyl or the 7- to 11-membered spiro heterocyclyl is partially unsaturated, the ring has 1 or 2 double bonds; 2 hydrogen atoms on the same carbon atom in any 1 or 2 carbon atoms in the 6- to 10-membered fused heterocyclyl or the 7- to 11-membered spiro heterocyclyl are optionally both replaced by =CR^{2a}R^{2b}; and the 6- to 10-membered fused heterocyclyl or the 7- to 11-membered spiro heterocyclyl is optionally further substituted with 1 or more (for example, 1, 2, 3, 4 or 5) R^{6a},
where R^{2a} and R^{2b} are each independently hydrogen, halogen, cyano, C1-C4 alkyl, C1-C4 haloalkyl, C6-C10 aryl or a 5-membered or 6-membered heteroaryl, in which the C6-C10 aryl or the 5-membered or 6-membered heteroaryl is optionally substituted with 1, 2 or 3 groups selected from halogen, cyano, C1-C4 alkyl, C1-C4 alkoxy, C1-C4 haloalkyl or C1-C4 haloalkoxy; or each pair of R^{2a} and R^{2b} independently forms, together with the carbon atoms to which they are attached, a C3-C6 monocyclic cycloalkyl or a 3- to 6-membered monocyclic heterocyclic group, in which the C3-C6 monocyclic cycloalkyl or the 3- to 6-membered monocyclic heterocyclic group is optionally substituted with 1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl;
R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O)phenyl, -NHC(O)phenyl-SO₂F, C1-C3 alkyl substituted pyrazolyl, aryl-C1-C3 alkyl-, t-butyldimethylsilyloxyCH₂-,-N(R^{6b})₂, (C1-C3 alkoxy)C1-C3 alkyl, (C1-C3 alkyl)C(O)-, oxo, (C1-C3 haloalkyl)C(O)-, -SO₂F, (C1-C3 alkoxy)C1-C3 alkoxy, -CH₂OC(O)N(R^{6b})₂, -CH₂NHC(O)OC1-C6 alkyl, -CH₂NHC(O)N(R^{6b})₂, -CH₂NHC(O)C1-C6 alkyl, -CH₂(pyrazolyl), -CH₂NHSO₂C1-C6 alkyl, -C1-C3 alkyl-OC(O)heterocyclyl, -OC(O)N(R^{6b})₂,-OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂,-OC(O)NH(C1-C3 alkyl)O(C1-C3 alkyl)phenyl, -OC(O)heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂heterocyclyl, in which the phenyl involved in the above groups is optionally substituted with-C(O)H or OH; the heterocyclyl in the -C1-C3 alkyl-heterocyclyl is optionally substituted with oxo; Q is a bond or O; R^{6b} is each independently hydrogen, C1-C6 alkyl, C1-C6 deuterated alkyl or C1-C6 haloalkyl; or two R^{6b} each independently form, together with the nitrogen atom to which they are attached, a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
each m4 is 2, 3 or 4;
m5, m6, m7, and m8 are each independently 0, 1, 2, or 3; m5 and m6 are not both 0; m7 and m8 are not both 0;
   or
R⁶ is hydrogen, -N(R³⁴)₂, heterocyclyl, C1-C6 alkyl, -L-heterocyclyl, -L-aryl, -L-heteroaryl, -L- cycloalkyl,-L-N(R³⁴)₂, -L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-aryl, -L-COOH or -L-C(O)OC1-C6 alkyl, where the heterocyclyl, the aryl in the -L-NR³⁴C(O)-aryl, the heterocyclyl in the -L-heterocyclyl, and the cycloalkyl in the -L- cycloalkyl are each optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; and the aryl in the -L-aryl and the heteroaryl in the -L-heteroaryl are each optionally substituted with one or more (for example, 1, 2, 3, 4 or 5) R³⁶,
where L is each independently a bond, C1-C4 alkylene or heteroaryl, in which 1 or 2 hydrogen atom(s) on any carbon atom(s) in the C1-C4 alkylene is/are optionally each independently substituted with deuterium, C1-C6 alkyl, or C1-C6 haloalkyl; or 2 hydrogen atoms on any carbon atom in the C1-C4 alkylene are optionally both replaced by -(CH₂)ₘ₃-, -(CH₂)ₘ₁-O-(CH₂)ₘ₂-, or -(CH₂)ₘ₁-NR⁹-(CH₂)ₘ₂-, to form a cyclic substituent; m3 is 1 or 2; m1 is each independently 0, 1, 2 or 3; m2 is each independently 0, 1, 2 or 3; and m1 and m2 are not both 0;
R⁹ is hydrogen, C1-C6 alkyl, C1-C6 deuterated alkyl, C1-C6 haloalkyl, C1-C6 alkyl-hydroxyl, C1-C6 alkyl-cyano, C1-C6 alkoxy, C1-C6 alkyl-C1-C6 alkoxy, C3-C6 monocyclic cycloalkyl, C3-C6 deuterated cycloalkyl or C3-C6 halocycloalkyl;
R³⁵ is each independently halogen, hydroxyl, C1-C3 alkyl, C1-C3 haloalkyl, C1-C3 alkoxy, C3-C6 cycloalkyl, -C1-C3 alkyl-C1-C3 alkoxy, -C1-C3 alkyl-hydroxyl, heterocyclyl (for example, a 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂,-CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl,-OC(O)heterocyclyl (for example, -OC(O)-3- to 6-membered heterocyclyl) or -CH₂heterocyclyl (for example,-CH₂-3- to 6-membered heterocyclyl), where the phenyl in the -NHC(O)phenyl or -OC(O)NH(C1-C3 alkyl)O(C1-C3 alkyl)phenyl is optionally substituted with-C(O)H or OH; the heterocyclyl in the -CH₂heterocyclyl is optionally substituted with oxo; and the hydrogen atom on the -C1-C3 alkyl- is substituted with 1 or 2 groups selected from halogen, deuterium, C1-C3 alkyl, and C1-C3 haloalkyl;
R³⁶ is each independently halogen, hydroxyl, HC(O)-, C1-C4 alkyl, C1-C4 alkoxy, C1-C4 haloalkyl, C1-C4 hydroxylalkyl or -N(R³⁴)₂; and
R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy.

In some embodiments, the compound is represented by Formula (II-1A), Formula (II-1B), Formula (II-2A), and Formula (II-2B): 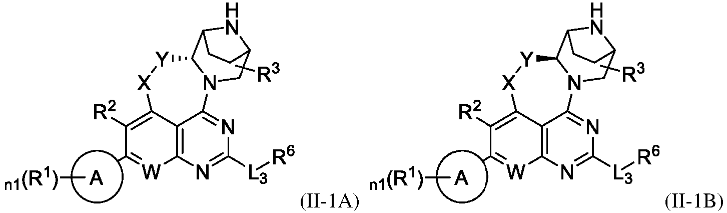 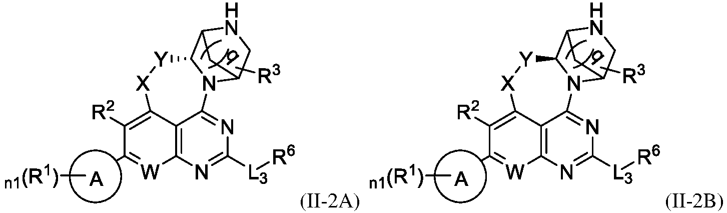 where in each formula, X, Y, W, R¹, R², R³, R⁶, n1, L₃, q, and ring A are each as defined in Formula (II-1) or Formula (II-2).

In one embodiment, the compound is represented by Formula (II-1A1), formula (II-1A2), Formula (II-1B1), Formula (II-1B2), Formula (II-1C1) or Formula (II-1C2): 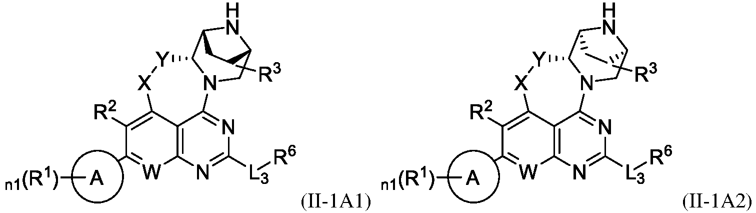 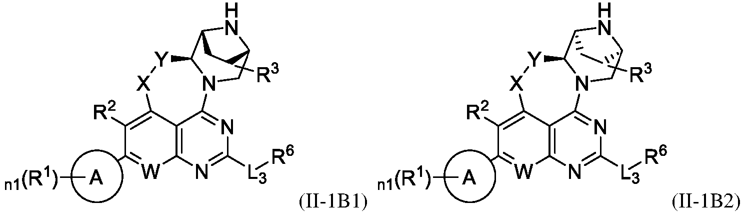 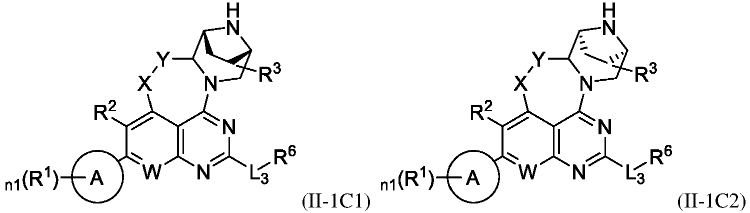 where in each formula, X, Y, W, R¹, R², R³, R⁶, n1, L₃, and ring A are each as defined in Formula (II-1) or Formula (II-2).

In one embodiment, the present invention provides a compound of Formula (III) or a pharmaceutically acceptable salt, stereoisomer or solvate thereof: 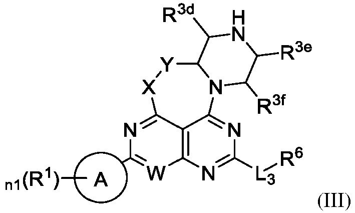 where in the formula,
X is O or NR¹¹, in which R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, cyano, C1-C3 alkyl, C1-C3 deuterated alkyl, and C1-C3 haloalkyl; and R¹⁶, R¹⁷, R¹⁸, and R¹⁹ are each independently selected from H, C1-C3 alkyl and halo;
W is N or CR²⁰, where R²⁰ is H, cyano, C1-C6 alkyl, halogen, C1-C6 haloalkyl, C1-C6 alkoxy, cycloalkyl, cycloalkyl-O-, heterocyclyl or heterocyclyl-O-, in which the cycloalkyl and the heterocyclyl are each independently substituted with halo;
ring A is selected from the group consisting of aryl and heteroaryl;
R¹ is a substituent at any position on ring A; and R¹ is each independently selected from C1-C6 alkyl, halogen, hydroxyl, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyl alkynyl, C1-C6cyano alkyl, triazolyl, -S-C1-C6 haloalkyl, C1-C6 hydroxylalkyl, -CH₂C(O)NR²⁷R²⁸, -C2-C6 alkynylNR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, in which
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxyl, cyano, heterocyclyl, NR²⁵¹R²⁵², C(O)NR²⁵³R²⁵⁴ or SO₂C1-C6 alkyl, where R²⁵¹, R²⁵², R²⁵³, and R²⁵⁴ are each independently H or C1-C6 alkyl; R²⁵¹ and R²⁵², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and R²⁵³ and R²⁵⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
n1 is 0, 1, 2, or 3;
R^{3d}, R^{3e}, and R^{3f} are each independently hydrogen, halogen, hydroxyl, cyano, C1-C3 alkyl, C1-C3 deuterated alkyl or C1-C3 haloalkyl; or
R^{3d} and R^{3e} are linked to form -CHR^{3d1}- or -CHR^{3d2}CHR^{3d3}-; and R^{3f} is hydrogen, halogen, hydroxyl, cyano, C1-C3 alkyl, C1-C3 deuterated alkyl or C1-C3 haloalkyl, where R^{3d1}, R^{3d2}, and R^{3d3} are each independently hydrogen, halogen, hydroxyl, cyano, C1-C3 alkyl, C1-C3 deuterated alkyl or C1-C3; or
R^{3d} and R^{3f} are linked to form -CHR^{3f1}- or -CHR^{3f2}CHR^{3f3}-; and R^{3e} is hydrogen, halogen, hydroxyl, cyano, C1-C3 alkyl, C1-C3 deuterated alkyl or C1-C3 haloalkyl, where R^{3f1}, R^{3f2}, and R^{3f3} are each independently hydrogen, halogen, hydroxyl, cyano, C1-C3 alkyl, C1-C3 deuterated alkyl or C1-C3 haloalkyl;
L₃ is a bond, O or NR³⁴;
R⁶ is -L-6 to 10-membered fused heterocyclyl, -L-7 to 11-membered spiro heterocyclyl, -L-spiro-substituted 6 to 10-membered fused heterocyclyl, -L-spiro-substituted 7 to 11-membered spiro heterocyclyl, -L-parallel-substituted 6 to 10-membered fused heterocyclyl, -L-parallel-substituted 7 to 11-membered spiro heterocyclyl, or -L-spiro- and parallel-substituted 6 to 10-membered fused heterocyclyl;
the spiro-substituted 6- to 10-membered fused heterocyclyl refers to a spiro-substituted 6- to 10-membered fused heterocyclic group formed when 2 hydrogen atoms on the same carbon atom in any 1 or 2 carbon atoms in a 6- to 10-membered fused heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the spiro-substituted 7- to 11-membered spiro heterocyclyl refers to a spiro-substituted 7- to 11-membered spiro heterocyclic group formed when 2 hydrogen atoms on the same carbon atom in any 1 or 2 carbon atoms in a 7- to 11-membered spiro heterocyclic group are both replaced by -(CH₂)ₘ₄-, -(CH₂)ₘ₅O(CH₂)ₘ₆- or-(CH₂)ₘ₇NRⁿ(CH₂)ₘ₈;
the parallel-substituted 6- to 10-membered fused heterocyclyl refers to a parallel-substituted 6- to 10-membered fused heterocyclic group formed when 1 hydrogen atom on each carbon atom in the same pair of carbon atoms in any 1 or 2 pairs of adjacent carbon atoms in a 6- to 10-membered fused heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 6- to 10-membered fused heterocyclyl refers to a parallel-substituted 6- to 10-membered fused heterocyclic group formed when hydrogen atoms on any 2 non-adjacent carbon atoms in a 6- to 10-membered fused heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the parallel-substituted 7- to 11-membered spiro heterocyclyl refers to a parallel-substituted 7- to 11-membered spiro heterocyclic group formed when 1 hydrogen atom on each carbon atom in the same pair of carbon atoms in any 1 or 2 pairs of adjacent carbon atoms in a 7- to 11-membered spiro heterocyclic group is replaced by -(CH₂)ₘ₄-, -(CH₂)ₘ₅O(CH₂)ₘ₆-, or -(CH₂)ₘ₇NRⁿ(CH₂)ₘ₈; or the parallel-substituted 7- to 11-membered spiro heterocyclyl refers to a parallel-substituted 7- to 11-membered spiro heterocyclic group formed when hydrogen atoms on any two non-adjacent carbon atoms in a 7- to 11-membered spiro heterocyclic group are replaced by-(CH₂)ₘ₄-, -(CH₂)ₘ₅O(CH₂)ₘ₆-, or -(CH₂)ₘ₇NRⁿ(CH₂)ₘ₈;
the spiro- and parallel-substituted 6- to 10-membered fused heterocyclyl refers to a spiro- and parallel-substituted 6- to 10-membered fused heterocyclic group formed when 1 hydrogen atom on each carbon atom in any 1 pair of adjacent carbon atoms in a 6- to 10-membered fused heterocyclic group is replaced by -(CH₂)ₘ₄-,-(CH₂)ₘ₅O(CH₂)ₘ₆-, or -(CH₂)ₘ₇NRⁿ(CH₂)ₘ₈ to form a parallel-substitution, and 2 hydrogen atoms on another carbon atom in the 6- to 10-membered fused heterocyclic group are both replaced by -(CH₂)ₘ₄-, -(CH₂)ₘ₅O(CH₂)ₘ₆-, or -(CH₂)ₘ₇NRⁿ(CH₂)ₘ₈ to form a spiro-substitution;
the 6- to 10-membered fused heterocyclyl or the 7- to 11-membered spiro heterocyclyl have 1, 2, 3 or 4 heteroatoms selected from N(R^{m}), S, S(=O), S(=O)₂, O as ring atoms;
the spiro-substituted 6- to 10-membered fused heterocyclyl, the spiro-substituted 7- to 11-membered spiro heterocyclyl, the parallel-substituted 6- to 10-membered fused heterocyclyl, the parallel-substituted 7- to 11-membered spiro heterocyclyl, or the spiro- and parallel-substituted 6- to 10-membered fused heterocyclyl each independently has 1, 2, 3, 4 or 5 heteroatoms selected from N(R^{m}), S, S(=O), S(=O)₂, and O as ring atoms;
Rⁿ is hydrogen, C1-C4 alkyl, deuterated C1-C4 alkyl, C1-C4 haloalkyl, C3-C20 cycloalkyl, or a 3-20 membered heterocyclyl;
R^{m} is absent, hydrogen, C1-C4 alkyl, deuterated C1-C4 alkyl, C1-C4 haloalkyl, C3-C20 cycloalkyl or 3-20 membered heterocyclyl;
where the 6- to 10-membered fused heterocyclyl or the 7 - to 11-membered spiro heterocyclyl is saturated or partially unsaturated, and when the 6- to 10-membered fused heterocyclyl or the 7- to 11-membered spiro heterocyclyl is partially unsaturated, the ring has 1 or 2 double bonds; 2 hydrogen atoms on the same carbon atom in any 1 or 2 carbon atoms in the 6- to 10-membered fused heterocyclyl or the 7- to 11-membered spiro heterocyclyl are optionally both replaced by =CR^{2a}R^{2b}; and the 6- to 10-membered fused heterocyclyl or the 7- to 11-membered spiro heterocyclyl is optionally further substituted with 1 or more (for example, 1, 2, 3, 4 or 5) R^{6a},
where R^{2a} and R^{2b} are each independently hydrogen, halogen, cyano, C1-C4 alkyl, C1-C4 haloalkyl, C6-C10 aryl or a 5-membered or 6-membered heteroaryl, in which the C6-C10 aryl or the 5-membered or 6-membered heteroaryl is optionally substituted with 1, 2 or 3 groups selected from halogen, cyano, C1-C4 alkyl, C1-C4 alkoxy, C1-C4 haloalkyl or C1-C4 haloalkoxy; or each pair of R^{2a} and R^{2b} independently forms, together with the carbon atoms to which they are attached, a C3-C6 monocyclic cycloalkyl or a 3- to 6-membered monocyclic heterocyclic group, in which the C3-C6 monocyclic cycloalkyl or the 3- to 6-membered monocyclic heterocyclic group is optionally substituted with 1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl;
R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy, cyano, -Q-phenyl, -Q-phenyl-SO₂F, -NHC(O)phenyl, -NHC(O)phenyl-SO₂F, C1-C3 alkyl substituted pyrazolyl, aryl-C1-C3 alkyl-, t-butyldimethylsilyloxyCH₂-, - N(R^{6b})₂, (C1-C3 alkoxy)C1-C3 alkyl, (C1-C3 alkyl)C(O)-, oxo, (C1-C3 haloalkyl)C(O)-, -SO₂F, (C1-C3 alkoxy)C1-C3 alkoxy, -CH₂OC(O)N(R^{6b})₂, -CH₂NHC(O)OC1-C6 alkyl, -CH₂NHC(O)N(R^{6b})₂, -CH₂NHC(O)Cl-C6 alkyl, -CH₂(pyrazolyl), -CH₂NHSO₂C1-C6 alkyl, -C1-C3 alkyl-OC(O)heterocyclyl, -OC(O)N(R^{6b})₂, - OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, - OC(O)NH(C1-C3 alkyl)O(C1-C3 alkyl)phenyl, -OC(O)heterocyclyl, -OC(O)C1-C6 alkyl, -OC(O)C1-C6 haloalkyl, -C1-C3 alkyl-OC(O)C1-C6 alkyl, -C1-C3 alkyl-OC(O)C1-C6 haloalkyl, -OC(O)phenyl, -C1-C3 alkyl-OC(O)phenyl or -CH₂heterocyclyl, in which the phenyl involved in the above groups is optionally substituted with-C(O)H or OH; the heterocyclyl in the -C1-C3 alkyl-heterocyclyl is optionally substituted with oxo; Q is a bond or O; R^{6b} is each independently hydrogen, C1-C6 alkyl, C1-C6 deuterated alkyl or C1-C6 haloalkyl; or two R^{6b} each independently form, together with the nitrogen atom to which they are attached, a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
each m4 is 2, 3 or 4;
m5, m6, m7, and m8 are each independently 0, 1, 2, or 3; m5 and m6 are not both 0; m7 and m8 are not both 0;
   or
R⁶ is hydrogen, -N(R³⁴)₂, heterocyclyl, C1-C6 alkyl, -L-heterocyclyl, -L-aryl, -L-heteroaryl, -L- cycloalkyl, - L-N(R³⁴)₂, -L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-aryl, -L-COOH or -L-C(O)OC1-C6 alkyl, where the heterocyclyl, the aryl in the -L-NR³⁴C(O)-aryl, the heterocyclyl in the -L-heterocyclyl, and the cycloalkyl in the -L- cycloalkyl are each optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; and the aryl in the -L-aryl and the heteroaryl in the -L-heteroaryl are each optionally substituted with one or more (for example, 1, 2, 3, 4 or 5) R³⁶,
where L is each independently a bond, C1-C4 alkylene or heteroaryl, in which 1 or 2 hydrogen atom(s) on any carbon atom(s) in the C1-C4 alkylene is/are optionally each independently substituted with deuterium, C1-C6 alkyl, or C1-C6 haloalkyl; or 2 hydrogen atoms on any carbon atom in the C1-C4 alkylene are optionally both replaced by -(CH₂)ₘ₃-, -(CH₂)ₘ₁-O-(CH₂)ₘ₂-, or -(CH₂)ₘ₁-NR⁹-(CH₂)ₘ₂-, to form a cyclic substituent; m3 is 1 or 2; m1 is each independently 0, 1, 2 or 3; m2 is each independently 0, 1, 2 or 3; and m1 and m2 are not both 0;
R⁹ is hydrogen, C1-C6 alkyl, C1-C6 deuterated alkyl, C1-C6 haloalkyl, C1-C6 alkyl-hydroxyl, C1-C6 alkyl-cyano, C1-C6 alkoxy, C1-C6 alkyl-C1-C6 alkoxy, C3-C6 monocyclic cycloalkyl, C3-C6 deuterated cycloalkyl or C3-C6 halocycloalkyl;
R³⁵ is each independently halogen, hydroxyl, C1-C3 alkyl, C1-C3 haloalkyl, C1-C3 alkoxy, C3-C6 cycloalkyl, -C1-C3 alkyl-C1-C3 alkoxy, -C1-C3 alkyl-hydroxyl, heterocyclyl (for example, a 3- to 6-membered heterocyclyl), cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl-C1-C3 alkyl-, t-butyldimethylsilylCH₂-, -N(R³⁴)₂, (C1-C3 alkoxy)C1-C3 alkyl, (C1-C3 alkyl)C(O), oxo, (C1-C3 haloalkyl)C(O)-, -SO₂F, (C1-C3 alkoxy)C1-C3 alkoxy, -CH₂OC(O)N(R³⁴)₂, - CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O)heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl)phenyl, - OC(O)heterocyclyl (for example, -OC(O)-3- to 6-membered heterocyclyl) or -CH₂heterocyclyl (for example, - CH₂-3- to 6-membered heterocyclyl), where the phenyl in the -NHC(O)phenyl or -OC(O)NH(C1-C3 alkyl)O(C1-C3 alkyl)phenyl is optionally substituted with-C(O)H or OH; the heterocyclyl in the -CH₂heterocyclyl is optionally substituted with oxo; and the hydrogen atom on the -C1-C3 alkyl- is substituted with 1 or 2 groups selected from halogen, deuterium, C1-C3 alkyl, and C1-C3 haloalkyl;
R³⁶ is each independently halogen, hydroxyl, HC(O)-, C1-C4 alkyl, C1-C4 alkoxy, C1-C4 haloalkyl, C1-C4 hydroxylalkyl or -N(R³⁴)₂; and
R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy.

In some embodiments, the compound is represented by Formula (III-1) or Formula (III-2): 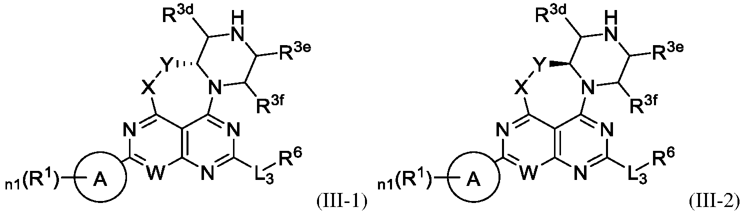 where in each formula, X, Y, W, R¹, R^{3d}, R^{3e}, R^{3f}, R⁶, n1, L₃, and ring A are each as defined in Formula (III).

In some embodiments, the compound is represented by Formula (III-1a), Formula (III-1b), Formula (III-2a), or Formula (III-2b): 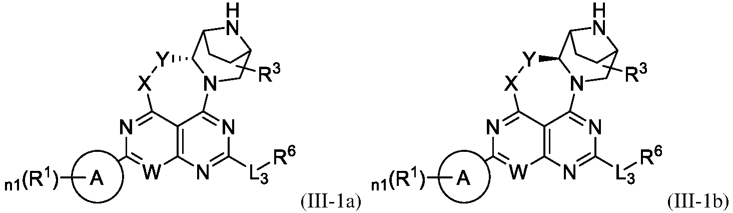 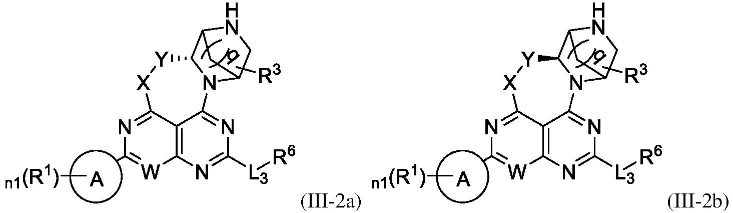 where in each formula, q is 1 or 2; R³ is a substitutent at any position on a bridged ring and is hydrogen, halogen, hydroxyl, cyano, C1-C3 alkyl, C1-C3 deuterated alkyl or C1-C3 haloalkyl; and X, Y, W, ring A, R¹, n1, L₃, and R⁶ are as defined in Formula (III-1) or Formula (III-2).

In one embodiment, the compound is represented by Formula (III-1a1), Formula (III-1a2), Formula (III-1b1), Formula (III-1b2), Formula (III-1cl) or Formula (III-1c2): 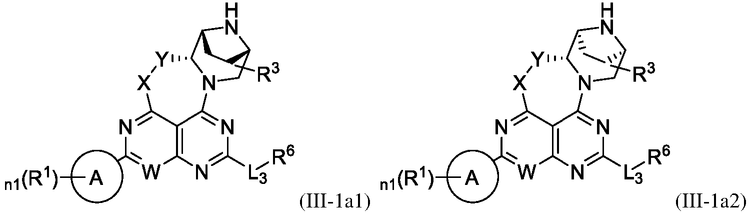 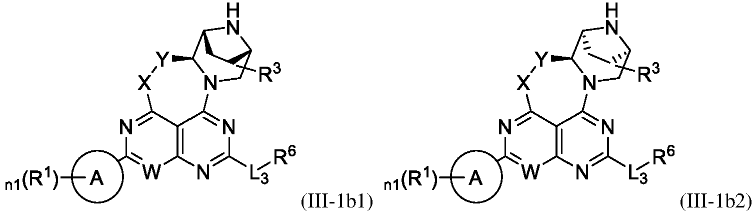 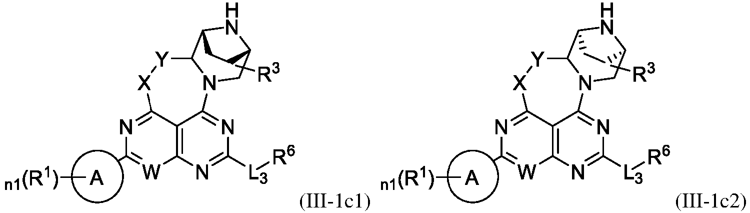 where in each formula, R³ is a substitutent at any position on a bridged ring and is hydrogen, halogen, hydroxyl, cyano, C1-C3 alkyl, C1-C3 deuterated alkyl or C1-C3 haloalkyl; and X, Y, W, ring A, R¹, n1, L₃, and R⁶ are as defined in Formula (III-1) or Formula (III-2).

In one embodiment, R³ is hydrogen.

In one embodiment, Y is -CH₂-.

In one embodiment, the compound is represented by Formula (III-1a3), Formula (III-1a4), Formula (III-1b3) or Formula (III-1b4): 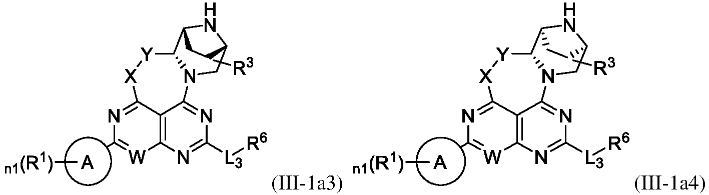 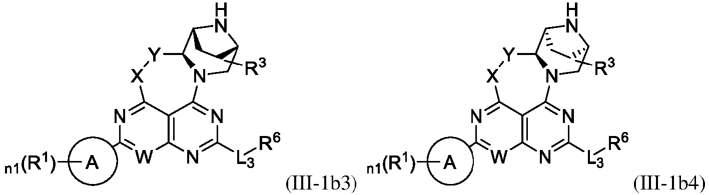 where in each formula,
R³ is a substitutent at any position on a bridged ring and is hydrogen, halogen, hydroxyl, cyano, C1-C3 alkyl, C1-C3 deuterated alkyl or C1-C3 haloalkyl;
Y is CR¹²R¹³, CR¹⁴R¹⁵CR¹⁶R¹⁷, C(O) or C(O)CR¹⁸R¹⁹, where
R¹² and R¹³ are each independently selected from H, halogen, hydroxyl, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, and -C1-C4 alkyl-NR¹²¹R¹²²; R¹² and R¹³ are not both H; or R¹² and R¹³, together with the carbon atom to which they are attached, form a cycloalkyl group;
R¹⁴ and R¹⁵ are each independently selected from H, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, -C1-C4 alkyl-NR¹²¹R¹²²; R¹⁴ and R¹⁵ are not both H; or R¹⁴ and R¹⁵, together with the carbon atom to which they are attached, form a cycloalkyl group;
R¹⁶ and R¹⁷ are each independently selected from H, halogen, hydroxyl, nitro, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, aryl, heteroaryl, aryl-O-, heteroaryl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, -C1-C4 alkyl-NR¹²¹R¹²²; and R¹⁶ and R¹⁷ are not both H;
R¹⁸ and R¹⁹ are each independently selected from H, C1-C3 alkyl and halo;
R¹²¹ and R¹²² are each independently selected from H and C1-C3 alkyl; or R¹²¹ and R¹²², together with the nitrogen atom to which they are attached, form a 3- to 6-membered nitrogen-containing heterocyclic group,
the cycloalkyl, the heterocyclyl, the aryl, the heteroaryl, and the 3- to 6-membered nitrogen-containing heterocyclic group are each independently optionally substituted with a group selected from the group consisting of halogen, hydroxyl, nitro, cyano, C1-C3 alkyl, C1-C3 deuterated alkyl, C1-C3 haloalkyl, C1-C3 alkoxy, and C1-C3 haloalkoxy;
X, W, ring A, R¹, n1, L₃, and R⁶ are as defined in Formula (III-1) or Formula (III-2).

In one embodiment, R³ is hydrogen.

In one embodiment, Y is CR¹²R¹³, in which R¹² is selected from halogen, hydroxyl, cyano, C1-C6 alkyl, C1-C6 deuterated alkyl, C1-C6 haloalkyl, C1-C6 alkoxy, C1-C6 haloalkoxy, cycloalkyl, cycloalkyl-O-, heterocyclyl, heterocyclyl-O-, -C1-C4 alkyl-C1-C6 alkoxy, -C1-C4 alkyl-C1-C6 haloalkoxy, -C1-C4 alkyl-hydroxyl, -C1-C4 alkyl-cyano, and -C1-C4 alkyl-NR¹²¹R¹²²; R¹³ is H; R¹²¹ and R¹²² are each independently selected from H and C1-C3 alkyl; or R¹²¹ and R¹²², together with the nitrogen atom to which they are attached, form a 3- to 6-membered nitrogen-containing heterocyclyl; or R¹² and R¹³, together with the carbon atom to which they are attached, form a cycloalkyl group, in which the cycloalkyl, the heterocyclyl, the aryl, the heteroaryl, the 3- to 6-membered nitrogen-containing heterocyclyl are each independently optionally substituted with a group selected from the group consisting of halogen, hydroxyl, nitro, cyano, C1-C3 alkyl, C1-C3 deuterated alkyl, C1-C3 haloalkyl, C1-C3 alkoxy, and C1-C3 haloalkoxy.

In one embodiment, in Formula (III-1a3), Formula (III-1a4), Formula (III-1b3) or Formula (III-1b4), Y is CR¹²R¹³, where R¹² is selected from halogen, hydroxyl, cyano, methyl, ethyl, n-propyl, isopropyl, trideuterated methyl, trifluoromethyl, cyclopropyl, -C1-C4 alkyl-methoxy, -C1-C4 alkyl-hydroxyl, -C1-C4 alkyl-cyano; R¹³ is H; or R¹² and R¹³, together with the carbon atom to which they are attached, form a cyclopropyl group.

In one embodiment, in Formula (III-1a3), Formula (III-1a4), Formula (III-1b3) or Formula (III-1b4), Y is - CH(CH₃)-, -CH(CH₂CH₃)-, -CH(CH₂CH₂CH₃)-, -CH(CH(CH₃)₂)-, -CH(cyclopropyl)-, -CH(CH₂OH)-, - CH(CH₂CH₂OH)-, -CH(CH₂CN)-, -CH(CH₂CH₂CN)-, -CH(CH₂CF₃)-, -CH(CH₂CF₂H)-, -CH(CH₂CH₂F)-, - CH(CH₂F)-, -CH(CF_{2H})-, -CH(CF₃)-, -CH(CN)-, -CH(CH₂OCH₃)-, 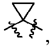 -C(CH₃)₂-, or -CH(CD₃)-.

In one embodiment, the cycloalkyl is C3-C20 cycloalkyl. Preferably, the cycloalkyl is C3-C12 cycloalkyl (further preferably C3-C8 monocyclic cycloalkyl), C5-C20 spiro cycloalkyl, C5-C20 fused cycloalkyl or C5-C20 bridged cycloalkyl.

In one embodiment, the heterocyclyl is a 3- to 20-membered heterocyclyl. Preferably, the heterocyclyl is a monocyclic heterocyclyl (for example, a 3- to 8-membered monocyclic heterocyclyl), a 5- to 20-membered spiro heterocyclyl, a 5- to 20-membered fused heterocyclyl, and a 5- to 20-membered bridged heterocyclyl.

In one embodiment, the aryl is C6-C14aryl. Preferably, the aryl is a monocyclic aryl, a non-fused polycyclic aryl, and an aromatic fused polycyclic group.

In one embodiment, the heteroaryl is a 5- to 14-membered heteroaryl. Preferably, the heteroaryl is a monocyclic heteroaryl (for example, a 5- or 6-membered monocyclic heteroaryl), a fused bicyclic heteroaryl (for example, a 8- to 10-membered bicyclic heteroaryl) or a fused tricyclic heteroaryl.

In one embodiment, W is N or CR²⁰, in which R²⁰ is H, cyano, C1-C3 alkyl, halogen, C1-C3 haloalkyl, C1-C3 alkoxy, C3-C6 monocyclic cycloalkyl, C3-C6 monocyclic cycloalkyl-O-, a 3- to 6-membered heterocyclyl or a 3- to 6-membered heterocyclyl-O-, in which the C3-C6 monocyclic cycloalkyl and the 3- to 6-membered heterocyclyl are each independently optionally substituted with halo.

In one embodiment, W is N or CR²⁰, in which R²⁰ is hydrogen, fluoro, methyl, methoxy or cyclopropyloxy.

In one embodiment, W is N or CR²⁰, in which R²⁰ is H, fluoro, chloro, methyl, cyclopropyl, methoxy or cyclopropyloxy.

In one embodiment, X is -N(CH₃)- or -N(cyclopropyl)-.

In one embodiment, X is -N(CD₃)-.

In one embodiment, X is O.

In one embodiment, Y is CR¹²R¹³ or CR¹⁴R¹⁵CR¹⁶R¹⁷, in which R¹², R¹³, R¹⁴ and R¹⁵ are each independently selected from H, cyano, C1-C6 alkyl, C1-C6 haloalkyl, C1-C6 alkoxy, C1-C6 haloalkoxy, C3-C6 monocyclic cycloalkyl, C3-C6 monocyclic cycloalkyl-O-, 3- to 6-membered heterocyclyl, 3- to 6-membered heterocyclyl-O-, phenyl, a 5- or 6-membered heteroaryl, phenyl-O-, 5- or 6-membered heteroaryl-O-, -C1-C2 alkyl-C1-C3 alkoxy, -C1-C2 alkyl-C1-C3 haloalkoxy, -C1-C2 alkyl-hydroxyl, -C1-C2 alkyl-cyano, and -C1-C2 alkyl-NR¹²¹R¹²²; R¹⁶ and R¹⁷ are each independently selected from H; R¹²¹ and R¹²² are each independently selected from H, and C1-C3 alkyl; or R¹²¹ and R¹²², together with the nitrogen atom to which they are attached, form a 3- to 6-membered nitrogen-containing heterocyclyl, in which the C3-C6 monocyclic cycloalkyl, the 3- to 6-membered heterocyclyl, the phenyl, the 5- or 6-membered heteroaryl, and the 3- to 6-membered nitrogen-containing heterocyclyl are each independently optionally substituted with a group selected from the group consisting of halogen, hydroxyl, nitro, cyano, C1-C3 alkyl, C1-C3 deuterated alkyl, C1-C3 haloalkyl, C1-C3 alkoxy, and C1-C3 haloalkoxy.

In one embodiment, Y is -CH₂-, -CH₂CH₂-, -CH(CH₂OCH₃)-, -CH(CN)-, -CH(OH)-, -CH(CH₃)-, -CH(CD₃)-, -C(CH₃)₂-, -CH(CF₃)-, -CH(CHF₂)-, -CH(CH₂F)-, -CH(CH₂CH₃)-, -CH(CH₂CH₂F)-, -CH(CH₂CF₂H)-, - CH(CH₂CF₃)-, -CH(CH₂CH₂CN)-, -CH(CH₂OH)-, -CH(CH₂CN)-, -CH(CH₂CH₂OH)-, -CH(cyclopropyl)-, - CH(isopropyl)-, -CH(CH₂CH₂CH₃)-, -CH(CH₂CH₂CH₂CH₃)- or

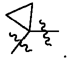

In one embodiment, Y is -CH₂-.

In one embodiment, Y is selected from the group consisting of:

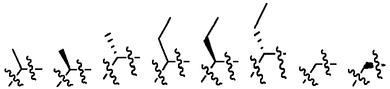

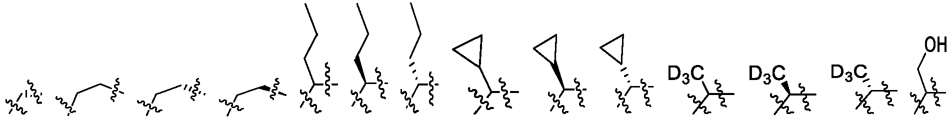

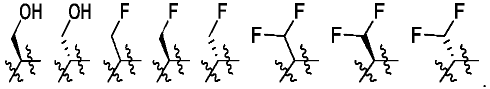

In one embodiment, R² is H, cyano, halogen, C1-C3 alkyl, C1-C3 alkoxy, C1-C3 haloalkyl, C1-C3 haloalkoxy, C1-C3 deuterated alkoxy, NR³¹R³², C2-C4 alkynyl or CH₂OR³³, where R³¹ and R³² are each independently hydrogen or C1-C6 alkyl; or form, together with the nitrogen atom to which they are attached, a 3- to 6-membered nitrogen-containing heterocyclyl; and R³³ is hydrogen or C1-C6 alkyl.

In one embodiment, R² is fluoro, chloro, hydroxyl, methoxy or cyano.

In one embodiment, R² is hydrogen, fluoro, chloro, hydroxyl, methoxy or cyano.

In one embodiment, ring A is phenyl, naphthalenyl, pyridinyl, pyrimidinyl, pyrazinyl, pyridazinyl, pyrrolyl, pyrazolyl, imidazolyl, 1,2,3,4-tetrahydronaphthalenyl, 2,3-dihydro-1H-indenyl, quinolinyl, isoquinolinyl, quinazolinyl, indolyl, indazolyl or benzo[d][1,3]dioxolanyl.

In one embodiment, ring A is selected from the group consisting of 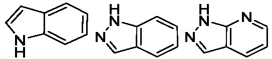 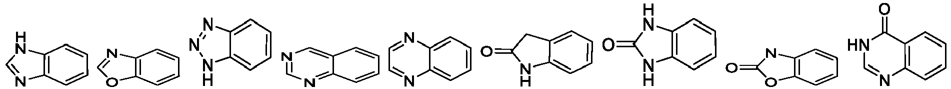 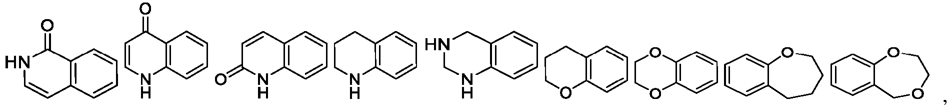 and these groups can be attached to the rest of the molecule through any suitable ring atom.

In one embodiment, R¹ is a substituent at any position on ring A; n1 is 0, 1, 2 or 3; and R¹ is each independently selected from C1-C3 alkyl, halogen, hydroxyl, C1-C3 alkoxy, C1-C3 haloalkyl, C1-C3 haloalkoxy, cyano, NR²¹R²², C(O)NR²³R²⁴, CH₂R²⁵, N=S(O)(C1-C3 alkyl)₂, S(O)C1-C3 alkyl, S(O)₂R²⁶, -S-C1-C3 alkyl, C2-C4 alkenyl, C2-C4 alkynyl, C2-C4hydroxyl alkynyl, C1-C3cyano alkyl, triazolyl, -S-C1-C3 haloalkyl, C1-C3 hydroxylalkyl, -CH₂C(O)NR²⁷R²⁸, -C2-C4 alkynylNR²⁹R³⁰, C2-C4 deuterated alkynyl, (C1-C3 alkoxy)C1-C3 haloalkyl- or cycloalkyl, in which the cycloalkyl is optionally substituted with halo or C1-C3 alkyl, where
R²¹ is H, C1-C3 alkyl, C1-C3 haloalkyl, C(O)C1-C3 alkyl or C(O)OC1-C3 alkyl; R²² is H, C1-C3 alkyl or C1-C3 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C3 alkyl, or C1-C3 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxyl, cyano, heterocyclyl, NR²⁵¹R²⁵², C(O)NR²⁵³R²⁵⁴ or SO₂C1-C3 alkyl, where R²⁵¹, R^{2S2}, R²⁵³, and R²⁵⁴ are each independently H or C1-C3 alkyl; R²⁵¹ and R²⁵², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and R²⁵³ and R²⁵⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
R²⁶ is C1-C3 alkyl, C1-C3 haloalkyl, or NR²⁶¹R²⁶², where R²⁶¹ and R²⁶² are each independently H or C1-C3 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C3 alkyl, or C1-C3 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C3 alkyl, or C1-C3 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
In one embodiment, L₂ is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂-, to from cyclopropyl or cyclobutyl.

In one embodiment, R^{3a}, R^{3b}, and R^{3c} are each independently hydrogen, fluoro, methyl or methoxy.

In one embodiment, 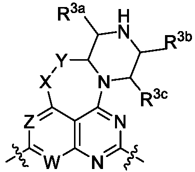 is selected from the group consisting of:

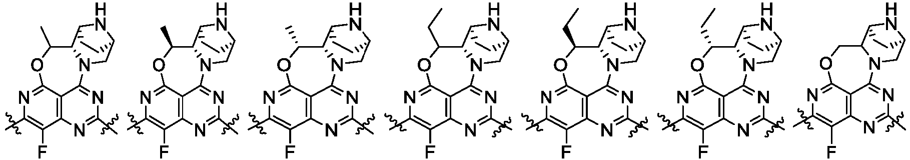

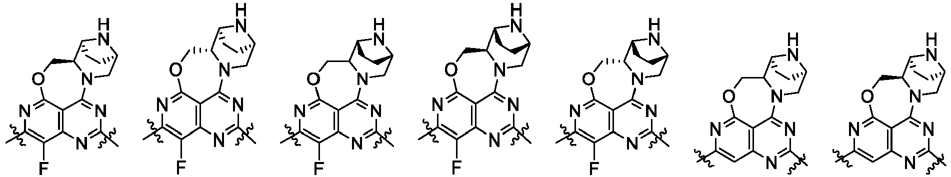

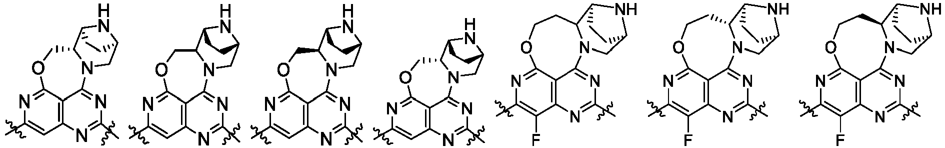

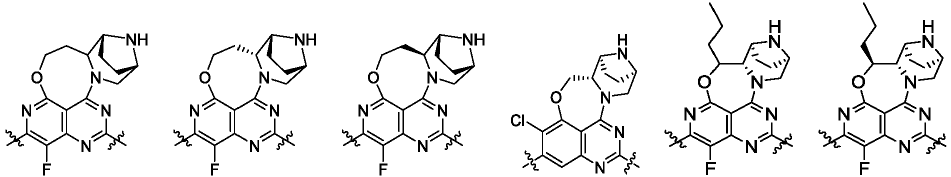

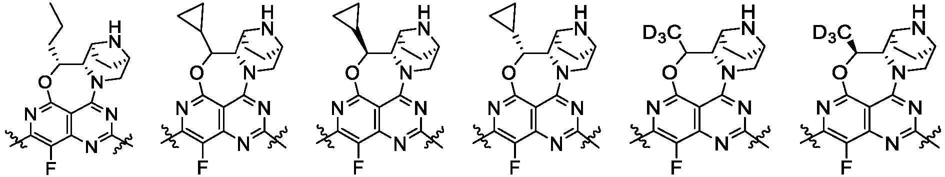

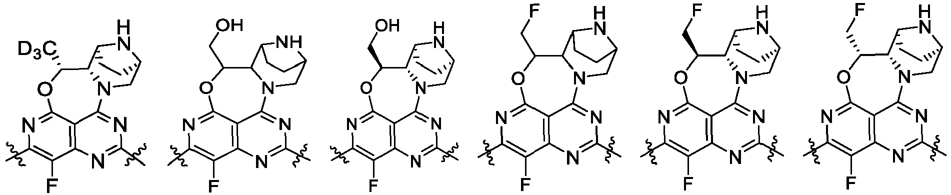

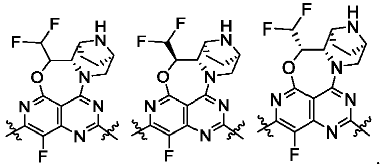

In one embodiment, 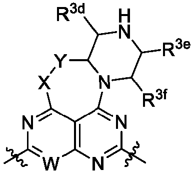 is selected from the group consisting of:

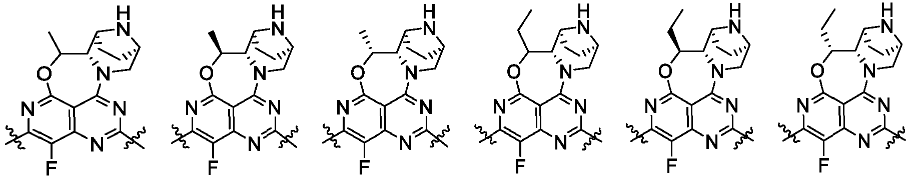

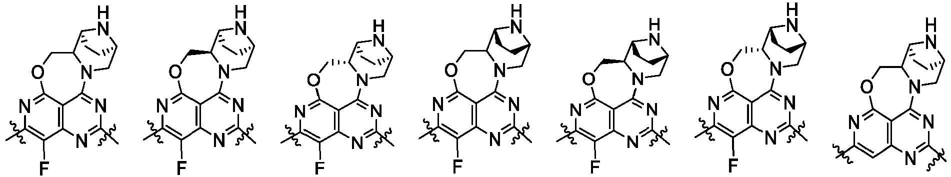

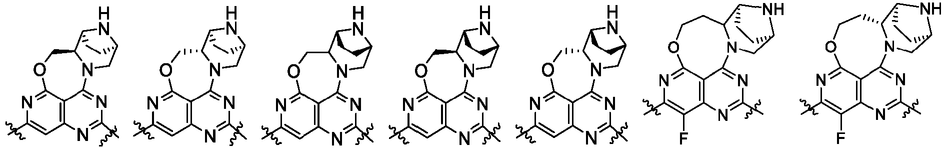

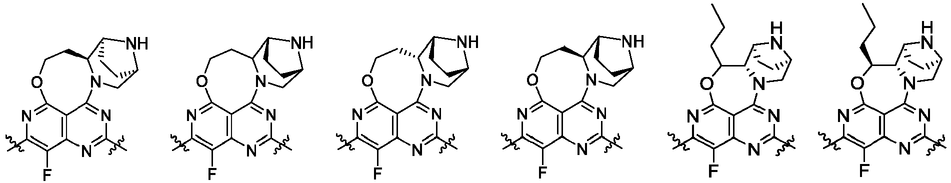

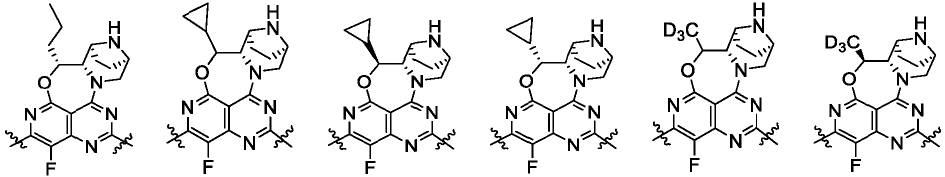

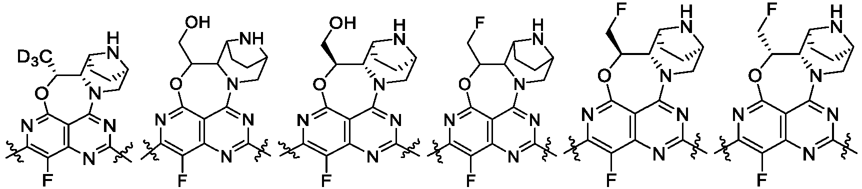

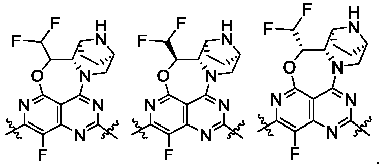

In one embodiment, 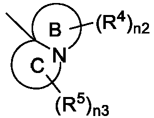 is selected from the group consisting of: 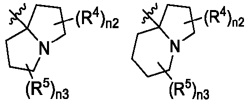 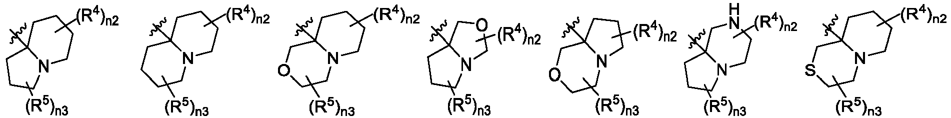 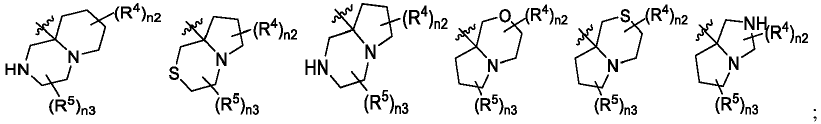 where in each formula, each R⁴, R⁵, n2, and n3 are each independently as defined in Formula (I).

In one embodiment, 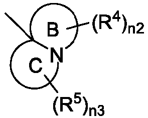 is selected from the group consisting of:

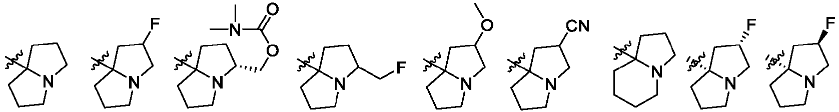

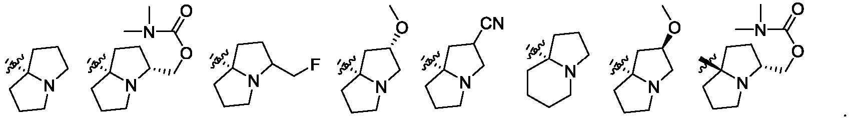

In one embodiment, R³ is hydrogen, fluoro, methyl or methoxy.

In one embodiment, R³ is hydrogen.

In one embodiment, R^{3d}, R^{3e}, and R^{3f} are each independently hydrogen, fluoro, methyl or methoxy; or
R^{3d} and R^{3e} are linked to form -CHR^{3d1}- or -CHR^{3d2}CHR^{3d3}-; R^{3f} is hydrogen, fluoro, methyl or methoxy; and R^{3d1}, R^{3d2}, and R^{3d3} are each independently hydrogen, fluoro, methyl or methoxy; or
R^{3d} and R^{3f} are linked to form -CHR^{3f1}- or -CHR^{3f2}CHR^{3f3}-; R^{3e} is hydrogen, fluoro, methyl or methoxy; and R^{3f1}, R^{3f2}, and R^{3f3} are each independently hydrogen, fluoro, methyl or methoxy.

In one embodiment, L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂-, to from cyclopropyl or cyclobutyl.

In one embodiment, R⁶ is hydrogen, -N(R³⁴)₂, a 3- to 20-membered heterocyclyl, C1-C6 alkyl, -L-3- to 20-membered heterocyclyl, -L-C6-C14 aryl, -L-5- to 14-membered heteroaryl, -L-C3-C20 cycloalkyl, -L-N(R³⁴)₂, - L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-C6-C14aryl, -L-COOH or -L-C(O)OC1-C6 alkyl, where the 3- to 20-membered heterocyclyl, the C6-C14aryl in the - L-NR³⁴C(O)-C6-C14 aryl, and the C3-C20 cycloalkyl are optionally substituted with one or more (for example, 1, 2, 3, 4 or 5) R³⁵, or optionally two hydrogen atoms on the same carbon atom are both replaced by -CH₂CH₂- or - CH₂CH₂CH₂- to form cyclopropyl or cyclobutyl; the C6-C14 aryl in the -L-C6-C14 aryl or the 5- to 14-membered heteroaryl in the -L-5- to 14-membered heteroaryl are optionally substituted with one or more (for example, 1, 2, 3, 4 or 5) R³⁶; and each L, each R³⁴, each R³⁵, and each R³⁶ are each as defined above.

In one embodiment, R⁶ is hydrogen, -N(R³⁴)₂, a 3- to 8-membered monocyclic heterocyclyl, a 5- to 20-membered spiro heterocyclyl, a 5- to 20-membered fused heterocyclyl, a 5- to 20-membered bridged heterocyclyl, C1-C6 alkyl, -L-3- to 8-membered monocyclic heterocyclyl, -L-5- to 20-membered spiro heterocyclyl, -L-5- to 20-membered fused heterocyclyl, -L-5- to 20-membered bridged heterocyclyl, -L-phenyl, -L-naphthalenyl, -L-5-to 14-membered heteroaryl, -L-C3-C12 cycloalkyl, -L-C5-C20 spiro cycloalkyl, C5-C20 fused cycloalkyl, -L-C5-C20 bridged cloalkyl, -L-N(R³⁴)₂, -L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-phenyl, -L-NR³⁴C(O)-naphthalenyl, -L-COOH or -L-C(O)OC1-C6 alkyl, where the 3- to 8-membered monocyclic heterocyclyl, 5- to 20-membered spiro heterocyclyl, 5- to 20-membered fused heterocyclyl, 5- to 20-membered bridged heterocyclyl, C3-C12 cycloalkyl, C5-C20 spiro cycloalkyl, C5-C20 fused cycloalkyl, C5-C20 bridged cycloalkyl, the phenyl in the -L-NR³⁴C(O)-phenyl, and the naphthalenyl in the -L-NR³⁴C(O)-naphthalenyl are optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; the phenyl in the -L-phenyl, the naphthalenyl in the -L-naphthalenyl, and the 5- to 14-membered heteroaryl in the -L-5- to 14-membered heteroaryl are optionally substituted with one or more (for example, 1, 2, 3, 4 or 5) R³⁶; and each L, each R³⁴, each R³⁵, and each R³⁶ are each as defined above.

In one embodiment, R⁶ is hydrogen or -N(R³⁴)₂. Preferably, each R³⁴ is each independently hydrogen or C1-C3 alkyl; or 1 R³⁴ is hydrogen and the other R³⁴ is C1-C3 alkyl.

In one embodiment, R³⁴ is hydrogen, C1-C3 alkyl or C1-C3 cyanoalkyl.

In one embodiment, C1-C6 alkyl is methyl, ethyl, isopropyl or isobutyl.

In one embodiment, L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂-, to from cyclopropyl or cyclobutyl.

In one embodiment, R⁶ is each independently 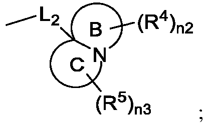 and R⁴, R⁵, L₂, ring B, ring C, n2, and n3 are as defined for various groups in Formula (I).

In one embodiment, the heterocyclyl in R⁶ is each independently hexahydro-1H-pyrrolizinyl, hexahydro-3H-pyrrolizin-3-one, hexahydro-1H-pyrrolo[2,1-c][1,4]oxazinyl, octahydroindolizinyl, hexahydropyrrolizin4(1H)-oxide, azetidinyl, pyrrolidinyl, pyrrolidin-2-one , oxacyclobutyl, piperidinyl, 1-azadicyclo[2.2.1]heptyl, morpholinyl, oxa-5-azadicyclo[2.2.1]hept-5-yl, thiopyranyl, 6-oxa-2-azaspiro [3.4]octyl, 7-oxa-2-azaspiro [3.5]nonyl, 2',3'-dihydrospiro [cyclopropane-1,1'-indenyl], (2S)-1-azadicyclo[2.2.1]hept-2-yl or tetrahydrofuranyl. The above groups are each indpendently optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; and each R³⁵ is as defined above.

In one embodiment, R⁶ is -L-heterocyclyl; and the heterocyclyl is hexahydro-1H-pyrrolizinyl.

In one embodiment, R⁶ is -L-heterocyclyl; the heterocyclyl is a hexahydro-1H-pyrrolizinyl substituted with one R³⁵ or a hexahydro-1H-pyrrolizinyl in which two hydrogen atoms on the same carbon atom are both replaced by -CH₂CH₂- or -CH₂CH₂CH₂- to form cyclopropyl or cyclobutyl, in which R³⁵ is halogen, hydroxyl, C1-C3 hydroxylalkyl, C1-C3 haloalkyl, C1-C3 alkyl, C1-C3 alkoxy, phenyl, pyrazolyl or -CH₂OC(O)N(R³⁴)₂.

In one embodiment, the halo is fluoro.

In one embodiment, the heterocyclyl is a hexahydro-1H-pyrrolizinyl that is further substituted with two additional R³⁵; and the two additional R³⁵ are each independently C1-C3 alkyl.

In one embodiment, R⁶ is -L-heterocyclyl; the heterocyclyl is an azetidinyl substituted with one R³⁵, or an azetidinyl in which two hydrogen atoms on the same carbon atom are both replaced by -CH₂CH₂- or -CH₂CH₂CH₂-to form cyclopropyl or cyclobutyl; and R³⁵ is C1-C3 alkyl.

In one embodiment, R⁶ is -L-heterocyclyl; the heterocyclyl is a pyrrolidinyl substituted with one R³⁵, or pyrrolidinyl in which two hydrogen atoms on the same carbon atom are both replaced by -CH₂CH₂- or - CH₂CH₂CH₂- to form cyclopropyl or cyclobutyl, where R³⁵ is hydroxylalkyl, haloalkyl, C1-C3 alkyl, alkoxy, aryl-C1-C3 alkyl, -phenyl, -O-phenyl, and -NHC(O)phenyl; in which the aryl in the aryl-C1-C3 alkyl, the phenyl, the phenyl in the -O-phenyl, or the phenyl in the -NHC(O)phenyl is each independently optionally substituted with one or more (for example, 1, 2, 3, 4 or 5) R³⁶.

In one embodiment, the phenyl, or the phenyl in the -O-phenyl or -NHC(O)phenyl is substituted with -SO₂F.

In one embodiment, R⁶ is -L-heterocyclyl; and the heterocyclyl is a pyrrolidinyl substituted with two R³⁵, in which one R³⁵ is C1-C3 alkyl, and the other R³⁵ is C1-C3 alkoxy or halo.

In one embodiment, R⁶ is -L-heterocyclyl; and the heterocyclyl is pyrrolidin-2-one substituted with one R³⁵ or pyrrolidin-2-one in which two hydrogen atoms on the same carbon atom are both replaced by -CH₂CH₂- or - CH₂CH₂CH₂- to form cyclopropyl or cyclobutyl, where R³⁵ is C1-C3 alkyl.

In one embodiment, R⁶ is -L-heterocyclyl; the heterocyclyl is a piperidinyl substituted with one R³⁵ or a piperidinyl in which two hydrogen atoms on the same carbon atom are both replaced by -CH₂CH₂- or - CH₂CH₂CH₂- to form cyclopropyl or cyclobutyl, where R³⁵ is acetyl, (C1-C3 alkoxy)C1-C3 alkoxy or - C(O)CH₂Cl.

In one embodiment, R⁶ is -L-heterocyclyl; and the heterocyclyl is morpholinyl or oxa-5-azadicyclo[2.2.1]hept-5-yl.

In one embodiment, R⁶ is -L-heteroaryl; and the heteroaryl is optionally substituted with one or more R³⁶.

In one embodiment, R⁶ is -L-heteroaryl; L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl; the heteroaryl is pyridinyl, pyrazolyl, imidazolyl, triazolyl, 4,5,6,7-tetrahydro-1H-indazolyl, benzoimidazolyl, imidazo[1, 2-a]pyridinyl or pyrimidinyl; and the above groups are each optionally substituted with one or more R³⁶.

In one embodiment, R⁶ is -L-heteroaryl; L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl; and the heteroaryl is substituted with one R³⁶ substituted pyridinyl, where R³⁶ is halogen, C1-C4 alkyl, -N(R⁵)₂ or C1-C4 alkoxy.

In one embodiment, R⁶ is -L-heteroaryl; L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl; and the heteroaryl is substituted with one R³⁶ substituted pyrazolyl, where R³⁶ is C1-C4 alkyl or -N(R³⁴)₂.

In one embodiment, R⁶ is -L-heteroaryl; L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl; and the heteroaryl is substituted with one R³⁶ substituted imidazolyl, where R³⁶ is C1-C4 alkyl, C1-C4 haloalkyl or C1-C4 hydroxylalkyl.

In one embodiment, R⁶ is -L-heteroaryl; L is methylene or ethylene or propylene, where 1 or 2 hydrogen atom(s) on any carbon atom(s) in the methylene or ethylene or propylene is/are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl; and the heteroaryl is substituted with one R³⁶ substituted triazolyl, where R³⁶ is C1-C4 alkyl.

In one embodiment, R⁶ is -L-aryl; and the aryl is optionally substituted with one or more R³⁶.

In one embodiment, R⁶ is -L- cycloalkyl; the cycloalkyl is optionally substituted with one or more R³⁵, or optionally two hydrogen atoms on the same carbon atom are both replaced by -CH₂CH₂- or -CH₂CH₂CH₂- to form cyclopropyl or cyclobutyl; and R³⁵ is each as defined above.

In one embodiment, R⁶ is -L-N(R³⁴)₂, where L is C1-C4 alkylene, in which 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by -CH₂CH₂-or -CH₂CH₂CH₂- to form cyclopropyl or cyclobutyl.

In one embodiment, R⁶ is -L-N(R³⁴)₂, where L is methylene, ethylene or propylene, in which 1 or 2 hydrogen atom(s) on any carbon atom(s) in the methylene, ethylene or propylene is/are optionally each independently substituted with deuterium or C1-C3 alkyl; or 2 hydrogen atoms on any carbon atom in the methylene, ethylene or propylene are optionally both replaced by -CH₂CH₂- or -CH₂CH₂CH₂- to form cyclopropyl or cyclobutyl; and each R³⁴ is independently selected from C1-C3 alkyl.

In one embodiment, R⁶ is -L-NC(=NH)-NH₂, where L is C1-C4 alkylene, in which 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by - CH₂CH₂- or -CH₂CH₂CH₂- to form cyclopropyl or cyclobutyl.

In one embodiment, L is ethylene or propylene.

In one embodiment, R⁶ is -L-C1-C6 haloalkyl, where L is C1-C4 alkylene, in which 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by - CH₂CH₂- or -CH₂CH₂CH₂- to form cyclopropyl or cyclobutyl.

In one embodiment, R⁶ is -L-OR³⁴, where L is C1-C4 alkylene, where 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by -CH₂CH₂- or - CH₂CH₂CH₂- to form cyclopropyl or cyclobutyl.

In one embodiment, R⁶ is -L-(CH₂OR³⁴)(CH₂)ₙOR³⁴, where L is C1-C4 alkylene, in which 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by -CH₂CH₂- or -CH₂CH₂CH₂- to form cyclopropyl or cyclobutyl.

In one embodiment, R⁶ is -L-NR³⁴C(O)-aryl, where L is C1-C4 alkylene, in which 1 or 2 hydrogen atom(s) on any carbon atom(s) in the C1-C4 alkylene is/are optionally each independently substituted with deuterium or C1-C3 alkyl; or 2 hydrogen atoms on any carbon atom in the C1-C4 alkylene are optionally both replaced by - CH₂CH₂- or -CH₂CH₂CH₂- to form cyclopropyl or cyclobutyl.

In one embodiment, R⁶ is -L-heterocyclyl; L is C1-C4 alkylene, in which 2 hydrogen atoms on any carbon atom in the C1-C4 alkylene optionally each independently substituted with deuterium or C1-C6 alkyl; or 2 hydrogen atoms on any carbon atom in the C1-C4 alkylene are optionally both replaced by -CH₂CH₂- or - CH₂CH₂CH₂- to form cyclopropyl or cyclobutyl.

In one embodiment, R⁶ is -L-6- to 10-membered fused heterocyclyl; 2 hydrogen atoms on the same carbon atom in any 1 or 2 carbon atoms in the 6- to 10-membered fused heterocyclyl are optionally both replaced by =CR^{2a}R^{2b}; and/or the 6- to 10-membered fused heterocyclyl is optionally further substituted with 1 or more R^{6a}, where R^{2a} and R^{2b} are each independently hydrogen, halogen, cyano, C1-C4 alkyl or C1-C4 haloalkyl; and each R^{6a} is each independently halo.

In one embodiment, L is methylene or ethylene or propylene, where 2 hydrogen atoms on any carbon atom in the methylene or ethylene or propylene are optionally each independently substituted with deuterium or C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl.

In one embodiment, W is CR²⁰; and R²⁰ is cyclopropyl, cyclopropyl-O-, cyclobutyl, cyclobutyl-O-, cyclopentyl, cyclopentyl-O-, tetrahydrofuranyl, tetrahydrofuran-O-, where the cyclopropyl, cyclobutyl, cyclopentyl, and tetrahydrofuranyl are each independently substituted with halo.

In one embodiment, W is CR²⁰; and R²⁰ is hydrogen, halogen, C1-C6 alkyl, C3-C6 cycloalkyl, C3-C6 cycloalkyl-O-, 3- to 6-membered heterocyclyl or 3- to 6-membered heterocyclyl-O-, where the C3-C6 cycloalkyl, and the 3- to 6-membered heterocyclyl are each independently substituted with halo.

In one embodiment, R²⁰ is halo or C1-C3 alkyl.

In one embodiment, R²⁰ is fluoro.

In one embodiment, R²⁰ is methyl.

In one embodiment, L₂ or L is methylene or ethylene or propylene, where 2 hydrogen atoms on any carbon atom in the methylene or ethylene or propylene are optionally each independently substituted with deuterium or

C1-C3 alkyl; or 2 hydrogen atoms on any carbon atom in the methylene or ethylene or propylene are optionally both replaced by -CH₂CH₂- or -CH₂CH₂CH₂- to form cyclopropyl or cyclobutyl.

In one embodiment, 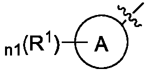 is phenyl substituted with one, two, three or four R¹, where each R¹ is independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 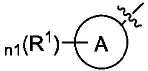 is naphthalenyl substituted with one, two, three or four R¹, where R¹ is each independently halogen, hydroxyl, ethynyl, methyl, methyl-O-, methyl-S-, trifluoromethyl, trifluoromethyl-O-, amino, isopropyl, cyano or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 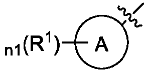 is naphthalenyl substituted with one, two or three R¹, where each R¹ is each independently halogen, hydroxyl, ethynyl, methyl, ethyl, amino, cyano or difluoromethyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 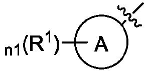 is naphthalenyl substituted with one or two R¹, where each R¹ is each independently halogen, ethynyl, or amino.

In one embodiment, 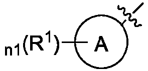 is heteroaryl optionally substituted with one or more R¹.

In one embodiment, 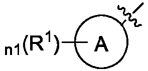 is pyridinyl substituted with one, two, three or four R¹, where each R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 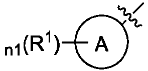 is quinolinyl, isoquinolinyl, indazolyl or benzo[d][1,3]dioxolanyl optionally substituted with one or more (for example, 1, 2, 3, 4 or 5) R¹.

In one embodiment, 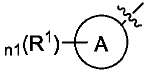 is isoquinolinyl substituted with one, two, three or four R¹, where R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 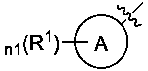 is isoquinolinyl substituted with one, two or three R¹, where R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 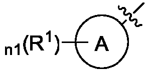 is quinolinyl substituted with one, two, three or four R¹, where R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, 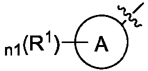 is indazolyl substituted with one, two or three R¹, where R¹ is each independently C1-C3 alkyl.

In one embodiment, 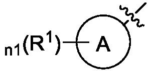 is benzo[d][1, 3]dioxolanyl substituted with two R¹ where R¹ is each independently selected from halo.

In one embodiment, 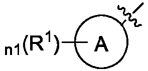 is selected from the group consisting of:

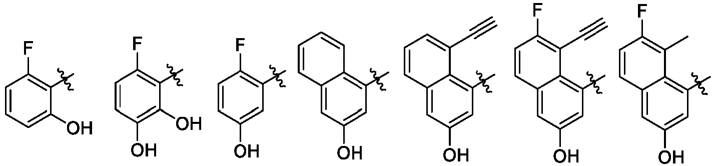

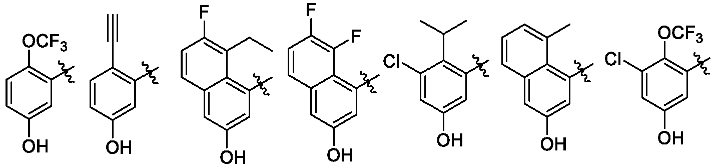

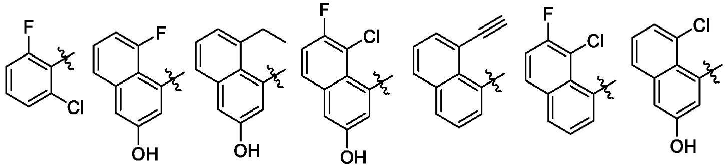

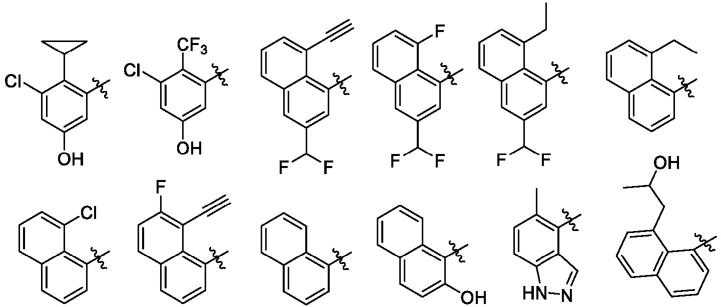

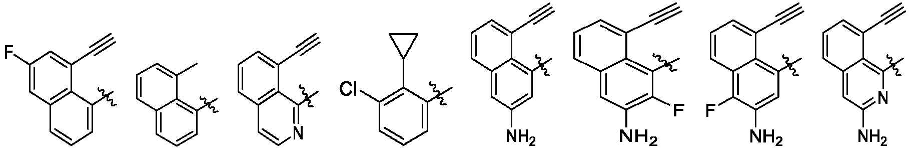

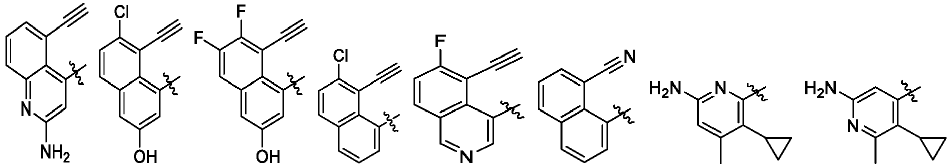

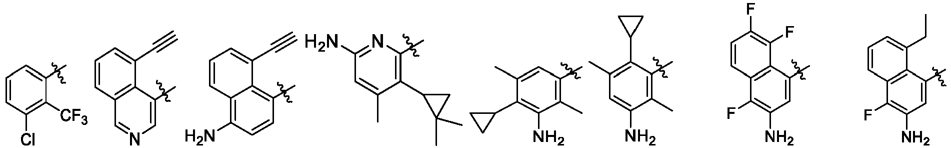

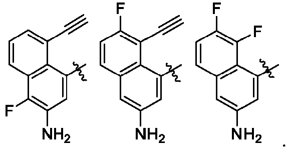

In one embodiment, 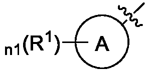 is selected from the group consisting of:

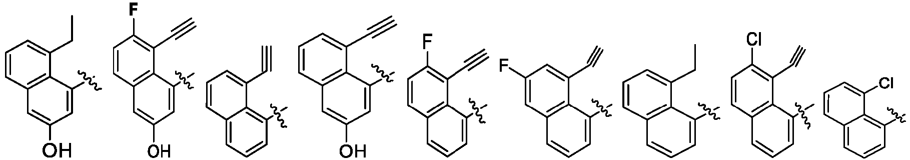

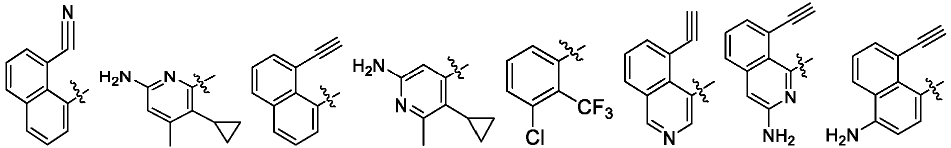

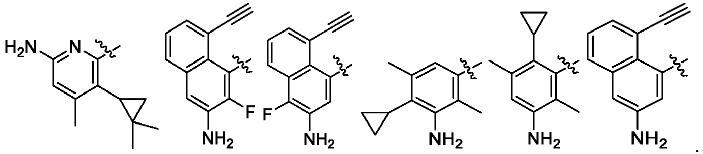

In one embodiment, the -L₃-R⁶ is selected from the group consisting of: 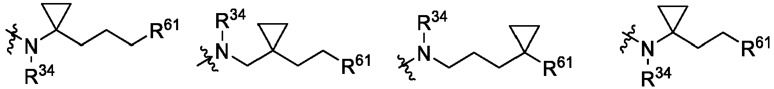 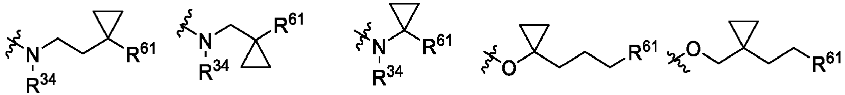 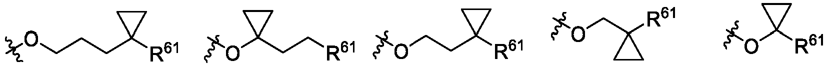 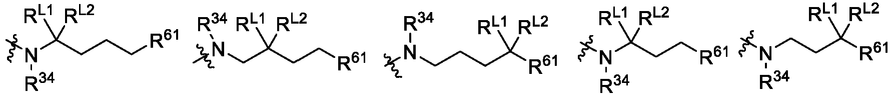 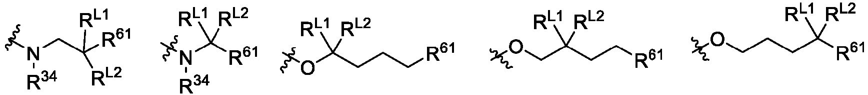 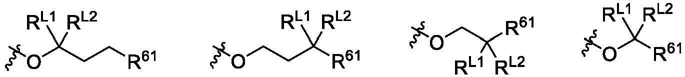 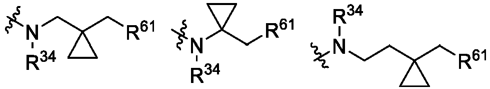 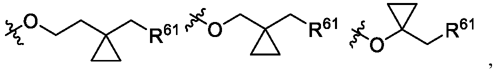 where in each formula, R^{L1} and R^{L2} are each independently hydrogen, deuterium, C1-C6 alkyl or C1-C6 haloalkyl; R⁶¹ is heterocyclyl, aryl, heteroaryl, cycloalkyl, -N(R³⁴)₂, -NHC(=NH)NH₂, -C(O)N(R³⁴)₂, -C1-C6 haloalkyl, -OR³⁴, -(CH₂OR³⁴)(CH₂)ₙOR³⁴, -NR³⁴C(O)-aryl, -COOH or -C(O)OC1-C6 alkyl, where the heterocyclyl, the aryl in the -NR³⁴C(O)-aryl, and the cycloalkyl are each optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; the aryl and the heteroaryl are each optionally substituted with one or more (for example, 1, 2, 3, 4 or 5) R³⁶; and each R³⁴, each R³⁵, and each R³⁶ are each as defined above. Preferably, in each formula, R³⁴ in N-R³⁴ is H.

In one embodiment, the -L₃-R⁶ is selected from the group consisting of: 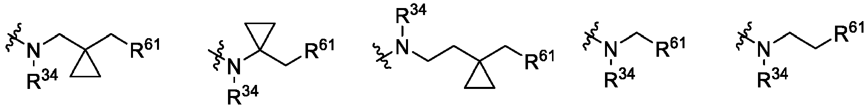 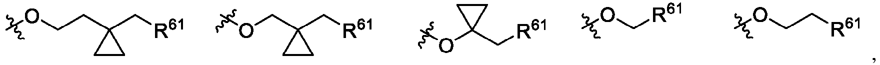 where in each formula, R⁶¹ is heterocyclyl, aryl, heteroaryl, cycloalkyl, -N(R³⁴)₂, -NHC(=NH)NH₂, - C(O)N(R³⁴)₂, -C1-C6 haloalkyl, -OR³⁴, -(CH₂OR³⁴)(CH₂)ₙOR³⁴, -NR³⁴C(O)-aryl, -COOH or -C(O)OC1-C6 alkyl, where the heterocyclyl needs to be substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; the aryl in -NR³⁴C(O)-aryl and the cycloalkyl are each optionally substituted with one or more (for example, 1, 2, 3, 4 or 5) R³⁵, or optionally two hydrogen atoms on the same carbon atom are both replaced by -CH₂CH₂- or -CH₂CH₂CH₂- to form cyclopropyl or cyclobutyl; the aryl and the heteroaryl are each optionally substituted with one or more (for example, 1, 2, 3, 4 or 5) R³⁶; and each R³⁴, each R³⁵, and each R³⁶ are each as defined above. Preferably, in each formula, R³⁴ in N-R³⁴ is H.

In one embodiment, the -L₃-R⁶ is -NH-R⁶¹, -OR⁶¹ or -R⁶¹; or the -L₃-R⁶ is selected from the group consisting of: 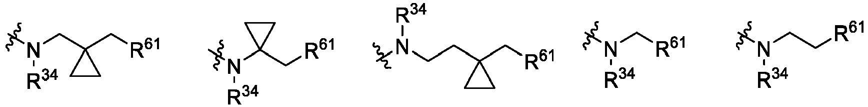 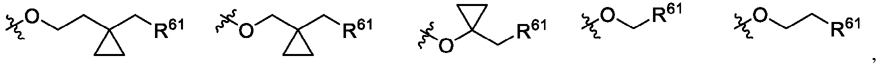 where in each formula, R³⁴ in NR³⁴ is H; and each R⁶¹ is independently selected from the group consisting of:

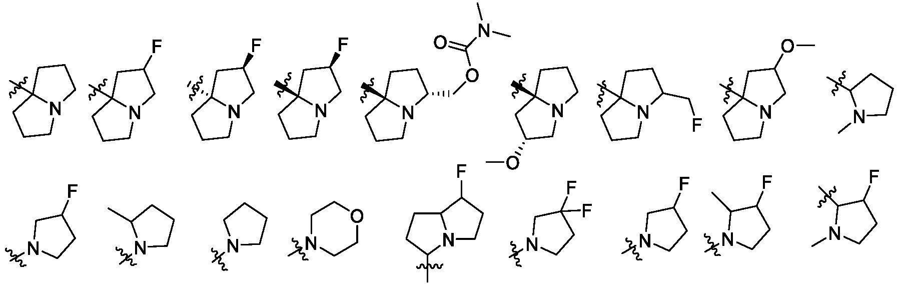

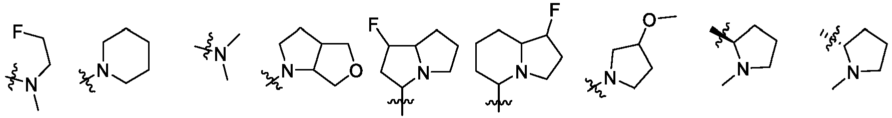

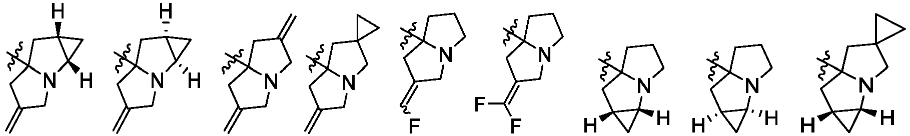

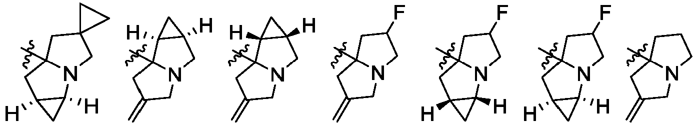

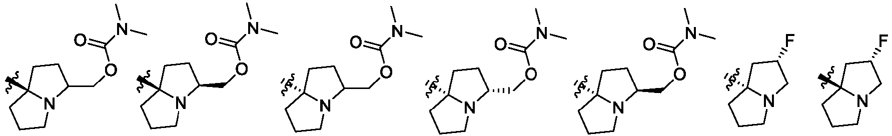

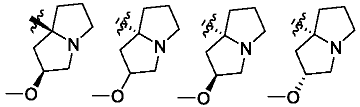

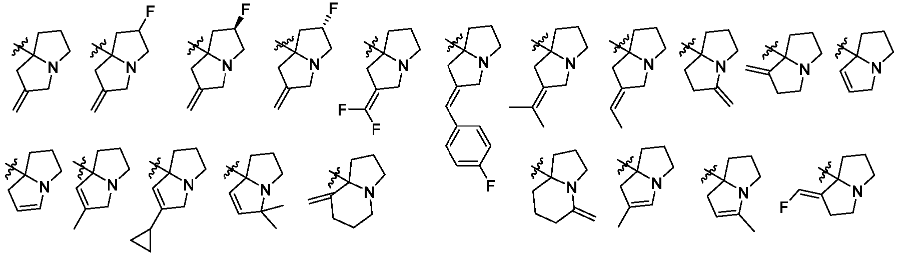

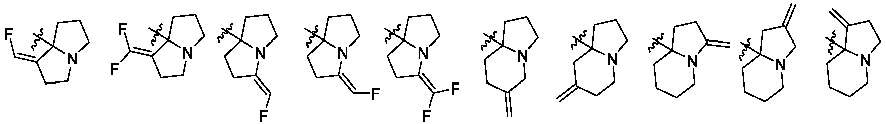

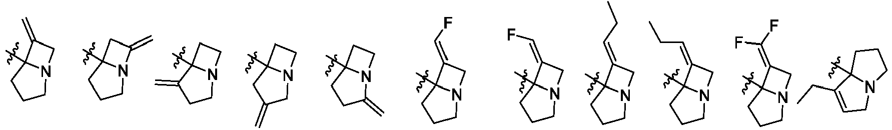

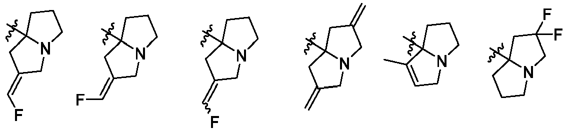

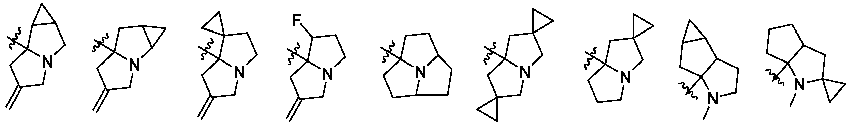

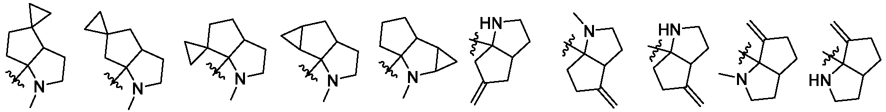

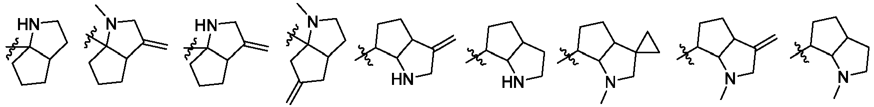

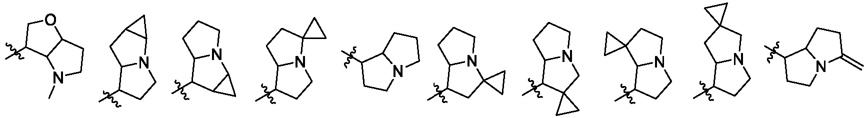

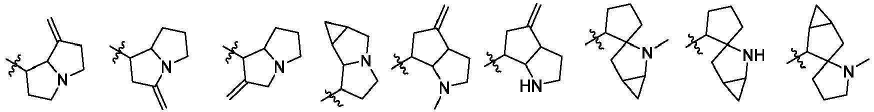

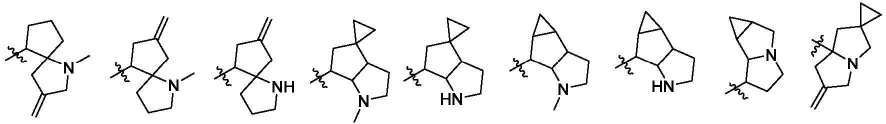

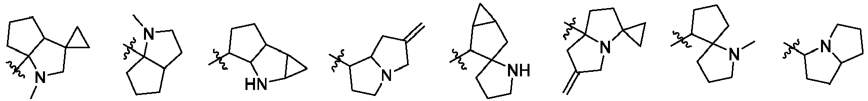

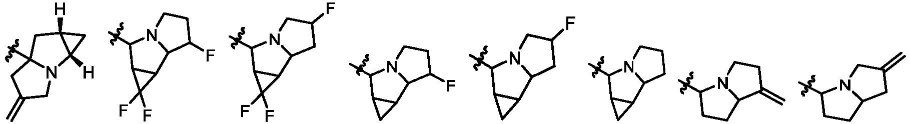

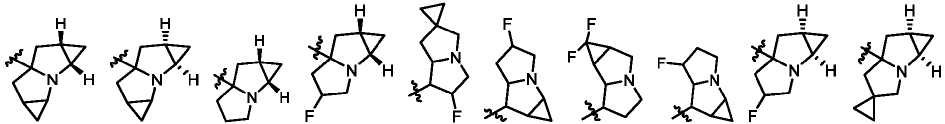

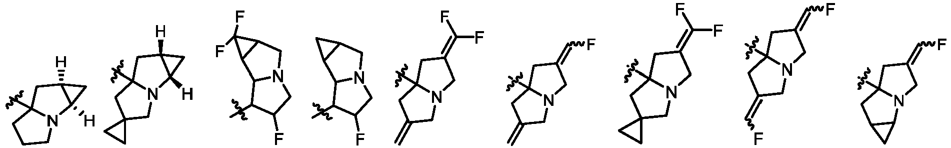

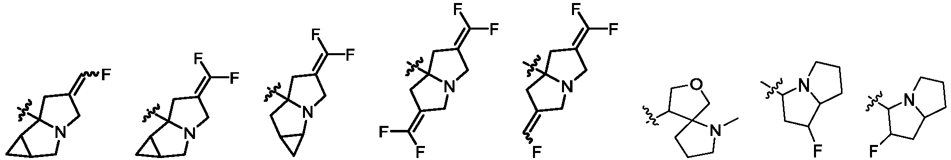

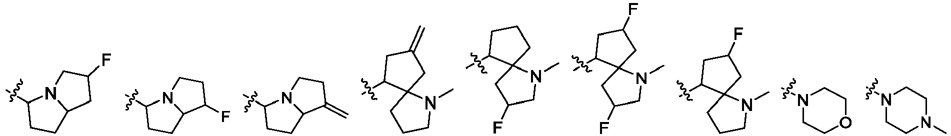

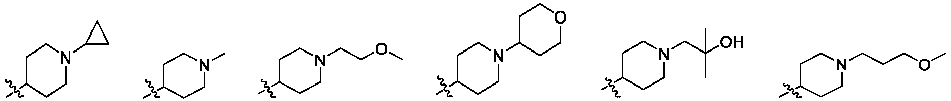

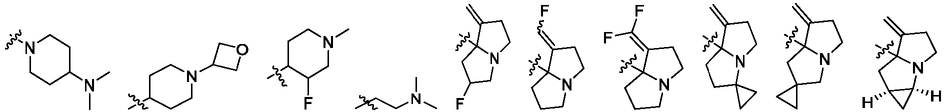

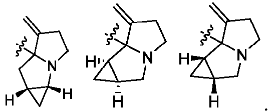

In one embodiment, the -L₃-R⁶ is -O-R⁶¹,

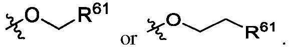

In one embodiment, R⁶¹ is independently selected from the group consisting of: 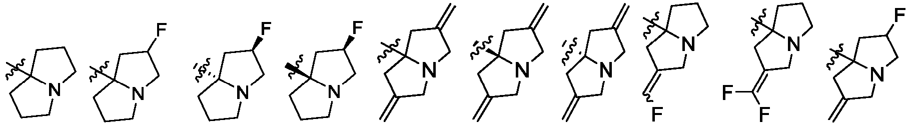 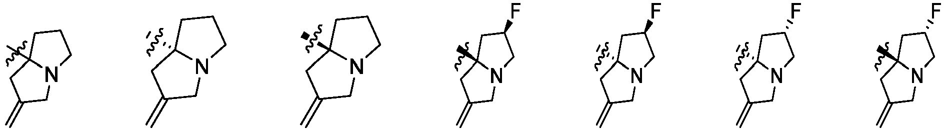 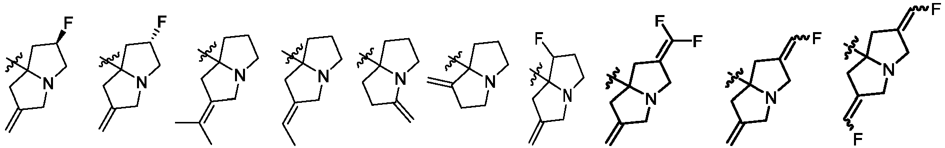 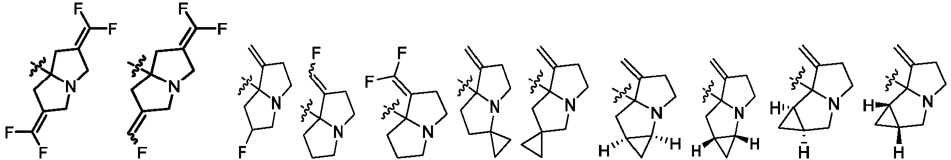 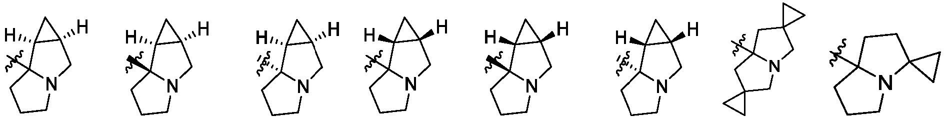 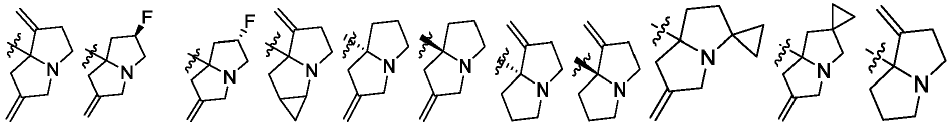 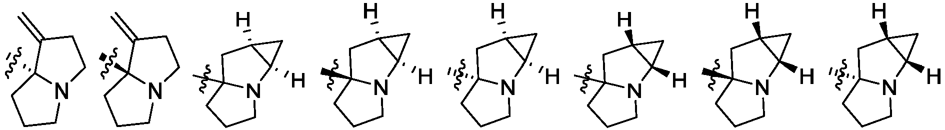 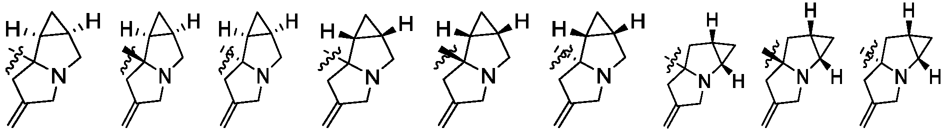 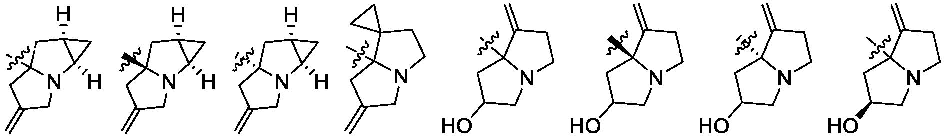 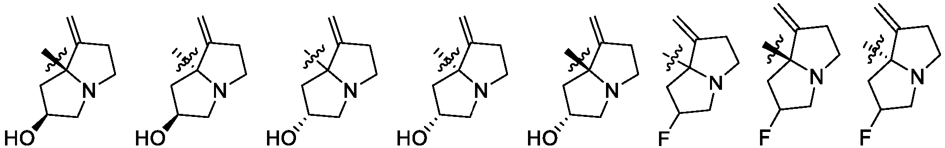 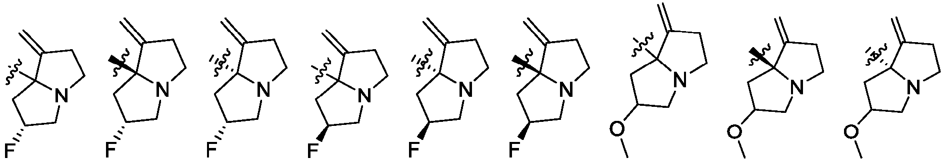 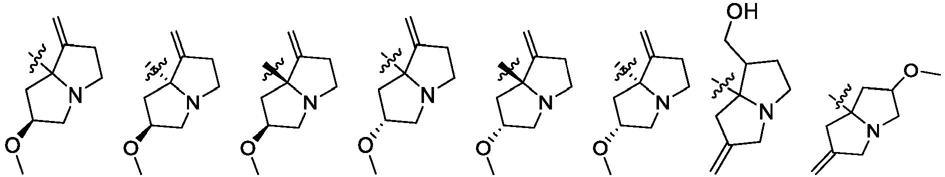 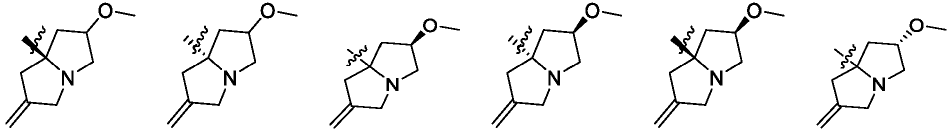 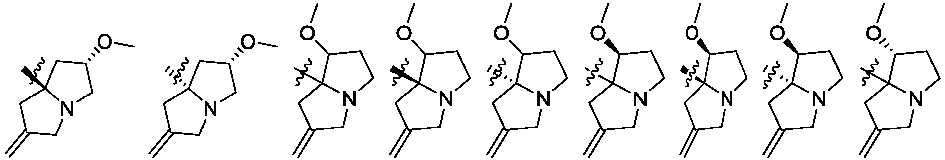 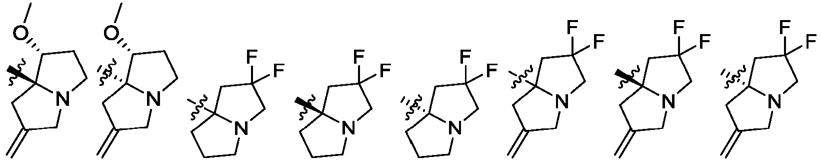 methyl

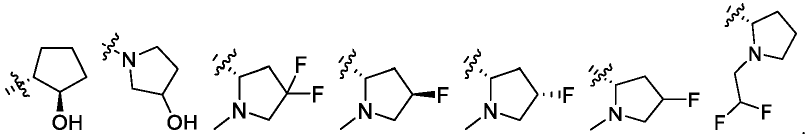

**In** one embodiment, the -L₃-R⁶ is 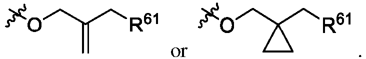 **In** one embodiment, R⁶¹ is - OR³⁴, -N(R³⁴)₂ or heterocyclyl, where the heterocyclyl is optionally substituted with one or more (for example, 1, 2, 3, 4 or 5) R³⁵, or optionally two hydrogen atoms on the same carbon atom are both replaced by -CH₂CH₂- or - CH₂CH₂CH₂- to form cyclopropyl or cyclobutyl; and each R³⁴ and each R³⁵ are each as defined above. Preferably, R³⁵ is each independently halogen, hydroxyl, C1-C3 alkyl, C2-C4 alkenylene, C2-C4 halo alkenylene or C1-C3 haloalkyl. preferably, R³⁴ is each independently hydrogen, C1-C3 alkyl or C1-C3 haloalkyl. In one embodiment, R⁶¹ is independently selected from the group consisting of:

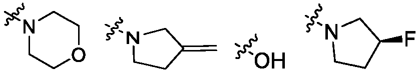

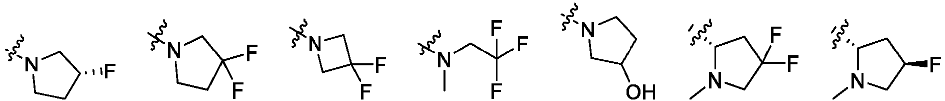

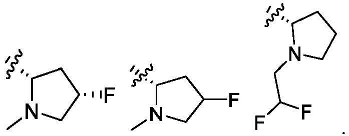

In one embodiment, R⁶¹ is hexahydro-1H-pyrrolizinyl.

In one embodiment, R⁶¹ is hexahydro-1H-pyrrolizinyl substituted with one R³⁵, where R³⁵ is halogen, hydroxyl, C1-C3 hydroxylalkyl, C1-C3 haloalkyl, C1-C3 alkyl, C1-C3 alkoxy, C2-C3 alkenyl, C2-C3 haloalkenyl, phenyl, pyrazolyl or -CH₂OC(O)N(R³⁴)₂; R³⁴ is each independently hydrogen, C1-C3 alkyl or C1-C3 haloalkyl; or two R³⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy.

In one embodiment, the 6- to 10-membered fused heterocyclic group is selected from the group consisting of:

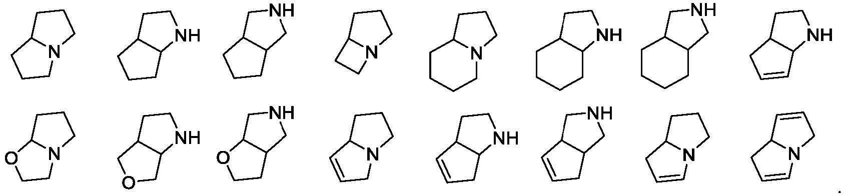

In one embodiment, the 6- to 10-membered fused heterocyclic group is selected from the group consisting of: 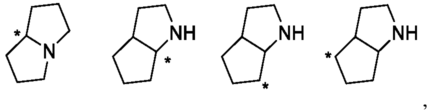 where the carbon atom marked with * is a carbon atom attached to the rest of the molecule.

In one embodiment, the 7- to 11-membered spiro heterocyclyl is selected from the group consisting of:

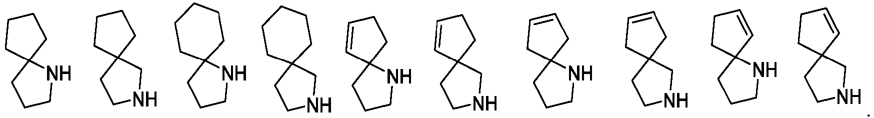

In one embodiment, the 7- to 11-membered spiro heterocyclyl is selected from the group consisting of: 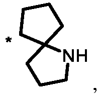 where the carbon atom marked with * is a carbon atom attached to the rest of the molecule.

In one embodiment, the sfpiro-substituted 6- to 10-membered fused heterocyclyl is selected from the group consisting of:

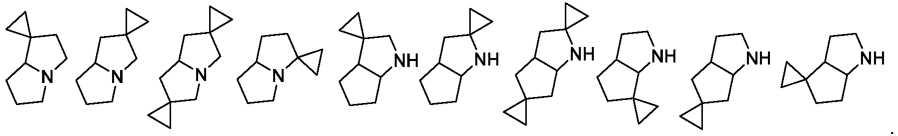

In one embodiment, the sfpiro-substituted 6- to 10-membered fused heterocyclyl is selected from the group consisting of: 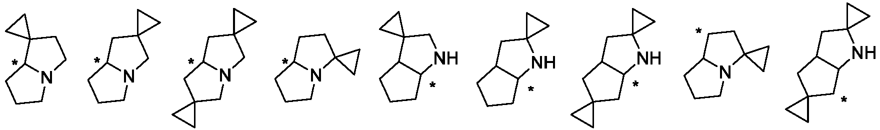 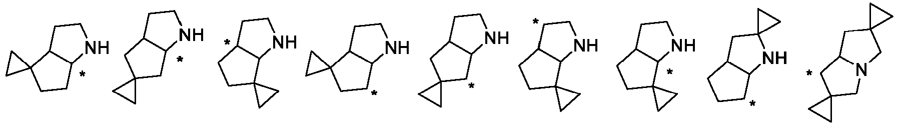 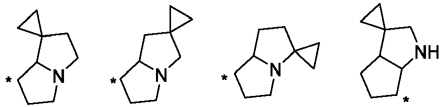 where the carbon atom marked with * is a carbon atom attached to the rest of the molecule.

In one embodiment, the spiro-substituted 7- to 11-membered spiro heterocyclyl is selected from the group consisting of:

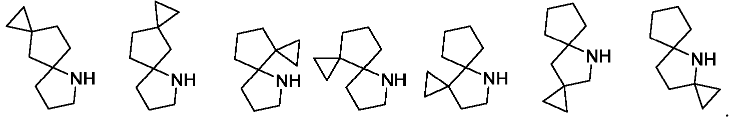

In one embodiment, the parallel-substituted 6- to 10-membered fused heterocyclyl is selected from the group consisting of:

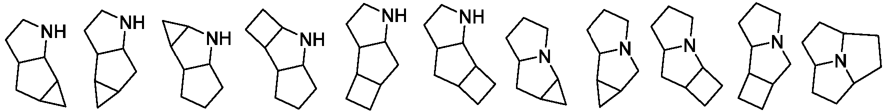

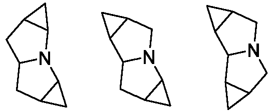

In one embodiment, the parallel-substituted 6- to 10-membered fused heterocyclyl is selected from the group consisting of: 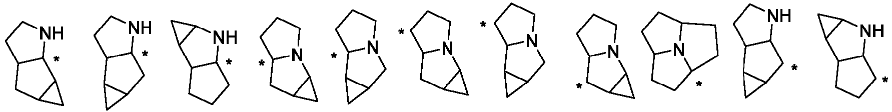 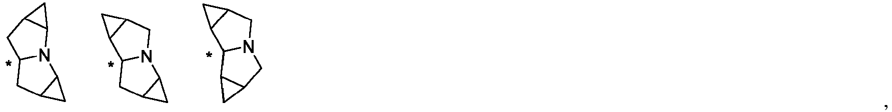 where the carbon atom marked with * is a carbon atom attached to the rest of the molecule.

In one embodiment, the parallel-substituted 7- to 11-membered spiro heterocyclyl is selected from the group consisting of:

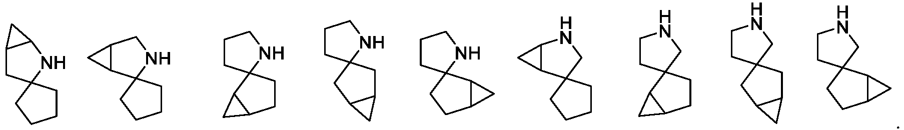

In one embodiment, the parallel-substituted 7- to 11-membered spiro heterocyclyl is selected from the group consisting of: 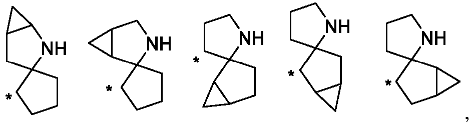 where the carbon atom marked with * is a carbon atom attached to the rest of the molecule.

In one embodiment, the spiro- and parallel- 6- to 10-membered fused heterocyclyl is selected from the group consisting of:

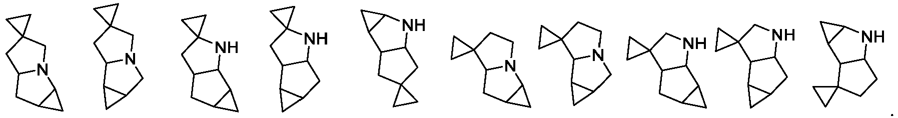

In one embodiment, the spiro- and parallel- 6- to 10-membered fused heterocyclyl is selected from the group consisting of: 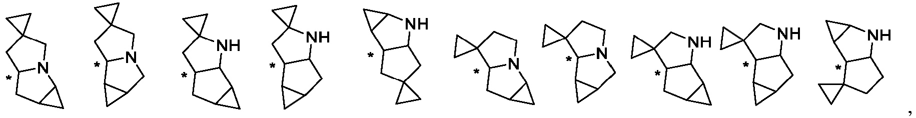 where the carbon atom marked with * is a carbon atom attached to the rest of the molecule.

In one embodiment, L₃ is O; R⁶ is -L-hexahydro-1H-pyrrolizinyl, where L is C1-C4 alkylene; and 2 hydrogen atoms on any 1 carbon atom in the hexahydro-1H-pyrrolizinyl are both replaced by =CR^{2a}R^{2b}, where R^{2a} and R^{2b} are each independently hydrogen, halogen, cyano, C1-C4 alkyl or C1-C4 haloalkyl. Preferably, the hexahydro-1H-pyrrolizinyl is further optionally substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, L₃ is O; R⁶ is -L-hexahydro-1H-pyrrolizinyl, where L is C1-C4 alkylene; and 2 hydrogen atoms on any 1 carbon atom in the hexahydro-1H-pyrrolizinyl are both replaced by =CR^{2a}R^{2b}, where R^{2a} and R^{2b}, together with the carbon atom to which they are attached, form a C3-C6 monocyclic cycloalkyl or a 3- to 6-membered monocyclic heterocyclyl; the C3-C6 monocyclic cycloalkyl or the 3- to 6-membered monocyclic heterocyclyl is optionally substituted with 1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl. Preferably, the hexahydro-1H-pyrrolizinyl is further optionally substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, L₃ is O; R⁶ is -L-hexahydro-1H-pyrrolizinyl, where L is C1-C4 alkylene; and 2 hydrogen atoms on one carbon atom in any 2 carbon atoms in the hexahydro-1H-pyrrolizinyl are both replaced by =CR^{2a}R^{2b}, where R^{2a} and R^{2b} are each independently hydrogen, halogen, cyano, C1-C4 alkyl or C1-C4 haloalkyl. Preferably, the hexahydro-1H-pyrrolizinyl is further optionally substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, L₃ is O; R⁶ is -L-hexahydro-1H-pyrrolizinyl, where L is C1-C4 alkylene; and 2 hydrogen atoms on one carbon atom in any 2 carbon atoms in the hexahydro-1H-pyrrolizinyl are both replaced by =CR^{2a}R^{2b}, where one pair of R^{2a} and R^{2b} is each independently hydrogen, halogen, cyano, C1-C4 alkyl or C1-C4 haloalkyl; and the other pair of R^{2a} and R^{2b}, together with the carbon atom to which they are attached, form a C3-C6 monocyclic cycloalkyl or a 3- to 6-membered monocyclic heterocyclyl; the C3-C6 monocyclic cycloalkyl or the 3- to 6-membered monocyclic heterocyclyl is optionally substituted with 1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl. Preferably, the hexahydro-1H-pyrrolizinyl is further optionally substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, L₃ is O; R⁶ is -L-hexahydro-1H-pyrrolizinyl, where L is C1-C4 alkylene; and 2 hydrogen atoms on one carbon atom in any 2 carbon atoms in the hexahydro-1H-pyrrolizinyl are both replaced by =CR^{2a}R^{2b}, where the two pairs of R^{2a} and R^{2b} are each independently, together with the carbon atom to which they are attached, form a C3-C6 monocyclic cycloalkyl or a 3- to 6-membered monocyclic heterocyclyl, in which the C3-C6 monocyclic cycloalkyl or the 3- to 6-membered monocyclic heterocyclyl is optionally substituted with1, 2, or 3 groups selected from halogen, cyano, C1-C4 alkyl, C1-C4 alkoxy, C1-C4 haloalkyl, C1-C4 haloalkoxy, C6-C10 aryl, or a 5- or 6-membered monocyclic heteroaryl. Preferably, the hexahydro-1H-pyrrolizinyl is further optionally substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, L₃ is O; and R⁶ is -L-hexahydro-1H-pyrrolizinyl, where L is C1-C4 alkylene; and the hexahydro-1H-pyrrolizinyl is substituted with 1 or more (for example, 1, 2, 3, 4 or 5) R^{6a}, where R^{6a} is each independently halogen, hydroxyl, C1-C6 hydroxylalkyl, C1-C6 alkyl, C2-C6 alkenyl, C2-C6 alkynyl, C1-C6 haloalkyl, C1-C6 alkoxy, C1-C6 haloalkoxy or cyano.

In one embodiment, R⁶ is selected from the group consisting of: 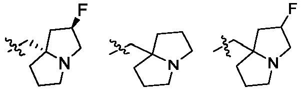 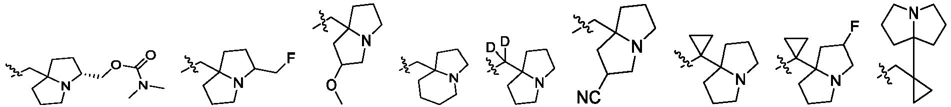 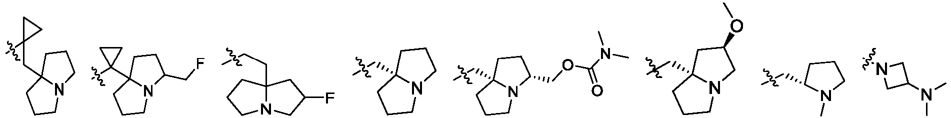 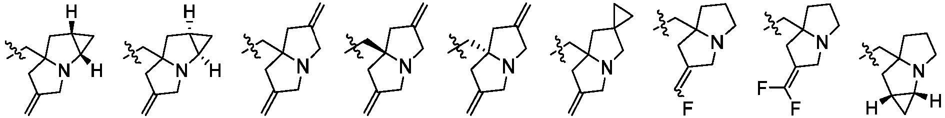 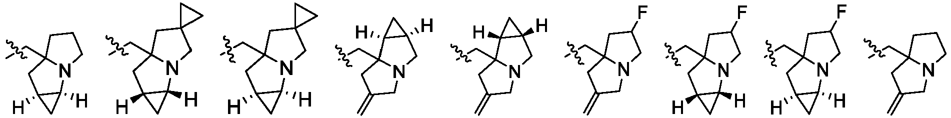 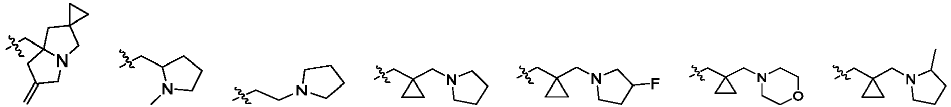 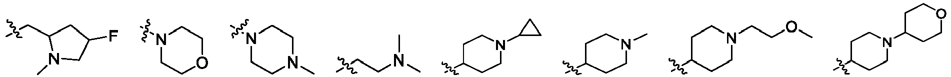 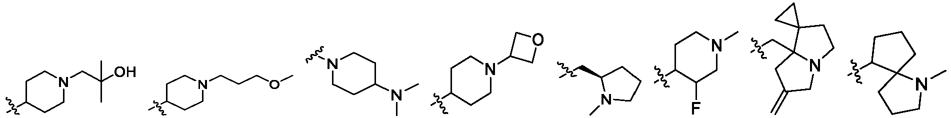 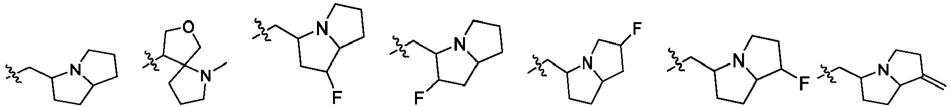 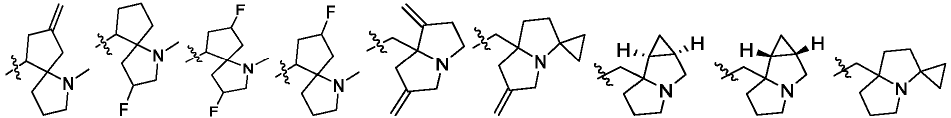 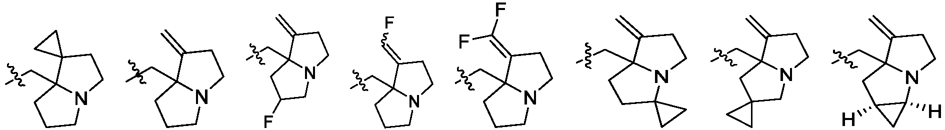 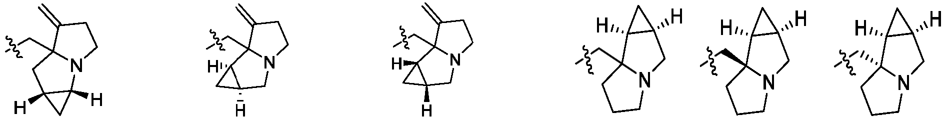 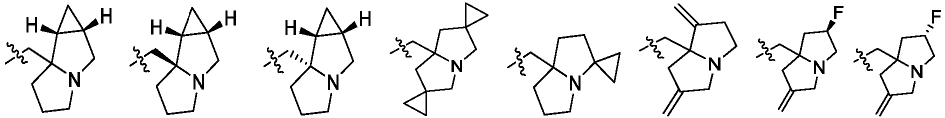 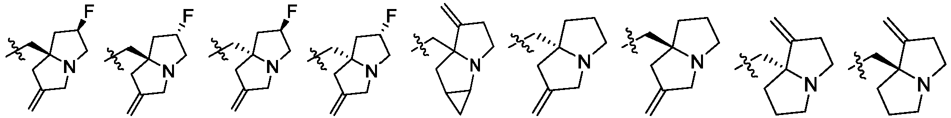 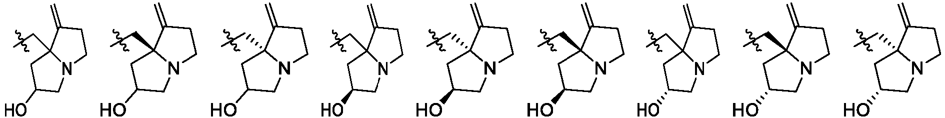 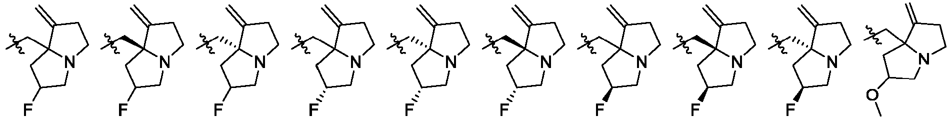 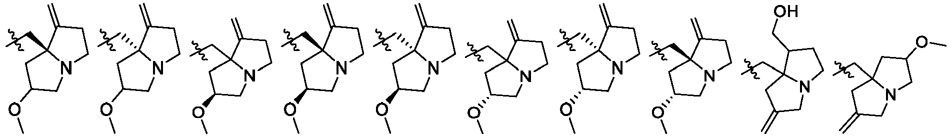 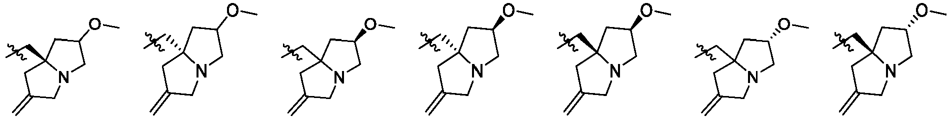 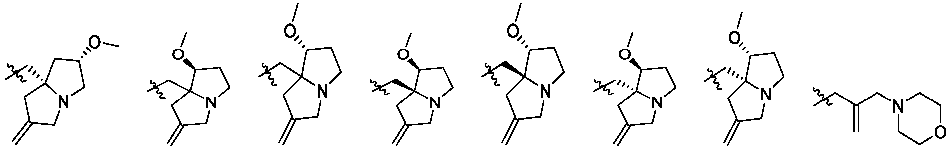 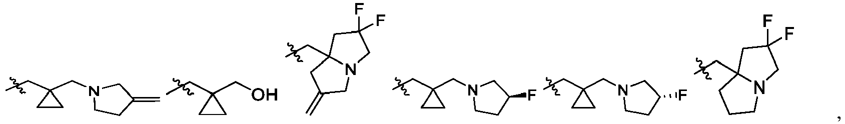 methyl,

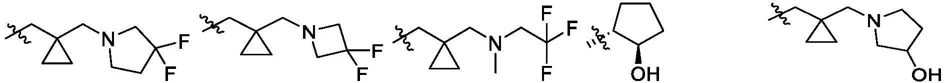

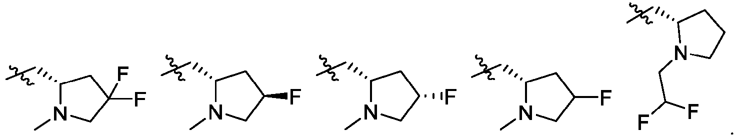

In one embodiment, R⁶ is a -L-6- to 10-membered fused heterocyclyl, where the 6- to 10-membered fused heterocyclyl has 1, 2, 3 or 4 heteratoms selected from N(R^{m}), S, S(=O), S(=O)₂, and O as the ring atom; and R^{m} is absent, hydrogen, C1-C4 alkyl, deuteratedC1-C4 alkyl, C1-C4 haloalkyl, C3-C20 cycloalkyl or 3- to 20-membered heterocyclyl, where L is a bond, C1-C4 alkylene or heteroaryl; the 6- to 10-membered fused heterocyclyl is saturated; 2 hydrogen atoms on the same carbon atom in any 1 or 2 carbon atoms in the 6- to 10-membered fused heterocyclyl are optionally both replaced by =CR^{2a}R^{2b}; the 6- to 10-membered fused heterocyclyl is optionally further substituted with 1 or more R^{6a}; and R^{2a}, R^{2b}, and R^{6a} are each as defined above. Preferably, R^{2a} and R^{2b} are each independently hydrogen, halo or C1-C4 alkyl, C1-C4 haloalkyl; and R^{6a} is halogen, hydroxyl, or C1-C6 alkyl.

In one embodiment, R⁶ is a -L-6- to 10-membered fused heterocyclyl; and the 6- to 10-membered fused heterocyclyl is hexahydro-1H-pyrrolizinyl, where L is C1-C4 alkylene; 2 hydrogen atoms on the same carbon atom in any 1 or 2 carbon atoms in the 6- to 10-membered fused heterocyclyl are optionally both replaced by =CR^{2a}R^{2b}; the 6- to 10-membered fused heterocyclyl is optionally further substituted with 1 or more R^{6a}; and R^{2a}, R^{2b}, and R^{6a} are each as defined above. Preferably, R^{2a} and R^{2b} are each independently hydrogen, halo or C1-C4 alkyl, C1-C4 haloalkyl; and R^{6a} is halogen, hydroxyl, or C1-C6 alkyl.

In one embodiment, R⁶ is a -L-6- to 10-membered fused heterocyclyl; and the 6- to 10-membered fused heterocyclyl is hexahydro-1H-pyrrolizinyl, where L is C1-C2 alkylene; 2 hydrogen atoms on the same carbon atom in any 1 or 2 carbon atoms in the 6- to 10-membered fused heterocyclyl are optionally both replaced by =CR^{2a}R^{2b}; the 6- to 10-membered fused heterocyclyl is optionally further substituted with 1 or more R^{6a}; R^{2a} and R^{2b} are each independently hydrogen or fluoro; and R^{6a} is halogen, hydroxyl or C1-C6 alkyl.

In one embodiment, R⁶ is selected from the group consisting of: 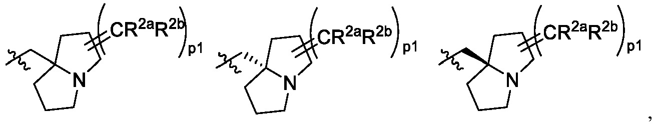 where in each formula, p1 is each independently 1 or 2; and R^{2a} and R^{2b} are each as defined above. Preferably, R^{2a} and R^{2b} are each independently hydrogen, halo or C1-C4 alkyl, C1-C4 haloalkyl. Preferably, the 1H-pyrrolizine is further optionally substituted with 1 or more R^{6a}; and R^{6a} is as defined above. Preferably, R^{6a} is each independently halo or C1-C6 alkoxy.

In one embodiment, R⁶ is selected from the group consisting of:

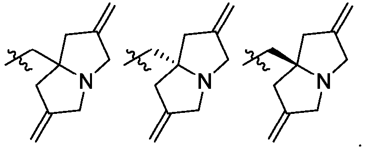

In one embodiment, R⁶ is selected from the group consisting of: 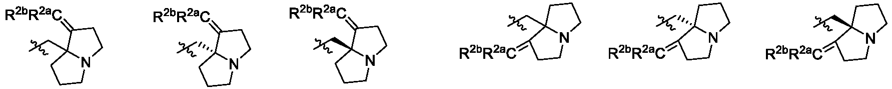 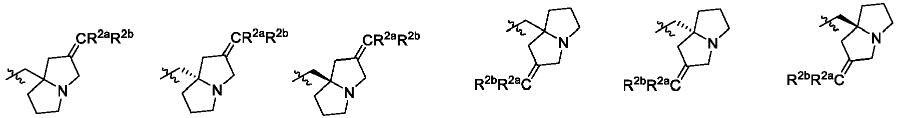 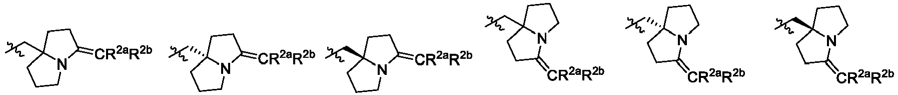 where in each formula, R^{2a} and R^{2b} are each as defined above. Preferably, R^{2a} and R^{2b} are each independently hydrogen, halo or C1-C4 alkyl, C1-C4 haloalkyl. Preferably, the 1H-pyrrolizine is further optionally substituted with 1 or more (for example, 2 or 3) R^{6a}; and R^{6a} is as defined above. Preferably, R^{6a} is each independently halo or C1-C6 alkoxy.

In some other embodiments, the compound is selected from Table (I):

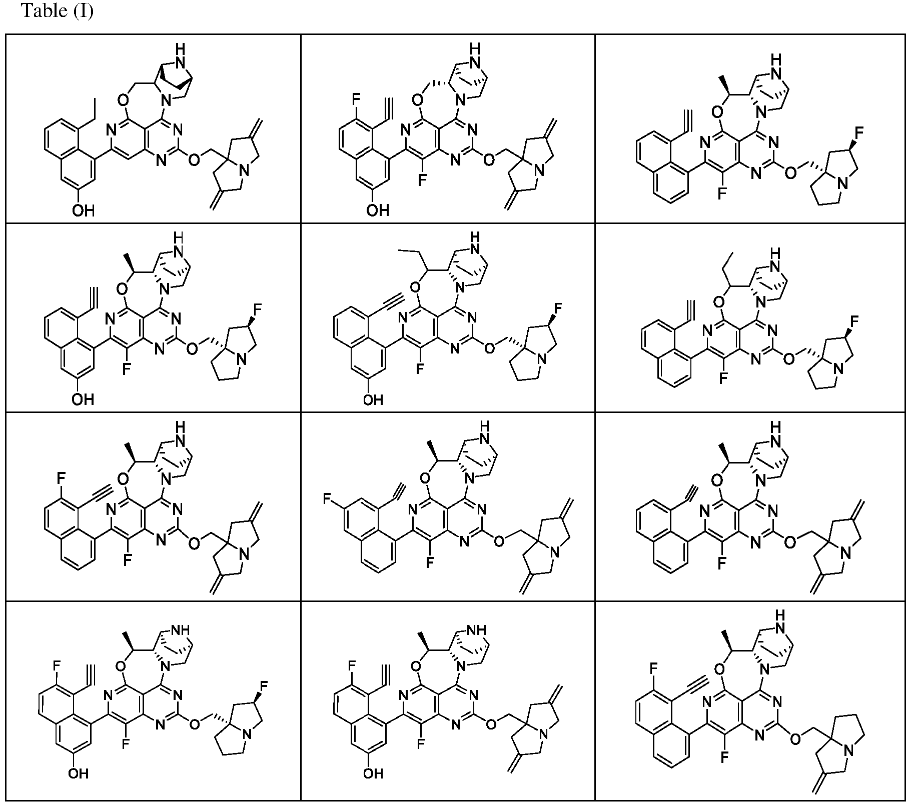

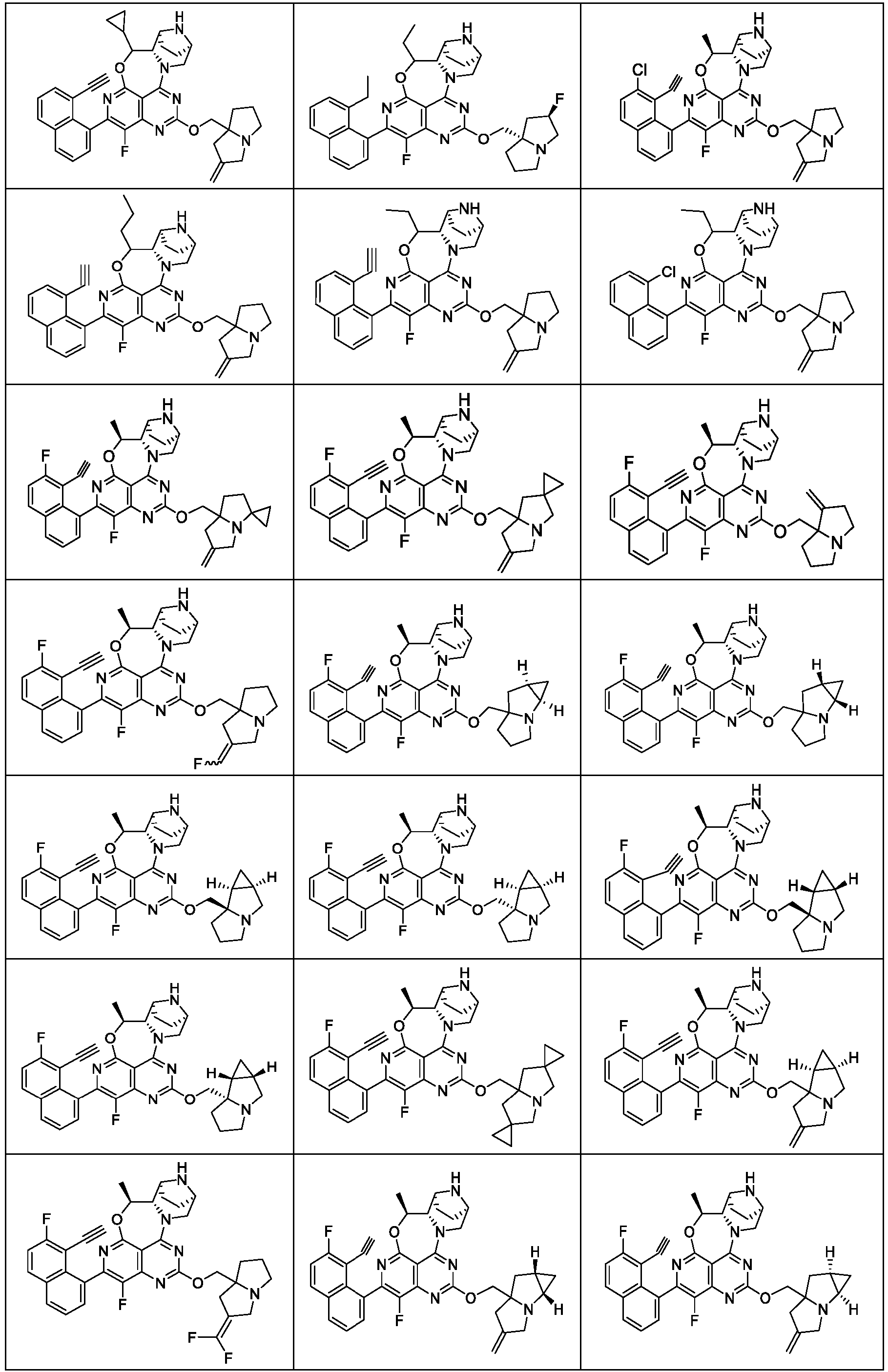

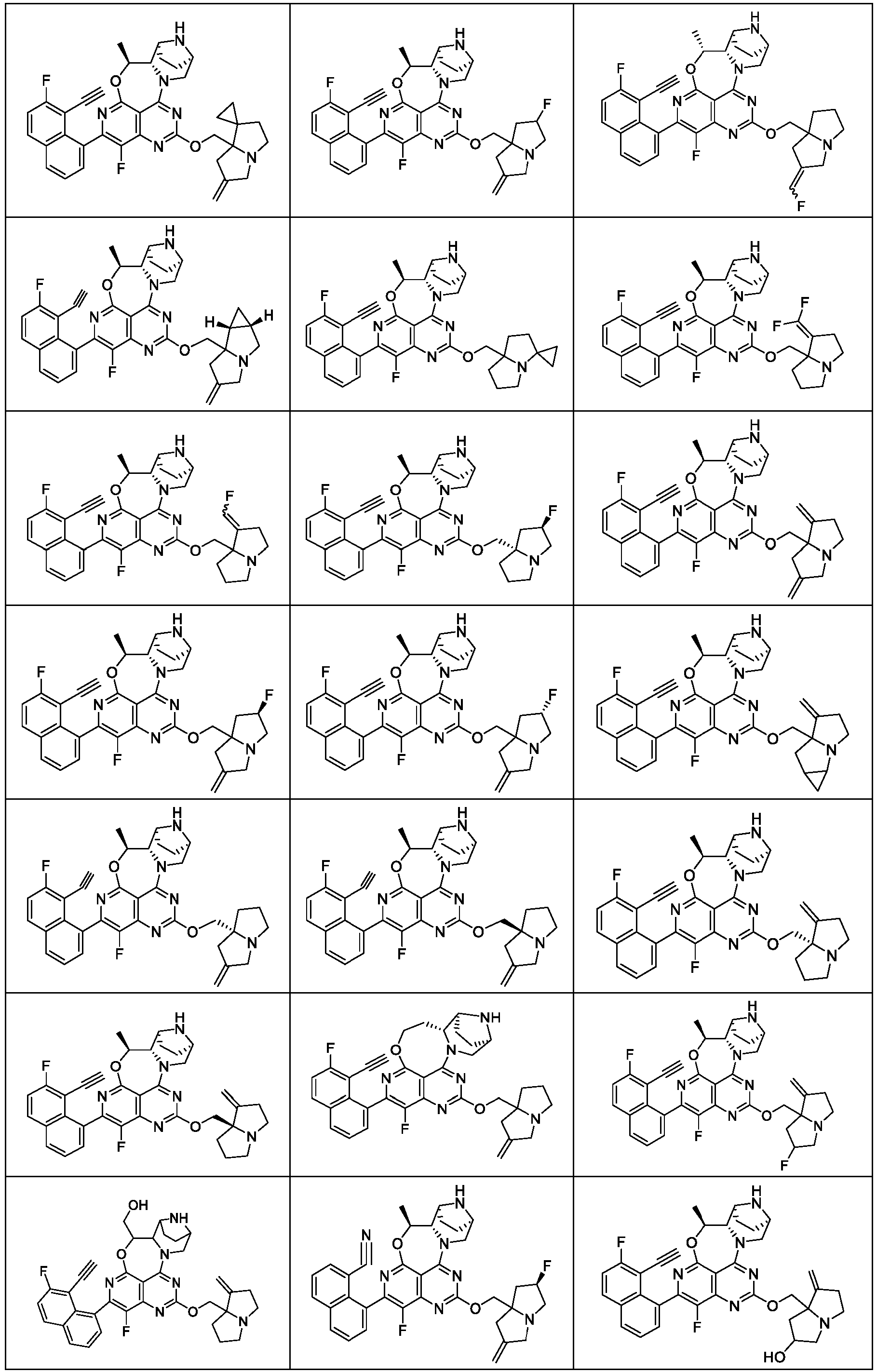

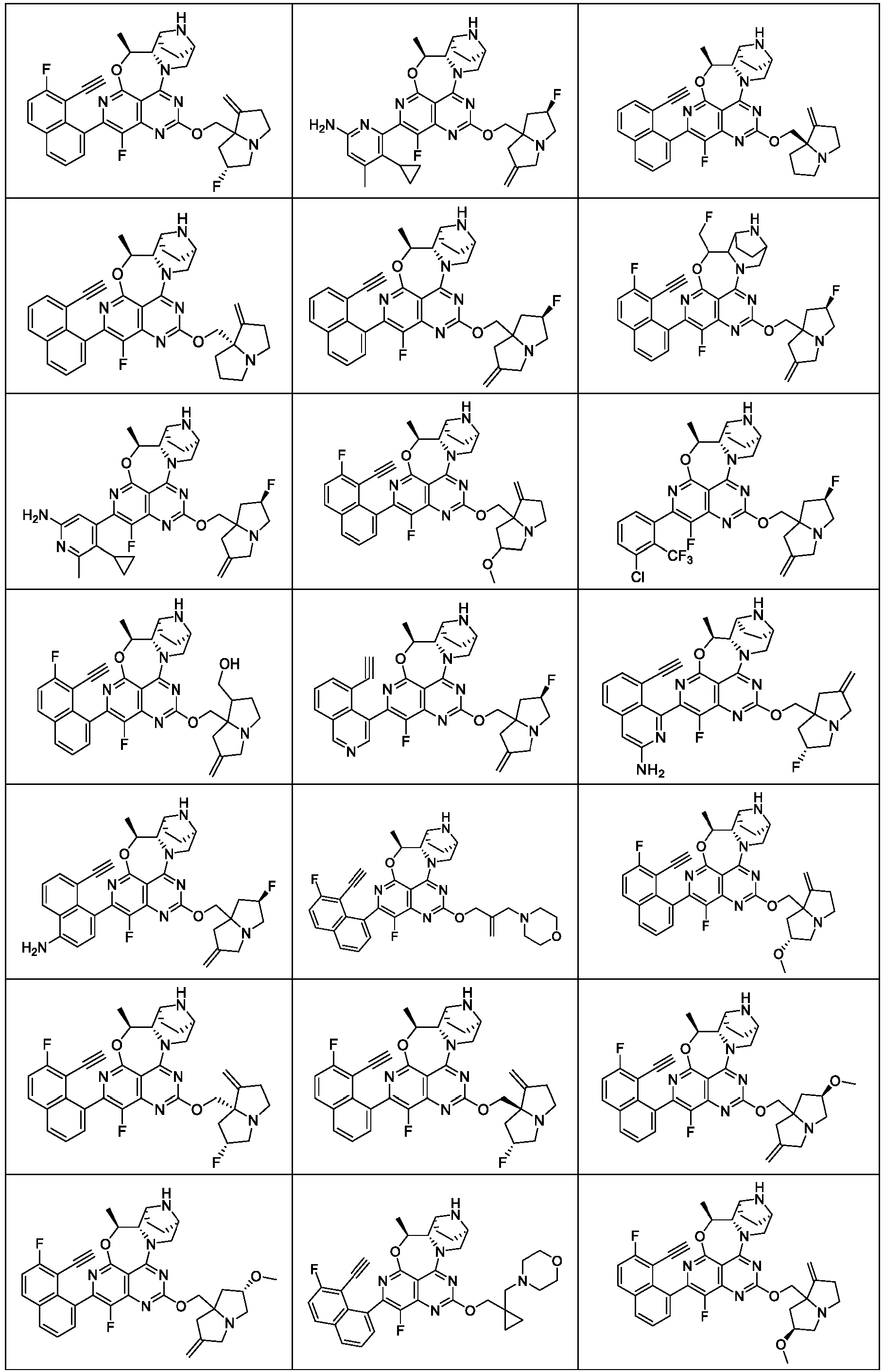

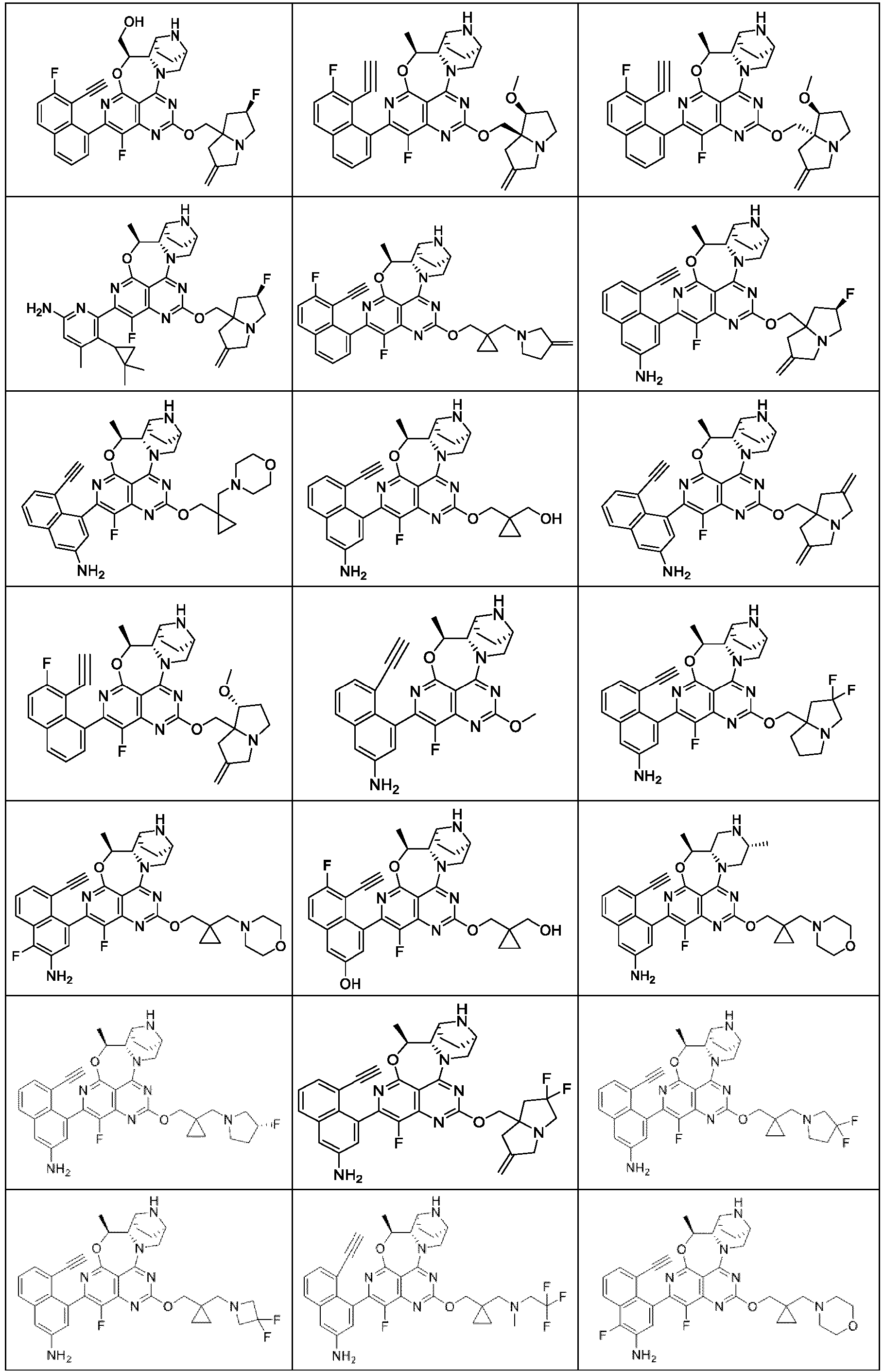

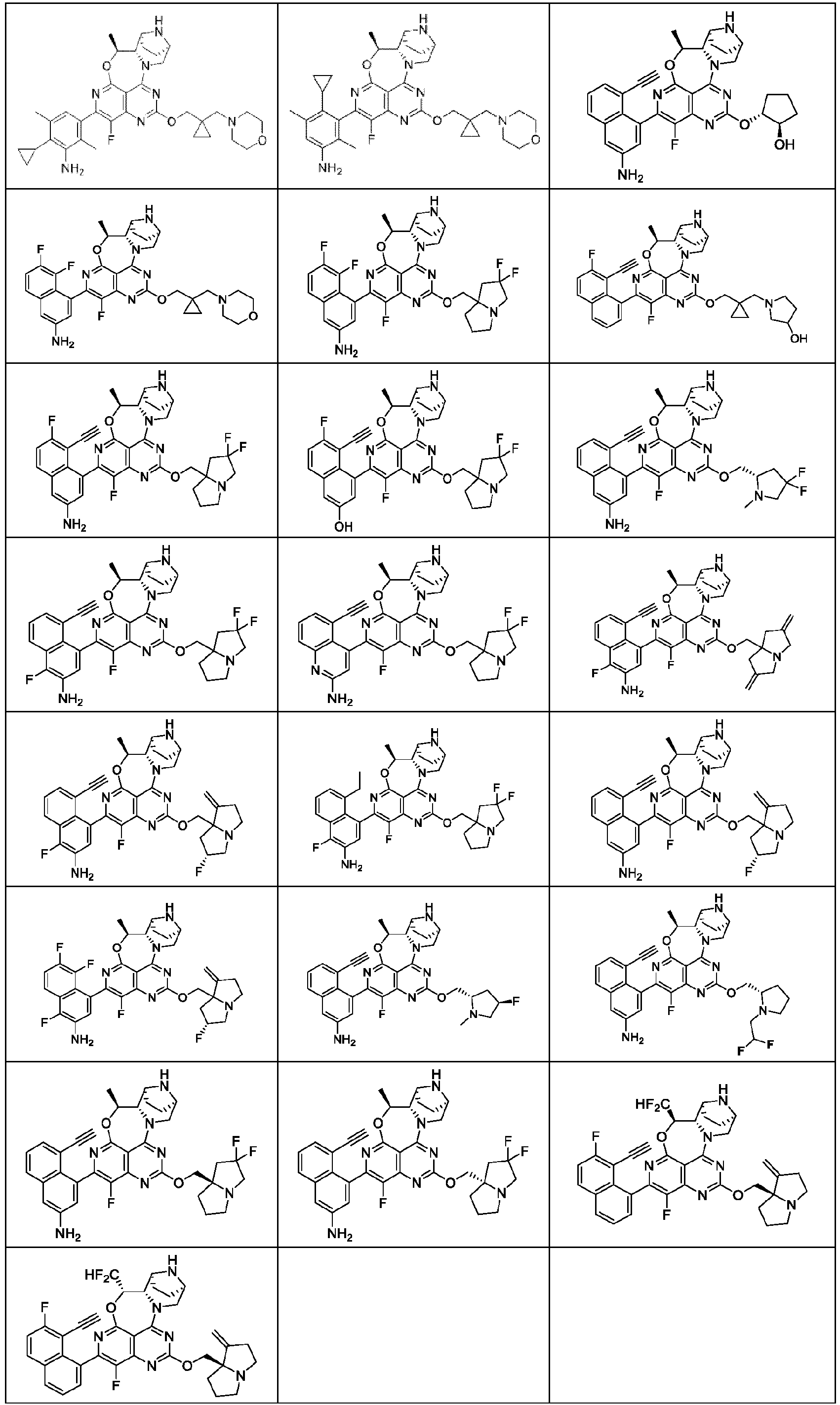

The first aspect of the present invention also provides a compound represented by Formula (Z1'), and a stereoisomer, a deuterated compound or a pharmaceutically acceptable salt thereof, 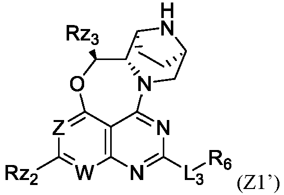 where:
W is N or CRz1, where Rz1 is hydrogen, halogen, C1-C6 alkyl or C1-C6 alkoxy;
Z is N or CRz11, where Rz11 is hydrogen, halogen, C1-C6 alkyl or C1-C6 alkoxy;
Rz2 is substituted or unsubstituted phenyl, naphthyl, quinoline, isoquinoline, benzo [b]thiophene, benzo[d]thiazole, indole, indazole or pyridine, where the substitution means that 1, 2, 3, 4 or 5 hydrogen atoms on phenyl, naphthyl, quinoline, isoquinoline, benzo[b]thiophene, benzo[d]thiazole, indole, indazole or pyridine are each independently replaced by R¹;
Rz3 is hydrogen, deuterium, methyl, deuterated methyl or halomethyl or -CD₂F or -CDF₂;
R¹ is selected from the group consisting of: C1-C6 alkyl, halogen, hydroxyl, cyano, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyalkynyl, C1-C6 cyanoalkyl, triazolyl, - S-C1-C6 haloalkyl, C1-C6 hydroxylalkyl, -CH₂C(O)NR²⁷R²⁸, -C2-C6 alkynyl-NR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, where
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxy, cyano, heterocyclyl, NR²⁵¹R²⁵², C(O)NR²⁵³R²⁵⁴ or SO₂C1-C6 alkyl, where R²⁵¹, R²⁵², R²⁵³ and R²⁵⁴ are each independently H or C1-C6 alkyl; or R²⁵¹ and R²⁵², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy; or R²⁵³ and R²⁵⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
-L₃-R⁶ is 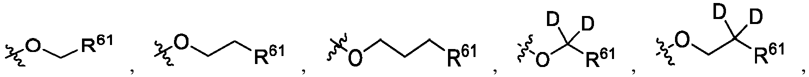 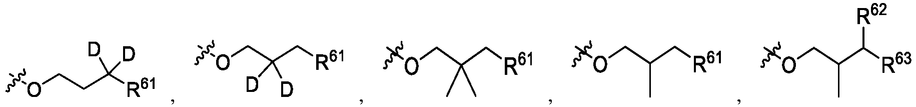 or 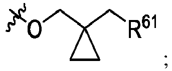
R⁶¹ is a 3- to 6-membered heterocyclic group or a 6- to 10-membered fused heterocyclic group;
the 3- to 6-membered heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 3- to 6-membered heterocyclyl are optionally both replaced by =CR^{2a}R^{2b}, where R^{2a} and R^{2b} is each independently hydrogen, halogen, C1-C4 alkyl or C1-C4 haloalkyl;
the 6- to 10-membered fused heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 6- to 10-membered fused heterocyclyl are optionally both replaced by deuterium or =CR^{2a}R^{2b}, where R^{2a} and R^{2b} are each independently hydrogen, halogen, C1-C4 alkyl or C1-C4 haloalkyl; or each pair of R^{2a} and R^{2b} independently form, together with the carbon atom to which they are attached, a C3-C6 monocyclic cycloalkyl or a 3- to 6-membered monocyclic heterocyclyl; and
the 3- to 6-membered heterocyclyl or the 6- to 10-membered fused heterocyclyl is further optionally substituted with 1 or 2 groups selected from C1-C4 alkyl, C1-C4 haloalkyl, and halo;
the R⁶² is hydroxyl or halo; and R⁶³ is C1-C4 alkyl, C1-C4 haloalkyl or halo.

In one embodiment, the compound represented by Formula (Z1') is a compound represented by Formula (Z1), Formula (Z2) or Formula (Z3): 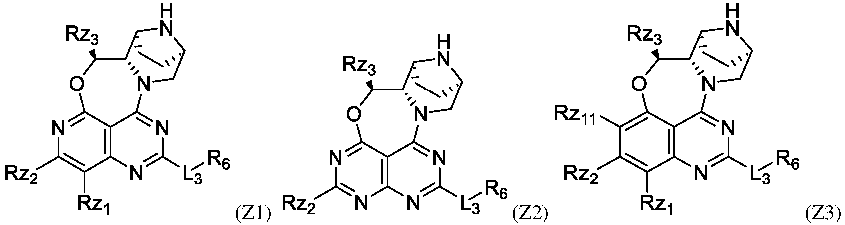 where in the formula, Rz11, Rz1, Rz2, Rz3, L₃, and R₆ are each as defined above.

In one embodiment, Rz3 is methyl, deuterated methyl or halomethyl.

In one embodiment, the compound represented by Formula (Z1') is a compound represented by Formula (Z), where in the formula, Rz1 and Rz2 are each as defined above; 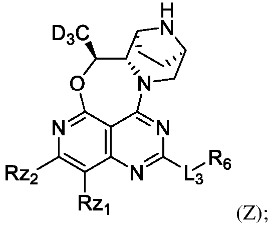 -L₃-R⁶ is 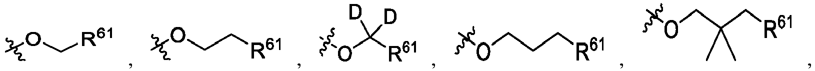 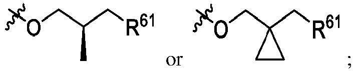 R⁶¹ is a 3- to 6-membered heterocyclyl or a 6- to 10-membered fused heterocyclyl; the 3- to 6-membered heterocyclyl is optionally substituted with 1 or 2 groups selected from C1-C4 alkyl, C1-C4 haloalkyl, and halo; and 2 hydrogen atoms on the same carbon atom in any 1 or 2 carbon atoms in the 6- to 10-membered fused heterocyclyl are optionally both replaced by deuterium or =CR^{2a}R^{2b}, where R^{2a} and R^{2b} are each independently hydrogen, halogen, C1-C4 alkyl or C1-C4 haloalkyl.

In one embodiment, the compound represented by Formula (Z1') is a compound represented by Formula (Z): 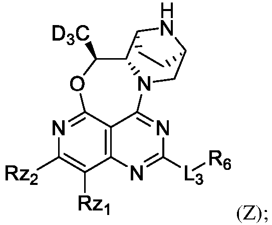 where in the formula, Rz1, Rz2, and R₆ are each as defined above; -L₃-R⁶ is 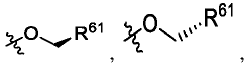 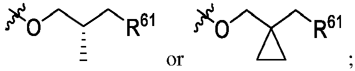 and R⁶¹ is as defined above. When used as a KRAS G12D inhibitor, the compound represented by Formula (Z) of the present invention has better bioavailability and higher AUC.

In one embodiment, the compound represented by Formula (Z1') is a compound represented by Formula (Z): 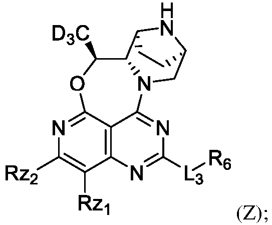 where in the formula,
Rz1 is halo or hydrogen;
Rz2 is a substituted or unsubstituted phenyl, naphthyl or pyridine, where the substitution means that 1, 2, 3, 4 or 5 hydrogen atoms on the phenyl, naphthyl or pyridine are independently replaced by R¹;
R¹ is selected from the group consisting of: C1-C6 alkyl, halogen, hydroxyl, cyano, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyalkynyl, C1-C6 cyanoalkyl, triazolyl, - S-C1-C6 haloalkyl, C1-C6 hydroxylalkyl, -CH₂C(O)NR²⁷R²⁸, -C2-C6 alkynyl-NR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, where
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxy, cyano, heterocyclyl, NR²⁵¹R²⁵², C(O)NR²⁵³R²⁵⁴ or SO₂C1-C6 alkyl, where R²⁵¹, R²⁵², R²⁵³ and R²⁵⁴ are each independently H or C1-C6 alkyl; or R²⁵¹ and R²⁵², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy; or R²⁵³ and R²⁵⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
-L₃-R⁶ is 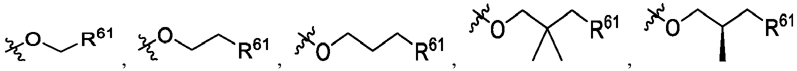 or 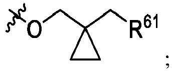 R⁶¹ is 3- to 6-membered heterocyclyl or 6- to 10-membered fused heterocyclyl; the 3- to 6-membered heterocyclyl is optionally substituted with 1 or 2 groups selected from C1-C4 alkyl, C1-C4 haloalkyl, and halo; 2 hydrogen atoms on the same carbon atom in any 1 or 2 carbon atoms in the 6- to 10-membered fused heterocyclyl are optionally both replaced by =CR^{2a}R^{2b}, where R^{2a} and R^{2b} are each independently hydrogen, halogen, C1-C4 alkyl or C1-C4 haloalkyl.

In one embodiment, Rz11 is chloro or hydrogen.

In one embodiment, Rz1 is fluoro or hydrogen.

In one embodiment, R⁶¹ is a 6- to 10-membered fused heterocyclyl; the 6- to 10-membered fused heterocyclyl is 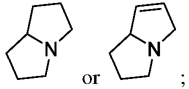 the 6- to 10-membered fused heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 6- to 10-membered fused heterocyclyl are optionally both replaced by =CR^{2a}R^{2b}, where R^{2a} and R^{2b} are each independently hydrogen, halogen, C1-C4 alkyl or C1-C4 haloalkyl; and the 6- to 10-membered fused heterocyclyl is further optionally substituted with 1 or 2 groups selected from C1-C4 alkyl, C1-C4 haloalkyl, and halo.

In one embodiment, R⁶¹ is a 3- to 6-membered heterocyclyl; the 3- to 6-membered heterocyclyl is tetrahydropyrrolyl, piperidinyl, piperazinyl or morpholinyl; the 3- to 6-membered heterocyclyl is optionally substituted with 1 or 2 groups selected from C1-C4 alkyl, C1-C4 haloalkyl, and halo; or 2 hydrogen atoms on the same carbon atom in any 1 or 2 carbon atoms in the 3- to 6-membered heterocyclyl are optionally both replaced by =CR^{2a}R^{2b}, where R^{2a} and R^{2b} are each independently hydrogen, halogen, C1-C4 alkyl or C1-C4 haloalkyl; and the 3- to 6-membered heterocyclyl is further optionally substituted with 1 or 2 groups selected from C1-C4 alkyl, C1-C4 haloalkyl, and halo.

In one embodiment, the 6 to 10 membered fused heterocyclyl is selected from the group consisting of:

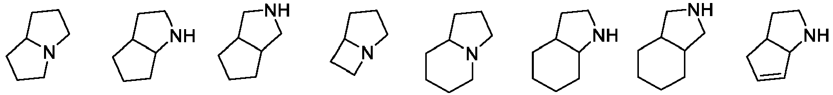

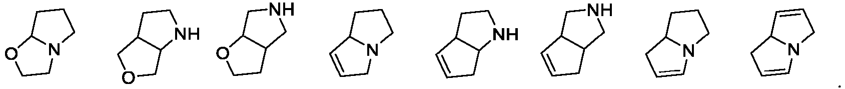

In one embodiment, the 6 to 10 membered fused heterocyclyl is selected from the group consisting of: 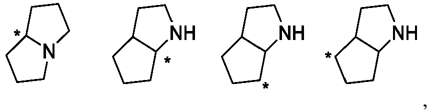 where the carbon atom marked with * is a carbon atom attached to the rest of the molecule.

**In** one embodiment, -L₃-R⁶ is

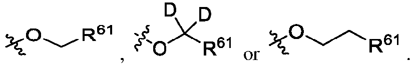

In one embodiment, R⁶¹ is independently selected from the group consisting of:

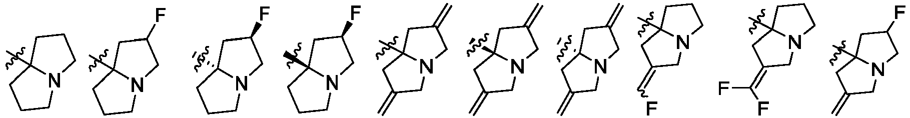

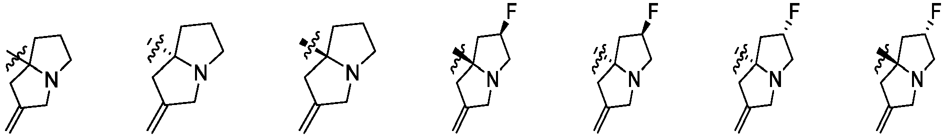

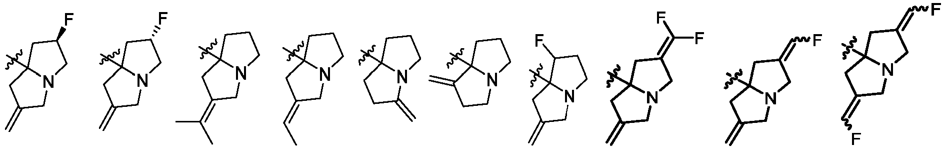

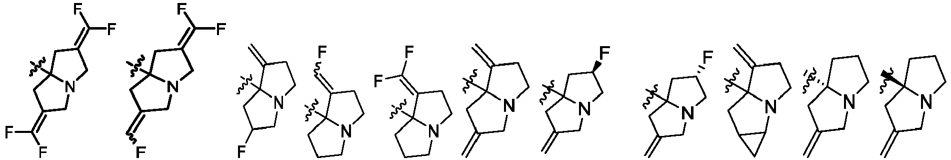

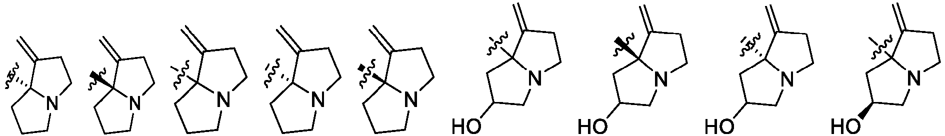

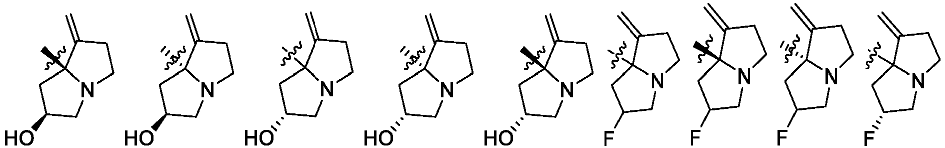

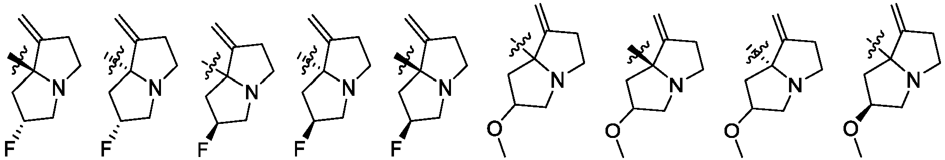

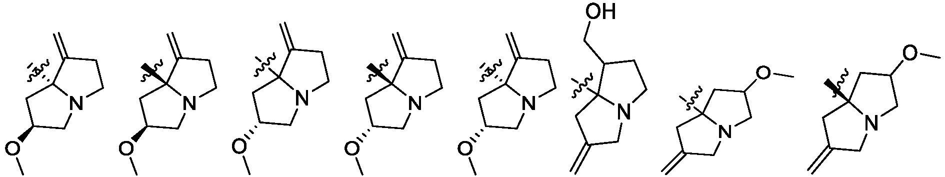

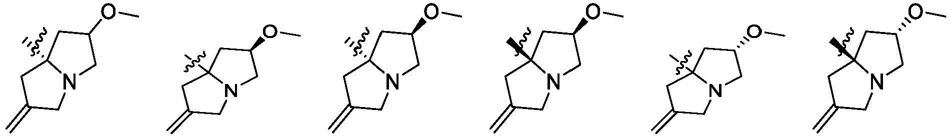

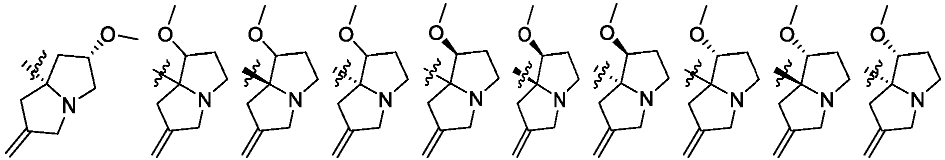

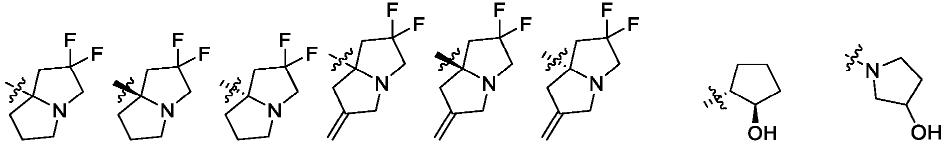

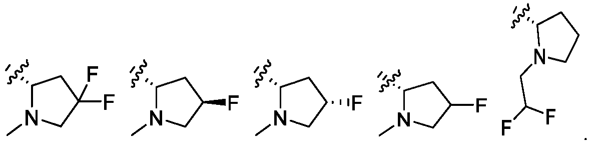

**In** one embodiment, -L₃-R⁶ is

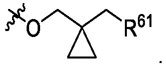

In one embodiment, R⁶¹ is independently selected from the group consisting of:

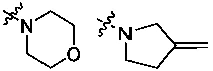

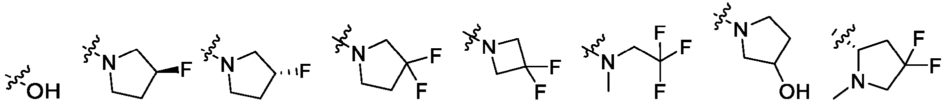

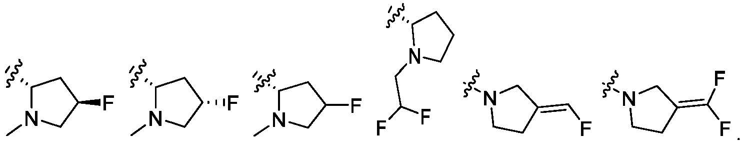

In one embodiment, -L₃-R⁶ is

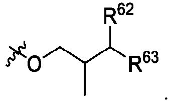

In one embodiment, R⁶² is hydroxyl or halo; and R⁶³ is methyl, ethyl, fluoromethyl, difluoromethyl or trifluoromethyl.

In one embodiment, R⁶² is hydroxyl, and R⁶³ is difluoromethyl.

In one embodiment, R⁶ is selected from the group consisting of: 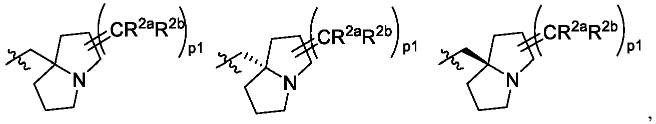 where in each formula, p1 is each independently 1 or 2; and R^{2a} and R^{2b} are each as defined above. Preferably, R^{2a} and R^{2b} are each independently hydrogen, halo or C1-C4 alkyl, C1-C4 haloalkyl. Preferably, the 1H-pyrrolizine is further optionally substituted with 1 or more R^{6a}; and R^{6a} is as defined above. Preferably, R^{6a} is each independently halo or C1-C6 alkoxy.

In one embodiment, R⁶¹ is selected from the group consisting of: 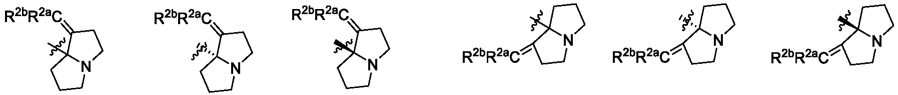 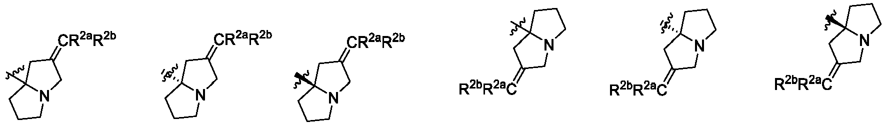 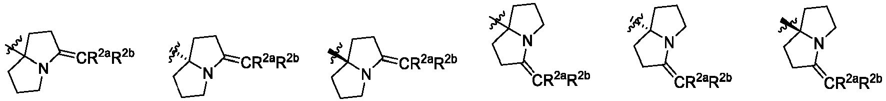 where in each formula, R^{2a} and R^{2b} are each independently hydrogen, halogen, C1-C4 alkyl or C1-C4 haloalkyl; or each pair of R^{2a}, R^{2b} independently form, together with the carbon atom to which they are attached, a C3-C6 monocyclic cycloalkyl or a 3- to 6-membered monocyclic heterocyclyl.

In one embodiment, in the above substituents, 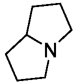 is optionally further substituted with 1 or 2 R^{6a}, in which R^{6a} is each independently hydrogen, deuterium or halo.

In one embodiment, in the above substituents, 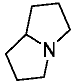 is optionally further substituted with 1 or 2 R^{6a}, in which R^{6a} is each independently deuterium or halo.

In one embodiment, R⁶¹ is selected from the group consisting of:

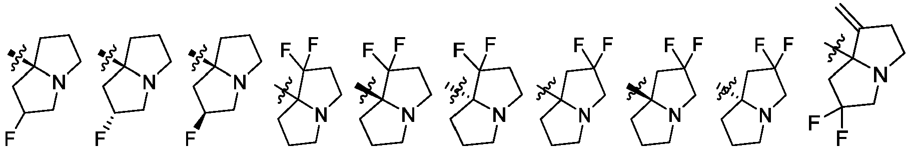

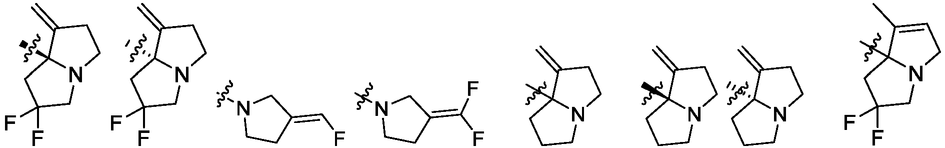

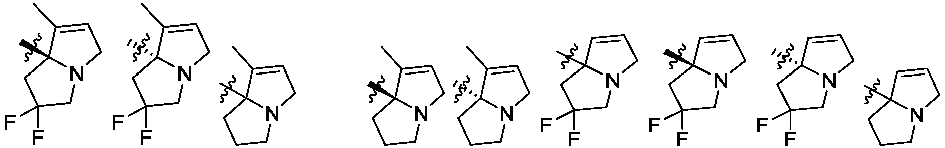

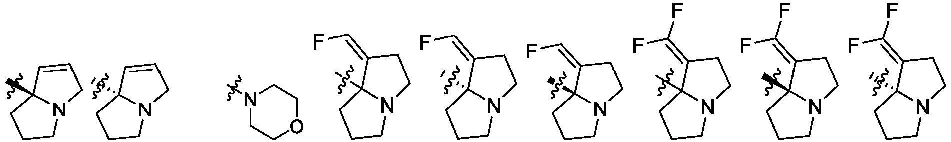

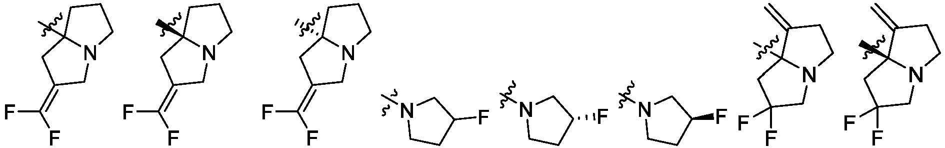

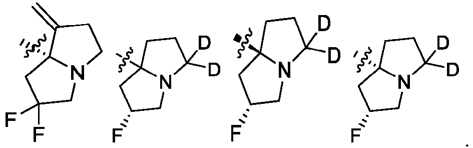

In one embodiment, R¹ is selected from: C1-C3 alkyl, halogen, hydroxyl, C1-C3 alkoxy, C1-C3 haloalkyl, C1-C3 haloalkoxy, cyano, NR²¹R²², C(O)NR²³R²⁴, CH₂R²⁵, N=S(O)(C1-C3 alkyl)₂, S(O)C1-C3 alkyl, S(O)₂R²⁶, - S-C1-C3 alkyl, C2-C4 alkenyl, C2-C4 alkynyl, C2-C4hydroxyalkynyl, C1-C3 cyanoalkyl, triazolyl, -S-C1-C3 haloalkyl, C1-C3 hydroxylalkyl, -CH₂C(O)NR²⁷R²⁸, -C2-C4 alkynylNR²⁹R³⁰, C2-C4 deuterated alkynyl, (C1-C3 alkoxy)C1-C3 haloalkyl- or cycloalkyl, where the cycloalkyl is optionally substituted with halo or C1-C3 alkyl, where
R²¹ is H, C1-C3 alkyl, C1-C3 haloalkyl, C(O)C1-C3 alkyl or C(O)OC1-C3 alkyl; R²² is H, C1-C3 alkyl or C1-C3 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C3 alkyl, or C1-C3 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁵ is hydroxyl, cyano, heterocyclyl, NR²⁵¹R²⁵², C(O)NR²⁵³R²⁵⁴ or SO₂C1-C3 alkyl, where R²⁵¹, R²⁵², R²⁵³ and R²⁵⁴ are each independently H or C1-C3 alkyl; R²⁵¹ and R²⁵², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy; and R²⁵³ and R²⁵⁴, together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy;
R²⁶ is C1-C3 alkyl, C1-C3 haloalkyl, or NR²⁶¹R²⁶², where R²⁶¹ and R²⁶² are each independently H or C1-C3 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C3 alkyl, or C1-C3 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁹ and R³⁰ are each independently H, C1-C3 alkyl, or C1-C3 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy.

In one embodiment, Rz2 is substituted or unsubstituted phenyl, naphthalenyl or pyridinyl, where the substitution means that 1, 2 or 3 hydrogen atoms on the phenyl, naphthalenyl or pyridinyl are each independently substituted with R¹; and R¹ is selected from the group consisting of: C1-C6 alkyl, halogen, C1-C6 haloalkyl, NR²¹R²²; R²¹ is H, C1-C3 alkyl, C1-C3 haloalkyl, C(O)C1-C3 alkyl or C(O)OC1-C3 alkyl; R²² is H, C1-C3 alkyl or C1-C3 haloalkyl; or R²¹ and R²², together with the nitrogen atom to which they are attached, form a 3- to 6-membered nitrogen containing heterocyclic group, where the 3- to 6-membered nitrogen containing heterocyclic group is optionally substituted with 1 or 2 groups selected from halogen, methyl, trifluoromethyl, methoxy, and trifluoromethoxy. Preferably, R²¹ is H; and R²² is H.

In one embodiment, Rz2 is phenyl substituted with 1, 2, 3 or 4 R¹, where each R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, Rz2 is phenyl substituted with 1, 2 or 3 R¹, where each R¹ is each independently halogen, hydroxyl, ethynyl, methyl, methyl-O-, methyl-S-, trifluoromethyl, trifluoromethyl-O-, amino, isopropyl or cyclopropyl.

In one embodiment, Rz2 is naphthalenyl substituted with 1, 2, 3 or 4 R¹, where each R¹ is each independently halogen, hydroxyl, ethynyl, methyl, methyl-O-, methyl-S-, trifluoromethyl, trifluoromethyl-O-, amino, isopropyl, cyano or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, Rz2 is naphthalenyl substituted with 1, 2 or 3 R¹, where each R¹ is each independently halogen, hydroxyl, ethynyl, methyl, ethyl, amino, cyano or difluoromethyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, Rz2 is naphthalenyl substituted with 1 or 2 R¹, where each R¹ is each independently halogen, ethynyl, or amino.

In one embodiment, Rz2 is pyridinyl substituted with 1, 2, 3 or 4 R¹, where each R¹ is each independently halogen, hydroxyl, ethynyl, methyl, methyl-O-, methyl-S-, trifluoromethyl, trifluoromethyl-O-, amino, isopropyl, or cyclopropyl; and the cyclopropyl is optionally substituted with halo or methyl. Preferably, one R¹ is ethynyl, and the remaining R¹ is each independently halogen, hydroxyl, ethynyl, methyl, methyl-O-, methyl-S-, trifluoromethyl, trifluoromethyl-O-, amino, isopropyl or cyclopropyl; and the cyclopropyl is optionally substituted with halo or methyl.

In one embodiment, Rz2 is selected from the group consisting of:

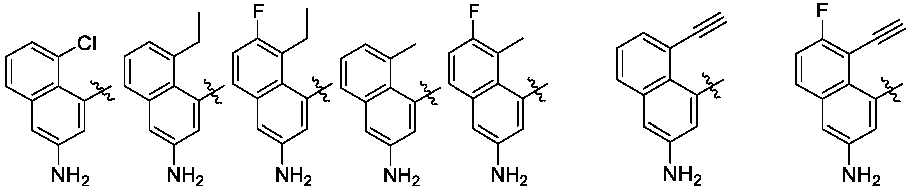

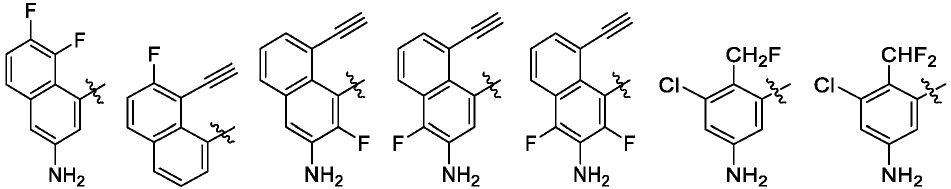

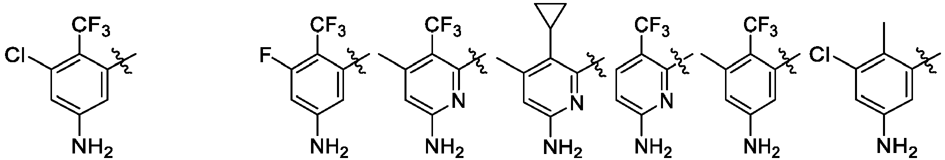

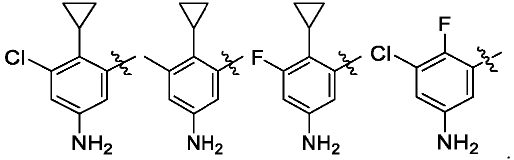

In some other embodiments, the compound is selected from Table A

In some other embodiments, the compound is selected from the group consisting of:

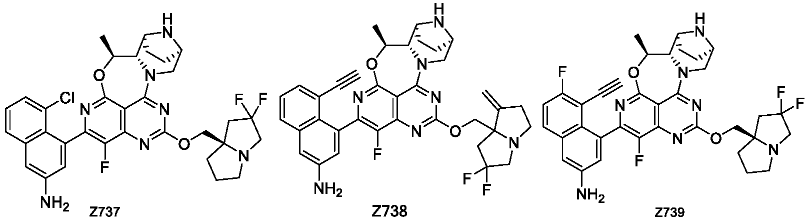

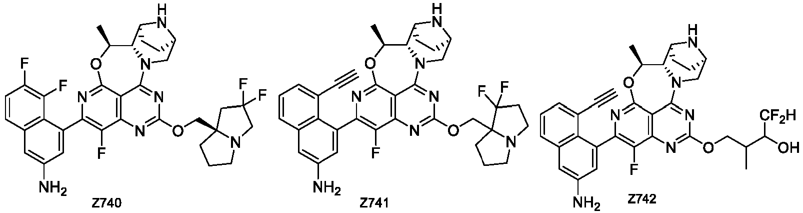

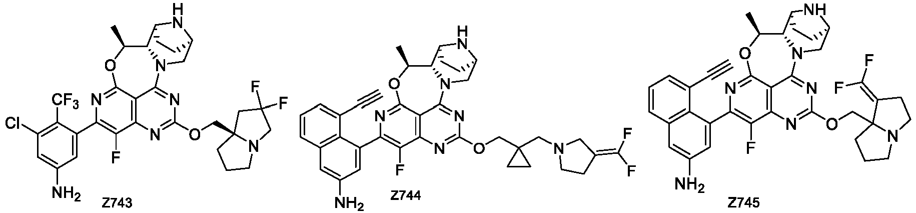

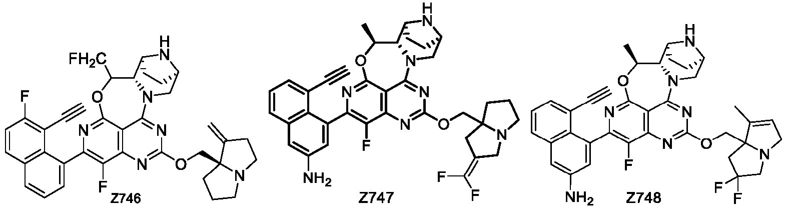

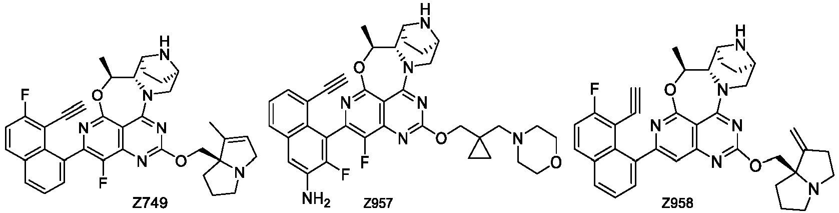

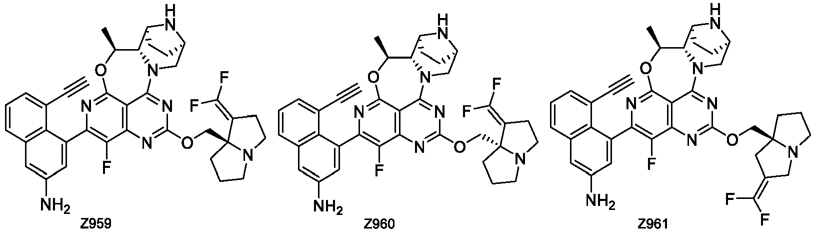

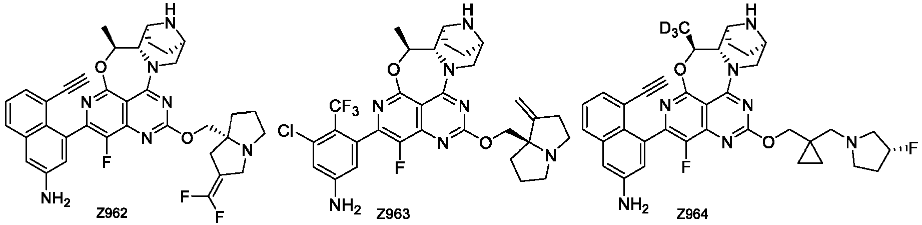

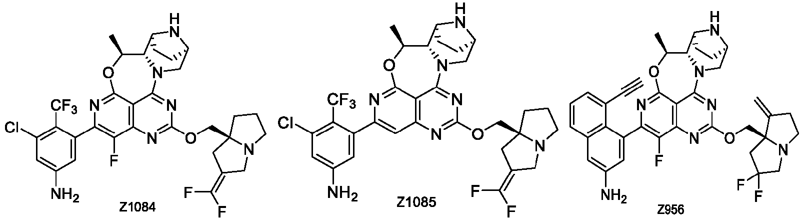

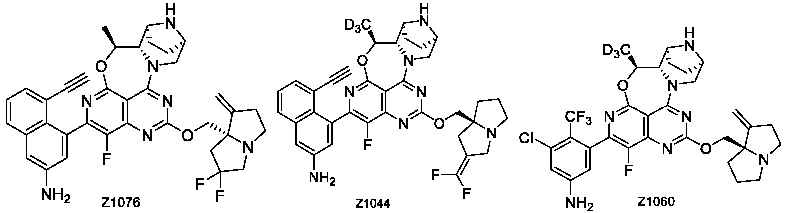

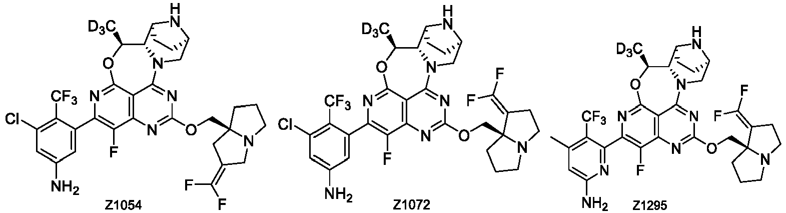

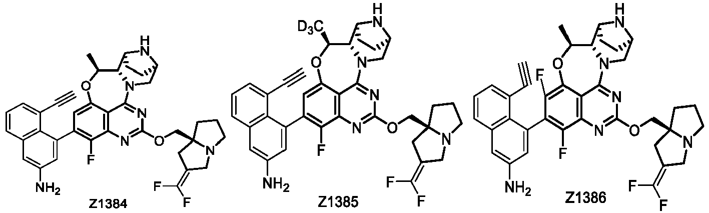

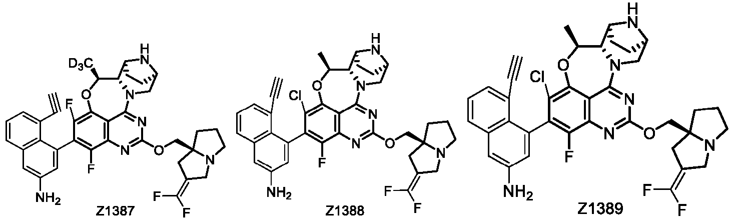

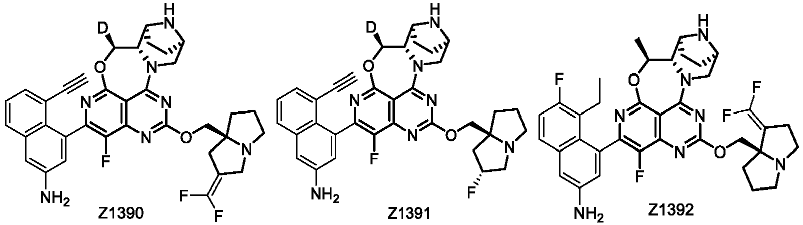

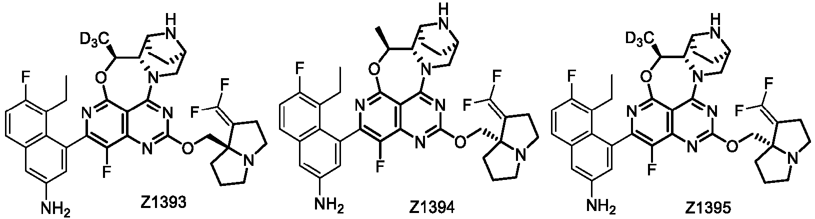

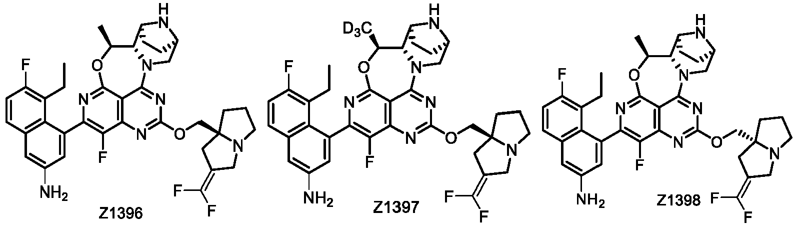

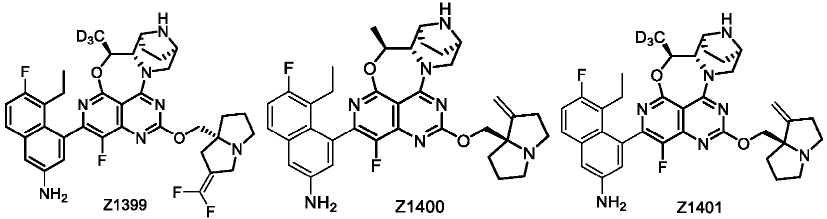

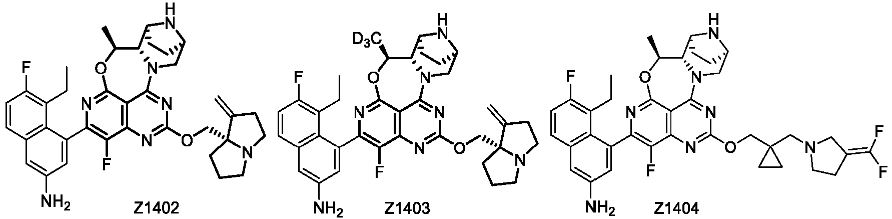

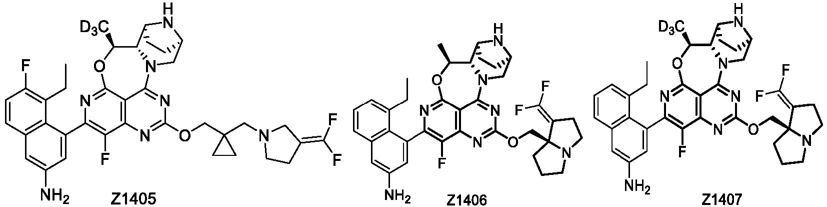

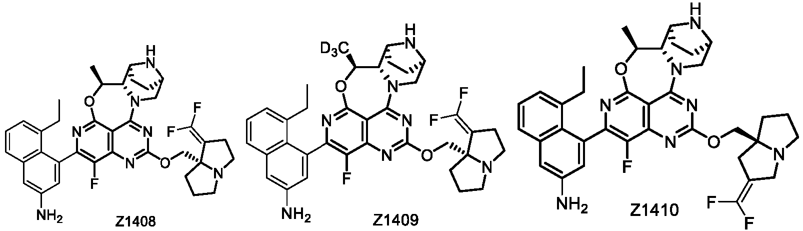

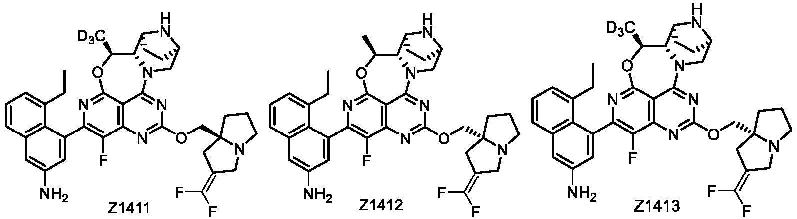

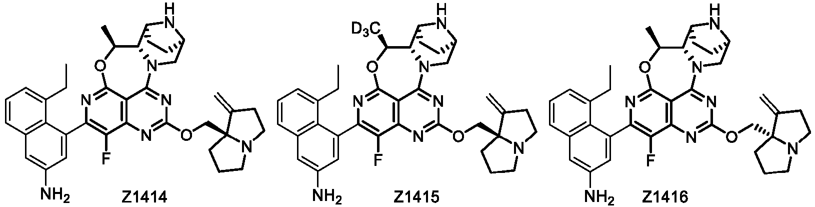

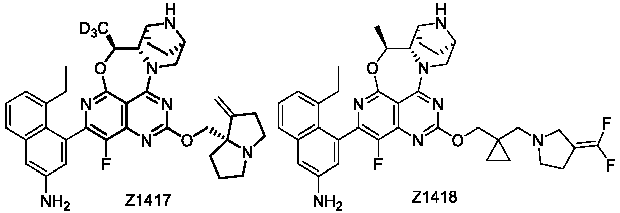

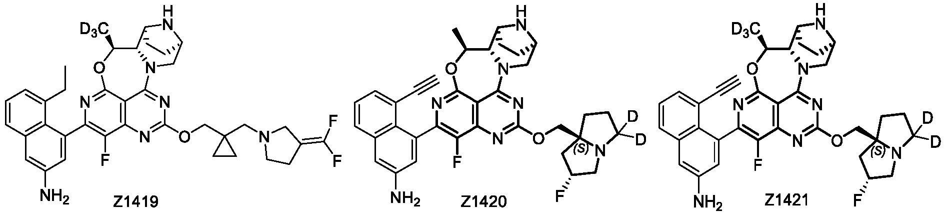

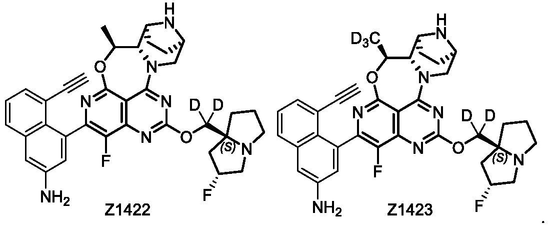

A second aspect of the present invention provides a pharmaceutical composition, which comprises the compound according to the first aspect or a pharmaceutically acceptable salt, stereoisomer, deuterated compound or solvate thereof, and a pharmaceutically acceptable carrier.

As used herein, the term "pharmaceutically acceptable carrier" refers to any representative carriers for preparations or carrier media, including water, oil, vegetables and minerals, paste base, lotion base, and ointment base, etc., which can deliver an effective amount of the active substance of the present invention without interfering with the biological activity of the active substance, and has no toxic or side effects on the host or subjects. These bases include suspending agents, tackifiers, and transdermal enhancers, etc. Their preparations are well known to those skilled in the art of cosmetics or topical medication

In an embodiment of the present invention, the pharmaceutical composition can be administered through any of the following routes: oral administration, spray inhalation, rectal administration, nasal administration, buccal administration, topical administration, parenteral administration, such as subcutaneous, intravenous, intramuscular, intraperitoneal, intrathecal, intraventricular, intrasternal and intracranial injection or infusion, or administration by means of an implanted reservoir. Among them, oral, intraperitoneal or intravenous administration is preferred. When orally administered, the compound of the present invention can be prepared into any form of orally acceptable preparations, including, but not limited to, tablets, capsules, aqueous solutions or aqueous suspensions. The carriers used in tablets generally include lactose and corn starch, and lubricants such as magnesium stearate can also be added. Diluents used in capsule preparations generally include lactose and dried corn starch. An aqueous suspension preparation are usually prepared by mixing an active ingredients with a suitable emulsifier and suspending agent. If needed, some sweeteners, fragrances or colorants can be added to the above oral preparations. When administered locally, particularly, to treat the affected face or organ that is easy to be reached by topical application, such as nervous diseases of eyes, skin or lower intestine, the compound of the present invention can be prepared into different forms of topical preparations according to different affected faces or organs. When administered locally to eyes, the compound of the present invention can be prepared into a preparation form of micro-suspensions or solutions, and the carrier used is isotonic sterile brine with a certain pH, in which a preservative such as a benzyl chloride alkoxide may or may not be added. For ophthalmic use, the compound can also be prepared into paste such as vaseline paste. When locally applied to the skin, the compound of the present invention can be prepared into an appropriate ointment, lotion or cream preparation form, in which the active ingredient is suspended or dissolved in one or more carriers. Carriers useful for ointment preparations include, but are not limited to, mineral oil, liquid vaseline, white vaseline, propylene glycol, polyethylene oxide, polypropylene oxide, emulsified wax and water. Carriers useful for lotions or creams include, but are not limited to, mineral oil, sorbitan monostearate, Tween 60, cetyl ester wax, hexadecenyl aromatic alcohol, 2- octyldodecanol, benzyl alcohol and water. The compound of the present invention can also be administered in the form of sterile injectable preparations, including sterile injectable aqueous or oily suspensions or sterile injectable solutions. Carriers and solvents that can be used include water, Ringer's solution and isotonic sodium chloride solution. In addition, sterilized non-volatile oil can also be used as a solvent or suspending medium, such as monoglyceride or diglyceride.

In another aspect, the present invention provides use of the compound according to the first aspect or a pharmaceutically acceptable salt, stereoisomer, deuterated compound or solvate thereof or the pharmaceutical composition according to the second aspect in the preparation of a medicine for preventing and/or treating diseases or disorders. The diseases or disorders are KRAS G12D-related diseases or disorders.

As used herein, "KRAS G12D" refers to a mutant form of mammalian KRAS protein, which contains an amino acid substitution of aspartic acid for glycine at amino acid position 12. The distribution of amino acid codon and residue position of human KRAS is based on the amino acid sequence identified by UniProtKB/Swiss-Prot P01116: Variantp.Gly12Asp.

As used herein, "KRAS G12D inhibitor" refers to the compound of the present invention or a pharmaceutically acceptable salt, stereoisomer, deuterated compound or solvate thereof. These compounds can negatively regulate or inhibit all or part of the enzyme activity of KRAS G12D.

As used herein, "KRAS G12D-related diseases or disorders" refer to diseases or disorders related to, mediated by or having KRAS G12D mutations. Non-limiting examples of KRAS G12D-related diseases or disorders are KRAS G12D-related cancers.

Another aspect of the present invention provides a method for treating KRAS G12D-related cancers. The method includes: administering a therapeutically effective amount of the compound according to the first aspect of the present invention or a pharmaceutically acceptable salt, stereoisomer, deuterated compound or solvate thereof to a subject in need thereof; or administering a therapeutically effective amount of the pharmaceutical composition according to the second aspect of the present invention to a subject in need thereof.

Herein, in one embodiment, the KRAS G12D-related cancers include, but are not limited to, lung cancer, prostate cancer, breast cancer, brain cancer, skin cancer, cervical cancer, testicular cancer and other tumors. In one embodiment, the KRAS G12D-related cancers include, but are not limited to, astrocytic cancer, breast cancer, cervical cancer, colorectal cancer, endometrial cancer, esophageal cancer, gastric cancer, head and neck cancer, hepatocellular cancer, laryngeal cancer, lung cancer, oral cancer, ovarian cancer, prostate cancer, thyroid cancer, sarcoma and the like. In one embodiment, the KRAS G12D-related cancers include, but are not limited to, cardiac cancers, such as sarcoma (angiosarcoma, fibrosarcoma, rhabdomyosarcoma, and liposarcoma), myxoma, rhabdomyoma, fibroma, lipoma and teratoma; lung cancers, for example, bronchial cancer (squamous cell, undifferentiated small cell, undifferentiated large cell, and adenocarcinoma), alveolar (bronchial) cancer, bronchial adenoma, sarcoma, lymphoma, chondromatous hamartoma, and mesothelioma; cancers of gastrointestinal tract, for example, esophagus (squamous cell carcinoma, adenocarcinoma, leiomyosarcoma, and lymphoma), stomach (carcinoma, lymphoma, and leiomyosarcoma), pancreas (ductal adenocarcinoma, insulinoma, glucagonoma, gastrinoma, carcinoid tumor, and vipoma), small intestine (adenocarcinoma, and carcinoid tumor), Kaposi's sarcoma, leiomyoma, hemangioma, lipoma, neurofibroma, and fibroma), large intestine (adenocarcinoma, tubular adenoma, villous adenoma, hamartoma, and leiomyoma); cancers of urogenital tract, for example, kidney (adenocarcinoma, Wilms tumor (nephroblastoma), lymphoma, and leukemia), bladder and urethra (squamous cell carcinoma, transitional cell carcinoma, and adenocarcinoma), prostate (adenocarcinoma, and sarcoma), testis (seminoma, teratoma, embryonic carcinoma, and teratoma), choriocarcinoma, sarcoma, interstitial cell carcinoma, fibroma, fibroadenoma, adenomatoid tumor, and lipoma); liver cancers, for example, hepatocellular carcinoma, cholangiocarcinoma, hepatoblastoma, angiosarcoma, hepatocellular adenoma, and hemangioma; cancers of biliary tract, for example, gallbladder cancer, ampullary cancer, and cholangiocarcinoma; bone cancers, for example, osteosarcoma, fibrosarcoma, malignant fibrous histiocytoma, chondrosarcoma, Ewing's sarcoma, malignant lymphoma (reticulocytoma), multiple myeloma, malignant giant cell tumor, chordoma, osteochondroma, benign chondroma, benign osteochondroma and giant cell tumor; cancer of nervous system, for example, skull (osteoma, hemangioma, granuloma, xanthoma, and osteitis deformans), meninges (meningioma, meningosarcoma, and glioma), brain (astrocytoma, medulloblastoma, glioma, ependymoma, germ cell tumor (pineoma), glioblastoma, glioblastoma multiforme, glioblastoma, and congenital tumors), spinal neurofibroma, meningioma, glioma, and sarcoma); gynecological cancers, for example, uterus (endometrial cancer), cervix (cervical cancer, precancerous cervical dysplasia), ovary (ovarian cancer (serous cystadenocarcinoma, mucinous cystadenocarcinoma, and unclassified carcinoma), granulosa-theca cell tumor, Sertoli-Leydig cell tumor, disgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, botryoid sarcoma (embryonic rhabdomyosarcoma), fallopian tube (carcinoma); hematological cancers, for example, blood (acute and chronic myeloid leukemia), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); skin cancers, for example, malignant melanoma, basal cell carcinoma, squamous cell carcinoma, Kaposi sarcoma, dysplastic nevus, lipoma, hemangioma, dermatofibroma, keloid, and psoriasis; and cancers of the adrenal gland, such as neuroblastoma.

In one embodiment, the KRAS G12D-related cancer is lung cancer.

In one embodiment, the KRAS G12D-related cancer is selected from the group consisting of: gastric cancer, gastric adenocarcinoma, non-small cell lung cancer, small cell lung cancer, colorectal cancer, rectal cancer or pancreatic cancer.

Another aspect of the present invention provides a method for treating a cancer in a subject in need thereof. The method includes:
(a) determining that the cancer is associated with KRAS G12D mutation (e.g., a KRAS G12D-related cancer); and
(b) administering to the subject a therapeutically effective amount of the compound according to the first aspect of the present invention or a pharmaceutically acceptable salt, stereoisomer, deuterated compound or solvate thereof; or administering to the subject a therapeutically effective amount of the pharmaceutical composition according to the second aspect of the present invention.

In one embodiment, the administration is performed by a route selected from parenteral, intraperitoneal, intradermal, intracardiac, intraventricular, intracranial, intracerebrospinal, intracavitary, intrasynovial, intrathecal, intramuscular injection, intravitreal injection, intravenous injection, intra-arterial injection, oral, buccal, sublingual, percutaneous, local, intratracheal, rectal, subcutaneous and topical administration.

Another aspect of the present invention provides a method for inhibiting the activity of KRAS G12D in cells. The method includes: contacting the cells with the compound according to the first aspect of the present invention or a pharmaceutically acceptable salt, stereoisomer, deuterated compound or solvate thereof; or contacting the cells with the pharmaceutical composition according to the second aspect of the present invention.

Another aspect of the present invention provides use of the compound according to the first aspect or a pharmaceutically acceptable salt, stereoisomer, deuterated compound or solvate thereof, or the pharmaceutical composition according to the second aspect in the preparation of a KRAS G12D inhibitor.

As used herein, the term "subject" refers to an animal, especially a mammal, preferably, human.

As used herein, the term "effective amount" or "therapeutically effective amount" refers to a sufficient amount of a drug or medicament that is non-toxic but can achieve the desired effect. In an embodiment of the present invention, when the patient is treated according to the present invention, the amount of a given drug depends on many factors, such as the specific dosing regimen, the type and severity of the disease or disorder, and the features (e.g., weight) of the subject or host that needs treatment. According to particular surrounding conditions, including, for example, the specific drug used, the administration route, the treated disorder, and the treated subject or host, the administered dosage can be determined by a method known in the art. Generally, in terms of dosage used for adult treatment, the administered dosage is typically in the range of 0.02-5000 mg/day, for example, about 1-1500 mg/day. The required dosage can be conveniently expressed as one dose, or simultaneously administered (or in a short time) or divided doses at appropriate intervals, for example, two, three, four or more doses per day. It can be understood by those skilled in the art that although the above dosage range is given, the specific effective amount can be appropriately adjusted according to the patient's condition and the doctor's determination.

As used herein, the term "pharmaceutically acceptable salt" refers to a salt of the compound of the present invention that is pharmaceutically acceptable and has the pharmacological activity of the parent compound. Such salts include acid addition salts with inorganic acids such as nitric acid, phosphoric acid, carbonic acid and the like; and organic acids such as propionic acid, caproic acid, cyclopentylpropionic acid, glycolic acid, pyruvic acid, gluconic acid, stearic acid, and muconic acid, etc; or salts formed when an acidic proton existing on the parent compound is replaced by a metal ion, such as an alkali metal ion or alkaline earth metal ion; or a coordination compound formed with an organic base such as ethanolamine. The pharmaceutically acceptable salt of the present invention can be synthesized by a conventional chemical method from the parent compound containing an acid or base radical. Generally, such a salt is prepared by reacting the compound in the form of a free acid or base with a stoichiometric amount of an appropriate base or acid in water or an organic solvent or a mixture of the two. Generally, non-aqueous media such as ether, ethyl acetate, ethanol, isopropanol or acetonitrile are preferred. In addition to the salt form, the compound provided in the present invention can also exist in a prodrug form. The prodrug of the compound described herein can easily undergo chemical changes under physiological conditions, thus being converted into the compound of the present invention. In addition, the prodrug can be chemically or biologically converted to the compound of the present invention in vivo.

As used herein, the term "solvate" refers to a substance formed by association of the compound of the present invention with a pharmaceutically acceptable solvent. The pharmaceutically acceptable solvent includes acetic acid and others. The solvate includes stoichiometric and non-stoichiometric solvates. Some compounds of the present invention can exist in a non-solvated form or a solvated form. Generally, the solvated form and the non-solvated form are equivalent and are both included in the scope of the present invention.

As used herein, the term "stereoisomer" includes conformational isomers and configurational isomers, where the configurational isomers mainly include cis-trans isomers and optical isomers. The compound of the present invention can exist in the form of a stereoisomer, and thus covers all possible stereoisomer forms, including, but not limited to, cis-trans isomers, tautomers, enantiomers, diastereomers, and atropisomers, etc. The compound of the present invention can also exist as any combination or any mixture of the aforementioned stereoisomers, for example, a mixture of equal amount of the mesomer, raceme, atropisomer, and others. For example, a single enantiomer, a single diastereomer or a mixture thereof, or a single atropisomer or a mixture thereof. When the compound of the present invention contains an olefin double bond, unless otherwise specified, it includes a cis isomer, a trans isomer, and any combination thereof. The atropisomer of the present invention is a stereoisomer with axial or planar chirality due to limited intramolecular rotation. As a drug, stereoisomers with excellent activity are preferred. The compound of the present invention has optical isomers derived from an asymmetric carbon, etc. If necessary, a single isomer can be obtained by resolution by methods known in the art, such as crystallization or chiral chromatography.

As used herein, the term "deuterated compound" refers to various deuterated forms of any compound disclosed in the present invention. Each available hydrogen atom attached to a carbon atom can be independently replaced by a deuterium atom. One of ordinary skill in the art knows how to synthesize deuterated compounds of any formula disclosed herein. For example, a deuterated material such as an alkyl group can be prepared by conventional techniques (see, for example, methyl-d3-amine, available from Aldrich Chemical Co.,Milwaukee,WI, Catalogue 489,689-2).

Herein, 2 hydrogen atoms on the same carbon atom being optionally both replaced by =CR^{2a}R^{2b} means that the carbon atom is attached to a carbon atom (C) in CR^{2a}R^{2b} through a double bond. That is, 2 hydrogen atoms on the same carbon atom being optionally both replaced by =CR^{2a}R^{2b} means that the carbon atom is substituted with

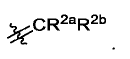

As used herein, when a group such as an alkyl group is located in the middle of a structural formula, the group is a ylene group. for example, the alkyl is an alkylene.

As used herein, the term "alkyl" refers to a chain-like (linear or branched) saturated aliphatic hydrocarbyl group. The term "alkyl" can be a linear or branched alkyl group (C1-20 alkyl group) containing 1 to 20 carbon atoms, preferably an alkyl group (C1-12 alkyl group) containing 1 to 12 carbon atoms. Non-limiting examples include methyl, ethyl, n-propyl, isopropyl, n-butyl, isobutyl, t-butyl, s-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. Further preferred is a lower alkyl (C1-6 alkyl) containing 1 to 6 carbon atoms. Non-limiting examples include methyl, ethyl, n-propyl, isopropyl, n-butyl, isobutyl, t-butyl, s-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl. Further preferred is a lower alkyl (C1-3 alkyl) containing 1 to 3 carbon atoms. Non-limiting examples include methyl, ethyl, n-propyl, and isopropyl.

As used herein, the term "C1-C6 alkyl" refers to a linear and branched aliphatic groups having 1-6 carbon atoms, preferably C1-C4 alkyl or C1-C3 alkyl. Examples of alkyl include, but are not limited to, methyl, ethyl, propyl, isopropyl, butyl, isobutyl, s-butyl, t-butyl, pentyl and hexyl.

As used herein, the term "C1-C6 haloalkyl" means that one or more hydrogen in C1-C6 alkyl is replaced by halogen, where the alkyl moiety is as defined above. C1-C3 haloalkyl or C1-C4 haloalkyl is preferred. Examples of haloalkyl include, but are not limited to, trifluoromethyl, difluoromethyl, and monofluoromethyl.

As used herein, the term "C1-C6 deuterated alkyl" means that one or more hydrogen in C1-C6 alkyl is replaced by deuterium atom, where the alkyl moiety is as defined above. C1-C3 deuterated alkyl or C1-C4 deuterated alkyl is preferred. Examples of haloalkyl include, but are not limited to, trifluoromethyl, difluoromethyl, and monofluoromethyl.

As used herein, the term "C1-C6 alkoxy" refers to -O-C1-C6 alkyl, preferably, C1-C3 alkoxy or C1-C4 alkoxy, where the alkyl moiety is as defined above.

As used herein, the term "C1-C6 haloalkoxy" refers to -O-C1-C6 haloalkyl, preferably C1-C3 haloalkoxy or C1-C4 haloalkoxy, where the haloalkyl moiety is as defined above.

As used herein, the term "C1-C6 deuterated alkoxy" refers to -O-C1-C6 deuterated alkyl, preferably C1-C3 deuterated alkoxy or C1-C4 deuterated alkoxy, where the deuterated alkyl moiety is as defined above.

As used herein, the term "C1-C6 hydroxylalkyl" refers to -C1-C6 alkyl-hydroxyl(OH), preferably C1-C3 hydroxylalkyl (that is, -C1-C3 alkyl-hydroxyl) or C1-C4 hydroxylalkyl (that is, -C1-C4 alkyl-hydroxyl), where the alkyl moiety is as defined above.

As used herein, the term "C1-C6 cyanoalkyl" refes to -C1-C6 alkyl-cyano, preferably C1-C3 cyanoalkyl or C1-C4 cyanoalkyl, where the alkyl moiety is as defined above.

As used herein, the term "C2-C6 alkynyl" refers to a linear and branched aliphatic group having 2-6 carbon atoms and 1 or 2 carbon-carbon triple bond, preferably C2-C4 alkynyl or C2-C3 alkynyl. Examples of alkynyl include, but are not limited to, ethynyl, propynyl, and butynyl.

As used herein, the term "C2-C6 haloalkynyl" means that one or more hydrogen in C2-C6 alkynyl is replaced by halogen, where the alkynyl moiety is as defined above. C2-C3 haloalkynyl or C2-C4 haloalkynyl is preferred. Examples of haloalkynyl include, but are not limited to, monofluoroethynyl and difluoroethynyl.

As used herein, the term "C2-C6 alkenyl" refers to a linear and branched aliphatic group having 2-6 carbon atoms and 1 or 2 carbon-carbon double bond, preferably C2-C4 alkenyl or C2-C3 alkenyl. Examples of alkenyl include, but are not limited to, ethenyl, propenyl, and butenyl.

As used herein, the term "C2-C6 haloalkenyl" means that one or more hydrogen in C2-C6 alkenyl is replaced by halogen, where the alkenyl moiety is as defined above. C2-C3 haloalkenyl or C2-C4 haloalkenyl is preferred. Examples of haloalkenyl include, but are not limited to, monofluoroethenyl, and difluoroethenyl.

As used herein, the term "C2-C6 hydroxyalkynyl" refers to -C2-C6 alkynyl-OH, where the alkynyl is as defined above. C2-C4 hydroxyalkynyl or C2-C3 hydroxyalkynyl is preferred.

As used herein, the term "C2-C6 hydroxyalkenyl" refres to -C2-C6 alkenyl-OH, where the alkenyl is as defined above. C2-C4hydroxyalkenyl or C2-C3hydroxyalkenyl is preferred.

As used herein, the term "C1-C4 alkylene" refers to a C1-C4 alkyl as defined above, which is located between the other two parts of the molecule and is used to connect them. Typical alkylene include, but are not limited to, methylene, ethylene, propylene and butylene.

In the present invention, the above-mentioned alkyl, alkenyl and alkynyl can be optionally substituted, and when substituted, the substituent group is preferably one or more substituent groups described in the present application.

As used herein, the term "cycloalkyl" and "cycloalkyl ring" are used interchangeably and refer to a saturated monocyclic or polycyclic hydrocarbyl group, for example, monocyclic cycloalkyl, spiro cycloalkyl, fused cycloalkyl, and bridged cycloalkyl. In the present invention, the ring carbon atoms of the cycloalkyl can be substituted with 1,2 or 3 oxo groups to form a cyclic ketone structure. For example, the term "C3-C20 cycloalkyl" refers to cycloalkyl with 3 to 20 ring carbon atoms, including monocyclic cycloalkyl, spiro cycloalkyl, fused cycloalkyl, and bridged cycloalkyl. C3-C12 cycloalkyl (further preferably C3-C8 monocyclic cycloalkyl), C5-C20 spiro cycloalkyl, C5-C20 fused cycloalkyl or C5-C20 bridged cycloalkyl is preferred.

The term "C3-C8 monocyclic cycloalkyl" refers to a saturated monocyclic hydrocarbyl with 3 to 8 ring carbon atoms. C3-C6 monocyclic cycloalkyl or C4-C6 monocyclic cycloalkyl is preferred. C3, C4, C5 or C6 monocyclic cycloalkyl is further preferred. Specific examples of monocyclic cycloalkyl include, but are not limited to, cyclopropyl, cyclobutyl, cyclopentyl, cyclohexyl, cycloheptyl, and cyclooctyl.

As used herein, the term "spiro cycloalkyl" and "spiro cycloalkyl ring" refers to a polycyclic hydrocarbyl group formed by sharing a carbon atom (spiro atom) by two or more monocyclic rings. Based on the number of shared spiro atoms between the rings, spiro heterocyclyl groups include mono-, di- or poly-spiroheterocyclyl group. The term "C5-C20 spirocycloalkyl" refers to a polycyclic hydrocarbyl group with 5 to 20 ring carbon atoms, where the monocyclic rings sharing a spiro atom are a C3-C8 monocyclic cycloalkyl ring. C6-C14 spiro cycloalkyl is preferred. C6-C14 monospiro cycloalkyl is further preferred. C7-C11spiro cycloalkyl is further preferred. 7- to 11-membered monospiro cycloalkyl is further preferred. Most preferred is C7 (C4 monocyclic cycloalkyl ring/C4 monocyclic cycloalkyl ring), C8(C4 monocyclic cycloalkyl ring/C5 monocyclic cycloalkyl ring), C9(C4 monocyclic cycloalkyl ring/C6 monocyclic cycloalkyl ring, C5 monocyclic cycloalkyl ring/C5 monocyclic cycloalkyl ring), C10(C5 monocyclic cycloalkyl ring/C6 monocyclic cycloalkyl ring) or C11(C6 monocyclic cycloalkyl ring/C6 monocyclic cycloalkyl ring) monospiro cycloalkyl. Specific examples of spiro cycloalkyl include, but are not limited to:

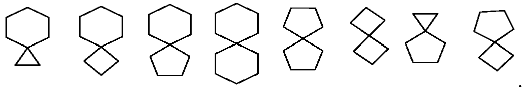

These spiro cycloalkyl groups can be attached to the rest of the molecule through any ring atom.

As used herein, the term "fused cycloalkyl" and "fused cycloalkyl ring" refer to a polycyclic hydrocarbyl group formed by sharing a pair of adjacent carbon atoms by two or more monocyclic rings. According to the number of rings contained, it can be divided into bicyclic, tricyclic, tetracyclic or polycyclic fused cycloalkyl. The term "C5-C20 fused cycloalkyl" refers to a polycyclic hydrocarbyl group with 5 to 20 ring carbon atoms, where the monocyclic rings sharing a pair of adjacent carbon atom are a C3-C8 monocyclic cycloalkyl ring. C6-C14 fused cycloalkyl is preferred. C6-C14 bifused cycloalkyl is further preferred. C7-C10 fused cycloalkyl is further preferred. C7-C10 bifused cycloalkyl is further preferred. Most preferred is C8 (C5 monocyclic cycloalkyl ring fused with C5 monocyclic cycloalkyl ring), C9 (C5 monocyclic cycloalkyl ring fused with C6 monocyclic cycloalkyl ring) or C10 (C6 monocyclic cycloalkyl ring fused with C6 monocyclic cycloalkyl ring) bicyclic fused cycloalkyl. Specific examples of fused cycloalkyl include, but are not limited to:

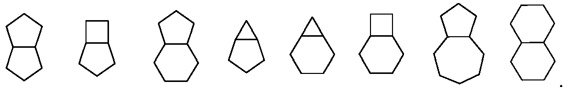

These fused cycloalkyl groups can be attached to the rest of the molecule through any ring atom.

As used herein, the term "bridged cycloalkyl" and "bridged cycloalkyl ring" refer to a polycyclic hydrocarbyl group formed by sharing two carbon atoms that are not directly connected by two or more monocyclic rings. According to the number of rings contained, it can be divided into bicyclic, tricyclic, tetracyclic or polycyclic bridged cycloalkyl. The term "C5-C20 bridged cycloalkyl" refers to a polycyclic hydrocarbyl with 5 to 20 ring carbon atoms, in which any two rings share two carbon atoms that are not directly connected. C6-C14 bridged cycloalkyl is preferred. C7-C10 bridged cycloalkyl is further preferred. Specific examples of bridged cycloalkyl include, but are not limited to:

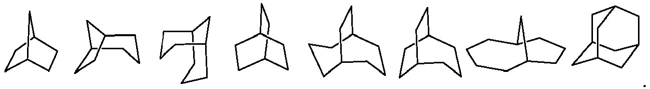

These bridged cycloalkyl groups can be attached to the rest of the molecule through any ring atom.

For example, examples of cycloalkyl herein include, but are not limited to, cyclopropyl, cyclobutyl, cyclopentyl, cyclopentenyl, cyclohexyl, cyclohexenyl, cycloheptyl, cyclooctyl, bicyclo[1.1.1]pentyl.

As used herein, the term "cycloalkoxy" refers to -O- cycloalkyl, in which the cycloalkyl is as defined above.

In the present invention, the above-mentioned cycloalkyl can be optionally substituted, and when substituted, the substituent group is preferably one or more substituent groups described in the present application.

As used herein, the term "heterocyclyl" and "heterocyclyl ring" are used interchangeably and refer to a saturated or partially unsaturated monocyclic or polycyclic hydrocarbyl, for example, monocyclic heterocyclyl, spiroheterocyclyl, fused heterocyclyl, and bridged heterocyclyl. In the present invention, the ring carbon atom of the heterocyclyl can be substituted with 1, 2 or 3 oxo groups to form a cyclic ketone, cyclic lactone or cyclic lactam structure. For example, , the term "3- to 20-membered heterocyclyl" refers to a saturated or partially unsaturated monocyclic or polycyclic hydrocarbyl group with 3 to 20 ring atoms, in which one or more (preferred is 1,2, 3 or 4) ring atoms is selected from heteroatoms such as nitrogen, oxygen, or S(=O)ₘ' (where m' is an integer from 0 to 2), but excluding the ring moiety of -O-O-, -O-S- or -S-S-; and the rest ring atoms are carbon. When the ring atom is a nitrogen atom, it may be substituted or unsubstituted (i.e., N or NR, in which R is hydrogen or other substituents as defined herein). In the present invention, the 3- to 20-membered heterocyclyl includes monocyclic heterocyclyl (for example, 3- to 8-membered monocyclic heterocyclyl), 5- to 20-membered spiro heterocyclyl, 5- to 20-membered fused heterocyclyl, and 5- to 20-membered bridged heterocyclyl.

As used herein, the term "3- to 20-membered nitrogen containing heterocyclyl" refers to a saturated or partially unsaturated monocyclic hydrocarbyl with 3 to 20 ring atoms containing at least 1 nitrogen atom. The group is attached to the rest of the molecule through the nitrogen atom. The group also optionally contains additional 1 or 2 heteroatoms selected from nitrogen, oxygen or S(=O)ₘ' (where m' is an integer from 0 to 2) as a ring atom. In the present invention, the 3- to 20-membered nitrogen containing heterocyclyl includes a monocyclic heterocyclyl (for example, 3- to 8-membered monocyclic nitrogen containing heterocyclyl), 5- to 20-membered nitrogen containing spiroheterocyclyl, 5- to 20-membered nitrogen containing fused heterocyclyl, and 5- to 20-membered nitrogen containing bridged heterocyclyl.

As used herein, the term "3- to 8-membered monocyclic heterocyclyl" and "3- to 8-membered monocyclic heterocyclyl ring" refers to a saturated or partially unsaturated monocyclic hydrocarbyl group with 3 to 8 ring atoms, in which 1, 2 or 3 ring atoms are heteroatoms selected from nitrogen, oxygen or S(=O)ₘ' (where m' is an integer from 0 to 2). The group is preferably a 3- to 6-membered monocyclic heterocyclyl having 3 to 6 ring atoms, in which 1 or 2 ring atoms are heteroatoms. The group is further preferably a 4- to 6-membered monocyclic heterocyclyl having 4 to 6 ring atoms, in which 1 or 2 ring atoms are heteroatoms. The group is preferably a 5- or 6-membered monocyclic heterocyclyl having 5 or 6 ring atoms, in which 1 or 2 ring atoms are heteroatoms. When the heteroatom is a nitrogen atom, the nitrogen atom may be substituted or unsubstituted (i.e., N or NR, in which R is hydrogen or other substituents as defined herein). When the heteroatom is a sulfur atom, the sulfur atom may be optionally oxidized (that is, S(=O)ₘ', in which m' is an integer from 0 to 2). The ring carbon atom of the monocyclic heterocyclyl may be optionally substituted with 1, 2 or 3 oxo groups to form a cycloketone, cyclolactone or cyclolactam structure. Specific examples of monocyclic heterocyclyl include, but are not limited to, aziridine, ethylene oxide, azetidine, azetidin-2-one, oxetane, oxetan-2-one, oxazolidine, pyrrolidin-2-one, pyrrolidin-2,5-dione, 1,3-dioxolanyl, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrole, 1,3-dioxolanyl-2-one, oxazolidine-2-one, imidazolidine-2-one, piperidine, piperazine, piperazine-2-one, morpholine, morpholine-3-one, morpholine-2-one, thiomorpholine-3-one 1,1-dioxide, thiomorpholine, thiomorpholine-1,1-dioxide, tetrahydropyran, 1,2-dihydroazacyclobutadiene, 1,2-dihydrooxacyclobutadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidine-2(1H)-one, 1,4-dioxane-2-one, 5,6-dihydro-2H-pyran-2-one, 5,6-dihydropyrimidine-4(3H)-one, 3,4-dihydropyridine-2(1H)-one, 5,6-dihydropyridine-2(1H)-one, 5,6-dihydropyrimidine-4(1H)-one, pyrimidine-4(3H)-one, pyrimidine-4(1H)-one, 4,5-dihydro-1H-imidazole, 2,3-dihydro-1H-imidazole, 2,3-dihydrooxazole, 1,3-dioxacyclopentene, 2,3-dihydrothiophene, 2,5-dihydrothiophene, 3,4-dihydro-2H-1,4-oxazine, 3,4-dihydro-2H-1,4-thiazinel,1-dioxide, 1,2,3,4-tetrahydropyrazine, 1,3-dihydro-2H-pyrrole-2-one, 1,5-dihydro-2H-pyrrole-2-one, 1H-pyrrole-2,5-dione, furan-2(3H)-one, furan-2(5H)-one, 1,3-dioxacyclopenten-2-one, oxazole-2(3H)-one, 1,3-dihydro-2H-imidazole-2-one, furan-2,5-dione, 3,6-dihydropyridine-2(1H)-one, pyridine-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, and 1,2,3,4-tetrahydropyrimidine.

As used herein, the term "3- to 6-membered nitrogen containing heterocyclyl" refers to a saturated or partially unsaturated monocyclic hydrocarbyl with 3 to 6 ring atoms containing at least 1 nitrogen atom as a ring atom. The group is attached to the rest of the molecule through the nitrogen atom, or attached to the rest of the molecule through other ring atoms of the group. The group also optionally contains additional 1 or 2 heteroatoms selected from nitrogen, oxygen or S(=O)ₘ' (where m' is an integer from 0 to 2) as a ring atom. Specific examples can be selected from a 3- to 8-member monocyclic heterocyclyl, including, but not limited to, tetrahydropyrrolyl, oxazolidinyl, isooxazolidinyl, piperidinyl, piperazinyl, morpholinyl, thiomorpholine, azetidinyl, and the like.

As used herein, the term "spiroheterocyclyl" and "spiroheterocyclyl ring" refers to a polycyclic heterocyclyl formed by sharing a carbon atom (spiro atom) by two or more saturated or partially unsaturated monocyclic rings, in which one or more (for example, 1, 2 or 3) ring atoms are heteroatoms selected from nitrogen, oxygen, or S(=O)ₘ' (where m' is an integer from 0 to 2), and the rest ring atoms are carbon. When the heteroatom is a nitrogen atom, the nitrogen atom may be substituted or unsubstituted (i.e., N or NR, in which R is hydrogen or other substituents as defined herein). Each monocyclic ring can contain one or more double bonds, but none of them has a fully conjugated π electron system. According to the number of spiro atoms shared between the rings, the spiroheterocyclyl groups are classified into monospiroheterocycyl, dispiroheterocycyl or polyspiroheterocycyl groups. The term "5- to 20-membered spiro heterocyclyl" refers to a spiro heterocyclyl having 5 to 20 ring atoms, in which one of the monocyclic rings sharing a spiro atom is a 3- to 8-membered monocyclic heterocyclyl ring, and the other monocyclic ring is a 3- to 8-membered monocyclic heterocyclyl ring or 3- to 8-membered monocyclic cycloalkyl ring. The group is preferably a 6- to 14-membered spiro heterocyclyl having 6 to 14 ring atoms, in which 1 or 2 ring atoms are heteroatoms. The group is further preferably a 7- to 11-membered spiro heterocyclyl having 7 to 11 ring atoms, in which 1 or 2 ring atoms are heteroatoms. Most preferred is a 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring) or 11-membered (6-membered monocyclic heterocyclyl ring/6-membered monocyclic heterocyclyl ring) monospiro heterocyclyl. Specific examples of spiro heterocyclyl include, but are not limited to: 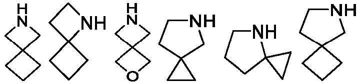 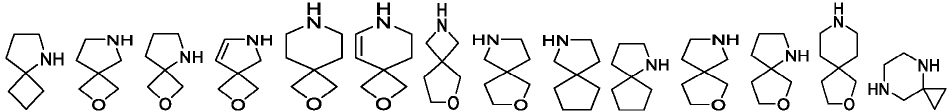 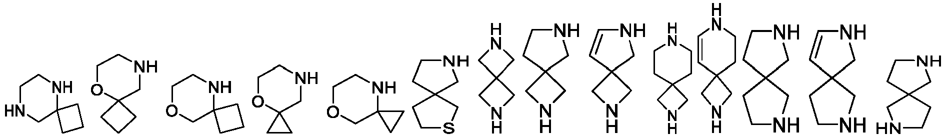 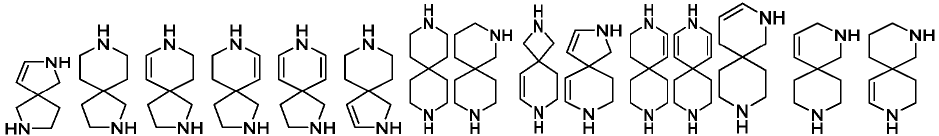 and

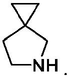

These spiro heterocyclyl can be attached to the rest of the molecule through any suitable ring atom.

As used herein, the term "5- to 20-membered nitrogen containing spiro heterocyclyl" refers to a spiro heterocyclyl with 5 to 20 ring atoms containing at least 1 nitrogen atom. The group is attached to the rest of the molecule through the nitrogen atom; and the group optionally contains additional 1 or 2 or 3 heteroatoms selected from nitrogen, oxygen, or S(=O)ₘ' (where m' is an integer from 0 to 2) as a ring atom.

As used herein, the term "fused heterocyclyl" and "fused heterocyclyl ring" a polycyclic heterocyclyl formed by sharing a pair of adjacent ring atoms by two or more saturated or partially unsaturated monocyclic rings, in which one or more (for example, 1, 2 or 3) ring atoms are heteroatoms selected from selected from nitrogen, oxygen or S(=O)ₘ' (where m' is an integer from 0 to 2), and the rest ring atoms are carbon. When the heteroatom is a nitrogen atom, the nitrogen atom may be substituted or unsubstituted (i.e., N or NR, in which R is hydrogen or other substituents as defined herein). Each monocyclic ring can contain one or more double bonds, but none of them has a fully conjugated π electron system. The shared pair of adjacent ring atoms can be C-C or N-C. According to the number of rings contained, it can be divided into bicyclic, tricyclic, tetracyclic or polycyclic fused heterocyclyl. The term "5- to 20-membered fused heterocyclyl" a fused heterocyclyl having 5 to 20 ring atoms, where the monocyclic ring sharing a pair of adjacent ring atoms is a 3- to 8-membered monocyclic heterocyclyl ring. The group is preferably a 6- to 14-membered fused heterocyclyl having 6 to 14 ring atoms, in which 1 or 2 ring atoms are heteroatoms. The group is further preferably a 6- to 10-membered, 6- to 9-membered, or 6- to 8-membered fused heterocyclyl having 6 to 10, 6 to 9, or 6 to 8 ring atoms, in which 1 or 2 ring atoms are heteroatoms. The group is further preferably a 8- to 10-membered fused heterocyclyl having 8 to 10 ring atoms, in which 1 or 2 ring atoms are heteroatoms. Most preferred is a 8-membered (5-membered monocyclic heterocyclyl ring fused with 5-membered monocyclic heterocyclyl ring), 9-membered (5-membered monocyclic heterocyclyl ring fused with 6-membered monocyclic heterocyclyl ring) or 10-membered (6-membered monocyclic heterocyclyl ring fused with 6-membered monocyclic heterocyclyl ring) bicyclic fused heterocyclyl. Specific examples of fused heterocyclyl include, but are not limited to:

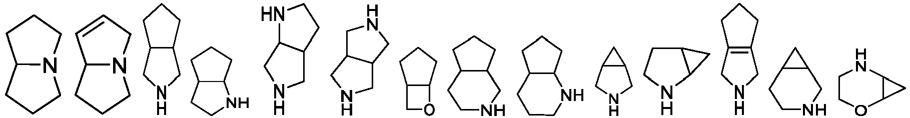

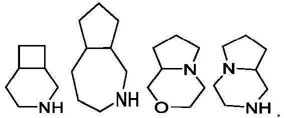

These fused heterocyclyl can be attached to the rest of the molecule through any suitable ring atom.

As used herein, the term "5- to 20-membered nitrogen containing fused heterocyclyl" refers to a fused heterocyclyl with 5 to 20 ring atoms containing at least 1 nitrogen atom. The group is attached to the rest of the molecule through the nitrogen atom; and the group optionally contains additional 1 or 2 or 3 heteroatoms selected from nitrogen, oxygen, or S(=O)ₘ' (where m' is an integer from 0 to 2) as a ring atom.

As used herein, the term "bridged heterocyclyl" and "bridged heterocyclyl ring" refers to a polycyclic heterocyclyl formed by sharing two ring atoms that are not directly connected by two or more saturated or partially unsaturated monocyclic rings, in which one or more (for example, 1, 2 or 3) ring atoms are heteroatoms selected from nitrogen, oxygen, or S(=O)ₘ' (where m' is an integer from 0 to 2), and the rest ring atoms are carbon. According to the number of rings contained, it can be divided into bicyclic, tricyclic, tetracyclic or polycyclic bridged cycloalkyl. The term "5- to 20-membered bridged heterocyclyl" refers to a saturated or partially unsaturated polycyclic heterocyclyl having 5 to 20 ring atoms, in which any two rings share two ring atoms that are not directly connected, and each monocyclic ring may contain one or more double bonds, but none of the rings has a fully conjugated π electron system. A 6- to 14-membered bridged heterocyclyl is preferred. A 7- to 10-membered bridged heterocyclyl is further preferred. Specific examples of bridged heterocyclyl include, but are not limited to:

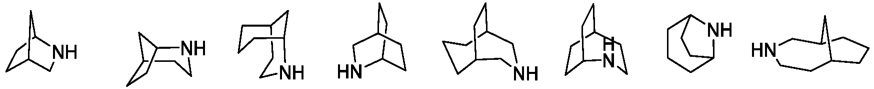

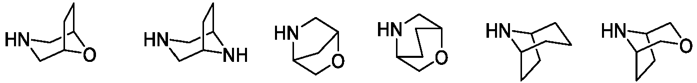

These bridged heterocyclyl groups can be attached to the rest of the molecule through any suitable ring atom.

As used herein, the term "5- to 20-membered nitrogen containing bridged heterocyclyl" refers to a bridged heterocyclyl with 5 to 20 ring atoms containing at least 1 nitrogen atom. The group is attached to the rest of the molecule through the nitrogen atom; and the group optionally contains additional 1 or 2 or 3 heteroatoms selected from nitrogen, oxygen, or S(=O)ₘ' (where m' is an integer from 0 to 2) as a ring atom.

In the present invention, the above-mentioned heterocyclyl can be optionally substituted, and when substituted, the substituent group is preferably one or more substituent groups described in the present application.

Specifically, in the present invention, specific examples of the heterocyclyl include, but are not limited to, epoxy, azetidinyl, tetrahydrofuranyl, tetrahydropyranyl, pyrrolidinyl, pyrrolidinonyl, piperidinyl, piperazinyl, imidazolyl, imidazopyridinyl, thiazolyl, oxazolidinyl, oxazolidinonyl, decahydrogenquinolinyl, piperidinonyl, morpholinyl, azabicyclohexyl, azabicycloheptyl, azabicyclooctyl, azabicyclononyl, azabicyclodecyl, azaspiroheptyl, azaspirooctyl, azaspirononyl, azaspirodecyl, tetrahydrospiro[cyclopropane-1,2'-pyrrolizin]yl, hexahydro-1H-pyrrolizinyl, hexahydro-1H-pyrrolo[2,1-c][1,4]oxazinyl, octahydroindolizinyl, oxaspiroheptyl, oxaspirooctyl, oxaspirononyl, oxaspirodecyl, diazaspirononyl, oxabicyclohexyl, oxabicycloheptyl, oxabicyclooctyl, and hexahydroalloxazin4(1H)-oxide.

As used herein, the term "aryl" refers to an all-carbon monocyclic, all-carbon non-fused polycyclic (the rings are connected by a covalent bond, and not fused) or all-carbon fused polycyclic (i.e., rings sharing an adjacent pair of carbon atoms) group, in which at least one ring is aromatic, i.e., has a conjugated π electron system. For example, the term "C6-C14 aryl" refers to an aryl having 6 to 14 ring atoms. C6-C10 aryl is preferred. In the present invention, the C6-C14 aryl includes monocyclic aryl, non-fused polycyclic aryl, and aromatic fused polycyclic aryl. Examples of monocyclic aryl include phenyl, and examples of non-fused polycyclic aryl include biphenylyl.

In the present invention, when C6-C14 aryl is an aromatic fused polycyclic aryl, the aromatic fused polycyclic aryl may be a polycyclic group formed by fusing a monoaryl ring with one or more monaryl rings, and non-limiting examples thereof include naphthyl, anthracenyl and the like.

In some embodiments of the present invention, when the C6-C14 aryl is an aromatic fused polycyclic ring, the aromatic fused polycyclic ring may also be a polycyclic group formed by fusing a monoaryl ring (such as phenyl) with one or more nonaromatic rings, where the ring connected to the parent structure is an aromatic ring or nonaromatic ring. The nonaromatic ring includes, but is not limited to, a 3- to 6-membered monocyclic heterocyclyl ring (preferably, a 5- or 6-membered monocyclic heterocyclyl ring, where the ring carbon atom in the monocyclic heterocyclyl ring can be substituted with 1 to 2 oxo groups to form a cyclolactam or cyclolactone structure), a 3- to 6-membered monocyclic cycloalkyl ring (preferably a 5- or 6-membered monocyclic cycloalkyl ring, where the ring carbon atom in the monocyclic cycloalkyl ring can be substituted with 1 or 2 oxo groups to form a cycloketone structure). The polycyclic group in which the monoaryl ring is fused with one or more nonaromatic rings can be connected to other groups or the parent structure through the nitrogen atom or carbon atom, and the ring connected to the parent structure is a monoaryl ring or a nonaromatic ring.

In the present invention, the above-mentioned aryl can be substituted or unsubstituted, and when substituted, the substituent group is preferably one or more substituent groups described in the present application.

As used herein, the term "aryl" refers to a C6-C14 aromatic group having 1 to 3 aromatic rings. It can be optionally substituted with one or more (e.g. 1, 2, 3, 4 or 5) R⁶ or one or more (for example, 1, 2, 3, 4 or 5) R⁷. In an example, the aryl is C6-C10 aryl. Examples of the aryl include, but are not limited to, phenyl, naphthalenyl, anthracenyl, fluorenyl, and dihydrobenzofuranyl. The "aryl" also refers to a bicyclic or tricyclic system, where one or two rings in the aryl group may be saturated or partially saturated, respectively. If the aryl group contains two saturated rings, the two saturated rings may form a fused ring system or a spiro ring system. Examples of the aryl containing two saturated rings (and a spiro ring system) include

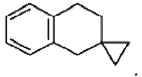

As used herein, the term "aryl-C1-C6 alkyl" or "aralkyl" is meant to include an aryl group covalently attached to an alkyl group. The aryl is as defined above, the alkyl is as defined above, and either or both of the aryl and alkyl can be independently optionally substituted or unsubstituted. An example of the aralkyl is (C6-C10) aryl (C1-C6) alkyl-. Specific examples include, but are not limited to, benzyl, phenylethyl and naphthylmethyl. An example of a substituted aryl-C1-C6 alkyl is one in which the alkyl is substituted by hydroxyalkyl.

As used herein, the term "heteroaryl" refers to a monocyclic or fused polycyclic (i.e., sharing an adjacent pair of ring atoms, which can be C-C or N-C) group in which the ring atom is replaced by at least one heteroatom independently selected from nitrogen, oxygen or sulfur, where the nitrogen and sulfur atom can be optionally oxidized, and the nitrogen atom can be optionally quaternized. The heteroaryl has shared 6, 10 or 14 π electrons, and at least one ring in the group is aromatic. For example, the term "5- to 14-membered heteroaryl" refers to a heteroaryl having 5 to 14 ring atoms, in which 1, 2, 3 or 4 ring atoms are heteroatoms selected from nitrogen, oxygen or S(=O)ₘ' (where m' is an integer from 0 to 2). The group is preferably a 5- to 10-membered heteroaryl having 5 to 10 ring atoms, in which 1, 2, 3 or 4 ring atoms are heteroatoms. In the present invention, the 5- to 14-membered heteroaryl may be a monocyclic heteroaryl (for example, a 5- or 6-membered monocyclic heteroaryl), a fused bicyclic heteroaryl (for example, a 8- to 10-membered bicyclic heteroaryl) or a fused tricyclic heteroaryl.

As used herein, the term "5- or 6-membered monocyclic heteroaryl" refers to a monocyclic heteroaryl having 5 or 6 ring atoms, where 1, 2 or 3 ring atoms are heteroatoms selected from nitrogen, oxygen or S(=O)ₘ' (where m' is an integer from 0 to 2). Specific examples of the monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isooxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, and pyrazine.

As used herein, the term "8 to 10-membered bicyclic heteroaryl" refers to a fused bicyclic heteroaryl having 8 to 10 ring atoms, in which 1, 2, 3, 4 or 5 ring atoms are heteroatoms selected from nitrogen, oxygen or S(=O)ₘ' (where m' is an integer from 0 to 2). The fused bicyclic heteroaryl can be a bicyclic group (preferably a 9 or 10-membered bicyclic heteroaryl ring) formed by fusing a monoaryl ring (such as phenyl) with a monocyclic heteroaryl ring (preferably a 5- or 6-membered monocyclic heteroaryl ring), or a bicyclic group formed by fusing a monocyclic heteroaryl ring (preferably a 5- or 6-membered monocyclic heteroaryl ring) with a monocyclic heteroaryl ring (preferably 5- or 6-membered monocyclic heteroaryl ring).

Any 2 ring atoms connected on the monocyclic heteroaryl ring, including C-C, N-C and N-N, can be fused with a cycloalkyl, heterocyclyl, aryl or heteroaryl such as a monocyclic cycloalkyl ring, a monocyclic heterocyclyl ring, a monoaryl ring, and a 5- or 6-membered monocyclic heteroaryl ring as defined herein, to form a fused polycyclic ring. The 2 ring atoms connected on the monocyclic heteroaryl ring that form a fused ring with other rings are preferably C-C, including, without limitation, 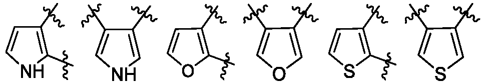 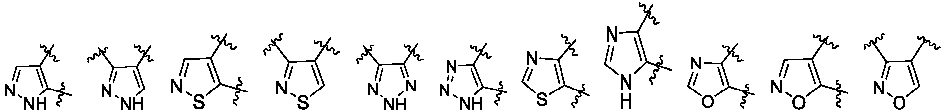 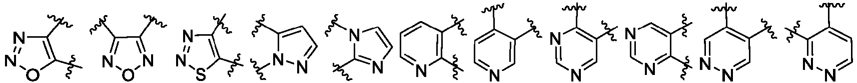 and

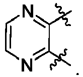

The ring atom marked with " 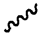 " in the above groups is attached to the rest of the molecule.

Non-limiting examples of the 8- to 10-membered bicyclic heteroaryl include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a] pyrimidine, and imidazo[1,2-b]pyridazine.

Specific examples of bicyclic heteroaryl include, but are not limited to: 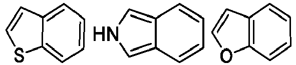 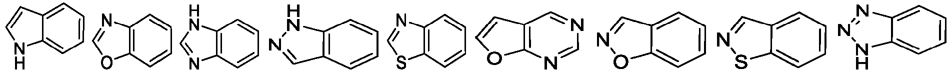 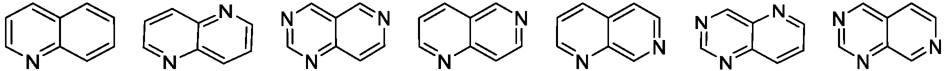 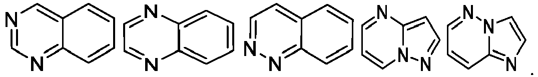 These groups can be attached to the rest of the molecule through any suitable ring atom. The ring connected to the parent structure can be a monocyclic heteroaryl ring or a benzene ring.

In some embodiments of the present invention, the fused bicyclic heteroaryl or fused tricyclic cycloheteroaryl may be a polycyclic group formed by fusing a monocyclic heteroaryl ring (preferably a 5- or 6-membered monocyclic heteroaryl ring) with one or more nonaromatic rings, where the ring connected to the parent structure is a monocyclic heteroaryl ring or nonaromatic ring. The nonaromatic ring includes, but is not limited to, a 3- to 6-membered monocyclic heterocyclyl ring (preferably, a 5- or 6-membered monocyclic heterocyclyl ring, where the ring carbon atom in the monocyclic heterocyclyl ring can be substituted with 1 to 2 oxo groups to form a cyclolactam or cyclolactone structure), a 3- to 6-membered monocyclic cycloalkyl ring (preferably a 5- or 6-membered monocyclic cycloalkyl ring, where the ring carbon atom in the monocyclic cycloalkyl ring can be substituted with 1 or 2 oxo groups to form a cycloketone structure). The polycyclic group formed by fusing the monocyclic heteroaryl ring is fused with one or more nonaromatic rings can be connected to other groups or the parent structure through the nitrogen atom or carbon atom, and the ring connected to the parent structure is a monocyclic heteroaryl ring or a nonaromatic ring.

In the present invention, the above-mentioned heteroaryl can be substituted or unsubstituted, and when substituted, the substituent group is preferably one or more substituent groups described in the present application.

As used herein, the term "acetyl" refers to -C(O)CH₃.

As used herein, the term "halo" refers to fluoro, chloro, bromo, and iodo.

Unless otherwise specified, the structure described in the present invention further includes a compound having one or more isotope-enriched atoms. Exemplary isotopes that can be incorporated into the compound of the present invention include isotopes of hydrogen, carbon, nitrogen, oxygen, phosphorus, sulfur, fluoro, chloro, chloro and iodo, for example, 2H, 3H, 11C, 13C, 14C, 13N, 15N, 150, 170, 180, 32P, 33P, 35S, 18F, 36C1, 123I, and 125I.

### Brief Description of the Drawings

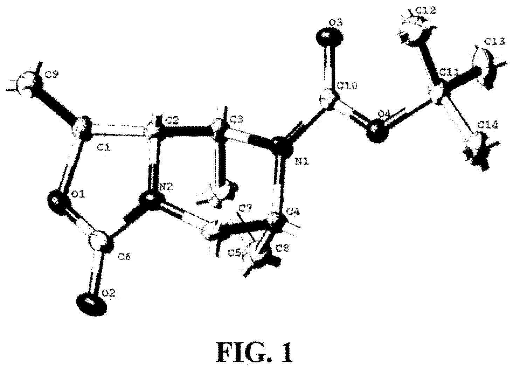

Fig. 1 is a three-dimensional structural diagram showing a molecule of Compound B obtained by single crystal diffraction.

### Detailed Description

The compound of the present invention can be prepared by a variety of synthesis methods well known to those skilled in the art, including the specific embodiments listed below, the embodiments formed by the combination of the listed synthesis methods with other chemical synthesis methods, and equivalent alternatives well-known to those skilled in the art. Preferred embodiments include, but are not limited to, examples of the present invention. Herein, some of the synthesis methods of the intermediates or starting compounds are shown in preparation examples, and some are shown a certain example. If the same intermediate or starting compound is used in the remaining examples, the preparation of this compound will not be described repeatedly.

The present invention will be described in detail by way of examples, these examples do not imply any adverse limitation to the present invention. The present invention is described in detail herein, and the specific embodiments thereof are also disclosed. It will be obvious for those skilled in the art to make various changes and improvements to the specific embodiments of the present invention without departing from the spirit and scope of the present invention. Where no specific conditions are given in the examples, conventional conditions or conditions recommended by the manufacturer are followed. The reagents or instruments for which no manufacturers are noted are all common products commercially available from the market.

For example, a compound of Formula (A-4) can be prepared in the following manner.

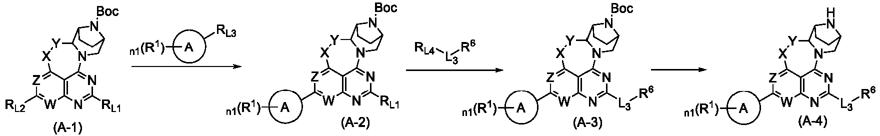

Firstly, a compound of Formula (A-1) is used as a raw material and reacted with 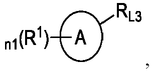 to form a compound of Formula (A-2); then the compound of Formula (A-2) is reacted with 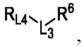 to form a compound of Formula (A-3); and then Boc is removed from the compound of Formula (A-3), to form the compound of Formula (A-4), in which R_{L1}, R_{L2}, R_{L3}, and R_{L4} are each independently a leaving group (for example, halogen, a boric acid group (for example, 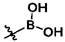 ), a borate group (for example, 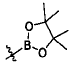 ), -S(=O)CH₃, -S(=O)₂CH₃, -OH, - SnBu₃, benzotriazolyl-O- (for example, 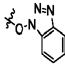 )); 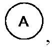 X, Y, Z, W, R¹, n1, L₃, and R⁶ are each as defined above.

For example, the compound of Formula (A-4) can be prepared in the following manner.

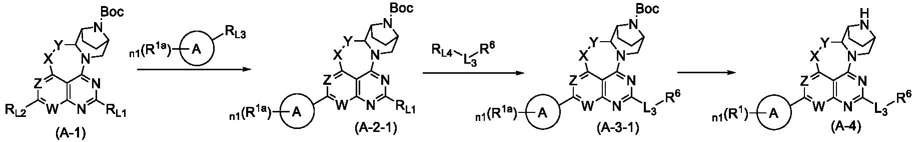

Firstly, a compound of Formula (A-1) is used as a raw material and reacted with 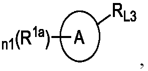 to form a compound of Formula (A-2-1); then the compound of Formula (A-2-1) is reacted with 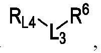 to form a compound of Formula (A-3-1); and then Boc is removed from the compound of Formula (A-3-1), to form the compound of Formula (A-4), in which R_{L1}, R_{L2}, R_{L3}, and R_{L4} are each independently a leaving group (for example, halogen, a boric acid (for example, 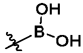 ), a borate group (for example, 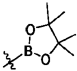 ), -S(=O)CH₃, -S(=O)₂CH₃, -OH, -SnBu₃, benzotriazolyl-O-(for example, 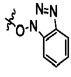 )); 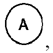 X, Y, Z, W, R¹, n1, L₃, and R⁶ are each as defined above; and R^{1a} is R¹ protected with a protecting group (for example, when ethynyl is present in R¹, the ethynyl may be protected with TIPS; and when amino is present in R¹, the amino may be protected with Boc).

For example, the compound of Formula (A-4) can be prepared in the following manner.

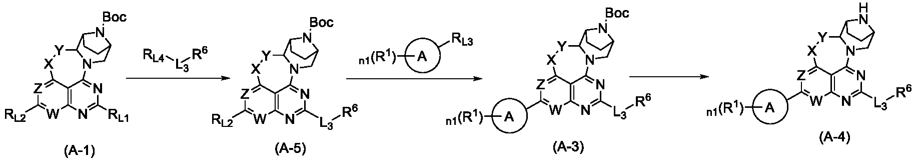

Firstly, a compound of Formula (A-1) is used as a raw material and reacted with 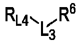 to form a compound of Formula (A-5); then the compound of Formula (A-5) is reacted with 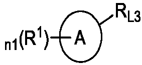 to form a compound of Formula (A-3); and then Boc is removed from the compound of Formula (A-3) to form the compound of Formula (A-4), in which 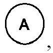 X, Y, Z, W, R¹, n1, L₃, and R⁶ are each as defined above.

The structure of the compound is determined by nuclear magnetic resonance (NMR), mass spectrometry (MS), and others.

The determination by NMR is performed by using Bruker Avance NEO 400 or Bruker Avance NEO 500 nuclear magnetic resonance instrument. The chemical shift (δ) is in parts per million (ppm). The solvents for determination are as shown in various examples, and the internal standard is tetramethylsilane (TMS).

The determination by MS is performed by using Agilent 1100 liquid chromatograph.

Waters 2695 high performance liquid chromatograph is used for HPLC analysis.

Waters 2767 high performance liquid chromatograph is used for preparative HPLC.

The chiral HPLC analysis is performed by using Waters 2695 high performance liquid chromatograph or Waters Investigator SFC system.

The preparative chiral separation is performed by using gilson gx-281 chromatograph or waters SFC-80 chromatograph.

The silica gel plate for TLC is Qingdao GF254 or Yantai Huanghai HSGF254 silica gel plate. The specification of silica gel plate used in thin layer chromatography (TLC) is 0.15mm-0.2mm. The specification of silica gel plate used for separation and purification of the products by thin-layer chromatography is 0.4 mm-0.5 mm.

Generally, ISCO CombiFlash NextGen 300 column chromatography system is used for column chromatography, and Agela Flash Column Silica-Cs series silica gel columns are used.

The preparative HPLC used in the following examples, unless otherwise specified, can adopt the following conditions:
Preparative HPLC (formic acid method 1): column: Waters XBridge C18, 19*250 mm, 5 um; mobile phase system: A: 0.1% formic acid aqueous solution; B: preparative separation-grade acetonitrile; flow rate: 15 mL/min; B%=20%-100%; column temperature: room temperature.
Preparative HPLC (formic acid method 2): chromatographic column: Welch Xtimate C18, 21.2*150 mm, 5 um; mobile phase A: 0.1% formic acid aqueous solution, mible phase B: acetonitrile; flow rate: 15 mL/min; column temperature: room temperature.
Preparative HPLC (ammonium bicarbonate method 1): column: Waters XBridge C18, 19*250 mm, 5 um; mobile phase system: A: 5 mmol/L ammonium bicarbonate aqueous solution; B: preparative separation-grade acetonitrile; flow rate: 15 mL/min; B%=20%-100%; column temperature: room temperature.
Preparative HPLC (ammonium bicarbonate method 2): chromatographic column: Welch Xtimate C18, 21.2*150 mm, 5 um; mobile phase A: 5 mmol/L ammonium bicarbonate aqueous solution, mobile phase B: acetonitrile; flow rate: 15mL/min; column temperature: room temperature.
Preparative HPLC (aqueous ammonia method): chromatographic column: Waters Xbridge C18, 19*150 mm, 5 µm; mobile phase A: 0.1% aqueous ammonia solution, mobile phase B: acetonitrile; flow rate: 15 mL/min; column temperature: room temperature.
Preparative HPLC (trifluoroacetic acid method): chromatographic column: Welch Xtimate C18, 21.2*150mm, Sum; mobile phase A: 0.1% trifluoroacetic acid-water, mobile phase B: acetonitrile; flow rate: 15mL/min; column temperature: room temperature.

### Preparation Example 1: Synthesis of t-butyl (1R,5S)-2-(hydroxymethyl)-3,8-diazabicyclo[3.2.1]octane-8-carboxylate (A1)

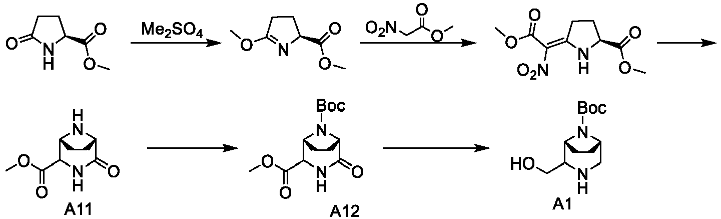

Step I: To a 1 L one-neck flask, methyl (S)-5-oxopyrrolidin-2-carboxylate (200 g, 1397 mmol) and dimethyl sulfate (134 mL) were added. Then, the system was reacted at 56°C for 18 hrs, until the reaction was shown to be completed by TLC. The reaction solution was cooled to room temperature, and then triethyl amine (292 mL) was added dropwise and stirred for 30 min. The reaction solution was added with water (400 mL) and extracted with ethyl acetate (400 mL X 3). The organic phase was washed with saturated brine (400 mL), dried over anhydrous sodium sulfate, and filtered. The filtrate was rotary evaporated to dryness, to obtain methyl (S)-5-methoxy-3,4-dihydro-2H-pyrrole-2-carboxylate (150 g, yield: 68.4%) as a yellow oil. ES-API:[M+1]⁺=158.1.

Step II: To a 1 L one-neck flask, methyl (S)-5-methoxy-3,4-dihydro-2H-pyrrole-2-carboxylate (150 g, 954.3 mmol) and methyl nitroacetate (123.8 g, 1040.2 mmol) were added. Then, the system was reacted at 60°C for 40 hrs, until the reaction was shown to be completed by NMR. Purification by column chromatography (dichloromethane/ethyl acetate =50/1) afforded methyl (S,Z)-5-(2-methoxy-1-nitro-2-oxyethylene)pyrrolidin-2-carboxylate (80.0 g, yield: 34.2%) as a yellow solid. ES-API:[M+1]⁺=245.1.

Step III: To a 1 L one-neck flask, methyl (S,Z)-5-(2-methoxy-1-nitro-2-oxyethylene)pyrrolidin-2-carboxylate (20.0 g, 81.9 mmol), palladium on carbon (2.0 g), and methanol (600 mL) were added. Then, the reactor was purged three time with hydrogen. The system was reacted at 60°C for 5 days, until the reaction was shown to be completed by LC- MS. 4 batches of materials were fed repeatedly. After filtration, the filtrate was concentrated to obtain methyl (1R,5S)-4-oxo-3,8-diazabicyclo[3.2.1]octane-2-carboxylate (A11, 60g, crude product). ES-API:[M+1]⁺=185.1

Step IV: To a 1 L one-neck flask, methyl (1R,5S)-4-oxo-3,8-diazabicyclo[3.2.1]octane-2-carboxylate (A1, 60.0 g, 325 mmol), triethyl amine (65.9 g, 651.5 mmol), and dichloromethane (600 mL) were added. After cooling to 0°C, di-t-butyl dicarbonate (106.6 g, 488.6 mmol) was added. Then, the system was reacted at room temperature overnight, until the reaction was shown to be completed by LC- MS. The reaction solution was added with water (1 L) and extracted with dichloromethane (1 L). The organic phase was rotary evaporated to dryness, and the crude product was purified by column chromatography (dichlorohexane/methanol =20:1), to obtain 8-(t-butyl) 2-methyl (1R,5S)-4-oxo-3,8-diazabicyclo[3.2.1]octane-2,8-dicarboxylate (A12, 70.0 g, yield: 75.8%) as a yellow solid. ES-API:[M-Boc+1]⁺=285.1.

Step V: To a 500 mL three-neck flask, 8-(t-butyl) 2-methyl (1R,5S)-4-oxo-3,8-diazabicyclo[3.2.1]octane-2,8-dicarboxylate (A12, 20.0 g, 70.3 mmol) and tetrahydrofuran (300 mL) were added. After cooling to 0°C, lithium aluminum hydride (5.87 g, 154.7 mmol) (while the temperature was controlled at 5°C or below) was added batchwise. Then, the system was reacted at room temperature overnight, until the reaction was shown to be completed by LC-MS. After cooling to 0°C, the reaction was quenched with water, stirred for 1 hr, and filtered. The filter cake was washed with dichloromethane/methanol (10:1). The filtrate was concentrated to obtain a crude product. The crude product was subjected to reverse preparative separation by preparative HPLC (ammonium bicarbonate method 1) to obtain t-butyl (1R,5S)-2-(hydroxymethyl)-3,8-diazabicyclo[3.2.1]octane-8-carboxylate (A1, 5.8 g, yield: 32.2%) as a yellow solid. ES-API:[M-Boc+1]⁺=243.2.

### Preparation Example 2: Synthesis of Isomer (A2-1)

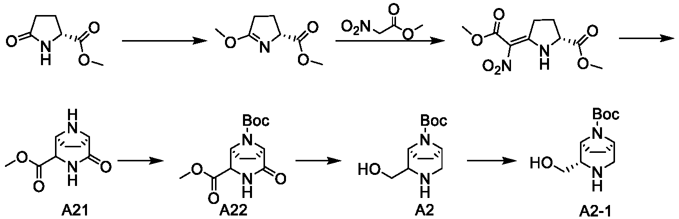

Step I: Methyl (R)-5-oxopyrrolidin-2-carboxylate (395.3 g, 2.762 mmol) was dissolved in dichloromethane (3500 mL). Trimethyloxonium tetrafluoroborate (612.697 g, 4.142 mol, 1.5 eq) was added batchwise at 5°C, and then reacted at 25°C for 24 hrs. The reaction was shown to be completed by TLC. The reaction solution was slowly poured into a saturated sodium carbonate aqueous solution (5000 mL), and the pH was 8 to 9. After separation, the aqueous phase was extracted with dichloromethane (1500 mL). The organic phases were combined, washed with saturated brine, dried over anhydrous magnesium sulfate, filtered, and rotary evaporated to dryness, to obtain methyl (R)-5-methoxy-3,4-dihydro-2H-pyrrole-2-carboxylate (406.7 g, 2.588 mol, yield: 93.702%) as a light yellow oil. ES-API:[M+1]⁺=158.1.

Step II: Methyl (R)-5-methoxy-3,4-dihydro-2H-pyrrole-2-carboxylate (406.7 g, 2.588 mol) was added to methyl nitroacetate (308.133 g, 2.588 mol, 1 eq), and reacted at 60°C for 48 hrs, until the reaction was shown to be completed by NMR. Purification by column chromatography (petroleum ether:ethyl acetate =4:1 to 2:1) afforded a crude product (250.0 g) as a yellow oil, which was slurried, purified, filtered, and dried to obtain methyl (R)-5-(2-methoxy-1-nitro-2-oxyethylene)pyrrolidin-2-carboxylate (146.2 g, 598.687 mmol, yield: 23.136%) as a light yellow solid. ES-API:[M+1]⁺=245.1.

Step III: To a 5L high-pressure reactor, methyl (R)-5-(2-methoxy-1-nitro-2-oxyethylene)pyrrolidin-2-carboxylate (146.2 g, 598.687 mmol) was dissolved in methanol (1500 mL) and tetrahydrofuran (1500 mL), and then Pd(OH)₂/C (15.00 g, 20% purity) was added. The reactor was purged 3 times with hydrogen, and then the reaction was continued under 0.4 MPa at 40°C for 72 hrs. During the reaction, hydrogen was supplemented several times. After cooling to room temperature, the reaction solution was discharged from the reactor. The reaction solution was rotary evaporated to dryness to obtain methyl (1S,5R)-4-oxo-3,8-diazabicyclo[3.2.1]octane-2-carboxylate (A21, 110.3 g, crude product) as a yellow oil. ES-API:[M+1]⁺=185.1.

Step IV: The crude product methyl (1S,5R)-4-oxo-3,8-diazabicyclo[3.2.1]octane-2-carboxylate (A21, 10.00 g, 54.291 mmol) was dissolved in water (50 mL) and tetrahydrofuran (50 mL), Na₂CO₃ (5.754 g, 54.291 mmol) was added, and then (Boc)₂O (11.849 g, 54.291 mmol) was added dropwise at 10°C, and reacted at 20°C for 12 hrs. The reaction was shown to be completed by TLC. The reaction solution was filtered, and the filter cake was washed with ethyl acetate (50 mL). After separation, the aqueous phase was extracted with ethyl acetate (50 mL). The organic phases were combined, rotary evaporated to dryness, and purified by column chromatography (petroleum ether:ethyl acetate =2:1 to 1:1) to obtain 8-(t-butyl) 2-methyl (1S,5R)-4-oxo-3,8-diazabicyclo[3.2.1]octane-2,8-dicarboxylate (A22, 3.61 g, 12.698 mmol, yield: 23.388%) as a white solid. ES-API:[M-Boc+1]⁺=285.1.

Step V: 8-(t-butyl) 2-methyl (1S,5R)-4-oxo-3,8-diazabicyclo[3.2.1]octane-2,8-dicarboxylate (A22, 2.00 g, 7.035 mmol) was dissolved in tetrahydrofuran (20 mL). Lithium aluminum hydride (400.489 mg, 10.552 mmol, 1.5 eq) was added batchwise at 0°C, and then reacted at 20°C for 12 hrs. The reaction was shown to be completed by TLC. At 0°C, water (0.4 mL),15% sodium hydroxide solution (0.4 mL), and water (1.2 mL) were sequentially added dropwise to the reaction solution, and then anhydrous magnesium sulfate was added and stirred for10 min. After filtration and concentration, a crude product was obtained. Reverse-phase preparative separation of the crude product afforded t-butyl (1S,5R)-2-(hydroxymethyl)-3,8-diazabicyclo[3.2.1]octane-8-carboxylate (A2, 0.65 g, yield: 38%) as a light yellow solid. ES-API: [M-Boc+1]⁺=243.2.

Step VI: T-butyl (1S, 5R)-2-(hydroxymethyl)-3,8-diazabicyclo[3.2.1]octane-8-carboxylate (A2, 100 mg) was resolved by chiral preparative SFC [column: CHIRALPAK-IG, 30*250 mm, 10 um, mobile phase: carbon dioxide:methanol (0.1% aqueous ammonia)=80:20 (v/v), flow rate: 150 mL/min, column temperature=35°C, detection wavelength210 nm] to obtain an isomeric compound that was t-butyl (1S,2S,5R)-2-(hydroxymethyl)-3,8-diazabicyclo[3.2.1]octane-8-carboxylate (A2-1, 58 mg, peak 2, retention time 10.391 min, this structure was inferred from the single-crystal diffraction data in Example 14). The isomer was analyzed by chiral HPLC (column: CHIRALPAK-AY, 4.6*250 mm, 5 um, mobile phase: hexane: ethanol (0.1% diethyl amine )=95:5 (v/v), flow rate: 1 mL/min, column temperature=37°C, detection wavelength 210 nm). A2-1: ¹H NMR (400 MHz, DMSO-*d6*): δ 4.57 (t, J = 5.2 Hz, 1H), 4.01-3.87 (m, 2H), 3.23-3.13 (m, 2H), 2.78-2.66 (m, 2H), 2.59-2.52 (m, 1H), 1.84-1.47 (m, 4H), 1.40 (s, 9H). ES-API:[M+1]⁺=243.2.

### Preparation Example 3: Synthesis of(R)-(1-methylenetetrahydro-1H-pyrrolizine-7a(5H)-yl)methanol

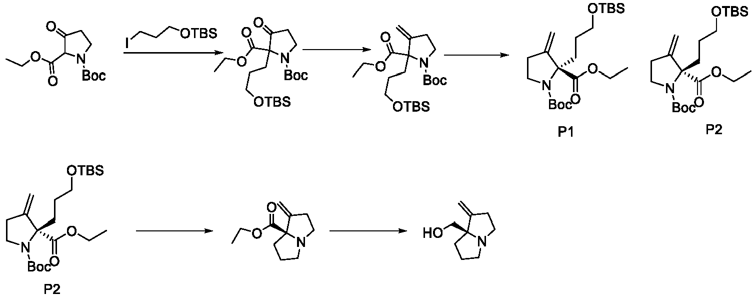

Step I: A mixture of ethyl N-Boc-3-oxopyrrolidin-2-formate (20.0g, 77.73 mmol), t-butyl (3-iodopropoxy)dimethylsilane (70.0 g, 233.106mmol), potassium carbonate (53.70g, 388.51 mmol), and *N, N-*dimethylformamide (150.0 mL) was stirred overnight under nitrogen atmosphere at room temperature. The reaction solution was added with water (500 mL), and extracted with ethyl acetate (500 mL X 2). The reaction solution was washed with saturated brine (400 mL X 3), dried over anhydrous sodium sulfate, and concentrated. The crude product was purified by flash silica gel column chromatography (ethyl acetate/petroleum ether: 0-10%) to obtain 1-(t-butyl) 2-ethyl 2-(3-((t-butyldimethylsilyl)oxy)propyl)-3-oxopyrrolidin-1,2-dicarboxylate (14.2g, 33.08mmol, yield: 43.0%) as a colorless transparent oil. ES-API: [M-Boc+H]⁺=330.3.

Step II: Methyltriphenylphosphonium bromide (59.0g, 165.260 mmol) was added to tetrahydrofuran (150.0 mL) and cooled to 0°C. Under a nitrogen atmosphere, a solution of potassium tert-butoxide in tetrahydrofuran (132.0 mL, 132.0 mmol, 1M) was added to the mixture at 0°C, and then the reaction was warmed to room temperature and stirred for 0.5 hr. 1-(t-butyl) 2-ethyl 2-(3-((t-butyldimethylsilyl)oxy)propyl)-3-oxopyrrolidin-1,2-dicarboxylate (14.20g, 33.052 mmol) was dissolved in tetrahydrofuran (50 mL), and slowly added to the above reaction solution under a nitrogen atmosphere. After that, the reaction solution was stirred at 60°C for 5 hrs. The reaction solution was added with saturated ammonium chloride (300 mL), and extracted with ethyl acetate (300 mLX3). The organic phase was washed with saturated brine (500mL), dried over anhydrous sodium sulfate, and concentrated. The crude product was purified by flash silica gel column chromatography (ethyl acetate /petroleum ether: 0%-8%) to obtain 1-(t-butyl) 2-ethyl 2-(3-((t-butyldimethylsilyl)oxy)propyl)-3-methylenepyrrolidin-1,2-dicarboxylate (9.0g, 21.045 mmol, yield: 63.67%) as a colorless transparent liquid. ES-API: [M-Boc+H]⁺=328.3.

Step III: 1-(t-butyl) 2-ethyl 2-(3-((t-butyldimethylsilyl)oxy)propyl)-3-methylenepyrrolidin-1,2-dicarboxylate (9.0g, 21.045mmol) was resolved (chromatographic column: Daicel CHIRALPAK^{®} IB 250*30 mm, 10µm; mobile phase A: n-hexane; mobile phase B: ethanol; detection wavelength: 254nm /214nm; flow rate: 25mL/min; isocratic elution procedure: mobile phase A: mobile phase B=99:1(V/V); column temperature: room temperature) to obtain two isomers. One structure was arbitrarily designated as 1-(t-butyl) 2-ethyl (S)-2-(3-((t-butyldimethylsilyl)oxy)propyl)-3-methylenepyrrolidin-1,2-dicarboxylate (4.20g, peak 1, retention time 4.21min, yield: 46.0%) as a light yellow oil. ES-API: [M-Boc+H]⁺=328.3. The other structure was arbitrarily designated as 1-(t-butyl) 2-ethyl (R)-2-(3-((t-butyldimethylsilyl)oxy)propyl)-3-methylenepyrrolidin-1,2-dicarboxylate (4.60g, peak 2, retention time4.725 min, yield: 46.0%) as a light yellow oil. ES-API: [M-Boc+H]⁺=328.3.

Step IV: Thionyl chloride (8.0mL,110mmol) was added to a solution of 1-(t-butyl) 2-ethyl (R)-2-(3-((t-butyldimethylsilyl)oxy)propyl)-3-methylenepyrrolidin-1,2-dicarboxylate (3.50g,8.184mmol) in dichloromethane (20.0 mL) in an ice bath, and then reacted at 40°C for 12 hrs. The solvent was removed by rotary evaporation under reduced pressure, and acetonitrile (40 ml) and potassium carbonate (1000mg, 7.24mmol) were added to the system and reacted at 70°C for 1 hr. The reaction solution was cooled to room temperature and filtered. The filtrate was rotary evaporated to dryness under reduced pressure and then purified by column chromatography [DCM:MeOH=100:0-90:10,(v/v)], to obtain ethyl (R)-1-methylenetetrahydro-1H-pyrrolizin-7a(5H)-carboxylate (830mg, 4.251mmol, yield: 51.94%).

Step V: Ethyl (R)-1-methylenetetrahydro-1H-pyrrolizin-7a(5H)-carboxylate (830mg, 4.251mmol) was dissolved in tetrahydrofuran (20.0 mL), and cooled to 0°C. Under a nitrogen atmosphere, a lithium aluminum hydride solution (8.50 mL, 8.50 mmol, 1M in THF) was added to the solution. The reaction was stirred at 0°C for 1 hr. The reaction was quenched with water (0.3 mL), and 15% sodium hydroxide aqueous solution (0.3 mL) and water (0.9 mL) were added. Tetrahydrofuran (10 mL) was added and filtered. The filtrate was concentrated, to obtain (R)-(1-methylenetetrahydro-1H-pyrrolizin-7a(5H)-yl)methanol (300.0mg, 1.958mmol, yield: 46.06%) as a colorless transparent liquid. ES-API: [M +H]⁺=154.1. ¹H NMR (400 MHz, CD₃OD) δ 5.04-5.01(m, 1H), 4.91-4.89(m, 1H),3.35-3.43(m,2H), 3.12-3.04 (m, 1H), 3.01-2.95(m, 1H), 2.77-2.69 (m, 1H), 2.68-2.46 (m, 3H), 2.00-1.90 (m, 1H), 1.88-1.72 (m, 3H).

### Example 1: Synthesis of 5-ethyl-4-((6R,9S)-12-(((2R,7aS)-2-fluorotetrahydro-1H-pyrrolazin-7a(5H)-yl)methoxy)-5a,6,7,8,9,10-hexahydro-5H-4-oxa-3,10a,11,13,14-pentaaza-6,9-methanonaphtho[1,8-ab]heptene-2-yl)naphthalen-2-ol (Z1)

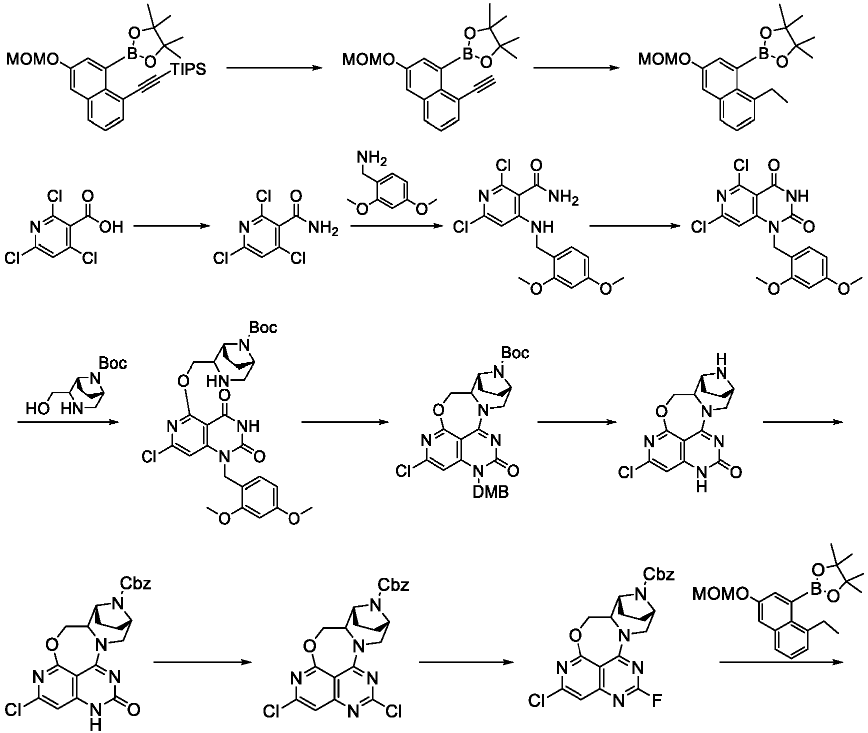

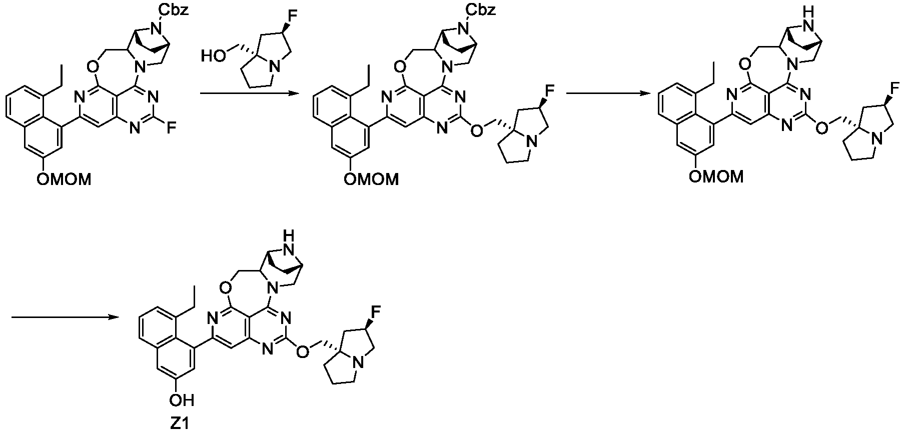

Step I: Cesium fluoride (3.0 g, 20.24 mmol) was added to a solution of triisopropyl ((6-(methoxymethoxy)-8-(4,4,5,5-tetramethyl-1,3,2-dioxaborolan-2-yl)naphthalen-1-yl)ethynyl) silane (500 mg, 1.012 mmol) in N,N-dimethylformamide (5.0 mL), and reacted at room temperature for 2 hrs. After the reaction, the reaction solution was extracted with ethyl acetate (100 mLx2). The ethyl acetate phases were combined and washed with saturated brine (100 mLx3), dried over anhydrous sodium sulfate, filtered, and rotary evaporated to dryness to obtain 2-(8-ethynyl-3-(methoxymethoxy)naphthalen-1-yl)-4,4,5,5-tetramethyl-1,3,2-dioxaborolane (558 mg, crude product). ES-API:[M+H]⁺=339.3.

Step II: 10 wt% palladium on carbon (60 mg) was added to a solution of 2-(8-ethynyl-3-(methoxymethoxy)naphthalen-1-yl)-4,4,5,5-tetramethyl-1,3,2-dioxaborolane (558 mg, crude product) in methanol (10.0 mL), purged 4 times with hydrogen, and then reacted under a hydrogen atmosphere at room temperature for 2 hrs. After the reaction, the reaction solution was filtered and rotary evaporated to dryness under reduced pressure to obtain 2-(8-ethyl-3-(methoxymethoxy) naphthalen-1-yl)-4,4,5,5-tetramethyl-1,3,2-dioxaborolane (284 mg, total yield of 2 steps: 82%). ES-API:[M+1]⁺=343.3.

Step III: At 0°C, 2,4,6-trichloronicotinic acid (5.0 g, 22.22 mmol) was dissolved in dichloromethane (40.0 mL). Oxalyl chloride (6.0 mL) was added dropwise to the system, and then 8 drops of N,N-dimethylformamide were added. The reaction system was reacted with stirring at room temperature for 1 hr. After 1 hr, the solvent was removed by rotary evaporation to dryness under reduced pressure, dry tetrahydrofuran (30.0 mL) was added, and cooled to 0-5°C. The solution was added dropwise to a mixed solution of aqueous ammonia (30.0 mL) and tetrahydrofuran (30.0 mL), and stirred at room temperature for 1 hr. The reaction solution was extracted with ethyl acetate (100 mLx2). The ethyl acetate phases were combined and washed with saturated brine(100 mLx1), dried over anhydrous sodium sulfate, filtered, and rotary evaporated to dryness, to obtain 2,4,6-trichloronicotinamide (6.0 g, crude product). ES-API: [M+H]⁺= 224.9/226.9.

Step IV: In a 500 mL three-neck round-bottom flask, (2,4-dimethoxyphenyl)methyl amine (4.45 g, 26.66 mmol) was dissolved in dry dioxane (90.0 mL). The reaction solution was cooled to 0-5°C in an iced water bath. Under a nitrogen atmosphere, N,N-diisopropylethyl amine (8.61 g, 66.66 mmol) was added, and reacted for about 10-15 min at this temperature. 2,4,6-trichloronicotinamide (6.0 g, crude product) was dissolved in dry dioxane (20 mL) and added dropwise to the above solution. The reaction was continued at 50°C for 5 hrs. After the reaction was shown to be completed by LC/MS. The reaction solution was poured into iced water (400 mL). The reaction solution was extracted with ethyl acetate (200 mLx1), dried over anhydrous sodium sulfate, filtered, and rotary evaporated to dryness. The crude product was purified by column chromatography [petroleum ether:ethyl acetate =100:0-50:50, (v/v)] to obtain 2,6-dichloro-4-((2,4-dimethoxybenzyl)amino)nicotinamide (4.16 g, total yield of 2 steps: 52%). ES-API:[M+H]⁺= 356.1/358.1.

Step V: 2,6-dichloro-4-((2,4-dimethoxybenzyl)amino)nicotinamide (3.723 g, 10.47 mmol) and dry tetrahydrofuran (60 mL) were added to a 500 mLthree-neck round-bottom flask at room temperature, and cooled to 0-5°C in an iced water bath. Sodium hydride (838 mg, 20.95 mmol) was added batchwise, and reacted for 20 min at this temperature. N,N'-carbonyldiimidazole (5.09 g, 31.41 mmol) was added to dry tetrahydrofuran (30 mL), added dropwise to the above solution, and reacted at this temperature for 0.5-1 hr. After the reaction was shown to be completed by LC/MS. The reaction solution was poured into iced water (300 mL), adjusted to pH 7-8 with 6 M hydrochloric acid in an iced water bath, and filtered to obtain 5,7-dichloro-1-(2,4-dimethoxybenzyl)pyrido[4,3-d]pyrimidine-2,4(1H,3H)-dione (3.7 g, yield: 92.7%). ES-API: [M+H]⁺ =382.0/384.0.

Step VI: At room temperature, dry tetrahydrofuran (100 mL) and then t-butyl (1R,5S)-2-(hydroxymethyl)-3,8-diazabicyclo[3.2.1]octane-8-carboxylate (358.0 mg, 1.480 mmol) were added to a 250 mL one-neck flask, and cooled to 0-5°C in an iced water bath. Sodium hydride (1.20 mg, 2.960 mmol) was then added. The reaction was continued for 10-20 min under a nitrogen atmosphere. 5,7-dichloro-1-(2,4-dimethoxybenzyl)pyrido[4,3-d]pyrimidine-2,4(1H,3H)-dione (565.0 mg, 1.480 mmol) was added batchwise, and further reacted at 0-5°C for 20-30 min. After the reaction was shown to be completed by LC/MS. The reaction solution was poured into iced water (300 mL), and adjusted to pH 7-8 with 6 M hydrochloric acid. The reaction solution was extracted with ethyl acetate (100 mLx2). The ethyl acetate phases were combined, washed with saturated brine(100 mLx1), dried over anhydrous sodium sulfate, filtered, and rotary evaporated to dryness to obtain t-butyl (1R,5S)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo[3.2.1]octane-8-carboxylate (850 mg, yield: 97%). ES-API:[M+H]⁺=588.3.

Step VII: At room temperature, dry N,N-dimethylformamide (20 mL) and t-butyl (1R,5S)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo[3.2.1]octane-8-carboxylate (850 mg, 1.448 mmol) were added to a 250 mL one-neck flask. 1-propylphosphoric anhydride (5.0 g, 7.856 mmol) was added and reacted at room temperature for 5 min, and then 1,8-diazadicyclo[5.4.0]undec-7-ene (1.14 g, 7.488 mmol) was added dropwise. The reaction was continued at room temperature for 1-2 hrs. After the reaction, the above solution was slowly added dropwise to 100 mL iced water. A large amount of a solid was precipitated out, filtered, and rotary evaporated to dryness under reduced pressure to obtain the target compound t-butyl (6R,9S)-2-chloro-13-(3,4-dimethoxybenzyl)-12-oxy-5a,6,7,8,9,10,12,13-octahydro-5H-4-oxa-3,10a,11,13,14-pentaaza-6,9-methanonaphtho[1,8-ab]hepten-14-carboxylate (620 mg, yield: 75.2%). ES-API: [M+H]⁺=570.3.

Step VIII: T-butyl (6R,9S)-2-chloro-13-(3,4-dimethoxybenzyl)-12-oxy-5a,6,7,8,9,10,12,13-octahydro-5H-4-oxa-3,10a,11,13,14-pentaaza-6,9-methanonaphtho[1,8-ab]hepten-14-carboxylate (420 mg, 0.7380 mmol) was added to trifluoroacetic acid (5 mL) and stirred at 55°C for 1 hr. After the reaction, the solvent was removed by rotary evaporation to dryness under reduced pressure, to obtain (6R,9S)-2-chloro-5a,6,7,8,9,10-hexahydro-5H-4-oxa-3,10a,11,13,14-pentaaza-6,9-methanonaphtho[1,8-ab]hepten-12(13H)-one (500 mg, crude product). ES-API: [M+H]⁺=320.1.

Step IX: Sodium carbonate (350 mg, 3.282 mmol) was added to a mixed solution of (6R,9S)-2-chloro-5a,6,7,8,9,10-hexahydro-5H-4-oxa-3,10a,11,13,14-pentaaza-6,9-methanonaphtho[1,8-ab]hepten-12(13H)-one (350 mg, 1.094 mmol) in tetrahydrofuran (5.0 mL) and water (5.0 mL) and cooled in an iced water bath. Benzyloxycarbonyl succinimide (354 mg, 1.422 mmol) was added, and reacted at room temperature for 3 hrs. After the reaction, the reaction solution was washed with dichloromethane (80 mL), saturated sodium bicarbonate aqueous solution (100 mL), and saturated brine (80 mL), dried over anhydrous sodium sulfate, and concentrated. The crude product was purified by flash silica gel column chromatography (ethyl acetate /petroleum ether: 0-50%) to obtain benzyl (6R,9S)-2-chloro-12-oxa-5a,6,7,8,9,10,12,13-octahydro-5H-4-oxa-3,10a,11,13,14-pentaaza-6,9-methanonaphtho[1,8-ab]hepten-14-carboxylate (460 mg, yield: 93%). ES-API: [M+H]⁺=454.1.

Step X: At 0°C, phosphorus oxychloride (465mg, 3.039mmol) and N,N-diisopropylethyl amine (392.0mg, 3.039mmol) were sequantially added to a solution of benzyl (6R,9S)-2-chloro-12-oxa-5a,6,7,8,9,10,12,13-octahydro-5H-4-oxa-3,10a,11,13,14-pentaaza-6,9-methanonaphtho[1,8-ab]hepten-14-carboxylate (460 mg, 1.013 mmol) in toluene (3.0 ml), and reacted at 125°C for 12 hrs. After the reaction, the reaction solution was cooled to room temperature, and the solvent was removed by rotary evaporation to dryness under reduced pressure to obtain benzyl (6R,9S)-2,12-dichloro-5a,6,7,8,9,10-hexahydro-5H-4-oxa-3,10a,11,13,14-pentaaza-6,9-methanonaphtho[1,8-ab]hepten-14-carboxylate (520mg, crude product). ES-API: [M+H]⁺=472.2.

Step XI: Potassium fluoride (500mg, 8.492mmol) was added to a solution of benzyl (6R,9S)-2,12-dichloro-5a,6,7,8,9,10-hexahydro-5H-4-oxa-3,10a,11,13,14-pentaaza-6,9-methanonaphtho [1,8-ab]hepten-14-carboxylate (200mg, 0.4246mmol) in N,N-dimethylformamide (3.0 mL), and reaced under a nitrogen atmosphere at 120°C for 4 hrs. After the reaction, the reaction solution was cooled to room temperature. The reaction solution was extracted with ethyl acetate (50 mLx2). The ethyl acetate phases were combined and washed with saturated brine(50 mLx4), dried over anhydrous sodium sulfate, filtered, rotary evaporated to dryness, to obtain benzyl (6R,9S)-2-chloro-12-fluoro-5a,6,7,8,9,10-hexahydro-5H-4-oxa-3,10a,11,13,14-pentaaza-6,9-methanonaphtho[1,8-ab]hepten-14-carboxylate (130mg, yield: 67.4%). ES-API: [M+H]⁺=456.1.

Step XII: Benzyl (6R,9S)-2-chloro-12-fluoro-5a,6,7,8,9,10-hexahydro-5H-4-oxa-3,10a,11,13, 14-pentaaza-6,9-methanonaphtho[1,8-ab]hepten-14-carboxylate (140mg, 0.3076mmol) was dissolved in tetrahydrofuran/water (2 mL/0.5 mL), and then 2-(8-ethyl-3-(methoxymethoxy)naphthalen-1-yl)-4,4,5,5-tetramethyl-1,3,2-dioxaborolane (250mg, 0.7309 mmol), potassium phosphate (250mg, 1.179 mmol), and [n-butyldi(1-adamantanyl)phosphine](2-amino-1,1'-biphenyl-2-yl)palladium (II) mesylate (40.0mg, 0.05494mmol) were added, purged 4 time with nitrogen, and reacted at 80°C under microwave for 1 hr. The reaction solution was cooled to room temperature, and extracted with ethyl acetate (80 mL) and water (60 mL). The organic phase was dried over anhydrous sodium sulfate, and filtered. The filtrate was rotary evaporated to dryness under reduced pressure, and the crude product was purified by flash silica gel column chromatography (ethyl acetate /petroleum ether: 0-70%) to obtain the product benzyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (144mg, yield: 73%). ES-API:[M/2+1]⁺=636.3.

Step XIII: In an iced water bath, sodium hydride (22mg, 0.5428mmol) was added to a solution of ((2R,7aS)-2-fluorotetrahydro-1H-pyrrolizin-7a(5H)-yl)methanol (43.0mg, 0.2714mmol) in tetrahydrofuran (5.0 mL), and reacted at room temperature for 0.5 hr. Benzyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (145mg, 0.1357 mmol) was added to the above reaction system, and reacted at room temperature for 1-2 hrs. After the reaction, the system was added with iced water (50 mL), and extracted with dichloromethane (50 mLx2). The dichloromethane phases were combined, dried over anhydrous sodium sulfate, and filtered. The filtrate was rotary evaporated to dryness under reduced pressure, and crude product was purified by flash silica gel column chromatography (methanol /dichloromethane: 0-10%) to obtain benzyl (6R,9S)-2-(8-ethyl-3-(methoxymethoxy)naphthalen-1-yl)-12-(((2R,7 aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170mg, crude product). ES-API: [M/2+1]⁺=775.3.

Step XIV: 10 wt% palladium on carbon (500mg) was added to a solution of benzyl (6R,9S)-2-(8-ethyl-3-(methoxymethoxy)naphthalen-1-yl)-12-(((2R,7 aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170mg, crude product) in methanol (10.0 mL). The reaction system was purged 4 times with hydrogen, and reacted under a hydrogen atmosphere at room temperature for 2 hrs to obtain (6R,9S)-2-(8-ethyl-3-(methoxymethoxy)naphthalen-1-yl)-12-(((2R,7 aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (162mg, crude product). ES-API:[M/2+1]⁺=7641.3.

Step XV: (6R,9S)-2-(8-ethyl-3-(methoxymethoxy)naphthalen-1-yl)-12-(((2R,7 aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (162mg, crude product) was dissolved in acetonitrile (6.0 mL), 4M hydrochloric acid/dioxane solution (2.0 mL, 8.0mmol) was added in an ice bath, and reacted for 0.5 hr in the ice bath. After the reaction, the reaction solution was concentrated to dryness, and added with dichloromethane (20 mL). In an iced water bath, triethyl amine (3.0 mL) was added. The reaction solution was stirred for10 min, and extracted with dichloromethane (100 mLx1) and water (50 mLx1). The organic phase was dried over anhydrous sodium sulfate, and filtered. The filtrate was rotary evaporated to dryness under reduced pressure, and the crude product was purified by preparative HPLC (formic acid method 1) to obtain 5-ethyl-4-((6R,9S)-12-(((2R,7 aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z1, 4.69mg, total yield of 3 steps: 5%, formate). ES-API:[M+1]⁺=597.3. ¹H NMR (500 MHz, CD₃OD)δ 8.48 (s, 1H), 7.58 (d, *J =* 8.0 Hz, 1H), 7.34 (t, *J* = 8.0 Hz, 1H), 7.25 -6.91 (m, 4H), 5.50 -5.31 (m, 1H), 5.11 -4.97 (m, 1H), 4.70 -4.30 (m, 4H), 4.20 -4.10 (m, 1H), 3.85 -3.73 (m, 2H), 3.65 -3.40 (m, 3H), 3.29 -3.14 (m, 2H), 2.56 -1.75 (m, 12H), 1.00 -0.85 (m, 3H).

### Example 2: Synthesis of 5-ethyl-4-((5aS,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z2)

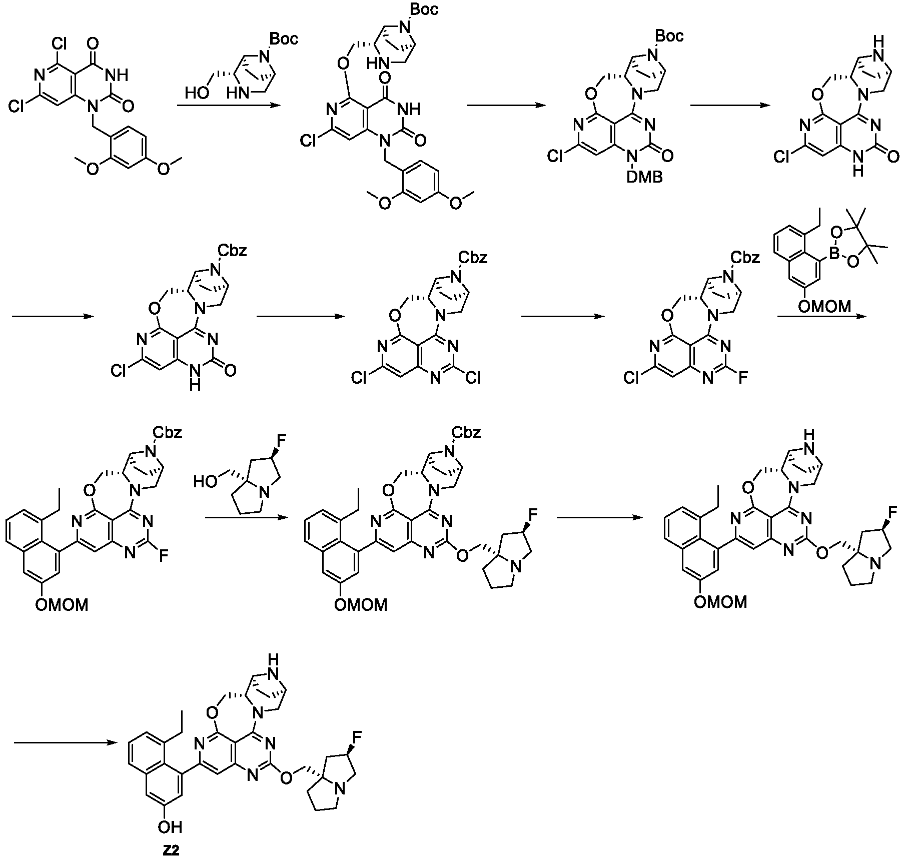

Step I: Dry tetrahydrofuran (100 mL) and then t-butyl (1S,2S,5R)-2-(hydroxymethyl)-3,8-diazabicyclo[3.2.1]octane-8-carboxylate (358.0 mg, 1.480 mmol) were added to a 250 mL one-neck flask at room temperature, cooled to 0-5°C in an iced water bath, and then added with sodium hydride (1.20 mg, 2.960 mmol). After reaction for 10-20 min under a nitrogen atmosphere, 5,7-dichloro-1-(2,4-dimethoxybenzyl)pyrido[4,3-d]pyrimidine-2,4(1H,3H)-dione (565.0mg, 1.480 mmol) was added batchwise, and reacted at 0-5°C for 20-30 min. The reaction was shown to be completed by LC-MS. The reaction solution was poured into iced water (300 mL), and adjusted to pH 7-8 with 6 M hydrochloric acid in an iced water bath. The reaction solution was extracted with ethyl acetate (100 mLx2). The ethyl acetate phases were combined and washed with saturated brine(100 mLx1). The resulting material was dried over anhydrous sodium sulfate, filtered, and rotary evaporated to dryness to obtain t-butyl (1S,2S,5R)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo [3.2.1]octane-8-carboxylate (850 mg, yield: 97%). ES-API:[M+H]⁺=588.3.

Step II: At room temperature, dry N,N-dimethylformamide (20 mL) and t-butyl (1S,2S,5R)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo[3.2.1]octane-8-carboxylate (850 mg, 1.448 mmol) were added to a 250 mL one-neck flask. Finally, 1-propylphosphoric anhydride (5.0 g, 7.856 mmol) was added, and reacted at room temperature for 5 min. Then, 1,8-diazabispiro[5.4.0]undec-7-ene (1.14 g, 7.488 mmol) was added dropwise. The reaction was continued at room temperature for 1-2 hrs. After the reaction, the solution was slowly added dropwise to iced water (100 mL). A large amount of a solid was precipitated out, and filtered. The filter cake was rotary evaporated to dryness under reduced pressure to obtain t-butyl (5aS,6S,9R)-2-chloro-13-(3,4-dimethoxybenzyl)-12-oxy-5a,6,7,8,9,10,12,13-octahydro-5H-4-oxa-3,10a,11,13,14-pentaaza-6,9-methanonaphtho[1,8-ab] hepten-14-carboxylate (620 mg, yield: 73%). ES-API: [M+H]⁺=570.3.

Step III: T-butyl (5aS,6S,9R)-2-chloro-13-(2,4-dimethoxybenzyl)-12-oxy-5a,6,7,8,9,10,12,13-octahydro-5H-4-oxa-3,10a,11,13,14-pentaaza-6,9-methanonaphtho[1,8-ab]hepten-14-carboxylate (420 mg, 0.7380 mmol) was added to trifluoroacetic acid (5.0 mL), and stirred at 55°C for 1 hr. After the reaction, the solvent was removed by rotary evaporation to dryness under reduced pressure, to obtain (5aS,6S,9R)-2-chloro-5a,6,7,8,9,10-hexahydro-5H-4-oxa-3,10a,11,13,14-pentaaza-6,9-methanonaphtho[1,8-ab]hepten-12(13H)-one (500 mg, crude product). ES-API: [M+H]⁺=320.1.

Step IV: Sodium carbonate (350 mg, 3.282 mmol) was added to a mixed solution of (5aS,6S,9R)-2-chloro-5a,6,7,8,9,10-hexahydro-5H-4-oxa-3,10a,11,13,14-pentaaza-6,9-methanonaphtho[1,8-ab]hepten-12(13H)-one (350 mg, 1.094 mmol) in tetrahydrofuran (5.0 mL) and water (5.0 mL). The system was cooled in an iced water bath, added with benzyloxycarbonyl succinimide (354 mg, 1.422 mmol), and reacted at room temperature for 3 hrs. After the reaction, the reaction solution was added with dichloromethane (80 mL), washed with saturated sodium bicarbonate aqueous solution (100mL) and saturated brine (80 mL), and dried over anhydrous sodium sulfate, and concentrated. The crude product was purified by flash silica gel column chromatography (ethyl acetate /petroleum ether: 0-50%) to obtain benzyl (5aS,6S,9R)-2-chloro-12-oxa-5a,6,7,8,9,10,12,13-octahydro-5H-4-oxa-3,10a,11,13,14-pentaaza-6,9-methanonaphtho[1,8-ab] hepten-14-carboxylate (460 mg, yield: 93%). ES-API: [M+H]⁺=454.1.

Step V: At 0°C, phosphorus oxychloride (465 mg, 3.039 mmol) and N,N-diisopropylethyl amine (392.0 mg, 3.039 mmol) were sequentially added to a solution of benzyl (5aS,6S,9R)-2-chloro-12-oxa-5a,6,7,8,9,10,12,13-octahydro-5H-4-oxa-3,10a,11,13,14-pentaaza-6,9-methanonaphtho[1,8-ab]hepten-14-carboxylate (460 mg, 1.013 mmol) in toluene (3.0 mL), and reacted at 125°C for 12 hrs. After the reaction, the reaction solution was cooled to room temperature, and the solvent was removed by rotary evaporation to dryness under reduced pressure to obtain benzyl (5aS,6S,9R)-2,12-dichloro-5a,6,7,8,9,10-hexahydro-5H-4-oxa-3,10a,11,13,14-pentaaza-6,9-methanonaphtho[1,8-ab]hepten-14-carbxylate (520 mg, crude product). ES-API: [M+H]⁺=472.2.

Step VI: Potassium fluoride (500 mg, 8.492 mmol) was added to a solution of benzyl (5aSs,6S,9R)-2,12-dichloro-5a,6,7,8,9,10-hexahydro-5H-4-oxa-3,10a,11,13,14-pentaaza-6,9-methanonaphtho[1,8-ab]hepten-14-carboxylate (200 mg,0.4246 mmol) in N,N-dimethylformamide (3.0 mL) and reacted under a nitrogen atmosphere at 120°C for 4 hrs. After the reaction, the reaction solution was cooled to room temperature, and extracted with ethyl acetate (50 mLx2). The ethyl acetate phases were combined and washed with saturated brine (50 mLx4). The ethyl acetate phase was dried over anhydrous sodium sulfate, filtered, and rotary evaporated to dryness, to obtain benzyl (5aS,6S,9R)-2-chloro-12-fluoro-5a,6,7,8,9,10-hexahydro-5H-4-oxa-3,10a,11,13,14-pentaaza-6,9-methanonaphtho[1,8-ab]hepten-14-carboxylate (130 mg, yield: 62%). ES-API: [M+H]⁺=456.1

Step VII: Benzyl (5aS,6S,9R)-2-chloro-12-fluoro-5a,6,7,8,9,10-hexahydro-5H-4-oxa-3,10a,11,13,14-pentaaza-6,9-methanonaphtho[1,8-ab]hepten-14-carboxylate (140 mg, 0.3076 mmol) was dissolved in tetrahydrofuran/water (2 mL/0.5 mL). 2-(8-ethyl-3-(methoxymethoxy)naphthalen-1-yl)-4,4,5,5-tetramethyl-1,3,2-dioxaborolane (250 mg, 0.7309 mmol), potassium phosphate (250 mg,1.179 mmol), and [n-butyldi(1-adamantanyl)phosphine](2-amino-1,1'-biphenyl-2-yl)palladium (II) mesylate (40.0 mg,0.05494 mmol) were added, purged 4 time with nitrogen, and reacted at 80°C under microwave for 1 hr. The reaction solution was cooled to room temperature, and extracted with ethyl acetate (80 mL) and water (60 mL).The organic phase was dried over anhydrous sodium sulfate, and filtered. The filtrate was rotary evaporated to dryness under reduced pressure, and the crude product was purified by flash silica gel column chromatography (ethyl acetate /petroleum ether: 0-70%) to obtain benzyl (5aS,6S,9R)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (144 mg, yield: 73%). ES-API:[M/2+1]⁺=636.3.

Step VIII: In an iced water bath, sodium hydride (22 mg,0.5428 mmol) was added to a solution of ((2R,7aS)-2-fluorotetrahydro-1H-pyrrolizin-7a(5H)-yl)methanol (43.0 mg, 0.2714 mmol) in tetrahydrofuran (5.0 mL), and reacted at room temperature for 0.5 hr. After 0.5 hr, benzyl (5aS,6S,9R)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (145 mg,0.1357 mmol) was added to the reaction system, and reacted at room temperature for 1-2 hrs. After the reaction, the system was added with iced water (50 mL), and extracted with dichloromethane (50 mLx2). The dichloromethane phases were combined, dried over anhydrous sodium sulfate, and filtered. The filtrate was concentrated, and then the residue was purified by flash silica gel column chromatography (methanol /dichloromethane: 0-10%) to obtain benzyl (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a, 6,7,8,9,10-hexahydro-5H-4-oxa-3, 10a, 11, 13, 14-pentaaza-6,9-methanonaphtho[1,8-ab]hepten-14-carboxylate (170 mg, crude product). ES-API:[M/2+1]⁺=775.3.

Step IX: 10 wt% palladium on carbon (500 mg) was added to a solution of benzyl (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170 mg, crude product) in methanol (10.0 mL), purged 4 times with hydrogen, and reacted under a hydrogen atmosphere at room temperature for 2 hrs, to obtain (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (162 mg, crude product). ES-API:[M/2+1]⁺=7641.3.

Step X: (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (162 mg, crude product) was dissolved in acetonitrile (6.0 mL), and 4M hydrochloric acid/dioxane solution (2.0 mL, 8.0 mmol) was added in an ice bath, and reacted for 0.5 hr in the ice bath. After the reaction, the reaction solution was concentrated to dryness, and added with dichloromethane (20 mL). In an iced water bath, triethyl amine (3.0 mL) was added. The reaction solution was stirred for 10 min, and extracted with dichloromethane (100 mLx1) and water (50 mLx1). The dichloromethane phase was dried over anhydrous sodium sulfate, and filtered. The filtrate was rotary evaporated to dryness under reduced pressure, and the crude product was purified by preparative HPLC (ammonium bicarbonate method 1) to obtain 5-ethyl-4-((5aS,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z2, 4.65 mg, yield of 3 steps: 2.73%). ES-API:[M+1]⁺=597.3. ¹H NMR (500 MHz, CD₃OD)δ 7.57 (d, *J* = 8.2 Hz, 1H), 7.32 (t, *J* = 7.5 Hz, 1H), 7.27 -7.09 (m, 3H), 7.06 -6.90 (m, 1H), 5.43 -5.18 (m, 1H), 5.01 (dd, *J* = 31.4, 13.5 Hz, 1H), 4.58 (d, *J =* 11.5 Hz, 1H), 4.53 -4.38 (m, 1H), 4.25 (d, *J =* 10.4 Hz, 1H), 4.19 (d, *J =* 10.4 Hz, 1H), 4.16 -4.04 (m, 1H), 3.71 (s, 1H), 3.63 (s, 1H), 3.27 -3.17 (m, 4H), 3.01 (td, *J =* 9.6, 5.5 Hz, 1H), 2.55 -2.08 (m, 5H), 2.05 -1.71 (m, 7H), 0.97 (t, *J* = 7.5 Hz, 1H), 0.90 (t, *J =* 7.4 Hz, 2H).

### Example 3: Synthesis of 4-((6R,9S)-12-((2,6-dimethylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)-5-ethylnaphthalen-2-ol (Z175-1)

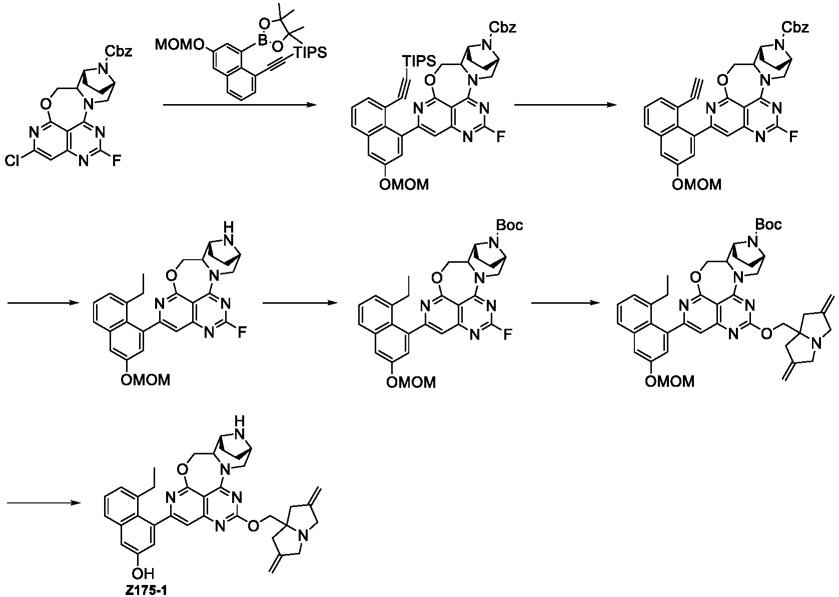

Step I: Benzyl (6R,9S)-2-chloro-12-fluoro-5a,6,7,8,9,10-hexahydro-5H-4-oxa-3,10a,11,13,14-pentaaza-6,9-methanonaphtho[1,8-ab]hepten-14-carboxylate (380.0 mg, 0.8351 mmol) was dissolved in tetrahydrofuran/water (12.0 mL/2.0 mL). Triisopropyl((6-(methoxymethoxy)-8-(4,4,5,5-tetramethyl-1,3,2-dioxaborolane -2-yl)naphthalen-1-yl)ethynyl)silane (825.0 mg, 1.670 mmol), potassium phosphate (800 mg, 3.773 mmol), and [n-butyldi(1-adamantanyl)phosphine](2-amino-1,1'-biphenyl-2-yl)palladium (II) mesylate (80.0 mg, 0.110 mmol) were added, purged 4 time with nitrogen, and reacted at 80°C under microwave for 1 hr. The reaction solution was cooled to room temperature, and extracted with ethyl acetate (150 mL) and water (100 mL). The organic phase was dried over anhydrous sodium sulfate, and filtered. The filtrate was rotary evaporated to dryness under reduced pressure, and the crude product was purified by flash silica gel column chromatography (ethyl acetate /petroleum ether: 0-70%) to obtain benzyl (6R,9S)-12-fluoro-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (500 mg, yield: 76%). ES-API:[M+1]⁺=788.3.

Step II: Cesium fluoride (1.93 g,12.69 mmol) was added to a solution of benzyl (6R,9S)-12-fluoro-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (500 mg, 0.6345 mmol) in N,N-dimethylformamide (20.0 mL), and stirred at room temperature for 1 hr. After the reaction, the reaction solution was extracted with ethyl acetate (150 mL) and water (100 mL). The organic phase was dried over anhydrous sodium sulfate, and filtered. The filtrate was rotary evaporated to dryness under reduced pressure. The crude product was purified by flash silica gel column chromatography (ethyl acetate /petroleum ether: 0-70%) to obtain benzyl (6R,9S)-2-(8-ethynyl-3-(methoxymethoxy)naphthalen-1-yl)-12-fluoro-5a,6,7,8,9, 10-hexahydro-5H-4-oxa-3,10a,11,13,14-pentaaza-6,9-methanonaphtho[1,8-ab]hepten-14-carboxylate (370 mg, yield: 92%). ES-API:[M+1]⁺=631.3.

Step III: 10 wt% palladium on carbon (500 mg) was added to a solution of benzyl (6R,9S)-2-(8-ethynyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (370 mg, 0.5863 mmol) in methanol (20.0 mL), purged 4 times with hydrogen, and reacted under a hydrogen atmosphere at room temperature for 2 hrs, to obtain (6R,9S)-2-(8-ethyl-3-(methoxymethoxy) naphthalen-1-yl)-12-fluoro-5a,6,7,8,9,10-hexahydro-5H-4-oxa-3,10a,11,13,14-pentaaza-6,9-methanonaphtho[1,8-ab]heptene (200 mg, yield: 68%). ES-API:[M+1]⁺=502.3.

Step IV: Triethyl amine (2.0 mL,14.41 mmol) and di-t-butyl dicarbonate (175 mg, 0.7968 mmol) were sequantially added to a solution of (6R,9S)-2-(8-ethyl-3-(methoxymethoxy) naphthalen-1-yl)-12-fluoro-5a,6,7,8,9,10-hexahydro-5H-4-oxa-3,10a,11,13,14-pentaaza-6,9-methanonaphtho[1,8-ab]heptene (200 mg, 0.3984 mmol) in dichloromethane (20.0 mL), and stirred at room temperature for 2 hrs. After the reaction, the reaction solution was extracted with ethyl acetate (150 mL) and water (100 mL). The organic phase was dried over anhydrous sodium sulfate, and filtered. The filtrate was rotary evaporated to dryness under reduced pressure, and the crude product was purified by flash silica gel column chromatography (ethyl acetate /petroleum ether: 0-70%) to obtain t-butyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (150 mg,yield: 62%). ES-API:[M+1]⁺=602.3.

Step V: Sodium hydride (13.28 mg,0.3322 mmol) was added to a solution of (2,6-dimethylenetetrahydro-1H-pyrrolizin-7a(5H)-yl)methanol (28.0 mg, 0.1661 mmol) in tetrahydrofuran (5.0 ml) in an iced water bath, and reacted at room temperature for 0.5 hr. After 0.5 hr, t-butyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (50 mg,0.0830 mmol) was added to the reaction system, and reacted at room temperature for 1-2 hrs. After the reaction, the system was added with iced water (50 mL), and extracted with dichloromethane (50 mLx2). The dichloromethane phases were combined, dried over anhydrous sodium sulfate, and filtered. The filtrate was concentrated, purified by flash silica gel column chromatography (methanol /dichloromethane: 0-10%) to obtain t-butyl (6R,9S)-12-((2,6-dimethylenetetrahydro-1H-pyrrolizin-7a(5H)-yl)methoxy)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]hepten-14-carboxylate (110 mg, crude product). ES-API: [M+1]⁺=747.3.

Step VI: T-butyl (6R,9S)-12-((2,6-dimethylenetetrahydro-1H-pyrrolizin-7a(5H)-yl)methoxy)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]hepten-14-carboxylate (110 mg, crude product) was dissolved in acetonitrile (6.0 mL), and 4M hydrochloric acid/dioxane solution (2.0 mL, 8.0 mmol) was added in an ice bath, and reacted for 0.5 hr in the ice bath. After the reaction, the reaction solution was concentrated to dryness, and added with dichloromethane (20 mL). In an iced water bath, triethyl amine (3.0 mL) was added. The reaction solution was stirred for 10 min, and extracted with dichloromethane (100 mLx1) and water (50 mLx1). The dichloromethane phase was dried over anhydrous sodium sulfate, and filtered. The filtrate was rotary evaporated to dryness under reduced pressure to obtain a crude product. The crude product was purified by preparative HPLC (ammonium bicarbonate method 1) to obtain 4-((6R,9S)-12-((2,6-dimethylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)-5-ethylnaphthalen-2-ol (Z175-1, 7.47 mg, yield of 2 steps: 14.9%). ES-API:[M+1]⁺=603.3.

### Example 4: Synthesis of 4-((6R, 9S)-12-(3-(dimethylamino)azetidin-1-yl)-5a,6,7,8,9,10-hexahydro-5H-4-oxa-3,10a,11,13,14-pentaaza-6,9-methanonaphtho[1,8-ab]heptene-2-yl)-5-ethylnaphthalen-2-ol (Z308)

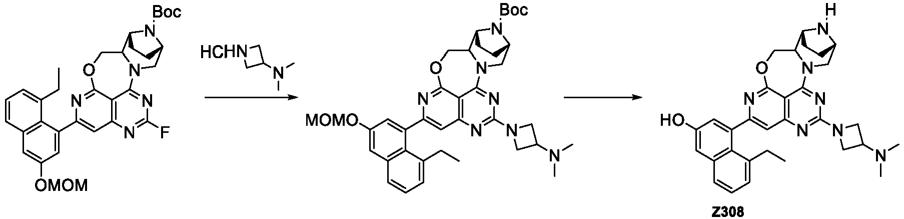

Step I: In an iced water bath, a solution of N,N-dimethylazetidin-3-amine hydrochloride (45.0 mg, 0.332 mmol) in dioxane (5.0 mL) was added with N,N-diisopropylethyl amine (430 mg, 3.322 mmol), and finally with t-butyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphtho[1,8-ab]hepten-14-carboxylate (100 mg, 0.1660 mmol). The reaction was continued at 110°C for 2 hrs. After 2 hrs, the system was added with iced water (50 mL), and extracted with dichloromethane (50 mLx2). The dichloromethane phases were combined, dried over anhydrous sodium sulfate, and filtered. The filtrate was rotary evaporated to dryness under reduced pressure, and purified by flash silica gel column chromatography (methanol /dichloromethane: 0-10%) to obtain t-butyl (6R,9S)-12-(3-(dimethylamino)azetidin-1-yl)-2-(8-ethyl-3-(methoxymethoxy)naphthalen-1-yl)-5a,6,7,8,9,10-hexahydro-5H-4-oxa-3,10a,11,13,14-pentaaza-6,9-methanonaphtho[1,8-ab]hepten-14-carboxylate (110 mg, yield: 97%). ES-API:[M+1]⁺=682.3.

Step II: T-butyl (6R,9S)-12-(3-(dimethylamino)azetidin-1-yl)-2-(8-ethyl-3-(methoxymethoxy) naphthalen-1-yl)-5a,6,7,8,9,10-hexahydro-5H-4-oxa-3,10a,11,13,14-pentaaza-6,9-methanonaphtho[1,8-ab]hepten-14-carboxylate (110 mg, 0.1612 mmol) was dissolved in acetonitrile (6.0 mL), and a 4M hydrochloric acid/dioxane solution (2.0 mL,8.0 mmol) was added in an ice bath, and reacted for 0.5 hr in the ice bath. After the reaction, the reaction solution was concentrated to dryness, and added with dichloromethane (20 mL). In an iced water bath, triethyl amine (3.0 mL) was added. The reaction solution was stirred for 10 min, and extracted with dichloromethane (100 mL) and water (50 mL). The dichloromethane phase was dried over anhydrous sodium sulfate, and filtered. The filtrate was rotary evaporated to dryness under reduced pressure to obtain a crude product. The crude product was purified by preparative HPLC (ammonium bicarbonate method 1) to obtain 4-((6R,9S)-12-(3-(dimethylamino)azetidin-1-yl)-5a,6,7,8,9,10-hexahydro-5H-4-oxa-3,10a,11,13,14-pentaaza-6,9-methanonaphtho[1,8-ab]hepten-2-yl)-5-ethylnaphthalen-2-ol (Z308, 14.92 mg, yield: 16.8%). ES-API:[M+1]⁺=538.3.

### Example 5: Synthesis of trans-2-(((5S, 5aS, 6S, 9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hept-12-yl)oxy)cyclopentan-1-ol (Z718)

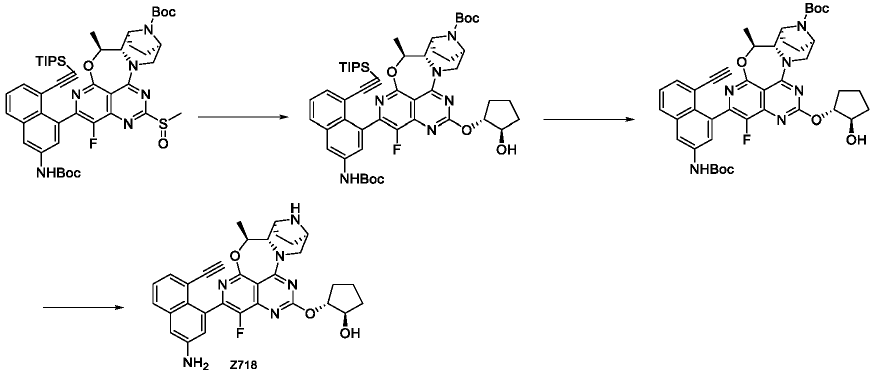

Step I: trans-cyclopentan-1,2-diol (46 mg, 0.45 mmol) was dissolved in tetrahydrofuran(5 mL). 60% sodium hydride (11 mg, 0.27 mmol) was added at 0°C. The reaction was stirred for 30 min at this temperature, and then a solution of butyl (5S, 5as, 6S, 9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (80 mg, 0.092 mmol) in tetrahydrofuran (2 mLl) was added dropwise, and reacted with stirring at this temperature for 30 min. The reaction solution was added with ethyl acetate (40 mL), and sequentially added with water (10 mL) and saturated brine (15 mL). The organic phase was dried over anhydrous sodium sulfate, and filtered. The filtrate was concentrated under reduced pressure. The crude product was purified by flash silica gel column chromatography (tetrahydrofuran/petroleum ether: 0-30%) to obtain the target product t-butyl (5S,5 aS,6S,9R)-2-(3-((t-butoxycarbonyl)amino)-8-((triisopropylsilyl)ethynyl)naphthalen-1-yl)-1-fluoro-12-(((trans -2-hydroxylcyclopentyl)oxy)-5-methyl-5a,6,7,8,9,10-hexahydro-5H-4-oxo-3,10a,11,13,14-pentaaza-6,9-methanonaphtho[1,8-ab]hepten-14-carboxylate (50 mg, yield: 59.9%) as a light yellow solid. ES-API: [M+H]⁺=923.3.

Step II: T-butyl (5S,5aS,6S,9R)-2-(3-((t-butoxycarbonyl)amino)-8-((triisopropylsilyl) ethynyl)naphthalen-1-yl)-1-fluoro-12-(((trans -2-hydroxylcyclopentyl)oxy)-5-methyl-5a,6,7,8,9, 10-hexahydro-5H-4-oxo-3,10a,11,13,14-pentaaza-6,9-methanonaphtho[1,8-ab]hepten-14-carboxylate (20 mg, 0.054 mmol) was dissolved in N,N-dimethylformamide (3 mL). Cesium fluoride (82 mg, 0.54 mmol) was added at room temperature, and the reaction was stirred at room temperature for 30 min. The reaction solution was added with ethyl acetate (30 mL), sequentially washed with water (5 mL), dilute brine (10mLx3), and saturated brine (10 mL). The organic phase was dried over anhydrous sodium sulfate, and filtered. The filtrate was concentrated under reduced pressure, to obtain the target product t-butyl (5S, 5aS, 6S, 9R)-2-(3-((t-butoxycarbonyl)amino)-8-ethynylnaphthalen-1-yl)-1-fluoro-12-(((trans -2-hydroxylcyclopentyl)oxy)-5-methyl-5a,6,7,8,9, 10-hexahydro-5H-4-oxo-3,10a,11,13,14-pentaaza-6,9-methylene[1,8-ab]hepten-14-carboxylate (40 mg, yield: 96.3%) as a light brown solid. ES-API: [M+H]⁺=767.2.

Step III: T-butyl (5S, 5aS, 6S, 9R)-2-(3-((t-butoxycarbonyl)amino)-8-ethynylnaphthalen-1-yl)-1-fluoro-12-((trans-2-hydroxylcyclopentyl)oxy)-5-methyl-5a,6,7,8,9,10-hexahydro-5H-4-oxo-3,10a,11,13,14-pentaaza-6,9-methanonaphtho[1,8-ab]hepten-14-carboxylate (40 mg, 0.018 mmol) was dissolved in ethyl acetate (1 mL). 4M hydrogen chloride/1, 4-dioxane (1.5 mL) was added at 0°C, and the reaction was stirred at room temperature for 2 hrs. The reaction solution was concentrated under reduce pressure. The crude product was purified by preparative HPLC (aqueous ammonia method) to obtain the target product trans-2-(((5S, 5aS, 6S, 9R)-2-(3-amino-8-ethynylnaphthalen-1-yl)-1-fluoro-5-methyl-5a,6,7,8,9,10-hexahydro-5H-4-oxo-3,10a,11,13,14-pentaaza-6,9-methanonaphtho[1,8-ab]hept-12-yl)oxy)cyclopentan-1-ol (Z718, 6 mg, yield: 20.4%, white solid). ES-API: [M+H]⁺= 567.2.

### Example 6: Synthesis of 5-ethyl-4-((5aS,6S,9R)-1-fluoro-12-(((2R,7aS s)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z140-1)

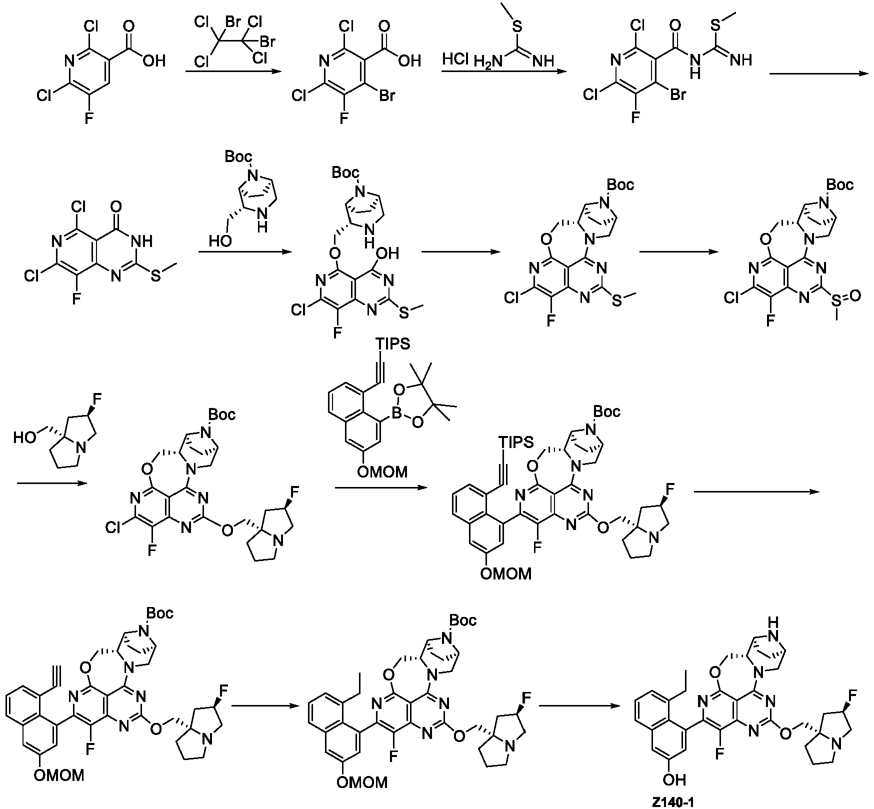

Step I: At -78°C, a solution of 2,6-dichloro-5-fluoronicotinic acid (8.0g, 38.095mmol) in dry tetrahydrofuran(150 mL) was added with methyl lithium (47.6 mL, 76.19mmol, 1.6M) dropwise, and then stirred at -20°C for 2 hrs. The system was cooled to -78°C, a solution of 1,2-dibromo-1,1,2,2-tetrachloroethane (12.405g, 38.095mmol) in tetrahydrofuran(30 mL) was added dropwise over 30 min, and then reacted at 0°C for 1.5 hrs. After the reaction, the reaction solution was poured into iced water (500 mL), and washed with chloroform (100 mLx1). The aqueous phase was adjusted to about pH=3 with hydrochloric acid (3M), and rotary evaporated to dryness under reduced pressure. The crude product was purified by column chromatography [dichloromethane:methanol =100:0-90:10,(v/v)] to obtain 4-bromo-2,6-dichloro-5-fluoronicotinic acid (7.6g, yield: 88%). ES-API:[M+H]⁺=287.9.

Step II: At room temperature, 4-bromo-2,6-dichloro-5-fluoronicotinic acid (6.0g, 20.90mmol) was dissolved in thionyl chloride (40.0 mL), and then 16 drops of N,N-dimethylformamide was added to the reaction system. The reaction system was reacted with stirring at 100°C for 1 hr. The solvent was removed by rotary evaporation to dryness under reduced pressure. At 0°C, the crude product was added to a mixed solvent of tetrahydrofuran (100 mL) and saturated sodium bicarbonate (100 mL), and finally methyl carbamimidothioate (12.91g, 68.607mmol) was added and reacted with stirring at this temperature for 30 min. The reaction was extracted with ethyl acetate (200 mL). The organic phase was washed with brine, dried over anhydrous sodium sulfate, filtered and concentrated, to obtain methyl (4-bromo-2,6-dichloro-5-fluoronicotinamido) carbamimidothioate (2.23g, yield:29.8%) as a yellow solid. ES-API:[M+1]⁺=359.5.

Step III: Under a nitrogen atmosphere, methyl (4-bromo-2,6-dichloro-5-fluoronicotinamido) carbamimidothioate (880 mg, 2.45 mmol) and N, N-diisopropylethyl amine (1.4mL, 8.37mmol) in dioxane (12 mL) were sealed, heated to 100°C, and reacted for 16 hrs. The reaction was concentrated under the volume was about 5mL. Water (15mL) and 2M hydrochloric acid (3mL) was added to the residue. A precipitate was formed, and filtered. The filter cake was washed with water (10mL). The filter cake was dried under reduced pressure to obtain 5,7-dichloro-8-fluoro-2-(methylthio)pyrido[4,3-d]pyrimidine-4(3H)-one (350mg, yield:51%) as a yellow solid. ES-API:[M+H]⁺=280.0.

Step IV: At 0°C, a suspension of sodium hydride (427.29 mg, 10.682 mmol) in tetrahydrofuran (20 mL) was added with t-butyl (1S,2S,5R)-2-(hydroxymethyl)-3,8-diazabicyclo[3.2.1]octane-8-carboxylate (813.53 mg, 3.357 mmol) and stirred at this temperature for 10 min. 5,7-dichloro-8-fluoro-2-(methylthio)pyrido[4,3-d]pyrimidine-4(3H)-one (854mg, 3.052 mmol) was added and stirred with heating at 60°C for 1 hr. After the reaction was completed, the reaction was quenched with water and extracted with dichloromethane/methanol (10:1, 25 mLx3). The organic phase was dried over anhydrous sodium sulfate, filtered and concentrated to obtain t-butyl (1S,2S,5R)-2-(((7-chloro-8-fluoro-4-hydroxyl-2-(methylthio)pyrido[4,3-d]pyrimidine-5-yl)oxy)methyl)-3,8-diazabicyclo[3.2.1]octane-8-carboxylate (1451mg, 2.992 mmol, yield: 98.02%) as a yellow solid. ES-API:[M+H]⁺=486.1.

Step V: Propylphosphoric anhydride (50% in ethyl acetate) (11422.33 mg, 17.949 mmol) was aded to a solution of t-butyl (1S,2S,5R)-2-(((7-chloro-8-fluoro-4-hydroxyl-2-(methylthio)pyrido [4,3-d]pyrimidine-5-yl)oxy)methyl)-3,8-diazabicyclo[3.2.1]octane-8-carboxylate (1451mg, 2.992 mmol) and N, N-diisopropylethyl amine (4.957mL, 29.916mmol) in dichloromethane (20mL) and stirred at room temperature for 3 hrs. The reaction was quenched with water and extracted with dichloromethane. The organic layer was dried over anhydrous sodium sulfate, filtered and concentrated. The obtained solid was suspended in acetonitrile and stirred at room temperature for 10 min. After filtration, a filter cake was collected, to obtain t-butyl (5aS,6S,9R)-2-chloro-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (623 mg, yield: 45.0%) as a white solid. ES-API:[M+H]⁺=468.1.

Step VI: At 0°C, m-chloroperoxybenzoic acid (322.61 mg, 1.589 mmol) was added to a solution of t-butyl (5aS,6S,9R)-2-chloro-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (540 mg, 1.153mmol) in dichloromethane (15 mL). The solution was stirred at room temperature for 1 hr. The reaction was quenched with saturated sodium sulfite and extracted with dichloromethane (100mLx2). The organic phase was dried over anhydrous sodium sulfate and concentrated to obtain a crude product. The crude product was purified by flash silica gel column chromatography (0-10% methanol /dichloromethane) to obtain t-butyl (5aS,6S,9R)-2-chloro-1-fluoro-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (452 mg,yield:81.0%) as a white solid. ES-API: [M+H]⁺=484.1.

Step VII: At 0°C, ((2R,7aS)-2-fluorotetrahydro-1H-pyrrolizin-7a(5H)-yl)methanol (239.16 mg, 1.502 mmol) was added to a solution of sodium hydride (45.07 mg, 1.878 mmol) in tetrahydrofuran. The reaction was stirred at room temperature for 10 min. After cooling to 0°C, a solution of t-butyl (5aS,6S,9R)-2-chloro-1-fluoro-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (363.5 mg, 0.751 mmol) in tetrahydrofuran (5 mL) was added. The reaction was stirred at 0°C for 1 hr. The reaction was quenched with a saturated sodium bicarbonate solution and extracted with ethyl acetate. The organic phase was washed with saturated brine, dried over anhydrous sodium sulfate, filtered, and concentrated. The crude product was purified by flash column chromatography (0-5% methanol /dichloromethane) to obtain t-butyl (5aR,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (134mg, yield: 30.85%) as a yellow solid. ES-API: [M+H]⁺=579.2.

Step VIII: Potassium phosphate (147.55 mg, 0.695mmol) and [n-butyldi(1-adamantanyl) phosphine](2-amino-1,1'-biphenyl-2-yl)palladium (II) mesylate (1.95mg, 0.003mmol) were added to t-butyl (5aR,6S,9R)-2-chloro-1-fluoro-12-(((2R,7aS)-2-fluorotetrahydro-1H-pyrrolizin-7a (5H)-yl)methoxy)-5a,6,7,8,9,10-hexahydro-5H-4-oxa-3,10a,11,13,14-pentaaza-6,9-methanonaphtho[1,8-ab]hepten-14-carboxylate (134.0 mg, 0.233 mmol) and triisopropyl((6-(methoxymethoxy)-8-(4,4,5,5-tetramethyl-1,3,2-dioxaborolane-2-yl)naphthalen-1-yl)ethynyl) silane (230.0mg,0.466mmol) in dioxane (6 mL) and water (1.5 mL). N₂ was bubbled through and the reaction was continued at 80°C under microwave for 40 min. After the reaction, the reaction solution was extracted with ethyl acetate (30 mLx2) and saturated brine (30 mL). The ethyl acetate phases were combined, dried over anhydrous sodium sulfate, and filtered. The filtrate was concentrated under reduced pressure. The crude product was purified by flash silica gel column chromatography (0-4% methanol /dichloromethane) to obtain t-butyl (5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (164 mg, yield: 77.33%) as a yellow solid. ES-API: [M+H]⁺=911.5.

Step IX: Cesium fluoride (127.55 mg, 0.840 mmol) was added to a solution of t-butyl (5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (165mg, 0.181 mmol) in N,N-dimethylformamide (3.0mL), and reacted with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (150mg, crude product) as a yellow oil. ES-API: [M+H]⁺=755.3.

Step X: Palladium on carbon (75.0 mg, 0.061 mmol) was added to a solution of t-butyl (5aS,6S,9R)-2-(8-ethynyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (100mg, 0.1324 mmol) in methanol (5.0Ml), purged with hydrogen and reacted under a hydrogen atmosphere at room temperature for 1 hr. The reaction solution was filtered and concentrated. The crude product was purified by flash silica gel column chromatography (0-3% methanol /dichloromethane) to obtain t-butyl (5aS,6S,9R)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (70mg, yield: 70%) as a yellow solid. ES-API: [M+H]⁺=759.4.

Step XI: A reaction solution of t-butyl (5aS,6S,9R)-2-(8-ethyl-3-(methoxymethoxy) naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (80mg, 0.105 mmol) in acetonitrile (2.0mL) and 4M hydrochloric acid/dioxane solution (0.500mL, 2.000mmol) was reacted at 0°C for 1 hr. After concentration, the crude product was purified by preparative HPLC(ammonium bicarbonate method 1) to obtain 5-ethyl-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z140-1, 9.65 mg, yield: 14.9%) as a yellow solid. ES-API:[M+1]⁺=615.3.

### Example 7: Synthesis of (5aS,6S,9R)-2-(8-ethynylnaphthalen-1-yl)-1-fluoro-12-(((2R, 7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z350)

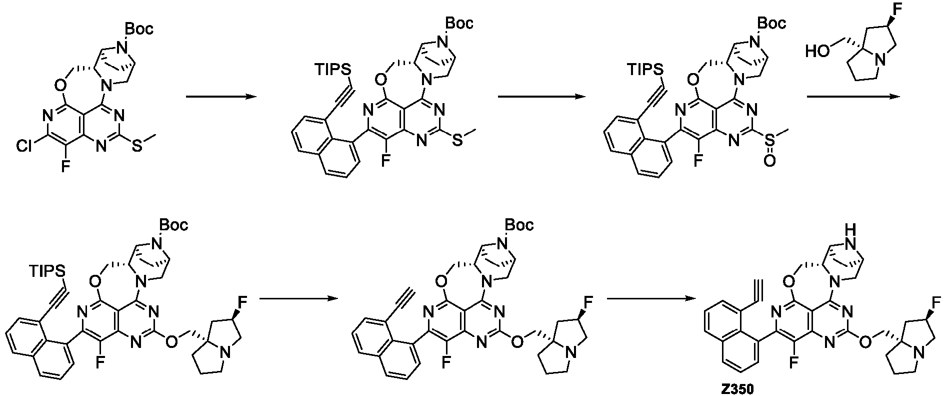

Step I: Potassium phosphate (300 mg, 1.415mmol) and [n-butyldi(1-adamantanyl) phosphine](2-amino-1,1'-biphenyl-2-yl)palladium (II) mesylate (100mg, 0.137mmol) were added to t-butyl (1S,2S,5R)-2-(((7-chloro-8-fluoro-(2-methylthio)-4-oxa-3,4-dihydropyridine[4,3-d] pyrimidine-5-yl)oxy)methyl)-3,8-diazabicyclo[3.2.1]octane-8-carboxylate (150.0mg, 0.321 mmol) and triisopropyl ((8-(4,4,5,5-tetramethyl-1,3,2-dioxaborolane-2-yl)naphthalen-1-yl)ethynyl)silane (220.0mg, 0.506mmol) in dioxane (6 mL) and water (1.5 mL). Nitrogen was bubbled therethrough and the reaction was continued at 80°C under microwave for 40 min. After the reaction, the reaction solution was extracted with ethyl acetate (30 mLx2) and saturated brine (30 mL). The ethyl acetate phases were combined, dried over anhydrous sodium sulfate, and filtered. The filtrate was concentrated under reduced pressure. The crude product was purified by column chromatography (methanol /dichloromethane=0-4%) to obtain t-butyl (5aS,6S,9R)-1-fluoro-12-(methylthio)-2-(8-((triisopropylsilyl)ethynyl)naphthalen-1-yl)-5a,6,7,8,9,10-hexahydro-5H-4-oxo-3,10a,11,13,14-pentaaza-6,9-methanonaphtho[1,8-ab]hepten-14-carboxylate (272mg, crude product) as a yellow solid. ES-API: [M+H]⁺=800.4.

Step II: At 0°C, m-chloroperoxybenzoic acid (67.12 mg, 0.389mmol) was added to a solution of t-butyl (5 aS, 6S,9R)-1-fluoro-12-(methylthio)-2-(8-((triisopropylsilyl)ethynyl)naphthalen-1-yl)-5 a,6,7, 8,9,10-hexahydro-5H-4-oxo-3,10a,11,13,14-pentaaza-6,9-methanonaphtho[1,8-ab]hepten-14-carboxylate (272mg, crude product) in dichloromethane(15 mL). The solution was stirred at room temperature for 1 hr. The reaction was quenched with saturated sodium sulfite and extracted with dichloromethane (100mLx2). The organic phase was dried over anhydrous sodium sulfate and concentrated to obtain a crude product. The crude product was purified by by silica gel column chromatography (methanol /dichloromethane=0-10%) to obtain t-butyl (5aS,6S,9R)-1-fluoro-12-(methylsulfinyl)-2-(8-((triisopropylsilyl)ethynyl)naphthalen-1-yl)-5a,6,7,8,9,10-hexahydro-5H-4-oxo-3,10a,11,13,14-pentaaza-6,9-methanonaphtho[1,8-ab]hepten-14-carboxylate (230 mg, total yield of 2 steps: 93.8%) as a white solid. ES-API:[M+H]⁺=756.3.

Step III: At 0°C, sodium hydride (50.0mg, 1.25mmol) was added to a solution of ((2R,7aS)-2-fluorotetrahydro-1H-pyrrolizin-7a(5H)-yl)methanol (100.0mg, 0.628mmol) in tetrahydrofuran (10.0 mL), and stirred at this temperature for 10 min. Then, a solution of t-butyl (5aS,6S,9R)-1-fluoro-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (230.0mg, 0.304mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (methanol /dichloromethane=0-5%) to obtain t-butyl (5aS,6S,9R)-1-fluoro-12-(((2R,7aS)-2-fluorotetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxo-3,10a,11,13,14-pentaaza-6,9-methanonaphtho[1,8-ab]hepten-14-carboxylate (260mg, crude product) as a yellow solid. ES-API: [M+H]⁺=851.5.

Step IV: Cesium fluoride (1000mg, 6.583mmol) was added to a solution of t-butyl (5aS,6S,9R)-1-fluoro-12-(((2R,7aS)-2-fluorotetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (260mg, crude product) in N,N-dimethylformamide (5.0 mL). The reaction was carried out with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (120mg, total yield of 2 steps: 57%) as a yellow oil. ES-API: [M+H]⁺=695.3.

Step V: A reaction solution of t-butyl (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (120mg, 0.173 mmol) in acetonitrile(5.0 mL) and 4M hydrochloric acid/dioxane solution (2.0mL, 8.000mmol) was reacted at 0°C for 1 hr. After concentrated, the crude product was purified by preparative HPLC (ammonium bicarbonate method 1) to obtain (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho [1,8-ab]heptene (Z350, 34mg, yield: 33%) as a yellow solid. ES-API:[M+1]⁺=595.3. ¹H NMR (500 MHz, CD₃OD)δ 8.07 (d, *J =* 8.2 Hz, 1H), 8.03 (dd, *J* = 7.8, 3.6 Hz, 1H), 7.73 (ddd, *J =* 13.6, 7.2, 1.2 Hz, 1H), 7.67 -7.47 (m, 3H), 5.30 (d, *J =* 54.1 Hz, 1H), 5.05 (ddd, *J =* 13.5, 6.6, 2.2 Hz, 1H), 4.59 (ddd, *J =* 13.2, 8.9, 2.0 Hz, 1H), 4.44 (ddd, *J =* 13.2, 7.4, 5.9 Hz, 1H), 4.28 (t, *J* = 10.1 Hz, 1H), 4.21 (dd, *J =* 10.5, 5.4 Hz, 1H), 4.12 (d, *J =* 6.2 Hz, 1H), 3.72 (s, 1H), 3.63 (d, *J =* 5.2 Hz, 1H), 3.27 -3.16 (m, 4H), 3.02 (td, *J =* 9.8, 5.9 Hz, 1H), 2.29 -2.21 (m, 1H), 2.16 -2.10 (m, 1H), 1.99 (dq, *J* = 13.5, 6.7 Hz, 2H), 1.95 -1.75 (m, 5H).

### Example 8: Synthesis of 4-((5aS,6S,9R)-12-((2,6-dimethylenetetrahydro-1H-pyrrolizin-7a(5H)-yl)methoxy)-1-fluoro-5a,6,7,8,9,10-hexahydro-5H-4-oxo-3,10a,11,13,14-pentaaza-6,9-methanonaphtho[1,8-ab]heptan-2-yl)-5-ethynyl-6-fluoronaphthalen-2-ol (Z380)

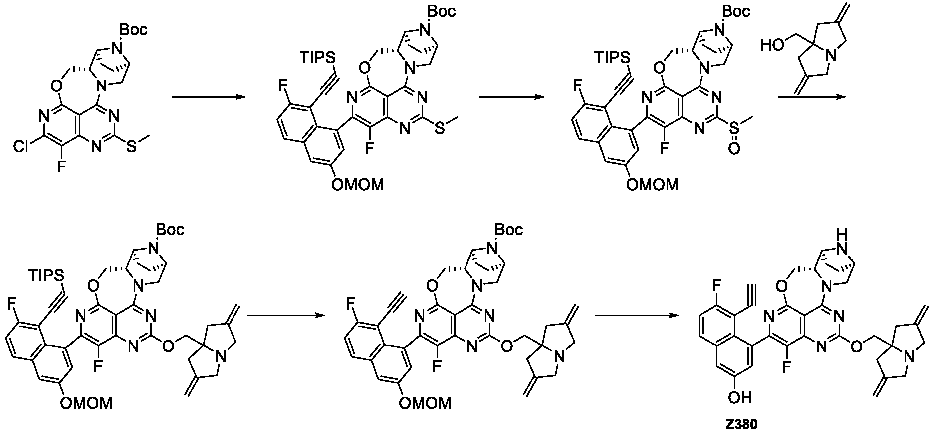

Step I: Potassium phosphate (200mg, 0.943mmol) and [n-butyldi(1-adamantanyl)phosphine] (2-amino-1,1'-biphenyl-2-yl)palladium (II) mesylate (50mg, 0.069mmol) were added to t-butyl (1S,2S,5R)-2-(((7-chloro-8-fluoro-(2-methylthio)-4-oxa-3,4-dihydropyridine[4,3-d]pyrimidine-5-yl)oxy)methyl)-3,8-diazabicyclo[3.2.1]octane-8-carboxylate (100mg, 0.214mmol) and ((2-fluoro-6-(methoxymethoxy)-8-(4,4,5,5-tetramethyl-1,3,2-dioxaborolane-2-yl)naphthalen-1-yl)ethynyl) triisopropylsilane (200.0mg, 0.390mmol) in tetrahydrofuran (6 mL) and water (1.5 mL). Nitrogen was bubbled therethrough and the reaction was continued at 80°C under microwave for 40 min. After the reaction, the reaction solution was extracted with ethyl acetate (30 mLx2) and saturated brine (30 mL). The ethyl acetate phases were combined, dried over anhydrous sodium sulfate, and filtered. The filtrate was concentrated under reduced pressure. The crude product was purified by silica gel column chromatography (methanol /dichloromethane=0-4%) to obtain t-butyl (5aS,6S,9R)-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (210mg, crude product) as a yellow solid. ES-API: [M+H]⁺=818.3.

Step II: At 0°C, m-chloroperoxybenzoic acid (55mg, 0.320mmol) was added to a solution of t-butyl (5aS,6S,9R)-1-fluoro-2-(7-fluoro-3-(methoxymethoxy)-8-((triisopropylsilyl)ethynyl) naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (210mg, crude product) in dichloromethane (15 mL). The solution was stirred at room temperature for 1 hr. The reaction was quenched with saturated sodium sulfite and extracted with dichloromethane (100mLx2). The organic phase was dried over anhydrous sodium sulfate and concentrated, to obtain a crude product, which was purified by silica gel column chromatography (methanol /dichloromethane=0-10%) to obtain t-butyl (5aS,6S,9R)-1-fluoro-2-(7-fluoro-3-(methoxymethoxy)-8-((triisopropylsilyl)ethynyl) naphthalen-1-yl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (210mg, crude product) as a white solid. ES-API:[M+H]⁺=834.4.

Step III: At 0°C, sodium hydride (50.0mg, 1.25mmol) was added to a solution of (2,6-dimethylenetetrahydro-1H-pyrrolizin-7a(5H)-yl)methanol (65.0mg, 0.393mmol) in tetrahydrofuran (10.0 ml), and stirred at this temperature for 10 min. Then, a solution of t-butyl (5aS,6S,9R)-1-fluoro-2-(7-fluoro-3-(methoxymethoxy)-S-((triisopropylsilyl)ethynyl)naphthalen-1-yl)-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (160.0mg, 0.192mmol) in tetrahydrofuran (5 ml) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography(0-5% methanol /dichloromethane) to obtain t-butyl (5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl) naphthalen-1-yl)-5a,6,7,8,9,10-hexahydro-5H-4-oxa-3,10a,11,13,14-pentaaza-6,9-methanonaphtho[1,8-ab]hepten-14-carboxylate (211mg, crude product) as a yellow solid. ES-API: [M+H]⁺=935.5.

Step IV: Cesium fluoride (1000mg, 6.583mmol) was added to a solution of t-butyl (5aS,6S,9R)-12-((2,6-dimethylenetetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (211mg, crude product) in N,N-dimethylformamide (5.0 ml). The reaction was carried out with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5a,6,7,8,9,10-hexahydro-5H-4-oxo-3,10a,11,13,14-pentaaza-6,9-methanonaphtho[1,8-ab]hepten-14-carboxylate (200mg, crude product) as a yellow oil. ES-API: [M+H]⁺=773.3.

Step V: A reaction solution of t-butyl (5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5a,6,7,8,9,10-hexahydro-5H-4-oxo-3,10a,11,13,14-pentaaza-6,9-methanonaphtho[1,8-ab]hepten-14-carboxylate (200mg, crude product) in acetonitrile (5.0mL) and 4M hydrochloric acid/dioxane solution (2.0mL, 8.000mmol) was reacted at 0°C for 1 hr. After concentration, the crude product was purified by preparative HPLC(ammonium bicarbonate method 1) to obtain 4-((5aS,6S,9R)-12-((2,6-dimethylenetetrahydro-1H-pyrrolizin-7a(5H)-yl)methoxy)-1-fluoro-5a,6,7,8,9,10-hexahydro-5H-4-oxo-3,10a,11,13,14-pentaaza-6,9-methanonaphtho[1,8-ab]heptan-2-yl)-5-ethynyl-6-fluoronaphthalen-2-ol (Z380, 8.95mg, total yield of 5 steps: 5.23%) as a yellow solid. ES-API:[M+1]⁺=635.3. ¹H NMR (500 MHz, CD₃OD) δ 7.86 -7.78 (m, 1H), 7.37 -7.27 (m, 2H), 7.19 (dd, *J* = 35.4, 2.5 Hz, 1H), 5.23 -4.95 (m, 5H), 4.69 -4.01 (m, 5H), 3.86 -3.46 (m, 5H), 3.35 (s, 2H), 3.21 (t, *J =* 12.9 Hz, 1H), 2.78 (d, *J =* 16.5 Hz, 2H), 2.58 (d, *J* = 16.4 Hz, 2H), 2.08 -1.65 (m, 5H).

### Example 9: Synthesis of 4-((5aS,6S,9R)-3-chloro-13-((2R,7aS)-2-fluorotetrahydro-1H-pyrrolizin-7a(5H)-ylmethoxy)-5a,6,7,8,9,10-hexahydro-5H-6,9-epiaminoazepin [2',1':3,4][1,4]oxazepin[5,6,7-de]quinazolin-2-yl)-5-ethylnaphthalen-2-ol (Z381)

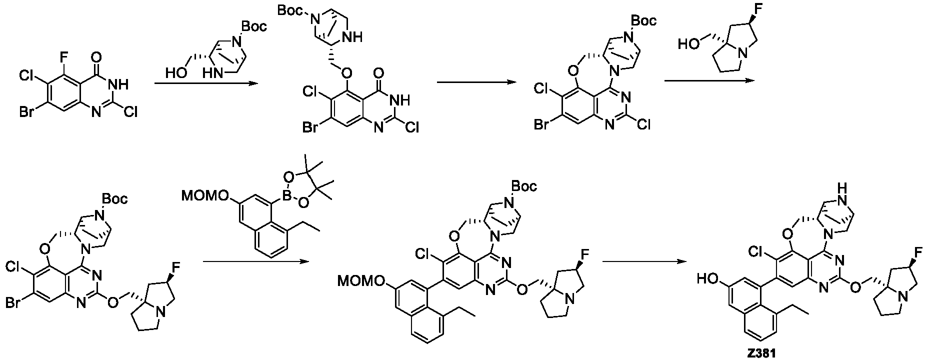

Step I: At 0°C, t-butyl (1S,2S,5R)-2-(hydroxymethyl)-3,8-diazacyclo[3.2.1]octane-8-carboxylate (131 mg, 0.54 mmol) was added to a suspension of sodium hydride (60% dispersed in an oil) (32 mg, 0.81 mmol) in tetrahydrofuran (10 mL) and stirred at this temperature for 10 min. 7-bromo-2,6-dichloro-5-fluoroquinazoline-4(3H)-one (170 mg, 0.54 mmol) was added and stirred with heating at 60°C for 1 hr. After the reaction was completed, the reaction was quenched with water and extracted with dichloromethane/methanol (10:1, 25 mL x 3). The organic phase was dried over anhydrous sodium sulfate and rotary evaporated to dryness, to obtain t-butyl (1S,2S,5R)-2-(((7-bromo-2,6-dichloro-4-oxy-3,4-dihydroquinazoline-5-yl)oxy)methyl)-3,8-diazacyclo [3.2.1]octan-8-carboxylate (150 mg, yield: 51.0%). ES-API:[M+H]⁺=533.2.

Step II: 1H-benzotriazole-1-yloxytripyrrolidinylphosphonium hexafluorophosphate (86 mg, 0.42 mmol) was added to a solution of t-butyl (1S,2S,5R)-2-(((7-bromo-2,6-dichloro-4-oxy-3,4-dihydroquinazoline-5-yl)oxy)methyl)-3,8-diazacyclo[3.2.1]octane-8-carboxylate (110 mg, 0.21 mmol) and N,N-diisopropylethyl amine (80 mg, 0.62 mmol) in super dry dichloromethane (10mL) and stirred at room temperature for 16 hrs. The reaction was quenched with water, extracted with dichloromethane (25 mLx 3), and washed with saturated brine (10 mL). The organic layer was dried over anhydrous sodium sulfate, filtered and concentrated. The obtained crude product was purified by reverse phase preparative HPLC (trifluoroacetic acid method) to obtain t-butyl (5aS,6S,9R)-2-bromo-3,13-dichloro-5a,6,7,8,9,10-hexahydro-5H-6,9-epiminoazepino[2',1':3,4][1,4]oxazepino[5,6,7-de]quinazoline-15-carboxylate (64 mg, yield: 60.3%). ES-API:[M+H]⁺= 515.1.

Step III: ((2R,7aS)-2-fluorotetrahydro-1H-pyrrolizin-7a(5H)-yl)methanol (29 mg, 0.18 mmol), potassium fluoride (51 mg, 0.9 mmol), and 4A molecular sieve were added to a solution of t-butyl (5aS,6S,9R)-2-bromo-3,13-dichloro-5a,6,7,8,9,10-hexahydro-5H-6,9-epiminoazepino [2',1':3,4][1,4]oxazepino[5,6,7-de]quinazoline-15-carboxylate (45 mg, 0.09mmol) in super dry N,N-dimethylformamide (10 mL). The reaction system was stirred for 16 hrs under nitrogen atmosphere at 120°C. After the reaction was completed, the reaction solution was quenched with water, extracted with dichloromethane (25 mL x 3), and washed with saturated brine (10 mL). The organic layer was dried over anhydrous sodium sulfate, filtered and concentrated. The obtained crude product was purified by preparative HPLC (trifluoroacetic acid method) to obtain t-butyl (5aS,6S,9R)-2-bromo-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (22 mg, yield: 38.4%). ES-API:[M+H]⁺= 638.2.

Step IV: Potassium phosphate (21 mg, 0.1mmol) and [n-butyldi(1-adamantanyl)phosphine](2-amino-1,1'-biphenyl-2-yl)palladium (II) mesylate (15 mg) were added to t-butyl (5aS,6S,9R)-2-bromo-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4][1,4]oxazepino[5,6,7-de]quinazoline-15-carboxylate (22 mg, 0.03 mmol) and 2-(8-ethyl-3-(methoxymethoxy)naphthalen-1-yl)-4,4,5,5-tetramethyl-1,3,2-dioxaborolane (17 mg, 0.05 mmol) in tetrahydrofuran (6 mL) and water (1.0 mL), and reacted under a nitrogen atmosphere at 60°C for 2 hrs. After the reaction, the reaction solution was extracted with ethyl acetate (30 mLx2) and saturated brine (10 mL). The ethyl acetate phases were combined, dried over anhydrous sodium sulfate, and filtered. The filtrate was concentrated under reduced pressure. The obtained crude product was purified by TLC (methanol /dichloromethane=1:15) to obtain t-butyl (5aS,6S,9R)-3-chloro-2-(8-ethyl-3-(methoxymethoxy) naphthalen-1-yl)-13-(((2R,7aS)-2-fluorotetrahydro-1H-pyrrolizin-7a(5H)-ylmethoxy)-5a,6,7,8,9,10-hexahydro-5H-6,9-epiminoazepino [2',1':3,4][1,4]oxazepino [5,6,7-de]quinazoline-15-carboxylate (10 mg, yield: 43%). ES-API: [M+H]⁺=774.1.

Step V: A reaction solution of t-butyl (5aS,6S,9R)-3-chloro-2-(8-ethyl-3-(methoxymethoxy) naphthalen-1-yl)-13-(((2R,7aS)-2-fluorotetrahydro-1H-pyrrolizin-7a(5H)-ylmethoxy)-5a,6,7,8,9,10-hexahydro-5H-6,9-epiminoazepino [2',1':3,4][1,4]oxazepino [5,6,7-de]quinazoline-15-carboxylate (10 mg, 0.013 mmol) in acetonitrile (2.0 mL) and 4M hydrochloric acid/dioxane solution (0.5 mL, 2.000 mmol) was reacted at 0°C for 1 hr. The reaction solution was concentrated and the obtained crude product was purified by preparative HPLC (formic acid method 1) to obtain 4-((5aS,6S,9R)-3-chloro-13-((2R,7aS)-2-fluorotetrahydro-1H-pyrrolizin-7a(5H)-ylmethoxy)-5a,6,7,8,9,10-hexahydro-5H-6,9-epiminoazepino[2',1':3,4][1,4]oxazepino[5,6,7-de]quinazoline-2-yl)-5-ethylnaphthalen-2-ol (Z381, 5.0 mg, yield: 61.1%, formate) as a yellow solid. ES-API:[M+1]⁺=630.3. ¹H NMR (400 MHz, CD₃OD)δ 8.51 (s, 2H), 7.59 (d, J = 8.1 Hz, 1H), 7.37 -7.27 (m, 1H), 7.24 -7.07 (m, 2H), 6.97 (d, J = 2.7 Hz, 1H), 6.79 (dd, J = 12.2, 2.6 Hz, 1H), 5.44 (d, J = 52.4 Hz, 1H), 5.09 (dd, J = 29.8, 11.5 Hz, 3H), 4.66 - 4.44 (m, 1H), 4.24 -4.08 (m, 1H), 3.92 -3.57 (m, 5H), 3.29 -3.22 (m, 2H), 2.63 -2.31 (m, 5H), 2.27 -2.13 (m, 2H), 1.99 (dd, J = 30.6, 18.8 Hz, 5H), 0.91 (dt, J = 14.5, 7.4 Hz, 3H).

### Example 10: Synthesis of 5-ethynyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-2-yl)naphthalen-2-ol (Z131-1)

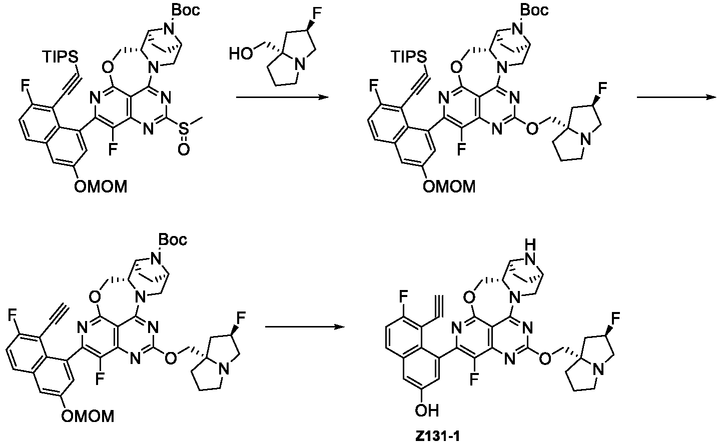

Step I: At 0°C, sodium hydride (50.0mg, 1.25mmol) was added to a solution of ((2R,7aS)-2-fluorotetrahydro-1H-pyrrolizin-7a(5H)-yl)methanol (100.0mg, 0.628mmol) in tetrahydrofuran (10.0mL), and stirred at this temperature for 10 min. Then, a solution of t-butyl (5aS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (210.0mg, 0.252mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain t-butyl (5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170.0mg, yield: 72.6%) as a yellow solid. ES-API: [M+H]⁺=929.3.

Step II: Cesium fluoride (1000mg, 6.583mmol) was added to a solution of t-butyl (5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-14-carboxylate (170mg, 0.183 mmol) in N,N-dimethylformamide (5.0mL). The reaction was carried out with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5aS,6S,9R)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9, 10-hexahydro-5H-4-oxo-3,10a,11,13,14-pentaaza-6,9-methanonaphtho[1,8-ab]hepten-14-carboxylate (200mg, crude product) as a yellow oil. ES-API: [M+H]⁺=773.3.

Step III: A reaction solution of t-butyl (5aS,6S,9R)-2-(8-ethynyl-7-fluoro-3-(methoxymethoxy) naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-14-carboxylate (200mg, crude product) in acetonitrile (10.0mL) and 4M hydrochloric acid/dioxane solution (2.0mL, 8.000mmol) was reacted at 0°C for 1 hr. After concentration, the crude product was purified by preparative HPLC(ammonium bicarbonate method 1) to obtain 5-ethynyl-6-fluoro-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z131-1, 8.95mg, total yield of 2 steps: 7.8%) as a yellow solid. ES-API:[M+1]⁺=629.3.

### Example 11: Synthesis of 5-ethyl-4-((5aS,6S,9R)-12-((1-methyl-1-azaspiro[4.4]nonan-6-yl)oxy)-5a,6,7,8,9,10-hexahydro-5H-4-oxa-3,10a,11,13,14-pentaaza[6,9]methanonaphtho [1,8-ab]hepten-2-yl)naphthalen-2-ol (Z260-1)

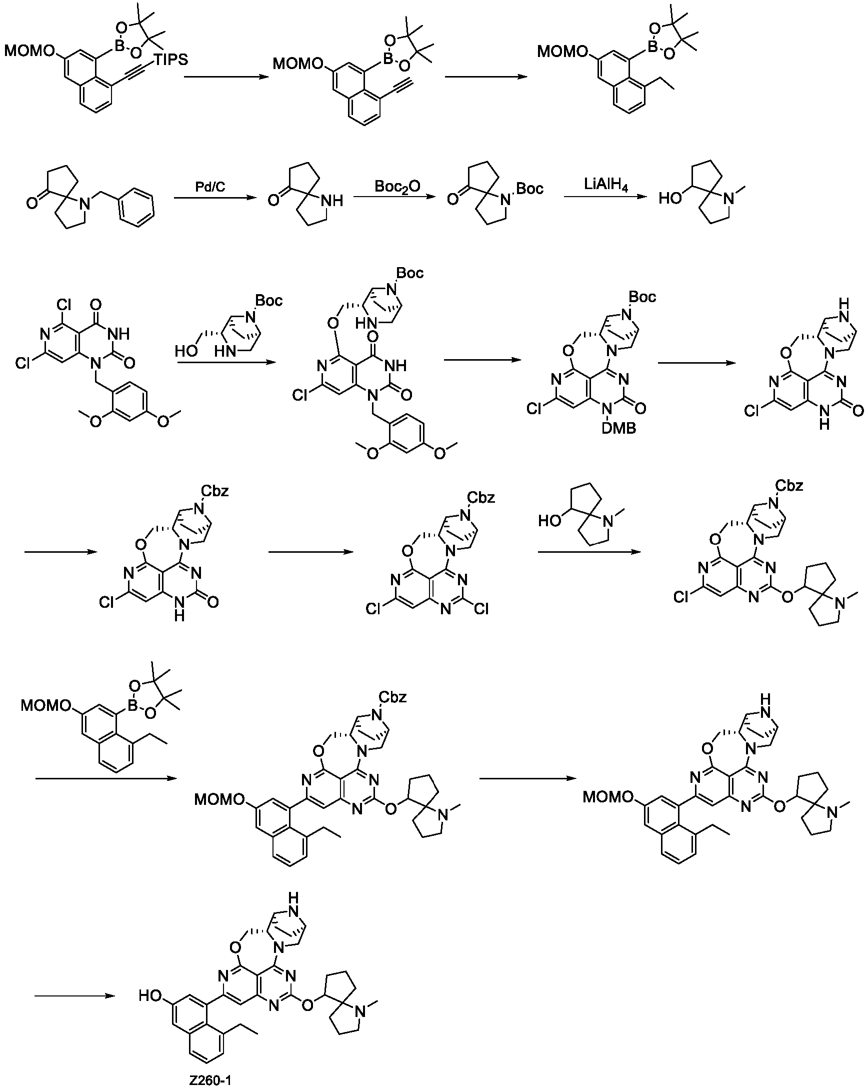

Step I: Cesium fluoride (3.0g, 20.24mmol) was added to a solution of triisopropyl((6-(methoxymethoxy)-8-(4,4,5,5-tetramethyl-1,3,2-dioxaborolane-2-yl)naphthalen-1-yl)ethynyl) silane (500mg, 1.012mmol) in N,N-dimethylformamide (5.0mL), and reacted at room temperature for 2 hrs. After the reaction, the reaction solution was extracted with ethyl acetate (100mLx2). The ethyl acetate phases were combined and washed with saturated brine (100mLx3). The ethyl acetate phase was dried over anhydrous sodium sulfate, filtered, and rotary evaporated to dryness, to obtain 2-(8-ethynyl-3-(methoxymethoxy)naphthalen-1-yl)-4,4,5,5-tetramethyl-1,3,2-dioxaborolane (558 mg, crude product). ES-API:[M+H]⁺=339.3.

Step II: 10 wt% palladium on carbon was added to a solution of 2-(8-ethynyl-3-(methoxymethoxy)naphthalen-1-yl)-4,4,5,5-tetramethyl-1,3,2-dioxaborolane (558mg, crude product) in methanol (10.0mL), purged 4 times with hydrogen, and reacted under hydrogen atmosphere at room temperature for 2 hrs. After the reaction, the reaction solution was filtered and rotary evaporated to dryness under reduced pressure to obtain 2-(8-ethyl-3-(methoxymethoxy) naphthalen-1-yl)-4,4,5,5-tetramethyl-1,3,2-dioxaborolane (284mg, total yield of 2 steps: 82%). ES-API:[M+1]⁺=343.3.

Step III: 1-benzyl-1-azaspiro[4.4]nonan-6-one (1.5 g, 6.5 mmol) was dissolved in methanol (10 mL), added with palladium on carbon (150 mg), purged 3 times with hydrogen, and reacted at normal temperature for 4 hrs. The reaction solution was filtered, washed with methanol, and concentrated, to obtain 1-azaspiro[4.4]nonan-6-one (800 mg, yield: 88.5%). ES-API:[M+H]⁺=140.1.

Step IV: 1-azaspiro[4.4]nonan-6-one (800mg, 5.7mmol) was dissolved in tetrahydrofuran (10 mL). Triethyl amine (0.86mg, 8.5 mmol) and di-t-butyl dicarbonate (1.8g, 8.5mmol) were added at normal temperature, and stirred at room temperature for 8 hrs. The reaction was shown to be completed by LC/MS. The reaction solution was added with water (10mL), extracted with ethyl acetate, dried over anhydrous sodium sulfate, and concentrated, to obtain a crude product, which was purified by silica gel column chromatography (ethyl acetate /petroleum ether=0-80%) to t-butyl 6-oxo-1-azaspiro[4.4]nonan-1-carboxylate (900mg, yield: 66%). ES-API:[M+H]⁺=240.1.

Step V: t-butyl 6-oxo-1-azaspiro[4.4]nonan-1-carboxylate (900mg, 3.7mmol) was dissolved in dry tetrahydrofuran (10 mL), and lithium aluminum hydride (11mL, 11mmol, 1 M in tetrahydrofuran) was added in an ice bath. The system was stirred at 60°C for 2 hrs. The reaction solution was added with water (10mL), extracted with ethyl acetate, dried over anhydrous sodium sulfate, and concentrated, to obtain 1-methyl-1-azaspiro[4.4]nonan-6-ol (450mg), which was directly used in the next step. ES-API:[M+Na]⁺=156.1.

Step VI: At 0°C, sodium hydride (80 mg, 2. 0 mmol) was added to a solution of t-butyl (1S,2S,5R)-2-(hydroxymethyl)-3,8-diazacyclo[3.2.1]octane-8-carboxylate (242 mg, 1.0 mmol) in tetrahydrofuran (10 mL). After reaction for 10-20 min under a nitrogen atmosphere, 5,7-dichloro-1-(2,4-dimethoxybenzyl)pyrido[4,3-d]pyrimidine-2,4(1H,3H)-dione (381 mg, 1.0 mmol) was added batchwise, and reacted at 0-5°C for 20-30 min. After the reaction was shown to be completed by LC/MS, the reaction solution was poured to iced water (300 mL), and adjusted to pH 7-8 with 6M hydrochloric acid in an iced water bath. The reaction solution was extracted with ethyl acetate (100 mLx2). The organic phases were combined, and washed with saturated brine (100 mLx1). The organic phase was dried over anhydrous sodium sulfate, filtered, and rotary evaporated to dryness to obtain the target compound t-butyl (1S,2S,5R)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo[3.2.1]octane-8-carboxylate (560 mg, yield: 87%). ES-API: [M+H]⁺=588.2.

Step VII: At room temperature, dry N,N-dimethylformamide (15 mL) and t-butyl (1S,2S,5R)-2-(((7-chloro-1-(2,4-dimethoxybenzyl)-2,4-dioxa-1,2,3,4-tetrahydropyrido[4,3-d]pyrimidine-5-yl)oxy)methyl)-3,8-diazabicyclo[3.2.1]octane-8-carboxylate (510 mg, 0.87 mmol) was added to a 250mL one-neck flask. Finally, BOP reagent (1.9 g, 4.35 mmol) was added, and reacted at room temperature for 5 min. 1,8-diazadicyclo[5.4.0]undec-7-ene (661 mg, 4.35 mmol) was added dropwise. The reaction was continued at room temperature for 1-2 hrs. After the reaction, the solution was slowly added dropwise to iced water (100 mL). A large amount of a solid was precipitated out, and filtered. The filter cake was rotary evaporated to dryness under reduced pressure, and then purified by column chromatography (petroleum ether/ethyl acetate =1/1) to obtain t-butyl (5aS,6S,9R)-2-chloro-13-(2,4-dimethoxybenzyl)-12-oxy-5a,6,7,8,9,10,12,13-octahydro-5H-4-oxa-3,10a,11,13,14-pentaaza-6,9-methanonaphtho[1,8-ab]hepten-14-carboxylate (300 mg, yield: 61%). ES-API: [M+H]⁺=568.2.

Step VIII: t-butyl (5aS,6S,9R)-2-chloro-13-(2,4-dimethoxybenzyl)-12-oxy-5a,6,7,8,9,10,12, 13-octahydro-5H-4-oxa-3,10a,11,13,14-pentaaza-6,9-methanonaphtho[1,8-ab]hepten-14-carboxylate (300 mg, 0.53 mmol) was added to trifluoroacetic acid (5 mL), and stirred at 55°C for 1 hr. After the reaction, the solvent was removed by rotary evaporation to dryness under reduced pressure, to obtain (5aS,6S,9R)-2-chloro-5a,6,7,8,9,10-hexahydro-5H-4-oxa-3,10a,11,13,14-pentaaza-6,9-methanonaphtho[1,8-ab]hepten-12(13H)-one (500 mg, crude product). ES-API: [M+H]⁺=320.1.

Step IX: Sodium carbonate (168 mg, 1.59 mmol) was added to a mixed solution of (5aS,6S,9R)-2-chloro-5a,6,7,8,9,10-hexahydro-5H-4-oxa-3,10a,11,13,14-pentaaza-6,9-methanonaphtho[1,8-ab]hepten-12(13H)-one (500 mg, crude product) in tetrahydrofuran (5.0 mL) and water (5.0 mL). The system was cooled in an iced water bath, added with benzyloxycarbonyl succinimide (171 mg, 0.68 mmol), and reacted at room temperature for 3 hrs. After the reaction, the reaction solution was added with dichloromethane (80 mL), washed with saturated sodium bicarbonate aqueous solution (100 mL) and saturated brine (80 mL), dried over anhydrous sodium sulfate and concentrated, to obtain a crude product. The crude product was purified by silica gel column chromatography (ethyl acetate /petroleum ether: 0-50%) to obtain the target product t-butyl (5aS,6S,9R)-2-chloro-12-oxa-5a,6,7,8,9,10,12,13-octahydro-5H-4-oxa-3,10a,11,13,14-pentaaza-6,9-methanonaphtho[1,8-ab]hepten-14-carboxylate (396 mg, yield: 93%). ES-API: [M+H]⁺=454.1.

Step X: At 0°C, phosphorus oxychloride (365 mg, 2.4 mmol) and N,N-diisopropylethyl amine (310 mg, 2.4 mmol) were sequentially added to a solution of benzyl (5aS,6S,9R)-2-chloro-12-oxa-5a,6,7,8,9,10,12,13-octahydro-5H-4-oxa-3,10a,11,13,14-pentaaza-6,9-methanonaphtho[1,8-ab]hepten-14-carboxylate (390 mg, 0.8 mmol) in toluene (5.0 mL) and reacted at 105°C for 12 hrs. After the reaction, the reaction solution was cooled to room temperature, and the solvent was removed by rotary evaporation to dryness under reduced pressure. The crude product was purified by preparative HPLC (ammonium bicarbonate method 1) to obtain benzyl (5aS,6S,9R)-2,12-dichloro-5a,6,7,8,9,10-hexahydro-5H-4-oxa-3,10a,11,13,14-pentaaza-6,9-methanonaphtho[1,8-ab]hepten-14-carboxylate (70 mg, yield:17%). ES-API: [M+H]⁺=472.1.

Step XI: Potassium fluoride (29 mg, 0.5 mmol), N,N-diisopropylethylene diamine (39 mg, 0.3 mmol), 1-methyl-1-azaspiro[4.4]nonan-6-ol (31 mg, 0.2 mmol), and 4A molecular sieve were added to a solution of benzyl (5aS,6S,9R)-2,12-dichloro-5a,6,7,8,9,10-hexahydro-5H-4-oxa-3,10a,11,13,14-pentaaza-6,9-methanonaphtho[1,8-ab]hepten-14-carboxylate (50 mg, 0.1 mmol) in dimethyl sulfoxide (3.0 mL), and reacted under a nitrogen atmosphere at 110°C for 4 hrs. After the reaction, the reaction solution was cooled to room temperature, and extracted with ethyl acetate (50mLx2). The ethyl acetate phases were combined and washed with saturated brine (50mLx4). The ethyl acetate phase was dried over anhydrous sodium sulfate, filtered, and rotary evaporated to dryness. The crude product was purified by silica gel column chromatography (petroleum ether/ethyl acetate =2:1) to obtain benzyl (5aS,6S,9R)-2-chloro-12-((1-methyl-1-azaspiro[4.4] nonan-6-yl)oxy)-5a,6,7,8,9,10-hexahydro-5H-4-oxa-3,10a,11,13,14-pentaaza-6,9-methanonaphtho[1,8-ab]hepten-14-carboxylate (28 mg, yield: 44%). ES-API: [M+H]⁺=591.2.

Step XII: Benzyl (5aS,6S,9R)-2-chloro-12-((1-methyl-1-azaspiro[4.4]nonan-6-yl)oxy)-5a, 6,7,8,9,10-hexahydro-5H-4-oxa-3,10a,11,13,14-pentaaza-6,9-methanonaphtho[1,8-ab]hepten-14-carboxylate (28 mg, 0.047 mmol) was dissolved in tetrahydrofuran/water (2 mL/0.5 mL). 2-(8-ethyl-3-(methoxymethoxy)naphthalen-1-yl)-4,4,5,5-tetramethyl-1,3,2-dioxaborolane (24 mg, 0.07 mmol), potassium phosphate (30 mg, 0.14 mmol), and [n-butyldi(1-adamantanyl)phosphine](2-amino-1,1'-biphenyl-2-yl)palladium (II) mesylate (10 mg, 0.014mmol) were added, purged 4 times with nitrogen, and reacted at 60°C for 1 hr. The reaction solution was cooled to room temperature, and extracted with ethyl acetate (80mL) and water (60mL). The organic phase was dried over anhydrous sodium sulfate, and filtered. The filtrate was rotary evaporated to dryness under reduced pressure, to obtain a crude product. The crude product was purified by silica gel column chromatography (ethyl acetate /petroleum ether: 0-70%) to obtain benzyl (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxy-3,10a,11,13,14-pentaaza-6,9-methanonaphtho[1,8-ab]hepten-14-carboxylate (26 mg, yield: 73%). ES-API: [M+H]⁺=771.4.

Step XIII: A solution of benzyl (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]hepten-14-carboxylate (26 mg, 0.034 mmol) in methanol (10.0 mL) was added with 10 wt% palladium on carbon (50 mg), purged 4 times with hydrogen, and reacted under a hydrogen atmosphere at room temperature for 2 hrs. Filtration afforded the crude target compound (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptene (17 mg, yield: 80%). ES-API: [M+H]⁺=637.3.

Step XIV: (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]heptene (17 mg, 0.027 mmol) was dissolved in acetonitrile (6.0 mL), and 4M hydrochloric acid/dioxane solution (1.0 mL, 4.0 mmol) was added in an ice bath, and reacted for 0.5 hr in the ice bath. After the reaction, the reaction solution was concentrated to dryness, and added with dichloromethane (20 mL). In an iced water bath, triethyl amine (3.0mL) was added. The reaction solution was stirred for 10 min, and extracted with dichloromethane (100 mLx1) and water (50 mLx1). The dichloromethane phase was dried over anhydrous sodium sulfate, and filtered. The filtrate was rotary evaporated to dryness under reduced pressure, to obtain a crude product, which was purified by preparative HPLC (formic acid method 1) to obtain 5-ethyl-4-((5aS,6S,9R)-12-((1-methyl-1-azaspiro[4.4]nonan-6-yl)oxy)-5a,6,7,8,9,10-hexahydro-5H-4-oxa-3,10a,11,13,14-pentaaza[6,9]methanonaphtho[1,8-ab]hepten-2-yl)naphthalen-2-ol (Z260-1, 1.6 mg, yield: 10%, formate). ES-API: [M+H]⁺=593.3.

### Example 12: Synthesis of (5aS,6S,9R)-1-fluoro-12-(((2R,7aS)-2-fluorotetrahydro-1H-pyrrolizin-7a(5H)-yl)methoxy)-2-(5-methyl-1H-indazole-4-yl)-5a,6,7,8,9,10-hexahydro-5H-4-oxo-3,10a,11,13,14-pentaaza-6,9-methanonaphtho[1,8-ab]heptene (Z382)

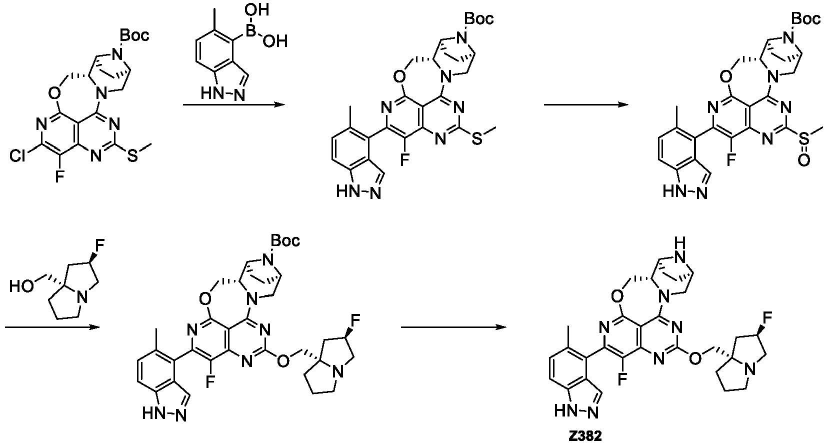

Step I: Cesium carbonate (560mg, 1.719mmol) and terakis(triphenylphosphine)palladium (100mg, 0.87mmol) were added to t-butyl (15,2S,5R)-2-(((7-chloro-8-fluoro-(2-methylthio)-4-oxa-3,4-dihydropyridine[4,3-d]pyrimidine-5-yl)oxy)methyl)-3,8-diazabicyclo[3.2.1]octane-8-carboxylate (200mg, 0.427mmol) and (5-methyl-1H-indazole-4-yl)boric acid (230.0mg, 1.307mmol) in tetrahydrofuran (10.0mL) and water (2.0mL). Nitrogen was bubbled therethrough and the reaction was continued with heating at 115°C for 6 hrs. After the reaction, the reaction solution was extracted with ethyl acetate (30 mLx2) and saturated brine (30 mL). The ethyl acetate phases were combined, dried over anhydrous sodium sulfate, and filtered. The filtrate was concentrated under reduced pressure. The crude product was purified by silica gel column chromatography (methanol /dichloromethane=0-4%) to obtain t-butyl (5aS,6S,9R)-1-fluoro-2-(5-methyl-1H-indazole-4-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (400mg, crude product) as a yellow solid. ES-API: [M+H]⁺=564.4.

Step II: At 0°C, m-chloroperoxybenzoic acid (85mg, 0.493mmol) was added to a solution of t-butyl (5aS,6S,9R)-1-fluoro-2-(5-methyl-1H-indazole-4-yl)-12-(methylthio)-5a,6,7,8,9,10-hexahydro-5H-4-oxo-3,10a,11,13,14-pentaaza-6,9-methanonaphtho[1,8-ab]hepten-14-carboxylate (180mg, 0.319mmol) in dichloromethane (15 mL). The solution was stirred at room temperature for 1 hr. The reaction was quenched with saturated sodium sulfite and extracted with dichloromethane (100mLx2). The organic phase was dried over anhydrous sodium sulfate and concentrated, to obtain a crude product, which was purified by silica gel column chromatography (methanol /dichloromethane=0-10%) to obtain t-butyl (5aS,6S,9R)-1-fluoro-2-(5-methyl-1H-indazole-4-yl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (200mg, crude product) as a white solid. ES-API:[M+H]⁺=580.3.

Step III: At 0°C, sodium hydride (55.0mg, 1.382mmol) was added to a solution of ((2R,7aS)-2-fluorotetrahydro-1H-pyrrolizin-7a(5H)-yl)methanol (239.16 mg, 1.502 mmol)(110.0mg, 0.691mmol) in tetrahydrofuran (10.0mL), and stirred at this temperature for 10 min. Then, a solution of t-butyl (5aS,6S,9R)-1-fluoro-2-(5-methyl-1H-indazole-4-yl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (200mg, crude product) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. A crude product was obtained, which was purified by silica gel column chromatography (methanol /dichloromethane=0-5%) to obtain t-butyl (5aS,6S,9R)-1-fluoro-12-(((2R,7aS)-2-fluorotetrahydro -1H-pyrrolizin-7a(5H)-yl)methoxy)-2-(5-methyl-1H-indazole-4-yl)-5a,6,7,8,9,10-hexahydro-5H-4-oxo-3,10a,11,13,14-pentaaza-6,9-methanonaphtho[1,8-ab]hepten-14-carboxylate (220mg, total yield of 3 steps: 94.5%) as a yellow solid. ES-API: [M+H]⁺=675.3.

Step IV: At 0°C, 4M hydrochloric acid/dioxane solution (2.0mL, 8.000mmol) was added to a solution of t-butyl (5aS,6S,9R)-1-fluoro-12-(((2R,7aS)-2-fluorotetrahydro-1H-pyrrolizin-7a(5H)-yl)methoxy)-2-(5-methyl-1H-indazole-4-yl)-5a,6,7,8,9,10-hexahydro-5H-4-oxo-3,10a,11,13,14-pentaaza-6,9-methanonaphtho[1,8-ab]hepten-14-carboxylate (200mg, 0.296mmol) in acetonitrile (5.0mL), and the reaction solution was reacted at 0°C for 1 hr. The reaction solution was concentrated. The crude product was subjected to preparative HPLC (ammonium bicarbonate method 1) to obtain (5aS,6S,9R)-1-fluoro-12-(((2R,7aS)-2-fluorotetrahydro-1H-pyrrolizin-7a(5H)-yl)methoxy)-2-(5-methyl-1H-indazole-4-yl)-5a,6,7,8,9,10-hexahydro-5H-4-oxo-3,10a,11,13,14-pentaaza-6,9-methanonaphtho[1, 8-ab]heptene (Z382, 30mg, yield: 17.6%) as a yellow solid. ES-API:[M+1]⁺=575.3. ¹H NMR (500 MHz, CD₃OD)δ 8.39 (s, 2H), 7.74 (d, *J =* 27.1 Hz, 1H), 7.59 (d, *J* = 8.6 Hz, 1H), 7.40 (dd, *J =* 8.6, 3.2 Hz, 1H), 5.52 (d, *J =* 52.0 Hz, 1H), 5.20 (t, *J =* 12.5 Hz, 1H), 4.82 -4.48 (m, 4H), 4.33 (d, *J =* 5.9 Hz, 1H), 4.20 -3.67 (m, 4H), 3.37 (dd, *J =* 23.7, 13.9 Hz, 2H), 2.73 -1.87 (m, 12H).

### Example 13: Synthesis of 5-ethynyl-6-fluoro-4-((5aS,6S,9R)-1-fluoro-12-((hexahydro-1H-pyrrolizin-3-yl)methoxy)-5a,6,7,8,9,10-hexahydro-5H-4-oxo-3,10a,11,13,14-pentaaza-6,9-methanonaphtho[1,8-ab]hepten-2-yl)naphthalen-2-ol (Z383)

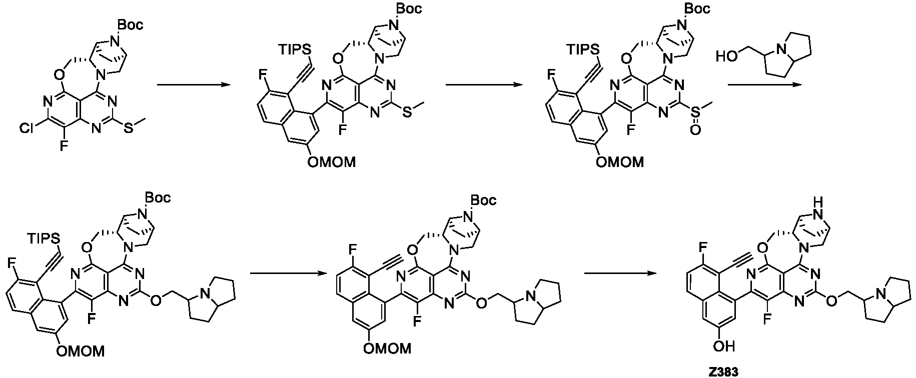

Step I: Potassium phosphate (200mg, 0.943mmol) and [n-butyldi(1-adamantanyl) phosphine](2-amino-1,1'-biphenyl-2-yl)palladium (II) mesylate (50mg, 0.069mmol) were added to t-butyl (1S,2S,5R)-2-(((7-chloro-8-fluoro-(2-methylthio)-4-oxa-3,4-dihydropyridine[4,3-d] pyrimidine-5-yl)oxy)methyl)-3,8-diazabicyclo[3.2.1]octane-8-carboxylate (100mg, 0.214mmol) and ((2-fluoro-6-(methoxymethoxy)-8-(4,4,5,5-tetramethyl-1,3,2-dioxaborolane -2-yl)naphthalen-1-yl)ethynyl)triisopropylsilane (200.0mg, 0.390mmol) in tetrahydrofuran (6 mL) and water (1.5 mL). Nitrogen was bubbled therethrough and the reaction was continued at 80°C under microwave for 40 min. After the reaction, the reaction solution was extracted with ethyl acetate (30 mLx2) and saturated brine (30 mL). The ethyl acetate phases were combined, dried over anhydrous sodium sulfate, and filtered. The filtrate was concentrated under reduced pressure. A crude product was obtained, which was purified by silica gel column chromatography (methanol /dichloromethane=0-4%) to obtain t-butyl (5aS,6S,9R)-1-fluoro-2-(7-fluoro-3-(methoxymethoxy)-8-((triisopropylsilyl) ethynyl)naphthalen-1-yl)-12-(methylthio)-5a,6,7,8,9,10-hexahydro-5H-4-oxo-3,10a,11,13,14-pentaaza-6,9-methanonaphtho[1,8-ab]hepten-14-carboxylate (210mg, crude product) as a yellow solid. ES-API: [M+H]⁺=818.3.

Step II: At 0°C, m-chloroperoxybenzoic acid (55mg, 0.320mmol) was added to a solution of t-butyl (5aS,6S,9R)-1-fluoro-2-(7-fluoro-3-(methoxymethoxy)-8-((triisopropylsilyl)ethynyl) naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (210mg, crude product) in dichloromethane (15 mL). The solution was stirred at room temperature for 1 hr. The reaction was quenched with saturated sodium sulfite and extracted with dichloromethane (100mLx2). The organic phase was dried over anhydrous sodium sulfate and concentrated, to obtain a crude product, which was purified by silica gel column chromatography (methanol /dichloromethane=0-10%) to obtain t-butyl (5aS,6S,9R)-1-fluoro-2-(7-fluoro-3-(methoxymethoxy)-8-((triisopropylsilyl)ethynyl) naphthalen-1-yl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (210mg, crude product) as a white solid. ES-API: [M+H]⁺=834.4.

Step III: At 0°C, sodium hydride (54.0mg, 1.34mmol) was added to a solution of (hexahydro-1H-pyrrolizin-3-yl)methanol (95.0mg, 0.673mmol) in tetrahydrofuran (10.0mL), and stirred at this temperature for 10 min. Then, a solution of t-butyl (5aS,6S,9R)-1-fluoro-2-(7-fluoro-3-(methoxymethoxy)-8-((triisopropylsilyl)ethynyl)naphthalen-1-yl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (270.0mg, 0.324mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. A crude product was obtained, which was purified by silica gel column chromatography (methanol /dichloromethane=0-5%) to obtain t-butyl (5aS,6S,9R)-1-fluoro-2-(7-fluoro-3-(methoxymethoxy) -8-((triisopropylsilyl)ethynyl)naphthalen-1-yl)-12-((hexahydro-1H-pyrrolizin-3-yl)methoxy)-5a,6,7,8,9,10-hexahydro-5H-4-oxo-3,10a,11,13,14-pentaaza-6,9-methanonaphtho[1,8-ab]hepten-14-carboxylate (300mg, crude product) as a yellow solid. ES-API: [M+H]⁺=911.5.

Step IV: Cesium fluoride (1000mg, 6.583mmol) was added to a solution of t-butyl (5aS,6S,9R)-1-fluoro-2-(7-fluoro-3-(methoxymethoxy)-8-((triisopropylsilyl)ethynyl)naphthalen-1-yl)-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]hepten-14-carboxylate (300mg, crude product) in N,N-dimethylformamide (5.0mL). The reaction was carried out with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5aS,6S,9R)-2-(8-ethynyl-7-fluoro-3-(methoxymethoxy)naphthalen-1-yl)-1-fluoro-12-((hexahydro-1H-pyrrolizin-3-yl)methoxy)-5a,6,7,8,9,10-hexahydro-5H-4-oxa-3,10a,11,13,14-pentaaza-6,9-methanonaphtho[1,8-ab]hepten-14-carboxylate (150mg, total yield of 3 steps: 60.3%) as a yellow oil. ES-API: [M+H]⁺=755.3.

Step V: A reaction solution of t-butyl (5aS,6S,9R)-2-(8-ethynyl-7-fluoro-3-(methoxymethoxy) naphthalen-1-yl)-1-fluoro-12-((hexahydro-1H-pyrrolizin-3-yl)methoxy)-5a,6,7,8,9,10-hexahydro-5H-4-oxo-3,10a,11,13,14-pentaaza-6,9-methanonaphtho[1,8-ab]hepten-14-carboxylate (150mg, 0.199mmol) in acetonitrile (5.0mL) and 4M hydrochloric acid/dioxane solution (2.0mL, 8.000mmol) was reacted at 0°C for 1 hr. After concentration, the crude product was purified by preparative HPLC (formic acid method 1) to obtain 5-ethynyl-6-fluoro-4-((5aS,6S,9R)-1-fluoro-12-((hexahydro-1H-pyrrolizin-3-yl)methoxy)-5a,6,7,8,9,10-hexahydro-5H-4-oxo-3,10a,11,13,14-pentaaza-6,9-methanonaphtho[1,8-ab]hepten-2-yl)naphthalen-2-ol (Z383, 49.0mg, yield: 41%, formate) as a yellow solid. ES-API:[M+1]⁺=611.3. ¹H NMR (500 MHz, CD₃OD) δ 8.43 (s, 2H), 7.88 -7.83 (m, 1H), 7.35 -7.16 (m, 3H), 5.07 (d, *J =* 13.7 Hz, 1H), 4.72 -4.60 (m, 2H), 4.57 -4.46 (m, 1H), 4.37 -4.19 (m, 2H), 4.11 (s, 1H), 3.86 (d, *J* = 21.8 Hz, 2H), 3.66 -3.40 (m, 3H), 2.47 -1.66 (m, 13H).

### Example 14: Synthesis of (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5,6,7,8,9,10-hexahydro-5H-4-oxy-3,10a,11,13,14-pentaaza-6,9-methanonaphtho[1,8-ab]heptene (Z360-1)

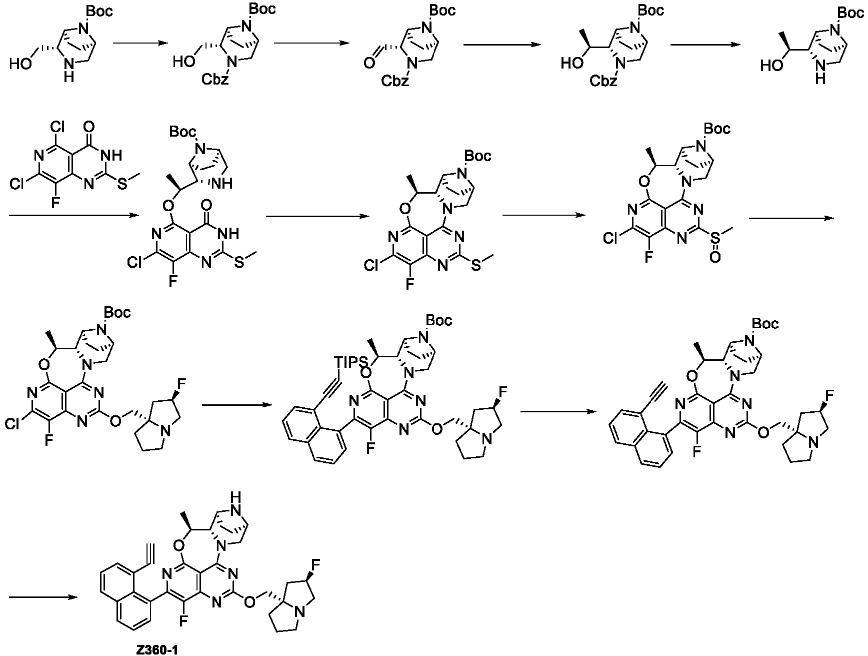

Step I: (1S,2S,5R)-t-butyl2-(hydroxymethyl)-3,8-diazacyclo[3.2.1]octane-8-carboxylate (3.0 g,12.40mmol) was dissolved in a mixed solution of ethyl acetate (30mL) and water (10mL). Benzyl chloroformate (2.54g, 14.88mmol) and sodium bicarbonate (2.98g, 32.0mmol) were added, and stirred overnight at room temperature. The reaction solution was added with water (50mL), extracted with ethyl acetate (100mLX3), washed with saturated brine (20mL), dried over anhydrous sodium sulfate, and concentrated. The crude product was purified by silica gel column chromatography (petroleum ether/ethyl acetate =2:1) to obtain 3-benzyl 8-(t-butyl) (1S,2S,5R)-2-(hydroxymethyl)-3,8-diazabicyclo[3.2.1]octane-3,8-dicarboxylate (4.35g, yield: 93.3%).

Step II: At -78°C and under a nitrogen atmosphere, a solution of dimethyl sulfoxide (156mg, 1.99mmol) in anhydrous dichloromethane(5.0mL) was slowly added dropwise to oxalyl chloride (223mg, 1.75mmol) in anhydrous dichloromethane (10mL), and stirred at this temperature for 15 min. A solution of 3-benzyl 8-(t-butyl)(1S,2S,5R)-2-(hydroxymethyl)-3,8-diazabicyclo [3.2.1]octane-3,8-dicarboxylate (300mg, 0.80mmol) in anhydrous dichloromethane (5.0mL) was slowly added dropwise, and reacted at -78°C for 40 min. Triethyl amine (646mg, 6.38mmol) was slowly added and reacted at -78°C for 1 hr. The reaction was quenched with saturated ammonium chloride (20mL). The reaction solution was extracted with ethyl acetate, dried over anhydrous sodium sulfate, and concentrated, to obtain crude 8-(t-butyl) 3-benzyl(1S,2S,5R)-2-formyl-3,8-diazabicyclo[3.2.1]octane-3,8-dicarboxylate (280mg, yield: 93.9%).

Step III: 8-(t-butyl) 3-benzyl (1S,2S,5R)-2-formyl-3,8-diazabicyclo[3.2.1]octane-3,8-dicarboxylate (280mg, 0.75mmol) was dissolved in dry tetrahydrofuran (5mL). After cooling to -78°C, a 3M methylmagnesium bromide solution in 2-methyltetrahydrofuran (0.3mL) was added dropwise under nitrogen atmosphere and reacted at -78°C for 2 hrs. A saturated ammonium chloride solution (5mL) was added. The reaction solution was extracted with ethyl acetate, dried over anhydrous sodium sulfate, concentrated, and purified by TLC (ethyl acetate /petroleum ether=1:2) to obtain 3-benzyl 8-(t-butyl)(1S,2S,5R)-2-((S)-1-hydroxyethyl)-3,8-diazabicyclo [3.2.1]octane-3,8-dicarboxylate (180mg, yield: 61.6%).

Step IV: 3-benzyl 8-(t-butyl) (1S,2S,5R)-2-((S)-1-hydroxyethyl)-3,8-diazabicyclo [3.2.1] octane-3,8-dicarboxylate (180mg, 0.46mmol) was dissolved in methanol (4mL) and water (0.5mL). Ammonium formate (290mg, 4.6mmol) and 10% palladium on carbon (20mg) was added and reacted at 60°C for 2 hrs while the tube was sealed. The reaction was filtered and concentrated. The residue was purified by preparative TLC (dichloromethane/methanol =10:1) to obtain t-butyl (15,2S,5R)-2-((S)-1-hydroxyethyl)-3,8-diazabicyclo[3.2.1]octane-8-carboxylate (100mg, yield: 85.5%).

Step V: At 0°C, a suspension of sodium hydride (60% dispersed in an oil) (41 mg, 0.58 mmol) in tetrahydrofuran (10 mL) was added with t-butyl (15,2S,5R)-2-((S)-1-hydroxyethyl)-3,8-diazabicyclo[3.2.1]octane-8-carboxylate (100 mg, 0.39 mmol) and stirred at this temperature for 10 min. 5,7-dichloro-8-fluoro-2-(methylthio)pyrido[4,3-d]pyrimidine4(3H)-one (112mg, 0.40 mmol) was added and stirred with heating 60°C for 1 hr. After the reaction was completed, the reaction was quenched with water and extracted with dichloromethane/methanol (10:1, 25 mLx3). The organic phase was dried over anhydrous sodium sulfate, filtered, and concentrated to obtain t-butyl (1S,25,5R)-2-((S)-1-((7-chloro-8-fluoro-2-(methylthio)-4-oxa-3,4-dihydropyrido[4,3-d] pyrimidine-5-yl)oxy)ethyl)-3,8-diazabicyclo[3.2.1]octane-8-carboxylate (160mg, yield: 82.0%). ES-API:[M+H]⁺=500.1.

Step VI: 1-propylphosphoric anhydride (50% solution in ethyl acetate) (265 mg, 0.42 mmol) was added to a solution of t-butyl (1S,2S,5R)-2-((S)-1-((7-chloro-8-fluoro-2-(methylthio)-4-oxa-3,4-dihydropyrido[4,3-d]pyrimidine-5-yl)oxy)ethyl)-3,8-diazabicyclo[3.2.1]octane-8-carboxylate (160mg, 0.32mmol) and N,N-diisopropylethylene diamine (82 mg, 0.64mmol) in dichloromethane (10mL) and stirred at room temperature for 3 hrs. The reaction was quenched with water and extracted with dichloromethane. The organic layer was dried over anhydrous sodium sulfate, filtered and concentrated. The obtained solid was suspended in acetonitrile and stirred at room temperature for 10 min. After filtration, a filter cake was collected, to obtain t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (100 mg, yield: 65.3%). ES-API:[M+H]⁺=482.1.

Step VII: At 0°C, m-chloroperoxybenzoic acid (40 mg, 0.23 mmol) was added to a solution of t-butyl (5S,5aS,6S,9R)-2-chloro-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (100 mg, 0.21mmol) in dichloromethane (15 mL), and stirred at room temperature for 1 hr. The reaction solution was diluted with dichloromethane (30mL) and washed with 10% sodium hydroxide solution (5.0mLx2). The organic phase was dried over anhydrous sodium sulfate and concentrated to obtain the crude product t-butyl (5S,5aS,6S,9R)-2-chloro-1-fluoro-5-methyl-12-(methylthionyl) -5a,6,7,8,9,10-hexahydro-5H-4-oxa-3,10a,11,13,14-pentaaza-6,9-methanonaphtho[1,8-ab]hepten-14-carboxylate (100mg, yield: 95.2%). ES-API:[M+H]⁺=498.1.

Step VIII: At 0°C, ((2R,7aS)-2-fluorotetrahydro-1H-pyrrolizin-7a(5H)-yl)methanol (40 mg, 0.28 mmol) was added to a solution of sodium hydride (60% dispersed in an oil) (15 mg, 0.36 mmol) in tetrahydrofuran. The reaction was stirred at room temperature for 10 min. After cooling to 0°C, a solution of t-butyl (5S,5aS,6S,9R)-2-chloro-1-fluoro-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (100 mg, 0.20 mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-chloro-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (50mg, yield: 42.4%). ES-API: [M+H]⁺=593.2.

Step IX: Potassium phosphate (34 mg, 0.16mmmol) and [n-butyldi(1-adamantanyl) phosphine](2-amino-1,1'-biphenyl-2-yl)palladium (II) mesylate (15 mg) were added to t-butyl (5S,5aS,6S,9R)-2-chloro-1-fluoro-12-((2R,7aS)-2-fluorotetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-carboxylate (50.0mg, 0.08mmol) and triisopropyl((8-(4,4,5,5-tetramethyl-1,3,2-dioxabenzaldehyde-2-yl)naphthalen-1-yl)ethynyl)silane (55mg, 0.13mmol) in tetrahydrofuran (6 mL) and water (1.0 mL). Nitrogen was bubbled therethrough and the reaction was continued at 60°C for 40 min. After the reaction, the reaction solution was extracted with ethyl acetate (30 mLx2) and saturated brine (10 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-1-fluoro-12-((2R,7aS)-2-fluorotetrahydrofuran-1H-pyrrolizin-7a(5H)-ylmethoxy)-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (45mg, yield: 65.2%). ES-API: [M+H]⁺=865.1.

Step X: Cesium fluoride (40mg, 0.26 mmol) was added to a solution of t-butyl (5S,5aS,6S,9R)-1-fluoro-12-((2R,7aS)-2-fluorotetrahydrofuran-1H-pyrrolizin-7a(5H)-ylmethoxy)-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]heptan-14-carboxylate (45mg, 0.052 mmol) in N,N-dimethylformamide (3.0mL). The reaction was carried out with stirring at room temperature for 0.5 hr. After the reaction, purification by preparative HPLC (trifluoroacetic acid method) afforded t-butyl (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (32mg, yield:86.5%). ES-API: [M+H]⁺=709.3.

Step XI: A reaction solution of t-butyl (5S,5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (32mg, 0.045mmol), acetonitrile (2.0mL), and 4M hydrochloric acid/dioxane solution (0.500mL, 2.000mmol) was reacted at 0°C for 1 hr. The reaction was concentrated, and the crude product was purified by preparative HPLC (formic acid method 1) to obtain (5S,5aS,6S,9R)-2-(8-ethynylnaphthalen-1-yl)-1-fluoro-12-((2R,7aS)-2-fluorotetrahydro-1H-pyrrolizin-7a(5H)-ylmethoxy)-5-methyl-5a,6,7,8,9,10-hexahydro-5H-4-oxy-3,10a,11,13,14-pentaaza-6,9-methanonaphtho[1,8-ab]heptene (Z360-1, 12mg, yield:44.4%, formate) as a yellow solid. ES-API:[M+1]⁺=609.3.

3-benzyl 8-(t-butyl) (15,2S,5R)-2-((S)-1-hydroxyethyl)-3,8-diazabicyclo[3.2.1]octane-3,8-dicarboxylate obtained in Step III was converted into Compound B, which was used to determine the absolute stereochemistry.

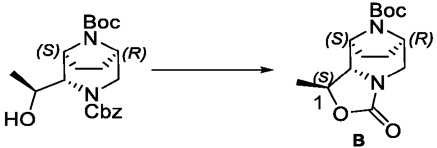

Under a nitrogen atmosphere, 1 M potassium tert-butoxide solution in tetrahydrofuran (0.62 mL, 0.62 mmol) was slowly added dropwise to a solution of 3-benzyl 8-(t-butyl)(1S,2S,5R)-2-((S)-1-hydroxyethyl)-3,8-diazabicyclo[3.2.1]octane-3,8-dicarboxylate (120 mg, 0.31 mmol) in tetrahydrofuran (26.0 mL) at 0°C. After reaction at 0°C for 1 hr, saturated ammonium chloride aqueous solution was slowly added. The reaction solution was extracted 3 times with ethyl acetate. The organic phases were combined, washed with saturated brine. The organic phase was dried over anhydrous sodium sulfate, and filtered. The filtrate was rotary evaporated to dryness under reduced pressure, and the crude product was slurried in petroleum ether and purified to obtain t-butyl (1*S*,6*R*,9*S*,9a*S*)-1-methyl-3-oxohexahydro-1H,3H-6,9-epiaminoazepin[3,4-a]aza-10-carboxylate (Compound B, 95 mg). ES-API: [M+H]⁺= 283.2. ¹H NMR (500 MHz, CDCl₃) δ 4.44 -3.98 (m, 3H), 3.67 -3.45 (m, 2H), 3.32 - 3.06 (m, 1H), 2.11 -1.96 (m, 1H), 1.87 (tdd, *J =* 22.1, 13.1, 4.8 Hz, 2H), 1.77 -1.68 (m, 1H), 1.48 (s, 9H), 1.45 (d, *J* = 6.3 Hz, 3H).¹³C NMR (126 MHz, CDCl₃) δ 157.7, 153.0, 80.6, 72.9, 63.3, 55.0, 53.5, 47.6, 28.4, 27.2, 22.4, 20.7.

### Single crystal cultivation

10 mg of crude Compound B obtained in the above step was weighed into a sample tube, dissolved in a small amount of ethyl acetate, and placed in a sealed sample bottle. At room temperature, the petroleum ether in the sample bottle slowly diffuses into the ethyl acetate solution of the compound, so as to obtain the desired single crystal. X-ray single crystal diffraction test was carried out by using Bruker D8 Venture instrument. The results are shown in Table 3 and Fig. 1. The absolute configuration of the substituent at position 1 of Compound B was confirmed as (S).

**Table 3**

| | |
|---|---|
| Empirical formula: | C14 H22 N2 O4 |
| Formula weight: | 282.33 |
| Temperature: | 213.00 K |
| Wavelength: | 1.34139 Å |
| Crystal system: | monoclinic |
| Space group: | C 1 2 1 |
| Unit cell dimensions: | a = 22.9206(4) Å a= 90° |
| b = 6.62200(3) Å | b= 103.4690(10)° |
| c = 10.0316(4) Å | g = 90° |
| Unit cell volume: | 1480.72(5) Å³ |
| Number of molecules in the unit cell: Z | 4 |
| Density (calculated): | 1.266 mg/m³ |
| Absorption coefficient: | 0.488 mm⁻¹ |
| Number of electrons in unit cell: F(000) | 608 |
| Crystal size: | 0.08 x 0.07 x 0.05 mm³ |
| Theta range for data collection: | 3.450 to 54.955° |
| Diffraction index range: | -27<=h<=27, -8<=k<=8, -12<=l<=12 |
| Diffractions collected: | 10113 |
| Independent diffractions: | 2791 [R(int) = 0.0501] |
| Completeness to theta = 53.594°: | 99.2% |
| Linear correction method (Absorption correction): | Semi-empirical absorption correction of equivalent diffraction points (Semi-empirical from equivalents) |
| Max. and min. transmission: | 0.7508 and 0.6140 |
| Refinement method: | Full matrix least squares refinement based on F² |
| Data / restraints / parameters: | 2791 / 1 / 185 |
| Goodness-of-fit on F²: | 0.993 |
| Final R indices [I>2sigma(I)]): | R1 = 0.0411, wR2 = 0.1220 |
| R indices (all data): | R1 = 0.0475, wR2 = 0.1292 |
| Absolute structure parameter: | 0.08(14) |
| Largest diff. peak and hole: | 0.206 and -0.206 e.Å⁻³ |

### Preparation Example 4: Synthesis of 3-benzyl 8-(t-butyl) (1S,2S,SR)-2-((R)-1-hydroxyethyl)-3,8-diazabicyclo[3.2.1]octane-3,8-dicarboxylate (A3)

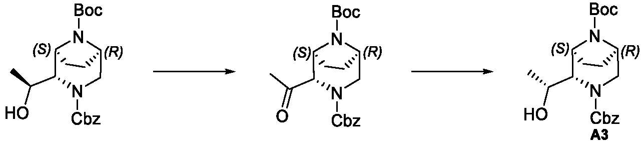

Step I: 3-benzyl 8-(t-butyl) (1S,2S,5R)-2-((S)-1-hydroxyethyl)-3,8-diazacyclo[3.2.1]octane-3,8-dicarboxylate (120 mg, 0.31 mmol) was dissolved in anhydrous dichloromethane (6 mL). At 0°C, Dess-Martin periodinane (196 mg, 0.46 mmol) was added, and reacted at room temperature for 2 hrs. The reaction was shown to be completed by LC/MS. The reaction solution was added with a saturated sodium thiosulphate solution (10 mL) and a saturated sodium bicarbonate (10 mL) aqueous solution, and extracted with ethyl acetate (20 mLX2). The organic phase was washed with saturated brine (20mL), dried over anhydrous sodium sulfate, filtered, and concentrated. The crude product was purified by flash silica gel column chromatography (0-25% ethyl acetate/petroleum ether) to obtain 3-benzyl 8-(t-butyl) (1S,2S,5R)-2-acetyl-3,8-diazacyclo[3.2.1]octane-3,8-dicarboxylate (100 mg, yield: 87%), ES-API: [M+H]⁺=389.2. HPLC (chromatographic column: X-Bridge C18, 4.6*150mm, 3.5um; mobile phase A: 10mmol/L ammonium bicarbonate aqueous solution, mobile phase B: acetonitrile; flow rate: 0.8mL/min; chromatographic condition: 5%-95% B/A, 15 min; column temperature: 40°C; retention time: 9.57 min).

Step II: 3-benzyl 8-(t-butyl) (1S,2S,5R)-2-acetyl-3,8-diazacyclo[3.2.1]octane-3,8-dicarboxylate (100 mg, 0.26 mmol) was dissolved in anhydrous ethanol (5 mL). At 0°C, a 2 M lithium borohydride solution in tetrahydrofuran (0.26 mL, 0.52 mmol) was added. The reaction was carried out at room temperature for 1 hr. The reaction was shown to be completed by LC/MS. The reaction solution was added with a saturated ammonium chloride aqueous solution (20 mL), and extracted with ethyl acetate (20 mLX2). The organic phase was washed with saturated brine (20mL), dried over anhydrous sodium sulfate, filtered, and concentrated. The crude product was purified by flash silica gel column chromatography (0-40% ethyl acetate/petroleum ether) to obtain 3-benzyl 8-(t-butyl) (1S,2S,5R)-2-((R)-1-hydroxyethyl)-3,8-diazacyclo[3.2.1]octane-3,8-dicarboxylate (A3, 80 mg, yield: 79.6%) as a colorless oil. ES-API: [M-55]⁺=335.2. HPLC (chromatographic column: X-Bridge C18, 4.6*150mm, 3.5um; mobile phase A: 10mmol/L ammonium bicarbonate aqueous solution, mobile phase B: acetonitrile; flow rate: 0.8mL/min; chromatographic condition: 5%-95% B/A, 15 min; column temperature: 40°C; retention time: 9.46 min).

### Example 15: Synthesis of 5-ethynyl-4-((5S,5aS,6S,9R)-1-fluoro-12-(((2R,7aS)-2-fluorotetrahydro-1H-pyrrolizin-7a(5H)-ylmethoxy)-5-methyl-5a,6,7,8,9,10-hexahydro-5H-4-oxy-3,10a,11,13,14-pentaaza-6,9-methanonaphtho[1,8-ab]hepten-2-yl)naphthalen-2-ol (Z384)

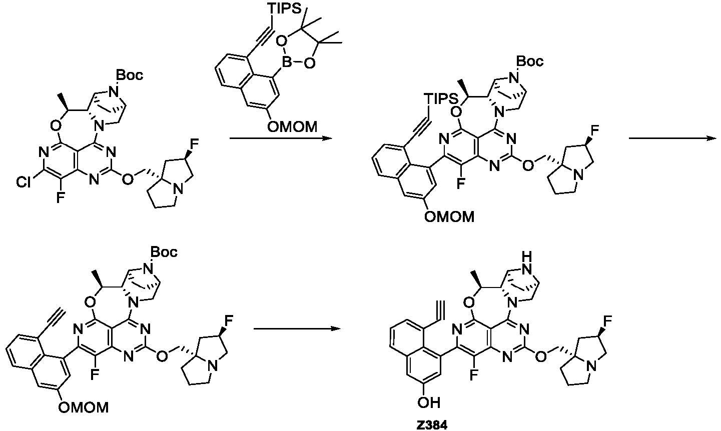

Step I: Potassium phosphate (34 mg, 0.16mmmol) and [n-butyldi(1-adamantanyl)phosphine](2-amino-1,1'-biphenyl-2-yl)palladium (II) mesylate (15 mg, 0.021mmol) were added to a solution of t-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40.0mg, 0.067mmol) and triisopropyl((6-(methoxymethoxy)-8-(4,4,5,5-tetramethyl-1,3,2-dioxaborolane -2-yl)naphthalen-1-yl)ethynyl)silane (55mg, 0.13mmol) in tetrahydrofuran (6 mL) and water (1.0 mL). Nitrogen was bubbled therethrough and the reaction was continued at 60°C for 40 min. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (methanol /dichloromethane=0-4%) to obtain t-butyl (5S,5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (40mg, yield: 64.5%). ES-API: [M+H]⁺=925.5.

Step II: Cesium fluoride (35mg, 0.22 mmol) was added to a solution of t-butyl (5S,5aS,6S,9R)-1-fluoro-12-(((2R,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5-methyl-5a,6,7,8,9,10-hexahydro-5H-4-oxy-3,10a,11,13,14-pentaaza-6,9-methanonaphtho[1,8-ab]hepten-14-carboxylate (40mg, 0.043 mmol) in N,N-dimethylformamide (3.0mL). The reaction was carried out with stirring at room temperature for 0.5 hr. After the reaction, the reaction solution was filtered, and the residue was purified by preparative HPLC (trifluoroacetic acid method) to obtain t-butyl (5S,5aS,6S,9R)-2-(8-ethynyl-3-(methoxymethoxy)naphthalen-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30mg, yield: 90.9%). ES-API: [M+H]⁺=769.3.

Step III: A reaction solution of t-butyl (5S,5aS,6S,9R)-2-(8-ethynyl-3-(methoxymethoxy) naphthalen-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30mg, 0.039 mmol) in acetonitrile (2.0mL) and 4M hydrochloric acid/dioxane solution (0.500mL, 2.000mmol) was reacted at 0°C for 1 hr. The reaction solution was concentrated, and the crude product was purified by preparative HPLC (formic acid method 1) to obtain 5-ethynyl-4-((5S,5aS,6S,9R)-1-fluoro-12-(((2R,7aS)-2-fluorotetrahydro-1H-pyrrolizin-7a(5H)-ylmethoxy)-5-methyl-5a,6,7,8,9,10-hexahydro-5H-4-oxa-3,10a,11,13,14-pentaaza-6,9-methanonaphtho[1,8-ab]hepten-2-yl)naphthalen-2-ol (Z384, 15mg, yield: 62.5%, formate) as a yellow solid. ES-API: [M+H]⁺=625.3. ¹H NMR (500 MHz, CD₃OD) δ 7.80 (t, *J* = 7.9 Hz, 1H), 7.51 (dd, *J =* 24.3, 6.9 Hz, 1H), 7.39 (dt, *J* = 11.6, 7.7 Hz, 1H), 7.31 (d, *J* = 2.2 Hz, 1H), 7.14 (dd, *J* = 52.3, 2.3 Hz, 1H), 5.49 (d, *J =* 52.1 Hz, 2H), 4.66 -4.42 (m, 3H), 4.21 (d, *J* = 6.5 Hz, 1H), 4.01 -3.56 (m, 5H), 3.35-3.07 (m, 3H), 2.71 -1.73 (m, 10H), 1.59 (t, *J* = 6.1 Hz, 3H).

### Example 16: Synthesis of 1-(8-((5aS,6S,9R)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-1-yl)propanol (Z385)

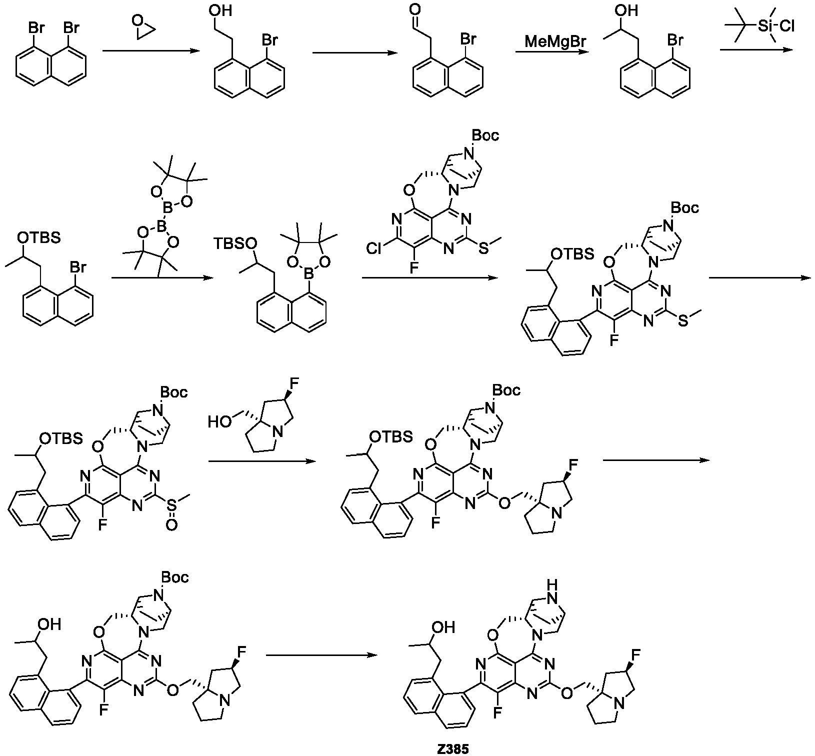

Step I: Under a nitrogen atmosphere, 1,8-dibromonaphthalene (4 g, 13.99 mmol) and tetrahydrofuran (40 mL) were added to a round-bottom flask. The solution was cooled to -70°C, and a 2.5 M butyl lithium solution in n-hexane (6.15 mL, 15.39 mmol) was added dropwise. After that, the reaction was stirred at -70°C for 30 hrs. A 3*M* ethylene oxide solution in tetrahydrofuran (28 mL, 84 mmol) was added dropwise. After that, the reaction was heated to 0°C, and stirred for 1 hr. Iced water (100 mL) was added. The reaction solution was extracted with ethyl acetate (50 mLX3). The organic phase was washed with saturated brine, dried over anhydrous sodium sulfate and concentrated to a obtain crude product. The crude product was purified by flash silica gel column chromatography (ethyl acetate/petroleum ether: 0-40%) to obtain 2-(8-bromonaphthalen-1-yl)ethan-1-ol (2 g, yield: 56.9%) as a yellow solid. ES-API: [M+Na]⁺=273.1.

Step II: 2-(8-bromonaphthalen-1-yl)ethan-1-ol (1.4 g, 5.75 mmol) and dichloromethane (30 mL) were added to a round-bottom flask. Under a nitrogen atmosphere, the reaction solution was cooled to 0°C. Dess-Martin periodinane (3.55 g, 8.36 mmol) was added to the reaction solution with stirring. The reaction was stirred at 0°C for 1 hr. A saturated sodium thiosulphate solution (50 mL) and a saturated sodium bicarbonate aqueous solution (50 mL) were added to the reaction solution, and stirred at room temperature for 5 min. The reaction solution was extracted with ethyl acetate (100 mLX3). The organic phase was washed with saturated brine, dried over anhydrous sodium sulfate and concentrated. The crude product was purified by flash silica gel column chromatography (ethyl acetate /petroleum ether: 0-20%) to obtain 2-(8-bromonaphthalen-1-yl)acetaldehyde (1.1 g, yield: 79.2%) as a yellow solid. ES-API: [M+H]⁺=249.1.

Step III: Under a nitrogen atmosphere, 2-(8-bromonaphthalen-1-yl)acetaldehyde (1.8 g, 7.23 mmol) and tetrahydrofuran (40 mL) were added to a reaction flask, and cooled to -30°C. A 3Mmethylmagnesium bromide solution in tetrahydrofuran (2.65mL, 7.95 mmol) was added dropwise to the reaction solution with stirring. The reaction was stirred at -20°C for 30 min. The ice bath was removed. The reaction solution was added with a saturated ammonium chloride aqueous solution (50 mL) and extracted with ethyl acetate (50 mLX3). The organic phase was washed with saturated brine, dried over anhydrous sodium sulfate, and concentrated. The crude product was purified by flash silica gel column chromatography (ethyl acetate /petroleum ether: 0-30%) to obtain 1-(8-bromonaphthalen-1-yl)propan-2-ol (1.4 g, yield: 73%). ES-API: [M+H]⁺=287.0.

Step IV: t-butyldimethylchlorosilane (1.03 g, 6.86 mmol) was added to a solution of 1-(8-bromonaphthalen-1-yl)propan-2-ol (1.4 g, 5.28 mmol) and imidazole(0.719 g, 10.56 mmol) in anhydrous N,N-dimethylformamide (15 mL) in a reaction flask. The reaction was carried out with stirring at room temperature for 2 hrs. The reaction solution was added with a saturated ammonium chloride aqueous solution (100 mL) and extracted with ethyl acetate (50 mLX2). The organic phase was washed with saturated brine, dried over anhydrous sodium sulfate, and concentrated. The crude product was purified by flash silica gel column chromatography (ethyl acetate /petroleum ether: 0-15%) to obtain ((1-(8-bromonaphthalen-1-yl)propan-2-yl)oxy)(t-butyl)dimethylsilane (1.9 g, yield: 94.8%). ES-API: [2M+Na]⁺=779.5.

Step V: ((1-(8-bromonaphthalen-1-yl)propan-2-yl)oxy)(t-butyl)dimethylsilane (1.8 g, 4.74 mmol), bis(pinacolato)diboron (2.41 g, 9.49 mmol), [1,1'-bis[1,1'- bis (diphenylphosphino) ferrocene] palladium dichloride (347 mg, 0.47 mmol), potassium acetate (1.40 g, 14.23 mmol), and 1,4-dioxane (30 mL) were added to a round-bottom flask. Under a nitrogen atmosphere, the system was stirred with heating in an oil bath at 95°C for 12 hrs. The reaction solution was added with water (50 mL), and extracted with ethyl acetate (30 mLX3). The organic phase was dried over anhydrous sodium sulfate and concentrated. The crude product was purified by flash silica gel column chromatography (ethyl acetate /petroleum ether: 0-10%) to obtain t-butyl dimethyl ((1-(8-(4,4,5,5-tetramethyl-1,3,2-dioxaborolane -2-yl)naphthalen-1-yl)propan-2-yl)oxy)silane (1.3 g, crude product) as a yellow oil. ES-API: [M+Na]⁺=449.4.

Step VI: t-butyldimethyl((1-(8-(4,4,5,5-tetramethyl-1,3,2-dioxaborolane -2-yl)naphthalen-1-yl)propan-2-yl)oxy)silane (260 mg, 0.43 mmol), t-butyl (5aS,6S,9R)-2-chloro-1-fluoro-12-(methylthio)-5a,6,7,8,9,10-hexahydro-5H-4-oxa-3,10a,11,13,14-pentaaza-6,9-methanonaphtho [1,8-ab]hepten-14-carboxylate (80 mg, 0.17 mmol), [n-butyldi(1-adamantanyl)phosphine](2-amino-1,1'-biphenyl-2-yl)palladium (II) mesylate (12.4 mg, 0.017 mmol), potassium phosphate (109 mg, 0.51 mmol), tetrahydrofuran (5 mL), and water (1 mL) were added to a round-bottom flask. Under a nitrogen atmosphere, the system was heated in a microwave reactor at 80°C for 40 min. The reaction solution was added with water (30 mL), and extracted with ethyl acetate (30 mLX3). The organic phase was dried over anhydrous sodium sulfate and concentrated. The crude product was purified by flash silica gel column chromatography (ethyl acetate /petroleum ether: 0-30%) to obtain t-butyl (5aS,6S,9R)-2-(8-(2-((t-butyldimethylsilyl)oxy)propyl)naphthalen-1-yl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (100 mg, yield: 79.9%) as a yellow solid. ES-API: [M+H]⁺=732.3.

Step VII: t-butyl (5aS,6S,9R)-2-(8-(2-((t-butyldimethylsilyl)oxy)propyl)naphthalen-1-yl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (100 mg, 0.14 mmol) and dichloromethane (8 mL) were added to a round-bottom flask. The reaction solution was cooled to 0°C, and m-chloroperoxybenzoic acid (36 mg, 0.20 mmol) was added. The reaction was stirred at 0°C for 1 hr. The reaction solution was added with water (30 mL), and extracted with ethyl acetate (30 mLX3). The organic phase was dried over anhydrous sodium sulfate and concentrated. The crude product was purified by flash silica gel column chromatography (ethyl acetate /petroleum ether: 0-100%) to obtain t-butyl (5aS,6S,9R)-2-(8-(2-((t-butyldimethylsilyl)oxy)propyl)naphthalen-1-yl)-1-fluoro-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, yield: 97.8%) as a yellow solid. ES-API: [M+H]⁺=748.3.

Step VIII: Sodium hydride (16 mg, 0.40 mmol) and tetrahydrofuran (5 mL) were added to a round-bottom flask. The reaction solution was cooled to 0°C, and ((2R,7aS)-2-fluorotetrahydro-1H-pyrrolizin-7a(5H)-yl)methanol (64 mg, 0.40 mmol) was added. The reaction was stirred at 0°C for 10 min. A solution of t-butyl (5aS,6S,9R)-2-(8-(2-((t-butyldimethylsilyl)oxy) propyl)naphthalen-1-yl)-1-fluoro-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, 0.13 mmol) in tetrahydrofuran (2 mL) was added dropwise. After that, the reaction was stirred at 0°C for 30 hrs. The reaction solution was added with water (30 mL), and extracted with ethyl acetate (30 mLX3). The organic phase was dried over anhydrous sodium sulfate and concentrated. The crude product was purified by TLC (ethyl acetate) to obtain t-butyl (5aS,6S,9R)-2-(8-(2-((t-butyldimethylsilyl)oxy)propyl)naphthalen-1-yl)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (20 mg, yield: 17.7%), ES-API: [M+H]⁺=843.3.

Step IX: T-butyl (5aS,6S,9R)-2-(8-(2-((t-butyldimethylsilyl)oxy)propyl)naphthalen-1-yl)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (20 mg, 0.024 mmol), tetrabutylammonium fluoride (1 mL, 1 mmol, 1Msolution in tetrahydrofuran), and tetrahydrofuran (1 mL) were added to a round-bottom flask. The reaction was stirred at room temperature for 24 hrs. The reaction solution was added with water (30 mL), and extracted with ethyl acetate (30 mLX2). The organic phase was washed 3 times with saturated brine. The organic phase was dried over anhydrous sodium sulfate and concentrated to obtain t-butyl (5aS,6S,9R)-1-fluoro-12-((2R,7aS)-2-fluorotetrahydro-1H-pyrrolizin-7a(5H)-ylmethoxy)-2-(8-(2-hydroxypropyl)naphthalen-1-yl)-5a,6,7,8,9,10-hexahydro-5H-4-oxa-3,10a,11,13,14-pentaaza-6,9methanonaphtho[1,8-ab]hepten-14-carboxylate (16 mg, crude product), which was directly used in the next step of reaction. ES-API: [M+H]⁺=729.3.

Step X: T-butyl (5aS,6S,9R)-1-fluoro-12-((2R,7aS)-2-fluorotetrahydro-1H-pyrrolizin-7a(5H)-ylmethoxy)-2-(8-(2-hydroxypropyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (16 mg, 0.022 mmol) and acetonitrile (1 mL) were added to a round-bottom flask. A 4M hydrogen chloride/dioxane solution (1 mL) was added. The reaction was stirred at room temperature for 1 hrs. The reaction solution was concentrated to dryness, and the crude product was purified by preparative HPLC (ammonium bicarbonate method 1) to obtain 1-(8-((5aS,6S,9R)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-1-yl)propanol (Z385, 6 mg, yield: 43.4%) as a white solid. ES-API:[M+H]⁺=629.3.

### Example 17: Synthesis of 4-((5S,5aS,6S,9R)-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-2-yl)-5-ethynylnaphthalen-2-ol (Z386)

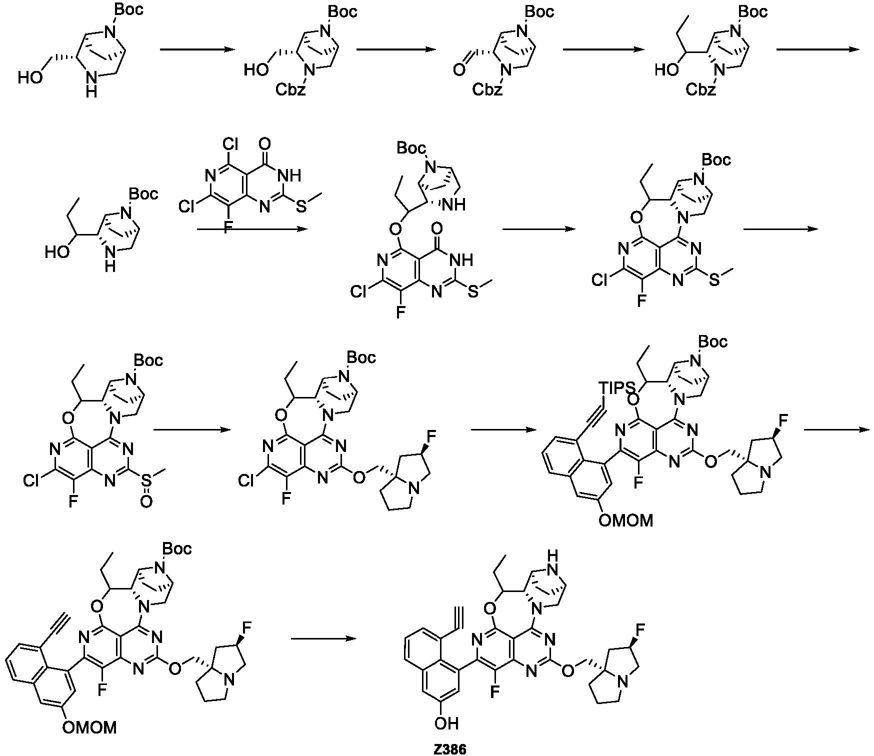

Step I: (1S,2S,5R)-t-butyl2-(hydroxymethyl)-3,8-diazacyclo[3.2.1]octane-8-carboxylate (3.0 g,12.40mmol) was dissolved in a mixed solution of ethyl acetate (30mL) and water (10mL). Benzyl chloroformate (2.54g, 14.88mmol) and sodium bicarbonate (2.98g, 32.0mmol) were added, and stirred overnight at room temperature. The reaction solution was added with water (50mL), extracted with ethyl acetate (100mLX3), washed with saturated brine (20mL), dried over anhydrous sodium sulfate, and concentrated. The crude product was purified by silica gel column chromatography (petroleum ether/ethyl acetate =2:1) to obtain 3-benzyl 8-(t-butyl) (1S,2S,5R)-2-(hydroxymethyl)-3,8-diazabicyclo[3.2.1]octane-3,8-dicarboxylate (4.35g, yield: 93.3%).

Step II: At -78°C and under a nitrogen atmosphere, a solution of dimethyl sulfoxide (156mg, 1.99mmol) in anhydrous dichloromethane(5.0mL) was slowly added dropwise to oxalyl chloride (223mg, 1.75mmol) in anhydrous dichloromethane (10mL), and stirred at this temperature for 15 min. A solution of 3-benzyl 8-(t-butyl)(1S,2S,5R)-2-(hydroxymethyl)-3,8-diazabicyclo [3.2.1]octane-3,8-dicarboxylate (300mg, 0.80mmol) in anhydrous dichloromethane (5.0mL) was slowly added dropwise, and reacted at -78°C for 40 min. Triethyl amine (646mg, 6.38mmol) was slowly added and reacted at -78°C for 1 hr. The reaction was quenched with saturated ammonium chloride (20mL). The reaction solution was extracted with ethyl acetate, dried over anhydrous sodium sulfate, and concentrated, to obtain crude 8-(t-butyl) 3-benzyl (1S,2S,5R)-2-formyl-3,8-diazabicyclo[3.2.1]octane-3,8-dicarboxylate (270mg, yield: 90.2%).

Step III: 8-(t-butyl) 3-benzyl (1S,2S,5R)-2-formyl-3,8-diazabicyclo[3.2.1]octane-3,8-dicarboxylate (270mg, 0.72mmol) was dissolved in dry tetrahydrofuran (5mL). After cooling to -78°C, a 3M ethylmagnesium bromide solution in ether (0.4mL) was added dropwise under nitrogen atmosphere and reacted at -78°C for 2 hrs. A saturated ammonium chloride solution (5mL) was added. The reaction solution was extracted with ethyl acetate, dried over anhydrous sodium sulfate, and concentrated. The crude product was purified by TLC (ethyl acetate/petroleum ether=1:3) to obtain 3-benzyl 8-(t-butyl) (1S,2S,5R)-2-((S)-1-hydroxyethyl)-3,8-diazabicyclo[3.2.1]octane-3,8-dicarboxylate (180mg, yield: 62.1%).

Step IV: 3-benzyl 8-(t-butyl) (1S,2S,5R)-2-((S)-1-hydroxypropyl)-3,8-diazabicyclo [3.2.1] octane-3,8-dicarboxylate (180mg, 0.45mmol) was dissolved in methanol (4mL) and water (0.5mL). Ammonium formate (290mg, 4.6mmol) and 10% palladium on carbon (20mg) were added, and reacted at 60°C for 2 hrs while the tube was sealed. The reaction was filtered and concentrated. The crude product was purified by preparative TLC (dichloromethane/methanol =10:1) to obtain t-butyl (1S,2S,5R)-2-((S)-1-hydroxypropyl)-3,8-diazabicyclo[3.2.1]octane-8-carboxylate (85mg, yield: 70.0%).

Step V: At 0°C, t-butyl (1S,2S,5R)-2-((S)-1-hydroxypropyl)-3,8-diazabicyclo[3.2.1]octane-8-carboxylate (85 mg, 0.31 mmol) was added to a suspension of sodium hydride (60% dispersed in an oil) (40 mg, 1.0 mmol) in tetrahydrofuran(10 mL) and stirred at this temperature for 10 min. 5,7-dichloro-8-fluoro-2-(methylthio)pyrido[4,3-d]pyrimidine4(3H)-one (112mg, 0.40 mmol) was added and stirred with heating 60°C for 1 hr. After the reaction was completed, the reaction was quenched with water and extracted with dichloromethane/methanol (10:1, 25mLX3). The organic phase was dried over anhydrous sodium sulfate, filtered, and concentrated, to obtain t-butyl (1S,2S,5R)-2-((S)-1-((7-chloro-8-fluoro-2-(methylthio)-4-oxa-3,4-dihydropyrido[4,3-d]pyrimidine-5-yl)oxy)propyl)-3,8-diazabicyclo[3.2.1]octane-8-carboxylate (120mg, yield: 75.5%). ES-API: [M+H]⁺=514.2.

Step VI: Propylphosphoric anhydride (50% ethyl acetate )(280 mg, 0.46 mmol) was added to a solution of t-butyl (1S,2S,5R)-2-((S)-1-((7-chloro-8-fluoro-2-(methylthio)-4-oxo-3,4-dihydropyrido[4,3-d]pyrimidine-5-yl)oxy)propyl)-3,8-diazabicyclo[3.2.1]octane-8-carboxylate (120mg, 0.23mmol) and N,N-diisopropylethylene diamine (85 mg, 0.69mmol) in dichloromethane(10mL) and stirred at room temperature for 5 hrs. The reaction was quenched with water and extracted with dichloromethane. The organic phase was dried over anhydrous sodium sulfate, filtered, and concentrated, to obtain a crude product, which was purified by silica gel column chromatography (petroleum ether/ethyl acetate =3:1) to obtain t-butyl (5S,5aS,6S,9R)-2-chloro-1-fluoro-5-ethyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, yield: 70.1%). ES-API: [M+H]⁺=496.2.

Step VII: At 0°C, m-chloroperoxybenzoic acid (36 mg, 0.20 mmol) was added to a solution of t-butyl (5S,5aS,6S,9R)-2-chloro-1-fluoro-5-ethyl-12-(methylthio)-5a,6,7,8,9,10-hexahydro-5H-4-oxa-3,10a,11,13,14-pentaaza-6,9-methanonaphtho[1,8-ab]hepten-14-carboxylate(80 mg, 0.16mmol) in dichloromethane (15 mL). The reaction was carried out with stirring at room temperature for 1 hr. The reaction solution was diluted with dichloromethane (30mL) and washed with 10% hydroxide solution (5.0mLX2). The organic phase was dried over anhydrous sodium sulfate and concentrated to obtain the crude product t-butyl (5S,5aS,6S,9R)-2-chloro-1-fluoro-5-ethyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (78mg, yield: 95.2%). ES-API: [M+H]⁺=512.2.

Step VIII: At 0°C, ((2R,7aS)-2-fluorotetrahydro-1H-pyrrolizin-7a(5H)-yl)methanol (40 mg, 0.28 mmol) was added to a solution of sodium hydride (60% dispersed in an oil) (15 mg, 0.36 mmol) in tetrahydrofuran. The reaction was stirred at room temperature for 10 min. After cooling to 0°C, a solution of t-butyl (5S,5aS,6S,9R)-2-chloro-1-fluoro-5-ethyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (80 mg, 0.16 mmol) in tetrahydrofuran (5 mL) was added. The reaction was stirred at 0°C for 1 hr. The reaction was quenched with a saturated sodium bicarbonate solution and extracted with ethyl acetate. The organic phase was washed with saturated brine, dried over anhydrous sodium sulfate, filtered, and concentrated. The crude product was purified by silica gel column chromatography (methanol /dichloromethane=0-5%) to obtain t-butyl (5S,5aS,6S,9R)-2-chloro-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (40mg, yield: 41.1%). ES-API: [M+H]⁺=607.3.

Step IX: Potassium phosphate (34 mg, 0.16mmmol) and [n-butyldi(1-adamantanyl) phosphine](2-amino-1,1'-biphenyl-2-yl)palladium (II) mesylate (15 mg, 0.021mmol) were added to t-butyl (5S,5aS,6S,9R)-2-chloro-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-14-carboxylate (40.0mg, 0.066mmol) and triisopropyl((8-(4,4,5,5-tetramethyl-1,3,2-dioxabenzaldehyde-2-yl)naphthalen-1-yl)ethynyl)silane (55mg, 0.13 mmol) in tetrahydrofuran (6 mL) and water (1.0 mL). Nitrogen was bubbled therethrough, and the reaction was continued at 60°C for 40 min. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (methanol /dichloromethane=0-4%) to obtain t-butyl (5S,5aS,6S,9R)-5-ethyl-1-fluoro-12-(((2R,7aS)-2-fluorotetrahydro-1H-pyrrolizin-7a(5H)-yl)methoxy)-2-(3-(methoxymethoxy)-8-((triisopropylsilyl) ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (38mg, yield: 61.3%). ES-API: [M+H]⁺=939.5.

Step X: Cesium fluoride (40mg, 0.26 mmol) was added to a solution of t-butyl (5S,5aS,6S,9R)-5-ethyl-1-fluoro-12-(((2r,7aS)-2-fluorotetrahydro-1H-pyrrolizin-7a(5H)-yl)methoxy)-2-(3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (38mg, 0.040 mmol) in N,N-dimethylformamide (3.0mL). The reaction was carried out with stirring at room temperature for 0.5 hr. After the reaction, the reaction solution was filtered, and the residue was purified by preparative HPLC (trifluoroacetic acid method) to obtain t-butyl (5S,5aS,6S,9R)-5-ethyl-2-(8-ethynyl-3-(methoxymethoxy)naphthalen-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (25mg, yield: 79.0%). ES-API: [M+H]⁺=783.3.

Step XI: A reaction soltion of t-butyl (5S,5aS,6S,9R)-5-ethyl-2-(8-ethynyl-3-(methoxymethoxy)naphthalen-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (25mg, 0.032 mmol) in acetonitrile (2.0mL) and 4M hydrochloric acid/dioxane solution (0.500mL, 2.000mmol) was reacted at 0°C for 1 hr. The reaction solution was concentrated, and the crude product was purified by preparative HPLC (formic acid method 1) to obtain 4-((5S,5aS,6S,9R)-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-ol (Z386, 8mg, yield: 40.0%, formate) as a yellow solid. ES-API: [M+H]⁺=639.3. ¹H NMR (400 MHz, CD₃OD) δ 8.44 (s, 2H), 7.81 (d, *J =* 5.2 Hz, 1H), 7.51 (dd, *J =* 14.2, 6.4 Hz, 1H), 7.39 (dd, *J =* 15.1, 6.9 Hz, 1H), 7.31 (s, 1H), 7.12 (dd, *J =* 41.3, 2.4 Hz, 1H), 5.60 -5.36 (m, 2H), 4.63 -4.47 (m, 2H), 4.48 -4.23 (m, 2H), 4.00 -3.44 (m, 5H), 3.39 (s, 0.49H), 3.35-3.30 (m, 2H), 3.08 (s, 0.51H), 2.73 -1.57 (m, 12H), 1.22 -1.10 (m, 3H).

### Example 18: Synthesis of (5S,5aS,6S,9R)-5-ethyl-2-(8- alkynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z387)

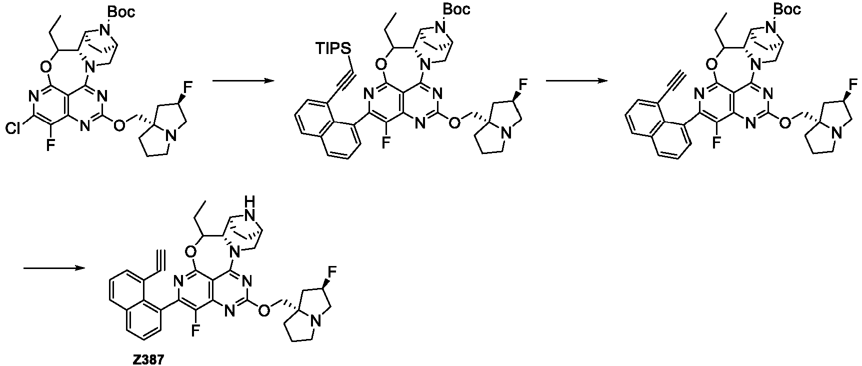

Step I: Potassium phosphate (34 mg, 0.16mmmol) and [n-butyldi(1-adamantanyl) phosphine](2-amino-1,1'-biphenyl-2-yl)palladium (II) mesylate (15 mg, 0.021mmol) were added to t-butyl (5S,5aS,6S,9R)-2-chloro-5-ethyl-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (40.0mg, 0.066mmol) and triisopropyl((8-(4,4,5,5-tetramethyl-1,3,2-dioxaborolane -2-yl)naphthalen-1-yl)ethynyl)silane (55mg, 0.13mmol) in tetrahydrofuran (6 mL) and water (1.0 mL). Nitrogen was bubbled therethrough and the reaction was continued at 60°C for 40 min. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (methanol /dichloromethane=0-4%) to obtain t-butyl (5S,5aS,6S,9R)-5-ethyl-1-fluoro-12-(((2R,7aS)-2-fluorotetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (40mg, yield: 69.0%).

Step II: Cesium fluoride (40mg, 0.23 mmol) was added to a solution of t-butyl (5S,5aS,6S,9R)-5-ethyl-1-fluoro-12-(((2R,7aS)-2-fluorotetrahydro-1H-pyrrolizin-7a(5H)-yl)methoxy)-2-(8-((triisopropylsilyl)ethyryl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (40mg, 0.046 mmol) in N,N-dimethylformamide (3.0mL). The reaction was carried out with stirring at room temperature for 0.5 hr. After the reaction, the reaction solution was filtered, and the residue was purified by preparative HPLC (trifluoroacetic acid method) to obtain t-butyl (5S,5aS,6S,9R)-5-ethyl-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (25mg, yield: 75.7%).

Step III: A reaction solution of t-butyl (5S,5aS,6S,9R)-5-ethyl-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (25mg, 0.034 mmol) in acetonitrile (2.0mL) and 4M hydrochloric acid/dioxane solution (0.500mL, 2.000mmol) was reacted at 0°C for 1 hr. The reaction solution was concentrated, and the crude product was purified by preparative HPLC (formic acid method 1) to obtain (5S,5aS,6S,9R)-5-ethyl-2-(8-alkynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z387, 10mg, yield: 46.5%, formate) as a yellow solid. ES-API: [M+H]⁺=623.2. ¹H NMR (400 MHz, CD₃OD) δ 8.36 (s, 2H), 8.15 -7.96 (m, 2H), 7.81 -7.35 (m, 4H), 5.50 (dd, *J =* 33.0, 19.1 Hz, 2H), 4.69 -4.28 (m, 4H), 4.00-3.65 (m, 5H), 3.45 (s, 0.55H), 3.42 -3.34 (m, 2H), 3.16 (s, 0.46H), 2.65 -1.79 (m, 12H), 1.17 (t, *J =* 6.7 Hz, 3H).

### Example 19: Synthesis of (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptene (Z388)

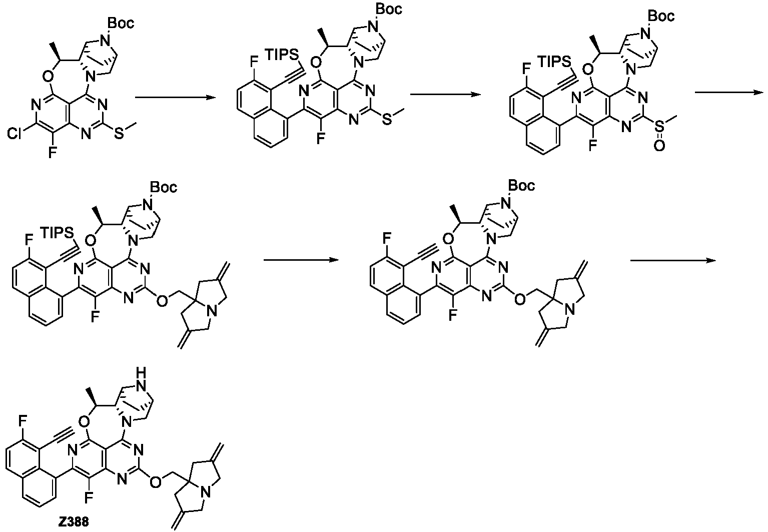

Step I: Under a nitrogen atmosphere, t-butyl (5S,5aS,6S,9R)-2-chloro-1-fluoro-5-methyl-12-(methylthio)-5a,6,7,8,9,10-hexahydro-5H-4-oxo-3,10a,11,13,14-pentaaza-6,9-methanonaphtho [1,8-ab]hepten-14-carboxylate (150mg, 0.311 mmol), ((2-fluoro-8-(4,4,5,5-tetramethyl-1,3,2-dioxaborolane -2-yl)naphthalen-1-yl)ethynyl)triisopropylsilane (211 mg, 0.466mmol), potassium phosphate (132 mg, 0.62 mmol) and [n-butyldi(1-adamantanyl)phosphine](2-amino-1,1'-biphenyl-2-yl)palladium (II) mesylate (23 mg, 0.031 mmol) were dissolved in tetrahydrofuran (6.0mL) and water (1.5 mL). The reaction mixture was heated to 80°C under microwave and stirred for 40 min. The reaction was shown to be completed by LC/MS. The reaction solution was concentrated under reduced pressure. The crude product was purified by silica gel column chromatography (dichloromethane: methanol =20:1) to obtain t-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (290 mg, crude product). ES-API: [M+H]⁺= 772.3.

Step II: T-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (290 mg, crude product) was dissolved in dichloromethane(10.0 mL). In an iced water bath, m-chloroperoxybenzoic acid (70 mg, 0.406mmol) was added, and reacted for 30 min in the iced water bath. The reaction was shown to be completed by LC/MS. The reaction solution was diluted with dichloromethane (20 mL), washed with saturated sodium sulfite solution (15 mL X 2) and saturated brine (15 mL X 3), dried over anhydrous sodium sulfate, filtered, and concentrated to obtain the crude product t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (280 mg, crude product). ES-API: [M+H]⁺= 788.3.

Step III: (2,6-dimethylenetetrahydro-1H-pyrrolizin-7a(5H)-yl)methanol (175mg, 1.059 mmol) was dissolved in anhydrous tetrahydrofuran (15mL). In an iced water bath, sodium hydride (85mg, 2.118 mmol) was added, and reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxo-3,10a,11,13,14-pentaaza-6,9-methanonaphtho[1,8-ab]hepten-14-carboxylate (280 mg, crude product) in tetrahydrofuran (5 mL) was added dropwise to the reaction solution, and reacted at room temperature for 30 min. The reaction was shown to be completed by LC/MS. The reaction solution was diluted with ethyl acetate (80 mL), washed with saturated ammonium chloride solution (15 mLX2) and saturated brine (60 mL X 3), dried over anhydrous sodium sulfate, filtered, and concentrated to obtain the crude product t-butyl (5S,5aS,6S,9R)-12-((2,6-dimethylenetetrahydro-1H-pyrrolizin-7a(5H)-yl)methoxy)- 1-fluoro-2-(7-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]heptanoate (460 mg, crude product). ES-API: [M+H]⁺= 889.3.

Step IV: Cesium fluoride (900mg, 5.921 mmol) was added to t-butyl (5S,5aS,6S,9R)-12-((2,6-dimethylenetetrahydro-1H-pyrrolizin-7a(5H)-yl)methoxy)- 1-fluoro-2-(7-fluoro-8-((triisopropylsilyl)ethynyl)naphthalen-1-yl)-5-methyl-5a,6,7,8,9,10-hexahydro-4-oxo-3,10a,11,13,14-pentaaza-6,9-methanonaphtho[1,8-ab]hepten-14-carboxylate (460 mg, crude product) in N,N-dimethylformamide (10 mL), and the reaction mixture was stirred at room temperature for 2 hrs. The reaction was shown to be completed by LC/MS. The reaction solution was diluted with ethyl acetate (60 mL), washed with water (15 mLX2) and saturated brine (60mLX3), dried over anhydrous sodium sulfate, filtered, and concentrated to obtain t-butyl (5S,5aS,6S,9R)-12-((2,6-dimethylenetetrahydro- 1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (480mg, crude product). ES-API: [M+H]⁺= 733.3.

Step V: In an iced water bath, 4 M hydrochloric acid-dioxane solution (5 mL) was slowly added to a solution of t-butyl (5S,5aS,6S,9R)-12-((2,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (480mg, crude product) in acetonitrile (10.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-12-((2,6-dimethylenetetrahydro- 1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxo-3,10a,11,13,14-pentaaza-6,9-methanonaphtho[1,8-ab]heptene (Z388, 36mg, total yield of 5 steps: 18.2%) as a light yellow solid. ES-API: [M+H]⁺= 633.3. ¹H NMR (500 MHz, CD₃OD) δ 8.13 -8.03 (m, 2H), 7.68 -7.54 (m, 2H), 7.43 (dt, *J =* 11.4, 8.9 Hz, 1H), 5.35 (dd, *J =* 13.3, 2.6 Hz, 1H), 5.02 (d, *J =* 8.3 Hz, 4H), 4.54 (dq, *J =* 12.9, 6.3 Hz, 1H), 4.34 -4.24 (m, 2H), 4.07 (d, *J =* 8.8 Hz, 1H), 3.67 (d, *J* = 5.8 Hz, 1H), 3.59 (s, 1H), 3.50 (s, 0H), 3.35 (s, 1H), 3.32 (s, 1H), 3.17 (t, *J =* 12.8 Hz, 1H), 2.79 (dd, *J=* 16.6, 7.6 Hz, 2H), 2.59 (d, *J =* 16.3 Hz, 2H), 2.09 -1.99 (m, 1H), 1.91 -1.73 (m, 3H), 1.57 (dd, *J =* 9.8, 6.4 Hz, 3H), 1.28 (s, 1H).

### Example 20: Synthesis of (5S,5aS,65,9R)-12-((2,6-dimethyltetrahydro-1H-pyrrolizin-7a(5H)-methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptene (Z550)

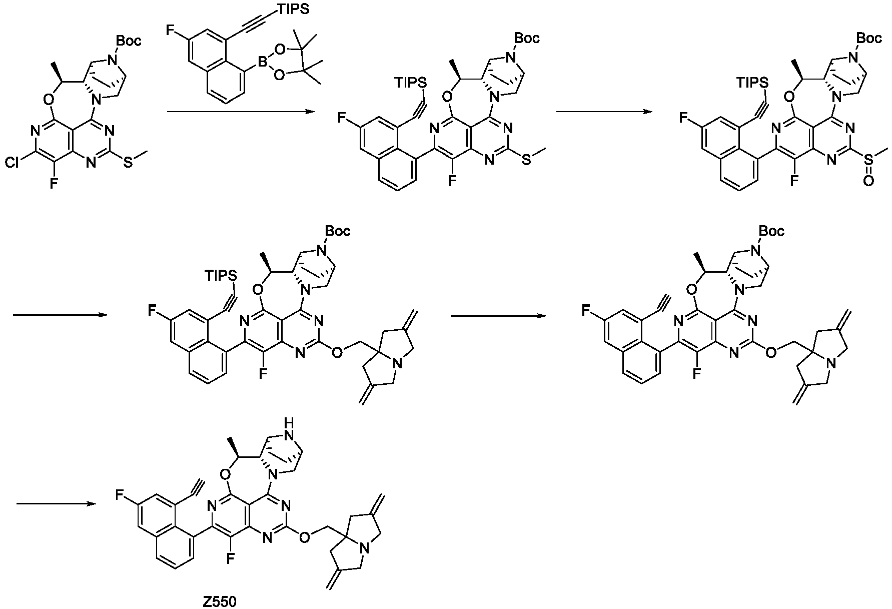

Step I: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (100 mg, 0.21 mmol), ((3-fluoro-8-(4,4,5,5-tetramethyl-1,3,2-dioxaborolane -2-yl)naphthalen-1-yl)ethynyl)triisopropylsilane (234 mg, 0.52 mmol), potassium phosphate (132 mg, 0.62 mmol), and [n-butyldi(1-adamantanyl)phosphine](2-amino-1,1'-biphenyl-2-yl)palladium (II) mesylate (23 mg, 0.031 mmol) were dissolved in tetrahydrofuran (5 mL) and water (1 mL). The reaction mixture was stirred at 80°C under micowave for 40 min. The reaction was shown to be completed by LC/MS. The reaction solution was concentrated under reduced pressure. The crude product was purified by silica gel column chromatography (dichloromethane: methanol =20:1) to obtain t-butyl (5S,5aS,6S,9R)-1-fluoro-2-(6-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (128 mg, yield: 80%). ES-API: [M+H]⁺= 772.3.

Step II: T-butyl (5S,5aS,6S,9R)-1-fluoro-2-(6-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (128 mg, 0.17 mmol) was dissolved in dichloromethane (5 mL). In an iced water bath, m-chloroperoxybenzoic acid (34 mg, 0.20 mmol) was added, and reacted at room temperature for 30 min. The reaction was shown to be completed by LC/MS. The reaction solution was diluted with dichloromethane (20 mL), washed with saturated sodium sulfite solution (15 mL X 2) and saturated brine (15 mL X 3), dried over anhydrous sodium sulfate, filtered, and concentrated to obtain the crude product t-butyl (5S,5aS,6S,9R)-1-fluoro-2-(6-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (170 mg, yield: 100%). ES-API: [M+H]⁺= 788.3.

Step III: (2,6-dimethylenetetrahydro-1H-pyrrolizin-7a(5H)-yl)methanol (36 mg, 0.21 mmol) was dissolved in anhydrous tetrahydrofuran (5 mL). In an iced water bath, sodium hydride (22 mg, 0.54 mmol) was added, and reacted for 30 min in the iced water bath. A solution of t-butyl (5S,5aS,6S,9R)-1-fluoro-2-(6-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (170 mg, 0.21 mmol) in tetrahydrofuran (5 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX2) and saturated brine (15 mLX 3), dried over anhydrous sodium sulfate, filtered, and concentrated to obtain the crude product t-butyl (5S,5aS,6S,9R)-12-((2,6-dimethylenetetrahydro-1H-pyrrolizin-7a(5H)-yl)methoxy)-1-fluoro-2-(6-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]hepten-14-carboxylate (165 mg, yield: 86%). ES-API: [M+H]⁺= 889.3.

Step IV: Cesium fluoride (282 mg, 1.86 mmol) was added to t-butyl (5S,5aS,6S,9R)-12-((2,6-dimethylenetetrahydro-1H-pyrrolizin-7a(5H)-yl)methoxy)-1-fluoro-2-(6-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]hepten-14-carboxylate (165 mg, 0.18 mmol) in N,N-dimethylformamide (3 mL), and the reaction mixture was stirred at room temperature for 2 hrs. The reaction was shown to be completed by LC/MS. The reaction solution was diluted with ethyl acetate (20 mL), washed with water (15 mLX 2) and saturated brine (15 mLX 3), dried over anhydrous sodium sulfate, filtered, and concentrated to obtain t-butyl (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (136 mg, yield: 100%). ES-API: [M+H]⁺= 733.3.

Step V: In an iced water bath, 4 M hydrochloric acid-dioxane solution (5 mL) was slowly added to a solution of t-butyl (5S,5aS,6S,9R)-12-((2,6-dimethylenetetrahydro-1H-pyrrolizin-7a(5H)-yl)methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (136 mg, 0.18 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-12-((2,6-dimethyltetrahydro-1H-pyrrolizin-7a(5H)-methoxy)-2-(8-ethynyl-6-fluoronaphthalen-1-yl)-1-fluoro-5-methyl-5a,6,7,8,9,10-hexahydro-5H-4-oxo-3,10a,11,13,14-pentaaza-6,9-methanonaphtho[1,8-ab]heptene (Z550, 20 mg, yield: 17%) as a light yellow solid. ES-API: [M+H]⁺= 633.3. ¹H NMR (500 MHz, CD₃OD)δ 8.04 (d, *J =* 7.5 Hz, 1H), 7.77-7.24 (m, 1H), 7.69-7.62 (m, 1H), 7.58-7.54 (m, 1H), 7.54-7.50 (m, 1H), 7.49-7.46 (m, 1H), 5.36-5.33 (m, 1H), 5.02 (d, *J* = 8.0 Hz, 4H), 4.55-4.51 (m, 1H), 4.33 -4.26 (m, 2H), 4.07 (d, *J* = 8.5 Hz, 1H), 3.76 (d, *J =* 14.5 Hz, 2H), 3.68-3.65 (m, 1H), 3.64 -3.55 (m, 1H), 3.35-3.29 (m, 3H), 3.16 (t, *J =* 11.5 Hz, 1H), 2.80-2.76 (m, 2H), 2.59 (d, *J =* 16.0 Hz, 2H), 2.05-2.01 (m, 1H), 1.93 -1.72 (m, 3H), 1.59-1.54 (m, 3H).

### Example 21: Synthesis of (5S,5aS,6S,9R)-12-((2,6-dimethyltetrahydro-1H-pyrrolizin-7a(5H)-methoxy)-2-(8-ethynyl-6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptene (Z551)

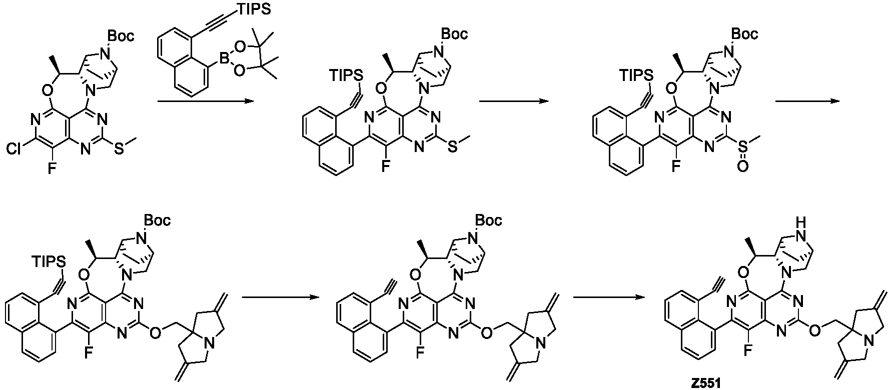

Step I: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (50 mg, 0.10 mmol), triisopropyl ((8-(4,4,5,5-tetramethyl-1,3,2-dioxaborolane -2-yl)naphthalen-1-yl)ethynyl)silane (113 mg, 0.25 mmol), potassium phosphate (66 mg, 0.30 mmol), and [n-butyldi(1-adamantanyl)phosphine](2-amino-1,1'-biphenyl-2-yl)palladium (II) mesylate (11 mg, 0.015 mmol) were dissolved in tetrahydrofuran (5 mL) and water (1 mL). The reaction mixture was stirred at 80°C under micowave for 40 min. The reaction was shown to be completed by LC/MS. The reaction solution was concentrated under reduced pressure. The crude product was purified by silica gel column chromatography (dichloromethane: methanol =20:1) to obtain t-butyl (5S,5aS,6S,9R)-1-fluoro-5-methyl-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (52 mg, yield: 66%). ES-API: [M+H]⁺= 754.3.

Step II: T-butyl (5S,5aS,6S,9R)-1-fluoro-5-methyl-12-(methylthio)-2-(8-((triisopropylsilyl) ethynyl)naphthalen-1-yl)-5a,6,7,8,9,10-hexahydro-5H-4-oxo-3,10a,11,13,14-pentaaza-6,9-methanonaphtho[1,8-ab]hepten-14-carboxylate (52 mg, 0.07 mmol) was dissolved in dichloromethane(5 mL). In an iced water bath, m-chloroperoxybenzoic acid (14 mg, 0.08 mmol) was added, and reacted at room temperature for 30 min. The reaction was shown to be completed by LC/MS. The reaction solution was diluted with dichloromethane (10 mL), washed with saturated sodium sulfite solution (10 mLX2) and saturated brine (10 mLX 3), dried over anhydrous sodium sulfate, filtered, and concentrated to obtain the crude product t-butyl (5S,5aS,6S,9R)-1-fluoro-5-methyl-12-(methylthionyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (53 mg, yield: 100%). ES-API: [M+H]⁺= 770.4.

Step III: (2,6-dimethylenetetrahydro-1H-pyrrolizin-7a(5H)-yl)methanol (23 mg, 0.14 mmol) was dissolved in anhydrous tetrahydrofuran (5 mL). In an iced water bath, sodium hydride (8 mg, 0.21 mmol) was added, and reacted for 30 min in the iced water bath. A solution of t-butyl (5S,5aS,6S,9R)-1-fluoro-5-methyl-12-(methylthionyl)-2-(8-((triisopropylsilyl)ethynyl) naphthalen-1-yl)-5a,6,7,8,9,10-hexahydro-5H-4-oxa-3,10a,11,13,14-pentaaza-6,9-methanonaphtho[1,8-ab]hepten-14-carboxylate (53 mg, 0.07 mmol) in tetrahydrofuran (5 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX 2) and saturated brine (15 mLX3), dried over anhydrous sodium sulfate, filtered, and concentrated to obtain the crude product t-butyl (5S,5aS,6S,9R)-12-((2,6-dimethyltetrahydro-1H-pyrrolizin-7a(5H)-yl)methoxy)-1-fluoro-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (60 mg, yield: 100%). ES-API: [M+H]⁺= 871.5.

Step IV: Cesium fluoride (103 mg, 0.7 mmol) was added to t-butyl (5S,5aS,6S,9R)-12-((2,6-dimethyltetrahydro-1H-pyrrolizin-7a(5H)-yl)methoxy)-1-fluoro-5-methyl-2-(8-((triisopropylsilyl) ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (60 mg, 0.07 mmol) in N,N-dimethylformamide (3 mL), and the reaction mixture was stirred at room temperature for 2 hrs. The reaction was shown to be completed by LC/MS. The reaction solution was diluted with ethyl acetate (20 mL), washed with water (15 mLX 2) and saturated brine (15 mLX 3), dried over anhydrous sodium sulfate, filtered, and concentrated to obtain the crude product t-butyl (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (48 mg, yield: 100%). ES-API: [M+H]⁺= 715.3.

Step V: In an iced water bath, 4 M hydrochloric acid-dioxane solution (5 mL) was slowly added to a solution of t-butyl (5S,5aS,6S,9R)-12-((2,6-dimethylenetetrahydro-1H-pyrrolizin-7a(5H)-yl)methoxy)-2-(8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (48 mg, 0.07mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) to obtain (5S,5aS,6S,9R)-12-((2,6-dimethyltetrahydro-1H-pyrrolizin-7a(5H)-methoxy)-2-(8-ethynyl -6-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptene (Z551, 11 mg, yield: 26%) as a light yellow solid. ES-API: [M+H]⁺= 615.3. ¹H NMR (500 MHz, CD₃OD) δ 8.10 -8.00 (m, 2H), 7.79 -7.70 (m, 1H), 7.67 -7.57 (m, 2H), 7.53-7.46 (m, 2H), 5.38-5.34 (m, 1H), 5.02 (d, *J =* 8.0 Hz, 4H), 4.57 -4.48 (m, 1H), 4.32 -4.26 (m, 2H), 4.07 (d, *J* = 9.0 Hz, 1H), 3.76 (d, *J =* 15.0 Hz, 2H), 3.67 (d, *J =* 5.5 Hz, 1H), 3.59 (d, *J* =5.0 Hz, 1H), 3.36-3.31 (m, 2H), 3.21 -3.12 (m, 2H), 2.79-2.75 (m, 2H), 2.59 (d, *J =* 16.5 Hz, 2H), 2.10 -1.99 (m, 1H), 1.90 -1.73 (m, 3H), 1.63-1.54 (m, 3H).

### Example 22: Synthesis of 5-ethynyl-6-fluoro-4-((5S,5aS,6S,9R)-1-fluoro-12-((2R,7aS)-2-fluorotetrahydro-1H-pyrrolizin-7a(5H)-ylmethoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-ol (Z552)

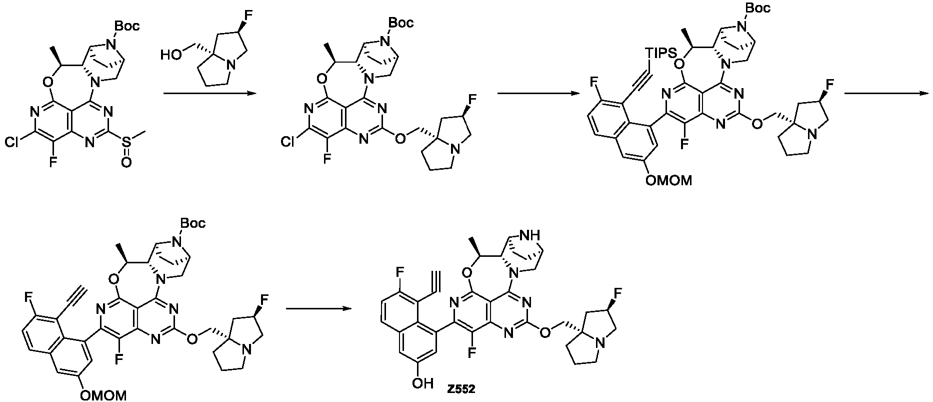

Step I: At 0°C, ((2R,7aS)-2-fluorotetrahydro-1H-pyrrolizin-7a(5H)-yl)methanol (115 mg, 0.72 mmol) was added to a solution of sodium hydride (60% dispersed in an oil) (58 mg, 1.44 mmol) in tetrahydrofuran, and stirred at room temperature for 10 min. After cooling to 0°C, a solution of t-butyl (5S,5aS,6S,9R)-2-chloro-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (200 mg, 0.40 mmol) in tetrahydrofuran (5 mL) was added, and reacted with stirring at 0°C for 1 hr. The reaction solution was quenched with a saturated ammonium chloride solution and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (90mg, yield:38.0%).

Step II: Potassium phosphate (68 mg, 0.30mmmol) and [n-butyldi(1-adamantanyl) phosphine](2-amino-1,1'-biphenyl-2-yl)palladium (II) mesylate (30 mg) were added to t-butyl (5S,5aS,6S,9R)-2-chloro-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (90.0mg,0.15mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (154mg, 0.30mmol) in tetrahydrofuran (6 mL) and water (1.0 mL). Nitrogen was bubbled therethrough and the reaction was continued at 60°C for 40 min. After the reaction, the reaction solution was added with ethyl acetate (100 mL), washed sequentially with water (10 mL) and saturated sodium chloride solution (10 mL), dried over anhydrous sodium sulfate, and filtered. The filtrate was concentrated under reduced pressure,. The crude product was purified by silica gel column chromatography (0-4% methanol /dichloromethane) to obtain t-butyl (5S,5aS,6S,9R)-1-fluoro-2-(7-fluoro-3-(methoxymethoxy)-8-((triisopropylsilyl)ethynyl) naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]-14-carboxylate (95mg, yield: 67.4%).

Step III: Cesium fluoride (70mg, 0.50 mmol) was added to a solution of t-butyl (5S,5aS,6S,9R)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]-14-carboxylate (95mg, 0.101 mmol) in N,N-dimethylformamide (3.0 mL), and reacted with stirring at room temperature for 0.5 hr. After the reaction, the reaction solution was filtered, and the residue was purified by preparative HPLC (trifluoroacetic acid method) to obtain t-butyl (5S,5aS,6S,9R)-2-(8-ethynyl-7-fluoro-3-(methoxymethoxy)naphthalen-1-fluoro-12-(((2R,7aS)-2-fluorotetrahydro-1H-pyrrolizin-7a(5H)-yl)methoxy)-5-methyl-5a,6,7,8,9,10-hexahydro-4-oxa-3,10a,11,13,14-pentaaza-6,9-methanonaphtho[1,8-ab]hepten-14-carboxylate (70mg, yield: 87.5%).

Step IV: A reaction solution of t-butyl (5S,5aS,6S,9R)-2-(8-ethynyl-7-fluoro-3-(methoxymethoxy)naphthalen-1-fluoro-12-(((2R,7aS)-2-fluorotetrahydro-1H-pyrrolizin-7a(5H)-yl)methoxy)-5-methyl-5a,6,7,8,9,10-hexahydro-4-oxa-3,10a,11,13,14-pentaaza-6,9-methanonaphtho[1,8-ab]hepten-14-carboxylate (70mg, 0.089 mmol) acetonitrile (2.0mL), and 4M hydrochloric acid/dioxane solution (0.500mL, 2.000mmol) was reacted at 0°C for 1 hr. The reaction solution was adjusted to pH 8 with saturated sodium bicarbonate, concentrated, and filtered. The residue was purified by preparative HPLC (formic acid method 1) to obtain 5-ethynyl-6-fluoro-4-((5S,5aS,6S,9R)-1-fluoro-12-((2R,7aS)-2-fluorotetrahydro-1H-pyrrolizin-7a(5H)-ylmethoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-ol (Z552, 40mg, yield: 67.8%, formate) as a yellow solid. ES-API:[M+1]⁺=643.2. ¹H NMR (400 MHz, DMSO-d6) δ 7.14 -6.99 (m, 1H), 6.62 -6.30 (m, 3H), 4.81 -4.55 (m, 2H), 3.89 -3.67 (m, 3H), 3.40 (d, *J =* 8.5 Hz, 1H), 3.13 -2.77 (m, 5H), 2.67 -2.47 (m, 3H), 1.89 -1.02 (m, 10H), 0.81 (t, *J =* 5.7 Hz, 3H).

### Example 23: Synthesis of 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynyl-6-fluoronaphthalen-2-ol (Z553)

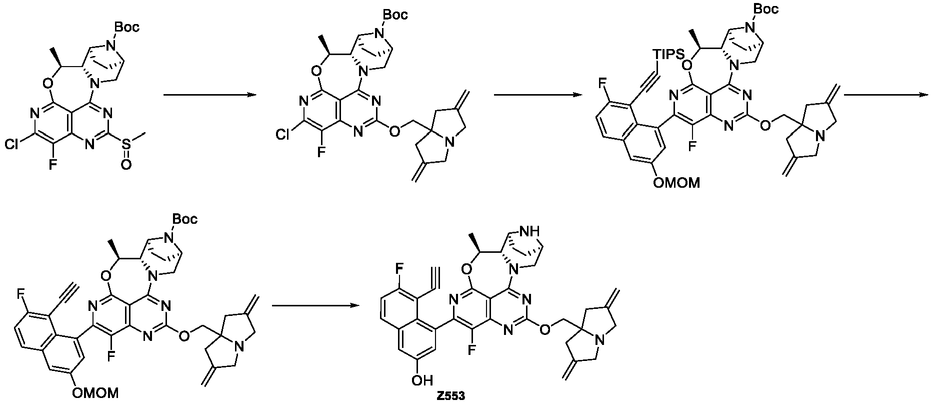

Step I: At 0°C, (2,6-dimethylenetetrahydro-1H-pyrrolizin-7a(5H)-yl)methanol (95 mg, 0.57 mmol) was added to a solution of sodium hydride (60% dispersed in an oil) (30 mg, 0.76 mmol) in tetrahydrofuran. The reaction was stirred at room temperature for 10 min. After cooling to 0°C, a solution of t-butyl (5S,5aS,6S,9R)-2-chloro-1-fluoro-5-methyl-12-(methylthionyl)-5a,6,7,8,9, 10-hexahydro-5H-4-oxy-3,10a,11,13,14-pentaaza-6,9-methanonaphtho[1,8-ab]hepten-14-carboxylate (190 mg, 0.38 mmol) in tetrahydrofuran (5 mL) was added, and reacted with stirring at 0°C for 1 hr. The reaction was quenched with a saturated sodium bicarbonate solution and extracted with ethyl acetate (20 mLX3). The organic phase was washed with saturated brine (20 mLX1), dried over anhydrous sodium sulfate, filtered, and concentrated. The crude product was purified by silica gel column chromatography (0-5% methanol /dichloromethane) to obtain t-butyl (5S,5aS,6S,9R)-2-chloro-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (80mg, yield: 35.0%). ES-API: [M+H]⁺=599.2.

Step II: Potassium phosphate (51 mg, 0.24mmmol) and [n-butyldi(1-adamantanyl) phosphine](2-amino-1,1'-biphenyl-2-yl)palladium (II) mesylate (20 mg) were added to t-butyl (5S,5aS,6S,9R)-2-chloro-12-((2,6-dimethylenetetrahydro-1H-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]hepten-14-carboxylate (80.0mg, 0.08mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (61mg, 0.12mmol) in tetrahydrofuran (6 mL) and water (1.0 mL). Nitrogen was bubbled therethrough and the reaction was continued at 60°C for 40 min. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain (5S,5aS,6S,9R)-12-((2,6-dimethylenetetrahydro-1H-pyrrolizin-7a(5H)-yl)-methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,13,14-pentaaza-6,9-methanonaphtho[1,8-ab]hepten-14-carboxylate (80mg, yield: 63%). ES-API: [M+H]⁺=949.3.

Step III: Cesium fluoride (40 mg, 0.26 mmol) was added to a solution of (5S,5aS,6S,9R)-12-((2,6-dimethylenetetrahydro-1H-pyrrolizin-7a(5H)-yl)-methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,13,14-pentaaza-6,9-methanonaphtho[1,8-ab]hepten-14-carboxylate (80 mg, 0.084 mmol) in N,N-dimethylformamide (3.0 mL), and reacted with stirring at room temperature for 0.5 hr. After the reaction, the reaction solution was filtered, and the residue was purified by preparative HPLC(trifluoroacetic acid method) to obtain (5S,5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (60mg, yield: 90.2%). ES-API: [M+H]⁺=793.1.

Step IV: A solution of (5S,5aS,6S,9R)-12-((2,6-dimethylenetetrahydro-1H-pyrrolizin-7a(5H)-yl)methoxy)-2-(8-ethynyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (60mg, 0.076 mmol), acetonitrile (2.0mL), and 4 M hydrochloric acid/dioxane solution (0.500mL, 2.000mmol) was reacted at 0°C for 1 hr. The reaction solution was adjusted to pH 8 with saturated sodium bicarbonate, concentration, and filtered. The residue was purified by preparative HPLC(formic acid method 1) to obtain 4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynyl-6-fluoronaphthalen-2-ol (Z553, 37mg, yield: 75.1%, formate). ES-API:[M+1]⁺=649.2. ¹H NMR (400 MHz, CD₃OD) δ 8.41 (s, 1H), 7.85 (dt, J = 9.1, 6.2 Hz, 1H), 7.41 -7.06 (m, 3H), 5.46 (d, J = 12.0 Hz, 1H), 5.15 (s, 4H), 4.68 -4.43 (m, 3H), 4.28 -3.85 (m, 5H), 3.74 -3.38 (m, 3H), 3.33 (d, J = 9.7 Hz, 1H), 3.00 -2.84 (m, 2H), 2.72 (d, J = 16.6 Hz, 2H), 2.02 (dd, J = 56.2, 44.8 Hz, 4H), 1.59 (t, J = 6.2 Hz, 3H).

### Example 24: Synthesis of (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z590)

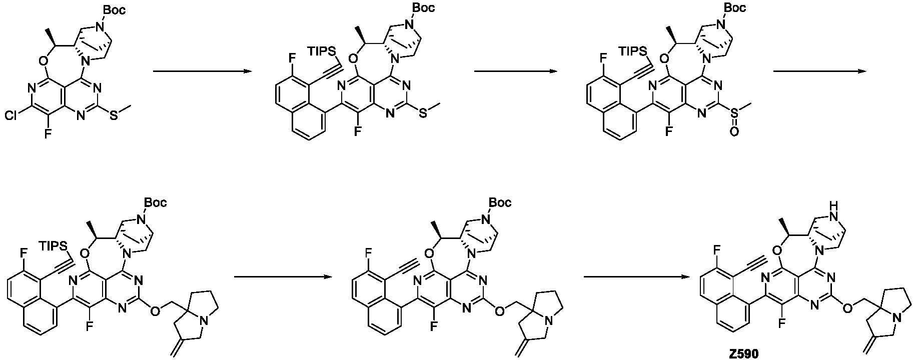

Step I: Under a nitrogen atmosphere, t-butyl (5S,5aS,6S,9R)-2-chloro-1-fluoro-5-methyl-12-(methylthio)-5a,6,7,8,9,10-hexahydro-5H-4-oxo-3,10a,11,13,14-pentaaza-6,9-methanonaphtho [1,8-ab]hepten-14-carboxylate (150mg, 0.311 mmol), ((2-fluoro-8-(4,4,5,5-tetramethyl-1,3,2-dioxaborolane -2-yl)naphthalen-1-yl)ethynyl)triisopropylsilane (211 mg, 0.466mmol), potassium phosphate (132 mg, 0.62 mmol) and [n-butyldi(1-adamantanyl)phosphine](2-amino-1,1'-biphenyl-2-yl)palladium (II) mesylate (23 mg, 0.031 mmol) were dissolved in tetrahydrofuran (6.0mL) and water (1.5 mL). The reaction mixture was heated to 80°C under microwave and stirred for 40 min. The reaction was shown to be completed by LC/MS. The reaction solution was concentrated under reduced pressure. The crude product was purified by silica gel column chromatography (dichloromethane: methanol =20:1) to obtain t-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (290 mg, crude product). ES-API: [M+H]⁺= 772.3.

Step II: T-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (290 mg, crude product) was dissolved in dichloromethane(10.0mL). In an iced water bath, m-chloroperoxybenzoic acid (70 mg, 0.406mmol) was added, and reacted for 30 min in the iced water bath. The reaction was shown to be completed by LC/MS. The reaction solution was diluted with dichloromethane (20 mL), washed with saturated sodium sulfite solution (15 mLX2) and saturated brine (15 mLX3), dried over anhydrous sodium sulfate, filtered, and concentrated to obtain the crude product t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (280 mg, crude product). ES-API: [M+H]⁺= 788.3.

Step III: (2,6-dimethylenetetrahydro-1H-pyrrolizin-7a(5H)-yl)methanol (25mg, 0.163 mmol) was dissolved in anhydrous tetrahydrofuran (5.0mL). In an iced water bath, sodium hydride (13mg, 0.326 mmol) was added, and reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (41.0mg, 0.052mmol) in tetrahydrofuran (2.0mL) was added dropwise to the reaction solution, and reacted at room temperature for 30 min. The reaction was shown to be completed by LC/MS. The reaction solution was diluted with ethyl acetate (80 mL), washed with saturated ammonium chloride solution (15 mLX2) and saturated brine (60 mLX3), dried over anhydrous sodium sulfate, filtered, and concentrated to obtain the crude product t-butyl (5S,5aS,6S,9R)-1-fluoro-2-(7-fluoro-8-((triisopropylsilyl)ethynyl) naphthalen-1-yl)-5-methyl-12-((2-methylenetetrahydro)-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (65 mg, crude product). ES-API: [M+H]⁺= 877.4.

Step IV: Cesium fluoride (160mg, 1.053mmol) was added to t-butyl (5S,5aS,6S,9R)-1-fluoro-2-(7-fluoro-8-((triisopropylsilyl)ethynyl)naphthalen-1-yl)-5-methyl-12-((2-methylenetetrahydro)-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (65 mg, crude product) in N, N-dimethylformamide (3.0mL), and the reaction mixture was stirred at room temperature for 2 hrs. The reaction was shown to be completed by LC/MS. The reaction solution was diluted with ethyl acetate (60 mL), washed with water (15 mLX2) and saturated brine (60mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-5-methyl-12-((2-methylenetetrahydro)-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (70mg, crude product). ES-API: [M+H]⁺= 721.2.

Step V: In an iced water bath, 4 M hydrochloric acid-dioxane solution (2mL) was slowly added to a solution of t-butyl (5S,5aS,6S,9R)-2-(8-ethynyl-7-fluoronaphthalen-1-yl)-1-fluoro-5-methyl-12-((2-methylenetetrahydro)-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (70mg, crude product) in acetonitrile (5.0mL). The reaction mixture was stirred at 0°C for 0.5 hr. The reaction was shown to be completed by LC/MS. The reaction solution was concentrated to dryness under reduced pressure, and the residue was purified by preparative HPLC(ammonium bicarbonate method 2) to obtain (5S,5aS,6S,9R)-2-(8-ethynyl-7-fluoronaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z590, 5.05mg, total yield of 5 steps: 15.6%) as a light yellow solid. ES-API: [M+H]⁺= 621.3.

### Example 25: Synthesis of (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z591)

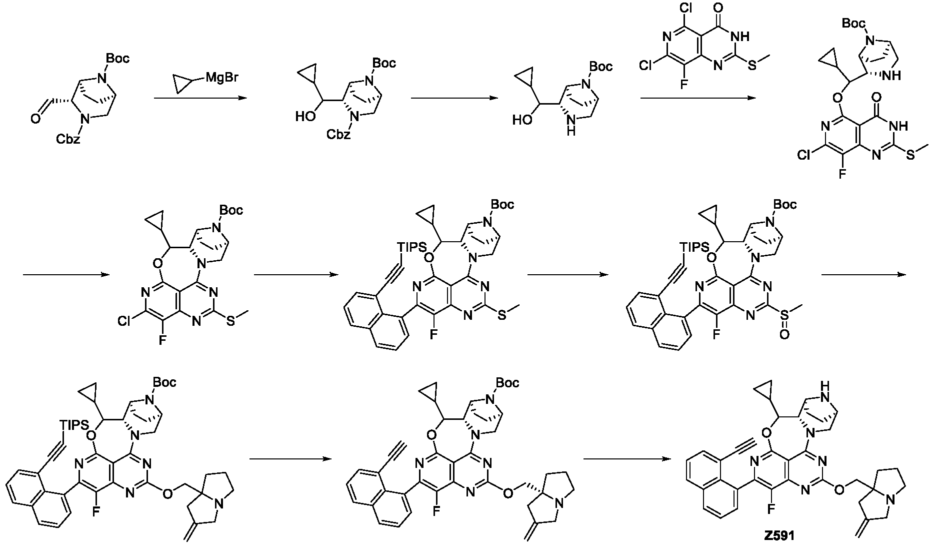

Step I: 8-(t-butyl) 3-benzyl (1S,2S,5R)-2-formyl-3,8-diazabicyclo[3.2.1]octane-3,8-dicarboxylate (1100mg, 2.938mmol) was dissolved in dry tetrahydrofuran (15mL). After cooling to -78°C, a 1M cyclopropylmagnesium bromide solution in tetrahydrofuran (4.4mL) was added dropwise under nitrogen atmosphere, and reacted at -78°C for 2 hrs. A saturated ammonium chloride solution (5mL) was added. The reaction solution was extracted with ethyl acetate, dried over anhydrous sodium sulfate, and concentrated. The residue was purified by silica gel column chromatography (ethyl acetate /petroleum ether=1:2) to obtain 3-benzyl 8-(t-butyl)(1S,2S,5R)-2-((S)-cyclopropyl(hydroxyl)methyl)-3,8-diazabicyclo[3.2.1]octane-3,8-dicarboxylate (1200mg, yield: 98.07%). ES-API:[M-18+H]⁺=399.3.

Step II: 3-benzyl 8-(t-butyl) (1S,2S,5R)-2-((S)-cyclopropyl(hydroxyl)methyl)-3,8-diazabicyclo[3.2.1]octane-3,8-dicarboxylate (1223mg, 2.938mmol) was dissolved in methanol (20mL) and aminomethanol (10.0mL, 7M). Palladium on carbon (10%) (500mg) was added, and the reaction was carried out under hydrogen atmosphere at room temperature for 2 hrs. The reaction was filtered and concentrated. The crude product was purified by preparative TLC (dichloromethane/methanol =10:1) to obtain t-butyl (1S,2S,5R)-2-((S)-cyclopropyl(hydroxyl) methyl)-3,8-diazabicyclo[3.2.1]octane-8-carboxylate (916mg, crude product).

Step III: At 0°C, t-butyl (1S,2S,5R)-2-((S)-cyclopropyl(hydroxyl)methyl)-3,8-diazabicyclo [3.2.1]octane-8-carboxylate (516mg, 1.827mmol) was added to a suspension of sodium hydride (60% dispersed in an oil)(370 mg, 9.25mmol) in tetrahydrofuran (20 mL), and stirred at this temperature for 30 min. 5,7-dichloro-8-fluoro-2-(methylthio)pyrido[4,3-d]pyrimidine4(3H)-one (1025mg, 3.66 mmol) was added and heated with strring at room temperature for 1 hr. After the reaction was completed, the reaction was quenched with water and extracted with dichloromethane/methanol (10:1, 80 mLX3). The organic phase was dried over anhydrous sodium sulfate, and concentrated to obtain t-butyl (1S,2S,5R)-2-((S)-((7-chloro-8-fluoro-2-(methylthio)-4-oxa-3,4-dihydropyrido[4,3-d]pyrimidine-5-yl)oxy)(cyclopropyl)methyl)-3,8-diazabicyclo [3.2.1]octane-8-carboxylate (700mg, yield:72.82%). ES-API:[M+H]⁺=526.0.

Step IV: 1-propylphosphoric anhydride (50% ethyl acetate )(1.27g, 3.992 mmol) was added to a solution of t-butyl (1S,2S,5R)-2-((S)-((7-chloro-8-fluoro-2-(methylthio)-4-oxa-3,4-dihydropyrido[4,3-d]pyrimidine-5-yl)oxy)(cyclopropyl)methyl)-3,8-diazabicyclo[3.2.1]octane-8-carboxylate (700mg, 1.331mmol) and N,N-diisopropylethylene diamine (516mg, 3.992mmol) in dichloromethane (40mL) and stirred at room temperature for 3 hrs. The reaction was quenched with water and extracted with dichloromethane (100mLX2). The organic layer was dried over anhydrous sodium sulfate, filtered and concentrated. The crude product was purified by silica gel column chromatography[petroleum ether:ethyl acetate =100:0-70:30,(v/v)] to obtain t-butyl (5S,5aS,6S,9R)-2-chloro-5-cyclopropane-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (200mg, yield: 29.58%). ES-API:[M+H]⁺=508.2.

Step V: Potassium phosphate (130 mg, 0.613mmmol) and [n-butyldi(1-adamantanyl) phosphine](2-amino-1,1'-biphenyl-2-yl)palladium (II) mesylate (20 mg,0.027mmol) were added to t-butyl (5S,5aS,6S,9R)-2-chloro-5-cyclopropane-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (80.0mg,0.157mmol) and triisopropyl((8-(4,4,5,5-tetramethyl-1,3,2-dioxaborolan-2-yl)naphthalen-1-yl)ethynyl)silane (150mg, 0.345mmol) in tetrahydrofuran (9mL) and water (2 mL). Nitrogen was bubbled therethrough for 3 min and the reaction was continued at 80°C under microwave for 40 min. After the reaction, the reaction solution was extracted with ethyl acetate (80 mLX2) and saturated brine (60 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-5-cyclopropyl-1-fluoro-12-(methylthio)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (150mg, crude product). ES-API: [M+H]⁺=780.3.

Step VI: At 0°C, m-chloroperoxybenzoic acid (45mg, 0.262 mmol) was added to a solution of t-butyl (5S,5aS,6S,9R)-5-cyclopropyl-1-fluoro-12-(methylthio)-2-(8-((triisopropylsilyl)ethynyl) naphthalen-1-yl)-5a,6,7,8,9,10-hexahydro-5H-4-oxa-3,10a,11,13,14-pentaaza-6,9-methanonaphtho[1,8-ab]hepten-14-carboxylate (150mg, crude product) in dichloromethane (15 mL). The solution was stirred for 1 hour in an ice bath. The reaction solution was diluted with dichloromethane (80mL) and washed with 10% sodium hydroxide (10.0mLX2). The organic phase was dried over anhydrous sodium sulfate and concentrated to obtain the crude product t-butyl (5S,5aS,6S,9R)-5-cyclopropyl-1-fluoro-12-(methylsulfinyl)-2-(8-((triisopropylsilyl)ethynyl) naphthalen-1-yl)-5a,6,7,8,9,10-hexahydro-5H-4-oxa-3,10a,11,13,14-pentaaza-6,9-methanonaphtho[1,8-ab]hepten-14-carboxylate (160g, crude product). ES-API:[M+H]⁺=796.3.

Step VII: At 0°C, (2-methylenetetrahydro-1H-pyrrolizin-7a(5H)-yl)methanol (75mg, 0.489 mmol) was added to a solution of sodium hydride (60%dispersed in an oil) (40.0mg, 0.978mmol) in tetrahydrofuran. The reaction was stirred at room temperature for 30 min. After cooling to 0°C, a solution of t-butyl (5S,5aS,6S,9R)-5-cyclopropyl-1-fluoro-12-(methylsulfinyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (100 mg, 0.20 mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine (80mL), dried over anhydrous sodium sulfate, filtered, and concentrated. The crude product was purified by silica gel column chromatography (0-5% methanol /dichloromethane) to obtain the product t-butyl (5S,5aS,6S,9R)-5-cyclopropyl-1-fluoro-12-((2-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (190mg, crude product). ES-API: [M+H]⁺=885.5.

Step VIII: Cesium fluoride (480mg, 3.158 mmol) was added to a solution of t-butyl (5S,5aS,6S,9R)-5-cyclopropyl-1-fluoro-12-((2-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (190mg, crude product) in N,N-dimethylformamide (10.0mL), and reacted with stirring at room temperature for 1.5 hrs. The organic phase was washed with saturated brine (80mLX5), dried over anhydrous sodium sulfate, filtered, and concentrated to obtain t-butyl (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (210mg, crude product). ES-API: [M+H]⁺=729.3.

Step IX: A reaction solution of t-butyl (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (210mg,crude product), acetonitrile (10.0mL), and 4M hydrochloric acid/dioxane solution (2.0mL, 8.000mmol) was reacted at 0°C for 1 hr. The reaction solution was concentrated, and the crude product was purified by preparative HPLC (formic acid method 1) to obtain (5S,5aS,6S,9R)-5-cyclopropyl-2-(8-ethynylnaphthalen-1-yl)-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z591, formate, 22mg, yield: 19.3%) as a yellow solid. ES-API:[M+1]⁺=629.3.

### Example 26: Synthesis of (5S,5aS,6S,9R)-5-ethyl-2-(8-ethylnaphthalen-1-yl)-1-fluoro-12-((2R,7aS)-2-fluorotetrahydro-1H-pyrrolizin-7a(5H)-ylmethoxy)-5a,6,7,8,9,10-hexahydro-5H-4-oxy-3,10a,11,13,14-pentaaza-6,9-methanonaphtho[1,8-ab]heptene (Z592)

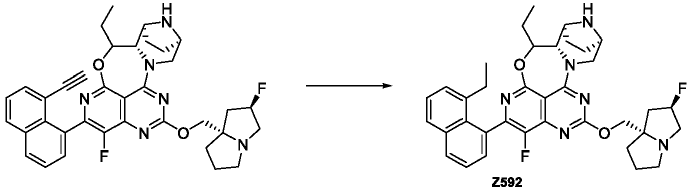

Step I: (5S,5aS,6S,9R)-5-ethyl-2-(8-ethynylnaphthalen-1-yl)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (20 mg, 2.07mmol) was dissolved in methanol (5.0mL). Palladium on carbon (10%) (10mg) was added, and the reaction was continued under hydrogen for 1 hr. The reaction solution was filtered, and concentrated. The crude product was purified by preparative HPLC (formic acid method 1) to obtain (5S,5aS,6S,9R)-5-ethyl-2-(8-ethylnaphthalen-1-yl)-1-fluoro-12-((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z592, 12mg, yield: 61.5%, formate) as a yellow solid. ES-API: [M+H]⁺=627.3. ¹H NMR (400 MHz, CD₃OD) δ 8.03 (d, *J* = 8.2 Hz, 1H), 7.85 (d, *J =* 8.1 Hz, 1H), 7.63 -7.25 (m, 4H), 5.52 (d, *J* = 51.2 Hz, 2H), 4.69 - 4.26 (m, 4H), 4.11 -3.59 (m, 5H), 3.42 -3.23 (m, 2H), 2.79 -1.72 (m, 14H), 1.15 (t, *J =* 7.2 Hz, 3H), 0.95 (dt, *J*= 45.5, 7.5 Hz, 3H).

### Example 27: Synthesis of (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z593)

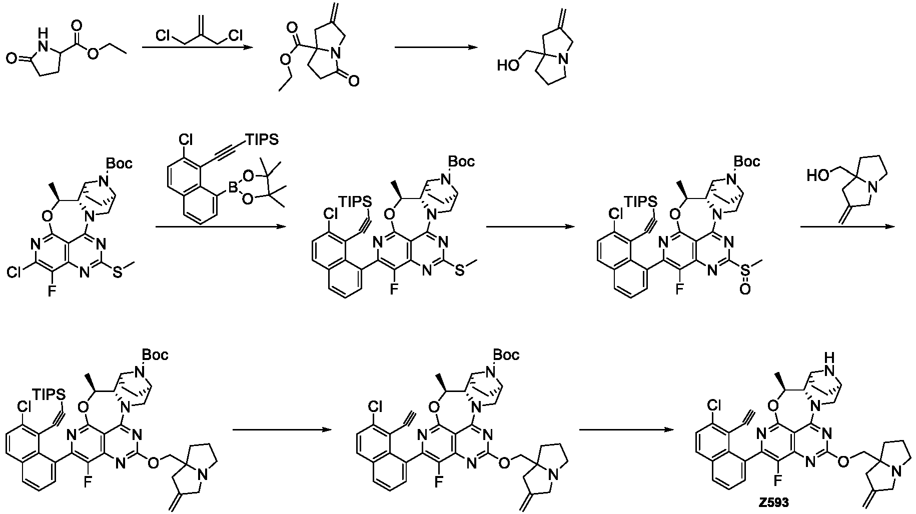

Step I: Ethyl 5-oxopyrrolidin-2-carboxylate (10g, 63.69mmol) and 3-chloro-2-(chloromethyl) prop-1-ene (12.64 g, 101.9 mmol) were dissolved in tetrahydrofuran (50mL). **1M** lithium bis(trimethylsilyl)amide (127mL) was added at -40°C, heated to room temperature and reacted for 16 hrs. The reaction was quenched with a ammonium chloride solution (50mL). The reaction solution was extracted with ethyl acetate (50mLx1). The organic phase was washed with brine, dried over anhydrous sodium sulfate, and concentrated. The residue was purified by column chromatography (petroleum ether/ethyl acetate =1/1) to obtain the product ethyl 2-methylene-5-oxotetrahydro-1H-pyrrolizin-7a(5H)-carboxylate (4.2g, yield: 31.56%). ES-API: [M+H]⁺= 210.1.

Step II: Lithium aluminium hydride (1.52g, 40.15mmol) was dissolved in tetrahydrofuran (20mL), and cooled to 0°C. A solution of ethyl 2-methylene-5-oxotetrahydro-1H-pyrrolizin-7a(5H)-carboxylate (4.2g, 20.07mmol) in tetrahydrofuran (20mL) was added dropwise, heated to 70°C, and reacted for 3 hrs. The reaction solution was quenched by adding water (1.5mL), 15% sodium hydroxide aqueous solution (1.5mL), and then water (4.5mL), filtered, and concentrated to obtain a crude product that was (2-methylenetetrahydro-1H-pyrrolizin-7a(5H)-yl)-methanol (2.5g, yield: 81.29%). ES-API: [M+H]⁺= 154.1.

Step III: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (100 mg, 0.21 mmol), ((2-chloro-8-(4,4,5,5-tetramethyl-1,3,2-dioxaborolan-2-yl)naphthalen-1-yl)ethynyl)triisopropylsilane (3589 mg, 0.84 mmol), potassium phosphate (132 mg, 0.62 mmol) and [n-butyldi(1-adamantanyl)phosphine](2-amino-1,1'-biphenyl-2-yl)palladium (II) mesylate (23 mg, 0.031 mmol) were dissolved in tetrahydrofuran (5 mL) and water (1 mL). The reaction mixture was stirred at 80°C under micowave for 40 min. The reaction was shown to be completed by LC/MS, and the reaction solution was concentrated under reduced pressure. The crude product was purified by silica gel column chromatography (dichloromethane: methanol =20:1) to obtain t-butyl (5S,5aS,6S,9R)-2-(7-chloro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (98 mg, yield: 60%). ES-API: [M+H]⁺= 788.3.

Step IV: T-butyl (5S,5aS,6S,9R)-2-(7-chloro-8-((triisopropylsilyl)ethynyl)naphthalen-1-yl)-1-fluoro-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (98 mg, 0.12 mmol) was dissolved in dichloromethane (5 mL). In an iced water bath, m-chloroperoxybenzoic acid (25 mg, 0.15 mmol) was added, and then reacted at room temperature for 30 min. The reaction was shown to be completed by LC/MS. The reaction solution was diluted with dichloromethane (20 mL), washed with saturated sodium sulfite solution (15 mLX2) and saturated brine (15 mLX3), dried over anhydrous sodium sulfate, filtered, and concentrated to obtain the crude product t-butyl (5S,5aS,6S,9R)-2-(7-chloro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (60 mg, yield: 60%). ES-API: [M+H]⁺= 804.3.

Step V: (2-methylenetetrahydro-1H-pyrrolizin-7a(5H)-yl)methanol (34 mg, 0.22 mmol) was dissolved in anhydrous tetrahydrofuran(5 mL). In an iced water bath, sodium hydride (11 mg, 0.45 mmol) was added, and then reacted for 30 min in the iced water bath. A solution of t-butyl (5S,5aS,6S,9R)-2-(7-chloro-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (60 mg, 0.075 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, filtered, and concentrated to obtain the crude product t-butyl (5S,5aS,6S,9R)-2-(7-chloro-8-((triisopropylsilyl)ethynyl)naphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (66 mg, yield: 100%). ES-API: [M+H]⁺= 893.3

Step VI: Cesium fluoride (112 mg, 0.74 mmol) was added to t-butyl (5S,5aS,6S,9R)-2-(7-chloro-8-((triisopropylsilyl)ethynyl)naphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (66 mg, 0.074 mmol) in N,N-dimethylformamide (3 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (55 mg, yield: 100%). ES-API: [M+H]⁺= 737.2.

Step VII: In an iced water bath, 4 M hydrochloric acid-dioxane solution (5 mL) was slowly added to a solution of t-butyl (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (55 mg, 0.074 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC(ammonium bicarbonate method 2) to obtain (5S,5aS,6S,9R)-2-(7-chloro-8-ethynylnaphthalen-1-yl)-1-fluoro-5-methyl-12-((2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z593, 6 mg, yield: 12%) as a light yellow solid. ES-API: [M+H]⁺= 637.2.

### Example 28: Synthesis of (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxy-3,10a,11,13,14-pentaaza-6,9-methanonaphtho[1,8-ab]heptene (Z594)

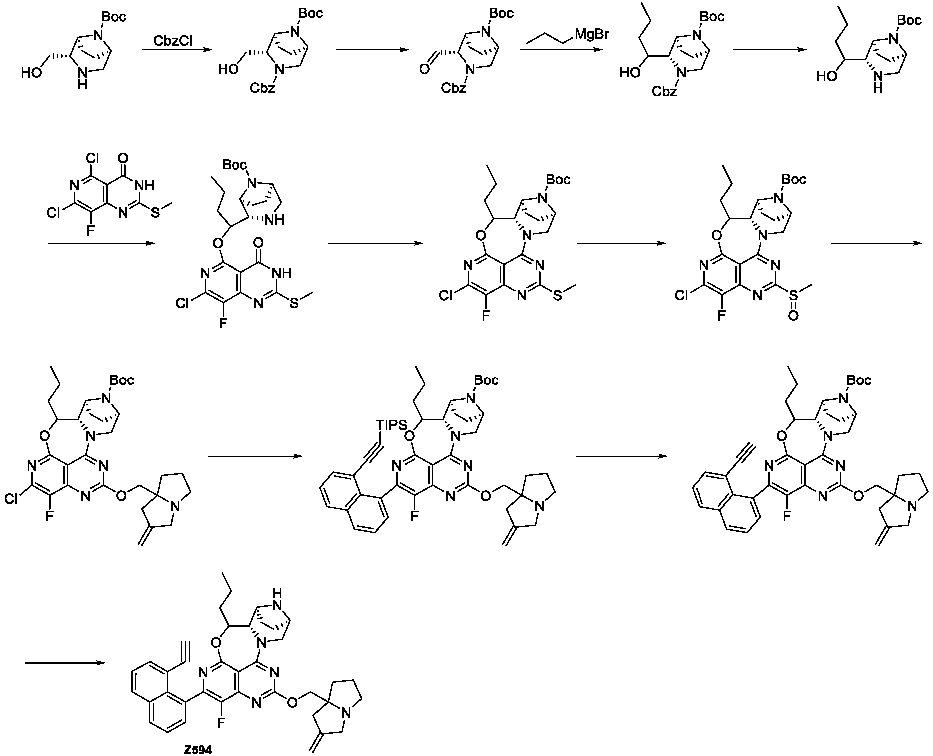

Step I: Benzyl chloroformate (2.54g,14.88mmol) and sodium bicarbonate (2.98g,32.0mmol) were added to a mixed solution of (1S,2S,5R)-t-butyl 2-(hydroxymethyl)-3,8-diazacyclo [3.2.1]octane-8-carboxylate (3.0 g,12.40mmol) dissolved in ethyl acetate (30mL) and water (10mL), and stirred overnight at room temperature. The reaction solution was added with water (50mL), extracted with ethyl acetate (100mLX3), washed with saturated brine (20mL), dried over anhydrous sodium sulfate, and concentrated. The residue was purified by column chromatography (petroleum ether/ethyl acetate =2:1) to obtain 3-benzyl 8-(t-butyl) (1S,2S,5R)-2-(hydroxymethyl)-3,8-diazabicyclo[3.2.1]octane-3,8-dicarboxylate (4.35g, yield: 93.3%).

Step II: At -78°C and under a nitrogen atmosphere, a solution of dimethyl sulfoxide (156mg,1.99mmol) in anhydrous dichloromethane (5.0mL) was slowly added dropwise to oxalyl chloride (223mg, 1.75mmol) in anhydrous dichloromethane (10mL), and stirred at this temperature for 15 min. A solution of 3-benzyl 8-(t-butyl)(1S,2S,5R)-2-(hydroxymethyl)-3,8-diazabicyclo[3.2.1]octane-3,8-dicarboxylate (300mg,0.80mmol) in anhydrous dichloromethane (5.0mL) was slowly added dropwise, and reacted at -78°C for 40 min. Triethyl amine (646mg,6.38mmol) was slowly added, and reacted at -78°C for 1 hr. The reaction was quenched with saturated ammonium chloride solution (20mL). The reaction solution was extracted with ethyl acetate, dried over anhydrous sodium sulfate, and concentrated, to obtain crude 8-(t-butyl) 3-benzyl (1S,2S,5R)-2-formyl-3,8-diazabicyclo[3.2.1]octane-3,8-dicarboxylate (270mg, yield: 90.2%).

Step III: 8-(t-butyl) 3-benzyl (1S,2S,5R)-2-formyl-3,8-diazabicyclo[3.2.1]octane-3,8-dicarboxylate (270mg, 0.72mmol) was dissolved in dry tetrahydrofuran (5mL). After cooling to -78°C, a 2M propylmagnesium bromide solution in ether (2 0.4mL) was added dropwise under nitrogen atmosphere. After reaction at -78°C for 2 hrs, a saturated ammonium chloride solution (10mL) was added. The reaction solution was extracted with ethyl acetate, dried over anhydrous sodium sulfate, and concentrated. The residue was purified by TLC (ethyl acetate /petroleum ether=1:3) to obtain 3-benzyl 8-(t-butyl) (1S,2S,5R)-2-((S)-1-hydroxylbutyl)-8-azabicyclo [3.2.1]octane-3,8-dicarboxylate (180mg, yield: 59.8%).

Step IV: 3-benzyl 8-(t-butyl) (1S,2S,5R)-2-((S)-1-hydroxylbutyl)-8-azabicyclo[3.2.1]octane-3,8-dicarboxylate (180mg, 0.43mmol) was dissolved in methanol (4 mL). Palladium on carbon (10%)(30mg) was added, and the reaction was carried out under hydrogen atmosphere at normal temperature for 2 hrs. The reaction was filtered and concentrated, to obtain t-butyl (1S,2S,5R)-2-((S)-1-hydroxylbutyl)-3,8-diazabicyclo[3.2.1]octane-8-carboxylate (90mg, yield: 73.8%).

Step V: At 0°C, t-butyl (1S,2S,5R)-2-((S)-1-hydroxylbutyl)-3,8-diazabicyclo[3.2.1]octane-8-carboxylate (90 mg, 0.32 mmol) was added to a suspension of sodium hydride (60% dispersed in an oil) (40 mg, 0.95 mmol) in tetrahydrofuran (5.0 mL), and stirred at this temperature for 10 min. 5,7-dichloro-8-fluoro-2-(methylthio)pyrido[4,3-d]pyrimidine4(3H)-one (112mg, 0.40 mmol) was added and stirred with heating 40°C for 1 hr. After the reaction was completed, the reaction was quenched with water and extracted with dichloromethane/methanol (10:1, 25 mLX3). The organic phase was dried over anhydrous sodium sulfate, and rotary evaporated to dryness to obtain t-butyl (1S,2S,5R)-2-((S)-1-((7-chloro-8-fluoro-2-(methylthio)-4-oxa-3,4-dihydropyrido[4,3-d]pyrimidine-5-yl)oxy)butyl)-3,8-diazabicyclo[3.2.1]octane-8-carboxylate (120mg, yield: 71.0%).

Step VI: Bromo-tris-pyrrolidino-phosphonium hexafluorophosphate (215 mg, 0.46 mmol) was added to a solution of t-butyl (1S,2S,5R)-2-((S)-1-((7-chloro-8-fluoro-2-(methylthio)-4-oxa-3,4-dihydropyrido[4,3-d]pyrimidine-5-yl)oxy)butyl)-3,8-diazabicyclo[3.2.1]octane-8-carboxylate (120mg, 0.23mmol) and N,N-diisopropylethylene diamine (85 mg, 0.69mmol) in dichloromethane (10mL), and stirred at room temperature for 16 hrs. The reaction was quenched with water and extracted with dichloromethane. The organic phase was dried over anhydrous sodium sulfate, filtered, and concentrated. The residue was purified by silica gel column chromatography (petroleum ether/ethyl acetate =3:1) to obtain t-butyl (5S,5aS,6S,9R)-2-chloro-1-fluoro-5-propyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, yield: 70.1%).

Step VII: At 0°C, m-chloroperoxybenzoic acid (36 mg, 0.20 mmol) was added to a solution of t-butyl (5S,5aS,6S,9R)-2-chloro-1-fluoro-5-propyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.16mmol) in dichloromethane (15 mL). The solution was stirred at room temperature for 1 hr. The reaction solution was diluted with dichloromethane (30mL) and washed with 10% sodium hydroxide (5.0mLX2). The organic phase was dried over anhydrous sodium sulfate and concentrated to obtain the crude product t-butyl (5S,5aS,6S,9R)-2-chloro-1-fluoro-5-propyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (78mg, yield: 95.0%).

Step VIII: At 0°C, (2-methylenetetrahydro-1H-pyrrolizin-7a(5H)-yl)methanol (43 mg, 0.28 mmol) was added to a solution of sodium hydride (60% dispersed in an oil) (15 mg, 0.36 mmol) in tetrahydrofuran. The reaction was stirred at room temperature for 10 min. After cooling to 0°C, a solution of t-butyl (5S,5aS,6S,9R)-2-chloro-1-fluoro-5-propyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (80 mg, 0.16 mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-chloro-5-propyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (40mg, yield: 41.1%). ES-API: [M+H]⁺=607.3.

Step IX: Potassium phosphate (34 mg, 0.16mmmol) and [n-butyldi(1-adamantanyl) phosphine](2-amino-1,1'-biphenyl-2-yl)palladium (II) mesylate (15 mg) were added to t-butyl (5S,5aS,6S,9R)-2-chloro-5-propyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (40.0mg, 0.066mmol) and triisopropyl((8-(4,4,5,5-tetramethyl-1,3,2-dioxaborolan-2-yl)naphthalen-1-yl)ethynyl)silane (56mg, 0.13mmol) in tetrahydrofuran (6 mL) and water (1.0 mL). Nitrogen was bubbled therethrough, and the reaction was continued at 60°C for 2 hrs. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (0-4% methanol /dichloromethane), to obtain t-butyl (5S,5aS,6S,9R)-1-fluoro-12-((2-methylenetetrahydro-1H-pyrrolizin-7a(5H)-yl)methoxy)-5-propyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (38mg, yield: 61.3%).

Step X: Cesium fluoride (40mg, 0.26 mmol) was added to a solution of t-butyl (5S,5aS,6S,9R)-1-fluoro-12-((2-methylenetetrahydro-1H-pyrrolizin-7a(5H)-yl)methoxy)-5-propyl-2-(8-((triisopropylsilyl)ethynyl)naphthalen-1-yl)-5a,6,7,8,9,10-hexahydro-5H-4-oxy-3,10a,11,13,14-pentaaza-6,9-methanonaphtho[1,8-ab]hepten-14-carboxylate (38mg, 0.043 mmol) in N,N-dimethylformamide (3.0mL), and reacted with stirring at room temperature for 0.5 hr. After the reaction, the reaction solution was filtered, and the residue was purified by preparative HPLC(trifluoroacetic acid method) to obtain t-butyl (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]hepten-14-carboxylate (25mg, yield: 79.0%).

Step XI: A reaction solution of t-butyl (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]hepten-14-carboxylate (25mg, 0.034 mmol) in acetonitrile (2.0mL) and 4M hydrochloric acid/dioxane solution (0.500mL, 2.000mmol) was reacted at 0°C for 1 hr. After concentration, the crude product was purified by preparative HPLC (formic acid method 1) to obtain (5S,5aS,6S,9R)-2-(8-ethynylnaphthalen-1-yl)-1-fluoro-12-((2-methylenetetrahydro-1H-pyrrolizin-7a(5H)-yl)methoxy)-5-propyl-5a,6,7,8,9,10-hexahydro-5H-4-oxa-3,10a,11,13,14-pentaaza-6,9-methanonaphtho[1,8-ab]heptene (Z594, 12mg, yield: 55.6%, formate) as a yellow solid. ES-API: [M+H]⁺=631.3.

### Example 29: Synthesis of (5S,5aS,6S,9R)-2-(8-ethynylnaphthalen-1-yl)-1-fluoro-12-((2-methylenetetrahydro-1H-pyrrolizin-7a(5H)-yl)methoxy)-5-ethyl-5a,6,7,8,9,10-hexahydro-5H-4-oxa-3,10a,11,13,14-pentaaza-6,9-methanonaphtho[1,8-ab]heptene (Z595)

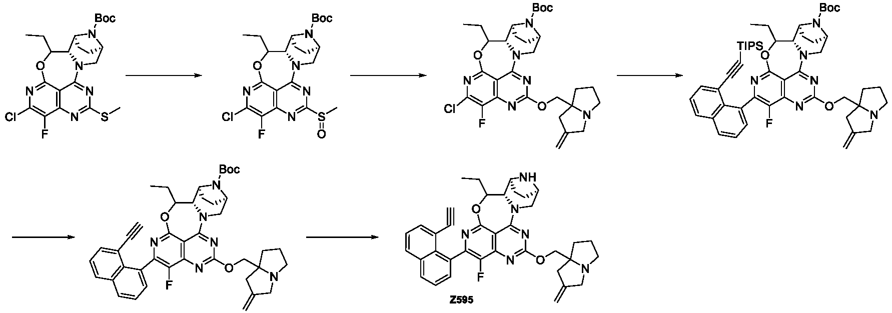

Step I: At 0°C, m-chloroperoxybenzoic acid (61 mg, 0.30 mmol) was added to a solution of t-butyl (5S,5aS,6S,9R)-2-chloro-1-fluoro-5-ethyl-12-(methylthio)-5a,6,7,8,9,10-hexahydro-5H-4-oxo-3,10a,11,13,14-pentaaza-6,9-methanonaphtho[1,8-ab]hepten-14-carboxylate (100 mg, 0.20mmol) in dichloromethane(15 mL), and stirred at room temperature for 1 hr. The reaction solution was diluted with dichloromethane (50mL) and washed with 10% sodium hydroxide (5.0mLX2). The organic phase was dried over anhydrous sodium sulfate and concentrated to obtain the crude product t-butyl (5S,5aS,6S,9R)-2-chloro-1-fluoro-5-ethyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100mg, yield: 94.8%).

Step II: At 0°C, (2-methylenetetrahydro-1H-pyrrolizin-7a(5H)-yl)methanol (52 mg, 0.34 mmol) was added to a solution of sodium hydride (60% dispersed in an oil) (20 mg, 0.51 mmol) in tetrahydrofuran. The reaction was stirred at room temperature for 10 min. After cooling to 0°C, a solution of t-butyl (5S,5aS,6S,9R)-2-chloro-1-fluoro-5-ethyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, 0.19 mmol) in tetrahydrofuran (5 mL) was added. The reaction was stirred at 0°C for 1 hr. The reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-chloro-5-ethyl-1-fluoro-12-((2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (40mg, yield: 35.0%).

Step III: Potassium phosphate (34 mg, 0.16mmmol) and [n-butyldi(1-adamantanyl) phosphine](2-amino-1,1'-biphenyl-2-yl)palladium (II) mesylate (15 mg) were added to t-butyl (5S,5aS,6S,9R)-2-chloro-5-propyl-1-fluoro-12-((2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (40.0mg, 0.067mmol) and triisopropyl((8-(4,4,5,5-tetramethyl-1,3,2-dioxaborolan-2-yl)naphthalen-1-yl)ethynyl)silane (56mg,0.13mmol) in tetrahydrofuran(6 mL) and water (1.0 mL). Nitrogen was bubbled therethrough, and the reaction was continued at 60°C for 2 hrs. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (0-4% methanol /dichloromethane), to obtain t-butyl (5S,5aS,6S,9R)-1-fluoro-12-((2-methylenetetrahydro-1H-pyrrolizin-7a(5H)-yl)methoxy)-5-ethyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (38mg, yield: 64.4%).

Step IV: Cesium fluoride (40mg, 0.26 mmol) was added to a solution of t-butyl (5S,5aS,6S,9R)-1-fluoro-12-((2-methylenetetrahydro-1H-pyrrolizin-7a(5H)-yl)methoxy)-5-ethyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (38mg, 0.043 mmol) in N,N-dimethylformamide (3.0mL). The reaction was carried out with stirring at room temperature for 0.5 hr. After the reaction, the reaction solution was filtered, and the residue was purified by preparative HPLC (trifluoroacetic acid method) to obtain t-butyl (5S,5aS,6S,9R)-2-(8-ethynylnaphthalen-1-yl)-1-fluoro-12-((2-methylenetetrahydro-1H-pyrrolizin-7a(5H)-ylmethoxy)-5-ethyl-5a,6,7,8,9,10-hexahydro-5H-4-oxa-3,10a,11,13,14-pentaaza-6,9-methanonaphtho[1,8-ab]hepten-14-carboxylate (25mg, yield:79.0%).

Step V: A reaction solution of t-butyl (5S,5aS,6S,9R)-2-(8-ethynylnaphthalen-1-yl)-1-fluoro-12-((2-methylenetetrahydro-1H-pyrrolizin-7a(5H)-yl)methoxy)-5-ethyl-5a,6,7,8,9,10-hexahydro-5H-4-oxa-3,10a,11,13,14-pentaaza-6,9-methanonaphtho[1,8-ab]hepten-14-carboxylate (25mg, 0.035 mmol) in acetonitrile (2.0mL) and 4M hydrochloric acid/dioxane solution (0.500mL, 2.000mmol) was reacted at 0°C for 1 hr. The reaction solution was concentrated, and the crude product was purified by preparative HPLC (formic acid method 1) to obtain (5S,5aS,6S,9R)-2-(8-ethynylnaphthalen-1-yl)-1-fluoro-12-((2-methylenetetrahydro-1H-pyrrolizin-7a(5H)-yl)methoxy) -5-ethyl-5a,6,7,8,9,10-hexahydro-5H-4-oxa-3,10a,11,13,14-pentaaza-6,9-methanonaphtho[1,8-ab]heptene (Z595, 12mg, yield: 55.6%, formate) as a yellow solid. ES-API: [M+H]⁺=617.3. ¹H NMR (400 MHz, CD₃OD) δ 8.07 (dd, *J =* 17.3, 9.4 Hz, 2H), 7.83 -7.43 (m, 4H), 5.37 (d, *J =* 84.9 Hz, 3H), 4.78 -4.50 (m, 2H), 4.32 (dd, *J =* 38.6, 24.6 Hz, 3H), 3.76 (dd, *J =* 64.3, 10.2 Hz, 4H), 3.54 -3.11 (m, 2H), 3.02 (d, *J* = 16.6 Hz, 1H), 2.76 (d, *J =* 16.4 Hz, 1H), 2.45 -1.70 (m, 10H), 1.16 (t, *J =* 6.5 Hz, 3H).

### Example 30: Synthesis of (5S,5aS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z596)

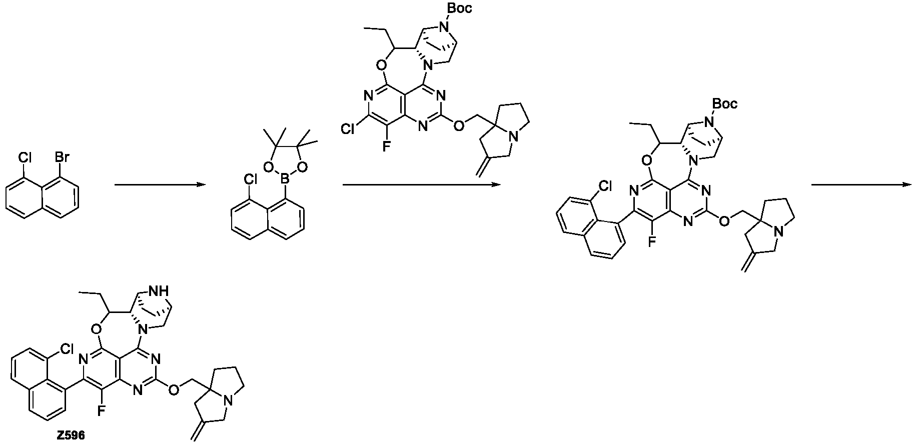

Step I: 1-bromo-8-chloronaphthalene (500 mg, 2.07mmol) was dissolved in anhydrous dioxane (5.0mL). Bis(pinacolato)diboron (631mg, 2.48mmol), [1,1'-bis(diphenylphosphino) ferrocene] palladium dichloride (151mg, 0.207mmol), and potassium acetate (282mg, 4.14mmol) were added, and reacted under a nitrogen atmosphere at 90°C for 12 hrs. The reaction solution was filtered, and concentrated. The crude product was purified by silica gel column chromatography (0-20% ethyl acetate /petroleum ether) to obtain 2-(8-chloronaphthalen-1-yl)-4,4,5,5-tetramethyl-1,3,2-dioxaborolane (300mg, yield: 50.2%).

Step II: Potassium phosphate (34 mg, 0.16mmmol) and [n-butyldi(1-adamantanyl) phosphine](2-amino-1,1'-biphenyl-2-yl)palladium (II) mesylate (15 mg) were added to t-butyl (5S,5aS,6S,9R)-2-chloro-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]hepten-14-carboxylate (40.0mg, 0.067mmol) and 2-(8-chloronaphthalen-1-yl)-4,4,5,5-tetramethyl-1,3,2-dioxaborolane (56mg, 0.13mmol) in tetrahydrofuran (6 mL) and water (1.0 mL). Nitrogen was bubbled therethrough, and the reaction was continued at 60°C for 2 hrs. After the reaction, the reaction solution was extracted with ethyl acetate (30 mLX2) and saturated brine (10 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(8-chloronaphthalen-1-yl)-5-ethyl-1-fluoro-12-((2-methylenetetrahydro-1H-pyrrolizin-7a(5H)-ylmethoxy)-5a,6,7,8,9,10-hexahydro-5H-4-oxa-3,10a,11,13,14-pentaaza-6,9-methanonaphtho[1,8-ab]hepten-14-carboxylate (38mg, yield: 64.4%).

Step III: A reaction solution of t-butyl (5S,5aS,6S,9R)-2-(8-chloronaphthalen-1-yl)-5-ethyl-1-fluoro-12-((2-methylenetetrahydro-1H-pyrrolizin-7a(5H)-ylmethoxy)-5a, 6,7,8,9,10-hexahydro-5H-4-oxa-3,10a, 11,13,14-pentaaza-6,9-methanonaphtho[1,8-ab]hepten-14-carboxylate (38mg, 0.052 mmol) in acetonitrile (2.0mL) and 4M hydrochloric acid/dioxane solution (0.500mL, 2.000mmol) was reacted at 0°C for 1 hr. The reaction solution was concentrated, and the crude product was purified by preparative HPLC (formic acid method 1) to obtain (5S,5aS,6S,9R)-2-(8-chloronaphthalen-1-yl)-5-ethyl-1-fluoro-12-((2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z596, 12mg, yield:36.8%, formate) as a yellow solid. ES-API: [M+H]⁺=627.3. ¹H NMR (400 MHz, CD₃OD) δ 8.13 (d, *J* = 8.0 Hz, 1H), 8.00 (d, *J =* 7.6 Hz, 1H), 7.82 -7.40 (m, 4H), 5.49 (d, *J =* 13.2 Hz, 1H), 5.28 (s, 2H), 4.57-4.37 (m, 4H), 4.16 -3.59 (m, 4H), 3.52 -3.13 (m, 2H), 3.06 (d, *J =* 16.3 Hz, 1H), 2.78 (d, *J =* 16.0 Hz, 1H), 2.40 (s, 1H), 2.45 -1.70 (m, 10H), 1.16 (t, *J =* 6.5 Hz, 3H).

### Example 31: Synthesis of (5S,5aS,6S,9R)-2-(8-ethynyl-7-fluoronaphthalen-1-yl)-1-fluoro-5-methyl-12-((6'-methylenetetrahydrospiro[cyclopropyl-1,3'-pyrrolizin]-7a'(5'H)-yl)methoxy)-5a,6,7,8,9,10-hexahydro-5H-4-oxy-3,10a,11,13,14-pentaaza-6,9-methanonaphtho[1,8-ab]heptene (Z597)

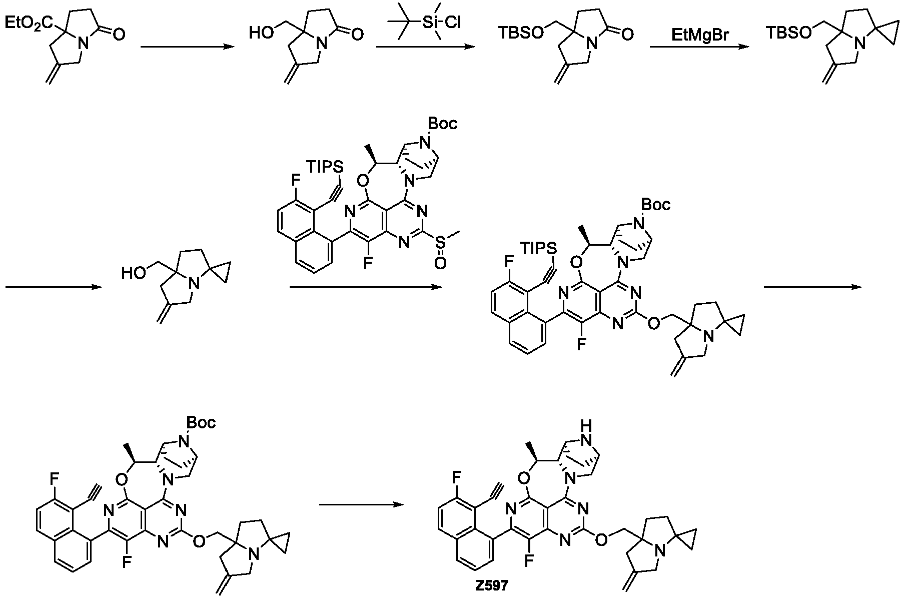

Step I: Under a nitrogen atmosphere, ethyl 2-methylene-5-oxotetrahydro-1H-pyrrolizin-7a(5H)-carboxylate *(1.5* g, 7.17 mmol) and tetrahydrofuran (15 mL) were added to a round-bottom flask. The solution was cooled to 0°C, and a lithium borohydride solution (9 mL, 18 mmol, 2M in tetrahydrofuran) was added dropwise. After that, the reaction was stirred at 0°C for 30 min. Then, the reaction was further stirred at room temperature for 20 min. After cooling to 0°C, 2M hydrochloric acid (10mL) was added dropwise until no bubbles were generated. 2M sodium hydroxide aqueous solution (30 mL) was added until the solution became clear. The reaction solution was extracted with dichloromethane/isopropanol (3:1, 50 mLX3). The organic phase was dried over anhydrous sodium sulfate, and concentrated to obtain 7a-(hydroxymethyl)-6-methylenehexahydro-3H-pyrrolizin-3-one *(1.05* g, yield: 87%) as a colorless oil. ES-API: [M+H]⁺=168.1.

Step II: Sodium hydride (595 mg, 14.87 mmol) and tetrahydrofuran (20 mL) were added to a round-bottom flask. Under a nitrogen atmosphere, the reaction solution was cooled to 0°C. A solution of 7a-(hydroxymethyl)-6-methylenehexahydro-3H-pyrrolizin-3-one (1g, 5.95 mmol) in tetrahydrofuran (2 mL) was added dropwise to the reaction solution with stirring. The reaction solution was stirred at 0°C for 10 min, and t-butylchlorodimethylsilane (1.3 g, 8.93 mmol) was added and stirred at room temperature for 1 hr. The reaction solution was added with a saturated ammonium chloride aqueous solution (50 mL) and extracted with ethyl acetate (50 mLX3). The organic phase was washed with saturated brine, dried over anhydrous sodium sulfate, and concentrated. The crude product was purified by flash silica gel column chromatography (dichloromethane/petroleum ether: 0-100%) to obtain 7a-(((t-butyldimethylsilyl)oxy)methyl)-6-methylenehexahydro-3H-pyrrolizin-3-one (750 mg, yield: 44%) as a colorless oil. ES-API: [M+H]⁺=282.3.

Step III: Under a nitrogen atmosphere, titanium tetraisopropoxide (1.3g, 4.62 mmol) and tetrahydrofuran(10 mL) were added to the reaction flask and cooled to -70°C. An ethylmagnesium bromide solution (9.24mL, 9.24 mmol, 1 M in tetrahydrofuran) was added to the reaction solution, and reacted at -70°C for 5 min with stirring. A solution of 7a-(((t-butyldimethylsilyl)oxy)methyl)-6-methylenehexahydro-3H-pyrrolizin-3-one (650 mg, 2.31 mmol) in tetrahydrofuran (3 mL) was added dropwise to the reaction solution, and stirred for 20 min. Then the ice bath was removed, and the reaction solution was stirred at room temperature for 2 hrs. The reaction solution was added with a saturated sodium bicarbonate aqueous solution (50 mL) and extracted with ethyl acetate (50 mLX3). The organic phase was washed with saturated brine, dried over anhydrous sodium sulfate, and concentrated. The crude product was purified by flash silica gel column chromatography (ethyl acetate /petroleum ether: 0-50%) to obtain 7a'-(((t-butyldimethylsilyl)oxy)methyl)-6'-methylenehexahydrospiro[cyclopropane-1,3'-pyrrolizin](200 mg, yield: 29%) as a yellow oil. ES-API: [M+H]⁺=294.3.

Step IV: 7a'-(((t-butyldimethylsilyl)oxy)methyl)-6'-methylenehexahydrospiro[cyclopropane-1,3'-pyrrolizin](200 mg, 0.68 mmol), methanol (1 mL), and 4M hydrogen chloride/dioxane solution (3 mL) were added to reaction flask. The reaction was stirred at room temperature for 1 hrs. The reaction solution was concentrated, to obtain (6'-methylenetetrahydrospiro[cyclopropane-1,3'-pyrrolizin]-7a'(5'H)-yl)methanol (122 mg, crude product), which was directly used in the next step of reaction without purification. ES-API: [M+H]⁺=180.2.

Step V: (6'-methylenetetrahydrospiro[cyclopropane-1,3'-pyrrolizin]-7a'(5'H)-yl)methanol (34 mg, 0.19 mmol) was dissolved in anhydrous tetrahydrofuran (5 mL). In an iced water bath, sodium hydride (15 mg, 0.38 mmol) was added, and reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, filtered, and concentrated to obtain the crude product t-butyl (5S,5aS,6S,9R)-1-fluoro-2-(7-fluoro-8-((triisopropylsilyl)ethynyl)naphthalen-1-yl)-5-methyl-12-((6'-methylenetetrahydrospiro[cyclopropyl-1,3'-pyrrolizin]-7a'(5'H)-yl)-methoxy)-5a,6,7,8,9,10-hexahydro-5H-4-oxa-3,10a,11,13,14-pentaaza-6,9-methanonaphtho[1,8-ab]hepten-14-carboxylate (26 mg, yield: 46%). ES-API: [M+H]⁺= 903.4.

Step VI: Cesium fluoride (44 mg, 0.29 mmol) was added to t-butyl (5S,5aS,6S,9R)-1-fluoro-2-(7-fluoro-8-((triisopropylsilyl)ethynyl)naphthalen-1-yl)-5-methyl-12-((6'-methylenetetrahydrospiro[cyclopropyl-1,3'-pyrrolizin]-7a'(5'H)-methoxy)-5a,6,7,8,9,10-hexahydro-5H-4-oxa-3,10a,11,13,14-pentaaza-6,9-methanonaphtho[1,8-ab]hepten-14-carboxylate (26 mg, 0.029 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-5-methyl-12-((6'-methylenetetrahydrospiro[cyclopropyl-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]hepten-14-carboxylate (22 mg, yield: 100%). ES-API: [M+H]⁺= 747.3.

Step VII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-((6'-methylenetetrahydrospiro[cyclopropyl-1,3'-pyrrolizin]-7a'(5'H)-methoxy)-5a,6,7,8,9,10-hexahydro-5H-4-oxy-3,10a,11,13,14-pentaaza-6,9-methanonaphtho[1,8-ab]hepten-14-carboxylate (22 mg, 0.029 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) to obtain (5S,5aS,6S,9R)-2-(8-ethynyl-7-fluoronaphthalen-1-yl)-1-fluoro-5-methyl-12-((6'-methylenetetrahydrospiro[cyclopropyl-1,3'-pyrrolizin]-7a'(5'H)-yl)methoxy)-5a,6,7,8,9,10-hexahydro-5H-4-oxa-3,10a,11,13,14-pentaaza-6,9-methanonaphtho [1,8-ab]heptene (Z597, 5 mg, yield: 26%) as a light yellow solid. ES-API: [M+H]⁺= 647.3. ¹H NMR (400 MHz, CD₃OD) δ 8.13 -8.03 (m, 2H), 7.68 -7.54 (m, 2H), 7.49 -7.36 (m, 1H), 5.42 -5.33 (m, 1H), 4.97 (d, *J =* 5.2 Hz, 2H), 4.57-4.51 (m, 1H), 4.45 -4.31 (m, 2H), 4.10-4.05 (m, 1H), 3.68 (d, *J* = 5.6 Hz, 1H), 3.64 -3.58 (m, 2H), 3.54 (s, 1H), 3.49 (d, *J* = 4.0 Hz, 1H), 3.23 -3.14 (m, 1H), 2.84-2.79 (m, 1H), 2.63 (d, *J* = 15.8 Hz, 1H), 2.39 -2.28 (m, 1H), 2.27 -2.16 (m, 1H), 2.13 - 1.97 (m, 2H), 1.93 -1.70 (m, 3H), 1.61 -1.53 (m, 4H), 1.03 -0.95 (m, 1H), 0.95 -0.85 (m, 1H), 0.70-0.60 (m, 2H), 0.53-0.49 (m, 1H).

### Example 32: Synthesis of (5S,5aS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizin]-7a'(5'H)-methoxy)-5a,6,7,8,9,10-hexahydro-4-oxy-3,10a,11,13,14-pentaaza-6,9-methanonaphtho[1,8-ab]heptene (Z599)

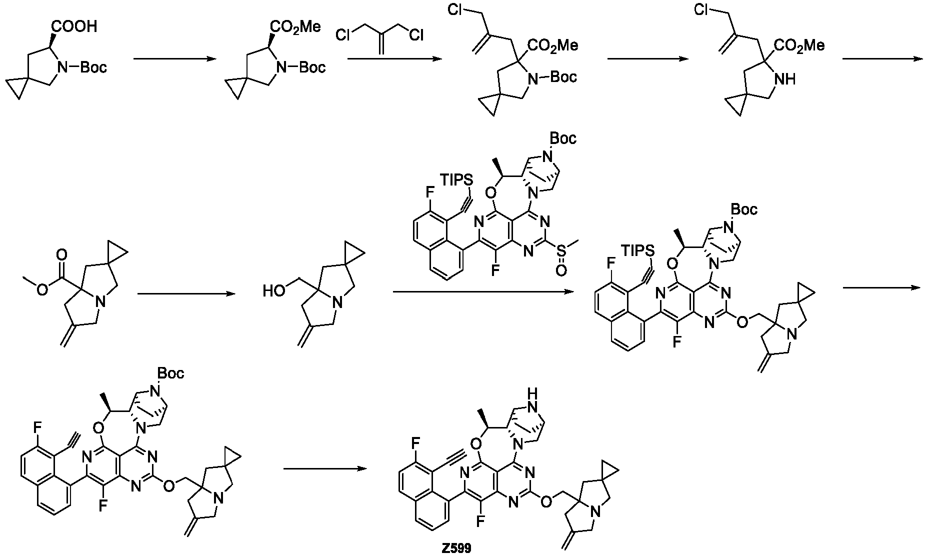

Step I: In an iced water bath, potassium carbonate (8.6 g, 62.25 mmol) and iodomethane (3.8mL, 62.25 mmol) were added to a solution of (S)-5-(t-butoxycarbonyl)-5-azaspiro[2.4]heptan-6-carboxylic acid (10g, 41.5 mmol) in N,N-dimethylformamide (50 mL), and stirred at room temperature for 1.5 hrs. The reaction was shown to be completed by LC/MS, filtered, and washed with ethyl acetate. The filtrate was concentrated under reduced pressure, to obtain 5-(t-butyl) 6-methyl (S)-5-azaspiro[2.4]heptan-5,6-dicarboxylate (11.2g, yield: 71%). ES-API: [M-55]⁺= 200.1.

Step II: Under a nitrogen atmosphere, 5-(t-butyl)6-methyl(S)-5-azaspiro[2.4]heptan-5,6-dicarboxylate (5.0 g, 20 mmol) was dissolved in anhydrous tetrahydrofuran (50mL), and the mixture was allowed to stand in a dry ice-acetone bath. A lithium bis(trimethylsilyl)amide solution (30 mL, 30 mmol, 1 M in THF) was slowly added dropwise to the above solution, and stirred at this temperature for 1 hr. A solution of 3-chloro-2-chloromethylpropylene (12.4 g, 100 mmol) in anhydrous tetrahydrofuran (20 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL), and extracted with ethyl acetate (50 mLX3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 5-(t-butyl) 6-methyl (R)-6-(2-(chloromethyl)allyl)-5-azaspiro[2.4]heptan-5,6-dicarboxylate (5.2g, yield: 75%). ES-API: [M-55]⁺= 288.1.

Step III: At room temperature, 4M hydrochloric acid/dioxane solution (15mL) was slowly added to a solution of 5-(t-butyl)6-methyl(R)-6-(2-(chloromethyl)allyl)-5-azaspiro[2.4]heptan-5,6-dicarboxylate (5.2g, 15 mmol) in acetonitrile(15 mL), and stirred at room temperature for 0.5 hr. The reaction was shown to be completed by LC/MS. The reaction solution was concentrated under reduced pressure, to obtain methyl (R)-6-(2-(chloromethyl)allyl)-5-azaspiro[2.4]heptan-6-carboxylate (3.65 g, yield: 100%). ES-API: [M+H]⁺= 244.1.

Step IV: At room temperature, sodium bicarbonate (6.3 g, 75 mmol) and potassium iodide (250 mg, 1.5 mmol) were added to a solution of methyl (R)-6-(2-(chloromethyl)allyl)-5-azaspiro[2.4]heptan-6-carboxylate (3.65g, 15 mmol) in acetonitrile (50 mL), and stirred at room temperature for 1.5 hrs. The reaction was shown to be completed by LC/MS, filtered, and washed with acetonitrile. The filtrate was concentrated under reduced pressure, and the crude product was purified by flash silica gel column chromatography (petroleum ether: ethyl acetate =15:1) to obtain methyl 6'-methylenedihydro-1'H,3'H-spiro[cyclopropane-1,2'-pyrrolizin]-7a'(5'H)-carboxylate (3.1 g, yield: 100%). ES-API: [M+H]⁺= 208.2.

Step V: Under a nitrogen atmosphere, methyl 6'-methylenedihydro-1'H,3'H-spiro[cyclopropane-1,2'-pyrrolizin]-7a'(5'H)-carboxylate (3.1g, 15 mmol) was dissolved in anhydrous tetrahydrofuran(50mL), and the mixture was allowed to stand in an iced water bath. A lithium aluminium hydride solution (30 mL, 30 mmol, 1 M in THF) was slowly added dropwise to the above solution, and stirred at this temperature for 0.5 hr. The reaction was shown to be completed by LC/MS. The reaction was quenched with sodium sulfafe decahydrate (3.2 g), stirred at room temperature for 0.5 hr, and filtered. The filtrate was concentrated under reduced pressure, to obtain (6'-methylenedihydro-1'H,3'H-spiro[cyclopropane-1,2'-pyrrolizin]-7a'(5'H)-yl)methanol (2.7 g, yield: 100%). ES-API: [M+H]⁺= 180.3.

Step VI: ((6'-methylenedihydro-1'H,3'H-spiro[cyclopropane-1,2'-pyrrolizin]-7a'(5'H)-yl) methanol (34 mg, 0.19 mmol) was dissolved in anhydrous tetrahydrofuran (5 mL). In an iced water bath, sodium hydride (15 mg, 0.38 mmol) was added, and reacted in the iced water bath for 30 min. A solution of t-butyl (SS,SaS,6S,9R)-1-fluoro-2-(7-fluoro-8-((triisopropylsilyl) ethynyl) naphthalen-1-yl)-5-methyl-12-(methylsulfinyl)-5a,6,7,8,9,10-hexahydro-5H-4-oxy-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, filtered, and concentrated to obtain the crude product t-butyl (SS,SaS,6S,9R)-1-fluoro-2-(7-fluoro-8-((triisopropylsilyl) ethynyl)naphthalen-1-yl)-5-methyl-12-((6'-methylenedihydro-1'H,3'H-spiro[cyclopropane-1,2'-pyrrolizin]-7a'(5'H)-methoxy)-5a,6,7,8,9,10-hexahydro-4-oxa-3,10a,11,13,14-pentaaza-6,9-methanonaphtho[1,8-ab]hepten-14-carboxylate (57 mg, yield: 100%). ES-API: [M+H]⁺= 903.4.

Step VII: Cesium fluoride (94 mg, 0.62 mmol) was added to t-butyl (5S,5aS,6S,9R)-1-fluoro-2-(7-fluoro-8-((triisopropylsilyl)ethynyl)naphthalen-1-yl)-5-methyl-12-((6'-methylenedihydro-1'H,3'H-spiro[cyclopropane-1,2'-pyrrolizin]-7a'(5'H)-methoxy)-5a,6,7,8,9,10-hexahydro-4-oxa-3,10a,11,13,14-pentaaza-6,9-methanonaphtho[1,8-ab]hepten-14-carboxylate (57 mg, 0.062 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-5-methyl-12-((6'-methylenedihydro-1'H,3'H-spiro[cyclopropyl-1,2'-pyrrolizin]-7a'(5'H)-methoxy)-5a,6,7,8,9,10-hexahydro-5H-4-oxa-3,10a,11,13,14-pentaaza-6,9-methanonaphtho[1,8-ab]hepten-14-carboxylate (46 mg, yield: 100%). ES-API: [M+H]⁺= 747.3.

Step VIII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-((6'-methylenedihydro-1'H,3'H-spiro[cyclopropyl-1,2'-pyrrolizin]-7a'(5'H)-methoxy)-5a,6,7,8,9,10-hexahydro-5H-4-oxa-3,10a,11,13,14-pentaaza-6,9-methanonaphtho[1,8-ab]hepten-14-carboxylate (46 mg, 0.062 mmol) in acetonitrile (2 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) to obtain (5S,5aS,6S,9R)-2-(8-ethynyl-7-fluoronaphthalen-1-yl)-1-fluoro-5-methyl-12-((6'-methylenedihydro-1'H,3'H-spiro[cyclopropane-1,2'-pyrrolizin]-7a'(5'H)-methoxy)-5a,6,7,8,9,10-hexahydro-4-oxa-3,10a,11,13,14-pentaaza-6,9-methanonaphtho[1,8-ab]heptene (Z599, 5 mg, yield: 12%) as a light yellow solid. ES-API: [M+H]⁺= 647.3.

### Example 33: Synthesis of (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z600)

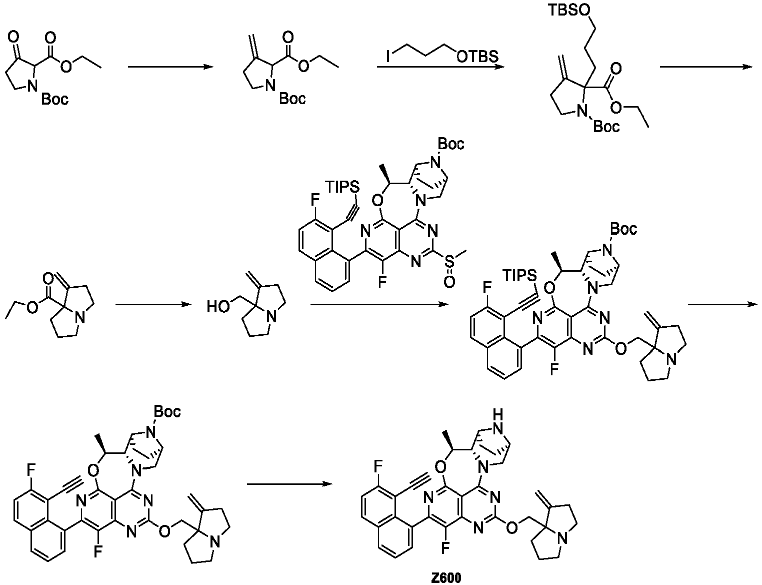

Step I: Methyltriphenylphosphonium bromide (10.41g, 29.15 mmol) was added to tetrahydrofuran (200 mL) and cooled to 0°C. Under a nitrogen atmosphere, a solution of potassium tert-butoxide in tetrahydrofuran (29.15mL, 29.15 mmol, 1M) was added to the mixture at 0°C, and then the reaction was warmed to room temperature and stirred for 1 hr. 1-(t-butyl) 2-ethyl3-oxopyrrolidin-1,2-dicarboxylate (3.0g, 12.82 mmol) was dissolved in tetrahydrofuran (10 mL), and slowly added to the reaction solution under a nitrogen atmosphere. After that, the reaction solution was stirred at room temperature for 3 hrs. The reaction solution was added with saturated ammonium chloride (30 mL), and extracted with ethyl acetate (100 mLX3). The organic phase was washed with saturated brine (200mL), dried over anhydrous sodium sulfate, and concentrated. The crude product was purified by flash silica gel column chromatography (ethyl acetate /petroleum ether: 0%-8%) to obtain 1-(t-butyl) 2-ethyl 3-methylenepyrrolidin-1,2-dicarboxylate (2.0g, yield: 61.6%) as a colorless transparent liquid. ES-API: [M-Boc+H]⁺=200.1.

Step II: A mixture of 1-(t-butyl) 2-ethyl 3-methylenepyrrolidin-1,2-dicarboxylate (0.5g, 1.96 mmol), t-butyl (3-iodopropoxy)dimethylsilane (3.0 g, 9.99 mmol), potassium carbonate(1.61 g, 11.61 mmol), and *N, N-*dimethylformamide (40 mL) was stirred overnight at room temperature under a nitrogen atmosphere. The reaction solution was added with water (100 mL), and extracted with ethyl acetate (100 mL X 4). The organic phase was washed with saturated brine (200 mLX3), dried over anhydrous sodium sulfate, and concentrated. The crude product was purified by flash silica gel column chromatography (ethyl acetate /petroleum ether: 0-10%) to obtain 1-(t-butyl) 2-ethyl 2-(3-((t-butyldimethylsilyl)oxy)propyl)-3-methylenepyrrolidin-1,2-dicarboxylate (720 mg, yield: 86%), as a colorless transparent oil. ES-API: [M-Boc+H]⁺=350.2.

Step III: In an iced water bath, 1-(t-butyl) 2-ethyl 2-(3-((t-butyldimethylsilyl)oxy)propyl)-3-methylenepyrrolidin-1,2-dicarboxylate (720mg, 1.682 mmol) was dissolved in dichloromethane (20mL), and thionyl chloride (7.0mL, 96.47mmol) was added. The reaction solution was stirred overnight at room temperature. The solvent was removed by rotary evaporation to dryness under reduced pressure. A mixed solvent (30mL, dichloromethane: methanol =10:1) was added to the crude product, and finally potassium carbonate (464mg, 3.36mmol) was added, and stirred at room temperature for 1 hr. After filtration, the filtrate was rotary evaporated to dryness under reduced pressure to obtain methyl 1-methylenetetrahydro-1H-pyrrolizin-7a(5H)-carboxylate (190mg, yield: 57%)as a colorless transparent liquid. ES-API: [M-Boc+H]⁺=196.1.

Step IV: Ethyl 1-methylenetetrahydro-1H-pyrrolizin-7a(5H)-carboxylate (190mg, 0.969 mmol) was dissolved in tetrahydrofuran (10mL), and cooled to 0°C. Under a nitrogen atmosphere, a lithium aluminum hydride solution (2.0 mL, 2.0mmol, 1M in tetrahydrofuran) was added to the solution. The reaction was stirred at 0°C for 1 hr. The reaction was quenched with water (0.03 mL), and 15% sodium hydroxide aqueous solution (0.03 mL) and water (0.09 mL) were added. Tetrahydrofuran (10 mL) was added, and filtered. The filtrate was concentrated to obtain (1-methylenetetrahydro-1*H*-pyrrolizin-7a(5H)-yl)methanol (120mg, yield: 80%) as a colorless transparent liquid. ES-API: [M +H]⁺=154.1.

Step V: (1-methylenetetrahydro-1*H*-pyrrolizin-7a(5H)-yl)methanol (60mg, 0.3896 mmol) was dissolved in anhydrous tetrahydrofuran (5 mL). In an iced water bath, sodium hydride (30 mg, 0.780 mmol) was added, and reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate *(50* mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (50 mL), washed with saturated ammonium chloride solution (15 mLX2) and saturated brine (50 mLX3), dried over anhydrous sodium sulfate, and filtered. The crude product was purified by silica gel column chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-((1-methylenetetrahydro)-1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (160 mg, crude product). ES-API: [M+H]⁺= 877.4.

Step VI: Cesium fluoride (96.0 mg, 0.63mmol) was added to t-butyl (5S,5aS,6S,9R)-1-fluoro-2-(7-fluoro-8-((triisopropylsilyl)ethynyl)naphthalen-1-yl)-5-methyl-12-((1-methylenetetrahydro)- 1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (160mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX2) and saturated brine (50 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (200mg, crude product). ES-API: [M+H]⁺= 721.3.

Step VII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-carboxylate (200mg, crude product) in acetonitrile (5.0mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) to obtain (5S,5aS,6S,9R)-2-(8-ethynyl-7-fluoronaphthalen-1-yl)-1-fluoro-5-methyl-12-((1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z600, 6.0 mg, total yield of 3 steps: 2.4%) as a light yellow solid. ES-API: [M+H]⁺= 621.3. ¹H NMR (400 MHz, CD₃OD) δ 8.10 -8.05 (m, 2H), 7.68 -7.52 (m, 2H), 7.46-7.39(m, 1H), 5.38-5.34(m, 1H), 5.10-5.08 (m, 2H), 4.59 -4.47 (m, 1H), 4.39 -4.27 (m, 2H), 4.08-4.06(m, 1H), 3.80 -3.42 (m, 3H), 3.26 -3.06 (m, 3H), 2.89 -2.77 (m, 1H), 2.74 -2.59 (m, 3H), 2.20-2.13(m, 1H), 2.10-2.00(m, 1H), 1.99 - 1.70 (m, 6H), 1.58-1.55 (m, 3H).

### Example 34: Synthesis of (5S, 5aS, 6S, 9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z601)

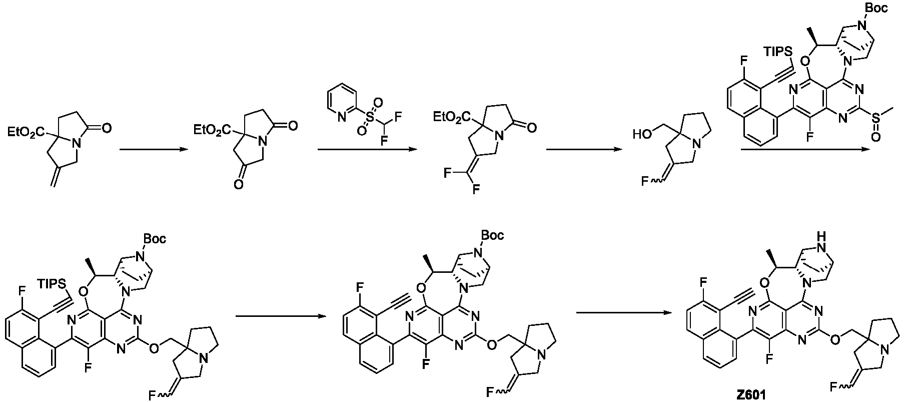

Step I: Under a nitrogen atmosphere, ethyl 2-methylene-5-oxotetrahydro-1H-pyrrolizin-7a(5H)-carboxylate (2g, 9.57mmol), potassium osmate dihydrate (176mg, 0.48mmol), water (40mL), and tetrahydrofuran (20mL) were added to a round-bottom flask, and cooled to 0°C. Sodium periodate (6.14g, 28.71mmol) was added batchwise to the mixture with stirring. The system was stirred at room temperature for 2 hrs. The reaction solution was added with saturated sodiums sulfite aqueous solution (50mL), and extracted with dichloromethane/methanol (10/1) (50mLx5). The organic phase was dried over anhydrous sodium sulfate, and concentrated. The crude product was purified by flash silica gel column chromatography (ethyl acetate /petroleum ether: 0-80%) to obtain ethyl 2,5-dioxotetrahydro-1H-pyrrolizin-7a(5H)-carboxylate (1.4g, yield: 69%) as a brown oil. ES-API: [M+H]⁺=212.1.

Step II: Ethyl 2,5-dioxotetrahydro-1H-pyrrolizin-7a(5H)-carboxylate (1g, 4.74mmol), difluoromethyl(2-pyridinyl)sulfone (1.65g, 8.52mmol) and N,N-dimethylformamide (24mL) were added to a round-bottom flask. Under a nitrogen atmosphere, the reaction solution was cooled to -60°C. A solution of potassium tert-butoxide in tetrahydrofuran (11.8mL, 11.84mmol) was slowly added dropwise to the reaction solution with stirring over 30 min. The reaction was naturally warmed to room temperature, and stirred at room temperature for 4 hrs. The reaction solution was added with a saturated sodium bicarbonate aqueous solution (100mL) and extracted with ethyl acetate (50mLx3). The organic phase was washed with saturated brine, dried over anhydrous sodium sulfate and concentrated. The crude product was purified by flash silica gel column chromatography (ethyl acetate /petroleum ether: 0-60%) to obtain ethyl 2-(difluoromethylene)-5-oxotetrahydro-1H-pyrrolizin-7a(5H)-carboxylate (130mg, yield: 11.1%) as a yellow oil. ES-API: [M+H]⁺=246.1.

Step III: Under a nitrogen atmosphere, ethyl 2-(difluoromethylene)-5-oxotetrahydro-1H-pyrrolizin-7a(5H)-carboxylate (130mg, 0.53mmol) and tetrahydrofuran (5mL) were added to a reaction flask, and cooled to 0°C. A 1*M* lithium aluminium hydride solution in tetrahydrofuran (2.1mL, 2.10mmol) was added dropwise to the reaction solution with stirring. The cold bath was removed and the reaction was stirred at 65°C for 2 hrs. After cooling to 0°C, the reaction was quenched with sodium sulfafe decahydrate, and filtered. The filter cake was washed with tetrahydrofuran. The filtrate was dried over anhydrous sodium sulfate, and concentrated to obtain (2-(fluoromethylene)tetrahydro-1H-pyrrolizin-7a(5*H*)-yl)methanol (90 mg, crude product) as a yellow oil. ES-API: [M+H]⁺=172.1.

Step IV: (2-(fluoromethylene)tetrahydro-1H-pyrrolizin-7a(5H)-yl)methanol (54 mg, 0.32 mmol) was dissolved in anhydrous tetrahydrofuran (3 mL). In an iced water bath, sodium hydride (12 mg, 0.31 mmol) was added and reacted for 20 min in the iced water bath. A solution of t-butyl (5s,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) in tetrahydrofuran (1 mL) was added dropwise to the reaction solution, and reacted at 0°C for 20 min. The reaction was shown to be completed by LC/MS. The reaction solution was added with a saturated ammonium chloride solution (30 mL). The reaction solution was extracted with ethyl acetate (20 mLX2). The organic phase was washed with saturated brine (15 mLX3), dried over anhydrous sodium sulfate, filtered, and concentrated to obtain t-butyl (5S,5aS,6S,9R)-1-fluoro-2-(7-fluoro-8-((triisopropylsilyl) ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (55 mg, crude product). ES-API: [M+H]⁺=895.3.

Step V: Cesium fluoride (55 mg, 0.61 mmol) was added to t-butyl (5S,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (55 mg, crude product) in N,N-dimethylformamide (2mL), and the reaction mixture was stirred at room temperature for 1 hr. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-12-((2-(fluoromethylene) tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40 mg, crude product). ES-API: [M+H]⁺= 739.3.

Step VI: In an iced water bath, 4 M hydrochloric acid-dioxane solution (0.68 mL) was slowly added to a solution of t-butyl (5S,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (40 mg, crude product) in acetonitrile(3 mL). The reaction mixture was stirred at 0°C for 1 hr. The reaction was shown to be completed by LC/MS. The reaction solution was concentrated to dryness, and the residue was purified by preparative HPLC (ammonium bicarbonate method 2) to obtain (5S, 5aS, 6S, 9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z601, 5 mg, yield: 14.4%) as a white solid. ES-API: [M+H]⁺= 639.3. ¹H NMR (400MHz, CD₃OD): 8.11-8.05(m, 2H), 7.66-7.54(m, 2H), 7.45-7.19(m, 1H), 6.82-6.54(m, 1H), 5.38-5.35(m, 1H), 4.57-4.50(m, 1H), 4.30-4.20(m, 2H), 4.09-4.07(m, 1H), 3.86-3.69(m, 2H), 3.59-3.57(m, 1H), 3.47-3.44(m, 1H), 3.35-3.34(m, 1H), 3.21-3.10(m, 2H), 2.85-2.66(m, 2H), 2.57-2.42(m, 1H), 2.15-1.77(m, 8H), 1.59-1.56(m, 3H).

### Example 35: Synthesis of (5S,5aS,6S,9R)-2-(8-ethynyl-7-fluoronaphthalen-1-yl)-1-fluoro-12-(((1aR,6aR)-hexahydrocyclopropane[b]pyrrolizin-5a(3H)-yl)methoxy)-5-methyl-5a,6,7,8,9,10-hexahydro-5H-4-oxy-3,10a,11,13,14-pentaaza-6,9-methanonaphtho[1,8-ab]heptene (Z624)

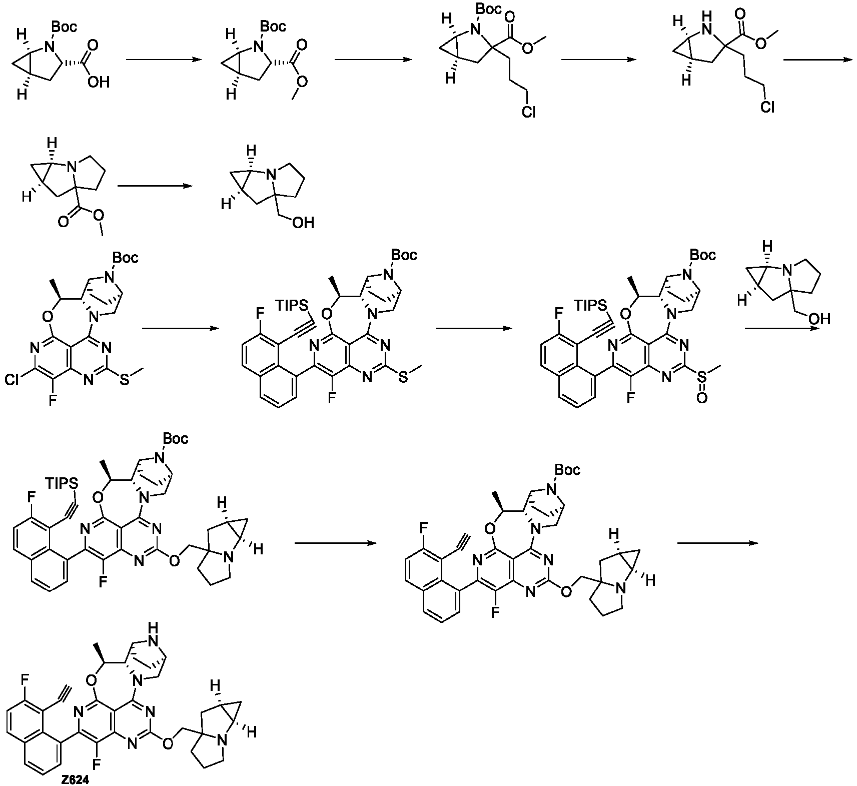

Step I: In an iced water bath, potassium carbonate (3.65 g, 26.4 mmol) and iodomethane (1.6mL, 26.4 mmol) were added to a solution of (1R,3S,5R)-2-(t-butoxycarbonyl)-2-azabicyclo [3.1.0]hexane-3-carboxylic acid (2g, 8.8 mmol) in N,N-dimethylformamide (50 mL), and stirred at room temperature for 1.5 hrs. The reaction was shown to be completed by LC/MS, filtered, and washed with ethyl acetate. The filtrate was concentrated under reduced pressure, to obtain 2-(t-butyl)3-methyl(1R,3S,5R)-2-azabicyclo[3.1.0]hexane-2,3-dicarboxylate (2.01g, yield: 95%). ES-API: [M-100]⁺= 142.1.

Step II: Under a nitrogen atmosphere, 2-(t-butyl) 3-methyl (1R,3S,5R)-2-azabicyclo [3.1.0]hexane-2,3-dicarboxylate (1.0 g, 4.1 mmol) was dissolved in anhydrous tetrahydrofuran (10mL), and the mixture was allowed to stand in a dry ice-acetone bath. A solution of lithium bis(trimethylsilyl)amide (8.3 mL, 8.3 mmol, 1 M in tetrahydrofuran) was slowly added dropwise to the above solution, and stirred at this temperature for 1 hr. A solution of 1-chloro-3-iodopropane (4.24 g, 20.7 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL), and extracted with ethyl acetate (50 mLX3). The organic phase was washed with saturated brine (50 mLX3), dried over anhydrous sodium sulfate, filtered, and concentrated, to obtain a crude product. The crude product was purified by flash silica gel column chromatography (petroleum ether: ethyl acetate =20:1) to obtain 2-(t-butyl) 3-methyl (1R,5R)-3-(3-chloropropyl)-2-azabicyclo[3.1.0]hexane-2,3-dicarboxylate (1.1g, yield: 83.5%). ES-API: [M-100]⁺= 218.2.

Step III: At room temperature, 4M hydrochloric acid/dioxane solution (15mL) was slowly added to a solution of 2-(t-butyl)3-methyl(1R,5R)-3-(3-chloropropyl)-2-azabicyclo[3.1.0]hexane-2,3-dicarboxylate (1.1g, 3.46 mmol) in acetonitrile(3 mL), and stirred at room temperature for 0.5 hr. The reaction was shown to be completed by LC/MS. The reaction solution was concentrated under reduced pressure, to obtain methyl (1R,5R)-3-(3-chloropropyl)-2-azabicyclo[3.1.0]hexane-3-carboxylate (0.75 g, yield: 100%). ES-API: [M+H]⁺= 218.2.

Step IV: At room temperature, sodium bicarbonate (1.4 g, 17.2 mmol) and potassium iodide (57 mg, 0.34 mmol) were added to methyl (1R,5R)-3-(3-chloropropyl)-2-azabicyclo[3.1.0]hexane-3-carboxylate (0.75 g, 3.4 mmol) in acetonitrile (10 mL), and stirred at room temperature for 1.5 hrs. The reaction was shown to be completed by LC/MS, filtered, and washed with acetonitrile. The filtrate was concentrated under reduced pressure, to obtain a crude product. The crude product was purified by flash silica gel column chromatography (petroleum ether: ethyl acetate =15:1) to obtain methyl (1aR,6aR)-hexahydrocyclopropane[b]pyrrolizin-5a(3H)-carboxylate (500 mg, yield:80%). ES-API: [M+H]⁺=182.2.

Step V: Under a nitrogen atmosphere, methyl (1aR,6aR)-hexahydrocyclopropane[b]pyrrolizin-5a(3H)-carboxylate (500 mg, 2.76 mmol) was dissolved in anhydrous tetrahydrofuran (10 mL), and the mixture was allowed to stand in an iced water bath. A solution of lithium aluminium hydride (5.5 mL, 5.5 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (500 mg), stirred at room temperature for 0.5 hr, and filtered. The filtrate was concentrated under reduced pressure, to obtain ((1aR,6aR)-hexahydrocyclopropane[b]pyrrolizin-5a(3H)-yl)methanol (300 mg, yield: 71%). ES-API: [M+H]⁺= 154.1.

Step VI: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (150mg, 0.311 mmol), ((2-fluoro-8-(4,4,5,5-tetramethyl-1,3,2-dioxaborolane -2-yl)naphthalen-1-yl)ethynyl)triisopropylsilane (211 mg, 0.466mmol), potassium phosphate (132 mg, 0.62 mmol), and [n-butyldi(1-adamantanyl)phosphine](2-amino-1,1'-biphenyl-2-yl)palladium (II) mesylate (23 mg, 0.031 mmol) were dissolved in tetrahydrofuran (6.0mL) and water (1.5 mL). The reaction mixture was heated to 80°C under microwave and stirred for 40 min. The reaction was shown to be completed by LC/MS. The reaction solution was concentrated under reduced pressure. The crude product was purified by silica gel column chromatography (dichloromethane: methanol =20:1) to obtain t-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a, 11, 13,14-pentaaza-6,9-methanonaphtho[1,8-ab]hepten-14-carboxylate (290 mg, crude product). ES-API: [M+H]⁺= 772.3.

Step VII: T-butyl (5S,5aS,6S,9R)-1-fluoro-2-(7-fluoro-8-((triisopropylsilyl)ethynyl) naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (290 mg, crude product) was dissolved in dichloromethane (10.0mL). In an iced water bath, m-chloroperoxybenzoic acid (70 mg, 0.406mmol) was added, and reacted for 30 min in the iced water bath. The reaction was shown to be completed by LC/MS. The reaction solution was diluted with dichloromethane (20 mL), washed with saturated sodium sulfite solution (15 mLX2) and saturated brine (15 mLX3), dried over anhydrous sodium sulfate, filtered, and concentrated to obtain the crude product t-butyl (SS,SaS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (280 mg, crude product). ES-API: [M+H]⁺= 788.3.

Step VIII: ((1aR,6aR)-hexahydrocyclopropane[b]pyrrolizin-5a(3H)-yl)methanol (29 mg,0.19 mmol) was dissolved in anhydrous tetrahydrofuran (5 mL). In an iced water bath, sodium hydride (15 mg,0.38 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, and filtered. The crude product was purified by silica gel column chromatography ((dichloromethane: methanol =15:1) mobile phase) to obtain t-butyl (5S,5aS,6S,9R)-1-fluoro-2-(7-fluoro-8-((triisopropylsilyl)ethynyl)naphthalen-1-yl)-12-((1aR,6aR)-hexahydrocyclopropane[b]pyrrolizin-5a(3H)-yl)methoxy)-5-methyl-5a,6,7,8,9,10-hexahydro-4-oxa-3,10a,11,13,14-pentaaza-6,9-methanonaphtho[1,8-ab]hepten-14-carboxylate (20 mg, yield: 36%). ES-API: [M+H]+= 877.5.

Step IX: Cesium fluoride (34 mg, 0.22 mmol) was added to t-butyl (5S,5aS,6S,9R)-1-fluoro-2-(7-fluoro-8-((triisopropylsilyl)ethynyl)naphthalen-1-yl)-12-((1aR,6aR)-hexahydrocyclopropane [b]pyrrolizin-5a(3H)-yl)methoxy)-5-methyl-5a,6,7,8,9,10-hexahydro-4-oxy-3,10a,11,13,14-pentaaza-6,9-methanonaphtho[1,8-ab]hepten-14-carboxylate (20 mg, 0.023 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-12-(((1aR,6aR)-hexahydrocyclopropane[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (16 mg, yield: 100%). ES-API: [M+H]+= 721.3

Step X: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-12-(((1aR,6aR)-hexahydrocyclopropane[b]pyrrolizin-5a(3H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (16 mg, 0.023 mmol) in acetonitrile (1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-(((1aR,6aR)-hexahydrocyclopropane[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptene (Z624, 1.2 mg, yield: 8.4%) as a light yellow solid. ES-API: [M+H]⁺= 621.3.

### Example 36: Synthesis of (5S,5aS,6S,9R)-2-(8-ethynyl-7-fluoronaphthalen-1-yl)-1-fluoro-12-(((1aS,6aS)-hexahydrocyclopropane[b]pyrrolizin-5a(3H)-yl)methoxy)-5-methyl-5a,6,7,8,9,10-hexahydro-5H-4-oxy-3,10a,11,13,14-pentaaza-6,9-methanonaphtho[1,8-ab]heptene (Z625)

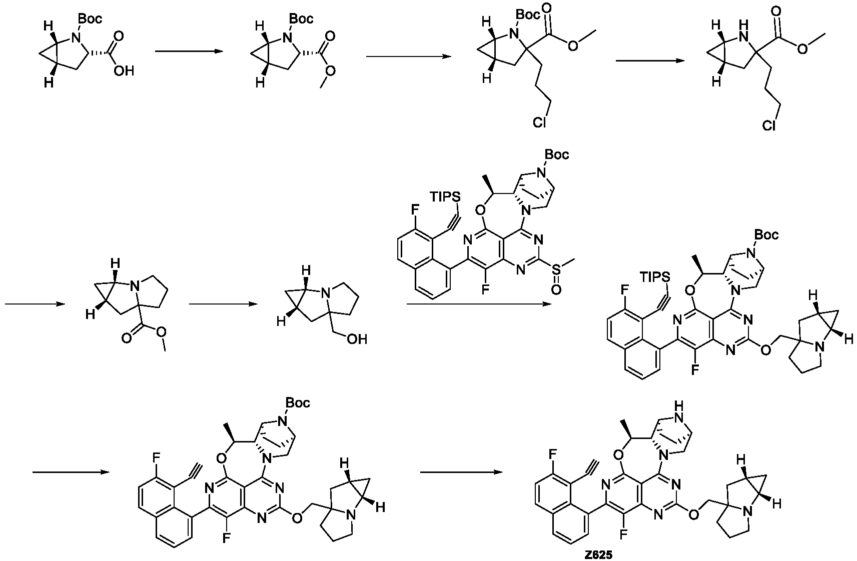

Step I: In an iced water bath, potassium carbonate (3.65 g, 26.4 mmol) and iodomethane (1.6mL, 26.4 mmol) were added to a solution of (1S,3S,5S)-2-(t-butoxycarbonyl)-2-azabicyclo [3.1.0]hexane-3-carboxylic acid (2g, 8.8 mmol) in N,N-dimethylformamide (50 mL), and stirred at room temperature for 1.5 hrs. The reaction was shown to be completed by LC/MS, filtered, and washed with ethyl acetate. The filtrate was concentrated under reduced pressure, to obtain 2-(t-butyl)3-methyl(1S,3S,5S)-2-azabicyclo[3.1.0]hexane-2,3-dicarboxylate (2.05g, yield: 96%). ES-API: [M-100]⁺= 142.1.

Step II: Under a nitrogen atmosphere, 2-(t-butyl) 3-methyl (1S,3S,5S)-2-azabicyclo [3.1.0]hexane-2,3-dicarboxylate (1.0 g, 4.1 mmol) was dissolved in anhydrous tetrahydrofuran (10 mL), and the mixture was allowed to stand in a dry ice-acetone bath. A solution of lithium bis(trimethylsilyl)amide (8.3 mL, 8.3 mmol, 1 M in tetrahydrofuran) was slowly added dropwise to the above solution, and stirred at this temperature for 1 hr. A solution of 1-chloro-3-iodopropane (4.24 g, 20.7 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL) and extracted with ethyl acetate (50 mLX3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 2-(t-butyl) 3-methyl (1S,5S)-3-(3-chloropropyl)-2-azabicyclo[3.1.0]hexane-2,3-dicarboxylate (1.2g, yield: 91%). ES-API: [M-100]⁺= 218.2.

Step III: At room temperature, 4M hydrochloric acid/dioxane solution (15mL) was slowly added to a solution of 2-(t-butyl)3-methyl(1S,5S)-3-(3-chloropropyl)-2-azabicyclo[3.1.0]hexane-2,3-dicarboxylate (1.2g, 3.7 mmol) in acetonitrile(3 mL), and stirred at room temperature for 0.5 hr. The reaction was shown to be completed by LC/MS. The reaction solution was concentrated under reduced pressure, to obtain methyl (1S,5S)-3-(3-chloropropyl)-2-azabicyclo[3.1.0]hexane-3-carboxylate (0.82 g, yield: 100%). ES-API: [M+H]⁺= 218.2.

Step IV: At room temperature, sodium bicarbonate *(1.5* g, 18.8 mmol) and potassium iodide (63 mg, 0.37 mmol) were added to methyl (1S,5S)-3-(3-chloropropyl)-2-azabicyclo[3.1.0]hexane-3-carboxylate (0.82 g, 3.7mmol) in acetonitrile (10 mL), and stirred at room temperature for 1.5 hrs. The reaction was shown to be completed by LC/MS, filtered, and washed with acetonitrile. The filtrate was concentrated under reduced pressure, and the crude product was purified by flash silica gel column chromatography (petroleum ether: ethyl acetate =15:1) to obtain methyl (1aS,6aS)-hexahydrocyclopropane[b]pyrrolizin-5a(3H)-carboxylate (521mg, yield:76%). ES-API: [M+H]⁺=182.1.

Step V: Under a nitrogen atmosphere, methyl (1aS,6aS)-hexahydrocyclopropane[b]pyrrolizin-5a(3H)-carboxylate (521mg, 2.87 mmol) was dissolved in anhydrous tetrahydrofuran (10 mL), and the mixture was allowed to stand in an iced water bath. A solution of lithium aluminium hydride (5.74 mL, 5.74 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (521 mg), stirred at room temperature for 0.5 hr, and filtered. The filtrate was concentrated under reduced pressure, to obtain ((1aS,6aS)-hexahydrocyclopropane[b]pyrrolizin-5a(3H)-yl)methanol (410 mg, yield: 93 %). ES-API: [M+H]⁺= 154.1.

Step VI: ((1aS,6aS)-hexahydrocyclopropane[b]pyrrolizin-5a(3H)-yl)methanol (29 mg, 0.19 mmol) was dissolved in anhydrous tetrahydrofuran (5 mL). In an iced water bath, sodium hydride (15 mg, 0.38 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, and filtered. The crude product was purified by silica gel column chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-12-((1aS,6aS)-hexahydrocyclopropane[b]pyrrolizin-5a(3H)-yl)methoxy)-5-methyl-5a,6,7,8,9,10-hexahydro-4-oxa-3,10a,11,13,14-pentaaza-6,9-methanonaphtho[1,8-ab]hepten-14-carboxylate (30 mg, yield: 53%). ES-API: [M+H]⁺= 877.5.

Step VII: Cesium fluoride (52 mg, 0.34 mmol) was added to t-butyl (5S,5aS,6S,9R)-1-fluoro-2-(7-fluoro-8-((triisopropylsilyl)ethynyl)naphthalen-1-yl)-12-((1aS,6aS)-hexahydrocyclopropane[b]pyrrolizin-5a(3H)-yl)methoxy)-5-methyl-5a,6,7,8,9,10-hexahydro-4-oxa-3,10a,11,13,14-pentaaza-6,9-methanonaphtho[1,8-ab]hepten-14-carboxylate (30 mg, 0.034 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S,5aS,6S,9R)-2-(8-ethynyl-7-fluoronaphthalen-1-yl)-1-fluoro-12-(((1aS,6aS)-hexahydrocyclopropane[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, yield: 100%). ES-API: [M+H]⁺= 721.3.

Step VIII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-12-(((1aS,6aS)-hexahydrocyclopropane[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.034 mmol) in acetonitrile(1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-(((1aS,6aS)-hexahydrocyclopropane[b]pyrrolizin-5a(3H)-yl)methoxy)-5-methyl-5a,6,7,8,9,10-hexahydro-5H-4-oxa-3,10a,11,13,14-pentaaza-6,9-methanonaphtho[1,8-ab]heptene (Z625, 4 mg, yield: 18.9%) as a light yellow solid. ES-API: [M+H]⁺= 621.3.

### Example 37: Synthesis of (5S,5aS,6S,9R)-2-(8-ethynyl-7-fluoronaphthalenyl-1-yl)-1-fluoro-12-(((1aR,6aR,6bS)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho [1,8-ab]heptene (Z626-1) and (5S,5aS,6S,9R)-2-(8-ethynyl-7-fluoronaphthalenyl-1-yl)-1-fluoro-12-(((1aR,6aS,6bS)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]heptene (Z626-2)

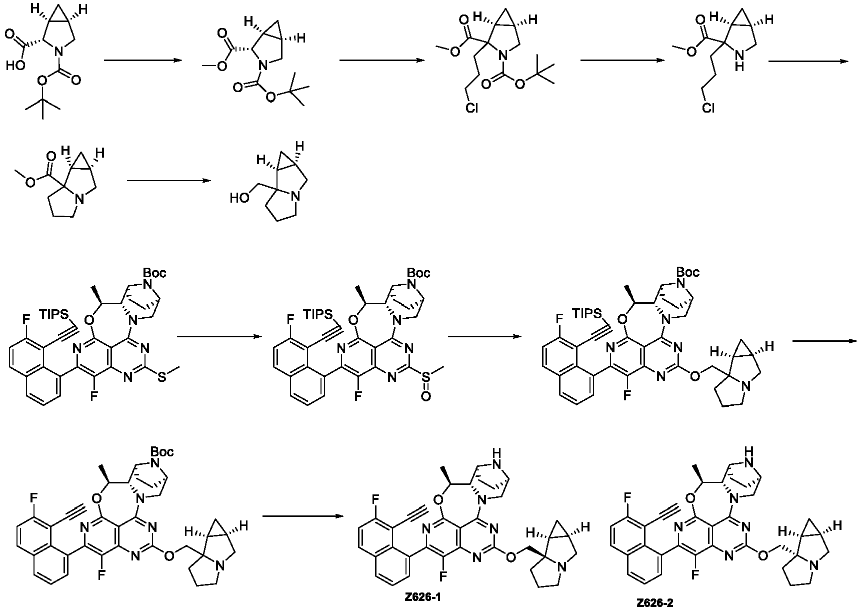

Step I: In a sealed tube, potassium carbonate (276 mg, 2.0 mmol) and iodomethane (0.19 mL, 3.0 mmol) were added to a solution of (1S,2S,5R)-3-(t-butoxycarbonyl)-3-azabicyclo [3.1.0]hexane-2-carboxylic acid (227 mg, 1.0 mmol) in N,N-dimethylformamide (10 mL). The reaction mixture was stirred at room temperature for 2 hrs. The reaction solution was diluted with water, and extracted three times with petroleum ether. The organic phase was washed with saturated brine, dried over anhydrous sodium sulfate, filtered, and concentrated. The obtained crude product 3-(t-butyl) 2-methyl (1S,2S,5R)-3-azabicyclo[3.1.0]hexane-2,3-dicarboxylate was directly used in the next step of reaction. ES-API: [M-55]⁺= 186.1.

Step II: Under a nitrogen atmosphere, 3-(t-butyl) 2-methyl (1S,2S,5R)-3-azabicyclo [3.1.0]hexane-2,3-dicarboxylate crude product was dissolved in anhydrous tetrahydrofuran (5.0 mL), and the mixture was allowed to stand in a dry ice-ethanol bath. Lithium bis(trimethylsilyl)amide (1.7 mL, 1.7 mmol, 1 M in tetrahydrofuran) was slowly added dropwise to the above solution, and stirred at this temperature for 15 min. Then 1-chloro-3-iodopropane (529 mg, 2.6 mmol) was slowly added to the reaction solution. The reaction was stirred at -78°C for 1 hr, and then quenched with a saturated ammonium chloride solution. The reaction solution was extracted three times with ethyl acetate. The organic phase was washed with saturated brine, dried over anhydrous sodium sulfate, filtered and concentrated. The crude product was purified by flash silica gel column chromatography (ethyl acetate /petroleum ether: 0-15%) to obtain 3-(t-butyl) 2-methyl (1S,5R)-2-(3-chloropropyl)-3-azabicyclo[3.1.0]hexane-2,3-dicarboxylate (236 mg, yield: 86%). ES-API: [M-99]⁺= 218.1.

Step III: At room temperature, 4 M hydrochloric acid-dioxane solution (1.0 mL) was slowly added to a solution of 3-(t-butyl) 2-methyl (1S,5R)-2-(3-chloropropyl)-3-azabicyclo[3.1.0]hexane-2,3-dicarboxylate (209 mg, 0.66 mmol) in acetonitrile (10.0 mL). The reaction mixture was stirred at room temperature for 1 hr. The reaction solution was concentrated to dryness under reduced pressure to obtain methyl (1S,5R)-2-(3-chloropropyl)-3-azabicyclo[3.1.0]hexane-2-carboxylate, and the crude product was directly used in the next step without purification. ES-API: [M+H]⁺= 218.1.

Step IV: At room temperature, sodium bicarbonate (276 mg, 3.3 mmol) and potassium iodide (11 mg, 0.07 mmol) were sequentially added to a solution of methyl (1S,5R)-2-(3-chloropropyl)-3-azabicyclo[3.1.0]hexane-2-carboxylate in acetonitrile (30.0 mL), and the reaction mixture was heated and stirred under reflux for 2 hrs. After filtration, the filtrate was concentrated under reduced pressure. The obtained crude product was purified by flash silica gel column chromatography (methanol/dichloromethane: 0-10%) to obtain methyl (1ar,6bS)-hexahydrocyclopropane [a]pyrrolizin-6a(4H)-ylcarboxylate (101 mg, yield: 85%). ES-API: [M+H]⁺= 182.2.

Step V: Under a nitrogen atmosphere, methyl (1ar,6bS)-hexahydrocyclopropane[a]pyrrolizin-6a(4H)-carboxylate (101 mg, 0.56 mmol) was dissolved in anhydrous tetrahydrofuran(10.0 mL), and the mixture was allowed to stand in an iced water bath. Lithium aluminium hydride (0.84 mL, 0.84 mmol, 1 M in tetrahydrofuran) was slowly added dropwise to the solution, and stirred at this temperature for 0.5 hr. Sodium sulfafe decahydrate was slowly added to quench to reaction, heated to room temperature, stirred for 0.5 hr, and filtered. The filtrate was concentrated under reduced pressure, to obtain ((1ar,6bS)-hexahydrocyclopropane[a]pyrrolizin-6a(4H)-yl)methanol (83 mg, yield: 97%). ES-API: [M+H]⁺= 154.3.

Step VI: At 0°C, m-chloroperoxybenzoic acid (17 mg, 0.08 mmol, 85% purity) was added to a solution of t-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptan-14-carboxylate (50 mg, 0.065 mmol) in dichloromethane (5.0 mL), and sitrred at 0°C for 0.5 hr. A saturated sodium bicarbonate aqueous solution and a saturated sodium sulfite aqueous solution were added, and vigorously stirred for 15 min. The organic phase was separated, and the aqueous phase was extracted with dichloromethane. The organic phase was collected, washed with saturated brine, dried over anhydrous sodium sulfate, filtered, and evaporated to dryness, to obtain t-butyl (5S,5aS,6S,9R)-1-fluoro-2-(7-fluoro-8-((triisopropylsilyl) ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate. The crude product was directly used in the next step of reaction without purification. ES-API: [M+H]⁺= 788.3.

Step VII: At 0°C, sodium hydride (60% dispersed in an oil) (10 mg, 0.26 mmol) was added to ((1aR,6bS)-hexahydrocyclopropane[a]pyrrolizin-6a(4H)-yl)methanol (25 mg, 0.16 mmol) in tetrahydrofuran (5.0 mL), and reacted for 15 min. Then, a solution of the crude product t-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate in tetrahydrofuran (1.0 mL) was added dropwise to the reaction solution. After reaction at 0°C for 0.5 hr, a saturated ammonium chloride aqueous solution was slowly added to the reaction solution, and the reaction solution was extracted three times with dichloromethane. The organic phase was collected, washed with saturated brine, dried over anhydrous sodium sulfate, filtered and evaporated to dryness. The crude product was purified by preparative TLC (ethyl acetate /petroleum ether 80%) to obtain the product t-butyl (5S,5aS,6S,9R)-1-fluoro-2-(7-fluoro-8-((triisopropylsilyl)ethynyl)naphthalen-1-yl)-12-(((1aR,6bS)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]hepten-14-carboxylate (23 mg, yield: 40%). ES-API: [M+H]⁺= 877.4.

Step VIII: T-butyl (5S,5aS,6S,9R)-1-fluoro-2-(7-fluoro-8-((triisopropylsilyl)ethynyl) naphthalen-1-yl)-12-(((1aR,6bS)-hexahydrocyclopropane[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.026 mmol) was dissolved in N,N-dimethylformamide (1.0 mL), cesium fluoride (40 mg, 0.26 mmol) was added, and reacted at room temperature for 1 hr. After the reaction, the reaction solution was added with water, and extracted three times with ethyl acetate. The organic phases were combined, washed with saturated brine, dried over anhydrous sodium sulfate, and filtered. The filtrate was rotary evaporated to dryness under reduced pressure, to obtain t-butyl (5S,5aS,6S,9R)-2-(8-ethynyl-7-fluoronaphthalenyl-1-yl)-1-fluoro-12-(((1aR,6bS)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]hepten-14-carboxylate. The crude product was directly used in the next step of reaction without purification. ES-API: [M+H]⁺= 721.3.

Step IX: The crude product t-butyl (5S,5aS,6S,9R)-2-(8-ethynyl-7-fluoronaphthalenyl-1-yl)-1-fluoro-12-(((1aR,6bS)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-nonaphtho[1,8-ab]hepten-14-carboxylate was dissolved in acetonitrile (5.0 mL). In an ice bath, 4M hydrochloric acid/dioxane solution (1.0 mL, 4.0 mmol) was added, reacted at 0 °C for 0.5 hr, heated to room temperature and reacted for 0.5 hr. After the reaction, the reaction solution was removed by rotary evaporation to dryness under reduced pressure to remove the solvent. The crude product was purified by preparative HPLC (ammonium bicarbonate method 1) (chromatographic condition: 15mL-55-95-13min) to obtain two isomers. One isomer was arbitrarily designated as (5S,5aS,6S,9R)-2-(8-ethynyl-7-fluoronaphthalenyl-1-yl)-1-fluoro-12-(((1aR, 6aR, 6bS)-hexahydrocyclopropane [a]pyrrolizin-6a(4H)-yl)methoxy)-5-methyl-5a,6,7,8,9,10-hexahydro-5H-4-oxa-3,10a,11,13,14-pentaaza-6,9-nonaphtho[1,8-ab]heptene (Z262-1, retention time7.03 min, 0.40 mg, yield: 2.5%), ES-API: [M+H]⁺= 621.3. The other isomer was arbitrarily designated as (5S,5aS,6S,9R)-2-(8-ethynyl-7-fluoronaphthalenyl-1-yl)-1-fluoro-12-(((1aR, 6aS, 6bS)-hexahydrocyclopropane [a]pyrrolizin-6a(4H)-yl)methoxy)5-methyl-5a,6,7,8,9,10-hexahydro-5H-4-oxa-3,10a,11,13,14-pentaaza-6,9-nonaphtho[1,8-ab]heptene (Z262-2, retention time8.22 min, 1.30 mg, yield: 8.1%), ES-API: [M+H]⁺= 621.3.

### Example 38: Synthesis of (5S,5aS,6S,9R)-2-(8-ethynyl-7-fluoronaphthalenyl-1-yl)-1-fluoro-12-(((1a8,6aR,6bR)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho [1,8-ab]heptene (Z627-1) and (5S,5aS,6S,9R)-2-(8-ethynyl-7-fluoronaphthalenyl-1-yl)-1-fluoro-12-(((1aS,6aS,6bR)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]heptene (Z627-2)

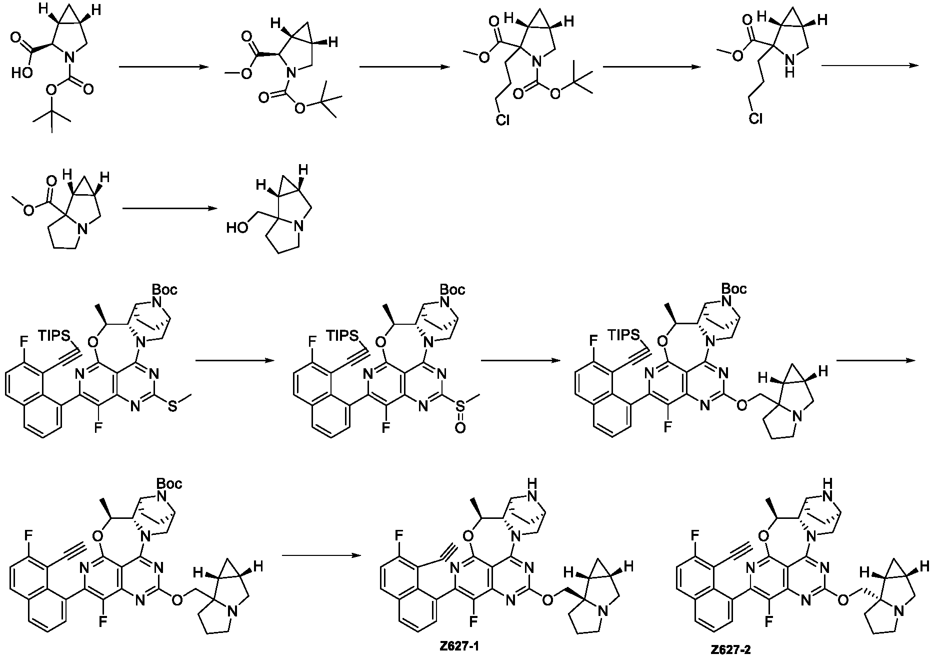

Step I: In a sealed tube, potassium carbonate (276 mg, 2.0 mmol) and iodomethane (0.19 mL, 3.0 mmol) were added to a solution of (1R,2R,5S)-3-(t-butoxycarbonyl)-3-azabicyclo [3.1.0]hexane-2-carboxylic acid (227 mg, 1.0 mmol) in N,N-dimethylformamide (10.0 mL). The reaction mixture was stirred at room temperature for 2 hrs. The reaction solution was diluted with water, and extracted three times with petroleum ether. The organic phase was washed with saturated brine, dried over anhydrous sodium sulfate, filtered, and concentrated. The obtained crude product 3-(t-butyl) 2-methyl (1R,2R,5S)-3-azabicyclo[3.1.0]hexane-2,3-dicarboxylate was directly used in the next step of reaction. ES-API: [M-55]⁺= 186.1.

Step II: Under a nitrogen atmosphere, 3-(t-butyl) 2-methyl (1R,2R,5S)-3-azabicyclo [3.1.0]hexane-2,3-dicarboxylate crude product was dissolved in anhydrous tetrahydrofuran (5.0 mL), and the mixture was allowed to stand in a dry ice-ethanol bath. Lithium bis(trimethylsilyl)amide (2.5 mL, 2.5 mmol, 1 M tetrahydrofuran) was slowly added dropwise to the above solution, and stirred at this temperature for 15 min. Then 1-chloro-3-iodopropane (613 mg, 3.0 mmol) was slowly added to the reaction solution. The reaction was stirred at -78°C for 2 hr, and then quenched with a saturated ammonium chloride solution. The reaction solution was extracted three times with ethyl acetate. The organic phase was washed with saturated brine, dried over anhydrous sodium sulfate, filtered and concentrated. The crude product was purified by flash silica gel column chromatography (ethyl acetate /petroleum ether: 0-15%) to obtain 3-(t-butyl) 2-methyl (1R,5S)-2-(3-chloropropyl)-3-azabicyclo[3.1.0]hexane-2,3-dicarboxylate (191 mg, yield: 60%). ES-API: [M-99]⁺= 218.1.

Step III: At room temperature, 4 M hydrochloric acid-dioxane solution (1.0 mL) was slowly added to a solution of 3-(t-butyl) 2-methyl (1R,5S)-2-(3-chloropropyl)-3-azabicyclo[3.1.0]hexane-2,3-dicarboxylate (191 mg, 0.60 mmol) in acetonitrile (10 mL). The reaction mixture was stirred at room temperature for 1 hr, and concentrated to dryness under reduced pressure to obtain methyl (1R,5S)-2-(3-chloropropyl)-3-azabicyclo[3.1.0]hexane-2-carboxylate. The crude product was directly used in the next step. ES-API: [M+H]⁺= 218.2.

Step IV: At room temperature, sodium bicarbonate (252 mg, 3.0 mmol) and potassium iodide (10 mg, 0.06 mmol) were sequentially added to a solution of methyl (1R,5S)-2-(3-chloropropyl)-3-azabicyclo[3.1.0]hexane-2-carboxylate in acetonitrile (30 mL), and the reaction mixture was heated and stirred under reflux for 2 hrs. After filtration, the filtrate was concentrated under reduced pressure. The obtained crude product was purified by flash silica gel column chromatography (methanol/dichloromethane: 0-10%) to obtain methyl (1aS,6bR)-hexahydrocyclopropane [a]pyrrolizin-6a(4H)-carboxylate (96 mg, yield: 88%). ES-API: [M+H]⁺= 182.1.

Step V: Under a nitrogen atmosphere, methyl (1aS,6bR)-hexahydrocyclopropane[a]pyrrolizin-6a(4H)-carboxylate (96 mg, 0.53 mmol) was dissolved in anhydrous tetrahydrofuran (5.0 mL), and the mixture was allowed to stand in an iced water bath. Lithium aluminium hydride (0.80 mL, 0.80 mmol, 1 M in tetrahydrofuran) was slowly added dropwise to the solution, and stirred at this temperature for 0.5 hr. Sodium sulfafe decahydrate was slowly added to quench the reaction, heated to room temperature, stirred for 0.5 hr, and filtered. The filtrate was concentrated under reduced pressure, to obtain ((1aS,6bR)-hexahydrocyclopropane[a]pyrrolizin-6a(4H)-yl)methanol (63 mg, yield: 78%). ES-API: [M+H]⁺= 154.3.

Step VI: At 0°C, m-chloroperoxybenzoic acid (17 mg, 0.08 mmol, 85% purity) was added to a solution of t-butyl (5S,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptan-14-carboxylate (50 mg, 0.065 mmol) in dichloromethane (5.0 mL), and sitrred at 0°C for 0.5 hr. A saturated sodium bicarbonate aqueous solution and a saturated sodium sulfite aqueous solution were added, and vigorously stirred for 15 min. The organic phase was separated, and the aqueous phase was extracted with dichloromethane. The organic phase was collected, washed with saturated brine, dried over anhydrous sodium sulfate, filtered, and evaporated to dryness, to obtain t-butyl (5S,5aS,6S,9R)-1-fluoro-2-(7-fluoro-8-((triisopropylsilyl) ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate. The crude product was directly used in the next step of reaction. ES-API: [M+H]⁺= 788.3.

Step VII: At 0°C, sodium hydride (60% dispersed in an oil) (10 mg, 0.26 mmol) was added to ((1aS,6bR)-hexahydrocyclopropane[a]pyrrolizin-6a(4H)-yl)methanol (25 mg, 0.16 mmol) in tetrahydrofuran (5.0 mL). After reaction for 15 min, a solution of t-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate as a crude product in tetrahydrofuran (1.0 mL) was added dropwise to the reaction solution. After reaction at 0°C for 0.5 hr, a saturated ammonium chloride aqueous solution was slowly added to the reaction solution, and the reaction solution was extracted three times with dichloromethane. The organic phase was collected, washed with saturated brine, dried over anhydrous sodium sulfate, filtered and evaporated to dryness. The crude product was purified by preparative TLC (ethyl acetate /petroleum ether 80%) to obtain the product t-butyl (5S,5aS,6S,9R)-1-fluoro-2-(7-fluoro-8-((triisopropylsilyl)ethynyl)naphthalen-1-yl)-12-(((1aS,6bR)-hexahydrocyclopropane[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, yield: 53%). ES-API: [M+H]⁺= 877.4.

Step VIII: T-butyl (5S,5aS,6S,9R)-1-fluoro-2-(7-fluoro-8-((triisopropylsilyl)ethynyl) naphthalen-1-yl)-12-(((1aS,6bR)-hexahydrocyclopropane[a]pyrrolizin-6a(4H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.034 mmol) was dissolved in N,N-dimethylformamide (1.0 mL), cesium fluoride (52 mg, 0.34 mmol) was added, and reacted at room temperature for 1 hr. After the reaction, the reaction solution was added with water, and extracted three times with ethyl acetate. The organic phases were combined and washed with saturated brine. The organic phase was dried over anhydrous sodium sulfate, and filtered. The filtrate was rotary evaporated to dryness under reduced pressure, to obtain t-butyl (5S,5aS,6S,9R)-2-(8-ethynyl-7-fluoronaphthalenyl-1-yl)-1-fluoro-12-(((1aS,6bR)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]hepten-14-carboxylate. The crude product was directly used in the next step of reaction. ES-API:[M+H]⁺= 721.3.

Step IX: T-butyl (5S,5aS,6S,9R)-2-(8-ethynyl-7-fluoronaphthalenyl-1-yl)-1-fluoro-12-(((1aS,6bR)-hexahydrocyclopropane[a]pyrrolizin-6a(4H)-yl)methoxy)5-methyl-5a,6,7,8,9,10-hexahydro-5H-4-oxa-3,10a,11,13,14-pentaaza-6,9-methanonaphtho[1,8-ab]hepten-14-carboxylate as a crude product was dissolved in acetonitrile (5.0 mL). In an ice bath, 4M hydrochloric acid/dioxane solution (1.0 mL, 4.0 mmol) was added, reacted at 0°C for 0.5 hr, heated to room temperature and reacted for 0.5 hr. After the reaction, the reaction solution was concentrated to dryness, and the crude product was purified by preparative HPLC (ammonium bicarbonate method 1)(chromatographic condition: 15mL-55-95-13min) to obtain two isomers. One isomer was arbitrarily designated as (5S,5aS,6S,9R)-2-(8-ethynyl-7-fluoronaphthalenyl-1-yl)-1-fluoro-12-(((1aS, 6ar,6bR)-hexahydrocyclopropa[a]pyrrolizin-6a(4H)-yl)methoxy)5-methyl-5a,6,7,8,9, 10-hexahydro-5H-4-oxa-3,10a,11,13,14-pentaaza-6,9-methanonaphtho[1,8-ab]heptene (Z627-1, retention time7.12 min, 1.15 mg, yield: 5.5%), ES-API: [M+H]⁺= 621.3. The other isomer was arbitrarily designated as (5S,5aS,6S,9R)-2-(8-ethynyl-7-fluoronaphthalenyl-1-yl)-1-fluoro-12-(((1aS, 6aS,6bR)-hexahydrocyclopropala]pyrrolizin-6a(4H)-yl)methoxy)5-methyl-5a,6,7,8,9, 10-hexahydro-5H-4-oxa-3,10a,11,13,14-pentaaza-6,9-methanonaphtho[1,8-ab]heptene (Z627-2, retention time8.37 min, 1.65 mg, yield: 7.8%), ES-API: [M+H]⁺= 621.3.

### Example 39: Synthesis of (5S,5aS,6S,9R)-12-((1'H,3'H,5'H-bispiro[cyclopropane-1,2'-pyrrolizin-6',1''-cyclopropane]-7a'(7'H)-yl)methoxy)-2-(8-ethynyl-7-fluoronaphthalen-1-yl)-1-fluoro-5-methyl-5a,6,7,8,9,10-hexahydro-5H-4-oxy-3,10a,11,13,14-pentaaza-6,9-methanonaphtho[1,8-ab]heptene (Z628)

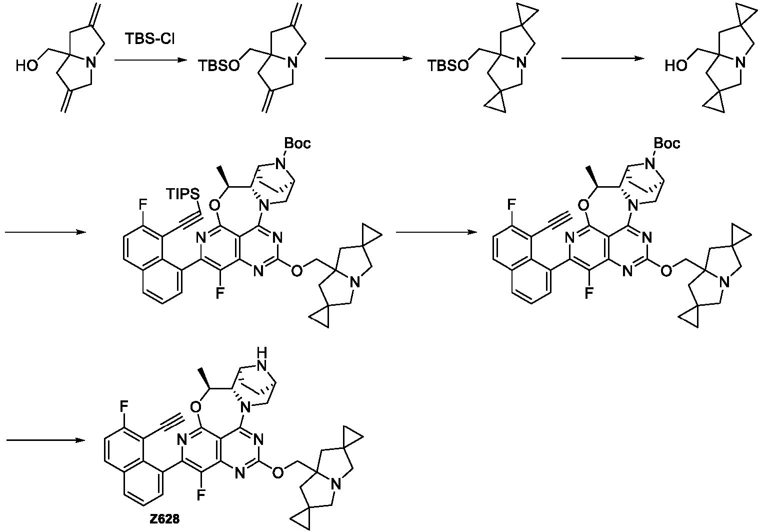

Step I: T-butyldimethylchlorosilane (912.13mg, 6.052mmol) and imidazole (823mg, 12.1mmol) were added to a solution of (2,6-dimethyltetrahydro-1H-pyrrolizin-7a(5H)-yl)methanol (1.0g, 6.052mmol) in dichloromethane(20.0mL), and stirred at room temperature for 3 hrs. After the reaction, the reaction solution was added with saturated ammonium chloride (30 mL), and extracted with ethyl acetate (100 mLX3). The organic phase was washed with saturated brine (200mL), dried over anhydrous sodium sulfate, filtered and concentrated. The crude product was purified by flash silica gel column chromatography (methanol /dichloromethane: 0%-5%) to obtain 7a-(((t-butyldimethylsilyl)oxy)methyl)-2,6-dimethylhexahydro-1H-pyrrolizine (1.5g, yield: 88%) as a colorless transparent liquid. ES-API: [M+H]⁺=280.3.

Step II: At -20°C, diethyl zinc (5.3mL, 5.30mmol) was added to a solution of 7a-(((t-butyldimethylsilyl)oxy)methyl)-2,6-dimethylhexahydro-1H-pyrrolizin(0.3g, 71.073mmol) in dichloromethane (10.0 mL), and stirred at this temperature for 20 min. After 20 min, diiodomethane (2.87g, 10.73mmol) was added under this condition, slowly warmed in an iced water bath, and reacted for 3 hrs. After the reaction, the reaction solution was added with saturated ammonium chloride (30 mL), and extracted with ethyl acetate (80 mLX2). The organic phase was washed with saturated brine (60mL), dried over anhydrous sodium sulfate, filtered and concentrated, to obtain the crude product 7a'-(((t-butyldimethylsilyl)oxy)methyl)dihydro-1'H,3'H,5'H-bispiro[cyclopropane-1,2'-pyrrolizin-6',1"-cyclopropane] (0.40g, crude product). ES-API: [M+H]⁺=308.2.

Step III: Hydrochloric acid/dioxane (4.0mL, 4M, 16.0mmol) was added to a solution of 7a'-(((t-butyldimethylsilyl)oxy)methyl)dihydro-1'H,3'H,5'H-bispiro[cyclopropane-1,2'-pyrrolizin-6",1"-cyclopropane] (0.40g, crude product) in acetonitrile (10.0mL), and reacted at room temperature for 3 hrs. After the reaction, the reaction solution was rotary evaporated to dryness under reduced pressure to remmove the solvent. The crude product was added with acetonitrile (20.0mL) and dry potassium carbonate (1000mg, 7.24mmol), stirred at room temperature for 1 hr, and filtered. The filtrate was rotary evaporated to dryness under reduced pressure to obtain (1'H,3'H,5'H-bispiro[cyclopropane-1,2'-pyrrolizin-6',1"-cyclopropane]-7a'(7'H)-yl)methanol (240mg, crude product). ES-API: [M+H]⁺=194.1.

Step IV: (1'H,3'H,5'H-bispiro[cyclopropane-1,2'-pyrrolizin-6',1"-cyclopropane]-7a'(7'H)-yl) methanol (240mg, crude product) was dissolved in anhydrous tetrahydrofuran (10.0 mL). In an iced water bath, sodium hydride (100mg, 2.50 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (80mg, 0.414 mmol) in tetrahydrofuran (2 mL) was added dropwise to the reaction solution, and reacted at 0°C for 30 min. The reaction was shown to be completed by LC/MS. The reaction solution was diluted with ethyl acetate (50 mL), washed with saturated a ammonium chloride solution (15 mLX2) and saturated brine (50 mLX3), dried over anhydrous sodium sulfate, and filtered. The crude product was purified by silica gel column chromatography (dichloromethane: methanol =15:1) to obtain t-butyl (5S,5aS,6S,9R)-12-((1'H,3'H,5'H-bispiro[cyclopropane-1,2'-pyrrolizin-6',1"-cyclopropane]-7a'(7'H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (20mg, total yield of 3 steps: 2.0%). ES-API: [M+H]⁺= 917.4

Step V: Cesium fluoride (96.0 mg, 0.63mmol) was added to t-butyl (5S,5aS,6S,9R)-12-((1'H,3'H,5'H-bispiro[cyclopropane-1,2'-pyrrolizin-6',1"-cyclopropane]-7a'(7'H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (20mg, 0.02183mmol) in N,N-dimethylformamide (2.0mL), and the reaction mixture was stirred at room temperature for 2 hrs. The reaction was shown to be completed by LC/MS. The reaction solution was diluted with ethyl acetate (60mL), washed with water (30 mLX2) and saturated brine (50 mLX3), dried over anhydrous sodium sulfate, filtered, and concentrated to obtain the crude product t-butyl (5S,5aS,6S,9R)-12-((1'H,3'H,5H-bispiro[cyclopropane-1,2'-pyrrolizin-6',1"-cyclopropane]-7a'(7'H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (27mg, crude product). ES-API: [M+H]⁺= 761.3.

Step VI: In an iced water bath, 4 M hydrochloric acid-dioxane solution (3 mL) was slowly added to a solution of t-butyl (5S,5aS,6S,9R)-12-((1'H,3'H,5'H-bispiro[cyclopropane-1,2'-pyrrolizin-6',1"-cyclopropane]-7a'(7'H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (27mg, crude product) in acetonitrile (5.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-12-((1'H,3'H,5'H-bispiro[cyclopropane-1,2'-pyrrolizin-6',1"-cyclopropane]-7a'(7'H)-yl)methoxy)-2-(8-ethynyl-7-fluoronaphthalen-1-yl)-1-fluoro-5-methyl-5a,6,7,8,9,10-hexahydro-5H-4-oxy-3,10a,11,13,14-pentaaza-6,9-methanonaphtho[1,8-ab]heptene (Z628, 2.68mg, total yield of 2 steps: 18.6%) as a light yellow solid. ES-API: [M+H]⁺= 661.3.

### Example 40 Synthesis of 4-cyclopropyl-3-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptene-2-yl)-2,5-dimethylaniline (Z717)

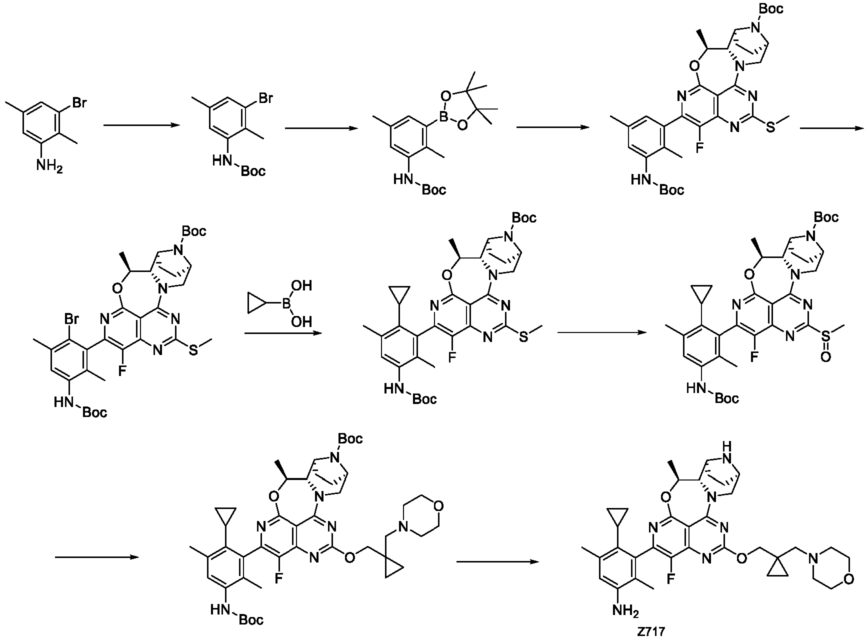

Step I: At room temperature, 3-bromo-2,5-dimethylaniline (1.0 g, 4.998 mmol) and di-t-butyl dicarbonate (1.42 g, 6.497 mmol) were sequentially added to a sodium hydroxide solution (2M, 20 mL), and reacted at 100°C for 1 hr. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. A crude product was obtained, which was purified by silica gel column chromatography (0-50% ethyl acetate /petroleum ether) to obtain t-butyl (3-bromo-2,5-dimethylphenyl)carbamate (1.40 g, yield: 93.31%). ES-API: [M-55+H]⁺=245.9.

Step II: T-butyl (3-bromo-2,5-dimethylphenyl)carbamate (0.9 g, 2.998 mmol), bis(pinacolato)diboron (1.52 g, 5.996 mmol), potassium acetate (1.173 g, 11.99 mmol), and [1,1'-bis(diphenylphosphino)ferrocene]palladium dichloride (0.323 g, 0.45 mmol) were added to N,N-dimethylformamide (30 mL), purged 4 time with nitrogen, and reacted at 80°C for 2 hrs. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. A crude product was obtained, which was purified by silica gel column chromatography (0-70% ethyl acetate /petroleum ether) to obtain t-butyl (2,5-dimethyl-3-(4,4,5,5-tetramethyl-1,3,2-dioxaborolane-2-yl)phenyl)carbamate (490 mg, yield: 47%). ES-API: [M-55+H]⁺=292.2.

Step III: Potassium phosphate (0.616 g, 2.90 mmol) and [n-butyldi(1-adamantanyl) phosphine](2-amino-1,1'-biphenyl-2-yl)palladium (II) mesylate (79.3 mg, 0.109 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab]hepten-14-carboxylate (350 mg, 0.726 mmol) and t-butyl (2,5-dimethyl-3-(4,4,5,5-tetramethyl-1,3,2-dioxaborolan-2-yl)phenyl)carbamate (0.490 g, 1.411 mmol) in tetrahydrofuran (30 mL) and water (3 mL). Nitrogen was bubbled therethrough and reacted in an oil bath at 80°C for 3 hrs. After the reaction, the reaction solution was extracted with ethyl acetate (30 mLX2) and saturated brine (30 mL). The ethyl acetate phases were combined, dried over anhydrous sodium sulfate, and filtered. The filtrate was concentrated under reduced pressure. A crude product was obtained, which was purified by silica gel column chromatography (0-4% methanol /dichloromethane) to obtain t-butyl (5S,5aS,6S,9R)-2-(3-((t-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (570 mg, crude product)as a yellow solid. ES-API: [M+H]⁺=667.2.

Step IV: At 0°C, t-butyl (5S,5aS,6S,9R)-2-(3-((t-butoxycarbonyl)amino)-2,5-dimethylphenyl) -1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (483.0 mg, 0.726 mmol) and then N-bromosuccinimide (168.0 mg, 0.944 mmol) were added to dichloromethane (20 mL), and reacted with stirring at this temperature for 0.5 hr. After the reaction, the reaction solution was extracted with ethyl acetate (100 mLX2) and saturated brine (100 mL). The ethyl acetate phases were combined, dried over anhydrous sodium sulfate, and filtered. The filtrate was concentrated under reduced pressure. A crude product was obtained, which was purified by silica gel column chromatography (0-70% ethyl acetate /petroleum ether) to obtain t-butyl (5S,5aS,6S,9R)-2-(2-bromo-5-((t-butoxycarbonyl)amino)-3,6-dimethylphenyl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (400 mg, yield: 74.0%). ES-API: [M+H]⁺=745.1.

step V: Potassium phosphate (455.4 mg, 2.146 mmol), 2-bicyclohexylphosphino-2',6'-dimethoxybiphenyl (66.06 mg, 0.161 mmol), and palladium acetate (24.04 mg, 0.107 mmol) were added to t-butyl (5S,5aS,6S,9R)-2-(2-bromo-5-((t-butoxycarbonyl)amino)-3,6-dimethylphenyl)-1-fluoro-5-methyl-12-(methylthio)-5 a,6,7, 8,9,10-hexahydro-5H-4-oxa-3,10a,11,13,14-pentaaza-6,9-methanonaphtho[1,8-ab]hepten-14-carboxylate (400 mg, 0.536 mmol) and cyclopropylboric acid (230.0 mg, 2.68 mmol) in toluene (50 mL) and water (5 mL). Nitrogen was bubbled therethrough, and the reaction was continued in an oil bath at 105°C for 2 hrs. After the reaction, the reaction solution was extracted with ethyl acetate (200 mLX2) and saturated brine (100 mLX4). The ethyl acetate phases were combined, dried over anhydrous sodium sulfate, and filtered. The filtrate was concentrated under reduced pressure. A crude product was obtained, which was purified by silica gel column chromatography (0-70% ethyl acetate /petroleum ether) to obtain t-butyl (5S,5aS,6S,9R)-2-(3-((t-butoxycarbonyl)amino)-6-cyclopropyl-2,5-dimethylphenyl)-1-fluoro-5-methyl-12-(methylthio)-5a,6,7,8,9, 10-hexahydro-5H-4-oxa-3, 10a, 11,13,14-pentaaza-6,9-methanonaphtho[1,8-ab]hepten-14-carboxylate (520 mg, crude product) as a yellow solid. ES-API: [M+H]⁺=707.2.

Step VI: At 0°C, m-chloroperoxybenzoic acid (188.3 mg, 1.09 mmol) was added to a solution of t-butyl (5S,5aS,6S,9R)-2-(3-((t-butoxycarbonyl)amino)-6-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (379.0 mg, 0.537 mmol) in dichloromethane (30.0 mL). The solution was stirred for 1 hour in an ice bath. The reaction was quenched with saturated sodium sulfite and extracted with dichloromethane(100 mLX2). The organic phase was dried over anhydrous sodium sulfate, filtered and concentrated to obtain a crude product. The crude product was purified by silica gel column chromatography (0-10% methanol /dichloromethane) to obtain t-butyl (5S,5aS,6S,9R)-2-(3-((t-butoxycarbonyl) amino)-6-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (390 mg, crude product) as a white solid. ES-API:[M+H]⁺=723.3.

Step VII: At 0°C, sodium hydride (70.0 mg, 1.754 mmol) was added to a solution of (1-(morpholinemethyl)cyclopropyl)methanol (100 mg, 0.5850 mmol) in tetrahydrofuran (10.0 mL), and stirred at this temperature for 10 min. Then a solution of t-butyl (5S,5aS,6S,9R)-2-(3-((t-butoxycarbonyl)amino)-6-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (390 mg, crude product) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(3-((t-butoxycarbonyl)amino)-6-cyclopropyl-2,5-dimethylphenyl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (150 mg, crude product) as a yellow solid. ES-API: [M+H]⁺=830.3.

Step VIII: In an iced water bath, t-butyl (5S,5aS,6S,9R)-2-(3-((t-butoxycarbonyl)amino)-6-cyclopropyl-2,5-dimethylphenyl)-1-fluoro-5-methyl-12-((1-(morpholinemethyl)cyclopropyl) methoxy)-5a,6,7,8,9,10-hexahydro-5H-4-oxa-3,10a,11,13,14-pentaaza-6,9-methanonaphtho[1,8-ab]hepten-14-carboxylate (150 mg, crude product) was slowly added to dichloromethane (10.0 mL), and finally hydrochloric acid-dioxane solution (5.0 mL, 4M, 20.0 mmol) was added. The reaction mixture was stirred at room temperature for 1.0 hrs. The reaction was shown to be completed by LC/MS. The reaction solution was concentrated to dryness under reduced pressure, and the residue was purified by preparative HPLC (ammonium bicarbonate method 2) to obtain 4-cyclopropyl-3-((5S,5aS,6S,9R)-1-fluoro-5-methyl-12-((1-(morpholinemethyl)cyclopropyl) methoxy)-5a,6,7,8,9,10-hexahydro-5H-4-oxa-3,10a,11,13,14-pentaaza-6,9-methanonaphtho[1,8-ab]hepten-2-yl)-2,5-dimethylaniline (Z717, 5.0 mg, yield:1.5%) as a white solid. ES-API: [M+H]⁺= 630.4.

### Example 41: Synthesis of (5S,5aS,6S,9R)-2-(8-ethynyl-7-fluoronaphthalen-1-yl)-1-fluoro-5-methyl-12-(((1aR,6bS)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptene (Z605)

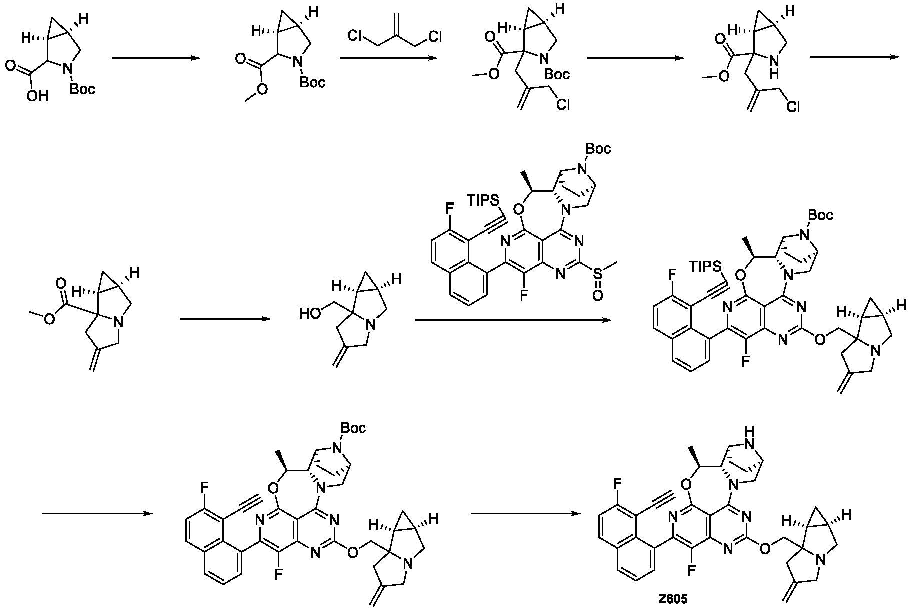

Step I: Potassium carbonate (548 mg, 3.960 mmol) and iodomethane (375 mg, 2.640 mmol) were added to a solution of (1S,5R)-3-(t-butoxycarbonyl)-3-azabicyclo[3.1.0]hexane-2-carboxylic acid (300 mg, 1.320 mmol) in N,N-dimethylformamide (10 mL), and stirred at room temperature for 2 hrs. The reaction was shown to be completed by LC/MS. The reaction solution was added with ethyl acetate (20 mL) and water (20 mL). After separation, the organic phase was washed 3 times with saturated brine, dried over anhydrous sodium sulfate, and filtered. The filtrate was concentrated under reduced pressure, to obtain a crude product that was 3-(t-butyl) 2-methyl (1S,5R)-3-azabicyclo[3.1.0]hexane-2,3-dicarboxylate (318 mg). ES-API: [M-55]⁺= 186.1.

Step II: Under a nitrogen atmosphere, 3-(t-butyl) 2-methyl (1S,5R)-3-azabicyclo[3.1.0]hexane-2,3-dicarboxylate (318 mg, 1.318 mmol) was dissolved in anhydrous tetrahydrofuran (10 mL), and the mixture was allowed to stand in a dry ice-acetone bath. A solution of lithium bis(trimethylsilyl)amide (2.6 mL, 2.636 mmol, 1 M in tetrahydrofuran) was slowly added dropwise to the above solution, and stirred at this temperature for 1 hr. A solution of 3-chloro-2-(chloromethyl)prop-1-ene (823 mg, 6.590 mmol) in anhydrous tetrahydrofuran (5 mL) was slowly added by a syringe to the solution, and the reaction solution was continuously stirred at -78°C for 1 hr, slowly heated to room temperature, and stirred overnight. The reaction was shown to be completed by LC/MS. A saturated ammonium chloride solution (20 mL) was added to quench the reaction. The reaction solution was extracted with ethyl acetate (15 mLX3). The organic phase was washed with saturated brine (15 mLX3), dried over anhydrous sodium sulfate, filtered, and concentrated under reduced pressure. The crude product was purified by flash silica gel column chromatography (ethyl acetate: petroleum ether= 0- 15%) to obtain 3-(t-butyl) 2-methyl (1S,5R)-2-(2-(chloromethyl)allyl)-3-azabicyclo[3.1.0]hexane-2,3-dicarboxylate (180 mg, yield: 41%). ES-API: [M-55]⁺= 274.

Step III: In an iced water bath, 4M hydrochloric acid/dioxane solution (2 mL) was slowly added to a solution of 3-(t-butyl) 2-methyl (1S,5R)-2-(2-(chloromethyl)allyl)-3-azabicyclo[3.1.0]hexane-2,3-dicarboxylate (180 mg, 0.546 mmol) in acetonitrile (2 mL), and stirred at 0°C for 1 hr. The reaction was shown to be completed by LC/MS. The reaction solution was concentrated under reduced pressure, to obtain a crude product that was methyl (1S,5R)-2-(2-(chloromethyl)allyl)-3-azabicyclo[3.1.0]hexane-2-carboxylate (125 mg). ES-API: [M+H]⁺= 230.2.

Step IV: At room temperature, sodium bicarbonate (229 mg, 2.721 mmol) and potassium iodide (26 mg, 0.054 mmol) was added to a solution of methyl (1S,5R)-2-(2-(chloromethyl)allyl)-3-azabicyclo[3.1.0]hexane-2-carboxylate (125 mg, 0.544 mmol) in acetonitrile (10 mL), and stirred overnight at room temperature. The reaction was shown to be completed by LC/MS. The reaction solution was filtered, and washed with acetonitrile. The filtrate was concentrated under reduced pressure, and the crude product was purified by flash silica gel column chromatography (methanol: dichloromethane= 0-10%) to obtain methyl (1aR,6bS)-5-methylenehexahydrocyclopropane [a]pyrrolizin-6a(4H)-carboxylate (89 mg, yield: 85%). ES-API: [M+H]⁺ = 194.2.

Step V: Under a nitrogen atmosphere, methyl (1aR,6bS)-5-methylenehexahydrocyclopropane [a]pyrrolizin-6a(4H)-carboxylate (89 mg, 0.461 mmol) was dissolved in anhydrous tetrahydrofuran(5 mL), and the mixture was allowed to stand in an iced water bath. A lithium aluminium hydride solution (1 mL, 0.921 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate, stirred at room temperature for 0.5 hr, and filtered. The filtrate was concentrated under reduced pressure, to obtain a crude product that was ((1aR,6bS)-5-methylenehexahydrocyclopropane[a]pyrrolizin-6a(4H)-yl)methanol (76 mg). ES-API: [M+H]⁺= 166.1.

Step VI: ((1aR,6bS)-5-methylenehexahydrocyclopropane[a]pyrrolizin-6a(4H)-yl)methanol (31 mg, 0.190 mmol) was dissolved in anhydrous tetrahydrofuran(5 mL). In an iced water bath, sodium hydride (7.6 mg, 0.190 mmol) was added, and then reacted for 30 min in the iced water bath. A solution of t-butyl (5S, 5aS, 6S, 9R)-1-fluoro-2-(7-fluoro-8-((triisopropylsilyl) ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 0°C for 30 min. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure, to obtain a crude product that was t-butyl (5S,5aS,6S,9R)-1-fluoro-2-(7-fluoro-8-((triisopropylsilyl)ethynyl)naphthalen-1-yl)-5-methyl-12-(((1aR,6bS)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate (48 mg). ES-API: [M+H]⁺= 889.3.

Step VII: Cesium fluoride (82 mg, 0.540 mmol) was added to t-butyl (5S,5aS,6S,9R)-1-fluoro-2-(7-fluoro-8-((triisopropylsilyl)ethynyl)naphthalen-1-yl)-5-methyl-12-(((1aR,6bS)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate (48 mg, 0.054 mmol) in N,N-dimethylformamide (2 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-(((1aR,6bS)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate (39 mg). ES-API: [M+H]⁺= 733.3.

Step VIII: In an iced water bath, 4 M hydrochloric acid-dioxane solution (2 mL) was slowly added to a solution of t-butyl (5S,5aS,6S,9R)-2-(8-ethynyl-7-fluoronaphthalen-1-yl)-1-fluoro-5-methyl-12-(((1aR,6bS)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate ((39 mg, 0.053 mmol) in acetonitrile (1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-50-70-13min) to obtain (5S,5aS,6S,9R)-2-(8-ethynyl-7-fluoronaphthalen-1-yl)-1-fluoro-5-methyl-12-(((1aR,6bS)-5-methylenehexahydrocyclopropane[a]pyrrolizin-6a(4H)-yl)methoxy)-5a,6,7,8,9,10-hexahydro-5H-4-oxy-3,10a,11,13,14-pentaaza-6,9-methanonaphtho[1,8-ab]heptene (Z605, 1.05 mg, yield: 3%) as an off-white solid. ES-API: [M+H]⁺= 633.3.

### Example 42: Synthesis of (5S,5aS,6S,9R)-2-(8-ethynyl-7-fluoronaphthalen-1-yl)-1-fluoro-5-methyl-12-((2-(difluoromethylene)tetrahydro-1H)-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z608)

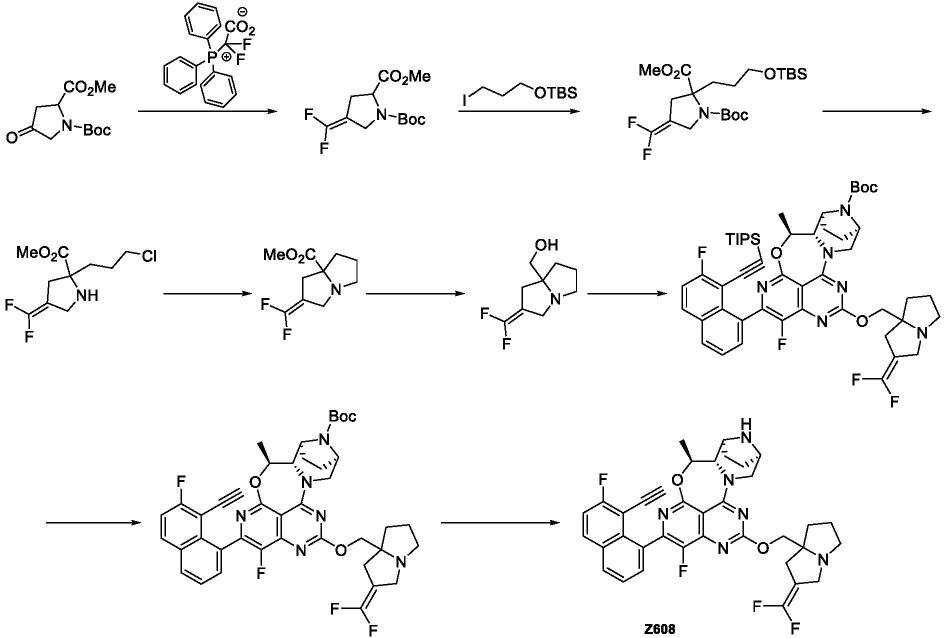

Step I: Under a nitrogen atmosphere, 1-(t-butyl) 2-methyl 4-oxopyrrolidin-1,2-dicarboxylate (2.5 g, 10.28 mmol), (triphenylphosphonio)difluoroacetate (9.5 g, 26.75 mmol) and N,N-dimethylformamide (30 mL) were added to a round-bottom flask, and the system was stirred at 80°C for 6 hrs. The reaction solution was added with water (100 mL), and extracted with ethyl acetate (50 mL X 2). The organic phase was washed with saturated brine (50 mLX2), dried over anhydrous sodium sulfate, and concentrated. The crude product was purified by flash silica gel column chromatography (ethyl acetate /petroleum ether: 0-8%) to obtain 1-(t-butyl) 2-methyl 4-(difluoromethylene)pyrrolidin-1,2-dicarboxylate (1.47 g, yield: 51%) as a colorless oil. ES-API: [M+Na]⁺=300.1.

Step II: 1-(t-butyl)2-methyl 4-(difluoromethylene)pyrrolidin-1,2-dicarboxylate (1.4 g, 5.05 mmol), t-butyl (3-iodopropoxy)dimethylsilane (3.03 g, 10.11 mmol), and tetrahydrofuran (20 mL) were added to a round-bottom flask. Under a nitrogen atmosphere, the reaction solution was cooled to -78°C. A potassium bis(trimethylsilyl)amide solution (10.11 mL, 10.11 mmol, 1 M in tetrahydrofuran) was added dropwise to the reaction solution with stirring. After that, the reaction was stirred at -78°C for 30 hrs. The ice bath was removed, and the reaction solution was poured into a saturated ammonium chloride aqueous solution (100 mL). The reaction solution was extracted with ethyl acetate (50 mLX3), washed with saturated brine, dried over anhydrous sodium sulfate, filtered, and concentrated. The crude product was purified by flash silica gel column chromatography (ethyl acetate /petroleum ether: 0-7%) to obtain 1-(t-butyl)2-methyl 2-(3-((t-butyldimethylsilyl)oxy)propyl)-4-(difluoromethylene)pyrrolidin-1,2-dicarboxylate (900 mg, yield: 40%) as a colorless oil. ES-API: [M+Na]⁺=472.3.

Step III: Under a nitrogen atmosphere, 1-(t-butyl) 2-methyl 2-(3-((t-butyldimethylsilyl) oxy)propyl)-4-(difluoromethylene)pyrrolidin-1,2-dicarboxylate (200 mg, 0.44 mmol), dichloromethane(1 mL), 4M hydrogen chloride/dioxane solution (0.6 mL), and thionyl chloride (3 mL) were sequentially added to a reaction flask. The reaction was stirred at 50°C for 4 hr. The reaction solution was concentrated, added with dichloromethane (30 mL), and washed with a saturated sodium bicarbonate aqueous solution (50 mL). The organic phase was dried over anhydrous sodium sulfate, and concentrated to obtain methyl 2-(3-chloropropyl)-4-(difluoromethylene)pyrrolidin-2-carboxylate (111 mg, crude product) as a yellow oil. ES-API: [M+H]⁺=254.1.

Step IV: Methyl 2-(3-chloropropyl)-4-(difluoromethylene)pyrrolidin-2-carbxylate (220 mg, crude product), potassium carbonate (119 mg, 0.86 mmol), potassium iodide (143 mg, 0.86 mmol), and N,N-dimethylformamide (5 mL) were added to a reaction flask. The reaction was stirred at 85°C for 2 hr. The reaction mixture was diluted with water (50 mL) and extracted with ethyl acetate (30 mLX3). The organic phase was washed with saturated brine, dried over anhydrous sodium sulfate, filtered, and concentrated. The crude product was purified by flash silica gel column chromatography (ethyl acetate /petroleum ether: 0-40%) to obtain methyl 2-(difluoromethylene) tetrahydro-1H-pyrrolizin-7a(5H)-carboxylate (120 mg, yield of 2 steps: 64%). ES-API: [M+H]⁺=218.1.

Step V: Under a nitrogen atmosphere, methyl 2-(difluoromethylene)tetrahydro-1H-pyrrolizin-7a(5H)-carboxylate (100 mg, 0.46 mmol) and tetrahydrofuran(15 mL) were added to a round-bottom flask. The solution was cooled to 0°C, and a lithium borohydride solution (0.46 mL, 0.92 mmol, 2M in tetrahydrofuran) was added dropwise. After that, the reaction was stirred at 0°C for 30 hrs. 4M hydrochloric acid (1 mL) was added dropwise and stirred at room temperature for 30 min. 2 M sodium hydroxide aqueous solution (5 mL) and water (20 mL) were added. The reaction solution was extracted with dichloromethane/methanol (10:1, 30 mLX3). The organic phase was dried over anhydrous sodium sulfate, and concentrated to obtain (2-(difluoromethylene)tetrahydro-1H-pyrrolizin-7a(5H)-yl)methanol (40 mg, yield: 46%) as a yellow oil. ES-API: [M+H]⁺=190.1.

Step VI: (2-(difluoromethylene)tetrahydro-1H-pyrrolizin-7a(5H)-yl)methanol (44.0mg, 0.233 mmol) was dissolved in anhydrous tetrahydrofuran (5 mL). In an iced water bath, sodium hydride (20mg, 0.50 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) in tetrahydrofuran (2 mL) was added dropwise to the reaction solution, and reacted at 40°C for 30 min. The reaction was shown to be completed by LC/MS. The reaction solution was diluted with ethyl acetate (50 mL), washed with a saturated ammonium chloride solution (15 mLX2) and saturated brine (50 mLX3), dried over anhydrous sodium sulfate, and filtered. The crude product was purified by silica gel column chromatography (dichloromethane: methanol =15:1) to obtain t-butyl (55,5aS,6S,9R)-12-((2-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (60mg, crude product). ES-API: [M+H]⁺= 913.3.

Step VII: Cesium fluoride (96.0 mg, 0.63mmol) was added to t-butyl (5S,5aS,6S,9R)-12-((2-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a, 11,13,14-pentaaza-6,9-methanonaphtho[1,8-ab]hepten-14-carboxylate (60mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX2) and saturated brine (50 mLX3), dried over anhydrous sodium sulfate, filtered, and concentrated to obtain a crude product that was t-butyl (5S,5aS,6S,9R)-12-((2-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (65mg, crude product). ES-API: [M+H]⁺= 757.3.

Step VIII: In an iced water bath, 4 M hydrochloric acid-dioxane solution (3 mL) was slowly added to a solution of t-butyl (58,5aS,6S,9R)-12-((2-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (65mg, crude product) in acetonitrile (5.0mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-((2-(difluoromethylene)tetrahydro-1H)-pyrrolizin-7a(5H)-yl)methoxy)-5a,6,7,8,9,10-**hexahydro-5H-4-oxa-3,10a,11,13,14-pentaaza-6,9-methanonaphtho[1,8-ab]heptene** (Z608, 1.0mg, total yield of 3 steps: 2.4%) as a light yellow solid. ES-API: [M+H]⁺= 657.3.

### Example 43: Synthesis of (5S,5aS,6S,9R)-2-(8-ethynyl-7-fluoronaphthalen-1-yl)-1-fluoro-5-methyl-12-((1aS,6aS)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptene (Z604)

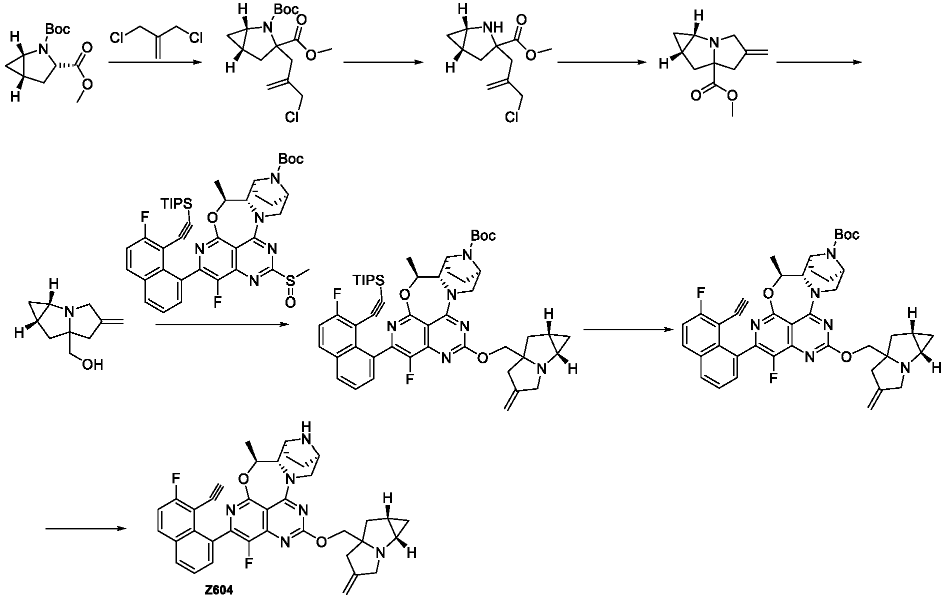

Step I: Under a nitrogen atmosphere, 2-(t-butyl) 3-methyl (1S,3S,5S)-2-azabicyclo [3.1.0]hexane-2,3-dicarboxylate (1.0 g, 4.1 mmol) was dissolved in anhydrous tetrahydrofuran (10 mL), and the mixture was allowed to stand in a dry ice-acetone bath. A lithium bis(trimethylsilyl)amide solution (8.3 mL, 8.3 mmol, 1 M in tetrahydrofuran) was slowly added dropwise to the above solution, and stirred at this temperature for 1 hr. A solution of 3-chloro-2-(chloromethyl)prop-1-ene (2.59 g, 20.7 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL), and extracted with ethyl acetate (50 mLX3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 2-(t-butyl) 3-methyl (1S,5S)-3-(2-(chloromethyl)allyl)-2-azabicyclo[3.1.0]hexane-2,3-dicarboxylate (1.28g, yield: 93%). ES-API: [M-56]⁺= 274.1.

Step II: At room temperature, a 4M hydrochloric acid/dioxane solution (15mL) was slowly added to a solution of 2-(t-butyl)3-methyl(1S,5S)-3-(2-(chloromethyl)allyl)-2-azabicyclo [3.1.0]hexane-2,3-dicarboxylate (1.28g, 3.7 mmol) in acetonitrile (3 mL), and stirred at room temperature for 0.5 hr. The reaction was shown to be completed by LC/MS. The reaction solution was concentrated under reduced pressure, to obtain methyl (1S,5S)-3-(2-(chloromethyl)allyl)-2-azabicyclo[3.1.0]hexane-3-carboxylate (0.89 g, yield: 100%). ES-API: [M+H]⁺= 230.2.

Step III: At room temperature, sodium bicarbonate (1.6 g, 19.5 mmol) and potassium iodide (65 mg, 0.39 mmol) were added to a solution of methyl (1S,5S)-3-(2-(chloromethyl)allyl)-2-azabicyclo[3.1.0]hexane-3-carboxylate (0.89 g, 3.9mmol) in acetonitrile (10 mL), and stirred at room temperature for 1.5 hrs. The reaction was shown to be completed by LC/MS. The reaction solution was filtered, and washed with acetonitrile. The filtrate was concentrated under reduced pressure, and the crude product was purified by flash silica gel column chromatography (petroleum ether: ethyl acetate =15:1) to obtain methyl (1aS,6aS)-4-methylenehexahydrocyclopropane [b]pyrrolizin-Sa(3H)-carboxylate (510mg, yield: 68 %). ES-API: [M+H]⁺=194.1.

Step IV: Under a nitrogen atmosphere, methyl (1aS,6aS)-4-methylenehexahydrocyclopropane [b]pyrrolizin-5a(3H)-carboxyalte (510mg, 2.6 mmol) was dissolved in anhydrous tetrahydrofuran(10mL), and the mixture was allowed to stand in an iced water bath. A Lithium aluminium hydride solution (5.4 mL, 5. 4 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (510 mg), stirred at room temperature for 0.5 hr, and filtered. The filtrate was concentrated under reduced pressure, to obtain ((1aS,6aS)-4-methylenehexahydrocyclopropane[b]pyrrolizin-5a(3H)-yl) methanol (410 mg, yield: 93%). ES-API: [M+H]⁺= 166.2.

Step V: ((1aS, 6aS)-4-methylenehexahydrocyclopropane[b]pyrrolizin-Sa(3H)-yl)methanol (47mg, 0.29 mmol) was dissolved in anhydrous tetrahydrofuran (5 mL). In an iced water bath, sodium hydride (11 mg, 0.29 mmol) was added, and then reacted for 30 min in the iced water bath. A solution of t-butyl (SS,SaS,6S,9R)-1-fluoro-2-(7-fluoro-8-((triisopropylsilyl)ethynyl)naphthalen -1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (75 mg, 0.095 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with a saturated ammonium chloride solution (15 mLX2) and saturated brine (15 mLX3), dried over anhydrous sodium sulfate, filtered, and concentrated under reduced pressure. to obtain a crude product that was t-butyl (5S,5aS,6S,9R)-1-fluoro-2-(7-fluoro-8-((triisopropylsilyl)ethynyl) naphthalen-1-yl)-5-methyl-12-(((1aS,6aS)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (85 mg, crude product). ES-API: [M+H]⁺= 889.5.

Step VI: Cesium fluoride (145 mg, 0.9 mmol) was added to t-butyl (5S,5aS,6S,9R)-1-fluoro-2-(7-fluoro-8-((triisopropylsilyl)ethynyl)naphthalen-1-yl)-5-methyl-12-(((1aS,6aS)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (85 mg, 0.09 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-(((1aS,6aS)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]hepten-14-carboxylate (69 mg, crude product). ES-API: [M+H]⁺= 733.3.

Step VII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-((1aS,6aS)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]hepten-14-carboxylate (69 mg, 0.09 mmol) in acetonitrile (1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-((1aS,6aS)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptene (Z604, 10 mg, yield: 16%) as a light yellow solid. ES-API: [M+H]⁺= 633.3.

### Example 44: Synthesis of (5S,5aS,6S,9R)-2-(8-ethynyl-7-fluoronaphthalen-1-yl)-1-fluoro-5-methyl-12-(((1aR,6aR)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]heptene (Z603)

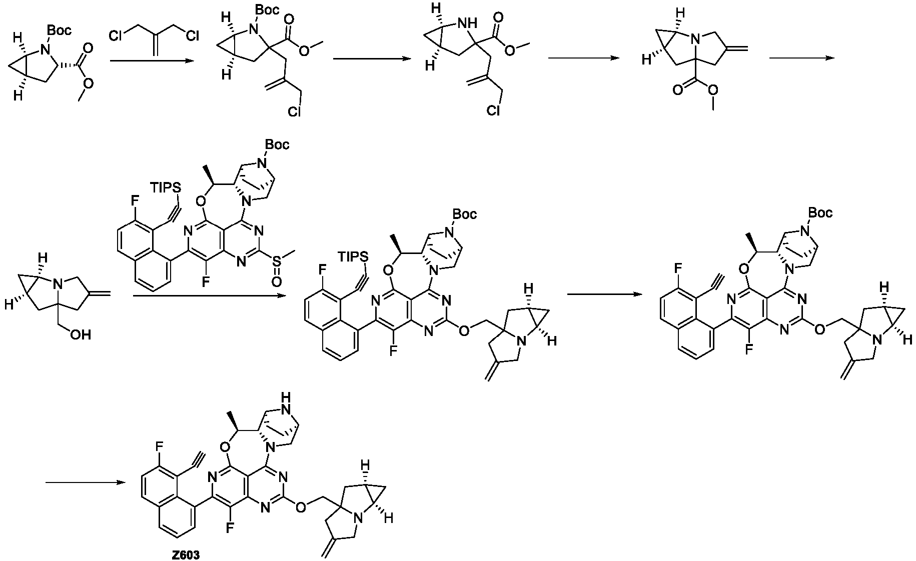

Step I: Under a nitrogen atmosphere, 2-(t-butyl) 3-methyl (1R,3S,5R)-2-azabicyclo [3.1.0]hexane-2,3-dicarboxylate (1.0 g, 4.1 mmol) was dissolved in anhydrous tetrahydrofuran (10mL), and the mixture was allowed to stand in a dry ice-acetone bath. A lithium bis(trimethylsilyl)amide solution (8.3 mL, 8.3 mmol, 1 M in tetrahydrofuran) was slowly added dropwise to the above solution, and stirred at this temperature for 1 hr. A solution of 3-chloro-2-(chloromethyl)prop-1-ene (2.59 g, 20.7 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL), and extracted with ethyl acetate (50 mLX3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 2-(t-butyl) 3-methyl (1R,5R)-3-(2-(chloromethyl)allyl)-2-azabicyclo[3.1.0]hexane-2,3-dicarboxylate (1.3g, yield: 95 %). ES-API: [M-56]⁺= 274.1.

Step II: At room temperature, a 4M hydrochloric acid/dioxane solution (10mL) was slowly added to a solution of 2-(t-butyl)3-methyl(1R,5R)-3-(2-(chloromethyl)allyl)-2-azabicyclo [3.1.0]hexane-2,3-dicarboxylate (1.3g, 3.7 mmol) in acetonitrile (3 mL), and stirred at room temperature for 0.5 hr. The reaction was shown to be completed by LC/MS. The reaction solution was concentrated under reduced pressure, to obtain methyl (1R,5R)-3-(2-(chloromethyl)allyl)-2-azabicyclo[3.1.0]hexane-3-carboxylate (0.91 g, yield: 100%). ES-API: [M+H]⁺= 230.2.

Step III: At room temperature, sodium bicarbonate (1.6 g, 19.5 mmol) and potassium iodide (65 mg, 0.39 mmol) were added to a solution of methyl (1S,5S)-3-(2-(chloromethyl)allyl)-2-azabicyclo[3.1.0]hexane-3-carboxylate (0.91 g, 3.9mmol) in acetonitrile (10 mL), and stirred at room temperature for 1.5 hrs. The reaction was shown to be completed by LC/MS. The reaction solution was filtered, and washed with acetonitrile. The filtrate was concentrated under reduced pressure, and the crude product was purified by flash silica gel column chromatography (petroleum ether: ethyl acetate =15:1) to obtain methyl (1aR,6aR)-4-methylenehexahydrocyclopropane [b]pyrrolizin-Sa(3H)-carboxylate (500mg, yield: 65%). ES-API: [M+H]⁺=194.1.

Step IV: Under a nitrogen atmosphere, methyl (1aR,6aR)-4-methylenehexahydrocyclopropane [b]pyrrolizin-5a(3H)-carboxyalte (500mg, 2.6 mmol) was dissolved in anhydrous tetrahydrofuran(10mL), and the mixture was allowed to stand in an iced water bath. A Lithium aluminium hydride solution (5.4 mL, 5. 4 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (500 mg), and stirred at room temperature for 0.5 hr. The reaction solution was filtered. The filtrate was concentrated under reduced pressure, to obtain ((1aR,6aR)-4-methylenehexahydrocyclopropane [b]pyrrolizin-5a(3H)-yl)methanol (400 mg, yield: 93 %). ES-API: [M+H]⁺= 166.2.

Step V: ((1aR,6aR)-4-methylenehexahydrocyclopropane[b]pyrrolizin-5a(3H)-yl)methanol (47mg, 0.29 mmol) was dissolved in anhydrous tetrahydrofuran(5 mL). In an iced water bath, sodium hydride (11 mg, 0.29 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen -1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (75 mg, 0.095 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, filtered, and concentrated under reduced pressure to obtain a crude product that was t-butyl (5S,5aS,6S,9R)-1-fluoro-2-(7-fluoro-8-((triisopropylsilyl)ethynyl) naphthalen-1-yl)-5-methyl-12-((1aR, 6aR)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (89 mg, crude product). ES-API: [M+H]⁺= 889.5.

Step VI: Cesium fluoride (145 mg, 0.9 mmol) was added to t-butyl (5S,5aS,6S,9R)-1-fluoro-2-(7-fluoro-8-((triisopropylsilyl)ethynyl)naphthalen-1-yl)-5-methyl-12-((1aR,6aR)-4-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (89 mg, 0.1 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated, to obtain the crude product t-butyl (5S,5aS,6S,9R)-2-(8-ethynyl-7-fluoronaphthalen-1-yl)-1-fluoro-5-methyl-12-(((1aR, 6aR)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]hepten-14-carboxylate (71 mg, crude product). ES-API: [M+H]⁺= 733.3.

Step VII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-(((1aR,6aR)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]hepten-14-carboxylate (75 mg, 0.1 mmol) in acetonitrile (1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-(((laR,6aR)-4-methylenehexahydrocyclopropane[b]pyrrolizin-5a(3H)-ylmethoxy)-5a,6,7,8,9,10-hexahydro-5H-4-oxa-3,10a,11,13,14-pentaaza-6,9-methanonaphtho[1,8-ab]heptene (Z603, 4.5 mg, yield: 7%) as a light yellow solid. ES-API: [M+H]⁺= 633.3.

### Example 45: Synthesis of (5S,5aS,6S,9R)-2-(8-ethynyl-7-fluoronaphthalen-1-yl)-1-fluoro-5-methyl-12-((6'-methylenetetrahydrospiro[cyclopropyl-1,1'-pyrrolizin]-7a'(5'H)-yl)methoxy)-5a,6,7,8,9,10-hexahydro-5H-4-oxy-3,10a,11,13,14-pentaaza-6,9-methanonaphtho[1,8-ab]heptene (Z602)

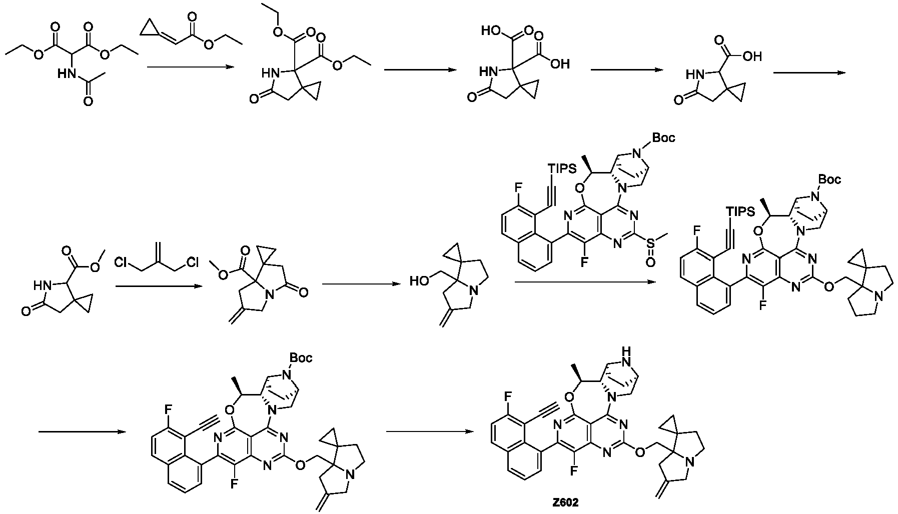

Step I: 2-cyclopropyleneethyl acetate (1261 mg, 10.0 mmol) and diethyl acetamidomalonate (1671 mg, 7.69 mmol) were dissolved in ethanol (20 mL). Newly prepared sodium ethoxide (157 mg, 2.31 mmol) was added, and reacted under a nitrogen atmosphere at 80°C for 3 hrs. After the reaction, the reaction solution was concentrated, added with ethyl acetate (200 mL), washed with water (20 mLX2) and saturated sodium chloride (20 mL) respectively, dried over anhydrous sodium sulfate, filtered, concentrated, and purified by silica gel column chromatography (10-50% petroleum ether/ethyl acetate) to obtain diethyl 6-oxo-5-azaspiro[2.4]heptan-4,4-dicarboxylate (1099 mg, yield: 56%).

Step II: Diethyl 6-oxo-5-azaspiro[2.4]heptan-4,4-dicarboxylate (1099 mg, 4.30 mmol) was dissolved in methanol (20 mL), and 10% sodium hydroxide solution (5.0 mL) was added. The reaction was continued at 60°C for 1 hr. The reaction solution was concentrated, diluted with water (20 mL), and adjusted to pH 4-5 with 2N hydrochloric acid, to obtain a solid, which was filtered and dried to obtain 6-oxo-5-azaspiro[2.4]heptan-4,4-dicarboxylic acid (770 mg, yield: 90.0%).

Step III: 6-oxo-5-azaspiro[2.4]heptan-4,4-dicarboxylic acid (770 mg, 3.87 mmol) was directly heated to 120°C and reacted for 1 hr. After the reaction was completed, 6-oxo-5-azaspiro[2.4]heptan-4-carboxylic acid (550 mg, yield: 91.7%) was obtained.

Step IV: 6-oxo-5-azaspiro[2.4]heptan-4-carboxylic acid (550 mg, 3.55 mmol) was dissolved in methanol (20 mL), concentrated sulfuric acid (0.2 mL) was added, and reacted at 60°C for 16 hrs. The reaction was shown to be completed by LC-MS. The reaction solution was concentrated, added with ethyl acetate (200 mL), washed with saturated sodium bicarbonate (20 mLX2) and saturated sodium chloride (10 mL) respectively, dried over anhydrous sodium sulfate, filtered, rotary evaporated to dryness and purified by silica gel column chromatography (10-70%petroleum ether/ethyl acetate ) to obtain methyl 6-oxo-5-azaspiro[2.4]heptan-4-carboxylate (500 mg, yield: 83.3%).

Step V: Methyl 6-oxo-5-azaspiro[2.4]heptan-4-carboxylate (500 mg, 2.96 mmol), 3-chloro-2-(chloromethyl)prop-1-ene (592 mg, 4.74 mmol) was dissolved in tetrahydrofuran (20 mL), lithium bis(trimethylsilyl)amide (6.0 mL, 1M) was added at -40°C, heated to room temperature and reacted for 16 hrs. The reaction was quenched with an ammonium chloride solution (40 mL). The reaction solution was extracted with ethyl acetate (50 mLX2). The organic phase was washed with brine, dried over anhydrous sodium sulfate, filtered and concentrated, to obtain a crude product. The crude product was purified by silica gel column chromatography (petroleum ether/ethyl acetate = 1:1) to obtain the product methyl 6'-methylene-3'-oxotetrahydrospiro[cyclopropane-1,1'-pyrrolizin]-7a'(5'H)-carboxylate (207 mg, yield: 31.56%).

Step VI: Methyl 6'-methylene-3'-oxotetrahydrospiro[cyclopropane-1,1'-pyrrolizin]-7a'(5'H)-carboxylate (207 mg, 0.93 mmol) was dissolved in tetrahydrofuran (10 mL), lithium aluminum hydride (4.5 mL, 1M) was added dropwise in an ice bath, heated to 70 °C and reacted for 3 hrs. The reaction was quenched with water (0.2 mL), 15% sodium hydroxide aqueous solution (0.2 mL), and then water (0.6 mL), filtered, and concentrated to obtain a crude product that was (6'-methylenetetrahydrospiro[cyclopropane-1,1'-pyrrolizin]-7a'(5'H)-yl)methanol (130 mg, yield: 78.3%).

Step VII: (6'-methylenetetrahydrospiro[cyclopropane-1,1'-pyrrolizin]-7a'(5'H)-yl)methanol (25 mg, 0.14 mmol) was dissolved in dry tetrahydrofuran (5 mL). 0°C, sodium hydride (28 mg, 0.70 mmol) was added and stirred at 0°C for 15 min. Then, t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) was added, and reacted at room temperature for 16 hrs. After the reaction, the reaction solution was added with water (10 mL), extracted with ethyl acetate (15 mLX3), dried over anhydrous sodium sulfate, filtered concentrated, and purified by silica gel column chromatography (50-100%ethyl acetate /petroleum ether) to obtain t-butyl (SS,SaS,6S,9R)-1-fluoro-2-(7-fluoro-8-((triisopropylsilyl)ethynyl)naphthalen-1-yl)-5-methyl-12-((6'-methylenetetrahydrospiro[cyclopropane-1,1'-pyrrolizin]-7a'(5'H)-methoxy)-5a,6,7,8,9,10-hexahydro-5H-4-oxa-3,10a,11,13,14-pentaaza-6,9-methanonaphtho[1,8-ab]hepten-14-carboxylate (35 mg, yield: 62.4%). ES-API:[M+H]⁺= 902.1.

Step VIII: T-butyl (5S,5aS,6S,9R)-1-fluoro-2-(7-fluoro-8-((triisopropylsilyl)ethynyl) naphthalen-1-yl)-5-methyl-12-((6'-methylenetetrahydrospiro[cyclopropane-1,1'-pyrrolin]-7a'(5'H)-methoxy)-5a,6,7,8,9,10-hexahydro-5H-4-oxy-3,10a,11,13,14-pentaaza-6,9-methanonaphtho[1,8-ab]hepten-14-carboxylate (35 mg, 0.039 mmol) was dissolved in N,N-dimethylformamide (2 mL), and then cesium fluoride (60 mg, 0.39 mmol) was added and reacted at room temperature for 1 hr. The reaction solution was extracted with ethyl acetate (10 mLX3). The organic phase was dried over anhydrous sodium sulfate, filtered, rotary evaporated to dryness, and purified by preparative HPLC (trifluoroacetic acid method) to obtain t-butyl (5S,5aS,6S,9R)-2-(8-ethynyl-7-fluoronaphthalen-1-yl)-1-fluoro-5-methyl-12-((6'-methylenetetrahydrospiro [cyclopropyl-1,1'-pyrrolizin]-7a'(5'H)-methoxy)-5a,6,7,8,9,10-hexahydro-5H-4-oxa-3,10a,11,13,14-pentaaza-6,9-methanonaphtho[1,8-ab]hepten-14-carboxylate (20 mg, yield: 68.7%). ES-API:[M+H]⁺=746.3.

Step IX: T-butyl (5S,5aS,6S,9R)-2-(8-ethynyl-7-fluoronaphthalen-1-yl)-1-fluoro-5-methyl-12-((6'-methylenetetrahydrospiro[cyclopropyl-1,1'-pyrrolizin]-7a'(5'H)-methoxy)-5a,6,7,8,9,10-hexahydro-5H-4-oxy-3,10a,11,13,14-pentaaza-6,9-methanonaphtho[1,8-ab]hepten-14-carboxylate (20 mg, 0.027 mmol) was dissolved in acetonitrile (5 mL). In an ice bath, 4M hydrochloric acid/dioxane solution (1 mL) was added, and reacted for 1 hr in the ice bath. The reaction solution was adjusted to pH 8 with saturated sodium bicarbonate, extracted with ethyl acetate (15 mLX3), rotary evaporated to dryness, and purified by preparative HPLC (trifluoroacetic acid method) to obtain (5S,5aS,6S,9R)-2-(8-ethynyl-7-fluoronaphthalen-1-yl)-1-fluoro-5-methyl-12-((6'-methylenetetrahydrospiro[cyclopropyl-1,1'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]heptene (Z602, 9.5 mg, yield: 54.4%, trifluoroacetate). ES-API:[M+H]⁺=647.4. ¹H NMR (400 MHz, CD₃OD) δ 8.44 (s, 2H), 8.20 -8.01 (m, 2H), 7.71 -7.36 (m, 3H), 5.41 (d, J = 13.6 Hz, 1H), 5.23 (s, 2H), 4.70 -4.40 (m, 3H), 4.34 -4.08 (m, 2H), 3.98 -3.44 (m, 5H), 3.26 (d, J = 8.6 Hz, 2H), 2.81 (s, 1H), 2.58 (d, J = 15.8 Hz, 1H), 2.36 -2.22 (m, 1H), 1.99 (ddd, J = 63.6, 17.5, 6.1 Hz, 5H), 1.69 -1.50 (m, 3H), 0.99 -0.77 (m, 3H), 0.78 -0.68 (m, 1H).

### Example 46: Synthesis of (55,5aS,65,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z607)

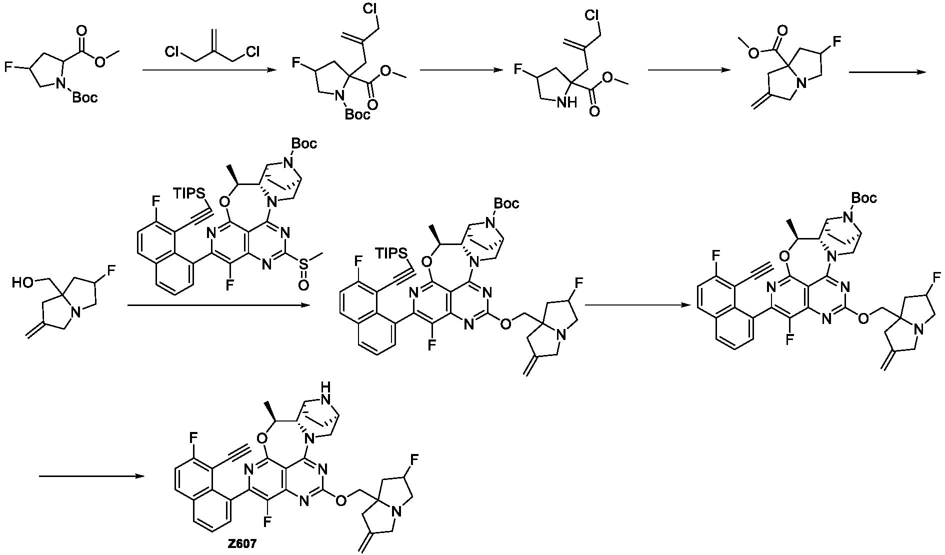

Step I: Under a nitrogen atmosphere, 1-(t-butyl) 2-methyl 4-fluoropyrrolidin-1,2-dicarboxylate (1 g, 4.044 mmol) was dissolved in anhydrous tetrahydrofuran (15 mL), and the mixture was allowed to stand in a dry ice-acetone bath. Lithium bis(trimethylsilyl)amide (6.1 mL, 6.066 mmol) was slowly added dropwise to the above solution, and stirred at this temperature for 1 hr. Then, a solution of 3-chloro-2-chloromethylpropene (2.53 g, 20.222 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (30 mL), and extracted with ethyl acetate (20 mLX3). The organic phase was washed with saturated brine (15 mLX3), dried over anhydrous sodium sulfate, filtered, and concentrated. The crude product was purified by silica gel column chromatography to obtain 1-(t-butyl) 2-methyl 2-(2-(chloromethyl)allyl)-4-fluoropyrrolidin-1,2-dicarboxylate (1354 mg, yield: 100%). ES-API: [M-55]⁺= 280.1.

Step II: At room temperature, 4 M hydrochloric acid-dioxane solution (10 mL) was slowly added to a solution of 1-(t-butyl)2-methyl 2-(2-(chloromethyl)allyl)-4-fluoropyrrolidin-1,2-dicarboxylate (1354 mg, 4.044 mmol) in acetonitrile (6 mL). The reaction mixture was stirred at room temperature for 0.5 hr. The reaction was shown to be completed by LC/MS. The reaction solution was concentrated to dryness under reduced pressure to obtain a crude product that was methyl 2-(2-(chloromethyl)allyl)-4-fluoropyrrolidin-2-carboxylate (1.2 g). ES-API: [M+H]⁺= 236.2.

Step III: At room temperature, sodium bicarbonate (1.93 g, 22.979 mmol) and potassium iodide (76 mg, 0.460 mmol) were added to a solution of methyl 2-(2-(chloromethyl)allyl)-4-fluoropyrrolidin-2-carboxylate (1.08 g, 4.596 mmol) in acetonitrile (60 mL), and the reaction mixture was stirred at room temperature for 1.5 hrs. The reaction was shown to be completed by LC-MS. The reaction solution was filtered, and the insoluble matter was washed with acetonitrile. The filtrate was concentrated under reduced pressure. The residue was purified by silica gel column chromatography to obtain methyl 2-fluoro-6-methylenetetrahydro-1H-pyrrolizin-7a(5H)-carboxylate (980 mg, yield: 96%). ES-API: [M+H]⁺= 200.1.

Step IV: Under a nitrogen atmosphere, methyl 2-fluoro-6-methylenetetrahydro-1H-pyrrolizin-7a(5H)-carboxylate (573 mg, 2.879 mmol) was dissolved in anhydrous tetrahydrofuran(10 mL), and the mixture was allowed to stand in an iced water bath. Lithium aluminium hydride (5.8 mL, 219 mg, 5.76 mmol, 1 M in tetrahydrofuran) was slowly added dropwise to the above solution, and the stirred at this temperature for 0.5 hr. The reaction was shown to be completed by LC/MS. The reaction solution was added with sodium sulfafe decahydrate (438 mg) to quench to reaction, stirred at room temperature for 0.5 hr, and filtered to remove the insoluble matter. The filtrate was concentrated under reduced pressure, to obtain (2-fluoro-6-methylenetetrahydro-1H-pyrrolizin-7a(5H)-yl)methanol (370 mg, yield: 56%). ES-API: [M+H]⁺= 172.1.

Step V: (2-fluoro-6-methylenetetrahydro-1H-pyrrolizin-7a(5H)-yl)methanol (53 mg, 0.308 mmol) was dissolved in anhydrous tetrahydrofuran (5 mL). In an iced water bath, sodium hydride (12 mg, 0.308 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (81 mg, 0.103 mmol) in tetrahydrofuran (2 mL) was added dropwise to the reaction solution, and reacted at 0°C for 30 min. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure, to obtain the crude product t-butyl (5S,5aS,6S,9R)-1-fluoro-12-((2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]hepten-14-carboxylate (88 mg). ES-API: [M+H]⁺= 895.4.

Step VI: Cesium fluoride (150 mg, 0.983 mmol) was added to t-butyl (5S,5aS,6S,9R)-1-fluoro-12-((2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-4-oxy-3,10a,11,13,14-pentaaza-6,9-methanonaphtho[1,8-ab]hepten-14-carboxylate (88 mg, 0.098 mmol) in N,N-dimethylformamide (5 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated, to obtain the crude product t-butyl (5S,5aS,6S,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a, 6,7,8,9,10-hexahydro-5H-4-oxy-3,10a,11,13,14-pentaaza-6,9-methanonaphtho[1,8-ab]hepten-14-carboxylate (72 mg). ES-API: [M+H]⁺= 739.3.

Step VII: In an iced water bath, 4 M hydrochloric acid-dioxane solution (5 mL) was slowly added to a solution of t-butyl (5S,5aS,6S,9R)-2-(8-ethynyl-7-fluoronaphthalen-1-yl)-1-fluoro-12-((2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (72 mg, 0.097 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-50-65-13min) to obtain (5S,5aS,6S,9R)-2-(8-ethynyl-7-fluoronaphthalen-1-yl)-1-fluoro-12-((2-fluoro-6-methylenetetrahydro- 1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z607, 11.3 mg, yield: 18%) as an off-white solid. ES-API: [M+H]⁺= 639.3.

### Example 47: Synthesis of (5R,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z630)

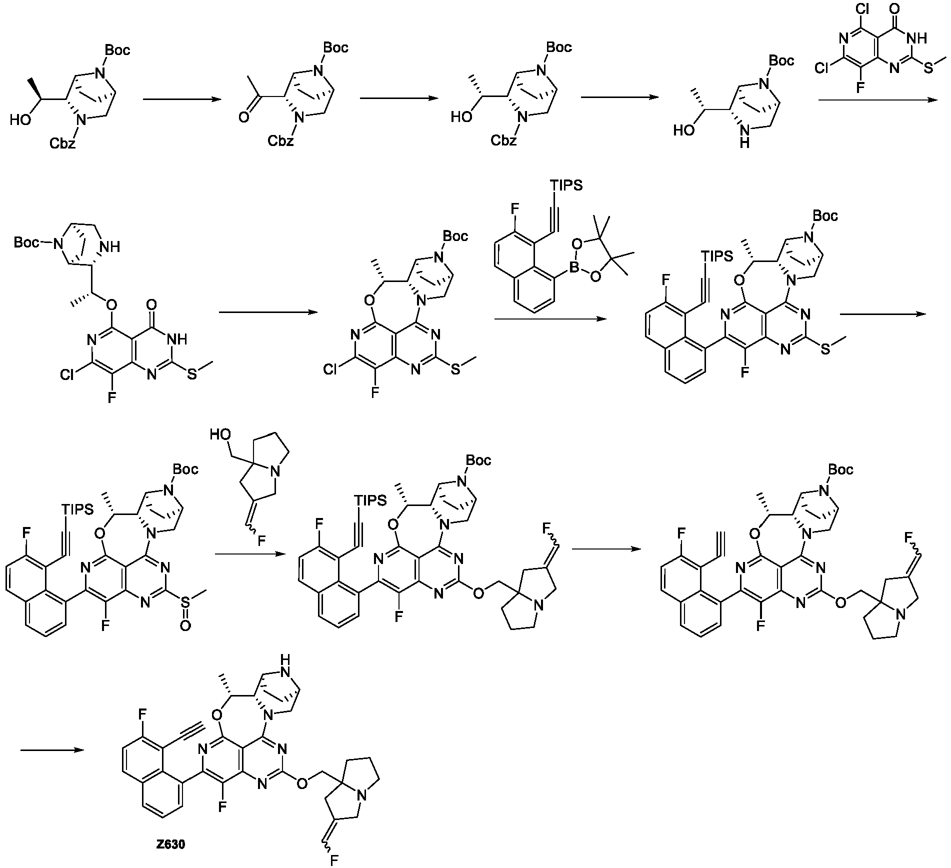

Step I: 3-benzyl 8-(t-butyl) (1S,2S,5R)-2-((S)-1-hydroxyethyl)-3,8-diazacyclo[3.2.1]octane-3,8-dicarboxylate (120 mg, 0.31 mmol) was dissolved in anhydrous dichloromethane (6 mL). At 0°C, Dess-Martin periodinane (196 mg, 0.46 mmol) was added, and reacted at room temperature for 2 hrs. The reaction was shown to be completed by LC/MS. The reaction solution was added with saturated sodium thiosulphate solution (10 mL) and saturated sodium bicarbonate aqueous solution (10 mL). The reaction solution was extracted with ethyl acetate (20 mLX2). The organic phase was washed with saturated brine (20mL), dried over anhydrous sodium sulfate, filtered, and concentrated. The crude product was purified by flash silica gel column chromatography (0-25% ethyl acetate /petroleum ether) to obtain 3-benzyl 8-(t-butyl)(1S,2S,5R)-2-acetyl-3,8-diazabicyclo [3.2.1]octane-3,8-dicarboxylate (100 mg, yield: 87%). ES-API: [M+H]⁺=389.2.

Step II: 3-benzyl 8-(t-butyl)(1S,2S,5R)-2-acetyl-3,8-diazabicyclo[3.2.1]octane-3,8-dicarboxylate (100 mg, 0.26 mmol) was dissolved in anhydrous ethanol (5 mL). At 0°C, a 2 M lithium borohydride solution in tetrahydrofuran (0.26 mL, 0.52 mmol) was added. The reaction was continued at room temperature for 1 hr. The reaction was shown to be completed by LC/MS. The reaction solution was added with saturated ammonium chloride aqueous solution (20 mL). The reaction solution was extracted with ethyl acetate (20 mLX2). The organic phase was washed with saturated brine (20mL), dried over anhydrous sodium sulfate, filtered, and concentrated. The crude product was purified by flash silica gel column chromatography (0-40% ethyl acetate/petroleum ether) to obtain 3-benzyl 8-(t-butyl) (1S,2S,5R)-2-((R)-1-hydroxyethyl)-3,8-diazabicyclo[3.2.1]octane-3,8-dicarboxylate (80 mg, yield: 79%) as a colorless oil. ES-API: [M-55]⁺=335.2.

Step III: 3-benzyl 8-(t-butyl) (1S,2S,5R)-2-((R)-1-hydroxyethyl)-3,8-diazabicyclo [3.2.1] octane-3,8-dicarboxylate (260 mg, 0.67 mmol), palladium on carbon (142 mg, 0.13 mmol), acetic acid (249 mg, 1.33 mmol) and 2,2,2-trifluoroether-1-ol (12 mL) were sequentially added to a round-bottom flask. The reaction was stirred at 30°C under hydrogen atmosphere for 16 hrs. The reaction was shown to be completed by LC/MS. The reaction solution was filtered through diatomite, and the filtrate was concentrated. The crude product was dissolved in water, and the pH was adjusted to 5 with 1M hydrochloric acid. The reaction solution was extracted with ethyl acetate (30 mLX1) and the organic phase was discarded. The aqueous phase was alkalinized with 2M sodium hydroxide, and extracted with dichloromethane/isopropanol (3: 1, 30 mLX3). The organic phase was dried over anhydrous sodium sulfate, filtered, and concentrated to obtain t-butyl (1S,25,5R)-2-((R)-1-hydroxyethyl)-3,8-diazabicyclo[3.2.1]octane-8-carboxylate (130 mg, yield: 76%)) as a yellow oil. ES-API: [M+H]⁺=257.1.

Step IV: At 0°C, 60% sodium hydride (61 mg, 2.54 mmol), t-butyl (15,2S,5R)-2-((R)-1-hydroxyethyl)-3,8-diazabicyclo[3.2.1]octane-8-carboxylate (130 mg, 0.51 mmol), and tetrahydrofuran (8 mL) were added to a round-bottom flask, and stirred at room temperature for 10 min. After cooling to 0°C, 5,7-dichloro-8-fluoro-2-(methylthio)pyridine[4,3-d]pyrimidine-4(3H)-one (284 mg, 1.01 mmol) was added. The reaction was stirred in an oil bath at 60°C for 60 min. The reaction was shown to be completed by LC-MS. The reaction solution was added with saturated ammonium chloride aqueous solution (50 mL). The reaction solution was extracted with ethyl acetate (50 mLX2). The organic phase was washed with saturated brine (50 mL), dried over anhydrous sodium sulfate, filtered, and concentrated. The crude product was purified by flash silica gel column chromatography (0-10%methanol /petroleum ether) to obtain t-butyl (1S,2S,5R)-2-((R)-1-((7-chloro-8-fluoro-2-(methylthio)-4-oxa-3,4-dihydropyridine[4,3-d]pyrimidine-5-yl)oxy)ethyl)-3,8-diazabicyclo[3.2.1]octane-8-carboxylate (200 mg, yield: 79%) as a yellow solid. ES-API: [M+H]⁺=500.2.

Step V: T-butyl (1S,2S,5R)-2-((R)-1-((7-chloro-8-fluoro-2-(methylthio)-4-oxa-3,4-dihydropyridine[4,3-d]pyrimidine-5-yl)oxy)ethyl)-3,8-diazabicyclo[3.2.1]octane-8-carboxylate (170 mg, 0.34 mmol), N,N-diisopropylethyl amine (0.21 mL, 1.19 mmol) and dichloromethane(15 mL) were added to a 100 mL round-bottom flask. After cooling to 0°C, 2-(7-azobenzotriazole)-N,N,N',N'-tetramethyluronium hexafluorophosphate (259 mg, 0.68 mmol) was added, and reacted with stirring at 30°C for 16 hrs. The reaction was shown to be completed by LC-MS. The reaction solution was added with dichloromethane (50 mL), and then washed with water (30 mL) and saturated sodium bicarbonate aqueous solution (30 mL) respectively. The organic phase was dried over anhydrous sodium sulfate, filtered and concentrated. The crude product was purified by flash silica gel column chromatography (ethyl acetate /petroleum ether: 0-30%) to obtain t-butyl (5R,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (120 mg, yield: 73%) as a yellow solid.

Step VI: T-butyl (5R,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (110 mg, 0.23 mmol), ((2-fluoro-8-(4,4,5,5-tetramethyl-1,3,2-dioxaborolan-2-yl)naphthalen-1-yl)ethynyl)triisopropylsilane (206 mg, 0.46 mmol), [n-butyldi(1-adamantanyl) phosphine](2-amino-1,1'-biphenyl-2-yl)palladium (II) mesylate (17 mg, 0.023 mmol), potassium phosphate (145 mg, 0.68 mmol), tetrahydrofuran (6mL), and water (1.5 mL) were added to a round-bottom flask. Under a nitrogen atmosphere, the system was heated in a microwave reactor at 75°C for 40 min. The reaction solution was added with water (30 mL), and extracted with ethyl acetate (30 mLX3). The organic phase was dried over anhydrous sodium sulfate, filtered and concentrated. The crude product was purified by flash silica gel column chromatography (ethyl acetate /petroleum ether: 0-25%) to obtain t-butyl (5R,5aS,6S,9R)-1-fluoro-2-(7-fluoro-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (130 mg, yield: 74%) as a yellow solid. ES-API: [M+H]⁺=772.3.

Step VII: T-butyl (5R,5aS,6S,9R)-1-fluoro-2-(7-fluoro-8-((triisopropylsilyl)ethynyl) naphthalen-1-yl)-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (130 mg, 0.17 mmol) and dichloromethane (6 mL) were added to a round-bottom flask. The reaction solution was cooled to 0°C, and m-chloroperoxybenzoic acid (44 mg, 0.25 mmol) was added, and reacted with stirring at room temperature for 1 hr. The reaction solution was added with a saturated sodium thiosulfate aqueous solution (30 mL) and a saturated sodium bicarbonate aqueous solution, and extracted with ethyl acetate (30 mLX3). The organic phase was dried and concentrated to obtain t-butyl (SR,SaS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (130 mg, yield: 97.8%) as a yellow solid. ES-API: [M+H]⁺=788.3.

Step VIII: Sodium hydride (15 mg, 0.38 mmol) and tetrahydrofuran (4 mL) were added to a round-bottom flask. The reaction solution was cooled to 0°C, and (2-(fluoromethylene)tetrahydro-1H-pyrrolizin-7a(5H)-yl)methanol (33 mg, 0.19 mmol) dissolved in anhydrous tetrahydrofuran (1 mL) was added to the reaction solution. The reaction was continued at room temperature for 20 min. T-butyl (5R,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (60 mg, 0.076 mmol) was added to the reaction solution, and reacted at 0°C for 20 min. The reaction was shown to be completed by LC/MS. The reaction solution was added with a saturated ammonium chloride solution (30 mL). The reaction solution was extracted with ethyl acetate (20 mLX2). The organic phase was washed with saturated brine (30 mL), dried over anhydrous sodium sulfate, filtered, and concentrated to obtain t-butyl (5R,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (60 mg, crude product). ES-API: [M+H]⁺=895.5.

Step IX: Cesium fluoride (102 mg, 0.67 mmol) was added to t-butyl (5R,5aS,6S,9R)-1-fluoro-2-(7-fluoro-8-((triisopropylsilyl)ethynyl)naphthalen-1-yl)-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (60 mg, crude product) in N,N-dimethyl formamide (3 mL). The reaction mixture was stirred at room temperature for 1 hr. The reaction was shown to be completed by LC/MS. The reaction solution was diluted with ethyl acetate (50 mL), washed with water (15 mLX2) and saturated brine (15 mLX3), dried over anhydrous sodium sulfate, filtered, and concentrated to obtain t-butyl (5R,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (49 mg, crude product). ES-API: [M+H]⁺= 739.3.

Step X: In an iced water bath, 4 M hydrochloric acid-dioxane solution (2 mL) was slowly added to a solution of t-butyl (5R,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (49 mg, crude product) in acetonitrile (2 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate method 1) to obtain (5R,5aS,6S,9R)-2-(8-ethynyl-7-fluoronaphthalen-1-yl)-1-fluoro-12-((2-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z630, 10 mg, yield: 23%) as a white solid. ES-API: [M+H]⁺= 639.2.

### Example 48: Synthesis of (5S,5aS,6S,9R)-2-(8-ethynyl-7-fluoronaphthalen-1-yl)-1-fluoro-5-methyl-12-(((1aS,6bR)-5-methylenehexahydrocyclopropane[a]pyrrolizin-6a(4H)-yl)methoxy)-5a,6,7,8,9,10-hexahydro-5H-4-oxy-3,10a,11,13,14-pentaaza-6,9-methanonaphtho[1,8-ab]heptene (Z606)

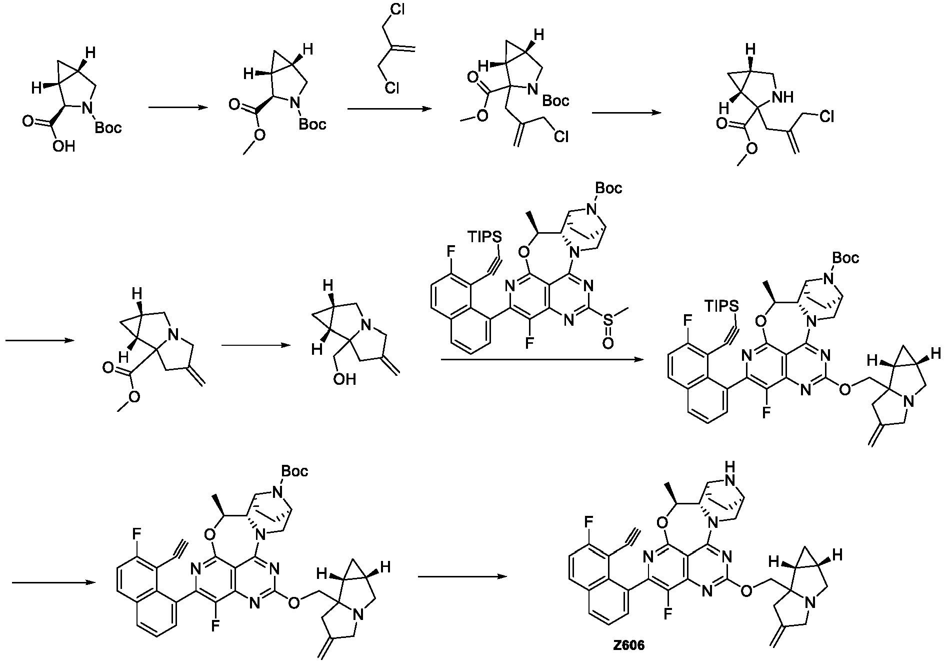

Step I: In an iced water bath, potassium carbonate (456 mg, 3.3 mmol) and iodomethane (312 mg, 2.2 mmol) were added to a solution of (1R,2R,5S)-3-(t-butoxycarbonyl)-3-azabicyclo [3.1.0]hexane-2-carboxylic acid (250 mg, 1.1 mmol) in N,N-dimethylformamide (5 mL), and stirred at room temperature for 1.5 hrs. The reaction was shown to be completed by LC/MS. The reaction solution was filtered, and washed with ethyl acetate. The filtrate was concentrated under reduced pressure, to obtain 3-(t-butyl) 2-methyl (1R,2R,5S)-3-azabicyclo[3.1.0]hexane-2,3-dicarboxylate (265 mg, yield: 100%). ES-API: [M-100]⁺= 186.1.

Step II: Under a nitrogen atmosphere, 3-(t-butyl) 2-methyl (1R,2R,5S)-3-azabicyclo [3.1.0]hexane-2,3-dicarboxylate (220 mg, 0.9 mmol) was dissolved in anhydrous tetrahydrofuran (10mL), and the mixture was allowed to stand in a dry ice-acetone bath. A lithium bis(trimethylsilyl)amide solution (1.8 mL, 1.8 mmol, 1 M in tetrahydrofuran) was slowly added dropwise to the above solution, and stirred at this temperature for 1 hr. A solution of 3-chloro-2-(chloromethyl)prop-1-ene (570 mg, 4.6 mmol) in anhydrous tetrahydrofuran (10 mL) was slowly added by a syringe to the solution, and the reaction solution was continuously stirred at -78°C for 1 hr. The reaction was shown to be completed by LC/MS. The reaction solution was quenched with a saturated ammonium chloride solution (50 mL) and extracted with ethyl acetate (50 mLX3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 3-(t-butyl) 2-methyl (1R,5S)-2-(2-(chloromethyl)allyl)-3-azabicyclo[3.1.0]hexane-2,3-dicarboxylate (160 mg, yield: 53%). ES-API: [M-56]⁺= 274.1.

Step III: At room temperature, 4M hydrochloric acid/dioxane solution (5mL) was slowly added to a solution of 3-(t-butyl) 2-methyl (1R,5S)-2-(2-(chloromethyl)allyl)-3-azabicyclo[3.1.0]hexane-2,3-dicarboxylate (160 mg, 0.5 mmol) in acetonitrile (2 mL), and stirred at room temperature for 0.5 hr. The reaction was shown to be completed by LC/MS. The reaction solution was concentrated under reduced pressure, to obtain methyl (1R,5S)-2-(2-(chloromethyl)allyl)-3-azabicyclo [3.1.0]hexane-2-carboxylate (111 mg, yield: 100%). ES-API: [M+H]⁺= 230.2.

Step IV: At room temperature, sodium bicarbonate (203 mg, 2.4 mmol) and potassium iodide (8 mg, 0.05 mmol) were added to a solution of methyl (1R,5S)-2-(2-(chloromethyl)allyl)-3-azabicyclo[3.1.0]hexane-2-carboxylate (111 mg, 0.5 mmol) in acetonitrile (10 mL), and stirred at room temperature for 1.5 hrs. The reaction was shown to be completed by LC/MS. The reaction solution was filtered, and washed with acetonitrile. The filtrate was concentrated under reduced pressure, to obtain methyl (1aS,6bR)-5-methylenehexahydrocyclopropane[a]pyrrolizin-6a(4H)-carboxylate (94 mg, crude product). ES-API: [M+H]⁺=194.1.

Step V: Under a nitrogen atmosphere, methyl (1aS,6bR)-5-methylenehexahydrocyclopropane [a]pyrrolizin-6a(4H)-carboxylate (94 mg, 0.5 mmol) was dissolved in anhydrous tetrahydrofuran (5mL), and the mixture was allowed to stand in an iced water bath. A lithium aluminium hydride solution (1.0 mL, 1.0 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (100 mg), and stirred at room temperature for 0.5 hr. The reaction solution was filtered, and the filtrate was concentrated under reduced pressure, to obtain ((1aS,6bR)-5-methylenehexahydrocyclopropane[a]pyrrolizin-6a(4H)-yl)methanol (43 mg, yield:54%). ES-API: [M+H]⁺= 166.1.

Step VI: ((1aS,6bR)-5-methylenehexahydrocyclopropane[a]pyrrolizin-6a(4H)-yl)methanol (34 mg, 0.2 mmol) was dissolved in anhydrous tetrahydrofuran (5 mL). In an iced water bath, sodium hydride (10 mg, 0.4 mmol) was added, and then reacted for 30 min in the iced water bath. A solution of t-butyl (SS,SaS,6S,9R)-1-fluoro-2-(7-fluoro-8-((triisopropylsilyl)ethynyl)naphthalen -1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (81 mg, 0.1 mmol) in tetrahydrofuran (2 mL) was added dropwise to the reaction solution, and reacted at 40°C for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, filtered, and concentrated. The crude product was purified by silica gel column chromatography(dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-(((1as,6bR)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate (17 mg, yield: 20%). ES-API: [M+H]⁺= 889.5.

Step VII: Cesium fluoride (30 mg, 0.2 mmol) was added to t-butyl (5S,5aS,6S,9R)-1-fluoro-2-(7-fluoro-8-((triisopropylsilyl)ethynyl)naphthalen-1-yl)-5-methyl-12-((1aS,6bR)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]hepten-14-carboxylate (17 mg, 0.02 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-((1aS,6bR)-5-methylenehexahydrocyclopropane[a]pyrrolizin-6a(4H)-yl)methoxy)-5a,6,7,8,9,10-hexahydro-5H-4-oxy-3,10a,11,13,14-pentaaza-6,9-methanonaphtho[1,8-ab]hepten-14-carboxylate (15 mg, crude product). ES-API: [M+H]⁺= 733.3.

Step VIII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-((1aS,6bR)-5-methylenehexahydrocyclopropane[a]pyrrolizin-6a(4H)-yl)methoxy)-5a,6,7,8,9,10-hexahydro-5H-4-oxy-3,10a,11,13,14-pentaaza-6,9-methanonaphtho[1,8-ab]hepten-14-carboxylate (15 mg, 0.02 mmol) in acetonitrile (1 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-(((1aS,6bR)-5-methylenehexahydrocyclopropane[a]pyrrolizin-6a(4H)-yl)methoxy)-5a,6,7,8,9,10-hexahydro-5H-4-oxa-3,10a,11,13,14-pentaaza-6,9-methanonaphtho[1,8-ab]heptene (Z606, 1.2 mg, yield: 9.5%) as a light yellow solid. ES-API: [M+H]⁺= 633.3.

### Example 49: Synthesis of (5S,5aS,6S,9R)-2-(8-ethynyl-7-fluoronaphthalen-1-yl)-1-fluoro-5-methyl-12-((tetrahydrospiro[cyclopropyl-1,3'-pyrrolizin]-7a'(5'H)-yl)-methoxy)-5a,6,7,8,9,10-hexahydro-5H-4-oxy-3,10a,11,13,14-pentaaza-6,9-methanonaphtho[1,8-ab]heptene (Z631)

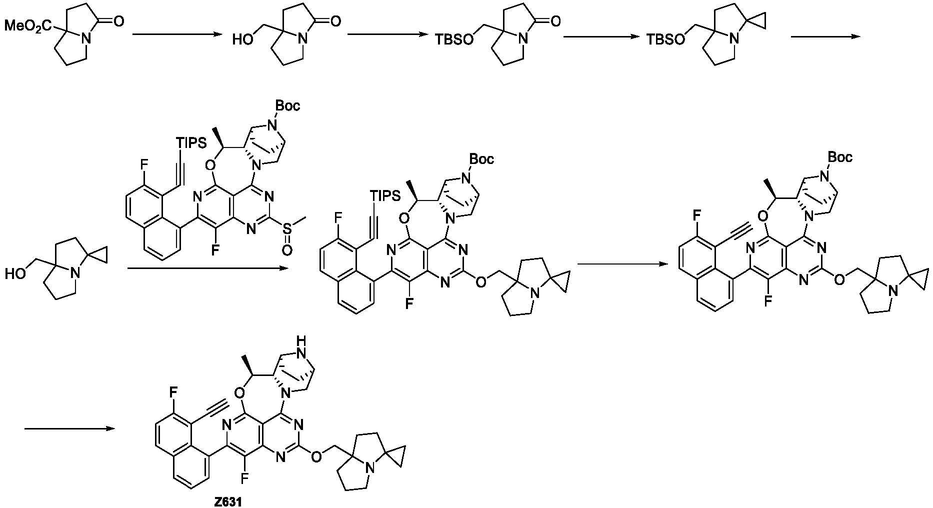

Step I: Methyl 3-oxytetrahydro-1H-pyrrolizin-7a(5H)-carboxylate (900 mg, 4.9 mmol) was dissolved in methanol (10 mL), cooled to 0°C, added with sodium borohydride (373 mg, 9.8 mmol), heated to room temperature and reacted for 16 hrs. After the reaction, the reaction was quenched with a saturated ammonium chloride aqueous solution, extracted with ethyl acetate (20 mLX3), washed with saturated sodium chloride (20 mL), dried over anhydrous sodium sulfate, filtered, and rotary evaporated to dryness to obtain 7a-(hydroxymethyl)hexahydro-3H-pyrrolizin-3-one (600 mg, yield: 79%) as a crude product.

Step II: 7a-(hydroxymethyl)hexahydro-3H-pyrrolizin-3-one (600 mg, 3.87 mmol) was dissolved in dichloromethane, imidazole (394 mg, 5.80 mmol) was added, and then t-butyldimethylchlorosilane (870 mg, 5.80 mmol) was slowly added, and reacted at room temperature for 16 hrs. After the reaction, the reaction solution was added with water, extracted with ethyl acetate (30 mLX3), washed with saturated sodium chloride (30 mL), dried over anhydrous sodium sulfate, filtered, rotary evaporated to dryness, and purified by silica gel column chromatography (petroleum ether/ethyl acetate = 0-50%) to obtain 7a-(((t-butyldimethylsilyl) oxy)methyl)hexahydro-3H-pyrrolizin-3-one (900 mg, yield: 82.7%).

Step III: 7a-(((t-butyldimethylsilyl)oxy)methyl)hexahydro-3H-pyrrolizin-3-one (900 mg, 3.2 mmol) and titanium isopropoxide (2.73g, 9.6mmol) were dissolved in anhydrous tetrahydrofuran. At 0°C, ethyl Grignard reagent (0.64 mL, 2.5 mol/L, 16 mmol) was slowly added dropwise, and then reacted at room temperature for 16 hrs. After the reaction, the reaction was quenched with saturated ammonium chloride aqueous solution, extracted with ethyl acetate (30 mLX3), washed with saturated sodium chloride (30 mL), dried over anhydrous sodium sulfate, filtered, rotary evaporated to dryness, and purified by silica gel column chromatography (petroleum ether/ethyl acetate = 0-30%) to obtain 7a'-(t-butyldimethylsilyl)oxy)methyl)hexahydrospiro[cyclopropane-1,3'-pyrrolizine] (350 mg, yield: 38.9%).

Step IV: 7a'-(t-butyldimethylsilyl)oxy)methyl)hexahydrospiro[cyclopropane-1,3'-pyrrolizine] (150 mg, 0.53 mmol) was dissolved in tetrahydrofuran (20 mL), and then tetrabutylammonium fluoride (2 mL) was added, and reacted at room temperature for 2 hrs. After the reaction, the reaction solution was concentrated, and the residue was pufified by column chromatography (petroleum ether/ethyl acetate =10-100%) to obtain (tetrahydrospiro[cyclopropane-1,3'-pyrrolizin]-7a'(5'H)-yl)methanol (40 mg, yield: 45.2%).

Step V: (tetrahydrospiro[cyclopropane-1,3'-pyrrolizin]-7a'(5'H)-yl)methanol (40 mg, 0.24 mmol) was dissolved in dry tetrahydrofuran (5 mL). At 0°C, sodium hydride (48 mg, 1.2 mmol) was added and stirred at 0°C for 15 min. Then, t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (50 mg, 0.063 mmol) was added, and reacted at room temperature for 16 hrs. After the reaction, the reaction solution was added with water (10 mL), extracted with ethyl acetate (15mLX3), dried over anhydrous sodium sulfate, filtered, concentrated, and purified by silica gel column chromatography (50-100% ethyl acetate /petroleum ether) to obtain t-butyl (5S,5aS,6S,9R)-1-fluoro-2-(7-fluoro-8-((triisopropylsilyl)ethynyl)naphthalen-1-yl)-5-methyl-12-((tetrahydrospiro[1,3"-pyrrolizin]-7a'(5'H)-ylmethoxy)-5a,6,7,8,9,10-hexahydro-4-oxa-3,10a,11,13,14-pentaaza-6,9-methanonaphtho[1,8-ab]hepten-14-carboxylate (30 mg, yield: 53.5%). ES-API: [M+H]⁺= 890.3.

Step VI: T-butyl (5S,5aS,6S,9R)-1-fluoro-2-(7-fluoro-8-((triisopropylsilylethynyl)naphthalen -1-yl)-5-methyl-12-((tetrahydrospiro[1,3'-pyrrolizin]-7a'(5'H)-ylmethoxy)-5a,6,7,8,9,10-hexahydro-4-oxa-3,10a,11,13,14-pentaaza-6,9-methanonaphtho[1,8-ab]hepten-14-carboxylate (30 mg, 0.034 mmol) was dissolved in N,N-dimethylformamide (2 mL), cesium fluoride (52 mg, 0.34 mmol) was added, and reacted at room temperature for 1 hr. The reaction solution was extracted with ethyl acetate (10 mLX3). The organic phase was dried over anhydrous sodium sulfate, filtered, andd rotary evaporated to dryness, to obtain a crude product that was purified by preparative HPLC (trifluoroacetic acid method) to obtain t-butyl (5S,5aS,6S,9R)-2-(8-ethynyl-7-fluoronaphthalen-1-yl)-1-fluoro-5-methyl-12-((tetrahydrospiro[cyclopropyl-1,3'-pyrrolizin]-7a'(5'H)-yl)methoxy)-5a,6,7,8,9,10-hexahydro-5H-4-oxa-3,10a,11,13,14-pentaaza-6,9-methanonaphtho[1,8-ab]hepten-14-carboxylate (15 mg, yield: 60.1%). ES-API: [M+H]⁺= 734.3.

Step VII: T-butyl (5S,5aS,6S,9R)-2-(8-ethynyl-7-fluoronaphthalen-1-yl)-1-fluoro-5-methyl-12-((tetrahydrospiro[cyclopropyl-1,3'-pyrrolidinyl]-7a'(5'H)-yl)methoxy)-5a,6,7,8,9,10-hexahydro-5H-4-oxa-3,10a,11,13,14-pentaaza-6,9-methanonaphtho[1,8-ab]hepten-14-carboxylate (15 mg, 0.020 mmol) was dissolved in acetonitrile (5 mL), 4M hydrochloric acid/dioxane solution (1 mL) was aded in an ice bath, and reacted for 1 hr in the ice bath. The reaction solution was adjusted to pH 8 with saturated sodium bicarbonate, extracted with ethyl acetate (15 mLX3), and rotary evaporated to dryness. The crude product was purified by preparative HPLC (trifluoroacetic acid method) to obtain (5S,5aS,6S,9R)-2-(8-ethynyl-7-fluoronaphthalen-1-yl)-1-fluoro-5-methyl-12-((tetrahydrospiro[cyclopropyl-1,3'-pyrrolizin]-7a'(5'H)-yl)-methoxy)-5a,6,7,8,9,10-hexahydro-5H-4-oxa-3,10a,11,13,14-pentaaza-6,9-methanonaphtho[1,8-ab]heptene (Z631, 5.0 mg, yield: 39.4%, trifluoroacetate). ES-API: [M+H]⁺= 634.4. ¹H NMR (400 MHz, CD₃OD) δ 8.52 (s, 1H), 8.10 (dd, J = 6.1, 3.0 Hz, 2H), 7.74 -7.24 (m, 3H), 5.39 (d, J = 10.9 Hz, 1H), 4.53 (dd, J = 23.5, 13.1 Hz, 2H), 4.12 (d, J = 9.0 Hz, 1H), 3.81 -3.37 (m, 4H), 3.18 (dd, J = 33.4, 23.2 Hz, 2H), 2.63 -1.69 (m, 12H), 1.59 (t, J = 6.3 Hz, 3H), 1.04 (s, 2H), 0.89 (s, 1H), 0.74 (s, 1H).

### Example 50: Synthesis of (5S,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxy-3,10a,11,13,14-pentaaza-6,9-methanonaphtho[1,8-ab]heptene (Z612)

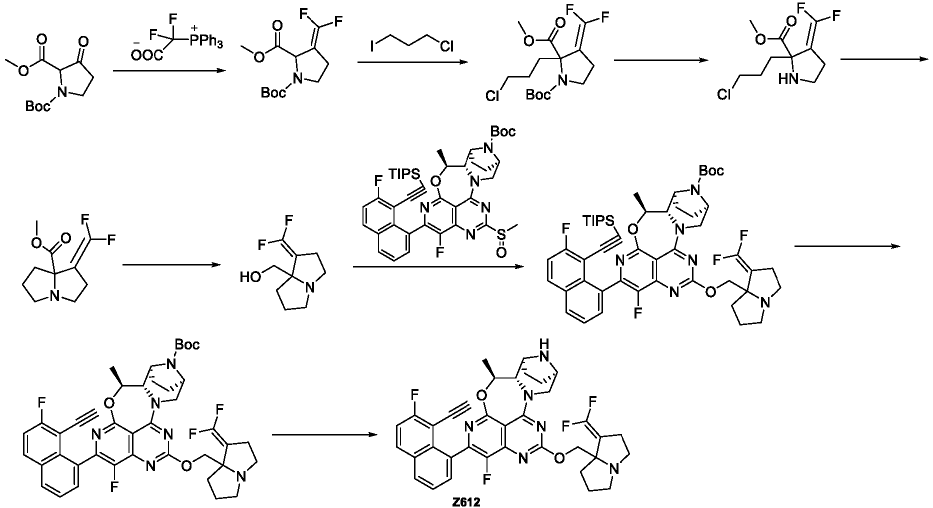

Step I: Under a nitrogen atmosphere, (triphenylphosphonio) difluoroacetate (10 g, 28.066 mmol) was added to 1-(t-butyl) 2-methyl 3-oxopyrrolidin-1,2-dicarboxylate (2.6 g, 10.688 mmol) in N,N-dimethylformamide (50 mL), and the reaction mixture was heated to 80°C and stirred for 2 hrs. The reaction was shown to be completed by LC/MS. The reaction solution was quenched with water (500 mL), and extracted with ethyl acetate (30 mLX3). The organic phase was washed with saturated brine (15 mLX3), dried over anhydrous sodium sulfate, filtered, and concentrated. The crude product was purified by silica gel column chromatography (ethyl acetate: petroleum ether= *0-* 5%) to obtain 1-(t-butyl) 2-methyl 3-(difluoromethylene)pyrrolidin-1,2-dicarboxylate (1.2 g, yield: 46%). ES-API: [M-55]⁺= 222.1.

Step II: Under a nitrogen atmosphere, 1-(t-butyl) 2-methyl 3-(difluoromethylene)pyrrolidin-1,2-dicarboxylate (1.2 g, 4.328 mmol) was dissolved in anhydrous tetrahydrofuran(15 mL), and the mixture was allowed to stand in a dry ice-acetone bath. Lithium diisopropylamide (8.6 mL, 8.656 mmol) was slowly added dropwise to the above solution, and stirred at this temperature for 1 hr. A solution of 1-chloro-3-iodopropane (2.654 g, 12.984 mmol) in anhydrous tetrahydrofuran (10 mL) was slowly added by a syringe to the solution, and the reaction solution was continuously stirred at -78°C for 1 hr, then slowly heated to room temperature, and stirred overnight at room temperature. The reaction was shown to be completed by LC/MS. The reaction was quenched with a saturated ammonium chloride solution (30 mL), and extracted with ethyl acetate (20 mLX3). The organic phase was washed with saturated brine (15 mLX3), dried over anhydrous sodium sulfate, filtered, and concentrated. The crude product was purified by silica gel column chromatography (ethyl acetate: petroleum ether= 0- 15%) to obtain 1-(t-butyl) 2-methyl 2-(3-chloropropyl)-3-(difluoromethylene)pyrrolidin-1,2-dicarboxylate (1128 mg, yield: 74%). ES-API: [M-55]⁺= 298.1.

Step III: At room temperature, 4 M hydrochloric acid-dioxane solution (10 mL) was slowly added to a solution of 1-(t-butyl)2-methyl2-(3-chloropropyl)-3-(difluoromethylene)pyrrolidin-1,2-dicarboxylate (581 mg, 1.642 mmol) in acetonitrile (2 mL). The reaction mixture was stirred at room temperature for 0.5 hr. The reaction was shown to be completed by LC/MS. The reaction solution was concentrated to dryness under reduced pressure to obtain a crude product that was methyl 2-(3-chloropropyl)-3-(difluoromethylene)pyrrolidin-2-carbxylate (416 mg). ES-API: [M+H]⁺= 254.6.

Step IV: At room temperature, sodium bicarbonate (689 mg, 8.20 mmol) and potassium iodide (27 mg, 0.164 mmol) were added to a solution of methyl 2-(3-chloropropyl)-3-(difluoromethylene)pyrrolidin-2-carboxylate (416 mg, 1.640 mmol) in acetonitrile (15 mL), and the reaction mixture was stirred overnight at room temperature. The reaction was shown to be completed by LC/MS. The reaction solution was filtered, and the insoluble matter was washed with acetonitrile. The filtrate was concentrated under reduced pressure, to obtain methyl 1-(difluoromethylene)tetrahydro-1H-pyrrolizin-7a(5H)-carboxylate (356 mg). ES-API: [M+H]⁺= 218.6.

Step V: Under a nitrogen atmosphere, methyl 1-(difluoromethylene)tetrahydro-1H-pyrrolizin-7a(5H)-carboxylate (356 mg, 1.639 mmol) was dissolved in anhydrous tetrahydrofuran (10 mL), and the mixture was allowed to stand in an iced water bath. Lithium aluminium hydride (3.3 mL, 3.278 mmol, 1 M in tetrahydrofuran) was slowly added dropwise to the above solution, and then stirred at this temperature for 0.5 hr. The reaction was shown to be completed by LC/MS. The reaction solution was added with sodium sulfafe decahydrate to quench to reaction, stirred at room temperature for 0.5 hr, and filtered to remove the insoluble matter. The filtrate was concentrated under reduced pressure and the crude product was purified by silica gel column chromatography (methanol: dichloromethane= 0- 40%) to obtain (1-(difluoromethylene)tetrahydro-1H-pyrrolizin-7a(5H)-yl)methanol (180 mg, yield: 58%). ES-API: [M+H]⁺= 190.1

Step VI: (1-(difluoromethylene)tetrahydro-1H-pyrrolizin-7a(5H)-yl)methanol (58 mg, 0.305 mmol) was dissolved in anhydrous tetrahydrofuran (5 mL). In an iced water bath, sodium hydride (12 mg, 0.305 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (80 mg, 0.102 mmol) in tetrahydrofuran (2 mL) was added dropwise to the reaction solution, and reacted at 0°C for 30 min. The reaction was shown to be completed by LC/MS. The reaction solution was diluted with ethyl acetate (10 mL), washed with a saturated ammonium chloride solution (10 mLX 2) and saturated brine (10 mLX3), dried over anhydrous sodium sulfate, filtered, and concentrated under reduced pressure, to obtain a crude product that was t-butyl (5S,5aS,6S,9R)-12-((1-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]hepten-14-carboxylate (92 mg). ES-API: [M+H]⁺= 913.4.

Step VII: Cesium fluoride (153 mg, 1.008 mmol) was added to t-butyl (5S,5aS,6S,9R)-12-((1-(difluoromethylene)tetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-4-oxa-3,10a,11,13,14-pentaaza-6,9-methanonaphtho[1,8-ab]hepten-14-carboxylate (92 mg, 0.101 mmol) in N,N-dimethylformamide (5 mL), and the reaction mixture was stirred at room temperature for 1 hr. The reaction was shown to be completed by LC/MS. The reaction solution was diluted with ethyl acetate (15 mL), washed with water (15 mLX 2) and saturated brine (15 mLX 3), dried over anhydrous sodium sulfate, filtered, and concentrated to obtain a crude product that was t-butyl (5S,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (76 mg). ES-API: [M+H]⁺= 757.2.

Step VIII: In an iced water bath, 4 M hydrochloric acid-dioxane solution (5 mL) was slowly added to a solution of t-butyl (58,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (76 mg, 0.100 mmol) in acetonitrile (2 mL). The reaction mixture was stirred at 0°C for 0.5 hr. The reaction was shown to be completed by LC/MS. The reaction solution was concentrated to dryness, and the residue was purified by preparative HPLC (trifluoroacetic acid method) (chromatographic condition: 15mL-10-30-13min) to obtain (5S,5aS,6S,9R)-12-((1-(difluoromethylene)tetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxy-3,10a,11,13,14-pentaaza-6,9-methanonaphtho[1,8-ab]heptene (Z612, 27.1 mg, yield: 41%, trifluoroacetate) as an off-white solid. ES-API: [M+H]⁺= 657.2. ¹H NMR (400 MHz, DMSO-d6) δ 8.24-8.16 (m, 3 H), 7.70-7.53 (m, 3 H), 5.16-5.13 (m, 1 H), 4.53-4.46 (m, 1 H), 4.31-4.21 (m, 2 H), 4.03-3.99 (m, 2 H), 3.63-3.62 (m, 1 H), 3.52-3.49 (m, 1 H), 3.12-3.05 (m, 2 H), 2.98-2.92 (m, 1 H), 2.78-2.55 (m, 3 H), 2.07-2.01 (m, 1 H), 1.93-1.57 (m, 8 H), 1.46-1.42 (m, 3 H).

### Example 51: Synthesis of (58,5aS,65,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z611)

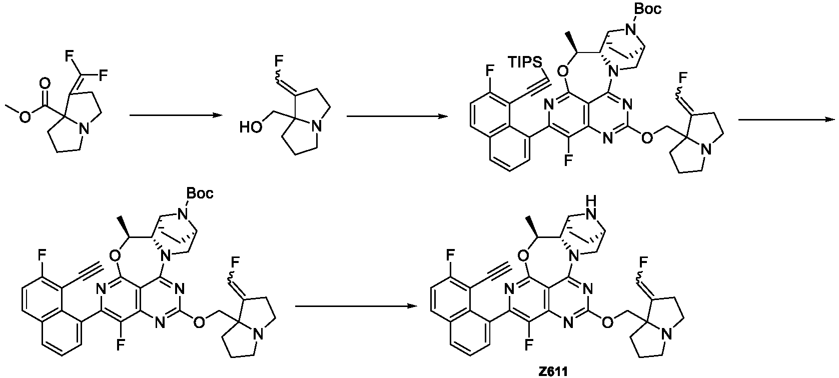

Step I: Under a nitrogen atmosphere, methyl 1-(difluoromethylene)tetrahydro-1H-pyrrolizin-7a(5H)-carboxylate (319.3mg, 1.47 mmol) and tetrahydrofuran(15 mL) were added to a round-bottom flask. The reaction solution was cooled to 0°C, and a lithium borohydride solution (10.0 mL, 10.0mmol, 1 M in tetrahydrofuran) was added dropwise to the solution. After that, the reaction was stirred at 0°C for 30 hrs. 4M hydrochloric acid (1 mL) was added dropwise and stirred at room temperature for 30 min. 2 M sodium hydroxide aqueous solution (5 mL) and water (20 mL) were added. The reaction solution was extracted with dichloromethane/methanol (10:1, 30 mLX3). The organic phase was dried over anhydrous sodium sulfate, filtered and concentrated to obtain (1-(fluoromethylene)tetrahydro-1H-pyrrolizin-7a(5H)-yl)methanol (130mg, yield: 46%) as a yellow oil. ES-API: [M+H]⁺=172.1.

Step II: (1-(fluoromethylene)tetrahydro-1H-pyrrolizin-7a(5H)-yl)methanol (50.0mg, 0.292 mmol) was dissolved in anhydrous tetrahydrofuran (5 mL). In an iced water bath, sodium hydride (48mg, 1.20 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (70.0mg, 0.089 mmol) in tetrahydrofuran (2 mL) was added dropwise to the reaction solution, and reacted at 40°C for 30 min. The reaction was shown to be completed by LC/MS. The reaction solution was diluted with ethyl acetate (50 mL), washed with saturated ammonium chloride solution (15 mLX2) and saturated brine (50 mLX3), dried over anhydrous sodium sulfate, and filtered. The crude product was purified by silica gel column chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-12-((1-(fluoromethylene) tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (130 mg, crude product). ES-API: [M+H]⁺= 895.3.

Step III: Cesium fluoride (96.0 mg, 0.63mmol) was added to t-butyl (5S,5aS,6S,9R)-1-fluoro-2-(7-fluoro-8-((triisopropylsilyl)ethynyl)naphthalen-1-yl)-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (130mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX2) and saturated brine (50 mLX3), dried over anhydrous sodium sulfate, filtered, and concentrated to obtain a crude product which was t-butyl (5S,5aS,6S,9R)-2-(8-ethynyl-7-fluoronaphthalen-1-yl)-1-fluoro-12-((1-(fluoromethylene) tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (100mg, crude product). ES-API: [M+H]⁺= 739.2.

Step IV: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (100mg, crude product) in acetonitrile (5.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-((1-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z611, 4.8mg, total yield of 3 steps: 2.4%) as a light yellow solid. ES-API: [M+H]⁺= 639.3.

### Example 52: Synthesis of (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]heptene (Z632)

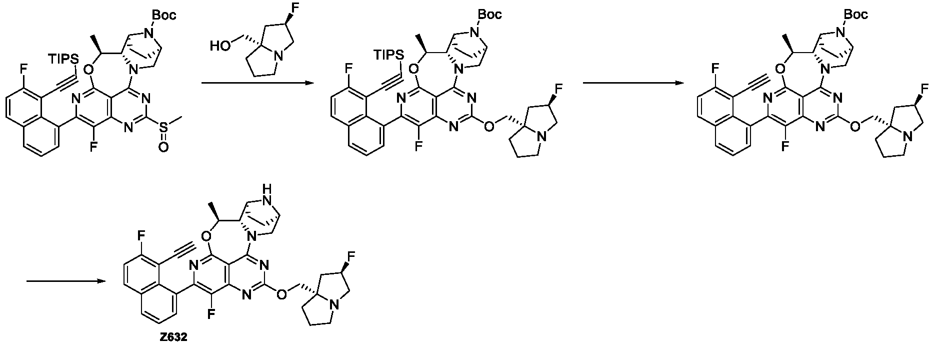

Step I: ((2R, 7aS)-2-fluorotetrahydro-1H-pyrrolizin-7a(5H)-yl)methanol (24mg, 0.15mmol) was dissolved in dry tetrahydrofuran (5 mL). At 0°C, sodium hydride (15mg, 0.38mmol) was added, and then stirred at 0°C for 15 min. Then, t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (60mg, 0.076mmol) was added, and reacted at 0°C for 1 hr. After the reaction, the reaction solution was added with water (10 mL), extracted with ethyl acetate (15mLX3), dried over anhydrous sodium sulfate, filtered, rotary evaporated to dryness and purified by silica gel column chromatography (50-100%ethyl acetate /petroleum ether) to obtain t-butyl (5S,5aS,6S,9R)-1-fluoro-2-(7-fluoro-8-((triisopropylsilyl)ethynyl)naphthalen-1-yl)-12-((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40mg, yield: 60.4%). ES-API:[M+H]⁺=882.3.

Step II: T-butyl (5S,5aS,6S,9R)-1-fluoro-2-(7-fluoro-8-((triisopropylsilyl)ethynyl)naphthalen-1-yl)-12-((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40mg, 0.045 mmol) was dissolved in N,N-dimethylformamide (2 mL), and then cesium fluoride (68mg, 0.45mmol) was added, and reacted at room temperature for 1 hr. The reaction solution was extracted with ethyl acetate (10 mLX3). The organic phase was dried over anhydrous sodium sulfate, filtered, rotary evaporated to dryness, and purified by preparative HPLC (trifluoroacetic acid method) to obtain t-butyl (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25mg, yield: 76.4%). ES-API:[M+H]⁺=727.2.

Step III: T-butyl (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (25mg, 0.034mmol) was dissolved in acetonitrile (5 mL), and 4M hydrochloric acid/dioxane solution (1mL) was added in an ice bath and reacted for 2 hrs in the ice bath. The reaction was adjusted to pH 8 with saturated sodium bicarbonate, extracted with ethyl acetate (15mLX3), and rotary evaporated to dryness. The crude product was purified by preparative HPLC (trifluoroacetic acid method) to obtain (5S,5aS,6S,9R)-2-(8-ethynyl-7-fluoronaphthalen-1-yl)-1-fluoro-12-(((2R,7aS)-2-fluorotetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z632, 10.5mg, yield: 47.9%, trifluoroacetate). ES-API:[M+H]⁺=627.3. ¹H NMR (400 MHz, CD₃OD) δ 8.38 (s, 1H), 8.21 -7.90 (m, 2H), 7.76 -7.26 (m, 3H), 5.63 - 5.35 (m, 2H), 4.71 -4.47 (m, 3H), 4.24 (s, 1H), 3.78 (dt, J = 29.2, 23.7 Hz, 6H), 3.33 (s, 2H), 2.29 (ddd, J = 136.4, 66.9, 25.3 Hz, 12H), 1.60 (t, J = 6.0 Hz, 3H).

### Example 53: Synthesis of (5S,5aS,6S,9R)-12-((1,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptadiene (Z610)

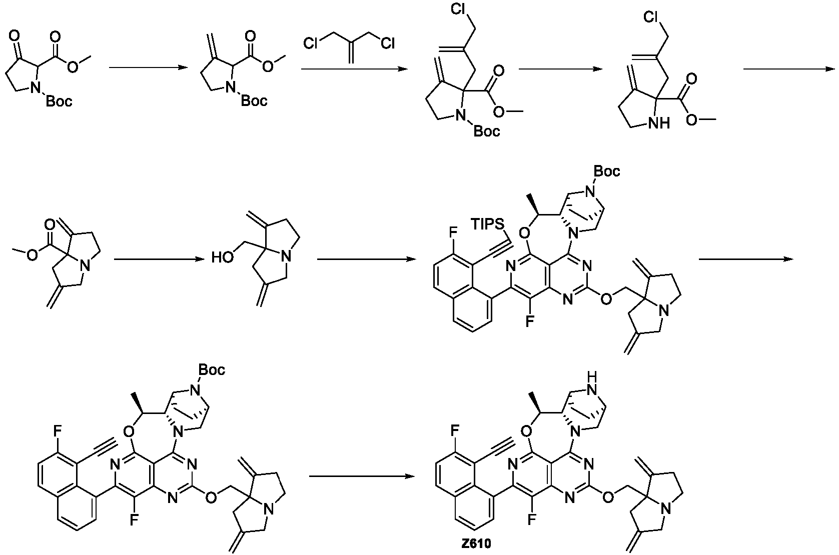

Step I: Methyltriphenylphosphonium bromide (18.35g, 51.38 mmol) was added to tetrahydrofuran (150 mL) and cooled to 0°C. Under a nitrogen atmosphere, a solution of potassium tert-butoxide in tetrahydrofuran (51.38mL, 51.38 mmol, 1M) was added to the mixture at 0°C, and then the reaction was warmed to room temperature and stirred for 1 hr. 1-(t-butyl) 2-methyl-3-oxopyrrolidin-1,2-dicarboxylate (5.0g, 20.55 mmol) was dissolved in tetrahydrofuran (20 mL), and slowly added to the reaction solution under a nitrogen atmosphere. After that, the reaction solution was stirred at room temperature for 3 hrs. The reaction solution was added with saturated ammonium chloride (30 mL), and extracted with ethyl acetate (100 mLX3). The organic phase was washed with saturated brine (200mL), dried over anhydrous sodium sulfate, filtered and concentrated. The crude product was purified by flash silica gel column chromatography (ethyl acetate /petroleum ether: 0%-8%) to obtain 1-(t-butyl) 2-ethyl 3-methylenepyrrolidin-1,2-dicarboxylate (1.2g, yield: 24.2%) as a colorless transparent liquid. ES-API: [M-Boc+H]⁺=186.1.

Step II: At -78°C, lithium bis(trimethylsilyl)amide (9.2mL, 9.2 mmol) was added dropwise to a solution of 1-(t-butyl)2-methyl3-methylenepyrrolidin-1,2-dicarboxylate (1.1g, 4.56 mmol) in dry tetrahydrofuran (15.0mL), and stirred at this temperature for 1 hr. After 1 hr, 3-chloro-2-chloromethylpropene (2.85 g, 22.794 mmol) was slowly added dropwise to the above solution, slowly heated to room temperature and reacted for 2 hrs. After the reaction, the reaction solution was added with water (100 mL), and extracted with ethyl acetate (100 mL X 4). The organic phase was washed with saturated brine (200 mLX3), dried over anhydrous sodium sulfate, filtered, and concentrated. The crude product was purified by flash silica gel column chromatography (ethyl acetate /petroleum ether: 0-10%) to obtain 1-(t-butyl) 2-methyl 2-(2-(chloromethyl)allyl)-3-methylenepyrrolidin-1,2-dicarboxylate (899 mg, yield: 57.8%) as a colorless transparent oil. ES-API: [M-Boc+H]⁺=274.1.

Step III: At 0°C, a hydrochloric acid-dioxane solution (10 mL, 10.000 mmol) was added to a solution of 1-(t-butyl) 2-methyl 2-(2-(chloromethyl)allyl)-3-methylenepyrrolidin-1,2-dicarboxylate (900mg, 2.729 mmol) in acetonitrile (5.0mL), and stirred at this temperature for 1.5 hrs. After the reaction, the solvent was removed by rotary evaporation to dryness under reduced pressure, to obtain a crude product that was methyl 2-(2-(chloromethyl)allyl)-3-methylenepyrrolidin-2-carboxylate (626.80mg, crude product), which was directly used in the next step of reaction. ES-API: [M+H]⁺=274.1.

Step IV: At 0°C, potassium iodide (45.24mg, 0.273mmol) and sodium bicarbonate (1144.76mg,13.626mmol) were added to a solution of methyl 2-(2-(chloromethyl)allyl)-3-methylenepyrrolidin-2-carboxylate (626.80mg, 2.725mmol) in acetonitrile (5.0mL), and reacted overnight at room temperature. After the reaction, the reaction solution was added with water (100 mL), and extracted with ethyl acetate (100 mL X 4). The organic phase was washed with saturated brine (200 mLX3), dried over anhydrous sodium sulfate, filtered and concentrated. The crude product was purified by flash silica gel column chromatography (methanol /dichloromethane: 0-10%) to obtain methyl 1,6-dimethyltetrahydro-1H-pyrrolizin-7a(5H)-carboxylate (400 mg, 75.96%). ES-API: [M+H]⁺=194.2.

Step V: Methyl 1,6-dimethyltetrahydro-1H-pyrrolizin-7a(5H)-carboxylate (200mg, 1.036 mmol) was dissolved in tetrahydrofuran (10mL), and cooled to 0°C. Under a nitrogen atmosphere, a lithium aluminum hydride solution (2.0 mL, 2.0mmol, 1 M in tetrahydrofuran) was added to the solution. The reaction was stirred at 0°C for 1 hr. The reaction was quenched with water (0.03 mL), 15% sodium hydroxide aqueous solution (0.03 mL) and water (0.09 mL) were added, and then tetrahydrofuran (10 mL) was added, and filtered. The filtrate was concentrated to obtain (1,6-dimethylenetetrahydro-1H-pyrrolizin-7a(5H)-yl)methanol (130mg, yield: 77%) as a colorless transparent liquid. ES-API: [M +H]⁺=166.1.

Step VI: (1,6-dimethylenetetrahydro-1H-pyrrolizin-7a(5H)-yl)methanol (60mg, 0.3610 mmol) was dissolved in anhydrous tetrahydrofuran (5 mL). In an iced water bath, sodium hydride (58.0mg, 1.444mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (90mg, 0.113 mmol) in tetrahydrofuran (2 mL) was added dropwise to the reaction solution, and reacted at 40°C for 30 min. The reaction was shown to be completed by LC/MS. The reaction solution was diluted with ethyl acetate (50 mL), washed with a saturated ammonium chloride solution (15 mLX 2) and saturated brine (50 mLX3), dried over anhydrous sodium sulfate, filtered, and concentrated. The crude product was purified by silica gel column chromatography (dichloromethane: methanol =15:1) to obtain t-butyl (5S,5aS,6S,9R)-12-((1,6-dimethylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (130mg, crude product). ES-API: [M+H]⁺= 889.3.

Step VII: Cesium fluoride (96.0 mg, 0.63mmol) was added to t-butyl (5S,5aS,6S,9R)-12-((1,6-dimethylenetetrahydro-1H-pyrrolizin-7a(5H)-yl)methoxy)- 1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (130mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX2) and saturated brine (50 mLX3), dried over anhydrous sodium sulfate, filtered, and concentrated to obtain a crude product that was t-butyl (5S,5aS,6S,9R)-12-((1,6-dimethylenetetrahydro-1H-pyrrolizin-7a(5H)-yl) methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110mg, crude product). ES-API: [M+H]⁺= 733.2.

Step VIII: In an iced water bath, 4 M hydrochloric acid-dioxane solution (3 mL) was slowly added to a solution of t-butyl (5S,5aS,6S,9R)-12-((1,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110mg, crude product) in acetonitrile (5.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-12-((1,6-dimethylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptadiene (Z610, 15.0mg, total yield of 3 steps: 20.8%) as a light yellow solid. ES-API: [M+H]⁺= 633.3.

### Example 54: Synthesis of (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z633)

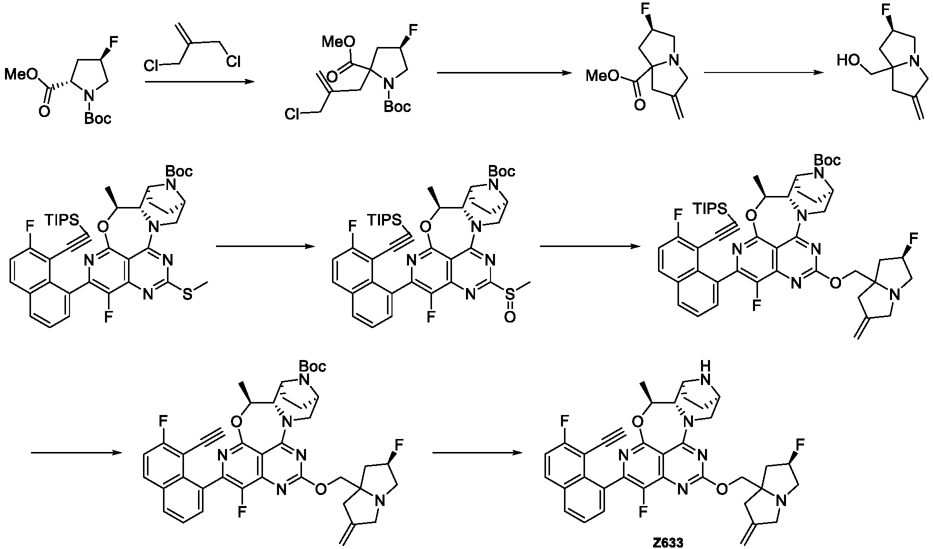

Step I: Under a nitrogen atmosphere, 1-(t-butyl) 2-methyl (2S,4R)-4-fluoropyrrolidin-1,2-dicarboxylate (1.24 g, 5.0 mmol) was dissolved in anhydrous tetrahydrofuran (20.0 mL), and the mixture was allowed to stand in a dry ice-ethanol bath. Lithium bis(trimethylsilyl)amide (10.0 mL, 10.0 mmol, 1 M in tetrahydrofuran) was slowly added dropwise to the above solution, and stirred at this temperature for 15 min. Then, 3-chloro-2-chloromethylpropene (1.39 mL, 15.0 mmol) was slowly added to the solution. After stirring at -78°C for 1 hr, the reaction was quenched with a saturated ammonium chloride solution, and extracted 3 times with ethyl acetate. The organic phase was washed with saturated brine, dried over anhydrous sodium sulfate, filtered and concentrated. The crude product was purified by flash silica gel column chromatography (ethyl acetate /petroleum ether: 0-20%) to obtain 1-(t-butyl) 2-methyl (4R)-2-(2-(chloromethyl)allyl)-4-fluoropyrrolidin-1,2-dicarboxylate (1.43 g, yield: 85%). ES-API: [M-55]⁺= 280.0.

Step II: At 0°C, 4 M hydrochloric acid-dioxane solution (2.5 mL) was slowly added to a solution of 1-(t-butyl) 2-methyl (4R)-2-(2-(chloromethyl)allyl)-4-fluoropyrrolidin-1,2-dicarboxylate (336 mg,1.0 mmol) in acetonitrile (10.0 mL). The reaction mixture was heated to room temperature and stirred for 1 hr. Then sodium bicarbonate (5 g) was added, and stirred at room temperature for 1 hr. After filtration under suction, the filtrate was collected, and concentrated under reduced pressure. The crude product was purified by flash silica gel column chromatography (methanol /dichloromethane: 0-8%) to obtain methyl (2R)-2-fluoro-6-methylenetetrahydro-1H-pyrrolizin-7a(5H)-carboxylate (200 mg, yield: 100%). ES-API: [M+H]⁺= 200.1.

Step III: Under a nitrogen atmosphere, methyl (2R)-2-fluoro-6-methylenetetrahydro-1H-pyrrolizin-7a(5H)-carboxylate (99 mg, 0.50 mmol) was dissolved in anhydrous tetrahydrofuran (5.0 mL), and the mixture was allowed to stand in an iced water bath. Lithium aluminium hydride (0.60 mL, 0.60 mmol, 1 M in tetrahydrofuran) was slowly added dropwise to the solution, and stirred at this temperature for 0.5 hr. Sodium sulfafe decahydrate was slowly added to quench the reaction, heated to room temperature, and stirred for 0.5 hr. After filtration under suction, the filtrate was concentrated under reduced pressure, to obtain ((2R)-2-fluoro-6-methylenetetrahydro-1H-pyrrolizin-7a(5H)-yl)methanol (74 mg, yield: 86%). ES-API: [M+H]⁺= 172.0.

Step IV: At 0°C, m-chloroperoxybenzoic acid (17 mg, 0.08 mmol, 85% purity) was added to a solution of t-butyl (5S,5aS,6S,9R)-1-fluoro-2-(7-fluoro-8-((triisopropylsilyl)ethynyl) naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]heptan-14-carboxylate (50 mg, 0.065 mmol) in dichloromethane (5.0 mL), and stirred at 0°C for 0.5 hr. A saturated sodium bicarbonate aqueous solution and a saturated sodium sulfite aqueous solution were added, and stirred vigorously for 15 min. The organic phase was separated, and the aqueous phase was extracted 2 times with dichloromethane. The organic phase was collected, washed with saturated brine, dried over anhydrous sodium sulfate, filtered, and rotary evaporated to dryness, to obtain t-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate (crude product), which was directly used in the next step of reaction without purification. ES-API: [M+H]⁺= 788.3.

Step V: At 0°C, sodium hydride (60% dispersed in an oil) (10 mg, 0.26 mmol) was added to ((2R)-2-fluoro-6-methylenetetrahydro-1H-pyrrolizin-7a(5H)-yl)methanol as a crude product in tetrahydrofuran (5.0 mL) obtained in Step III. After reaction for 15 min, a solution of t-butyl (5S,5aS,6S,9R)-1-fluoro-2-(7-fluoro-8-((triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]heptan-14-carboxylate as a crude product obtained in Step IV in tetrahydrofuran (1.0 mL) was added dropwise to the reaction solution. After reaction at 0°C for 0.5 hr, the reaction solution was slowly added with a saturated ammonium chloride aqueous solution, and extracted 3 times with dichloromethane. The organic phase was collected and washed with saturated brine, dried over anhydrous sodium sulfate, filtered, and rotary evaporated to dryness. The crude product was purified by preparative TIC (ethyl acetate /petroleum ether 80%) to obtain t-butyl (5S,5aS,6S,9R)-1-fluoro-12-(((2R)-2-fluoro-6-methylenetetrahydro-1H-pyrrolizin-7a(5H) -yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptan-14-carboxylate (18 mg, yield:31%). ES-API: [M+H]⁺= 895.3.

Step VI: T-butyl (5S,5aS,6S,9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptan-14-carboxylate (30 mg, 0.034 mmol) was dissolved in N, N-dimethylformamide (2.0 mL), and cesium fluoride (31 mg, 0.20 mmol) was added, and reacted at room temperature for 0.5 hr. After the reaction, the reaction solution was added with water, and extracted 3 times with ethyl acetate. The organic phases were combined, washed with saturated brine, dried over anhydrous sodium sulfate, filtered, and rotary evaporated to dryness, to obtain t-butyl (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (crude product), which was directly used in the next step of reaction without purification. ES-API:[M+H]⁺= 739.0.

Step VII: T-butyl (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (crude product) was dissolved in acetonitrile (5.0 mL). In an ice bath, 4M hydrochloric acid/dioxane solution (3.0 mL, 13.0 mmol) was added, heated to room temperature and reacted for 3 hrs. After the reaction, the reaction solution was concentrated to dryness, and the crude product was purified by preparative HPLC (ammonium bicarbonate method 1) to obtain (5S,5aS,6S,9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z633, 3.69 mg, total yield: 29%). ES-API: [M+H]⁺= 639.2.

### Example 55: Synthesis of (5S,5aS,6S,9R)-2-(8-ethynyl-7-fluoronaphthalen-1-yl)-1-fluoro-12-((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z634)

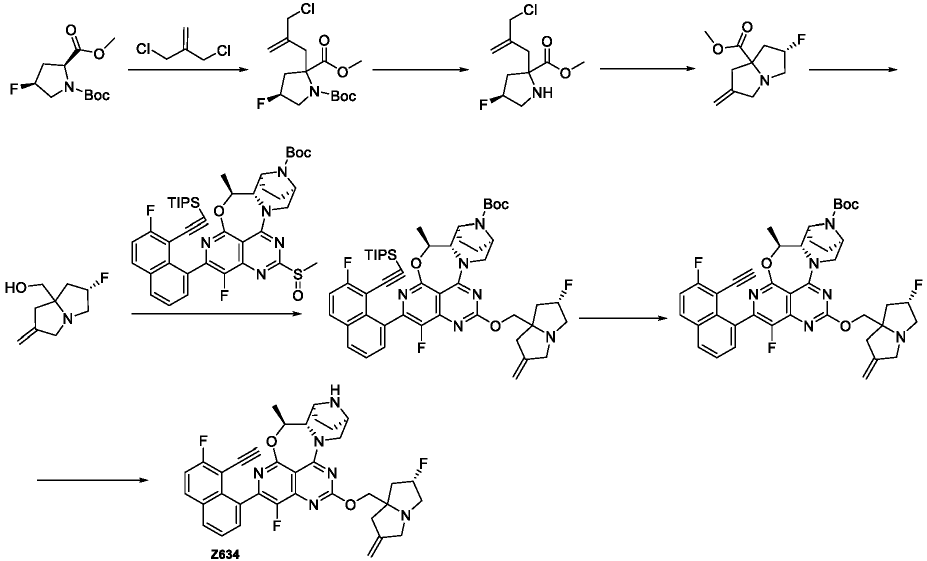

Step I: Under a nitrogen atmosphere, N-t-butoxycarbonyl-cis -4-fluoro-L-proline methyl ester (1 g, 4.044 mmol) was dissolved in anhydrous tetrahydrofuran (20 mL), and the mixture was allowed to stand in a dry ice-acetone bath. Lithium bis(trimethylsilyl)amide (8.0 mL, 8.088 mmol) was slowly added dropwise to the above solution, and stirred at this temperature for 1 hr. Then, a solution of 3-chloro-2-chloromethylpropene (1.516 g, 12.132 mmol) in anhydrous tetrahydrofuran (10 mL) was slowly added by a syringe to the solution, and the reaction solution was continuously stirred at -78°C for 1 hr, and then slowly heated to room temperature and stirred for 2 hrs. The reaction was shown to be completed by LC/MS. The reaction was quenched with a saturated ammonium chloride solution (30 mL), and extracted with ethyl acetate (20 mLX 3). The organic phase was washed with saturated brine (15 mLX3), dried over anhydrous sodium sulfate, filtered, and concentrated. The crude product was purified by silica gel column chromatography(ethyl acetate: petroleum ether= 0- 15%) to obtain 1-(t-butyl) 2-methyl (4S)-2-(2-(chloromethyl)allyl)-4-fluoropyrrolidin-1,2-dicarboxylate (1190 mg, yield: 88%). ES-API: [M-55]⁺= 280.1.

Step II: At room temperature, a 4 M hydrochloric acid-dioxane solution (15 mL) was slowly added to a solution of 1-(t-butyl) 2-methyl (4S)-2-(2-(chloromethyl)allyl)-4-fluoropyrrolidin-1,2-dicarboxylate (1190 mg, 3.544 mmol) in acetonitrile (3 mL). The reaction mixture was stirred at room temperature for 3 hrs. The reaction was shown to be completed by LC/MS. The reaction solution was concentrated to dryness under reduced pressure to obtain a crude product that was methyl (4S)-2-(2-(chloromethyl)allyl)-4-fluoropyrrolidin-2-carboxylate (835 mg). ES-API: [M+H]⁺= 236.1.

Step III: At room temperature, sodium bicarbonate (1.488 g, 17.715 mmol) and potassium iodide (59 mg, 0.354 mmol) were added to a solution of methyl (4S)-2-(2-(chloromethyl)allyl)-4-fluoropyrrolidin-2-carboxylate (835 mg, 3.543 mmol) in acetonitrile (25 mL), and the reaction mixture was stirred at room temperature for 2 hrs. The reaction was shown to be completed by LC-MS. The reaction solution was filtered, and the insoluble matter was washed with acetonitrile. The filtrate was concentrated under reduced pressure. The residue was purified by silica gel column chromatography (ethyl acetate: petroleum ether= 0- 45%) to obtain methyl (2S)-2-fluoro-6-methylenetetrahydro-1H-pyrrolizin-7a(5H)-carboxylate (611 mg, yield: 87%). ES-API: [M+H]⁺= 200.2.

Step IV: Under a nitrogen atmosphere, methyl (2S)-2-fluoro-6-methylenetetrahydro-1H-pyrrolizin-7a(5H)-carboxylate (200 mg, 1.004 mmol) was dissolved in anhydrous tetrahydrofuran(5 mL), and the mixture was allowed to stand in an iced water bath. Lithium aluminium hydride (2 mL, 2.008 mmol, 1 M in tetrahydrofuran) was slowly added dropwise to the above solution, and then stirred at this temperature for 0.5 hr. The reaction was shown to be completed by LC/MS. The reaction solution was added with sodium sulfafe decahydrate, stirred at room temperature for 0.5 hr, and filtered to remove the insoluble matter. The filtrate was concentrated under reduced pressure, to obtain ((2S)-2-fluoro-6-methylenetetrahydro-1H-pyrrolizin-7a(5H)-yl)methanol (171 mg) as a crude product. ES-API: [M+H]⁺= 172.1.

Step V: ((2S)-2-fluoro-6-methylenetetrahydro-1H-pyrrolizin-7a(5H)-yl)methanol (52 mg, 0.305 mmol) was dissolved in anhydrous tetrahydrofuran (5 mL). In an iced water bath, sodium hydride (12 mg, 0.305 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (81 mg, 0.103 mmol) in tetrahydrofuran (2 mL) was added dropwise to the reaction solution, and reacted at 0°C for 30 min. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure, to obtain a crude product that was t-butyl (5S,5aS,6S,9R)-1-fluoro-12-((2S)-2-fluoro-6-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (90 mg). ES-API: [M+H]⁺= 895.3.

Step VI: Cesium fluoride (153 mg, 1.010 mmol) was added to t-butyl (5S,5aS,6S,9R)-1-fluoro-12-((2S)-2-fluoro-6-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.101 mmol) in N,N-dimethylformamide (5 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-12-((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptan-14-carboxylate (74 mg). ES-API: [M+H]⁺= 739.3.

Step VII: In an iced water bath, 4 M hydrochloric acid-dioxane solution (5 mL) was slowly added to a solution of t-butyl (5S,5aS,6S,9R)-2-(8-ethynyl-7-fluoronaphthalen-1-yl)-1-fluoro-12-((2S)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptan-14-carboxylate (74 mg, 0.100 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-50-60-13min) to obtain (5S,5aS,6S,9R)-2-(8-ethynyl-7-fluoronaphthalen-1-yl)-1-fluoro-12-((2S)-2-fluoro-6-methylenetetrahydro-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]heptene (Z634, 11.36 mg, yield: 18%) as an off-white solid. ES-API: [M+H]⁺= 639.3. ¹H NMR (400 MHz, DMSO-d6) δ 8.24-8.16 (m, 2 H), 7.70-7.52 (m, 3 H), 5.45-5.29 (m, 1 H), 5.15-5.12 (m, 1 H), 4.94-4.93 (m, 2 H), 4.53-4.45 (m, 1 H), 4.10-3.97 (m, 3 H), 3.61-3.58 (m, 2 H), 3.50-3.47 (m, 1 H), 3.27-3.18 (m, 2 H), 3.09-3.03 (m, 1 H), 2.93-2.80 (m, 1 H), 2.67-2.63 (m, 1 H), 2.45-2.00 (m, 5 H), 1.85-1.55 (m, 4 H), 1.46-1.40 (m, 3 H).

### Example 56: Synthesis of (5S,5aS,6S,9R)-2-(8-ethynyl-7-fluoronaphthalen-1-yl)-1-fluoro-5-methyl-12-((5-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptene (Z635)

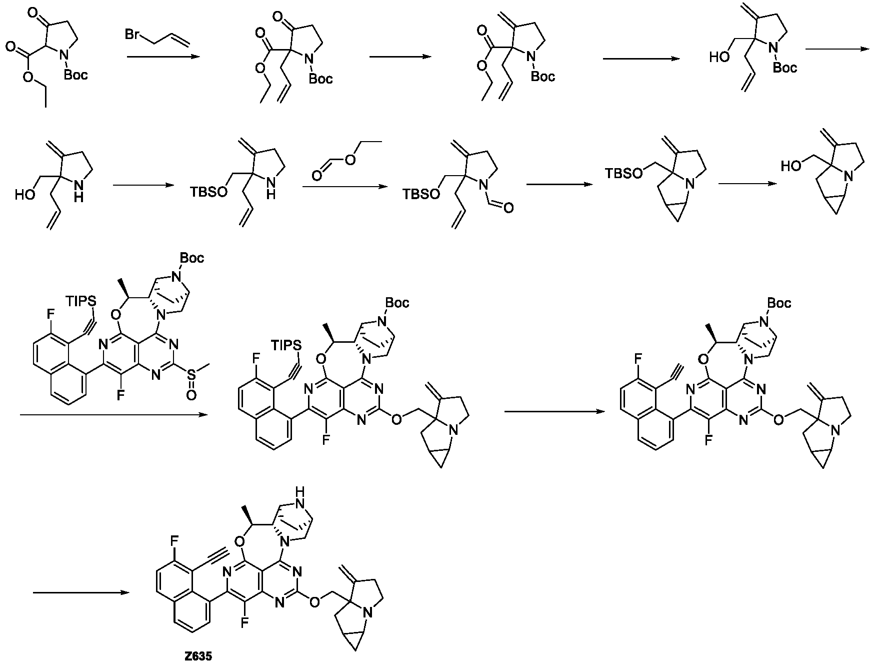

Step I: A mixture of ethyl *N*-Boc-3-oxopyrrolidin-2-carboxylate (2000 mg, 7.773 mmol), 3-bromoprop-1-ene (1410.63 mg, 11.660 mmol), potassium carbonate (2148.55 mg, 15.547 mmol), potassium iodide (2580.75 mg, 15.547 mmol), and *N, N*-dimethylformamide (50 mL) was stirred overnight at room temperature under a nitrogen atmosphere. The reaction solution was added with water (100 mL) and extracted with ethyl acetate (100 X2). The organic phase was washed with saturated brine (200 mLX3), dried over anhydrous sodium sulfate, filtered, and concentrated. The crude product was purified by flash silica gel column chromatography (ethyl acetate /petroleum ether: 0-15%) to obtain 1-(t-butyl) 2-ethyl2-allyl-3-oxopyrrolidin-1,2-dicarboxylate (1700 mg, yield: 73.55%) as a colorless transparent liquid. ES-API: [M-C₄H₈+H]⁺ =242.2

Step II: Methyltriphenylphosphonium bromide (10.21 g, 28.586 mmol) was added to tetrahydrofuran (100 mL), and cooled to 0°C. Under a nitrogen atmosphere, a solution of potassium tert-butoxide in tetrahydrofuran (1 M, 22.869 mL, 22.869 mmol) was added to the mixture at 0°C, and then the reaction was heated to room temperature and stirred for 0.5 hr. 1-(t-butyl) 2-ethyl2-allyl-3-oxopyrrolidin-1,2-dicarboxylate (1700 mg, 5.717 mmol) was dissolved in tetrahydrofuran(5 mL), and slowly added to the reaction solution under a nitrogen atmosphere. After that, the reaction was continuously stirred at 50°C for 5 hrs. The reaction solution was added with saturated ammonium chloride (30 mL) and extracted with ethyl acetate (60 mLX3). The organic phase was washed with saturated brine (200 mL), dried over anhydrous sodium sulfate, filtered and concentrated. The crude product was purified by flash silica gel column chromatography (ethyl acetate /petroleum ether: 0%-8%) to obtain 1-(t-butyl) 2-ethyl 2-allyl-3-methylenepyrrolidin-1,2-dicarboxylate (1200 mg, yield: 71.06%) as a colorless transparent liquid. ES-API: [M-Boc+H]⁺=196.0.

Step III: 1-(t-butyl) 2-ethyl2-allyl-3-methylenepyrrolidin-1,2-dicarboxylate (1000 mg, 3.385 mmol) was dissolved in tetrahydrofuran (10 mL) and ethanol (2 mL), and cooled to 0°C. Under a nitrogen atmosphere, a solution of lithium borohydride in tetrahydrofuran (2*M*, 3.385 mL, 6.771 mmol) was added to the solution. After that, the reaction was stirred at 40°C for 1 hr. The reaction solution was added with a dilute hydrochloric acidaqueous solution (2 *M*) to quench the reaction, and then adjusted to pH 8 with a 15% sodium hydroxide aqueous solution. The obtained mixture was extracted with dichloromethane/methanol (10:1, *v*/*v,* 30 mL X3). The organic phase was washed with saturated brine (30 mL), dried over anhydrous sodium sulfate, filtered, and concentrated. The crude product was purified by flash silica gel column chromatography (methanol /dichloromethane: 0%-8%) to obtain t-butyl 2-allyl-2-(hydroxymethyl)-3-methylenepyrrolidin-1-carboxylate (650 mg, yield: 75.79%) as a colorless transparent liquid. ES-API: [M-C₄H₈+H]⁺=198.1.

Step IV: T-butyl 2-allyl-2-(hydroxymethyl)-3-methylenepyrrolidin-1-carboxylate (650 mg, 2.566 mmol) was dissolved in hydrogen chloride in dioxane (4 *M,* 10 mL), and stirred at room temperature for 3 hrs. The reaction solution was concentrated to obtain (2-allyl-3-methylenepyrrolidin-2-yl)methanol hydrochloride (486.67 mg, yield: 100%), ES-API: [M+H]⁺=154.1.

Step V: (2-allyl-3-methylenepyrrolidin-2-yl)methanol hydrochloride (390 mg, 2.056 mmol) was dissolved in dichloromethane, and cooled to 0°C. Under a nitrogen atmosphere, triethyl amine (0.857 mL, 6.168 mmol) and t-butyldimethylchlorosilane (402.86 mg, 2.673 mmol) were added to the solution at 0°C. After that, the reaction solution was heated to room temperature and stirred for 17 hrs. After the reaction, the reaction solution was concentrated to obtain a crude product. The crude product was purified by flash silica gel column chromatography (methanol /dichloromethane: 0%-2%) to obtain 2-allyl-2-(((t-butyldimethylsilyl)oxy)methyl)-3-methylenepyrrolidine (500 mg, 90.91 %) as a colorless transparent liquid. ES-API: [M+H]⁺=268.1.

Step VI: In a sealed tube reactor, 2-allyl-2-(((t-butyldimethylsilyl)oxy)methyl)-3-methylenepyrrolidine (400 mg, 1.495 mmol) and ethyl formate (5 mL) were added. The reaction was stirred at 100°C for 17 hrs after the reactor is sealed. After the reaction, the reaction solution was concentrated to obtain 2-allyl-2-(((t-butyldimethylsilyl)oxy)methyl)-3-methylenepyrrolidin-1-carboxaldehyde (400 mg, 90.52%) as a colorless transparent liquid. ES-API: [M+H]⁺=296.1.

Step VII: 2-allyl-2-(((t-butyldimethylsilyl)oxy)methyl)-3-methylenepyrrolidin-1-carboxaldehyde (400 mg, 1.36 mmol) was dissolved in anhydrous tetrahydrofuran, and cooled to 0°C. Under a nitrogen atmosphere, titanium isopropoxide (425 mg, 1.5 mmol) and cyclohexylmagnesium bromide (4.76 mL, 4.76 mmol 1.0 M solution of THF) were added to the solution at 0°C. After that, the reaction solution was heated to room temperature and stirred for 17 hrs. After the reaction, the reaction solution was diluted with ethyl acetate (50 mL), washed with saturated ammonium chloride solution (15 mLX 2) and saturated brine (15 mLX3), dried over anhydrous sodium sulfate, and filtered. The filtrate was concentrated under reduced pressure, and the crude product was purified by flash silica gel column chromatography (methanol /dichloromethane: 0%-5%) to obtain 5a-(((t-butyldimethylsilyl)oxy)methyl)-5-methyleneoctahydrocyclopropan[b]pyrrolizine (260 mg, yield: 61%) as a colorless transparent liquid. ES-API: [M+H]⁺=280.3.

Step VIII: At room temperature, 4M hydrochloric acid/dioxane solution (10 mL) was slowly added to a solution of 5a-(((t-butyldimethylsilyl)oxy)methyl)-5-methyleneoctahydrocyclopropan [b]pyrrolizine (260 mg, 0.93 mmol) in acetonitrile (2 mL), and stirred at room temperature for 0.5 hr. The reaction was shown to be completed by LC/MS. The reaction solution was concentrated under reduced pressure, to obtain (5-methylenehexahydrocyclopropane[b]pyrrolizin-5a(3H)-yl)methanol (153 mg, yield: 100%) as a crude product. ES-API: [M+H]⁺=166.2.

Step IX: (5-methylenehexahydrocyclopropane[b]pyrrolizin-5a(3H)-yl)methanol (44 mg, 0.26 mmol) was dissolved in anhydrous tetrahydrofuran (2 mL). In an iced water bath, sodium hydride (14 mg, 0.36 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (102 mg, 0.13 mmol) in tetrahydrofuran(2 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, and filtered. The filtrate was concentrated under reduced pressure, to obtain a crude product that was t-butyl (5S,5aS,6S,9R)-1-fluoro-2-(7-fluoro-8-((triisopropylsilyl)ethynyl)naphthalen-1-yl)-5-methyl-12-((5-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (115 mg, yield: 100%). ES-API: [M+H]⁺= 889.3.

Step X: Cesium fluoride (196 mg, 1.3 mmol) was added to t-butyl (5S,5aS,6S,9R)-1-fluoro-2-(7-fluoro-8-((triisopropylsilyl)ethynyl)naphthalen-1-yl)-5-methyl-12-((5-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (115 mg, 0.13 mmol) in N,N-dimethylformamide (2.0 mL), and the reaction mixture was stirred at room temperature for 2 hrs. The reaction was shown to be completed by LC/MS. The reaction solution was diluted with ethyl acetate (20 mL), washed with water (15 mLX 2) and saturated brine (15 mLX 3), dried over anhydrous sodium sulfate, filtered, and concentrated, to obtain the crude product t-butyl (5S,5aS,6S,9R)-2-(8-ethynyl-7-fluoronaphthalen-1-yl)-1-fluoro-5-methyl-12-((5-methylenehexahydrocyclopropane[b]pyrrolizin-5a(3H)-yl)methoxy)-5a,6,7,8,9,10-hexahydro-5H-4-oxy-3,10a,11,13,14-pentaaza-6,9-methanonaphtho[1,8-ab]hepten-14-carboxylate (94 mg, yield: 100%). ES-API: [M+H]⁺= 733.2.

Step XI: In an iced water bath, 4 M hydrochloric acid-dioxane solution (5 mL) was slowly added to a solution of t-butyl (5S,5aS,6S,9R)-2-(8-ethynyl-7-fluoronaphthalen-1-yl)-1-fluoro-5-methyl-12-((5-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]hepten-14-carboxylate (94 mg, 0.13 mmol) in acetonitrile (2 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-((5-methylenehexahydrocyclopropane[b]pyrrolizin-5a(3H)-yl)methoxy)-5a,6,7,8,9,10-hexahydro-5H-4-oxa-3,10a,11,13,14-pentaaza-6,9-methanonaphtho[1,8-ab]heptene (Z635, 8 mg, yield: 9%) as a light yellow solid. ES-API: [M+H]⁺= 633.3

### Example 57: Synthesis of (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z636)

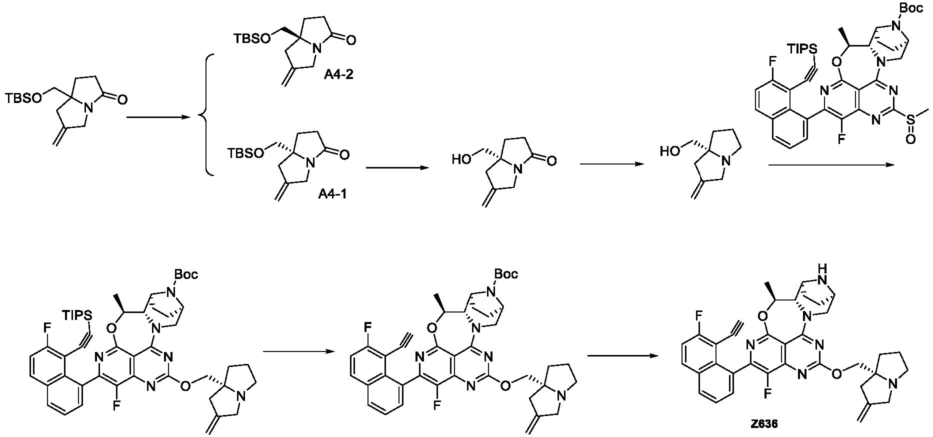

Step I: 7a-(((t-butyldimethylsilyl)oxy)methyl)-6-methylenehexahydro-3H-pyrrolizin-3-one (3.1 g) was resolved by chiral preparative chromatography (separation column IC, 250mm*4.6mm*5um, mobile phase: n-hexane:ethanol=90:10: 0.2, flow rate:1mL/min, column temperature: 30°C) to obtain two isomeric compounds. One structure was arbitrarily designated as (R)-7a-(((t-butyldimethylsilyl)oxy)methyl)-6-methylenehexahydro-3H-pyrrolizin-3-one (A4-1, 1.2 g, peak 1, retention time 7.385min, 100%) as a colorless liquid. ES-API: [M+H]+= 282.2. The other structure was arbitrarily designated as (S)-7a-(((t-butyldimethylsilyl)oxy)methyl)-6-methylenehexahydro-3H-pyrrolizin-3-one (A4-2, 1.3 g, peak 2, retention time 9.205min, 100%), as a colorless liquid. ES-API: [M+H]⁺= 282.2.

Step II: At room temperature, 4M hydrochloric acid/dioxane solution (10 mL) was slowly added to a solution of (R)-7a-((((t-butyldimethylsilyl)oxy)methyl)-6-methylenehexahydro-3H-pyrrolizin-3-one (A4-1, retention time 7.385min, 1.2 g, 4.3 mmol) in acetonitrile (2 mL), and stirred at room temperature for 0.5 hr. The reaction was shown to be completed by LC/MS. The reaction solution was concentrated under reduced pressure, to obtain a crude product that was (R)-7a-(hydroxymethyl)-6-methylenehexahydro-3H-pyrrolizin-3-one (712 mg, yield: 100%). ES-API: [M+H]⁺=168.1.

Step III: Under a nitrogen atmosphere, (R)-7a-(hydroxymethyl)-6-methylenehexahydro-3H-pyrrolizin-3-one (712 mg, 4.3 mmol) was dissolved in anhydrous tetrahydrofuran (5mL), and the mixture was allowed to stand in an iced water bath. A lithium aluminium hydride solution (4.3 mL, 4.3 mmol, 1 M in tetrahydrofuran) was slowly added dropwise to the above solution, and stirred at 60°C for 3 hrs. The reaction was shown to be completed by LC/MS. The reaction was quenched with sodium sulfafe decahydrate (430 mg), stirred at room temperature for 0.5 hr, and filtered. The filtrate was concentrated under reduced pressure, to obtain a crude product that was (R)-(2-methylenetetrahydro-1H-pyrrolizin-7a(5H)-yl)methanol (650 mg, yield:99%). ES-API: [M+H]⁺= 154.1.

Step IV: (R)-(2-methylenetetrahydro-1H-pyrrolizin-7a(5H)-yl)methanol (27 mg, 0.18 mmol) was dissolved in anhydrous tetrahydrofuran (2 mL). In an iced water bath, sodium hydride (14 mg, 0.36 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (70 mg, 0.09 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX 2) and saturated brine (15 mLX 3), dried over anhydrous sodium sulfate, and filtered. The filtrate was concentrated under reduced pressure, to obtain the crude product t-butyl (5S,5aS,6S,9R)-1-fluoro-2-(7-fluoro-8-((triisopropylsilyl)ethynyl)naphthalen-1-yl)-5-methyl-12-((R)-2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-heptanoate (77 mg, yield: 100%). ES-API: [M+H]⁺= 877.3.

Step V: Cesium fluoride (135 mg, 0.9 mmol) was added to t-butyl (5S,5aS,6S,9R)-1-fluoro-2-(7-fluoro-8-((triisopropylsilyl)ethynyl)naphthalen-1-yl)-5-methyl-12-((R)-2-methylenetetrahydro -1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-heptanoate (77 mg, 0.09 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated, to obtain the crude product t-butyl (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-carboxylate (64 mg, yield: 100%). ES-API: [M+H]⁺= 721.3.

Step VI: In an iced water bath, 4 M hydrochloric acid-dioxane solution (5 mL) was slowly added to a solution of t-butyl (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (64 mg, 0.09 mmol) in acetonitrile (1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-(((R)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z636, 10 mg, yield: 18%) as a light yellow solid. ES-API: [M+H]⁺= 621.3.

### Example 58: Synthesis of (5S,5aS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z637)

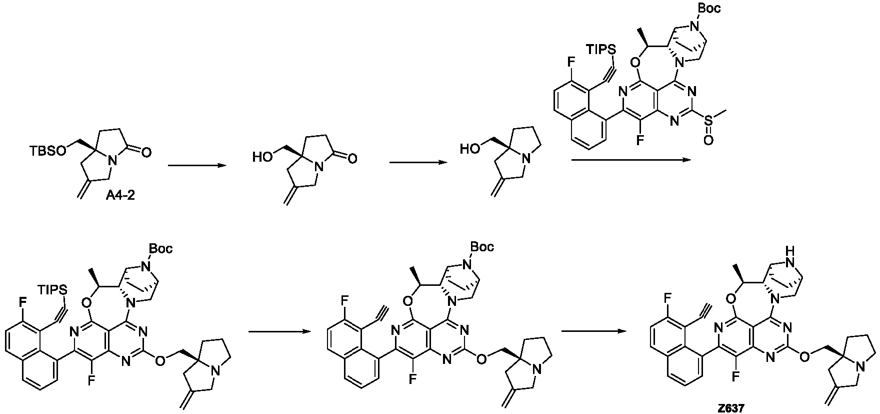

Step I: At room temperature, 4M hydrochloric acid/dioxane solution (10 mL) was slowly added to a solution of (S)-7a-((((t-butyldimethylsilyl)oxy)methyl)-6-methylenehexahydro-3H-pyrrolizin-3-one (A4-2, retention time 9.205min, 1.3 g, 4.6 mmol) in acetonitrile (2 mL), and stirred at room temperature for 0.5 hr. The reaction was shown to be completed by LC/MS. The reaction solution was concentrated under reduced pressure, to obtain a crude product that was (S)-7a-(hydroxymethyl)-6-methylenehexahydro-3H-pyrrolizin-3-one (772 mg, yield: 100%). ES-API: [M+H]⁺=168.1.

Step II: Under a nitrogen atmosphere, (S)-7a-(hydroxymethyl)-6-methylenehexahydro-3H-pyrrolizin-3-one (772 mg, 4.6 mmol) was dissolved in anhydrous tetrahydrofuran (5mL), and the mixture was allowed to stand in an iced water bath. A lithium aluminium hydride solution (4.6 mL, 4.6 mmol, 1 M in tetrahydrofuran) was slowly added dropwise to the above solution, and stirred at 60°C for 3 hrs. The reaction was shown to be completed by LC/MS. The reaction was quenched with sodium sulfafe decahydrate (460 mg), stirred at room temperature for 0.5 hr, and filtered. The filtrate was concentrated under reduced pressure, to obtain a crude product that was (S)-(2-methylenetetrahydro-1H-pyrrolizin-7a(5H)-yl)methanol (700 mg, yield:99%). ES-API: [M+H]⁺= 154.1.

Step III: (S)-(2-methylenetetrahydro-1H-pyrrolizin-7a(5H)-yl)methanol (27 mg, 0.18 mmol) was dissolved in anhydrous tetrahydrofuran (2 mL). In an iced water bath, sodium hydride (14 mg, 0.36 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (70 mg, 0.09 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, and filtered. The filtrate was concentrated under reduced pressure, to obtain the crude product t-butyl (5S,5aS,6S,9R)-1-fluoro-2-(7-fluoro-8-((triisopropylsilyl)ethynyl)naphthalen-1-yl)-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-heptanoate (77 mg, yield: 100%). ES-API: [M+H]⁺= 877.3.

Step IV: Cesium fluoride (135 mg, 0.9 mmol) was added to t-butyl (5S,5aS,6S,9R)-1-fluoro-2-(7-fluoro-8-((triisopropylsilyl)ethynyl)naphthalen-1-yl)-5-methyl-12-((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-heptanoate (77 mg, 0.09 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (64 mg, yield: 100%). ES-API: [M+H]⁺= 721.3.

Step V: In an iced water bath, 4 M hydrochloric acid-dioxane solution (5 mL) was slowly added to a solution of t-butyl (5S,5aS,6S,9R)-2-(8-ethynyl-7-fluoronaphthalen-1-yl)-1-fluoro-5-methyl-12-((S)-2-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (64 mg, 0.09 mmol) in acetonitrile(1 mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-(((S)-2-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z637, 12 mg, yield: 22%) as a light yellow solid. ES-API: [M+H]⁺= 621.3.

### Example 59: Synthesis of (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z638)

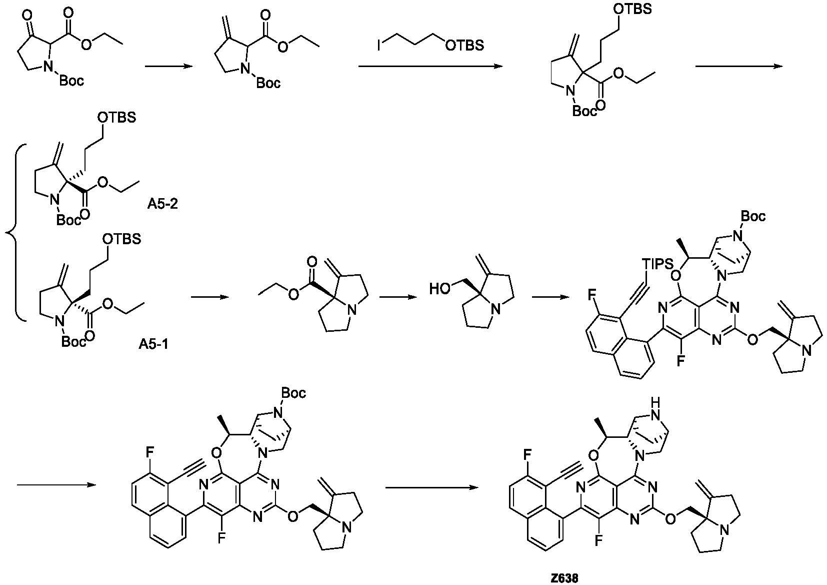

Step I: Methyltriphenylphosphonium bromide (10.41g, 29.15 mmol) was added to tetrahydrofuran (200 mL) and cooled to 0°C. Under a nitrogen atmosphere, a solution of potassium tert-butoxide in tetrahydrofuran (29.15mL, 29.15 mmol, 1M) was added to the mixture at 0°C, and then the reaction was warmed to room temperature and stirred for 1 hr. 1-(t-butyl) 2-ethyl3-oxopyrrolidin-1,2-dicarboxylate (3.0g, 12.82 mmol) was dissolved in tetrahydrofuran (10 mL), and slowly added to the reaction solution under a nitrogen atmosphere. After that, the reaction solution was stirred at room temperature for 3 hrs. The reaction solution was added with saturated ammonium chloride (30 mL), and extracted with ethyl acetate (100 mLX3). The organic phase was washed with saturated brine (200mL), dried over anhydrous sodium sulfate, filtered and concentrated. The crude product was purified by flash silica gel column chromatography (ethyl acetate /petroleum ether: 0%-8%) to obtain 1-(t-butyl) 2-ethyl 3-methylenepyrrolidin-1,2-dicarboxylate (2.0g, yield: 61.6%) as a colorless transparent liquid. ES-API: [M-Boc+H]⁺=200.1.

Step II: At -78°C, lithium bis(trimethylsilyl)amide (4.0mL, 4.0mmol) was added dropwise to a solution of 1-(t-butyl)2-methyl3-methylenepyrrolidin-1,2-dicarboxylate (0.50g, 1.96 mmol) in dry tetrahydrofuran (20.0mL), and stirred at this temperature for 1 hr. After 1 hr, t-butyl (3-iodopropoxy)dimethylsilane (3.0 g, 9.99 mmol) was slowly added dropwise to the above solution, slowly heated to room temperature and reacted for 2 hrs. After the reaction, the reaction solution was added with a saturated ammonium chloride aqueous solution (100 mL), and extracted with ethyl acetate (100 mLX4). The organic phase was washed with saturated brine (200 mLX1), dried over anhydrous sodium sulfate, filtered and concentrated. The crude product was purified by flash silica gel column chromatography (ethyl acetate /petroleum ether: 0-10%) to obtain 1-(t-butyl) 2-ethyl2-(3-((t-butyldimethylsilyl)oxy)propyl)-3-methylenepyrrolidin-1,2-dicarboxylate (720 mg, yield: 86%), as a colorless transparent oil. ES-API: [M-Boc+H]⁺=328.3.

Step III: 1-(t-butyl) 2-ethyl2-(3-((t-butyldimethylsilyl)oxy)propyl)-3-methylenepyrrolidin-1,2-dicarboxylate (4.4g, 10.30 mmol) was resolved under the following conditions: (chromatographic column: Daicel CHIRALPAK^{®} IB 250*30 mm, 10µm; mobile phase A: n-hexane; mobile phase B: ethanol; detection wavelength: 254nm /214nm; flow rate: 25mL/min; isocratic elution procedure: mobile phase A: mobile phase B=99.5:0.5(V/V); column temperature: room temperature) to obtain two isomers. One structure was arbitrarily designated as 1-(t-butyl) 2-ethyl (R)-2-(3-((t-butyldimethylsilyl)oxy)propyl)-3-methylenepyrrolidin-1,2-dicarboxylate (A5-1, 2.01g, peak 1, retention time 7.0 min, yield: 40%) as a light yellow oil. ES-API: [M-Boc+H]⁺=350.2. The other structure was arbitrarily designated as 1-(t-butyl) 2-ethyl (S)-2-(3-((t-butyldimethylsilyl)oxy)propyl)-3-methylenepyrrolidin-1,2-dicarboxylate (A5-2, 1.91g, peak 2, retention time 9.2 min, yield: 38%) as a light yellow oil. ES-API: [M-Boc+H]⁺=328.3.

Step IV: In an iced water bath, 1-(t-butyl) 2-ethyl (R)-2-(3-((t-butyldimethylsilyl)oxy)propyl)-3-methylenepyrrolidin-1,2-dicarboxylate (A5-1, retention time 7.0 min, 2.01g, 4.70mmol) was dissolved in dichloromethane (20 mL), and then thionyl chloride (15.0mL, 207.0mmol) was added, and stirred overnight at room temperature. The reaction solution was rotary evaporated to dryness under reduced pressure to remove the solvent. A mixed solvent (dichloromethane: methanol =10:1, 30 mL) was added to potassium carbonate (1.30g, 9.40mmol) was added to the crude product, and stirred at room temperature for 1 hr. After filtration, the filtrate was rotary evaporated to dryness under reduced pressure to obtain ethyl (R)-1-methylenetetrahydro-1H-pyrrolizin-7a(5H)-carboxylate (650mg, yield: 70.83%) as a colorless transparent liquid. ES-API: [M+H]⁺=196.1.

Step V: Ethyl (R)-1-methylenetetrahydro-1H-pyrrolizin-7a(5H)-carboxylate (100mg, 0.512mmol) was dissolved in tetrahydrofuran (10 mL), and cooled to 0°C. Under a nitrogen atmosphere, a lithium aluminum hydride solution (2.0 mL, 2.0mmol, 1M in tetrahydrofuran) was added to the solution. The reaction was stirred at 0°C for 1 hr. The reaction was quenched with water (0.03 mL), and 15% sodium hydroxide aqueous solution (0.03 mL) and water (0.09 mL) were added. Tetrahydrofuran (10 mL) was added and filtered. The filtrate was concentrated, to obtain (R)-(1-methylenetetrahydro-1H-pyrrolizin-7a(5H)-yl)methanol (70 mg, yield: 89%) as a colorless transparent liquid. ES-API: [M+H]⁺=154.1.

Step VI: (R)-(1-methylenetetrahydro-1H-pyrrolizin-7a(5H)-yl)methanol (70mg, 0.457 mmol) was dissolved in anhydrous tetrahydrofuran (5 mL). Sodium hydride (73.0mg, 1.83 mmol) was added in an iced water bath, and reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100mg, 0.127 mmol) in tetrahydrofuran (2 mL) was added dropwise to the above solution, and reacted at 0°C for 30 min. The reaction was shown to be completed by LC/MS. The reaction solution was diluted with ethyl acetate (50 mL), washed with saturated ammonium chloride solution (15 mLX 2) and saturated brine (50 mLX3), dried over anhydrous sodium sulfate, and filtered. The crude product was purified by automated flash chromatography on silica gel (dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-(((R)-1-methylenetetrahydro)-1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (150mg, crude product). ES-API: [M+H]⁺= 877.4.

Step VII: Cesium fluoride (1500mg, 9.86mmol) was added to t-butyl (5S,5aS,6S,9R)-1-fluoro-2-(7-fluoro-8-((triisopropylsilyl)ethynyl)naphthalen-1-yl)-5-methyl-12-(((R)-1-methylenetetrahydro)-1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (150mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX 2) and saturated brine (50 mLX 3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170mg, crude product). ES-API: [M+H]⁺= 721.3.

Step VIII: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (170mg, crude product) in acetonitrile (5.0mL). The reaction mixture was stirred at 0°C for 0.5 hr. The reaction was shown to be completed by LC/MS. The reaction solution was concentrated to dryness, and the residue was purified by preparative HPLC (ammonium bicarbonate method 2) to obtain (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z638, 10.0mg, total yield of 3 steps: 12.6%) as a light yellow solid. ES-API: [M+H]⁺= 621.3. ¹HNMR(400MHz, DMSO-*d₆*): 8.20-8.17(m, 2H), 7.66-7.55 (m, 3H), 5.49-5.07 (m, 2H), 4.60-4.20(m, 3H), 4.12-3.66(m, 4H), 3.35-3.10 (m, 5H), 2.78-2.50(m, 2H), 2.01-1.65(m, 9H), 1.58-1.45 (m, 3H).

### Example 60: Synthesis of (5S,5aS,6S,9R)-2-(8-ethynyl-7-fluoronaphthalen-1-yl)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z639)

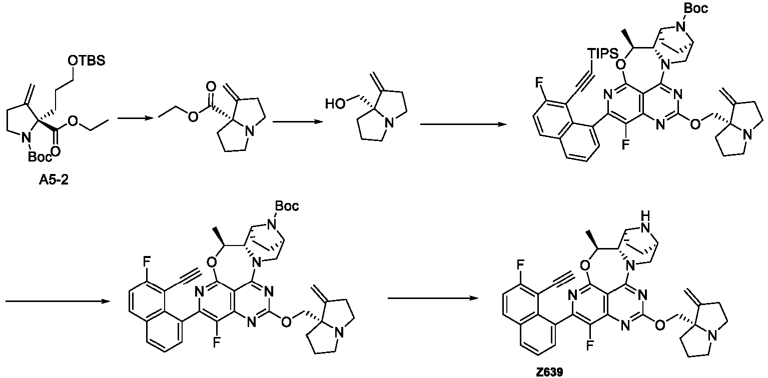

Step I: In an iced water bath, 1-(t-butyl) 2-ethyl (S)-2-(3-((t-butyldimethylsilyl)oxy)propyl)-3-methylenepyrrolidin-1,2-dicarboxylate (A5-2, retention time 9.2 min, 1.91g, 4.46mmol) was dissolved in dichloromethane (20 mL), and thionyl chloride (15.0mL, 207.0mmol) was added. The reaction solution was stirred overnight at room temperature. The reaction solution was rotary evaporated to dryness under reduced pressure to remove the solvent. A mixed solvent (dichloromethane: methanol =10:1, 30mL) was added to potassium carbonate (1.30g, 9.40mmol) was added to the crude product, and stirred at room temperature for 1 **hr.** After filtration, the filtrate was rotary evaporated to dryness under reduced pressure to obtain ethyl (S)-1-methylenetetrahydro-1H-pyrrolizin-7a(5H)-carboxylate (850mg, yield: 97.0%) as a colorless transparent liquid. ES-API: [M+H]⁺=196.1.

Step II: Ethyl (S)-1-methylenetetrahydro-1H-pyrrolizin-7a(5H)-carboxylate (150mg, 0.768mmol) was dissolved in tetrahydrofuran(10 mL), and cooled to 0°C. Under a nitrogen atmosphere, a lithium aluminum hydride solution (2.0 mL, 2.0mmol, **1M** in tetrahydrofuran) was added to the solution. The reaction was stirred at 0°C for 1 hr. The reaction was quenched with water (0.03 mL), and 15% sodium hydroxide aqueous solution (0.03 mL) and water (0.09 mL) were added. Tetrahydrofuran (10 mL) was added and filtered. The filtrate was concentrated, to obtain (S)-(1-methylenetetrahydro-1H-pyrrolizin-7a(5H)-yl)methanol (88mg, yield: 74.5%) as a colorless transparent liquid. ES-API: [M +H]⁺=154.1.

Step VI: (S)-(1-methylenetetrahydro-1H-pyrrolizin-7a(5H)-yl)methanol (80mg, 0.522 mmol) was dissolved in anhydrous tetrahydrofuran (5 mL). In an iced water bath, sodium hydride (83.0mg, 2.088 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100mg, 0.127 mmol) in tetrahydrofuran (2 mL) was added dropwise to the reaction solution, and reacted at 0°C for 30 min. The reaction was shown to be completed by LC/MS. The reaction solution was diluted with ethyl acetate (50 mL), washed with saturated ammonium chloride solution (15 mLX 2) and saturated brine (50 mLX3), dried over anhydrous sodium sulfate, and filtered. The crude product was purified by flash silica gel column chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-(((S)-1-methylenetetrahydro)-1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (150mg, crude product). ES-API: [M+H]⁺= 877.4.

Step IV: Cesium fluoride (1500mg, 9.86mmol) was added to t-butyl (5S,5aS,6S,9R)-1-fluoro-2-(7-fluoro-8-((triisopropylsilyl)ethynyl)naphthalen-1-yl)-5-methyl-12-(((S)-1-methylenetetrahydro)-1H-pyrrolizin-7a(5H)-yl)methoxy)-5a,6,7,8,9,10-hexahydro-4-oxa-3,10a,11,13,14-pentaaza-6,9-methanonaphtho[1,8-ab]hepten-14-carboxylate (150mg, crude product) in N,N-dimethylformamide (5.0mL), and the reaction mixture was stirred at room temperature for 2 hrs. The reaction was shown to be completed by LC/MS. The reaction solution was diluted with ethyl acetate (60mL), washed with water (30 mLX 2) and saturated brine (50 mLX 3), dried over anhydrous sodium sulfate, filtered, and concentrated to obtain a crude product that was t-butyl (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (175mg, crude product). ES-API: [M+H]⁺= 721.3.

Step V: In an iced water bath, 4 M hydrochloric acid-dioxane solution (3 mL) was slowly added to a solution of t-butyl (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-carboxylate (175mg, crude product) in acetonitrile (5.0mL). The reaction mixture was stirred at 0°C for 0.5 hr. The reaction was shown to be completed by LC/MS. The reaction solution was concentrated to dryness, and the residue was purified by preparative HPLC (formic acid method 2) (chromatographic condition: 15mL-35-85-13min) to obtain (5S,5aS,6S,9R)-2-(8-ethynyl-7-fluoronaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z639, 11.0mg, total yield of 3 steps: 13.9%, formate) as a light yellow solid. ES-API: [M+H]⁺= 621.3.

### Example 61 Synthesis of (6aR,7S,10R)-2-(8-ethynyl-7-fluoronaphthalen-1-yl)-1-fluoro-13-((2-methylenetetrahydro-1H-pyrrolazin-7a(5H)-yl)methoxy)-5,6,6a,7,8,9,10,11-octahydro-4-oxa-3,11a,12,14,15-pentaaza-7,10-methylcycloheptan[4,5]cyclooctano[1,2,3-de]naphthalene (Z640)

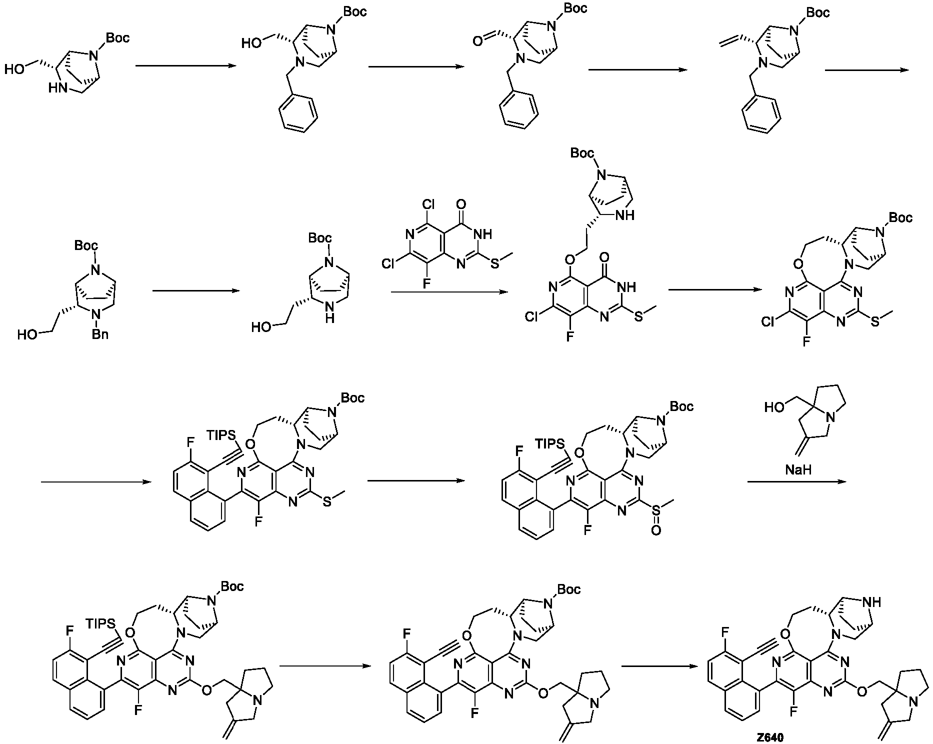

Step I: T-butyl (1S,2S,5R)-2-(hydroxymethyl)-3,8-diazabicyclo[3.2.1]octane-8-carboxylate (2.00 g, 8.25 mmol) was dissolved in acetonitrile (50 mL), and benzyl bromide (1.18 mL, 9.90 mmol) and anhydrous potassium carbonate (1.71 g, 12.38 mmol) were added sequentially. After stirring at room temperature for 1 hr, the solvent was removed by rotary evaporation to dryness. The residue was added with water, and extracted 3 times with dichloromethane. The organic phase was collected, washed with saturated brine, dried over anhydrous sodium sulfate, filtered, and rotary evaporated to dryness. The obtained crude product was purified by flash silica gel column chromatography (tetrahydrofuran/petroleum ether: 0-30%) to obtain t-butyl (1S,2S,5R)-3-benzyl-2-(hydroxymethyl)-3,8-diazabicyclo[3.2.1]octane-8-carboxylate (2.74 g, yield: 100%). ES-API: [M+H]⁺= 333.3.

Step II: T-butyl (1S,2S,5R)-3-benzyl-2-(hydroxymethyl)-3,8-diazabicyclo[3.2.1]octane-8-carboxylate (2.74 g, 8.12 mmol) in dichloromethane (80 mL) was cooledd to 0 °C, and Dess-Martin periodinane (4.46 g, 10.56 mmol) was added batchwise. After stirring at 0°C for 1 hr, a saturated sodium bicarbonate aqueous solution and a saturated sodium sulfite aqueous solution were added, and stirred at room temperature for 15 min. The organic phase was separated. The aqueous phase was extracted 2 times with dichloromethane. The organic phase was collected and washed with saturated brine, dried over anhydrous sodium sulfate, filtered, andd rotary evaporated to dryness, to obtain t-butyl (1*S*,2*S*,5*R*)-3-benzyl-2-formyl-3,8-diazabicyclo[3.2.1]octane-8-carboxylate as a crude product (2.6 g), which was directly used in the next step of reaction without purification. ES-API: [M+H]⁺= 331.2.

Step III: Under a nitrogen atmosphere, a 1 M potassium tert-butoxide solution in tetrahydrofuran (19.7 mL, 19.7 mmol) was slowly added dropwise to a suspension of methyltriphenylphosphonium bromide (5.6 g, 15.74 mmol) in tetrahydrofuran (60 mL) at 0 °C. After reaction for 15 min, a solution of t-butyl (1S,2S,5R)-3-benzyl-2-formyl-3,8-diazabicyclo[3.2.1]octane-8-carboxylate as a crude product (2.6 g, 7.87 mmol) in tetrahydrofuran (20 mL) was added dropwise. After that, the reaction solution was heated to room temperature and stirred overnight. A saturated ammonium chloride aqueous solution was slowly added. The reaction solution was extracted 3 times with ethyl acetate. The organic phase was collected, washed with saturated brine, dried over anhydrous sodium sulfate, filtered, and rotary evaporated to dryness, to obtain a crude product. The crude product was purified by flash silica gel column chromatography (ethyl acetate /petroleum ether: 0-8%) to obtain t-butyl (1S,2R,5R)-3-benzyl-2-ethenyl-3,8-diazabicyclo[3.2.1]octane-8-carboxylate (2.10 g, yield of 2 steps: 79%). ES-API: [M+H]⁺= 329.3.

Step IV: Under a nitrogen atmosphere, at 0 °C, a 0.5 M 9-borabicyclo[3.3.1]nonane solution in tetrahydrofuran (42.00 mL, 21.00 mmol) was added dropwise to t-butyl (1S,2R,5R)-3-benzyl-2-ethenyl-3,8-diazabicyclo[3.2.1]octane-8-carboxylate (2.3 g, 7.00 mmol) in tetrahydrofuran (20.0 mL). The reaction solution was heated to room temperature and stirred overnight. The reaction solution was cooled to 0°C, and a 3 M sodium hydroxide aqueous solution (15.0 mL) and 30% hydrogen peroxide (15.0 mL) were slowly added, and stirred at room temperature for 3 hrs. The reaction solution was diluted with water, and extracted 2 times with ethyl acetate. The organic phase was collected, washed with saturated brine, dried over anhydrous sodium sulfate, filtered, and rotary evaporated to dryness. The obtained crude product was purified by flash silica gel column chromatography (tetrahydrofuran/petroleum ether: 0-30%) to obtain t-butyl (1R,2R,5S)-3-benzyl-2-(2-hydroxyethyl)-3,8-diazabicyclo[3.2.1]octane-8-carboxylate (1.85 g, yield: 76%). ES-API: [M+H]⁺= 347.3.

Step V: T-butyl (1R,2R,5S)-3-benzyl-2-(2-hydroxyethyl)-3,8-diazabicyclo[3.2.1]octane-8-carboxylate (1.85 g, 5.34 mmol) obtained in the previous step was dissolved in anhydrous methanol (20.0 mL) in a two-neck flask, and then 10% palladium on carbon (500 mg) was added. The reactor was purged 3 times. Under a hydrogen atmosphere, 7.0 M ammonia/methanol solution (4.0 mL) was added, and stirred overnight at room temperature. After filtration under suction, the filtrate was rotary evaporated to dryness to obtain t-butyl (1R,2R,5S)-2-(2-hydroxyethyl)-3,8-diazabicyclo[3.2.1]octane-8-carboxylate (1.36 g, crude product), which was directly used in the next step of reaction without purification. ES-API: [M+H]⁺= 257.2.

Step VI: Under a nitrogen atmosphere, sodium hydride (60% dispersed in an oil) (94 mg, 2.34 mmol) was added batchwise to a solution of t-butyl (1R,2R,5S)-2-(2-hydroxyethyl)-3,8-diazabicyclo[3.2.1]octane-8-carboxylate as a crude product (150 mg, 0.59 mmol) in anhydrous tetrahydrofuran (20.0 mL) at 0°C. After reaction for 15 min, the reaction solution was added with 5,7-dichloro-8-fluoro-2-(methylthio)pyrido[4,3-d]pyrimidine-4(3H)-one (492 mg, 1.76 mmol). The reaction solution was heated to 40°C, reacted for 6 hrs, added with a saturated ammonium chloride aqueous solution slowly, and extracted 3 times with dichloromethane. The organic phase was washed with saturated brine, dried over anhydrous sodium sulfate, filtered, and rotary evaporated to dryness, to obtain a crude product. The crude product was purified by flash silica gel column chromatography (dichloromethane/methanol: 0-15%) to obtain t-butyl (15,2R,5R)-2-(2-((7-chloro-8-fluoro-2-(methylthio)-4-oxa-3,4-dihydropyrido[4,3-d]pyrimidine-5-yl)oxy)ethyl)-3,8-diazabicyclo[3.2.1]octane-8-carboxylate (220 mg, yield: 75%). ES-API: [M+H]⁺= 500.1.

Step VII: At room temperature, 2-(7-azobenzotriazole)-N,N,N',N'-tetramethyluronium hexafluorophosphate (335 mg, 0.88 mmol) was added to a solution of t-butyl (15,2R,5R)-2-(2-((7-chloro-8-fluoro-2-(methylthio)-4-oxa-3,4-dihydropyrido[4,3-d]pyrimidine-5-yl)oxy)ethyl)-3,8-diazabicyclo[3.2.1]octane-8-carboxylate (220 mg, 0.44 mmol) in N,N-dimethylformamide (22.0 mL) and stirred for 5 min. Then N,N-diisopropylethyl amine (0.38 mL, 2.20 mmol) was added. After reaction overnight, the reaction solution was diluted with ethyl acetate. The organic phase was washed 3 times with saturated brine, dried over anhydrous sodium sulfate, filtered, and rotary evaporated to dryness, to obtain a crude product. The crude product was purified by flash silica gel column chromatography (ethyl acetate /petroleum ether: 0-30%) to obtain t-butyl (6aR,7S,10R)-2-chloro-1-fluoro-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate (73 mg, yield: 34%). ES-API: [M+H]⁺= 482.1.

Step VIII: To a microwave tube, t-butyl (6a*R,*7*S*,10*R*)-2-chloro-1-fluoro-13-(methylthio)-5,6,6a,7,8,9,10,11-octahydro-4-oxa-3,11a,12,14,15-pentaaza-7,10-methanocyclohepta [4,5]cycloocta[1,2,3-de]naphthalen-15-carboxylate (73 mg, 0.15 mmol), ({2-fluoro-8-(4,4,5,5-tetramethyl-1,3,2-dioxaborolan-2-yl)-1-naphthalenyl}ethynyl)triisopropylsilane (171 mg, 0.38 mmol), potassium phosphate (96 mg, 0.45 mmol), [n-butyldi(1-adamantanyl)phosphine](2-amino-1,1'-biphenyl-2-yl)palladium (II) mesylate (22 mg, 0.03 mmol), tetrahydrofuran (2.0 mL), and water (0.4 mL) were added, purged 4 times with nitrogen, and reacted in an oil bath at 80°C for 2 hrs. The reaction solution was cooled to room temperature added with water, and extracted 3 times with ethyl acetate. The organic phases were combined, and washed with saturated brine. The organic phase was dried over anhydrous sodium sulfate, and filtered. The filtrate was rotary evaporated to dryness under reduced pressure, and the crude product was purified by flash silica gel column chromatography (ethyl acetate /petroleum ether: 0-40%) to obtain the product t-butyl (6aR,7S,10R)-1-fluoro-2-(7-fluoro-8-((triisopropylsilyl)ethynyl)naphthalen-1-yl)-13-(methylthio)-5,6,6a,7,8,9,10,11-octahydro-4-oxo-3,11a,12,14,15-pentaaza-7,10-methanocyclohepta[4,5]cycloocta[1,2,3-de]naphthalen-15-carboxylate (61 mg, yield: 52%). ES-API: [M+H]⁺= 772.3.

Step IX: At 0°C, m-chloroperoxybenzoic acid (24 mg, 0.12 mmol) was added to a solution of t-butyl (6aR,7S,10R)-1-fluoro-2-(7-fluoro-8-((triisopropylsilyl)ethynyl)naphthalen-1-yl)-13-(methylthio)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate (61 mg, 0.08 mmol) in dichloromethane (5.0 mL), and stirred for 0.5 hr. A saturated sodium bicarbonate aqueous solution and a saturated sodium sulfite aqueous solution were added, and stirred vigorously for 15 min. The organic phase was separated, and the aquesous phase was extracted 2 times with dichloromethane. The organic phase was collected and washed with saturated brine, dried over anhydrous sodium sulfate, filtered, and rotary evaporated to dryness to obtain t-butyl (6aR,7S,10R)-1-fluoro-2-(7-fluoro-8-((triisopropylsilyl)ethynyl)naphthalen-1-yl)-13-(methylsulfinyl)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate as a crude product, which was directly used in the next step of reaction without purification. ES-API: [M+H]⁺= 788.3.

Step X: At 0°C, sodium hydride (60% dispersed in an oil)(19 mg, 0.47 mmol) was added to (2-methylenetetrahydro-1H-pyrrolizin-7a(5H)-yl)methanol (61 mg, 0.40 mmol) in tetrahydrofuran (5.0 mL). After reaction for 15 min, a solution of t-butyl (6aR,7S,10R)-1-fluoro-2-(7-fluoro-8-((triisopropylsilyl)ethynyl)naphthalen-1-yl)-13-(methylsulfinyl)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate crude product in tetrahydrofuran (2.0 mL) was added dropwise to the reaction solution. After reaction at 0°C for 0.5 hr, the reaction solution was slowly added with a saturated ammonium chloride aqueous solution, and extracted 3 times with dichloromethane. The organic phase was collected, washed with saturated brine, dried over anhydrous sodium sulfate, filtered, and rotary evaporated to dryness. The crude product was purified by flash silica gel column chromatography (dichloromethane/methanol: 0-10%) to obtain the product t-butyl (6aR,7S,10R)-1-fluoro-2-(7-fluoro-8-((triisopropylsilyl)ethynyl)naphthalenyl-1-yl)-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate (16 mg, yield:23%). ES-API: [M+H]⁺= 877.3.

Step XI: T-butyl (6aR,7S,10R)-1-fluoro-2-(7-fluoro-8-((triisopropylsilyl)ethynyl)naphthalenyl -1-yl)-13-((2-methylenetetrahydro-1H-pyrrolizin-7a)(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate (16 mg, 0.018 mmol) was dissolved in N,N-dimethylformamide (1.0 mL). Cesium fluoride (28 mg, 0.18 mmol) was added, and reacted at room temperature for 1 hr. After the reaction, the reaction solution was added with water, and extracted 3 times with ethyl acetate. The organic phases were combined, and washed with saturated brine. The organic phase was dried over anhydrous sodium sulfate, and filtered. The filtrate was rotary evaporated to dryness under reduced pressure, to obtain crude product t-butyl (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate. The crude product was directly used in the next step of reaction without purification. ES-API:[M+H]⁺=721.2.

Step XII: T-butyl (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalen-15-carboxylate as a crude product was dissolved in acetonitrile (2.0 mL), and 4M hydrochloric acid/dioxane solution (2.0 mL, 8.0 mmol) was added in an ice bath, heated to room temperature and reacted for 1 hr. After the reaction, the reaction solution was concentrated to dryness, and the crude product was purified by preparative HPLC (ammonium bicarbonate method 1) to obtain the target compound (6aR,7S,10R)-2-(8-ethynyl-7-fluoronaphthalen-1-yl)-1-fluoro-13-((2-methylenetetrahydro-1H-pyrrolizin-7a(5H)-yl)methoxy)-5,6,6a,7,8,9,10,11-octahydro-4-oxa-3,11a,12,14,15-pentaaza-7,10-methanocyclohepta[4,5]cycloocta[1,2,3-de]naphthalene (Z640, 1.60 mg, yield: 14%). ES-API: [M+H]⁺=621.3.

### Example 62: Synthesis of (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z609)

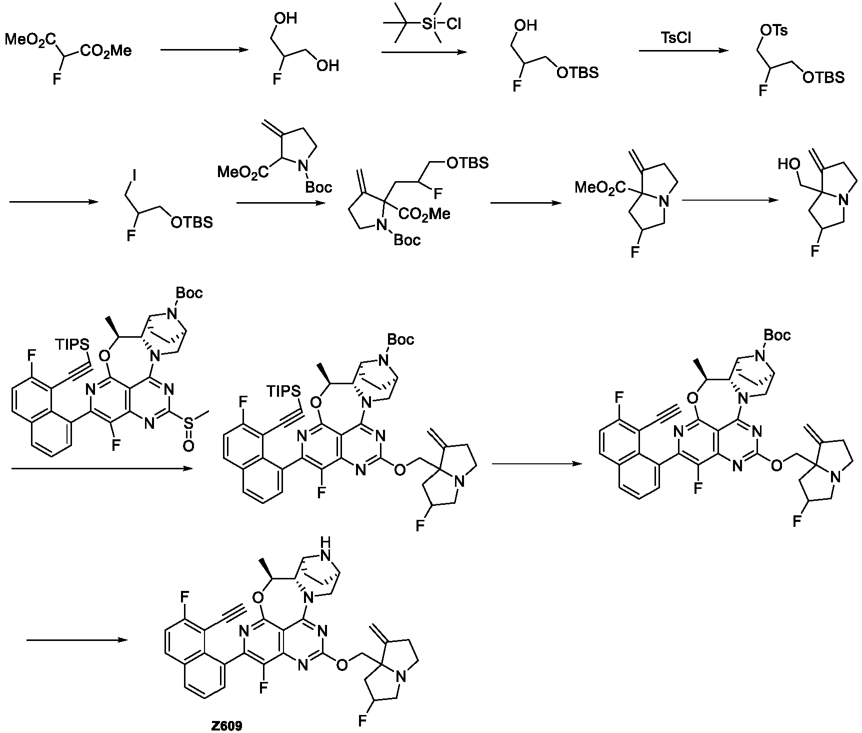

Step I: At room temperature, a dimethyl 2-fluoromalonate (10 g, 66.62 mmol)/tetrahydrofuran(100 mL) solution was added dropwise to a mixture of sodium borohydride (10.08 g, 266.472 mmol) and lithium chloride (2.82 g, 66.618 mmol) in anhydrous ethanol (80 mL)/tetrahydrofuran (100 mL). After that, the reaction was stirred at room temperature for 16 hrs. The reaction solution was cooled to 0°C and quenched with concentrated hydrochloric acid (10 mL). The reaction solution was added with ethyl acetate (100 mL), and filtered through diatomite. The filtrate was concentrated. The filter cake was suspended in dichloromethane/methanol (10:1, 150mL) and stirred at room temperature for 30 min. After filtration, the filtrate was concentrated. The crude product was purified by flash silica gel column chromatography (0-10%methanol /dichloromethane) to obtain 2-fluoropropan-1,3-diol (2 g, yield: 32%). ¹H NMR(400M, CDCl₃): 4.65(m, 1 H), 3.84-3.62 (m, 4H).

Step II: At 0°C, t-butyldimethylchlorosilane (3.2 g, 21.26 mmol) was added to a solution of 2-fluoropropan-1,3-diol (2 g, 21.25 mmol) and imidazole (2.17g, 31.88 mmol) in dichloromethane (50 mL). The reaction was stirred at room temperature for 1 hrs. The reaction was quenched with water (100 mL), and extracted with dichloromethane (50 mLX2). The organic phase was washed with saturated brine (50 mL), dried over anhydrous sodium sulfate, filtered, and concentrated. The residue was purified by flash silica gel column chromatography (0-15%ethyl acetate /petroleum ether) to obtain 3-((t-butyldimethylsilyl)oxy)-2-fluoropropan-1-ol (1 g, yield: 22.5%). ES-API: [M+H]⁺=209.2.

Step III: At 0°C, 4-dimethylaminopyridine (322mg, 2.64 mmol) and triethyl amine (0.55 mL, 3.96 mmol) were sequentially added a solution of 3-((t-butyldimethylsilyl)oxy)-2-fluoropropan-1-ol (500 mg, 2.64 mmol) in dichloromethane (10 mL). p-toluenesulfonyl chloride (754 mg, 3.96 mmol) was added, and stirred at room temperature for 2 hrs. The reaction was quenched with 1M hydrochloric acid (20 mL), and extracted with dichloromethane (30 mLX2). The organic layer was washed with saturated sodium bicarbonate. The organic phase was dried over anhydrous sodium sulfate, filtered and concentrated to obtain 3-((t-butyldimethylsilyl)oxy)-2-fluoropropyl4-toluene sulfonate (900 mg, yield: 86.2%). ES-API: [M+H]⁺=363.1.

Step IV: Sodium iodide (3.72 g, 24.83 mmol) was added to a solution of 3-((t-butyldimethylsilyl)oxy)-2-fluoropropyl4-toluene sulfonate (900 mg, 2.48 mmol) in acetone (15 mL). The reaction tube was sealed and the reaction was heated at 75°C for 5 hrs. The reaction was quenched with water (50 mL) and extracted with ethyl acetate (30 mLX2). The organic phase was washed with saturated brine, dried over anhydrous sodium sulfate, filtered, and concentrated. The residue was purified by flash silica gel column chromatography (0-5% ethyl acetate /petroleum ether) to obtain t-butyl (2-fluoro-3-iodopropoxy)dimethylsilane (700 mg, yield: 88.6%).

Step V: At -78°C, lithium bis(trimethylsilyl)amide (2.6 mL, 2.6 mmol) was added to a solution of 1-(t-butyl)2-methyl3-methylenepyrrolidin-1,2-dicarboxylate (350 mg, 1.45 mmol) in tetrahydrofuran (10 mL). The reaction was stirred at -78°C for 1 hr. A solution of t-butyl (2-fluoro-3-iodopropoxy)dimethylsilane (692 mg, 2.17 mmol) in tetrahydrofuran (1.5 mL) was added dropwise to the reaction solution, and stirred at -78°C for 5 min. The reaction was slowly heated to room temperature and stirred for 2 hrs. The reaction solution was cooled to 0°C, quenched with saturated ammonium chloride (30 mL), and extracted with ethyl acetate (30 mLX2). The organic phase was washed with saturated brine, dried over anhydrous sodium sulfate, filtered, and concentrated. The residue was purified by flash silica gel column chromatography (0-8%ethyl acetate /petroleum ether) to obtain 1-(t-butyl)2-methyl2-(3-((t-butyldimethylsilyl)oxy)-2-fluoropropyl)-3-methylenepyrrolidin-1,2-dicarboxylate (300 mg, yield: 47.9%). ES-API: [M+Na]⁺=454.3.

Step VI: Thionyl chloride (2.4 mL, 33.59 mmol) was added dropwise to a solution of 1-(t-butyl) 2-methyl 2-(3-((t-butyldimethylsilyl)oxy)-2-fluoropropyl)-3-methylenepyrrolidin-1,2-dicarboxylate (290mg, 0.67 mmol) in dichloromethane (10 mL), and stirred at 35°C for 12 hrs. The reaction was heated at 55°C for 48 hrs. The reaction solution was concentrated. The residue was dissolved in acetonitrile, and sodium bicarbonate (282 mg, 3.36 mmol) and potassium iodide (111 mg, 0.67 mmol) were added. The reaction was stirred at 60°C for 1 hr. The reaction solution was cooled to room temperature and filtered. The filtrate was concentrated. The residue was purified by flash silica gel column chromatography (0-5%methanol /dichloromethane), to obtain methyl 6-fluoro-1-methylenetetrahydro-1H-pyrrolizin-7a(5H)-carboxylate (130 mg, yield: 97.1%). ES-API: [M+H]⁺=200.1.

Step VII: A lithium aluminium hydride /tetrahydrofuran solution (1.3 mL, 1.3 mmol) was added dropwise to a solution of methyl 6-fluoro-1-methylenetetrahydro-1H-pyrrolizin-7a(5H)-carboxylate (130 mg, 0.65 mmol) in tetrahydrofuran (5 mL), and stirred at 0°C for 1 hr. 3 drops of water and a 15% sodium hydroxide aqueous solution (0.1 mL) were sequentially added to quench the reaction. The mixture was stirred at room temperature for 30 min, and filtered through diatomite. The filtrate was concentrated to obtain a crude product that was (6-fluoro-1-methylenetetrahydro-1H-pyrrolizin-7a(5H)-yl)methanol (100 mg, yield: 89.4%) as a yellow oil. ES-API: [M+H]⁺=172.1.

Step VIII: Sodium hydride (9 mg, 0.23 mmol) and tetrahydrofuran (3 mL) were added to a round-bottom flask. The reaction solution was cooled to 0°C, and a solution of (6-fluoro-1-methylenetetrahydro-1H-pyrrolizin-7a(5H)-yl)methanol (26 mg, 0.15 mmol) in anhydrous tetrahydrofuran (1 mL) was added. The reaction was continued at room temperature for 20 min. T-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (60 mg, 0.076 mmol) was added to the reaction solution, and reacted at 0°C for 20 min. The reaction was shown to be completed by LC/MS. The reaction solution was added with a saturated ammonium chloride solution (30 mL). The reaction solution was extracted with ethyl acetate (20 mLX2). The organic phase was washed with saturated brine (30 mL), dried over anhydrous sodium sulfate, filtered, and concentrated to obtain t-butyl (5S,5aS,6S,9R)-1-fluoro-12-((6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-heptanoate (65 mg, crude product). ES-API: [M+H]⁺=895.3.

Step IX: Cesium fluoride (110 mg, 0.73 mmol) was added to t-butyl (5S,5aS,6S,9R)-1-fluoro-12-((6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-heptanoate (65 mg, crude product) in N,N-dimethylformamide (3 mL). The reaction mixture was stirred at room temperature for 1 hr. The reaction was shown to be completed by LC/MS. The reaction solution was diluted with ethyl acetate (50 mL), washed with water (15 mLX2) and saturated brine (15 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, crude product). ES-API: [M+H]⁺= 739.3.

Step X: In an iced water bath, 4 M hydrochloric acid-dioxane solution (1 mL) was slowly added to a solution of t-butyl (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, crude product) in acetonitrile (2 mL). The reaction mixture was stirred at room temperature for 1 hr. The reaction was shown to be completed by LC/MS. The reaction solution was concentrated to dryness, and the residue was purified by preparative HPLC (ammonium bicarbonate method 2) to obtain (5S,5aS,6S,9R)-2-(8-ethynyl-7-fluoronaphthalen-1-yl)-1-fluoro-12-((6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z609, 1 mg, diastereoisomer ratio =65:35, retention time: 6.31 min and 6.44min, yield: 2%). ES-API: [M+H]⁺= 639.2.

### Example 63: Synthesis of (2-(8-ethynyl-7-fluoronaphthalen-1-yl)-1-fluoro-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-5-yl)methanol (Z672)

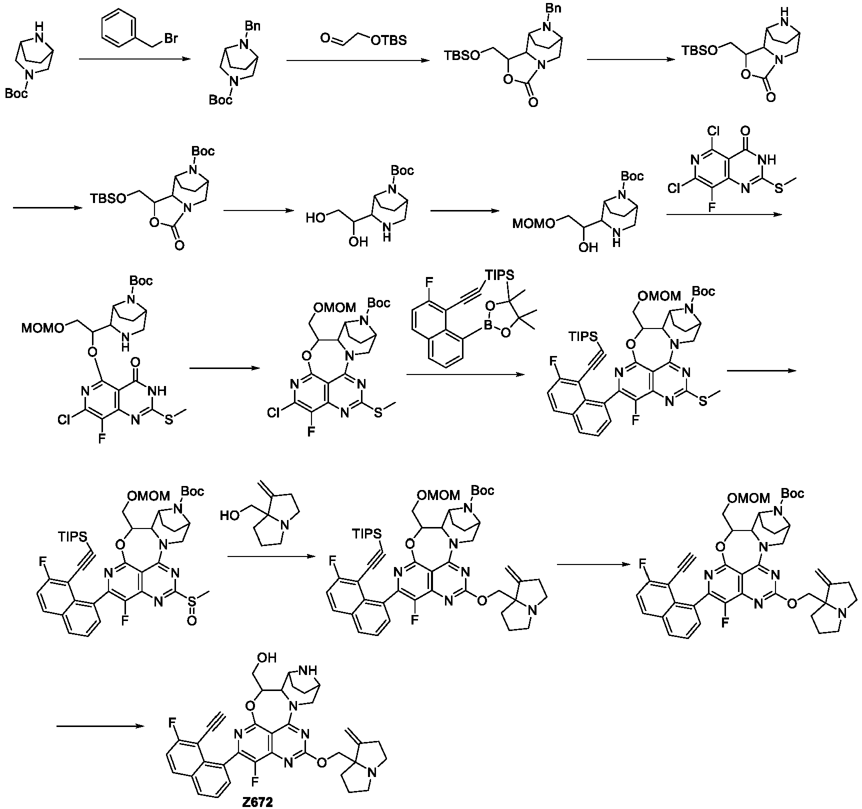

Step I: t-butyl 3,8-diazadicyclo[3.2.1]octane-3-carboxylate (2 g, 9.421 mmol) was dissolved in acetonitrile (20 mL), and potassium carbonate (1.432 g, 10.363 mmol) and benzyl bromide (1.934 g, 11.305 mmol) were sequentially added to the reaction solution at room temperature. The reaction solution was heated to 60°C and stirred for 5 hrs. The reaction was shown to be completed by LC/MS. The reaction solution was added with water (30 mL) to quench the reaction, and then extracted with ethyl acetate (30 mLX3). The organic phases were combined, washed with saturated brine (20 mLX2), dried over anhydrous sodium sulfate, filtered and concentrated. The crude product was purified by automated flask silica gel chromatography (ethyl acetate: petroleum ether= 0-40%) to obtain t-butyl 8-benzyl-3,8-diazadicyclo[3.2.1]octane-3-carboxylate (2.748 g, yield: 96%). ES-API: [M+ H]⁺= 303.2.

Step II: Under a nitrogen atmosphere, t-butyl 8-benzyl-3,8-diazadicyclo[3.2.1]octane-3-carboxylate (2.3 g, 7.605 mmol) and N,N,N',N'-tetramethylethylene diamine (4.419 g, 38.027 mmol) were dissolved in anhydrous tetrahydrofuran (30 mL), and cooled to -78°C in a dry ice-acetone bath. S-butyl lithium (17.5 mL, 22.816 mmol, 1.3 M solution in hexane) was slowly added to the reaction solution, and stirred at -78°C for 1 hr. Then, the reaction solution was added with a solution of 2-(t-butyldimethylsilyl)oxy)acetaldehyde (3.977 g, 22.816 mmol) in tetrahydrofuran (10 mL), slowly heated to room temperature and stirred overnight. The reaction was shown to be completed by LC/MS. The reaction solution was quenched with a saturated ammonium chloride solution (50 mL), and extracted with ethyl acetate (30 mLX3). The organic phases were combined, washed with saturated brine (15 mLX2), dried over anhydrous sodium sulfate, filtered and concentrated. The crude product was purified by automated flash silica gel chromatography (ethyl acetate: petroleum ether= 0- 15%) to obtain (6S, 9R)-10-benzyl-1-(((t-butyldimethylsilyl) oxy)methyl)hexahydro-1H, 3H-6,9-epiaminooxazolo[3,4-a]azapyridin-3-one (806 mg, yield: 26%). ES-API: [M+ H]⁺= 403.3.

Step III: Under hydrogen atmosphere, (6S, 9R)-10-benzyl-1-(((t-butyldimethylsilyl) oxy)methyl)hexahydro-1H, 3H-6,9-epiaminooxazolo[3,4-a]azapyridine-3-one (806 mg, 2.002 mmol) was dissolved in dichloromethane (30 mL) and an ammonia-methanol solution (6 mL, 7M solution in methanol). The reaction solution was added with 10%palladium on carbon (300 mg), and the reaction mixture was stirred overnight at room temperature. The reaction was shown to be completed by LC/MS. The reaction solution was filtered through diatomite, and concentrated under reduced pressure, to obtain a crude product (6S, 9R)-1-(((t-butyldimethylsilyl) oxy) methyl)hexahydro-1H, 3H-6,9-epiaminooxazolo[3,4-a]azapyridine-3-one (625 mg). ES-API: [M+ H]⁺= 313.3.

Step IV: (6S, 9R)-1-(((t-butyldimethylsilyloxy)methyl)hexahydro-1H, 3H-6,9-epiaminooxazolo[3,4-a]azapyridine-3-one (625 mg, 2.000 mmol) was dissolved in dichloromethane (20 mL), and the mixture was allowed to stand in an iced water bath. The reaction solution was added with di-t-butyl dicarbonate (0.70 mL, 3.000 mmol) and N,N-diisopropylethyl amine (0.7 mL, 4.000 mmol), slowly heated to room temperature and stirred for 1 hr. The reaction was shown to be completed by LC/MS. The reaction solution was concentrated. The crude product was purified by automated flash silica gel chromatography (ethyl acetate: petroleum ether= 0- 20%) to obtain t-butyl (6S, 9R)-1-(((t-butyldimethylsilyl)oxy)methyl)-3-oxyhexahydro-1H, 3H-6,9-epiaminooxazolo[3,4-a]azapyridine-10-carboxylate (558 mg, yield: 68%). ES-API: [M+ H]⁺= 413.2.

Step V: At room temperature, sodium hydroxide (1.082 g, 27.048 mmol) was added to a solution of t-butyl (6S, 9R)-1-(((t-butyldimethylsilyl)oxy)methyl)-3-oxyhexahydro-1H, 3H-6,9-epiaminooxazolo[3,4-a]azapyridine-10-carboxylate (558 mg, 1.352 mmol) in ethanol (30 mL) and water (6 mL). The reaction mixture was heated to 80°C and stirred overnight. The reaction was shown to be completed by LC/MS. The reaction solution was concentrated to dryness under reduced pressure. The crude product was dissolved in water (1.5 mL), and extracted with dichloromethane and isopropanol (15 mLX4, V/V= 5:1). The organic phases were combined, washed with saturated brine (10 mLX2), dried over anhydrous sodium sulfate, filtered, and concentrated to obtain a crude product that was t-butyl (1R, 5S)-2-(1,2-dihydroxyethyl)-3,8-diazadicyclo[3.2.1]octane-8-carboxylate (388 mg). ES-API: [M+ H]⁺= 273.2.

Step VI: In an iced water bath, N,N-diisopropylethyl amine (342 mg, 2.644 mmol) was added to a solution of t-butyl (1R, 5S)-2-(1,2-dihydroxyethyl)-3,8-diazadicyclo[3.2.1]octane-8-carboxylate (360 mg, 1.322 mmol) in dichloromethane (20 mL), and the reaction mixture was stirred at 0°C for 0.5 hr. The reaction solution was added with bromomethylmethyl ether (198 mg, 1.586 mmol), and continuously stirred at 0°C for 2.5 hrs. The reaction was shown to be completed by LC/MS. The reaction solution was concentrated and the crude product was purified by automated flash silica gel chromatography (methanol: dichloromethane= 0- 10%) to obtain t-butyl (1R, 5S)-2-(1-hydroxyl-2-(methoxymethoxy)ethyl)-3,8-diazadicyclo[3.2.1]octane-8-carboxylate (420 mg). ES-API: [M+ H]⁺= 317.2.

Step VII: In an iced water bath, t-butyl (1R, 5S)-2-(1-hydroxyl-2-(methoxymethoxy)ethyl)-3,8-diazadicyclo[3.2.1]octane-8-carboxylate (420 mg, 1.327 mmol) was dissolved in tetrahydrofuran (10 mL). The reaction solution was added with sodium hydride (212 mg, 5.310 mmol, 60% dispersed in an oil), and stirred at 0°C for 30 min. At 0°C, the reaction solution was added with 5,7-dichloro-8-fluoro-2-(methylthio)pyridine[4,3-d]pyrimidine-4(3H)-one (743 mg, 2.655 mmol), then slowly heated to 60°C and stirred for 1.5 hrs. The reaction was shown to be completed by LC/MS. The reaction solution was slowly added with a saturated ammonium chloride solution (100 mL), and extracted with dichloromethane (50 mLX3). The organic phase was dried over anhydrous sodium sulfate, filtered, and concentrated to obtain t-butyl (1R, 5S)-2-(1-(7-chloro-8-fluoro-2-(methylthio)-4-oxa-3,4-dihydropyridine[4,3-d]pyrimidine-5-yl)oxy)-2-(methoxymethoxy)ethyl)-3,8-diazadicyclo[3.2.1]octane-8-carboxylate (520 mg, yield: 70%). ES-API: [M+ H]⁺= 560.2.

Step VIII: In an iced water bath, 2-(7-azobenzotriazole)-N,N,N',N'-tetramethyluronium hexafluorophosphate (706 mg, 1.857 mmol) was added to a solution of t-butyl (1R, 5S)-2-(1-(7-chloro-8-fluoro-2-(methylthio)-4-oxa-3,4-dihydropyridine[4,3-d]pyrimidine-5-yl)oxy)-2-(methoxymethoxy)ethyl)-3,8-diazadicyclo[3.2.1]octane-8-carboxylate (520 mg, 0.929 mmol) and N,N-diisopropylethyl amine (480 mg, 3.714 mmol) in dichloromethane (20 mL). The reaction mixture was stirred at 0°C for 0.5 hr, heated to room temperature and stirred for 1.5 hrs. The reaction was shown to be completed by LC/MS. The reaction solution was concentrated and the crude product was purified by automated flash silica gel chromatography (ethyl acetate: petroleum ether= 0- 20%) to obtain t-butyl 2-chloro-1-fluoro-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]hepten-14-carboxylate (225 mg, yield: 45%). ES-API: [M+ H]⁺= 542.1.

Step IX: Under a nitrogen atmosphere, t-butyl 2-chloro-1-fluoro-5-((methoxymethoxy) methyl)-12-(methylthio)-5a,6,7,8,9,10-hexahydro-5H-4-oxo-3,10a,11,13,14-pentaaza-6,9-methanonaphtho[1,8-ab]hepten-14-carboxylate (60 mg,0.111 mmol), ((2-fluoro-8-(4,4,5,5-tetramethyl-1,3,2-dioxabenzaldehyde-2-yl)naphthalen-1-yl)ethynyl)triisopropylsilane (125 mg, 0.277 mmol), potassium phosphate (70 mg, 0.332 mmol), and [n-butyldi(1-adamantanyl) phosphine](2-amino-1,1'-biphenyl-2-yl)palladium (II) mesylate (16 mg, 0.022 mmol) were dissolved in tetrahydrofuran (5 mL) and water (1 mL). The reaction mixture was stirred at 80°C in a micowave generator for 0.5 hr. The reaction was shown to be completed by LC/MS. The reaction solution was concentrated and the crude product was purified by automated flash silica gel chromatography (petroleum ether/ethyl acetate = 0 - 20%), to obtain t-butyl 1-fluoro-2-(7-fluoro-8-((triisopropylsilyl)ethynyl)naphthalen-1-yl)-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]hepten-14-carboxylate (49 mg, yield: 53%). ES-API: [M+ H]⁺= 832.3.

Step X: T-butyl 1-fluoro-2-(7-fluoro-8-(triisopropylsilyl)ethynyl)naphthalen-1-yl)-5-((methoxymethoxy)methyl)-12-(methylthio)-5a,6,7,8,9,10-hexahydro-5H-4-oxo-3,10a,11,13,14-pentaaza-6,9-methanonaphtho[1,8-ab]hepten-14-carboxylate (49 mg, 0.059 mmol) was dissolved in anhydrous dichloromethane (10 mL). m-chloroperoxybenzoic acid (15 mg, 0.088 mmol) was added to the solution, and stirred at room temperature for 0.5 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain a crude product that was t-butyl 1-fluoro-2-(7-fluoro-8-((triisopropylsilyl)ethynyl)naphthalen-1-yl)-5-((methoxymethoxy)methyl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (49 mg). ES-API: [M+ H]⁺= 848.3.

Step XI: (1-methylenetetrahydro-1H-pyrrolizin-7a(5H)-yl)methanol (27 mg, 0.173 mmol) was dissolved in anhydrous tetrahydrofuran (5 mL). In an iced water bath, sodium hydride (7 mg, 0.173 mmol) was added, and then reacted for 10 min in the iced water bath. A solution of t-butyl 1-fluoro-2-(7-fluoro-8-((triisopropylsilyl)ethynyl)naphthalen-1-yl)-5-((methoxymethoxy)methyl)-12-(methylthionyl)-5a,6,7,8,9,10-hexahydro-5H-4-oxy-3,10a,11,13,14-pentaaza-6,9-methanonaphtho[1,8-ab]hepten-14-carboxylate (49 mg, 0.058 mmol) in tetrahydrofuran (5 mL) was added dropwise to the reaction solution, and reacted at 0 °C for 30 min. The reaction was shown to be completed by LC/MS. The reaction solution was diluted with ethyl acetate (10 mL), washed with a saturated ammonium chloride solution (10 mLX2) and saturated brine (10 mLX 3), dried over anhydrous sodium sulfate, filtered, and concentrated under reduced pressure. The crude product was purified by automated flash silica gel chromatography (methanol /dichloromethane= 0 - 3%) to obtain t-butyl 1-fluoro-2-(7-fluoro-8-((triisopropylsilyl)ethynyl)naphthalen-1-yl)-5-((methoxymethoxy)methyl)-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (50 mg, yield: 92%). ES-API: [M+ H]⁺= 937.4.

Step XII: Cesium fluoride (52 mg, 0.341 mmol) was added to t-butyl 1-fluoro-2-(7-fluoro-8-((triisopropylsilyl)ethynyl)naphthalen-1-yl)-5-((methoxymethoxy)methyl)-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (32 mg, 0.034 mmol) in N,N-dimethylformamide (3 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered and concentrated. The crude product was purified by TLC (dichloromethane/methanol = 20: 1) to obtain t-butyl 2-(8-ethynyl-7-fluoronaphthalen-1-yl)-1-fluoro-5-(methoxymethoxy)methyl)-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-naphtho[1,8-ab]hepten-14-carboxylate (26 mg, yield: 98%). ES-API: [M+ H]⁺= 781.2.

Step XIII: In an iced water bath, a 4 M hydrochloric acid-dioxane solution (3 mL) was slowly added to a solution of t-butyl 2-(8-ethynyl-7-fluoronaphthalen-1-yl)-1-fluoro-5-(methoxymethoxy) methyl)-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (26 mg, 0.033 mmol) in acetonitrile (2 mL). The reaction mixture was stirred at 0°C for 1 hr. The reaction was shown to be completed by LC/MS. The reaction solution was concentrated to dryness under reduced pressure. The residue was added with a saturated sodium bicarbonate solution (3 mL) until the pH of the solution was > 7.0, and then extracted with dichloromethane (5 mLX3). The organic phases were combined and concentrated under reduced pressure, to obtain (2-(8-ethynyl-7-fluoronaphthalen-1-yl)-1-fluoro-12-(1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-5-yl)methanol (Z672, 17 mg, yield: 80%) as an off-white solid. ES-API: [M+H]⁺= 637.2.

### Example 64: Synthesis of 8-((5S, 5aS, 6S, 9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-1-naphthonitrile (Z673)

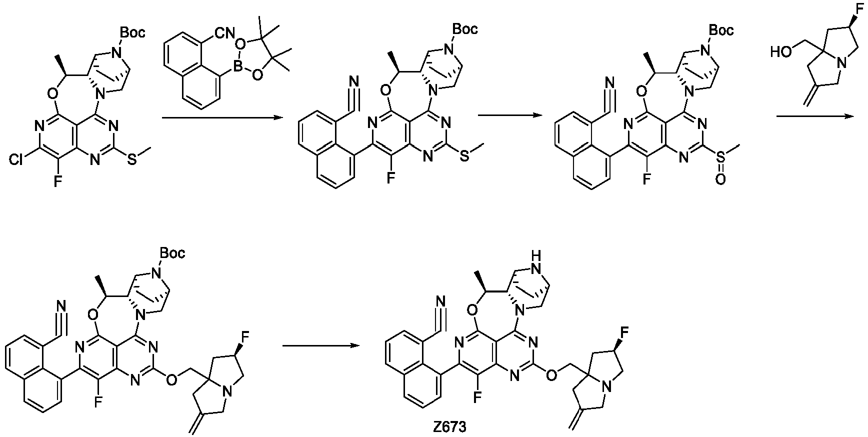

Step I: Under a nitrogen atmosphere, t-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (220 mg, 0.46 mmol), 8-(4,4,5,5-tetramethyl-1,3,2-dioxaborolan-2-yl)-1-naphthonitrile (319 mg, 1.14mmol), potassium phosphate (291 mg, 1.37 mmol), and CataXiumAPdG3 (50 mg, 0.068mmol) were dissolved in tetrahydrofuran (2.5 mL) and water (0.5 mL). The reaction mixture was heated to 80 °C for 3 hrs. The reaction was shown to be completed by LC/MS. The reaction solution was concentrated and the crude product was purified by automated flash silica gel chromatography (petroleum ether: ethyl acetate =20:1) to obtain t-butyl (5S, 5aS, 6S, 9R)-2-(8-cyanonaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (190 mg, yield: 69.5%). ES-API: [M+H]⁺=599.2.

Step II: m-chloroperoxybenzoic acid (43 mg, 0.25mmol) was added to a solution of t-butyl (5S, 5aS,6S,9R)-2-(8-cyanonaphthalen-1-yl)-1-fluoro-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (100 mg, 0.17 mmol) in dichloromethane (5mL), and the reaction mixture was stirred at room temperature for 1 hr. The reaction was shown to be completed by LC/MS. The reaction solution was diluted with ethyl acetate (20 mL), washed with a saturated sodium sulfite solution (15 mLX2) and saturated brine (15 mLX3), dried over anhydrous sodium sulfate, filtered, and concentrated to obtain a crude product that was t-butyl (5S, 5aS, 6S, 9R)-2-(8-cyanonaphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (102.7 mg, yield: 100%). ES-API: [M+H]⁺=615.2.

Step III: ((2R)-2-fluoro-6-methylenetetrahydro-1H-pyrrolizin-7a(5H)-yl)methanol (56.8 mg, 0.33 mmol) was dissolved in anhydrous tetrahydrofuran (5 mL). In an iced water bath, sodium hydride (16 mg, 0.67 mmol) was added, and then reacted for 30 min in the iced water bath. A solution of t-butyl (5S,5aS,6S,9R)-2-(8-cyanonaphthalen-1-yl)-1-fluoro-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (102.7 mg, 0.17 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX2) and saturated brine (15 mLX 3), dried over anhydrous sodium sulfate, filtered, and concentrated to obtain a crude product. The crude product was purified by automated flash silica gel chromatography (dichloromethane: methanol =20:1) to obtain t-butyl (5S, 5aS, 6S, 9R)-2-(8-cyanonaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, yield: 42%). ES-API: [M+H]⁺= 722.2.

Step IV: In an iced water bath, 4 M hydrochloric acid-dioxane solution (3 mL) was slowly added to a solution of t-butyl (5S, 5aS, 6S, 9R)-2-(8-cyanonaphthalen-1-yl)-1-fluoro-12-((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, 0.069 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain 8-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-1-naphthonitrile (Z673, 10 mg, yield: 23%) as a light yellow solid. ES-API: [M+H]⁺= 647.3. ¹H NMR (400 MHz, CD₃OD) δ 8.35 (t, J = 7.8 Hz, 1H), 8.20 (d, J = 8.0 Hz, 1H), 8.08-8.01 (m, 1H), 7.78 (d, J = 4.0 Hz, 1H), 7.76 -7.69 (m, 1H), 7.68 (d, J = 8.0 Hz, 1H), 5.45-5.36 (m, 2H), 5.24 (d, J = 16.0 Hz, 1H), 5.07-5.01 (m, 2H), 4.54-5.48 (m, 2H), 4.45-4.36 (m, 1H), 4.31-4.27 (m, 1H), 4.12 (d, J = 8.0 Hz, 1H), 3.78-3.70 (m, 2H), 3.67-3.59 (m, 1H), 3.51 (s, 1H), 3.49-3.45 (m, 1H), 3.41-3.39 (m, 1H), 3.25-3.19 (m, 1H), 3.05-2.93 (m, 1H), 2.92-2.89 (m, 1H), 2.86-2.83 (m, 1H), 2.81-2.78 (m, 1H), 2.55-2.47 (m, 2H), 2.42-2.37 (m, 1H), 2.23 -2.15 (m, 1H), 2.10-2.04 (m, 2H).

### Example 65: Synthesis of 7a-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy)methyl)-7-methylenehexahydro-1H-pyrrolizin-2-ol (Z674)

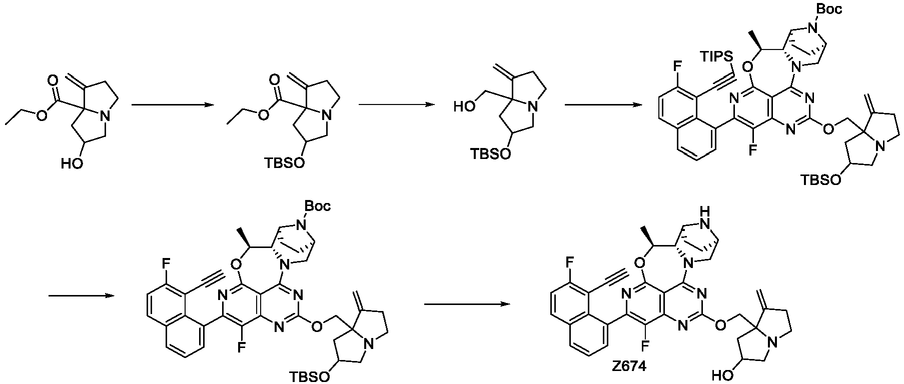

Step I: At 0°C, imidazole(87.0 mg, 1.278 mmol) and t-butyldimethylchlorosilane (256.84 mg, 1.704 mmol) were added to a solution of ethyl 6-hydroxyl-1-methylenetetrahydro-1H-pyrrolizin-7a(5H)-carboxylate (180 mg,0.852 mmol) in N,N-dimethylformamide (5.0 mL), and stirred overnight at room temperature. The reaction was shown to be completed by LC/MS. The reaction solution was diluted with ethyl acetate (60 mL), washed with water (30 mLX2) and saturated brine (50 mLX3), dried over anhydrous sodium sulfate, filtered, and concentrated to obtain methyl 6-((t-butyldimethylsilyl)oxy)-1-methylenetetrahydro-1H-pyrrolizin-7a(5H)-carboxylate (200mg, yield: 72%). ES-API: [M+H]⁺= 326.2.

Step II: Ethyl 6-((t-butyldimethylsilyl)oxy)-1-methylenetetrahydro-1H-pyrrolizin-7a(5H)-carboxylate (200 mg, 0.614 mmol) was dissolved in tetrahydrofuran (10 mL), and cooled to 0°C. Under a nitrogen atmosphere, a lithium aluminum hydride solution (1.3 mL, 1.3mmol, 1M solution in tetrahydrofuran) was added to the solution. The reaction was stirred at 0°C for 1 hr. The reaction was quenched with water (0.03 mL), and 15% sodium hydroxide aqueous solution (0.03 mL) and water (0.09 mL) were added. Tetrahydrofuran (10 mL) was added and filtered. The filtrate was concentrated, to obtain (6-((t-butyldimethylsilyl)oxy)-1-methylenetetrahydro-1H-pyrrolizin-7a(5H)-yl)methanol (140mg, yield: 80.38%) as a colorless transparent liquid. ES-API: [M +H]⁺ = 284.2.

Step III: (6-((t-butyldimethylsilyl)oxy)-1-methylenetetrahydro-1H-pyrrolizin-7a(5H)-yl) methanol (140 mg, 0.494 mmol) was dissolved in anhydrous tetrahydrofuran(5 mL). In an iced water bath, sodium hydride (40.0 mg, 0.988 mmol) was added, and then reacted for 30 min in the iced water bath. A solution of t-butyl (5S, 5aS, 6S, 9R)-1-fluoro-2-(7-fluoro-8-((triisopropylsilyl) ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (600mg, 0.761mmol) in tetrahydrofuran(2 mL) was added dropwise to the reaction solution, and reacted at 0°C for 30 min. The reaction was shown to be completed by LC/MS. The reaction solution was diluted with ethyl acetate (50 mL), washed with a saturated ammonium chloride solution (15 mLX2) and saturated brine (50 mLX3), dried over anhydrous sodium sulfate, and filtered. The crude product was purified by automated flash silica gel chromatography (dichloromethane: methanol =15:1) to obtain t-butyl (5S,5aS,6S,9R)-12-((6-((t-butyldimethylsilyl)oxy)-1-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho [1,8-ab]hepten-14-carboxylate (160 mg, yield: 32.16%). ES-API: [M+H]⁺= 1007.5.

Step IV: Cesium fluoride (100.0 mg, 0.658 mmol) was added to t-butyl (5S,5aS,6S,9R)-12-((6-((t-butyldimethylsilyl)oxy)-1-methylenetetrahydro-1H-pyrrolizin-7a(5H)-yl)methoxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (60 mg, 0.060 mmol) in N,N-dimethylformamide (5.0 mL), and the reaction mixture was stirred at room temperature for 2 hrs. The reaction was shown to be completed by LC/MS. The reaction solution was added with ethyl acetate (60 mL), washed with water (30 mLX2) and saturated brine (50 mLX3), dried over anhydrous sodium sulfate, filtered, and concentrated to obtain t-butyl (5S,5aS,6S,9R)-12-((6-((t-butyldimethylsilyl)oxy)-1-methylenetetrahydro-1H-pyrrolizin-7a(5H)-yl)methoxy)-2-(8-ethynyl-7-fluoronaphthalenyl-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (108 mg, crude product). ES-API: [M+H]⁺= 851.2.

Step V: In an iced water bath, 4 M hydrochloric acid-dioxane solution (1.0 mL) was slowly added to a solution of t-butyl (5S,5aS,6S,9R)-12-((6-((t-butyldimethylsilyl)oxy)-1-methylenetetrahydro-1H-pyrrolizin-7a(5H)-yl)methoxy)-2-(8-ethynyl-7-fluoronaphthalenyl-1-yl)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (51.06 mg, 0.060 mmol) in acetonitrile (5.0 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain 7a-((((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy)methyl)-7-methylenehexahydro-1H-pyrrolizin-2-ol (Z674, 3.0mg, yield:7.85%) as a light yellow solid. ES-API: [M+H]⁺= 637.2.

### Example 66: Synthesis of (5S, 5aS, 6S, 9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z675)

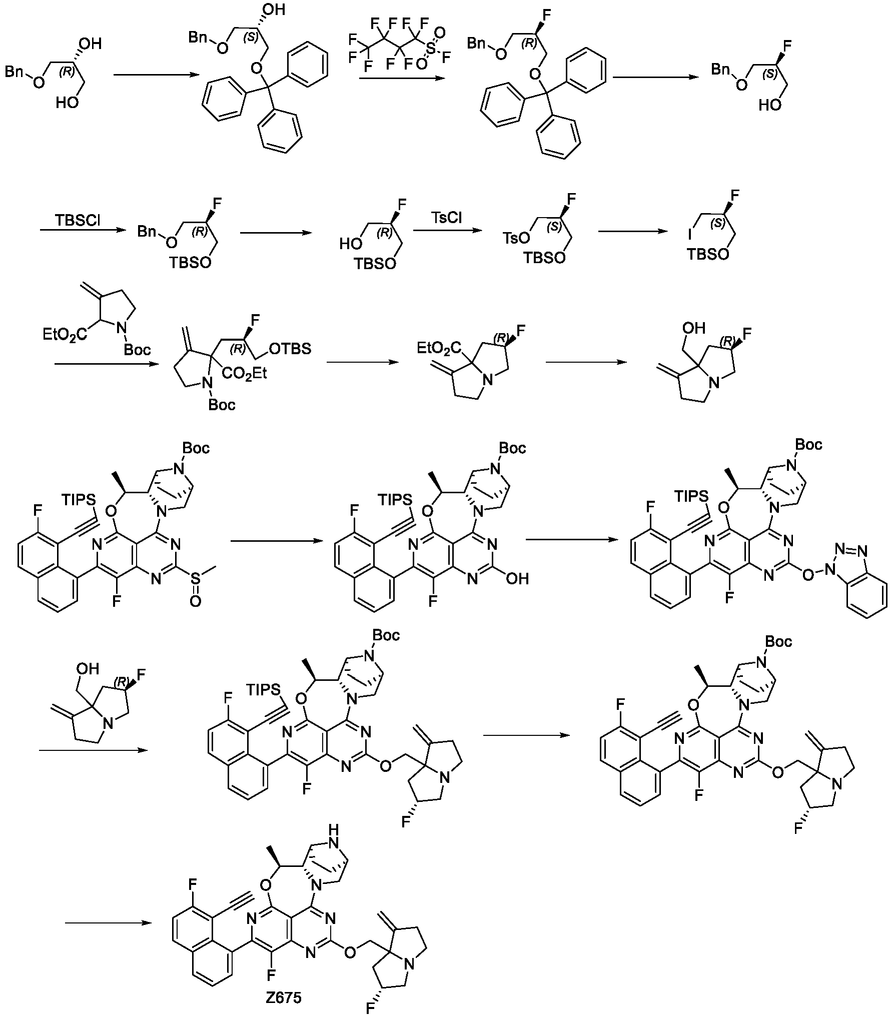

Step I: 4-dimethylaminopyridine (1.01g, 8.23 mmol), triphenylchloromethane (18.36 g, 65.85 mmol), triethyl amine (30.5 mL, 219.51 mmol), (R)-(+)-1-benzylglycerol (10 g, 54.879 mmol) and tetrahydrofuran(300 mL) were sequentially added to a round-bottom flask. Under a nitrogen atmosphere, the reaction was stirred in an oil bath at 65°C for 17 hrs. The reaction was quenched with water, extracted with ethyl acetate (150 mLX2), dried over anhydrous sodium sulfate, filtered and concentrated. The residue was purified through flash silica gel column chromatography (0-10%ethyl acetate /petroleum ether) to obtain (S)-1-(benzyloxy)-3-(trioxy)propan-2-ol (19 g, yield: 81%). ES-API:[M+Na]⁺=447.3.

Step II: At 0°C, (S)-1-(benzyloxy)-3-(trioxy)propan-2-ol (19 g, 44.75 mmol) and toluene (200 mL) were added to a round-bottom flask sequentially. Perfluorobutylsulfonyl floride (14.47 mL, 80.56 mmol) was added dropwise. Then, a solution of 1,8-diazadicyclo[5.4.0]undec-7-ene(12 mL, 80.56 mmol) in toluene (20 mL) was added dropwise. The reaction was stirred at 0°C for 30 min. The reaction solution was heated to room temperature and stirred for 3 hrs. The reaction was quenched with water, extracted with ethyl acetate (150 mLX2), dried over anhydrous sodium sulfate, filtered and concentrated. The residue was purified by flash silica gel column chromatography (0-15%ethyl acetate /petroleum ether) to obtain (R)-((3-(benzyloxy)-2-fluoropropoxy)formyl)triphenyl (15 g, yield: 78%) as a yellow oil. ES-API:[M+Na]⁺=449.2.

Step III: (R)-((3-(benzyloxy)-2-fluoropropoxy)formyl)triphenyl (15 g, 35.17 mmol) in water (20 mL), 1, 4-dioxane (20 mL) and acetic acid (100 mL) were sequentially added to a round-bottom flask. The reaction was stirred at 90°C for 5 hrs. The reaction solution was concentrated, to remove acetic acid. The residue was added with water (100 mL), and extracted with ethyl acetate (100 mLX2). The organic phase was washed with saturated sodium bicarbonate, dried over anhydrous sodium sulfate, filtered and concentrated. The residue was purified by flash silica gel column chromatography (0-35%ethyl acetate /petroleum ether) to obtain (S)-3-(benzyloxy)-2-fluoropropan-1-ol (5 g, yield: 77%) as a yellow oil. ES-API:[M+Na]⁺=207.1.

Step IV: At 0°C, t-butyldimethylchlorosilane (4.91 g, 32.57 mmol) was added to a solution of (S)-3-(benzyloxy)-2-fluoropropan-1-ol (5 g, 27.14 mmol), 4-dimethylaminopyridine(0.33 g, 2.71 mmol) and imidazole (2.77 g, 40.71 mmol) in dichloromethane (90 mL). The reaction was stirred at room temperature for 5 hrs. The reaction was quenched with water (100 mL) and extracted with ethyl acetate (100 mLX2). The organic phase was washed with saturated brine, dried over anhydrous sodium sulfate, filtered, and concentrated. The residue was purified by flash silica gel column chromatography (0-12% ethyl acetate /petroleum ether) to obtain (R)-(3-(benzyloxy)-2-fluoropropoxy)(t-butyl)dimethylsilane (8 g, yield: 98%) as a colorless oil. ES-API:[M+H]⁺=299.1.

Step V: 10% palladium hydroxide on carbon (1g, wetted with 50% water) was added to a solution of (R)-(3-(benzyloxy)-2-fluoropropoxy)(t-butyl)dimethylsilane (8 g, 26.80 mmol) in methanol (30 mL). The reaction was stirred under a hydrogen atmosphere at room temperature for 48 hrs. The reaction solution was filtered through diatomite. The filtrate was concentrated to obtain (R)-3-((t-butyldimethylsilyl)oxy)-2-fluoropropan-1-ol (5.4g, yield: 96%). ES-API:[M+H]⁺=209.2.

Step VI: 4-dimethylaminopyridine(0.63 g, 5.18 mmol), (R)-3-((t-butyldimethylsilyl)oxy)-2-fluoropropan-1-ol (5.4 g, 25.92 mmol), triethyl amine (7.2 mL, 51.84 mmol), and dichloromethane (100 mL) were sequentially added to a round-bottom flask. The reaction solution was cooled to 0°C, and p-toluenesulfonyl chloride (7.41 g, 38.88 mmol) was added. The reaction was stirred at 0°C for 2 hrs. The reaction was quenched with water (60 mL) and 1M hydrochloric acid (60 mL), and extracted with dichloromethane (100 mLX2). The organic phase was washed with saturated sodium bicarbonate, dried over anhydrous sodium sulfate, filtered, and concentrated to obtain (S)-3-((t-butyldimethylsilyl)oxy)-2-fluoropropyl4-methylbenzene sulfonate (9.2 g, yield: 97%). ES-API:[M+H]⁺=363.2.

Step VII: Sodium iodide (19.02 g, 126.89 mmol) was added to a solution of (S)-3-((t-butyldimethylsilyl)oxy)-2-fluoropropyl4-methylbenzene sulfonate (9.2 g, 25.38 mmol) in acetone (100 mL). The reaction was stirred at 75°C for 5 hrs. The reaction solution was concentrated. The residue was added with ethyl acetate (100 mL), and diluted with water (50 mL) and saturated sodium sulfite (30 mL). The reaction solution was extracted with ethyl acetate (100 mL). The organic phase was washed with saturated brine, dried over anhydrous sodium sulfate, filtered, and concentrated. The residue was purified by flash silica gel column chromatography (0-5% ethyl acetate /petroleum ether) to obtain (S)-t-butyl (2-fluoro-3-iodopropoxy)dimethylsilane (7.7 g, yield: 95%). ¹H NMR: (CDCl₃, 400MHz) 4.50-4.35 (m, 1H), 3.80-3.75 (m, 2H), 3.41-3.15 (m, 2H), 0.81 (s, 9H), 0.01(s, 6H).

Step VIII: 1-(t-butyl) 2-ethyl3-methylenepyrrolidin-1,2-dicarboxylate (800 mg, 3.13 mmol) and tetrahydrofuran (15 mL) were added to a round-bottom flask. The reaction solution was cooled to -78°C, and a solution of sodium bis(trimethylsilyl)amide in tetrahydrofuran (6.27 mL, 6.27 mmol) was added dropwise. After that, the reaction solution was stirred at -78°C for 1 hr. A solution of (S)-t-butyl (2-fluoro-3-iodopropoxy)dimethylsilane (1.6 g, 5.02 mmol) in tetrahydrofuran (3 mL) was added dropwise to the reaction solution. After that, the reaction was continuously stirred at -78°C for 30 hrs. The reaction was stirred at room temperature for 2 hrs. The reaction was quenched with a saturated ammonium chloride aqueous solution (100 mL), and extracted with ethyl acetate (100 mLX2). The organic phase was washed with saturated brine, dried over anhydrous sodium sulfate, filtered, and concentrated. The residue was purified by flash silica gel column chromatography (0-8%ethyl acetate /petroleum ether) to obtain 1-(t-butyl) 2-ethyl 2-((R)-3-((t-butyldimethylsilyl)oxy)-2-fluoropropyl)-3-methylenepyrrolidin-1,2-dicarboxylate (700 m g, yield: 50%). ES-API:[M+Na]⁺=468.3.

Step IX: Thionyl chloride (10 mL, 137.86 mmol) was added to 1-(t-butyl) 2-ethyl2-((R)-3-((t-butyldimethylsilyl)oxy)-2-fluoropropyl)-3-methylenepyrrolidin-1,2-dicarboxylate (700 mg, 1.57 mmol). The reaction was stirred at 45°C for 24 hrs. The reaction solution was added with thionyl chloride (10 mL, 137.86 mmol). The reaction was stirred at 65°C for 2 days. The reaction solution was concentrated. The residue was added with dichloromethane (5 mL), and cooled to 0°C. Triethyl amine (0.22 mL, 1.57 mmol) was added. The reaction was stirred at room temperature for 30 min. The reaction was quenched with water (50 mL), and extracted with dichloromethane (30 mLX2). The organic phase was washed with saturated brine (30 mL), dried over anhydrous sodium sulfate, filtered and concentrated. The residue was purified by flash silica gel column chromatography (0-60%ethyl acetate /petroleum ether) to obtain ethyl (6R)-6-fluoro-1-methylenetetrahydro-1H-pyrrolizin-7a(5H)-carboxylate (170 mg, yield: 50%). ES-API:[M+H]₊=214.2.

Step X: A lithium aluminium hydride /tetrahydrofuran solution (1.59 mL, 1.59 mmol) was added dropwise to a solution of ethyl (6R)-6-fluoro-1-methylenetetrahydro-1H-pyrrolizin-7a(5H)-carboxylate (170 mg, 0.80 mmol) in tetrahydrofuran (5 mL), and stirred at 0°C for 1 hr. The reaction was quenched by sequentially adding 3 drops of water and a 15% sodium hydroxide aqueous solution (0.1 mL). The mixture was stirred at room temperature for 30 min, and filtered through diatomite. The filtrate was concentrated to obtain a crude product that was ((6R)-6-fluoro-1-methylenetetrahydro-1H-pyrrolizin-7a(SH)-yl)methanol (130 mg, yield: 95%) as a yellow oil. ES-API: [M+H]⁺=172.1.

Step XI: T-butyl (5S, 5aS, 6S, 9R)-1-fluoro-2-(7-fluoro-8-((triisopropylsilyl)ethynyl) naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (400 mg, 0.51 mmol) and tetrahydrofuran(8 mL) were added to a potassium hydroxide (142mg, 2.54 mmol) aqueous solution (3 mL). The reaction was stirred at 60°C for 1 hr, and quenched with water (50 mL). The reaction solution was extracted with ethyl acetate (100 mL). The organic phase was washed with saturated brine, dried over anhydrous sodium sulfate, filtered, and concentrated to obtain t-butyl (5S, 5aS, 6S, 9R)-1-fluoro-2-(7-fluoro-8-((triisopropylsilyl)ethynyl)naphthalen-1-yl)-12-hydroxyl -5-methyl-5a,6,7,8,9,10-hexahydro-5H-4-oxy-3,10a,11,13,14-pentaaza-6,9-methanonaphtho [1,8-ab]hepten-14-carboxylate (376mg, yield: 99%). ES-API: [M+H]⁺=742.3.

Step XII: BOP reagent (448 mg, 1.01 mmol) was added to t-butyl (5S, 5aS, 6S, 9R)-1-fluoro-2-(7-fluoro-8-((triisopropylsilyl)ethynyl)naphthalen-1-yl)-12-hydroxyl-5-methyl-5a, 6,7,8,9,10-hexahydro-5H-4-oxa-3,10a,11,13,14-pentaaza-6,9-methanonaphtho[1,8-ab]hepten-14-carboxylate (376 mg, 0.51 mmol), cesium carbonate (330 mg, 1.01 mmol) and tetrahydrofuran (15 mL). The reaction was stirred at room temperature for 1 hrs, quenched with water (50 mL), and extracted with ethyl acetate (100 mL). The organic phase was washed with saturated brine, dried over anhydrous sodium sulfate, filtered, and concentrated. The residue was purified by flash silica gel column chromatography (0-50%ethyl acetate /petroleum ether) to obtain t-butyl (5S, 5aS, 6S, 9R)-12-(1H-benzo[d][1,2,3]triazole-1-yl)oxy)-1-fluoro-2-(7-fluoro-8-((triisopropylsilyl)ethynyl) naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (390mg, yield: 89%). ES-API: [M+H]⁺=859.2.

Step XIII: Sodium hydride (21 mg, 0.52 mmol) and tetrahydrofuran(8 mL) were added to a round-bottom flask. The reaction solution was cooled to 0°C, and a solution of ((6R)-6-fluoro-1-methylenetetrahydro-1H-pyrrolizin-7a(5H)-yl)methanol (60 mg, 0.35 mmol) in anhydrous tetrahydrofuran (2 mL) was added, and reacted at room temperature for 10 min. The reaction solution was cooled to 0°C. T-butyl (5S, 5aS, 6S, 9R)-12-(1H-benzo[d][1,2,3]triazole-1-yl)oxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (150 mg, 0.17 mmol) was added to the reaction solution, and reacted at room temperature for 1 hr. The reaction solution was added with water (30 mL), and The reaction solution was extracted with ethyl acetate (20 mLX2). The organic phase was washed with saturated brine (30 mL), dried over anhydrous sodium sulfate, filtered, and concentrated to obtain t-butyl (5S,5aS,6S,9R)-1-fluoro-12-((6R)-6-fluoro-1-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (150 mg, yield: 95%). ES-API: [M+H]⁺=895.3.

Step XIV: Cesium fluoride (509 mg, 3.35 mmol) was added to t-butyl (5S, 5aS, 6S, 9R)-1-fluoro-12-((6R)-6-fluoro-1-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (150 mg, crude product) in N,N-dimethylformamide (5 mL). The reaction mixture was stirred at room temperature for 2 hr. The reaction was shown to be completed by LC/MS. The reaction solution was diluted with ethyl acetate (100 mL), and washed with water (30 mL) and saturated brine (30 mLX2). The organic phase, dried over anhydrous sodium sulfate, filtered and , concentrated. The residue was purified by flash silica gel column chromatography (0-5%methanol /dichloromethane) to obtain t-butyl (5S, 5aS, 6S, 9R)-2-(8-ethynyl-7-fluoronaphthalen-1-yl)-1-fluoro-12-((6R)-6-fluoro-1-methylenetetrahydro-1H-pyrrolizin-7a(5H)-yl)methoxy)-5-methyl-5a, 6,7,8,9,10-hexahydro-5H-4-oxa-3,10a,11,13,14-pentaaza-6,9-methanonaphtho[1,8-ab]hepten-14-carboxylate (120 mg, yield: 96%). ES-API: [M+H]⁺= 739.2.

Step XV: In an iced water bath, 4 M hydrochloric acid-dioxane solution (3 mL) was slowly added to a solution of t-butyl (5S, 5aS, 6S, 9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (120 mg, 0.16mmol) in acetonitrile (2 mL). The reaction mixture was stirred at room temperature for 1 hr. The reaction was shown to be completed by LC/MS. The reaction solution was concentrated to dryness under reduced pressure, and the residue was dissolved in saturated sodium bicarbonate aqueous solution and dichloromethane. The reaction solution was extracted with dichloromethane/methanol (10: 1, 50mL). The organic phase was washed with saturated brine (30 mL), dried over anhydrous sodium sulfate, filtered, and concentrated to obtain (5S,5aS,6S,9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z675, 80mg, yield: 77%) as a yellow solid. ES-API: [M+H]⁺= 639.2.

### Example 67: Synthesis of 5-cyclopropyl-6-(5S, 5aS, 6S, 9R)-1-fluoro-12-(((2R)-2-fluoro-6-methylenetetrahydro1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-4-methylpyridine-2-amine (Z676)

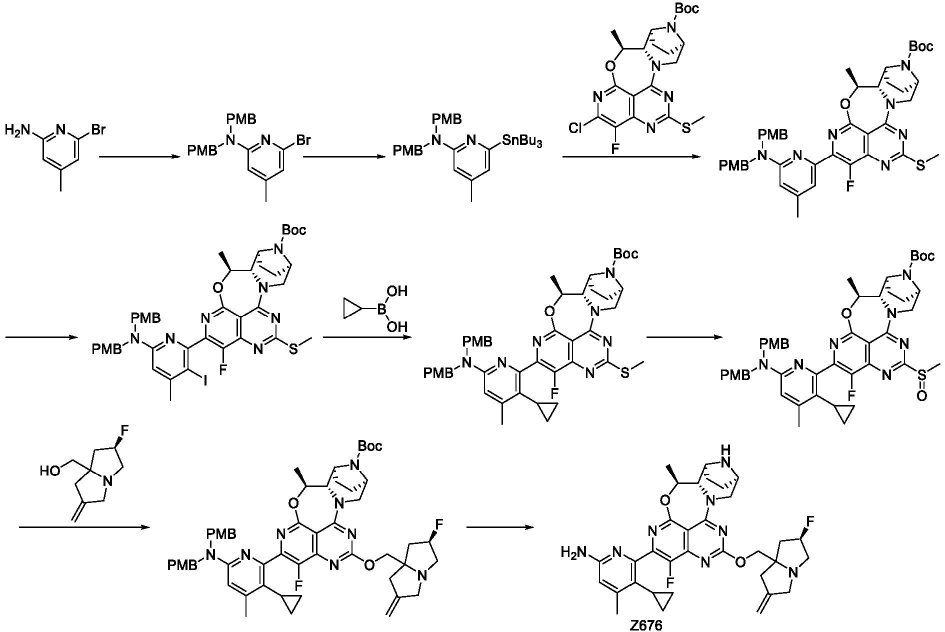

Step I: 6-bromo-4-methylpyridine-2-amine (2 g, 10.69 mmol) was dissolved in N,N-dimethylformamide (40 mL). 60% sodium hydride ((2.14 g, 53.46 mmol) was added at 0°C, and the reaction was stirred at room temperature for 1 hr. Then p-methoxybenzyl chloride (3.68 g, 23.52 mmol) was added dropwise, and the reaction was stirred at room temperature for 2 hrs. The reaction solution was quenched with a saturated ammonium chloride solution (50 mL), and extracted with ethyl acetate (100 mL). The organic phase was washed with saturated brine (50 mL×3), dried over anhydrous sodium sulfate, and filtered. The filtrate was concentrated under reduced pressure. The crude product was purified by flash silica gel column chromatography (ethyl acetate /petroleum ether: 0-15%) to obtain the target product 6-bromo-N, N-bis(4-methoxybenzyl)-4-methylpyridine-2-amine (3.8 g, yield: 83.2%) as a white solid. ES-API: [M+H]⁺= 427.1, 428.9.

Step II: 6-bromo-N, N-bis(4-methoxybenzyl)-4-methylpyridine-2-amine (1.20 g, 2.81 mmol), hexabutylditin (4.89 g, 8.42 mmol), tris(dibenzylideneacetone) dipalladium (257 mg, 0.28 mmol), tricyclohexyl phosphine (157 mg, 0.56 mmol), lithium chloride (595 mg, 14.04 mmol) and 1,4-dioxane (20 mL) were added to a 100 mL round-bottom flask, purged 3 times with nitrogen, and reacted at 110°C for 5 hrs. The cooled reaction solution was concentrated under reduce pressure. The crude product was purified by flash silica gel column chromatography (ethyl acetate /petroleum ether: 0-10%) to obtain the target product N, N-bis(4-methoxybenzyl)-4-methyl-6-(tributyltin)pyridine-2-amine (1.60 g, yield: 89.4%) as a light yellow solidd. ES-API: [M+H]⁺=639.3.

Step III: T-butyl (5S,5aS,6S,9R)-2-chloro-1-fluoro-5-methyl-12-(2-methylthio)-5a,6,7,8,9,10-hexahydro-5H-4-oxy-3,10a,11,13,14-pentaaza-6,9-methanonaphtho[1,8-ab]hepten-14-carboxylate (450 mg, 0.93 mmol), N, N-bis(4-methoxybenzyl)-4-methyl-6-(tributyltin)pyridine-2-amine (1.20 g, 1.88 mmol), cuprous iodide (178 mg, 0.93 mmol), lithium chloride (158 mg, 3.74 mmol) and N,N-dimethylacetamide (20 mL) were added to a 100 mL round-bottom flask, purged 3 times with nitrogen, and reacted at 120°C for 3 hrs. The cooled reaction solution was added with ethyl acetate (60 mL), and sequentially washed with dilute brine (30 mL×3), and saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-10%) to obtain the target product t-butyl (5S, 5aS, 6S, 9R)-2-(6-(bis(4-methoxybenzyl)amino)-4-methylpyridine-2-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (500 mg, yield: 67.4%) as an off-white solid. ES-API: [M+H]⁺=794.2.

Step IV: T-butyl (5S, 5aS, 6S, 9R)-2-(6-(bis(4-methoxybenzyl)amino)-4-methylpyridine-2-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (450 mg, 0.57 mmol) and p-toluenesulfonic acid monohydrate (11 mg, 0.057 mmol) were dissolved in N, N-dimethylformamide (10 mL). N-iodosuccinimide (638 mg, 2.83 mmol) was added at room temperature, and the reaction was stirred at room temperature for 2 hrs. The reaction solution was added with ethyl acetate (60 mL), and sequentially washed with saturated sodium thiosulphate (40 mL) and saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5S, 5aS, 6S, 9R)-2-(6-(bis(4-methoxybenzyl)amino)-3-iodo-4-methylpyridine-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (450 mg, yield: 86.37%) as a yellow solid. ES-API: [M+H]⁺=920.0.

Step V: Potassium phosphate (277 mg, 1.31 mmol), 2-bicyclohexylphosphino-2',6'-dimethoxybiphenyl (36 mg, 0.088 mmol) and palladium acetate (10 mg, 0.043 mmol) were added to t-butyl (5S, 5aS, 6S, 9R)-2-(6-(bis(4-methoxybenzyl)amino)-3-iodo-4-methylpyridine-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (400 mg, 0.44 mmol) and cyclopropylboronic acid (187 mg, 2.17 mmol) in toluene (10 mL) and water (1 mL), purged 3 times with nitrogen, and reacted at 105°C for 3 hrs. The reaction solution was added with ethyl acetate (60 mL), and washed with saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5S, 5aS, 6S, 9R)-2-(6-(bis(4-methoxybenzyl)amino)-3-cyclopropyl-4-methylpyridine-2-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (350 mg, yield: 96.5%) as a light yellow solid. ES-API: [M+H]⁺=834.2.

Step VI: T-butyl (5S, 5aS, 6S, 9R)-2-(6-(bis(4-methoxybenzyl)amino)-3-cyclopropyl-4-methylpyridine-2-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (250 mg, 0.30 mmol) was dissolved in dichloromethane (10 mL). m-chloroperoxybenzoic acid (91 mg, 0.45 mmol) was added at room temperature, and the reaction was stirred at room temperature for 1 hr. The reaction solution was added with dichloromethane (50 mL), sequentially washed with saturated sodium thiosulphate (15 mL), saturated sodium bicarbonate (15 mL), and saturated brine (30 mL). The organic phase was dried over anhydrous sodium sulfate, and filtered. The filtrate was concentrated under reduced pressure, to obtain the target product t-butyl (5S, 5aS, 6S, 9R)-2-(6-(bis(4-methoxybenzyl)amino)-3-cyclopropyl-4-methylpyridine-2-yl)-1-fluoro-5-methyl-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (250 mg, yield: 98.1%) as a white solid. ES-API:[M+H]⁺=850.2.

Step VII: T-butyl (5S, 5aS, 6S, 9R)-2-(6-(bis(4-methoxybenzyl)amino)-3-cyclopropyl-4-methylpyridine-2-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (250 mg, 0.29 mmol) and ((2R)-2-fluoro-6-methylenetetrahydro-1H-pyrrolizin-7a(5H)-yl)methanol (76 mg, 0.44mmol) were dissolved in tetrahydrofuran (10mL). A 1.3M lithium bis(trimethylsilyl)amide solution in tetrahydrofuran (0.45 mL, 0.59 mmol) was added dropwise at 0°C, and reacted with stirring at this temperature for 30 min. The reaction solution was added with ethyl acetate (60 mL), and sequentially added with water (20 mL) and saturated brine (30 mL). The organic phase was dried over anhydrous sodium sulfate, and filtered. The filtrate was concentrated under reduced pressure. The crude product was purified by preparative T1C (dichloromethane/7.0Maminomethanol =25: 1) to obtain the target product t-butyl (5S, 5aS, 6S, 9R)-2-(6-(bis(4-methoxybenzyl)amino)-3-cyclopropyl-4-methylpyridine-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (80 mg, yield: 28.4%) as a light yellow solid. ES-API: [M+H]⁺=957.3.

Step VIII: T-butyl (5S, 5aS, 6S, 9R)-2-(6-(bis(4-methoxybenzyl)amino)-3-cyclopropyl-4-methylpyridine-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (75 mg, 0.078 mmol) was dissolved in trifluoroacetic acid (4 mL), and reacted with stirring at 50°C for 2 hrs. The reaction solution was concentrated under reduced pressure to dryness. Dichloromethane (5 mL) and 7.0M aminomethanol (5 mL) were added, and concentrated under reduced pressure. The crude product was purified by flash silica gel column chromatography (dichloromethane/7.0M aminomethanol: 0-15%) to obtain a target product that was 5-cyclopropyl-6-(5S, 5aS, 6S, 9R)-1-fluoro-12-(((2R)-2-fluoro-6-methylenetetrahydro1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-4-methylpyridine-2-amine (Z676, 35 mg, yield: 72.4%). ES-API: [M+H]⁺= 617.2.

### Example 68: Synthesis of (5S, 5aS, 6S, 9R)-2-(8-ethynylnaphthalen-1-yl)-1-fluoro-5-methyl-12-((R)-1-methylenetetrahydro-1H-pyrrolazin-7a(5H)-yl)methoxy)-5a,6,7,8,9,10-hexahydro-5H-4-oxa-3,10a,11,13,14-pentaaza-6,9-methylenenaphthal[1,8-ab]heptene (Z677) and (5S, 5aS, 6S, 9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z702)

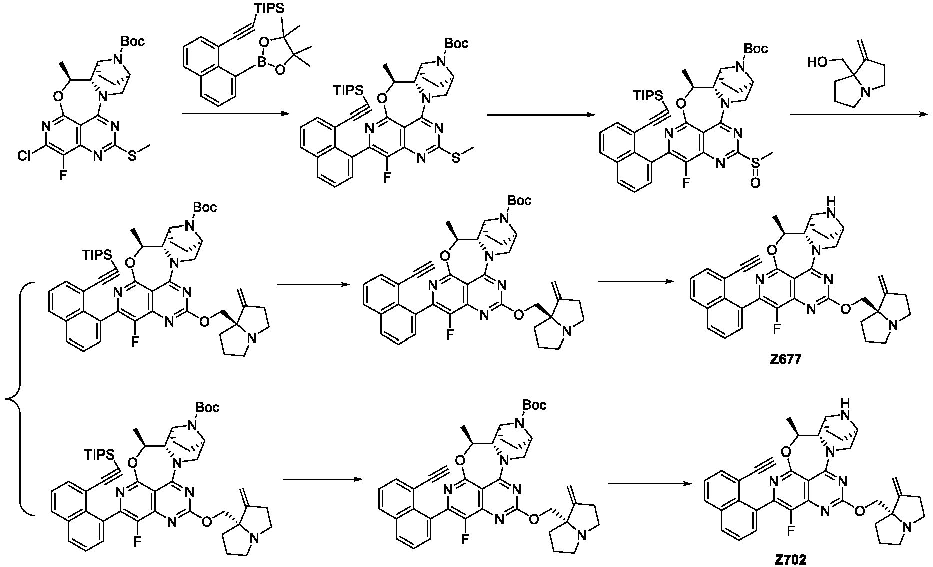

Step I: Under a nitrogen atmosphere, t-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (100 mg, 0.207 mmol), triisopropyl((8-(4,4,5,5-tetramethyl-1,3,2-dioxaborolan-2-yl)naphthalen-1-yl)ethynyl)silane (180 mg, 0.415 mmol), potassium phosphate (132 mg, 0.622 mmol) and [n-butyldi(1-adamantanyl)phosphine](2-amino-1,1'-biphenyl-2-yl)palladium (II) mesylate (23 mg, 0.031 mmol) were dissolved in tetrahydrofuran(12 mL) and water (2.5 mL). The reaction mixture was heated to 80°C in a sealed tube and stirred for 3 hrs. The reaction was shown to be completed by LC/MS. The reaction solution was concentrated and the crude product was purified by automated flash silica gel chromatography (petroleum ether/ethyl acetate = 0 - 20%), to obtain t-butyl (5S, 5aS, 6S, 9R)-1-fluoro-5-methyl-12-(methylthio)-2-(8-((triisopropylsilyl)ethynyl)naphthalen-1-yl)-5a, 6,7,8,9,10-hexahydro-5H-4-oxa-3,10a,11,13,14-pentaaza-6,9-methanonaphtho[1,8-ab]hepten-14-carboxylate (156 mg, yield: 99%). ES-API: [M+H]⁺= 754.3.

Step II: T-butyl (5S, 5aS, 6S, 9R)-1-fluoro-5-methyl-12-(methylthio)-2-(8-((triisopropylsilyl) ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (156 mg, 0.207 mmol) was dissolved in anhydrous dichloromethane (10 mL). m-chloroperoxybenzoic acid (54 mg, 0.310 mmol) was added to the solution, and the reaction solution was stirred at room temperature for 0.5 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S, 5aS, 6S, 9R)-1-fluoro-5-methyl-12-(methylthionyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (159 mg). ES-API: [M+ 1]⁺= 770.3.

Step III: (1-methylenetetrahydro-1H-pyrrolizin-7a(5H)-yl)methanol (63 mg, 0.413 mmol) was dissolved in anhydrous tetrahydrofuran (5 mL). In an iced water bath, sodium hydride (25 mg, 0.619 mmol) was added, and reacted for 10 min in the iced water bath. A solution of t-butyl (5S, 5aS, 6S, 9R)-1-fluoro-5-methyl-12-(methylthionyl)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (159 mg, 0.206 mmol) in tetrahydrofuran (5 mL) was added dropwise to the reaction solution, and reacted at 0 °C for 30 min. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure. The crude product was purified by automated flash silica gel chromatography (methanol /dichloromethane= 0 - 3%) to obtain two isomeric compounds. One isomer structure was arbitrarily designated as t-butyl (5S, 5aS, 6S, 9R)-1-fluoro-5-methyl-12-((R)-1-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a, 11,13,14-pentaaza-6,9-methanonaphtho[1,8-ab]hepten-14-carboxylate (47 mg, yield: 27%). ES-API: [M+ H]⁺= 859.4. (retention time: 1.787 min, analytical method: '1-POS-3MIN-2.7', mobile phase: A: water (0.05% trifluoroacetic acid ) B: acetonitrile (0.05% trifluoroacetic acid ), gradient: 5%-95% B in 1.6min, flow rate: 1.8 mL/min, chromatographic column: SunFire C18,4.6*50mm,3.5um, column temperature: 40°C). The other isomer structure was arbitrarily designated as t-butyl (5S, 5aS, 6S, 9R)-1-fluoro-5-methyl-12-((S)-1-methylenetetrahydro-1H-pyrrolizin-7a(5H-yl)methoxy) -2-(8-(triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (72 mg, yield: 41%). ES-API: [M+ H]⁺= 859.4 (retention time: 1.749 min, analytical method: '1-POS-3MIN-2.7', mobile phase: A: water (0.05%trifluoroacetic acid ) B: acetonitrile (0.05% trifluoroacetic acid ), gradient: 5%-95% B in 1.6min, flow rate: 1.8 mL/min, chromatographic column: SunFire C18,4.6 * 50mm,3.5um, column temperature: 40°C).

Step IV: Cesium fluoride (83 mg, 0.547 mmol) was added to t-butyl (5S, 5aS, 6S, 9R)-1-fluoro-5-methyl-12-((R)-1-methylenetetrahydro-1H-pyrrolizin-7a(5H)-yl)methoxy)-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (47 mg, 0.055 mmol) in N, N-dimethylformamide (2 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX 3), dried over anhydrous sodium sulfate, filtered and concentrated. The crude product was purified by automated flash silica gel chromatography (methanol /dichloromethane= 0 - 5%) to obtain t-butyl (5S, 5aS, 6S, 9R)-2-(8-ethynylnaphthalen-1-yl)-1-fluoro-5-methyl-12-((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (28 mg, yield: 73%). ES-API: [M+ H]⁺= 703.3.

Step V: In an iced water bath, a 4 M hydrochloric acid-dioxane solution (3 mL) was slowly added to a solution of t-butyl (5S, 5aS, 6S, 9R)-2-(8-ethynylnaphthalen-1-yl)-1-fluoro-5-methyl-12-((R)-1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]hepten-14-carboxylate (28 mg, 0.040 mmol) in acetonitrile (2 mL). The reaction mixture was stirred at 0°C for 1 hr. The reaction was shown to be completed by LC/MS. The reaction solution was concentrated to dryness under reduced pressure. The residue was added with a saturated sodium bicarbonate solution (3 mL) until the pH of the solution was > 7.0, and then extracted with dichloromethane (5 mLX 3). The organic phases were combined and concentrated under reduced pressure, to obtain (5S, 5aS, 6S, 9R)-2-(8-ethynylnaphthalen-1-yl)-1-fluoro-5-methyl-12-((R)-1-methylenetetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]heptene (Z677, 9 mg, yield: 37%) as an off-white solid. ES-API: [M+H]⁺= 603.3.

Step VI: Cesium fluoride (127 mg, 0.838 mmol) was added to t-butyl (5S, 5aS, 6S, 9R)-1-fluoro-5-methyl-12-((S)-1-methylenetetrahydro-1H-pyrrolizin-7a(5H-yl)methoxy)-2-(8-(triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (72 mg, 0.084 mmol) in N,N-dimethylformamide (2 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX 3), dried over anhydrous sodium sulfate, filtered and concentrated. The crude product was purified by automated flash silical gel chromatography (methanol /dichloromethane= 0 - 5%) to obtain t-butyl (5S, 5aS, 6S,9R)-2-(8-ethynylnaphthalen-1-yl)-1-fluoro-5-methyl-12-((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (43 mg, yield: 73%). ES-API: [M+ H]⁺= 703.3.

Step VII: In an iced water bath, a 4 M hydrochloric acid-dioxane solution (3 mL) was slowly added to a solution of t-butyl (5S, 5aS, 6S, 9R)-2-(8-ethynylnaphthalen-1-yl)-1-fluoro-5-methyl-12-((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-nonaphtho[1,8-ab]hepten-14-carboxylate (43 mg, 0.061 mmol) in acetonitrile (2 mL). The reaction mixture was stirred at 0 °C for 1 hr. The reaction was shown to be completed by LC/MS. The reaction solution was concentrated to dryness under reduced pressure. The residue was added with a saturated sodium bicarbonate solution (3 mL) until the pH of the solution was > 7.0, and then extracted with dichloromethane (5 mLX3). The organic phases were combined and concentrated under reduced pressure to obtain (5S, 5aS, 6S, 9R)-2-(8-ethynylnaphthalen-1-yl)-1-fluoro-5-methyl-12-(((S)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z702, 29 mg, yield: 79%) as an off-white solid. ES-API: [M+H]⁺= 603.3.

### Example 69: Synthesis of (5S, 5aS, 6S, 9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z678)

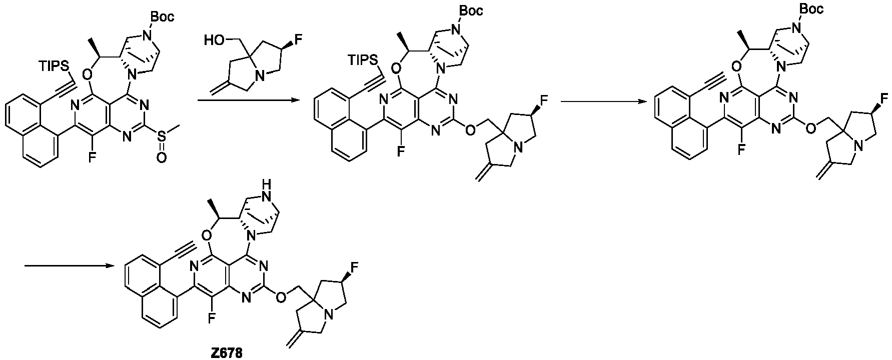

Step I: ((2R)-2-fluoro-6-methylenetetrahydro-1H-pyrrolizin-7a(5H)-yl)methanol (62 mg, 0.362 mmol) was dissolved in anhydrous tetrahydrofuran (5 mL). In an iced water bath, sodium hydride (25 mg, 0.619 mmol) was added, and reacted for 10 min in the iced water bath. A solution of t-butyl (5S, 5aS, 6S, 9R)-1-fluoro-5-methyl-12-(methylthionyl)-2-(8-((triisopropylsilyl)ethynyl) naphthalen-1-yl)-5a,6,7,8,9,10-hexahydro-5H-4-oxa-3,10a,11,13,14-pentaaza-6,9-methanonaphtho[1,8-ab]hepten-14-carboxylate (159 mg, 0.206 mmol) in tetrahydrofuran (5 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure. The crude product was purified by automated flash silica gel chromatography (methanol /dichloromethane= 0 - 3%) to obtain t-butyl (5S, 5aS, 6S, 9R)-1-fluoro-12-((2R)-2-fluoro-6-methylenetetrahydro-1H-pyrrolizin-7a(5H)-ylmethoxy)-5-methyl-2-(8-((triisopropylsilyl)ethynyl)naphthalen-1-yl)-5a,6,7,8,9,10-hexahydro-5H-4-oxa-3,10a,11,13,14-pentaaza-6,9-methanonaphtho[1,8-ab]hepten-14-carboxylate (88 mg, yield: 49%). ES-API: [M+ H]⁺= 877.3.

Step II: Cesium fluoride (152 mg, 1.003 mmol) was added to t-butyl (5S, 5aS, 6S, 9R)-1-fluoro-12-((2R)-2-fluoro-6-methylenetetrahydro-1H-pyrrolizin-7a(5H)-ylmethoxy)-5-methyl-2-(8-(triisopropylsilyl)ethynyl)naphthalen-1-yl)-5a,6,7,8,9,10-hexahydro-5H-4-oxa-3,10a, 11,13,14-pentaaza-6,9-methanonaphtho[1,8-ab]hepten-14-carboxylate (88 mg, 0.100 mmol) in N,N-dimethylformamide (3 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), and dried over anhydrous sodium sulfate. The reaction was filtered and concentrated. The crude product was purified by TLC (dichloromethane/methanol = 15: 1) to obtain t-butyl (5S, 5aS, 6S, 9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (68 mg, yield: 94%). ES-API: [M+ H]⁺= 721.2.

Step III: In an iced water bath, a 4 M hydrochloric acid-dioxane solution (3 mL) was slowly added to a solution of t-butyl (5S, 5aS, 6S, 9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (68 mg, 0.094 mmol) in acetonitrile (3 mL). The reaction mixture was stirred at 0°C for 1 hr. The reaction was shown to be completed by LC/MS. The reaction solution was concentrated to dryness under reduced pressure. The residue was added with a saturated sodium bicarbonate solution (3 mL) until the pH of the solution was > 7.0, and then extracted with dichloromethane (5 mLX3). The organic phases were combined and concentrated under reduced pressure, to obtain (5S, 5aS, 6S, 9R)-2-(8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z678, 44 mg, yield: 75%) as an off-white solid. ES-API: [M+H]⁺= 621.3. ¹H NMR (400 MHz, CD₃OD) δ 8.08-8.01 (m, 2 H), 7.77-7.48 (m, 4 H), 5.44-5.27 (m, 2 H), 5.02 (s, 2 H), 4.59-4.54 (m, 1 H), 4.43-4.38 (m, 1 H), 4.30 (d, J= 13.0 Hz, 1 H), 4.15 (d, J= 10.5 Hz, 1 H), 3.82-3.73 (m, 3 H), 3.70-3.36 (m, 3 H), 3.28-3.15 (m, 2 H), 3.01-2.85 (m, 2 H), 2.55-2.46 (m, 2 H), 2.23-2.05 (m, 2 H), 1.98-1.85 (m, 3 H), 1.58 (t, J= 8.5 Hz, 3 H).

### Example 70: Synthesis of 2-(8-ethynyl-7-fluoronaphthalen-1-yl)-1-fluoro-12-((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptene (Z679)

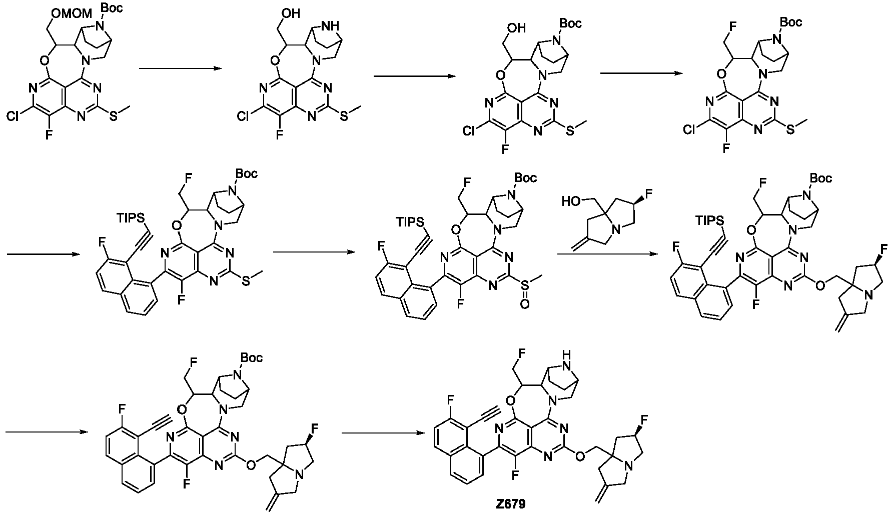

Step I: In an iced water bath, t-butyl 2-chloro-1-fluoro-5-((methoxymethoxy)methyl)-12-(methylthio)-5a,6,7,8,9,10-hexahydro-5H-4-oxa-3,10a,11,13,14-pentaaza-6,9-methanonaphtho[1,8-ab]hepten-14-carboxylate (148.5 mg, 0.275 mmol) was dissolved in a hydrochloric acid-methanol solution (10 mL, 4 M), and the reaction solution was stirred at 0°C for 3 hrs. The reaction was shown to be completed by LC/MS, and concentrated under reduced pressure. to obtain the crude product (2-chloro-1-fluoro-12-(methylthio)-5a,6,7,8,9,10-hexahydro-5H-4-oxa-3,10a,11,13,14-pentaaza-6,9-methanonaphtho[1,8-ab]hepten-5-yl)methanol (104.5 mg). ES-API: [M+ H]⁺= 398.0.

Step II: In an iced water bath, (2-chloro-1-fluoro-12-(methylthio)-5a,6,7,8,9,10-hexahydro-5H-4-oxa-3,10a,11,13,14-pentaaza-6,9-methanonaphtho[1,8-ab]hepten-5-yl)methanol (100 mg, 0.250 mmol) was dissolved in dichloromethane(20 mL). he reaction solution was added with di-t-butyl dicarbonate (165 mg, 0.755 mmol) and N,N-diisopropylethyl amine (195 mg, 1.510 mmol), and the reaction solution was stirred at 0°C for 3 hrs. The reaction was shown to be completed by LC/MS. The reaction solution was concentrated and the crude product was purified by automated flash silica gel chromatography (ethyl acetate: petroleum ether= 0- 20%) to obtain t-butyl - chloro-1-fluoro-5-(hydroxymethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (125 mg, yield: 99%). ES-API: [M+ H]⁺= 498.1.

Step III: In an iced water bath, t-butyl -chloro-1-fluoro-5-(hydroxymethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (125 mg, 0.250 mmol) was dissolved in dichloromethane (25 mL). Diethylaminosulfur trifluoride (160 mg, 1.005 mmol) was slowly added dropwise to the reaction solution, and the reaction solution was stirred at 0°C for 3 hrs. The reaction was shown to be completed by LC/MS. The reaction solution was diluted with dichloromethane (20 mL), washed with a saturated sodium bicarbonate solution (20 mLX 2) and saturated brine (20 mLX2), dried over anhydrous sodium sulfate, filtered, and concentrated to obtain the crude product t-butyl 2-chloro-1-fluoro-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (120 mg). ES-API: [M+ H]⁺= 500.0.

Step IV: Under a nitrogen atmosphere, t-butyl 2-chloro-1-fluoro-5-(fluoromethyl)-12-(methylthio)-5a,6,7,8,9,10-hexahydro-5H-4-oxy-3,10a,11,13,14-pentaaza-6,9-methanonaphtho [1,8-ab]hepten-14-carboxylate (100 mg, 0.200 mmol), {[2-fluoro-8-(4,4,5,5-tetramethyl-1,3,2-dioxacyclopropane-2-yl)-1-naphthalenyl]ethynyl}[3(prop-2-yl)]silane (226 mg, 0.500 mmol), potassium phosphate (127 mg, 0.600 mmol) and [n-butyldi(1-adamantanyl)phosphine](2-amino-1,1'-biphenyl-2-yl)palladium (II) mesylate (30 mg, 0.040 mmol) were dissolved in tetrahydrofuran (12 mL) and water (2.5 mL). The reaction mixture was stirred at 80°C in a microwave generator for 0.5 hr. The reaction was shown to be completed by LC/MS. The reaction solution was concentrated and the crude product was purified by automated flash silica gel chromatography (petroleum ether/ethyl acetate = 0 - 20%), to obtain t-butyl 1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (60 mg, yield: 38%). ES-API: [M+ H]⁺= 790.2.

Step V: T-butyl 1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (60 mg, 0.076 mmol) was dissolved in anhydrous dichloromethane (10 mL). m-chloroperoxybenzoic acid (20 mg, 0.114 mmol) was added to the solution, and the reaction solution was stirred at room temperature for 0.5 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl 1-fluoro-2-(7-fluoro-8-((triisopropylsilyl)ethynyl)naphthalen-1-yl)-5-(fluoromethyl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (61 mg). ES-API: [M+ H]⁺= 822.1.

Step VI: ((2R)-2-fluoro-6-methylenetetrahydro-1H-pyrrolizin-7a(5H)-yl)methanol (26 mg, 0.152 mmol) was dissolved in anhydrous tetrahydrofuran(5 mL). In an iced water bath, sodium hydride (9 mg, 0.228 mmol) was added, and reacted for 10 min in the iced water bath. A solution of t-butyl1-fluoro-2-(7-fluoro-8-((triisopropylsilyl)ethynyl)naphthalen-1-yl)-5-(fluoromethyl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (61 mg, 0.076 mmol) in tetrahydrofuran (5 mL) was added dropwise to the reaction solution, and reacted for 30 min at 0°C. The reaction was shown to be completed by LC/MS. The reaction solution was diluted with ethyl acetate (10 mL), washed with a saturated ammonium chloride solution (10 mLX 2) and saturated brine (10 mLX 3), dried over anhydrous sodium sulfate, filtered, and concentrated under reduced pressure. The crude product was purified by automated flash silica gel chromatography (methanol /dichloromethane= 0 - 3%) to obtain t-butyl 1-fluoro-12-((2R)-2-fluoro-6-methylenetetrahydro-1H-pyrrolizin-7a(5H)-ylmethoxy)-2-(7-fluoro-8-((triisopropylsilyl)ethynyl)naphthalen-1-yl)-5-(fluoromethyl)-5a,6,7,8,9,10-hexahydro-5H-4-oxa-3,10a,11,13,14-pentaaza-6,9-methanonaphtho[1,8-ab]hepten-14-carboxylate (69 mg, yield: 99%). ES-API: [M+ H]⁺= 913.3.

Step VII: Cesium fluoride (115 mg, 0.756 mmol) was added to t-butyl 1-fluoro-12-((2R)-2-fluoro-6-methylenetetrahydro-1H-pyrrolizin-7a(5H)-ylmethoxy)-2-(7-fluoro-8-((triisopropylsilyl)ethynyl)naphthalen-1-yl)-5-(fluoromethyl)-5a,6,7,8,9,10-hexahydro-5H-4-oxa-3,10a,11,13,14-pentaaza-6,9-methanonaphtho[1,8-ab]hepten-14-carboxylate (69 mg, 0.076 mmol) in N,N-dimethylformamide (5 mL), and the reaction mixture was stirred at room temperature for 1 hr. The reaction was shown to be completed by LC/MS. The reaction solution was diluted with ethyl acetate (15 mL), washed with water (15 mLX2) and saturated brine (15 mLX3), and dried over anhydrous sodium sulfate. The reaction was filtered and concentrated. The crude product was purified by TLC (dichloromethane/methanol = 50: 1) to obtain the crude product t-butyl 2-(8-ethynyl-7-fluoronaphthalen-1-yl)-1-fluoro-12-((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]hepten-14-carboxylate (60 mg). ES-API: [M+ H]⁺= 757.2.

Step VIII: In an iced water bath, 4 M hydrochloric acid-dioxane solution (5 mL) was slowly added to a solution of t-butyl2-(8-ethynyl-7-fluoronaphthalen-1-yl)-1-fluoro-12-((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]hepten-14-carboxylate (60 mg, 0.079 mmol) in acetonitrile (2 mL). The reaction mixture was stirred at 0 °C for 1 hr. The reaction was shown to be completed by LC/MS. The reaction solution was concentrated to dryness under reduced pressure. The residue was added with a saturated sodium bicarbonate solution (3 mL) until the pH of the solution was > 7.0, and then extracted with dichloromethane (5 mL X 3). The organic phases were combined and concentrated under reduced pressure to obtain 2-(8-ethynyl-7-fluoronaphthalen-1-yl)-1-fluoro-12-((2R)-2-fluoro-6-methylenetetrahydro-1H-pyrrolizin-7a(5H)-yl)methoxy)-5-(fluoromethyl)-5a,6,7,8,9,10-hexahydro-5H-4-oxa-3,10a,11,13,14-pentaaza-6,9-methanonaphtho[1,8-ab]heptene (Z679, 29 mg, yield: 56%) as an off-white solid. ES-API: [M+H]⁺= 657.2.

### Example 71: Synthesis of 5-cyclopropyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-6-methylpyridine-2-amine (Z680)

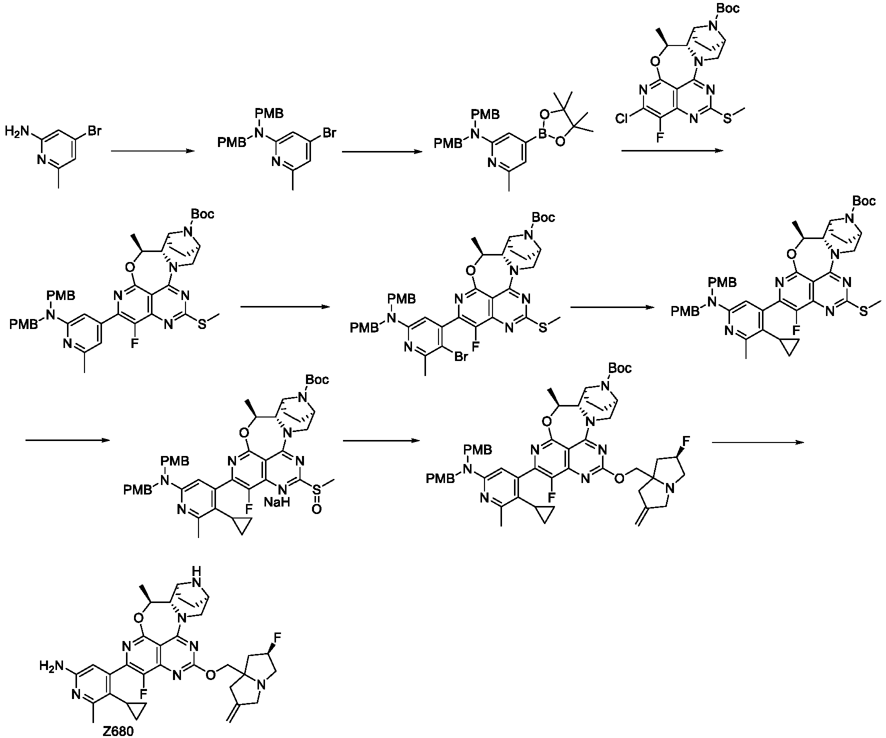

Step I: At 0°C, sodium hydride (256.0 mg, 6.415 mmol) was added to a solution of N,N-dimethylformamide (10 mL) in 4-bromo-6-methylpyridine-2-amine (1000 mg, 5.346 mmol), and stirred at this temperature for 0.5 hr. Then p-methoxybenzyl chloride (921 mg, 5.881 mmol) was added, and reacted overnight at room temperature. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-50% ethyl acetate /petroleum ether) to obtain 4-bromo-N,N-bis(4-methoxybenzyl)-6-methylpyridine-2-amine (1.56 g, yield: 68.28%). ES-API: [M+H]⁺=427.1.

Step II: 4-bromo-N,N-bis(4-methoxybenzyl)-6-methylpyridine-2-amine (1.20 g, 2.808 mmol), bis(pinacolato)diboron (1.43 g, 0.616 mmol), potassium acetate (0.93 g, 11.232 mmol) and [1,1'-bis(diphenylphosphino)ferrocene]palladium dichloride (0.31 g, 0.421 mmol) were added to N,N-dimethylformamide (20 mL), purged 4 time with nitrogen, and reacted at 120°C for 1.5 hrs. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-70% ethyl acetate /petroleum ether) to obtain N,N-bis(4-methoxybenzyl)-6-methyl-4-(4,4,5,5-tetramethyl-1,3,2-dioxaborolan-2-yl) pyridine-2-amine (1.4 g, crude product). ES-API: [M+H]⁺=475.3.

Step III: Potassium phosphate (2.50 g, 11.80 mmol), tricyclohexyl phosphine(827 mg, 2.95 mmol) and tris(dibenzylideneacetone) dipalladium (405 mg, 0.443 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab]hepten-14-carboxylate (500 mg, 1.037 mmol) and N,N-bis(4-methoxybenzyl)-6-methyl-4-(4,4,5,5-tetramethyl-1,3,2-dioxaborolan-2-yl) pyridine-2-amine (1.40 g, 2.95 mmol) in dioxane (30 mL) and water (3 mL). Nitrogen was bubbled therethrough, and the reaction was continued at 100°C for 5 hrs. After the reaction, the reaction solution was extracted with ethyl acetate (30 mLX2) and saturated brine (30 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(2-(bis(4-methoxybenzyl)amino)-6-methylpyridine-4-yl)-1-fluoro-5-methyl-12-(methylthio)-hexahydro-5H-4-oxa-3,10a,11,13,14-pentaaza-6,9-methanonaphtho[1,8-ab]hepten-14-carboxylate (860 mg, crude product) as a yellow solid. ES-API: [M+H]⁺=794.2.

Step IV: At 0°C, t-butyl (5S,5aS,6S,9R)-2-(2-(bis(4-methoxybenzyl)amino)-6-methylpyridine-4-yl)-1-fluoro-5-methyl-12-(methylthio)-hexahydro-5H-4-oxa-3,10a,11,13,14-pentaaza-6,9-methanonaphtho[1,8-ab]hepten-14-carboxylate (800 mg, 1.008 mmol) was added to dichloromethane (20 mL), and then N-bromosuccinimide (233.32 mg, 1.311 mmol) was added, and reacted at this temperature for 0.5 hr. After the reaction, the reaction solution was extracted with ethyl acetate (100 mLX2) and saturated brine (100 mL). The ethyl acetate phases were combined, dried over anhydrous sodium sulfate, and filtered. The filtrate was concentrated under reduced pressure. The crude product was purified by silica gel column chromatography (0-70% ethyl acetate/petroleum ether) to obtain t-butyl (5S,5aS,6S,9R)-2-(6-(bis(4-methoxybenzyl) amino)-3-bromo-2-methylpyridine-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (840 mg, yield: 95.57%). ES-API: [M+H]⁺=872/874.

Step V: Potassium phosphate (860 mg, 4.0523 mmol), 2-bicyclohexylphosphino-2',6'-dimethoxy-1,1'-biphenyl (120mg,0.293mmol) and palladium acetate (34.4mg,6.007mmol) were added to t-butyl (5S,5aS,6S,9R)-2-(6-(bis(4-methoxybenzyl)amino)-3-bromo-2-methylpyridine-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (860 mg, 0.985 mmol) and cyclopropylboronic acid (516 mg, 6.007 mmol) in toluene (50 mL) and water (5 mL). Nitrogen was bubbled therethrough and the reaction was continued in an oil bath at 105°C for 2 hrs. After the reaction, the reaction solution was extracted with ethyl acetate (200 mLX2) and saturated brine (100 mLX4). The ethyl acetate phases were combined, dried over anhydrous sodium sulfate, and filtered. The filtrate was concentrated under reduced pressure. The crude product was purified by silica gel column chromatography (0-70% ethyl acetate /petroleum ether) to obtain t-butyl (5S,5aS,6S,9R)-2-(6-(bis(4-methoxybenzyl)amino)-3-cyclopropyl-2-methylpyridine-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (830 mg, crude product) as a yellow solid. ES-API: [M+H]⁺=834.3.

Step VI: At 0°C, m-chloroperoxybenzoic acid (188.3 mg, 1.09 mmol) was added to a solution of t-butyl (5S,5aS,6S,9R)-2-(6-(bis(4-methoxybenzyl)amino)-3-cyclopropyl-2-methylpyridine-4-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (700 mg, 0.839 mmol) in dichloromethane(30.0 mL). The solution was stirred for 1 hour in an ice bath. The reaction was quenched with saturated sodium sulfite and extracted with dichloromethane(100 mLX2). The organic phase was dried over anhydrous sodium sulfate and concentrated to obtain a crude product. The crude product was purified by silica gel column chromatography (0-10% methanol /dichloromethane) to obtain t-butyl (5S,5aS,6S,9R)-2-(6-(bis(4-methoxybenzyl)amino)-3-cyclopropyl-2-methylpyridine-4-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (520 mg, yield: 72.89%) as a white solid. ES-API:[M+H]⁺=850.2.

Step VII: At 0°C, sodium hydride (56.48 mg, 1.412 mmol) was added to a solution of ((2R)-2-fluoro-6-methylenetetrahydro-1H-pyrrolizin-7a(5H)-yl)methanol (120 mg, 0.701 mmol) in tetrahydrofuran (10.0 mL), and stirred at this temperature for 10 min. Then, a solution of t-butyl (5S,5aS,6S,9R)-2-(6-(bis(4-methoxybenzyl)amino)-3-cyclopropyl-2-methylpyridine-4-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (300 mg, 0.353 mmol) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography(0-5% methanol /dichloromethane) to obtain t-butyl (5S,5aS,6S,9R)-2-(6-(bis(4-methoxybenzyl)amino)-3-cyclopropyl-2-methylpyridine-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (175 mg, yield: 51.8) as a yellow solid. ES-API: [M+H]⁺=957.3.

Step VIII: In an iced water bath, t-butyl (5S,5aS,6S,9R)-2-(6-(bis(4-methoxybenzyl)amino)-3-cyclopropyl-2-methylpyridine-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (175 mg, 0.183 mmol) was slowly added to trifluoroacetic acid (10.0 mL), and the reaction mixture was stirred at 50°C for 1.0 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain 5-cyclopropyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-6-methylpyridine-2-amine (Z680, 15 mg, yield:13%) as a light yellow solid. ES-API: [M+H]⁺= 617.2.

### Example 72: Synthesis of (5S,5aS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z681)

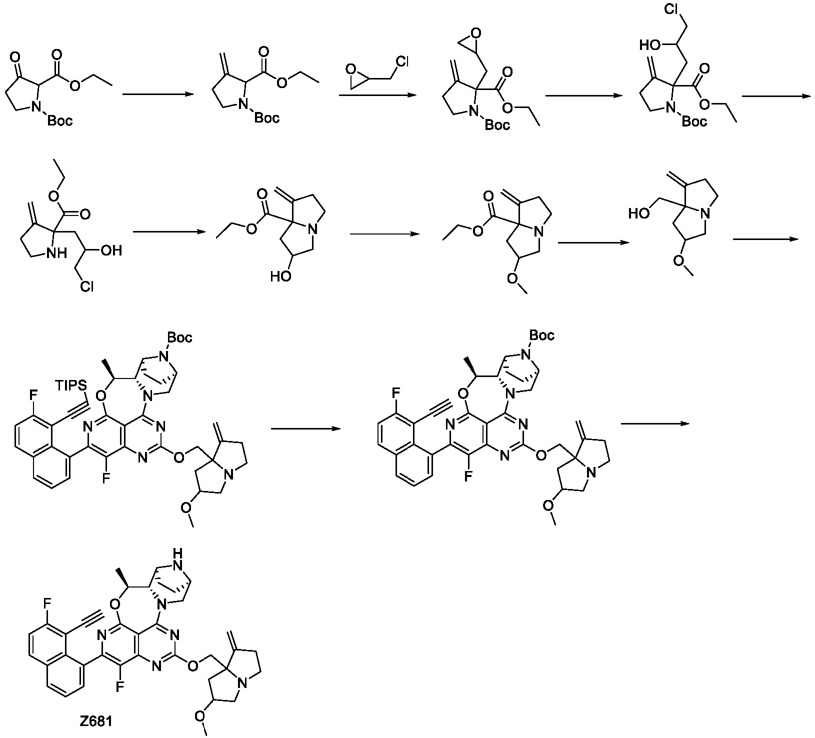

Step I: Methyltriphenylphosphonium bromide (20.82 g, 58.30 mmol) was added to tetrahydrofuran (400 mL) and cooled to 0°C. Under a nitrogen atmosphere, a solution of potassium tert-butoxide in tetrahydrofuran (58.30 mL, 58.30 mmol, 1M) was added to the mixture at 0°C, and then the reaction was warmed to room temperature and stirred for 1 hr. 1-(t-butyl) 2-ethyl3-oxopyrrolidin-1,2-dicarboxylate (6.0 g, 25.64 mmol) was dissolved in tetrahydrofuran (20.0 mL), and slowly added to the reaction solution under a nitrogen atmosphere. After that, the reaction solution was stirred at room temperature for 3 hrs. The reaction solution was added with saturated ammonium chloride (60 mL), and extracted with ethyl acetate (200 mLX3). The organic phase was washed with saturated brine (400 mL), dried over anhydrous sodium sulfate, filtered and concentrated. The crude product was purified by flash silica gel column chromatography (ethyl acetate /petroleum ether: 0%-8%) to obtain 1-(t-butyl) 2-ethyl 3-methylenepyrrolidin-1,2-dicarboxylate (4.0 g, yield: 61.6%) as a colorless transparent liquid. ES-API: [M-Boc+H]⁺=200.1.

Step II: At -70°C, lithium bis(trimethylsilyl)amide (54.8 mL, 1M, 54.8mmol) was added to a solution of 1-(t-butyl) 2-ethyl3-methylenepyrrolidin-1,2-dicarboxylate (7.0 g, 27.418 mmol) in dry tetrahydrofuran (200.0 mL), and stirred at this temperature for 1 hr. After 1 hr, 2-(chloromethyl) ethylene oxide (7.61 g, 82.25 mmol) was added to the reaction solution, and stirred overnight at room temperature. After the reaction, a saturated ammonium chloride solution (30 mL) was added to the solution, and extracted with ethyl acetate (200 mLX4). The organic phase was washed with saturated brine (200 mLX3), dried over anhydrous sodium sulfate, and concentrated. The crude product was purified by flash silica gel column chromatography [dichloromethane:methanol =100:0-90:10,(v/v)] to obtain the target compound 1-(t-butyl) 2-ethyl3-methylene-2-( ethylene oxide -2-ylmethyl)pyrrolidin-1,2-dicarboxylate (6.0 g, yield: 70.28%). ES-API: [M-56+H]⁺=256.1.

Step III: At room temperature, acetic acid (6.0 mL, 104.813 mmol) and lithium chloride (5.0 g, 117.952 mmol) were added to a solution of 1-(t-butyl) 2-ethyl 3-methylene-2-(ethylene oxide -2-ylmethyl)pyrrolidin-1,2-dicarboxylate (2.0 g, 6.423 mmol) in dry tetrahydrofuran (30.0 mL), and stirred at this temperature for 12 hrs. After the reaction, the solution was added with saturated ammonium chloride (100 mL), and extracted with ethyl acetate (200 mLX2). The organic phases were combined, dried over anhydrous sodium sulfate, and filtered. The filtrate was rotary evaporated to dryness under reduced pressure to remove the solvent, to obtain the crude product 1-(t-butyl) 2-ethyl 2-(3-chloro-2-hydroxypropyl)-3-methylenepyrrolidin-1,2-dicarboxylate (2.04 g, yield: 91%). ES-API: [M-56+H]⁺=256.1.

Step IV: Hydrochloric aciddioxane solution (16.0 mL, 4M, 64.00 mmol) was added to a solution of 1-(t-butyl) 2-ethyl 2-(3-chloro-2-hydroxypropyl)-3-methylenepyrrolidin-1,2-dicarboxylate (2.0 g, 6.423 mmol) in dichloromethane (20.0 mL), and reacted with stirring at room temperature for 2 hrs. After the reaction, the solvent was removed by rotary evaporation to dryness under reduced pressure to obtain the crude product the target compound ethyl 2-(3-chloro-2-hydroxypropyl)-3-methylenepyrrolidin-2-carboxylate (1.95 g, crude product). ES-API: [M+H]⁺=248.1.

Step V: Sodium bicarbonate (4.87 g, 58.65 mmol) was added to a solution of ethyl 2-(3-chloro-2-hydroxypropyl)-3-methylenepyrrolidin-2-carboxylate (1.45 g, 5.865 mmol) in acetonitrile (30.0 mL), and reacted at 70°C for 0.5 hr. After the reaction, the reaction solution was cooled to room temperature, and filtered. The solvent was removed from the filtrate by rotary evaporation to dryness under reduced pressure to obtain the crude product, which was purified by flash silica gel column chromatography (methanol /dichloromethane: 0%-10%) to obtain ethyl 6-hydroxyl-1-methylenetetrahydro-1H-pyrrolizin-7a(5H)-carboxylate (780 mg, yield: 62.95%). ES-API: [M+H]⁺=212.1.

Step VI: At 0°C, sodium hydride (147 mg, 3.692 mmol) was added to a solution of ethyl 6-hydroxyl-1-methylenetetrahydro-1H-pyrrolizin-7a(5H)-carboxylate (390 mg, 1.846 mmol) in N,N-dimethylformamide (5.0 mL), stirred at this temperature for 10 min, heated to room temperature and stirred for 0.5 hr. After the reaction, the reaction solution was cooled to 0°C, and the system was added with iodomethane (340 mg, 2.40 mmol) and reacted for 0.5 hr. After the reaction, the reaction solution was diluted with ethyl acetate (60 mLx2), washed with water (30 mLX2) and saturated brine (50 mLX3), dried over anhydrous sodium sulfate, filtered, and concentrated to obtain a crude product, which was purified by flash silica gel column chromatography (methanol /dichloromethane: 0%-8%) to obtain ethyl 6-methoxy-1-methylenetetrahydro-1H-pyrrolizin-7a(5H)-carboxylate (120 mg, yield: 28.85%). ES-API: [M+H]⁺= 226.1.

Step VII: Ethyl 6-methoxy-1-methylenetetrahydro-1H-pyrrolizin-7a(5H)-carboxylate (120 mg, 0.533 mmol) was dissolved in tetrahydrofuran (10 mL), and cooled to 0°C. Under a nitrogen atmosphere, a lithium aluminum hydride solution (1.0 mL, 1.0 mmol, 1M solution in tetrahydrofuran) was added to the solution. The reaction was stirred at 0°C for 1 hr. The reaction was quenched with water (0.03 mL), and 15% sodium hydroxide aqueous solution (0.03 mL) and water (0.09 mL) were added. Tetrahydrofuran (10 mL) was added and filtered. The filtrate was concentrated, to obtain (6-methoxy-1-methylenetetrahydro-1H-pyrrolizin-7a(5H)-yl)methanol (85 mg, yield: 87%) as a colorless transparent liquid. ES-API: [M +H]⁺=184.1.

Step VIII: (6-methoxy-1-methylenetetrahydro-1H-pyrrolizin-7a(5H)-yl)methanol (85 mg, 0.464 mmol) was dissolved in anhydrous tetrahydrofuran (5 mL). In an iced water bath, sodium hydride (37.25 mg, 0.931 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (180 mg, 0.233 mmol) in tetrahydrofuran (2 mL) was added dropwise to the reaction solution, and reacted at 40°C for 30 min. The reaction was shown to be completed by LC/MS. The reaction solution was diluted with ethyl acetate (50 mL), washed with a saturated ammonium chloride solution (15 mLX2) and saturated brine (50 mLX3), dried over anhydrous sodium sulfate, and filtered. The crude product was purified by automated flash silica gel chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-12-((6-methoxy-1-methylenetetrahydro)-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (120 mg, yield: 56.8%). ES-API: [M+H]⁺= 907.1.

Step IX: Cesium fluoride (100.0 mg, 0.658 mmol) was added to t-butyl (5S,5aS,6S,9R)-1-fluoro-2-(7-fluoro-8-((triisopropylsilyl)ethynyl)naphthalen-1-yl)-12-((6-methoxy-1-methylenetetrahydro)-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (120 mg, 0.132 mmol) in N,N-dimethylformamide (5.0 mL), and the reaction mixture was stirred at room temperature for 2 hrs. The reaction was shown to be completed by LC/MS. The reaction solution was diluted with ethyl acetate (60 mL), washed with water (30 mLX2) and saturated brine (50 mLX 3), dried over anhydrous sodium sulfate, filtered, and concentrated to obtain t-butyl (5S,5aS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (80.0 mg, yield: 80.5%). ES-API: [M+H]⁺= 751.1.

Step X: In an iced water bath, a 4 M hydrochloric acid-dioxane solution (1.0 mL) was slowly added to a solution of t-butyl (5S,5aS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (80.0 mg, 0.107 mmol) in acetonitrile (5.0 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-((6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z681, 60.0mg, yield: 86.54%) as a light yellow solid. ES-API: [M+H]⁺= 651.2.

### Example 73: Synthesis of (5S,5aS,6S,9R)-2-(3-chloro-2-(trifluoromethyl)phenyl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z682)

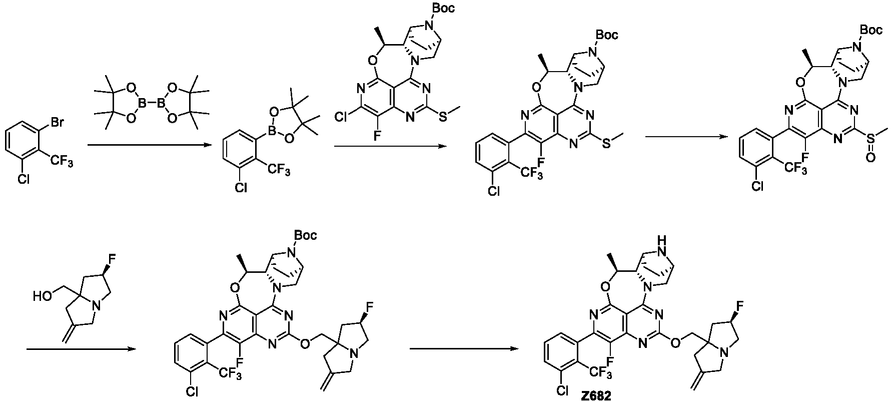

Step I: 2-chloro-6-bromotrifluoromethylbenzene (778.35 mg, 3 mmol), bis(pinacolato)diboron (1523.64 mg, 6.000 mmol), potassium acetate (735.00 mg, 7.500 mmol), and [1,1'-bis(diphenylphosphino)ferrocene]palladium dichloride (219.51 mg, 0.300 mmol) were dissolved in 1, 4-dioxane (5 mL), stirred at 110°C for 2 hrs, and concentrated to dryness under reduced pressure. The cruded product was purified by column chromatography (petroleum ether/ethyl acetate =90/10), to obtain 2-(3-chloro-2-(trifluoromethyl)phenyl)-4,4,5,5-tetramethyl-1,3,2-dioxaborolane (450 mg, 1.468 mmol, yield: 48.94%).

Step II: 2-(3-chloro-2-(trifluoromethyl)phenyl)-4,4,5,5-tetramethyl-1,3,2-dioxaborolane (127.19 mg, 0.415 mmol) and t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (100 mg, 0.207 mmol) were dissolved in tetrahydrofuran(2 mL) and water (0.5 mL), [n-butyldi(1-adamantanyl)phosphine](2-amino-1,1'-biphenyl-2-yl)palladium (II) mesylate (15.10 mg, 0.021 mmol) and potassium phosphate (132.12 mg, 0.622 mmol) were added. After that, the reaction was stirred at 65 °C for 5 hrs. After the reaction, the reaction solution was added with water (15 mL) and extracted with ethyl acetate (15 mLX3). The organic phase was washed with saturated brine (30 mL), dried over anhydrous sodium sulfate, filtered and concentrated. The crude product was purified by flash silica gel column chromatography (ethyl acetate/petroleum ether: 0-30%) to obtain t-butyl (5S,5aS,6S,9R)-2-(3-chloro-2-(trifluoromethyl) phenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (45 mg, 0.072 mmol, yield: 34.64%) as a white solid. ES-API: [M +H]⁺= 626.1.

Step III: T-butyl (5S,5aS,6S,9R)-2-(3-chloro-2-(trifluoromethyl)phenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (42 mg, 0.067 mmol) was dissolved in dichloromethane (5 mL). At room temperature, m-chloroperoxybenzoic acid (15.05 mg, 0.087 mmol) was added to the solution. After that, the reaction was stirred at room temperature for 1 hr. The reaction solution was added with a saturated sodium thiosulphate solution (5 mL) and extracted with dichloromethane (10 mLX3). The organic phases were combined, and then washed sequentially with a saturated sodium bicarbonate aqueous solution (20 mL) and saturated sodium chloride (20 mL). The organic phase was dried over anhydrous sodium sulfate, filtered and concentrated to obtain t-butyl (5S,5aS,6S,9R)-2-(3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (40 mg, 0.062 mmol, yield: 92.87%). ES-API: [M +H]⁺= 642.0.

Step IV: T-butyl (5S,5aS,6S,9R)-2-(3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (40 mg, 0.062 mmol, yield: 92.87%) was dissolved in tetrahydrofuran (3 mL) and cooled to 0°C. Under a nitrogen atmosphere, sodium hydride (10.47 mg, 0.262 mmol) was added to the solution and stirred for 30 min. Then, [(2R)-2-fluoro-6-methylenetetrahydro-1H-pyrrolizin-7a(5H)-yl]methanol (22.40 mg, 0.131 mmol) was slowly added to the solution and stirred at 0°C for 2 hrs. The reaction solution was added with water (5 mL) to quench the reaction, and then extracted with ethyl acetate (20 mL X3). The organic phase was washed with saturated brine (30 mLX3), dried over anhydrous sodium sulfate, filtered and concentrated to obtain a crude product. The crude product was purified by flash silica gel column chromatography (ethyl acetate /petroleum ether: 0-70%) to obtain t-butyl (5S,5aS,6S,9R)-2-(3-chloro-2-(trifluoromethyl)phenyl)-1-fluoro-5-methyl-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.033 mmol, yield: 51.01%). ES-API: [M+H]⁺=749.0.

Step V: T-butyl (5S,5aS,6S,9R)-2-(3-chloro-2-(trifluoromethyl)phenyl)-1-fluoro-5-methyl-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate was dissolved in dichloromethane, trifluoroacetic acid was added, and reacted at room temperature for 2 hrs. After the reaction, the crude product was purified by preparative HPLC (ammonium bicarbonate method 1) to obtain (5S,5aS,6S,9R)-2-(3-chloro-2-(trifluoromethyl) phenyl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z682, 10 mg, 0.015 mmol, yield: 76.92%). ES-API: [M+H]⁺=649.2.

### Example 74: Synthesis of (7a-((((5S, 5aS, 6S, 9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy)methyl)-6-methylenehexahydro-1H-pyrrolizin-1-yl)methanol (Z683)

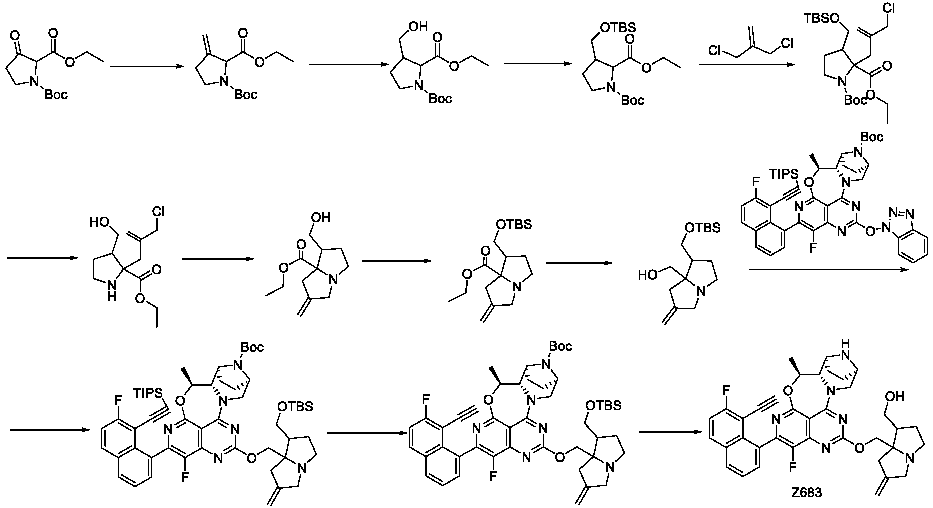

Step I: Methyltriphenylphosphonium bromide (18.34 g, 51.385 mmol) was dissolved in tetrahydrofuran (220 mL), and cooled to 0°C in an iced water bath. A potassium tert-butoxide solution (51.4 mL, 51.385 mmol) was added dropwise to the reaction solution, and then the reaction solution was slowly heated to room temperature and stirred for 1 hr. A solution of 1-(t-butyl) 2-ethyl3-oxypyrrolidin-1,2-dicarboxylate (5.0 g, 20.544 mmol) in tetrahydrofuran (220 mL) was added to the solution, and the reaction mixture was stirred at room temperature for 3 hrs. The reaction was shown to be completed by LC/MS. The reaction solution was extracted with ethyl acetate (200 mLX2). The organic phase was washed with saturated ammonium chloride (60 mLX 2), dried over anhydrous sodium sulfate, filtered and concentrated. The crude product was purified by automated flash silica gel chromatography (ethyl acetate: petroleum ether= 0- 10%) to obtain 1-(t-butyl) 2-ethyl 3-methylenepyrrolidin-1,2-dicarboxylate (2240 mg, yield: 43%). ES-API: [M-55]⁺= 200.1.

Step II: 1-(t-butyl) 2-ethyl 3-methylenepyrrolidin-1,2-dicarboxylate (670 mg, 2.624 mmol) was dissolved in tetrahydrofuran (20 mL), and cooled to 0°C in an iced water bath. A borane-tetrahydrofuran solution (13 mL, 13.121 mmol, 1 M in solution in tetrahydrofuran) was slowly added to the reaction solution, and then the reaction solution was heated to room temperature and stirred for 3 hrs. The reaction was shown to be completed by LC/MS. 3 M sodium hydroxide solution (4 mL) and hydrogen peroxide (3 mL) were added to the reaction solution, and the reaction mixture was stirred at room temperature for 1 hr. Water (30 mL) was added to the solution, and the reaction solution was extracted with dichloromethane (20 mLX3). The organic phase was washed with saturated brine (15 mLX2), dried over anhydrous sodium sulfate, filtered, and concentrated to obtain the crude product 1-(t-butyl) 2-ethyl 3-(hydroxymethyl)pyrrolidin-1,2-dicarboxylate (859 mg). ES-API: [M-55]⁺= 218.1.

Step III: 1-(t-butyl) 2-ethyl3-(hydroxymethyl)pyrrolidin-1,2-dicarboxylate (859 mg, 3.143 mmol) was dissolved in dichloromethane (20 mL). Imidazole(1070 mg, 15.714 mmol) and t-butyldimethylchlorosilane (710 mg, 4.714 mmol) were sequentially added to the reaction solution, and the reaction mixture was stirred overnight at room temperature. The reaction was shown to be completed by LC/MS. The reaction solution was diluted with dichloromethane (30 mL), and washed with water (10 mLX2) and saturated brine (15 mL). The organic phase was dried over anhydrous sodium sulfate, filtered and concentrated. The crude product was purified by automated flash silica gel chromatography (ethyl acetate: petroleum ether= 0- 4%) to obtain 1-(t-butyl) 2-ethyl3-(((t-butyldimethylsilyl)oxy)methyl)pyrrolidin-1,2-dicarboxylate (553 mg, yield: 45%). ES-API: [M-99]⁺= 288.2.

Step IV: Under a nitrogen atmosphere, 1-(t-butyl) 2-ethyl 3-(((t-butyldimethylsilyl) oxy)methyl)pyrrolidin-1,2-dicarboxylate (553 mg, 1.427 mmol) was dissolved in anhydrous tetrahydrofuran(20 mL), and the mixture was allowed to stand in a dry ice-acetone bath. Lithium diisopropylamide (1.5 mL, 2.854 mmol) was slowly added dropwise to the above solution, and stirred at this temperature for 1 hr. A solution of 3-chloro-2-chloromethylpropene (892 mg, 7.134 mmol) in anhydrous tetrahydrofuran (3 mL) was slowly added by a syringe to the solution, and the reaction solution was continuously stirred at -78°C for 1 hr, then slowly heated to room temperature, and stirred overnight at room temperature. The reaction was shown to be completed by LC/MS. The reaction was quenched with a saturated ammonium chloride solution (30 mL). The reaction solution was extracted with ethyl acetate (20 mLX3). The organic phase was washed with saturated brine (15 mLX3), dried over anhydrous sodium sulfate, filtered and concentrated. The crude product was purified by automated flash silica gel chromatography (ethyl acetate: petroleum ether= 0- 3%) to obtain 1-(t-butyl) 2-ethyl 3-(((t-butyldimethylsilyl)oxy)methyl)-2-(2-(chloromethyl)allyl) pyrrolidin-1,2-dicarboxylate (176 mg, yield: 26%). ES-API: [M-55]⁺= 420.2.

Step V: At room temperature, a 4 M hydrochloric acid-dioxane solution (5 mL) was slowly added to a solution of 1-(t-butyl) 2-ethyl 3-(((t-butyldimethylsilyl)oxy)methyl)-2-(2-(chloromethyl) allyl)pyrrolidin-1,2-dicarboxylate (176 mg, 0.370 mmol) in acetonitrile (2 mL). The reaction mixture was stirred at room temperature for 2 hrs. The reaction was shown to be completed by LC/MS. The reaction solution was concentrated to dryness under reduced pressure to obtain the crude product ethyl 2-(2-(chloromethyl)allyl)-3-(hydroxymethyl)pyrrolidin-2-carboxylate (96 mg). ES-API: [M+ H]⁺= 262.1.

Step VI: At room temperature, sodium bicarbonate (276 mg, 3.286 mmol) and potassium iodide (11 mg, 0.066 mmol) were added to a solution of ethyl 2-(2-(chloromethyl)allyl)-3-(hydroxymethyl)pyrrolidin-2-carboxylate (86 mg, 0.329 mmol) in acetonitrile (10 mL), and the reaction mixture was stirred at room temperature for 1 hr. The reaction was shown to be completed by LC/MS. The reaction solution was filtered, and the insoluble matter was washed with acetonitrile. The filtrate was concentrated under reduced pressure, to obtain the crude product ethyl 1-(hydroxymethyl)-6-methylenetetrahydro-1H-pyrrolizin-7a(5H)-carboxylate (127 mg). ES-API: [M+H]⁺= 226.1.

Step VII: Ethyl 1-(hydroxymethyl)-6-methylenetetrahydro-1H-pyrrolizin-7a(5H)-carboxylate (127 mg, 0.564 mmol) was dissolved in dichloromethane (10 mL). Imidazole (192 mg, 2.819 mmol) and t-butyldimethylchlorosilane (110 mg, 0.733 mmol) were sequentially added to the reaction solution, and the reaction mixture was stirred overnight at room temperature. The reaction was shown to be completed by LC/MS. The reaction solution was diluted with dichloromethane (30 mL), and washed with water (10 mLX2) and saturated brine (15 mL). The organic phase was dried over anhydrous sodium sulfate, filtered and concentrated. The crude product was purified by automated flash silica gel chromatography (ethyl acetate: petroleum ether= 0- 4%) to obtain ethyl 1-(((t-butyldimethylsilyl)oxy)methyl-6-methylenetetrahydro-1H-pyrrolizin-7a(5H)-carboxyalte (20 mg, yield: 10%). ES-API: [M+ 1]⁺= 340.3.

Step VIII: Under a nitrogen atmosphere, ethyl 1-(((t-butyldimethylsilyl)oxy)methyl-6-methylenetetrahydro-1H-pyrrolizin-7a(5H)-carboxylate (20 mg, 0.059 mmol) was dissolved in anhydrous tetrahydrofuran (5 mL), and the mixture was allowed to stand in an iced water bath. Lithium aluminium hydride (0.2 mL, 0.20 mmol, 1 M solution in tetrahydrofuran) was slowly added dropwise to the above solution, and then stirred at this temperature for 0.5 hr. The reaction was shown to be completed by LC/MS. The reaction solution was added with sodium sulfafe decahydrate (2.0 eq) to quench the reaction, stirred at room temperature for 0.5 hr, and filtered to remove the insoluble matter. The filtrate was concentrated under reduced pressure, to obtain the crude product (1-(((t-butyldimethylsilyloxy)methyl)-6-methylenetetrahydro-1H-pyrrolizin-7a (5H)-yl)methanol (17.5 mg). ES-API: [M+H]⁺= 298.2.

Step IX: (1-(((t-butyldimethylsilyloxy)methyl)-6-methylenetetrahydro-1H-pyrrolizin-7a(5H)-yl)methanol (17 mg, 0.058 mmol) was dissolved in anhydrous tetrahydrofuran (5 mL). In an iced water bath, sodium hydride (2.3 mg, 0.058 mmol) was added and reacted for 10 min in the iced water bath. A solution of t-butyl (5S, 5aS, 6S, 9R)-12-((1H-benzo[d][1,2,3]triazole-1-yl)oxy)-1-fluoro-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.029 mmol) in tetrahydrofuran (2 mL) was added dropwise to the reaction solution, reacted for 30 min at 0°C, then heated to 35 °C, and stirred for 2 hrs. The reaction was shown to be completed by LC/MS. The reaction solution was diluted with ethyl acetate (10 mL), washed with saturated ammonium chloride solution (10 mLX2) and saturated brine (10 mLX 3), dried over anhydrous sodium sulfate, filtered, and concentrated under reduced pressure, to obtain a crude product that was t-butyl (5S, 5aS, 6S, 9R)-12-((1-(((t-butyldimethylsilyl)oxy)methyl)-6-methylenetetrahydro-1H-pyrrolizin-7a(5H)-yl)methoxy)-1-fluoro-2-(7-fluoro-8-(triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (86 mg). ES-API: [M+H]⁺= 1021.4.

Step X: Cesium fluoride (128 mg, 0.842 mmol) was added to t-butyl (5S, 5aS, 6S, 9R)-12-((1-(((t-butyldimethylsilyl)oxy)methyl)-6-methylenetetrahydro-1H-pyrrolizin-7a(5H)-yl)methoxy)-1-fluoro-2-(7-fluoro-8-(triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (86 mg, 0.084 mmol) in N,N-dimethylformamide (2 mL), and the reaction mixture was stirred at room temperature for 0.5 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain a crude product that was t-butyl (5S, 5aS, 6S, 9R)-12-((1-(((t-butyldimethylsilyl) oxy)methyl)-6-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (72 mg). ES-API: [M+H]⁺= 865.3.

Step XI: In an iced water bath, 4 M hydrochloric acid-dioxane solution (5 mL) was slowly added to a solution of t-butyl (5S, 5aS, 6S, 9R)-12-((1-(((t-butyldimethylsilyl)oxy)methyl)-6-methylenetetrahydro-1H-pyrrolizin-7a(5H)-yl)methoxy)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (72 mg, 0.083 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-40-60-13 min) to obtain (7a-((((5S, 5aS, 6S, 9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy)methyl)-6-methylenehexahydro-1H-pyrrolizin-1-yl)methanol (Z683, 6.73 mg, yield of three steps: 18%) as an off-white solid. ES-API: [M+H]⁺= 651.3. ¹H NMR (400 MHz, CD₃OD) δ 8.11-8.06 (m, 2 H), 7.66-7.54 (m, 2 H), 7.46-7.39 (m, 1 H), 5.39-5.35 (m, 1 H), 5.03 (s, 2 H), 4.57-4.52 (m, 1 H), 4.33-4.07 (m, 4 H), 3.78-3.60 (m, 4 H), 3.47-3.35 (m, 2 H), 3.21-3.12 (m, 2 H), 2.69-2.65 (m, 1 H), 2.53-2.48 (m, 2 H), 2.05-2.03 (m, 1 H), 1.93-1.78 (m, 4 H), 1.57 (t, J= 8.5 Hz, 3 H), 1.18-1.16 (m, 3 H), 1.00-0.98 (m, 1 H).

### Example 75: Synthesis of (5S, 5aS, 6S, 9R)-2-(5-ethynylisoquinolin-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z684)

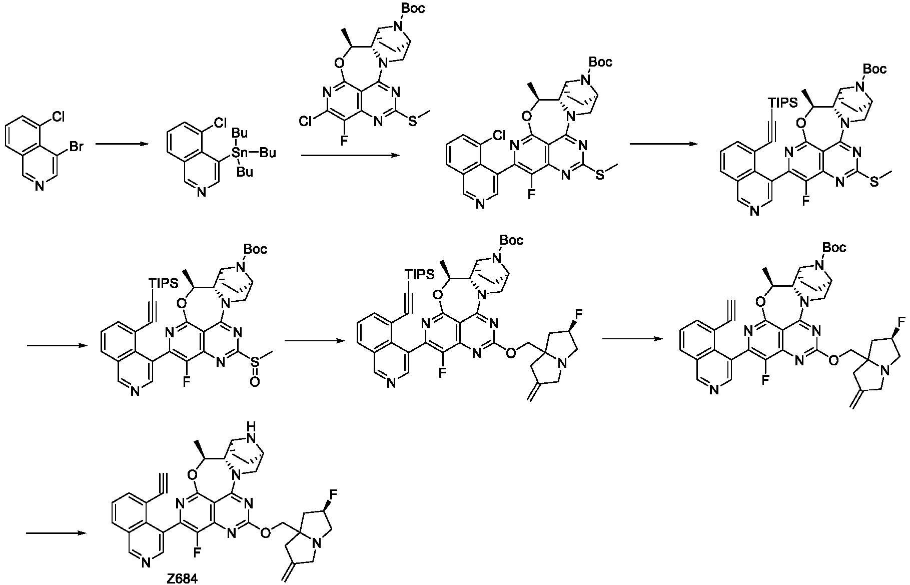

Step I: Under a nitrogen atmosphere, 4-bromo-5-chloroisoquinoline (900 mg, 3.7 mmol), hexabutylditin (4.3 g, 7.4 mmol), tris(dibenzylideneacetone) dipalladium (340 mg, 0.37 mmol), tricyclohexyl phosphine(208 mg, 0.02 mmol) and anhydrous lithium chloride (787 mg, 18.6 mmol) were dissolved in dioxane (5 mL). The reaction mixture was heated to 110 °C and stirred for 16 hrs. The reaction was shown to be completed by LC/MS. The reaction solution was concentrated and the crude product was purified by automated flash silica gel chromatography (petroleum ether: ethyl acetate =30:1) to obtain 5-chloro-4-(tributylstannyl)isoquinoline (800 mg, yield: 47.6%). ES-API: [M+H]⁺=454.1.

Step II: Under a nitrogen atmosphere, t-butyl (5S, 5aS, 6S, 9R)-2-chloro-1-fluoro-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (290 mg, 0.6 mmol), 5-chloro-4-(tributylstannyl)isoquinoline (681 mg, 1.5 mmol), [1,1'-bis(diphenylphosphino)ferrocene] palladium dichloride (44 mg, 0.06 mmol), 1,1'-binaphthyl-2.2'-diphemyl phosphine (75 mg, 0.12 mmol) and cuprous iodide (34 mg, 0.18 mmol) were dissolved in toluene (10 mL). The reaction mixture was heated to 90 °C and stirred for 16 hrs. The reaction was shown to be completed by LC/MS. The reaction solution was concentrated and the crude product was purified by automated flash silica gel chromatography (petroleum ether: ethyl acetate =20:1) to obtain t-butyl (5S, 5aS, 6S,9R)-2-(5-chloroisoquinoline-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (240 mg, yield: 65%). ES-API: [M+H]⁺=609.1.

Step III: Under a nitrogen atmosphere, t-butyl (5S, 5aS, 6S, 9R)-2-(5-chloroisoquinoline -4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (190 mg, 0.3 mmol), triisopropylsilylethyne (284 mg, 1.56 mmol), cesium carbonate (203 mg, 0.6 mmol), 2-dicyclohexylphosphino-2',4',6'-triisopropylbiphenyl (89 mg, 0.19 mmol) and bis(acetonitrile) palladium chloride (II)(16 mg, 0.06 mmol) were dissolved in acetonitrile (10 mL). The reaction mixture was heated to 85 °C and stirred for 16 hrs. The reaction was shown to be completed by LC/MS. The reaction solution was concentrated and the crude product was purified by automated flash silica gel chromatography (dichloromethane: methanol =20:1) to obtain t-butyl (5S, 5aS, 6S, 9R)-1-fluoro-5-methyl-12-(methylthio)-2-(5-(triisopropylsilyl)ethynyl)isoquinoline -4-yl)-5a,6,7,8,9,10-hexahydro-5H-4-oxa-3,10a,11,13,14-pentaaza-6,9-methanonaphtho[1,8-ab]hepten-14-carboxylate (130 mg, yield: 55%). ES-API: [M+H]⁺=755.2.

Step IV: m-chloroperoxybenzoic acid (36 mg, 0.21mmol) was added to a solution of t-butyl (5S, 5aS, 6S, 9R)-1-fluoro-5-methyl-12-(methylthio)-2-(5-(triisopropylsilyl)ethynyl)isoquinoline -4-yl)-5a,6,7,8,9,10-hexahydro-5H-4-oxa-3,10a,11,13,14-pentaaza-6,9-methanonaphtho[1,8-ab]hepten-14-carboxylate (130 mg, 0.17 mmol) in dichloromethane (5mL), and the reaction mixture was stirred at room temperature for 1 hr. The reaction was shown to be completed by LC/MS. The reaction solution was diluted with ethyl acetate (20 mL), washed with a saturated sodium sulfite solution (15 mLX2) and saturated brine (15 mLX3), dried over anhydrous sodium sulfate, filtered, and concentrated to obtain the crude product t-butyl (5S, 5aS, 6S, 9R)-1-fluoro-5-methyl-12-(methylthionyl)-2-(5-((triisopropylsilyl)ethynyl)isoquinoline-4-yl)-5a,6,7,8,9,10-hexahydro-5H-4-oxa-3,10a,11,13,14-pentaaza-6,9-methanonaphtho[1,8-ab]hepten-14-carboxylate (132.7 mg, yield: 100%). ES-API: [M+H]⁺=771.2.

Step V: ((2R)-2-fluoro-6-methylenetetrahydro-1H-pyrrolizin-7a(5H)-yl)methanol (59 mg, 0.34 mmol) was dissolved in anhydrous tetrahydrofuran(5 mL). In an iced water bath, sodium hydride (27 mg, 0.69 mmol) was added, and then reacted for 30 min in the iced water bath. A solution of t-butyl (5S, 5aS, 6S, 9R)-1-fluoro-5-methyl-12-(methylthionyl)-2-(5-(triisopropylsilyl) ethynyl)isoquinoline-4-yl)-5a,6,7,8,9,10-hexahydro-5H-4-oxa-3,10a,11,13,14-pentaaza-6,9-methanonaphtho[1,8-ab]hepten-14-carboxylate (132.7 mg, 0.17 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX2) and saturated brine (15 mLX3), dried over anhydrous sodium sulfate, filtered, and concentrated to obtain a crude product. The crude product was purified by automated flash silica gel chromatography (dichloromethane: methanol =20:1) to obtain t-butyl (5S, 5aS, 6S, 9R)-1-fluoro-12-(((2R)-2-fluoro-6-methylenetetrahydro-1H-pyrrolizin-7a(5H)-yl)methoxy)-5-methyl-2-(5-(triisopropylsilyl)ethynyl)isoquinoline-4-yl)-5a,6,7,8,9,10-hexahydro-5H-4-oxa-3,10a,11,13,14-pentaaza-6,9-methanonaphtho[1,8-ab]hepten-14-carboxylate (40 mg, yield: 26.6%). ES-API: [M+H]⁺=878.2.

Step VI: Cesium fluoride (69 mg, 0.46 mmol) was added to t-butyl (5S, 5aS, 6S, 9R)-1-fluoro-12-(((2R)-2-fluoro-6-methylenetetrahydro-1H-pyrrolizin-7a(5H)-yl)methoxy)-5-methyl-2-(5-(triisopropylsilyl)ethynyl)isoquinoline -4-yl)-5a,6,7,8,9,10-hexahydro-5H-4-oxa-3,10a,11,13,14-pentaaza-6,9-methanonaphtho[1,8-ab]hepten-14-carboxylate (40 mg, 0.046 mmol) in N,N-dimethylformamide (3 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S, 5aS, 6S, 9R)-2-(5-ethynylisoquinoline-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (32 mg, yield: 97%). ES-API: [M+H]⁺=722.2.

Step VII: In an iced water bath, a 4 M hydrochloric acid-dioxane solution (5 mL) was slowly added to a solution of t-butyl (5S, 5aS, 6S, 9R)-2-(5-ethynylisoquinoline -4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (32 mg, 0.044 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(5-ethynylisoquinoline-4-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z684, 2 mg, yield: 7%) as a light yellow solid. ES-API: [M+H]⁺= 622.2.

### Example 76: Synthesis of 8-ethynyl-1-((5S, 5aS, 6S, 9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)isoquinoline -3-amine (Z685)

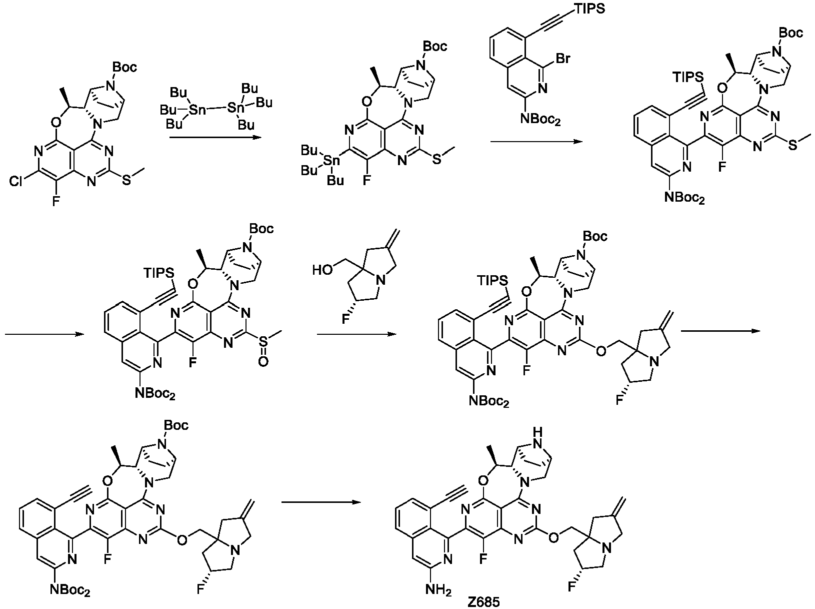

Step I: T-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (500 mg, 1.037 mmol), hexabutylditin (1.046 mL, 2.074 mmol), tris (dibenzylideneacetone) dipalladium (95.00 mg, 0.104 mmol), tricyclohexyl phosphine (58.19 mg, 0.207 mmol), lithium chloride (220 mg, 5.19 mmol) and 1,4-dioxane (15 mL) were added to a round-bottom flask. Under a nitrogen atmosphere, the reaction was stirred in an oil bath at 110°C for 18 hrs. The reaction solution was concentrated, and the residue was purified by flash silica gel column chromatography (0-15%ethyl acetate /petroleum ether) to obtain t-butyl (5S, 5aS, 6S, 9R)-1-fluoro-5-methyl-12-(methylthio)-2-(tributyltin)-5a,6,7,8,9,10-hexahydro-5H-4-oxa-3,10a,11,13,14-pentaaza-6,9-methanonaphtho[1,8-ab]hepten-14-carboxylate (370mg, yield: 48%). ES-API:[M+Na]⁺=738.1.

Step II: T-butyl (5S, 5aS, 6S, 9R)-1-fluoro-5-methyl-12-(methylthio)-2-(tributyltin)-5a,6,7,8,9,10-hexahydro-5H-4-oxy-3,10a,11,13,14-pentaaza-6,9-methanonaphtho[1,8-ab]hepten-14-carboxylate (146 mg, 0.20 mmol), dit-butyl (1-bromo-8-((triisopropylsilyl)ethynyl) isoquinoline -3-yl)carbamate (80 mg, 0.13 mmol), cuprous iodide (25 mg, 0.13 mmol), terakis(triphenylphosphine)palladium (23 mg, 0.02 mmol), and anhydrous lithium chloride (22 mg, 0.53 mmol) in N,N-dimethyl acetamide (4 mL) were added to a round-bottom flask. Under a nitrogen atmosphere, the reaction was stirred in an oil bath at 130°C for 4 hrs. The reaction solution was added with water (30 mL), and extracted with ethyl acetate (20 mLX2). The organic phase was washed with saturated brine (30 mL), dried over anhydrous sodium sulfate, filtered, and concentrated. The residue was purified by flash silica gel column chromatography (0-30% ethyl acetate /petroleum ether) to obtain di-t-butyl (5S,5aS,6S,9R)-2-(3-((t-butoxycarbonyl)amino)-8-((triisopropylsilyl)ethynyl)isoquinoline-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (25 mg, yield: 21%). ES-API:[M+Na]⁺=870.3.

Step III: Di-t-butyl (5S,5aS,6S,9R)-2-(3-((t-butoxycarbonyl)amino)-8-((triisopropylsilyl) ethynyl)isoquinoline-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (25 mg, 0.029 mmol) and dichloromethane (3 mL) were added to a round-bottom flask. The reaction solution was cooled to 0°C, and m-chloroperoxybenzoic acid (10 mg, 0.057 mmol) was added. The reaction was stirred at room temperature for 1 hrs, added with water (30 mL), and extracted with ethyl acetate (20 mLX2). The organic phase was washed with saturated brine (30 mL), dried over anhydrous sodium sulfate, filtered, and concentrated to obtain di-t-butyl 5 (5S,5aS,6S,9R)-2-(3-((t-butoxycarbonyl)amino)-8-((triisopropylsilyl)ethynyl)isoquinoline-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (25 mg, crude product). ES-API:[M+Na]⁺=886.3.

Step IV: 60% sodium hydride (4 mg, 0.14 mmol) and tetrahydrofuran (3 mL) were added to a round-bottom flask. The reaction solution was cooled to 0°C, and a solution of ((2R)-2-fluoro-6-methylenetetrahydro-1H-pyrrolizin-7a(5H)-yl)methanol (14 mg, 0.085 mmol) in anhydrous tetrahydrofuran (1 mL) was added, and reacted at room temperature for 10 min. The reaction solution was cooled to 0°C. Di-t-butyl (5S,5aS,6S,9R)-2-(3-((t-butoxycarbonyl)amino)-8-((triisopropylsilyl)ethynyl)isoquinoline-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxyalte (25 mg, crude product) was added to the reaction solution, and reacted at 0°C for 0.5 hr. The reaction solution was added with water (30 mL), and extracted with ethyl acetate (20 mLX2). The organic phase was washed with saturated brine (30 mL), dried over anhydrous sodium sulfate, filtered, and concentrated. The residue was purified by flash silica gel column chromatography (0-3%methanol /dichloromethane) to obtain di-t-butyl (5S,5aS,6S,9R)-2-(3-((t-butoxycarbonyl)amino)-8-((triisopropylsilyl)ethynyl)isoquinoline-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (15 mg, yield: 53%). ES-API: [M+H]⁺=993.3.

Step V: Cesium fluoride (23 mg, 0.15 mmol) was added to di-t-butyl (5S,5aS,6S,9R)-2-(3-((t-butoxycarbonyl)amino)-8-((triisopropylsilyl)ethynyl)isoquinoline-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (15 mg, crude product) in N,N-dimethylformamide (2 mL). The reaction mixture was stirred at room temperature for 1 hr. The reaction was shown to be completed by LC/MS. The reaction solution was diluted with ethyl acetate (30 mL), and washed with water (30 mL) and saturated brine (30 mLX2). The organic phase was dried over anhydrous sodium sulfate, filtered, and concentrated to obtain di-t-butyl (5S,5aS,6S,9R)-2-(3-((t-butoxycarbonyl)amino)-8-ethynylisoquinoline-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11, 13,14-pentaaza-6,9-methanonaphtho[1,8-ab]hepten-14-carboxylate (12 mg, crude product). ES-API: [M+H]⁺= 837.1.

Step VI: In an iced water bath, a 4 M hydrochloric acid-dioxane solution (0.5 mL) was slowly added to a solution of di-t-butyl (5S,5aS,6S,9R)-2-(3-((t-butoxycarbonyl)amino)-8-ethynylisoquinoline-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (10 mg, crude product) in acetonitrile(1 mL). The reaction mixture was stirred at room temperature for 1 hr. The reaction was shown to be completed by LC/MS. The reaction solution was concentrated to dryness under reduced pressure. The crude product was purified by preparative HPLC (ammonium bicarbonate method 1) to obtain 8-ethynyl-1-((5S, 5aS, 6S, 9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)isoquinoline-3-amine (Z685, 0.67 mg, yield: 8%) as a white solid. ES-API: [M+H]⁺= 637.2.

### Example 77: Synthesis of 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-1-amine (Z686)

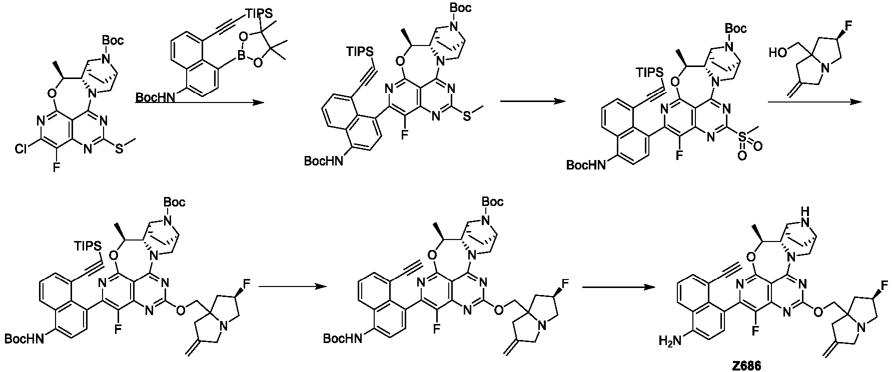

Step I: T-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (120 mg, 0.249 mmol) and t-butyl (4-(4,4,5,5-tetramethyl-1,3,2-dioxaborolan-2-yl)-5-((triisopropylsilyl)ethynyl)naphthalen-1-yl)carbamate (164.22 mg, 0.299 mmol) were dissolved in tetrahydrofuran (10 mL) and water (1 mL). [n-butyldi(1-adamantanyl)phosphine](2-amino-1,1'-biphenyl-2-yl)palladium (II) mesylate (18.13 mg, 0.025 mmol) and potassium phosphate (158.55 mg, 0.747 mmol) were added to the solution. After that, the reaction was stirred at 65°C for 5 hrs. After the reaction, the reaction solution was added with water (15 mL) and extracted with ethyl acetate (15 mLX3). The organic phase was washed with saturated brine (30 mL), dried over anhydrous sodium sulfate, filtered and concentrated, to obtain a crude product. The crude product was purified by flash silica gel column chromatography (ethyl acetate /petroleum ether: 0-30%) to obtain t-butyl (5S,5aS,6S,9R)-2-(4-((t-butoxycarbonyl)amino)-8-((triisopropylsilyl)ethynyl) naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (130 mg, 0.150 mmol, yield: 60.07%) as a white solid. ES-API: [M +H]⁺= 869.3.

Step II: T-butyl (5S,5aS,6S,9R)-2-(4-((t-butoxycarbonyl)amino)-8-((triisopropylsilyl)ethynyl) naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (130 mg, 0.150 mmol) was dissolved in dichloromethane (5 mL). At room temperature, m-chloroperoxybenzoic acid (purity 85%, 91.10 mg, 0.449 mmol) was added to the solution. After that, the reaction was stirred at room temperature for 1 hr. The reaction solution was added with a saturated sodium thiosulphate solution (5 mL) and extracted with dichloromethane (10 mLX3). The organic phases were combined, and then washed sequentially with a saturated sodium bicarbonate aqueous solution (20 mL) and saturated sodium chloride (20 mL). The organic phase was dried and concentrated to obtain t-butyl (5S,5aS,6S,9R)-2-(4-((t-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (120 mg, 0.133 mmol, yield: 89.03%) as a light yellow solid. ES-API: [M +H]⁺= 901.0.

Step III: ((2R)-2-fluoro-6-methylenetetrahydro-1H-pyrrolizin-7a(5H)-yl)-methanol (26.60 mg, 0.155 mmol) was dissolved in tetrahydrofuran (3 mL) and cooled to 0°C. Under a nitrogen atmosphere, sodium hydride (60%, 12.43 mg, 0.311 mmol) was added to the solution and stirred for 30 min. After that, t-butyl (5S,5aS,6S,9R)-2-(4-((t-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (70 mg, 0.078 mmol) was slowly added to the solution and stirred at 0°C for 2 hrs. The reaction solution was added with water (5 mL) to quench the reaction, and then extracted with ethyl acetate (20 mLX3). The organic phase was washed with saturated brine (30 mLX3), dried over anhydrous sodium sulfate, filtered and concentrated to obtain a crude product. The crude product was purified by flash silica gel column chromatography (ethyl acetate /petroleum ether: 0-70%) to obtain t-butyl (5S,5aS,6S,9R)-2-(4-((t-butoxycarbonyl)amino)-8-((triisopropylsilyl) ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.030 mmol, yield: 38.92%) as a yellow solid. ES-API: [M+H]⁺=992.1.

Step IV: A mixed solution of t-butyl (5S,5aS,6S,9R)-2-(4-((t-butoxycarbonyl)amino)-8-((triisopropylsilyl)ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.030 mmol), cesium fluoride (45.92 mg, 0.302 mmol) and N, N-dimethylformamide (2 mL) was stirred at room temperature for 2 hrs. The reaction mixture was added with water (10 mL) and extracted with ethyl acetate (15 mLX2). The organic phase was washed with saturated brine (15 mLX3), dried over anhydrous sodium sulfate, filtered and concentrated to obtain t-butyl (5S,5aS,6S,9R)-2-(4-((t-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (18 mg, 0.022 mmol, yield: 71.22%) as a yellow oil. ES-API: [M+H]⁺=835.1.

Step V: T-butyl (5S,5aS,6S,9R)-2-(4-((t-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (15 mg, 0.018 mmol) was dissolved in acetonitrile (2 mL), and cooled to 0°C. A hydrogen chloride-dioxane solution (4 M, 4 mL) was added to the solution, and reacted with stirring at 0°C for 2 hrs. The reaction solution was concentrated, and the residue was taken up in dichloromethane (3 mL) again and alkalized with ammonia in methanol (7 M) to an alkaline pH (pH 8). The obtained solution was concentrated, and the crude product was purified by preparative HPLC (ammonium bicarbonate method 1) to obtain 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-1-amine (Z686, 2.5 mg, 0.004 mmol, yield: 20.82%) as a brown solid. ES-API: [M+H]⁺=636.2.

### Example 78: Synthesis of (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptene (Z687)

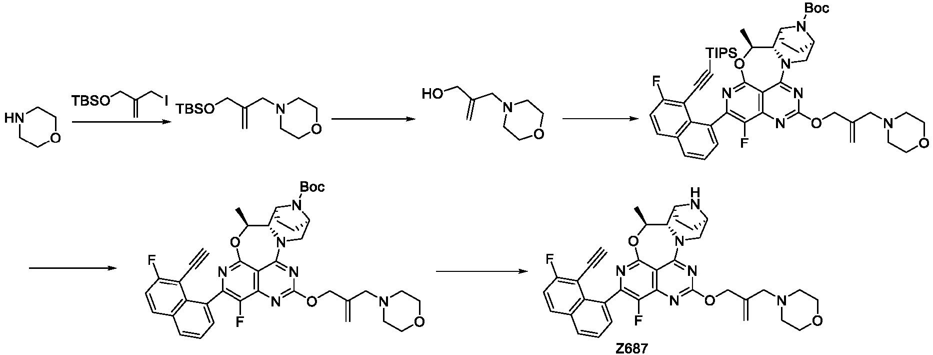

Step I: At room temperature, t-butyl ((2-(iodomethyl)allyl)oxy)dimethylsilane (3.58 g, 11.478 mmol) and potassium carbonate(4.0 g, 28.94 mmol) were sequentially added to a solution of morpholine (1.0 g, 11.478 mmol) in acetonitrile (50.0 mL), and stirred at room temperature for 3 hrs. After the reaction, the reaction solution was added with a saturated ammonium chloride solution (30 mL), washed with ethyl acetate (200 mL) and saturated brine (50 mLX3), dried over anhydrous sodium sulfate, and filtered. The crude product was purified by silica gel column chromatography[petroleum ether: ethyl acetate =100: 0-50: 50, (v/v)] to obtain 4-(2-(((t-butyldimethylsilyl)oxy)methyl)allyl)morpholine (2.67 g, yield: 85.8%). ES-API: [M+H]⁺=272.2.

Step II: In an iced water bath, a 4 M hydrochloric acid-dioxane solution (4.0 mL, 4 M, 16.0 mmol) was slowly added to a solution of 4-(2-(((t-butyldimethylsilyl)oxy)methyl)allyl)morpholine (700 mg, 2.578 mmol) in acetonitrile (10.0 mL). The reaction mixture was stirred at 0°C for 0.5 hr. The reaction was shown to be completed by LC/MS. The reaction solution was concentrated to dryness under reduced pressure. The residue was added to methanol (50 mL), and then sodium bicarbonate (2.0 g) was added, and stirred at room temperature for 1 hr. After filtration, the solvent was removed from the filtrate by rotary evaporation to dryness under reduced pressure to obtain 2-(morpholinemethyl) prop-2-en-1-ol (0.4 g, yield: 98%). ES-API: [M+H]⁺=158.2.

Step III: 2-(morpholinemethyl)prop-2-en-1-ol (18 0mg, 1.145 mmol) was dissolved in anhydrous tetrahydrofuran (10 mL). In an iced water bath, sodium hydride (90.8 mg, 2.27 mmol) was added, and then reacted for 30 min in the iced water bath. A solution of t-butyl (5S,5aS 6S 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (137.7 mg, 0.175 mmol) in tetrahydrofuran (2 mL) was added dropwise to the reaction solution, and reacted at 25°C for 30 min. The reaction was shown to be completed by LC/MS. The reaction solution was diluted with ethyl acetate (50 mL), washed with a saturated ammonium chloride solution (15 mLX2) and saturated brine (50 mLX3), dried over anhydrous sodium sulfate, and filtered. The crude product was purified by silica gel column chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab ]hepten-14-carboxylate (400 mg, crude product). ES-API: [M+H]⁺= 881.2.

Step IV: Cesium fluoride (600.0 mg, 3.947 mmol) was added to t-butyl (5S,5aS,6S,9R)-1-fluoro-2-(7-fluoro-8-((triisopropylsilyl)ethynyl)naphthalen-1-yl)-5-methyl-12-((2-(morpholinomethyl)allyl)oxy)-5a,6,7,8,9,10-hexahydro-5H-4-oxy-3,10a,11,13,14-pentaaza-6,9-methanonaphtho[1,8-ab]hepten-14-carboxylate (154.0 mg, 0.175 mmol) in N,N-dimethylformamide (10.0 mL), and the reaction mixture was stirred at room temperature for 2 hrs. The reaction was shown to be completed by LC/MS. The reaction solution was diluted with ethyl acetate (60 mL), washed with water (30 mLX2) and saturated brine (50 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]hepten-14-carboxylate (180 mg, crude product). ES-API: [M+H]⁺= 725.2.

Step V: In an iced water bath, a 4 M hydrochloric acid-dioxane solution (2.0 mL, 4 M, 8.0 mmol) was slowly added to a solution of t-butyl (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab ]hepten-14-carboxylate (127 mg, 0.175 mmol) in acetonitrile (10.0 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-((2-(morpholinomethyl)allyl)oxy)-5a,6,7,8,9,10-hexahydro-5H-4-oxa-3,10a,11,13,14-pentaaza-6,9-methanonaphtho[1,8-ab]heptene (Z687, 60 mg, yield: 54.88%) as a light yellow solid. ES-API: [M+H]⁺= 625.2. ¹H NMR (400 MHz, CD₃OD) δ 8.10-8.05 (m, 2H), 7.66 -7.55 (m, 2H), 7.45-7.38 (m, 1H), 5.39-5.31 (m, 2H), 5.20 (s, 1H), 5.06 -4.97(m, 2H), 4.56-4.49 (m, 1H), 4.09-4.04 (m, 1H), 3.79 -3.48 (m, 7H), 3.31-3.30(m, 2H), 3.20-3.10(m, 3H), 2.44 (s, 3H), 2.08-2.04(m, 1H), 1.86-1.76(m, 3H), 1.59-1.55(m, 3H).

### Example 79: Synthesis of (5S,5aS,6S,9R)-2-(8-ethynyl-7-fluoro-naphthalen-1-yl)-1-fluoro-12-((6R)-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z688)

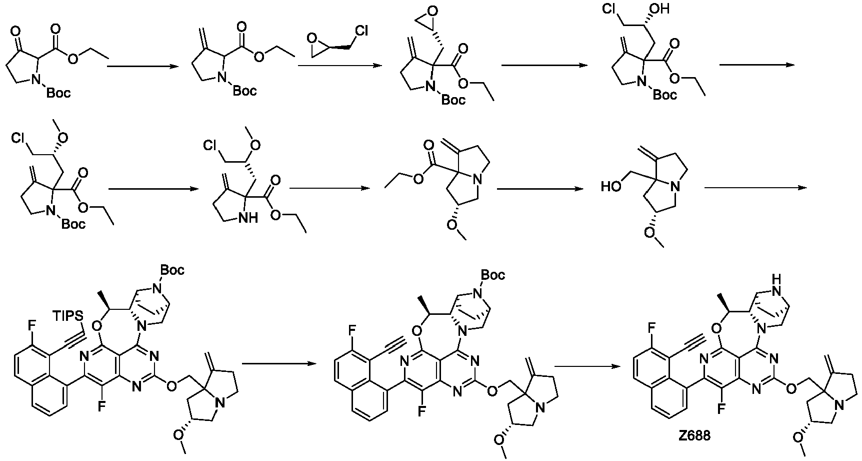

Step I: Methyltriphenylphosphonium bromide (22.6g, 63.3 mmol) was added to tetrahydrofuran (200 mL) and cooled to 0°C. Under a nitrogen atmosphere, a solution of potassium tert-butoxide in tetrahydrofuran (63 mL, 63 mmol, 1M) was added to the mixture at 0°C, and then the reaction was warmed to room temperature and stirred for 1 hr. 1-(t-butyl) 2-ethyl3-oxopyrrolidin-1,2-dicarboxylate (6.5 g, 25.3 mmol) was dissolved in tetrahydrofuran (20 mL), and slowly added dropwise to the above reaction solution under a nitrogen atmosphere. After that, the reaction solution was stirred at room temperature for 3 hrs. The reaction solution was added with saturated ammonium chloride (60 mL), and extracted with ethyl acetate (200 mLX3). The organic phase was washed with saturated brine (400 mL), dried over anhydrous sodium sulfate, filtered and concentrated. The crude product was purified by flash silica gel column chromatography (ethyl acetate/petroleum ether: 0%-8%) to obtain 1-(t-butyl) 2-ethyl 3-methylenepyrrolidin-1,2-dicarboxylate (2.8 g, 10.9 mmol, yield: 43.4%) as a colorless transparent liquid. ES-API: [M-55]⁺= 200.1.

Step II: At -70°C, lithium bis(trimethylsilyl)amide (22 mL, 1M, 21.8 mmol) was added to a solution of 1-(t-butyl) 2-ethyl 3-methylenepyrrolidin-1,2-dicarboxylate (2.8 g, 10.9 mmol) in dry tetrahydrofuran (20 mL), and stirred at this temperature for 1 hr. (R)-2-(chloromethyl) ethylene oxide (3.0 g, 32.7 mmol) was added to the reaction solution, and stirred overnight at room temperature. After the reaction, a saturated ammonium chloride solution (30 mL) was added to the solution, and extracted with ethyl acetate (200 mLX3). The organic phase was washed with saturated brine (200 mLX3), dried over anhydrous sodium sulfate, filtered and concentrated. The crude product was purified by flash silica gel column chromatography [dichloromethane:methanol =100:0-90:10, (v/v)] to obtain the target compound 1-(t-butyl)-2-ethyl3-methylene-2-(((R)- ethylene oxide -2-yl)methyl)pyrrolidin-1,2-dicarboxylate (2.4g, 7.72mmol, yield: 70.8%). ES-API: [M-55]⁺=256.1.

Step III: At room temperature, acetic acid (3 mL) and lithium chloride (3.2 g, 77.2 mmol) were added to a solution of 1-(t-butyl) 2-ethyl 3-methylene-2-(((R)- ethylene oxide -2-yl) methyl)pyrrolidin-1,2-dicarboxylate (2.4g, 7.72mmol) in dry acetonitrile (30 mL) and stirred at this temperature for 12 hrs. After the reaction, the solution was added with saturated ammonium chloride (20 mL), and extracted with ethyl acetate (30 mLX2). The organic phases were combined, dried over anhydrous sodium sulfate, and filtered. The solvent was removed from the filtrate by rotary evaporation to dryness under reduced pressure to obtain the crude target compound 1-(t-butyl) 2-ethyl2-((R)-3-chloro-2-hydroxypropyl)-3-methylenepyrrolidin-1,2-dicarboxylate (1.8 g, 5.17 mmol, yield: 67.0%). ES-API: [M-99]⁺=248.1.

Step IV: 1-(t-butyl) 2-ethyl 2-((R)-3-chloro-2-hydroxypropyl)-3-methylenepyrrolidin-1,2-dicarboxylate (420 mg, 1.21 mmol) was dissolved in dichloromethane(10mL). 1,8-bis(dimethylamino)naphthalene (906 mg, 4.23 mmol) and trimethyloxonium tetrafluoroborate (572 mg, 3.86 mmol) were sequentially added, and stirred at room temperature for 16 hrs. After the reaction, the reaction solution was diluted with dichloromethane (30 mLx2), washed with water (10 mLX2) and saturated brine (50 mLX3), dried over anhydrous sodium sulfate, filtered, and concentrated, to obtain a crude product. The crude product was purified by flash silica gel column chromatography (ethyl acetate /petroleum ether: 0%-30%) to obtain 1-(t-butyl) 2-ethyl2-((R)-3-chloro-2-methoxypropyl)-3-methylenepyrrolidin-1,2-dicarboxylate (278 mg, 0.77 mmol, yield: 63.6%). ES-API: [M-99]⁺= 262.1.

Step V: A hydrochloric acid-dioxane solution (5 mL, 4M, 20 mmol) was added to a solution of 1-(t-butyl) 2-ethyl 2-((R)-3-chloro-2-methoxypropyl)-3-methylenepyrrolidin-1,2-dicarboxylate (278 mg, 0.77 mmol) in dichloromethane (5 mL), and reacted with stirring at room temperature for 4 hrs. After the reaction, the solvent was removed by rotary evaporation to dryness under reduced pressure to obtain ethyl 2-((R)-3-chloro-2-methoxypropyl)-3-methylenepyrrolidin-2-carboxylate (156 mg, crude product). ES-API: [M+H]⁺=262.1.

Step VI: Sodium bicarbonate (504 mg, 6.00 mmol) was added to a solution of ethyl 2-((R)-3-chloro-2-methoxypropyl)-3-methylenepyrrolidin-2-carboxylate (156 mg, 0.60 mmol) in acetonitrile (5 mL), and reacted at 70°C for 4 hrs. After the reaction, the reaction solution was cooled to room temperature, and filtered. The solvent was removed from the filtrate by rotary evaporation to dryness under reduced pressure, to obtain a crude product. The crude product was purified by flash silica gel column chromatography (methanol /dichloromethane: 0%-10%) to obtain ethyl (6R)-6-methoxy-1-methylenetetrahydro-1H-pyrrolizin-7a(5H)-carboxylate (96 mg, 0.43 mmol, yield of two steps: 55.4%). ES-API: [M+H]⁺=226.1.

Step VII: Ethyl (6R) -6-methoxy-1-methylenetetrahydro-1H-pyrrolizin-7a(5H)-carboxylate (96 mg, 0.43 mmol) was dissolved in tetrahydrofuran (3 mL), and cooled to 0°C. Under a nitrogen atmosphere, a lithium aluminum hydride solution (2 mL, 2.00 mmol, 1 M solution in tetrahydrofuran) was slowly added dropwise to the solution, and stirred at 0°C for 2 hrs. After the reaction, sodium sulfate decahydrate (1.3 g, 4.00 mmol) was slowly added, stirred for 0.5 hr, dried over sodium sulfate, and filtered. The filtrate was concentrated, to obtain ((6R)-6-methoxy-1-methylenetetrahydro-1H-pyrrolizin-7a(5H)-yl)methanol (73 mg, 0.40 mmol, yield: 92.8%) as a colorless transparent liquid. ES-API: [M+H]⁺=184.1.

Step VIII: ((6R)-6-methoxy-1-methylenetetrahydro-1H-pyrrolizin-7a(5H)-yl)methanol (73 mg, 0.40 mmol) was dissolved in anhydrous tetrahydrofuran (4 mL). In an iced water bath, sodium hydride (48 mg, 1.20 mmol) was added, and reacted for 20 min in the ice bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (157 mg, 0.20 mmol) in tetrahydrofuran (2 mL) was added dropwise to the reaction solution, and stirred at normal temperature for 30 min. The reaction was shown to be completed by LC/MS. The reaction solution was diluted with ethyl acetate (10mL), washed with a saturated ammonium chloride solution (5 mLX2) and saturated brine (10 mLX3), dried over anhydrous sodium sulfate, and filtered. The crude product was purified by automated flash silica gel chromatography [(dichloromethane: methanol =0%-6%)mobile phase] to obtain t-butyl (5S,5aS,6S,9R)-1-fluoro-2-(7-fluoro-8-((triisopropylsilyl) ethynyl)naphthalen-1-yl)-12-(((6R)-6-methoxy-1-methylenetetrahydro)-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (65 mg, 0.07 mmol, yield: 35.9%). ES-API: [M+H]⁺= 907.1.

Step IX: Cesium fluoride (106 mg, 0.70 mmol) was added to t-butyl (5S,5aS,6S,9R)-1-fluoro-2-(7-fluoro-8-((triisopropylsilyl)ethynyl)naphthalen-1-yl)-12-(((6R)-6-methoxy-1-methylenetetrahydro)-1H-pyrrolizin-7a(5H)-yl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (65 mg, 0.07 mmol) in N,N-dimethylformamide (3 mL), and the reaction mixture was stirred at room temperature for 2 hrs. The reaction was shown to be completed by LC/MS. The reaction solution was diluted with ethyl acetate (10mL), washed with water (10 mLX2) and saturated brine (10 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-12-(((6R)-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (42 mg, 0.06 mmol, yield: 80%). ES-API: [M+H]⁺= 751.1.

Step X: In an iced water bath, a 4 M hydrochloric acid-dioxane solution (3.0 mL) was slowly added to t-butyl (5S,5aS,6S,9R)-2-(8-ethynyl-7-fluoronaphthalen-1-yl)-1-fluoro-12-(((6R)-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (42 mg, 0.06 mmol) in acetonitrile (2.0 mL). The reaction mixture was stirred at 0°C for 5 hrs. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-(((6R)-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z688, 22 mg, 0.03 mmol, yield: 56.4%) as a light yellow solid. ES-API: [M+H]⁺= 651.2.

### Example 80: Synthesis of (5S, 5aS, 6S, 9R)-2-(8-ethynyl-7-fluoronaphthalen-1-yl)-1-fluoro-12-(((6R)-6-flusoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho [1,8-ab]heptene (Z689) and (5S, 5aS, 6S, 9R)-2-(8-ethynyl-7-fluoronaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z690)

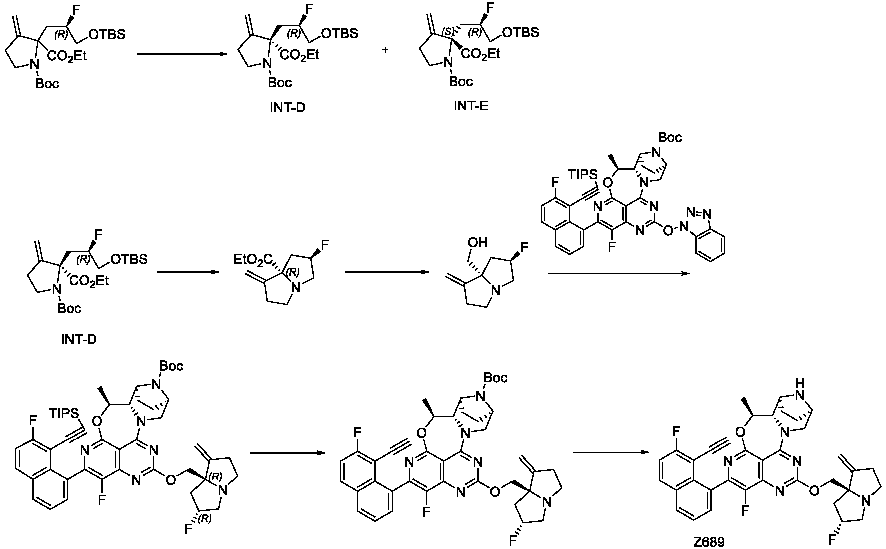

Step I: 1-(t-butyl) 2-ethyl 2-((R)-3-(t-butyldimethylsilyl)oxy)-2-fluoropropyl)-3-methylenepyrrolidin-1,2-dicarboxylate (1.5 g) was chirally resolved (separation column: CHIRALPAK IG 250mm*4.6mm*5um; mobile phase: isopropanol: n-hexane=5:95; flow rate:1 mL/min; column temperature: 30°C) to obtain two diastereomers. One isomeric compound was arbitrarily designated as 1-(t-butyl) 2-ethyl (R)-2-((R)-3-(t-butyldimethylsilyl)oxy)-2-fluoropropyl)-3-methylenepyrrolidin-1,2-dicarboxylate (INT-D, 720mg, peak 1, retention time=3.68 min), ES-API:[M+Na]⁺=468.3. The other isomeric compound was arbitrarily designated as 1-(t-butyl) 2-ethyl (S)-2-((R)-3-(t-butyldimethylsilyl)oxy)-2-fluoropropyl)-3-methylenepyrrolidin-1,2-dicarboxylate (INT-E, 330 mg, peak 2, retention time=4.44 min), ES-API:[M+Na]⁺=468.3.

Step II: Thionyl chloride (10 mL, 137.86 mmol) was added to 1-(t-butyl) 2-ethyl (R)-2-((R)-3-(t-butyldimethylsilyl)oxy)-2-fluoropropyl)-3-methylenepyrrolidin-1,2-dicarboxylate (INT-D, 700 mg, 1.57 mmol). The reaction was stirred at 45°C for 24 hrs. The reaction solution was added with thionyl chloride (10 mL, 137.86 mmol). The reaction was stirred at 65°C for 2 days. The reaction solution was concentrated. The residue was added with dichloromethane (5 mL), and cooled to 0°C. Triethyl amine (0.22 mL, 1.57 mmol) was added. The reaction was stirred at room temperature for 30 min. The reaction was quenched with water (50 mL), and extracted with dichloromethane (30 mLX2). The organic phase was washed with saturated brine (30 mL), dried over anhydrous sodium sulfate, filtered and concentrated. The residue was purified by flash silica gel column chromatography (0-60%ethyl acetate /petroleum ether) to obtain ethyl (6R,7aR)-6-fluoro-1-methylenetetrahydro-1H-pyrrolizin-7a(5H)-carboxylate (200 mg, yield: 58%). ES-API:[M+H]⁺=214.2.

Step III: A lithium aluminium hydride /tetrahydrofuran solution (1.8 mL, 1.8 mmol) was added dropwise to a solution of ethyl (6R,7aR)-6-fluoro-1-methylenetetrahydro-1H-pyrrolizin-7a(5H)-carboxylate (200 mg, 0.94 mmol) in tetrahydrofuran (6 mL), and stirred at 0°C for 1 hr. The reaction was quenched by sequentially adding 3 drops of water and a 15% sodium hydroxide aqueous solution (0.1 mL). The mixture was stirred at room temperature for 30 min, and filtered through diatomite. The filtrate was concentrated to obtain the crude product

((6R,7aR)-6-fluoro-1-methylenetetrahydro-1H-pyrrolizin-7a(5H)-yl)methanol (120 mg, yield: 74%) as a yellow oil. ES-API: [M+H]⁺=172.1.

Step IV: Sodium hydride (71 mg, 1.77 mmol) and tetrahydrofuran(10 mL) were added to a round-bottom flask. The reaction solution was cooled to 0°C, and a solution of ((6R,7aR)-6-fluoro-1-methylenetetrahydro-1H-pyrrolizin-7a(5H)-yl)methanol (91 mg, 0.53 mmol) in anhydrous tetrahydrofuran (1 mL) was added to the reaction solution, and reacted at room temperature for 10 min. The reaction solution was cooled to 0°C. T-butyl (5S,5aS,6S,9R)-12-(1H-benzo [d][1,2,3]triazole-1-yl)oxy)-1-fluoro-2-(7 -fluoro-S-(triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho [1,8-ab]hepten-14-carboxylate (380 mg, 0.44 mmol) was added to the reaction solution, and reacted at room temperature for 1 hr. The reaction solution was added with water (30 mL), and extracted with ethyl acetate (20 mLX2). The organic phase was washed with saturated brine (30 mL), dried over anhydrous sodium sulfate, filtered, and concentrated to obtain t-butyl (5S, 5aS, 6S, 9R)-1-fluoro-12-(((6R,7aR)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (375 mg, yield: 94%). ES-API: [M+H]⁺=895.3.

Step V: Cesium fluoride (1 g, 6.58 mmol) was added to t-butyl (5S, 5aS, 6S, 9R)-1-fluoro-12-(((6R,7aR)-6-fluoro-1-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (490 mg, crude product) in N,N-dimethylformamide (5 mL). The reaction mixture was stirred at room temperature for 2 hr. The reaction was shown to be completed by LC/MS. The reaction solution was diluted with ethyl acetate (100 mL), and washed with water (30 mL) and saturated brine (30 mLX2). The organic phase, dried over anhydrous sodium sulfate, filtered and, concentrated. The residue was purified by flash silica gel column chromatography (0-5%methanol /dichloromethane) to obtain t-butyl (5S, 5aS, 6S, 9R)-2-(8-ethynyl-7-fluoronaphthalen-1-yl)-1-fluoro-12-(((6R,7aR)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (300 mg, yield: 74%). ES-API: [M+H]⁺= 739.2.

Step VI: In an iced water bath, a 4 M hydrochloric acid-dioxane solution (6mL) was slowly added to a solution of t-butyl (5S, 5aS, 6S, 9R)-2-(8-ethynyl-7-fluoronaphthalen-1-yl)-1-fluoro-12-(((6R,7aR)-6-fluoro-1-methylenetetrahydro-1H-pyrrolizin-7a(5H)-yl)methoxy)-5-methyl-5a, 6,7,8,9,10-hexahydro-5H-4-oxa-3,10a,11,13,14-pentaaza-6,9-methanonaphtho[1,8-ab]hepten-14-carboxylate (300 mg, crude product) in acetonitrile (3 mL). The reaction mixture was stirred at room temperature for 1 hr. The reaction was shown to be completed by LC/MS. The reaction solution was concentrated to dryness under reduced pressure, and the residue was dissolved in saturated sodium bicarbonate aqueous solution and dichloromethane. The reaction solution was extracted with dichloromethane/methanol (10: 1, 50mL). The organic phase was washed with saturated brine (30 mL), dried over anhydrous sodium sulfate, filtered, and concentrated to obtain (5S,5aS,6S,9R)-2-(8-ethynyl-7-fluoronaphthalen-1-yl)-1-fluoro-12-(((6R,7aR)-6-fluoro-1-methylenetetrahydro- 1H-pyrrolizin-7a(5H)-yl)methoxy)-5-methyl-5 a,6,7, 8, 9,10-hexahydro-5H-4-oxa-3,10a,11,13,14-pentaaza-6,9-methanonaphtho[1,8-ab]heptene (Z689, 230mg, yield: 88%) as a yellow solid. ES-API: [M+H]⁺= 639.2. ¹H NMR (400MHz, CD₃OD): 8.10-8.05 (m, 2H), 7.80-7.38 (m, 3H), 5.43-5.20 (m, 3H), 4.60-4.40 (m, 3H), 4.20-3.80 (m, 2H), 3.68-3.50 (m, 4H), 3.20-3.14 (m, 1H), 2.83-2.71 (m, 1H), 2.27-1.95 (m, 3H), 1.90-1.77 (m, 6H), 1.58-1.55 (m, 3H), 0.90-0.85 (m, 1H).

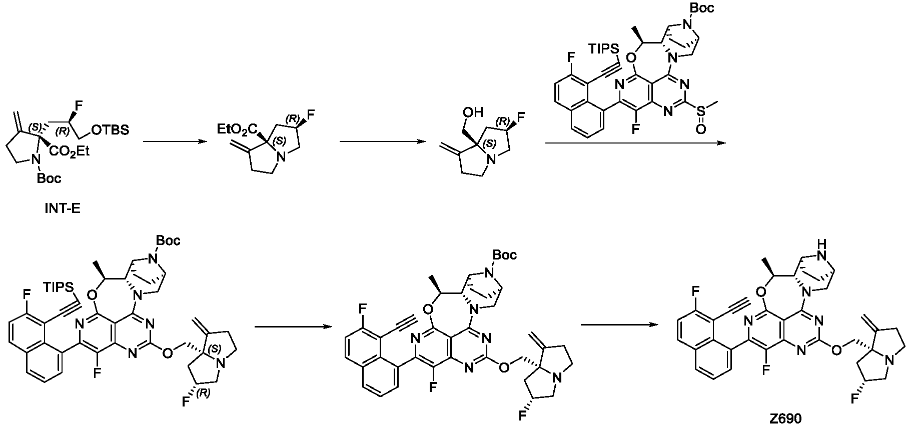

Step I: Thionyl chloride (10 mL, 137.86 mmol) was added to 1-(t-butyl) 2-ethyl (S)-2-((R)-3-(t-butyldimethylsilyl)oxy)-2-fluoropropyl)-3-methylenepyrrolidin-1,2-dicarboxylate (INT-E, 330 mg, 0.74 mmol). The reaction was stirred at 45°C for 24 hrs. The reaction solution was added with thionyl chloride (10 mL, 137.86 mmol). The reaction was stirred at 65°C for 2 days. The reaction solution was concentrated. The residue was added with dichloromethane (5 mL), and cooled to 0°C. The reaction was quenched with water (50 mL), and extracted with dichloromethane (30 mLX2). The organic phase was washed with saturated brine (30 mL), dried over anhydrous sodium sulfate, filtered and concentrated. The residue was purified by flash silica gel column chromatography (0-60%ethyl acetate /petroleum ether) to obtain ethyl (6R,7aS)-6-fluoro-1-methylenetetrahydro-1H-pyrrolizin-7a(5H)-carboxylate (75 mg, yield: 47%). ES-API:[M+H]⁺= 214.2.

Step II: A lithium aluminium hydride /tetrahydrofuran solution (0.75 mL, 0.75 mmol) was added dropwise to a solution of ethyl (6R,7aS)-6-fluoro-1-methylenetetrahydro-1H-pyrrolizin-7a(SH)-carboxylate (75 mg, 0.37 mmol) in tetrahydrofuran (5 mL), and stirred at 0°C for 1 hr. The reaction was quenched by sequentially adding 3 drops of water and a 15% sodium hydroxide aqueous solution (0.1 mL). The mixture was stirred at room temperature for 30 min, and filtered through diatomite. The filtrate was concentrated to obtain the crude product ((6R,7aS)-6-fluoro-1-methylenetetrahydro-1H-pyrrolizin-7a(5H)-yl)methanol (45 mg, yield: 69%) as a yellow oil. ES-API: [M+H]⁺=172.1.

Step III: 60% sodium hydride (16 mg, 0.41 mmol) and tetrahydrofuran (6 mL) were added to a round-bottom flask. The reaction solution was cooled to 0°C, and a solution of ((6R,7aS)-6-fluoro-1-methylenetetrahydro-1H-pyrrolizin-7a(5H)-yl)methanol (26 mg, 0.15 mmol) in anhydrous tetrahydrofuran (1 mL) was added to the reaction solution, and reacted at room temperature for 10 min. The reaction solution was cooled to 0°C. T-butyl (5S, 5aS, 6S, 9R)-1-fluoro-2-(7-fluoro-8-(triisopropylsilyl)ethynyl)naphthalen-1-yl)-5-methyl-12-(methylsulfinyl)-5a,6,7,8,9,10-hexahydro-5H-4-oxa-3,10a, 11,13,14-pentaaza-6,9-methanonaphtho[1,8-ab]hepten-14-carboxylate (80 mg, 0.1 mmol) was added to the reaction solution, and reacted at room temperature for 1 hr. The reaction solution was added with water (30 mL), and extracted with ethyl acetate (20 mLX2). The organic phase was washed with saturated brine (30 mL), dried over anhydrous sodium sulfate, filtered, and concentrated to obtain t-butyl (5S, 5aS, 6S, 9R)-1-fluoro-12-(((6R,7aS)-6-fluoro-1-methylenetetrahydro-1H-pyrrolizin-7a(5H)-yl)methoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (90 mg, crude product). ES-API: [M+H]⁺=895.3.

Step IV: Cesium fluoride (300 mg, 1.97 mmol) was added to t-butyl (5S, 5aS, 6S, 9R)-1-fluoro-12-(((6R,7aS)-6-fluoro-1-methylenetetrahydro-1H-pyrrolizin-7a(5H)-ylmethoxy)-2-(7-fluoro-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]hepten-14-carboxylate (90 mg, crude product) in N,N-dimethylformamide (3 mL). The reaction mixture was stirred at room temperature for 2 hr. The reaction was shown to be completed by LC/MS. The reaction solution was diluted with ethyl acetate (100 mL), and washed with water (30 mL) and saturated brine (30 mLX2). The organic phase, dried over anhydrous sodium sulfate, filtered and , concentrated. The residue was purified by flash silica gel column chromatography (0-5%methanol /dichloromethane) to obtain t-butyl (5S, 5aS, 6S, 9R)-2-(8-ethynyl-7-fluoronaphthalen-1-yl)-1-fluoro-12-(((6R,7aS)-6-fluoro-1-methylenetetrahydro-1H-pyrrolizin-7a(5H)-yl)methoxy)-5-methyl-5a, 6,7,8,9,10-hexahydro-5H-4-oxa-3,10a,11,13,14-pentaaza-6,9-methanonaphtho[1,8-ab]hepten-14-carboxylate (70 mg, crude product). ES-API: [M+H]⁺= 739.2.

Step V: In an iced water bath, a 4 M hydrochloric acid-dioxane solution (3 mL) was slowly added to a solution of t-butyl (5S, 5aS, 6S, 9R)-2-(8-ethynyl-7-fluoronaphthalen-1-yl)-1-fluoro-12-(((6R,7aS)-6-fluoro-1-methylenetetrahydro-1H-pyrrolizin-7a(5H)-yl)methoxy)-5-methyl-5a, 6,7,8,9,10-hexahydro-5H-4-oxa-3,10a,11,13,14-pentaaza-6,9-methanonaphtho[1,8-ab]hepten-14-carboxylate (70 mg, crude product) in acetonitrile (3 mL). The reaction mixture was stirred at room temperature for 1 hr. The reaction was shown to be completed by LC/MS. The reaction solution was concentrated to dryness under reduced pressure. The residue was purified by preparative HPLC (ammonium bicarbonate method 1) to obtain (5S, 5aS, 6S, 9R)-2-(8-ethynyl-7-fluoronaphthalen-1-yl)-1-fluoro-12-(((6R,7aS)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z690, 10mg, yield: 16%) as a white solid. ES-API: [M+H]⁺= 639.2. ¹HNMR(400MHz, CD₃OD): 8.10-8.05(m, 2H), 7.80-7.38 (m, 3H), 5.43-5.21 (m, 3H), 4.52-4.32 (m, 3H), 4.20-3.94 (m, 2H), 3.68-3.50 (m, 4H), 3.32-3.19(m, 3H), 2.27-2.05 (m, 3H), 1.90-1.80(m, 6H), 1.58-1.55 (m, 3H).

### Example 81: Synthesis of (5S, 5aS, 6S, 9R)-2-(8-ethynyl-7-fluoronaphthalen-1-yl)-1-fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z691)

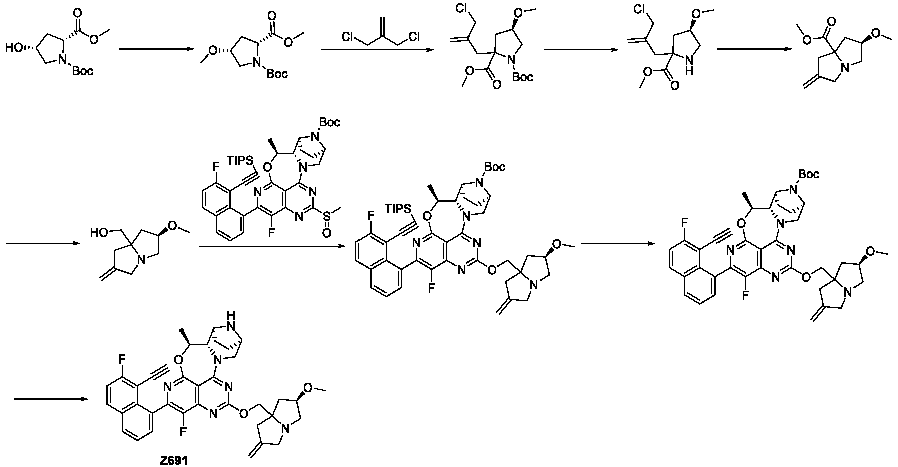

Step I: Methyl (2R, 4R)-4-hydroxyl-1-{(2-methylprop-2-yl)oxy]carbonyl}tetrahydropyrrole-2-carboxylate (3 g, 12.231 mmol) was dissolved in tetrahydrofuran (40 mL), and cooled to 0°C in an iced water bath. Sodium hydride (465 mg, 11.619 mmol) was added batchwise to the reaction solution, and then stirred at 0°C for 30 min. Iodomethane (1.5 mL, 24.462 mmol) was added to the solution, and the reaction mixture was slowly heated to room temperature and stirred for 2 hrs. The reaction was shown to be completed by LC/MS. The reaction was quenched with saturated ammonium chloride solution (20 mL). The reaction solution was extracted with ethyl acetate (15 mLX 3). The organic phase was washed with saturated brine (15 mLX 2), and dried over anhydrous sodium sulfate, filtered and concentrated. The crude product was purified by automated flash silica gel chromatography (ethyl acetate: petroleum ether= 0- 20%) to obtain 1-(t-butyl) 2-methyl (2R, 4R)-4-methoxypyrrolidin-1,2-dicarboxylate (1807 mg, yield: 57%). ES-API: [M-55]⁺= 204.1.

Step II: Under a nitrogen atmosphere, 1-(t-butyl) 2-methyl (2R, 4R)-4-methoxypyrrolidin-1,2-dicarboxylate (1 g, 3.857 mmol) was dissolved in anhydrous tetrahydrofuran (30 mL), and the mixture was allowed to stand in a dry ice-acetone bath. Lithium diisopropylamide (3.9 mL, 7.713 mmol) was slowly added dropwise to the above solution, and stirred at this temperature for 1 hr. A solution of 3-chloro-2-chloromethylpropene (2.410 g, 19.283mmol) in anhydrous tetrahydrofuran (5 mL) was slowly added by a syringe to the solution, and the reaction solution was continuously stirred at -78°C for 1 hr, then slowly heated room temperature and stirred overnight. The reaction was shown to be completed by LC/MS. The reaction was quenched with a saturated ammonium chloride solution (30 mL). The reaction solution was extracted with ethyl acetate (20 mLX3). The organic phase was washed with saturated brine (15 mLX3), dried over anhydrous sodium sulfate, filtered and concentrated. The crude product was purified by automated flash silica gel chromatography (ethyl acetate: petroleum ether= 0- 9.5%) to obtain 1-(t-butyl) 2-methyl (4R)-2-(2-(chloromethyl)allyl)-4-methoxypyrrolidin-1,2-dicarboxylate (943 mg, yield: 70%). ES-API: [M-55]⁺= 292.1.

Step III: At room temperature, a 4 M hydrochloric acid-dioxane solution (10 mL) was slowly added to a solution of 1-(t-butyl) 2-methyl (4R)-2-(2-(chloromethyl)allyl)-4-methoxypyrrolidin-1,2-dicarboxylate (943 mg, 2.711 mmol) in acetonitrile (5 mL). The reaction mixture was stirred at room temperature for 2 hrs. The reaction was shown to be completed by LC/MS. The reaction solution was concentrated to dryness under reduced pressure to obtain the crude product methyl (4R)-2-(2-(chloromethyl)allyl)-4-methoxypyrrolidin-2-carboxylate (671 mg). ES-API: [M+H]⁺= 248.2.

Step IV: At room temperature, sodium bicarbonate (1.138 g, 13.545 mmol) and potassium iodide (90 mg, 0.542 mmol) were added to a solution of methyl (4R)-2-(2-(chloromethyl)allyl)-4-methoxypyrrolidin-2-carboxylate (671 mg, 2.709 mmol) in acetonitrile (15 mL), and the reaction mixture was stirred at room temperature for 1.5 hrs. The reaction was shown to be completed by LC-MS. The reaction solution was filtered, and the insoluble matter was washed with acetonitrile. The filtrate was concentrated under reduced pressure. The residue was purified by silica gel column chromatography (ethyl acetate: petroleum ether= 0- 45%) to obtain the crude product methyl (2R)-2-methoxy-6-methylenetetrahydro-1H-pyrrolizin-7a(5H)-carboxylate (613 mg). ES-API: [M+H]⁺= 212.2.

Step V: Under a nitrogen atmosphere, methyl (2R)-2-methoxy-6-methylenetetrahydro-1H-pyrrolizin-7a(5H)-carboxylate (211 mg, 0.999 mmol) was dissolved in anhydrous tetrahydrofuran (5 mL), and the mixture was allowed to stand in an iced water bath. Lithium aluminium hydride (2 mL, 1.998 mmol, 1 M solution in tetrahydrofuran) was slowly added dropwise to the above solution, and then stirred at this temperature for 0.5 hr. The reaction was shown to be completed by LC/MS. The reaction solution was added with sodium sulfafe decahydrate (2.0 eq) to quench the reaction, stirred at room temperature for 0.5 hr, and filtered to remove the insoluble matter. The filtrate was concentrated under reduced pressure, to obtain the crude product ((2R)-2-methoxy-6-methylenetetrahydro-1H-pyrrolizin-7a(5H)-yl)methanol (162 mg, yield: 89%). ES-API: [M+H]⁺= 184.1.

Step VI: ((2R)-2-methoxy-6-methylenetetrahydro-1H-pyrrolizin-7a(5H)-yl)methanol (56 mg, 0.305 mmol) was dissolved in anhydrous tetrahydrofuran (4 mL). In an iced water bath, sodium hydride (12 mg, 0.305 mmol) was added, and reacted for 10 min in the iced water bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (80 mg, 0.102 mmol) in tetrahydrofuran (2 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure, to obtain the crude product t-butyl (5S, 5aS, 6S, 9R)-1-fluoro-2-(7-fluoro-8-(triisopropylsilyl) ethynyl)naphthalen-1-yl)-12-((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (110 mg). ES-API: [M+H]⁺= 907.3.

Step VII: Cesium fluoride (184 mg, 1.213 mmol) was added to t-butyl (5S, 5aS, 6S, 9R)-1-fluoro-2-(7-fluoro-8-(triisopropylsilyl)ethynyl)naphthalen-1-yl)-12-((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-nonaphtho[1,8-ab]hepten-14-carboxylate (110 mg, 0.121 mmol) in N,N-dimethylformamide (5 mL), and the reaction mixture was stirred at room temperature for 0.5 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain the crude product t-butyl (5S, 5aS, 6S, 9R)-2-(8-ethynyl-7-fluoronaphthalen-1-yl)-1-fluoro-12-((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (91 mg). ES-API: [M+H]⁺= 751.3.

Step VIII: In an iced water bath, a 4 M hydrochloric acid-dioxane solution (5 mL) was slowly added to a solution of t-butyl (5S, 5aS, 6S, 9R)-2-(8-ethynyl-7-fluoronaphthalen-1-yl)-1-fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (91 mg, 0.121 mmol) in acetonitrile (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15 mL-45-55-13 min) to obtain (5S,5aS,6S,9R)-2-(8-ethynyl-7-fluoronaphthalen-1-yl)-1-fluoro-12-(((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z691, 18.51 mg, yield of three steps: 28%) as an off-white solid. ES-API: [M+H]⁺= 651.2. ¹H NMR (400 MHz, CD₃OD) δ 8.11-8.05 (m, 2 H), 7.66-7.54 (m, 2 H), 7.46-7.39 (m, 1 H), 5.39 (d, J= 16.0 Hz, 1 H), 4.99 (s, 2 H), 4.56-4.52 (m, 1 H), 4.39 (t, J= 12.5 Hz, 1 H), 4.29-4.25 (dd, J1= 3.5 Hz, J2= 12.5 Hz, 1 H), 4.09-4.07 (m, 2 H), 3.79-3.49 (m, 4 H), 3.29-3.15 (m, 7 H), 2.87-2.80 (m, 2 H), 2.49-2.46 (m, 1 H), 2.34-2.31 (m, 1 H), 2.07-1.99 (m, 2 H), 1.87-1.76 (m, 3 H), 1.57 (t, J= 9.0 Hz, 3 H).

### Example 82: Synthesis of (5S, 5aS, 6S, 9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z692)

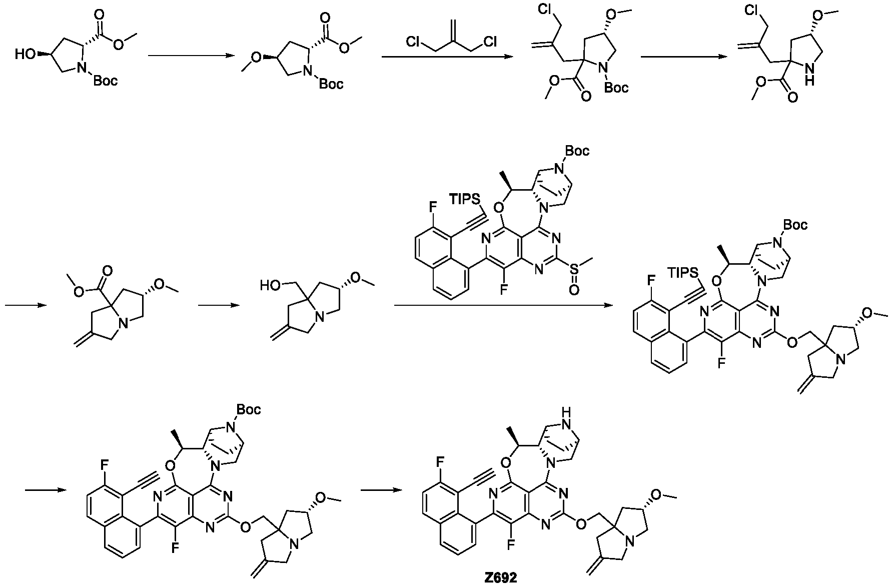

Step I: 1-(t-butyl) 2-methyl (2R,4S)-4-hydroxylpyrrolidin-1,2-dicarboxylate (3 g, 12.231 mmol) was dissolved in acetonitrile (60 mL). Iodomethane (26.04 g, 183.464 mmol) and silver oxide (14.17 g, 61.155 mmol) were added to the solution. The reaction was stirred at room temperature for 17 hrs. The reaction was shown to be completed by LC/MS. The reaction solution was filtered, and concentrated to obtain a crude product. The crude product was purified by automated flash silica gel chromatography (ethyl acetate: petroleum ether= 0- 20%) to obtain 1-(t-butyl) 2-methyl (2R,4S)-4-methoxypyrrolidin-1,2-dicarboxylate (3 g, 11.570 mmol, yield: 94.59%). ES-API: [M-Boc+H]⁺= 159.1.

Step II: Under a nitrogen atmosphere, 1-(t-butyl) 2-methyl (2R,4S)-4-methoxypyrrolidin-1,2-dicarboxylate (1000 mg, 3.857 mmol) was dissolved in anhydrous tetrahydrofuran(30 mL), and the mixture was allowed to stand in a dry ice-acetone bath. Lithium diisopropylamide (3.9 mL, 7.713 mmol) was slowly added dropwise to the above solution, and stirred at this temperature for 1 hr. A solution of 3-chloro-2-chloromethylpropene (2.410 g, 19.283mmol) in anhydrous tetrahydrofuran(5 mL) was slowly added by a syringe to the solution, and the reaction solution was continuously stirred at -78°C for 1 hr, then slowly heated room temperature and stirred overnight. The reaction was shown to be completed by LC/MS. The reaction was quenched with a saturated ammonium chloride solution (30 mL). The reaction solution was extracted with ethyl acetate (20 mLx3). The organic phase was washed with saturated brine (15 mLX3), dried over anhydrous sodium sulfate, filtered and concentrated. The crude product was purified by automated flash silica gel chromatography (ethyl acetate: petroleum ether= 0- 9.5%) to obtain 1-(t-butyl) 2-methyl (4S)-2-(2-(chloromethyl)allyl)-4-methoxypyrrolidin-1,2-dicarboxylate (500 mg, 1.437 mmol, yield: 37.27%). ES-API: [M-55]⁺= 292.1.

Step III: At room temperature, a 4 M hydrochloric acid-dioxane solution (2 mL) was slowly added to a solution of 1-(t-butyl) 2-methyl (4S)-2-(2-(chloromethyl)allyl)-4-methoxypyrrolidin-1,2-dicarboxylate (500 mg, 1.437 mmol) in acetonitrile (2 mL). The reaction mixture was stirred at room temperature for 2 hrs. The reaction was shown to be completed by LC/MS. The reaction solution was concentrated to dryness under reduced pressure to obtain the crude product methyl (4S)-2-(2-(chloromethyl)allyl)-4-methoxypyrrolidin-2-carboxylate (408.49 mg, 1.437 mmol, yield: 100.00%). ES-API: [M+H]⁺= 248.2.

Step IV: At room temperature, sodium bicarbonate (482.55 mg, 5.744 mmol) and potassium iodide (238.38 mg, 1.436 mmol) were added to a solution of methyl (4S)-2-(2-(chloromethyl)allyl)-4-methoxypyrrolidin-2-carboxylate (408 mg, 1.436 mmol) in acetonitrile (10 mL), and the reaction mixture was stirred at room temperature for 1.5 hrs. The reaction was shown to be completed by LC-MS. The reaction solution was filtered, and the insoluble matter was washed with acetonitrile. The filtrate was concentrated under reduced pressure, and the residue was purified by flash silica gel column chromatography (methanol: dichloromethane= 0- 3%) to obtain the crude product methyl (2S)-2-methoxy-6-methylenetetrahydro-1H-pyrrolizin-7a(5H)-carboxylate (200 mg, 0.947 mmol, yield: 65.93%). ES-API: [M+H]⁺= 212.2.

Step V: Under a nitrogen atmosphere, methyl (2S)-2-methoxy-6-methylenetetrahydro-1H-pyrrolizin-7a(5H)-carboxylate (150 mg, 0.710 mmol) was dissolved in anhydrous tetrahydrofuran (5 mL), and the mixture was allowed to stand in an iced water bath. Lithium aluminium hydride (2 mL, 1.998 mmol, 1 M solution in tetrahydrofuran) was slowly added dropwise to the above solution, and then stirred at this temperature for 0.5 hr. The reaction was shown to be completed by LC/MS. The reaction solution was added with sodium sulfafe decahydrate (2.0 eq) to quench the reaction, stirred at room temperature for 0.5 hr, and filtered to remove the insoluble matter. The filtrate was concentrated under reduced pressure, to obtain the crude product ((2S)-2-methoxy-6-methylenetetrahydro-1H-pyrrolizin-7a(5H)-yl)methanol (100 mg, 0.546 mmol, yield: 76.86%). ES-API: [M+H]⁺= 184.1.

Step VI: ((2S)-2-methoxy-6-methylenetetrahydro-1H-pyrrolizin-7a(5H)-yl)methanol (34.19 mg, 0.187 mmol) was dissolved in anhydrous tetrahydrofuran(4 mL). In an iced water bath, sodium hydride (14.92 mg, 0.373 mmol) was added, and reacted for 10 min in the iced water bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (75 mg, 0.093 mmol) in tetrahydrofuran (2 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure, to obtain the crude product t-butyl (5S, 5aS, 6S, 9R)-1-fluoro-2-(7-fluoro-8-(triisopropylsilyl) ethynyl)naphthalen-1-yl)-12-(((2S)-2-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (60 mg, 0.066 mmol, yield: 70.90%). ES-API: [M+H]⁺= 907.3.

Step VII: Cesium fluoride (100.46 mg, 0.661 mmol) was added to t-butyl (5S, 5aS, 6S, 9R)-1-fluoro-2-(7-fluoro-8-(triisopropylsilyl)ethynyl)naphthalen-1-yl)-12-((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (60 mg, 0.066 mmol) in N,N-dimethylformamide (5 mL), and the reaction mixture was stirred at room temperature for 0.5 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain the crude product t-butyl (5S, 5aS, 6S, 9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40 mg, 0.053 mmol, yield: 80.55%). ES-API: [M+H]⁺= 751.3.

Step VIII: In an iced water bath, hydrogen chloride in dioxane solution (4M, 5 mL) was slowly added to a solution of t-butyl (5S, 5aS, 6S, 9R)-2-(8-ethynyl-7-fluoronaphthalen-1-yl)-1-fluoro-12-((2R)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (40 mg, 0.053 mmol) in acetonitrile (2 mL). The reaction mixture was stirred at 0 °C for 0.5 hr. The reaction was shown to be completed by LC/MS. The reaction solution was concentrated to dryness under reduced pressure, and the residue was purified by preparative HPLC (ammonium bicarbonate method 1) to obtain (5S, 5aS, 6S, 9R)-2-(8-ethynyl-7-fluoronaphthalen-1-yl)-1-fluoro-12-(((2S)-2-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene as a white solid (Z692, 15 mg, 0.023 mmol, yield: 43.27%). ES-API: [M+H]⁺= 651.2.

### Example 83: Synthesis of (5S,5aS,6S,9R)-2-(8-ethynyl-7-fluoronaphthalen-1-yl)-1-fluoro-5-methyl-12-((1-(morpholinomethyl)cyclopropyl)-5a,6,7,8,9,10-hexahydro-5H-4-oxa-3,10a,11,13,14-pentaaza-6,9-methanonaphtho[1,8-ab]heptene (Z693)

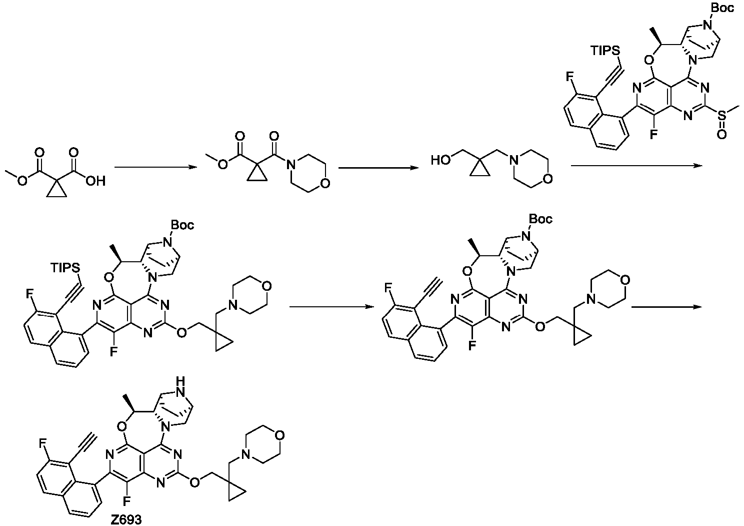

Step I: At 0°C, oxalyl chloride (6.86 mL, 79.93 mmol) and N,N-dimethylformamide (0.05 mL) were added to a solution of 1-(methoxycarbonyl)cyclopropane-1-carboxylic acid (2.88 g, 19.99 mmol) in dichloromethane (50.0 mL), and then the reaction solution was stirred at 35°C for 3 hrs. After 3 hrs, the solvent was removed by rotary evaporation to dryness under reduced pressure. At 0°C, dichloromethane (80.0 mL), triethyl amine (8.33 mL, 59.95 mmol) and morpholine (2.089 g, 23.98 mmol) were added, and reacted with stirring at room temperature for 3 hrs. After the reaction, the reaction solution was filtered. The solvent was removed from the filtrate by rotary evaporation to dryness under reduced pressure to obtain a crude product. The crude product was purified by flash silica gel column chromatography (ethyl acetate /petroleum ether: 0%-8%) to obtain methyl 1-(morpholine-4-carbonyl)cyclopropane-1-carboxylate (2.0 g, yield: 47%) as a light yellow viscous liquid. ES-API: [M+H]⁺=214.1.

Step II: Methyl 1-(morpholine-4-carbonyl)cyclopropane-1-carboxylate (1.50 g, 7.01 mmol) was added to dry tetrahydrofuran (20.0 mL), and cooled to 0-5°C in an iced water bath. A solution of lithium aluminium hydride in tetrahydrofuran (14.0 mL, 1M, 14.0 mmol) was added dropwise. After reaction at this temperature for 1 hr, water (0.3 mL) and 15% sodium hydroxide solution (0.3 mL) were sequentially added, and the reaction solution was filtered through diatomite. The solvent was removed from the filtrate by rotary evaporation to dryness under reduced pressure to obtain (1-(morpholinemethyl)cyclopropyl)methanol (2.0 g, yield: 60%). ES-API: [M+H]⁺=172.1.

Step III: (1-(morpholinemethyl)cyclopropyl)methanol (200 mg, 1.162 mmol) was dissolved in anhydrous tetrahydrofuran (10 mL). In an iced water bath, sodium hydride (116.3 mg, 2.907 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (408 mg, 0.5188 mmol) in tetrahydrofuran (2 mL) was added dropwise to the reaction solution, and reacted at 25°C for 30 min. The reaction was shown to be completed by LC/MS. The reaction solution was diluted with ethyl acetate (50 mL), washed with a saturated ammonium chloride solution (15 mLX2) and saturated brine (50 mLX3), dried over anhydrous sodium sulfate, and filtered. The crude product was purified by automated flash silica gel chromatography (dichloromethane: methanol =15:1) to obtain t-butyl (5S,5aS,6S,9R)-1-fluoro-2-(7-fluoro-8-((triisopropylsilyl)ethynyl)naphthalen-1-yl)-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (200 mg, yield: 43%). ES-API: [M+H]⁺= 895.3.

Step IV: Cesium fluoride (679.0 mg, 4.467 mmol) was added to t-butyl (5S,5aS,6S,9R)-1-fluoro-2-(7-fluoro-8-((triisopropylsilyl)ethynyl)naphthalen-1-yl)-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (200 mg, 0.223 mmol) in N,N-dimethylformamide (10.0 mL), and the reaction mixture was stirred at room temperature for 2 hrs. The reaction was shown to be completed by LC/MS. The reaction solution was diluted with ethyl acetate (60 mL), washed with water (30 mLX2) and saturated brine (50 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-5-methyl-12-((1-(morpholinomethyl)cyclopropyl) methoxy)-5a,6,7,8,9,10-hexahydro-5H-4-oxa-3,10a,11,13,14-pentaaza-6,9-methanonaphtho[1,8-ab]hepten-14-carboxylate (160 mg, yield: 96%). ES-API: [M+H]⁺= 739.2.

Step V: In an iced water bath, a 4 M hydrochloric acid-dioxane solution (5.0 mL, 4M, 20.0 mmol) was slowly added to a solution of t-butyl (5S,5aS,6S,9R)-2-(8-ethynyl-7-fluoronaphthalen-1-yl)-1-fluoro-5-methyl-12-((1-(morpholinomethyl)cyclopropyl)methoxy)-5a,6,7,8,9,10-hexahydro-5H-4-oxy-3,10a,11,13,14-pentaaza-6,9-methanonaphtho[1,8-ab]hepten-14-carboxylate (160 mg, 0.217 mmol) in acetonitrile (10.0 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-5-methyl-12-((1-(morpholinomethyl)cyclopropyl)-5a, 6,7,8,9,10-hexahydro-5H-4-oxa-3,10a,11,13,14-pentaaza-6,9-methanonaphtho[1,8-ab]heptene (Z693, 27 mg, yield: 19.52%) as a light yellow solid. ES-API: [M+H]+= 639.2. ¹H NMR (400 MHz, CD₃OD) δ 8.10 -8.05 (m, 2H), 7.66 -7.54 (m, 2H), 7.42 (q, *J* = 9.1 Hz, 1H), 5.42 -5.33 (m, 1H), 4.56 -4.49 (m, 1H), 4.49 -4.43 (m, 1H), 4.34-4.38 (m, 1H), 4.094.07 (m, 1H), 3.77-3.47(m, 7H), 3.21 -3.15(m, 1H), 2.51 -2.34 (m, 6H), 2.07 (m, 1H), 1.88-1.78 (m, 3H), 1.59-1.55(m, 3H), 0.72 -0.69 (m, 2H), 0.52-0.46 (m, 2H).

### Example 84: Synthesis of (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z694)

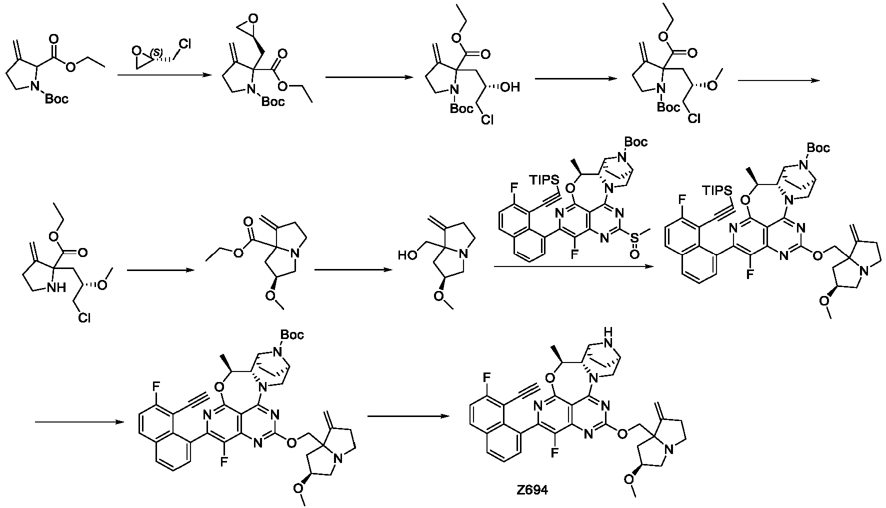

Step I: At -70°C, lithium bis(trimethylsilyl)amide (4.218 mL, 5.484 mmol) was added to a solution of 1-(t-butyl) 2-ethyl 3-methylenepyrrolidin-1,2-dicarboxylate (700 mg, 2.742 mmol) in dry tetrahydrofuran (20.0 mL), and stirred at this temperature for 1 hr. After 1 hr, (S)-(+)-epichlorohydrin (761.00 mg, 8.225 mmol) was added to the reaction solution, and stirred overnight at room temperature. After the reaction, a saturated ammonium chloride solution (30 mL) was added to the solution, and extracted with ethyl acetate (20 mLX4). The organic phase was washed with saturated brine (20 mLX3), dried over anhydrous sodium sulfate, and filtered. The crude product was purified by flash silica gel column chromatography [dichloromethane:methanol =100:0-90:10, (v/v)] to obtain the target compound 1-(t-butyl) 2-ethyl 3-methylene-2-(((S)- ethylene oxide -2-yl)methyl)pyrrolidin-1,2-dicarboxylate (400 mg, 1.285 mmol, yield: 46.85%). ES-API: [M-56+H]⁺=255.9.

Step II: At room temperature, acetic acid (1 mL, 17.469 mmol) and lithium chloride (1000 mg, 23.590 mmol) were added to a solution of 1-(t-butyl) 2-ethyl 3-methylene-2-(((S)- ethylene oxide -2-yl)methyl)pyrrolidin-1,2-dicarboxylate (360 mg, 1.156 mmol) in dry tetrahydrofuran (10.0 mL), and stirred at this temperature for 12 hrs. After the reaction, the solution was added with saturated ammonium chloride (10 mL), and extracted with ethyl acetate (20 mLX2). The organic phases were combined, dried over anhydrous sodium sulfate, and filtered. The solvent was removed from the filtrate by rotary evaporation to dryness under reduced pressure to obtain the target compound 1-(t-butyl) 2-ethyl 2-((S)-3-chloro-2-hydroxylpropyl)-3-methylenepyrrolidin-1,2-dicarboxylate (150 mg, 0.431 mmol, yield: 37.30%). ES-API: [M-Boc+H]⁺=248.2.

Step III: 1-(t-butyl) 2-ethyl 2-((S)-3-chloro-2-hydroxylpropyl)-3-methylenepyrrolidin-1,2-dicarboxylate (280 mg, 1.058 mmol) was dissolved in dichloromethane (5 mL). [8-(dimethylamino)-1-naphthalenyl]dimethyl amine (792.44 mg, 3.703 mmol) and trimethyloxonium tetrafluoroborate (469.34 mg, 3.173 mmol) were added, and reacted overnight at room temperature. After the reaction, the solution was added with a saturated ammonium chloride solution (10 mL), and extracted with dichloromethane (20 mLX4). The organic phase was washed with saturated brine (10 mLX3), dried over anhydrous sodium sulfate, filtered and concentrated. The crude product was purified by flash silica gel column chromatography [dichloromethane:methanol =100:0-90:10,(v/v)] to obtain the target compound 1-(t-butyl) 2-ethyl 2-((S)-3-chloro-2-methoxypropyl)-3-methylenepyrrolidin-1,2-dicarboxylate (180 mg, 0.646 mmol, yield: 61.05%). ES-API: [M-Boc+H]⁺=262.1.

Step IV: 1-(t-butyl) 2-ethyl 2-((S)-3-chloro-2-methoxypropyl)-3-methylenepyrrolidin-1,2-dicarboxylate (100 mg, 0.276 mmol) was dissolved in acetonitrile (1 mL). Hydrochloric acid-dioxane (2 mL, 0.415 mmol) was added, and reacted at room temperature for 1 hr. The reaction solution was concentrated under reduced pressure to obtain ethyl 2-((S)-3-chloro-2-methoxypropyl)-3-methylenepyrrolidin-2-carboxylate (50 mg, 0.191 mmol, yield: 69.12%).

Step V: Sodium bicarbonate (100 mg, 1.190 mmol) was added to a solution of ethyl 2-((S)-3-chloro-2-methoxypropyl)-3-methylenepyrrolidin-2-carboxylate (45 mg, 0.172 mmol) in acetonitrile (3.0 mL), and reacted at 70°C for 0.5 hrs. After the reaction, the reaction solution was cooled to room temperature, and filtered. The solvent was removed from the filtrate by rotary evaporation to dryness under reduced pressure, to obtain a crude product. The crude product was purified by flash silica gel column chromatography (methanol /dichloromethane: 0%-10%) to obtain ethyl (6S)-6-methoxy-1-methylenetetrahydro-1H-pyrrolizin-7a(5H)-carboxylate (35 mg, 0.155 mmol, yield: 90.37%). ES-API: [M+H]⁺=226.1.

Step VI: Ethyl (6S)-6-methoxy-1-methylenetetrahydro-1H-pyrrolizin-7a(5H)-carboxylate (30 mg, 0.133 mmol) was dissolved in tetrahydrofuran (2mL), and cooled to 0°C. Under a nitrogen atmosphere, a lithium aluminum hydride solution (0.399 mL, 0.399 mmol) was added to the solution. The reaction was stirred at 0°C for 1 hr. The reaction solution was added with sodium sulfafe decahydrate (500 mg), dried over anhydrous sodium sulfate, and filtered. The filtrate was concentrated to obtain ((6S)-6-methoxy-1-methylenetetrahydro-1H-pyrrolizin-7a(5H)-yl) methanol (22 mg, 0.120 mmol, yield: 90.16%) as a colorless transparent liquid. ES-API: [M +H]⁺=184.1.

Step VII: ((6S)-6-methoxy-1-methylenetetrahydro-1H-pyrrolizin-7a(5H)-yl)methanol (16.28 mg, 0.089 mmol) was dissolved in anhydrous tetrahydrofuran(2 mL). In an iced water bath, sodium hydride (1.07 mg, 0.044 mmol) was added, and then reacted for 30 min in the iced water bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (35 mg, 0.044 mmol) in tetrahydrofuran (2 mL) was added dropwise to the reaction solution, and reacted at room temperature for 30 min. The reaction was shown to be completed by LC/MS. The reaction solution was diluted with ethyl acetate (10 mL), washed with a saturated ammonium chloride solution (5 mLX 2) and saturated brine (50 mLX 3), dried over anhydrous sodium sulfate, filtered and concentrated. The crude product was purified by automated flash silica gel chromatography (dichloromethane: methanol =15:1) to obtain t-butyl (5S,5aS,6S,9R)-1-fluoro-2-(7-fluoro-8-((triisopropylsilyl)ethynyl) naphthalen-1-yl)-12-(((6S)-6-methoxy-1-methylenetetrahydro)-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (32 mg, 0.035 mmol, yield: 79.42%). ES-API: [M+H]⁺= 907.3.

Step VIII: Cesium fluoride (33.49 mg, 0.220 mmol) was added to t-butyl (5S,5aS,6S,9R)-1-fluoro-2-(7-fluoro-8-((triisopropylsilyl)ethynyl)naphthalen-1-yl)-12-(((6S)-6-methoxy-1-methylenetetrahydro)-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (20 mg, 0.022 mmol) in N,N-dimethylformamide (1.0 mL), and the reaction mixture was stirred at room temperature for 2 hrs. The reaction was shown to be completed by LC/MS. The reaction solution was diluted with ethyl acetate (10 mL), washed with water (10 mLX2) and saturated brine (10 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (15 mg, 0.020 mmol, yield: 90.81%). ES-API: [M+H]⁺= 751.3.

Step IX: In an iced water bath, a 4 M hydrochloric acid-dioxane solution (1.0 mL) was slowly added to a solution of t-butyl (5S,5aS,6S,9R)-2-(8-ethynyl-7-fluoronaphthalen-1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (13 mg, 0.017 mmol) in acetonitrile (1.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen- 1-yl)-1-fluoro-12-(((6S)-6-methoxy-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z694, 5 mg, 0.008 mmol, yield: 44.38%) as a light yellow solid. ES-API: [M+H]⁺= 651.3.

### Example 85: Synthesis of ((5R, 5aS, 6S, 9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-5-yl)methanol (Z654)

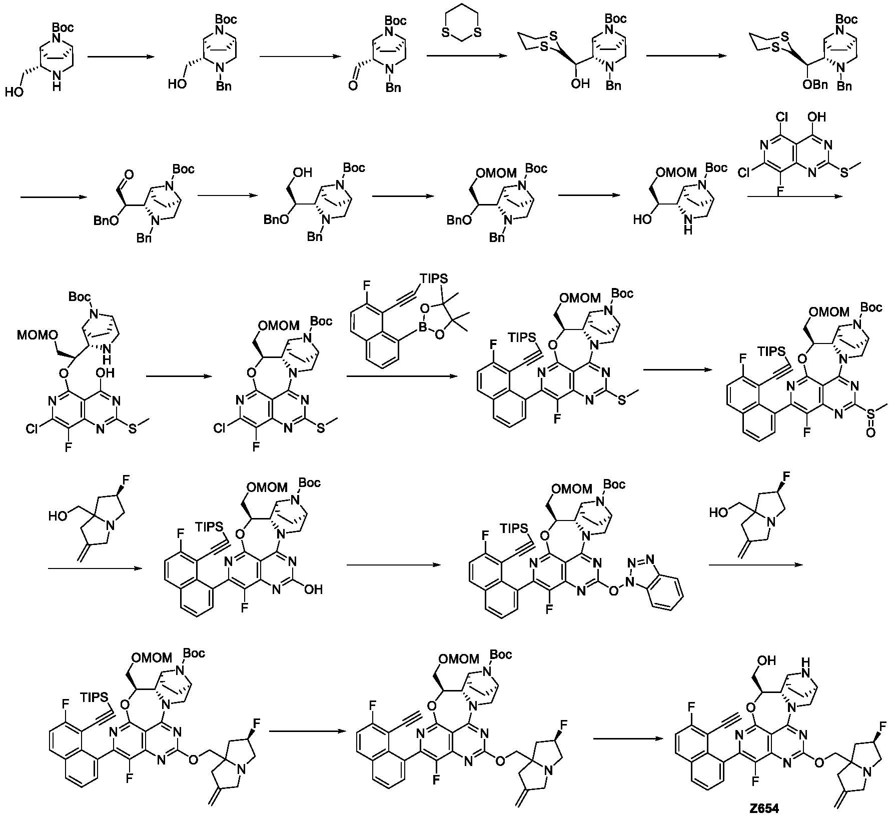

Step I: T-butyl (1R, 2S, 5S)-2-(hydroxymethyl)-3,8-diazadicyclo[3.2.1]octane-8-carboxylate (800 mg, 3.301 mmol) was dissolved in acetonitrile (20 mL). At room temperature, potassium carbonate (685 mg, 4.952 mmol) and benzyl bromide (0.589 mL, 4.952 mmol) were sequentially added to the reaction solution, and the reaction solution was stirred overnight at room temperature. The reaction was shown to be completed by LC/MS. The reaction solution was added with water (30 mL) to quench the reaction, and then extracted with ethyl acetate (30 mLX3). The organic phases were combined, washed with saturated brine (20 mLX2), dried over anhydrous sodium sulfate, filtered and concentrated. The crude product was purified by automated flash silica gel chromatography (tetrahydrofuran: petroleum ether= 0- 30%) to obtain t-butyl (1R, 2S, 5S)-3-benzyl-2-(hydroxymethyl)-3,8-diazadicyclo[3.2.1]octane-8-carboxylate (1 g, yield: 91%). ES-API: [M+ H]⁺= 333.3.

Step II: In an iced water bath, t-butyl (1R, 2S, 5S)-3-benzyl-2-(hydroxymethyl)-3,8-diazadicyclo[3.2.1]octane-8-carboxylate (1 g, 3.008 mmol) was dissolved in dichloromethane (50 mL). Dess-Martin periodinane (1.66 g, 3.910 mmol) was added to the reaction solution, and stirred at 0°C for 1 hr. The reaction was shown to be completed by LC/MS. The reaction solution was diluted with dichloromethane (30 mL), and added with a saturated sodium sulfite solution (30 mL) to quench the reaction. The organic phase was washed with a saturated sodium bicarbonate solution (20 mLX2), dried over anhydrous sodium sulfate, filtered, and concentrated to obtain the crude product t-butyl (1R, 2S, 5S)-3-benzyl-2-formyl-3,8-diazadicyclo[3.2.1]octane-8-carboxylate (1 g, yield: 100.6%). ES-API: [M+ H]⁺= 331.1.

Step III: Under a nitrogen atmosphere, n-butyl lithium (1.816 mL, 4.540 mmol) was slowly added to a solution of 1,3-dithiane (728 mg, 6.053 mmol) in anhydrous tetrahydrofuran (30 mL) at -20°C, and the reaction solution was stirred at -20°C for 0.5 hr. Then, the reaction solution was cooled to -78°C in a dry ice-acetone bath, s-butyl lithium (17.5 mL, 22.816 mmol, 1.3 M solution in hexane) was slowly added to the reaction solution, and stirred at -78°C for 1 hr. The reaction solution was added with a solution of t-butyl (1R, 2S, 5S)-3-benzyl-2-formyl-3,8-diazadicyclo[3.2.1]octane-8-carboxylate (1 g, 3.026 mmol) in tetrahydrofuran (10 mL), slowly heated to room temperature and stirred for 1 hr. The reaction was shown to be completed by LC/MS. The reaction solution was quenched with a saturated ammonium chloride solution (50 mL), and extracted with ethyl acetate (30 mL X 3). The organic phases were combined, washed with saturated brine (15 mL X 2), dried over anhydrous sodium sulfate, filtered, and concentrated to obtain the crude product t-butyl (1R, 2S, 5S)-2-((R)-(1,3-dithio-2-yl)(hydroxyl)methyl)-3-benzyl-3,8-diazadicyclo[3.2.1]octane-8-carboxylate (1352 mg). ES-API: [M+ H]⁺= 451.1.

Step IV: In an iced water bath, t-butyl (1R, 2S, 5S)-2-((R)-(1,3-dithio-2-yl)(hydroxyl)methyl)-3-benzyl-3,8-diazadicyclo[3.2.1]octane-8-carboxylate (1352 mg, 3.000 mmol) was dissolved in N,N-dimethylformamide (10 mL). At 0°C, the reaction solution was added with sodium hydride (240 mg, 6.000 mmol) and stirred at this temperature for 10 min. Then, benzyl bromide (0.714 mL, 6.000 mmol) was added, and the reaction solution was stirred at 0°C for 0.5 hr, heated to room temperature and stirred for 0.5 hr. The reaction was shown to be completed by LC/MS. The reaction solution was added with water (30 mL) to quench the reaction, and then extracted with ethyl acetate (30 mLX3). The organic phases were combined, washed with saturated brine (20 mLX2), dried over anhydrous sodium sulfate, filtered and concentrated. The crude product was purified by automated flash silica gel chromatography (tetrahydrofuran: petroleum ether= 0- 15%) to obtain t-butyl (1R, 2S, 5S)-3-benzyl-2-((R)-(benzyloxy)(1,3-dithio-2-yl)methyl)-3,8-diazadicyclo[3.2.1]octane-8-carboxylate (974 mg, yield: 60%). ES-API: [M+ H]⁺= 541.1.

Step V: At -10°C, a solution of t-butyl (1R, 2S, 5S)-3-benzyl-2-((R)-(benzyloxy)(1,3-dithio-2-yl)methyl)-3,8-diazadicyclo[3.2.1]octane-8-carboxylate (938 mg, 1.735 mmol) in acetonitrile (10 mL) was slowly added dropwise to a solution of N-bromosuccinimide (3.087 g, 17.345 mmol) in acetonitrile(16 mL) and water (4 mL), and the reaction solution was stirred at -10°C for 0.5 hr. The reaction was shown to be completed by LC/MS. The reaction solution was added with a saturated sodium sulfite solution (30 mL) to quench the reaction, and then extracted with dichloromethane (20 mLX3). The organic phases were combined, washed with saturated brine (15 mLX2), dried over anhydrous sodium sulfate, filtered, and concentrated to obtain the crude product t-butyl (1S, 2S, 5R)-3-benzyl-2-((R)-1-(benzyloxy)-2-oxyethyl)-3,8-diazadicyclo[3.2.1]octane-8-carboxylate (781 mg). ES-API: [M+ H]⁺= 451.2.

Step VI: Under a nitrogen atmosphere, in an iced water bath, a borane-tetrahydrofuran solution (8.5 mL, 8.5 mmol) was slowly added dropwise to a solution of t-butyl (1S, 2S, 5R)-3-benzyl-2-((R)-1-(benzyloxy)-2-oxyethyl)-3,8-diazadicyclo[3.2.1]octane-8-carboxylate (766 mg, 1.7 mmol) in tetrahydrofuran (30 mL). The reaction solution was stirred at 0°C for 0.5 hr, slowly heated to room temperature and stirred for 0.5 hr. The reaction was shown to be completed by LC/MS. The reaction solution was cooled to 0°C, slowly added with methanol to quench the reaction, stirred at room temperature for 0.5 hr and concentrated under reduced pressure, to obtain the crude product t-butyl (1S, 2S, 5R)-3-benzyl-2-((R)-1-(benzyloxy)-2-hydroxyethyl)-3,8-diazadicyclo[3.2.1] octane-8-carboxylate (768 mg). ES-API: [M+ H]⁺= 453.3.

Step VII: In an iced water bath, N,N-diisopropylethyl amine (0.162 mL, 0.928 mmol) was added to a solution of t-butyl (1S, 2S, 5R)-3-benzyl-2-((R)-1-(benzyloxy)-2-hydroxyethyl)-3,8-diazadicyclo[3.2.1]octane-8-carboxylate (210 mg, 0.464 mmol) in dichloromethane (10 mL), and the reaction mixture was stirred at 0°C for 0.5 hr. The reaction solution was added with bromomethylmethyl ether (0.057 mL, 0.696 mmol), and continuously stirred at 0°C for 2.5 hrs. The reaction was shown to be completed by LC/MS. The reaction solution was concentrated and the crude product was purified by automated flash silica gel chromatography (tetrahydrofuran: petroleum ether= 0- 10%) to obtain t-butyl (1S, 2S, 5R)-3-benzyl-2-((R)-1-(benzyloxy)-2-(methoxymethoxy)ethyl)-3,8-diazadicyclo[3.2.1]octane-8-carboxylate (185 mg, yield: 80%). ES-API: [M+ H]⁺= 497.4.

Step VIII: Under hydrogen atmosphere, catalytically effective amounts of glacial acetic acid and 10% palladium on carbon (53 mg) were sequentially added to a solution of t-butyl (1S, 2S, 5R)-3-benzyl-2-((R)-1-(benzyloxy)-2-(methoxymethoxy)ethyl)-3,8-diazadicyclo[3.2.1]octane-8-carboxylate (61 mg, 0.123 mmol) in 2,2,2-trifluoroether-1-ol (2 mL), and the reaction mixture was stirred overnight at room temperature. The reaction was shown to be completed by LC/MS. The reaction solution was filtered through diatomite, and concentrated under reduced pressure to obtain the crude product t-butyl (1S, 2S, 5R)-2-((R)-1-hydroxyl-2-(methoxymethoxy)ethyl)-3,8-diazadicyclo[3.2.1]octane-8-carboxylate (51 mg). ES-API: [M+ H]⁺= 317.3.

Step IX: In an iced water bath, t-butyl (1S, 2S, 5R)-2-((R)-1-hydroxyl-2-(methoxymethoxy) ethyl)-3,8-diazadicyclo[3.2.1]octane-8-carboxylate (76 mg, 0.240 mmol) were dissolved in tetrahydrofuran (10 mL). The reaction solution was added with sodium hydride (48 mg, 1.200 mmol, 60% dispersed in an oil), and stirred at 0°C for 30 min. At 0°C, the reaction solution was added with 5,7-dichloro-8-fluoro-2-(methylthio)pyridine[4,3-d]pyrimidine-4-ol (202 mg, 0.720 mmol), and the reaction mixture was slowly heated to 60°C and stirred for 1.5 hrs. The reaction was shown to be completed by LC/MS. The reaction solution was slowly added with a saturated ammonium chloride solution (20 mL), and extracted with dichloromethane (20 mLX3). The organic phase was dried over anhydrous sodium sulfate, filtered, and concentrated to obtain t-butyl (1S, 2S, 5R)-2-((R)-1-(7-chloro-8-fluoro-4-hydroxyl-2-(methylthio)pyridine[4,3-d] pyrimidine-5-yl)oxy)-2-(methoxymethoxy)ethyl)-3,8-diazadicyclo[3.2.1]octane-8-carboxylate (129 mg, yield: 96%). ES-API: [M+ H]⁺= 560.2.

Step X: In an iced water bath, 2-(7-azobenzotriazole)-N,N,N',N'-tetramethyluronium hexafluorophosphate (162 mg, 0.425 mmol) was added to a solution of t-butyl (1S, 2S, 5R)-2-((R)-1-(7-chloro-8-fluoro-4-hydroxyl-2-(methylthio)pyridine[4,3-d]pyrimidine-5-yl)oxy)-2-(methoxymethoxy)ethyl)-3,8-diazadicyclo[3.2.1]octane-8-carboxylate (119 mg, 0.212 mmol) and N,N-diisopropylethyl amine (0.15 mL, 0.850 mmol) in dichloromethane (10 mL). The reaction mixture was stirred at 0°C for 0.5 hr, heated to room temperature and stirred for 1.5 hrs. The reaction was shown to be completed by LC/MS. The reaction solution was concentrated and the crude product was purified by automated flash silica gel chromatography (ethyl acetate: petroleum ether= 0- 20%) to obtain t-butyl (5R, 5aS, 6S, 9R)-2-chloro-1-fluoro-5-((methoxymethoxy) methyl)-12-(methylthio)-5a,6,7,8,9,10-hexahydro-5H-4-oxa-3,10a,11,13,14-pentaaza-6,9-methanonaphtho[1,8-ab]hepten-14-carboxylate (79 mg, yield:69%). ES-API: [M+ H]⁺= 542.2.

Step XI: Under a nitrogen atmosphere, t-butyl (5R, 5aS, 6S, 9R)-2-chloro-1-fluoro-5-((methoxymethoxy)methyl)-12-(methylthio)-5a, 6,7,8,9,10-hexahydro-5H-4-oxa-3,10a, 11,13,14-pentaaza-6,9-methanonaphtho[1,8-ab]hepten-14-carboxylate (79 mg, 0.146 mmol), ((2-fluoro-8-(4,4,5,5-tetramethyl-1,3,2-dioxabenzaldehyde-2-yl)naphthalen-1-yl)ethynyl)triisopropylsilane (132 mg, 0.292 mmol), potassium phosphate (93 mg, 0.438 mmol) and [n-butyldi(1-adamantanyl)phosphine](2-amino-1,1'-biphenyl-2-yl)palladium (II) mesylate(16 mg, 0.022 mmol) were dissolved in tetrahydrofuran (5 mL) and water (1 mL). The reaction mixture was stirred at 80°C in a microwave generator for 0.5 hr. The reaction was shown to be completed by LC/MS. The reaction solution was concentrated and the crude product was purified by automated flash silica gel chromatography (petroleum ether/ethyl acetate = 0 - 20%), to obtain t-butyl (5R, 5aS, 6S, 9R)-1-fluoro-2-(7-fluoro-8-((triisopropylsilyl)ethynyl)naphthalen-1-yl)-5-((methoxymethoxy) methyl)-12-(methylthio)-5a,6,7,8,9,10-hexahydro-5H-4-oxa-3,10a,11,13,14-pentaaza-6,9-methanonaphtho[1,8-ab]hepten-14-carboxylate (79 mg, yield: 65%). ES-API: [M+ H]⁺= 832.3.

Step XII: T-butyl (5R, 5aS, 6S, 9R)-1-fluoro-2-(7-fluoro-8-((triisopropylsilyl)ethynyl) naphthalen-1-yl)-5-((methoxymethoxy)methyl)-12-(methylthio)-5a, 6,7,8,9,10-hexahydro-5H-4-oxa-3,10a,11,13,14-pentaaza-6,9-methanonaphtho[1,8-ab]hepten-14-carboxylate (79 mg, 0.095 mmol) was dissolved in anhydrous dichloromethane (10 mL). m-chloroperoxybenzoic acid (16 mg10 mL 0.143 mmol) was added to the solution, and the reaction solution was stirred at room temperature for 0.5 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5R, 5aS, 6S, 9R)-1-fluoro-2-(7-fluoro-8-((triisopropylsilyl)ethynyl) naphthalen-1-yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (81 mg). ES-API: [M+ H]⁺= 848.3.

Step XIII: ((2R)-2-fluoro-6-methylenetetrahydro-1H-pyrrolizin-7a(5H)-yl)methanol (33 mg, 0.190 mmol) was dissolved in anhydrous tetrahydrofuran (5 mL). In an iced water bath, sodium hydride (11 mg, 0.285 mmol) was added, and reacted for 10 min in the iced water bath. A solution of t-butyl (5R, 5aS, 6S, 9R)-1-fluoro-2-(7-fluoro-8-((triisopropylsilyl)ethynyl)naphthalen-1-yl)-5-((methoxymethoxy)methyl)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (81 mg, 0.095 mmol) in tetrahydrofuran (5 mL) was added dropwise to the reaction solution, and reacted for 30 min at 0 °C. The reaction was detected by LCMS and showed that the raw materials disappeared, and a hydrolysate was largely produced. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure. The crude product was purified by automated flash silica gel chromatography (methanol /dichloromethane= 0 - 10%) to obtain t-butyl (5R, 5aS, 6S, 9R)-1-fluoro-2-(7-fluoro-8-((triisopropylsilyl)ethynyl) naphthalen-1-yl)-12-hydroxyl-5-((methoxymethoxy)methyl)-5a,6,7,8,9,10-hexahydro-5H-4-oxa-3,10a,11,13,14-pentaaza-6,9-methanonaphtho[1,8-ab]hepten-14-carboxylate (70 mg, yield: 92%). ES-API: [M+ H]⁺= 802.3.

Step XIV: BOP reagent (78 mg, 0.174 mmol) was added to t-butyl (5R, 5aS, 6S, 9R)-1-fluoro-2-(7-fluoro-8-((triisopropylsilyl)ethynyl)naphthalen-1-yl)-12-hydroxyl-5-((methoxymethoxy) methyl)-5a,6,7,8,9,10-hexahydro-5H-4-oxa-3,10a, 11,13,14-pentaaza-6,9-methanonaphtho[1,8-ab]hepten-14-carboxylate (70 mg, 0.087 mmol), cesium carbonate (57 mg, 0.174 mmol) and tetrahydrofuran(10 mL). The reaction was stirred at room temperature for 1 hrs, quenched with water (20 mL), and extracted with ethyl acetate (20 X 2). The organic phase was washed with saturated brine (10 mL), dried over anhydrous sodium sulfate, filtered and concentrated. The crude product was purified by automated flash silica gel chromatography (ethyl acetate /petroleum ether= 0 - 50%) to obtain t-butyl (5R, 5aS, 6S, 9R)-12-((1H-benzo[d][1,2,3]triazole-1-yl)oxy)-1-fluoro-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]hepten-14-carboxylate (71 mg, yield: 89%). ES-API: [M+H]⁺= 919.4.

Step XV: ((2R)-2-fluoro-6-methylenetetrahydro-1H-pyrrolizin-7a(5H)-yl)methanol (13 mg, 0.116 mmol) was dissolved in anhydrous tetrahydrofuran (5 mL). In an iced water bath, sodium hydride (6 mg, 0.155 mmol) was added, and reacted for 10 min in the iced water bath. A solution of t-butyl (5R, 5aS, 6S, 9R)-12-((1H-benzo[d][1,2,3]triazole-1-yl)oxy)-1-fluoro-2-(7-fluoro-8-((triisopropylsilyl)ethynyl)naphthalen-1-yl)-5-((methoxymethoxy)methyl)-5a,6,7,8,9,10-hexahydro-5H-4-oxa-3,10a,11,13,14-pentaaza-6,9-methanonaphtho[1,8-ab]hepten-14-carboxylate (71 mg, 0.077 mmol) in tetrahydrofuran (5 mL) was added dropwise to the reaction solution, and reacted for 30 min at 0 °C. The reaction was detected by LCMS and showed that the raw materials disappeared, and a hydrolysate was largely produced. The reaction solution was diluted with ethyl acetate (10 mL), washed with a saturated ammonium chloride solution (10 mLX 2) and saturated brine (10 mLX3), dried over anhydrous sodium sulfate, filtered, and concentrated under reduced pressure. The crude product was purified by automated flash silica gel chromatography (methanol /dichloromethane= 0 - 3%) to obtain t-butyl (5R, 5aS, 6S, 9R)-1-fluoro-12-(((2R)-2-fluoro-6-methylenetetrahydro-1H-pyrrolizin-7a(5H)-yl)methoxy)-2-(7-fluoro-8-(triisopropylsilyl)ethynyl)naphthalen-1-yl)-5-(methoxymethoxy)-5a,6,7,8,9,10-hexahydro-5H-4-oxa-3,10a,11,13,14-pentaaza-6,9-methanonaphtho[1,8-ab]hepten-14-carboxylate (23 mg, yield: 31%). ES-API: [M+ H]⁺= 955.4.

Step XVI: Cesium fluoride (37 mg, 0.241 mmol) was added to t-butyl (5R, 5aS, 6S, 9R)-1-fluoro-12-(((2R)-2-fluoro-6-methylenetetrahydro-1H-pyrrolizin-7a(5H)-yl)methoxy)-2-(7-fluoro-8-((triisopropylsilyl)ethynyl)naphthalen-1-yl)-5-(methoxymethoxy)-5a,6,7,8,9,10-hexahydro-5H-4-oxa-3,10a,11,13,14-pentaaza-6,9-methanonaphtho[1,8-ab]hepten-14-carboxylate (23 mg, 0.024 mmol) in N,N-dimethylformamide (2 mL), and the reaction mixture was stirred at room temperature for 0.5 hr. The reaction was shown to be completed by LC/MS. The reaction solution was diluted with ethyl acetate (15 mL), washed with water (15 mLX2) and saturated brine (15 mLX3), dried over anhydrous sodium sulfate, filtered, and concentrated to obtain the crude product t-butyl (5R, 5aS, 6S, 9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-((methoxymethoxy)-5a,6,7,8,9,10-hexahydro-5H-4-oxa-3,10a,11,13,14-pentaaza-6,9-methanonaphtho[1,8-ab]hepten-14-carboxylate (19 mg). ES-API: [M+ H]⁺= 799.3.

Step XVII: In an iced water bath, a 4 M hydrochloric acid-dioxane solution (2 mL) was slowly added to a solution of t-butyl (5R, 5aS, 6S, 9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5-((methoxymethoxy)-5a,6,7,8,9,10-hexahydro-5H-4-oxa-3,10a,11,13,14-pentaaza-6,9-methylenenaphthalenyl[1,8-ab]hepten-14-carboxylate (19 mg, 0.024 mmol) in acetonitrile (2 mL). The reaction mixture was stirred at 0 °C for 1 hr. The reaction was shown to be completed by LC/MS. The reaction solution was concentrated to dryness under reduced pressure, and the residue was purified by preparative HPLC (ammonium bicarbonate method 2) (chromatographic condition: 15mL-40-60-13 min) to obtain ((5R, 5aS, 6S, 9R)-2-(8-ethynyl-7-fluoronaphthalen-1-yl)-1-fluoro-12-(((2R)-2-fluoro-6-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-5-yl)methanol (Z654, 6.65 mg, yield: 42%) as an off-white solid. ES-API: [M+H]⁺= 655.2. ¹H NMR (400 MHz, CD₃OD) δ 8.12-8.07 (m, 2 H), 7.68-7.56 (m, 2 H), 7.47-7.40 (m, 1 H), 5.44-5.24 (m, 2 H), 5.02-5.00 (m, 2 H), 4.39 (s, 2 H), 4.36 (d, J= 3.0 Hz, 1 H), 4.27 (d, J= 13.0 Hz, 1 H), 4.07-4.02 (m, 1 H), 3.95-3.89 (m, 1 H), 3.80-3.53 (m, 4 H), 3.48-3.37 (m, 1 H), 3.21-3.11 (m, 2 H), 2.98-2.83 (m, 2 H), 2.54-2.44 (m, 2 H), 2.21-2.02 (m, 2 H), 1.88-1.77 (m, 3 H), 1.37-1.24 (m, 2 H).

### Example 86: Synthesis of (5S, 5aS, 6S, 9R)-2-(8-ethynyl-7-fluoronaphthalen-1-yl)-1-fluoro-12-(((1S, 7aS)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho [1,8-ab]heptene (Z695) and (5S, 5aS, 6S, 9R)-2-(8-ethynyl-7-fluoronaphthalen-1-yl)-1-fluoro-12-(((1S,7aR)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z696)

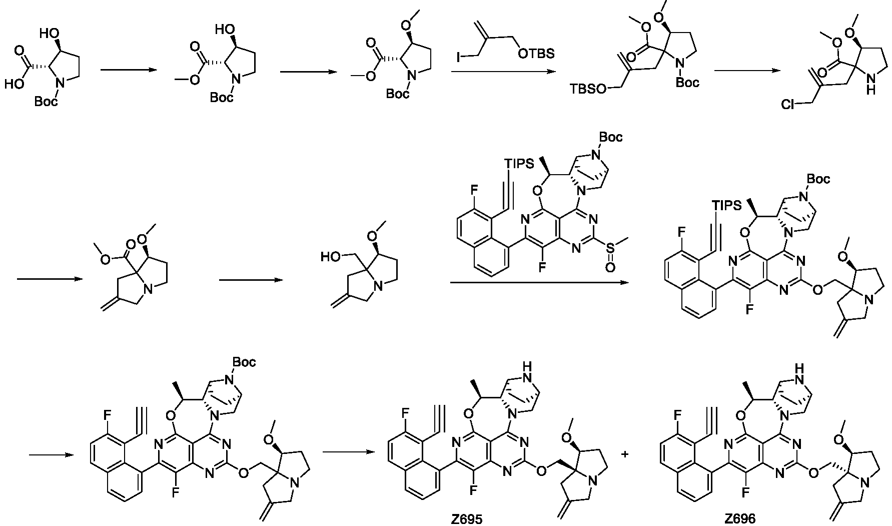

Step I: In an iced water bath, potassium carbonate (1.79 g, 12.9 mmol) and iodomethane (1.84 g, 12.9 mmol) were added to a solution of (2S, 3S)-1-(t-butoxycarbonyl)-3-hydroxylpyrrolidin-2-carboxylic acid (1 g, 4.3 mmol) in N,N-dimethylformamide (10 mL), and stirred at room temperature for 2 hrs. The reaction was shown to be completed by LC/MS. The reaction solution was filtered, and washed with ethyl acetate. The filtrate was added with water (15 mLX3), washed with saturated brine (15 mLX3), dried over anhydrous sodium sulfate, filtered and concentrated under reduced pressure. The crude product was purified by flash silica gel column chromatography (petroleum ether: ethyl acetate =10:1) to obtain 1-(t-butyl) 2-methyl (2S,3S)-3-hydroxylpyrrolidin-1,2-dicarboxylate (1.01g, yield: 94%). ES-API: [M-100]⁺= 190.1.

Step II: Under a nitrogen atmosphere, silver oxide (9.45 g, 40.8 mmol) and iodomethane (5.79 g, 40.8 mmol) were added to a solution of 1-(t-butyl) 2-methyl (2S,3S)-3-hydroxylpyrrolidin-1,2-dicarboxylate (1.01g, 4.08 mmol) in acetonitrile (10 mL), and stirred at room temperature for 48 hrs. The reaction was shown to be completed by LC/MS. The reaction was filtered and washed with ethyl acetate. The filtrate was concentrated under reduced pressure. The crude product was purified by flash silica gel column chromatography (petroleum ether: ethyl acetate =15:1) to obtain 1-(t-butyl) 2-methyl (2S, 3S)-3-methoxypyrrolidin-1,2-dicarboxylate (1.01g, yield: 95%). ES-API: [M-100]⁺= 160.1.

Step III: Under a nitrogen atmosphere, 1-(t-butyl) 2-methyl (2S, 3S)-3-methoxypyrrolidin-1,2-dicarboxylate (900 mg, 3.47 mmol) was dissolved in anhydrous tetrahydrofuran (10mL), and cooled to -78°C. A lithium bis(trimethylsilyl)amide solution (6.9 mL, 6.9 mmol, 1 M solution in tetrahydrofuran) was slowly added dropwise to the above solution, and stirred at this temperature for 1 hr. A solution of t-butyl ((2-(iodomethyl)allyl)oxy)dimethylsilane (2.17 g, 6.9 mmol) in anhydrous tetrahydrofuran (10 mL) was slowly added by a syringe to the solution, and the reaction solution was slowly heated to room temperature and continuously stirred for 2 hrs. The reaction was shown to be completed by LC/MS. The reaction was quenched with a saturated ammonium chloride solution (50 mL). The reaction solution was extracted with ethyl acetate (50 mLX 3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 1-(t-butyl) 2-methyl (3S)-2-(2-(((t-butyldimethylsilyl)oxy)methyl)allyl)-3-methoxypyrrolidin-1,2-dicarboxylate (1.0g, yield: 65%). ES-API: [M-55]⁺= 388.2.

Step IV: At room temperature, thionyl chloride (15mL) was slowly added to a solution of 1-(t-butyl) 2-methyl (3S)-2-(2-(((t-butyldimethylsilyl)oxy)methyl)allyl)-3-methoxypyrrolidin-1,2-dicarboxylate (1.0g, 2.25 mmol) in dichloromethane (7 mL), and stirred overnight at room temperature. The reaction was shown to be completed by LC/MS. The reaction solution was concentrated and the crude product was purified by flash silica gel column chromatography (petroleum ether: ethyl acetate =10:1) to obtain methyl (3S)-2-(2-(chloromethyl)allyl)-3-methoxypyrrolidin-2-carboxylate (400 mg, yield: 71.6%). ES-API: [M+H]⁺= 248.1.

Step V: At room temperature, sodium bicarbonate (678 mg, 8.0 mmol) and potassium iodide (26.8 mg, 0.164 mmol) were added to a solution of methyl (3S)-2-(2-(chloromethyl)allyl)-3-methoxypyrrolidin-2-carboxylate (400 mg, 1.6 mmol) in acetonitrile (10 mL), and stirred at room temperature for 1.5 hrs. The reaction was shown to be completed by LC/MS. The reaction was filtered, and washed with acetonitrile. The filtrate was concentrated under reduced pressure, and the crude product was purified by flash silica gel column chromatography (dichloromethane: methanol =15: 1) to obtain methyl (1S)-1-methoxy-6-methylenetetrahydro-1H-pyrrolizin-7a(5H)-carboxylate (300 mg, yield: 88%). ES-API: [M+H]⁺=182.2.

Step VI: Under a nitrogen atmosphere, methyl (1S)-1-methoxy-6-methylenetetrahydro-1H-pyrrolizin-7a(5H)-carboxylate (300 mg, 1.42 mmol) was dissolved in anhydrous tetrahydrofuran(5mL), and the mixture was allowed to stand in an iced water bath. A lithium aluminium hydride solution (2.84 mL, 2.84 mmol, 1 M solution in tetrahydrofuran) was slowly added dropwise to the above solution, and stirred at this temperature for 0.5 hr. The reaction was shown to be completed by LC/MS. The reaction was quenched with sodium sulfafe decahydrate (500 mg), stirred at room temperature for 0.5 hr, and filtered. The filtrate was concentrated under reduced pressure, to obtain ((1S)-1-methoxy-6-methylenetetrahydro-1H-pyrrolizin-7a(5H)-yl)methanol (170 mg, yield: 65%). ES-API: [M+H]⁺= 184.1.

Step VII: ((1S)-1-methoxy-6-methylenetetrahydro-1H-pyrrolizin-7a(5H)-yl)methanol (48 mg, 0.26 mmol) was dissolved in anhydrous tetrahydrofuran (5 mL). In an iced water bath, sodium hydride (21 mg, 0.52 mmol) was added, and then reacted for 30 min in the iced water bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, 0.13 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX2) and saturated brine (15 mLX3), dried over anhydrous sodium sulfate, and filtered. The crude product was purified by automated flash silica gel chromatography ((dichloromethane: methanol =15:1)mobile phase) to obtain t-butyl (5S, 5aS, 6S, 9R)-1-fluoro-2-(7-fluoro-8-(triisopropylsilyl)ethynyl) naphthalen-1-yl)-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (20 mg, yield: 17%). ES-API: [M+H]⁺= 907.2.

Step VIII: Cesium fluoride (34 mg, 0.22 mmol) was added to t-butyl (5S, 5aS, 6S, 9R)-1-fluoro-2-(7-fluoro-8-(triisopropylsilyl)ethynyl)naphthalen-1-yl)-12-(((1S)-1-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (20 mg, 0.022 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S, 5aS, 6S, 9R)-2-(8-ethynyl-7-fluoronaphthalen-1-yl)-1-fluoro-12-(((1S)-1-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (16 mg, yield: 97%). ES-API: [M+H]⁺= 751.2.

Step IX: In an iced water bath, 4 M hydrochloric acid-dioxane solution (5 mL) was slowly added to a solution of t-butyl (5S, 5aS, 6S, 9R)-2-(8-ethynyl-7-fluoronaphthalen-1-yl)-1-fluoro-12-(((1S)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (16 mg, 0.021 mmol) in acetonitrile (1 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain two isomeric compounds. One isomeric compound was arbitrarily designated as (5S, 5aS, 6S, 9R)-2-(8-ethynyl-7-fluoronaphthalen-1-yl)-1-fluoro-12-(((1S,7aS)-1-methoxy-6-methylenetetrahydro -1H-pyrrolizin-7a(5H)-yl)methoxy)-5-methyl-5a,6,7,8,9,10-hexahydro-5H-4-oxo-3,10a, 11, 13,14-pentaaza-6,9-methanonaphtho[1,8-ab]heptene (Z695, 1.2 mg, yield: 8.8%, peak 1, retention time: 5.86min). ES-API: [M+H]⁺= 651.2. The other isomeric compound was arbitrarily designated as (5S, 5aS, 6S, 9R)-2-(8-ethynyl-7-fluoronaphthalen-1-yl)-1-fluoro-12-(((1S, 7aR)-1-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]heptene (Z696, 0.8 mg, yield: 5.9%, peak 2, retention time: 6.87min). ES-API: [M+H]⁺= 651.2.

### Example 87: Synthesis of 5-(2,2-dimethylcyclopropyl)-6-(5S, 5aS, 6S, 9R)-1-fluoro-12-(2R)-2-fluoro-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-2-yl)-4-methylpyridine-2-amine (Z697)

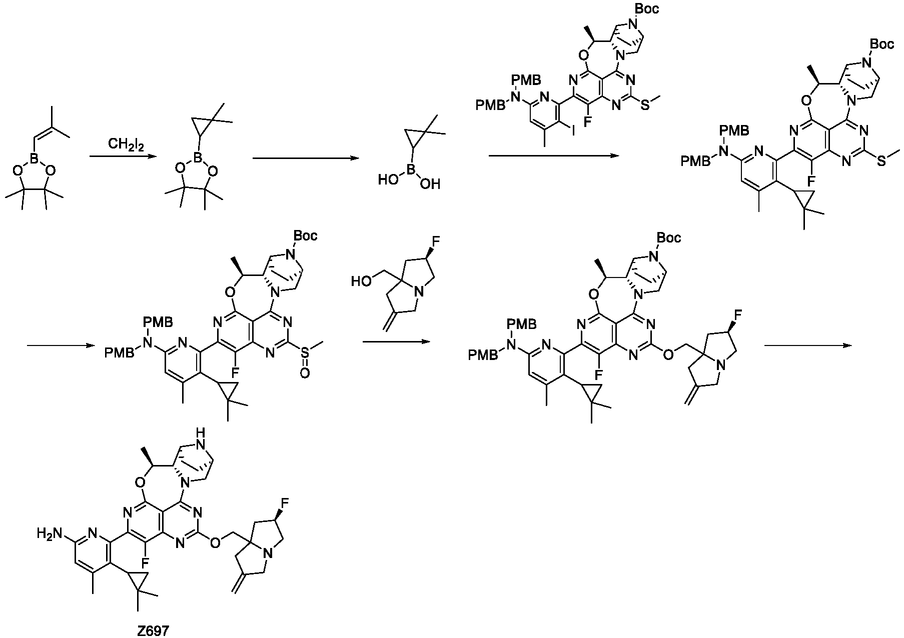

Step I: 1.0 M diethyl zinc solution in n-hexane (22.0 mL, 22.0 mmol) was dissolved in dichloromethane (22 mL). At 0°C, a solution of trifluoroacetic acid (1.67 mL, 21.97 mmol) in dichloromethane (10 mL) was added dropwise, and reacted with stirring at 0°C for 30 min. Then a solution of diiodomethane (1.84 mL, 21.97 mmol) in dichloromethane (10 mL) was slowly added dropwise, and reacted with stirring at 0°C for 30 min. A solution of 2-methyl-1-propenylborate pinacol ester (2 g, 11.0 mmol) in dichloromethane (20 mL) was added dropwise, and the reaction was stirred at room temperature for 18 hrs. The reaction solution was quenched with 1M dilute hydrochloric acid (20 mL). The organic phase was separated, washed with saturated brine (30 mL×2), dried over anhydrous sodium sulfate, and filtered. The filtrate was concentrated under reduced pressure. The crude product was purified by flash silica gel column chromatography (tetrahydrofuran/petroleum ether: 0-8%) to obtain the target product 2-(2,2-dimethylcyclopropyl) borate pinacol ester (1.9 g, yield: 88.2%) as a colorless liquid. ES-API: [M+H]⁺= 197.1. ¹H NMR (400 MHz, CD₃OD) δ1.26 -1.18 (m, 12H), 1.15 (s, 3H), 1.12 (s, 3H), 0.63 -0.51 (m, 2H), -0.28 (dd, *J =* 9.2, 6.8 Hz, 1H).

Step II: 2-(2,2-dimethylcyclopropyl)borate pinacol ester (700 mg, 3.57 mmol) was dissolved in tetrahydrofuran(24 mL) and water (6 mL), sodium periodate (2.29 g, 10.71 mmol) was added, and the reaction was stirred at room temperature for 5 min. 2 M hydrochloric acid (1.18 mL, 2.36 mmol) was then added, and the reaction was stirred at room temperature for 12 hrs. The reaction solution was added with water (10 mL), and extracted with ethyl acetate (60 mL). The organic phase was sequentially washed with water (10 mL) and saturated brine (30 mL). The organic phase was dried over anhydrous sodium sulfate, and filtered. The filtrate was concentrated under reduced pressure, to obtain the target product 2-(2,2-dimethylcyclopropyl)boric acid (340 mg, yield: 83.6%), which was directly used in the next step of reaction without purification.

Step III: Potassium phosphate (277 mg, 1.31 mmol), and [n-butyldi(1-adamantanyl) phosphine](2-amino-1,1'-biphenyl-2-yl)palladium (II) mesylate (63 mg, 0.087 mmol) were added to t-butyl (5S, 5aS, 6S, 9R)-2-(6-(bis(4-methoxybenzyl)amino)-3-iodo-4-methylpyridine-2-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (400 mg, 0.44 mmol) and 2-(2,2-dimethylcyclopropyl)boric acid (248 mg, 2.17 mmol) in toluene (10 mL) and water (1 mL), purged 3 times with nitrogen, and reacted at 90°C for 18 hrs. The reaction solution was added with ethyl acetate (60 mL), and washed with saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5S, 5aS, 6S, 9R)-2-(6-(bis(4-methoxybenzyl)amino)-3-(2,2-dimethylcyclopropyl)-4-methylpyridine-2-yl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (265 mg, yield: 70.7%) as a light yellow solid. ES-API: [M+H]⁺=862.3.

Step IV: T-butyl (5S, 5aS, 6S, 9R)-2-(6-(bis(4-methoxybenzyl)amino)-3-(2,2-dimethylcyclopropyl)-4-methylpyridine-2-yl)-1-fluoro-5-methyl-12-(methylthio)-5a,6,7,8,9,10-hexahydro-5H-4-oxa-3,10a,1 1,13,14-pentaaza-6,9-methanonaphtho[1,8-ab]hepten-14-carboxylate (240 mg, 0.28 mmol) was dissolved in dichloromethane (15 mL). m-chloroperoxybenzoic acid (74 mg, 0.36 mmol) was added at room temperature, and the reaction was stirred at room temperature for 1 hr. The reaction solution was added with dichloromethane (50 mL), sequentially washed with saturated sodium thiosulphate (15 mL), saturated sodium bicarbonate (15 mL), and saturated brine (30 mL). The organic phase was dried over anhydrous sodium sulfate, and filtered. The filtrate was concentrated under reduced pressure, to obtain the target product t-butyl (5S, 5aS, 6S, 9R)-2-(6-(bis(4-methoxybenzyl)amino)-3-(2,2-dimethylcyclopropyl)-4-methylpyridine-2-yl)-1-fluoro-5-methyl-12-(methylthionyl)-5a,6,7,8,9,10-hexahydro-5H-4-oxo-3,10a,11,13,14-pentaaza-6,9-methanonaphtho[1,8-ab]hepten-14-carboxylate (240 mg, yield: 98.1%) as a white solid. ES-API:[M+H]⁺=878.2.

Step V: ((2R)-2-fluoro-6-methylenetetrahydro-1H-pyrrolizin-7a(5H)-yl)methanol (140 mg, 0.82 mmol) was dissolved in tetrahydrofuran(8 mL). At 0°C, 60% sodium hydride (38 mg, 0.96 mmol) was added, and reacted with stirring at this temperature for 30 min. A solution of t-butyl (5S,5aS,6S,9R)-2-(6-(bis(4-methoxybenzyl)amino)-3-(2,2-dimethylcyclopropyl)-4-methylpyridine-2-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (240 mg, 0.27mmol) in tetrahydrofuran (2 mL) was added dropwise, and reacted with stirring at this temperature for 30 min. The reaction solution was added with ethyl acetate (60 mL), and sequentially added with water (20 mL) and saturated brine (30 mL). The organic phase was dried over anhydrous sodium sulfate, and filtered. The filtrate was concentrated under reduced pressure. The crude product was purified by preparative TLC (petroleum ether/tetrahydrofuran=3: 2) to obtain the target product t-butyl (5S, 5aS, 6S, 9R)-2-(6-(bis(4-methoxyphenylbenzylethoxybenzyl)-amino)-3-(2,2-dimethylcyclopropyl)-4-methylpyridine-2-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxo-3,10a, 11,13,14-pentaaza-6,9-methanonaphtho[1,8-ab]hepten-14-carboxylate (95 mg, yield: 35.3%) as a light yellow solid. ES-API: [M+H]⁺=985.3.

Step VI: T-butyl ((5S, 5aS, 6S, 9R)-2-(6-(bis(4-methoxyphenylbenzylethoxybenzyl)-amino)-3-(2,2-dimethylcyclopropyl)-4-methylpyridine-2-yl)-1-fluoro-12-((2R)-2-fluoro-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-14-carboxylate (80 mg, 0.081 mmol) was dissolved in trifluoroacetic acid (4 mL), and the reaction was stirred at 50°C for 2 hrs. The reaction solution was concentrated under reduced pressure to dryness. Dichloromethane (5 mL) and 7.0M aminomethanol (5 mL) were added, and concentrated under reduced pressure. The crude product was purified by preparative HPLC (ammonium bicarbonate method 1) to obtain the target product 5-(2,2-dimethylcyclopropyl)-6-(5S, 5aS, 6S, 9R)-1-fluoro-12-(2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-4-methylpyridine-2-amine (Z697, 23 mg, yield: 43.9%) as a light yellow solid. ES-API: [M+H]⁺= 645.1. ¹H NMR (400 MHz, CD₃OD) δ 6.57 (s, 1H), 5.44 -5.23 (m, 2H), 5.06 -4.97 (m, 2H), 4.59 -4.47 (m, 1H), 4.37 (d, *J* = 10.0 Hz, 1H), 4.28 (d, *J* = 10.0 Hz, 1H), 4.07 (d, *J* = 8.4 Hz, 1H), 3.74 (d, *J* = 14.0 Hz, 1H), 3.66 (d, *J* = 5.6 Hz, 1H), 3.62 -3.55 (m, 1H), 3.47 -3.37 (m, 1H), 3.30 -3.26 (m, 1H), 3.16 (d, *J =* 13.6 Hz, 1H), 3.02 -2.81 (m, 2H), 2.58 -2.43 (m, 2H), 2.41 -2.27 (m, 3H), 2.21 - 1.99 (m, 2H), 1.92 -1.65 (m, 4H), 1.57 (d, *J =* 6.4 Hz, 3H), 1.34 -0.94 (m, 3H), 0.66 (s, 3H), 0.54 -0.24 (m, 1H),-0.08 --0.51 (m, 1H).

### Example 88: Synthesis of (5S, 5aS, 6S, 9R)-2-(8-ethynyl-7-fluoronaphthalen-1-yl)-1-fluoro-5-methyl-12-(1-(3-methylenepyrrolidin-1-yl)methyl)cyclopropyl)methoxy)-5a,6,7,8,9,10-hexahydro-5H-4-oxa-3,10a,11,13,14-pentaaza-6,9-methanonaphtho[1,8-ab]heptene (Z698)

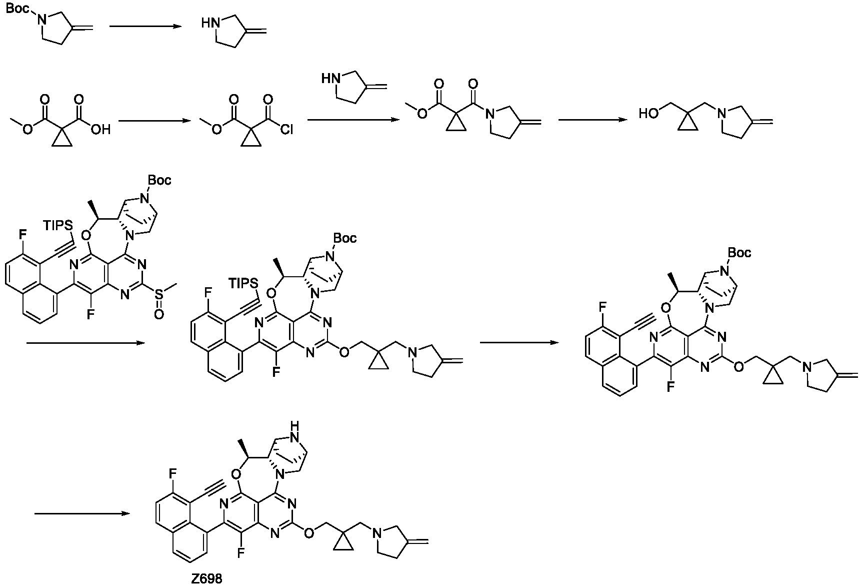

Step I: In an iced water bath, A 4 M hydrochloric acid-dioxane solution (13.64 mL, 54.57 mmol) was added to a solution of t-butyl 3-methylenepyrrolidin-1-carboxylate (2 g, 10.9 mmol) in dichloromethane (5 mL). The reaction mixture was stirred at room temperature for 1 hr. The reaction solution was concentrated to obtain 3-methylenepyrrolidine hydrochloride (907 mg, crude product), which was directly used in the next step of reaction without purification.

Step II: 1-(methoxycarbonyl)cyclopropane-1-carboxylic acid (1.2 g, 8.33 mmol) was dissolved in a mixed solution of dichloromethane (10 mL) and N,N-dimethylformamide (0.032 mL). After cooling to 0°C, the reaction solution was added with oxalyl chloride (2.82 mL, 33.30 mmol). The reaction solution was stirred at 35°C for 3.5 hrs. The reaction solution was concentrated to obtain methyl 1-(chlorocarbonyl)cyclopropane-1-carboxylate (1.35g, crude product), which was directly used in the next step of reaction without purification.

Step III: A solution of 3-methylenepyrrolidine hydrochloride (900 mg, crude product) and N,N-diisopropylethyl amine (7.2 mL, 41.52 mmol) in dichloromethane (20 mL) was cooled to 0°C. A solution of methyl 1-(chlorocarbonyl)cyclopropane-1-carboxylate (1.35g, crude product) in dichloromethane (10 mL) was added dropwise. After that, the reaction was stirred at room temperature for 1 hr. 2M hydrochloric acid (30 mL) was added to quench the reaction. The reaction solution was extracted with dichloromethane (30 mLX2). The organic phase was washed with saturated sodium bicarbonate and brine, dried over anhydrous sodium sulfate, filtered, and concentrated to obtain methyl 1-(3-methylenepyrrolidin-1-carbonyl)cyclopropane-1-carboxylate (1.7g, yield: 97%). ES-API: [M+H]⁺=210.1.

Step IV: At -20°C, a lithium aluminium hydride /tetrahydrofuran solution (7.65 mL, 7.65 mmol) was added dropwise to a solution of methyl 1-(3-methylenepyrrolidin-1-carbonyl)cyclopropane-1-carboxylate (800 mg, 3.82 mmol) in tetrahydrofuran (15 mL). After that, the reaction was continuously stirred at -10°C for 30 min, and quenched with a 15% sodium hydroxide aqueous solution (0.5 mL). The mixture was stirred at room temperature for 30 min, and filtered through diatomite. The filtrate was concentrated to obtain (1-(3-methylenepyrrolidin-1-yl)methyl) cyclopropyl)methanol (430 mg, yield: 67%) as a yellow oil. ES-API: [M+H]⁺=168.1.

Step V: 60% sodium hydride (9.75 mg, 0.406 mmol) and tetrahydrofuran (6 mL) were added to a round-bottom flask. The reaction solution was cooled to 0°C, and a solution of (1-(3-methylenepyrrolidin-1-yl)methyl)cyclopropyl)methanol (25.47 mg, 0.152 mmol) in anhydrous tetrahydrofuran (1 mL) was added to the reaction solution, and reacted at room temperature for 10 min. The reaction solution was cooled to 0°C. T-butyl (5S, 5aS, 6S, 9R)-1-fluoro-2-(7-fluoro-8-(triisopropylsilyl)ethynyl)naphthalen-1-yl)-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (80 mg, 0.1 mmol) was added to the reaction solution, and reacted at room temperature for 1 hr. The reaction solution was added with water (30 mL), and extracted with ethyl acetate (20 mLX2). The organic phase was washed with saturated brine (30 mL), dried over anhydrous sodium sulfate, filtered, and concentrated to obtain t-butyl (5S, 5aS, 6S, 9R)-1-fluoro-2-(7-fluoro-8-(triisopropylsilyl)ethynyl)naphthalen-1-yl)-5-methyl-12-(1-(3-methylenepyrrolidin-1-yl)methyl)cyclopropyl)methoxy)-5a, 6,7,8,9,10-hexahydro-5H-4-oxy-3,10a,11,13,14-pentaaza-6,9-methanonaphtho[1,8-ab]hepten-14-carboxylate (80 mg, crude product). ES-API: [M+H]⁺= 891.3.

Step VI: Cesium fluoride (109.09 mg, 0.718 mmol) was added to t-butyl (5S, 5aS, 6S, 9R)-1-fluoro-2-(7-fluoro-8-(triisopropylsilyl)ethynyl)naphthalen-1-yl)-5-methyl-12-(1-(3-methylenepyrrolidin-1-yl)methyl)cyclopropyl)methoxy)-5a,6,7,8,9,10-hexahydro-5H-4-oxy-3,10a,11,13,14-pentaaza-6,9-methanonaphtho[1,8-ab]hepten-14-carboxylate (80 mg, crude product) in N,N-dimethylformamide (3 mL). The reaction mixture was stirred at room temperature for 1 hr. The reaction was shown to be completed by LC/MS. The reaction solution was diluted with ethyl acetate (50 mL), and washed with water (30 mL) and saturated brine (30 mLX2). The organic phase was dried over anhydrous sodium sulfate, filtered, and concentrated to obtain t-butyl (55,5aS,6S,9R)-2-(8-ethynyl-7-fluoronaphthalen-1-yl)-1-fluoro-5-methyl-12-((1-(3-methylenepyrrolidin-1-yl)methyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (60 mg, crude product). ES-API: [M+H]⁺= 735.3.

Step VII: In an iced water bath, 4 M hydrochloric acid-dioxane solution (3mL) was slowly added to a solution of t-butyl (5S, 5aS, 6S, 9R)-2-(8-ethynyl-7-fluoronaphthalen-1-yl)-1-fluoro-5-methyl-12-(1-(3-methylenepyrrolidin-1-yl)methyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (60 mg, crude product) in acetonitrile (3 mL). The reaction mixture was stirred at room temperature for 1 hr. The reaction was shown to be completed by LC/MS. The reaction solution was concentrated to dryness under reduced pressure. The residue was purified by preparative HPLC(ammonium bicarbonate method 1) to obtain (5S, 5aS, 6S, 9R)-2-(8-ethynyl-7-fluoronaphthalen-1-yl)-1-fluoro-5-methyl-12-(1-(3-methylenepyrrolidin-1-yl)methyl)cyclopropyl)methoxy)-5a,6,7,8,9,10-hexahydro-5H-4-oxy-3,10a,11,13,14-pentaaza-6,9-methanonaphtho[1,8-ab]heptene (Z698, 10mg, yield: 19%), ES-API: [M+H]⁺= 635.2. ¹H NMR(400MHz, CD₃OD): 8.10-8.05 (m, 2H), 7.64-7.38 (m, 3H), 5.39-5.36 (m, 1H), 4.92-4.87 (m, 2H), 4.54-4.34 (m, 3H), 4.08-4.06 (m, 1H), 3.76-3.46 (m, 3H), 3.25-3.13 (m, 3H), 2.90-2.40 (m, 6H), 2.10-2.02 (m, 1H), 1.82-1.76 (m, 3H), 1.58-1.55 (m, 3H), 0.71-0.55 (m, 4H).

### Example 89: Synthesis of 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z699)

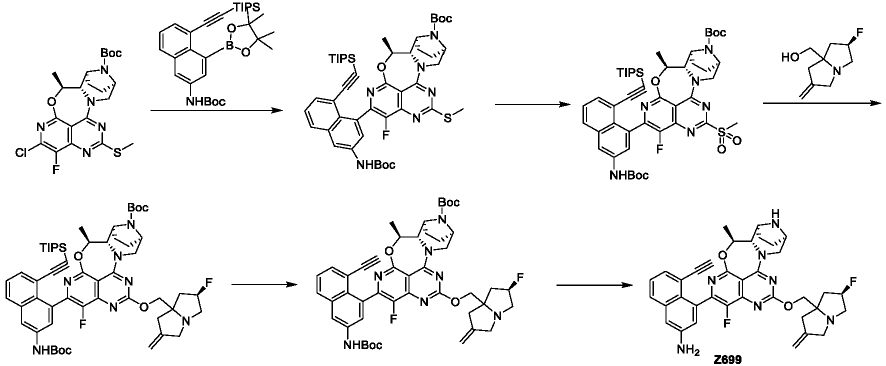

Step I: T-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.166 mmol) and t-butyl (4-(4,4,5,5-tetramethyl-1,3,2-dioxaborolan-2-yl)-5-((triisopropylsilyl)ethynyl)naphthalen-2-yl)carbamate (91.24 mg, 0.166 mmol) were dissolved in tetrahydrofuran (10 mL) and water (1 mL). [n-butyldi(1-adamantanyl)phosphine](2-amino-1,1'-biphenyl-2-yl)palladium (II) mesylate (12.09 mg, 0.017 mmol) and potassium phosphate (105.70 mg, 0.498 mmol) were added to the solution. After that, the reaction was stirred at 65 °C for 5 hrs. After the reaction, the reaction solution was added with water (15 mL) and extracted with ethyl acetate (15 mLX3). The organic phase was washed with saturated brine (30 mL), dried over anhydrous sodium sulfate, filtered and concentrated. The crude product was purified by flash silica gel column chromatography (ethyl acetate /petroleum ether: 0-30%) to obtain t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.092 mmol, yield: 55.44%) as a white solid. ES-API: [M +H]⁺= 869.3.

Step II: T-butyl (5S,5aS,6S,9R)-2-(3-((t-butoxycarbonyl)amino)-8-((triisopropylsilyl)ethynyl) naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.092 mmol) was dissolved in dichloromethane (5 mL). At room temperature, m-chloroperoxybenzoic acid (purity 85%, 56.06 mg, 0.276 mmol) was added to the solution. After that, the reaction was stirred at room temperature for 1 hr. The reaction solution was added with a saturated sodium thiosulphate solution (5 mL) and extracted with dichloromethane (10 mLX3). The organic phases were combined, and then washed sequentially with a saturated sodium bicarbonate aqueous solution (20 mL) and saturated sodium chloride (20 mL). The organic phase was dried and concentrated to obtain t-butyl (5S,5aS,6S,9R)-2-(3-((t-butoxycarbonyl)amino)-8-((triisopropylsilyl)ethynyl) naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (80 mg, 0.089 mmol, yield: 96.45%) as a light yellow solid. ES-API: [M +H]⁺= 901.0.

Step III: ((2R)-2-fluoro-6-methylenetetrahydro-1H-pyrrolizin-7a(5H)-yl)-methanol (30.40 mg, 0.178 mmol) was dissolved in tetrahydrofuran (3 mL) and cooled to 0°C. Under a nitrogen atmosphere, sodium hydride (60%, 14.20 mg, 0.355 mmol) was added to the solution and stirred for 30 min. After that, t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (80 mg, 0.089 mmol) was slowly added to the solution and stirred at 0°C for 2 hrs. The reaction solution was added with water (5 mL) to quench the reaction, and then extracted with ethyl acetate (20 mL X3). The organic phase was washed with saturated brine (30 mLX3), dried over anhydrous sodium sulfate, filtered and concentrated to obtain a crude product. The crude product was purified by flash silica gel column chromatography (ethyl acetate /petroleum ether: 0-70%) to obtain t-butyl (5S,5aS,6S,9R)-2-(3-((t-butoxycarbonyl)amino)-8-((triisopropylsilyl) ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, 0.050 mmol, yield: 56.76%) as a yellow solid. ES-API: [M+H]⁺=992.1.

Step IV: A mixed solution of t-butyl (5S,5aS,6S,9R)-2-(3-((t-butoxycarbonyl)amino)-8-((triisopropylsilyl)ethynyl)naphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (50 mg, 0.050 mmol), cesium fluoride (76.54 mg, 0.504 mmol), and N, N-dimethylformamide (2 mL) was stirred at room temperature for 2 hrs. The reaction solution was added with water (10 mL) and extracted with ethyl acetate (15 X2). The organic phase was washed with saturated brine (15 mLX3), dried over anhydrous sodium sulfate, filtered and concentrated to obtain t-butyl (5S,5aS,6S,9R)-2-(3-((t-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.036 mmol, yield: 71.22%) as a yellow oil. ES-API: [M+H]⁺=835.1.

Step V: T-butyl (5S,5aS,6S,9R)-2-(3-((t-butoxycarbonyl)amino)-8-ethynylnaphthalen-1-yl)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.036 mmol) was dissolved in methanol (2 mL), and cooled to 0°C. A hydrogen chloride-dioxane solution (4 M, 4 mL) was added to the solution, and reacted with stirring at 0°C for 2 hrs. The reaction solution was concentrated, and the residue was taken up in dichloromethane (3 mL) again and alkalized with ammonia in methanol (7 M) to an alkaline pH (pH 8). The obtained solution was concentrated, and the crude product was purified by preparative HPLC (ammonium bicarbonate method 1) to obtain 5-ethynyl-4-((5S,5aS,6S,9R)-1-fluoro-12-(((2R)-2-fluoro-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z699, 9 mg, 0.014 mmol, yield: 39.45%) as a white solid. ES-API: [M+H]⁺=636.2.

### Example 90: Synthesis of 5-ethynyl-4-((5S,5aS,6S,9R)-1-fluoro-5-methyl-12-((1-(morpholinemethyl)cyclopropyl)methoxy)-5a,6,7,8,9,10-hexahydro-5H-4-oxo-3,10a,11,13,14-pentaaza-6,9-methanonaphtho[1,8-ab]hepten-2-yl)naphthalen-2-amine (Z700)

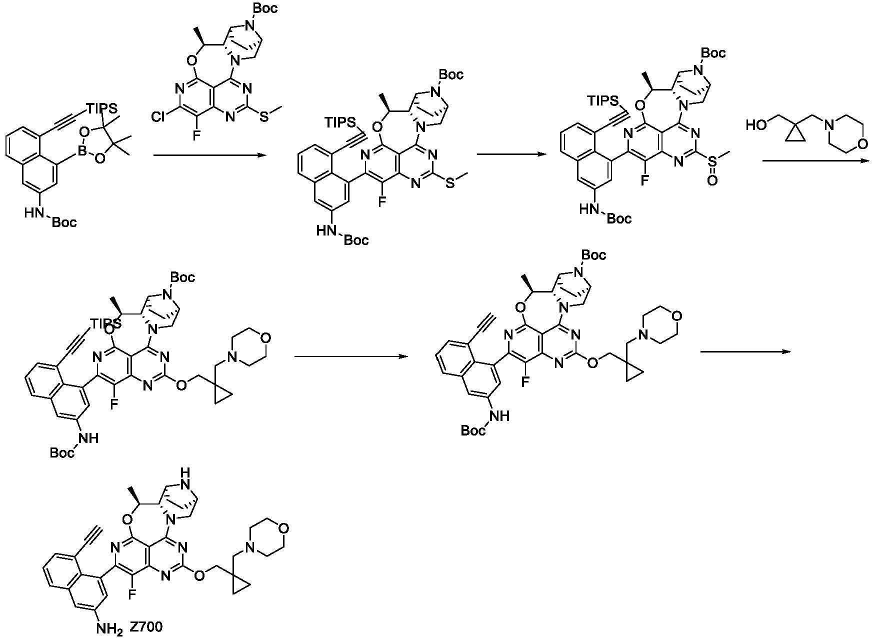

Step I: Potassium phosphate (1.23 g, 5.80 mmol), and [n-butyldi(1-adamantanyl)phosphine](2-amino-1,1'-biphenyl-2-yl)palladium (II) mesylate (158 mg, 0.218 mmol) were added to t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (700 mg, 1.452 mmol) and t-butyl (4-(4,4,5,5-tetramethyl-1,3,2-dioxaborolan-2-yl)-5-((triisopropylsilyl)ethynyl) naphthalen-2-yl)carbamate (700 mg, 1.274 mmol) in tetrahydrofuran (30 mL) and water (5 mL). Nitrogen was bubbled through and the reaction was continued in an oil bath at 80°C for 3 hrs. After the reaction, the reaction solution was extracted with ethyl acetate (30 mLX2) and saturated brine (30 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthio)-5a,6,7,8,9, 10-hexahydro-5H-4-oxAo-3,10a,11,13,14-pentaaza-6,9-methanonaphtho[1,8-ab]hepten- 14-carboxylate (900 mg, yield: 71.29%) as a yellow solid. ES-API: [M+H]⁺=869.2.

Step II: At 0°C, m-chloroperoxybenzoic acid (206.5 mg, 1.197 mmol) was added to a solution of t-butyl (5S,5aS,6S,9R)-2-(3-((t-butoxycarbonyl)amino)-8-((triisopropylsilyl)ethynyl) naphthalen-1-yl)-1-fluoro-5-methyl-12-(methylthio)-5a,6,7,8,9,I0-hexahydro-5H-4-oxo-3,10a,11,13,14-pentaaza-6,9-methanonaphtho[1,8-ab]hepten-14-carboxylate (800 mg, 0.920 mmol) in dichloromethane (15 mL). The solution was stirred at room temperature for 1 hr. The reaction was quenched with saturated sodium sulfite and extracted with dichloromethane(100 mLX2). The organic layer was dried over anhydrous sodium sulfate and concentrated to obtain crude product. The crude product was purified by silica gel column chromatography (0-10% methanol /dichloromethane) to obtain t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (700 mg, yield: 85.92%) as a white solid. ES-API:[M+H]⁺=885.3.

Step III: At 0°C, sodium hydride (268.6 mg, 6.71 mmol) was added to a solution of (1-(morpholinemethyl)cyclopropyl)methanol (460 mg, 2.686 mmol) in tetrahydrofuran (10.0 mL), and stirred at this temperature for 10 min. Then, a solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (830 mg, 0.938 mmol) in tetrahydrofuran (5 mL) was added. The reaction was continued with stirring at 0°C for 10 min, and at room temperature for 0.5 hr. After the reaction was completed, the reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(3-((t-butoxycarbonyl)amino)-8-((triisopropylsilyl)ethynyl)naphthalen- 1-yl)-1-fluoro-5-methyl-12-((1-(morpholinemethyl) cyclopropyl)methoxy)-5a,6,7,8,9, 10-hexahydro-5H-4-oxo-3,10a,11,13,14-pentaaza-6,9-methanonaphtho[1,8-ab]heptan-14-carboxylate (900 mg, yield: 96.73) as a yellow solid. ES-API: [M+H]⁺=993.2.

Step IV: Cesium fluoride (2.75 g, 18.14 mmol) was added to a solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]heptan-14-carboxylate (900 mg, 0.907 mmol) in N,N-dimethylformamide (10.0 mL), and reacted with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-1-fluoro-5-methyl-12-((1-(morpholinomethyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (510 mg, yield: 67.26%) as a yellow oil. ES-API: [M+H]⁺=836.2.

Step V: In an iced water bath, 4 M hydrochloric acid-dioxane solution (10.0 mL) was slowly added to a solution of t-butyl (5S,5aS,6S,9R)-2-(3-((t-butoxycarbonyl)amino)-8-ethynylnaphthalen-1-yl)-1-fluoro-5-methyl-12-((1-(morpholinomethyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (510 mg, 0.610 mmol) in methanol (15.0 mL). The reaction mixture was stirred at room temperature for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain 5-ethynyl-4-((5S,5aS,6S,9R)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine as a light yellow solid (Z700, 73mg, yield:18.82%). ES-API: [M+H]⁺= 636.2. ¹H NMR (400 MHz, CD₃OD) δ 7.65-7.69 (m, 1H), 7.35-7.25 (m, 2H), 7.13 -7.00 (m, 2H), 5.39-5.34 (m, 1H), 4.51-4.42 (m, 2H), 4.37-4.33 (m, 1H), 4.06 (d, *J =* 8.0 Hz, 1H), 3.69 -3.58 (m, 6H), 3.14-3.19 (m, 1H), 2.50 -2.37 (m, 6H), 2.07 -2.04 (m, 1H), 1.87 -1.76 (m, 3H), 1.58 -1.54 (m, 3H), 0.75-0.65 (m, 2H), 0.53-0.45 (m, 2H).

### Example 91: Synthesis of (1-((((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy)methyl)cyclopropyl)methanol (Z701)

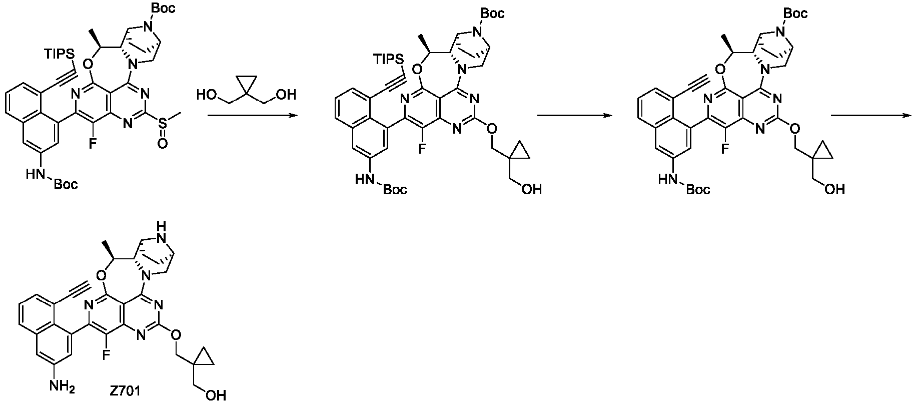

Step I: Cyclopropan-1,1-yldimethanol (8mg,0.07mmol) was dissolved in anhydrous tetrahydrofuran (2 mL). In an iced water bath, sodium hydride (14mg, 0.34mmol) was added, and reacted for 10 min in the ice bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (60mg,0.07mmol) in tetrahydrofuran (1 mL) was added dropwise to the reaction solution, and stirred at normal temperature for 30 min. The reaction was shown to be completed by LC/MS. The reaction solution was diluted with ethyl acetate (5 mL), washed with a saturated ammonium chloride solution (5 mLX2) and saturated brine (5 mLX3), dried over anhydrous sodium sulfate, and filtered. The crude product was purified by automated flash silica gel chromatography [(dichloromethane: methanol = 0%-6%) mobile phase] to obtain t-butyl (5S,5aS,6S,9R)-2-(3-((t-butoxycarbonyl)amino)-8-((triisopropylsilyl)ethynyl) naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptan-14-carboxylate (23 mg, 0.02 mmol, yield: 35.6%). ES-API: [M+H]⁺= 923.1.

Step II: Cesium fluoride (34 mg,0.20 mmol) was added to t-butyl (5S,5aS,6S,9R)-2-(3-((t-butoxycarbonyl)amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptan-14-carboxylate (23 mg, 0.02 mmol) in N,N-dimethylformamide (2 mL), and the reaction mixture was stirred at room temperature for 2 hrs. The reaction was shown to be completed by LC/MS. The reaction solution was diluted with ethyl acetate (5 mL), washed with water (5 mLX2) and saturated brine (5 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-1-fluoro-12-((1-(hydroxymethyl)cyclopropyl))methoxy)-5-methyl-5a,6,7,8,9,10-hexahydro-5H-4-oxa-3,10a,11,13,14-pentaaza-6,9-methanonaphtho[1,8-ab]hepten-14-carboxylate (13 mg,0.02 mmol, yield: 84.9%). ES-API: [M+H]⁺= 767.1.

Step III: In an iced water bath, a 4 M hydrochloric acid-dioxane solution (2.0 mL) was slowly added to a solution of t-butyl (5S,5aS,6S,9R)-2-(3-((t-butoxycarbonyl)amino)-8-ethynylnaphthalen-1-yl)-1-fluoro-12-((1-(hydroxymethyl)cyclopropyl))methoxy)-5-methyl-5a,6,7,8,9,10-hexahydro-5H-4-oxa-3,10a,11,13,14-pentaaza-6,9-methanonaphtho[1,8-ab]hepten-14-carboxylate (13 mg, 0.02 mmol) in methanol (2.0 mL). The reaction mixture was stirred at 0°C for 4 hrs. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (1-((((5S,5aS,6S,9R)-2-(3-amino-8-ethynylnaphthalen-1-yl)-1-fluoro-5-methyl-5a,6,7,8,9,10-hexahydro-5H-4-oxa-3,10a,11,13,14-pentaaza-6,9-methanonaphtho[1,8-ab]hept-12-yl)oxy)methyl)cyclopropyl)methanol (Z701, 6.14mg, 0.01mmol, yield: 54.2%). ES-API: [M+H]⁺= 567.1.

### Example 92: Synthesis of 4-((5S,5aS,6S,9R)-12-((2,6-dimethyltetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z703)

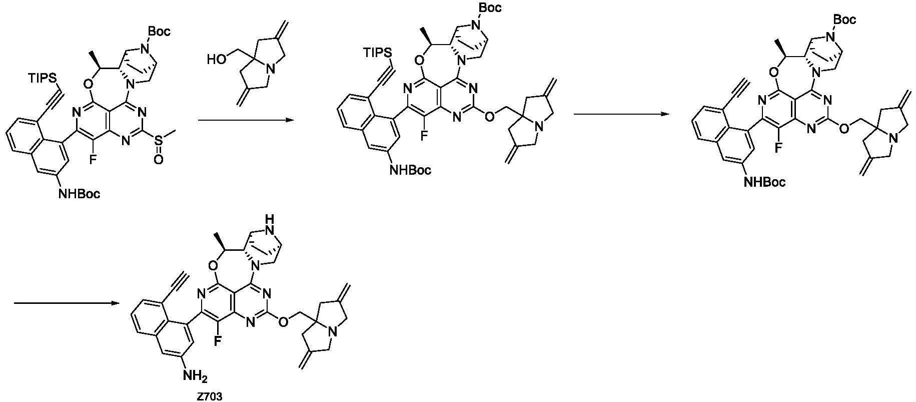

Step I: (2,6-dimethylenetetrahydro-1H-pyrrolizin-7a(5H)-yl)methanol (25 mg, 0.151 mmol) was dissolved in anhydrous tetrahydrofuran (2 mL). In an iced water bath, sodium hydride (10.85 mg, 0.452 mmol) was added, and then reacted for 30 min in the iced water bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (50 mg, 0.056 mmol) in tetrahydrofuran (2 mL) was added dropwise to the reaction solution, and reacted at room temperature for 30 min. The reaction was shown to be completed by LC/MS. The reaction solution was diluted with ethyl acetate (10 mL), washed with saturated ammonium chloride solution (5 mLX2) and saturated brine (50mLX3), dried over anhydrous sodium sulfate, and filtered. The crude product was purified by silica gel column chromatography (dichloromethane: methanol =15:1) to obtain t-butyl (5S,5aS,6S,9R)-2-(3-((t-butoxycarbonyl)amino)-8-((triisopropylsilyl) ethynyl)naphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (44 mg, 0.045 mmol, yield: 78.98%). ES-API: [M+H]⁺= 986.3.

Step II: Cesium fluoride (33.49 mg, 0.220 mmol) was added to t-butyl (5S,5aS,6S,9R)-2-(3-((t-butoxycarbonyl)amino)-8-((triisopropylsilyl)ethynyl)naphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (35 mg, 0.035 mmol) in N,N-dimethylformamide (1.0mL), and the reaction mixture was stirred at room temperature for 2 hrs. The reaction was shown to be completed by LC/MS. The reaction solution was diluted with ethyl acetate (10mL), washed with water (10 mLX2) and saturated brine (10 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((2,6-dimethylenetetrahydro- 1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (28 mg, 0.034 mmol, yield: 95.08%). ES-API: [M+H]⁺=830.2.

Step III: In an iced water bath, a 4 M hydrochloric acid-dioxane solution (1.0 mL) was slowly added to a solution of t-butyl (5S,5aS,6S,9R)-2-(3-((t-butoxycarbonyl)amino)-8-ethynylnaphthalen-1-yl)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.030 mmol) in ethyl acetate (1.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 1) (chromatographic condition: 15mL-35-85-13min) to obtain 4-((5S,5aS,6S,9R)-12-((2,6-dimethyltetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine as a white solid (Z703, 8 mg, 0.013 mmol, yield: 42.35%). ES-API: [M+H]⁺= 630.2. ¹H NMR (400 MHz, CD₃OD) δ 7.67 (t, *J =* 7.2 Hz, 1H), 7.41 -7.35 (m, 1H), 7.32 -7.26 (m, 1H), 7.13 -7.02 (m, 2H), 5.51 -5.27 (m, 1H), 5.02 (d, *J =* 6.8 Hz, 4H), 4.53 -4.47 (m, 1H) 4.30 (s, 2H), 4.06 (d, *J =* 8.4 Hz, 1H), 3.79 - 3.75 (m, 2H), 3.67 (d, *J* = 5.2 Hz, 1H), 3.59 (s, 1H), 3.37 -3.33 (m, 2H), 3.31 -2.99 (m, 2H), 2.81 -2.78 (m, 2H), 2.61 -2.57 (m, 2H), 2.03 -2.02 (m, 1H), 1.94 -1.69 (m, 3H), 1.56 (t, *J =* 6.8 Hz, 3H).

### Example 93: Synthesis of (5S, 5aS, 6S, 9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z704)

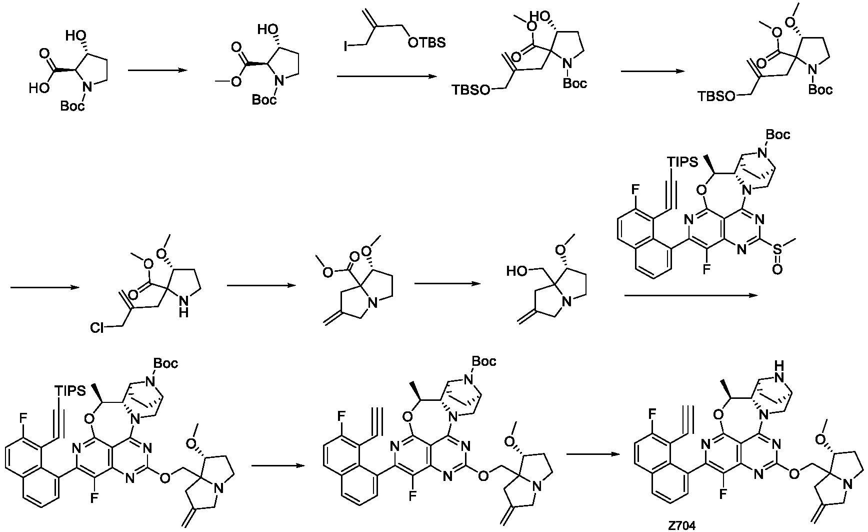

Step I: In an iced water bath, potassium carbonate (1.79 g, 12.9 mmol) and iodomethane (1.84 g, 12.9 mmol) were added to a solution of (2R,3R)-1-(t-butoxycarbonyl)-3-hydroxylpyrrolidin-2-carboxylic acid (1 g, 4.3 mmol) in N,N-dimethylformamide (10 mL), and stirred at room temperature for 2 hrs. The reaction was shown to be completed by LC/MS. The reaction solution was filtered, and washed with ethyl acetate. The filtrate was washed with water (15 mLX3) and saturated brine (15 mLX 3), dried over anhydrous sodium sulfate, filtered and concentrated under reduced pressure. The crude product was purified by flash silica gel column chromatography (petroleum ether: ethyl acetate =10:1) to obtain 1-(t-butyl) 2-methyl(2R,3R)-3-hydroxylpyrrolidin-1,2-dicarboxylate (1.0g, yield: 94%). ES-API: [M-100]⁺= 190.1.

Step II: Under a nitrogen atmosphere, 1-(t-butyl) 2-methyl (2R,3R)-3-methoxypyrrolidin-1,2-dicarboxylate (1.0 g, 4.1 mmol) was dissolved in anhydrous tetrahydrofuran (20 mL), and cooled to -78°C. A lithium diisopropylamide solution (6.1 mL, 12.2 mmol, 2 M solution in tetrahydrofuran) was slowly added dropwise to the above solution, and stirred at this temperature for 1 hr. A solution of t-butyl ((2-(iodomethyl)allyl)oxy)dimethylsilane (2.55 g, 8.2 mmol) in anhydrous tetrahydrofuran (10 mL) was slowly added by a syringe to the solution, and the reaction solution was slowly heated to room temperature, and continuously stirred for 2 hrs. The reaction was shown to be completed by LC/MS. The reaction was quenched with a saturated ammonium chloride solution (50 mL). The reaction solution was extracted with ethyl acetate (50 mLX 3). The organic phase was washed with saturated brine (50 mLX3), dried over anhydrous sodium sulfate, filtered, and concentrated. The crude product was purified by flash silica gel column chromatography (petroleum ether: ethyl acetate =20:1) to obtain 1-(t-butyl) 2-methyl (3R)-2-(2-(((t-butyldimethylsilyl)oxy)methyl)allyl)-3-hydroxylpyrrolidin-1,2-dicarboxylate (450 mg, yield: 26%). ES-API: [M-55]⁺= 330.3.

Step III: Under a nitrogen atmosphere, silver oxide (4.8 g, 20.9 mmol) and iodomethane (3.0 g, 20.9 mmol) were added to a solution of 1-(t-butyl) 2-methyl (3R)-2-(2-(((t-butyldimethylsilyl)oxy)methyl)allyl)-3-hydroxylpyrrolidin-1,2-dicarboxylate (450 mg, 1.1 mmol) in acetonitrile (10 mL), and stirred at room temperature for 48 hrs. The reaction was shown to be completed by LC/MS. The reaction solution was filtered, and washed with ethyl acetate. The filtrate was concentrated under reduced pressure, and the crude product was purified by flash silica gel column chromatography (petroleum ether: ethyl acetate =15:1) to obtain 1-(t-butyl) 2-methyl (3R)-2-(2-(((t-butyldimethylsilyl)oxy)methyl)allyl)-3-methoxypyrrolidin-1,2-dicarboxylate (340 mg, yield: 73%). ES-API: [M-100]⁺= 344.2.

Step IV: room temperature, thionyl chloride (15 mL) was slowly added to a solution of 1-(t-butyl) 2-methyl (3R)-2-(2-(((t-butyldimethylsilyl)oxy)methyl)allyl)-3-methoxypyrrolidin-1,2-dicarboxylate (340 mg, 0.76 mmol) in dichloromethane (7 mL), and stirred overnight at room temperature. The reaction was shown to be completed by LC/MS. The reaction solution was concentrated under reduced pressure, and the crude product was purified by flash silica gel column chromatography (petroleum ether: ethyl acetate =10:1) to obtain methyl (3R)-2-(2-(chloromethyl)allyl)-3-methoxypyrrolidin-2-carboxylate (189 mg, yield: 100%). ES-API: [M+H]⁺= 248.2.

Step V: At room temperature, sodium bicarbonate (320 mg, 3.8 mmol) and potassium iodide (13 mg, 0.076 mmol) were added to a solution of methyl (3R)-2-(2-(chloromethyl)allyl)-3-methoxypyrrolidin-2-carboxylate (189 mg, 0.76 mmol) in acetonitrile (5 mL), and stirred at room temperature for 1.5 hrs. The reaction was shown to be completed by LC/MS. The reaction solution was filtered, and washed with acetonitrile. The filtrate was concentrated under reduced pressure, to obtain the crude product methyl (1R)-1-methoxy-6-methylenetetrahydro-1H-pyrrolizin-7a(5H)-carboxylate (161 mg, yield:100%). ES-API: [M+H]⁺=212.2.

Step VI: Under a nitrogen atmosphere, methyl (1R)-1-methoxy-6-methylenetetrahydro-1H-pyrrolizin-7a(5H)-carboxylate (161 mg, 0.76 mmol) was dissolved in anhydrous tetrahydrofuran (5 mL), and the mixture was allowed to stand in an iced water bath. A lithium aluminium hydride solution (1.5 mL, 1.5 mmol, 1 M in tetrahydrofuran) was slowly added dropwise to the above solution, and stirred at this temperature for 0.5 hr. The reaction was shown to be completed by LC/MS. The reaction was quenched with sodium sulfafe decahydrate (500 mg), stirred at room temperature for 0.5 hr, and filtered. The filtrate was concentrated under reduced pressure, to obtain ((1R)-1-methoxy-6-methylenetetrahydro-1H-pyrrolizin-7a(5H)-yl)methanol (129 mg, yield: 92%). ES-API: [M+H]⁺= 184.1.

Step VII: ((1R)-1-methoxy-6-methylenetetrahydro-1H-pyrrolizin-7a(SH)-yl)methanol (24 mg, 0.13 mmol) was dissolved in anhydrous tetrahydrofuran (5 mL). In an iced water bath, sodium hydride (16 mg, 0.39 mmol) was added, and then reacted for 30 min in the iced water bath. A solution of t-butyl (5S, 5aS, 6S, 9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (102 mg, 0.13 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with saturated ammonium chloride solution (15 mLX2) and saturated brine (15 mLX3), dried over anhydrous sodium sulfate, and filtered. The crude product was purified by silica gel column chromatography (dichloromethane: methanol =15:1) to obtain t-butyl (5S, 5aS, 6S, 9R)-1-fluoro-2-(7-fluoro-8-((triisopropylsilyl)ethynyl)naphthalen-1-yl)-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, yield: 26%). ES-API: [M+H]⁺= 907.2.

Step VIII: Cesium fluoride (50 mg, 0.33 mmol) was added to t-butyl (5S, 5aS, 6S, 9R)-1-fluoro-2-(7-fluoro-8-((triisopropylsilyl)ethynyl)naphthalen-1-yl)-12-(((1R)-1-methoxy-6-methylenetetrahydro-1H-pyrrolidin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.033 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product t-butyl (5S, 5aS, 6S, 9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, yield: 100%). ES-API: [M+H]⁺= 751.2.

Step IX: In an iced water bath, 4 M hydrochloric acid-dioxane solution (5 mL) was slowly added to a solution of t-butyl (5S, 5aS, 6S, 9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25 mg, 0.033 mmol) in acetonitrile (1 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain (5S,5aS,6S,9R)-2-(8-ethynyl-7-fluoronaphthalen-1-yl)-1-fluoro-12-(((1R)-1-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]heptene (Z704, 3.3 mg, yield:16% ). ES-API: [M+H]⁺= 651.2.

### Example 94: Synthesis of 5-ethynyl-4-((5S, 5aS, 6S, 9R)-1-fluoro-12-methoxy-5-methyl-5a,6,7,8,9,10-hexahydro-5H-4-oxy-3,10a,11,13,14-pentaaza-6,9-methanonaphtho[1,8-ab]hepten-2-yl)naphthalen-2-amine (Z705)

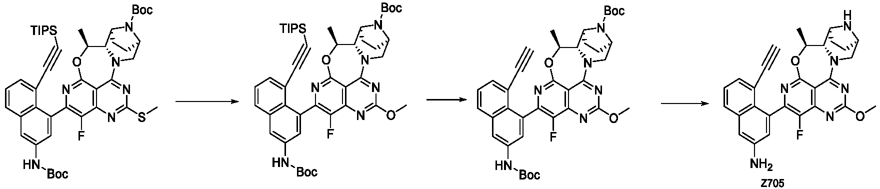

Step I: T-butyl (5S, 5aS, 6S, 9R)-2-(3-((t-butoxycarbonyl)amino)-8-((triisopropylsilyl) ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.092 mmol) was dissolved in methanol (1.5 mL) and dichloromethane(1.0 mL). In an iced water bath, m-chloroperoxybenzoic acid (48 mg, 0.27 mmol) was added, and reacted for 60 min in the iced water bath. Sodium methoxide (2 mg, 0.02 mmol) was added to the above reaction solution, and reacted at room temperature for 30 min. The reaction was shown to be completed by LC/MS. The reaction solution was diluted with dichloromethane (20 mL), washed with saturated ammonium chloride solution (15 mLX2) and saturated brine (15 mLX3), dried over anhydrous sodium sulfate, filtered, and concentrated to obtain crude product. The crude product was purified by silica gel column chromatography (mobile phase: petroleum ether: ethyl acetate =20:1) to obtain t-butyl (5S, 5aS, 6S, 9R)-2-(3-((t-butoxycarbonyl)amino)-8-((triisopropylsilyl)ethynyl)naphthalen-1-yl)-1-fluoro-12-methoxy-5-methyl-5a,6,7,8,9,10-hexahydro-5H-4-oxa-3,10a,11,13,14-pentaaza-6,9-methanonaphtho[1,8-ab]hepten-14-carboxylate (30 mg, yield: 38%). ES-API: [M+H]⁺=853.3.

Step II: Cesium fluoride (53 mg, 0.35 mmol) was added to t-butyl (5S, 5aS, 6S, 9R)-2-(3-((t-butoxycarbonyl)amino)-8-((triisopropylsilyl)ethynyl)naphthalen-1-yl)-1-fluoro-12-methoxy-5-methyl-5a,6,7,8,9,10-hexahydro-5H-4-oxo-3,10a,11,13,14-pentaaza-6,9-methanonaphtho[1,8-ab]hepten-14-carboxylate (30 mg, 0.035 mmol) in N,N-dimethylformamide (3 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S, 5aS, 6S, 9R)-2-(3-((t-butoxycarbonyl)amino)-8-ethynylnaphthalen-1-yl)-1-fluoro-12-methoxy-5-methyl-5a,6,7,8,9,10-hexahydro-5H-4-oxa-3,10a,11,13,14-pentaaza-6,9-methanonaphtho[1,8-ab]hepten-14-carboxylate (24.5 mg, yield: 100%). ES-API: [M+H]⁺=697.1.

Step III: In an iced water bath, a 4 M hydrochloric acid-dioxane solution (5 mL) was slowly added to a solution of t-butyl (5S, 5aS, 6S, 9R)-2-(3-((t-butoxycarbonyl)amino)-8-ethynylnaphthalen-1-yl)-1-fluoro-12-methoxy-5-methyl-5a,6,7,8,9,10-hexahydro-5H-4-oxy-3,10a,11,13,14-pentaaza-6,9-methanonaphtho[1,8-ab]hepten-14-carboxylate (24.5 mg, 0.035 mmol) in methanol (2 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) (chromatographic condition: 15mL-35-85-13min) to obtain 5-ethynyl-4-((5S, 5aS, 6S, 9R)-1-fluoro-12-methoxy-5-methyl-5a,6,7,8,9,10-hexahydro-5H-4-oxy-3,10a,11,13,14-pentaaza-6,9-methanonaphtho[1,8-ab]hepten-2-yl)naphthalen-2-amine (Z705, 3.4 mg, yield: 19%) as a light yellow solid. ES-API: [M+H]⁺= 497.2.

### Example 95: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z706)

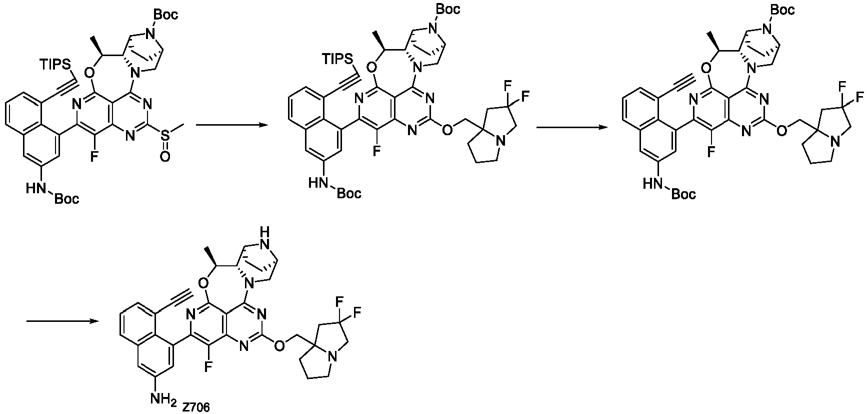

Step I: (2,2-difluorotetrahydro-1H-pyrrolizin-7a(5H)-yl)methanol (45 mg, 0.2542 mmol) was dissolved in anhydrous tetrahydrofuran (15 mL). In an iced water bath, sodium hydride (25.4 mg, 0.6356 mmol) was added and reacted for 30 min in the iced water bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (112.0mg, 0.127mmol) in tetrahydrofuran (5 mL) was added dropwise to the above reaction solution, and reacted at room temperature for 30 min. The reaction was shown to be completed by LC/MS. The reaction solution was added with ethyl acetate (80 mL), washed with saturated ammonium chloride solution (15 mLX2) and saturated brine (60 mLX3), dried over anhydrous sodium sulfate, filtered, and concentrated to obtain t-butyl (5S,5aS,6S,9R)-2-(3-((t-butoxycarbonyl)amino)-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11, 13,14-pentaaza-6,9-methanonaphtho[1,8-ab]hepten-14-carboxylate (180mg, crude product). ES-API: [M+H]⁺= 998.3.

Step II: Cesium fluoride (900 mg, 5.921 mmol) was added to t-butyl (5S,5aS,6S,9R)-2-(3-((t-butoxycarbonyl)amino)-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (180mg, crude product) in N,N-dimethylformamide (10 mL), and the reaction mixture was stirred at room temperature for 2 hrs. The reaction was shown to be completed by LC/MS. The reaction solution was diluted with ethyl acetate (60 mL), washed with water (15 mLX2) and saturated brine (60 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110mg, crude product). ES-API: [M+H]⁺= 842.2.

Step III: In an iced water bath, 4 M hydrochloric acid/dioxane solution (5 mL) was slowly added to a solution of t-butyl (5S,5aS,6S,9R)-2-(3-((t-butoxycarbonyl)amino)-8-ethynylnaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110mg, crude product) in acetonitrile (10.0mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) to obtain4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-2-yl)-5-ethynylnaphthalen-2-amine (Z706, 3.06mg, total yield of 5 steps: 18.2%) as a light yellow solid. ES-API: [M+H]⁺= 642.2. ¹H NMR (400 MHz, CD₃OD) δ7.69-7.66(m, 1H), 7.41-7.25 (m, 2H), 7.14-7.00 (m, 2H), 5.43-5.40 (m, 1H), 4.60-4.10(m, 4H), 3.82-3.72(m, 2H), 3.41-3.39(m, 1H), 3.25-2.84(m, 4H), 2.87-2.85 (m, 1H), 2.60-2.56(m, 1H), 2.35-2.28 (m, 1H), 2.21-1.84 (m, 8H), 1.59-1.56 (m, 3H).

### Example 96: Synthesis of 5-ethynyl-3-fluoro-4-((5S,5aS,6S,9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z707)

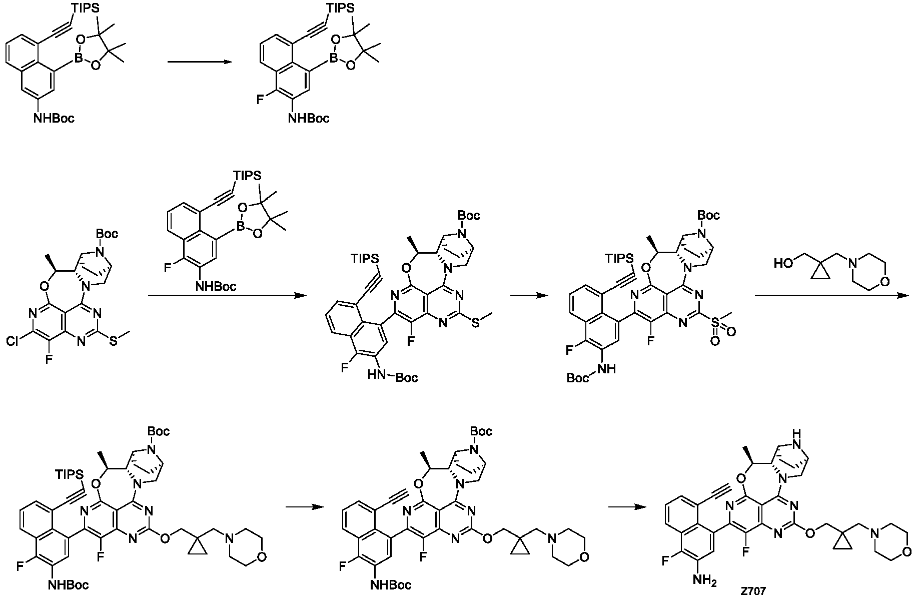

Step I: 1-chloromethyl-4-fluoro-1,4-diazabicyclo[2.2.2]octane bis(tetrafluoroborate) (354 mg, 1 mmol) was added to a solution of t-butyl (4-(4,4,5,5-tetramethyl-1,3,2-dioxaborolan-2-yl)-5-((triisopropylsilyl)ethynyl)naphthalen-2-yl)carbamate (550 mg, 1 mmol) in acetonitrile(40 mL), and the reaction solution was stirred at room temperature for 8 hrs. The reaction was shown to be completed by LC/MS. The reaction solution was concentrated under reduced pressure, and the crude product was purified by flash silica gel column chromatography (petroleum ether/ ethyl acetate = 0 - 1%) to obtain t-butyl (1-fluoro-4-(4,4,5,5-tetramethyl-1,3,2-dioxaborolan-2-yl)-5-((triisopropylsilyl)ethynyl)naphthalen-2-yl)carbamate (148 mg, yield: 26%) as a light yellow solid. ES-API: [M+ H]⁺= 568.3.

Step II: T-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (80 mg, 0.166 mmol) and t-butyl (1-fluoro-4-(4,4,5,5-tetramethyl-1,3,2-dioxaborolan-2-yl)-5-((triisopropylsilyl)ethynyl)naphthalen-2-yl)carbamate (90 mg, 0.159 mmol) were dissolved in tetrahydrofuran (5 mL) and water (1 mL). [n-butyldi(1-adamantanyl)phosphine](2-amino-1,1'-biphenyl-2-yl)palladium (II) mesylate (12.09 mg, 0.017 mmol) and potassium phosphate (105.70 mg, 0.498 mmol) were added to the solution. After that, the reaction was stirred at 65 °C for 5 hrs. After the reaction, the reaction solution was added with water (15 mL) and extracted with ethyl acetate (15 mLX3). The organic phase was washed with saturated brine (30 mL), dried over anhydrous sodium sulfate, filtered and concentrated. The crude product was purified by flash silica gel column chromatography (ethyl acetate /petroleum ether: 0-30%) to obtain t-butyl (5S,5aS,6S,9R)-2-(3-((t-butoxycarbonyl)amino)-4-fluoro-8-((triisopropylsilyl) ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (35 mg, 0.039 mmol, yield: 23.77%) as a white solid. ES-API: [M +H]⁺= 887.3.

Step III: T-butyl (5S, 5aS, 6S, 9R)-2-(3-((t-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (35 mg, 0.039 mmol) was dissolved in dichloromethane (3 mL). At room temperature, m-chloroperoxybenzoic acid (purity 85%, 24.03 mg, 0.118 mmol) was added to the solution. After that, the reaction was stirred at room temperature for 1 hr. The reaction solution was added with a saturated sodium thiosulphate (2 mL) and extracted with dichloromethane (3 mLX3). The organic phases were combined, and then washed sequentially with a saturated sodium bicarbonate aqueous solution (10 mL) and saturated sodium chloride (10 mL). The organic phase was dried over anhydrous sodium sulfate, filtered and concentrated to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (35 mg, 0.038 mmol, yield: 96.53%) as a light yellow solid. ES-API: [M +H]⁺= 919.3.

Step IV: (1-(morpholinemethyl)cyclopropyl)methanol (3 mg, 0.018 mmol) was dissolved in tetrahydrofuran (1 mL) and cooled to 0°C. Under a nitrogen atmosphere, sodium hydride (60%, 3.05 mg, 0.076 mmol) was added to the solution and stirred for 30 min. After that, butyl (5S,5aS,6S,9R)-2-(3-((t-butoxycarbonyl)amino)-4-fluoro-8-((triisopropylsilyl)ethynyl) naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (35 mg, 0.038 mmol) was slowly added to the solution and stirred 0°C for 2 hrs. The reaction solution was added with water (2 mL) to quench the reaction, and then extracted with ethyl acetate (5 mLx3). The organic phase was washed with saturated brine (5 mLX3), dried over anhydrous sodium sulfate, filtered and concentrated to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (30 mg, crude product) as a yellow solid. ES-API: [M+H]⁺=1010.3.

Step V: A mixed solution of t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (30 mg, crude product), cesium fluoride (45.10 mg, 0.297 mmol) and *N, N-*dimethylformamide (1 mL) was stirred at room temperature for 2 hrs. The reaction mixture was added with water (5 mL) and extracted with ethyl acetate (5 mL X2). The organic phase was washed with saturated brine (10 mLX3), dried over anhydrous sodium sulfate, filtered and concentrated to obtain t-butyl (5S,5aS,6S,9R)-2-(3-((t-butoxycarbonyl)amino)-8-ethynyl-4-fluoronaphthalenyl-1-yl)-1-fluoro-5-methyl-12-((1-(morpholinemethyl)cyclopropyl) methoxy)-5a,6,7,8,9,10-hexahydro-5H-4-oxa-3,10a,11,13,14-pentaaza-6,9-methanonaphtho[1,8-ab]hepten-14-carboxylate (25 mg, crude product) as a yellow oil. ES-API: [M+H]⁺=854.2.

Step VI: T-butyl (5S, 5aS, 6S, 9R)-2-(3-((t-butoxycarbonyl)amino)-8-ethynyl-4-fluoronaphthalenyl-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (25 mg, crude product) was dissolved in methanol (1 mL), and cooled to 0°C. A hydrogen chloride-dioxane solution (4 M, 4 mL) was added to the solution, and reacted with stirring at 0°C for 2 hrs. The reaction solution was concentrated, dissolved in dichloromethane (2 mL), and alkalized with ammonia in methanol (7 M) to pH 8. The obtained solution was concentrated, and the crude product was purified by preparative HPLC (formic acid method 1) to obtain 5-ethynyl-1-fluoro-4-((5S, 5aS, 6S, 9R)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z707, 1.5 mg, 0.002 mmol, diformate, yield of three steps: 5%) as a white solid. ES-API: [M+H]⁺=654.2.

### Example 97: Synthesis of 5-ethynyl-6-fluoro-4-((5S, 5aS, 6S, 9R)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-2-yl)naphthalen-2-ol (Z708)

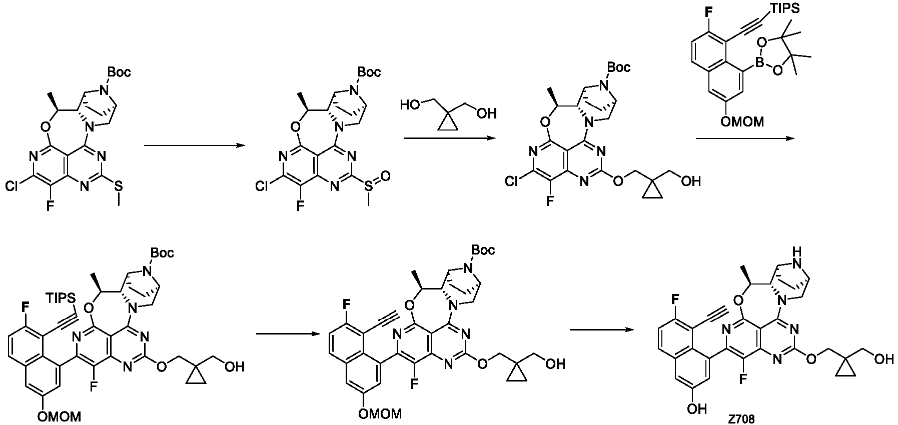

Step I: T-butyl (5S, 5aS, 6S, 9R)-2-chloro-1-fluoro-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (100 mg, 0.21 mmol) and dichloromethane (3 mL) were added to a round-bottom flask. The reaction solution was cooled to 0°C, and m-chloroperoxybenzoic acid (54 mg, 0.31 mmol) was added. The reaction was stirred at room temperature for 1 hr. The reaction solution was added with saturated sodium bicarbonate (30 mL), and extracted with ethyl acetate (20 mLX2). The organic phase was washed with saturated brine (30 mL), dried over anhydrous sodium sulfate, filtered, and concentrated to obtain t-butyl (5S, 5aS, 6S, 9R)-2-chloro-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (98 mg, crude product). ES-API: [M+H]⁺=498.2

Step II: 60% sodium hydride (33 mg, 1.38 mmol) and tetrahydrofuran (6 mL) were added to a round-bottom flask. The reaction solution was cooled to 0°C, and a solution of cyclopropane-1,1-diyldimethanol (121 mg, 1.18 mmol) in anhydrous tetrahydrofuran (1 mL) was added to the reaction solution, and reacted at room temperature for 10 min. The reaction solution was cooled to 0°C. T-butyl (5S, 5aS, 6S, 9R)-2-chloro-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (98 mg, crude product) was added to the reaction solution, and reacted at room temperature for 0.5 hr. The reaction solution was added with water (30 mL), and extracted with ethyl acetate (20 mLX2). The organic phase was washed with saturated brine (30 mL), dried over anhydrous sodium sulfate, filtered, and concentrated. The residue was purified by flash silica gel column chromatography (0-30% ethyl acetate /petroleum ether) to obtain t-butyl (5S, 5aS, 6S, 9R)-2-chloro-1-fluoro-12-((1-(hydroxymethyl)cyclopropyl)methoxy)-5-methyl-5a,6,7,8,9,10-hexahydro-5H-4-oxy-3,10a,11,13,14-pentaaza-6,9-methanonaphtho[1,8-ab]hepten-14-carboxylate (40 mg, yield: 37%). ES-API: [M+H]⁺=536.2.

Step III: T-butyl (5S, 5aS, 6S, 9R)-2-chloro-1-fluoro-12-((1-(hydroxymethyl) cyclopropyl)methoxy)-5-methyl-52a,6,7,8,9,10-hexahydro-5H-4-oxa-3,10a,11,13,14-pentaaza-6,9-methanonaphtho[1,8-ab]hepten-14-carboxylate (40 mg, 0.075 mmol), ((2-fluoro-6-(methoxymethoxy)-8-(4,4,5,5-tetramethyl-1,3,2-dioxaborolan-2-yl)naphthalen-1-yl)ethynyl) triisopropylsilane (76 mg, 0.15 mmol), [n-butyldi(1-adamantanyl)phosphine](2-amino-1,1'-biphenyl-2-yl)palladium (II) mesylate(5 mg, 0.007 mmol), potassium phosphate (47 mg, 0.22 mmol), tetrahydrofuran (3 mL) and water (0.6 mL) were added to a round-bottom flask. Under a nitrogen atmosphere, the system was heated in a microwave reactor at 80°C for 40 min. The reaction solution was added with water (30 mL), and extracted with ethyl acetate (30 mLX3). The organic phase was dried over anhydrous sodium sulfate, filtered and concentrated. The crude product was purified by flash silica gel column chromatography (ethyl acetate /petroleum ether: 0-50%) to obtain t-butyl (5S, 5aS, 6S, 9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (35 mg, yield: 53%). ES-API: [M+H]⁺=886.2.

Step IV: T-butyl (5S, 5aS, 6S, 9R)-1-fluoro-2-(7-fluoro-3-(methoxymethoxy)-8-((triisopropylsilyl)ethynyl)naphthalen-1-yl)-12-(1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (35 mg, 0.039 mmol), cesium fluoride (60 mg, 0.39 mmol) and N, N-dimethylformamide (2 mL) were added to a round-bottom flask. The reaction was stirred at room temperature for 1 hr. The reaction solution was added with water (30 mL), and extracted with ethyl acetate (30 mLx3). The organic phase was washed with saturated brine (30 mLX2), dried over anhydrous sodium sulfate, filtered, and concentrated to obtain t-butyl (5S, 5aS, 6S, 9R)-2-(8-ethynyl-7-fluoro-3-(methoxymethoxy)naphthalen- 1-yl)-1-fluoro-12-((1-(hydroxymethyl) cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25mg, yield: 86%). ES-API: [M+H]⁺=730.2.

Step V: T-butyl (5S, 5aS, 6S, 9R)-2-(8-ethynyl-7-fluoro-3-(methoxymethoxy)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (25mg, 0.034 mmol) was dissolved in acetonitrile (2 mL). The solution was cooled to 0°C, and a 4M hydrogen chloride/dioxane solution (2 mL, 8 mmol) was added. The reaction was stirred at 5°C for 1 hr. The reaction solution was concentrated to dryness at 40°C, and the crude product was purified by preparative HPLC (ammonium bicarbonate method 1) to obtain 5-ethynyl-6-fluoro-4-((5S,5aS,6S,9R)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-2-yl)naphthalen-2-ol (Z708, 4mg, yield: 20%). ¹HNMR(400 MHz, CD₃OD): 7.85-7.80 (m, 1H), 7.33-7.27 (m, 2H), 7.22-7.11 (m, 1H), 5.42-5.38 (m, 1H), 4.57-4.52 (m, 1H), 4.42 (s, 2H), 4.11-4.08 (m, 1H), 3.76-3.52 (m, 3H), 3.22-3.12 (m, 1H), 2.10-2.03 (m, 1H), 1.95-1.81 (m, 3H), 1.59-1.56 (m, 3H), 1.40-1.25 (m, 2H), 0.67-0.60 (m, 4H).ES-API: [M+H]⁺=586.1.

### Example 98 Synthesis of 5-ethynyl-4-((5S, 5aS, 8R)-1-fluoro-5,8-dimethyl-11-((1-(morpholinomethyl)cyclopropyl)methoxy)-5,5a,6,7,8,9-hexahydro-4-oxa-3,7,9a,10,12-pentaazabenzo[4,5]cycloheptyl[1,2,3-de]naphthalen-2-yl)naphthalen-2-amine (Z709)

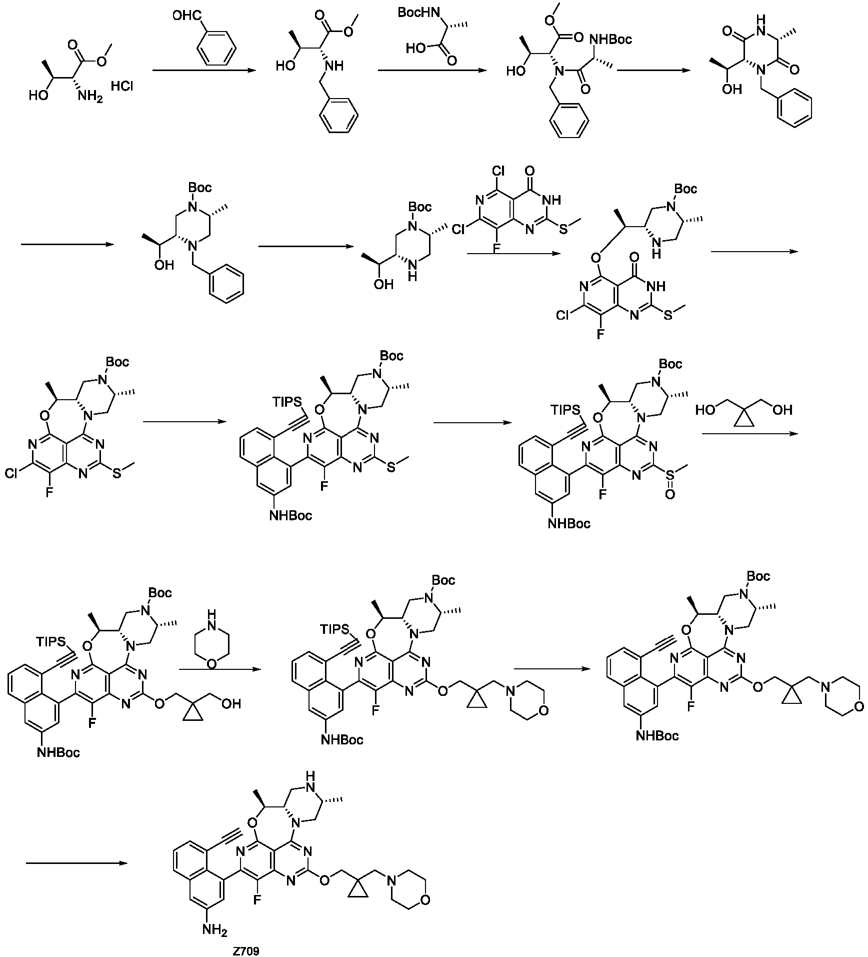

Step I: D-threonine methyl ester hydrochloride (4.5 g, 26.53 mmol) was dissolved in methanol (50 mL). At 0°C, benzaldehyde (2.82 g, 26.53 mmol) was added. The reaction was stirred at 0°C for 10 min, and then stirred at room temperature for 6 hrs. The reaction solution was cooled to 0°C, sodium borohydride (1.5 g, 39.80 mmol) was added batchwise, and the reaction was stirred at room temperature for 18 hrs. The reaction solution was quenched with a saturated ammonium chloride solution (30 mL) and water (30 mL), and extracted with ethyl acetate (60 mL×3). The organic phases were combined and washed with saturated brine (50 mL). The organic phase was dried over anhydrous sodium sulfate, filtered and concentrated. The crude product was purified by flash silica gel column chromatography (tetrahydrofuran/petroleum ether: 0-20%) to obtain the target product benzyl-D-threonine methyl ester (5.0g, yield: 84.4%) as a colorless liquid. ES-API: [M+H]⁺= 224.0.

Step II: N-(t-butoxycarbonyl)-D-alanine (8.47 g, 44.79 mmol) and N-methylmorpholine (4.53 g, 44.79 mmol) were dissolved in tetrahydrofuran (80 mL). At 0°C, isobutyl chloroformate (4.59 g, 33.59 mmol) was added dropwise, and the reaction was slowly heated to room temperature and stirred at room temperature for 1 **hr.** The reaction solution was cooled to 0°C. A solution of benzyl-D-threonine methyl ester (5.0 g, 22.39 mmol) and N-methylmorpholine (3.40 g, 33.59 mmol) in tetrahydrofuran (20 mL) was added, and the reaction was stirred at room temperature for 48 hrs. The reaction solution was added with ethyl acetate (200 mL), sequentially washed with 1N dilute hydrochloric acid (100 mL × 2), a saturated sodium bicarbonate solution (60 mL), and saturated brine (60 mL). The organic phase was dried over anhydrous sodium sulfate, and filtered. The filtrate was concentrated under reduced pressure. The crude product was purified by flash silica gel column chromatography (tetrahydrofuran/petroleum ether: 0-30%) to obtain the target product N-benzyl-N-(t-butoxycarbonyl)-D-alanine)-D-threonine methyl ester (2.3 g, yield: 26.0%) as a colorless liquid. ES-API: [M+H]⁺= 395.2.

Step III: N-benzyl-N-(t-butoxycarbonyl)-D-alanine)-D-threonine methyl ester (2.3 g, 5.83 mmol) was dissolved in dichloromethane (25 mL), trifluoroacetic acid (5 mL) was added, and the reaction was stirred at room temperature for 2 hrs. The reaction solution was concentrated under reduced pressure, added with dichloromethane (20 mL), and alkalized with a saturated sodium bicarbonate solution. The reaction was stirred at room temperature for 1 hr. The reaction solution was extracted with dichloromethane (50 mL). The organic phase was dried over anhydrous sodium sulfate, and filtered. The filtrate was concentrated under reduced pressure. The crude product was purified by flash silica gel column chromatography (tetrahydrofuran/petroleum ether: 0-70%) to obtain the target product (3R, 6R)-1-benzyl-6-((S)-1-hydroxyethyl)-3-methylpiperazine-2,5-dione (1.2 g, yield: 78.5%) as a white solid. ES-API: [M+H]⁺= 263.1.

Step IV: (3R, 6R)-1-benzyl-6-((S)-1-hydroxyethyl)-3-methylpiperazine-2,5-dione(850 mg, 3.24 mmol) was dissolved in ethylene glycol dimethyl ether (50 mL). Under a nitrogen atmosphere, sodium borohydride (1.23 g, 32.40 mmol) was added at 0 °C, then boron trifluoride etherate (2.76 g, 19.44 mmol) was slowly added dropwise, and the reaction solution was heated to reflux for 4 hrs. The reaction solution was cooled to 0°C, methanol (40 mL) was slowly added, then concentrated hydrochloric acid (17 mL) was added dropwise, and the reaction solution was heated to reflux for 70 min. The reaction solution was cooled to 0 °C, adjusted to pH 10 with a 5 M sodium hydroxide aqueous solution. Dichloromethane(40 mL) and di-t-butyl dicarbonate (1.06 g, 4.86 mmol) were added, the reaction was stirred at room temperature for 30 min. The reaction solution was extracted with dichloromethane (50 mL). The organic phase was dried over anhydrous sodium sulfate, and filtered. The filtrate was concentrated under reduced pressure. The crude product was purified by flash silica gel column chromatography (tetrahydrofuran/petroleum ether: 0-20%) to obtain the target product t-butyl (2R, 5S)-4-benzyl-5-((S)-1-hydroxyethyl)-2-methylpiperazine-1-carboxylate (920 mg, yield: 84.9%) as a white solid. ES-API: [M+H]⁺= 335.2.

Step V: T-butyl (2R, 5S)-4-benzyl-5-((S)-1-hydroxyethyl)-2-methylpiperazine-1-carboxylate (500 mg, 1.50 mmol) was dissolved in methanol (15 mL) and a 7 M aminomethanol solution (1.5 mL). 10% palladium on carbon (200 mg) was added, and the reaction was stirred under a hydrogen atmosphere at room temperature for 18 hrs. The reaction solution was filtered through diatomite, and the filter cake was washed with methanol. The filtrate was concentrated to obtain a the target product t-butyl (2R, 5S)-5-((S)-1-hydroxyethyl)-2-methylpiperazine-1-carboxylate (365 mg, yield: 100%) as a colorless liquid. ES-API: [M+H]⁺= 245.1.

Step VI: T-butyl (2R, 5S)-5-((S)-1-hydroxyethyl)-2-methylpiperazine-1-carboxylate (365 mg, 1.49 mmol) was dissolved in tetrahydrofuran (25 mL). 60% sodium hydride (299 mg, 7.47 mmol) was added at 0 °C and the reaction was stirred at 0°C for 30 min. Then 5,7-dichloro-8-fluoro-2-(methylthio)pyridine[4,3-d]pyrimidine-4(3H)-one (544 mg, 1.94 mmol) was added, and the reaction was stirred at room temperature for 18 hrs. The reaction solution was quenched with a saturated ammonium chloride solution (4 mL) and water (10 mL), and extracted with ethyl acetate (60 mL×2). The organic phases were combined and washed with saturated brine (30 mL). The organic phase was dried over anhydrous sodium sulfate, filtered and concentrated to obtain the target product t-butyl (2R,5S)-5-((S)-1-((7-chloro-8-fluoro-2-(methylthio)-4-oxo-3,4-dihydropyrido[4,3-d]pyrimidine-5-yl)oxy)ethyl)-2-methylpiperazine-1-carboxylate (728 mg, yield: 100%) as a light yellow solid. ES-API: [M+H]⁺= 488.0.

Step VII: T-butyl (2R,5S)-5-((S)-1-((7-chloro-8-fluoro-2-(methylthio)-4-oxo-3,4-dihydropyrido[4,3-d]pyrimidine-5-yl)oxy)ethyl)-2-methylpiperazine-1-carboxylate (728 mg, 1.49 mmol) and diisopropylethyl amine (964 mg, 7.46 mmol) were dissolved in dichloromethane (15 mL). 50% 1-propylphosphoric anhydride solution ethyl acetate (2.85 g, 4.48 mmol) was added at room temperature, and the reaction was stirred at room temperature for 3 hrs. The reaction solution was added with dichloromethane (30 mL), sequentially washed with water (20 mL×2) and saturated brine (20 mL), dried over anhydrous sodium sulfate, filtered and concentrated. The crude product was purified by flash silica gel column chromatography (tetrahydrofuran/petroleum ether: 0-20%) to obtain the target product t-butyl (5S, 5aS, 8R)-2-chloro-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalen-7(5H)-carboxylate (500 mg, yield: 71.3%) as a white solid. ES-API: [M+H]⁺= 470.1.

Step VIII: Potassium phosphate (136 mg, 0.64 mmol) and [n-butyldi(1-adamantanyl) phosphine](2-amino-1,1'-biphenyl-2-yl)palladium (II) mesylate (12 mg, 0.017 mmol) were added to t-butyl (5S, 5aS, 8R)-2-chloro-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalen-7(5H)-carboxylate (100 mg, 0.21 mmol) and t-butyl (4-(4,4,5,5-tetramethyl-1,3,2-dioxaborolan-2-yl)-5-(triisopropylsilyl) ethynyl)naphthalen-2-yl)carbamate (117 mg, 0.21 mmol) in tetrahydrofuran (5 mL) and water (1 mL), purged 3 times with nitrogen, and reacted at 80°C for 3 hrs. The reaction solution was added with ethyl acetate (60 mL), and washed with saturated brine (30 mL). The organic phase was dried over anhydrous sodium sulfate, and filtered. The filtrate was concentrated under reduced pressure. The crude product was purified by flash silica gel column chromatography (tetrahydrofuran/petroleum ether: 0-25%) to obtain the target product t-butyl (5S, 5aS, 8R)-2-(3-((t-butoxycarbonyl)amino)-8-((triisopropylsilyl)ethynyl)naphthalen-1-yl)-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalen-7(5H)-carboxylate (80 mg, yield: 43.9%) as a light yellow solid. ES-API: [M+H]⁺=857.2.

Step IX: T-butyl (5S, 5aS, 8R)-2-(3-((t-butoxycarbonyl)amino)-8-((triisopropylsilyl)ethynyl) naphthalen-1-yl)-1-fluoro-5,8-dimethyl-11-(methylthio)-5a,6,8,9-tetrahydro-4-oxa-3,7,9a,10,12-pentaazabenzo[4,5]cyclohepta[1,2,3-de]naphthalen-7(5H)-carboxylate (80 mg, 0.093 mmol) was dissolved in dichloromethane (10 mL). m-chloroperoxybenzoic acid (28 mg, 0.14 mmol) was added at room temperature, and the reaction was stirred at room temperature for 1 hr. The reaction solution was added with dichloromethane (20 mL), and sequentially washed with saturated sodium thiosulphate (2 mL), saturated sodium bicarbonate (5 mL), and saturated brine (5 mL). The organic phase was dried over anhydrous sodium sulfate, and filtered. The filtrate was concentrated under reduced pressure, to obtain the target product t-butyl (5S, 5aS, 8R)-2-(3-((t-butoxycarbonyl)amino)-8-((triisopropylsilyl)ethynyl)naphthalen-1-yl)-1-fluoro-5,8-dimethyl-11-(methylsulfinyl)-5a,6,8,9-tetrahydro-4-oxa-3,7,9a,10,12-pentaazabenzo[4,5]cyclohepta[1,2,3-de]naphthalen-7(5H)-carboxylate (80 mg, yield: 98.2%) as a light yellow solid. ES-API:[M+H]⁺=873.3.

Step X: 1,1-cyclopropyldimethanol (37 mg, 0.37 mmol) was dissolved in tetrahydrofuran(8 mL). At 0°C, 60% sodium hydride (11 mg, 0.28 mmol) was added, and the reaction was stirred at this temperature for 30 min. Then, a solution of t-butyl (5S, 5aS, 8R)-2-((3-(t-butoxycarbonyl)amino)-8-((triisopropylsilyl)ethynyl)naphthalen-1-yl)-1-fluoro-5,8-dimethyl-11-(methylsulfinyl)-5a,6,8,9-tetrahydro-4-oxa-3,7,9a,10,12-pentaazabenzo[4,5]cyclohepta[1,2,3-de]naphthalen-7(5H)-carboxylate (80 mg, 0.092 mmol) in tetrahydrofuran (2 mL) was added dropwise, and stirred at this temperature for 30 min. The reaction solution was added with ethyl acetate (40 mL), and sequentially added with water (10 mL) and saturated brine (15 mL). The organic phase was dried over anhydrous sodium sulfate, and filtered. The filtrate was concentrated under reduced pressure. The crude product was purified by flash silica gel column chromatography (tetrahydrofuran/petroleum ether: 0-50%) to obtain the target product t-butyl (5S,5aS,8R)-2-(3-((t-butoxycarbonyl)amino)-8-((triisopropylsilyl)ethynyl)naphthalen-1-yl)-1-fluoro-11-((1-(hydroxymethyl)cyclopropyl)methoxy)-5,8-dimethyl-5a,6,8,9-tetrahydro-4-oxa-3,7,9a,10,12-pentaazabenzo[4,5]cyclohepta[1,2,3-de]naphthalen-7(5H)-carboxylate (50 mg, yield: 59.9%) as a light yellow solid. ES-API: [M+H]⁺=911.3.

Step XI: T-butyl (5S,5aS,8R)-2-(3-((t-butoxycarbonyl)amino)-8-((triisopropylsilyl)ethynyl) naphthalen-1-yl)-1-fluoro-11-((1-(hydroxymethyl)cyclopropyl)methoxy)-5,8-dimethyl-5a,6,8,9-tetrahydro-4-oxa-3,7,9a,10,12-pentaazabenzo[4,5]cyclohepta[1,2,3-de]naphthalen-7(5H)-carboxylate (50 mg, 0.055 mmol) and diisopropylethyl amine (35 mg, 0.275 mmol) were dissolved in N,N-dimethylformamide (3 mL). At 0°C, methanesulfonyl chloride (19 mg, 0.165 mmol) in tetrahydrofuran(0.5 mL) was added dropwise, and the reaction was stirred at this temperature for 1 hr. Then morpholine (96 mg, 1.10 mmol) and potassium carbonate(61 mg, 0.44 mmol) were added dropwise, and the reaction was stirred at 50°C for 24 hrs. The reaction solution was added with ethyl acetate (40 mL), and sequentially washed with water (10 mL) and saturated brine (15 mL×3). The organic phase was dried over anhydrous sodium sulfate, and filtered. The filtrate was concentrated under reduced pressure. The crude product was purified by TlC (dichloromethane/methanol =25: 1) to obtain the target product t-butyl (5S,5aS,8R)-2-(3-((t-butoxycarbonyl)amino)-8-((triisopropylsilyl)ethynyl)naphthalen-1-yl)-1-fluoro-5,8-dimethyl-11-((1-(morpholinomethyl)cyclopropyl)methoxy)-5a,6,8,9-tetrahydro-4-oxa-3,7,9a, 10, 12-pentaazabenzo[4,5]cyclohepta[1,2,3-de]naphthalen-7(5H)-carboxylate (20 mg, yield: 37.2%) as a white solid. ES-API: [M+H]⁺=980.3.

Step XII: T-butyl (5S,5aS,8R)-2-(3-((t-butoxycarbonyl)amino)-8-((triisopropylsilyl)ethynyl) naphthalen-1-yl)-1-fluoro-5,8-dimethyl-11-((1-(morpholinomethyl)cyclopropyl)methoxy)-5a,6,8,9-tetrahydro-4-oxo-3,7,9a,10,12-pentaazabenzo[4,5]cyclohepta[1,2,3-de]naphthalen-7(5H)-carboxylate (20 mg, 0.020 mmol) was dissolved in N,N-dimethylformamide (1.5 mL). Cesium fluoride (31 mg, 0.20 mmol) was added at room temperature, and the reaction was stirred at room temperature for 30 min. The reaction solution was added with ethyl acetate (25 mL), and sequentially washed with water (5 mL), dilute saline (10 mL×3), and saturated brine (10 mL). The organic phase was dried over anhydrous sodium sulfate, and filtered. The filtrate was concentrated under reduced pressure, to obtain the target product t-butyl (5S,5aS,8R)-2-(3-((t-butoxycarbonyl)amino)-8-ethynylnaphthalen-1-yl)-1-fluoro-5,8-dimethyl-11-((1-(morpholinemethyl)cyclopropyl)methoxy)-5a,6,8,9-tetrahydro-4-oxa-3,7,9a,10,12-pentaazabenzo[4,5]cyclohepta[1,2,3-de]naphthalen-7(5H)-carboxylate (15 mg,yield: 89.2%) as a light yellow solid. ES-API: [M+H]⁺=824.2.

Step XIII: T-butyl (5S,5aS,8R)-2-(3-((t-butoxycarbonyl)amino)-8-ethynylnaphthalen-1-yl)-1-fluoro-5,8-dimethyl-11-((1-(morpholinemethyl)cyclopropyl)methoxy)-5a,6,8,9-tetrahydro-4-oxa-3,7,9a,10,12-pentaazabenzo[4,5]cyclohepta[1,2,3-de]naphthalen-7(5H)-carboxylate (15 mg, 0.018 mmol) was dissolved in ethyl acetate (1 mL). 4 M hydrogen chloride1,4-dioxane (2 mL) was added at 0°C, and the reaction was stirred at room temperature for 2 hrs. The reaction solution was concentrated under reduce pressure. The crude product was purified by preparative HPLC(ammonium bicarbonate method 1) to obtain the target product 5-ethynyl-4-((5S, 5aS, 8R)-1-fluoro-5,8-dimethyl-11-((1-(morpholinemethyl)cyclopropyl)methoxy)-5,5a,6,7,8,9-hexahydro-4-oxa-3,7,9a,10,12-pentaazabenzo[4,5]cyclohepta[1,2,3-de]naphthalen-2-yl)naphthalen-2-amine (Z709, 4 mg, yield: 35.2%) as a white solid. ES-API: [M+H]⁺= 624.2.

### Example 99: Synthesis of 5-ethynyl-4-((5S,5aS,6S,9R)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-nonaphtho[1,8-ablhepten-2-yl)naphthalen-2-amine (Z710)

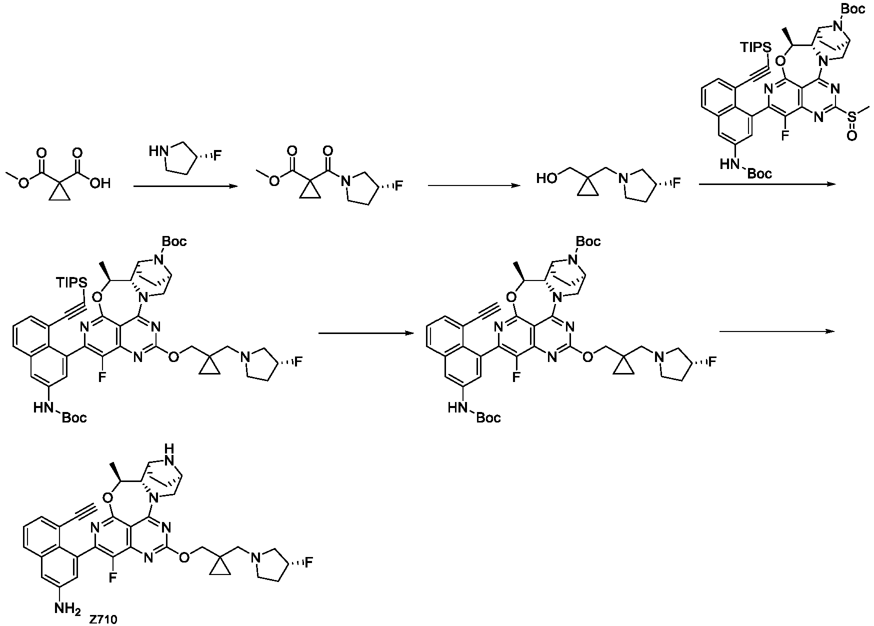

Step I: Oxalyl chloride (3.288 mL, 38.854 mmol) and N,N-dimethylformamide (0.05 mL) were added to a solution of 1-(methoxycarbonyl)cyclopropane-1-carboxylic acid (1.4g, 9.713mmol) in dichloromethane (100 mL), and reacted with stirring at 0°C and 35°C for 3 hrs. The reaction solution was concentrated. The residue was dissolved in dichloromethane (50 mL). At 0°C, triethyl amine (6.750 mL, 48.565 mmol) and (3R)-3-fluorotetrahydropyrrole (0.95 g, 10.685 mmol) were added, reacted with stirring at room temperature for 3 hrs, filtered and concentrated. The residue was purified by silica gel column chromatography (eluting with methanol in dichloromethane=1: 20), to obtain methyl (R)-1-(3-fluoropyrrolidin-1-carbonyl)cyclopropane-1-carboxylate (650 mg, 3.020 mmol, yield: 31.09%). ES-API: [M+H]⁺=216.1.

Step II: At -20°C, lithium aluminum hydride (1.859 mL, 1.859 mmol, 1 M solution in tetrahydrofuran) was added to a solution of methyl (R)-1-(3-fluoropyrrolidin-1-carbonyl)cyclopropane-1-carboxylate (650 mg, 3.020 mmol) in tetrahydrofuran (15 mL). The reaction was stirred at -10°C for 30 min, quenched with a 15%sodium hydroxide aqueous solution (0.5 mL), and filtered through diatomite. The filtrate was dried over anhydrous sodium sulfate, filtered and concentrated, to obtain (R)-(1-((3-fluoropyrrolidin-1-yl)methyl)cyclopropyl)methanol (100 mg, 0.577 mmol, yield: 62.12%), as a yellow oil. ES-API: [M+H]⁺=174.1.

Step III: In an iced water bath, (R)-(1-((3-fluoropyrrolidin-1-yl)methyl)cyclopropyl)methanol (100 mg, 0.577 mmol) was dissolved in dry tetrahydrofuran (5 mL), and then 60% sodium hydride (12.88 mg, 0.322 mmol) was added, and reacted for 10 min under a nitrogen atmosphere in an iced water bath. Then, t-butyl (5S,5aS,6S,9R)-2-(3-((t-butoxycarbonyl)amino)-8-((triisopropylsilyl) ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (95 mg, 0.107 mmol) was added. After the reaction, the reaction was added with an ammonium chloride aqueous solution (5 mL) and extracted with ethyl acetate (30 mLx3). The organic phases were combined, washed with saturated brine (30 mLx1), dried over anhydrous sodium sulfate, filtered, and concentrated. The residue was purified by silica gel column chromatography (petroleum ether/ethyl acetate =1/5) to obtain t-butyl (5S,5aS,6S,9R)-2-(3-((t-butoxycarbonyl)amino)-8-((triisopropylsilyl)ethynyl)naphthalen-1-ethyl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.030 mmol, yield: 28.2%). ES-API: [M+H]⁺=994.5.

Step IV: Cesium fluoride (45.6 mg, 0.3 mmol) was added to a solution of t-butyl (5S,5aS,6S,9R)-2-(3-((t-butoxycarbonyl)amino)-8-((triisopropylsilyl)ethynyl)naphthalen-1-ethyl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (30 mg, 0.030 mmol) in N,N-dimethylformamide (3 mL), and stirred at room temperature for 2 hrs. The reaction was shown to be completed by LC/MS. The reaction solution was washed with ethyl acetate (20 mL), water (15 mLX2), and saturated brine (15 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (24.5 mg, yield: 98%). ES-API: [M+H]⁺= 838.3.

Step V: 4 M hydrochloric acid/dioxane solution (2 mL) was slowly added to a solution of t-butyl (5S,5aS,6S,9R)-2-(3-((t-butoxycarbonyl)amino)-8-ethynylnaphthalen-1-yl)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (24.5 mg, 0.029 mmol) in ethyl acetate (1mL) in an iced water bath, and stirred at room temperature for 0.5 hr. The reaction was shown to be completed by LC/MS. The reaction solution was concentrated to dryness under reduced pressure, and the crude product was purified by preparative HPLC (ammonium bicarbonate method 2) to obtain5-ethynyl-4-((5S,5aS,6S,9R)-1-fluoro-12-((1-(((R)-3-fluoropyrrolidin-1-yl)methyl)cyclopropyl)methoxy)-5-methyl-5a,6,7,8,9,10-hexahydro-5H-4-oxa-3,10a,11,13,14-pentaaza-6,9-nonaphtho[1,8-ab]hepten-2-yl)naphthalen-2-amine (Z710, 0.9 mg, yield: 4.9%) as a light yellow solid. ES-API: [M+H]⁺=638.3.

### Example 100: Synthesis of 4-((5S,5aS,6S,9R)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z711)

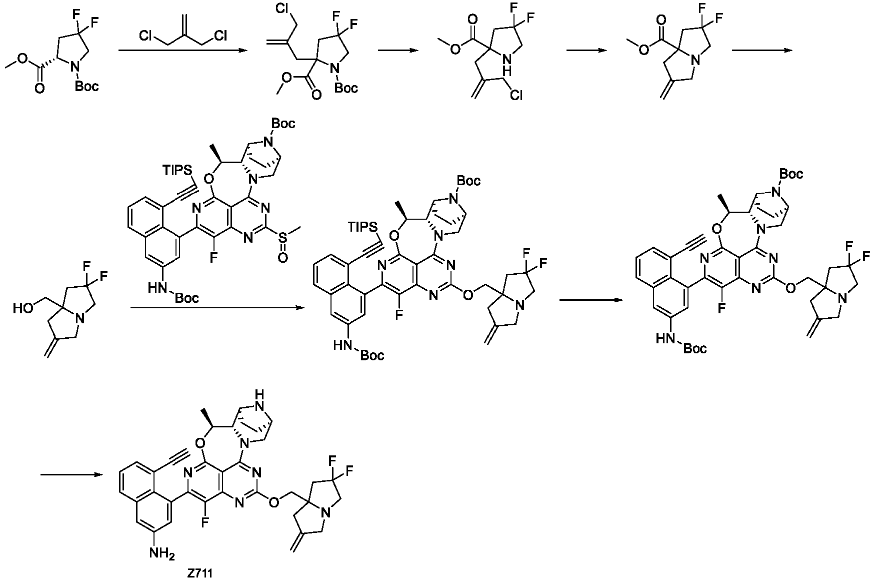

Step I: Under a nitrogen atmosphere, (S)-1-t-butyl 2-methyl 4,4-difluoropyrrolidin-1,2-dicarboxylate (1 g, 3.77 mmol) was dissolved in tetrahydrofuran (10 mL), and cooled to -60°C. Lithium diisopropylamide (7.54 mL, 7.54 mmol) was slowly added, and stirred for 1 hr. Then a solution of 3-chloro-2-chloromethylpropene (0.71 g, 5.66 mmol) in tetrahydrofuran (5 mL) was added to the solution, slowly heated to room temperature, and stirred for 2 hrs. After the reaction, the reaction solution was quenched with saturated ammonium chloride (10 mL) and extracted with ethyl acetate (10 mL X 3). The organic phases were combined, dried over anhydrous sodium sulfate, filtered, concentrated, and purified by flash silica gel column chromatography (0-20% tetrahydrofuran/petroleum ether), to obtain 1-(t-butyl)2-methyl2-(2-(chloromethyl)allyl)-4,4-difluoropyrrolidin-1,2-dicarboxylate (450 mg, yield: 34%) as a yellow oil. ES-API: [M+H⁺-56]= 298.0.

Step II: In an ice bath, 1-(t-butyl) 2-methyl 2-(2-(chloromethyl)allyl)-4,4-difluoropyrrolidin-1,2-dicarboxylate (450 mg, 1.27 mmol) was dissolved in dichloromethane (6 mL), and then trifluoroacetic acid (3 mL) was added and stirred at room temperature for 1 hr. After the reaction, the reaction solution was concentrated to obtain methyl 2-(2-(chloromethyl)allyl)-4,4-difluoropyrrolidin-2-carboxylate (320 mg, crude product). ES-API: [M+H⁺]= 254.1.

Step III: Methyl 2-(2-(chloromethyl)allyl)-4,4-difluoropyrrolidin-2-carboxylate (320 mg, 1.26 mmol) was dissolved in acetonitrile (10 mL), and then sodium bicarbonate (546 mg, 6.51 mmol) and potassium iodide (22 mg, 0.13 mmol) were added, and stirred at room temperature for 1 hr. The reaction solution was added to water (10 mL), and extracted with ethyl acetate (10 mLX3). The organic phases were combined, dried over anhydrous sodium sulfate, filtered and concentrated. The residue was purified by flash silica gel column chromatography (0-50% tetrahydrofuran/petroleum ether), to obtain methyl 2,2-difluoro-6-methylenetetrahydro-1H-pyrrolizin-7a(5H)-carboxylate (220 mg, yield of two steps: 78%) as a yellow oil. ES-API[M+H⁺]= 218.1.

Step IV: In an ice bath, a 1 M lithium aluminium hydride solution in tetrahydrofuran (0.46 mL, 0.46 mmol) was added to a solution of methyl 2,2-difluoro-6-methylenetetrahydro-1H-pyrrolizin-7a(5H)-carboxylate (100 mg, 0.46 mmol) in tetrahydrofuran (2 mL), and stirred for 1 hr. After the reaction, the reaction solution was quenched with sodium sulfate decahydrate (2 g), stirred for 10 min, filtered and concentrated to obtain (2,2-difluoro-6-methylenetetrahydro-1H-pyrrolizin-7a(5H)-yl)methanol (85 mg, crude product). ES-API: [M+H⁺]= 190.1.

Step V: In an ice bath, (2,2-difluoro-6-methylenetetrahydro-1H-pyrrolizin-7a(5H)-yl)methanol (72 mg, 0.38 mmol) in tetrahydrofuran (1 mL) was added to a solution of sodium hydride (40 g, 1.00 mmol) in tetrahydrofuran (1 mL), and stirred for 0.5 hr. Then, a solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (112 mg, 0.13 mmol) in tetrahydrofuran (0.5 mL) was added, and continuously stirred for 0.5 hr. After the reaction, the reaction solution was quenched with a saturated ammonium chloride solution (10 mL), and extracted with ethyl acetate (10 mLX3). The organic phases were combined, dried over anhydrous sodium sulfate, filtered, concentrated, and purified by flash silica gel column chromatography (0-100%ethyl acetate/petroleum ether), to obtain t-butyl (5S,5aS,6S,9R)-2-(3-((t-butoxycarbonyl)amino)-8-((triisopropylsilyl)ethynyl)naphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (60 mg, yield: 47%) as a yellow oil. ES-API[M+H⁺]= 1010.2.

Step VI: Under a nitrogen atmosphere, t-butyl (5S,5aS,6S,9R)-2-(3-((t-butoxycarbonyl) amino)-8-((triisopropylsilyl)ethynyl)naphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (70 mg, 0.07 mmol) was dissolved in N,N-dimethylformamide (1 mL), and then cesium fluoride (105 mg, 0.69 mmol) was added, and stirred at room temperature for 1 hr. The reaction solution was added with ethyl acetate (10 mL), and sequentially washed with saturated brine (5 mL) and water (5 mL). The organic phase was dried over anhydrous sodium sulfate, filtered and concentrated to obtain t-butyl (5S,5aS,6S,9R)-2-(3-((t-butoxycarbonyl)amino)-8-ethynylnaphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methylnaphtho[1,8-ab]hepten-14-carboxylate (59 mg, crude product). ES-API[M+H⁺]= 854.2.

Step VII: T-butyl (5S,5aS,6S,9R)-2-(3-((t-butoxycarbonyl)amino)-8-ethynylnaphthalen-1-yl)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (59 mg, 0.07 mmol) was dissolved in ethyl acetate (1 mL), 4 M hydrochloric acid-dioxane (2 mL) was added, and stirred at room temperature for 2 hrs. After the reaction, the reaction solution was quenched with saturated sodium bicarbonate, alkalized to pH 8, and extracted with dichloromethane (4 mLX3). The organic phases were combined, dried over anhydrous sodium sulfate, filtered and concentrated. The residue was purified by preparative HPLC (ammonium bicarbonate 1), to obtain 4-((5S,5aS,6S,9R)-12-((2,2-difluoro-6-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z711, 11.95 mg, yield: 26%). ES-API[M+H⁺]= 654.3.

### Example 101: Synthesis of 4-(5S, 5aS, 6S, 9R)-12-(1-(3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z712)

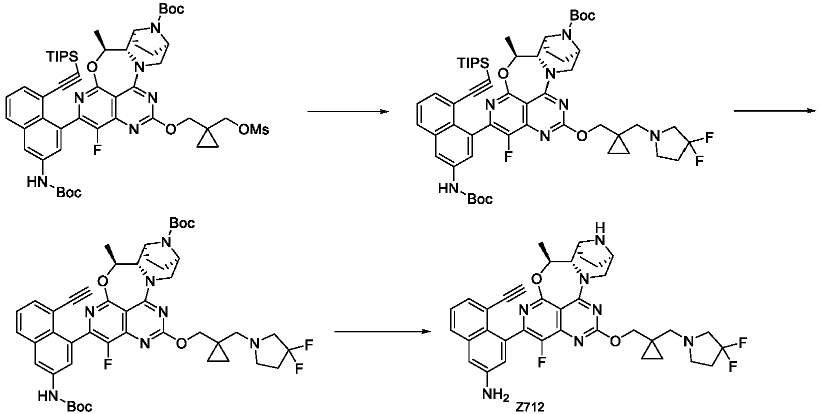

Step I: T-butyl (5S, 5aS, 6S, 9R)-2-(3-((t-butoxycarbonyl)amino)-8-((triisopropylsilyl) ethynyl)naphthalen-1-yl)-1-fluoro-5-methyl-12-((1-(((methylsulfonyl)oxy)methyl)cyclopropyl) methoxy)-5a,6,7,8,9,10-hexahydro-5H-4-oxa-3,10a,11,13,14-pentaaza-6,9-methanonaphtho[1,8-ab]hepten-14-carboxylate (45 mg, 0.05 mmol) was dissolved in acetone (2 mL). 3,3-difluoropyrrolidine hydrochloride (19 mg, 0.14 mmol), potassium carbonate(37 mg, 0.27 mmol), and sodium iodide (3 mg, 0.02 mmol) were added, and reacted at 80°C under microwave for 1 hr. After the reaction, the reaction solution was diluted with ethyl acetate (5 mL), washed with water (5 mLX2) and sodium chloride (5 mLX2), dried over anhydrous sodium sulfate, and filtered. The crude product was purified by automated flash silica gel chromatography (dichloromethane: methanol =15:1) to obtain t-butyl (5S, 5aS, 6S, 9R)-2-(3-(((t-butoxycarbonyl)amino)-8-((triisopropylsilyl)ethynyl)naphthalen-1-yl)-12-((1-((3,3-difluoropyrrolidin-1-yl)methyl) cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (15 mg, 0.01 mmol, yield: 32.9%). ES-API: [M/2+H]⁺= 507.1.

Step II: Cesium fluoride (7 mg, 0.05mmol) was added to t-butyl (5S, 5aS, 6S, 9R)-2-(3-((t-butoxycarbonyl)amino)-8-((triisopropylsilyl)ethynyl)naphthalen-1-yl)-12-((1-((3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (15 mg, 0.01 mmol) in N,N-dimethylformamide (2 mL), and the reaction mixture was stirred at room temperature for 2 hrs. The reaction was shown to be completed by LC/MS. The reaction solution was diluted with ethyl acetate (5 mL), washed with water (4 mLX2) and saturated brine (4 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((1-(3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (12 mg, crude product). ES-API: [M+H]⁺= 857.2.

Step III: In an iced water bath, a 4 M hydrochloric acid-dioxane solution (2.0 mL, 4M, 8.0mmol) was slowly added to a solution of t-butyl (5S, 5aS, 6S, 9R)-2-(3-((t-butoxycarbonyl)amino)-8-ethynylnaphthalen-1-yl)-12-((1-(3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (12 mg, 0.01 mmol) in ethyl acetate (1 mL). The reaction mixture was stirred at 0°C for 3 hrs. The reaction was shown to be completed by LC-MS. The reaction solution was concentrated to dryness under reduced pressure, and the residue was purified by preparative HPLC (formic acid method 2) to obtain 4-(5S, 5aS, 6S, 9R)-12-(1-(3,3-difluoropyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z712, 0.18 mg, yield of two steps: 2.7%, formate). ES-API: [M+H]⁺= 656.1.

### Example 102: Synthesis of 4-((5S,5aS,6S,9R)-12-((1-((3,3-difluoroazetidin-1-yl)methyl) cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z713)

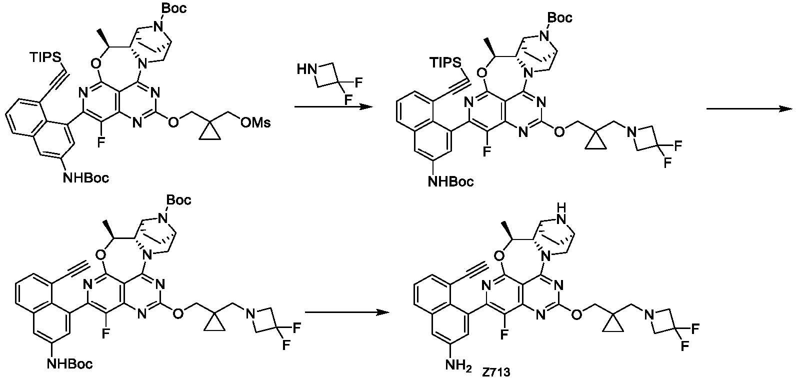

Step I: T-butyl (5S,5aS,6S,9R)-2-(3-((t-butoxycarbonyl)amino)-8-((triisopropylsilyl)ethynyl) naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (80 mg, 0.080 mmol) was dissolved in acetone (1 mL). 3,3-difluorotrimethyleneimine hydrochloride (74.37 mg, 0.719 mmol), potassium carbonate (44.10 mg, 0.320 mmol), and potassium iodide (53.05 mg, 0.320 mmol) were added, and reacted at 80°C under microwave 1 hr. After the reaction, the reaction solution was diluted with ethyl acetate (10 mL), washed with water (10 mL) and sodium chloride (10mL), dried over anhydrous sodium sulfate, and filtered. The crude product was purified by silica gel column chromatography (dichloromethane: methanol =15:1) to obtain t-butyl (5S,5aS,6S,9R)-2-(3-((t-butoxycarbonyl) amino)-8-((triisopropylsilyl)ethynyl)naphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (50 mg, 0.050 mmol, yield: 62.69%). ES-API: [M+H]⁺= 998.4.

Step II: Cesium fluoride (33.49 mg, 0.220 mmol) was added to t-butyl (5S,5aS,6S,9R)-2-(3-((t-butoxycarbonyl)amino)-8-((triisopropylsilyl)ethynyl)naphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (35 mg, 0.035 mmol) in N,N-dimethylformamide (1.0 mL), and the reaction mixture was stirred at room temperature for 2 hrs. The reaction was shown to be completed by LC/MS. The reaction solution was diluted with ethyl acetate (10 mL), washed with water (10 mLX2) and saturated brine (10 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (28 mg, 0.033 mmol, yield: 94.86%). ES-API: [M+H]⁺=842.3.

Step III: In an iced water bath, trifluoroacetic acid (1.0 mL) was slowly added to a solution of t-butyl (5S,5aS,6S,9R)-2-(3-((t-butoxycarbonyl)amino)-8-ethynylnaphthalen-1-yl)-12-((1-((3,3-difluoroazetidin-1-yl)methyl)cyclopropyl)methoxy)-1-fluoro-5-methyl-5a6,7,8,9,10-hexahydro-5H-4-oxa-3,10a,11,13,14-pentaaza-6,9-methanonaphtho[1,8-ab]hepten-14-carboxylate (25 mg, 0.030 mmol) in dichloromethane (1.0 mL). The reaction mixture was stirred at 0°C for 0.5 hr. The reaction was shown to be completed by LC/MS. The reaction solution was concentrated to dryness under reduced pressure, and the residue was purified by preparative HPLC (formic acid method 2) to obtain 4-((5S,5aS,6S,9R)-12-((1-((3,3-difluoroazetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z713, 1.7 mg, 0.002 mmol, yield: 20.81%, formate). ES-API: [M+H]⁺= 642.2.

### Example 103: Synthesis of 5-ethynyl-1-fluoro-4-((5S, 5aS, 6S, 9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z714)

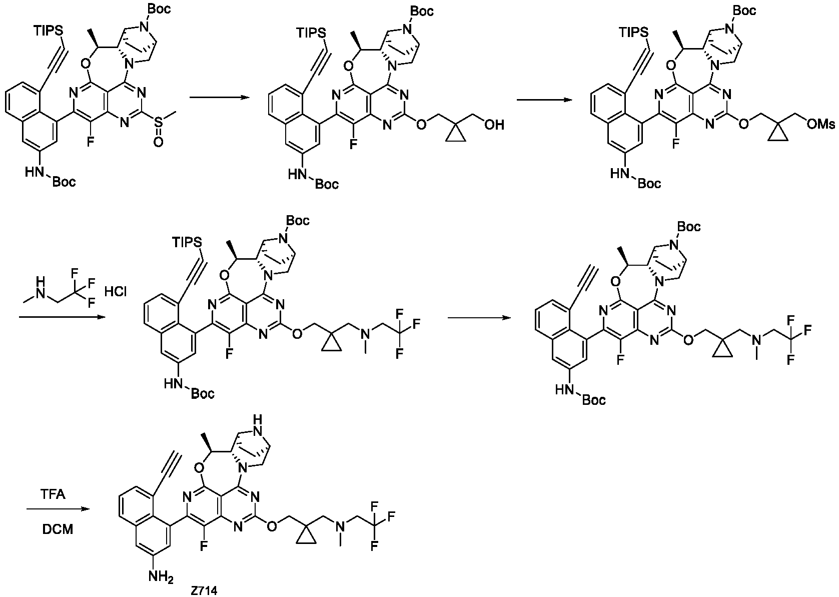

Step I: 1, 1-cyclopropandimethanol (220 mg, 0.25 mmol) was dissolved in anhydrous tetrahydrofuran(5 mL). In an iced water bath, sodium hydride (40 mg, 1.0 mmol) was added and then reacted for 30 min in the iced water bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (127 mg, 1.2 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX 2) and saturated brine (15 mLX3), dried over anhydrous sodium sulfate, filtered, and concentrated, to obtain a crude product. The crude product was purified by automated flash silica gel chromatography ((dichloromethane: methanol =15:1) mobile phase) to obtain t-butyl (5S, 5aS, 6S, 9R)-2-(3-((t-butoxycarbonyl) amino)-8-((triisopropylsilyl)ethynyl)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (180 mg, yield: 78%). ES-API: [M+H]⁺= 923.3.

Step II: T-butyl (5S,5aS,6S,9R)-2-(3-((t-butoxycarbonyl)amino)-8-((triisopropylsilyl)ethynyl) naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (180 mg, 0.19 mmol) was dissolved in anhydrous dichloromethane (5 mL). In an iced water bath, anhydrous N,N-diisopropylethyl amine (76 mg, 0.6 mmol) and methanesulfonic anhydride (102 mg, 0.6 mmol) were added, and the reaction mixture was stirred at room temperature for 2 hrs. The reaction was shown to be completed by LC/MS. The reaction solution was diluted with dichloromethane (20 mL), washed with saturated sodium bicarbonate (15 mLX 2) and saturated brine (15 mLX 3), dried over anhydrous sodium sulfate, filtered, and concentrated to obtain the crude product t-butyl (5S, 5aS, 6S, 9R)-2-(3-((t-butoxycarbonyl)amino)-8-((triisopropylsilyl)ethynyl)naphthalen-1-yl)-1-fluoro-5-methyl-12-((1-(((methylsulfonyl)oxy) methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (180 mg, yield: 92%). ES-API: [M+H]⁺= 1001.3.

Step III: T-butyl (5S, 5aS, 6S, 9R)-2-(3-((t-butoxycarbonyl)amino)-8-((triisopropylsilyl) ethynyl)naphthalen-1-yl)-1-fluoro-5-methyl-12-((1-(((methylsulfonyl)oxy)methyl)cyclopropyl) methoxy)-5a,6,7,8,9,10-hexahydro-5H-4-oxa-3,10a,11,13,14-pentaaza-6,9-methanonaphtho[1,8-ab]hepten-14-carboxylate (60 mg, 0.06 mmol) was dissolved in anhydrous N,N-dimethylformamide (3 mL). Potassium carbonate (83 mg, 0.6 mmol) and N-methyl-2,2,2-trifluoroethyl amine hydrochloride (89 mg, 0.6 mmol) were added, and the reaction mixture was stirred overnight at 50°C. The reaction solution was diluted with ethyl acetate (20 mL), washed with water (15 mLX2) and saturated brine (15 mLX3), dried over anhydrous sodium sulfate, filtered, and concentrated to obtain a crude product. The crude product was purified by preparative TLC (dichloromethane: methanol =30: 1) to obtain t-butyl (5S, 5aS, 6S, 9R)-2-(3-((t-butoxycarbonyl)amino)-8-((triisopropylsilyl)ethynyl)naphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (15 mg, yield: 24%). ES-API: [M+H]⁺= 1018.2.

Step IV: Cesium fluoride (46 mg, 0.30 mmol) was added to t-butyl (5S, 5aS, 6S, 9R)-2-(3-((t-butoxycarbonyl)amino)-8-((triisopropylsilyl)ethynyl)naphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (15 mg, 0.015 mmol) in N,N-dimethylformamide (2 mL), and the reaction mixture was stirred at room temperature for 1 hr. The reaction was shown to be completed by LC/MS. The reaction solution was diluted with ethyl acetate (20 mL), washed with water (15 mLX 2) and saturated brine (15 mLX 3), dried over anhydrous sodium sulfate, filtered, and concentrated to obtain t-butyl (5S, 5aS, 6S, 9R)-2-(3-((t-butoxycarbonyl)amino)-8-ethynylnaphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (13 mg,yield: 100%). ES-API: [M+H]⁺=862.3.

Step V: In an iced water bath, trifluoroacetic acid (2 mL) was slowly added to a solution of t-butyl (5S, 5aS, 6S, 9R)-2-((3-(t-butoxycarbonyl)amino)-8-ethynylnaphthalen-1-yl)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (13 mg, 0.015 mmol) in dichloromethane (1 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) to obtain 5-ethynyl-4-((5S, 5aS, 6S, 9R)-1-fluoro-5-methyl-12-((1-((methyl(2,2,2-trifluoroethyl)amino)methyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine(Z714, 0.7 mg, yield: 7% ). ES-API: [M+H]⁺= 662.2.

### Example 104: Synthesis of 5-ethynyl-1-fluoro-4-((5S, 5aS, 6S, 9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z715)

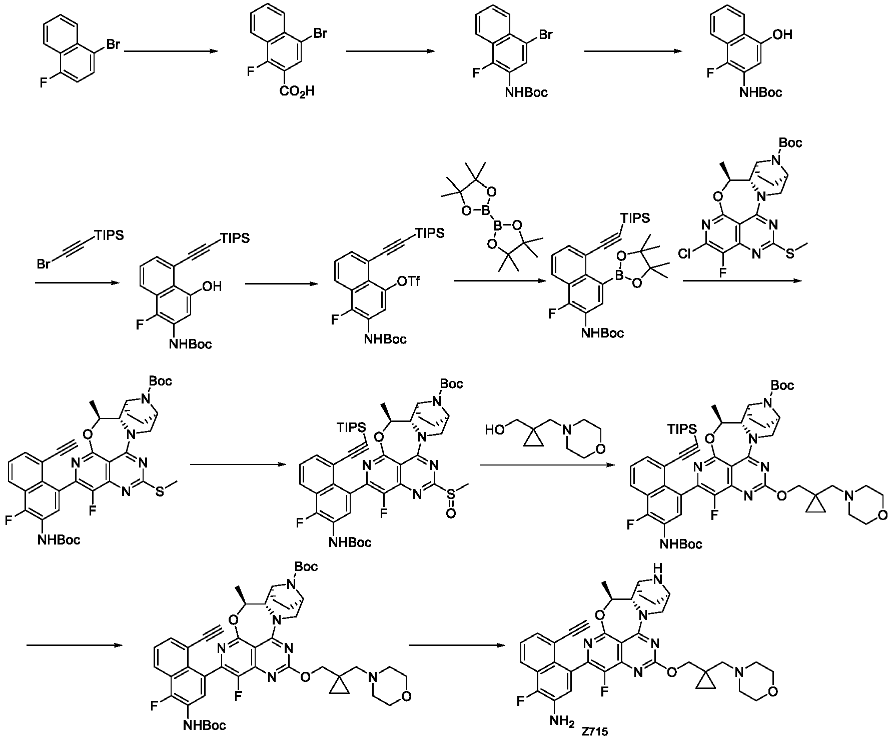

Step I: 2,2,6,6-tetramethylpiperidine (9.75 mL, 57.76 mmol) and tetrahydrofuran (70 mL) were added to a round-bottom flask. Under a nitrogen atmosphere, the reaction solution was cooled to -78°C, and a n-butyl lithium solution in n-hexane (23.10 mL, 57.76 mmol) was added. After that, the reaction was stirred -78°C for 30 hrs. A solution of 1-bromo-4-fluoronaphthalene (10 g, 44.433 mmol) in tetrahydrofuran (70 mL) was added dropwise to the reaction solution, and stirred at -78°C for 1 hr. Dry ice (solid carbon dioxide, 19.55 g, 444.326 mmol) was added batchwise to the reaction solution. The reaction was stirred at -78°C for 1 hr. The reaction solution was heated to 0°C, quenched with water, and adjusted to pH 5 with 6M hydrochloric acid. A large amount of white solid was precipitated. The solid was filtered, and the filter cake was washed with water. The filter cake was collected, and dried under vacuum to obtain 4-bromo-1-fluoro-2-naphthalenecarboxylic acid (9 g, yield: 75%).

Step II: 4-bromo-1-fluoro-2-naphthalenecarboxylic acid (7.5 g, 27.87 mmol), triethyl amine (7.75 mL, 55.75 mmol), t-butanol (60 mL) and toluene (10 mL) were added to a round-bottom flask. Diphenylphosphoryl azide (9 mL, 41.81 mmol) was added to the reaction solution. Under a nitrogen atmosphere, the reaction was stirred at 90°C for 4 hrs. The reaction solution was concentrated. The residue was purified by flash silica gel column chromatography (ethyl acetate /petroleum ether: 0-5%) to obtain t-butyl (4-bromo-1-fluoronaphthalen-2-yl)carbamate (7.3g, yield: 77%). ES-API: [M+H-56]⁺=284.0.

Step III: T-butyl (4-bromo-1-fluoronaphthalen-2-yl)carbamate (7.5 g, 22.05 mmol), potassium hydroxide (4.95 g, 88.19 mmol), tris (dibenzylideneacetone)palladium (1g,1.10 mmol), 2-di-t-butylphosphino-2',4',6'-triisopropylbiphenyl (1.4 g, 3.31 mmol), 1,4-dioxane (60 mL) and water (50 mL) were added to a round-bottom flask. The reaction solution was purged three times with nitrogen. The reaction solution was stirred under a nitrogen atmosphere in an oil bath at 100°C for 5 hrs. The reaction was quenched with water and extracted with ethyl acetate. The organic phase was washed with saturated brine, dried over anhydrous sodium sulfate, and filtered. The filtrate was concentrated. The crude product was purified by flash silica gel column chromatography (ethyl acetate /petroleum ether: 0-15%) to obtain t-butyl (1-fluoro-4-hydroxylnaphthalen-2-yl)carbamate (4.5 g, yield: 73%). ES-API: [M+H-56]⁺=222.1.

Step IV: T-butyl (1-fluoro-4-hydroxylnaphthalen-2-yl)carbamate (4.5 g, 16.22 mmol), (bromo alkynyl)triisopropylsilane (5.51 g, 21.09 mmol), potassium acetate (3.18 g, 32.45 mmol), dichlorobis(4-methylisopropylphenyl)ruthenium (II) (795 mg, 1.30 mmol) and 1, 4-dioxane (60 mL) were added to a round-bottom flask. The reaction solution was stirred under a nitrogen atmosphere in an oil bath at 100°C for 5 hrs. The reaction solution was filtered. The filtrate was concentrated. The crude product was purified by flash silica gel column chromatography (ethyl acetate/petroleum ether: 0-2%) to obtain t-butyl (1-fluoro-4-hydroxyl-5-((triisopropylsilyl) ethynyl)naphthalen-2-yl)carbamate (5 g, yield: 67%). ES-API: [M+H]⁺=458.2.

Step V: Under a nitrogen atmosphere, t-butyl (1-fluoro-4-hydroxyl-5-((triisopropylsilyl) ethynyl)naphthalen-2-yl)carbamate (5 g, 10.92 mmol), N,N-diisopropylethyl amine (4.76 mL, 27.31 mmol) and dichloromethane (60 mL) were added to a round-bottom flask. The reaction solution was cooled to -40°C, and trifluoromethansulfonic anhydride (2.76 mL, 16.39 mmol) and was slowly added dropwise. The reaction was stirred at -40°C for 1 hr. The reaction was quenched with saturated sodium bicarbonate, and extracted with dichloromethane. The organic phase was washed with saturated brine, dried over sodium sulfate, and filtered. The filtrate was concentrated. The crude product was purified by flash silica gel column chromatography (ethyl acetate /petroleum ether: 0-5%) to obtain 3-((t-butoxycarbonyl)amino)-4-fluoro-8-((triisopropylsilyl) ethynyl)naphthalen-1-yl trifluoromethansulfonate (5.5 g, yield: 85%). ES-API: [M+Na]⁺=612.0.

Step VI: 3-((t-butoxycarbonyl)amino)-4-fluoro-8-((triisopropylsilyl)ethynyl)naphthalen-1-yltrifluoromethansulfonate (1 g, 1.70 mmol), bis(pinacolato)diboron (861 mg, 3.39 mmol), [1,1'-bis[1,1'- bis (diphenylphosphino) ferrocene] palladium(OO) dichloride (99 mg, 0.13 mmol), potassium acetate (499 mg, 5.09 mmol) and 1,4-dioxane (12 mL) were added to a sealed tube. The reaction solution was purged with nitrogen for 2 min. The reaction was allowed to stand in a microwave reactor and stirred at 115°C for 4 hrs. The reaction was quenched with water. The reaction solution was extracted with ethyl acetate. The organic phase was washed with saturated brine, dried over sodium sulfate, and filtered. The filtrate was concentrated. The crude product was purified by flash silica gel column chromatography (ethyl acetate /petroleum ether: 0-3%) to obtain t-butyl (1-fluoro-4-(4,4,5,5-tetramethyl-1,3,2-dioxaborolan-2-yl)-5-((triisopropylsilyl)ethynyl) naphthalen-2-yl)carbamate (260 mg, yield: 27%). ES-API: [M+H]⁺=568.3.

Step VII: T-butyl (1-fluoro-4-(4,4,5,5-tetramethyl-1,3,2-dioxaborolan-2-yl)-5-((triisopropylsilyl)ethynyl)naphthalen-2-yl)carbamate (212 mg, 0.37 mmol), t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (120 mg, 0.25 mmol), [n-butyldi(1-adamantanyl)phosphine](2-amino-1,1'-biphenyl-2-yl)palladium (II) meslate (18 mg, 0.025 mmol), potassium phosphate (158 mg, 0.75 mmol), 1, 4-dioxane (4 mL) and water (0.8 mL) were added to a round-bottom flask. Under a nitrogen atmosphere, the system was heated in a microwave reactor at 75°C for 2 hrs. The reaction solution was added with water (30 mL), and extracted with ethyl acetate (30 mLX3). The organic phase was dried over anhydrous sodium sulfate, filtered and concentrated. The crude product was purified by flash silica gel column chromatography (ethyl acetate /petroleum ether: 0-30%) to obtain t-butyl (5S,5aS,6S,9R)-2-(3-((t-butoxycarbonyl)amino)-8-ethynyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (210 mg, yield: 95%). ES-API: [M+H]⁺=887.2.

Step VIII: T-butyl (5S,5aS,6S,9R)-2-(3-((t-butoxycarbonyl)amino)-8-ethynyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (210 mg, 0.23 mmol) and dichloromethane (6 mL) were added to a round-bottom flask. The reaction solution was cooled to 0°C, and m-chloroperoxybenzoic acid (53mg, 0.31 mmol) was added. The reaction was stirred at room temperature for 0.5 hr. The reaction solution was added with a saturated sodium thiosulfate aqueous solution (30 mL) and a saturated sodium bicarbonate aqueous solution, and extracted with ethyl acetate (30 mLX3). The organic phase was dried and concentrated to obtain t-butyl (5S,5aS,6S,9R)-2-(3-((t-butoxycarbonyl)amino)-4-fluoro-8-((triisopropylsilyl)ethynyl) naphthalen-1-yl)-1-fluoro-5-methyl-12-(methylthionyl)-5a,6,7,8,9,10-hexahydro-5H-4-oxo-3,10a,11,13,14-pentaaza-6,9-methanonaphtho[1,8-ab]hepten-14-carboxylate (180 mg, yield: 84%). ES-API: [M+H]⁺=903.2.

Step IX: Sodium hydride (32 mg, 0.80 mmol) and tetrahydrofuran (8 mL) were added to a round-bottom flask. The reaction solution was cooled to 0°C, and (1-(morpholinemethyl)cyclopropyl)methanol (68 mg, 0.40 mmol) dissolved in anhydrous tetrahydrofuran (1 mL) was added to the reaction solution, and reacted at room temperature for 5 min. T-butyl (5S, 5aS, 6S, 9R)-2-(3-((t-butoxycarbonyl)amino)-4-fluoro-8-((triisopropylsilyl) ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (180 mg, 0.20 mmol) was added to the reaction solution, and reacted at 0°C for 30 min. The reaction was shown to be completed by LC/MS. The reaction solution was added with a saturated ammonium chloride solution (30 mL), and extracted with ethyl acetate (20 mLX2). The organic phase was washed with saturated brine (30 mL), dried over anhydrous sodium sulfate, filtered, and concentrated. The crude product was purified by flash silica gel column chromatography ( dichloromethane/methanol: 0-10%) to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (60 mg, yield: 30%). ES-API: [M+H]⁺=1010.2.

Step X: T-butyl (5S, 5aS, 6S, 9R)-2-(3-((t-butoxycarbonyl)amino)-4-fluoro-8-((triisopropylsilyl)ethynyl)naphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (60 mg, 0.06 mmol), cesium fluoride (89 mg, 0.59 mmol), and N, N-dimethylformamide (3 mL) were added to a round-bottom flask. The reaction was stirred at room temperature for 1 hr. The reaction solution was added with water (30 mL), and extracted with ethyl acetate (30 mLX3). The organic phase was washed with saturated brine (30 mLX2), dried over anhydrous sodium sulfate, filtered, and concentrated to obtain t-butyl (5S, 5aS, 6S, 9R)-2-(3-((t-butoxycarbonyl)amino)-8-ethynyl-4-fluoronaphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (50mg, crude product). ES-API: [M+H]⁺=854.2.

Step XI: T-butyl (5S, 5aS, 6S, 9R)-2-(3-((t-butoxycarbonyl)amino)-8-ethynyl-4-fluoronaphthalen-1-yl)-1-fluoro-5-methyl-12-((1-(morpholinemethyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (50mg, crude product) was dissolved in ethyl acetate (1.5 mL). The solution was cooled to 0°C, and a 4M hydrogen chloride/dioxane solution (1.5 mL, 6 mmol) was added. The reaction was stirred at room temperature for 1 hr. The reaction solution was concentrated to dryness at 40°C, and the crude product was purified by preparative HPLC (ammonium bicarbonate method 2) to obtain 5-ethynyl-1-fluoro-4-((5S, 5aS, 6S, 9R)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z715, 12mg, yield: 31%). ES-API: [M+H]⁺=654.2.

### Example 105: Synthesis of 2-cyclopropyl-5-(5S,5aS,6S,9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)-3,6-dimethylaniline(Z716)

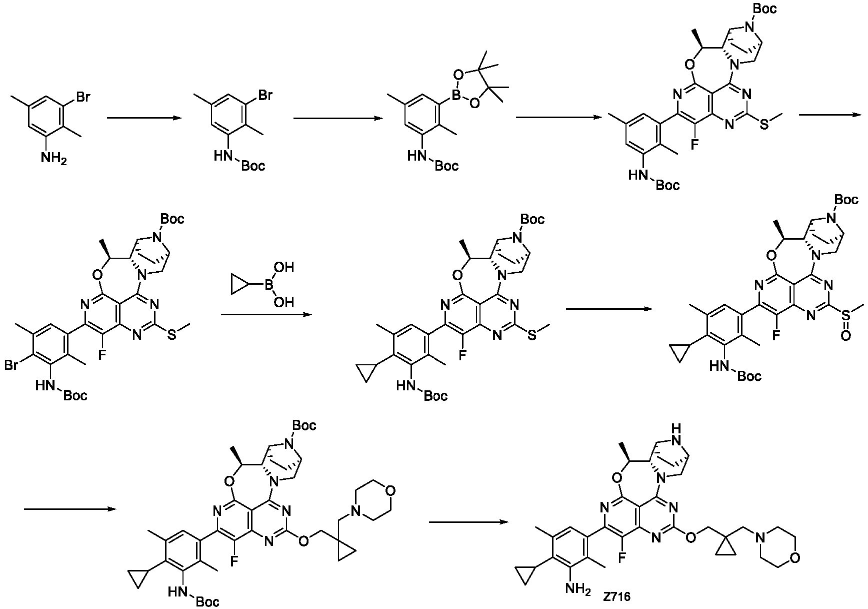

Step I: At room temperature, 3-bromo-2,5-dimethylaniline (1.0 g, 4.998 mmol) and di-t-butyl dicarbonate (1.42 g, 6.497 mmol) were sequentially added to a sodium hydroxide solution (2M, 20 mL), and reacted at 100°C for 1 hr. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. A crude product was obtained, which was purified by silica gel column chromatography (0-50% ethyl acetate /petroleum ether) to obtain t-butyl (3-bromo-2,5-dimethylphenyl)carbamate (1.40 g, yield: 93.31%). ES-API: [M-55+H]⁺=245.9.

Step II: T-butyl (3-bromo-2,5-dimethylphenyl)carbamate (0.9 g, 2.998 mmol), bis(pinacolato) diboron (1.52 g, 5.996 mmol), potassium acetate (1.173 g, 11.99 mmol), and [1,1'-bis(diphenylphosphino)ferrocene]palladium dichloride (0.323 g, 0.45 mmol) were added to N,N-dimethylformamide (30 mL), purged 4 time with nitrogen, and reacted at 80°C for 2 hrs. After the reaction, the reaction solution was extracted with ethyl acetate (200 mLX2) and saturated brine (80 mL). The ethyl acetate phases were combined, dried over anhydrous sodium sulfate, and filtered. The filtrate was concentrated under reduced pressure. A crude product was obtained, which was purified by silica gel column chromatography (0-70% ethyl acetate /petroleum ether) to obtain t-butyl (2,5-dimethyl-3-(4,4,5,5-tetramethyl-1,3,2-dioxaborolane -2-yl)phenyl) carbamate (490 mg, yield: 47%). ES-API: [M-55+H]⁺=292.2.

Step III: Potassium phosphate (0.616 g, 2.90 mmol) and [n-butyldi(1-adamantanyl)phosphine](2-amino-1,1'-biphenyl-2-yl)palladium (II) mesylate (79.3 mg, 0.109 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab]hepten-14-carboxylate (350 mg, 0.726 mmol) and t-butyl (2,5-dimethyl-3-(4,4,5,5-tetramethyl-1,3,2-dioxaborolan-2-yl)phenyl)carbamate (0.490 g, 1.411 mmol) in tetrahydrofuran (30 mL) and water (3 mL). Nitrogen was bubbled therethrough and reacted in an oil bath at 80°C for 3 hrs. After the reaction, the reaction solution was extracted with ethyl acetate (30 mLX2) and saturated brine (30 mL). The ethyl acetate phases were combined, dried over anhydrous sodium sulfate, and filtered. The filtrate was concentrated under reduced pressure. A crude product was obtained, which was purified by silica gel column chromatography (0-4% methanol /dichloromethane) to obtain t-butyl (5S,5aS,6S,9R)-2-(3-((t-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (570 mg, crude product) as a yellow solid. ES-API: [M+H]⁺=667.2.

Step IV: At 0°C, t-butyl (5S,5aS,6S,9R)-2-(3-((t-butoxycarbonyl)amino)-2,5-dimethylphenyl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (483.0 mg, 0.726 mmol) and then N-bromosuccinimide (168.0 mg, 0.944 mmol) were added to dichloromethane (20 mL), and reacted with stirring at this temperature for 0.5 hr. After the reaction, the reaction solution was extracted with ethyl acetate (100 mLX2) and saturated brine (100 mL). The ethyl acetate phases were combined, dried over anhydrous sodium sulfate, and filtered. The filtrate was concentrated under reduced pressure. A crude product was obtained, which was purified by silica gel column chromatography (0-70% ethyl acetate /petroleum ether) to obtain t-butyl (5S,5aS,6S,9R)-2-(4-bromo-3-((t-butoxycarbonyl)amino)-2,5-dimethylphenyl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (400 mg, yield: 74.0%). ES-API: [M+H]⁺=745.1.

Step V: Potassium phosphate (455.4 mg, 2.146 mmol), 2-bicyclohexylphosphino-2',6'-dimethoxybiphenyl (66.06 mg, 0.161 mmol), and palladium acetate (24.04 mg, 0.107 mmol) were added to t-butyl (5S,5aS,6S,9R)-2-(4-bromo-3-((t-butoxycarbonyl)amino)-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (400 mg, 0.536 mmol) and cyclopropylboronic acid (230.0 mg, 2.68 mmol) in toluene (50 mL) and water (5 mL). Nitrogen was bubbled therethrough and the reaction was continued in an oil bath at 105°C for 2 hrs. After the reaction, the reaction solution was extracted with ethyl acetate (200 mLX2) and saturated brine (100 mLX4). The ethyl acetate phases were combined, dried over anhydrous sodium sulfate, and filtered. The filtrate was concentrated under reduced pressure. The crude product was purified by silica gel column chromatography (0-70% ethyl acetate /petroleum ether) to obtain t-butyl (5S,5aS,6S,9R)-2-(3-((t-butoxycarbonyl)amino)-4-cyclopropyl-2,5-dimethylphenyl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (520 mg, crude product) as a yellow solid. ES-API: [M+H]⁺=707.2.

Step VI: At 0°C, m-chloroperoxybenzoic acid (188.3 mg, 1.09 mmol) was added to a solution of t-butyl (5S,5aS,6S,9R)-2-(3-((t-butoxycarbonyl)amino)-4-cyclopropyl-2,5-dimethylphenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (379.0 mg, 0.537 mmol) in dichloromethane (30.0 mL). The solution was stirred for 1 hour in an ice bath. The reaction was quenched with saturated sodium sulfite and extracted with dichloromethane (100 mLX2). The organic phase was dried over anhydrous sodium sulfate, filtered and concentrated to obtain crude product. The crude product was purified by silica gel column chromatography (0-10% methanol /dichloromethane) to obtain t-butyl (5S,5aS,6S,9R)-2-(3-((t-butoxycarbonyl)amino)-4-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (390 mg, crude product) as a white solid. ES-API:[M+H]⁺=723.3.

Step VII: At 0°C, sodium hydride (70.0 mg, 1.754 mmol) was added to a solution of (1-(morpholinemethyl)cyclopropyl)methanol (100 mg, 0.5850 mmol) in tetrahydrofuran (10.0 mL), and stirred at this temperature for 10 min. Then a solution of t-butyl (5S,5aS,6S,9R)-2-(3-((t-butoxycarbonyl)amino)-4-cyclopropyl-2,5-dimethylphenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (390 mg, crude product) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(3-((t-butoxycarbonyl)amino)-4-cyclopropyl-2,5-dimethylphenyl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (150 mg, crude product) as a yellow solid. ES-API: [M+H]⁺=830.3.

Step VIII: In an iced water bath, t-butyl (5S,5aS,6S,9R)-2-(3-((t-butoxycarbonyl)amino)-4-cyclopropyl-2,5-dimethylphenyl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (150 mg, crude product) was slowly added to dichloromethane (10.0 mL), and finally hydrochloric acid-dioxane solution (5.0 mL, 4M, 20.0 mmol) was added. The reaction mixture was stirred at room temperature for 1.0 hrs. The reaction was shown to be completed by LC/MS. The reaction solution was concentrated to dryness under reduced pressure, and the residue was purified by preparative HPLC (ammonium bicarbonate method 2) to obtain 2-cyclopropyl-5-(5S,5aS,6S,9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)-3,6-dimethylaniline (Z716, 3.0mg, yield: 0.8%) as a white solid. ES-API: [M+H]⁺= 630.4.

### Example 106: Synthesis of 5,6-difluoro-4-((5S, 5aS, 6S, 9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]heptan-2-yl)naphthalen-2-amine (Z719)

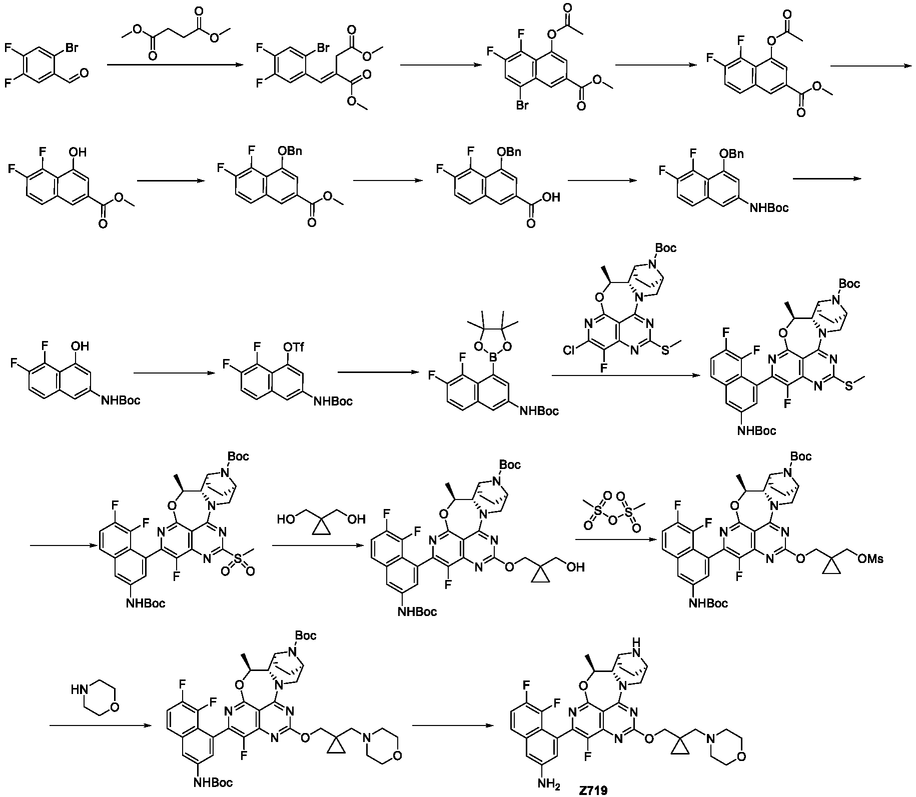

Step I: Under a nitrogen atmosphere, dimethyl succinate (7.04 g, 48.19 mmol) and a 30% sodium methoxide solution in methanol (4.5 mL, 22.62 mmol) were dissolved in toluene (5 mL). The reaction mixture was heated to 75°C. A solution of 2-bromo-4,5-difluorobenzaldehyde (5.0 g, 22.62 mmol) in toluene (10 mL) was slowly added dropwise to the reaction solution. After that, the reaction solution was heated to 85^{°C} and stirred for 5 hrs. The reaction was shown to be completed by LC/MS. The reaction solution was cooled to room temperature, added to methyl t-butyl ether (100 mL), and stirred for 5 min. The reaction mixture was poured into water (100 mL). The aqueous phase was separated, the organic phase was washed with water (30 mL X 2), and the aqueous phases were combined. The aqueous phase was adjusted to an acidic pH with 2 M dilute hydrochloric acid, and then extracted with methyl t-butyl ether (30 mLX 3). The organic phases were combined, washed with water (30 mL) and saturated brine (30 mL), dried over anhydrous sodium sulfate, filtered, and concentrated under reduced pressure to obtain the crude product 2-(2-bromo-4,5-difluorobenzylene)dimethyl succinate (6.591 g, yield: 83%). ES-API: [M- 2H]⁺= 346.0.

Step II: 2-(2-bromo-4,5-difluorobenzylene)dimethyl succinate (6.591 g, 18.878 mmol) was dissolved in acetic anhydride (15 mL). The reaction solution was added with anhydrous sodium acetate (5.661 g, 69.011 mmol), heated to 140^{°C} and stirred overnight. The reaction was shown to be completed by LC/MS. The reaction solution was cooled to room temperature, added with toluene (30 mL), and concentrated under reduced pressure to remove the solvent. The residue was added with dilute hydrochloric acid (30 mL), and extracted with ethyl acetate (30 mL X 3). The organic phases were combined, washed with water (30 mL) and saturated brine (30 mL), dried over anhydrous sodium sulfate, filtered, and concentrated under reduced pressure. The residue was purified by silica gel column chromatography(ethyl acetate: petroleum ether= 0 - 4%) to obtain methyl 4-acetoxy-8-bromo-5,6-difluoro-2-naphthalenecarboxylate (1.2 g, yield: 18%) as a white solid. ES-API: [M+H]⁺= 359.0.

Step III: Methyl 4-acetoxy-8-bromo-5,6-difluoro-2-naphthalenecarboxylate (1.2 g, 3.342 mmol) and triethyl amine (406 mg, 4.010 mmol) were dissolved in ethyl acetate (36 mL). The reaction solution was added with 10% palladium on carbon (356 mg), and stirred under a hydrogen atmosphere at room temperature for 1 hr. The reaction was shown to be completed by LC/MS. The reaction solution was filterd through diatomite, and the filtrate was concentrated under reduced pressure, to obtain the crude product methyl 4-acetoxy-5,6-difluoro-2-naphthalenecarboxylate (936 mg, yield: 99%). ES-API: [M+ H]⁺= 281.1.

Step IV: Methyl 4-acetoxy-5,6-difluoro-2-naphthalenecarboxylate (936 mg, 3.340 mmol) was dissolved in methanol (20 mL). The reaction solution was added with sodium methoxide (271 mg, 5.010 mmol), and stirred at room temperature for 1 hr. The reaction was shown to be completed by LC/MS. The reaction solution was adjusted to pH 2.0 with 1M dilute hydrochloric acid, and a large amount of solid was precipitated. After filtration, the filter cake was dissolved in ethyl acetate (20 mL), dried over anhydrous sodium sulfate, filtered, and concentrated under reduced pressure, to obtain the crude product methyl 5,6-difluoro-4-hydroxyl-2-naphthalenecarboxylate (748 mg, yield: 94%). ES-API: [M+ H]⁺= 239.0.

Step V: Methyl 5,6-difluoro-4-hydroxyl-2-naphthalenecarboxylate (748 mg, 3.140 mmol), potassium carbonate (868 mg, 6.281 mmol) and benzyl bromide (806 mg, 4.711 mmol) were dissolved in acetone (20 mL), and the reaction solution was heated to reflux for 2 hrs. The reaction was shown to be completed by LC/MS. The reaction solution was cooled to room temperature. After filtration, the reaction solution was concentrated under reduced pressure and dried to obtain methyl 4-(benzyloxy)-5,6-difluoro-2-naphthalenecarboxylate (1.031 g) as an off-white solid. ES-API: [M+ H]⁺= 329.1.

Step VI: Methyl 4-(benzyloxy)-5,6-difluoro-2-naphthalenecarboxylate (1031 mg, 3.140 mmol) was dissolved in toluene (12 mL). The reaction solution was added with a sodium hydroxide aqueous solution (26 mL, 12 M), heated to 105°C and stirred for 16 hrs. The reaction was shown to be completed by LC/MS. The reaction solution was concentrated under reduced pressure to remove most of the solvent. The residue was dissolved in ethyl acetate (100 mL), and adjusted to about pH 2.0 with 6M dilute hydrochloric acid. The organic phase was separated, dried over anhydrous sodium sulfate, filtered, and concentrated under reduced pressure, to obtain the crude product 4-(benzyloxy)-5,6-difluoro-2-naphthalenecarboxylic acid (1081 mg) as an off-white solid. ES-API: [M+ H]⁺= 315.3.

Step VII: 4-(benzyloxy)-5,6-difluoro-2-naphthalenecarboxylic acid (981 mg, 3.121 mmol), diphenylphosphoryl azide (1.0 mL, 4.682 mmol) and triethyl amine (2.0 mL, 14.046 mmol) were dissolved in toluene (15 mL) and t-butanol (15 mL). The reaction solution was heated to 90°C and stirred overnight. The reaction solution was cooled to room temperature, and concentrated under reduced pressure. The crude product was purified by silica gel column chromatography (petroleum ether/ ethyl acetate = 0 - 3%) to obtain t-butyl (4-(benzyloxy)-5,6-difluoronaphthalen-2-yl)carbamate (350 mg, yield: 26%) as an off-white solid. ES-API: [M-55]⁺= 330.1.

Step VIII: T-butyl (4-(benzyloxy)-5,6-difluoronaphthalen-2-yl)carbamate (350 mg, 0.908 mmol) was dissolved in methanol (10 mL) and tetrahydrofuran (1 mL). The reaction solution was added with 10% palladium on carbon (97 mg), and stirred under a hydrogen atmosphere at room temperature for 3 hrs. The reaction was shown to be completed by LC/MS. The reaction solution was filtered through diatomite, and the filtrate was concentrated under reduced pressure, to obtain the crude product t-butyl (5,6-difluoro-4-hydroxylnaphthalen-2-yl)carbamate (268 mg). ES-API: [M-55]⁺= 240.1.

Step IX: At -40°C, t-butyl (5,6-difluoro-4-hydroxylnaphthalen-2-yl)carbamate (250 mg, 0.847 mmol) was dissolved in dichloromethane (15 mL). The reaction solution was sequentially added with N,N-diisopropylethyl amine (0.7 mL, 5.080 mmol) and trifluoromethanesulfonic anhydride (358 mg, 1.270 mmol), and stirred at - 40°C for 0.5 hr. The reaction was shown to be completed by LC/MS. The reaction solution was added with a saturated sodium bicarbonate solution (30 mL) to quench the reaction. The reaction solution was extracted with dichloromethane (20 mLX3). The organic phases were combined, and concentrated under reduced pressure. The crude product was purified by silica gel column chromatography (petroleum ether/ ethyl acetate = 0 -6%) to obtain 3-((t-butoxycarbonyl)amino)-7,8-difluoronaphthalen-1-yl trifluoromethansulfonate (269 mg, yield: 74%). ES-API: [M-55]⁺= 372.0.

Step X: Under a nitrogen atmosphere, 3-((t-butoxycarbonyl)amino)-7,8-difluoronaphthalen-1-yl trifluoromethansulfonate (269 mg, 0.629 mmol), bis(pinacolato)diboron (400 mg, 1.574 mmol), potassium acetate (185 mg, 1.888 mmol) and 1,1'-bis(diphenylphosphino)ferrocene]palladium dichloride (92 mg, 0.126 mmol) were dissolved in 1,4-dioxane (15 mL). The reaction solution was heated to 105°C and stirred overnight. The reaction was shown to be completed by LC/MS. The reaction solution was cooled to room temperature, and concentrated under reduced pressure. The crude product was purified by silica gel column chromatography (petroleum ether/ ethyl acetate = 0 - 2%) to obtain t-butyl (5,6-difluoro-4-(4,4,5,5-tetramethyl-1,3,2-dioxaborolan-2-yl)naphthalen-2-yl)carbamate (307 mg) as an off-white solid. ES-API: [M-55]⁺= 350.1.

Step XI: Under a nitrogen atmosphere, t-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (90 mg, 0.187 mmol), (5,6-difluoro-4-(4,4,5,5-tetramethyl-1,3,2-dioxaborolan-2-yl)naphthalen-2-yl)carbamate (83 mg, 0.205 mmol), potassium phosphate (119 mg, 0.560 mmol) and [n-butyldi(1-adamantanyl)phosphine](2-amino-1,1'-biphenyl-2-yl)palladium (II) mesylate (20 mg, 0.028 mmol) were dissolved in tetrahydrofuran (4 mL) and water (0.8 mL). The reaction mixture was stirred 70°C under microwave for 40 min. The reaction was shown to be completed by LC/MS. The reaction solution was dried over anhydrous sodium sulfate, filtered and concentrated under reduced pressure. The crude product was purified by silica gel column chromatography (petroleum ether/ ethyl acetate = 0 - 30%) to obtain t-butyl (5S,5aS,6S,9R)-2-(3-((t-butoxycarbonyl)amino)-7,8-difluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (150 mg). ES-API: [M+H]⁺= 725.2.

Step XII: In an iced water bath, t-butyl (5S,5aS,6S,9R)-2-(3-((t-butoxycarbonyl)amino)-7,8-difluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (150 mg, 0.207 mmol) was dissolved in anhydrous dichloromethane (10 mL). m-chloroperoxybenzoic acid (55 mg, 0.269 mmol) was added to the solution, and the reaction solution was stirred at 0°C for 1 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S, 5aS, 6S, 9R)-2-(3-((t-butoxycarbonyl)amino)-7,8-difluoronaphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (153 mg). ES-API: [M+ H]⁺= 741.1.

Step XIII: cyclopropane-1,1-diyldimethanol (105 mg, 1.026 mmol) was dissolved in anhydrous tetrahydrofuran (6 mL). In an iced water bath, sodium hydride (21 mg, 0.515 mmol) was added, and reacted for 10 min in the iced water bath. A solution of t-butyl (5S, 5aS, 6S, 9R)-2-(3-((t-butoxycarbonyl)amino)-7,8-difluoronaphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (76 mg, 0.103 mmol) in tetrahydrofuran(4 mL) was added dropwise to the reaction solution, and reacted for 30 min at 0 °C. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure. The crude product was purified by TLC on silica gel (dichloromethane/ methanol = 30: 1) to obtain t-butyl (5S,5aS,6S,9R)-2-(3-((t-butoxycarbonyl)amino)-7,8-difluoronaphthalen-1-yl)-1-fluoro-12-((1-(hydroxymethyl) cyclopropyl)methoxy)-5-methyl-5a,6,7,8,9,10-hexahydro-5H-4-oxa-3,10a,11,13,14-pentaaza-6,9-methanonaphtho[1,8-ab]hept-14-carboxylate (46 mg, yield: 58%). ES-API: [M+ H]⁺= 779.2.

Step XIV: In an iced water bath, t-butyl (5S,5aS,6S,9R)-2-(3-((t-butoxycarbonyl)amino)-7,8-difluoronaphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hept-14-carboxylate (46 mg, 0.059 mmol) was dissolved in anhydrous dichloromethane (5 mL). The reaction solution was sequentially added with N,N-diisopropylethyl amine (46 mg, 0.354 mmol) and methanesulfonic anhydride (174 mg, 0.354 mmol), and stirred at 0°C for 1 hr. The reaction was shown to be completed by LC/MS. The reaction solution was diluted with dichloromethane (15 mL), quenched with a saturated sodium bicarbonate solution (30 mL), and then extracted with dichloromethane (15 mLX3). The organic phase was dried over anhydrous sodium sulfate, filtered, and concentrated to obtain the crude product t-butyl (5S,5aS,6S,9R)-2-(3-((t-butoxycarbonyl)amino)-7,8-difluoronaphthalen-1-yl)-1-fluoro-5-methyl-12-((1-((methylsulfonyl) oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (55 mg). ES-API: [M+ H]⁺= 857.0.

Step XV: T-butyl (5S,5aS,6S,9R)-2-(3-((t-butoxycarbonyl)amino)-7,8-difluoronaphthalen-1-yl)-1-fluoro-5-methyl-12-((1-((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (45 mg, 0.053 mmol), morpholine (23 mg, 0.263 mmol), potassium carbonate (36 mg, 0.263 mmol) and potassium iodide (8.7 mg, 0.053 mmol) were dissolved in acetone (3 mL). The reaction mixture was heated to 80°C and stirred for 1 hr in a sealed tube. The reaction was shown to be completed by LC/MS. The reaction solution was added with water (10 mL) and ethyl acetate (10 mL). The organic phase was separated, washed with saturated brine (10 mLX2), dried over anhydrous sodium sulfate, filtered, and concentrated to obtain the crude product t-butyl (5S, 5aS, 6S, 9R)-2-(3-((t-butoxycarbonyl)amino)-7,8-difluoronaphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (44 mg). ES-API: [M+H]⁺= 848.2.

Step XVI: In an iced water bath, a 4 M hydrochloric acid-dioxane solution (5 mL) was slowly added to a solution of t-butyl (5S,5aS,6S,9R)-2-(3-((t-butoxycarbonyl)amino)-7,8-difluoronaphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (44 mg, 0.052 mmol) in methanol (2 mL). The reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 5,6-difluoro-4-((5S, 5aS, 6S, 9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]heptan-2-yl)naphthalen-2-amine (Z719, 4.13 mg, yield: 10%) as an off-white solid. ES-API: [M+H]⁺= 648.2.

### Example 107: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5,6-difluoronaphthalen-2-amine (Z720)

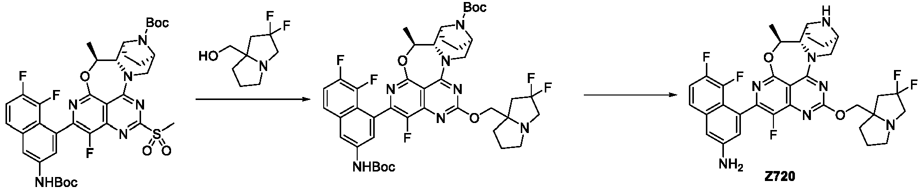

Step I: (2,2-difluorotetrahydro-1H-pyrrolizin-7a(5H)-yl)methanol (36 mg, 0.205 mmol) was dissolved in anhydrous tetrahydrofuran (5 mL). In an iced water bath, sodium hydride (12 mg, 0.308 mmol) was added, and reacted for 10 min in the iced water bath. A solution of t-butyl (5S,5aS,6S,9R)-2-(3-((t-butoxycarbonyl)amino)-7,8-difluoronaphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (70 mg, 0.103 mmol) in tetrahydrofuran (5 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX 2) and saturated brine (10 mLX 3), dried over anhydrous sodium sulfate, filtered, and concentrated under reduced pressure, to obtain the crude product t-butyl (5S,5aS,6S,9R)-2-(3-((t-butoxycarbonyl)amino)-7,8-difluoronaphthalen-1-yl)-12-((2,2-difluorotetrahydro- 1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (87 mg). ES-API: [M+ H]⁺= 854.2.

Step II: In an iced water bath, a 4 M hydrochloric acid-dioxane solution (5 mL) was slowly added to a solution of t-butyl (5S,5aS,6S,9R)-2-(3-((t-butoxycarbonyl)amino)-7,8-difluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (87 mg, 0.102 mmol) in methanol (1 mL). The reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5,6-difluoronaphthalen-2-amine (Z720, 16.83 mg, yield: 25%) as an off-white solid. ES-API: [M+H]⁺= 654.2.

### Example 108: Synthesis of 1-((1-(((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptan-12-yl)oxy)methyl)cyclopropyl)methyl)pyrrolidin-3-ol (Z721)

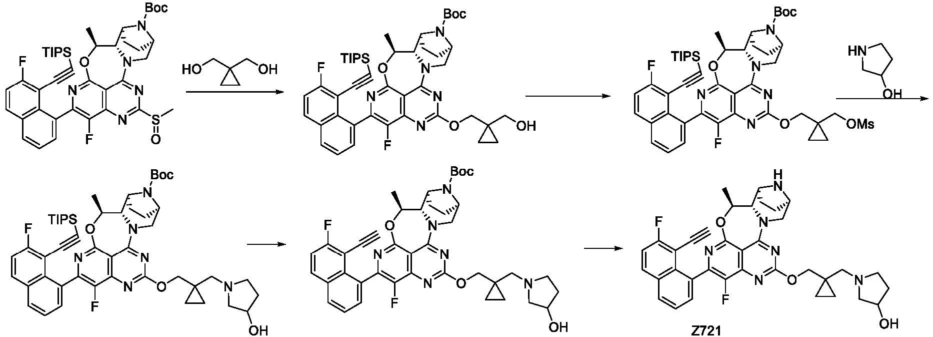

Step I: 1,1-cyclopropandimethanol (58 mg, 0.57 mmol) was dissolved in anhydrous tetrahydrofuran(2 mL). In an iced water bath, 60% sodium hydride (38 mg, 0.95 mmol) was added, and then reacted for 30 min in the iced water bath. A solution of t-butyl (5S,5aS,6S,9R)-1-fluoro-2-(7-fluoro-8-((triisopropylsilyl)ethynyl)naphthalen-1-yl)-5-methyl-12-(methylthionyl)-5a,6,7,8,9,10-hexahydro-5H-4-oxy-3,10a,11,13,14-pentaaza-6,9-methanonaphtho[1,8-ab]hepten-14-carboxylate (150 mg, 0.19 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX2) and saturated brine (15 mLX3), dried over anhydrous sodium sulfate, filtered, and concentrated. A crude product was obtained, which was purified by silica gel column chromatography (ethyl acetate: petroleum ether=0-80%) to obtain t-butyl (5S,5aS,6S,9R)-1-fluoro-2-(7-fluoro-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (90 mg, yield: 57.3%) as a white solid. ES-API: [M+H]⁺= 826.3.

Step II: T-butyl (5S,5aS,6S,9R)-1-fluoro-2-(7-fluoro-8-((triisopropylsilyl)ethynyl)naphthalen-1-yl)-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (59 mg, 0.071 mmol) was dissolved in anhydrous dichloromethane (5 mL). In an iced water bath, triethyl amine (36 mg, 0.36 mmol) and methanesulfonic anhydride (37 mg, 0.21 mmol) were added, and the reaction mixture was stirred at room temperature for 2 hrs. The reaction was shown to be completed by LC/MS. The reaction solution was diluted with dichloromethane (20 mL), washed with saturated sodium bicarbonate (15 mLX2) and saturated brine (15 mLX3), dried over anhydrous sodium sulfate, filtered, and concentrated. A crude product was obtained, which was purified by silica gel column chromatography (ethyl acetate: petroleum ether=0-60%) to obtain t-butyl (5S,5aS,6S,9R)-1-fluoro-2-(7-fluoro-8-((triisopropylsilyl)ethynyl)naphthalen-1-yl)-12-((1-((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (57 mg, yield: 88.3%) as a colorless liquid. ES-API: [M+H]⁺= 904.1.

Step III: T-butyl (5S,5aS,6S,9R)-1-fluoro-2-(7-fluoro-8-((triisopropylsilyl)ethynyl) naphthalen-1-yl)-12-((1-((methylsulfonyl)oxy)methyl)cyclopropyl)methoxy)-5a,6,7,8,9,10-hexahydro-5H-4-oxo-3,10a,11,13,14-pentaaza-6,9-methanonaphtho[1,8-ab]hepten-14-carboxylate (57 mg, 0.063 mmol) was dissolved in acetone (2 mL). Potassium carbonate(26 mg, 0.19 mmol), sodium iodide (10 mg, 0.063 mmol) and pyrrolidin-3-ol (17 mg, 0.19 mmol) were added, and the reaction mixture was stirred at 80°C in a microwave generator for 40 min. The reaction solution was concentrated, and a crude product was obtained, which was purified by silica gel column chromatography (ethyl acetate: petroleum ether=0-100%) to obtain t-butyl (5S,5aS,6S,9R)-1-fluoro-2-(7-fluoro-8-((triisopropylsilyl)ethynyl)naphthalen-1-yl)-12-((1-((3-hydroxylpyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (55 mg, yield: 97.5%) as a yellow solid. ES-API: [M+H]⁺= 895.4.

Step IV: Cesium fluoride (93 mg, 0.61 mmol) was added to t-butyl (5S,5aS,6S,9R)-1-fluoro-2-(7-fluoro-8-((triisopropylsilyl)ethynyl)naphthalen-1-yl)-12-((1-((3-hydroxylpyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (55 mg, 0.0161 mmol) in N,N-dimethylformamide (1 mL), and the reaction mixture was stirred at room temperature for 1 hr. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S,5aS,6S,9R)-2-(8-ethynyl-7-fluoronaphthalen-1-yl)-1-fluoro-12-((1-((3-hydroxylpyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (45 mg, yield: 99.1%). ES-API: [M+H]⁺=739.2.

Step V: T-butyl (5S,5aS,6S,9R)-2-(8-ethynyl-7-fluoronaphthalen-1-yl)-1-fluoro-12-((1-((3-hydroxylpyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (45 mg, 0.061 mmol) was dissolved in ethyl acetate (1 mL). At 0°C, 4M hydrogen chloride in 1, 4-dioxane (2 mL) was added, and the reaction was stirred at room temperature for 1 hr. The reaction solution was concentrated under reduce pressure. The crude product was purified by preparative HPLC (formic acid method 1) to obtain the target product 1-((1-(((5S,5aS,6S,9R)-2-(8-ethynyl-7-fluoronaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptan-12-yl)oxy)methyl)cyclopropyl)methyl)pyrrolidin-3-ol (Z721, 5 mg, yield: 12.8%, formate) as a white solid. ES-API: [M+H]⁺= 639.2.

### Example 109: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-6-fluoronaphthalen-2-amine (Z722)

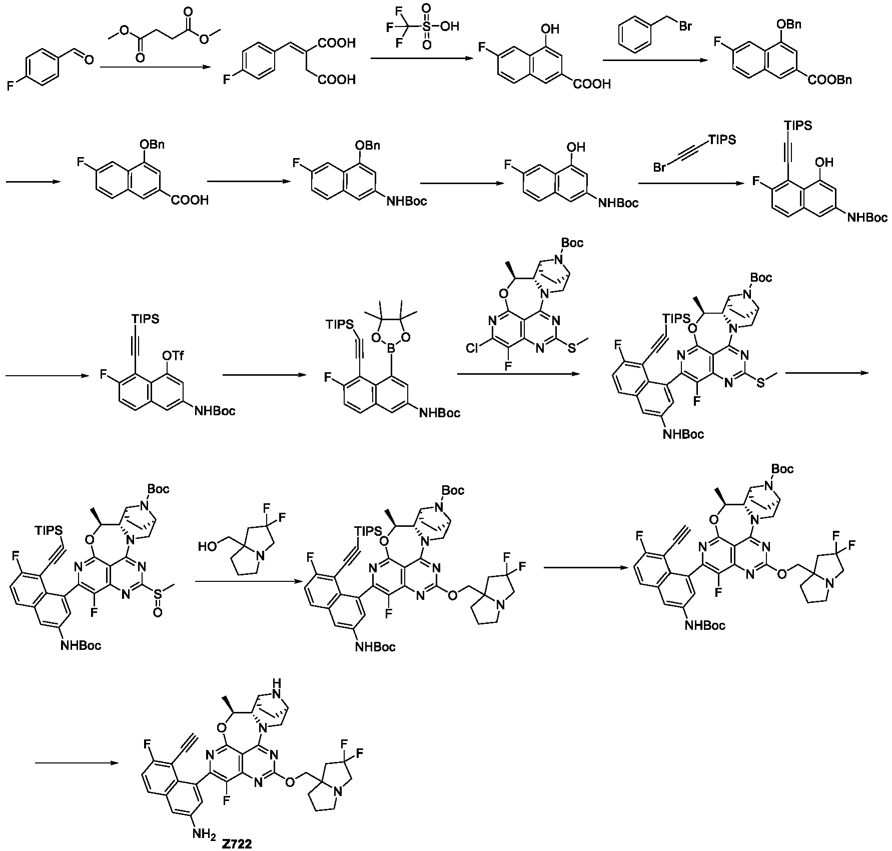

Step I: Potassium tert-butoxide (14.92 g, 0.133 mol) was dissolved in t-butanol (120 mL), and the reaction mixture was heated to reflux. Dimethyl succinate (21.15 g, 0.145 mol) and 4-fluorobenzaldehyde (15 g, 0.121 mol) in t-butanol (30 mL) was slowly added dropwise to the reaction solution, the reaction solution was stirred overnight under reflux. The reaction was shown to be completed by LC/MS. The reaction solution was cooled to room temperature, and concentrated under reduced pressure to remove the solvent. The residue was added to 150 mL dilute hydrochloric acid (1 M), and extracted with ethyl acetate (50 mLX3). The organic phases were combined, dried, filtered, and concentrated to obtain an oil. The oil was dissolved in methanol (150 mL), sodium hydroxide solution (150 mL, 2M) was added, and the reaction mixture was heated to reflux overnight. The reaction solution was concentrated under reduced pressure. The crude product was dissolved in water (200 mL), and extracted with ethyl acetate (100 mLX2). The aqueous phase was adjusted to about pH 2.0 with concentrated hydrochloric acid, extracted with ethyl acetate (100 mL X 3), dried over anhydrous sodium sulfate, filtered, and concentrated under reduced pressure to obtain a crude product. The crude product was slurried in petroleum ether to obtain 2-(4-fluorobenzylene)succinic acid (16.517 g, yield: 61%) as a white solid. ES-API: [M+ H]⁺= 225.1.

Step II: 2-(4-fluorobenzylene)succinic acid (10 g, 44.605 mmol) was dissolved in trifluoromethanesulfonic acid (60 mL), and stirred overnight at room temperature. The reaction was shown to be completed by LC/MS. The reaction solution was slowly added to iced water (200 mL), and a large amount of yellow solid was precipitated. The reaction solution was extracted with 2-methyltetrahydrofuran(30 mL X 3), dried over anhydrous sodium sulfate, filtered, and concentrated under reduced pressure. The crude product was purified by silica gel column chromatography (ethyl acetate: petroleum ether = 0 - 20%) to obtain 6-fluoro-4-hydroxyl-2-naphthalenecarboxylic acid (5.3 g, yield: 58%). ES-API: [M+18]⁺= 224.1.

Step III: 6-fluoro-4-hydroxyl-2-naphthalenecarboxylic acid (5.3 g, 25.707 mmol), potassium carbonate (12.434 g, 89.974 mmol) and benzyl bromide (9.673 g, 56.555 mmol) were dissolved in N,N-dimethylformamide (60 mL), and the reaction solution was stirred overnight at 70°C. The reaction was shown to be completed by LC/MS. The reaction solution was cooled to room temperature, and added to iced water (100 mL). The reaction solution was extracted with ethyl acetate (30 mLX3). The organic phases were combined, washed with saturated brine (50 mLX3), dried over anhydrous sodium sulfate, filtered, and concentrated under reduced pressure. The crude product was purified by silica gel column chromatography (ethyl acetate / petroleum ether = 0 - 2%) to obtain benzyl 4-(benzyloxy)-6-fluoro-2-naphthalenecarboxylate (7.029 g, yield: 71%) as an off-white solid. ES-API: [M+ 18]⁺= 404.1.

Step IV: In an ice bath, benzyl 4-(benzyloxy)-6-fluoro-2-naphthalenecarboxylate (7.0 g, 18.115 mmol) was dissolved in a potassium hydroxide solution (20 wt%, 100 mL). The reaction solution was slowly heated to room temperature and then to 90°C and stirred overnight. The reaction was shown to be completed by LC/MS. The reaction solution was cooled to room temperature, and adjusted to about pH 1.0 with concentrated hydrochloric acid. A large amount of white solid was precipitated. The reaction solution was filtered, and concentrated under reduced pressure. The crude product was beaten in ethyl acetate / petroleum ether (V:V= 1:10) to obtain 4-(benzyloxy)-6-fluoro-2-naphthalenecarboxylic acid (4.917 g, yield: 92%) as an off-white solid. ES-API: [M+18]⁺= 314.0.

Step V: 4-(benzyloxy)-6-fluoro-2-naphthalenecarboxylic acid (4.4 g, 14.850 mmol), diphenylphosphoryl azide (6.13 g, 22.275 mmol) and triethyl amine (3.01 g, 29.700 mmol) were dissolved in toluene (50 mL) and t-butanol (50 mL). The reaction mixture was then heated to 90°C and stirred for 4 hrs. The reaction solution was cooled to room temperature, and concentrated under reduced pressure. The crude product was purified by silica gel column chromatography (ethyl acetate/petroleum ether= 0 - 3%) to obtain t-butyl (4-(benzyloxy)-6-fluoronaphthalen-2-yl)carbamate (5.316 g, yield: 97%) as an off-white solid. ES-API: [M-55]⁺= 312.1.

Step VI: 10% palladium on carbon (1 g) was added to a solution of t-butyl (4-(benzyloxy)-6-fluoronaphthalen-2-yl)carbamate (5.32 g, 14.468 mmol) in methanol (60 mL), and the reaction solution was stirred overnight under a hydrogen atmosphere at room temperature. The reaction was shown to be completed by LC/MS. The reaction solution was concentrated under reduced pressure, to obtain the crude product t-butyl (6-fluoro-4-hydroxylnaphthalen-2-yl)carbamate (4.01 g). ES-API: [M-55]⁺= 222.1.

Step VII: Under a nitrogen atmosphere, t-butyl (6-fluoro-4-hydroxylnaphthalen-2-yl)carbamate (4.01 g, 14.468 mmol), (bromoethynyl)triisopropylsilane (4.536 g, 17.362 mmol), potassium acetate (2.84 g, 28.936 mmol) and dichloro(p-methylisopropylphenyl)ruthenium (II) dimer (886 mg, 1.447 mmol) were dissolved in 1,4-dioxane (50 mL), and the reaction solution was heated to 100°C and stirred for 5 hrs. The reaction was shown to be completed by LC/MS. The reaction solution was cooled to room temperature, and concentrated under reduced pressure. The crude product was purified by silica gel column chromatography (ethyl acetate / petroleum ether= 0 - 2%) to obtain t-butyl (6-fluoro-4-hydroxyl-5-((triisopropylsilyl)ethynyl)naphthalen-2-yl)carbamate (4.01 g, yield: 61%) as a yellow solid. ES-API: [M-55]⁺= 402.2.

Step VIII: At -40°C, t-butyl (6-fluoro-4-hydroxyl-5-((triisopropylsilyl)ethynyl)naphthalen-2-yl)carbamate (1 g, 2.185 mmol) was dissolved in dichloromethane (20 mL). N,N-diisopropylethyl amine (0.8 mL, 13.110 mmol) and trifluoromethanesulfonic anhydride (925 mg, 3.278 mmol) were sequentially added to the reaction solution. The reaction solution was stirred at -40°C for 1 hr. The reaction was shown to be completed by LC/MS. The reaction solution was added with a saturated sodium bicarbonate solution (30 mL) to quench the reaction. The reaction solution was extracted with dichloromethane (20 mLX3). The organic phases were combined, and concentrated under reduced pressure. The crude product was purified by silica gel column chromatography (ethyl acetate/petroleum ether = 0 - 2%) to obtain 3-((t-butoxycarbonyl)amino)-7-fluoro-8-((triisopropylsilyl)ethynyl)naphthalen-1-yl trifluoromethylsulfonate (1107 mg, yield: 86%) as a light yellow solid. ES-API: [M-55]⁺= 534.1.

Step IX: Under a nitrogen atmosphere, 3-((t-butoxycarbonyl)amino)-7-fluoro-8-((triisopropylsilyl)ethynyl)naphthalen-1-yl trifluoromethylsulfonate (1.1 g, 1.865 mmol), bis(pinacolato)diboron (710 mg, 2.797 mmol), potassium acetate (458 mg, 4.662 mmol) and [1,1'-bis(diphenylphosphino)ferrocene]palladium dichloride (136 mg, 0.187 mmol) were dissolved in 1,4-dioxane (40 mL). The reaction solution was heated to 100°C and stirred overnight. The reaction was shown to be completed by LC/MS. The reaction solution was cooled to room temperature, and concentrated under reduced pressure. The crude product was purified by silica gel column chromatography (ethyl acetate / petroleum ether= 0 - 5%) to obtain t-butyl (6-fluoro-4-(4,4,5,5-tetramethyl-1,3,2-dioxaborolan-2-yl)-5-((triisopropylsilyl)ethynyl)naphthalen-2-alkyl)carbamate (185 mg, yield: 17%) as an off-white solid. ES-API: [M+ H]⁺= 568.3.

Step X: Under a nitrogen atmosphere, t-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (70 mg, 0.145 mmol), t-butyl (6-fluoro-4-(4,4,5,5-tetramethyl-1,3,2-dioxaborolan-2-yl)-5-((triisopropylsilyl)ethynyl)naphthalen-2- alkyl)carbamate (87 mg, 0.152 mmol), potassium phosphate (92 mg, 0.436 mmol) and [n-butyldi(1-adamantanyl)phosphine](2-amino-1,1'-biphenyl-2-yl)palladium (II) mesylate (106 mg, 0.145 mmol) were dissolved in tetrahydrofuran (3 mL) and water (0.6 mL), and the reaction mixture was stirred at 80°C under microwave for 1 hr. The reaction was shown to be completed by LC/MS. The reaction solution was dried over anhydrous sodium sulfate, and concentrated under reduced pressure. The crude product was purified by silica gel column chromatography (ethyl acetate / petroleum ether= 0 - 20%) to obtain t-butyl (5S,5aS,6S,9R)-2-(3-((t-butoxycarbonyl)amino)-7-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (135 mg). ES-API: [M+H]⁺= 887.3.

Step XI: In an iced water bath, t-butyl (5S,5aS,6S,9R)-2-(3-((t-butoxycarbonyl)amino)-7-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (135 mg, 0.152 mmol) was dissolved in anhydrous dichloromethane (6 mL). m-chloroperoxybenzoic acid (40 mg, 0.198 mmol) was added to the solution, and the reaction solution was stirred at 0°C for 30 min. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S,5aS,6S,9R)-2-(3-((t-butoxycarbonyl)amino)-7-fluoro-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hept-14-carboxylate (92 mg, yield: 67%). ES-API: [M+ H]⁺= 903.3.

Step XII: (2,2-difluorotetrahydro-1H-pyrrolizin-7a(5H)-yl)methanol (36 mg, 0.204 mmol) was dissolved in anhydrous tetrahydrofuran(5 mL). In an iced water bath, sodium hydride (12 mg, 0.306 mmol) was added, and reacted for 10 min in the iced water bath. A solution of t-butyl (5S,5aS,6S,9R)-2-(3-((t-butoxycarbonyl)amino)-7-fluoro-8-((triisopropylsilyl)ethynyl) naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hept-14-carboxylate (92 mg, 0.102 mmol) in tetrahydrofuran (5 mL) was added dropwise to the reaction solution, and reacted for 30 min at 0 °C. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure, to obtain the crude product t-butyl (5S,5aS,6S,9R)-2-(3-((t-butoxycarbonyl)amino)-7-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (103 mg). ES-API: [M+ H]⁺= 1016.5.

Step XIII: Cesium fluoride (307 mg, 2.020 mmol) was added to t-butyl (5S,5aS,6S,9R)-2-(3-((t-butoxycarbonyl)amino)-7-fluoro-8-((triisopropylsilyl)ethynyl)naphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (103 mg, 0.101 mmol) in N,N-dimethylformamide (5 mL), and the reaction mixture was stirred at room temperature for 1 hr. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S,5aS,6S,9R)-2-(3-((t-butoxycarbonyl)amino)-8-ethynyl-7-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-nonaphtho[1,8-ab]hepten-14-carboxylate (87 mg). ES-API: [M+H]⁺= 859.3.

Step XIV: In an iced water bath, a 4 M hydrochloric acid-dioxane solution (5 mL) was slowly added to a solution of t-butyl (5S,5aS,6S,9R)-2-(3-((t-butoxycarbonyl)amino)-8-ethynyl-7-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-nonaphtho[1,8-ab]hepten-14-carboxylate (87 mg, 0.101 mmol) in methanol (1 mL), and the reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-6-fluoronaphthalen-2-amine (Z722, 13.86 mg, yield: 21%) as a light yellow solid. ES-API: [M+H]⁺= 660.3.

### Example 110: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynyl-6-fluoronaphthalen-2-ol (Z723)

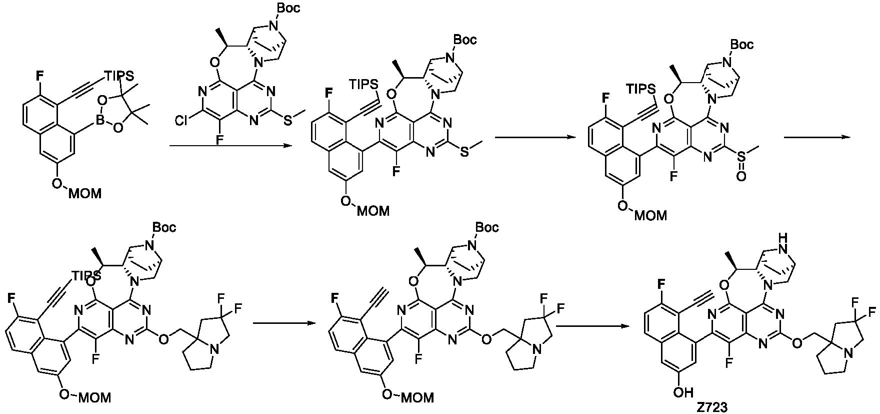

Step I: Potassium phosphate (352.0 mg, 1.660 mmol) and [n-butyldi(1-adamantanyl) phosphine](2-amino-1,1'-biphenyl-2-yl)palladium (II) mesylate (45.31 mg, 0.062 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab]hepten-14-carboxylate (200 mg, 0.415 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (425 mg, 0.829 mmol) in tetrahydrofuran (30 mL) and water (5 mL). N₂ was bubbled therethrough and the reaction was continued in an oil bath at 80°C for 3 hrs. After the reaction, the reaction solution was extracted with ethyl acetate (30 mLX2) and saturated brine (30 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-1-fluoro-2-(7-fluoro-3-(methoxymethoxy)-8-((triisopropylsilyl)ethynyl)naphthalen-1-yl)-5-methyl-12 -(methylthio)-5a,6,7,8,9,10-hexahydro-5H-4-oxa-3,10a,11,13,14-pentaaza-6,9-methanonaphtho[1,8-ab]hepten-14-carboxylate (310 mg, yield: 89.78%) as a yellow solid. ES-API: [M+H]⁺=832.2.

Step II: At 0°C, m-chloroperoxybenzoic acid (83.58 mg, 0.484 mmol) was added to a solution of t-butyl (5S,5aS,6S,9R)-1-fluoro-2-(7-fluoro-3-(methoxymethoxy)-8-((triisopropylsilyl) ethynyl)naphthalen-1-yl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (310 mg, 0.373 mmol) in dichloromethane (15 mL). The solution was stirred at room temperature for 1 hr. The reaction was quenched with saturated sodium sulfite and extracted with dichloromethane (100 mLX2). The organic phase was dried over anhydrous sodium sulfate, filtered and concentrated to obtain crude product. The residue was purified by silica gel column chromatography (methanol /dichloromethane0-10%) to obtain t-butyl (5S,5aS,6S,9R)-1-fluoro-2-(7-fluoro-3-(methoxymethoxy)-8-((triisopropylsilyl)ethynyl)naphthalen-1-yl)-5-methyl-12 -(methylsulfinyl)-5a,6,7,8,9,10-hexahydro-5H-4-oxa-3,10a,11,13,14-pentaaza-6,9-methanonaphtho[1,8-ab]hepten-14-carboxylate (350 mg, crude product) as a white solid. ES-API:[M+H]⁺=848.2.

Step III: At 0°C, sodium hydride (75.0 mg, 1.865 mmol) was added to a solution of (2,2-difluorotetrahydro-1H-pyrrolizin-7a(5H)-yl)methanol (132.18 mg, 0.746 mmol) in tetrahydrofuran (10.0 mL), and stirred at this temperature for 10 min. Then, a solution of t-butyl (5S,5aS,6S,9R)-1-fluoro-2-(7-fluoro-3-(methoxymethoxy)-8-((triisopropylsilyl)ethynyl) naphthalen-1-yl)-5-methyl-12-(methylsulfinyl)-5a,6,7,8,9,10-hexahydro-5H-4-oxa-3,10a,11,13, 14-pentaaza-6,9-methanonaphtho[1,8-ab]hepten-14-carboxylate (314.86 mg, 0.373 mmol) in tetrahydrofuran (5 mL) was added. The reaction was continued with stirring at 0°C for 10 min, and at room temperature for 0.5 hr. After the reaction was completed, the reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (methanol /dichloromethane0-5%) to obtain t-butyl (5S,5aS,6S,9R)-12-((2,2-difluorotetrahydro-1H-pyrrolizin-7a(5H)-yl)methoxy)-1-fluoro-2-(7-fluoro-3-(methoxymethoxy)-8-((triisopropylsilyl) ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptan-14-carboxylate (280 mg, yield: 78.48%) as a yellow solid. ES-API: [M+H]⁺=961.3.

Step IV: Cesium fluoride (2.75 g, 18.14 mmol) was added to a solution of t-butyl (5S,5aS,6S,9R)-12-((2,2-difluorotetrahydro-1H-pyrrolizin-7a(5H)-yl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methanonaphtho[1,8-ab]heptan-14-carboxylate (280.0 mg, 0.299 mmol) in N,N-dimethylformamide (15.0 mL), and reacted with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (5S,5aS,6S,9R)-12-((2,2-difluorotetrahydro-1H-pyrrolizin-7a(5H)-yl)methoxy)-2-(8-ethynyl-7-fluoro-3-(methoxymethoxy) naphthalen-1-yl)-1-fluoro-5-methyl-5a,6,7,8,9,10-hexahydro-5H-4-oxa-3,10a,11,13,14-pentaaza-6,9-methanonaphtho[1,8- ab]hepten-14-carboxylate (300 mg, crude product) as a yellow oil. ES-API: [M+H]⁺=805.2.

Step V: In an iced water bath, a 4 M hydrochloric acid-dioxane solution (10.0 mL) was slowly added to t-butyl (5S,5aS,6S,9R)-12-((2,2-difluorotetrahydro-1H-pyrrolizin-7a(5H)-yl) methoxy))-2-(8-ethynyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (240.0 mg, 0.2990 mmol) in methanol (15.0 mL). The reaction mixture was stirred at room temperature for 0.5 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynyl-6-fluoronaphthalen-2-ol(Z723, 77.90 mg, yield: 18.82%) as a light yellow solid. ES-API: [M+H]⁺= 661.2.

### Example 111: Synthesis of 4-((5S,5aS,6S,9R)-12-((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynylnaphthalen-2-amine (Z724)

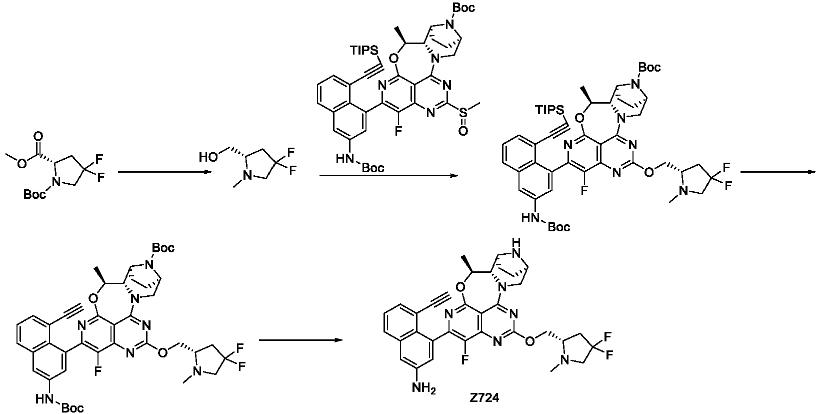

Step I: Under a nitrogen atmosphere, 1-t-butyl 2-methyl (2S)-4,4-difluoropyrrolidin-1,2-dicarboxylate (1.5 g, 5.65 mmol) was dissolved in anhydrous tetrahydrofuran (5 mL), and the mixture was allowed to stand in an iced water bath. A lithium aluminium hydride solution (17.0 mL, 17.0 mmol, 1 M solution in tetrahydrofuran) was slowly added dropwise to the above solution, heated to room temperature and stirred for 1 hr. The reaction was shown to be completed by LC/MS. The reaction was quenched with sodium sulfafe decahydrate, stirred at room temperature for 0.5 hr, and filtered. The filtrate was concentrated under reduced pressure, to obtain the crude product (S)-(4,4-difluoro-1-methylpyrrolidin-2-yl)methanol (850 mg, yield: 99.4). ES-API: [M+H]⁺= 152.1.

Step II: (S)-(4,4-difluoro-1-methylpyrrolidin-2-yl)methanol (67 mg, 0.44 mmol) was dissolved in anhydrous tetrahydrofuran (5 mL). In an iced water bath, sodium hydride (29 mg, 0.73 mmol) was added, and then reacted for 30 min in the iced water bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (130 mg, 0.15 mmol) in tetrahydrofuran (5 mL) was added dropwise to the reaction solution, and reacted for 30 min at 0 °C. The reaction was shown to be completed by LC/MS. The reaction solution was diluted with ethyl acetate (30 mL), and washed with saturated ammonium chloride solution (10 mLX 2) and saturated brine (10 mLX 3). The organic phase was dried over anhydrous sodium sulfate, and filtered. The filtrate was concentrated under reduced pressure. The crude product was purified by flash silica gel column chromatography (ethyl acetate /petroleum ether: 0-80%) to obtain the target product t-butyl (5S,5aS,6S,9R)-2-(3-((t-butoxycarbonyl)amino)-8-((triisopropylsilyl)ethynyl)naphthalen-1-yl)- 12-((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-nonaphtho[1,8-ab]hepten-14-carboxylate (100 mg, yield: 70.0%) as a light yellow solid. ES-API: [M+H]⁺=972.3.

Step III: Cesium fluoride (156 mg, 1.03 mmol) was added to t-butyl (5S,5aS,6S,9R)-2-(3-((t-butoxycarbonyl)amino)-8-((triisopropylsilyl)ethynyl)naphthalen-1-yl)-12-((S)-4,4-difluoro-1-methylpyrrolidin-2-yl)methoxy)-1-fluoro-5-methyl-5a,6,7,8,9,10-hexahydro-5H-4-oxy-3,10a,11,13,14-pentaaza-6,9-nonaphtho[1,8-ab]hepten-14-carboxylate (100 mg, 0.10mmol) in N,N-dimethylformamide (2 mL), and the reaction mixture was stirred at room temperature for 1 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((S)-4,4-difluoro-1-methylpyrrolidin-2-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (84 mg, yield: 100%) as a light yellow solid. ES-API: [M+ H]⁺= 816.3.

Step IV: T-butyl (5S,5aS,6S,9R)-2-(3-((t-butoxycarbonyl)amino)-8-ethynylnaphthalen-1-yl)- 12-((S)-4,4-difluoro-1-methylpyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (60 mg, 0.074 mmol) was dissolved in ethyl acetate (1.5 mL). 4 M hydrogen chloride/1, 4-dioxane (1.5 mL) was added at 0°C, and the reaction was stirred at room temperature for 1 hr. The reaction solution was concentrated under reduced pressure, alkalized with saturated sodium bicarbonate solution, extracted with ethyl acetate (20 mLX2), dried over anhydrous sodium sulfate, filtered, and concentrated to obtain a crude product. The crude product was purified by preparative HPLC (ammonium bicarbonate method 1) to obtain the target product 4-((5S,5aS,6S,9R)-12-((S)-4,4-difluoro-1-methylpyrrolidin-2-yl)methoxy)-1-fluoro-5-methyl-5a,6,7,8,9,10-hexahydro-5H-4-oxy-3,10a,11,13,14-pentaaza-6,9-methanonaphtho[1,8-ab]heptan-2-yl)-5-ethynylnaphthalen-2-amine (Z724, 12 mg, yield: 26.5%) as a white solid. ¹H NMR (400 MHz, DMSO-d6) δ 7.72 - 7.62 (m, 1H), 7.33 - 7.22 (m, 2H), 7.03 - 6.85 (m, 2H), 5.63 (d, *J* = 12.1 Hz, 2H), 5.22 - 5.07 (m, 1H), 4.56 - 4.40 (m, 2H), 4.39 - 4.27 (m, 1H), 3.97 (d, *J =* 8.8 Hz, 1H), 3.85 - 3.50 (m, 2H), 3.46 (t, *J* = 5.2 Hz, 1H), 3.42 - 3.33 (m, 2H), 3.10 - 3.01 (m, 1H), 2.96 (s, 1H), 2.74 - 2.60 (m, 1H), 2.38 (s, 3H), 2.29 - 2.14 (m, 1H), 1.90 - 1.80 (m, 1H), 1.78 - 1.53 (m, 3H), 1.49 - 1.39 (m, 3H).ES-API: [M+H]⁺= 616.2.

### Example 112: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-1-fluoronaphthalen-2-amine (Z725)

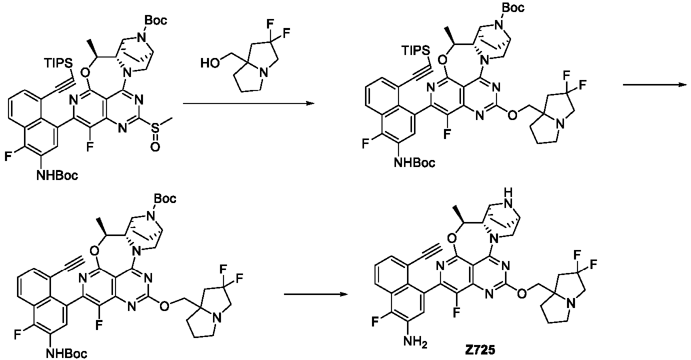

Step I: Sodium hydride (39 mg, 0.97 mmol) and tetrahydrofuran (8 mL) were added to a round-bottom flask. The reaction solution was cooled to 0°C, and (2,2-difluorotetrahydro-1H-pyrrolizin-7a(5H)-yl)methanol (74 mg, 0.41 mmol) was added, and reacted at room temperature for 5 min. T-butyl(5S,5aS,6S,9R)-2-(3-((t-butoxycarbonyl)amino)-4-fluoro-8-((triisopropylsilyl)ethynyl) naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (250 mg, 0.28 mmol) was added to the reaction solution, and reacted at room temperature for 30 min. The reaction was shown to be completed by LC/MS. The reaction solution was added with a saturated ammonium chloride solution (30 mL), and extracted with ethyl acetate (20 mLX2). The organic phase was washed with saturated brine (30 mL), dried over anhydrous sodium sulfate, filtered, and concentrated. The crude product was purified by flash silica gel column chromatography (ethyl acetate/petroleum ether: 0-30%) to obtain t-butyl (5S,5aS,6S,9R)-2-(3-((t-butoxycarbonyl)amino)-4-fluoro-8-((triisopropylsilyl)ethynyl)naphthalen-1-yl)-12-((2,2-difluorotetrahydro-1H-pyrrolidin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (130 mg, yield: 46%). ES-API: [M+H]⁺=1016.2.

Step II: T-butyl (5S,5aS,6S,9R)-2-(3-((t-butoxycarbonyl)amino)-4-fluoro-8-((triisopropylsilyl) ethynyl)naphthalen-1-yl)-12-((2,2-difluorotetrahydro-1H-pyrrolidin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (130 mg, 0.13 mmol), cesium fluoride (194 mg, 1.28 mmol) and N, N-dimethylformamide (3 mL) were added to a round-bottom flask. The reaction was stirred at room temperature for 1 hr. The reaction solution was added with water (30 mL), and extracted with ethyl acetate (30 mLX3). The organic phase was washed with saturated brine (30 mLX2), dried over anhydrous sodium sulfate, filtered, and concentrated to obtain t-butyl (5S,5aS,6S,9R)-2-(3-((t-butoxycarbonyl)amino)-8-ethynyl-4-fluoronaphthalen-1-yl)-12-((2,2-difluorotetrahydro-1H-pyrrolidin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110 mg, crude product). ES-API: [M+H]⁺=860.1.

Step III: T-butyl (5S,5aS,6S,9R)-2-(3-((t-butoxycarbonyl)amino)-8-ethynyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (110 mg, crude product) was dissolved in ethyl acetate (3 mL). The solution was cooled to 0°C, and a 4M hydrogen chloride/dioxane solution (3 mL, 12 mmol) was added. The reaction was stirred at room temperature for 1 hr. The reaction solution was concentrated to dryness at 40°C. The crude product was purified by preparative HPLC (ammonium bicarbonate method 1) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-1-fluoronaphthalen-2-amine (Z725, 35mg, yield: 41%). ES-API: [M+H]⁺=660.2. ¹HNMR(400 MHz, CD₃OD): 8.00-7.98(m, 1H), 7.50-7.39(m, 2H), 7.24-7.13 (m, 1H), 5.39-5.35(m, 1H), 4.50-4.47(m, 1H), 4.27-4.22(m, 2H), 4.06-4.03(m, 1H), 3.73-3.60(m, 2H), 3.48-3.15(m, 5H), 2.88-2.85(m, 1H), 2.60-2.55(m, 1H), 2.36-2.25(m, 1H), 2.16-1.77(m, 8H), 1.55-1.53(m, 3H).

### Example 113: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5- alkynylquinoline-2-amine (Z726)

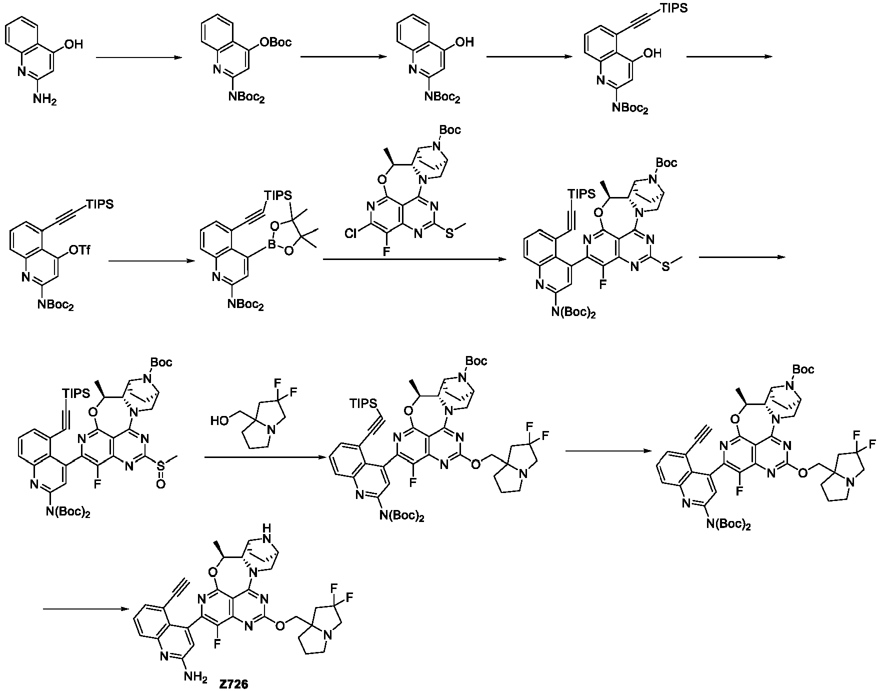

Step I: Di-t-butyl dicarbonate (10.8 mL, 46.82 mmol) and 4-dimethylaminopyridine(343 mg, 2.809 mmol) were added to a solution of 2-aminoquinoline-4-ol (1.5 g, 9.364 mmol) in tetrahydrofuran (50 mL), and the reaction solution was stirred at room temperature for 3 hrs. The reaction was shown to be completed by LC/MS, and concentrated under reduced pressure to remove most of the solvent. The residue was dissolved in ethyl acetate (20 mL), and washed with water (20 mL). The aqueous phase was extracted with ethyl acetate (15 mL X3). The organic phases were combined, dried over anhydrous sodium sulfate, filtered, and concentrated to obtain the crude product t-butyl 2-(N,N-di-t-butoxycarbonyl)aminoquinolin-4-yl carbonate(4.312 g). ES-API: [M+ H]⁺= 461.2.

Step II: Sodium carbonate (992 mg, 9.363 mmol) was added to a solution of t-butyl 2-(N,N-di-t-butoxycarbonyl)aminoquinoline-4-yl carbonate (4.312 g, 9.363 mmol) in methanol (50 mL) and water (10 mL), and the reaction solution was stirred overnight at room temperature. The reaction was shown to be completed by LC/MS, and concentrated under reduced pressure to remove most of the solvent. The residue was dissolved in water (15 mL), and adjusted to a weakly acidic pH with oxalic acid. A large amount of insoluble matter was precipitated. The reaction solution was filtered, concentrated under reduced pressure, and dried to obtain the crude product 2-(N,N-di-t-butoxycarbonyl)aminoquinoline-4-ol (2.908 g, yield: 86%). ES-API: [M+ H]⁺= 361.1.

Step III: Under a nitrogen atmosphere, 2-(N,N-di-t-butoxycarbonyl)aminoquinoline-4-ol (1.5 g, 4.162 mmol), (bromoethynyl)triisopropylsilane (1.305 g, 4.9945 mmol), potassium acetate (817 mg, 8.324 mmol) and dichloro(p-methylisopropylphenyl)ruthenium (II) dimer (255 mg, 0.416 mmol) were dissolved in 1,4-dioxane (30 mL). The reaction solution was heated to 100°C and stirred overnight. The reaction was shown to be completed by LC/MS. The reaction solution was cooled to room temperature, and concentrated under reduced pressure. The crude product was purified by silica gel column chromatography (ethyl acetate / petroleum ether= 0 - 10%) to obtain 2-(N,N-di-t-butoxycarbonyl)amino-5-((triisopropylsilyl)ethynyl)quinoline-4-ol (1.442 g, yield: 64%) as a yellow solid. ES-API: [M+ H]⁺= 541.3.

Step IV: At -40°C, 2-(N,N-di-t-butoxycarbonyl)amino-5-((triisopropylsilyl)ethynyl)quinoline-4-ol (1.442 mg, 2.667 mmol) was dissolved in dichloromethane (30 mL). N,N-diisopropylethyl amine (2.2 mL, 15.999 mmol) and trifluoromethanesulfonic anhydride (0.7 mL, 4.000 mmol) were sequentially added to the reaction solution. The reaction solution was stirred at - 40°C for 30 min. The reaction was shown to be completed by LC/MS. The reaction solution was added with a saturated sodium bicarbonate solution (30 mL) to quench the reaction. The reaction solution was extracted with dichloromethane (20 mLX3). The organic phases were combined, and concentrated under reduced pressure. The crude product was purified by silica gel column chromatography (ethyl acetate / petroleum ether= 0 - 1.9%) to obtain 2-(N,N-di-t-butoxycarbonyl)amino-5-((triisopropylsilyl)ethynyl)quinoline-4-yl trifluoromethansulfonate (1.443 g, yield: 80%) as a yellow oil. ES-API: [M+ H]⁺= 673.2.

Step V: Under a nitrogen atmosphere, 2-(N,N-di-t-butoxycarbonyl)amino-5-((triisopropylsilyl) ethynyl)quinoline-4-yl trifluoromethansulfonate (1.443 g, 2.145 mmol), bis(pinacolato)diboron (1.362 g, 5.362 mmol), potassium acetate (526 mg, 5.362 mmol) and [1,1'-bis (diphenylphosphino)ferrocene]palladium dichloride (300 mg, 0.429 mmol) were dissolved in 1,4-dioxane (30 mL), and the reaction solution was heated to 105°C and stirred overnight. The reaction was shown to be completed by LC/MS. The reaction solution was cooled to room temperature, and concentrated under reduced pressure. The crude product was purified by silica gel column chromatography (ethyl acetate / petroleum ether= 0 - 2%) to obtain 4-(4,4,5,5-tetramethyl-1,3,2-dioxaborolan-2-yl)-5-((triisopropylsilyl)ethynyl)quinoline-2-(N,N-di-t-butoxycarbonyl)amine (998 mg, yield: 72%) as a light yellow solid. ES-API: [M+ H]⁺= 651.3.

Step VI: Under a nitrogen atmosphere, t-butyl (5S,5aS,6S,9R)-2-chloro-1-fluoro-5-methyl-12-(methylthio)-5a,6,7,8,9,10-hexahydro-5H-4-oxy-3,10a,11,13,14-pentaaza-6,9-methanonaphtho [1,8-ab]hepten-14-carboxylate (105 mg, 0.218 mmol), 4-(4,4,5,5-tetramethyl-1,3,2-dioxaborolan-2-yl)-5-((triisopropylsilyl)ethynyl)quinoline-2-(N,N-di-t-butoxycarbonyl)amine (149 mg, 0.229 mmol), potassium phosphate (139 mg, 0.654 mmol) and [n-butyldi(1-adamantanyl)phosphine](2-amino-1,1'-biphenyl-2-yl)palladium (II) mesylate(24 mg, 0.033 mmol) were dissolved in tetrahydrofuran (3 mL) and water (0.6 mL). The reaction mixture was stirred at 80°C under microwave for 40 min. The reaction was shown to be completed by LC/MS. The reaction solution was dried over anhydrous sodium sulfate, and concentrated under reduced pressure. The crude product was purified by silica gel column chromatography (ethyl acetate / petroleum ether= 0 - 15%) to obtain t-butyl (5S,5aS,6S,9R)-2-(2-(N,N-di-t-butoxycarbonyl)amino-5-((triisopropylsilyl) ethynyl)quinoline-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (184 mg, yield: 87%). ES-API: [M+H]⁺= 970.3.

Step VII: In an iced water bath, t-butyl (5S,5aS,6S,9R)-2-(2-(N,N-di-t-butoxycarbonyl)amino-5-((triisopropylsilyl)ethynyl)quinoline-4-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (184 mg, 0.190 mmol) was dissolved in anhydrous dichloromethane (8 mL). m-chloroperoxybenzoic acid (50 mg, 0.247 mmol) was added to the solution, and the reaction solution was stirred at 0°C for 30 min. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S,5aS,6S,9R)-2-(2-(N,N-di-t-butoxycarbonyl)amino-5-((triisopropylsilyl)ethynyl)quinoline-4-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (187 mg, yield: 67%). ES-API: [M+ H]⁺= 986.3.

Step VIII: In an iced water bath, (2,2-difluorotetrahydro-1H-pyrrolizin-7a(5H)-yl)methanol (33 mg, 0.189 mmol) was dissolved in anhydrous tetrahydrofuran (3 mL). Sodium hydride (11.3 mg, 0.283 mmol) was added, and reacted for 10 min in the iced water bath. A solution of t-butyl (5S,5aS,6S,9R)-2-(2-(N,N-di-t-butoxycarbonyl)amino-5-((triisopropylsilyl)ethynyl)quinoline-4-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (93 mg, 0.094 mmol) in tetrahydrofuran (3 mL) was added dropwise to the reaction solution, and reacted for 30 min at 0 °C. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure to obtain a crude product that was t-butyl (5S,5aS,6S,9R)-2-(2-(N,N-di-t-butoxycarbonyl)amino-5-((triisopropylsilyl)ethynyl)quinoline-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (103 mg). ES-API: [M-100]⁺= 998.5.

Step IX: Cesium fluoride (143 mg, 0.940 mmol) was added to t-butyl (5S,5aS,6S,9R)-2-(2-(N,N-di-t-butoxycarbonyl)amino-5-((triisopropylsilyl)ethynyl)quinoline-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (103 mg, 0.094 mmol) in N,N-dimethylformamide (5 mL), and the reaction mixture was stirred at room temperature for 1 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S,5aS,6S,9R)-2-(2-(N,N-di-t-butoxycarbonyl)amino-5-ethynylquinoline-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (88 mg). ES-API: [M-100]⁺= 843.2.

Step X: In an iced water bath, a 4 M hydrochloric acid-dioxane solution (10 mL) was slowly added to a solution of t-butyl (5S,5aS,6S,9R)-2-(2-(N,N-di-t-butoxycarbonyl)amino-5-ethynylquinoline-4-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (88 mg, 0.093 mmol) in methanol (1 mL). The reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5- alkynylquinoline-2-amine (Z726, 12.99 mg, yield: 22%) as a white solid. ES-API: [M+H]⁺= 643.0. ¹H NMR (400 MHz, CD₃OD) δ 7.67- 7.64 (m, 1 H), 7.54-7.47 (m, 1 H), 7.44- 7.37 (m, 1 H), 6.91- 6.82 (m, 1 H), 5.40- 5.34 (m, 1 H), 4.57- 4.51 (m, 1 H), 4.31- 4.23 (m, 2 H), 4.09 (d, J= 8.4 Hz, 1 H), 3.72- 3.70 (m, 1 H), 3.63- 3.61 (m, 1 H), 3.48- 3.40 (m, 1 H), 3.22- 3.09 (m, 4 H), 2.89- 2.83 (m, 1 H), 2.61- 2.52 (m, 1 H), 2.39- 2.28 (m, 1 H), 2.21- 1.75 (m, 8 H), 1.57 (t, J1= 6.4 Hz, J2= 12.8 Hz, 3 H).

### Example 114: Synthesis of (4-((5S,5aS,6S,9R)-12-(2,6-dimethylenetetrahydro-1-hydrogen-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-1-fluoronaphthalen-2-amine (Z727)

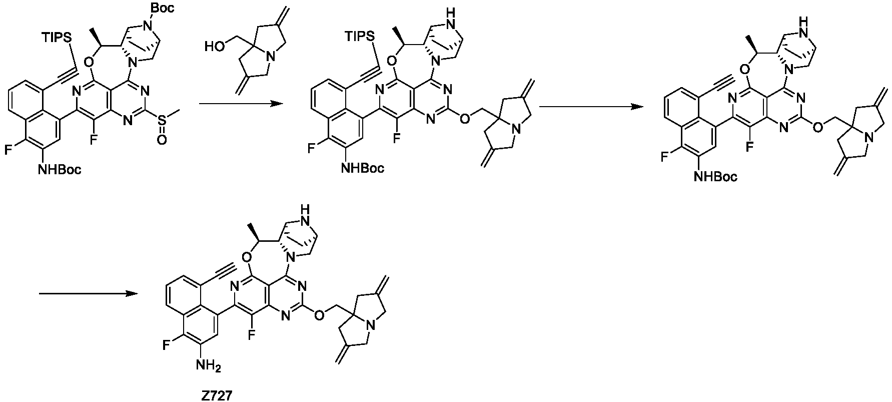

Step I: At 0°C, sodium hydride (12 mg, 0.5 mmol) was added to a solution of (2,6-dimethylenetetrahydro-1H-pyrrolizin-7a(5H)-yl)methanol (40.25 mg, 0.244 mmol) in tetrahydrofuran (10.0 mL), and stirred at this temperature for 10 min. Then, a solution of t-butyl (5S,5aS,6S,9R)-2-(3-((t-butoxycarbonyl)amino)-4-fluoro-8-((triisopropylsilyl)ethynyl) naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (110 mg, 0.122 mmol) in tetrahydrofuran (5 mL) was added. The reaction was continued with stirring at 0°C for 10 min, and at room temperature for 0.5 hr. After the reaction was completed, the reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (methanol /dichloromethane=0-5%) to obtain t-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-1-fluoro-5-((triisopropylsilyl)ethynyl)naphthalen-2-yl)carbamate (70 mg, 0.070 mmol, yield: 57.23%) as a yellow solid. ES-API: [M+H]⁺=1004.2.

Step II: Cesium fluoride (105.87 mg, 0.697 mmol) was added to a solution of t-butyl (4-((5S,5aS,6S,9R)-12-((2,6-dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-1-fluoro-5-((triisopropylsilyl)ethynyl)naphthalen-2-yl)carbamate (70 mg, 0.070 mmol) in N,N-dimethylformamide (1.0 mL), and reacted with stirring at room temperature for 0.5 hr. After the reaction, the reaction was quenched with water and extracted with ethyl acetate. The organic phase was washed with saturated brine, dried over anhydrous sodium sulfate, filtered, and concentrated to obtain t-butyl (4-((5S,5aS,6S,9R)-12-(2,6-dimethylenetetrahydro-1-hydrogen-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynyl-1-fluoronaphthalen-2-yl)carbamate (35 mg, 0.044 mmol, yield: 83.78%) as a yellow oil. ES-API: [M+H]⁺=848.3.

Step III: In an iced water bath, 4 M hydrochloric acid-dioxane solution (1.0mL) was slowly added to a solution of t-butyl (4-((5S,5aS,6S,9R)-12-(2,6-dimethylenetetrahydro-1-hydrogen-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]heptan-2-yl)-5-ethynyl-1-fluoronaphthalen-2-yl)carbamate (35 mg, 0.044 mmol) in ethyl acetate (1.0 mL). The reaction mixture was stirred at room temperature for 0.5 hr. The reaction was shown to be completed by LC/MS. The reaction solution was concentrated to dryness under reduced pressure, and the residue was purified by preparative HPLC (ammonium bicarbonate method 2) to obtain (4-((5S,5aS,6S,9R)-12-(2,6-dimethylenetetrahydro-1-hydrogen-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]heptan-2-yl)-5-ethynyl-1-fluoronaphthalen-2-amine (Z727, 7.1mg, yield: 23.24%) as a light yellow solid. ES-API: [M+H]⁺= 648.0.

### Example 115: Synthesis of 5-ethynyl-1-fluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z728)

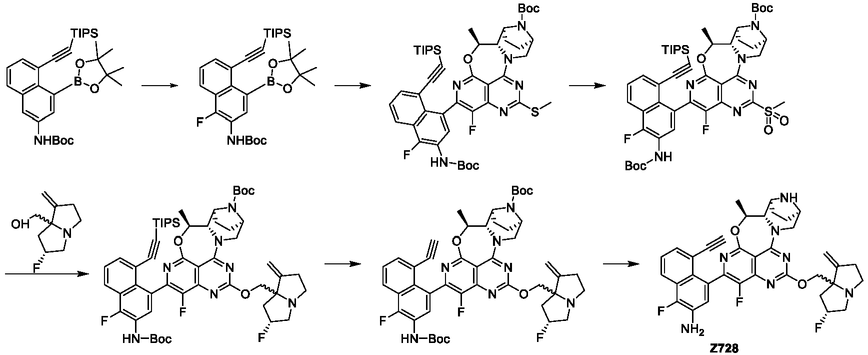

Step I: T-butyl (4-(4,4,5,5-tetramethyl-1,3,2-dioxaborolan-2-yl)-5-((triisopropylsilyl)ethynyl) naphthalen-2-yl)carbamate (1000 mg, 1.819 mmol) was dissolved in acetonitrile (50 mL). 1-Chloromethyl-4-fluoro-1,4-diazabicyclo[2.2.2]octanebis(tetrafluoroborate) (837.91 mg, 2.365 mmol) was added to the solution at room temperature. After that, the reaction was stirred at room temperature for 17 hr. After the reaction, the reaction solution was concentrated to remove acetonitrile. The crude product was purified by flash silica gel column chromatography (ethyl acetate/petroleum ether: 0-2%) to obtain t-butyl (1-fluoro-4-(4,4,5,5-tetramethyl-1,3,2-dioxaborolan-2-yl)-5-((triisopropylsilyl)ethynyl)naphthalen-2-yl)carbamate (550 mg, 0.969 mmol, yield: 53.26%) as a reddish brown solid. ES-API: [m +H]⁺= 568.1.

Step II: T-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (400 mg, 0.830 mmol) and t-butyl (1-fluoro-4-(4,4,5,5-tetramethyl-1,3,2-dioxaborolan-2-yl)-5-((triisopropylsilyl)ethynyl)naphthalen-2-yl)carbamate (942.17 mg, 1.660 mmol) were dissolved in dioxane (15 mL) and water (2 mL). [n-butyldi(1-adamantanyl) phosphine](2-amino-1,1'-biphenyl-2-yl)palladium (II) mesylate (60.42 mg, 0.083 mmol) and potassium phosphate (528.51 mg, 2.490 mmol) were added to the solution. After that, the reaction was stirred at 90°C under microwave for 1 hrs. After the reaction, the reaction solution was added with water (20 mL) and extracted with ethyl acetate (20 mLX3). The organic phase was washed with saturated brine (30 mL), dried over anhydrous sodium sulfate, filtered and concentrated. The crude product was purified by flash silica gel column chromatography (ethyl acetate /petroleum ether: 0-18%) to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (600 mg, 0.676 mmol, yield: 81.49%) as a white solid. ES-API: [M +h]⁺= 887.3.

Step III: T-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab] hepten-14-carboxylate (720 mg, 0.812 mmol) was dissolved in dichloromethane (15 mL). At room temperature, m-chloroperoxybenzoic acid (purity 85%, 494.30 mg, 2.435 mmol) was added to the solution. After that, the reaction was stirred at room temperature for 1 hr. The reaction solution was added with a saturated sodium thiosulphate (10 mL) and extracted with dichloromethane (15 mLX3). The organic phases were combined, and then washed sequentially with a saturated sodium bicarbonate aqueous solution (15 mL) and saturated sodium chloride (30 mL). The organic phase was dried and concentrated to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab] hepten-14-carboxylate (720 mg, 0.783 mmol, yield: 96.52%) as a light yellow solid. ES-API: [M +H]⁺= 919.3.

Step IV: ((6R)-6-fluoro-1-methylenetetrahydro-1H-pyrrolizin-7a(5H)-yl)methanol (150 mg, 0.876 mmol) was dissolved in tetrahydrofuran (5 mL) and cooled to 0°C. Under a nitrogen atmosphere, sodium hydride (60%, 94.00 mg, 2.350 mmol) was added to the solution and stirred for 30 min. After that, t-butyl (5S,5aS,6S,9R)-2-(3-((t-butoxycarbonyl)amino)-4-fluoro-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (720 mg, 0.783 mmol) was slowly added to the solution and stirred 0°C for 2 hrs. The reaction solution was added with water (20 mL) to quench the reaction, and then extracted with ethyl acetate (20 mL X3). The organic phase was washed with saturated brine (10 mLX3), dried over anhydrous sodium sulfate, filtered and concentrated to obtain a crude product. The crude product was purified by flash silica gel column chromatography (methanol /dichloromethane: 0-3%) to obtain t-butyl (5S,5aS,6S,9R)-2-(3-((t-butoxycarbonyl)amino)-4-fluoro-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (550 mg, 0.544 mmol, yield: 69.50%) as a light yellow solid. ES-API: [M+H]⁺=1010.3.

Step V: A mixture of t-butyl (5S,5aS,6S,9R)-2-(3-((t-butoxycarbonyl)amino)-4-fluoro-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (550 mg, 0.544 mmol), cesium fluoride (826.94 mg, 5.444 mmol) and N, N-dimethylformamide (10 mL) was stirred at room temperature for 2 hrs. The reaction mixture was added with water (30 mL) and extracted with ethyl acetate (20 mL X2). The organic phase was washed with saturated brine (30 mLX3), dried over anhydrous sodium sulfate, filtered and concentrated to obtain t-butyl (5S,5aS,6S,9R)-2-(3-((t-butoxycarbonyl)amino)-8-ethynyl-4-fluoronaphthalenyl-1-yl)-1-fluoro-12-(((6R) )-6-fluoro-1-methylenetetrahydro-1H-pyrrolizin-7a(5H)-yl)methoxy)-5-5-methyl-5a,6,7,8,9,10-hexahydro-5H-4-oxa-3,10a,11,13,14-pentaaza-6,9-methanonaphtho[1,8-ab]hepten-14-carboxylate (400 mg, 0.468 mmol, yield: 86.04%) as a light yellow solid. ES-API: [M+H]⁺=854.2.

Step VI: T-butyl (5S,5aS,6S,9R)-2-(3-((t-butoxycarbonyl)amino)-8-ethynyl-4-fluoronaphthalenyl-1-yl)-1-fluoro-12-(((6R))-6-fluoro-1-methylenetetrahydro-1H-pyrrolizin-7a(5H)-yl)methoxy)-5-5-methyl-5a,6,7,8,9,10-hexahydro-5H-4-oxa-3,10a,11,13,14-pentaaza-6,9-methanonaphtho[1,8-ab]hepten-14-carboxylate (400 mg, 0.468 mmol) was dissolved in ethyl acetate (5 mL), and cooled to 0°C. A hydrogen chloride-dioxane solution (4 M, 15 mL) was added to the solution, and reacted with stirring at 0°C for 2 hrs. The reaction solution was concentrated, and the residue was taken up in dichloromethane (2 mL) again and alkalized with ammonia in methanol (7 M) to an alkaline pH (pH 8). The obtained solution was concentrated, and the crude product was purified by preparative HPLC (formic acid method 1) to obtain 5-ethynyl-1-fluoro-4-((5S,5aS,6S,9R)-1-fluoro-12-(((6R)-6-fluoro-1-methylenetetrahydro-1H-pyrrolizin-7a(5H)- yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z728, 130mg,0.199 mmol, yield: 42.45%, formate) as a white solid. ES-API: [M+H]⁺=654.2.

### Example 116: Synthesis of 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethyl-1-fluoronaphthalen-2-amine (Z729)

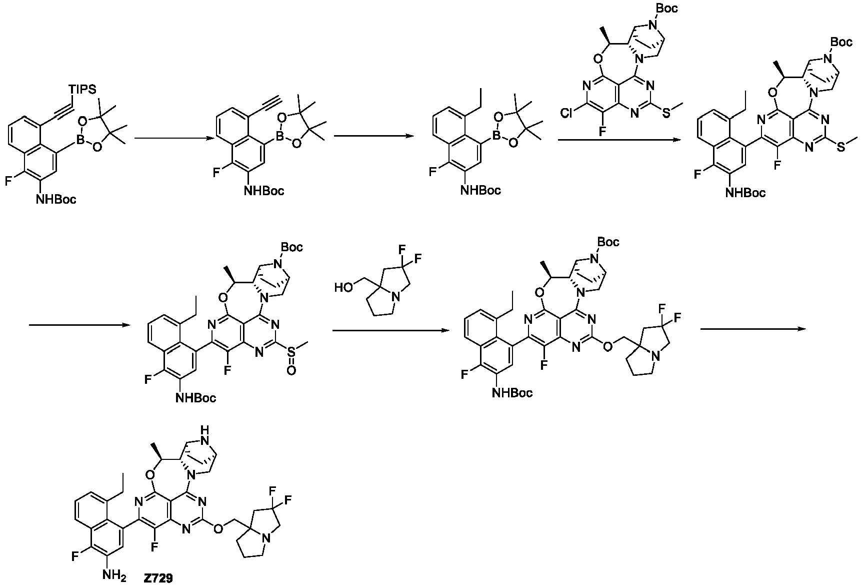

Step I: Cesium fluoride (803 mg, 5.285 mmol) was added to t-butyl (1-fluoro-4-(4,4,5,5-tetramethyl-1,3,2-dioxaborolan-2-yl)-5-((triisopropylsilyl)ethynyl)naphthalen-2-yl)carbamate (300 mg, 0.529 mmol) in N,N-dimethylformamide (5 mL), and the reaction mixture was stirred at room temperature for 1 hrs. The reaction was shown to be completed by LC/MS. The reaction solution was diluted with ethyl acetate (20 mL), washed with water (15 mLX 2) and saturated brine (15 mLX3), dried over anhydrous sodium sulfate, filtered, and concentrated to obtain t-butyl (5-ethynyl-1-fluoro-4-(4,4,5,5-tetramethyl-1,3,2-dioxaborolan-2-yl)naphthalen-2-carbamate(217 mg). ES-API: [M-55]⁺= 356.1.

Step II: T-butyl (5-ethynyl-1-fluoro-4-(4,4,5,5-tetramethyl-1,3,2-dioxaborolan-2-yl) naphthalen-2-carbamate (217 mg, 0.528 mmol) was dissolved in methanol (10 mL). The reaction solution was added with 10% palladium on carbon (45 mg), and then the reaction solution was stirred under a hydrogen atmosphere at room temperature for 2 hrs. The reaction was shown to be completed by LC/MS. The reaction solution was filtered through diatomite, and the filtrate was concentrated under reduced pressure, to obtain the crude product t-butyl (5-ethyl-1-fluoro-4-(4,4,5,5-tetramethyl-1,3,2-dioxaborolan-2-yl)naphthalen-2-methyl)carbamate(219 mg). ES-API: [M-55]⁺= 360.2.

Step III: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (80 mg, 0.166 mmol), t-butyl (5-ethyl-1-fluoro-4-(4,4,5,5-tetramethyl-1,3,2-dioxaborolan-2-yl)naphthalen-2-methyl)carbamate (103 mg, 0.249 mmol), potassium phosphate (106 mg, 0.498 mmol) and [n-butyldi(1-adamantanyl)phosphine](2-amino-1,1'-biphenyl-2-yl)palladium (II) mesylate(18 mg, 0.025 mmol) were dissolved in tetrahydrofuran (5 mL) and water (1 mL). The reaction mixture was stirred at 80°C under microwave for 45 min. The reaction was shown to be completed by LC/MS. The reaction solution was dried over anhydrous sodium sulfate, and concentrated under reduced pressure. The crude product was purified by silica gel column chromatography (ethyl acetate / petroleum ether= 0 - 20%) to obtain t-butyl (5S,5aS,6S,9R)-2-(3-((t-butoxycarbonyl)amino)-8-ethyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (115 mg, yield: 94%). ES-API: [M+H]⁺= 735.3.

Step IV: In an iced water bath, t-butyl (5S,5aS,6S,9R)-2-(3-((t-butoxycarbonyl)amino)-8-ethyl-4-fluoronaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (143 mg, 0.195 mmol) was dissolved in anhydrous dichloromethane (6 mL). m-chloroperoxybenzoic acid (51 mg, 0.254 mmol) was added to the solution, and the reaction solution was stirred at 0°C for 30 min. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S,5aS,6S,9R)-2-(3-((t-butoxycarbonyl)amino)-8-ethyl-4-fluoronaphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (146 mg). ES-API: [M+ H]⁺= 751.3.

Step V: (2,2-difluorotetrahydro-1H-pyrrolizin-7a(5H)-yl)methanol (69 mg, 0.389 mmol) was dissolved in anhydrous tetrahydrofuran (5 mL). In an iced water bath, sodium hydride (23 mg, 0.583 mmol) was added, and reacted for 10 min in the iced water bath. A solution of t-butyl (5S,5aS,6S,9R)-2-(3-((t-butoxycarbonyl)amino)-8-ethyl-4-fluoronaphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (146 mg, 0.389 mmol) in tetrahydrofuran (5 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure, to obtain the crude product t-butyl (5S,5aS,6S,9R)-2-(3-((t-butoxycarbonyl)amino)-8-ethyl-4-fluoronaphthalen-1-yl)-12-((2,2-difluorotetrahydro- 1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (167 mg). ES-API: [M+ H]⁺= 864.3.

Step VI: In an iced water bath, a 4 M hydrochloric acid-dioxane solution (10 mL) was slowly added to a solution of t-butyl (5S,5aS,6S,9R)-2-(3-((t-butoxycarbonyl)amino)-8-ethyl-4-fluoronaphthalen-1-yl)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (167 mg, 0.194 mmol) in methanol (1 mL). the reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethyl-1-fluoronaphthalen-2-amine (Z729, 13.05 mg, yield: 10%) as a white solid. ES-API: [M+H]⁺= 664.0. ¹H NMR (400 MHz, CD₃OD) δ 7.85- 7.83 (m, 1 H), 7.43- 7.37 (m, 1 H), 7.19- 6.98 (m, 2 H), 5.39- 5.34 (m, 1 H), 4.58- 4.52 (m, 1 H), 4.31- 4.25 (m, 2 H), 4.10-4.05 (m, 1 H), 3.70- 3.69 (m, 1 H), 3.63-3.59 (m, 1 H), 3.49- 3.39 (m, 1 H), 3.21- 3.09 (m, 3 H), 2.88- 2.82 (m, 1 H), 2.61- 2.47 (m, 2 H), 2.39- 2.17 (m, 3 H), 2.07- 1.77 (m, 7 H), 1.57- 1.54 (m, 3 H), 1.01 (t, J1= 7.6 Hz, J2= 15.2 Hz, 1 H), 0.88 (t, J1= 7.6 Hz, J2= 15.2 Hz, 2H).

### Example 117: Synthesis of 5-ethynyl-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z730)

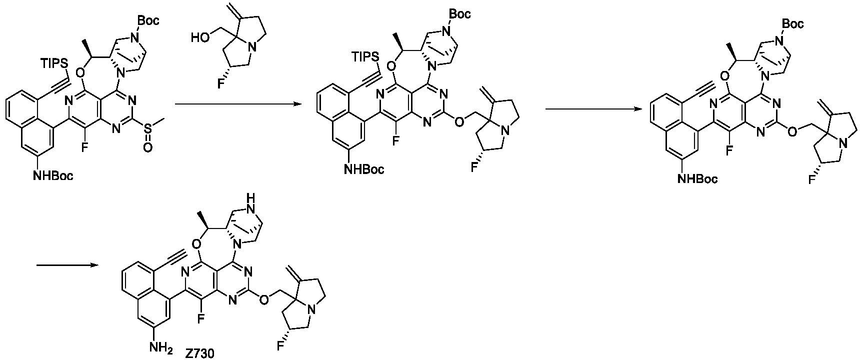

Step I: 60% sodium hydride (25 mg, 0.62 mmol) and tetrahydrofuran (5 mL) were added to a round-bottom flask. The reaction solution was cooled to 0°C, and ((6R)-6-fluoro-1-methylenetetrahydro-1H-pyrrolizin-7a(5H)-yl)methanol (25mg, 0.15 mmol) was added to the reaction solution, and reacted at room temperature for 5 min. T-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxy-3,10a,11,13,14-pentaaza-6,9-methanonaphtho[1,8-ab]hept-14-carboxylate (110 mg, 0.12 mmol) was added to the reaction solution, and reacted at room temperature for 30 min. The reaction was shown to be completed by LC/MS. The reaction solution was added with a saturated ammonium chloride solution (30 mL), and extracted with ethyl acetate (20 mLX2). The organic phase was washed with saturated brine (30 mL), dried over anhydrous sodium sulfate, filtered, and concentrated. The crude product was purified by flash silica gel column chromatography (methanol /dichloromethane: 0-3%) to obtain t-butyl (5S,5aS,6S,9R)-2-(3-((t-butoxycarbonyl)amino)-8-((triisopropylsilyl)ethynyl)naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (75 mg, yield: 60%). ES-API: [M+H]⁺=992.3.

Step II: T-butyl (5S,5aS,6S,9R)-2-(3-((t-butoxycarbonyl)amino)-8-((triisopropylsilyl)ethynyl) naphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (75 mg, 0.076 mmol), cesium fluoride (115 mg, 0.76 mmol), and N, N-dimethylformamide (3 mL) were added to a round-bottom flask. The reaction was stirred at room temperature for 1 hr. The reaction solution was added with water (30 mL), and extracted with ethyl acetate (30 mLX3). The organic phase was washed with saturated brine (30 mLX2), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (60 mg, crude product). ES-API: [M+H]⁺=836.0.

Step III: T-butyl (5S,5aS,6S,9R)-2-(3-((t-butoxycarbonyl)amino)-8-ethynylnaphthalen-1-yl)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (60 mg, crude product) was dissolved in ethyl acetate (1 mL). The solution was cooled to 0°C, and a 4M hydrogen chloride/dioxane solution (1 mL, 4 mmol) was added. The reaction was stirred at room temperature for 1 hr. The reaction solution was concentrated to dryness at 40°C, and the crude product was purified by preparative HPLC (formic acid method 1) to obtain 5-ethynyl-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z730, 15mg, yield: 28%, formate). ES-API: [M+H]⁺=636.3. ¹HNMR(400 MHz, CD₃OD): 8.41(s, 2H, Formic acid proton), 7.71-7.67(m, 1H), 7.42-7.27(m, 2H), 7.15-7.01(m, 2H), 5.51-5.20(m, 3H), 4.90-4.86(m, 1H), 4.61-4.45(m, 2H), 4.26-4.18(m, 2H), 3.93-3.79(m, 3H), 3.63-3.48(m, 1H), 3.31-2.95(m, 3H), 2.49-2.26(m, 2H), 2.20-2.10(m, 1H), 1.98-1.85(m, 6H), 1.60-1.57(m, 3H).

### Example 118: Synthesis of 1,5,6-trifluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z731)

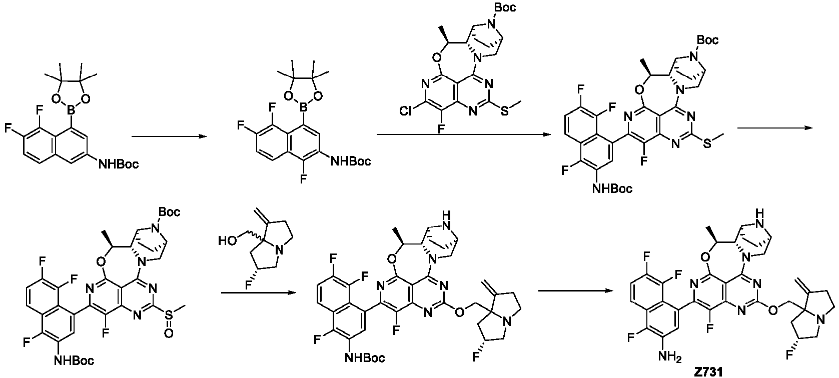

Step I: 1-chloromethyl-4-fluoro-1,4-diazabicyclo[2.2.2]octane bis(tetrafluoroborate) (136 mg, 0.385 mmol) was added to a solution of t-butyl (5,6-difluoro-4-(4,4,5,5-tetramethyl-1,3,2-dioxaborolan-2-yl)naphthalen-2-carbamate (120 mg, 0.296 mmol) in acetonitrile (50 mL), and the reaction mixture was stirred overnight at room temperature. The reaction was shown to be completed by LC/MS, and concentrated under reduced pressure to remove the solvent. The residue was purified by automated flash silica gel chromatography (ethyl acetate / petroleum ether= 0 - 10%) to obtain t-butyl to obtain (1,5,6-trifluoro-4-(4,4,5,5-tetramethyl-1,3,2-dioxaborolan-2-yl)naphthalen-2-methyl)carbamate (43 mg, yield: 34%). ES-API: [M+ H]⁺= 424.0.

Step II: Under a nitrogen atmosphere, t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (48 mg, 0.100 mmol), (1,5,6-trifluoro-4-(4,4,5,5-tetramethyl-1,3,2-dioxaborolan-2-yl)naphthalen-2-methyl)carbamate (43 mg, 0.102 mmol), potassium phosphate (64 mg, 0.300 mmol) and [n-butyldi(1-adamantanyl)phosphine](2-amino-1,1'-biphenyl-2-yl)palladium (II) mesylate (11 mg, 0.015 mmol) were dissolved in tetrahydrofuran (2 mL) and water (0.4 mL). The reaction mixture was stirred at 80°C under microwave for 45 min. The reaction was shown to be completed by LC/MS. The reaction solution was dried over anhydrous sodium sulfate, and concentrated under reduced pressure. The crude product was purified by TLC on silica gel (ethyl acetate / petroleum ether = 1: 3) to obtain t-butyl (5S,5aS,6S,9R)-2-(3-((t-butoxycarbonyl)amino)-4,7,8-trifluoronaphthalen-1-yl)-1-fluoro-5-methyl-12-(methylthio)-5a,6,7,9,9,10-hexahydro-5H-4-oxa-3,10a,11,13,14-pentaaza-6,9-methanonaphtho[1,8-ab]hepten-14-carboxylate (66 mg, yield: 89%). ES-API: [M+H]⁺= 743.2.

Step III: In an iced water bath, t-butyl (5S,5aS,6S,9R)-2-(3-((t-butoxycarbonyl)amino)-4,7,8-trifluoronaphthalen-1-yl)-1-fluoro-5-methyl-12-(methylthio)-5a,6,7,9,9,10-hexahydro-5H-4-oxa-3,10a,11,13,14-pentaaza-6,9-methanonaphtho[1,8-ab]hepten-14-carboxylate (66 mg, 0.089 mmol) was dissolved in anhydrous dichloromethane (6 mL). m-chloroperoxybenzoic acid (24 mg, 0.116 mmol) was added to the solution, and the reaction solution was stirred at 0°C for 30 min. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S,5aS,6S,9R)-2-(3-((t-butoxycarbonyl)amino)-4,7,8-trifluoronaphthalen-1-yl)-1-fluoro-5-methyl-12-(methylthionyl)-5a,6,7,9,9,10-hexahydro-5H-4-oxy-3,10a,11,13,14-pentaaza-6,9-methanonaphtho[1,8-ab]hepten-14-carboxylate (76 mg). ES-API: [M+ H]⁺= 759.2.

Step IV: ((6R)-6-fluoro-1-methylenetetrahydro-1H-pyrrolizin-7a(5H)-yl)methanol (34.3 mg, 0.200 mmol) was dissolved in anhydrous tetrahydrofuran(5 mL). In an iced water bath, sodium hydride (23 mg, 0.583 mmol) was added, and reacted for 10 min in the iced water bath. A solution of t-butyl (5S,5aS,6S,9R)-2-(3-((t-butoxycarbonyl)amino)-4,7,8-trifluoronaphthalen-1-yl)-1-fluoro-5-methyl-12-(methylthionyl)-5a,6,7,9,9,10-hexahydro-5H-4-oxy-3,10a,11,13,14-pentaaza-6,9-methanonaphtho[1,8-ab]hepten-14-carboxylate (76 mg, 0.100 mmol) in tetrahydrofuran (3 mL) was added dropwise to the reaction solution, and reacted at 0°C for 30 min. The reaction was shown to be completed by LC/MS. The reaction solution was diluted with ethyl acetate (10 mL), washed with a saturated ammonium chloride solution (10 mLX2) and saturated brine (10 mLX3), dried over anhydrous sodium sulfate, filtered, and concentrated under reduced pressure, to obtain the crude product t-butyl (1,5,6-trifluoro-4-((5S,5aS,6S,9R)-1-fluoro-12-((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptan-2-yl)naphthalen-2-yl)carbamate (86 mg). ES-API: [M+ H]⁺= 866.3.

Step V: In an iced water bath, a 4 M hydrochloric acid-dioxane solution (10 mL) was slowly added to a solution of t-butyl (1,5,6-trifluoro-4-((5S,5aS,6S,9R)-1-fluoro-12-((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptan-2-yl)naphthalen-2-yl)carbamate (86 mg, 0.099 mmol) in methanol (1 mL), and the reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 1,5,6-trifluoro-4-((5S,5aS,6S,9R)-1-fluoro-12-(((6R)-6-fluoro-1-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z731, 5.32 mg, yield: 8%) as a white solid. ES-API: [M+H]⁺= 666.0.

### Example 119: Synthesis of 5-ethynyl-4-((5S,5aS,6S,9R)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hept-2-methyl)naphthalen-2-amine (Z732)

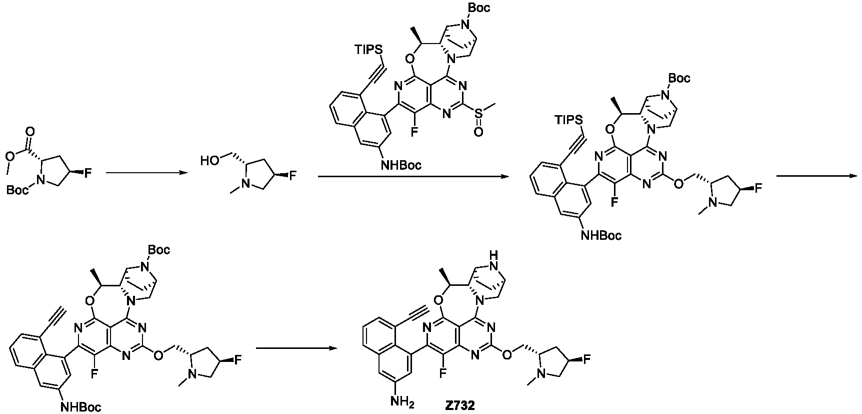

Step I: Under a nitrogen atmosphere, 1-(t-butyl) 2-methyl (2S, 4R)-4-fluoropyrrolidin-1,2-dicarboxylate(1046 mg, 4.23 mmol) was dissolved in anhydrous tetrahydrofuran (25 mL), and the mixture was allowed to stand in an iced water bath. A lithium aluminium hydride solution (12.7 mL, 12.69 mmol, 1 M solution in tetrahydrofuran) was slowly added dropwise to the above solution. After that, the reaction solution was heated to room temperature and stirred overnight. The reaction was shown to be completed by LC/MS. The reaction solution was quenched with sodium sulfafe decahydrate, stirred at room temperature for 0.5 hr, and filtered. The filtrate was concentrated under reduced pressure, to obtain the crude product ((2S,4R)-4-fluoro-1-methylpyrrolidin-2-yl)methanol (563 mg). ES-API: [M+H]⁺= 134.2.

Step II: ((2S,4R)-4-fluoro-1-methylpyrrolidin-2-yl)methanol (28 mg, 0.208 mmol) was dissolved in anhydrous tetrahydrofuran (5 mL). In an iced water bath, sodium hydride (12.5 mg, 0.312 mmol) was added, and reacted for 10 min in the iced water bath. A solution of ((2S,4R)-4-fluoro-1-methylpyrrolidin-2-yl)methanol t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (92 mg, 0.104 mmol) in tetrahydrofuran (5 mL) was added dropwise to the reaction solution, and reacted at 0°C for 30 min. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure, to obtain the crude product t-butyl (5S,5aS,6S,9R)-2-(3-((t-butoxycarbonyl)amino)-8-((triisopropylsilyl)ethynyl)naphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (99 mg). ES-API: [M+ H]⁺= 954.4.

Step III: Cesium fluoride (158 mg, 1.037 mmol) was added to t-butyl (5S,5aS,6S,9R)-2-(3-((t-butoxycarbonyl)amino)-8-((triisopropylsilyl)ethynyl)naphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (99 mg, 0.104 mmol) in N,N-dimethylformamide (5 mL), and the reaction mixture was stirred at room temperature for 1 hr. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (82 mg). ES-API: [M+ H]⁺= 798.1.

Step IV: In an iced water bath, a 4 M hydrochloric acid-dioxane solution (10 mL) was slowly added to a solution of t-butyl (5S,5aS,6S,9R)-2-(3-((t-butoxycarbonyl)amino)-8-ethynylnaphthalen-1-yl)-1-fluoro-12-(((2S, 4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (82 mg, 0.103 mmol) in methanol (1 mL). The reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 5-ethynyl-4-((5S,5aS,6S,9R)-1-fluoro-12-(((2S,4R)-4-fluoro-1-methylpyrrolidin-2-yl)methoxy)-5-methyl-5a,6,7,8,9,10-hexahydro-5H-4-oxa-3,10a,11,13,14-pentaaza-6,9-methanonaphtho[1,8-ab]hept-2-yl)naphthalen-2-amine (Z732, 7.59 mg, yield: 12%) as a white solid. ES-API: [M+H]⁺= 598.0. H NMR (400 MHz, CD₃OD) δ 7.69- 7.66 (m, 1 H), 7.41- 7.35 (m, 1 H), 7.32- 7.26 (m, 1 H), 7.14- 7.00 (m, 2 H), 5.41- 5.35 (m, 1 H), 5.27- 5.10 (m, 1 H), 4.54- 4.45 (m, 3 H), 4.10- 4.07 (m, 1 H), 3.70- 3.69 (m, 1 H), 3.62- 3.60 (m, 1 H), 3.57- 3.46 (m, 1 H), 3.21- 3.13 (m, 2 H), 2.72- 2.61 (m, 1 H), 2.56 (m, 3 H), 2.35- 2.25 (m, 1 H), 2.09- 1.77 (m, 6 H), 1.57 (t, J1= 6.8 Hz, J2= 13.2 Hz, 3 H).

### Example 120: Synthesis of 4-((5S,5aS,6S,9R)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynylnaphthalen-2-amine (Z733)

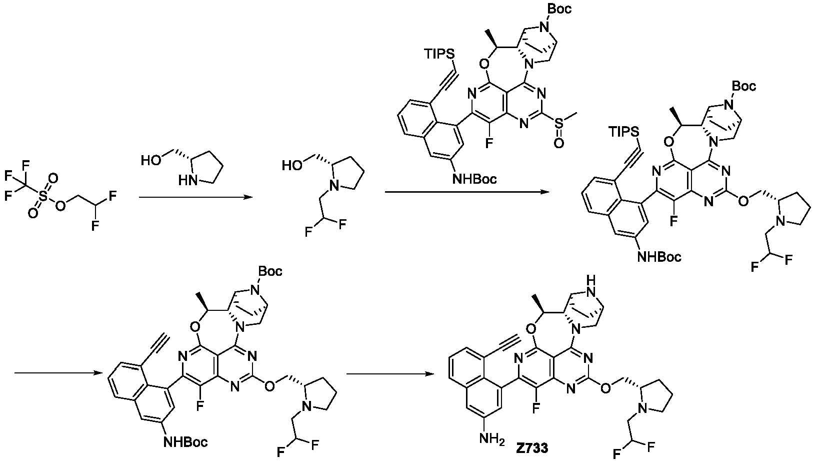

Step I: (S)-pyrrolidin-2-ylmethanol (505 mg, 4.993 mmol) was dissolved in acetonitrile (10 mL), and the mixture was allowed to stand in an iced water bath. The reaction solution was added with 2,2-difluoroethyl trifluoromethansulfonate (1.1 g, 5.138 mmol) and potassium carbonate (760 mg, 5.499 mmol). After that, the reaction solution was stirred at 0°C for 1 h, heated to room temperature and stirred for 3 hrs. The reaction was shown to be completed by LC/MS. The reaction solution was quenched with water (100 mL), and extracted with ethyl acetate (30 mL* 3). The organic phases were combined, dried over anhydrous sodium sulfate, filtered, and concentrated under reduced pressure, to obtain the crude product (S)-(1-(2,2-difluoroethyl)pyrrolidin-2-yl)methanol (600 mg, yield: 73%). ES-API: [M+H]⁺= 166.1.

Step II: (S)-(1-(2,2-difluoroethyl)pyrrolidin-2-yl)methanol (34 mg, 0.208 mmol) was dissolved in anhydrous tetrahydrofuran (5 mL). In an iced water bath, sodium hydride (12.5 mg, 0.312 mmol) was added, and reacted for 10 min in the iced water bath. A solution of ((2S, 4R)-4-fluoro-1-methylpyrrolidin-2-yl)methanol t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (92 mg, 0.104 mmol) in tetrahydrofuran (5 mL) was added dropwise to the reaction solution, and reacted for 30 min at 0 °C. The reaction was shown to be completed by LC/MS. The reaction solution was diluted with ethyl acetate (10 mL), washed with a saturated ammonium chloride solution (10 mLX2) and saturated brine (10 mLX 3), dried over anhydrous sodium sulfate, filtered, and concentrated under reduced pressure, to obtain the crude product t-butyl (5S,5aS,6S,9R)-2-(3-((t-butoxycarbonyl)amino)-8-((triisopropylsilyl)ethynyl)naphthalen-1-yl)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (102 mg). ES-API: [M+ H]⁺= 986.4.

Step III: Cesium fluoride (157 mg, 1.034 mmol) was added to t-butyl (5S,5aS,6S,9R)-2-(3-((t-butoxycarbonyl)amino)-8-((triisopropylsilyl)ethynyl)naphthalen-1-yl)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (102 mg, 0.103 mmol) in N,N-dimethylformamide (5 mL), and the reaction mixture was stirred at room temperature for 1 hr. The reaction was shown to be completed by LC/MS. The reaction solution was diluted with ethyl acetate (20 mL), washed with (15 mLX2) and saturated brine (15 mLX 3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-(((S)-1-(2,2-difluoroethyl) pyrrolidin-2-yl)methoxy)-1-fluoro-5-methyl-5a, 6,7,8,9,10-hexahydro-5H-4-oxa-3,10a, 11,13,14-pentaaza-6,9-methanonaphtho[1,8-ab]hepten-14-carboxylate (85 mg). ES-API: [M+ H]⁺= 830.0.

Step IV: In an iced water bath, a 4 M hydrochloric acid-dioxane solution (10 mL) was slowly added to a solution of t-butyl (5S,5aS,6S,9R)-2-(3-((t-butoxycarbonyl)amino)-8-ethynylnaphthalen-1-yl)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (85 mg, 0.102 mmol) in methanol (1 mL), and the reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-(((S)-1-(2,2-difluoroethyl)pyrrolidin-2-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5-ethynylnaphthalen-2-amine (Z733, 12.01 mg, yield: 19%) as a white solid. ES-API: [M+H]⁺= 630.1. ¹H NMR (400 MHz, CD₃OD) δ 7.70- 7.66 (m, 1 H), 7.42- 7.27 (m, 2 H), 7.14 (d, J= 2.4 Hz, 1 H), 7.11- 7.00 (m, 1 H), 6.08- 5.78 (m, 1 H), 5.52- 5.48 (m, 1 H), 4.64- 4.57 (m, 1 H), 4.48-4.43 (m, 1 H), 4.37- 4.32 (m, 1 H), 4.22- 4.19 (m, 1 H), 4.06- 4.05 (m, 1 H), 3.96- 3.94 (m, 1 H), 3.48- 3.36 (m, 2 H), 3.26- 3.21 (m, 1 H), 3.13- 3.07 (m, 1 H), 2.91- 2.83 (m, 1 H), 2.54- 2.47 (m, 1 H), 2.35- 2.19 (m, 1 H), 2.07-1.70 (m, 8 H), 1.58 (t, J1= 6.8 Hz, J2= 13.2 Hz, 3 H).

### Example 121: Synthesis of 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z734) and 4-((5S,5aS,6S,9R)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z735)

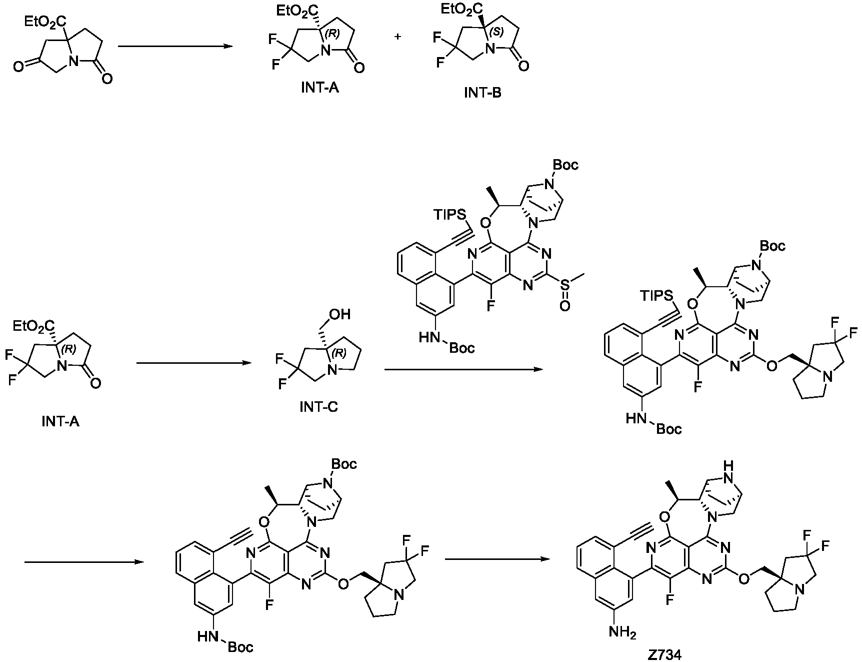

Step I: In an iced water bath, diethylaminosulfur trifluoride (1.9 mL, 14.2 mmol) was slowly added to a solution of ethl 2,5-dioxotetrahydro-1H-pyrrolizin-7a(5H)-carboxylate (1 g, 4.73 mmol) in dichloromethane (15 mL). The ice bath was removed. The reaction was stirred at room temperature for 16 hrs, In an iced water bath, the reaction solution was quenched with saturated sodium bicarbonate, and extracted with ethyl acetate. The organic phase was dried over anhydrous sodium sulfate, and filtered. The filtrate was concentrated. The residue was purified by flash silica gel column chromatography (ethyl acetate /petroleum ether: 0-40%) to obtain a racemate (800mg). The racemate was chirally resolved (chromatographic column: Daicel CHIRALPAK^{®} IG 250*4.6 mm, 5µM; mobile phase: ethanol: n-hexane=30:70; flow rate:1 mL/min; column temperature: 30°C) to obtain two isomers. One isomer was designated as ethyl (R)-2,2-difluoro-5-oxotetrahydro-1H-pyrrolizin-7a(5H)-carboxylate (INT-A, 270 mg, peak 1, retention time=7.419 min) as a white solid. ES-API:[M+H]⁺=234.1. The other isomer was designated as ethyl (S)-2,2-difluoro-5-oxotetrahydro-1H-pyrrolizin-7a(5H)-carboxylate (INT-B, 220 mg, peak 2, retention time=12.34 min) as a white solid. ES-API:[M+H]⁺=234.1.

Step II: In an iced water bath, a 1MLithium aluminium hydride solution in tetrahydrofuran (4.63 mL, 4.63 mmol) was slowly added to a solution of ethyl (R)-2,2-difluoro-5-oxotetrahydro-1H-pyrrolizin-7a(5H)-carboxylate (INT-A, 270 mg, 1.16 mmol) in tetrahydrofuran (5 mL). The ice bath was removed. The reaction was stirred at 65°C for 5 hrs. The reaction solution was cooled to 0°C. A 15%sodium hydroxide aqueous solution (0.1 mL) was added dropwise. The reaction solution was filtered through diatomite. The filtrate was concentrated to obtain (R)-(2,2-difluorotetrahydro-1H-pyrrolizin-7a(5H)-yl)methanol (130 mg, yield: 63%). ES-API:[M+Na]⁺ = 178.0.

Step III: (R)-(2,2-difluorotetrahydro-1H-pyrrolizin-7a(5H)-yl)methanol (50 mg, 0.28 mmol) was dissolved in anhydrous tetrahydrofuran (6 mL). In an iced water bath, sodium hydride (24 mg, 0.59 mmol) was added, and reacted at room temperature for 5 min. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab] hepten-14-carboxylate (150 mg, 0.17 mmol) in tetrahydrofuran (2 mL) was added dropwise to the reaction solution, and reacted at room temperature for 30 min. The reaction solution was quenched with water (30 mL), extracted with ethyl acetate (30 mLX2), washed with saturated brine (60 mL), dried over anhydrous sodium sulfate, filtered, and concentrated. The crude product was purified by flash silica gel column chromatography (ethyl acetate /petroleum ether: 0-30%) to obtain t-butyl (5S,5aS,6S,9R)-2-(3-((t-butoxycarbonyl)amino)-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100mg, yield: 59%). ES-API: [M+H]⁺= 998.2.

Step IV: Cesium fluoride (152 mg, 1 mmol) was added to a solution of t-butyl (5S,5aS,6S,9R)-2-(3-((t-butoxycarbonyl)amino)-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.1 mmol) in N,N-dimethylformamide (3 mL), and the reaction mixture was stirred at room temperature for 1 hr. The reaction was shown to be completed by LC/MS. The reaction solution was diluted with ethyl acetate (30 mL), washed with saturated brine (30mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methylmethanonaphtho[1,8-ab]hepten-14-carboxylate (80mg, crude product). ES-API: [M+H]⁺= 842.0.

Step V: In an iced water bath, a 4 M hydrochloric acid-dioxane solution (3 mL) was slowly added to a solution of t-butyl (5S,5aS,6S,9R)-2-(3-((t-butoxycarbonyl)amino)-8-ethynylnaphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (80mg, crude product) in acetonitrile (2 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate method 1) to obtain 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z734, 15 mg, yield: 24%). ES-API: [M+H]⁺= 642.2.

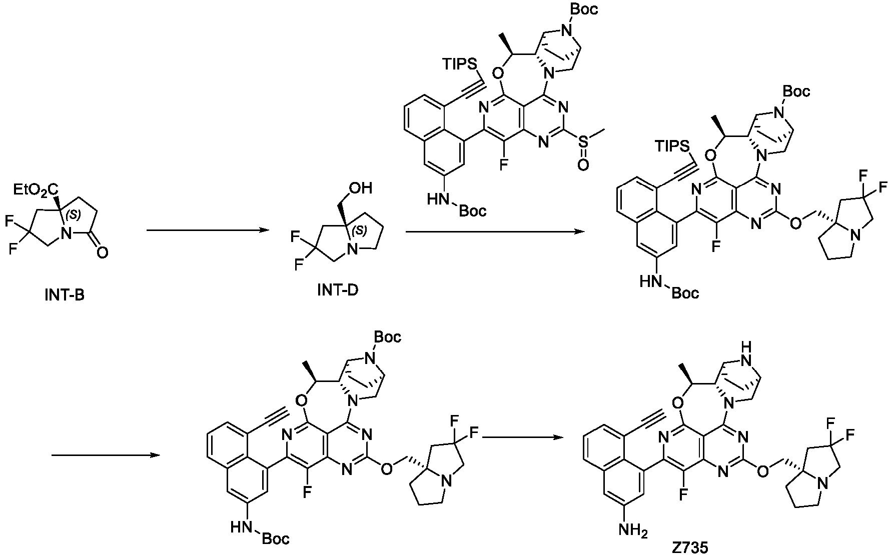

Step I: In an iced water bath, a 1MLithium aluminium hydride solution in tetrahydrofuran (3.77 mL, 3.77 mmol) was slowly added to a solution of ethyl (S)-2,2-difluoro-5-oxotetrahydro-1H-pyrrolizin-7a(SH)-carboxylate (INT-B, 220 mg, 0.94 mmol) in tetrahydrofuran (5 mL). The ice bath was removed. The reaction was stirred at 65°C for 5 hrs. The reaction solution was cooled to 0°C. A 15%sodium hydroxide aqueous solution (0.1 mL) was added dropwise. The reaction solution was filtered through diatomite. The filtrate was concentrated to obtain (S)-(2,2-difluorotetrahydro-1H-pyrrolizin-7a(5H)-yl)methanol (100 mg, yield: 59%). ES-API:[M+Na]⁺ = 178.0.

Step II: (S)-(2,2-difluorotetrahydro-1H-pyrrolizin-7a(5H)-yl)methanol (50 mg, 0.28 mmol) was dissolved in anhydrous tetrahydrofuran (6 mL). In an iced water bath, sodium hydride (24 mg, 0.59 mmol) was added, and reacted at room temperature for 5 min. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab] hepten-14-carboxylate (150 mg, 0.17 mmol) in tetrahydrofuran (2 mL) was added dropwise to the reaction solution, and reacted at room temperature for 30 min. The reaction solution was quenched with water (30 mL), extracted with ethyl acetate (30 mL*2), washed with saturated brine (60 mL), dried over anhydrous sodium sulfate, filtered, and concentrated. The crude product was purified by flash silica gel column chromatography (ethyl acetate /petroleum ether: 0-30%) to obtain t-butyl (5S,5aS,6S,9R)-2-(3-((t-butoxycarbonyl)amino)-8-((triisopropylsilyl)ethynyl)naphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (40mg, yield: 23%). ES-API: [M+H]⁺= 998.2.

Step III: Cesium fluoride (61 mg, 0.4 mmol) was added to t-butyl (5S,5aS,6S,9R)-2-(3-((t-butoxycarbonyl)amino)-8-((triisopropylsilyl)ethynyl)naphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (40 mg, 0.04 mmol) in N,N-dimethylformamide (2 mL), and the reaction mixture was stirred at room temperature for 1 hr. The reaction was shown to be completed by LC/MS. The reaction solution was diluted with ethyl acetate (30 mL), washed with saturated brine (30mLX 3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (30mg, crude product). ES-API: [M+H]⁺= 842.0.

Step IV: In an iced water bath, a 4 M hydrochloric acid-dioxane solution (2 mL) was slowly added to a solution of t-butyl (5S,5aS,6S,9R)-2-(3-((t-butoxycarbonyl)amino)-8-ethynylnaphthalen-1-yl)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methylnaphtho[1,8-ab]hepten-14-carboxylate (30mg, crude product) in acetonitrile (2 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate method 1) to obtain 4-((5S,5aS,6S,9R)-12-(((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z735, 5 mg, yield: 21%). ES-API: [M+H]⁺= 642.2. ¹HNMR(400MHz,CD₃OD): 7.69-7.65 (m,1H), 7.41-7.26 (m,2H), 7.13-6.99 (m,2H), 5.40-5.36 (m,1H), 4.57-4.48 (m,1H), 4.30-4.22 (m,2H), 4.09-4.06 (m,1H), 3.71-3.61 (m,2H), 3.48-3.38 (m,1H), 3.21-2.99 (m,4H), 2.90-2.75 (m,1H), 2.65-2.50 (m,1H), 2.38-2.28 (m,1H), 2.20-1.75 (m,8H), 1.58-1.55 (m,3H).

### Example 122: Synthesis of (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z648)

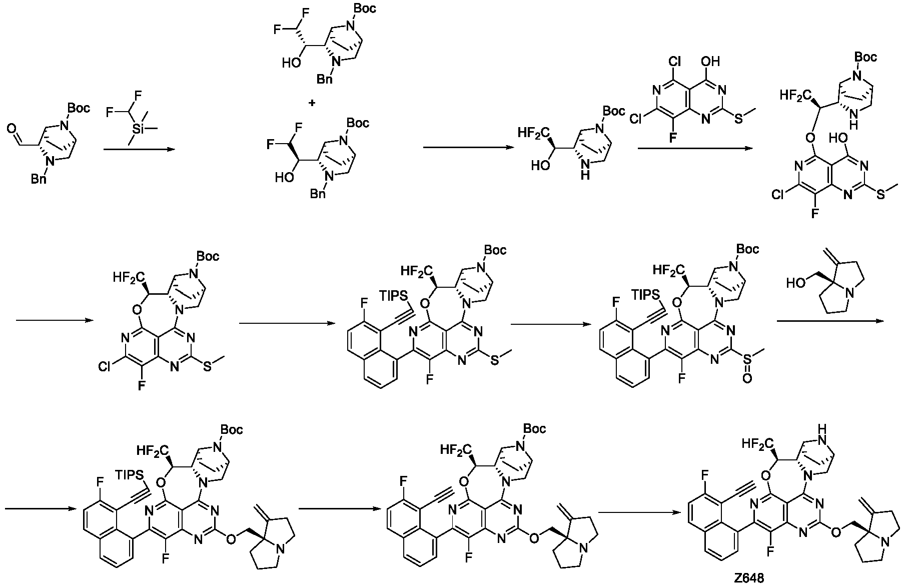

Step I: T-butyl (1S,2S,5R)-3-benzyl-2-formyl-3,8-diazabicyclo[3.2.1]octane-8-carboxylate (1.15 g, 3.48 mmol) and (difluoromethyl)trimethylsilane (1.08 g, 8.70 mmol) were dissolved in N, N-dimethylformamide (10 mL). At 0°C, cesium fluoride (159 mg, 1.04 mmol) was added, and the reaction was stirred at room temperature for 18 hrs. A 1.0 M tetrabutylammonium fluoride solution in tetrahydrofuran (3.48 mL, 3.48 mmol) was added, and the reaction was continuously stirred at room temperature for 1 hr. The reaction solution was quenched with water (10 mL), and extracted with ethyl acetate (40 mL). The organic phase was washed with saturated brine (15 mL×3), dried over anhydrous sodium sulfate, and concentrated. The crude product was purified by flash silica gel column chromatography (tetrahydrofuran/petroleum ether: 0-10%) to obtain t-butyl (1S,2S,5R)-3-benzyl-2-((S)-2,2-difluoro-1-hydroxyethyl)-3,8-diazabicyclo[3.2.1]octane-8-carboxylate (isomer 1, 470 mg, yield: 35.3%) as a colorless liquid, ES-API: [M+H]⁺= 383.0 (column: X-Bridge C18, 4.6*50mm,3.5um; mobile phase system: A: 10mmol ammonium bicarbonate aqueous solution; B: preparative separation-grade acetonitrile; flow rate: 1.7mL/min; B%=10%-95%; column temperature: 40°C; retention time: 1.74min) and t-butyl (1S,2S,5R)-3-benzyl-2-((R)-2,2-difluoro-1-hydroxyethyl)-3,8-diazabicyclo[3.2.1]octane-8-carboxylate (isomer 2, 700 mg, yield: 52.6%) as a colorless liquid, ES-API: [M+H]⁺= 383.0 (column: X-Bridge C18, 4.6*50mm,3.5um; mobile phase system: A: 10mmol ammonium bicarbonate aqueous solution; B: preparative separation-grade acetonitrile; flow rate: 1.7mL/min; B%=10%-95%; column temperature: 40°C; retention time: 1.71min).

Step II: T-butyl (1S,2S,5R)-3-benzyl-2-((R)-2,2-difluoro-1-hydroxyethyl)-3,8-diazabicyclo [3.2.1]octane-8-carboxylate (760 mg, 1.99 mmol) was dissolved in methanol (15 mL) and a 7M aminomethanol solution (1 mL). 10% palladium on carbon (150 mg) was added, and the reaction was stirred under a hydrogen atmosphere at room temperature for 18 hrs. The reaction solution was filtered through diatomite and the filter cake was washed with methanol. The filtrate was concentrated to obtain the target product t-butyl (1S,2S,5R)-2-((R)-2,2-difluoro-1-hydroxyethyl)-3,8-diazabicyclo[3.2.1]octane-8-carboxylate (580 mg, yield: 99.8%) as a colorless liquid. ES-API: [M+H]⁺= 293.0

Step III: T-butyl (1S,2S,5R)-2-((R)-2,2-difluoro-1-hydroxyethyl)-3,8-diazabicyclo[3.2.1] octane-8-carboxylate (550 mg, 1.88 mmol) was dissolved in tetrahydrofuran (30 mL). At 0 °C, 60% sodium hydride (376 mg, 9.41 mmol) was added. The reaction was stirred at 0 °C for 30 min. 5,7-dichloro-8-fluoro-2-(methylthio)pyrido[4,3-d]pyrimidine-4(3H)-ol (632 mg, 2.26 mmol) was added, and the reaction was stirred at room temperature for 18 hrs. The reaction solution was quenched with a saturated ammonium chloride solution (2 mL) and water (20 mL), and extracted with ethyl acetate (100 mL). The organic phases were combined and washed with saturated brine (30 mL). The organic phase was dried over anhydrous sodium sulfate, and concentrated to obtain the target product t-butyl (1R,2S,5S)-2-((R)-1-((7-chloro-8-fluoro-4-hydroxyl-2-(methylthio) pyrido[4,3-d]pyrimidine-5-yl)oxy)-2,2-difluoroethyl)-3,8-diazabicyclo[3.2.1]octane-8-carboxylate (1.0g, yield: 99.2%) as a light yellow solid. ES-API: [M+H]⁺= 535.9

Step IV: T-butyl (1R,2S,5S)-2-((R)-1-((7-chloro-8-fluoro-4-hydroxyl-2-(methylthio)pyrido [4,3-d]pyrimidine-5-yl)oxy)-2,2-difluoroethyl)-3,8-diazabicyclo[3.2.1]octane-8-carboxylate (1.0 g, 1.87 mmol) and diisopropylethyl amine (1.21 g, 9.33 mmol) were dissolved in dichloromethane (25 mL). At room temperature, a 50% 1-propylphosphoric anhydride solution in ethyl acetate (3.56 g, 5.60 mmol) was added, and the reaction was stirred at room temperature for 18 hrs. The reaction solution was added with dichloromethane (50 mL), sequentially washed with water (25 mL×2) and saturated brine (25 mL), dried over anhydrous sodium sulfate, filtered and concentrated. The crude product was purified by flash silica gel column chromatography (tetrahydrofuran/petroleum ether: 0-20%) to obtain the target product t-butyl (5R,5aS,6S, 9R)-2-chloro-5-(difluoromethyl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (435 mg, yield: 45.0%) as a light orange solid. ES-API: [M+H]⁺= 517.9

Step V: T-butyl (5R,5aS,6S,9R)-2-chloro-5-(difluoromethyl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (130 mg, 0.25 mmol), ((2-fluoro-8-(4,4,5,5-tetramethyl-1,3,2-dioxaborolan-2-yl)naphthalen-1-yl)ethynyl)triisopropylsilane (182 mg, 0.40 mmol), potassium phosphate (160 mg, 0.75 mmol), [n-butyldi(1-adamantanyl)phosphine](2-amino-1,1'-biphenyl-2-yl)palladium (II) mesylate (18 mg, 0.025 mmol), tetrahydrofuran(2.5 mL) and water (0.5 mL) were added to a 5 mL microwave tube, and purged with nitrogen for 30 sec. The reaction was stirred at 80 °C in a microwave generator for 30 min. The reaction solution was added with ethyl acetate (40 mL) and water (10 mL). The organic phase was separated, washed with saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-30%) to obtain the target product t-butyl (5R,5aS,6S,9R)-5-(difluoromethyl)-1-fluoro-2-(7-fluoro-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (200 mg, yield: 98.6%) as a light yellow solid. ES-API: [M+H]⁺=808.2.

Step VI: T-butyl (5R,5aS,6S,9R)-5-(difluoromethyl)-1-fluoro-2-(7-fluoro-8-((triisopropylsilyl) ethynyl)naphthalen-1-yl)-12-(methylthio)-5a,6,7,8,9,10-hexahydro-5H-4-oxy-3,10a,11,13,14-pentaaza-6,9-methanonaphtho[1,8-ab]hepten-14-carboxylate (200 mg, 0.25 mmol) was dissolved in dichloromethane (15 mL). At room temperature, m-chloroperoxybenzoic acid (65 mg, 0.32 mmol) was added, and the reaction was stirred for 1 hr in an ice bath. The reaction solution was added with dichloromethane (30 mL), and sequentially washed with saturated sodium thiosulphate (2 mL), saturated sodium bicarbonate (15 mL), and saturated brine (15 mL). The organic phase was dried over anhydrous sodium sulfate, and filtered. The filtrate was concentrated under reduced pressure, to obtain the target product t-butyl (5R,5aS,6S,9R)-5-(difluoromethyl)-1-fluoro-2-(7-fluoro-8-((triisopropylsilyl)ethynyl)naphthalen-1-yl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (195 mg,yield: 95.6%) as a light yellow solid. ES-API:[M+H]⁺=824.2.

Step VII: (R)-(1-methylenetetrahydro-1H-pyrrolizin-7A(5H)-yl)methanol (73 mg, 0.47 mmol) was dissolved in tetrahydrofuran(10 mL). At 0°C, 60% sodium hydride (57 mg, 1.42 mmol) was added, and the reaction was stirred at this temperature for 30 min. Then, a solution of t-butyl (5R,5aS,6S,9R)-5-(difluoromethyl)-1-fluoro-2-(7-fluoro-8-((triisopropylsilyl)ethynyl)naphthalen -1-yl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (195 mg, 0.24 mmol) in tetrahydrofuran (3 mL) was added dropwise. The reaction was stirred for 30 min in the ice bath. The reaction solution was quenched with a saturated ammonium chloride solution (2 mL) and water (5 mL), and extracted acetate (40 mL). The organic phase was washed with saturated brine (15 mL), dried over anhydrous sodium sulfate, and filtered. The filtrate was concentrated under reduced pressure,. The crude product was purified by flash silica gel column chromatography (methanol /dichloromethane: 0-5%) to obtain the target product t-butyl (5R,5aS,6S,9R)-5-(difluoromethyl)-1-fluoro-2-(7-fluoro-8-((triisopropylsilyl)ethynyl)naphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (100 mg, yield: 46.3%) as a light brown solid. ES-API: [M+H]⁺=913.3.

Step VIII: T-butyl (5R,5aS,6S,9R)-5-(difluoromethyl)-1-fluoro-2-(7-fluoro-8-((triisopropylsilyl)ethynyl)naphthalen-1-yl)-12-(R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (100 mg, 0.11 mmol) was dissolved in N,N-dimethylformamide (5 mL). At room temperature, cesium fluoride (166 mg, 1.10 mmol) was added, and the reaction was stirred at room temperature for 1 hr. The reaction solution was added with ethyl acetate (50 mL), and sequentially washed with water (15 mL), dilute saline (15 mL×3), and saturated brine (15 mL). The organic phase was dried over anhydrous sodium sulfate, and filtered. The filtrate was concentrated under reduced pressure, to obtain the target product t-butyl (5R,5aS,6S,9R)-5-(difluoromethyl)-2-(8-ethynyl7-fluoronaphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (82 mg, yield: 98.9%) as a light brown solid. ES-API: [M+H]⁺=757.2.

Step IX: T-butyl (5R,5aS,6S,9R)-5-(difluoromethyl)-2-(8-ethynyl7-fluoronaphthalen-1-yl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (77 mg, 0.10 mmol) was dissolved in acetonitrile (2 mL). At 0°C, 4 M hydrogen chloride in 1, 4-dioxane (2 mL) was added, and the reaction was stirred at room temperature for 2 hrs. The reaction solution was concentrated under reduce pressure. The crude product was purified by preparative HPLC (formic acid method 1) to obtain the target product (5R,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho [1,8-ab]heptene (Z648, 28 mg, yield: 36.7%, formate) as a light yellow solid. ES-API: [M+H]⁺= 657.2 ¹H NMR (400 MHz, CD₃OD) δ 8.39 (s, 2H), 8.18 - 8.05 (m, 2H), 7.71 - 7.53 (m, 2H), 7.50 - 7.38 (m, 1H), 6.59 - 6.29 (m, 1H), 5.66 - 5.14 (m, 3H), 5.04 - 4.92 (m, 1H), 4.85 - 4.42 (m, 4H), 4.02 - 3.48 (m, 5H), 3.42 - 3.21 (m, 2H), 3.01 - 2.90 (m, 1H), 2.54 - 1.76 (m, 9H).

### Example 123: Synthesis of (5S,5aS,6S,9R)-5-(difluoromethyl)-2-(8-ethynyl-7-fluoronaphthalen-1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z736)

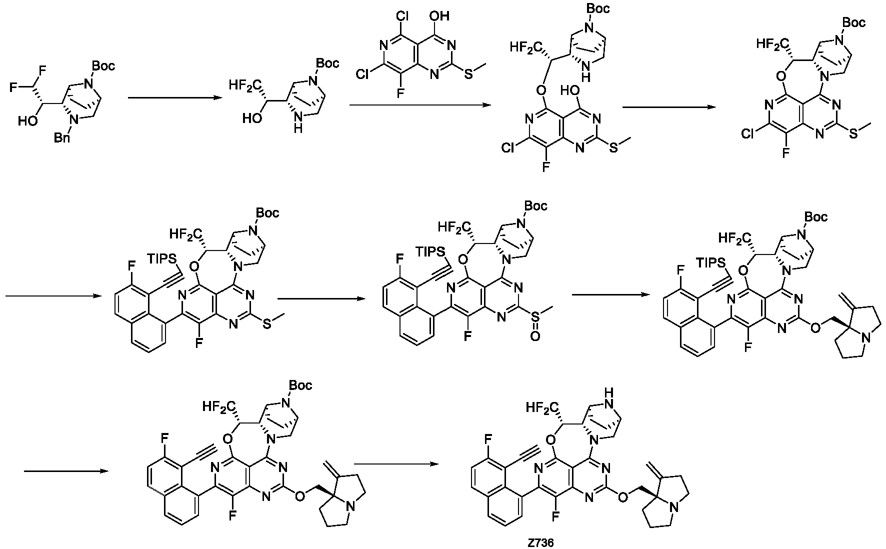

Step I: T-butyl (1S,2S,5R)-3-benzyl-2-((S)-2,2-difluoro-1-hydroxyethyl)-3,8-diazabicyclo [3.2.1]octane-8-carboxylate (470 mg, 1.23 mmol) was dissolved in methanol (10 mL) and a 7 M aminomethanol solution (0.7 mL). 10% palladium on carbon (100 mg) was added, and the reaction was stirred under a hydrogen atmosphere at room temperature for 18 hrs. The reaction solution was filtered through diatomite and the filter cake was washed with methanol. The filtrate was concentrated to obtain the target product t-butyl (1S,2S,5R)-2-((S)-2,2-difluoro-1-hydroxyethyl)-3,8-diazabicyclo[3.2.1]octane-8-carboxylate (350 mg, yield: 97.4%) as a colorless liquid. ES-API: [M+H]⁺= 293.0

Step II: T-butyl (1S,2S,5R)-2-((S)-2,2-difluoro-1-hydroxyethyl)-3,8-diazabicyclo[3.2.1] octane-8-carboxylate (350 mg,1.20 mmol) was dissolved in tetrahydrofuran (25 mL). At 0 °C, 60% sodium hydride (240 mg, 5.99 mmol) was added, and the reaction was stirred at 0 °C for 30 min. Then 5,7-dichloro-8-fluoro-2-(methylthio)pyrido[4,3-d]pyrimidine-4(3H)-one (402 mg, 1.44 mmol) was added, and the reaction was stirred at room temperature for 18 hrs. The reaction solution was quenched with a saturated ammonium chloride solution (2 mL) and water (20 mL), and extracted with ethyl acetate (100 mL). The organic phases were combined and washed with saturated brine (30 mL). The organic phase was dried over anhydrous sodium sulfate, filtered and concentrated. The crude product was purified by flash silica gel column chromatography (tetrahydrofuran/petroleum ether: 0-50%) to obtain the target product t-butyl (1R,2S,5S)-2-((S)-1-((7-chloro-8-fluoro-4-hydroxyl-2-(methylthio)pyrido[4,3-d]pyrimidine-5-yl)oxy)-2,2-difluoroethyl)-3,8-diazabicyclo[3.2.1]octane-8-carboxylate (500 mg,yield: 77.9%) as a light yellow solid. ES-API: [M+H]⁺= 535.9

Step III: T-butyl (1R,2S,5S)-2-((S)-1-((7-chloro-8-fluoro-4-hydroxyl-2-(methylthio)pyrido [4,3-d]pyrimidine-5-yl)oxy)-2,2-difluoroethyl)-3,8-diazabicyclo[3.2.1]octane-8-carboxylate (500 mg, 0.93 mmol) and diisopropylethyl amine (603 mg, 4.66 mmol) were dissolved in dichloromethane (15 mL). At room temperature, a 50% 1-propylphosphoric anhydride solution in ethyl acetate (1.78 g, 2.80 mmol) was added, and the reaction was stirred at room temperature for 18 hrs. The reaction solution was added with dichloromethane (50 mL), sequentially washed with water (25 mL×2) and saturated brine (25 mL), dried over anhydrous sodium sulfate, filtered and concentrated. The crude product was purified by flash silica gel column chromatography (tetrahydrofuran/petroleum ether: 0-20%) to obtain the target product t-butyl (5S,5aS,6S,9R)-2-chloro-5-(difluoromethyl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (350 mg, yield: 72.4%) as a white solid. ES-API: [M+H]⁺= 517.9

Step IV: T-butyl (5S,5aS,6S,9R)-2-chloro-5-(difluoromethyl)-1-fluoro-12-(methylthio)-5a,6,7,8,9,10-hexahydro-5H-4-oxa-3,10a,11,13,14-pentaaza-6,9-methanonaphtho[1,8-ab]hepten-14-carboxylate (100 mg,0.19 mmol), ((2-fluoro-8-(4,4,5,5-tetramethyl-1,3,2-dioxaborolan-2-yl)naphthalen-1-yl)ethynyl)triisopropylsilane (140 mg,0.31 mmol), potassium phosphate (123 mg,0.58 mmol), [n-butyldi(1-adamantanyl)phosphine](2-amino-1,1'-biphenyl-2-yl)palladium (II) mesylate (14 mg,0.019 mmol), tetrahydrofuran (2.5 mL) and water (0.5 mL) were added to a 5 mL microwave tube, and purged with nitrogen for 30 sec. The reaction was stirred at 80 °C in a microwave reactor for 30 min. The reaction solution was added with ethyl acetate (40 mL) and water (10 mL). The organic phase was separated, washed with saturated brine (10 mL), dried over anhydrous sodium sulfate, and filtered. The filtrate was concentrated under reduced pressure. The crude product was purified by flash silica gel column chromatography (tetrahydrofuran/petroleum ether: 0-30%) to obtain the target product t-butyl (5S,5aS,6S,9R)-5-(difluoromethyl)-1-fluoro-2-(7-fluoro-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (150 mg, yield: 96.1%) as a light yellow solid. ES-API: [M+H]⁺=808.2.

Step V: T-butyl (5S,5aS,6S,9R)-5-(difluoromethyl)-1-fluoro-2-(7-fluoro-8-((triisopropylsilyl) ethynyl)naphthalen-1-yl)-12-(methylthio)-5a,6,7,8,9,10-hexahydro-5H-4-oxa-3,10a,11,13,14-pentaaza-6,9-methanonaphtho[1,8-ab]hepten-14-carboxylate (150 mg, 0.19 mmol) was dissolved in dichloromethane (15 mL). At room temperature, m-chloroperoxybenzoic acid (49 mg,0.24 mmol) was added, and the reaction was stirred for 1 hr in an ice bath. The reaction solution was added with dichloromethane (30 mL), and sequentially washed with saturated sodium thiosulphate (2 mL), saturated sodium bicarbonate (15 mL), and saturated brine (15 mL). The organic phase was dried over anhydrous sodium sulfate, and filtered. The filtrate was concentrated under reduced pressure, to obtain the target product t-butyl (5S,5aS,6S,9R)-5-(difluoromethyl)-1-fluoro-2-(7-fluoro-8-((triisopropylsilyl)ethynyl)naphthalen-1-yl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (150 mg,yield: 98.1%) as a light yellow solid. ES-API:[M+H]⁺=824.0.

Step VI: (R)-(1-methylenetetrahydro-1H-pyrrolizin-7a(5H)-yl)methanol (56 mg, 0.36 mmol) was dissolved in tetrahydrofuran (10 mL). At 0°C, 60% sodium hydride (44 mg, 1.09 mmol) was added, and the reaction was stirred at this temperature for 30 min. Then, a solution of t-butyl (5S,5aS,6S,9R)-5-(difluoromethyl)-1-fluoro-2-(7-fluoro-8-((triisopropylsilyl)ethynyl)naphthalen-1-yl)-12-(methylthionyl)-5a,6,7,8,9,10-hexahydro-5H-4-oxy-3,10a,11,13,14-pentaaza-6,9-methanonaphtho[1,8-ab]hepten-14-carboxylate (150 mg, 0.18 mmol) in tetrahydrofuran (3 mL) was added dropwise, and the reaction was stirred for 30 min in an ice bath. The reaction solution was quenched with a saturated ammonium chloride solution (2 mL) and water (5 mL), and extracted acetate (40 mL). The organic phase was washed with saturated brine (15 mL), dried over anhydrous sodium sulfate, and filtered. The filtrate was concentrated under reduced pressure. The crude product was purified by flash silica gel column chromatography (methanol /dichloromethane: 0-5%) to obtain the target product t-butyl (5S,5aS,6S,9R)-5-(difluoromethyl)-1-fluoro-2-(7-fluoro-8-((triisopropylsilyl)ethynyl)naphthalen-1-yl)-12-(R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (78 mg,yield: 46.9%) as a light brown solid. ES-API: [M+H]⁺=913.3.

Step VII: T-butyl (5S,5aS,6S,9R)-5-(difluoromethyl)-1-fluoro-2-(7-fluoro-8-((triisopropylsilyl)ethynyl)naphthalen-1-yl)-12-(R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (78 mg,0.085 mmol) was dissolved in N,N-dimethylformamide (4 mL). At room temperature, cesium fluoride (130 mg, 0.85 mmol) was added, and the reaction was stirred at room temperature for 1 hr. The reaction solution was added with ethyl acetate (50 mL), and sequentially washed with water (15 mL), dilute saline (15 mL×3), and saturated brine (15 mL). The organic phase was dried over anhydrous sodium sulfate, and filtered. The filtrate was concentrated under reduced pressure, to obtain the target product t-butyl (5S,5aS,6S,9R)-5-(difluoromethyl)-2-(8-ethynyl7-fluoronaphthalen-1-yl)-12-(R)-1-methylenetetrahydro- 1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (64 mg,yield: 99.0%) as a light brown solid. ES-API: [M+H]⁺=757.2.

Step VIII: T-butyl (5R,5aS,6S,9R)-5-(difluoromethyl)-2-(8-ethynyl7-fluoronaphthalen-1-yl)-12-(R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (64 mg,0.085 mmol) was dissolved in acetonitrile(1.5 mL). At 0°C, 4M hydrogen chloride in 1, 4-dioxane (1.5 mL) was added, and the reaction was stirred at room temperature for 2 hrs. The reaction solution was concentrated under reduce pressure. The crude product was purified by preparative HPLC (ammonium bicarbonate method 1) to obtain the target product (5S,5aS,6S,9R)-5-(difluoromethyl)-2-(8-ethynyl-7-fluoronaphthalen- 1-yl)-1-fluoro-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z736, 18 mg, yield: 32.4%) as a white solid. ES-API: [M+H]⁺= 657.2. ¹H NMR (400 MHz, CD₃OD) δ 8.15 - 8.05 (m, 2H), 7.67 - 7.54 (m, 2H), 7.47 - 7.39 (m, 1H), 6.42 (td, *J =* 53.2, 7.2 Hz, 1H), 5.45 - 5.06 (m, 3H), 4.79 - 4.55 (m, 1H), 4.46 - 4.25 (m, 2H), 4.06 - 3.89 (m, 2H), 3.83 - 3.71 (m, 2H), 3.56 - 3.42 (m, 1H), 3.28 - 3.10 (m, 2H), 2.88 - 2.63 (m, 3H), 2.25 - 1.68 (m, 9H).

### Example 124: Synthesis of 5-chloro-4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)naphthalen-2-amine (Z737)

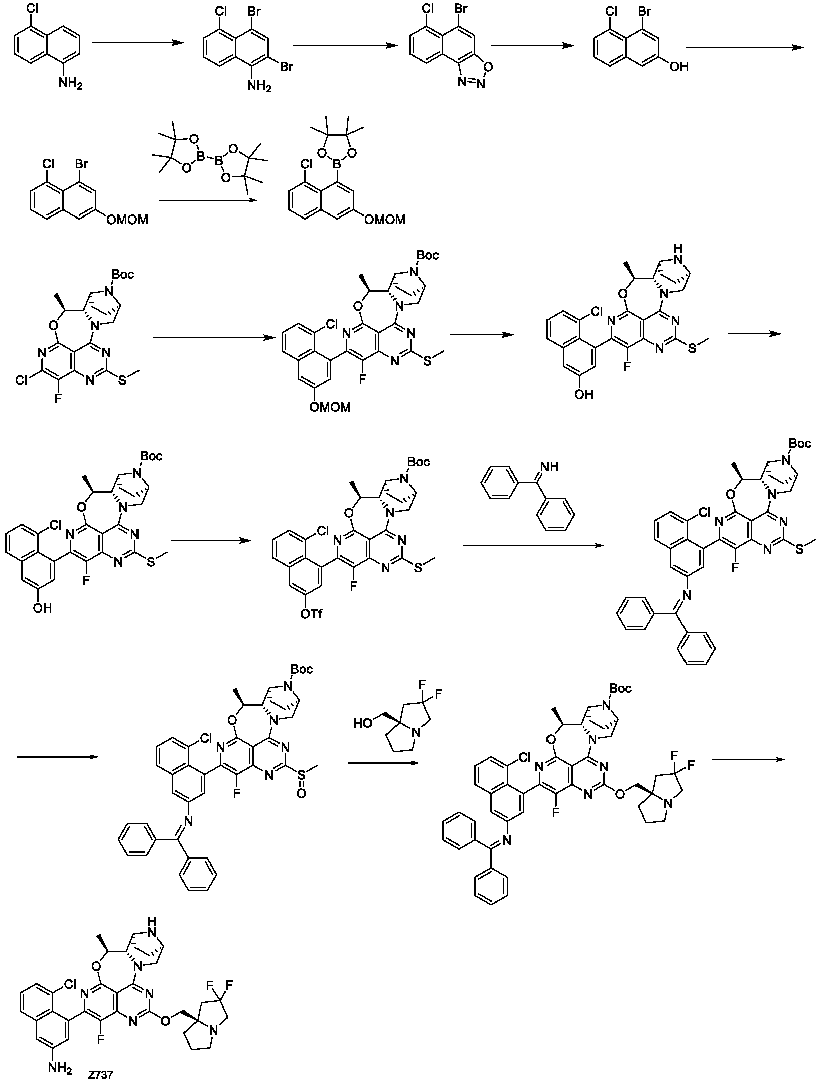

Step I: 5-chloronaphthalen-1-amine (6.2 g, 34.904 mmol) was dissolved in acetic acid (150 mL). A solution of bromine (4.217 mL, 76.789 mmol) in acetic acid (150 mL) was added, reacted at 70°C for 4 hrs, cooled to room temperature, and filtered under suction. The solid was dissolved in 20% sodium hydroxide solution (600 mL), stirred at room temperature for 20 min, and filtered under suction. The obtained solid was dissolved in dichloromethane (50 mL). The organic phase was water (30 mL) and a sodium chloride solution (20 mL), dried over anhydrous sodium sulfate, filtered under suction, and concentrated to obtain 2,4-dibromo-5-chloronaphthalen-1-amine (9 g, 26.832 mmol, yield: 76.87%). ES-API: [M+H]⁺=335.2.

Step II: 2,4-dibromo-5-chloronaphthalen-1-amine (4.5 g, 13.416 mmol) was dissolved in acetic acid (87 mL) and propionic acid (14 mL). At 5°C, sodium nitrite (1.30 g, 18.782 mmol) was added, stirred for 1 hr, and filtered under suction. The material was poured into water (20 mL), and filtered under suction. The solid was dissolved in dichloromethane (50 mL). The organic phase was water (30 mL) and sodium chloride solution (20 mL), dried over anhydrous sodium sulfate, filtered under suction, and concentrated to obtain 5-bromo-6-chloronaphtho[1,2-d][1,2,3]oxadiazole(3.3 g, 11.640 mmol, yield: 86.76%). ES-API: [M+H]⁺=284.8.

Step III: 5-bromo-6-chloronaphtho[1,2-d][1,2,3]oxadiazole(1.2 g, 4.233 mmol) was dissolved in ethanol (14 mL). In an ice bath, sodium borohydride (0.32 g, 8.465 mmol) was added, reacted at room temperature for 2 hrs, filtered under suction, pressurized and concentrated. The residue was poured into water (25 mL), and extracted with dichloromethane(25 mL). The organic phase was washed with water (30 mL) and sodium chloride solution (20 mL), dried over anhydrous sodium sulfate, filtered under suction, and concentrated to obtain 4-bromo-5-chloronaphthalen-2-ol (0.8 g, 3.107 mmol, yield: 73.40%).

Step IV: 4-bromo-5-chloronaphthalen-2-ol (0.8 g, 3.107 mmol) was dissolved in dichloromethane. In an iced water bath, N,N-diisopropylethylene diamine (1706.54 mg, 13.203 mmol) and bromomethylmethyl ether (536.22 mg, 4.291 mmol) were added, reacted overnight at room temperature, and concentrated under reduced pressure. The crude product was purified by silica gel column chromatography (ethyl acetate: petroleum ether=95: 5) to obtain 1-bromo-8-chloro-3-(methoxymethoxy)naphthalene (300 mg, 0.995 mmol, yield: 30.14%) as a yellow solid.

Step V: 1-bromo-8-chloro-3-(methoxymethoxy)naphthalene (0.45 g, 1.492 mmol), bis(pinacolato)diboron (0.76 g, 2.984 mmol), [1,1'-bis(diphenylphosphino)ferrocene]palladium dichloride (0.11 g, 0.149 mmol), and potassium acetate (0.37 g, 3.731 mmol) were dissolved in 1,4-dioxane (8 mL), reacted under a nitrogen atmosphere at 95°C for 2 hrs, and concentrated under reduced pressure. The crude product was purified by silica gel column chromatography (dichloromethane: petroleum ether=25: 75) to obtain 2-(8-chloro-3-(methoxymethoxy)naphthalen-1-yl)-4,4,5,5-tetramethyl-1,3,2-dioxaborolane (0.22 g, 0.631 mmol, yield: 42.29%) as a white solid. ES-API: [M+H]⁺=349.0.

Step VI: 2-(8-chloro-3-(methoxymethoxy)naphthalen-1-yl)-4,4,5,5-tetramethyl-1,3,2-dioxaborolane (130.20 mg, 0.373 mmol) and t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (150 mg, 0.311 mmol) were dissolved in tetrahydrofuran (2 mL) and water (0.5 mL). Potassium phosphate (132.12 mg, 0.622 mmol), and (2-dicyclohexylphosphino-2',4',6'-triisopropyl-1,1'-biphenylyl)[2-(2'-amino-1,1'-biphenyl)] palladium (II) chloride (3.26 mg, 0.004 mmol) were added. After that, the reaction was stirred at 65°C for 15 hrs. After the reaction, the reaction solution was added with water (5 mL) and extracted with ethyl acetate (5 mLX3). The organic phase was washed with saturated brine (10 mL), dried over anhydrous sodium sulfate, filtered and concentrated. The crude product was purified by flash silica gel column chromatography (ethyl acetate /petroleum ether: 0-30%) to obtain t-butyl (5S,5aS,6S,9R)-2-(8-chloro-3-(methoxymethoxy)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (88 mg, 0.132 mmol, yield: 42.32%). ES-API: [M+H]⁺=667.9.

Step VII: T-butyl (5S,5aS,6S,9R)-2-(8-chloro-3-(methoxymethoxy)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (88 mg, 0.132 mmol) was dissolved in acetonitrile (3 mL). Hydrochloric acid in dioxane (1 mL, 0.132 mmol) was added, and reacted at room temperature for 1 hr. The reaction solution was concentrated under reduced pressure to obtain 5-chloro-4-((5S,5aS,6S,9R)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-2-yl)naphthalen-2-ol (65 mg, 0.124 mmol, yield: 94.19%). ES-API: [M+H]⁺=523.9

Step VIII: 5-chloro-4-((5S,5aS,6S,9R)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-2-yl)naphthalen-2-ol (65 mg, 0.124 mmol) was dissolved in dichloromethane (4 mL). Di-t-butyl dicarbonate (0.031 mL, 0.136 mmol) and triethyl amine (25.06 mg, 0.248 mmol) were added, reacted at room temperature for 18 hrs, and concentrated under reduced pressure. The crude product was purified by silica gel column chromatography (ethyl acetate /petroleum ether: 0-40%) to obtain t-butyl (5S,5aS,6S,9R)-2-(8-chloro-3-hydroxylnaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (70 mg, 0.112 mmol, yield: 90.42%) ES-API: [M+H]⁺=624.0.

Step IX: At -40°C, t-butyl (5S,5aS,6S,9R)-2-(8-chloro-3-hydroxylnaphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (70 mg, 0.112 mmol) was dissolved in dichloromethane. N,N-diisopropylethylene diamine (31.06 mg, 0.240 mmol) and trifluoromethanesulfonic anhydride (54.25 mg, 0.192 mmol) were added, reacted for 2 hrs, and concentrated under reduced pressure. The crude product was purified by silica gel column chromatography (ethyl acetate /petroleum ether: 0-20%) to obtain t-butyl (5S,5aS,6S,9R)-2-(8-chloro-3-(((trifluoromethyl)sulfonyl)oxy)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (77 mg, 0.102 mmol, yield: 90.79%).

Step X: T-butyl (5S,5aS,6S,9R)-2-(8-chloro-3-(((trifluoromethyl)sulfonyl)oxy)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (59.74 mg, 0.079 mmol), benzophenone imine (18.61 mg, 0.103 mmol), tris(dibenzylideneacetone) dipalladium (7.23 mg, 0.008 mmol, 4,5-bis(diphenylphosphino)-9,9-dimethylxanthene (9.14 mg, 0.016 mmol), and cesium carbonate (64.35 mg, 0.197 mmol) were dissolved in 1,4-dioxane (1 mL), reacted under a nitrogen atmosphere at 110°C for 2 hrs, and concentrated under reduced pressure. The crude product was purified by silica gel column chromatography (ethyl acetate /petroleum ether: 0-30%) to obtain t-butyl (5S,5aS,6S,9R)-2-(8-chloro-3-((benzophenone imine )naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (50 mg, 0.064 mmol, yield: 80.39%) ES-API: [M+H]⁺=786.9.

Step XI: T-butyl (5S,5aS,6S,9R)-2-(8-chloro-3-((benzophenone imine )naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (50 mg, 0.064 mmol) was dissolved in dichloromethane (2 mL). m-chlorobenzoic acid (19.95 mg, 0.116 mmol) and reacted at room temperature for 2 hrs. After the reaction, the reaction solution was added with a sodium sulfite solution (5 mL), and then extracted with dichloromethane (5 mLX3). The organic phase was washed with saturated brine (10 mL), dried over anhydrous sodium sulfate, filtered and concentrated, to obtain t-butyl (5S,5aS,6S,9R)-2-(8-chloro-3-((benzophenone imine )naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (50 mg, 0.06 mmol, yield: 98%). ES-API: [M+H]⁺=803.0.

Step XII: (R)-(2,2-difluorotetrahydro-1H-pyrrolizin-7a(5H)-yl)methanol (16.54 mg, 0.093 mmol) was dissolved in tetrahydrofuran(5 mL). Sodium hydride (5.97 mg, 0.249 mmol) and t-butyl (5S,5aS,6S,9R)-2-(8-chloro-3-((benzophenone imine )naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (50 mg, 0.064 mmol) were added, and reacted at room temperature for 2 hrs. After the reaction, the reaction solution was added with water (5 mL) and extracted with ethyl acetate (5 mLX3). The organic phase was washed with saturated brine (10 mL), dried over anhydrous sodium sulfate, filtered and concentrated. The crude product was purified by flash silica gel column chromatography (ethyl acetate /petroleum ether: 0-70%) to obtain t-butyl (5S,5aS,6S,9R)-2-(8-chloro-3-((benzophenone imine )naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (40 mg, 0.044 mmol, yield: 70.13%). ES-API: [M+H]⁺=916.0.

Step XIII: T-butyl (5S,5aS,6S,9R)-2-(8-chloro-3-((benzophenone imine )naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (35 mg, 0.038 mmol) was dissolved in ethyl acetate (1 mL). Hydrochloric acid in dioxane (1 mL, 4.000 mmol) was added, reacted at room temperature for 2 hrs, and concentrated under reduced pressure. The residue was purified by preparative HPLC (formic acid method 2) to obtain 5-chloro-4-((5S,5aS,6S,9R)-12-(((R)-2,2-difluorotetrahydro-1H-pyrrolizin-7a(5H)-yl) methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)naphthalen-2-amine (Z737, 9.6 mg, yield: 38.54%, formate). ES-API: [M+H]⁺= 652.0. ¹H NMR (400 MHz, CD₃OD) δ 8.48-8.42 (m, 1H), 7.65-7.61 (m, 1H), 7.36-7.09 (m, 3H), 5.48 (d, *J* = 13.6 Hz, 1H), 4.65-4.61 (m, 1H), 4.38 - 4.26 (m, 2H), 4.20-4.18 (m, 1H), 3.98-3.94 (m, 1H), 3.88-3.85 (m, 1H), 3.49-3.43 (m, 1H), 3.32-3.27 (m, 1H), 3.22-3.13 (m, 2H), 2.93-2.89 (m, 1H), 2.73-2.55 (m, 1H), 2.43-2.38 (m, 1H), 2.43-2.20 (m, 2H), 2.04-1.98 (m, 6H), 1.66-1.54 (m, 3H).

### Example 125: Synthesis of (5S,5aS,6S,9R)-2-(8-ethynyl-7-fluoronaphthalen-1-yl)-1-fluoro-12-((1S,7aS)-1-methoxy-6-methylenetetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]heptene (Z738)

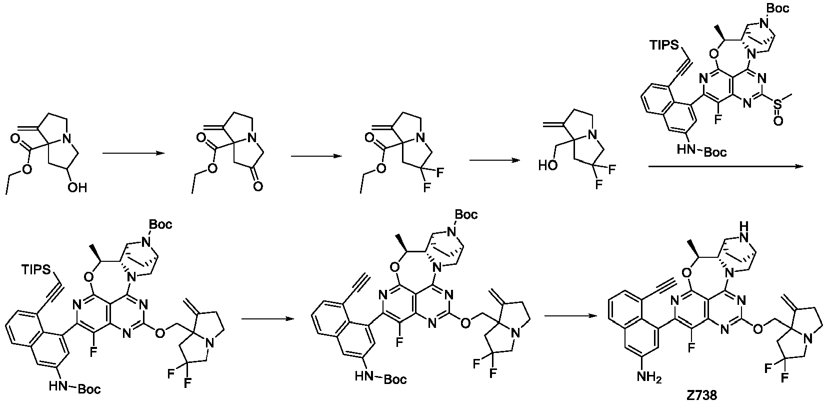

Step I: At -78°C, dimethyl sulfoxide (0.672 mL, 9.467 mmol) was slowly added to a solution of oxalyl chloride (2.840 mL, 5.680 mmol) in dichloromethane (3 mL). Then a solution of ethyl 6-hydroxyl-1-methylenetetrahydro-1H-pyrrolizin-7a(5H)-carboxylate (800 mg, 3.787 mmol) in dichloromethane (3 mL) was added, and stirred at - 78°C for 1 hr. Triethyl amine (2.632 mL, 18.934 mmol) was slowly added to the reaction system, stirred at -78°C for 30 min, and slowly heated to room temperature. The reaction was shown to be completed by LC/MS. The reaction solution was diluted with dichloromethane (20 mL), washed with water (15 mLX 3) and saturated brine (15 mLX 3), dried over anhydrous sodium sulfate, filtered and concentrated under reduced pressure. The crude product was purified by flash silica gel column chromatography (dichloromethane: methanol =20:1) to obtain ethyl 1-methylene-6-oxotetrahydro-1H-pyrrolizin-7a(5H)-carboxylate (350 mg, yield: 44%). ES-API: [M+1]⁺= 210.1.

Step II: Under a nitrogen atmosphere, diethylaminosulfur trifluoride (0.578 mL, 4.301 mmol) was added to a solution of ethyl 1-methylene-6-oxotetrahydro-1H-pyrrolizin-7a(5H)-carboxylate (300 mg, 1.434 mmol) in dichloromethane (5 mL), and stirred at room temperature for 16 hrs. The reaction was shown to be completed by LC/MS. The reaction solution was washed with a saturated sodium bicarbonate solution (10 mL), and extracted with ethyl acetate (20 mL). The filtrate was concentrated under reduced pressure, and the crude product was purified by flash silica gel column chromatography (petroleum ether: ethyl acetate =10:1) to obtain ethyl 6,6-difluoro-1-methylenetetrahydro-1H-pyrrolizin-7a(5H)-carboxylate(180 mg, yield: 54%). ES-API: [M+1]⁺=232.1.

Step III: Under a nitrogen atmosphere, ethyl 6,6-difluoro-1-methylenetetrahydro-1H-pyrrolizin-7a(5H)-carboxylate (150 mg, 0.649 mmol) was dissolved in anhydrous tetrahydrofuran (5mL), and the mixture was allowed to stand in an iced water bath. A lithium aluminium hydride solution (0.973 mL, 0.973 mmol, 1 M solution in tetrahydrofuran) was slowly added dropwise to the above solution, and stirred at this temperature for 0.5 hr. The reaction was shown to be completed by LC/MS. The reaction solution was quenched with sodium sulfafe decahydrate (300 mg), stirred at room temperature for 0.5 hr, and filtered. The filtrate was concentrated under reduced pressure, to obtain (6,6-difluoro-1-methylenetetrahydro-1H-pyrrolizin-7a(5H)-yl)methanol (100 mg, yield: 81%). ES-API: [M+H]⁺= 190.1.

Step IV: (6,6-difluoro-1-methylenetetrahydro-1H-pyrrolizin-7a(5H)-yl)methanol (64.13 mg, 0.339 mmol) was dissolved in anhydrous tetrahydrofuran (5 mL). In an iced water bath, sodium hydride (36.15 mg, 0.904 mmol) was added, and then reacted for 30 min in the iced water bath. A solution of t-butyl (5S, 5aS, 6S, 9R)-2-(3-((t-butoxycarbonyl)amino)-8-((triisopropylsilyl) ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (200 mg, 0.226 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLx2) and saturated brine (15 mL X 3), dried over anhydrous sodium sulfate, filtered, and concentrated. A crude product was obtained, which was purified by silica gel column chromatography (dichloromethane: methanol =15:1) to obtain t-butyl (5S,5aS,6S,9R)-2-(3-((t-butoxycarbonyl)amino)-8-((triisopropylsilyl)ethynyl)naphthalen-1-yl)-12-((6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (190 mg, yield: 83.23%). ES-API: [M+H]⁺= 1010.3.

Step V: Cesium fluoride (285.67 mg, 1.881 mmol) was added to t-butyl (5S,5aS,6S,9R)-2-(3-((t-butoxycarbonyl)amino)-8-((triisopropylsilyl)ethynyl)naphthalen-1-yl)-12-((6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (190 mg, 0.188 mmol) in N,N-dimethylformamide (1.5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated to obtain the crude product. The crude product was purified by silica gel column chromatography ((dichloromethane: methanol =10:1)mobile phase) to obtain t-butyl (5S,5aS,6S,9R)-2-(3-((t-butoxycarbonyl)amino)-8-ethynylnaphthalen-1-yl)-12-((6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (80 mg, yield: 50%). ES-API: [M+H]⁺= 854.2.

Step VI: In an iced water bath, a 4 M hydrochloric acid-dioxane solution (5 mL) was slowly added to a solution of t-butyl (5S,5aS,6S,9R)-2-(3-((t-butoxycarbonyl)amino)-8-ethynylnaphthalen-1-yl)-12-((6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (80 mg, 0.09 mmol) in ethyl acetate (1 mL). The reaction mixture was stirred at room temperature for 2 hrs. The reaction was shown to be completed by LC/MS. The reaction solution was concentrated to dryness under reduced pressure, and the residue was purified by preparative HPLC (ammonium bicarbonate method 2) to obtain (5S,5aS,6S,9R)-2-(8-ethynyl-7-fluoronaphthalen-1-yl)-1-fluoro-12-((1S,7aS)-1-methoxy-6-methylenetetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptene (Z738, 5 mg, yield: 8% ). ES-API: [M+H]⁺= 654.1. ¹H NMR (400 MHz, CD₃OD) δ 8.50-8.21 (m, 1H), 7.68 (t, J =4.0 Hz, 1H), 7.41-7.30 (m, 1H), 7.29-7.26 (m, 1H), 7.14-7.10 (m, 1H), 7.10-7.01 (m, 1H), 5.40-5.31 (m, 1H), 5.19 (s, 1 H), 5.16 (s, 1 H), 4.59-4.51 (m, 1H), 4.42-4.30 (m, 2H), 4.13 (d, J = 8.0 Hz, 1H), 3.85 (d, J = 4.0 Hz, 1H), 3.74 (s, 1H), 3.59 -3.40 (m, 1H), 3.26-3.18 (m, 2H), 3.15 - 3.09 (m, 2H), 2.90-2.84 (m, 1H), 2.76-2.65 (m, 3H), 2.57-2.39 (m, 1H), 2.21-2.14 (m, 2H), 1.94-1.83 (m, 3H), 1.57 (t, J = 8.0 Hz, 3H).

### Example 126: Synthesis of 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-2-yl)-5-ethynylnaphthalen-2-amine (Z739)

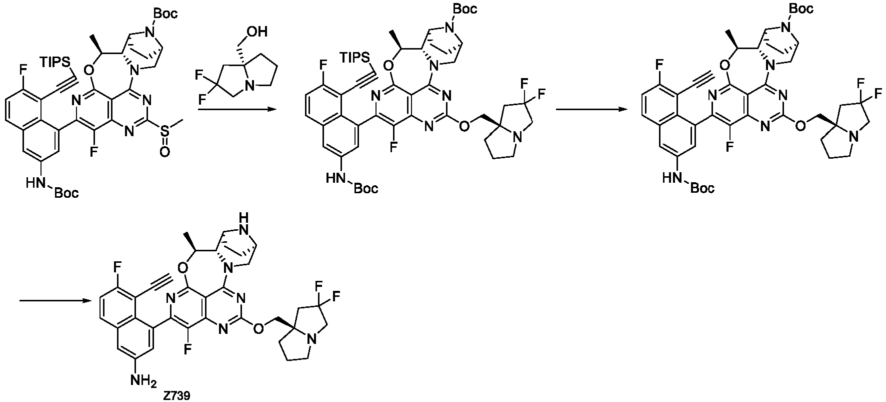

Step I: (R)-(2,2-difluorotetrahydro-1H-pyrrolizin-7a(5H)-yl)methanol (162 mg, 0.91 mmol) was dissolved in anhydrous tetrahydrofuran(6 mL). In an iced water bath, sodium hydride (73 mg, 0.82 mmol) was added, and reacted at room temperature for 10 min. The reaction solution was cooled to 0°C. A solution of t-butyl (5S,5aS,6S,9R)-2-(3-((t-butoxycarbonyl)amino)-7-fluoro-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (550 mg, 0.61 mmol) in tetrahydrofuran (2 mL) was added dropwise to the reaction solution, and reacted at 0°C for 10 min. The reaction solution was quenched with saturated sodium bicarbonate (50 mL), extracted with ethyl acetate (30 mLX2), washed with saturated brine (60 mL), dried over anhydrous sodium sulfate, filtered, and concentrated to obtain t-butyl (5S,5aS,6S,9R)-2-(3-((t-butoxycarbonyl)amino)-7-fluoro-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (550mg, yield: 88%). ES-API: [M+H]⁺=1016.5.

Step II: Cesium fluoride (822 mg, 5.41 mmol) was added to t-butyl (5S,5aS,6S,9R)-2-(3-((t-butoxycarbonyl)amino)-7-fluoro-8-((triisopropylsilyl)ethynyl)naphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (550 mg, 0.54 mmol) in N,N-dimethylformamide (5 mL), and the reaction mixture was stirred at room temperature for 1 hr. The reaction was shown to be completed by LC/MS. The reaction solution was diluted with ethyl acetate (30 mL), washed with saturated brine (30mLX3), dried over anhydrous sodium sulfate, filtered, and concentrated. The crude product was purified by flash silica gel column chromatography (ethyl acetate /petroleum ether: 0-50%) to obtain t-butyl (5S,5aS,6S,9R)-2-(3-((t-butoxycarbonyl)amino)-8-ethynyl-7-fluoro-o-xylylen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (330 mg, yield: 71%). ES-API: [M+H]⁺= 860.2

Step III: In an iced water bath, a 4 M hydrochloric acid-dioxane solution (5 mL) was slowly added to a solution of t-butyl (5S,5aS,6S,9R)-2-(3-((t-butoxycarbonyl)amino)-8-ethynyl-7-fluoroxylylen-1-yl)-12-(((R)-2,2-difluorotetrahydro-1H-pyrrolin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-14-carboxylate (330 mg, 0.38 mmol) in ethyl acetate (3 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate 2) to obtain 4-((SS,SaS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z739, 155 mg, yield: 61%). ES-API: [M+H]⁺= 660.2. ¹H NMR (400Hz, CD₃OD) 7.74-7.68 (m,1H), 7.25-7.05 (m,3H), 5.38-5.35 (m,1H), 4.52-4.98 (m,1H), 4.30-4.24 (m,2H), 4.09-4.07 (m, 1H), 3.69-3.31 (m,4H), 3.21-3.08 (m,3H), 2.84-2.81 (m,1H), 2.61- 2.52 (m,1H), 2.35-2.26 (m,1H), 2.15-1.76 (m,8H), 1.58-1.54 (m,3H).

### Example 127: Synthesis of 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5,6-difluoronaphthalen-2-amine (Z740)

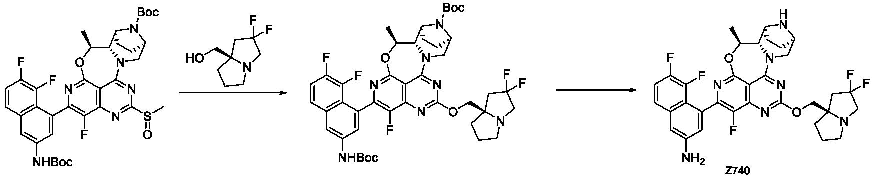

Step I: (R)-(2,2-difluorotetrahydro-1H-pyrrolizin-7a(5H)-yl)methanol (363 mg, 2.048 mmol) was dissolved in anhydrous tetrahydrofuran (15 mL). In an iced water bath, sodium hydride (189 mg, 4.726 mmol) was added, and reacted for 10 min in the iced water bath. A solution of t-butyl (5S,5aS,6S,9R)-2-(3-((t-butoxycarbonyl)amino)-7,8-difluoronaphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]heptan-14-carboxylate (1167 mg, 1.575 mmol) in tetrahydrofuran (15 mL) was added dropwise to the reaction solution, and reacted for 30 min at 0 °C. The reaction was shown to be completed by LC/MS. The reaction solution was diluted with ethyl acetate (20 mL), washed with a saturated ammonium chloride solution (20 mLX2) and saturated brine (20 mLX 3), dried over anhydrous sodium sulfate, filtered, and concentrated under reduced pressure, to obtain the crude product t-butyl (5S,5aS,6S,9R)-2-(3-((t-butoxycarbonyl)amino)-7,8-difluoronaphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (1345 mg). ES-API: [M+ H]⁺= 854.2.

Step II: In an iced water bath, a 4 M hydrochloric acid-dioxane solution (10 mL) was slowly added to a solution of t-butyl (5S,5aS,6S,9R)-2-(3-((t-butoxycarbonyl)amino)-7,8-difluoronaphthalen-1-yl)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (1345 mg, 1.575 mmol) in methanol (2 mL), and the reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptan-2-yl)-5,6-difluoronaphthalen-2-amine (Z740, 237.08 mg, yield: 23%) as an off-white solid. ES-API: [M+H]⁺= 654.0. ¹H NMR (400 MHz, CD₃OD) δ 7.48- 7.43 (m, 1 H), 7.33- 7.24 (m, 1 H), 7.17- 7.04 (m, 2 H), 5.39- 5.35 (m, 1 H), 4.59- 4.53 (m, 1 H), 4.32- 4.25 (m, 2 H), 4.10- 4.06 (m, 1 H), 3.70- 3.68 (m, 1 H), 3.60- 3.59 (m, 1 H), 3.48- 3.39 (m, 1 H), 3.21- 3.09 (m, 3 H), 2.89- 2.83 (m, 1 H), 2.62- 2.53 (m, 1 H), 2.39- 2.29 (m, 1 H), 2.21- 2.17 (m, 1 H), 2.07- 1.77 (m, 7 H), 1.59- 1.56 (m, 3 H).

### Example 128: Synthesis of 4-((5S,5aS,6S,9R)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z741)

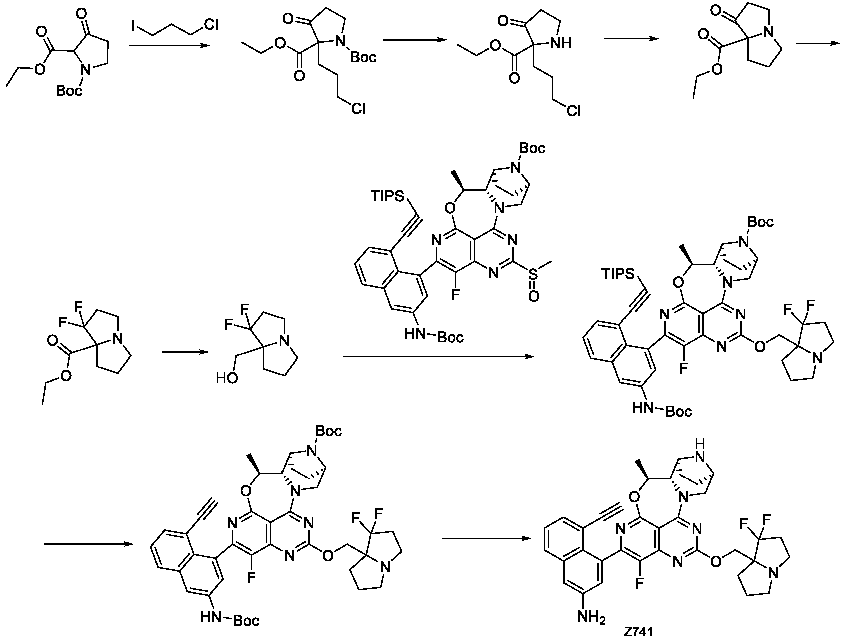

Step I: Potassium carbonate (7.73 g, 55.968 mmol) and 1-chloro-3-iodopropane (15.26 g, 74.624 mmol) were added to a solution of 1-(t-butyl) 2-ethyl3-oxopyrrolidin-1,2-dicarboxylate (4.8 g, 18.6 mmol) in N,N-dimethylformamide (20 mL), and stirred overnight at room temperature. The reaction was shown to be completed by LC/MS. The reaction solution was filtered, and washed with ethyl acetate. The filtrate was added with water (50 mLX3), washed with saturated brine (50 mLX3), dried over anhydrous sodium sulfate, filtered, and concentrated under reduced pressure. The crude product was purified by flash silica gel column chromatography (petroleum ether: ethyl acetate =10:1) to obtain 1-(t-butyl) 2-ethyl 2-(3-chloropropyl)-3-oxopyrrolidin-1,2-dicarboxylate (3.5g, yield: 56%). ES-API: [M-99]⁺= 234.1.

Step II: In an iced water bath, a 4 M hydrochloric acid-dioxane solution (10 mL) was slowly added to a solution of 1-(t-butyl) 2-ethyl 2-(3-chloropropyl)-3-oxopyrrolidin-1,2-dicarboxylate (2.0g, 5.99 mmol) in acetonitrile (5 mL). The reaction mixture was stirred at room temperature for 2 hrs. The reaction was shown to be completed by LC/MS. The reaction solution was concentrated to dryness under reduced pressure to obtain the crude product ethyl 2-(3-chloropropyl)-3-oxopyrrolidin-2-carboxylate (1.4g, yield:100%). ES-API: [M+1]⁺= 234.1.

Step III: At room temperature, sodium bicarbonate (2.52 g, 29.954 mmol) and potassium iodide (99.6 mg, 0.6 mmol) were added to a solution of ethyl 2-(3-chloropropyl)-3-oxopyrrolidin-2-carboxylate (1.4 g, 5.991 mmol) in acetonitrile (10 mL), and stirred at room temperature for 1.5 hrs. The reaction was shown to be completed by LC/MS. The reaction solution was filtered, and washed with acetonitrile. The filtrate was concentrated under reduced pressure, and the crude product was purified by flash silica gel column chromatography (dichloromethane: methanol =15:1) to obtain ethyl 1-oxotetrahydro-1H-pyrrolizin-7a(5H)-carboxylate (1.0 g, yield:85%). ES-API: [M+H]⁺=198.1.

Step IV: Under a nitrogen atmosphere, diethylaminosulfur trifluoride (1.839 mL, 13.690 mmol) was added to a solution of ethyl 1-oxotetrahydro-1H-pyrrolizin-7a(5H)-carboxylate (900 mg, 4.563 mmol) in dichloromethane (5 mL), and stirred at room temperature for 16 hrs. The reaction was shown to be completed by LC/MS. The reaction solution was washed with a saturated sodium bicarbonate solution (10 mL), and extracted with ethyl acetate (20 mL). The filtrate was concentrated under reduced pressure, and the crude product was purified by flash silica gel column chromatography (petroleum ether: ethyl acetate =10:1) to obtain ethyl 1,1-difluorotetrahydro-1H-pyrrolizin-7a(5H)-carboxylate (210 mg, yield: 21%). ES-API: [M+1]⁺=220.1.

Step V: Under a nitrogen atmosphere, ethyl 1,1-difluorotetrahydro-1H-pyrrolizin-7a(5H)-carboxylate (150 mg, 0.684 mmol) was dissolved in anhydrous tetrahydrofuran (3 mL), and the mixture was allowed to stand in an iced water bath. A lithium aluminium hydride solution (1.026 mL, 1.026 mmol, 1 M in THF) was slowly added dropwise to the above solution, and stirred at this temperature for 0.5 hr. The reaction was shown to be completed by LC/MS. The reaction was quenched with sodium sulfafe decahydrate (300 mg), stirred at room temperature for 0.5 hr, and filtered. The filtrate was concentrated under reduced pressure, to obtain the crude product (1,1-difluorotetrahydro-1H-pyrrolizin-7a(5H)-yl)methanol (121 mg, yield: 100%). ES-API: [M+H]⁺= 190.1.

Step VI: (1,1-difluorotetrahydro-1H-pyrrolizin-7a(5H)-yl)methanol (45.64 mg, 0.258 mmol) was dissolved in anhydrous tetrahydrofuran (5 mL). In an iced water bath, sodium hydride (27.47 mg, 0.687 mmol) was added, and then reacted for 30 min in the iced water bath. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxo-3,10a,11,13,14-pentaaza-6,9-methanonaphtho[1,8-ab]hepten-14-carboxylate (152 mg, 0.172 mmol) in tetrahydrofuran (2 mL) was added dropwise to the reaction solution, and reacted at room temperature for 30 min. The reaction was shown to be completed by LC/MS. The reaction solution was diluted with ethyl acetate (20 mL), washed with a saturated ammonium chloride solution (15 mLX2) and saturated brine (15 mLX 3), dried over anhydrous sodium sulfate, and filtered. The crude product was purified by silica gel column chromatography ((dichloromethane: methanol =15:1) mobile phase) to obtain t-butyl (5S,5aS,6S,9R)-2-(3-((t-butoxycarbonyl)amino)-8-((triisopropylsilyl)ethynyl)naphthalen-1-yl)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (150 mg, yield: 85%). ES-API: [M+H]⁺= 998.3.

Step VII: Cesium fluoride (228.24 mg, 1.503 mmol) was added to t-butyl (5S,5aS,6S,9R)-2-(3-((t-butoxycarbonyl)amino)-8-((triisopropylsilyl)ethynyl)naphthalen-1-yl)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (150 mg, 0.150 mmol) in N,N-dimethylformamide (2 mL), and the reaction mixture was stirred at room temperature for 2 hrs. The reaction was shown to be completed by LC/MS. The reaction solution was diluted with ethyl acetate (20 mL), washed with water (15 mLx2) and saturated brine (15 mLX 3), dried over anhydrous sodium sulfate, filtered, and concentrated to obtain a crude product. The crude product was purified by silica gel column chromatography ((dichloromethane: methanol =15:1) mobile phase) to obtain t-butyl (5S,5aS,6S,9R)-2-(3-((t-butoxycarbonyl)amino)-8-ethynylnaphthalen-1-yl)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (75 mg, yield: 51%). ES-API: [M+H]⁺=984.2.

Step VIII: In an iced water bath, a 4 M hydrochloric acid-dioxane solution (5 mL) was slowly added to a solution of t-butyl (5S,5aS,6S,9R)-2-(3-((t-butoxycarbonyl)amino)-8-ethynylnaphthalen-1-yl)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (75 mg, 0.076 mmol) in acetonitrile (1 mL). The reaction mixture was stirred at 0°C for 0.5 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((1,1-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z741, 10 mg, yield: 21% )ES-API: [M+H]⁺= 642.1. ¹H NMR (400 MHz, CD₃OD) δ 7.58 (t, J = 4.0 Hz, 1H), 7.35-7.25 (m, 1H), 7.22-7.16 (m, 1H), 7.05-7.00 (m, 1H), 6.96-6.90 (m, 1H), 5.32-5.26 (m, 1H), 4.49- 4.36 (m, 1H), 4.26 (s, 2H), 3.98 (d, J = 8.0 Hz, 1H), 3.61 (s, 1H), 3.51 (s, 1H), 3.22-3.15 (m, 1H), 3.13-3.04 (m, 1H), 3.03-2.86 (m, 2H), 2.74-2.65 (m, 2H), 2.60-2.50 (m, 2H), 2.30-2.21 (m, 1H), 2.19- 2.08 (m, 1H), 1.96-1.90 (m, 1H), 1.85-1.75 (m, 3H), 1.80-1.70 (m, 4H), 1.47 (t, J = 4.0 Hz, 3H).

### Example 129: Synthesis of 4-(((5S,5aS,6S,9R)-2-(3-amino-8-ethynyl-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy-1,1-difluoro-3-methylbutan-2-ol (Z742)

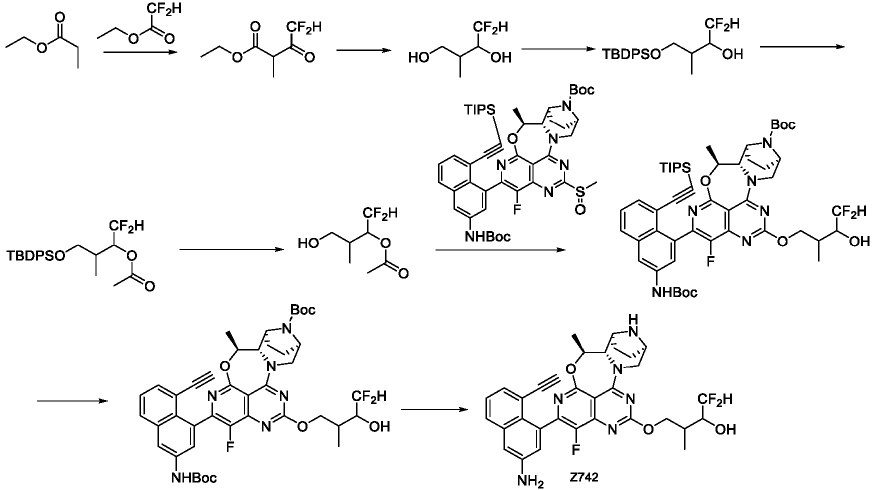

Step I: Ethyl propionate (3.522 mL, 30.623 mmol) was dissolved in tetrahydrofuran (35 mL). Sodium hydride (1.22 g, 30.623 mmol) was added, 2,2-difluoroethyl acetate (7.6 g, 61.246 mmol) was added dropwise, and reacted at 50°C for 18 hrs. After the reaction, the reaction solution was poured into water (30 mL) and extracted with ethyl acetate (3×20 mL). The organic phase was washed with water (20 mL) and saturated brine (30 mL), dried over anhydrous sodium sulfate, filtered and concentrated. The crude product was purified by flash silica gel column chromatography (ethyl acetate /petroleum ether: 0-40%) to obtain ethyl 4,4-difluoro-2-methyl-3-oxobutyrate (3500 mg, 19.428 mmol, yield: 63.44%). ES-API: [M+H]⁺=181.1.

Step II: Ethyl 4,4-difluoro-2-methyl-3-oxobutyrate (1800 mg, 9.992 mmol) was dissolved in tetrahydrofuran (15 mL). In an iced water bath, lithium aluminium hydride (15 mL, 15 mmol) was added, reacted at room temperature for 2 hrs, added with water (1.5 mL), 15% sodium hydroxide solution (1.5 mL), water (3 mL), filtered under suction, dried over anhydrous sodium sulfate, and filtered. The filtrate was concentrated under reduced pressure, to obtain 4,4-difluoro-2-methylbutan-1,3-diol (750 mg, 5.352 mmol, yield: 53.57%). ES-API: [M+H]⁺=141.1.

Step III: 4,4-difluoro-2-methylbutan-1,3-diol (700 mg, 4.995 mmol) was dissolved in dichloromethane (15 mL). Imidazole (1700 mg, 25 mmol) and t-butyldiphenylchlorosilane (1373.08 mg, 5 mmol) were added, and reacted overnight at room temperature. After the reaction, the reaction solution was poured into water (30 mL) and extracted with dichloromethane (3×20 mL). The organic phase was washed with water (20 mL) and saturated brine (30 mL), dried over anhydrous sodium sulfate, filtered, and concentrated. The crude product was purified by flash silica gel column chromatography (ethyl acetate /petroleum ether: 0-40%) to obtain 4-((t-butyldiphenylsilyl)oxy)-1,1-difluoro-3-methylbutan-2-ol (1300 mg, 3.434 mmol, yield: 68.75%). ES-API: [M+H]⁺=379.1.

Step IV: 4-((t-butyldiphenylsilyl)oxy)-1,1-difluoro-3-methylbutan-2-ol (1200 mg, 3.170 mmol) was dissolved in dichloromethane(10 mL). In an iced water bath, acetic anhydride (0.328 mL, 3.487 mmol), N,N-diisopropylethyl amine (384.22 mg, 3.804 mmol), and 4-dimethylaminopyridine (38.73 mg, 0.317 mmol) were added, and reacted overnight at room temperature. After the reaction, the reaction solution was concentrated under reduced pressure. The crude product was purified by flash silica gel column chromatography (ethyl acetate /petroleum ether: 0-30%) to obtain ethyl 4-((t-butyldiphenylsilyl)oxy)-1,1-difluoro-3-methylbutan-2-carboxylate (1000 mg, 2.378 mmol, yield: 75.00%). ES-API: [M+H]⁺=421.2.

Step V: 4-((t-butyldiphenylsilyl)oxy)-1,1-difluoro-3-methylbutan-2-acetate (420 mg, 1 mmol) was dissolved in tetrahydrofuran(2 mL). A tetrahydroammonium fluoide solution in tetrahydrofuran (1.5 mL, 1.5 mmol) was added, and reacted at room temperature for 2 hrs. The reaction solution was concentrated under reduced pressure. The crude product was purified by flash silica gel column chromatography (ethyl acetate /petroleum ether: 0-70%) to obtain 1,1-difluoro-4-hydroxyl-3-methylbutan-2-acetate (100 mg, 0.549 mmol, yield: 54.97%).

Step VI: 1,1-difluoro-4-hydroxyl-3-methylbutan-2-acetate (80 mg, 0.44 mmol) was dissolved in tetrahydrofuran (5 mL). Sodium hydride (21.69 mg, 0.904 mmol) and t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (200 mg, 0.226 mmol) were added, and reacted at room temperature for 2 hrs. After the reaction, the reaction solution was added with water (5 mL) and extracted with ethyl acetate (5 mLX3). The organic phase was washed with saturated brine (10 mL), dried over anhydrous sodium sulfate, filtered and concentrated. The crude product was purified by flash silica gel column chromatography (ethyl acetate /petroleum ether: 0-70%) to obtain t-butyl (5S,5aS,6S,9R)-2-(3-((t-butoxycarbonyl)amino)-8-((triisopropylsilyl)ethynyl) naphthalen-1-yl)-12-(4,4-difluoro-3-hydroxyl-2-methylbutoxy)- 1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.094 mmol, yield: 41.44%) ES-API: [M+H]⁺=961.3.

Step VII: T-butyl (5S,5aS,6S,9R)-2-(3-((t-butoxycarbonyl)amino)-8-((triisopropylsilyl) ethynyl)naphthalen-1-yl)-12-(4,4-difluoro-3-hydroxyl-2-methylbutoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (80 mg, 0.083 mmol) was dissolved in N,N-dimethylformamide (1 mL). Cesium fluoride (126.42 mg, 0.832 mmol) was added, and reacted at room temperature for 2 hrs. After the reaction, the reaction solution was added with water (5 mL) and extracted with ethyl acetate (5 mLX3). The organic phase was washed with saturated brine (10 mL), dried over anhydrous sodium sulfate, filtered and concentrated. The crude product was purified by flash silica gel column chromatography (ethyl acetate /petroleum ether: 0-70%) to obtain t-butyl (5S,5aS,6S,9R)-2-(3-((t-butoxycarbonyl)amino)-8-ethynylnaphthalen-1-yl)-12-(4,4-difluoro-3-hydroxyl-2-methylbutoxy)-1-fluoro-5-methyl-5a,6,7,8,9,10-hexahydro-5H-4-oxa-3,10a,11,13,14-pentaaza-6,9-methanonaphtho[1,8-ab]heptan-14-carboxylate (60 mg, 0.075 mmol, yield: 89.57%) ES-API: [M+H]⁺=805.2.

Step VIII: T-butyl (5S,5aS,6S,9R)-2-(3-((t-butoxycarbonyl)amino)-8-ethynylnaphthalen-1-yl)-12-(4,4-difluoro-3-hydroxyl-2-methylbutoxy)-1-fluoro-5-methyl-5a,6,7,8,9,10-hexahydro-5H-4-oxa-3,10a,11,13,14-pentaaza-6,9-methanonaphtho[1,8-ab]heptan-14-carboxylate (60 mg, 0.075 mmol) was dissolved in ethyl acetate (1 mL). Hydrochloric acid in dioxane (1 mL, 4.000 mmol) was added, and reacted at room temperature for 2 hrs. The reaction solution was concentrated under reduced pressure, and the residue was purified by preparative HPLC (trifluoroacetic acid method) to obtain 4-(((5S,5aS,6S,9R)-2-(3-amino-8-ethynyl-1-yl)-1-fluoro-5-methyl-5a,6,7,8,9,10-hexahydro-5H-4-oxa-3,10a,11,13,14-pentaaza-6,9-methanonaphtho[1,8-ab]hepten-12-yl)oxy-1,1-difluoro-3-methylbutan-2-ol (Z742, 16 mg, 17.95%, trifluoroacetate). ES-API: [M+H]⁺= 605.2. ¹H NMR (400 MHz, CD₃OD) δ 7.85-7.81 (m, 1H), 7.61-7.36 (m, 3H), 7.26 -7.18 (m, 1H), 6.12 -5.85 (m, 1H), 5.71-5.60 (m, 1H), 4.74 - 4.61 (m, 2H), 4.53-4.32 (m, 4H), 4.23-4.21 (m, 1H), 3.94-3.72 (m, 1H), 3.47-3.42 (m, 1H), 3.11-3.07 (m, 1H), 2.35-2.17 (m, 5H), 1.62-1.59 (m, 3H), 1.13-1.08 (m, 3H).

### Example 130: Synthesis of 3-chloro-5-((5S,5aS,6S,9R)-12-(((R)- 2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z743)

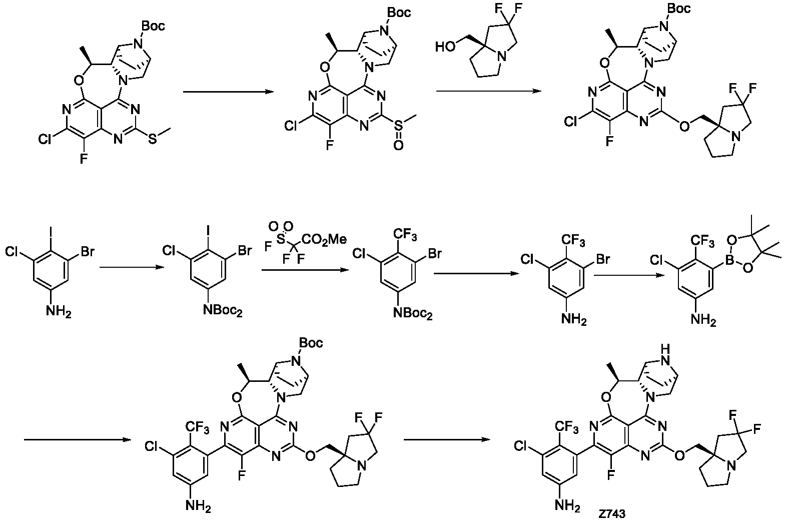

Step I: At 0°C, m-chloroperoxybenzoic acid (163.5 mg, 0.9451 mmol) was added to a solution of t-butyl (5S,5aS,6S,9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (350 mg, 0.727 mmol) in dichloromethane (30.0 mL). The solution was stirred at room temperature for 1 hr. The reaction was quenched with saturated sodium sulfite and extracted with dichloromethane(100 mLX2). The organic phase was dried over anhydrous sodium sulfate and concentrated to obtain a crude product. The crude product was purified by silica gel column chromatography (methanol /dichloromethane 0-10%) to obtain t-butyl (5S,5aS,6S,9R)-2-chloro-1-fluoro-5-methyl-12-(methylsulfinyl)-5a,6,7,8,9,10-hexahydro-5H-4-oxy-3,10a,11,13,14-pentaaza-6,9-methanonaphtho[1,8-ab]hepten-14-carboxylate (400 mg, crude product) as a white solid. ES-API:[M+H]⁺=498.2.

Step II: At 0°C, sodium hydride (292.0 mg, 7.308 mmol) was added to a solution of (R)-(2,2-difluorotetrahydro-1H-pyrrolizin-7a(5H)-yl)methanol (257.30 mg, 1.452 mmol) in tetrahydrofuran (10.0 mL), and stirred at this temperature for 10 min. Then, a solution of t-butyl (5S,5aS,6S,9R)-2-chloro-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (400 mg, crude product) in tetrahydrofuran (5 mL) was added. The reaction was continued with stirring at 0°C for 10 min, and at room temperature for 0.5 hr. After the reaction was completed, the reaction was quenched with a saturated sodium bicarbonate solution and the reaction solution was extracted with ethyl acetate. The organic phase was washed with saturated brine, dried over anhydrous sodium sulfate, filtered, and concentrated. The crude product was purified by silica gel column chromatography (methanol /dichloromethane 0-5%) to obtain t-butyl (5S,5aS,6S,9R)-2-chloro-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (130.0 mg, yield: 30.0%) as a yellow solid. ES-API: [M+H]⁺=611.2.

Step III: 3-bromo-5-chloro-4-iodoaniline (600 mg, 1.805 mmol) and di-t-butyl dicarbonate (0.829 mL, 3.611 mmol) were added to a sodium hydroxide aqueous solution (2M, 100 mL), heated to 100°C, and stirred for 2 hrs. After the reaction, the reaction solution was extracted with ethyl acetate (100mLX2) and saturated brine (30 mL). The ethyl acetate phases were combined, dried over anhydrous sodium sulfate, and filtered. The filtrate was concentrated under reduced pressure. The crude product was purified by silica gel column chromatography (ethyl acetate /petroleum ether 0-10%) to obtain 2-methylprop-2-yl [(3-bromo-5-chloro-4-iodophenyl){[(2-methylprop-2-yl)oxy]carbonyl}amino]carboxylate (890 mg, 1.671 mmol, yield: 92.56%). ES-API: [M+H]⁺ = 431.3.

Step IV: 2-methylprop-2-yl [(3-bromo-5-chloro-4-iodophenyl){ [(2-methylprop-2-yl)oxy] carbonyl}amino]carboxylate (1.10 g, 2.065 mmol), cuprous iodide (2.78 g, 14.457 mmol) and methyl 2,2-difluoro-2-(fluorosulfonyl)acetate (2.75 g, 14.457 mmol) were added to dry N,N-dimethylformamide (10.0 mL), heated to 90°C and reacted for 2 hrs. After the reaction, the reaction solution was cooled to room temperature and filtered. The filtrate was extracted with ethyl acetate (100 mLX2) and saturated brine (80 mL). The ethyl acetate phases were combined, dried over anhydrous sodium sulfate, and filtered. The filtrate was concentrated under reduced pressure. The crude product was purified by silica gel column chromatography (ethyl acetate /petroleum ether 0-10%) to obtain 2-methylprop-2-yl {[3-bromo-5-chloro-4-(trifluoromethyl)phenyl]{[(2-methylprop-2-yl)oxy]carbonyl}amino}carboxylate (680 mg, yield: 69.36%). ES-API: [M+Na]⁺ = 496.0.

Step V: Trifluoroacetic acid (702 mg, 7.162 mmol) was added to a solution of 2-methylprop-2-yl {[3-bromo-5-chloro-4-(trifluoromethyl)phenyl]{[(2-methylprop-2-yl)oxy]carbonyl}amino} carboxylate (340.0 mg, 0.716 mmol) in dichloromethane (10.0 mL), and stirred overnight at room temperature. After the reaction, the solvent was removed by rotary evaporation to dryness under reduced pressure. The reaction solution was added with ethyl acetate (100 mL), and washed with a saturated sodium bicarbonate solution (100 mLX2). The ethyl acetate phase was dried over anhydrous sodium sulfate, and filtered. The solvent was removed by rotary evaporation to dryness under reduced pressure to obtain 3-bromo-5-chloro-4-(trifluoromethyl)aniline (250 mg, crude product). ES-API: [M+H]⁺=274.0/276.0.

Step VI: 3-bromo-5-chloro-4-(trifluoromethyl)aniline (250 mg, 0.911 mmol, crude product), bis(pinacolato)diboron (300.69 mg, 1.184 mmol), [1,1'-bis(diphenylphosphino)ferrocene] palladium dichloridedichloromethane complex (74.38 mg, 0.091 mmol) and potassium acetate (268.17 mg, 2.733 mmol) were added to dry 1,4-dioxane (5.0 mL), purged 4 time with nitrogen, heated to 90°C and reacted for 18 hrs. After the reaction, the reaction solution was cooled to room temperature, added with ethyl acetate (100 mLX2) and extracted with saturated brine (80 mL). The ethyl acetate phases were combined, dried over anhydrous sodium sulfate, and filtered. The filtrate was concentrated under reduced pressure. The crude product was purified by silica gel column chromatography (ethyl acetate /petroleum ether 0-50%) to obtain 3-chloro-5-(4,4,5,5-tetramethyl-1,3,2-dioxaborolan-2-yl)-4-(trifluoromethyl)aniline (200.0mg, yield: 68.29%). ES-API: [M+H]⁺= 322.1.

Step VII: 3-chloro-5-(4,4,5,5-tetramethyl-1,3,2-dioxaborolan-2-yl)-4-(trifluoromethyl)aniline (200 mg, 0.622 mmol), t-butyl (5S,5aS,6S,9R)-2-chloro-12-(((R)- 2,2-difluorotetrahydro-1H-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]hepten-14-carboxylate (380.10 mg, 0.622 mmol), potassium phosphate (396.09 mg, 1.866 mmol), tris(dibenzylideneacetone) dipalladium (56.96 mg, 0.062 mmol) and tricyclohexyl phosphine (69.78 mg, 0.249 mmol) were added to a mixed solution of N,N-dimethylaniline (5 mL) and water (1 mL), purged 4 time with nitrogen, heated to 80°C, and stirred for 3 hrs. After the reaction, the reaction solution was cooled to room temperature, and extracted with ethyl acetate (100 mLX2) and saturated brine (80 mL). The ethyl acetate phases were combined, dried over anhydrous sodium sulfate, and filtered. The filtrate was concentrated under reduced pressure. The crude product was purified by silica gel column chromatography (methanol /dichloromethane 0-10%) to obtain t-butyl (5S,5aS,6S,9R)-2-(5-amino-3-chloro-2-(trifluoromethyl)phenyl)-12-((((S)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza- 6,9-methanonaphtho[1,8-ab]hepten-14-carboxylate (105.0 mg, yield: 21.92%). ES-API: [M+H]⁺=770.2.

Step VIII: T-butyl (5S,5aS,6S,9R)-2-(5-amino-3-chloro-2-(trifluoromethyl)phenyl)-12-((((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (105 mg, 0.136 mmol) was added to a mixed solution of dichloromethane (2 mL) and trifluoroacetic acid (1 mL), and reacted at room temperature for 4 hrs. After the reaction, the solvent was removed by rotary evaporation to dryness under reduced pressure. The obtained crude product was purified by preparative HPLC(formic acid method 1) to obtain 3-chloro-5-((5S,5aS,6S,9R)-12-(((R)-2,2-difluorotetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z743, 11.25 mg, yield: 12.32%, formate). ES-API: [M+H]⁺=670.2. ¹H NMR (400 MHz, CD₃OD) δ 8.38 (s, 1H), 6.88 (s, 1H), 6.50 - 6.40 (m, 1H), 5.47 -5.44 (m, 1H), 4.62 (s, 1H), 4.33 - 4.25 (m, 2H), 4.22 - 4.20 (m, 1H), 4.08 - 4.0 (m, 1H), 3.94 (s, 1H), 3.50 - 3.38 (m, 1H), 3.20 - 3.11 (m, 2H), 2.92 - 2.83 (m, 1H), 2.64 - 2.53 (m, 1H), 2.40 - 2.30(m, 1H), 2.26 - 2.13 (m, 2H), 2.08 - 1.84 (m, 6H), 1.58 (d, *J* = 6.3 Hz, 3H), 1.28 (s, 1H).

### Example 131: Synthesis of 4-((5S,5aS,6S,9R)-12-((1-((3-(difluoromethyl)pyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z744)

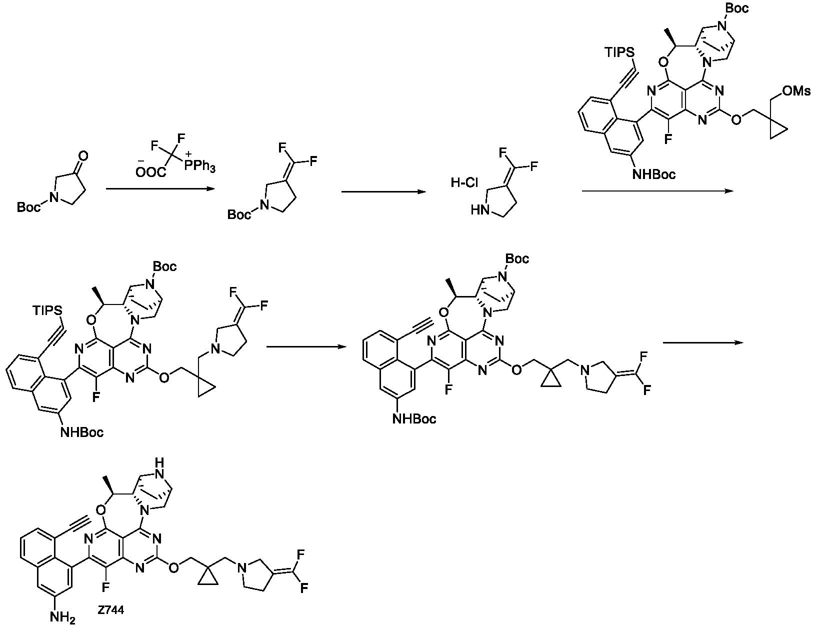

Step I: Under a nitrogen atmosphere, 2,2-difluoro-2-(triphenylphosphoryl)acetate (2.53 g, 7.100 mmol) was added to a solution of t-butyl 3-oxypyrrolidin-1-carboxylate (500 mg, 2.699 mmol) in N,N-dimethylformamide (30 mL). The reaction mixture was heated to 80°C and stirred overnight. The reaction mixture was poured into ethyl acetate (50 mL), and washed with saturated brine (30 mLX3). The organic phase was dried over anhydrous sodium sulfate, filtered, and concentrated under reduced pressure. The crude product was purified by silica gel column chromatography (ethyl acetate: petroleum ether= 0 - 5%) to obtain t-butyl 3-(difluoromethyl)pyrrolidin-1-carboxylate (316 mg, yield: 53%) as a clear oil. ES-API: [M-55]⁺= 164.1.

Step II: In an iced water bath, a 4 M hydrochloric acid-dioxane solution (2 mL) was slowly added to a solution of t-butyl 3-(difluoromethyl)pyrrolidin-1-carboxylate (115 mg, 0.525 mmol) in methanol (1 mL), and the reaction mixture was heated to room temperature and stirred for 1 hr. The reaction was shown to be completed by LC/MS. The reaction solution was concentrated to dryness under reduced pressure, to obtain 3-(difluoromethyl)pyrrolidine hydrochloride (62 mg, crude product). ES-API: [M+H]⁺= 120.1.

Step III: T-butyl (5S,5aS,6S,9R)-2-(3-((t-butoxycarbonyl)amino)-8-((triisopropylsilyl)ethynyl) naphthalen-1-yl)-1-fluoro-5-methyl-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (102 mg, 0.102 mmol), 3-(difluoromethyl)pyrrolidine hydrochloride (61 mg, 0.509 mmol), potassium carbonate(70 mg, 0.102 mmol) and potassium iodide (17 mg, 0.102 mmol) were dissolved in acetone (15 mL). The reaction mixture was heated to 80°C and stirred overnight in a sealed tube. The reaction was shown to be completed by LC/MS. Water (10 mL) and ethyl acetate (10 mL) were added to the reaction solution. The organic phase was separated, washed with saturated brine (10 mLX2), dried over anhydrous sodium sulfate, filtered, and concentrated. The crude product was purified by silica gel column chromatography (methanol: dichloromethane= 0 - 5%) to obtain t-butyl (5S,5aS,6S,9R)-2-(3-((t-butoxycarbonyl)amino)-8-((triisopropylsilyl) ethynyl)naphthalen-1-yl)-12-((1-(((3-(difluoromethyl)pyrrolidin-1-methyl)cyclopropyl) methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (58 mg, yield: 56%). ES-API: [M+H]⁺= 1024.3.

Step IV: Cesium fluoride (86 mg, 0.566 mmol) was added to t-butyl (5S,5aS,6S,9R)-2-(3-((t-butoxycarbonyl)amino)-8-((triisopropylsilyl)ethynyl)naphthalen-1-yl)-12-((1-(((3-(difluoromethyl)pyrrolidin-1-methyl)cyclopropyl)methoxy)-1-fluoro-5-methyl-5a, 6,7,8,9,10-hexahydro-5H-4-oxo-3,10a,11,13,14-pentaaza-6,9-methanonaphtho[1,8-ab]hepten-14-carboxylate (58 mg, 0.057 mmol) in N,N-dimethylformamide (2 mL). The reaction mixture was stirred at room temperature for 1 hr. The reaction was shown to be completed by LC/MS. The reaction solution was diluted with ethyl acetate (10 mL), washed with (10 mLX 2) and saturated brine (10 mLX2), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((1-((3-(difluoromethyl)pyrrolidin-1-ylmethyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (49 mg). ES-API: [M+H]⁺= 868.2.

Step V: In an iced water bath, a 4 M hydrochloric acid-dioxane solution (2.5 mL) was slowly added to a solution of t-butyl (5S,5aS,6S,9R)-2-(3-((t-butoxycarbonyl)amino)-8-ethynylnaphthalen-1-yl)-12-((1-((3-(difluoromethyl)pyrrolidin-1-ylmethyl)cyclopropyl)methoxy) -1-fluoro-5-methyl-5a,6,7,8,9,10-hexahydro-5H-4-oxa-3,10a,11,13,14-pentaaza-6,9-methanonaphtho[1,8-ab]hepten-14-carboxylate (49 mg, 0.056 mmol) in methanol (1 mL). The reaction mixture was heated to room temperature and stirred for 1 hr. The reaction was shown to be completed by LC/MS. The reaction solution was concentrated to dryness under reduced pressure, and the residue was purified by preparative HPLC (ammonium bicarbonate method 2) to obtain 4-((5S,5aS,6S,9R)-12-((1-((3-(difluoromethyl)pyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine as a white solid (Z744, 3.31 mg, yield: 9%). ES-API: [M+H]⁺= 668.0. ¹H NMR (400 MHz, DMSO-d₆) δ 7.69- 7.65 (m, 1 H), 7.30- 7.25 (m, 2 H), 6.99- 6.86 (m, 2 H), 5.64 (d, J= 12.0 Hz, 2 H), 5.17- 5.13 (m, 1 H), 4.51-4.41 (m, 1 H), 4.26- 4.20 (m, 2 H), 3.98-3.96 (m, 1 H), 3.81-3.52 (m, 2 H), 3.49- 3.47 (m, 1 H), 3.17 (bs, 2 H), 3.07- 3.01 (m, 1 H), 2.67- 2.62 (m, 2 H), 2.46- 2.42 (m, 2 H), 2.38- 2.33 (m, 3 H), 1.85- 1.53 (m, 4 H), 1.45-1.42 (m, 3 H), 0.65- 0.63 (m, 2 H), 0.47- 0.46 (m, 2 H).

### Example 132: Synthesis of 4-((5S,5aS,6S,9R)-12-((1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z745)

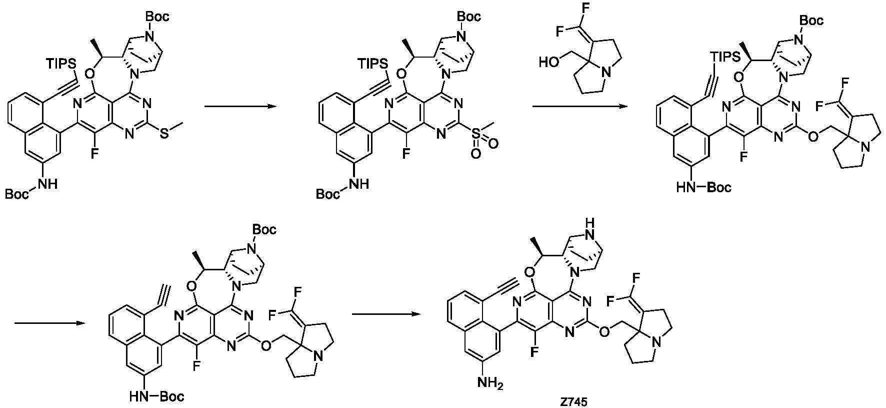

Step I: T-butyl (5S,5aS,6S,9R)-2-(4-((t-butoxycarbonyl)amino)-8-((triisopropylsilyl)ethynyl) naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab] hepten-14-carboxylate (130 mg, 0.150 mmol) was dissolved in dichloromethane (5 mL). At room temperature, m-chloroperoxybenzoic acid (purity 85%, 91.10 mg, 0.449 mmol) was added to the solution. After that, the reaction was stirred at room temperature for 1 hr. The reaction solution was added with a saturated sodium thiosulphate solution (5 mL) and extracted with dichloromethane (10 mL*3). The organic phases were combined, and then washed sequentially with a saturated sodium bicarbonate aqueous solution (20 mL) and saturated sodium chloride (20 mL). The organic phase was dried and concentrated to obtain t-butyl (5S,5aS,6S,9R)-2-(4-((t-butoxycarbonyl)amino)-8-((triisopropylsilyl)ethynyl)naphthalen-1-yl)-1-fluoro-5-methyl-12-(methylsulfonyl)-5a,6,7,8,9,10-hexahydro-5H-4-oxa-3,10a,11,13,14-pentaaza-6,9-methanonaphtho[1,8-ab] hepten-14-carboxylate (120 mg, 0.133 mmol, yield: 89.03%) as a light yellow solid. ES-API: [M +H]⁺= 901.0.

Step II: (1-(difluoromethylene)tetrahydro-1H-pyrrolizin-7a(5H)-yl)methanol (62.99 mg, 0.333 mmol) was dissolved in tetrahydrofuran (4 mL) and cooled to 0°C. Under a nitrogen atmosphere, sodium hydride (60%, 33.29 mg, 0.832 mmol) was added to the solution and stirred for 30 min. Then, t-butyl (5S,5aS,6S,9R)-2-(3-((t-butoxycarbonyl)amino)-8-((triisopropylsilyl)ethynyl) naphthalen-1-yl)-1-fluoro-5-methyl-12-(methylsulfonyl)-hexahydro-5H-4-oxa-3,10a,11,13,14-pentaaza-6,9-methanonaphtho[1,8-ab]hepten-14-carboxylate (150 mg, 0.166 mmol) was slowly added to the solution, and stirred at 0°C for 2 hrs. The reaction solution was added with water (20 mL) to quench the reaction, and then extracted with ethyl acetate (20 mLX3). The organic phase was washed with saturated brine (10 mLX3), dried over anhydrous sodium sulfate, filtered and concentrated to obtain a crude product. The crude product was purified by flash silica gel column chromatography (methanol /dichloromethane: 0-3%) to obtain t-butyl (5S,5aS,6S,9R)-2-(3-((t-butoxycarbonyl)amino)-8-((triisopropylsilyl)ethynyl)naphthalen-1-yl)-12-((1-(difluoromethylene) tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol, yield: 59.47%) as a light yellow solid. ES-API: [M+H]⁺=1010.3.

Step III: A mixed solution of t-butyl (5S,5aS,6S,9R)-2-(3-((t-butoxycarbonyl)amino)-8-((triisopropylsilyl)ethynyl)naphthalen-1-yl)-12-((1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol), cesium fluoride (150.35 mg, 0.990 mmol), and *N, N*-dimethylformamide (5 mL) were stirred at room temperature for 2 hrs. The reaction mixture was added with water (30 mL) and extracted with ethyl acetate (20 mLX). The organic phase was washed with saturated brine (30 mLX3), dried over anhydrous sodium sulfate, filtered and concentrated, to obtain t-butyl (5S,5aS,6S,9R)-2-(3-((t-butoxycarbonyl)amino)-8-ethynylnaphthalen-1-yl)-12-((1-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol, yield: 88.73%) as a light yellow solid. ES-API: [M+H]⁺=854.2.

Step IV: T-butyl (5S,5aS,6S,9R)-2-(3-((t-butoxycarbonyl)amino)-8-ethynylnaphthalen-1-yl)-12-((1-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol) was dissolved in ethyl acetate (2 mL), and cooled to 0°C. A hydrogen chloride/dioxane solution (4 M, 4 mL) was added to the solution, and reacted with stirring at 0 °C for 2 hrs. The reaction solution was concentrated, and the residue was taken up in dichloromethane (2 mL) again and alkalized with ammonia in methanol (7 M) to an alkaline pH (pH 8). The obtained solution was concentrated, and the crude product was purified by preparative HPLC (ammonium bicarbonate method 1) to obtain 4-((5S,5aS,6S,9R)-12-((1-(difluoromethylene)tetrahydro-1H-pyrrolizin-7a(5H)-yl)methoxy)-1-fluoro-5-methyl-5a,6, 7,8,9,10-hexahydro-5H-4-oxa-3,10a,11,13,14-pentaaza-6,9-methanonaphtho[1,8-ab]hepten-2-yl)-5-ethynylnaphthalen-2-amine (Z745, 18 mg, yield: 26.13%) as a white solid. ES-API: [M+H]⁺=654.2. ¹H NMR (400 MHz, DMSO-d₆) δ 7.71 - 7.63 (m, 1H), 7.34 - 7.22 (m, 2H), 7.03 - 6.85 (m, 2H), 5.64 (d, *J* = 11.6 Hz, 2H), 5.17 - 5.10 (m, 1H), 4.56 - 4.38 (m, 1H), 4.33 - 4.18 (m, 2H), 3.96 (d, *J* = 9.2 Hz, 1H), 3.83 - 3.42 (m, 3H), 3.14 - 3.00 (m, 2H), 2.98 - 2.90 (m, 1H), 2.79 - 2.71 (m, 1H), 2.69 - 2.51 (m, 3H), 2.10 - 1.97 (m, 1H), 1.97 - 1.76 (m, 4H), 1.73- 1.50 (m, 3H), 1.44 (t, *J =* 6.8 Hz, 3H).

### Example 133: Synthesis of (5aS,6S,9R)-2-(8-ethynyl-7-fluoronaphthalen-1-yl)-1-fluoro-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z746)

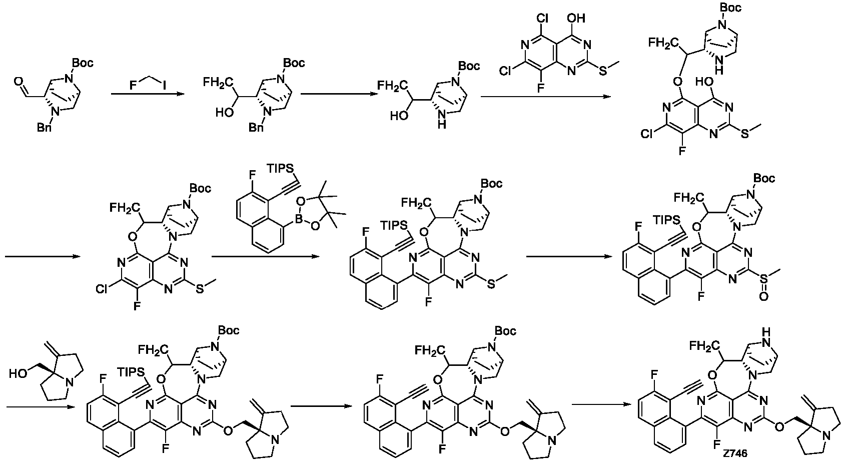

Step I: T-butyl (1S,2S,5R)-3-benzyl-2-formyl-3,8-diazabicyclo[3.2.1]octane-8-carboxylate (310 mg, 0.94 mmol) was dissolved in dry tetrahydrofuran(5 mL) and anhydrous ether (5 mL). At -78°C, fluoroiodomethane (100 mg, 0.63 mmol) was added, and then a solution of methyl lithium-lithium bromide complex in ether (0.83 mL, 1.25 mmol, 1.5M) was added dropwise, and reacted with stirring at -78°C for 5 min. The reaction solution was quenched with a saturated ammonium chloride solution (1 mL), added with water (10 mL), and extracted with ethyl acetate (30 mL). The organic phase was washed with saturated brine (15 mL), dried over anhydrous sodium sulfate, filtered, and concentrated. The crude product was purified by flash silica gel column chromatography (tetrahydrofuran/petroleum ether: 0-15%) to obtain t-butyl (15,2S,5R)-3-benzyl-2-(2-fluoro-1-hydroxyethyl)-3,8-diazadicyclo[3.2.1]octane-8-carboxylate (200 mg, yield: 87.8%) as a colorless liquid. ES-API: [M+H]⁺= 365.0

Step II: T-butyl (1S,2S,5R)-3-benzyl-2-(2-fluoro-1-hydroxyethyl)-3,8-diazadicyclo [3.2.1] octane-8-carboxylate (600 mg, 1.65 mmol) was dissolved in methanol (15 mL) and a 7M aminomethanol solution (1 mL). 10% palladium on carbon (250 mg) was added, and the reaction was stirred under a hydrogen atmosphere at room temperature for 18 hrs. The reaction solution was filtered through diatomite and the filter cake was washed with methanol. The filtrate was concentrated to obtain the target product t-butyl (1S,2S,5R)-2-(2-fluoro-1-hydroxyethyl)-3,8-diazadicyclo[3.2.1]octane-8-carboxylate (451 mg, yield: 99.9%) as a colorless liquid. ES-API: [M+H]⁺= 275.0

Step III: T-butyl (1S,2S,5R)-2-(2-fluoro-1-hydroxyethyl)-3,8-diazadicyclo[3.2.1]octane-8-carboxylate (451 mg,1.64 mmol) was dissolved in tetrahydrofuran (20 mL). At 0°C, 60%sodium hydride (395 mg, 9.86 mmol) was added, and the reaction was stirred at 0°C for 30 min. 5,7-dichloro-8-fluoro-2-(methylthio)pyridine[4,3-D]pyrimidine-4(3H)-one (599 mg, 2.14 mmol) was added, and the reaction was stirred at room temperature for 5 hrs. The reaction solution was quenched with a saturated ammonium chloride solution (3 mL) and water (15 mL), and extracted with ethyl acetate (80 mL). The organic phases were combined and washed with saturated brine (30 mL). The organic phase was dried over anhydrous sodium sulfate, fitlered, and concentrated. The crude product was purified by flash silica gel column chromatography (tetrahydrofuran/petroleum ether: 0-70%) to obtain the target product t-butyl (1R,2S,5S)-2-(1-((7-chloro-8-fluoro-4-hydroxyl-2-(methylthio)pyrimidino[4,3-d]pyrimidine-5-yl)oxy)-2-fluoroethyl)-3,8-diazadicyclo[3.2.1]octane-8-carboxylate (700 mg, yield: 82.2%) as a light yellow solid. ES-API: [M+H]⁺= 517.9

Step IV: T-butyl (1R,2S,5S)-2-(1-((7-chloro-8-fluoro-4-hydroxyl-2-(methylthio)pyrimidino [4,3-d]pyrimidine-5-yl)oxy)-2-fluoroethyl)-3,8-diazadicyclo[3.2.1]octane-8-carboxylate (700 mg, 1.35 mmol) and diisopropylethyl amine (1.22 g, 9.46 mmol) were dissolved in dichloromethane (20 mL). At room temperature, a 50% 1-propylphosphoric anhydride solution in ethyl acetate (4.30 g, 6.76 mmol) was added, and the reaction was stirred at room temperature for 18 hrs. The reaction solution was added with dichloromethane (50 mL), sequentially washed with water (25 mL×2) and saturated brine (25 mL), dried over anhydrous sodium sulfate, filtered and concentrated. The crude product was purified by flash silica gel column chromatography (tetrahydrofuran/petroleum ether: 0-20%) to obtain the target product t-butyl (5aS,6S,9R)-2-chloro-1-fluoro-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (240 mg, yield: 35.5%) as a white solid. ES-API: [M+H]⁺= 499.9

Step V: T-butyl (5aS,6S,9R)-2-chloro-1-fluoro-5-(fluoromethyl)-12-(methylthio)-6a,6,7,8,9, 10-hexahydro-5H-4-oxa-3,10a,11,13,14-pentaaza-6,9-methanonaphtho[1,8-ab]hepten-14-carboxylate (120 mg, 0.24 mmol), ((2-fluoro-8-(4,4,5,5-tetramethyl-1,3,2-dioxaborolan-2-yl)naphthalen-1-yl)ethynyl)triisopropylsilane (162 mg, 0.36 mmol), potassium phosphate (153 mg, 0.72 mmol), [n-butyldi(1-adamantanyl)phosphine](2-amino-1,1'-biphenyl-2-yl)palladium (II) mesylate (18 mg, 0.025 mmol), tetrahydrofuran(2.5 mL) and water (0.5 mL) were added to a 5 mL microwave tube, and purged with nitrogen for 30. The reaction was stirred at 80°C in a microwave reactor for 30 min. The reaction solution was added with ethyl acetate (40 mL) and water (10 mL). The organic phase was separated, washed with saturated brine (10 mL), dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5aS,6S,9R)-1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (110 mg, yield: 58.0%) as a light yellow solid. ES-API: [M+H]⁺=790.3.

Step VI: T-butyl (5aS,6S,9R)-1-fluoro-2-(7-fluoro-8-((triisopropylsilyl)ethynyl)naphthalen-1-yl)-5-(fluoromethyl)-12-(methylthio)-5a,6,7,8,9,10-hexahydro-5H-4-oxa-3,10a,11,13,14-pentaaza-6,9-methanonaphtho[1,8-ab]hepten-14-carboxylate (110 mg, 0.14 mmol) was dissolved in dichloromethane (12 mL). At room temperature, 85%m-chloroperoxybenzoic acid (42 mg, 0.21 mmol) was added, and reacted with stirring in an ice bath for 1 hr. The reaction solution was added with dichloromethane (30 mL), and sequentially washed with saturated sodium thiosulphate (2 mL), saturated sodium bicarbonate (15 mL), and saturated brine (15 mL). The organic phase was dried over anhydrous sodium sulfate, filtered and concentrated, to obtain a crude product. The crude product was purified by flash silica gel column chromatography (ethyl acetate /petroleum ether: 0-100%) to obtain the target product t-butyl (5aS,6S,9R)-1-fluoro-2-(7-fluoro-8-((triisopropylsilyl)ethynyl)naphthalen-1-yl)-5-(fluoromethyl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (90 mg, yield: 80.2%) as a light yellow solid. ES-API:[M+H]⁺=806.3.

Step VII: (R)-(1-methylenetetrahydro-1H-pyrrolizin-7a(5H)-yl)methanol (86 mg, 0.56 mmol) was dissolved in tetrahydrofuran(10 mL). At 0°C, 60% sodium hydride (54 mg, 1.34 mmol) was added, and reacted with stirring at this temperature for 30 min. Then a solution of t-butyl (5aS,6S,9R)-1-fluoro-2-(7-fluoro-8-((triisopropylsilyl)ethynyl)naphthalen-1-yl)-5-(fluoromethyl)-12-(methylthionyl)-5a,6,7,8,9,10-hexahydro-5H-4-oxa-3,10a,11,13,14-pentaaza-6,9-methanonaphtho[1,8-ab]hepten-14-carboxylate (180 mg, 0.22 mmol) in tetrahydrofuran (2 mL) was added dropwise, and the reaction was stirred in an ice bath for 30 min. The reaction solution was quenched with a saturated ammonium chloride solution (2 mL) and water (5 mL), and extracted acetate (40 mL). The organic phase was washed with saturated brine (15 mL), dried over anhydrous sodium sulfate, and filtered. The filtrate was concentrated under reduced pressure. The crude product was purified by preparative TLC (methanol /dichloromethane=20: 1) to obtain the target product t-butyl (5aS,6S,9R)-1-fluoro-2-(7-fluoro-8-((triisopropylsilyl)ethynyl)naphthalen-1-yl)-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (120 mg, yield: 60.0%) as a light yellow solid. ES-API: [M+H]⁺=895.3.

Step VIII: T-butyl (5aS,6R,9R)-1-fluoro-2-(7-fluoro-8-((triisopropylsilyl)ethynyl)naphthalen-1-yl)-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (120 mg, 0.13 mmol) was dissolved in N,N-dimethylformamide (5 mL). At room temperature, cesium fluoride (204 mg, 1.10 mmol) was added, and the reaction was stirred at room temperature for 1 hr. The reaction solution was added with ethyl acetate (50 mL), and sequentially washed with water (15 mL), dilute saline (15 mLX3), and saturated brine (15 mL). The organic phase was dried over anhydrous sodium sulfate, and filtered. The filtrate was concentrated under reduced pressure. The crude product was purified by flash silica gel column chromatography (methanol /dichloromethane: 0-10%) to obtain the target product t-butyl (5aS,6S,9R)-2-(8-ethynyl-7-fluoronaphthalen-1-yl)-1-fluoro-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (90 mg, yield: 98.9%) as an off-white solid. ES-API: [M+H]⁺=739.2.

Step IX: T-butyl (5aS,6S,9R)-2-(8-ethynyl-7-fluoronaphthalen-1-yl)-1-fluoro-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (90 mg, 0.12 mmol) was dissolved in acetonitrile (2 mL). At 0°C, 4 M hydrogen chloride in 1,4-dioxane (2 mL) was added, and the reaction was stirred at room temperature for 1 hr. The reaction solution was concentrated under reduced pressure, added with saturated sodium bicarbonate (6 mL), and extracted with ethyl acetate (25 mLX2). The organic phases were combined, washed with saturated brine (15 mL), dried over anhydrous sodium sulfate, and concentrated. The crude product was purified by preparative HPLC (ammonium bicarbonate method 1) to obtain the target product (5aS,6S,9R)-2-(8-ethynyl-7-fluoronaphthalen-1-yl)-1-fluoro-5-(fluoromethyl)-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (Z746, 40 mg, yield: 51.4%) as a white solid. ES-API: [M+H]⁺= 639.2. ¹H NMR (400 MHz, CD₃OD) δ 8.16 - 8.04 (m, 2H), 7.67 - 7.54 (m, 2H), 7.43 (dd, *J* = 16.8, 8.8 Hz, 1H), 5.45 - 5.35 (m, 1H), 5.13 - 5.06 (m, 2H), 5.03 - 4.93 (m, 1H), 4.92 -4.79 (m, 1H), 4.71 - 4.54 (m, 1H), 4.45 - 4.30 (m, 3H), 3.78 - 3.47 (m, 3H), 3.25 - 3.16 (m, 2H), 3.15 - 3.07 (m, 1H), 2.87 - 2.77 (m, 1H), 2.75 - 2.60 (m, 3H), 2.23 - 2.02 (m, 2H), 2.01 - 1.72 (m, 6H).

### Example 134: Synthesis of 4-((5S,5aS,6S,9R)-12-((2-(difluoromethyl)tetrahydro-1H-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]hepten-2-yl)-5-ethynylnaphthalen-2-amine (Z747)

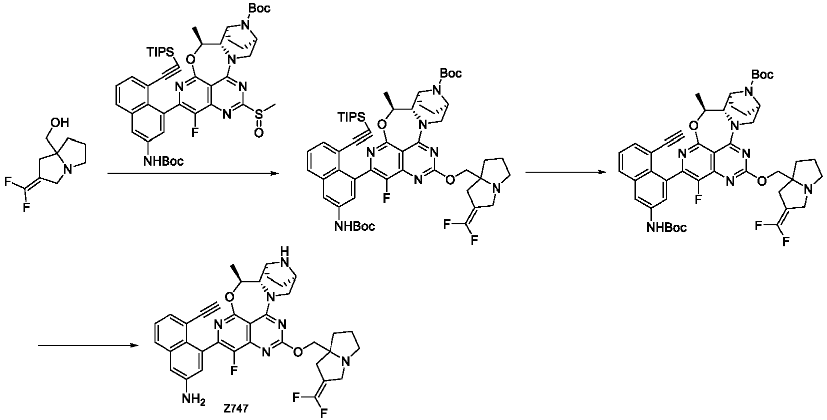

Step I: ((2-(difluoromethyl)tetrahydro-1H-pyrrolizin-7a(5H)-yl)methanol (40mg, 0.21 mmol) was dissolved in anhydrous tetrahydrofuran (8 mL). In an iced water bath, 60% sodium hydride (18 mg, 0.45 mmol) was added, and reacted at room temperature for 10 min. The reaction solution was cooled to 0°C. A solution of t-butyl (5S,5aS,6S,9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, 0.11 mmol) in tetrahydrofuran (2 mL) was added dropwise to the reaction solution, and reacted at room temperature for 30 min. The reaction solution was quenched with water (50 mL), extracted with ethyl acetate (30 mLX2), washed with saturated brine (60 mL), dried over anhydrous sodium sulfate, filtered, and concentrated to obtain t-butyl (5S,5aS,6S,9R)-2-(3-((t-butoxycarbonyl)amino)-8-((triisopropylsilyl)ethynyl)naphthalen-1-yl)-12-((2-(difluoromethyl)tetrahydro-1H-pyrrolizin-7a(5H)-yl)methoxy)-l-fluoro-5-methyl-5a,6,7,8,9,10-hexahydro-5H-4-oxa-3,10a,11,13,14-pentaaza-6,9-methanonaphtho[1,8-ab]hepten-14-carboxylate (114 mg, crude product). ES-API: [M+H]⁺=1010.3.

Step II: Cesium fluoride (171 mg, 1.12 mmol) was added to t-butyl (5S,5aS,6S,9R)-2-(3-((t-butoxycarbonyl)amino)-8-((triisopropylsilyl)ethynyl)naphthalen-1-yl)-12-((2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (114 mg, crude product) in N,N-dimethylformamide (3 mL), and the reaction mixture was stirred at room temperature for 2 hrs. The reaction was shown to be completed by LC/MS. The reaction solution was diluted with ethyl acetate (30 mL), washed with saturated brine (30 mLX3), dried over anhydrous sodium sulfate, filtered, and concentrated to obtain t-butyl (5S,5aS,6S,9R)-2-(3-((t-butoxycarbonyl)amino)-8-ethynylnaphthalen-1-yl)-12-((2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (96 mg, crude product). ES-API: [M+H]⁺= 854.0

Step III: In an iced water bath, a 4 M hydrochloric acid-dioxane solution (2 mL) was slowly added to a solution of t-butyl (5S,5aS,6S,9R)-2-(3-((t-butoxycarbonyl)amino)-8-ethynylnaphthalen-1-yl)-12-((2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (96 mg, crude product) in ethyl acetate (2 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate method 1) to obtain 4-((5S,5aS,6S,9R)-12-((2-(difluoromethyl)tetrahydro-1H-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]hepten-2-yl)-5-ethynylnaphthalen-2-amine (Z747, 6 mg, yield: 8%). ES-API: [M+H]⁺= 654.2. ¹H NMR (400Hz, DMSO-d₆) 7.67-7.68(m, 1H), 7.28-7.25(m,2H), 6.99-6.86(m,2H), 5.65-5.62 (m,2H), 5.15-5.11(m,1H), 4.52-4.48(m,1H), 4.08-4.00(m,2H), 3.98-3.47(m,5H), 3.04-3.02(m,2H), 2.61-2.51(m,2H), 2.48-2.32(m,2H), 1.96-1.65(m,8H), 1.50-1.43(m,3H).

### Example 135: Synthesis of 5-ethynyl-3-fluoro-4-((5S, 5aS, 6S, 9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z957)

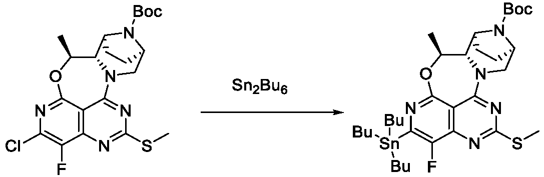

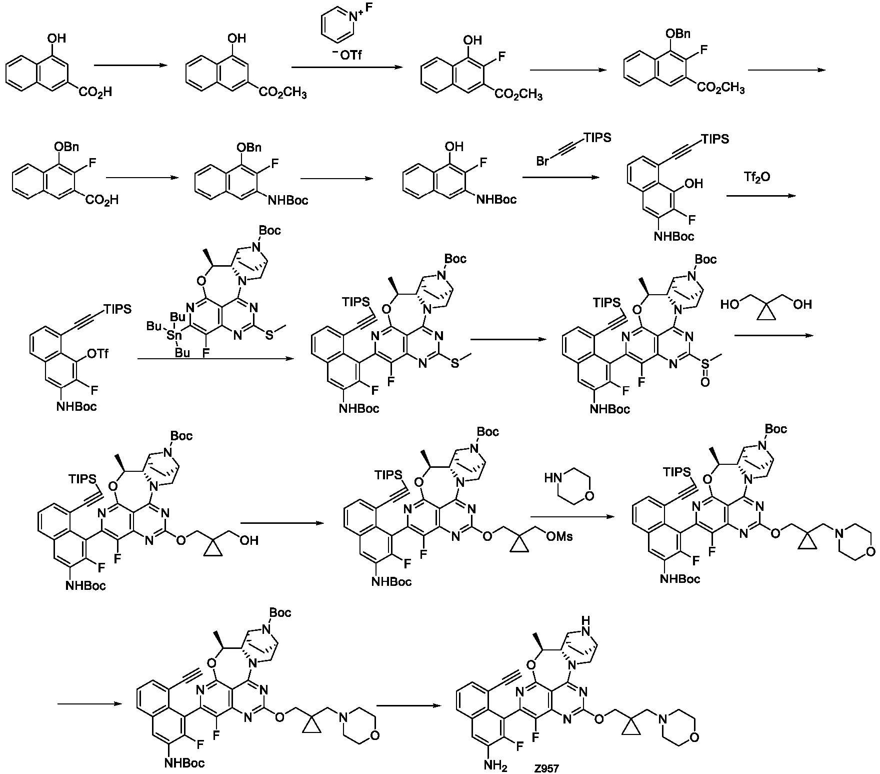

Step I: A mixture of t-butyl (5S, 5aS, 6S, 9R)-2-chloro-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (500 mg, 1.04 mmol), hexabutylditin (1.81 g, 3.11 mmol), tris (dibenzylideneacetone) dipalladium (95 mg, 0.10 mmol), tricyclohexyl phosphine(58 mg, 0.21 mmol), and lithium chloride (220 mg, 5.19 mmol) was dissolved in 1,4-dioxane (15 mL), purged 3 times with nitrogen, and stirred at 110°C for 18 hrs. The reaction solution was cooled to room temperature and concentrated. The crude product was purified by flash silica gel column chromatography (tetrahydrofuran/petroleum ether: 0-15%) to obtain the target product t-butyl (5S,5aS,6S,9R)-1-fluoro-5-methyl-12-(methylthio)-2-(tributyltin)-5a,6,7,8,9,10-hexahydro-5H-4-oxa-3,10a,11,13,14-pentaaza-6,9-methanonaphtho[1,8-ab]hepten-14-carboxylate (400 mg, yield: 52.3%) as a yellow liquid. ES-API: [M+H]⁺=738.2.

Step II: 4-hydroxyl-2-naphthoic acid (6.5 g, 34.54 mmol) was suspended in anhydrous methanol (100 mL). Concentrated sulfuric acid (3.7 mL, 69.08 mmol) was added, and reacted with stirring at 80°C for 16 hrs. The reaction solution was concentrated, diluted with ethyl acetate (300 mL), washed with a saturated sodium bicarbonate solution (80 mL×2) and saturated brine (80 mL). The organic phase was dried over anhydrous sodium sulfate, filtered and concentrated. The crude product was purified by flash silica gel column chromatography (ethyl acetate /petroleum ether: 0-20%) to obtain the target product methyl 4-hydroxylnaphthalen-2-carboxylate (6.5 g, yield: 93.1%) as an orange solid. ES-API: [M+H]⁺= 203.1.

Step III: Methyl 4-hydroxylnaphthalen-2-carboxylate (6.0 g, 29.67 mmol) was dissolved in chlorobenzene (100 mL). N-fluoropyridine trifluoromethanesulfonate (8.07 g, 32.64 mmol) was added, and the reaction was heated to reflux and stirred for 16 hrs. The reaction solution was cooled to room temperature, concentrated under reduced pressure, added with ethyl acetate (150 mL), and sequentially washed with 1N dilute hydrochloric acid (50 mL ×3), a saturated sodium bicarbonate solution (50 mL), and saturated brine (50 mL). The organic phase was dried over anhydrous sodium sulfate, and filtered. The filtrate was concentrated under reduced pressure. The crude product was purified by flash silica gel column chromatography (ethyl acetate /petroleum ether: 0-12%) to obtain the target product methyl 3-fluoro-4-hydroxyl-2-naphthoate (3.4 g, yield: 52.0%) as a light yellow solid. ¹H NMR (400 MHz, DMSO-*d₆*) δ 10.50 (s, 1H), 8.17 (d, *J* = 8.4 Hz, 1H), 8.02 (dd, *J* = 7.2, 4.0 Hz, 2H), 7.63 (t, *J =* 7.6 Hz, 1H), 7.54 (t, *J* = 7.6 Hz, 1H), 3.90 (S, 3H). ES-API: [M+H]⁺= 221.1.

Step IV: Methyl 3-fluoro-4-hydroxyl-2-naphthoate (3.4 g, 15.44 mmol) was dissolved in N, N-dimethylformamide (30 mL). Potassium carbonate (5.34 g, 38.60 mmol) and benzyl bromide (3.43 g, 20.07 mmol) were sequentially added, and the reaction was stirred at room temperature for 16 hrs. The reaction mixture was added with water (80 mL) and extracted with ethyl acetate (120 mL). The organic phase was washed with saturated brine (50 mL×3), dried over anhydrous sodium sulfate, and filtered. The filtrate was concentrated under reduced pressure. The crude product was slurried in petroleum ether to obtain the target product methyl 4-(benzyloxy)-3-fluoro-2-naphthoate (3.5 g, yield: 73.0%) as a white solid. ¹H NMR (400 MHz, DMSO-*d₆*) δ 8.35 (d, *J* = 6.4 Hz, 1H), 8.11 (dd, *J* = 11.6, 8.4 Hz, 2H), 7.69 (t, *J* = 7.6 Hz, 1H), 7.59 (t, *J* = 7.6 Hz, 1H), 7.54 (d, *J* = 6.8 Hz, 2H), 7.46-7.33 (m, 3H), 5.28 (s, 2H), 3.93 (s, 3H). eS-API: [M+H]⁺= 311.2.

Step V: Methyl 4-(benzyloxy)-3-fluoro-2-naphthoate (3.35 g, 10.80 mmol) was dissolved in tetrahydrofuran (60 mL), methanol (20 mL) and water (20 mL). Lithium hydroxide (1.36 g, 32.39 mmol) was added, and the reaction solution was stirred at room temperature for 1 hr. The reaction solution was adjusted to pH<1 with 1N dilute hydrochloric acid, and concentrated under reduced pressure to remove the organic solvent. The precipitated solid was filtered, and dried under vacuum to obtain the target product 4-(benzyloxy)-3-fluoro-2-naphthalencarboxylic acid (3.1 g, yield: 96.9%) as a white solid. ES-API: [M+H]⁺= 297.1.

Step VI: (4-(benzyloxy)-3-fluoro-2-naphthalencarboxylic acid (3.1 g, 10.46 mmol) and triethyl amine (2.91 mL, 20.93 mmol) was dissolved in t-butanol (60 mL) and toluene (12 mL). Diphenylphosphoryl azide (3.38 mL, 15.69 mmol) was added, and the reaction solution was heated to reflux and stirred for 6 hrs. The reaction solution was concentrated under reduced pressure, added with ethyl acetate (100 mL), and sequentially washed with water (30 mL) and saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-12%) to obtain the target product t-butyl (4-(benzyloxy)-3-fluoronaphthalen-2-yl)carbamate (3.5 g, yield: 91.1%) as a white solid. ES-API: [M+Na]⁺= 390.1.

Step VII: T-butyl (4-(benzyloxy)-3-fluoronaphthalen-2-yl)carbamate (3.15 g, 8.57 mmol) was dissolved in methanol (40 mL) and tetrahydrofuran(4 mL). 10% palladium on carbon (1 g) was added, and the reaction was stirred under a hydrogen atmosphere at room temperature for 3 hrs. The reaction solution was filtered through diatomite and the filter cake was washed with methanol. The filtrate was concentrated to obtain the target product t-butyl (3-fluoro-4-hydroxylnaphthalen-2-yl)carbamate (2.37 g, yield: 100%) as a light pink solid. ES-API: [M-55]⁺= 221.9.

Step VIII: T-butyl (3-fluoro-4-hydroxylnaphthalen-2-yl)carbamate (2.37g, 8.55 mmol) and (2-bromoethynyl)triisopropylsilane (2.68 g, 10.26 mmol) were dissolved in 1, 4-dioxane (50 mL). At room temperature, potassium acetate (168 g, 17.09 mmol) and dichloro(p-methylisopropylphenyl)ruthenium (II) dimer (54 mg, 0.088 mmol) were added, purged 3 times with nitrogen, and stirred at 100°C for 1 hr. The reaction solution was filtered, and the filtrate was concentrated under reduced pressure. The crude product was purified by flash silica gel column chromatography (ethyl acetate /petroleum ether: 0-3%) to obtain the target product t-butyl (3-fluoro-4-hydroxyl-5-((triisopropylsilyl)ethynyl)naphthalen-2-yl)carbamate (3.75 g, yield: 95.9%) as a light yellow solid. ES-API: [M+H]⁺= 458.0

Step IX: T-butyl (3-fluoro-4-hydroxyl-5-((triisopropylsilyl)ethynyl)naphthalen-2-yl) carbamate (3.55 g, 7.58 mmol) and N,N-diisopropylethyl amine (3.38 mL, 19.39) were dissolved in dichloromethane (50 mL). At -40°C, trifluoromethanesulfonic anhydride (3.28 g, 11.64 mmol) was added, and reacted with stirring at -40°C for 1 hr. The reaction solution was added with water (50 mL), and extracted with dichloromethane (50 mL). The organic phase was washed with saturated brine (30 mL), dried over anhydrous sodium sulfate, and filtered. The filtrate was concentrated under reduced pressure,. The crude product was purified by flash silica gel column chromatography (ethyl acetate /petroleum ether: 0-3%) to obtain the target product 3-((t-butoxycarbonyl)amino)-2-fluoro-8-((triisopropylsilyl)ethynyl)naphthalen-1-yl trifluoromethylsulfonate (4.5 g, yield: 98.4%) as a light yellow solid. ES-API: [M+H]⁺=590.0.

Step X: A mixture of t-butyl (5S, 5aS, 6S, 9R)-1-fluoro-5-methyl-12-(methylthio)-2-(tributyltin)-5a,6,7,8,9,10-hexahydro-5H-4-oxa-3,10a,11,13,14-pentaaza-6,9-methanonaphtho [1,8-ab]hepten-14-carboxylate (400 mg, 0.54 mmol) and 3-((t-butoxycarbonyl)amino)-2-fluoro-8-((triisopropylsilyl)ethynyl)naphthalen-1-yl trifluoromethylsulfonate (480 mg, 0.82 mmol) were dissolved in anhydrous N, N-dimethylacetamide (12 mL). Cuprous iodide (103 mg, 0.54 mmol), terakis(triphenylphosphine)palladium (94 mg, 0.08 mmol) and lithium chloride (92 mg, 2.17 mmol) were added, purged 3 times with nitrogen, and stirred at 120°C for 3 hrs. The reaction mixture was added with water (30 mL) and extracted with ethyl acetate (100 mL). The organic phase was washed with dilute saline (50 mL×3), dried over anhydrous sodium sulfate, and filtered. The filtrate was concentrated under reduced pressure. The crude product was purified by flash silica gel column chromatography (tetrahydrofuran/petroleum ether: 0-20%) to obtain the target product t-butyl (5S, 5aS, 6S, 9R)-2-(3-((t-butoxycarbonyl)amino)-2-fluoro-8-((triisopropylsilyl)ethynyl) naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (90 mg, yield: 18.7%) as a yellow solid. ES-API: [M+H]⁺=887.3.

Step XI: T-butyl (5S, 5aS, 6S, 9R)-2-(3-((t-butoxycarbonyl)amino)-2-fluoro-8-((triisopropylsilyl)ethynyl)naphthalen-1-yl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (90 mg, 0.10 mmol) was dissolved in dichloromethane (15 mL). In an ice bath, m-chloroperoxybenzoic acid (27 mg, 0.13 mmol) was added, and the reaction was stirred in the ice bath for 1 hr. The reaction solution was added with dichloromethane (20 mL), and sequentially washed with saturated sodium thiosulphate (2 mL), saturated sodium bicarbonate (5 mL), and saturated brine (5 mL). The organic phase was dried over anhydrous sodium sulfate, and filtered. The filtrate was concentrated under reduced pressure. The crude product was purified by flash silica gel column chromatography (tetrahydrofuran/petroleum ether: 0-70%) to obtain the target product t-butyl (5S, 5aS, 6S, 9R)-2-(3-((t-butoxycarbonyl)amino)-2-fluoro-8-((triisopropylsilyl) ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (90 mg, yield: 98.2%) as a light yellow solid. ES-API:[M+H]⁺=903.2.

Step XII: 1,1-cyclopropandimethanol (102 mg, 1.0 mmol) was dissolved in tetrahydrofuran (8 mL). At 0°C, 60% sodium hydride (20 mg, 0.50 mmol) was added, and the reaction was stirred at this temperature for 30 min. Then a solution of t-butyl (5S, 5aS, 6S, 9R)-2-(3-((t-butoxycarbonyl)amino)-2-fluoro-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]heptan-14-carboxylate (90 mg, 0.10 mmol) in tetrahydrofuran (2 mL) was added dropwise, and the reaction was stirred at this temperature for 30 min. The reaction solution was added with ethyl acetate (40 mL), and sequentially added with water (10 mL) and saturated brine (15 mL). The organic phase was dried over anhydrous sodium sulfate, and filtered. The filtrate was concentrated under reduced pressure. The crude product was purified by flash silica gel column chromatography (tetrahydrofuran/petroleum ether: 0-40%) to obtain the target product t-butyl (5S, 5aS, 6S, 9R)-2-(3-((t-butoxycarbonyl)amino)-2-fluoro-8-((triisopropylsilyl)ethynyl) naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (10 mg, yield: 10.7%) as a white solid. ES-API: [M+H]⁺=941.4.

Step XIII: T-butyl (5S, 5aS, 6S, 9R)-2-(3-((t-butoxycarbonyl)amino)-2-fluoro-8-((triisopropylsilyl)ethynyl)naphthalen-1-yl)-1-fluoro-12-((1-(hydroxymethyl)cyclopropyl) methoxy)-5-methyl-5a,6,7,8,9,10-hexahydro-5H-4-oxa-3,10a,11,13,14-pentaaza-6,9-methanonaphtho[1,8-ab]hepten-14-carboxylate (70 mg, 0.074 mmol) and diisopropylethyl amine (48 mg, 0.37 mmol) were dissolved in dichloromethane (8 mL). At 0°C, methanesulfonic anhydride (44 mg, 0.26 mmol) was added dropwise, and the reaction mixture was stirred at room temperature for 2 hrs. The reaction was shown to be completed by LC/MS. The reaction solution was diluted with dichloromethane (30 mL), washed with saturated sodium bicarbonate (15 mLX2) and saturated brine (15 mL), dried over anhydrous sodium sulfate, filtered, and concentrated to obtain the target product t-butyl (5S, 5aS, 6S, 9R)-2-(3-((t-butoxycarbonyl)amino)-2-fluoro-8-((triisopropylsilyl)ethynyl)naphthalen-1-yl)-1-fluoro-12-((1-(((methylsulfonyl)oxy)methyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (75 mg, yield: 98.9%) as a white solid. ES-API: [M+H]⁺=1019.3.

Step XIV: T-butyl (5S, 5aS, 6S, 9R)-2-(3-((t-butoxycarbonyl)amino)-2-fluoro-8-((triisopropylsilyl)ethynyl)naphthalen-1-yl)-1-fluoro-12-((1-(((methylsulfonyl)oxy)methyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (75 mg, 0.074 mmol) dissolved in acetone (4 mL), potassium carbonate (51 mg, 0.37 mmol), potassium iodide (12 mg, 0.074 mmol) and morpholine (64 mg, 0.74 mmol) were added to a 15 mL sealed tube, and the reaction was stirred at 80°C for 16 hrs. The reaction mixture was added with water (10 mL) and extracted with ethyl acetate (50 mL). The organic phase was washed with saturated brine (25 mL), dried over anhydrous sodium sulfate, and filtered. The filtrate was concentrated under reduced pressure. The crude product was purified by flash silica gel column chromatography (methanol /dichloromethane: 0-8%) to obtain the target product t-butyl (5S, 5aS, 6S, 9R)-2-(3-((t-butoxycarbonyl)amino)-2-fluoro-8-((triisopropylsilyl)ethynyl)naphthalen-1-yl)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]heptan-14-carboxylate (70 mg, yield: 94.2%) as a yellow solid. ES-API: [M+H]⁺= 1010.3.

Step XV: T-butyl (5S, 5aS, 6S, 9R)-2-(3-((t-butoxycarbonyl)amino)-2-fluoro-8-((triisopropylsilyl)ethynyl)naphthalen-1-yl)-1-fluoro-5-methyl-12-((1-(morpholinemethyl) cyclopropyl)methoxy)-5a,6,7,8,9,10-hexahydro-5H-4-oxa-3,10a,11,13,14-pentaaza-6,9-methanonaphtho[1,8-ab]heptan-14-carboxylate (70 mg, 0.069 mmol) was dissolved in N,N-dimethylformamide (3 mL). At room temperature, cesium fluoride (105 mg, 0.69 mmol) was added, and the reaction was stirred at room temperature for 1 hr. The reaction solution was added with ethyl acetate (50 mL), and sequentially washed with water (10 mL), dilute saline (15 mL×3), and saturated brine (15 mL). The organic phase was dried over anhydrous sodium sulfate, and filtered. The filtrate was concentrated under reduced pressure, to obtain the target product t-butyl (5S, 5aS, 6S, 9R)-2-(3-((t-butoxycarbonyl)amino)-8-ethynyl-2-fluoronaphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]heptan-14-carboxylate (59 mg, yield: 100%) as a yellow solid. ES-API: [M+H]⁺=854.3.

Step XVI: T-butyl (5S, 5aS, 6S, 9R)-2-(3-((t-butoxycarbonyl)amino)-8-ethynyl-2-fluoronaphthalen-1-yl)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]heptan-14-carboxylate (54 mg, 0.063 mmol) was dissolved in ethyl acetate (3 mL). At 0°C, 4M hydrogen chloride in 1, 4-dioxane (3 mL) was added, and the reaction was stirred at room temperature for 2 hrs. The reaction solution was concentrated under reduce pressure. The reaction solution was concentrated under reduced pressure, added with saturated sodium bicarbonate (6 mL), and extracted with ethyl acetate (25 mL×2). The organic phases were combined, washed with saturated brine (15 mL), dried over anhydrous sodium sulfate, and concentrated. The crude product was purified by preparative HPLC (ammonium bicarbonate method 1) to obtain 5-ethynyl-3-fluoro-4-((5S,5aS,6S,9R)-1-fluoro-5-methyl-12-((1-(morpholinemethyl)cyclopropyl)methoxy)-5a,6,7,8,9,10-hexahydro-5H-4-oxa-3,10a,11,13,14-pentaaza-6,9-methanonaphtho[1,8-ab]hepten-2-yl)naphthalen-2-amine (Z957, 17 mg, yield: 41.1%) as a white solid. ¹H NMR (400 MHz, CD₃OD) δ 7.73 - 7.66 (m, 1H), 7.48 - 7.38 (m, 1H), 7.33 - 7.24 (m, 2H), 5.44 - 5.32 (m, 1H), 4.59 - 4.35 (m, 3H), 4.08 (d, *J* = 8.8 Hz, 1H), 3.75 - 3.56 (m, 6H), 3.33 (s, 0.5H), 3.14 - 3.23 (m, 1H), 3.00 (s, 0.5H), 2.61 - 2.34 (m, 6H), 2.14 - 2.02 (m, 1H), 1.94 - 1.71 (m, 3H), 1.63 - 1.51 (m, 3H), 0.75 - 0.67 (m, 2H), 0.56 - 0.43 (m, 2H). ES-API: [M+H]⁺= 654.3.

### Example 136: Synthesis of (5S, 5aS, 6S, 9R)-2-(8-ethynyl-7-fluoronaphthalen-1-yl)-5-methyl-12-(((R)-1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z958)

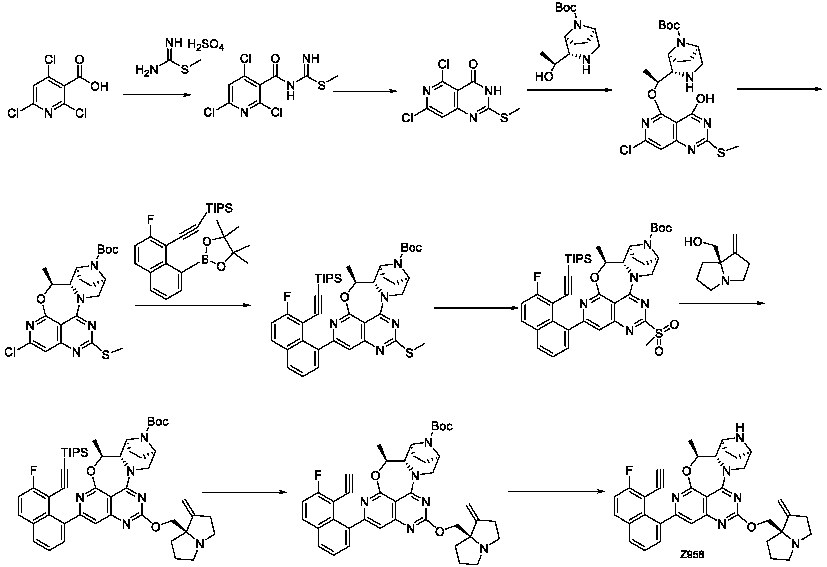

Step I: At 0°C, oxalyl chloride (18 mL, 212.72 mmol) was added dropwise to a solution of 2,4,6-trichloronicotinic acid (8 g, 35.33 mmol) in N,N-dimethylformamide (0.5 mL) and dichloromethane(100 mL). After that, the reaction solution was stirred at room temperature for 1 hr. The reaction solution was concentrated to dryness, to obtain an acyl chloride. Sodium bicarbonate (29.5 g, 351.18 mmol) and S-methylsothiourea sulfate (26.44g, 140.47 mmol) were sequentially added to a mixed solution of tetrahydrofuran (100 mL) and water (200 mL), and the system was stirred at room temperature for 10 min, and then cooled to 0°C. The acyl chloride as a crude product was dissolved in tetrahydrofuran (100 mL), and added dropwise to the above mixed solution. After that, the reaction was stirred 0°C for 30 min. The reaction solution was quenched with water (100 mL), extracted with ethyl acetate (200 mL), washed with saturated brine (200 mL), dried over anhydrous sodium sulfate, filtered, and concentrated, to obtain methyl (2,4,6-trichloronitrophenylformyl)aminoimidazolthiocarboxylate (9 g, yield: 85%). ES-API: [M+H]⁺= 298.0

Step II: Methyl (2,4,6-trichloronitrophenylformyl)aminoimidazolthiocarboxylate (9 g, crude product) and N,N-diisopropylethyl amine (11 mL, 66.38 mmol) were added to 1, 4-dioxane (120 mL). The reaction was stirred at 105°C for 16 hrs. The reaction solution was concentrated to about 30 mL. Water (80 mL) was added to the residue. Concentrated hydrochloric acid (3 mL) was slowly added dropwise. A large amount of solid was precipitated. The system was filtered, and the filter cake was washed with water. The filter cake was dried to obtain the crude product 5,7-dichloro-2-(methylthio)pyrimidine[4,3-d]pyrimidine-4(3H)-one (7.3 g). ES-API: [M+H]⁺= 262.0

Step III: T-butyl (1S, 2S, 5R)-2-((S)-1-hydroxyethyl)-3,8-diazadicyclo[3.2.1]octane-8-carboxylate (1.2 g, 4.68 mmol) was dissolved in anhydrous tetrahydrofuran (30 mL). In an iced water bath, 60%sodium hydride (460 mg, 11.44 mmol) was added, and reacted at room temperature for 15 min. After cooling to 0°C, a solution of 5,7-dichloro-2-(methylthio)pyrimidine[4,3-d]pyrimidine-4(3H)-one (1 g, 3.81 mmol) in tetrahydrofuran(2 mL) was added dropwise to the reaction solution, and reacted at 60°C in an oil bath for 1 hr. The reaction solution was cooled to 0°C, quenched with water (50 mL), extracted with ethyl acetate (30 mLX2), washed with saturated brine (60 mL), dried over anhydrous sodium sulfate, filtered, and concentrated. The crude product was purified by flash silica gel column chromatography (methanol/dichloromethane: 0-2%) to obtain t-butyl (1S, 2S, 5R)-2-((S)-1-((7-chloro-4-hydroxyl-2-(methylthio)pyrimidine[4,3-d]pyrimidine-5-yl)oxy)ethyl)-3,8-diazadicyclo[3.2.1]octane-8-carboxylate (900 mg, yield: 48%). ES-API: [M+H]⁺= 482.0.

Step IV: Propylphosphoric anhydride (3 mL, 50% solution in ethyl acetate) was added dropwise to a solution of t-butyl (1S, 2S, 5R)-2-((S)-1-((7-chloro-4-hydroxyl-2-(methylthio) pyrimidine[4,3-d]pyrimidine-5-yl)oxy)ethyl)-3,8-diazadicyclo[3.2.1]octane-8-carboxylate (460 mg, 1.86 mmol) and N,N-diisopropylethyl amine (3 mL, 17.18 mmol) in dichloromethane (8 mL). The reaction solution was stirred at room temperature for 16 hrs. The reaction solution was quenched with water (50 mL), extracted with ethyl acetate (30 mLX2), washed with saturated brine (60 mL), dried over anhydrous sodium sulfate, filtered, and concentrated. The crude product was purified by flash silica gel column chromatography (ethyl acetate /petroleum ether: 0-30%) to obtain t-butyl (5S, 5aS, 6S, 9R)-2-chloro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (400 mg, yield: 46%). ES-API: [M+H]⁺= 464.2.

Step V: T-butyl (5S, 5aS, 6S, 9R)-2-chloro-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (350 mg, 0.75 mmol), (2-fluoro-8-(4,4,5,5-tetramethyl-1,3,2-dioxaborolan-2-yl)naphthalen-1-yl)ethynyl)triisopropylsilane (439 mg, 0.97 mmol), [n-butyldi(1-adamantanyl) phosphine](2-amino-1,1'-biphenyl-2-yl)palladium (II) mesylate (38 mg, 0.052mmol), potassium phosphate (412 mg, 1.94 mmol), tetrahydrofuran (8 mL), and water (2 mL) were added to a round-bottom flask. Under a nitrogen atmosphere, the system was heated in a microwave reactor at 80°C for 60 min. The reaction solution was added with water (30 mL), and extracted with ethyl acetate (60 mLX3). The organic phase was dried over anhydrous sodium sulfate, filtered and concentrated. The crude product was purified by flash silica gel column chromatography (ethyl acetate /petroleum ether: 0-30%) to obtain t-butyl (5S, 5aS, 6S, 9R)-2-(7-fluoro-8-((triisopropylsilyl)ethynyl)naphthalen-1-yl)-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (450 mg, yield: 79%). ES-API: [M+H]⁺=754.3.

Step VI: T-butyl (5S, 5aS, 6S, 9R)-2-(7-fluoro-8-((triisopropylsilyl)ethynyl)naphthalen-1-yl)-5-methyl-12-(methylthio)-5a,6,7,8,9,10-hexahydro-5H-4-oxy-3,10a,11,13,14-pentaaza-6,9-methanonaphtho[1,8-ab]hepten-14-carboxylate (450 mg, 0.60 mmol) and dichloromethane (10 mL) were added to a round-bottom flask. The reaction solution was cooled to 0°C, and m-chloroperoxybenzoic acid (257 mg, 1.49 mmol) was added. The reaction was stirred at 0°C for 2 hrs. The reaction solution was added with a saturated sodium thiosulfate aqueous solution (30 mL) and a saturated sodium bicarbonate aqueous solution, and extracted with ethyl acetate (30 mLX3). The organic phase was dried over anhydrous sodium sulfate, filtered and concentrated to obtain t-butyl (5S, 5aS, 6S, 9R)-2-(7-fluoro-8-((triisopropylsilyl)ethynyl)naphthalen-1-yl)-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (450 mg, crude product). ES-API: [M+H]⁺=786.3.

Step VII: 60% sodium hydride (27 mg, 0.68 mmol) and tetrahydrofuran (10 mL) were added to a round-bottom flask. The reaction solution was cooled to 0°C, and ((R)-1-methylenetetrahydro-1H-pyrrolizin-7a(5H)-yl)methanol (40 mg, 0.26 mmol) dissolved in anhydrous tetrahydrofuran (1 mL) was added to the reaction solution, and reacted at room temperature for 10 min. The reaction solution was cooled to 0°C. T-butyl (5S, 5aS, 6S, 9R)-2-(7-fluoro-8-((triisopropylsilyl) ethynyl)naphthalen-1-yl)-5-methyl-12-(methylsulfonyl)-5a,6,7,8,9,10-hexahydro-5H-4-oxa-3,10a,11,13,14-pentaaza-6,9-methanonaphtho[1,8-ab]hepten-14-carboxylate (250 mg, crude product) was added to the reaction solution, and reacted at 0°C for 30 min. The reaction was shown to be completed by LC/MS. The reaction solution was added with a saturated ammonium chloride solution (30 mL), and extracted with ethyl acetate (20 mLX2). The organic phase was washed with saturated brine (30 mL), dried over anhydrous sodium sulfate, filtered, and concentrated, to obtain t-butyl (5S, 5aS, 6S, 9R)-2-(7-fluoro-8-((triisopropylsilyl)ethynyl)naphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (190 mg). ES-API: [M+H]⁺=859.3.

Step VIII: T-butyl (5S, 5aS, 6S, 9R)-2-(7-fluoro-8-((triisopropylsilyl)ethynyl)naphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (190 mg, crude product), cesium fluoride (300 mg, 1.98 mmol) and N, N-dimethylformamide (5 mL) were added to a round-bottom flask. The reaction was stirred at room temperature for 1 hrs. The reaction solution was added with water (30 mL), and extracted with ethyl acetate (30 mLX3). The organic phase was washed with saturated brine (30 mLX2), dried over anhydrous sodium sulfate, filtered, and concentrated, to obtain t-butyl (5S, 5aS, 6S, 9R)-2-(8-ethynyl-7-fluoronaphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (155 mg, crude product). ES-API: [M+H]⁺=703.3.

Step IX: T-butyl (5S, 5aS, 6S, 9R)-2-(8-ethynyl-7-fluoronaphthalen-1-yl)-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (155 mg, crude product) was dissolved in acetonitrile (3 mL). The solution was cooled to 0°C, and a 4M hydrogen chloride/dioxane solution (4 mL, 16 mmol) was added. The reaction was stirred at room temperature for 1 hrs. The reaction solution was concentrated to dryness at 40°C, and the crude product was purified by preparative HPLC (aqueous ammonia method 1) to obtain (5S, 5aS, 6S, 9R)-2-(8-ethynyl-7-fluoronaphthalen-1-yl)-5-methyl-12-(((r)-1-methylenetetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene (Z958, 30 mg, yield: 22%). ES-API: [M+H]⁺=603.3. ¹HNMR(400Hz, MeOD-d4)8.08-8.03(m,2H), 7.60-7.50(m,2H), 7.48-7.37(m,1H), 7.17(s,1H), 5.35-5.32(m,1H), 5.08-5.06(m,2H), 4.52-4.51(m,1H), 4.32-4.31(m,2H), 4.10-4.05 (m,1H), 3.75-3.49(m,3H), 3.29-3.10(m,3H), 2.83-2.66(m,4H), 2.21-1.78(m,8H), 1.59-1.55(m,3H).

### Example 137: Synthesis of 4-((5S,5aS,6S,9R)-12-(((R)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z959) and 4-((5S,5aS,6S,9R)-12-(((S)-1-(difluoromethylene)tetrahydro-1H-pyrrolin-7a(5H)-yl)methoxy)-1-fluoro-5-methyl-5a,6, 7,8,9,10-hexahydro-5H-4-oxa-3,10a,11,13,14-pentaaza-6,9-methanonaphtho[1,8-ab]hept-2-yl)-5-ethynylnaphthalen-2-amine (Z960)

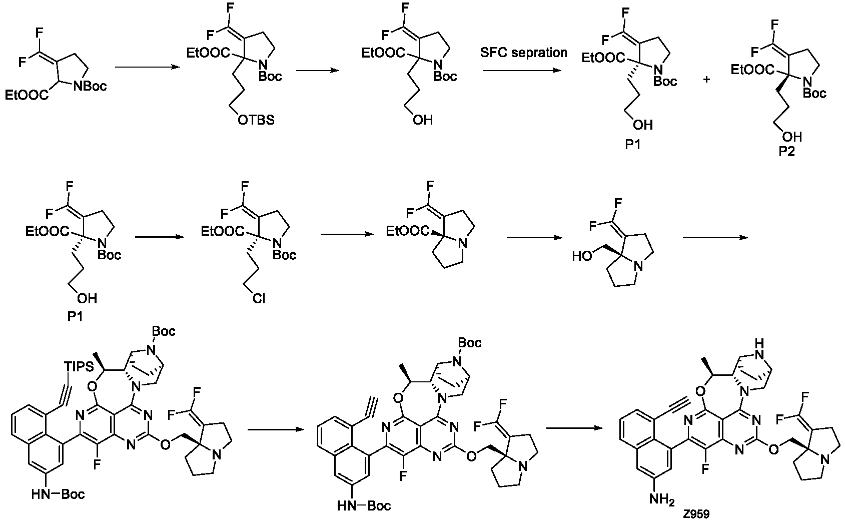

Step I: 1-(t-butyl) 2-ethyl 3-(difluoromethylene)pyrrolidin-1,2-dicarboxylate (6 g, 20.598 mmol) was dissolved in tetrahydrofuran (60 mL). At -78°C, a lithium bis(trimethylsilyl)amide solution in tetrahydrofuran (1 M, 41.196 mL, 41.196 mmol) under a nitrogen atmosphere. After that, the reaction was continuously stirred at - 78°C for 30 min. Then, t-butyl (3-iodopropoxy)dimethylsilane (11.13 g, 37.076 mmol) was dissolved in tetrahydrofuran (10 mL) and added slowly to the reaction solution dropwise at -78°C under a nitrogen atmosphere. After that, the reaction solution was heated to room temperature and stirred for 16 hrs. The reaction solution was added with saturated ammonium chloride (30 mL) to quench the reaction and extracted with ethyl acetate (100 mL X3). The organic phase was washed with saturated brine (200 mL), dried over anhydrous sodium sulfate, filtered and concentrated to obtain a crude product. The crude product was purified by flash silica gel column chromatography (ethyl acetate /petroleum ether: 0%-6%) to obtain 1-(t-butyl) 2-ethyl 2-(3-((t-butyldimethylsilyl)oxy)propyl)-3-(difluoromethylene)pyrrolidin-1,2-dicarboxylate (5 g, 10.784 mmol, yield: 52.36%) as a colorless transparent liquid. ES-API: [M-Boc+H]⁺=364.3.

Step II: 1-(t-butyl) 2-ethyl2-(3-((t-butyldimethylsilyl)oxy)propyl)-3-(difluoromethylene) pyrrolidin-1,2-dicarboxylate (10 g, 21.568 mmol) was dissolved in a tetrabutylammonium fluoride solution in tetrahydrofuran (1 M, 26 mmol, 26 mL) and stirred for 2 hrs. The reaction solution was added with water (15 mL) and extracted with ethyl acetate (30 X2). The organic phase was washed with saturated brine (100 mL), dried over anhydrous sodium sulfate, filtered and concentrated to obtain a crude product. The crude product was purified by flash silica gel column chromatography (ethyl acetate /petroleum ether: 0%-40%) to obtain a racemate that was 1-(t-butyl) 2-ethyl 3-(difluoromethylene)-2-(3-hydroxypropyl)pyrrolidin-1,2-dicarboxylate (5 g, 14.31 mmol, yield: 66.35%) as a colorless transparent liquid. ES-API: [M-Boc+H]⁺=250.1.

Step III: The racemate 1-(t-butyl) 2-ethyl 3-(difluoromethylene)-2-(3-hydroxypropyl) pyrrolidin-1,2-dicarboxylate (5 g, 14.31 mmol) was chirally resolved (column: Daicel CHIRALPAK^{®} IC 250*4.6 mm, 5µm; mobile phase system: A: n-hexane; B: isopropanol; flow rate: 1 mL/min; A:B =90:10(V/V); column temperature: 30 °C) to obtain two isomers. One isomer structure was arbitrarily designated as 1-(t-butyl) 2-ethyl (R)-3-(difluoromethylene)-2-(3-hydroxypropyl)pyrrolidin-1,2-dicarboxylate (P1, 2.4g, 6.870 mmol, yield: 48%, retention time: 12.876 min); and the other isomer structure was arbitrarily designated as 1-(t-butyl) 2-ethyl (S)-3-(difluoromethylene)-2-(3-hydroxypropyl)pyrrolidin-1,2-dicarboxylate (P2, 2.3g, 6.583 mmol, yield: 46%, retention time: 19.228 min). They were both a white clear liquid. ES-API: [M-Boc+H]⁺=250.1.

Step IV: At room temperature, 1-(t-butyl) 2-ethyl (R)-3-(difluoromethylene)-2-(3-hydroxypropyl)pyrrolidin-1,2-dicarboxylate (P1, 2.4, 6.870 mmol) was dissolved in thionyl chloride (10 mL). The reaction was heated to 35°C and stirred for 17 hrs. The reaction was concentrated to obtain ethyl (R)-2-(3-chloropropyl)-3-(difluoromethylene)pyrrolidin-2-carboxylate (2076 mg, hydrochloride, crude product) as a colorless clear liquid. ES-API: [M +H]⁺= [M-Boc+H]⁺=268.1

Step V: Ethyl (R)-2-(3-chloropropyl)-3-(difluoromethylene)pyrrolidin-2-carboxylate (2076 mg, hydrochloride, crude product) was dissolved in acetonitrile (20 mL) and water (20 mL). Sodium bicarbonate (2667.48 mg, 31.752 mmol) was added to the solution. After that, the reaction was stirred at room temperature for 0.5 hr. The reaction solution was added with water (50 mL) and extracted with ethyl acetate (50 X3). The organic phase was washed with saturated brine (100 mL), dried over anhydrous sodium sulfate, filtered and concentrated to obtain ethyl (R)-1-(difluoromethylene)tetrahydro-1H-pyrrolizin-7a(5H)-carboxylate (1300 mg, 5.62 mmol, yield of two steps: 81.8%) as a colorless transparent liquid.

Step VI: Ethyl (R)-1-(difluoromethylene)tetrahydro-1H-pyrrolizin-7a(5H)-carboxylate (1300 mg, 5.62 mmol) was dissolved in tetrahydrofuran(20 mL), and cooled to 0 °C. Under a nitrogen atmosphere, a lithium aluminum hydride solution in tetrahydrofuran (1M, 12 mL, 12 mmol) was added to the solution. The reaction was stirred at 0°C for 1 hr. The reaction solution was added with water (0.45 mL) to quench to the reaction, then added with a 15% sodium hydroxide aqueous solution (0.45 mL) and water (1.35 mL), and dried over anhydrous sodium sulfate. The obtained mixture was diluted with tetrahydrofuran (30 mL) and filtered through a glass sand funnel. The filtrate was concentrated to obtain (R)-(1-(difluoromethylene)tetrahydro-1H-pyrrolin-7a(5H)-yl)methanol (800 mg, 4.228 mmol, yield: 75.21 %) as a colorless transparent liquid. ES-API: [M +H]⁺=190.1.

Step VII: (R)-(1-(difluoromethylene)tetrahydro-1H-pyrrolizin-7a(5H)-yl)methanol (62.99 mg, 0.333 mmol) was dissolved in tetrahydrofuran (4 mL) and cooled to 0°C. Under a nitrogen atmosphere, sodium hydride (60%, 33.29 mg, 0.832 mmol) was added to the solution and stirred for 30 min. T-butyl (5S,5aS,6S,9R)-2-(3-((t-butoxycarbonyl)amino)-8-((triisopropylsilyl)ethynyl) naphthalen-1-yl)-1-fluoro-5-methyl-12-(methylsulfonyl)-hexahydro-5H-4-oxa-3,10a,11,13,14-pentaaza-6,9-methanonaphtho[1,8-ab]hepten-14-carboxylate (150 mg, 0.166 mmol) was slowly added to the solution and stirred at 0 °C for 0.5 hr. The reaction solution was added with water (20 mL) to quench the reaction, and then extracted with ethyl acetate (20 mL X3). The organic phase was washed with saturated brine (10 mL*3), dried over anhydrous sodium sulfate, filtered and concentrated to obtain a crude product. The crude product was purified by flash silica gel column chromatography (methanol/dichloromethane: 0-3%) to obtain t-butyl (5S,5aS,6S,9R)-2-(3-((t-butoxycarbonyl)amino)-8-((triisopropylsilyl)ethynyl)naphthalen-1-yl)-12-(((R)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol, yield: 60%) as a light yellow solid. ES-API: [M+H]⁺=1010.3.

Step VIII: A mixed solution of t-butyl (5S,5aS,6S,9R)-2-(3-((t-butoxycarbonyl)amino)-8-((triisopropylsilyl)ethynyl)naphthalen-1-yl)-12-(((R)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol), cesium fluoride (150.35 mg, 0.990 mmol), and N, N-dimethylformamide (5 mL) was stirred at room temperature for 2 hrs. The reaction mixture was added with water (30 mL) and extracted with ethyl acetate (20 mL X2). The organic phase was washed with saturated brine (30 mLX3), dried over anhydrous sodium sulfate, filtered and concentrated to obtain t-butyl (5S,5aS,6S,9R)-2-(3-((t-butoxycarbonyl)amino)-8-ethynylnaphthalen-1-yl)-12-(((R)-1-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol, yield: 89%) as a light yellow solid. ES-API: [M+H]⁺=854.2.

Step IX: T-butyl (5S,5aS,6S,9R)-2-(3-((t-butoxycarbonyl)amino)-8-ethynylnaphthalen-1-yl)-12-(((R)-1-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol) was dissolved in ethyl acetate (2 mL), and cooled to 0°C. A hydrogen chloride-dioxane solution (4 M, 4 mL) was added to the solution, and reacted with stirring at 0°C for 2 hrs. The reaction solution was concentrated, and the residue was taken up in dichloromethane (2 mL) again and alkalized with ammonia in methanol (7 M) to an alkaline pH (pH 8). After the obtained solution was concentrated, the crude product was purified by preparative HPLC(ammonium bicarbonate method 1) to obtain 4-((5S,5aS,6S,9R)-12-(((R)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z959, 20 mg, 0.031 mmol, yield: 29.5%) as a white solid. ES-API: [M+H]⁺=654.2. ¹H NMR (400 MHz, CD₃OD) δ 7.68 (t, *J =* 6.8 Hz, 1H), 7.41 -7.23 (m, 2H), 7.18 - 7.00 (m, 2H), 5.43 (d, *J* = 13.2 Hz, 1H), 4.65 - 4.47 (m, 3H), 4.24 - 4.15 (m, 1H), 3.98 - 3.90 (m, 1H), 3.87 - 3.79 (m, 1H), 3.52 - 3.42 (m, 1H), 3.30 -3.23 (m, 2H), 3.13 - 3.02 (m, 1H), 3.01 - 2.90 (m, 2H), 2.87 - 2.77 (m, 1H), 2.76 - 2.65 (m, 1H), 2.29 - 2.10 (m, 3H), 2.08 - 1.82 (m, 5H), 1.57 (t, *J* = 6.4 Hz, 3H).

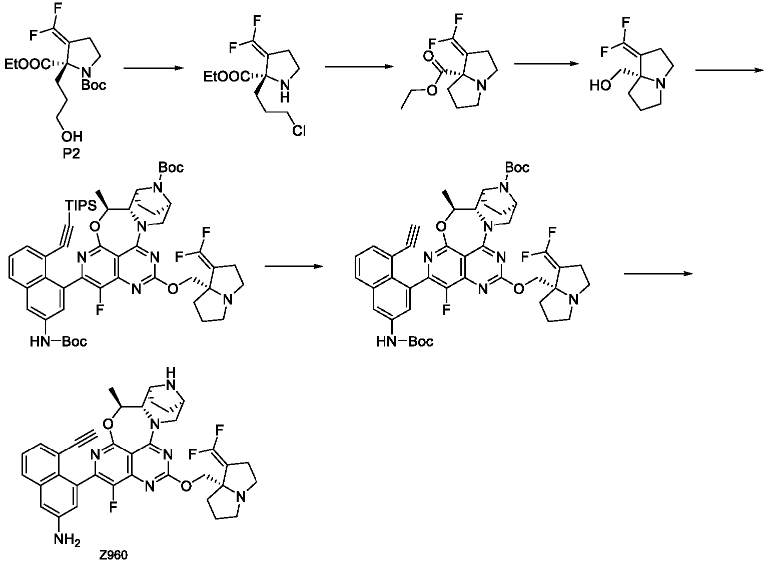

Step I: At room temperature, 1-(t-butyl) 2-ethyl (S)-3-(difluoromethylene)-2-(3-hydroxypropyl) pyrrolidin-1,2-dicarboxylate (P2,2.3 g, 6.583 mmol) was dissolved in thionyl chloride (10 mL). The reaction was heated to 35°C and stirred for 17 hrs. The reaction was concentrated to obtain ethyl (S)-2-(3-chloropropyl)-3-(difluoromethylene)pyrrolidin-2-carboxylate (2000 mg, hydrochloride, crude product) as a colorless clear liquid. ES-API: [M +H]⁺= [M-Boc+H]⁺=268.1

Step II: Ethyl (S)-2-(3-chloropropyl)-3-(difluoromethylene)pyrrolidin-2-carboxylate hydrochloride as a crude product (2000 mg) was dissolved in acetonitrile (20 mL) and water (20 mL). Sodium bicarbonate (2667.48 mg, 31.752 mmol) was added to the solution. After that, the reaction was stirred at room temperature for 0.5 hr. The reaction solution was added with water (50 mL) and extracted with ethyl acetate (50 X3). The organic phase was washed with saturated brine (100 mL), dried over anhydrous sodium sulfate, filtered and concentrated to obtain Ethyl (S)-1-(difluoromethylene)tetrahydro-1H-pyrrolizin-7a(5H)-carboxylate (1300 mg, 5.62 mmol, yield of two steps: 85.3%) as a colorless transparent liquid.

Step III: Ethyl (S)-1-(difluoromethylene)tetrahydro-1H-pyrrolizin-7a(5H)-carboxylate (1300 mg, 5.62 mmol) was dissolved in tetrahydrofuran(20 mL), and cooled to 0 °C. Under a nitrogen atmosphere, a lithium aluminum hydride solution in tetrahydrofuran (1M, 12 mL, 12 mmol) was added to the solution. The reaction was stirred at 0°C for 1 hr. The reaction solution was added with water (0.45 mL) to quench to the reaction, then added with a 15% sodium hydroxide aqueous solution (0.45 mL) and water (1.35 mL), and dried over anhydrous sodium sulfate. The obtained mixture was diluted with tetrahydrofuran (30 mL) and filtered through a glass sand funnel. The filtrate was concentrated to obtain (S)-(1-(difluoromethylene)tetrahydro-1H-pyrrolin-7a(5H)-yl)methanol (800 mg, 4.228 mmol, yield: 75.21%) as a colorless transparent liquid. ES-API: [M +H]⁺=190.1.

Step IV: (S)-(1-(difluoromethylene)tetrahydro-1H-pyrrolizin-7a(5H)-yl)methanol (62.99 mg, 0.333 mmol) was dissolved in tetrahydrofuran (4 mL) and cooled to 0°C. Under a nitrogen atmosphere, sodium hydride (60%, 33.29 mg, 0.832 mmol) was added to the solution and stirred for 30 min. Then, t-butyl (5S,5aS,6S,9R)-2-(3-((t-butoxycarbonyl)amino)-8-((triisopropylsilyl) ethynyl)naphthalen-1-yl)-1-fluoro-5-methyl-12-(methylsulfonyl)-hexahydro-5H-4-oxa-3,10a,11,13,14-pentaaza-6,9-methanonaphtho[1,8-ab]hepten-14-carboxylate (150 mg, 0.166 mmol) was slowly added to the solution, and stirred at 0°C for 0.5 hrs. The reaction solution was added with water (20 mL) to quench the reaction, and then extracted with ethyl acetate (20 mL X3). The organic phase was washed with saturated brine (10 mLX3), dried over anhydrous sodium sulfate, filtered and concentrated to obtain a crude product. The crude product was purified by flash silica gel column chromatography (methanol /dichloromethane: 0-3%) to obtain t-butyl (5S,5aS,6S,9R)-2-(3-((t-butoxycarbonyl)amino)-8-((triisopropylsilyl)ethynyl)naphthalen-1-yl)-12-(((S)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol, yield: 60%) as a light yellow solid. ES-API: [M+H]⁺=1010.3.

Step V: A mixed solution of t-butyl (5S,5aS,6S,9R)-2-(3-((t-butoxycarbonyl)amino)-8-((triisopropylsilyl)ethynyl)naphthalen-1-yl)-12-(((S)-1-(difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (100 mg, 0.099 mmol), cesium fluoride (150.35 mg, 0.990 mmol), and N, N-dimethylformamide (5 mL) was stirred at room temperature for 2 hrs. The reaction mixture was diluted with water (30 mL) and extracted with ethyl acetate (20 mL X2). The organic phase was washed with saturated brine (30 mLX3), dried over anhydrous sodium sulfate, filtered and concentrated to obtain t-butyl (5S,5aS,6S,9R)-2-(3-((t-butoxycarbonyl)amino)-8-ethynylnaphthalen-1-yl)-12-(((S)-1-(difluoromethylene) tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol, yield: 89%) as a light yellow solid. ES-API: [M+H]⁺=854.2.

Step VI: T-butyl (5S,5aS,6S,9R)-2-(3-((t-butoxycarbonyl)amino)-8-ethynylnaphthalen-1-yl)-12-(((S)-1-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (90 mg, 0.105 mmol) was dissolved in ethyl acetate (2 mL), and cooled to 0 °C. A hydrogen chloride-dioxane solution (4 M, 4 mL) was added to the solution, and reacted with stirring at 0°C for 2 hrs. The reaction solution was concentrated, and the residue was taken up in dichloromethane (2 mL) again and alkalized with ammonia in methanol (7 M) to an alkaline pH (pH 8). After the obtained solution was concentrated, the crude product was purified by preparative HPLC (ammonium bicarbonate method 1) to obtain 4-((5S,5aS,6S,9R)-12-(((S)-1-(difluoromethylene)tetrahydro-1H-pyrrolin-7a(5H)-yl)methoxy)-1-fluoro-5-methyl-5a,6, 7,8,9,10-hexahydro-5H-4-oxa-3,10a,11,13,14-pentaaza-6,9-methanonaphtho[1,8-ab]hept-2-yl)-5-ethynylnaphthalen-2-amine(Z960, 15 mg, 0.023 mmol, yield: 21.9%) as a white solid. ES-API: [M+H]⁺=654.2. ¹H NMR (400 MHz, CD₃OD) δ 7.67 (t, *J* = 7.2 Hz, 1H), 7.43 - 7.25 (m, 2H), 7.16 - 6.99 (m, 2H), 5.36 (d, *J* = 13.2 Hz, 1H), 4.55 - 4.36 (m, 3H), 4.07 (d, *J =* 8.6 Hz, 1H), 3.75 - 3.67 (m, 1H), 3.57 - 3.63 (m, 1H), 3.38 - 3.35 (m, 1H), 3.22 - 3.05 (m, 2H), 3.01 - 2.86 (m, 2H), 2.85 - 2.70 (m, 2H), 2.69 - 2.63 (m, 1H), 2.23 - 1.99 (m, 3H), 1.99 - 1.71 (m, 5H), 1.55 (t, *J =* 7.2 Hz, 3H).

### Example 138: Synthesis of 4-((5S, 5aS, 6S, 9R)-12-(((S)-2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z961) and 4-((5S, 5aS, 6S, 9R)-12-(((R)-2-(difluoromethyl)tetrahydro-1H-pyrrolizin-7a(5H)-yl)methoxy)-1-fluoro-5-methyl-5a,6,7,8,9,10-hexahydro-5H-4-oxa -3,10a,11,13,14-pentaaza-6,9-methanonaphtho[1,8-ab]hepten-2-yl)-5-ethynylnaphthalen-2-amine (Z962)

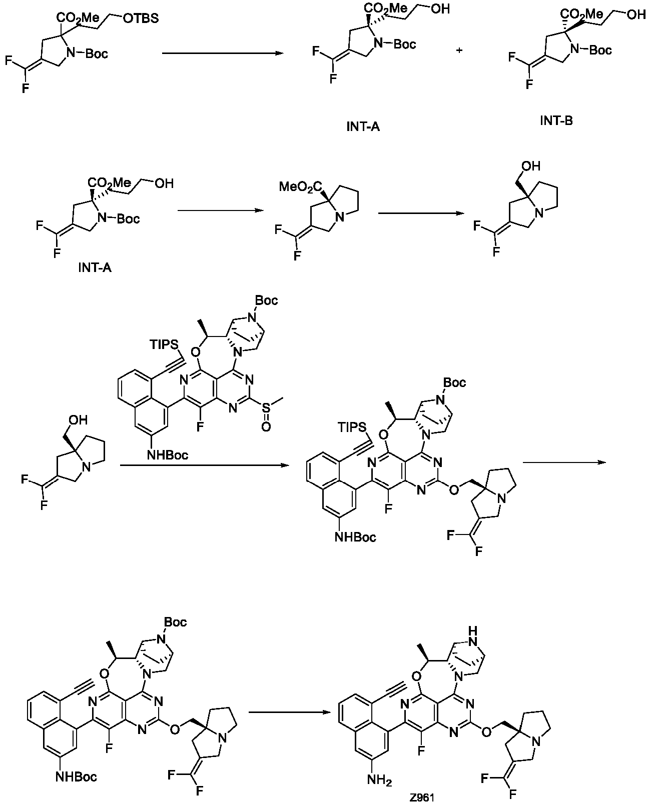

Step I: A 1 M tetrabutylammonium fluoride solution in tetrahydrofuran (5.77 mmol, 5.77 mL) was added to a solution of 1-(t-butyl)2-methyl-2-(3-((t-butyldimethylsilyl)oxy)propyl)-4-(difluoromethyl)pyrrolidin-1,2-dicarboxylate (2g, 4.44mmol) in tetrahydrofuran (3mL). The reaction was stirred at room temperature for 2 hrs. The reaction solution was quenched with water (50 mL), extracted with ethyl acetate (30 mLX2), washed with saturated brine (60 mLX2), dried over anhydrous sodium sulfate, filtered, and concentrated. The crude product was purified by flash silica gel column chromatography (ethyl acetate /petroleum ether: 0-30%) to obtain a racemate (1g). The racemate was chirally resolved to obtain two enantiomers (separation column: CHIRALPAK IG 250mm*4.6mm*5um; mobile phase: methanol /ethanol/n-hexane=2:3:95; flow rate:1 mL/min; column temperature 30°C). One isomer structure was arbitrarily designated as 1-(t-butyl) 2-methyl (S)-4-(difluoromethyl)-2-(3-hydroxypropyl)pyrrolidin-1,2-dicarboxylate (INT-A, 290mg, peak 1, retention time=9.893 min); ES-API: [M+Na]⁺=358.1; and the other isomer structure was arbitrarily designated as 1-(t-butyl) 2-methyl (R)-4-(difluoromethyl)-2-(3-hydroxypropyl)pyrrolidin-1,2-dicarboxylate (INT-B, 300mg, peak 1, retention time=11.278 min); ES-API: [M+Na]⁺=358.1.

Step II: Thionyl chloride(6 mL, 82.72 mmol) was added to 1-(t-butyl) 2-methyl (S)-4-(difluoromethyl)-2-(3-hydroxypropyl)pyrrolidin-1,2-dicarboxylate (INT-A, 280 mg, 0.84 mmol). The reaction was stirred at 35°C for 16 hrs. The reaction solution was concentrated. The residue was added with acetonitrile (5 mL), and then saturated sodium bicarbonate (5 mL). The reaction was stirred at room temperature for 60 min. The reaction was quenched with water (50 mL), and extracted with ethyl acetate (30 mLX2). The organic phase was washed with saturated brine (30 mL), dried over anhydrous sodium sulfate, filtered and concentrated, to obtain methyl (S)-2-(difluoromethyl)tetrahydro-1H-pyrrolizin-7a(SH)-carboxylate (170 mg, yield: 93%) as a yellow oil. ES-API: [M+Na]⁺=218.2.

Step III: A lithium aluminium hydride /tetrahydrofuran solution (1.4 mL, 1.4 mmol) was added dropwise to a solution of methyl (S)-2-(difluoromethyl)tetrahydro-1H-pyrrolizin-7a(SH)-carboxylate (170 mg, 0.78 mmol) in tetrahydrofuran (4 mL), and stirred at 0°C for 30 min. The reaction was quenched by sequentially adding 3 drops of water and a 15% sodium hydroxide aqueous solution (0.1 mL). The mixture was stirred at room temperature for 30 min, and filtered through diatomite. The filtrate was concentrated, to obtain (S)-(2-(difluoromethyl)tetrahydro-1H-pyrrolizin-7a(5H)-yl)methanol (140 mg, yield: 94%) as a yellow oil. ES-aPI: [M+H]⁺=190.1.

Step IV: (S)-(2-(difluoromethyl)tetrahydro-1H-pyrrolizin-7a(5H)-yl)methanol (28mg, 0.15 mmol) was added to a 60% suspension of sodium hydride (14 mg, 0.35 mmol) in anhydrous tetrahydrofuran (6 mL) in an iced water bath, and reacted at room temperature for 5 min. The reaction solution was cooled to 0°C. A solution of t-butyl (5S, 5aS, 6S, 9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]hepten-14-carboxylate (100 mg, 0.11 mmol) in tetrahydrofuran (2 mL) was added dropwise to the reaction solution, and reacted at room temperature for 30 min. The reaction solution was quenched with water (50 mL), extracted with ethyl acetate (30 mLX2), washed with saturated brine (60 mL), dried over anhydrous sodium sulfate, filtered, and concentrated to obtain t-butyl (5S, 5aS, 6S, 9R)-2-(3-((t-butoxycarbonyl)amino)-8-((triisopropylsilyl)ethynyl)naphthalen-1-yl)-12-((((S)-2-difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (114 mg, crude product). ES-API: [M+H]⁺=1010.3.

Step V: Cesium fluoride (171 mg, 1.12 mmol) was added to t-butyl (5S, 5aS, 6S, 9R)-2-(3-((t-butoxycarbonyl)amino)-8-((triisopropylsilyl)ethynyl)naphthalen-1-yl)-12-((((S)-2-difluoromethyl)tetrahydro-1H-pyrrolizin-7A(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (114 mg, crude product) in N,N-dimethylformamide (3 mL), and the reaction mixture was stirred at room temperature for 2 hrs. The reaction was shown to be completed by LC/MS. The reaction solution was diluted with ethyl acetate (30 mL), washed with saturated brine (30mLX3), dried over anhydrous sodium sulfate, filtered, and concentrated to obtain t-butyl (5S, 5aS,6S,9R)-2-(3-((t-butoxycarbonyl)amino)-8-ethynylnaphthalen-1-yl)-12-(((S)-2-(difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (96 mg, crude product). ES-API: [M+H]⁺= 854.3.

Step VI: In an iced water bath, a 4 M hydrogen chloride-dioxane solution (2 mL) was slowly added to a solution of t-butyl (5S, 5aS, 6S, 9R)-2-(3-((t-butoxycarbonyl)amino)-8-ethynylnaphthalen-1-yl)-12-(((S)-2-(difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (96 mg, crude product) in ethyl acetate (2 mL). The reaction mixture was stirred at room temperature for 1 hr. The reaction was shown to be completed by LC/MS. The reaction solution was concentrated to dryness under reduced pressure, and the residue was purified by preparative HPLC (aqueous ammonia method 1) to obtain 4-((5S, 5aS, 6S, 9R)-12-(((S)-2-(difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-2-yl)-5-ethynylnaphthalen-2-amine (Z961, 5 mg, yield: 6.8%). ES-API: [M+H]⁺= 654.2. ¹HNMR(400MHz,CD₃OD): 7.69-7.66 (m,1H), 7.41-7.25 (m,2H), 7.13-6.99 (m,2H), 5.39-5.34 (m,1H), 4.53-4.48 (m,1H), 4.34-4.22 (m,2H), 4.07-4.05 (m,1H), 3.81-3.77 (m,1H), 3.69-3.59 (m,2H), 3.44-3.41 (m,1H), 3.30-2.99 (m,3H), 2.82-2.67 (m,2H), 2.52-2.48 (m,1H), 2.12-1.77 (m,8H), 1.58-1.55 (m,3H).

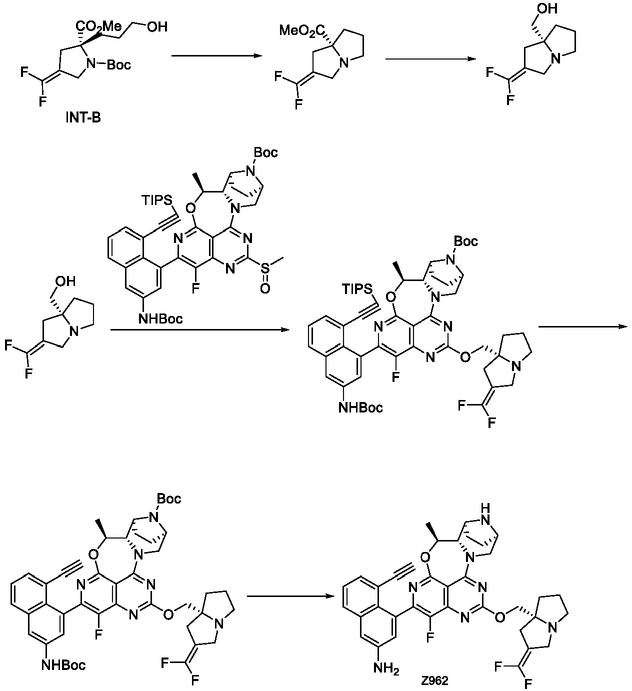

Step I: Thionyl chloride (6 mL, 82.72 mmol) was added to 1-(t-butyl) 2-methyl (R)-4-(difluoromethyl)-2-(3-hydroxypropyl)pyrrolidin-1,2-dicarboxylate (INT-B, 300mg, 0.89 mmol). The reaction was stirred at 35°C for 16 hrs. The reaction solution was concentrated. The residue was added with acetonitrile (5 mL), and then saturated sodium bicarbonate (5 mL). The reaction was stirred at room temperature for 60 min. The reaction was quenched with water (50 mL), and extracted with ethyl acetate (30 mLX2). The organic phase was washed with saturated brine (30 mL), dried over anhydrous sodium sulfate, filtered and concentrated, to obtain methyl (R)-2-(difluoromethyl)tetrahydro-1H-pyrrolizin-7a(5H)-carboxylate (170 mg, yield: 93%) as a yellow oil. ES-API: [M+Na]⁺=218.2.

Step II: A lithium aluminium hydride /tetrahydrofuran solution (1.4 mL, 1.4 mmol) was added dropwise to a solution of methyl (R)-2-(difluoromethyl)tetrahydro-1H-pyrrolizin-7a(5H)-carboxylate (170 mg, 0.78 mmol) in tetrahydrofuran (4 mL), and stirred at 0°C for 30 min. The reaction was quenched by sequentially adding 3 drops of water and a 15% sodium hydroxide aqueous solution (0.2 mL). The mixture was stirred at room temperature for 30 min, and filtered through diatomite. The filtrate was concentrated, to obtain (R)-(2-(difluoromethyl)tetrahydro-1H-pyrrolizin-7a(5H)-yl)methanol (140 mg, yield: 94%) as a yellow oil. ES-API: [M+H]⁺=190.1.

Step III: (R)-(2-(difluoromethyl)tetrahydro-1H-pyrrolizin-7a(5H)-yl)methanol (28mg, 0.15 mmol) was added to a 60% suspension of sodium hydride (14 mg, 0.35 mmol) in anhydrous tetrahydrofuran(6 mL) in an iced water bath, and reacted at room temperature for 5 min. The reaction solution was cooled to 0°C. A solution of t-butyl (5S, 5aS, 6S, 9R)-2-(3-((t-butoxycarbonyl)amino)-8-((triisopropylsilyl)ethynyl)naphthalen-1-yl)-1-fluoro-5-methyl-12-(methylthionyl)-5a,6,7,8,9,10-hexahydro-5H-4-oxa-3,10a,11,13,14-pentaaza-6,9-methanonaphtho[1,8-ab]heptan-14-carboxylate (100 mg, 0.11 mmol) in tetrahydrofuran (2 mL) was added dropwise to the reaction solution, and reacted at room temperature for 30 min. The reaction solution was quenched with water (50 mL), extracted with ethyl acetate (30 mLX2), washed with saturated brine (60 mL), dried over anhydrous sodium sulfate, filtered, and concentrated to obtain t-butyl (5S, 5aS, 6S, 9R)-2-(3-((t-butoxycarbonyl)amino)-8-((triisopropylsilyl)ethynyl)naphthalen-1-yl)-12-((((R)-2-difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaz-6,9-methanonaphtho[1,8-ab]hepten-14-carboxylate (114 mg, crude product). ES-API: [M+H]⁺=1010.3.

Step IV: Cesium fluoride (171 mg, 1.12 mmol) was added to t-butyl (5S, 5aS, 6S, 9R)-2-(3-((t-butoxycarbonyl)amino)-8-((triisopropylsilyl)ethynyl)naphthalen-1-yl)-12-((((R)-2-difluoromethyl)tetrahydro-1H-pyrrolizin-7a(5H)-yl)methoxy)-1-fluoro-5-methyl-5a,6,7,8,9,10-hexahydro-5H-4-oxy-3,10a,11,13,14-pentaaza-6,9-methanonaphtho[1,8-ab]hepten-14-carboxylate (114 mg, crude product) in N,N-dimethylformamide (3 mL), and the reaction mixture was stirred at room temperature for 2 hrs. The reaction was shown to be completed by LC/MS. The reaction solution was diluted with ethyl acetate (30 mL), wahsed with saturated brine (30mLX3), dried over anhydrous sodium sulfate, filtered, and concentrated to obtain t-butyl (5S, 5aS,6S,9R)-2-(3-((t-butoxycarbonyl)amino)-8-ethynylnaphthalen-1-yl)-12-(((R)-2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (96 mg, crude product). ES-API: [M+H]⁺= 854.3.

Step V: In an iced water bath, a 4 M hydrogen chloride-dioxane solution (2 mL) was slowly added to a solution of t-butyl (5S, 5aS, 6S, 9R)-2-(3-((t-butoxycarbonyl)amino)-8-ethynylnaphthalen-1-yl)-12-(((R)-2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (96 mg, crude product) in ethyl acetate (2 mL). The reaction mixture was stirred at room temperature for 1 hr. The reaction was shown to be completed by LC/MS. The reaction solution was concentrated to dryness under reduced pressure, and the residue was purified by preparative HPLC (aqueous ammonia method 1) to obtain 4-((5S, 5aS, 6S, 9R)-12-(((R)-2-(difluoromethyl)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z962, 10 mg, yield: 13.6%). ES-API: [M+H]⁺= 654.2. ¹HNMR (400MHz, CD₃OD):7.69-7.65 (m,1H), 7.41-7.25 (m,2H), 7.13-7.00 (m,2H), 5.39-5.34 (m,1H), 4.53-4.47 (m,1H), 4.34-4.22 (m,2H), 4.07-4.05 (m,1H), 3.81-3.77 (m,1H), 3.68-3.58 (m,2h), 3.44-3.41 (m,1H), 3.30-2.98 (m,3H), 2.82-2.67 (m,2H), 2.52-2.48 (m,1H), 2.14-1.77 (m,8H), 1.58-1.54 (m,3H).

### Example 139: Synthesis of 3-chloro-5-((5S,5aS,6S,9R)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptadien-2-yl)-4-(trifluoromethyl)aniline (Z963)

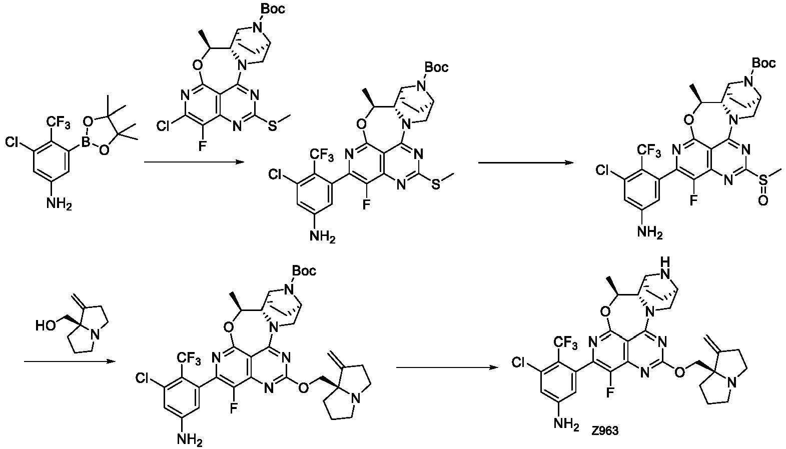

Step I: Potassium phosphate (2.64 g, 12.468 mmol), [n-butyldi(1-adamantanyl)phosphine](2-amino-1,1'-biphenyl-2-yl)palladium (II) mesylate (340.0 mg, 0.465 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab] hepten-14-carboxylate (1.50 g, 3.117 mmol) and 3-chloro-5-(4,4,5,5-tetramethyl-1,3,2-dioxaborolan-2-yl)-4-(trifluoromethyl)aniline (2.10 g, 6.540 mmol) in tetrahydrofuran (30 mL) and water (3 mL). Nitrogen was bubbled therethrough and reacted at 80°C under microwave for 45 min. After the reaction, the reaction solution was extracted with ethyl acetate (80 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(5-amino-3-chloro-2-(trifluoromethyl)phenyl)-1-fluoro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (2.04 mg, crude product) as a yellow solid. ES-API: [M+H]⁺=641.2.

Step II: At 0°C, m-chloroperoxybenzoic acid (810.0 mg, 4.68 mmol) was added to a solution of t-butyl (5S,5aS,6S,9R)-2-(5-amino-3-chloro-2-(trifluoromethyl)phenyl)-1-fluoro-5-methyl-12-(methylthio)-5a,6,7,8,9,10-hexahydro-5H-4-oxo-3,10a,11,13,14-pentaaza-6,9-methanonaphtho[1,8-ab]hepten-14-carboxylate (2.0 g, 3.12 mmol) in dichloromethane (30.0 mL). The solution was stirred for 1 hour in an ice bath. The reaction was quenched with saturated sodium sulfite and extracted with dichloromethane(100 mLX2). The organic phase was dried over anhydrous sodium sulfate, filtered and concentrated to obtain a crude product. The crude product was purified by by silica gel column chromatography (0-10% MeOH/DCM) to obtain t-butyl (5S,5aS,6S,9R)-2-(5-amino-3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (2.20 g, crude product) as a white solid. ES-API:[M+H]⁺=657.1.

Step III: At 0°C, sodium hydride (230.0 mg, 5.750 mmol) was added to a solution of (R)-(1-methylenetetrahydro-1H-pyrrolizin-7a(5H)-yl)methanol (440 mg, 2.875 mmol) in tetrahydrofuran (10.0 mL), and stirred at this temperature for 10 min. Then, a solution of t-butyl (5S,5aS,6S,9R)-2-(5-amino-3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (1.70 g, crude product) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(5-amino-3-chloro-2-(trifluoromethyl)phenyl)-1-fluoro-5-methyl-12-(((R)-1-methylenetetrahydro-1H--pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (650 mg, yield: 33.58) as a yellow solid. ES-API: [M+H]⁺=746.2.

Step IV: In an iced water bath, t-butyl (5S,5aS,6S,9R)-2-(5-amino-3-chloro-2-(trifluoromethyl)phenyl)-1-fluoro-5-methyl-12-(((R)-1-methylenetetrahydro-1H--pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (650.0 mg, 0.870 mmol) was slowly added to dichloromethane (30.0 mL), and finally trifluoroacetic acid (855.0 mg, 8.70 mmol was added. The reaction mixture was stirred at room temperature for 3.0 hrs. The reaction was shown to be completed by LC/MS. The reaction solution was concentrated to dryness under reduced pressure, and the residue was purified by preparative HPLC (ammonium bicarbonate method 2) to obtain 3-chloro-5-((5S,5aS,6S,9R)-1-fluoro-5-methyl-12-(((R)-1-methylenetetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptadiene-2-yl)-4-(trifluoromethyl)aniline (Z963, 300.0mg, yield: 53%) as a white solid. ES-API: [M+H]⁺= 646.2. ¹H NMR (400 MHz, CD₃OD) δ 6.89-6.87 (m, 1H), 6.50-6.40 (m, 1H), 5.34-5.30 (m, 1H), 5.10-5.08 (m, 2H), 4.514.49 (m, 1H), 4.34 (s, 2H), 4.06-4.02 (m, 1H), 3.66-3.64 (m, 1H), 3.57 (s, 1H), 3.26-3.08 (m, 3H), 2.852.82 (m, 1H), 2.75-2.61 (m, 3H), 2.25-2.14 (m, 1H), 1.99-1.71 (m, 7H), 1.56 (d, J = 6.4 Hz, 3H).

### Example 140: Synthesis of 5-ethynyl-4-((5S, 5aS, 6S, 9R)-1-fluoro-12-((1-(((R)-3-fluoropyrrolidin-1-yl)methyl)cyclopropyl)methoxy)-5-(methyl-d3)-5a, 6,7,8,9,10-hexahydro-5H-4-oxa-3,10a,11,13,14-pentaaza-6,9-methanonaphtho[1,8-ab]hepten-2-yl)naphthalen-2-amine (Z964)

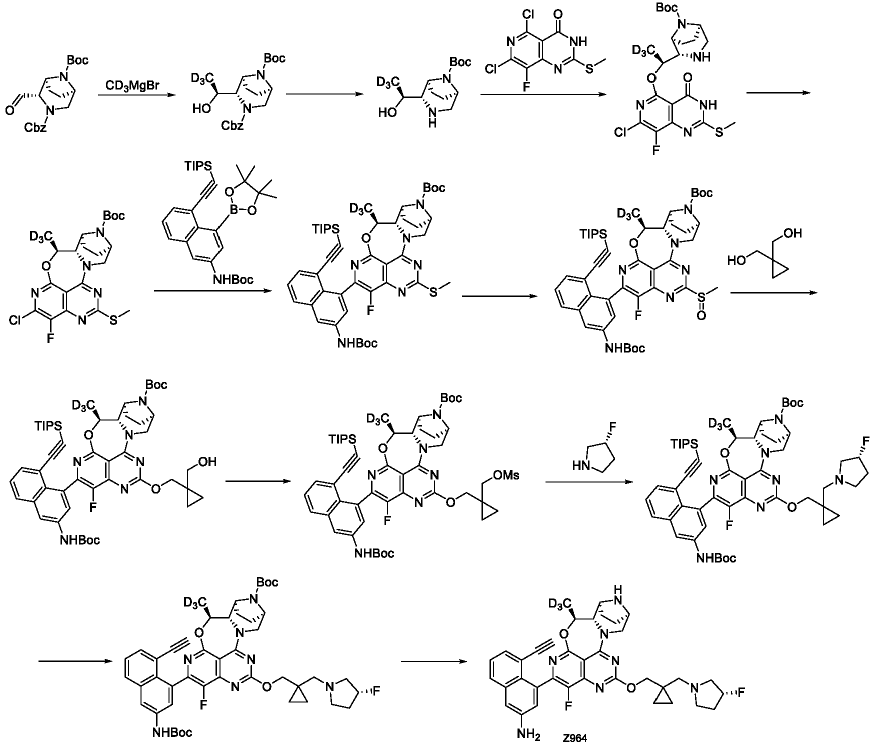

Step I: 3-benzyl 8-(t-butyl) (1S, 2S, 5R)-2-formyl-3,8-diazadicyclo[3.2.1]octane-3,8-dicarboxylate (1738 mg, 4.642 mmol) was dissolved in dry tetrahydrofuran (20 mL). After cooling to -78°C, (methyl-d3)magnesium bromide (9.3 mL, 9.283 mmol) was added dropwise under a nitrogen atmosphere, and reacted at -78°C for 1 hr. The reaction was quenched with a saturated ammonium chloride solution (15 mL), extracted with ethyl acetate (20 mL* 3), dried over anhydrous sodium sulfate, and concentrated. The crude product was purified by automated flash silica gel chromatography (tetrahydrofuran/ petroleum ether= 0- 30%) to obtain 3-benzyl 8-(t-butyl) (1S, 2S, 5R)-2-((S)-1-hydroxyethyl-2,2,2-d3)-3,8-diazadicyclo[3.2.1]octane-3,8-dicarboxylate (725 mg, yield: 40%). ES-API: [M-55]⁺= 338.0.

Step II: 3-benzyl 8-(t-butyl) (1S, 2S, 5R)-2-((S)-1-hydroxyethyl-2,2,2-d3)-3,8-diazadicyclo [3.2.1]octane-3,8-dicarboxylate (680 mg, 1.728 mmol) was dissolved in dichloromethane (20 mL) and methanol (2 mL). The reaction solution was added with triethylsilane (2.0 mL, 6.835 mmol) and 10% palladium on carbon (184 mg), and the reaction solution was stirred at room temperature for 5 hrs. After filtration and concentration, a crude product was obtained, which was t-butyl (1S, 2S, 5R)-2-((S)-1-hydroxyethyl-2,2,2-d3)-3,8-diazadicyclo[3.2.1]octane-8-carboxylate (448 mg). ES-API: [M+ H]⁺= 260.0.

Step III: At 0°C, a sodium hydride solution (691 mg, 17.273 mmol, 60% dispersed in an oil) in tetrahydrofuran (20 mL) was added to t-butyl (1S, 2S, 5R)-2-((S)-1-hydroxyethyl-2,2,2-d3)-3,8-diazadicyclo[3.2.1]octane-8-carboxylate (448 mg, 1.727 mmol), and stirred at this temperature for 10 min. Then 5,7-dichloro-8-fluoro-2-(methylthio)pyrido[4,3-d]pyrimidine4(3H)-one (726 mg, 2.591 mmol) was slowly added to the reaction solution at 0°C, and then the reaction solution was heated to 60°C and heated overnight. After the reaction was completed, the reaction was quenched with a saturated ammonium chloride solution (30 mL), extracted with ethyl acetate (20 mLX 3), dried over anhydrous sodium sulfate, filtered and concentrated. The crude product was purified by silica gel column chromatography (ethyl acetate /petroleum ether= 0- 77%) to obtain t-butyl (1S, 2S,5R)-2-((S)-1-((7-chloro-8-fluoro-2-(methylthio)-4-oxa-3,4-dihydropyridine[4,3-d]pyrimidine-5-yl)oxy)ethyl-2,2,2-d3)-3,8-diazadicyclo[3.2.1]octane-8-carboxylate (526 mg, yield: 61%). ES-API:[M+H]⁺= 503.0.

Step IV: In an iced water bath, 1-propylphosphoric anhydride (50% solution in ethyl acetate) (4.5 mL, 7.559 mmol) was added to a solution of t-butyl (1S, 2S, 5R)-2-((S)-1-((7-chloro-8-fluoro-2-(methylthio)-4-oxo-3,4-dihydropyridine[4,3-d]pyrimidine-5-yl)oxy)ethyl-2,2,2-d3)-3,8-diazadicyclo[3.2.1]octane-8-carboxylate (510 mg, 1.014 mmol) and N,N-diisopropylethylene diamine (1.5 mL, 8.449 mmol) in dichloromethane (15 mL). After that, the reaction solution was heated to room temperature and stirred for 4 hrs. The reaction was quenched with water and extracted with dichloromethane. The organic phase was dried over anhydrous sodium sulfate, filtered, and concentrated. The crude product was purified by silica gel column chromatography (ethyl acetate /petroleum ether= 0- 30%) to obtain t-butyl (5S, 5aS, 6S, 9R)-2-chloro-1-fluoro-5-(methyl-d3)-12-(methylthio)-5a,6,7,8,9,10-hexahydro-5H-4-oxa-3,10a, 11,13,14-pentaaza-6,9-methanonaphtho[1,8-ab]hepten-14-carboxylate (380 mg, yield: 77%). ES-API:[M+H]⁺= 485.0.

Step V: Under a nitrogen atmosphere, t-butyl (5S, 5aS, 6S, 9R)-2-chloro-1-fluoro-5-(methyl-d3)-12-(methylthio)-5a,6,7,8,9,10-hexahydro-5H-4-oxa-3,10a,11,13,14-pentaaza-6,9-methanonaphtho[1,8-ab]hepten-14-carboxylate (500 mg, 1.031 mmol), 4-(4,4,5,5-tetramethyl-1,3,2-dioxaborolan-2-yl)-5-((triisopropylsilyl)ethynyl)naphthalen-2- alkyl)carbamate (850 mg, 1.546 mmol), potassium phosphate (656 mg, 3.093 mmol) and [n-butyldi(1-adamantanyl)phosphine](2-amino-1,1'-biphenyl-2-yl)palladium (II) mesylate(113 mg, 0.155 mmol) were dissolved in tetrahydrofuran (15 mL) and water (3 mL), and the reaction mixture was stirred under a nitrogen atmosphere at 75°C for 3 hrs. The reaction was shown to be completed by LC/MS. The reaction solution was dried over anhydrous sodium sulfate, filtered and concentrated under reduced pressure. The crude product was purified by silica gel column chromatography (ethyl acetate / petroleum ether= 0- 20%) to obtain t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d3)-12-(methylthio)-5a,6,7,8,9,10-hexahydro-5H-4-oxy-3,10a,11,13,14-pentaaza-6,9-methanonaphtho[1,8-ab]hepten-14-carboxylate (692 mg, yield: 77%). ES-API: [M+H]⁺= 872.5.

Step VI: In an iced water bath, t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d3)-12-(methylthio)-5a, 6,7,8,9,10-hexahydro-5H-4-oxa-3,10a,11,13,14-pentaaza-6,9-methanonaphtho[1,8-ab]hepten-14-carboxylate (692 mg, 0.793 mmol) was dissolved in anhydrous dichloromethane (20 mL). m-chloroperoxybenzoic acid (205 mg, 1.190 mmol) was added to the solution, and the reaction solution was stirred at 0°C for 1 hr. The reaction was shown to be completed by LC/MS. The reaction solution was diluted with dichloromethane (15 mL), quenched with a saturated sodium sulfite solution (15 mL), and then washed with a sodium bicarbonate solution (15 mL) and saturated brine (15 mL). The organic phase was dried over anhydrous sodium sulfate, filtered, and concentrated to obtain the crude product t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d3)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (704 mg). ES-API: [M+ H]⁺= 888.4.

Step VII: Cyclopropane-1,1-diyldimethanol (405 mg, 3.963 mmol) was dissolved in anhydrous tetrahydrofuran(20 mL). In an iced water bath, sodium hydride (159 mg, 3.963 mmol) was added, and then reacted for 30 min in the iced water bath. A solution of t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d3)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (704 mg, 0.793 mmol) in tetrahydrofuran (10 mL) was added dropwise to the reaction solution, and reacted for 30 min at 0 °C. The reaction was shown to be completed by LC/MS. The reaction solution was diluted with ethyl acetate (10 mL), washed with saturated ammonium chloride solution (10 mLX2) and saturated brine (10 mLX3), dried over anhydrous sodium sulfate, filtered, and concentrated under reduced pressure. The crude product was purified by silica gel column chromatography (tetrahydrofuran/ petroleum ether= 0- 30%) to obtain t-butyl (5S,5aS,6S,9R)-2-(3-((t-butoxycarbonyl)amino)-8-((triisopropylsilyl) ethynyl)naphthalen-1-yl)-1-fluoro-12-((1-(hydroxymethyl)cyclopropyl)methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (435 mg, yield: 60%). ES-API: [M+ H]⁺= 926.5.

Step VIII: T-butyl (5S,5aS,6S,9R)-2-(3-((t-butoxycarbonyl)amino)-8-((triisopropylsilyl) ethynyl)naphthalen-1-yl)-1-fluoro-12-((1-(hydroxymethyl)cyclopropyl)methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (435 mg, 0.470 mmol) was dissolved in anhydrous dichloromethane (15 mL). In an iced water bath, N,N-diisopropylethyl amine (0.5 mL, 2.818 mmol) and methanesulfonic anhydride (245 mg, 1.409 mmol) were sequentially slowly added to the reaction solution. After that, the reaction solution was slowly heated to room temperature and stirred for 3 hrs. The reaction was shown to be complete by LCMS and TLC. The reaction solution was diluted with dichloromethane (30 mL), washed with a saturated sodium bicarbonate solution (15 mLX2) and saturated brine (15 mLX2), dried over anhydrous sodium sulfate, filtered, and concentrated under reduced pressure. The crude product was purified by automated flash silica gel chromatography (tetrahydrofuran/ petroleum ether= 5- 40%) to obtain t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d3)-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (315 mg, yield: 67%). ES-API: [M+ H]⁺= 1004.5.

Step IX: T-butyl (5S,5aS,6S,9R)-2-(3-((t-butoxycarbonyl)amino)-8-((triisopropylsilyl) ethynyl)naphthalen-1-yl)-1-fluoro-5-(methyl-d3)-12-((1-(((methylsulfonyl)oxy)methyl) cyclopropyl)methoxy)-5a,6,7,8,9,10-hexahydro-5H-4-oxa-3,10a,11,13,14-pentaaza-6,9-methanonaphtho[1,8-ab]hepten-14-carboxylate (238 mg, 0.237 mmol), (R)-3-fluoropyrrolidine (127 mg, 1.422 mmol), potassium carbonate (98 mg, 0.711 mmol) and potassium iodide (39 mg, 0.237 mmol) were dissolved in acetone (20 mL). The reaction solution was heated to 80°C and stirred overnight in a sealed tube. The reaction was detected by LC/MS. The reaction solution was diluted with ethyl acetate (30 mL), and washed with water (15 mLX2) and saturated brine (15 mLX 2). The organic phase was dried over anhydrous sodium sulfate, filtered, and concentrated under reduced pressure. The residue was purified by silica gel column chromatography (methanol / dichloromethane= 0- 1.5%) to obtain the crude product t-butyl (5S, 5aS, 6S, 9R)-2-(3-((t-butoxycarbonyl)amino)-8-((triisopropylsilyl)ethynyl)naphthalen-1-yl)-1-fluoro-12-((1-(((R)-3-fluoropyrrolidin-1-methyl)cyclopropyl)methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (202 mg). ES-API: [M+ H]⁺= 997.3.

Step X: Cesium fluoride (308 mg, 2.025 mmol) was added to t-butyl (5S, 5aS, 6S, 9R)-2-(3-((t-butoxycarbonyl)amino)-8-((triisopropylsilyl)ethynyl)naphthalen-1-yl)-1-fluoro-12-((1-(((R)-3-fluoropyrrolidin-1-methyl)cyclopropyl)methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (202 mg, 0.203 mmol) in N,N-dimethylformamide (5 mL), and the reaction mixture was stirred at room temperature for 2 hrs. The reaction was shown to be completed by LC/MS. The reaction solution was diluted with ethyl acetate (20 mL), washed with water (15 mLX2) and saturated brine (15 mLX3), dried over anhydrous sodium sulfate, filtered, and concentrated. The residue was purified by automated flash silica gel chromatography (methanol / dichloromethane= 0- 1.0%) to obtain the crude product t-butyl (5S, 5aS, 6S, 9R)-2-(3-((t-butoxycarbonyl)amino)-8-ethynylnaphthalen-1-yl)-1-fluoro-12-((1-(((R)-3-fluoropyrrolidin-1-methyl)cyclopropyl)methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (170 mg). ES-API: [M+H]⁺= 841.2.

Step XI: In an iced water bath, a 4 M hydrochloric acid-dioxane solution (5 mL) was slowly added to a solution of t-butyl (5S, 5aS, 6S, 9R)-2-(3-((t-butoxycarbonyl)amino)-8-ethynylnaphthalen-1-yl)-1-fluoro-12-((1-(((R)-3-fluoropyrrolidin-1-methyl)cyclopropyl) methoxy)-5-(methyl-d3)-5a,6,7,8,9,10-hexahydro-5H-4-oxa-3,10a,11,13,14-pentaaza-6,9-methanonaphtho[1,8-ab]hepten-14-carboxylate (170 mg, 0.202 mmol) in ethyl acetate (2 mL). The reaction mixture was heated to room temperature and stirred for 2 hrs. The reaction was shown to be completed by LC/MS. The reaction solution was concentrated to dryness under reduced pressure, and the residue was purified by preparative HPLC (chromatographic column: Welch Xtimate, 21.2*150mm, 5um; mobile phase A: 5mmol/L NH4HCO3aqueous solution, mobile phase B: ACN; flow rate: 15mL/min; chromatographic condition: 15mL-45-60-13min; column temperature: room temperature) to obtain 5-ethynyl-4-((5S, 5aS, 6S, 9R)-1-fluoro-12-((1-(((R)-3-fluoropyrrolidin-1-yl)methyl)cyclopropyl)methoxy)-5-(methyl-d3)-5a,6,7,8,9,10-hexahydro-5H-4-oxy-3,10a,11,13,14-pentaaza-6,9-methanonaphtho[1,8-ab]hepten-2-yl)naphthalen-2-amine (Z964, 10.72 mg, yield: 8%) as an off-white solid. ES-API: [M+H]⁺= 641.2. ¹H NMR (400 MHz, CD₃OD) δ 7.72 - 7.61 (m, 1H), 7.44 - 7.25 (m, 2H), 7.13 (d, *J =* 2.4 Hz, 1H), 7.11 - 6.98 (m, 1H), 5.43 - 5.32 (m, 1H), 5.24 - 5.02 (m, 1H), 4.61 - 4.41 (m, 2H), 4.35 - 4.22 (m, 1H), 4.05 (dd, *J =* 8.8 Hz, 0.8Hz, 1H), 3.68 (d, *J =* 5.6 Hz, 1H), 3.57 (d, *J* = 4.8 Hz, 1H), 3.29 - 2.87 (m,4H), 2.76 - 2.47 (m, 3H), 2.46 - 2.37 (m, 1H), 2.23 - 1.72 (m, 6H), 0.78 - 0.63 (m, 2H), 0.59 - 0.43 (m, 2H).

### Example 141: Synthesis of 3-chloro-5-((5S,5aS,6S,9R)-12-(((S)-2-(difluoromethylene) tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z1084)

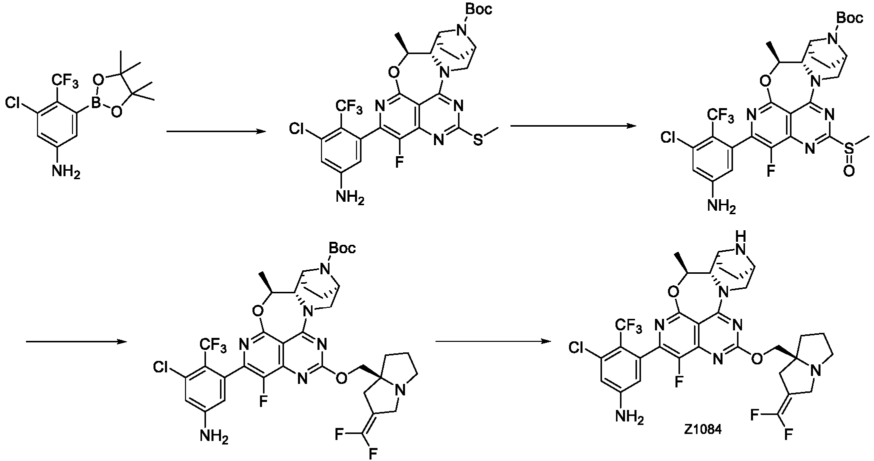

Step I: Potassium phosphate (1.762 g, 8.316 mmol) and [n-butyldi(1-adamantanyl) phosphine](2-amino-1,1'-biphenyl-2-yl)palladium (II) mesylate (226.7 mg, 0.3118 mmol) were added to t-butyl (5S,5aS,6S,9R)-2-chloro-1-fluoro-5-methyl-12-(2-methylthio)-5a,6,7,8,9,10-hexahydro-5H-4-oxa-3,10a,11,13,14-pentaaza-6,9-methanonaphtho[1,8-ab]hepten-14-carboxylate (1.0 g, 2.079 mmol) and 3-chloro-5-(4,4,5,5-tetramethyl-1,3,2-dioxaborolan-2-yl)-4-(trifluoromethyl)aniline (1.30 g, 4.049 mmol) in tetrahydrofuran(30 mL) and water (3 mL). Nitrogen was bubbled therethrough, and the reaction was continued at 85°C under microwave for 50 min. After the reaction, the reaction solution was extracted with ethyl acetate (80 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(5-amino-3-chloro-2-(trifluoromethyl)phenyl)-1-fluoro-5-methyl-12-(methylthio)-5a,6,7,8,9,10-hexahydro-5H-4-oxa-3,10a,11,13,14-pentaaza-6,9-methanonaphtho [1,8-ab]hepten-14-carboxylate (1.0 g, yield: 75.18%) as a yellow solid. ES-API: [M+H]⁺=641.2.

Step II: At 0°C, m-chloroperoxybenzoic acid (404.8 mg, 2.34 mmol) was added to a solution of t-butyl (5S,5aS,6S,9R)-2-(5-amino-3-chloro-2-(trifluoromethyl)phenyl)-1-fluoro-5-methyl-12-(methylthio)-5a,6,7,8,9,10-hexahydro-5H-4-oxo-3,10a,11,13,14-pentaaza-6,9-methanonaphtho [1,8-ab]hepten-14-carboxylate (1.0 g, 1.56 mmol) in dichloromethane (30.0 mL). The solution was stirred for 1 hour in an ice bath. The reaction was quenched with saturated sodium sulfite and extracted with dichloromethane (100 mLX2). The organic phase was dried over anhydrous sodium sulfate, filtered, and concentrated to obtain a crude product. The crude product was purified by by silica gel column chromatography (0-10% methanol /dichloromethane) to obtain t-butyl (5S,5aS,6S,9R)-2-(5-amino-3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (1.0 g, yield: 97.56%) as a white solid. ES-API:[M+H]⁺=657.1.

Step III: At 0°C, sodium hydride (126.3 mg, 3.157 mmol) was added to a solution of (S)-(1-(difluoromethylene)tetrahydro-1H-pyrrolizin-7a(5H)-yl)methanol (240.0 mg, 1.263 mmol) in tetrahydrofuran (10.0 mL), and stirred at this temperature for 10 min. Then, a solution of t-butyl (5S,5aS,6S,9R)-2-(5-amino-3-chloro-2-(trifluoromethyl)phenyl)-1-fluoro-5-methyl-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (1.0 g, 1.522 mmol) in tetrahydrofuran (5 mL) was added. The reaction was continued with stirring at 0°C for 10 min, and at room temperature for 0.5 hr. After the reaction, the reaction solution was quenched with a saturated ammonium chloride solution (20 mL) and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(5-amino-3-chloro-2-(trifluoromethyl)phenyl)-12-(((S)-2-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (1.20 g, crude product) as a yellow solid. ES-API: [M+H]⁺=782.2.

Step IV: In an iced water bath, t-butyl (5S,5aS,6S,9R)-2-(5-amino-3-chloro-2-(trifluoromethyl)phenyl)-12-(((S)-2-(difluoromethylene)tetrahydro-1H-pyrrolizin)-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-14-carboxylate (1.20 g, crude product) was slowly added to dichloromethane (30.0 mL). Finally, trifluoroacetic acid (10.0 mL, 130.6 mmol) was added. The reaction mixture was stirred at room temperature for 3.0 hrs. The reaction was shown to be completed by LC/MS. The reaction solution was concentrated to dryness under reduced pressure, and the residue was purified by preparative HPLC (chromatographic column: Welch Xtimate, 21.2*150mm, 5um; mobile phase A: 5mmol/L NH4HCO3aqueous solution, mobile phase B: ACN; flow rate: 15mL/min; chromatographic condition: 15mL-35-85-13min; column temperature: room temperature) to obtain 3-chloro-5-((5S,5aS,6S,9R)-12-(((S)-2-(difluoromethylene)tetrahydro-1H-pyrrolizin-7a(5H)-yl)methoxy)-1-fluoro-5-methyl-5a,6,7,8,9,10-hexahydro-5H-4-oxo-3,10a,11,13,14-pentaaza-6,9-methanonaphtho[1,8-ab]hepten-2-yl)-4-(trifluoromethyl)aniline (Z1084, 280.0 mg, yield: 26.7%) as a white solid. ES-API: [M+H]⁺= 682.2.

### Example 142: Synthesis of 3-chloro-5-((5S,5aS,6S,9R)-12-(((S)-2-(difluoromethylene) tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z1085)

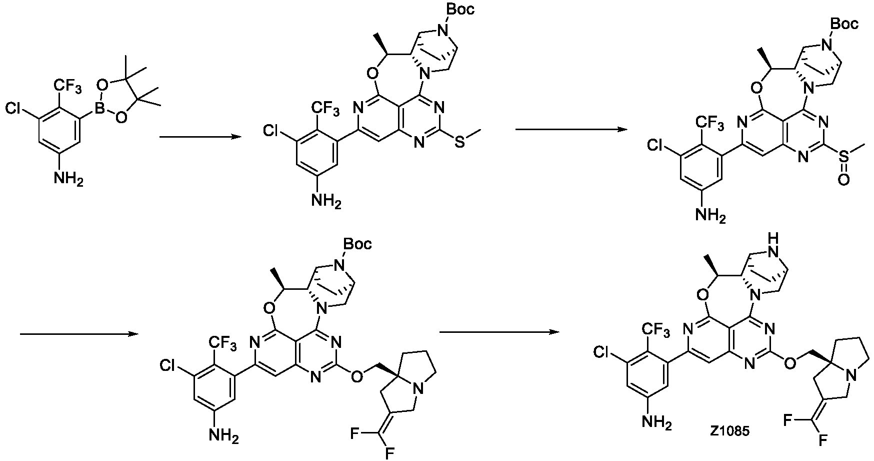

Step I: Potassium phosphate (2.20 g, 10.345 mmol) and [n-butyldi(1-adamantanyl)phosphine](2-amino-1,1'-biphenyl-2-yl)palladium (II) mesylate (280.0 mg, 0.388 mmol) were added to t-butyl (5S,5aS,6S,9R)-2-chloro-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (1.10 g, 2.375 mmol) and 3-chloro-5-(4,4,5,5-tetramethyl-1,3,2-dioxaborolan-2-yl)-4-(trifluoromethyl)aniline (1.68 g, 5.23 mmol) in tetrahydrofuran (30 mL) and water (3 mL). Nitrogen was bubbled therethrough, and the reaction was continued at 80°C under microwave for 45 min. After the reaction, the reaction solution was extracted with ethyl acetate (80 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(5-amino-3-chloro-2-(trifluoromethyl)phenyl)-5-methyl-12-(methylthio)-5a,6,7,8,9,10-hexahydro-5H-4-oxa-3,10a,11,13,14-pentaaza-6,9-methanonaphtho[1,8-ab]hepten-14-carboxylate (1.56 mg, crude product) as a yellow solid. ES-API: [M+H]⁺=623.2.

Step II: At 0°C, m-chloroperoxybenzoic acid (650.0 mg, 3.755 mmol) was added to a solution of t-butyl (5S,5aS,6S,9R)-2-(5-amino-3-chloro-2-(trifluoromethyl)phenyl)-5-methyl-12-(methylthio)-5a,6,7,8,9, 10-hexahydro-5H-4-oxo-3,10a,11,13,14-pentaaza-6,9-methanonaphtho [1,8-ab]hepten-14-carboxylate (1.56 mg, crude product) in dichloromethane (30.0 mL). The solution was stirred for 1 hour in an ice bath. The reaction was quenched with saturated sodium sulfite and extracted with dichloromethane (100 mLX2). The organic phase was dried over anhydrous sodium sulfate, and concentrated to obtain a crude product. The crude product was purified by silica gel column chromatography (0-10% methanol /dichloromethane) to obtain t-butyl (5S,5aS,6S,9R)-2-(5-amino-3-chloro-2-(trifluoromethyl)phenyl)-5-methyl-12-(methylsulfinyl)-5a,6,7,8, 9,10-hexahydro-5H-4-oxa-3,10a,11,13,14-pentaaza-6,9-methanonaphtho[1,8-ab]hepten-14-carboxylate (1.20 g, yield: 75.47%) as a white solid. ES-API:[M+H]⁺=639.0.

Step III: At 0°C, sodium hydride (188.0 mg, 4.70 mmol) was added to a solution of (S)-(2-(difluoromethylene)tetrahydro-1H-pyrrolizin-7a(5H)-yl)methanol (360.0 mg, 1.878 mmol) in tetrahydrofuran (10.0 mL), and stirred at this temperature for 10 min. Then, a solution of t-butyl (5S,5aS,6S,9R)-2-(5-amino-3-chloro-2-(trifluoromethyl)phenyl)-5-methyl-12-(methylsulfinyl)-5a,6,7,8, 9,10-hexahydro-5H-4-oxa-3,10a,11,13,14-pentaaza-6,9-methanonaphtho[1,8-ab]hepten-14-carboxylate (1.20 g, 1.878 mmol) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(5-amino-3-chloro-2-(trifluoromethyl)phenyl)-12-((((S)-2-(difluoromethylene)tetrahydro-1H-(difluoromethylene)tetrahydro-1H-pyrrolizin)-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (1.20 g, crude product) as a yellow solid. ES-API: [M+H]⁺=764.2.

Step IV: In an iced water bath, t-butyl (5S,5aS,6S,9R)-2-(5-amino-3-chloro-2-(trifluoromethyl)phenyl)-12-(((S)-2-(difluoromethylene)tetrahydro-1H-(difluoromethylene) tetrahydro-1H-pyrrolizin)-7a(5H)-yl)methoxy)-5-methyl-5a,6,7,8,9,10-hexahydro-5H-4-oxa-3,10a,11,13,14-pentaaza-6,9-methanonaphtho[1,8-ab]hepten-14-carboxylate (1.20 g, crude product) was slowly added to dichloromethane (30.0 mL). Finally, trifluoroacetic acid (10.0 mL, 130.0 mmol) was added. The reaction mixture was stirred at room temperature for 3.0 hrs. The reaction was shown to be completed by LC/MS. The reaction solution was concentrated to dryness under reduced pressure, and the residue was purified by preparative HPLC (chromatographic column: Welch Xtimate, 21.2*150mm, 5um; mobile phase A: 5mmol/L NH4HCO3aqueous solution, mobile phase B: ACN; flow rate: 15mL/min; chromatographic condition: 15mL-35-85-13min; column temperature: room temperature) to obtain 3-chloro-5-((5S,5aS,6S,9R)-12-(((S)-2-(difluoromethylene)tetrahydro-1H-pyrrolizin-7a(5H)-yl)methoxy)-5-methyl-5a,6,7,8,9,10-hexahydro-5H-4-oxo-3,10a,11,13,14-pentaaza-6,9-methanonaphtho[1,8-ab]hepten-2-yl)-4-(trifluoromethyl)aniline (Z1085, 180.0 mg, yield: 14.44%) as a white solid. ES-API: [M+H]⁺= 664.2 ¹H NMR (400 MHz, CD₃OD) δ 7.00 (s, 1H), 6.83-6.81(m, 1H), 6.47-6.45 (m, 1H), 5.32-5.28 (m, 1H), 4.55-4.52 (m, 1H), 4.30-4.27 (m, 1H), 4.23-4.21 (m, 1H), 4.07-4.04(m, 1H), 3.80-3.76 (m, 1H), 3.67-3.65 (m, 1H), 3.60-3.58 (m, 1H), 3.45-3.42 (m, 1H), 3.19-3.08 (m, 2H), 2.83-2.66 (m, 2H), 2.53-2.48 (m, 1H), 2.13-1.73 (m, 8H), 1.58-1.56 (m, 3H).

### Example 143: Synthesis of 4-((5S,5aS,6S,9R)-12-(((R)-6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z956)

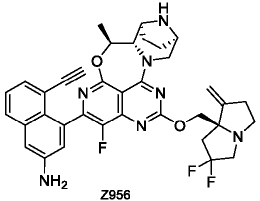

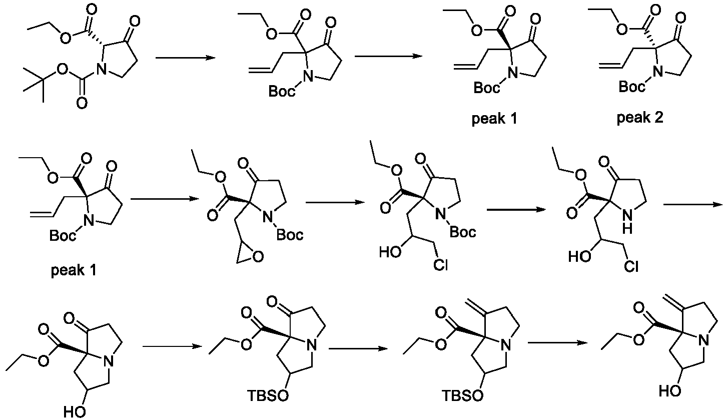

Step I: A mixture of 1-(t-butyl) 2-ethyl (S)-3-oxypyridine-1,2-dicarboxylate (6.0 g, 23.3 mmol), 3-bromoprop-1-ene (14.0 g, 116.5 mmol), potassium carbonate (6.4 g, 46.6 mmol) and N, N-dimethylformamide (100 mL) was stirred at room temperature under a nitrogen atmosphere for 6 hrs. The reaction mixture was added with water (200 mL) and extracted with ethyl acetate (300 mLX2). The organic phase was washed with saturated brine (200 mLX3), dried over anhydrous sodium sulfate, filtered and concentrated to obtain a crude product. The crude product was purified by flash silica gel column chromatography (ethyl acetate /petroleum ether: 0-15%) to obtain 1-(t-butyl) 2-ethyl 2-allyl-3-oxopyrrolidin-1,2-dicarboxylate (3.7 g, yield: 53%) as a colorless transparent liquid. ES-API: [M-55]⁺= 242.2.

Step II: 1-(t-butyl) 2-ethyl2-allyl-3-oxopyrrolidin-1,2-dicarboxylate (3.7 g) was chirally resolved (separation column: IG (250mm*4.6mm*5um); mobile phase: HEX: ETOH=85:15; flow rate: 1.0 mL/min; column temperature: 30.0°C) to obtain two isomers. One isomer structure was arbitrarily designated as 1-(t-butyl) 2-ethyl (R)-2-acyl-3-oxypyridin-1,2-dicarboxylate (peak 1, 1.41 g, purity: 100%, de value: 99%, retention time: 4.985min). ¹H NMR (400 MHz, CDCl3) δ 5.61-5.50 (m, 1H), 5.13 (s, 1H), 5.11 - 5.04 (m, 1H), 4.24-4.10 (m, 2H), 3.95-3.81 (m, 1H), 3.70-3.58 m, 1H), 3.13-3.01 (m, 1H), 2.95-2.85 (m, 1H), 2.75-2.65 (m, 1H), 2.53-2.43 (m, 1H), 1.49-1.43 (m, 9H), 1.30 - 1.21 (m, 3H). ES-API: [M-55]⁺= 242.2. The other isomer structure was arbitrarily designated as 1-(t-butyl) 2-ethyl (S)-2-acyl-3-oxypyridin-1,2-dicarboxylate (peak 2, 1.35 g, purity: 100%, de value: 99%, retention time: 6.344min): ¹H NMR (400 MHz, CDCl3) δ 5.70-5.44 (m, 1H), 5.17 (s, 1H), 5.17 - 5.06 (m, 1H), 4.29-4.13 (m, 2H), 3.98-3.83 (m, 1H), 3.81-3.62 m, 1H), 3.19-3.06 (m, 1H), 2.97-2.87 (m, 1H), 2.79-2.69 (m, 1H), 2.54-2.45 (m, 1H), 1.51-1.45 (m, 9H), 1.31 - 1.23 (m, 3H). ES-API: [M-55]⁺= 242.2.

Step III: 1-(t-butyl) 2-ethyl (R)-2-acyl-3-oxypyridin-1,2-dicarboxylate (peak 1, 1.41 g, 4.72 mmol) was dissolved in dichloromethane (10 mL). Then m-chloroperoxybenzoic acid (2.05 g, 11.855 mmol) was added, and reacted overnight at room temperature. The reaction solution was added with saturated sodium sulfite (50 mL) and extracted with dichloromethane (50 mL X2). The organic phase was washed with saturated brine (50 mLX3), dried over anhydrous sodium sulfate, filtered and concentrated to obtain the crude product 1-(t-butyl) 2-ethyl (2R)-2-(cyclooxy-2-ylmethyl)-3-oxypyrrolidin-1,2-dicarboxylate (1485.87 mg, 4.742 mmol, yield: 100%). ES-API: [M+H]⁺=314.2.

Step IV: 1-(t-butyl) 2-ethyl (2R)-2-(cyclooxy-2-ylmethyl)-3-oxypyrrolidin-1,2-dicarboxylate (1485.87 mg, 4.742 mmol) was dissolved in acetonitrile (10 mL). Then, acetic acid (5 mL, 87.344 mmol) and lithium chloride (1.89 g, 44.678 mmol) were added, and reacted at room temperature for 5 hrs. The reaction solution was concentrated to obtain a crude product. The crude product was added with saturated ammonium chloride (50 mL) and extracted with ethyl acetate (50 mL X2). The organic phase was washed with saturated brine (50 mLX3), dried over anhydrous sodium sulfate, filtered and concentrated to obtain a crude product. The crude product was purified by flash silica gel column chromatography (ethyl acetate /petroleum ether: 0-15%) to obtain 1-(t-butyl) 2-ethyl (2R)-2-(3-chloro-2-hydroxylpropyl)-3-oxypyrrolidin-1,2-dicarboxylate (1.4 g, 4.002 mmol, yield: 89.74%). ES-API: [M-99]⁺= 250.1.

Step V: In an iced water bath, trifluoroacetic acid (10 mL) was slowly added to a solution of 1-(t-butyl) 2-ethyl (2R)-2-(3-chloro-2-hydroxylpropyl)-3-oxypyrrolidin-1,2-dicarboxylate (1.4 g, 4.002 mmol) in dichloromethane (5 mL). The reaction mixture was stirred at room temperature for 2 hrs. The reaction was shown to be completed by LC/MS. The reaction solution was concentrated to dryness under reduced pressure to obtain the crude product ethyl(2R)-2-(3-chloro-2-hydroxylpropionyl)-3-oxypyrrolidin-2-carboxylate (999.30 mg, 4.002 mmol, yield: 100%). ES-API: [M-99]⁺= 250.1.

Step VI: Ethyl (2R)-2-(3-chloro-2-hydroxylpropionyl)-3-oxypyrrolidin-2-carboxylate (999.30 mg, 4.002 mmol) was dissolved in acetonitrile (10 mL). Then, sodium bicarbonate (1680.60 mg, 20.005 mmol) and potassium iodide (66.42 mg, 0.400 mmol) was added and reacted at 70°C for 5 hrs. The reaction solution was filtered, to remove the filter residue. The filter residue was washed with ethyl acetate (50 mL X2), to obtain a filtrate. The filtrate was concentrated to obtain the crude product ethyl (7aR)-6-hydroxyl-1-oxymethyl-1H-pyrrolizin-7a(5H)-carboxylate (850.00 mg, 3.986 mmol, yield: 100%). ES-API: [M+1]⁺=214.1.

Step VII: Ethyl (7aR)-6-hydroxyl-1-oxymethyl-1H-pyrrolizin-7a(5H)-carboxylate (850.00 mg, 3.986 mmol was dissolved in dichloromethane (10 mL). Then, 4-dimethylaminopyridine (48.70 mg, 0.399 mmol), imidazole (407.08 mg, 5.979 mmol) and t-butyldimethylchlorosilane (717.54 mg, 4.784 mmol) were added, and reacted at room temperature for 2 hrs. The reaction solution was added with saturated sodium bicarbonate (50 mL) and extracted with dichloromethane (50 mL X2). The organic phase was washed with saturated brine (50 mLX3), dried over anhydrous sodium sulfate, filtered and concentrated to obtain a crude product. The crude product was purified by flash silica gel column chromatography (methanol /dichloromethane: 0-15%) to obtain ethyl (7aR)-6-(((tertt-butylmethylsilyl)oxy)-1-oxymethyl-1H-pyrrolizin-7a(5H)-carboxylate (630 mg, 1.924 mmol, yield: 48.09%). ES-API: [M+H]⁺=328.3.

Step VIII: Under a nitrogen atmosphere, methyltriphenylphosphonium bromide (3433.74 mg, 9.618 mmol) was dissolved in dry tetrahydrofuran (50 mL). In an iced water bath, a potassium tert-butoxide solution in tetrahydrofuran (7.695 mL, 1 M in tetrahydrofuran) was slowly added dropwise, and reacted at room temperature for 1 hr. A solution of ethyl (7aR)-6-(((t-butylmethylsilyl)oxy)-1-oxymethyl-1H-pyrrolizin-7a(5H)-carboxylate (630 mg, 1.924 mmol) in tetrahydrofuran (5 mL) was slowly added dropwise, and reacted overnight at 65°C. The reaction solution was added with saturated ammonium chloride (50 mL) and extracted with ethyl acetate (50 mL X2). The organic phase was washed with saturated brine (50 mLX3), dried over anhydrous sodium sulfate, filtered and concentrated to obtain a crude product. The crude product was purified by flash silica gel column chromatography (methanol /dichloromethane: 0-15%) to obtain ethyl (7aR)-6-(((t-butyldimethylsilyl)oxy)-1-methylphthalic acid-1H-pyrrolizin-7a(5H)-carboxylate (156.55 mg, 0.481 mmol, yield: 25%). ES-API: [M+H]⁺=326.2.

Step IX: In an iced water bath, concentrated hydrochloric acid (2 mL) was slowly added to a solution of ethyl (7aR)-6-(((t-butyldimethylsilyl)oxy)-1-methylphthalic acid-1H-pyrrolizin-7a(5H)-carboxylate (156.55 mg, 0.481 mmol) in methanol (3 mL). The reaction mixture was stirred at room temperature for 2 hrs. The reaction was shown to be completed by LC/MS. The reaction solution was concentrated to dryness under reduced pressure to obtain a crude product. The crude product was purified by flash silica gel column chromatography (methanol /dichloromethane: 0-15%) to obtain ethyl (7aR)-6-hydroxyl-1-methylenetetrahydro-1H-pyrrolizin-7a(5H)-carboxylate (72.5 mg, 0.34 mmol, yield: 71%). ES-API: [M+H]⁺=212.2.

Starting from ethyl (7aR)-6-hydroxyl-1-methylenetetrahydro-1H-pyrrolizin-7a(5H)-carboxylate obtained in Step IX, Compound Z956 was finally obtained by reaction following the steps in Example 125. ES-API: [M+H]⁺=654.3.

### Example 144: Synthesis of 4-((5S,5aS,6S,9R)-12-(((S)-6,6-difluoro-1-methylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z1076)

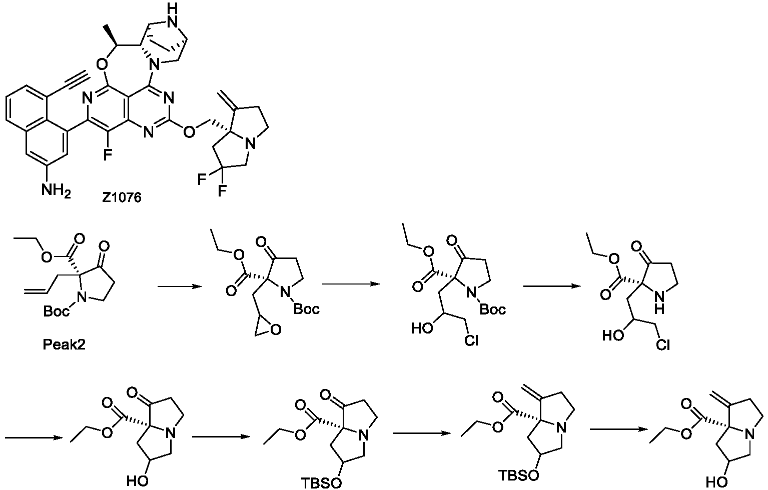

Step I: 1-(t-butyl) 2-ethyl (S)-2-acyl-3-oxypyridine1,2-dicarboxylate (peak 2, 1.35 g, 4.540 mmol) was dissolved in dichloromethane (10 mL). Then, m-chloroperoxybenzoic acid (1.96 g, 11.350 mmol) was added, and reacted overnight at room temperature. The reaction solution was added with saturated sodium sulfite (50 mL) and extracted with dichloromethane (50 mL X2). The organic phase was washed with saturated brine (50 mLX3), dried over anhydrous sodium sulfate, filtered and concentrated to obtain the crude product 1-(t-butyl) 2-ethyl (2S)-2-(cyclooxy-2-ylmethyl)-3-oxypyrrolidin-1,2-dicarboxylate (1422.61 mg, 4.540 mmol, yield: 100%). ES-API: [M+H]⁺=314.2.

Step II: 1-(t-butyl) 2-ethyl (2S)-2-(cyclooxy-2-ylmethyl)-3-oxypyrrolidin-1,2-dicarboxylate (1.4 g, 4.468 mmol) was dissolved in acetonitrile (10 mL). Then acetic acid (5 mL, 87.344 mmol) and lithium chloride (1.89 g, 44.678 mmol) were added, and reacted at room temperature for 5 hrs. The reaction solution was concentrated to obtain a crude product. The crude product was added with saturated ammonium chloride (50 mL) and extracted with ethyl acetate (50 mL X2). The organic phase was washed with saturated brine (50 mLX3), dried over anhydrous sodium sulfate, filtered and concentrated to obtain a crude product. The crude product was purified by flash silica gel column chromatography (ethyl acetate /petroleum ether: 0-15%) to obtain 1-(t-butyl) 2-ethyl (2S)-2-(3-chloro-2-hydroxylpropyl)-3-oxypyrrolidin-1,2-dicarboxylate (1560.00 mg, 4.460 mmol, yield: 100%). ES-API: [M-99]⁺= 250.1.

Step III: In an iced water bath, trifluoroacetic acid (10 mL) was slowly added to a solution of 1-(t-butyl) 2-ethyl (2S)-2-(3-chloro-2-hydroxylpropyl)-3-oxypyrrolidin-1,2-dicarboxylate (1.56 g, 4.460 mmol) in dichloromethane (5 mL). The reaction mixture was stirred at room temperature for 2 hrs. The reaction was shown to be completed by LC/MS. The reaction solution was concentrated to dryness under reduced pressure to obtain the crude product ethyl(2S)-2-(3-chloro-2-hydroxylpropionyl)-3-oxypyrrolidin-2-carboxylate (1113.51 mg, 4.460 mmol, yield: 100%). ES-API: [M-99]⁺= 250.1.

Step IV: Ethyl (2S)-2-(3-chloro-2-hydroxylpropionyl)-3-oxypyrrolidin-2-carboxylate (1.1 g, 4.405 mmol) was dissolved in acetonitrile (10 mL). Then, sodium bicarbonate (1.85 g, 22.027 mmol) and potassium iodide (0.07 g, 0.441 mmol) were added, and reacted at 70°C for 5 hrs. The reaction solution was filtered, to remove the filter residue. The filter residue was washed with ethyl acetate (50 mL X2), to obtain a filtrate. The filtrate was concentrated to obtain the crude product ethyl (7aS)-6-hydroxyl-1-oxymethyl-1H-pyrrolizin-7a(5H)-carboxylate (939.38 mg, 4.405 mmol, yield: 100%). ES-API: [M+1]⁺=214.1.

Step V: Ethyl (7aS)-6-hydroxyl-1-oxymethyl-1H-pyrrolizin-7a(5H)-carboxylate -carboxylate (939 mg, 4.404 mmol) was dissolved in dichloromethane (10 mL). Then, 4-dimethylaminopyridine (53.80 mg, 0.440 mmol), imidazole (449.71 mg, 6.606 mmol) and t-butyldimethylchlorosilane (792.67 mg, 5.284 mmol) were added, and reacted at room temperature for 2 hrs. The reaction solution was added with saturated sodium bicarbonate (50 mL) and extracted with dichloromethane (50 mL X2). The organic phase was washed with saturated brine (50 mLX3), dried over anhydrous sodium sulfate, filtered and concentrated to obtain a crude product. The crude product was purified by flash silica gel column chromatography (methanol /dichloromethane: 0-15%) to obtain ethyl (7aS)-6-(((t-butylmethylsilyl)oxy)-1-oxymethyl-1H-pyrrolizin-7a(5H)-carboxylate (640 mg, 1.954 mmol, yield: 44.38%). ES-API: [M+H]⁺=328.3.

Step VI: Under a nitrogen atmosphere, methyltriphenylphosphonium bromide (3488.24 mg, 9.771 mmol) was dissolved in dry tetrahydrofuran (50 mL). In an iced water bath, a potassium tert-butoxide solution in tetrahydrofuran (7.8 mL, 1 M solution in tetrahydrofuran) was slowly added dropwise, and reacted at room temperature for 1 hr. A solution of ethyl (7aS)-6-(((t-butylmethylsilyl)oxy)-1-oxymethyl-1H-pyrrolizin-7a(5H)-carboxylate (640 mg, 1.954 mmol) in tetrahydrofuran (5 mL) was slowly added dropwise, and reacted overnight at 65°C. The reaction solution was added with saturated ammonium chloride (50 mL) and extracted with ethyl acetate (50 mL X2). The organic phase was washed with saturated brine (50 mLX3), dried over anhydrous sodium sulfate, filtered and concentrated to obtain a crude product. The crude product was purified by flash silica gel column chromatography (methanol /dichloromethane: 0-15%) to obtain ethyl (7aS)-6-(((t-butyldimethylsilyl)oxy)-1-methylphthalic acid-1H-pyrrolizin-7a(5H)-carboxylate (146.31 mg, 0.449 mmol, yield: 23%). ES-API: [M+H]⁺=326.2.

Step VII: In an iced water bath, concentrated hydrochloric acid (2 mL) was slowly added to a solution of ethyl (7aS)-6-(((t-butyldimethylsilyl)oxy)-1-methylphthalic acid-1H-pyrrolizin-7a(5H)-carboxylate (146 mg, 0.449 mmol) in methanol (3 mL). The reaction mixture was stirred at room temperature for 2 hrs. The reaction was shown to be completed by LC/MS. The reaction solution was concentrated to dryness under reduced pressure to obtain a crude product. The crude product was purified by flash silica gel column chromatography (methanol /dichloromethane: 0-15%) to obtain ethyl (7aS)-6-hydroxyl-1-methylenetetrahydro-1H-pyrrolizin-7a(5H)-carboxylate (70 mg, 0.33 mmol, yield: 73 %). ES-API: [M+H]⁺=212.2.

Starting from ethyl (7aS)-6-hydroxyl-1-methylenetetrahydro-1H-pyrrolizin-7a(5H)-carboxylate obtained in Step VII, Compound Z1076 was finally obtained by reaction following the steps in Example 125. ES-API:[M+H]⁺=654.3.

### Example 145: Synthesis of 3-chloro-5-((5S,5aS,6S,9R)- 1-fluoro-5-(methyl-d₃)-12-(((R)- 1-methylenetetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-2-yl)-4-(trifluoromethyl)aniline (Z1060)

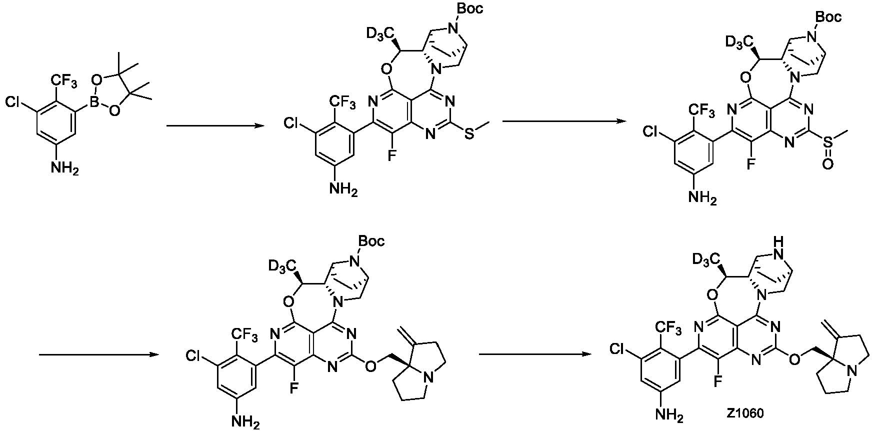

Step I: Potassium phosphate (2.64 g, 12.468 mmol) and [n-butyldi(1-adamantanyl) phosphine](2-amino-1,1'-biphenyl-2-yl)palladium (II) mesylate (340.0 mg,0.465 mmol) were added to t-butyl (5S,5aS,6S,9R)-2-chloro-1-fluoro-5-(methyl-d₃)-12-(2-methylthio)-5a,6,7,8,9,10-hexahydro-5H-4-oxa-3,10a,11,13,14-pentaaza-6,9-methanonaphtho[1,8-ab]hepten-14-carboxylate (2.10 g, 6.531 mmol) and 3-chloro-5-(4,4,5,5-tetramethyl-1,3,2-dioxaborolan-2-yl)-4-(trifluoromethyl)aniline(1.40 g, 2.905 mmol) in tetrahydrofuran (30 mL) and water (3 mL). N₂ was bubbled therethrough, and the reaction was continued at 80°C under microwave for 45 min. After the reaction, the reaction solution was extracted with ethyl acetate (80 mLX2) and saturated brine (80 mL). The ethyl acetate phases were combined, dried over anhydrous sodium sulfate, and filtered. The filtrate was concentrated under reduced pressure. The crude product was purified by silica gel column chromatography (0-4% methanol /dichloromethane) to obtain t-butyl (5S,5aS,6S,9R)-2-(5-amino-3-chloro-2-(trifluoromethyl)phenyl)-1-fluoro-5-(methyl-d₃)-12-(methylthio)-5a,6,7,8,9,10-hexahydro-5H-4-oxa-3,10a,11,13,14-pentaaza-6,9-methanonaphtho[1,8-ab]hepten-14-carboxylate (1.70 g, yield: 91.29%) as a yellow solid. ES-API: [M+H]⁺=644.0.

Step II: At 0°C, m-chloroperoxybenzoic acid (810.0 mg, 4.68 mmol) was added to a solution of t-butyl (5S,5aS,6S,9R)-2-(5-amino-3-chloro-2-(trifluoromethyl)phenyl)-1-fluoro-5-(methyl-d₃)-12-(methylthio)-5a,6,7,8,9,10-hexahydro-5H-4-oxa-3,10a,11,13,14-pentaaza-6,9-methanonaphtho[1,8-ab]hepten-14-carboxylate (1.70 g, 2.639 mmol) in dichloromethane (30.0 mL). The solution was stirred for 1 hour in an ice bath. The reaction was quenched with saturated sodium sulfite and extracted with dichloromethane (100 mLX2). The organic phase was dried over anhydrous sodium sulfate, filtered and concentrated to obtain a crude product. The crude product was purified by silica gel column chromatography (0-10% methanol/dichloromethane) to obtain t-butyl (5S,5aS,6S,9R)-2-(5-amino-3-chloro-2-(trifluoromethyl)phenyl)-1-fluoro-5-(methyl-d3)-12-(methylsulfinyl)-5a,6,7,8,9,10-hexahydro-5H-4-oxo-3,10a,11,13,14-pentaaza-6,9-methanonaphtho[1,8-ab]hepten-14-carboxylate (1.55 g,yield: 88.98%) as a white solid. ES-API:[M+H]⁺=660.1.

Step III: At 0°C, sodium hydride (261.0 mg, 6.52 mmol) was added to a solution of (R)-(1-methylenetetrahydro-1H-pyrrolizin-7a(5H)-yl)methanol (500 mg, 3.263 mmol) in trahydrofuran (10.0 mL), and stirred at this temperature for 10 min. Then, a solution of t-butyl (5S,5aS,6S,9R)-2-(5-amino-3-chloro-2-(trifluoromethyl)phenyl)-1-fluoro-5-(methyl-d₃)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (1.55 g,2.348 mmol) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(5-amino-3-chloro-2-(trifluoromethyl)phenyl)-1-fluoro-5-(methyl-d₃)-12-(((R)-1- methylenetetrahydro-1H--pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-14-carboxylate (660 mg, yield: 37.52%) as a yellow solid. ES-API: [M+H]⁺=749.2.

Step IV: In an iced water bath, t-butyl (5S,5aS,6S,9R)-2-(5-amino-3-chloro-2-(trifluoromethyl)phenyl)-1-fluoro-5-(methyl-ds)-12-(((R)-1-methylenetetrahydro- 1H--pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-14-carboxylate (660.0 mg,0.882 mmol) was slowly added to dichloromethane (30.0 mL). Finally, trifluoroacetic acid (20.0 mL,261.0 mmol) was added. The reaction mixture was stirred at room temperature for 3.0 hrs. The reaction was shown to be completed by LC/MS. The reaction solution was concentrated to dryness under reduced pressure, and the residue was purified by preparative HPLC (ammonium bicarbonate method 2) to obtain 3-chloro-5-((5S,5aS,6S,9R)-1-fluoro-5-(methyl-d₃)-12-(((R)- 1-methylenetetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-2-yl)-4-(trifluoromethyl)aniline as a white solid (Z1060, 340.0 mg, yield: 59%). ES-API: [M+H]⁺= 649.2. ¹H NMR (400 MHz, CD₃OD) δ 6.87 (d, *J =* 2.0 Hz, 1H), 6.51-6.39 (m, 1H), 5.34-5.30 (m, 1H), 5.10-5.08 (m, 2H), 4.51-4.49 (m, 1H), 4.34 (s, 2H), 4.04-4.01(m, 1H), 3.67-3.65 (m, 1H), 3.56 (s, 1H), 3.23 - 3.07 (m, 3H), 2.87 - 2.77(m, 1H), 2.73-2.62 (m, 3H), 2.22-2.12 (m, 1H), 2.05 - 1.71 (m, 7H).

### Example 146: Synthesis of 4-((5S, 5aS, 6S, 9R)-12-(((S)-2-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z1044)

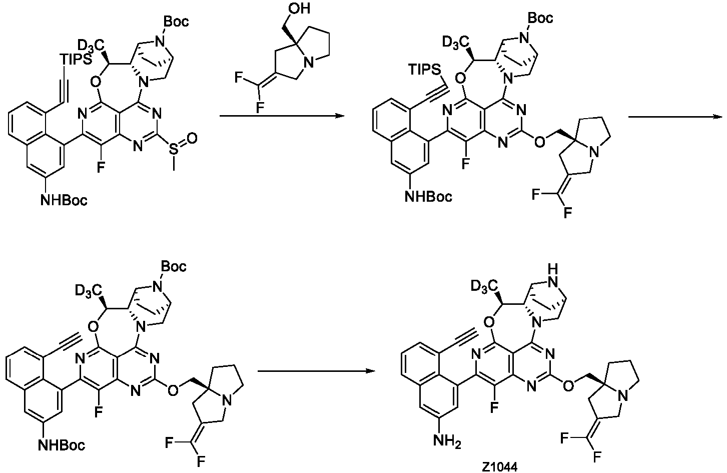

Step I: 60% sodium hydride (380 mg, 9.50 mmol) and tetrahydrofuran (20 mL) were added to a round-bottom flask. The reaction solution was cooled to 0°C, and (S)-(2-(difluoromethylene)tetrahydro-1H-pyrrolizin-7a(5H)-yl)methanol (562 mg, 2.97 mmol) dissolved in anhydrous tetrahydrofuran (3 mL) was added to the reaction solution, and reacted at room temperature for 10 min. The reaction solution was cooled to 0°C. A solution of t-butyl (5S, 5aS, 6S, 9R)-2-(3-((t-butoxycarbonyl)amino)-8-((triisopropylsilyl)ethynyl)naphthalen-1-yl)-1-fluoro-5-(methyl-d₃)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (2.4 g, crude product) in tetrahydrofuran (10 mL) was added to the reaction solution, and reacted at 0°C for 30 min. The reaction was shown to be completed by LC/MS. The reaction solution was added with a saturated ammonium chloride solution (30 mL), and extracted with ethyl acetate (30 mLX2). The organic phase was washed with saturated brine (30 mL), dried over anhydrous sodium sulfate, filtered, and concentrated. The crude product was purified by flash silica gel column chromatography (tetrahydrofuran/petroleum ether: 0-30%) to obtain t-butyl (5S,5aS,6S,9R)-2-(3-((t-butoxycarbonyl)amino)-8-((triisopropylsilyl) ethynyl)naphthalen-1-yl)-12-(((S)-2-difluoromethylene)tetrahydro-1H-pyrrolizin-7a(SH)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (1.2 g, yield: 43%). ES-API: [M+H]⁺=1013.3.

Step II: T-butyl (5S,5aS,6S,9R)-2-(3-((t-butoxycarbonyl)amino)-8-((triisopropylsilyl) ethynyl)naphthalen-1-yl)-12-(((S)-2-difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (1.2 g, 1.18 mmol), cesium fluoride (1.80 g, 11.84 mmol), and N, N-dimethylformamide (10 mL) were added to a round-bottom flask. The reaction was stirred at room temperature for 1 hr. The reaction solution was added with water (100 mL), and extracted with ethyl acetate (30 mLX2). The organic phase was washed with saturated brine (30 mLX2), dried over anhydrous sodium sulfate, filtered, and concentrated. The crude product was purified by flash silica gel column chromatography (tetrahydrofuran/petroleum ether: 0-30%) to obtain t-butyl (5S, 5aS, 6S, 9R)-2-(3-((t-butoxycarbonyl)amino)-8-ethynylnaphthalen-1-yl)-12-(((S)-2-difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho [1,8-ab]hepten-14-carboxylate (900 mg, yield: 88%). ES-API:[M+H]⁺=857.2.

Step III: T-butyl (5S, 5aS, 6S, 9R)-2-(3-((t-butoxycarbonyl)amino)-8-ethynylnaphthalen-1-yl)-12-(((S)-2-difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (900 mg, 1.05mmol) was dissolved in ethyl acetate (8 mL). The solution was cooled to 0°C, and a 4M hydrogen chloride/dioxane solution (10 mL, 40 mmol) was added. The reaction was stirred at room temperature for 1 hr. The reaction solution was concentrated to dryness at 40°C, and the crude product was purified by preparative HPLC (aqueous ammonia method) to obtain 4-((5S, 5aS, 6S, 9R)-12-(((S)-2-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-5-ethynylnaphthalen-2-amine (Z1044, 500 mg, yield: 72%). ES-API: [M+H]⁺=657.1. ¹H NMR (400MHz, CD₃OD): 7.68-7.65 (m,1H), 7.41-7.25 (m,2H), 7.16-7.00 (m,2H), 5.38-5.33 (m,1H), 4.50-4.44 (m,1H), 4.34-4.22 (m,2H), 4.06-4.05 (m,1H), 3.80-3.76 (m,1H), 3.67-3.66 (m,1H), 3.58-3.57 (m,1H), 3.43-3.40 (m,1H), 3.29-2.98 (m,3H), 2.81-2.65 (m,2H), 2.52-2.48 (m,1H), 2.14-1.75 (m,8H).

### Example 147: Synthesis of 3-chloro-5-((5S,5aS,6S,9R)-12-(((S)-2-(difluoromethylene) tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z1054)

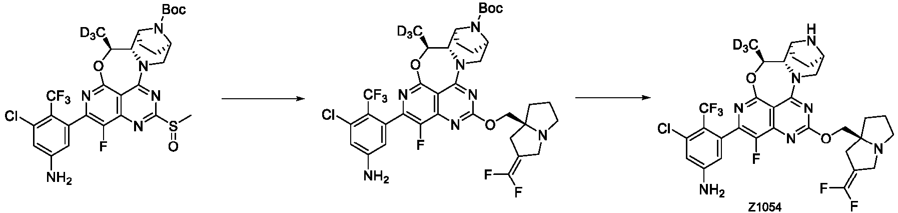

Step I: (S)-(2-(difluoromethylene)tetrahydro-1H-pyrrolizin-7a(5H)-yl)methanol (400 mg, 2.114 mmol) was dissolved in tetrahydrofuran(10 mL). Sodium hydride (153.60 mg, 6.400 mmol) was added, and reacted at room temperature for 15 min. Then, a solution of t-butyl (5S,5aS,6S,9R)-2-(5-amino-3-chloro-2-(trifluoromethyl)phenyl)-1-fluoro-5-(methyl-d₃)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (1056.16 mg, 1.6 mmol) in tetrahydrofuran(10 mL) was added, and reacted at room temperature for 2 hrs. After the reaction, the reaction was quenched with a saturated ammonium chloride solution (10 mL) and extracted with ethyl acetate (30 mL). The organic phase was washed with saturated brine (20 mL), dried over anhydrous sodium sulfate, filtered, and concentrated. The crude product was purified by silica gel column chromatography (0-40% tetrahydrofuran/petroleum ether) to obtain t-butyl (5S,5aS,6S,9R)-2-(5-amino-3-chloro-2-(trifluoromethyl)phenyl)-12-(((S)-2-(2-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (0.5 g, 0.637 mmol, yield: 39.80%) as a yellow solid. ES-API: [M+H]⁺=785.0.

Step II: In an iced water bath, trifluoroacetic acid (10.0 mL) was slowly added to t-butyl (5S,5aS,6S,9R)-2-(5-amino-3-chloro-2-(trifluoromethyl)phenyl)-12-(((S)-2-(2-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (0.5 g, 0.637 mmol) in dichloromethane(10.0 mL). The reaction mixture was stirred at room temperature for 1 hr. The reaction was shown to be completed by LC/MS. The reaction solution was concentrated to dryness under reduced pressure, and the residue was purified by preparative HPLC (ammonium bicarbonate method 2) to obtain 3-chloro-5-((5S,5aS,6S,9R)-12-(((S)-2-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-4-(trifluoromethyl)aniline (Z1054, 0.16 g, 0.234 mmol, yield: 36.68%). ES-API: [M+H]+= 685.0. ¹H NMR (400 MHz, CD₃OD) δ 6.88 (d, *J =* 1.6 Hz, 1H), 6.54-6.40 (m, 1H), 5.33 (dd, *J =* 13.2, 2.4 Hz, 1H), 4.51-4.50 (m, 1H), 4.34-4.26 (m, 1H), 4.05-4.03(m, 1H), 3.80-3.56(m, 3H), 3.47-3.41 (m, 1H), 3.19-3.08 (m, 2H), 2.87-2.62 (m, 2H), 2.51-2.48 (m, 1H), 2.20-1.70 (m, 10H).

### Example 148: Synthesis of 6-((5S,5aS,6S,9R)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-4-methyl-5-(trifluoromethyl)pyridine-2-amine (Z1295)

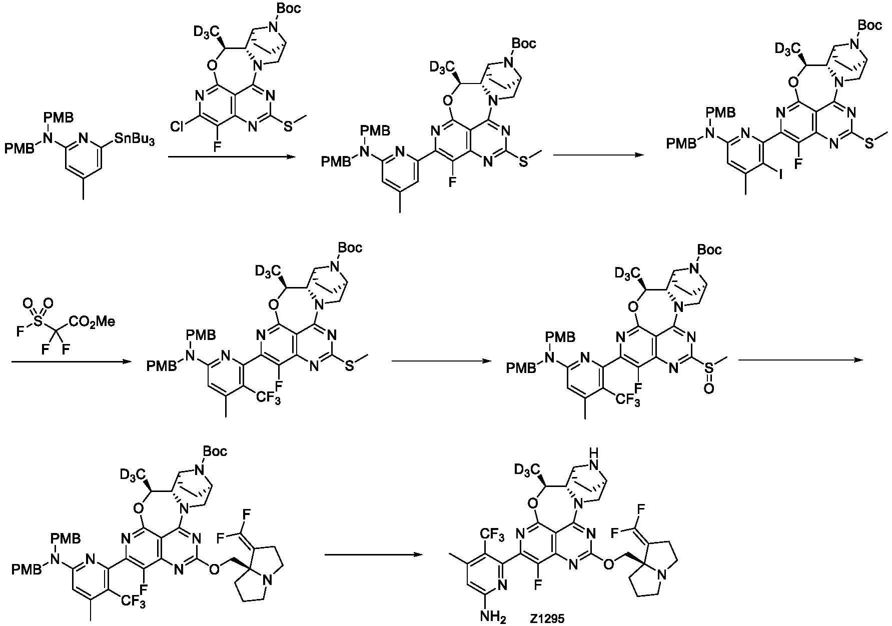

Step I: T-butyl (5S,5aS,6S,9R)-2-chloro-1-fluoro-5-(methyl-d₃)-12-(methylthio)-5a,6,7,8,9,10-hexahydro-5H-4-oxa-3,10a,11,13,14-pentaaza-6,9-methanonaphtho[1,8-ab]hepten-14-carboxylate (900.0 mg,1.86 mmol), N,N-bis(4-methoxybenzyl)-4-methyl-6-(tributyltin) pyridine-2-amine (2.40g, 3.76mmol), cuprous iodide (366.0 mg, 1.86 mmol), lithium chloride (316.0 mg, 7.48mmol) and N,N-dimethylacetamide (30.0 mL) were added to a 100 mL round-bottom flask, purged 3 times with nitrogen, and reacted at 120°C for 3 hrs. The cooled reaction solution was added with ethyl acetate (60 mL), and sequentially washed with dilute brine (30 mL×3), and saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-10%) to obtain the target t-butyl product (5S,5aS,6S,9R)-2-(6-(bis(4-methoxybenzyl)amino)-4-methylpyridine-2-yl)-1-fluoro-5-(methyl-d₃)-12-(methylthio)-5a,6,7,8,9,10-hexahydro-5H-4-oxa-3,10a,11,13,14-pentaaza-6,9-methanonaphtho[1,8-ab]hepten-14-carboxylate (936.0 mg, yield: 63.29%) as an off-white solid. ES-API: [M+H]⁺=797.2.

Step II: T-butyl (5S, 5aS, 6S, 9R)-2-(6-(bis(4-methoxybenzyl)amino)-4-methylpyridine-2-yl)-1-fluoro-5-(methyl-d3)-12-(methylthio)-5a, 6,7,8,9,10-hexahydro-5H-4-oxa-3,10a, 11,13,14-pentaaza-6,9-methanonaphtho[1,8-ab]hepten-14-carboxylate (936.0 mg,1.176mol) and p-toluenesulfonic acid monohydrate (11 mg,0.057 mmol) were dissolved in N,N-dimethylformamide (20.0 mL). At room temperature, N-iodosuccinimide (529.0 mg, 2.351 mmol) was added and the reaction was stirred at room temperature for 2 hrs. The reaction solution was added with ethyl acetate (60 mL), and sequentially washed with saturated sodium thiosulphate (40 mL) and saturated brine (30 mL). The organic phase was dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5S, 5aS, 6S, 9R)-2-(6-(bis(4-methoxybenzyl)amino)-3-iodo-4-methylpyridine-2-yl)-1-fluoro-5-(methyl-d₃)-12-(methylthio)-5a, 6,7,8,9,10-hexahydro-5H-4-oxa-3,10a, 11,13,14-pentaaza-6,9-methanonaphtho[1,8-ab]hepten-14-carboxylate (635.0 mg, yield: 58.72%) as a yellow solid. ES-API: [M+H]⁺=923.0.

Step III: Cuprous iodide (917.30 mg, 4.816 mmol) was added to a solution of t-butyl (5S, 5aS, 6S,9R)-2-(6-(bis(4-methoxybenzyl)amino)-3-iodo-4-methylpyridine-2-yl)-1-fluoro-5-(methyl-d3)-12-(methylthio)-5a,6,7,8,9,10-hexahydro-5H-4-oxa-3,10a,11,13,14-pentaaza-6,9-methanonaphtho[1,8-ab]hepten-14-carboxylate (635.0 mg, 0.688 mmol) and methyl 2,2-difluoro-2-(fluorosulfonyl)acetate (925.30 mg, 4.816mol) in dry N,N-dimethylformamide (10 mL), purged 3 times with nitrogen, and reacted at 90°C for 2 hrs. The reaction solution was added with ethyl acetate (100 mL), and washed with saturated brine (60 mLX3). The organic phase was dried over anhydrous sodium sulfate, and filtered. The filtrate was concentrated under reduced pressure. The crude product was purified by flash silica gel column chromatography (ethyl acetate /petroleum ether: 0-40%) to obtain the target product t-butyl (5S,5aS,6S,9R)-2-(6-(bis(4-methoxybenzyl)amino)-4-methyl-3-(trifluoromethyl)pyridine-2-yl)-1-fluoro-5-(methyl-d₃)-12-(methylthio)-5a,6,7,8,9,10-hexahydro-5H-4-oxo-3,10a,11,13,14-pentaaza-6,9-methanonaphtho[1,8-ab]hepten-14-carboxylate (360 mg, yield: 60.7%) as a light yellow solid. ES-API: [M+H]⁺=865.2.

Step IV: T-butyl (5S,5aS,6S,9R)-2-(6-(bis(4-methoxybenzyl)amino)-4-methyl-3-(trifluoromethyl)pyridine-2-yl)-1-fluoro-5-(methyl-d₃)-12-(methylthio)-5a,6,7,8,9,10-hexahydro-5H-4-oxo-3,10a,11,13,14-pentaaza-6,9-methanonaphtho[1,8-ab]hepten-14-carboxylate (560.0 mg, 0.647 mmol) was dissolved in dichloromethane (10 mL).m-chloroperoxybenzoic acid (168.0 mg, 0.971 mmol) was added at room temperature, and the reaction was stirred at room temperature for 1 hr. The reaction solution was added with dichloromethane (100 mL), and sequentially washed with saturated sodium thiosulphate (40 mL), saturated sodium bicarbonate (50mL), and saturated brine (80 mL). The organic phase was dried over anhydrous sodium sulfate, and filtered. The filtrate was concentrated under reduced pressure, to obtain the target product t-butyl (5S,5aS,6S,9R)-2-(6-(bis(4-methoxybenzyl)amino)-4-methyl-3-(trifluoromethyl)pyridine-2-yl)-1-fluoro-5-(methyl-d₃)- 12-(methylsulfinyl)-5a, 6,7,8,9,10-hexahydro-5H-4-oxa-3,10a, 11,13,14-pentaaza-6,9-methanonaphtho[1,8-ab]hepten-14-carboxylate (400.0 mg, yield: 70.13%) as a white solid. ES-API:[M+H]⁺=881.3.

Step V: (R)-(1-(difluoromethylene)tetrahydro-1H-pyrrolizin-7a(5H)-yl)methanol (128.0 mg, 0.681 mmol) was dissolved in tetrahydrofuran (10mL). At 0°C, sodium hydride (54.5 mg, 1.362 mol) was added, and the reaction was stirred at this temperature for 10 min. Then, t-butyl (5S,5aS,6S,9R)-2-(6-(bis(4-methoxybenzyl)amino)-4-methyl-3-(trifluoromethyl)pyridine-2-yl)-1-fluoro-5-(methyl-d₃)-12-(methylsulfinyl)-5a,6,7,8,9,10-hexahydro-5H-4-oxa-3,10a,11,13,14-pentaaza-6,9-methanonaphtho[1,8-ab]hepten-14-carboxylate (400.0 mg, 0.454 mmol) was added, and reacted at this temperature for 10 min. After the reaction, the reaction solution was added with ethyl acetate (60 mL), and sequentially washed with water (20 mL) and saturated brine (30 mL). The organic phase was dried over anhydrous sodium sulfate, and filtered. The filtrate was concentrated under reduced pressure. The crude product was purified by preparative TLC (dichloromethane/7.0M aminomethanol = 25: 1) to obtain the crude target product t-butyl (5S,5aS,6S,9R)-2-(6-(bis(4-methoxybenzyl)amino)-4-methyl-3-(trifluoromethyl)pyridine-2-yl)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (390 mg, crude product) as a light yellow solid. ES-API: [M+H]⁺=1006.3.

Step VI: T-butyl (5S,5aS,6S,9R)-2-(6-(bis(4-methoxybenzyl)amino)-4-methyl-3-(trifluoromethyl)pyridine-2-yl)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (390 mg, crude product) was dissolved in trifluoroacetic acid (10 mL), and the reaction was stirred at 50°C for 2 hrs. The reaction solution was concentrated under reduced pressure to dryness. Dichloromethane (20 mL) and 7.0 M aminomethanol (20 mL) were added, and concentrated under reduced pressure. The crude product was purified by preparative HPLC (ammonium bicarbonate method 2) to obtain the target product 6-((5S,5aS,6S,9R)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-4-methyl-5-(trifluoromethyl)pyridine-2-amine as a white solid (Z1295, 33.0 mg, total yield of two steps: 10.9%). ES-API: [M+H]⁺= 666.2.

### Example 149: Synthesis of 6-((5S,5aS,6S,9R)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-2-yl)-4-methyl-5-(trifluoromethyl)pyridine-2-amine (Z1072)

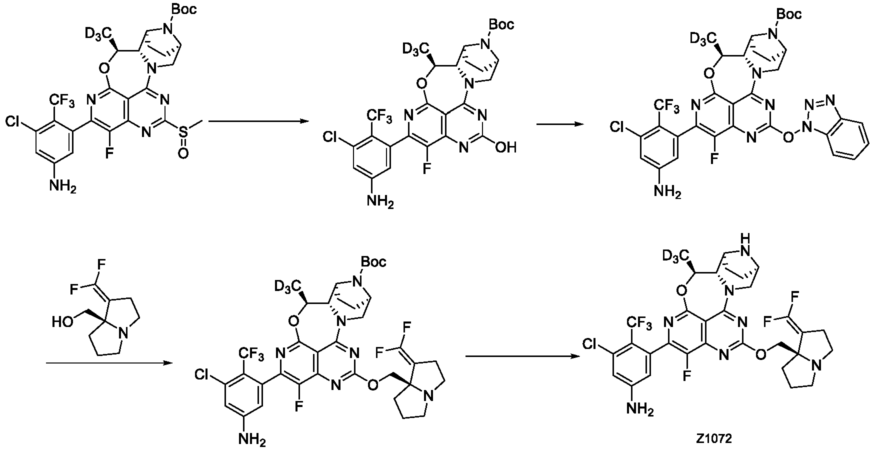

Step I: T-butyl (5S,5aS,6S,9R)-2-(5-amino-3-chloro-2-(trifluoromethyl)phenyl)-1-fluoro-5-(methyl-d3)-12-(methylsulfinyl)-5a,6,7,8,9,10-hexahydro-5H-4-oxa-3,10a,11,13,14-pentaaza-6,9-nonaphtho[1,8-ab]hepten-14-carboxylate (400 mg, 0.607 mmol) was added to a mixture of potassium hydroxide (142.40 mg, 2.538 mmol) in water (3 mL) and tetrahydrofuran(8 mL), and heated at 60°C for 1 hr. The reaction was quenched with water and extracted with ethyl acetate. The organic layer was washed with brine, dried over anhydrous sodium sulfate, filtered and concentrated, to obtain t-butyl (5S,5aS,6S,9R)-2-(5-amino-3-chloro-2-(trifluoromethyl)phenyl)-1-fluoro-12-hydroxyl-5-(methyl-d3)-5a,6,7,8,9,10-hexahydro-5H-4-oxa-3,10a,11,13,14-pentaaza-6,9-methanonaphtho[1,8-ab]hepten-14-carboxylate (376 mg, crude product) as a yellow solid. ES-API:[M+H]⁺=614.2.

Step II: T-butyl (5S,5aS,6S,9R)-2-(5-amino-3-chloro-2-(trifluoromethyl)phenyl)-1-fluoro-12-hydroxyl-5-(methyl-d3)-5a,6,7,8,9,10-hexahydro-5H-4-oxa-3,10a,11,13,14-pentaaza-6,9-methanonaphtho[1,8-ab]hepten-14-carboxylate (540.0 mg, 0.879 mmol) and cesium carbonate (573.09 mg, 1.759 mmol) were added to tetrahydrofuran (20.0 mL), and (benzo[d][1,2,3]triazole-1-yloxy)tris(dimethylamino)phosphonium hexafluoro-λ5-phosphate (777.96 mg, 1.759 mmol) was added to the system. The reaction mixture was stirred at room temperature for 1 hr. The reaction was quenched with water and extracted with ethyl acetate. The organic phase was washed with brine, dried over anhydrous sodium sulfate, filtered and concentrated. The residue was purified by flash silica gel column chromatography (0-50% ethyl acetate in petroleum ether) to obtain t-butyl (5S,5aS,6S,9R)-12-((1H-benzo[d][1,2,3]triazole-1-yl)oxy)-2-(5-amino-3-chloro- 2-(trifluoromethyl)phenyl)-1-fluoro-5-(methyl-d3)-5a,6,7,8,9,10-hexahydro-5H-4-oxa-3,10a,11,13,14-pentaaza-6,9-methanonaphtho[1,8-ab]hepten-14-carboxylate (640 mg, yield: 98.7%) as a yellow solid. ES-API:[M+H]⁺=731.2.

Step III: At 0°C, sodium hydride (130.0 mg, 3.267 mmol) was added to a solution of (R)-(1-(difluoromethylene)tetrahydro-1H-pyrrolizin-7a(5H)-yl)methanol (137.0 mg, 0.725 mmol) in tetrahydrofuran (10.0 mL), and stirred at this temperature for 10 min. Then, a solution of t-butyl (5S,5aS,6S,9R)-12-((1H-benzo[d][1,2,3]triazole-1-yl)oxy)-2-(5-amino-3-chloro-2-(trifluoromethyl)phenyl)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (530 mg, 0.725 mmol) in tetrahydrofuran (5 mL) was added. The reaction was continued with stirring at 0°C for 10 min, and at room temperature for 0.5 hr. After the reaction, the reaction solution was quenched with saturated ammonium chloride solution (20 mL), and extracted with ethyl acetate. The organic phase was washed with saturated brine, dried over anhydrous sodium sulfate, filtered, and concentrated. The crude product was purified by silica gel column chromatography (0-5% methanol /dichloromethane) to obtain t-butyl (5S,5aS,6S,9R)-2-(5-amino-3-chloro-2-(trifluoromethyl)phenyl)-12-(((R)-1-(difluoromethylene)tetrahydro- 1H-pyrrolizin-7a(5H)-yl)methoxy)-1-fluoro-5-(methyl-d₃)-5a,6,7,8,9,10-hexahydro-5H-4-oxa-3,10a,11,13,14-pentaaza-6,9-methanonaphtho[1,8-ab]hepten-14-carboxylate (540 mg, yield: 94.8%) as a yellow solid. ES-API: [M+H]⁺=758.1.

Step IV: In an iced water bath, t-butyl (5S,5aS,6S,9R)-2-(5-amino-3-chloro-2-(trifluoromethyl)phenyl)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxo-3,10a,11,13,14-pentaaza-6,9-methanonaphtho[1,8-ab]hepten-14-carboxylate (540.0 mg, 0.688 mmol) was slowly added to dichloromethane (30.0 mL). Finally, trifluoroacetic acid (20.0 mL, 261.0 mmol) was added. The reaction mixture was stirred at room temperature for 3.0 hrs. The reaction was shown to be completed by LC/MS. The reaction solution was concentrated to dryness under reduced pressure, and the residue was purified by preparative HPLC (ammonium bicarbonate method 2) to obtain 3-chloro-5-((5S,5aS,6S,9R)-12-(((R)-1-(difluoromethylene)tetrahydro-1H-pyrrolizin-7a(5H)-yl)methoxy)-1-fluoro-5-(methyl-d₃)-5a,6,7,8,9,10-hexahydro-5H-4-oxo-3,10a,11,13,14-pentaaza-6,9-methanonaphtho[1,8-ab]hepten-2-yl)-4-(trifluoromethyl)aniline (Z1072, 289.0 mg, yield: 61%) as a white solid. ES-API: [M+H]⁺= 685.2. ¹H NMR (400 MHz, CD₃OD) δ 6.87 (d, *J* = 4.0 Hz, 1H), 6.54-6.41(m, 1H), 5.34-5.30 (m, 1H), 4.50-4.47(m, 2H), 4.40-4.37(m, 1H), 4.06-4.02 (m, 1H), 3.68-3.66 (m, 1H), 3.60-3.55 (m, 1H), 3.15-3.06(m, 2H), 2.92-2.90 (m, 1H), 2.77-2.73 (m, 2H), 2.69-2.59 (m, 1H), 2.20-1.71 (m, 9H).

The compounds shown in Table A were respectively prepared by changing some raw materials according to the preparative method in the examples above.

**Table A**

| Structure and No. | MS [M+H]⁺ | Structure and No. | MS [M+H]⁺ |
|---|---|---|---|
| 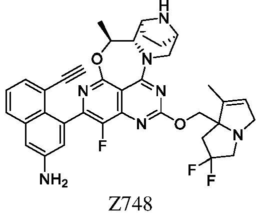 | 654.3 | 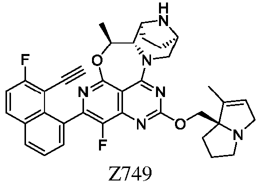 | 621.3 |
| 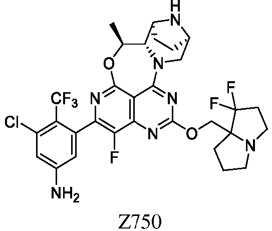 | 670.2 | 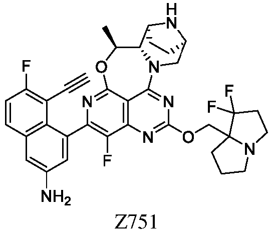 | 660.3 |
| 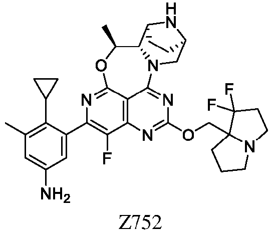 | 622.3 | 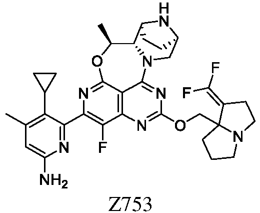 | 635.3 |
| 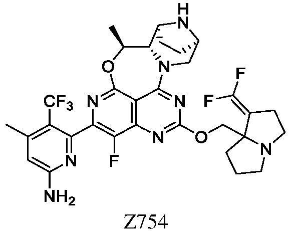 | 663.3 | 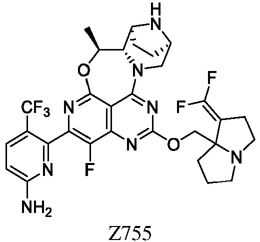 | 649.2 |
| 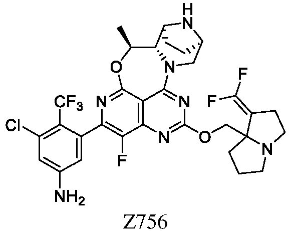 | 682.2 | 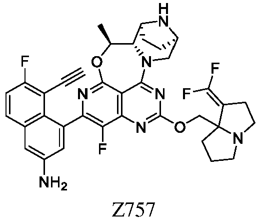 | 672.3 |
| 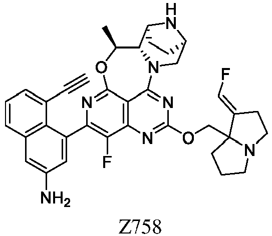 | 636.3 | 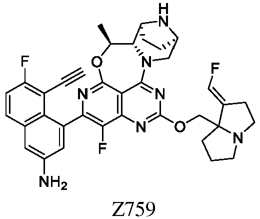 | 654.3 |
| 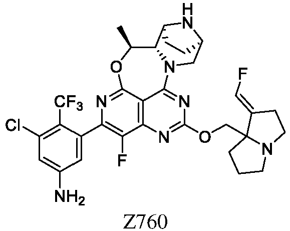 | 664.2 | 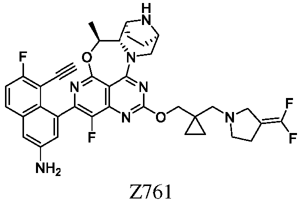 | 686.3 |
| 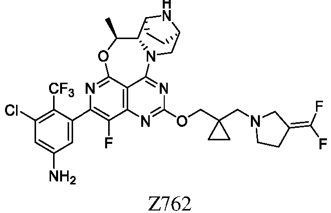 | 696.2 | 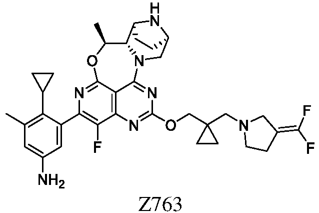 | 648.3 |
| 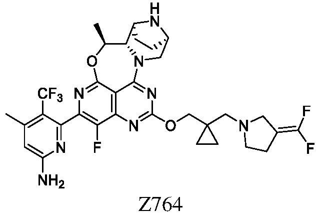 | 677.3 | 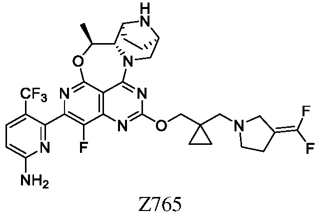 | 663.3 |
| 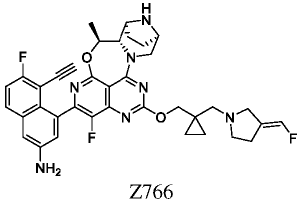 | 668.3 | 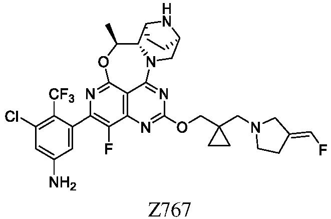 | 678.2 |
| 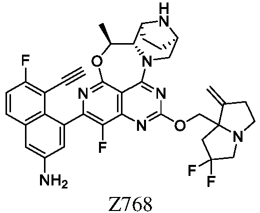 | 672.3 | 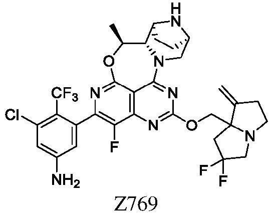 | 682.2 |
| 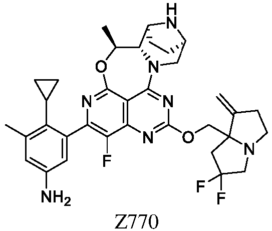 | 634.3 | 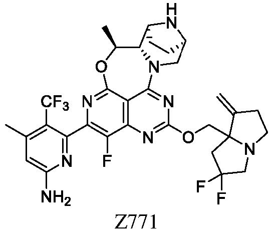 | 663.3 |
| 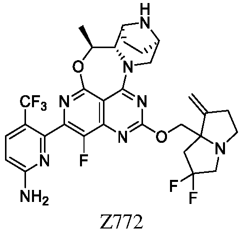 | 649.2 | 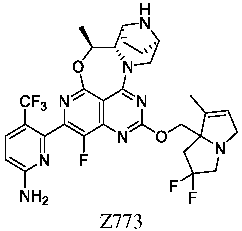 | 649.2 |
| 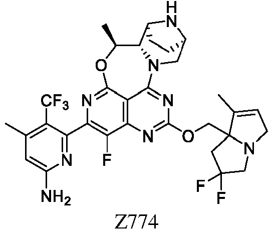 | 663.3 | 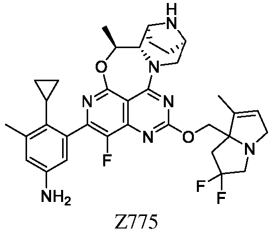 | 634.3 |
| 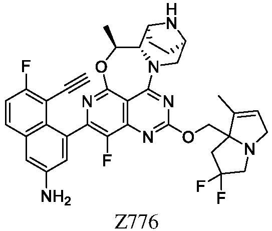 | 672.3 | 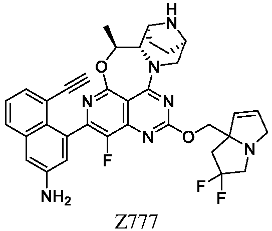 | 640.3 |
| 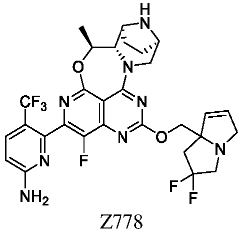 | 635.2 | 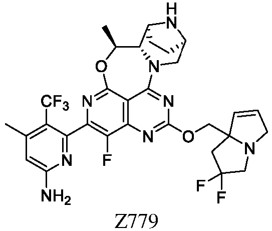 | 649.2 |
| 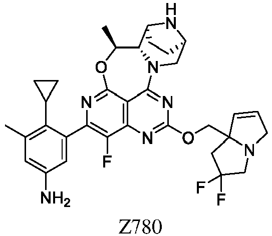 | 620.3 | 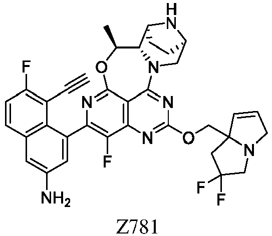 | 658.2 |
| 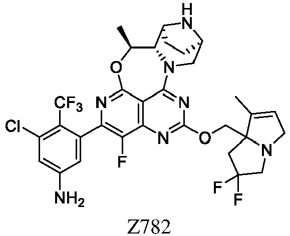 | 682.2 | 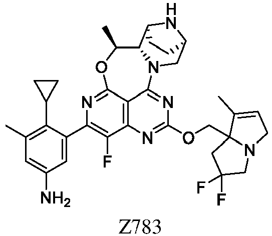 | 634.3 |
| 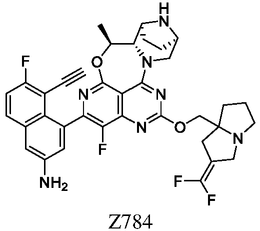 | 672.3 | 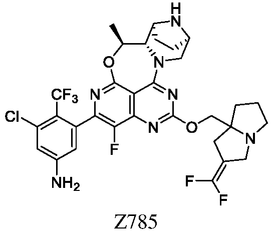 | 682.2 |
| 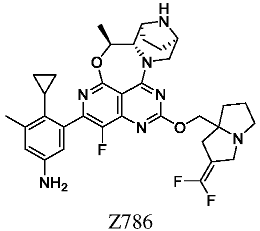 | 634.3 | 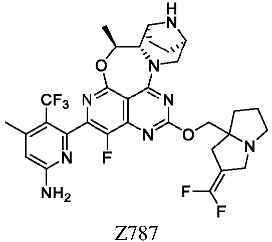 | 663.3 |
| 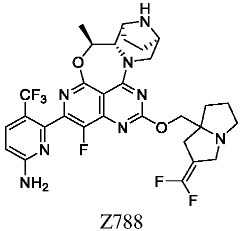 | 649.2 | 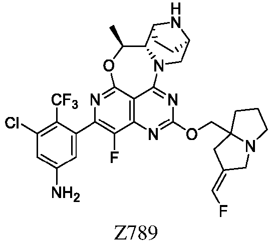 | 664.2 |
| 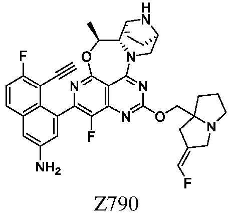 | 654.3 | 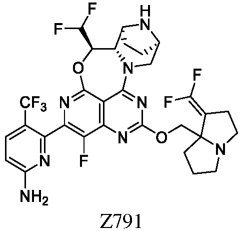 | 685.2 |
| 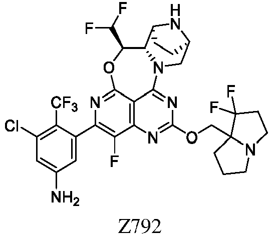 | 706.2 | 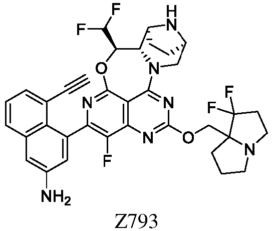 | 678.3 |
| 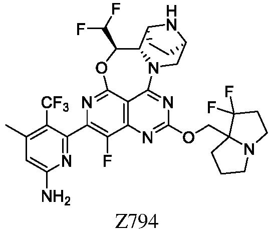 | 687.2 | 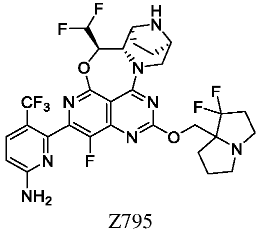 | 673.2 |
| 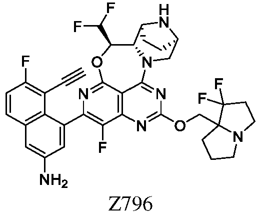 | 696.2 | 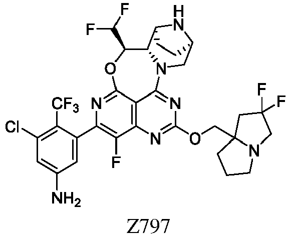 | 706.2 |
| 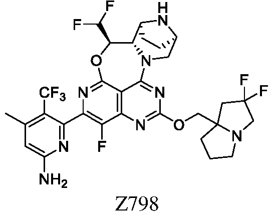 | 687.2 | 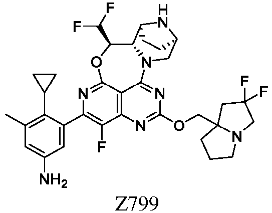 | 658.3 |
| 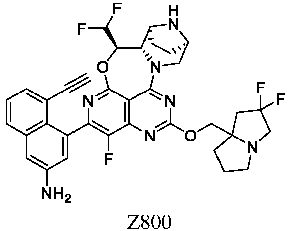 | 678.3 | 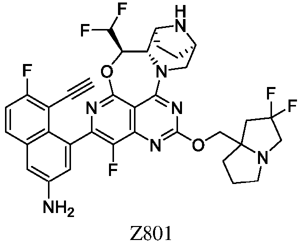 | 696.2 |
| 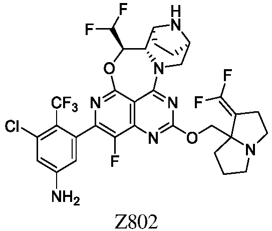 | 718.2 | 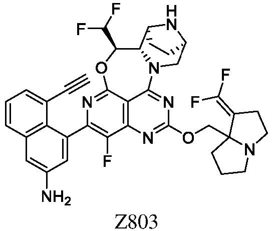 | 690.3 |
| 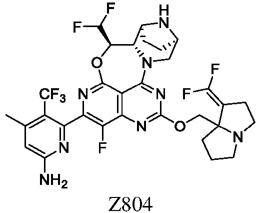 | 699.2 | 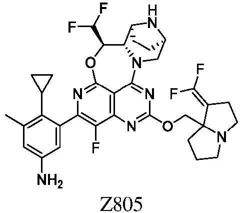 | 670.3 |
| 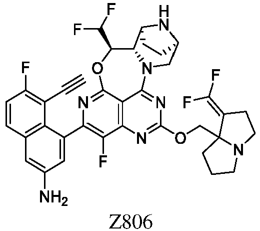 | 708.2 | 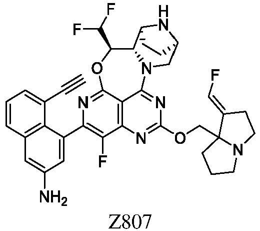 | 672.3 |
| 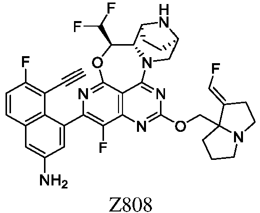 | 690.3 | 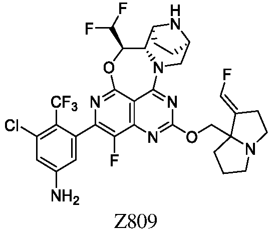 | 700.2 |
| 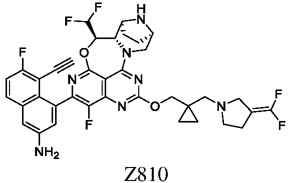 | 721.3 | 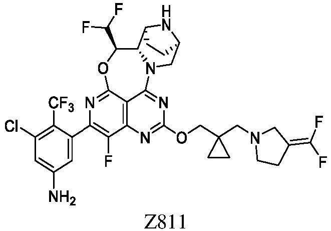 | 732.2 |
| 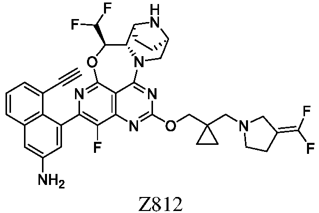 | 704.3 | 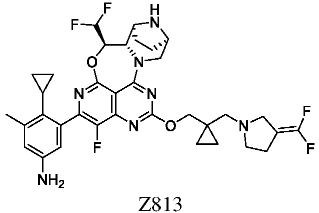 | 684.3 |
| 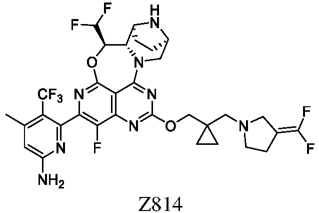 | 713.3 | 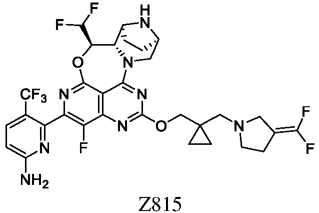 | 699.2 |
| 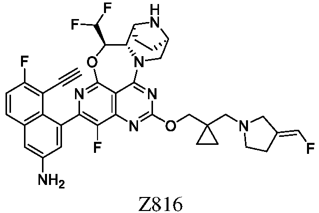 | 704.3 | 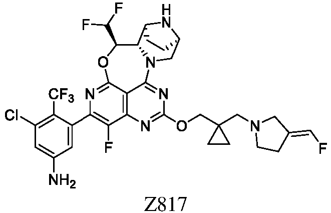 | 714.2 |
| 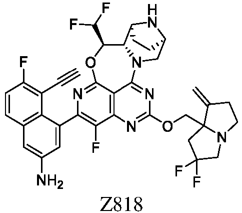 | 708.2 | 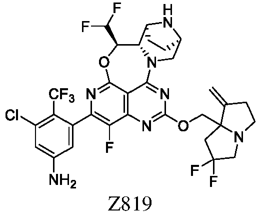 | 718.2 |
| 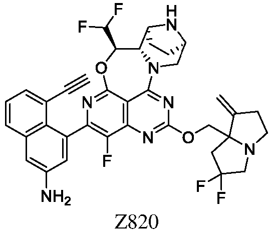 | 690.3 | 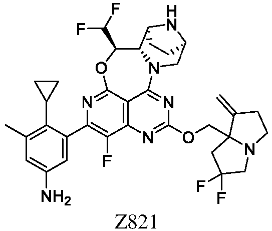 | 670.3 |
| 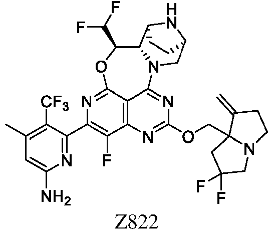 | 699.2 | 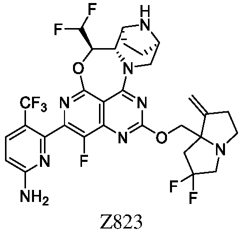 | 685.2 |
| 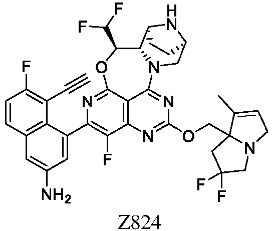 | 708.2 | 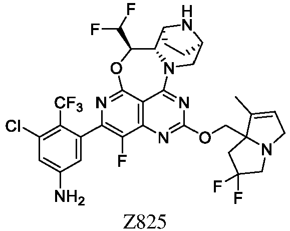 | 718.2 |
| 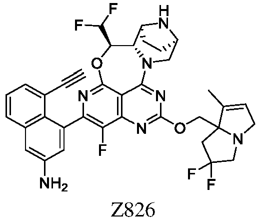 | 690.3 | 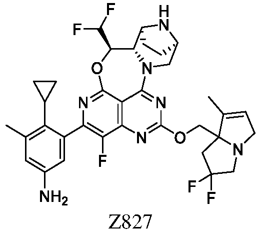 | 670.3 |
| 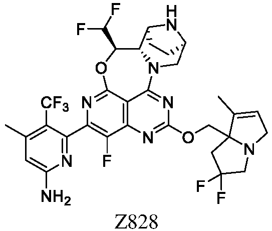 | 699.2 | 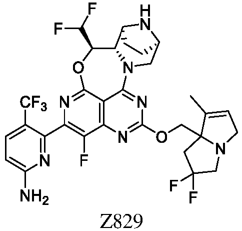 | 685.2 |
| 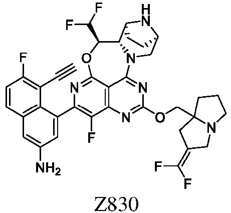 | 708.2 | 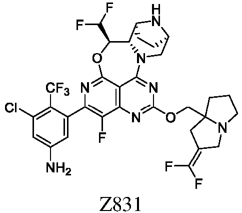 | 718.2 |
| 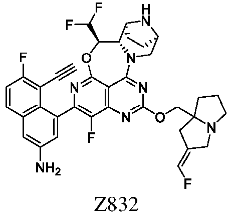 | 690.3 | 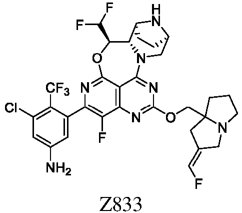 | 700.2 |
| 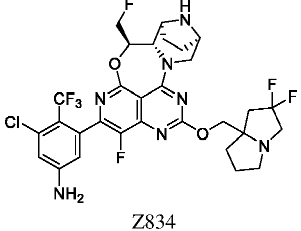 | 688.2 | 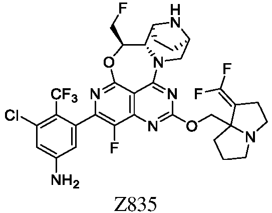 | 700.2 |
| 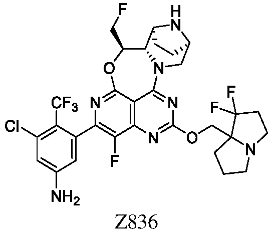 | 688.2 | 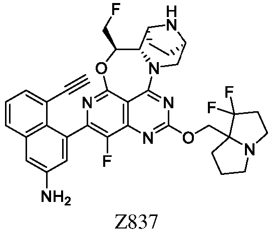 | 660.3 |
| 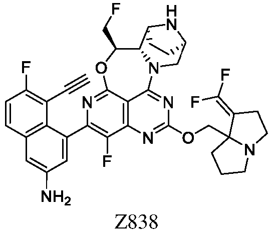 | 690.3 | 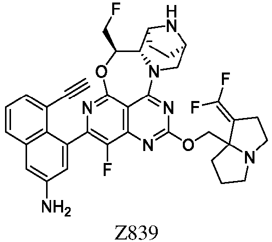 | 672.3 |
| 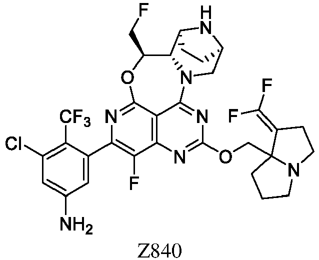 | 700.2 | 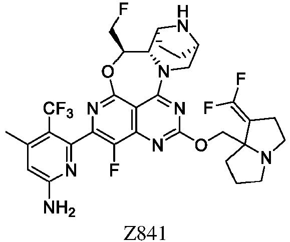 | 681.2 |
| 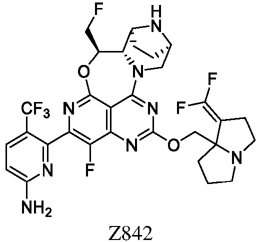 | 667.2 | 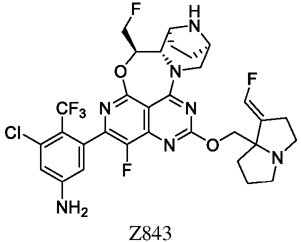 | 682.2 |
| 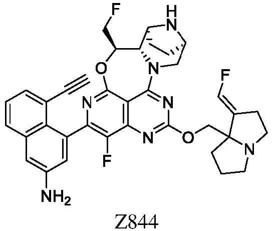 | 654.3 | 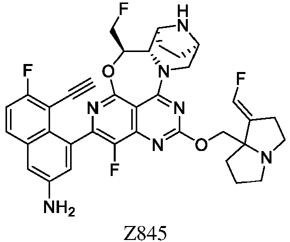 | 672.3 |
| 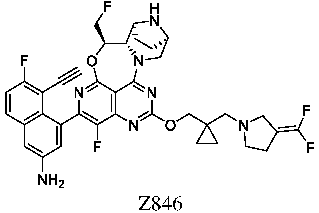 | 704.3 | 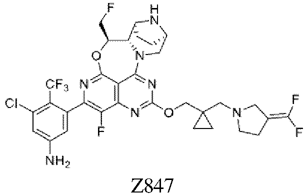 | 714.2 |
| 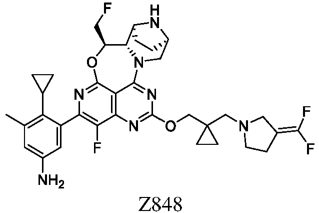 | 666.3 | 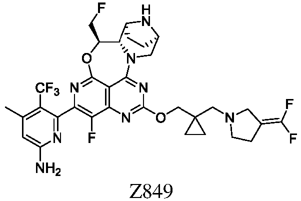 | 695.3 |
| 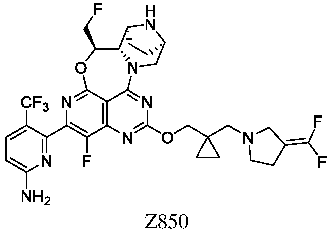 | 681.2 | 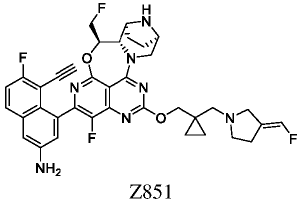 | 686.3 |
| 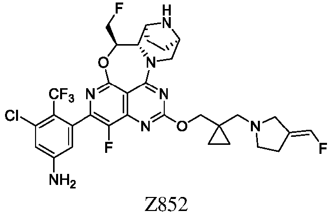 | 696.2 | 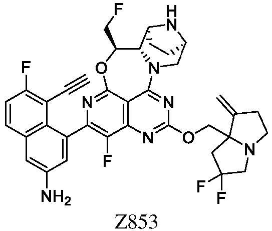 | 690.3 |
| 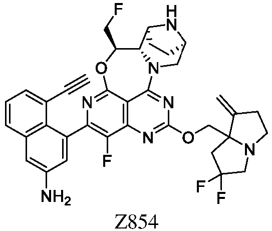 | 672.3 | 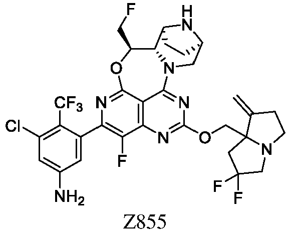 | 700.2 |
| 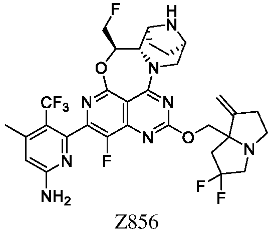 | 681.2 | 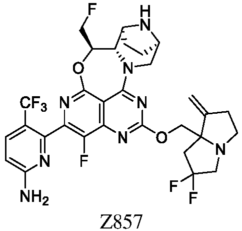 | 667.2 |
| 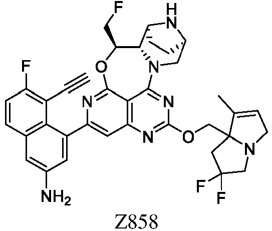 | 672.3 | 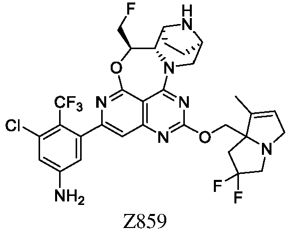 | 682.2 |
| 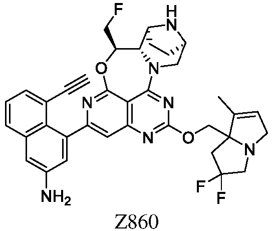 | 654.3 | 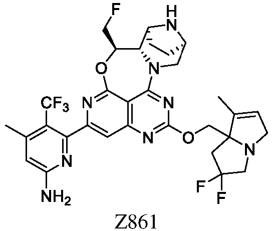 | 663.3 |
| 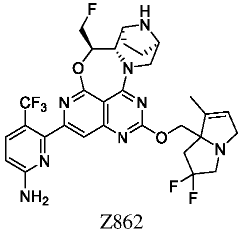 | 649.2 | 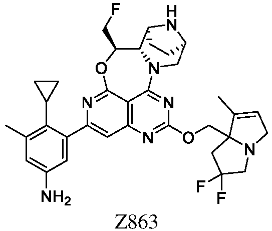 | 634.3 |
| 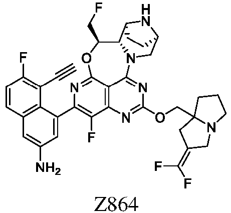 | 690.3 | 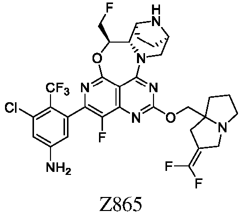 | 700.2 |
| 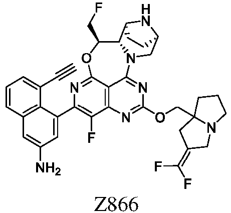 | 672.3 | 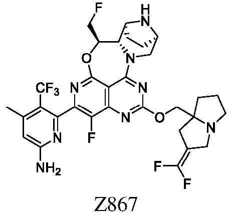 | 681.2 |
| 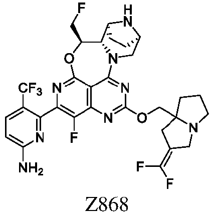 | 667.2 | 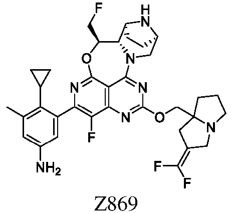 | 652.3 |
| 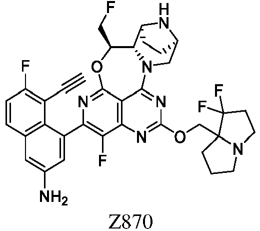 | 678.3 | 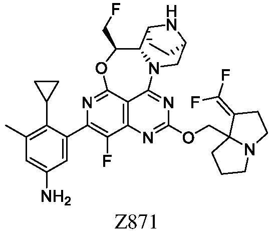 | 652.3 |
| 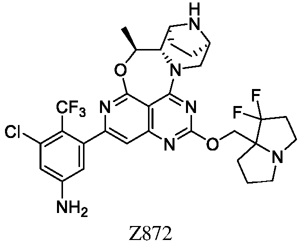 | 652.2 | 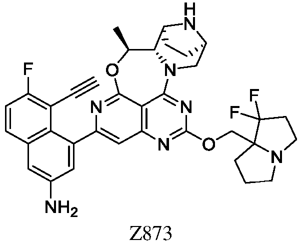 | 642.3 |
| 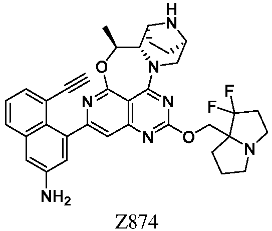 | 624.3 | 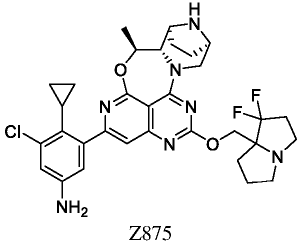 | 624.3 |
| 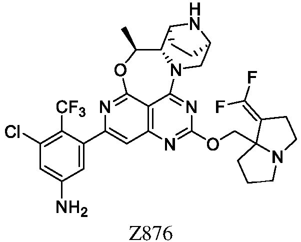 | 664.2 | 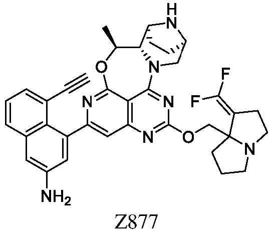 | 636.3 |
| 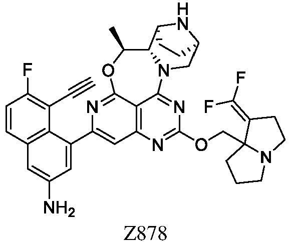 | 654.3 | 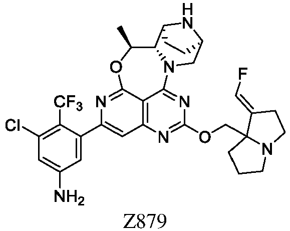 | 646.2 |
| 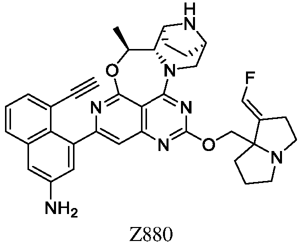 | 618.3 | 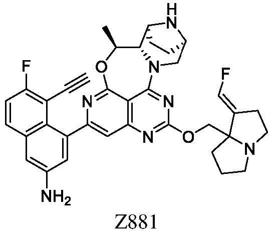 | 636.3 |
| 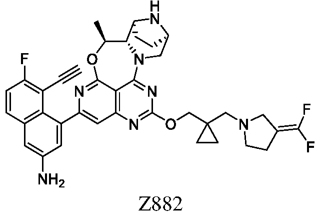 | 668.3 | 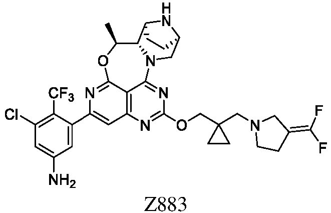 | 678.2 |
| 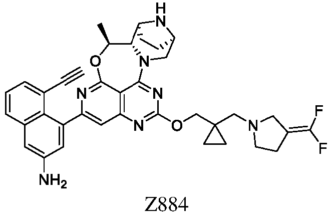 | 650.3 | 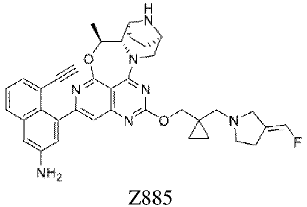 | 632.3 |
| 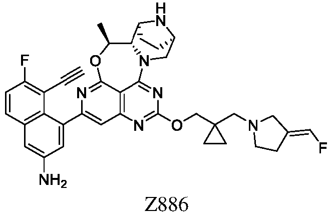 | 650.3 | 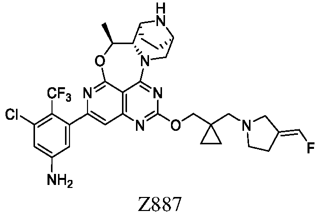 | 660.2 |
| 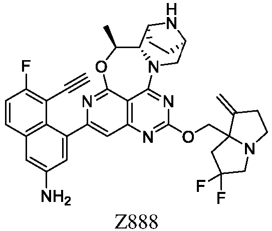 | 672.3 | 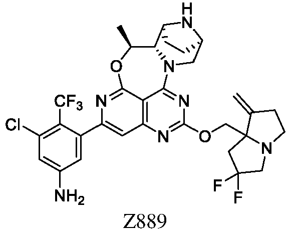 | 682.2 |
| 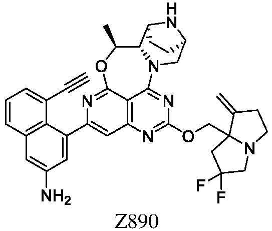 | 636.3 | 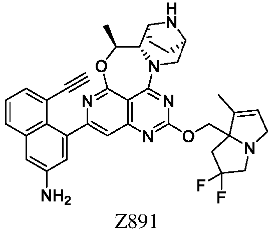 | 636.3 |
| 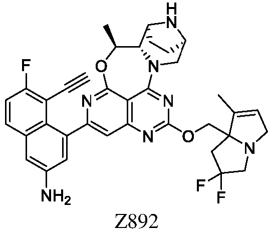 | 654.3 | 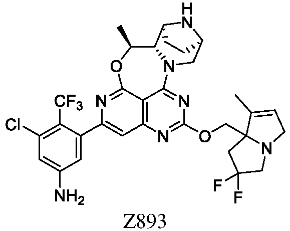 | 664.2 |
| 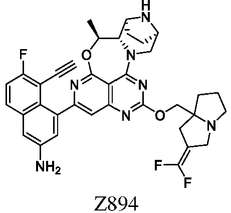 | 654.3 | 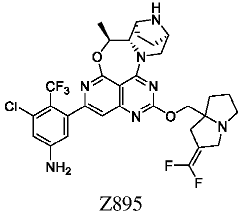 | 664.2 |
| 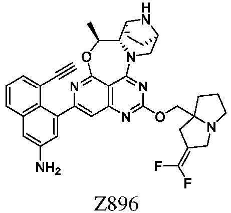 | 636.3 | 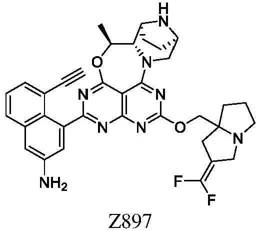 | 637.3 |
| 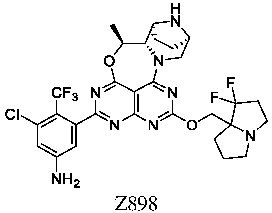 | 653.2 | 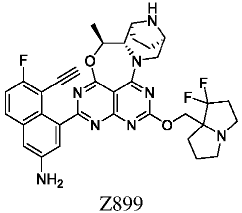 | 643.3 |
| 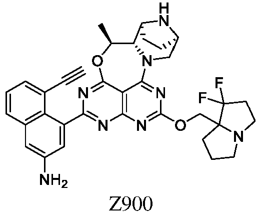 | 625.3 | 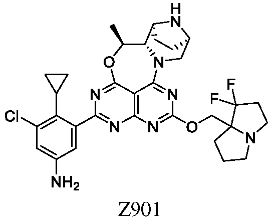 | 625.3 |
| 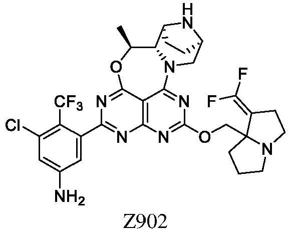 | 665.2 | 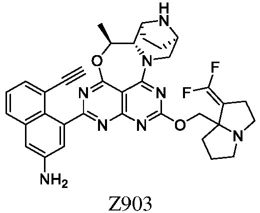 | 637.3 |
| 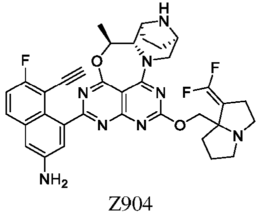 | 655.3 | 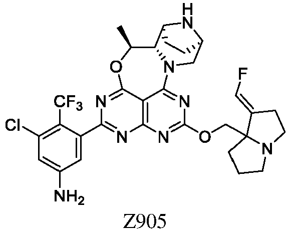 | 647.2 |
| 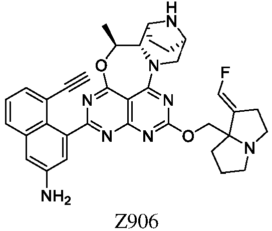 | 619.3 | 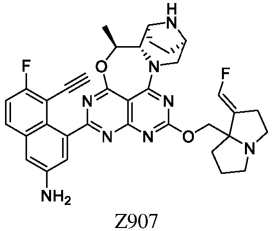 | 637.3 |
| 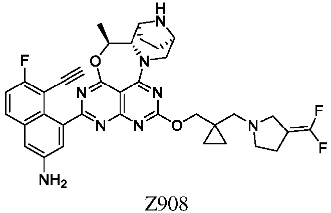 | 669.3 | 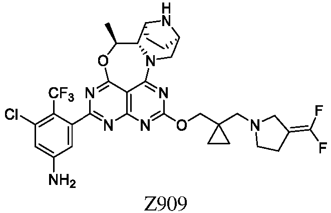 | 679.2 |
| 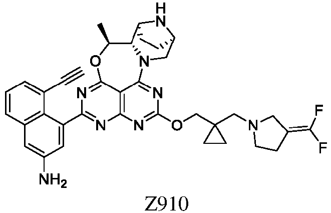 | 651.3 | 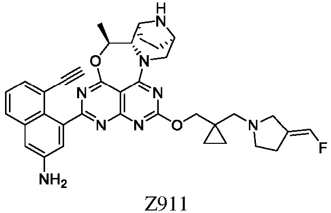 | 633.3 |
| 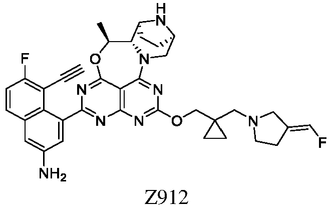 | 651.3 | 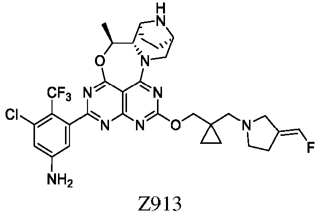 | 661.2 |
| 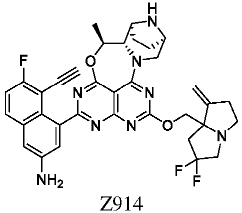 | 655.3 | 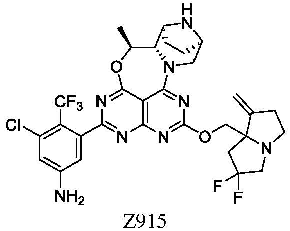 | 665.2 |
| 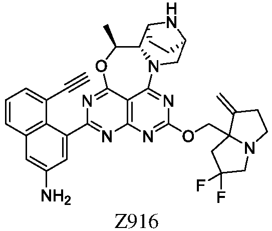 | 637.3 | 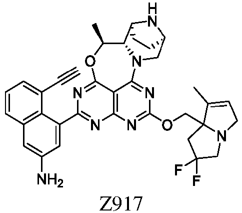 | 637.3 |
| 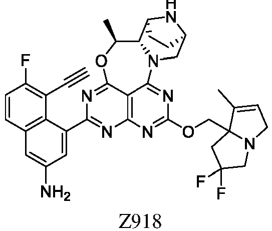 | 655.3 | 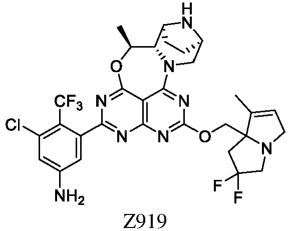 | 665.2 |
| 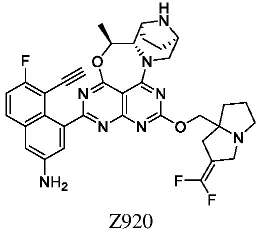 | 655.3 | 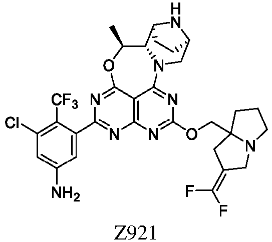 | 665.2 |
| 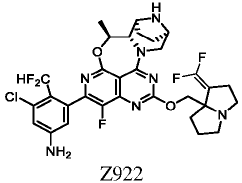 | 664.2 | 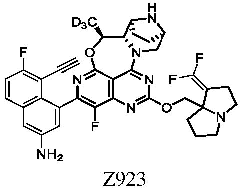 | 675.3 |
| 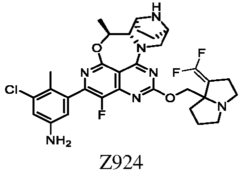 | 628.2 | 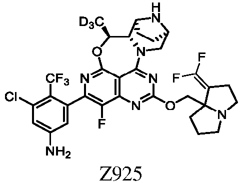 | 685.2 |
| 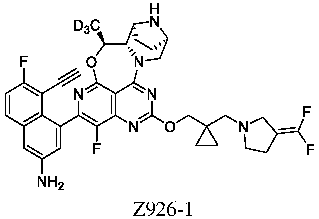 | 689.3 | 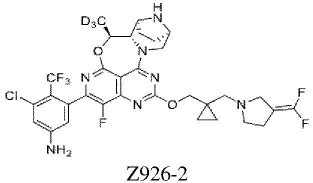 | 699.2 |
| 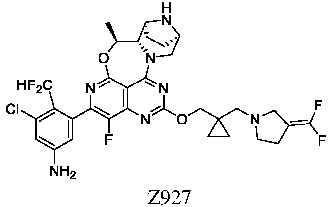 | 678.2 | 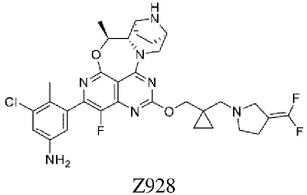 | 642.2 |
| 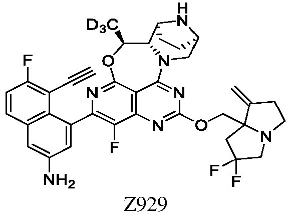 | 675.3 | 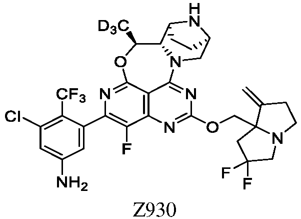 | 685.2 |
| 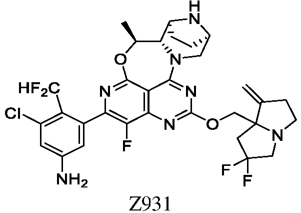 | 664.2 | 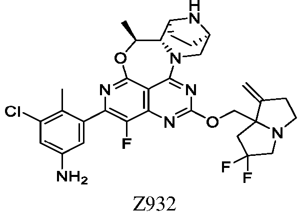 | 628.2 |
| 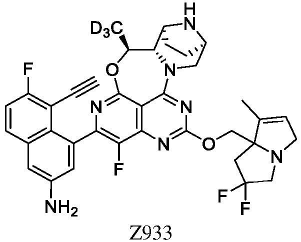 | 675.3 | 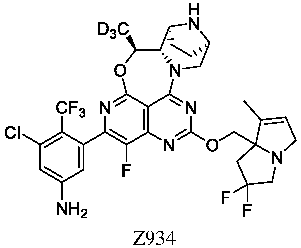 | 685.2 |
| 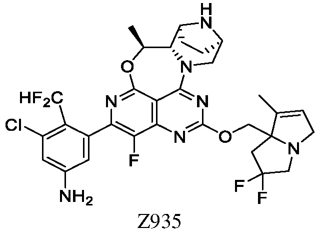 | 664.2 | 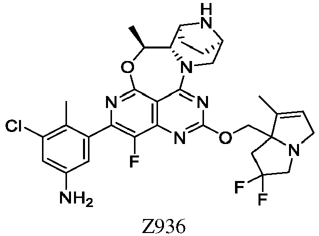 | 628.2 |
| 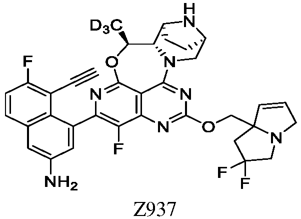 | 661.3 | 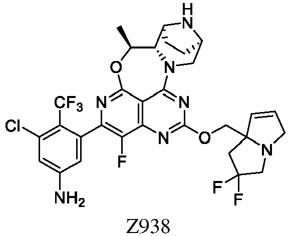 | 668.2 |
| 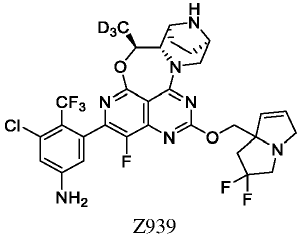 | 671.2 | 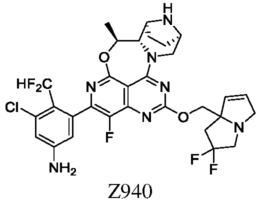 | 650.2 |
| 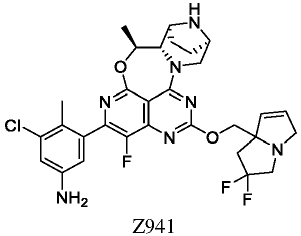 | 614.2 | 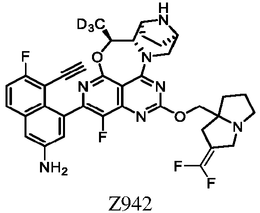 | 675.3 |
| 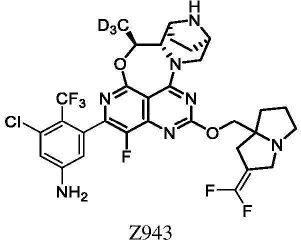 | 685.2 | 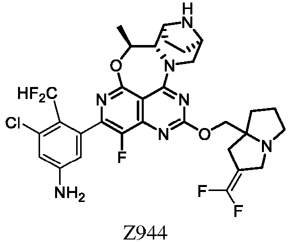 | 664.2 |
| 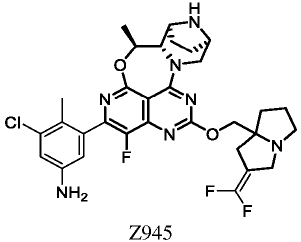 | 628.2 | 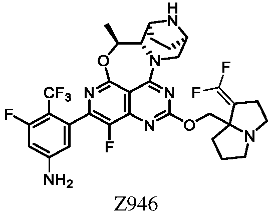 | 666.2 |
| 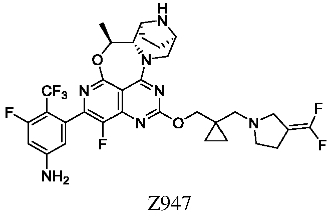 | 680.3 | 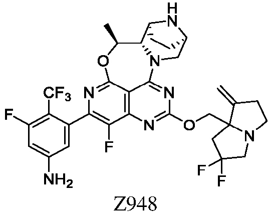 | 666.2 |
| 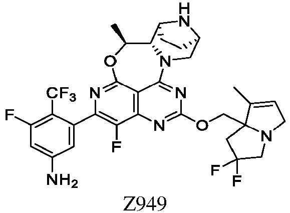 | 666.2 | 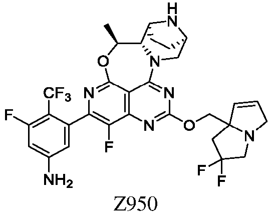 | 652.2 |
| 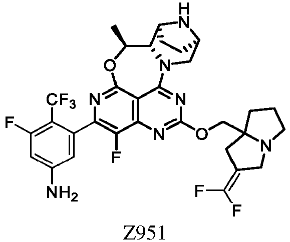 | 666.2 | 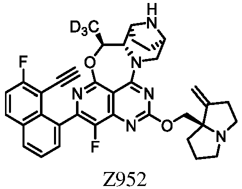 | 624.3 |
| 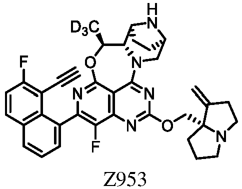 | 624.3 | 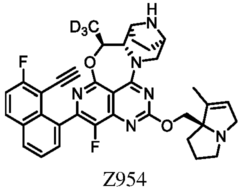 | 624.3 |
| 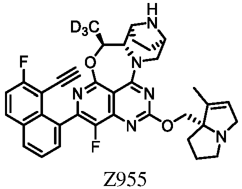 | 624.3 | 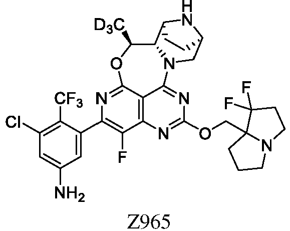 | 673.2 |
| 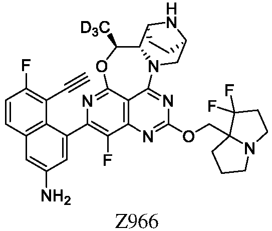 | 663.3 | 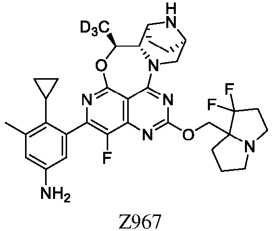 | 625.3 |
| 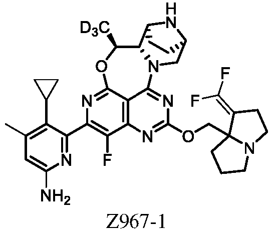 | 638.3 | 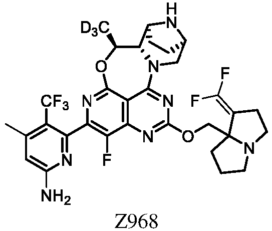 | 666.3 |
| 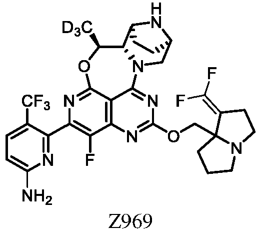 | 652.2 | 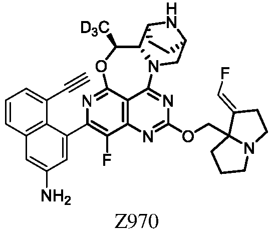 | 639.3 |
| 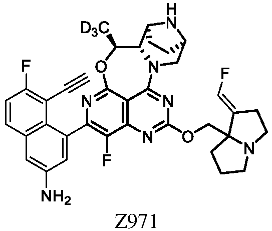 | 657.3 | 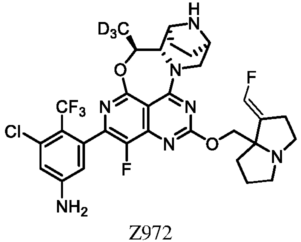 | 667.2 |
| 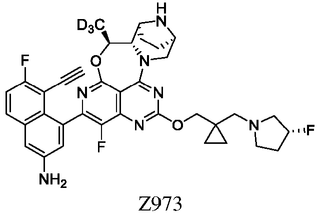 | 659.3 | 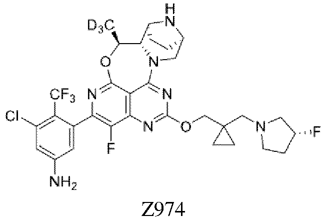 | 669.2 |
| 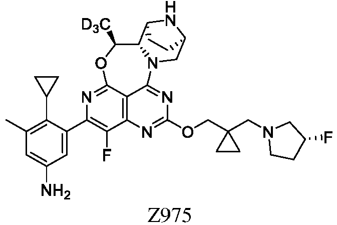 | 621.3 | 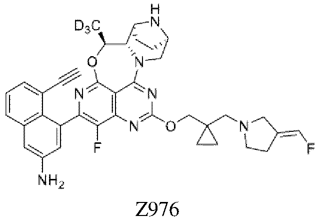 | 653.3 |
| 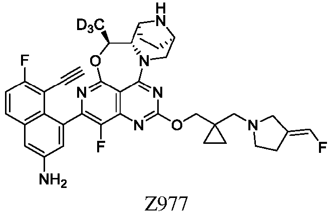 | 671.3 | 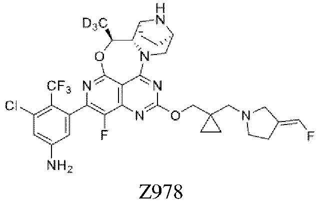 | 681.2 |
| 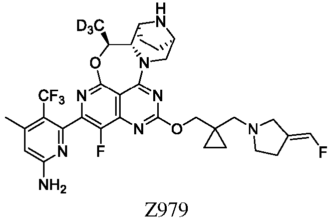 | 662.3 | 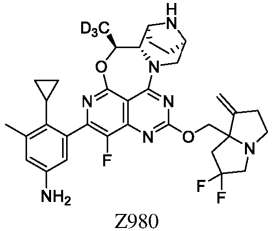 | 637.3 |
| 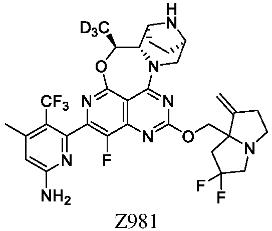 | 666.3 | 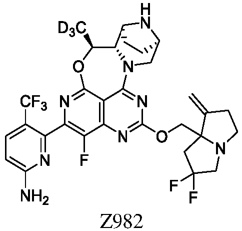 | 652.2 |
| 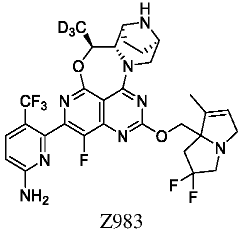 | 652.2 | 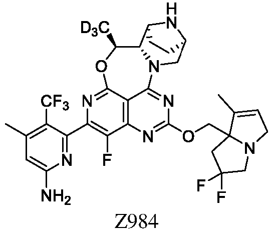 | 666.3 |
| 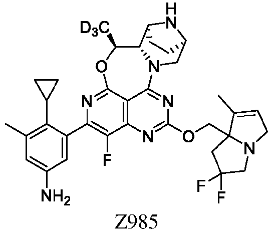 | 637.3 | 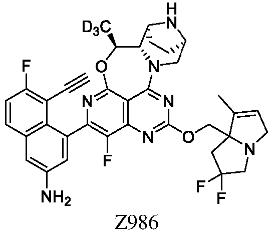 | 675.3 |
| 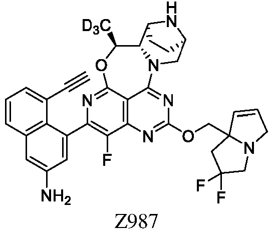 | 643.3 | 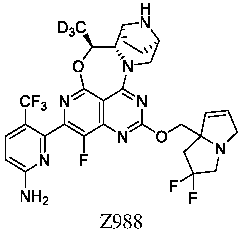 | 638.2 |
| 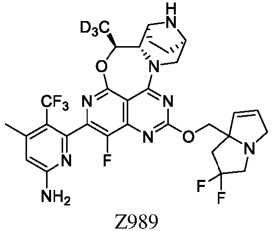 | 652.2 | 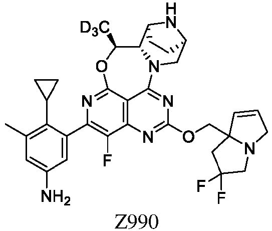 | 623.3 |
| 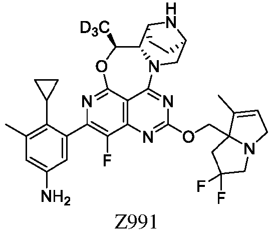 | 637.3 | 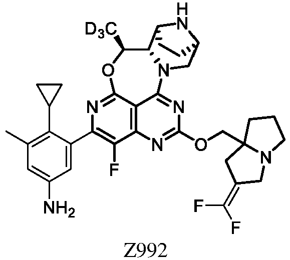 | 637.3 |
| 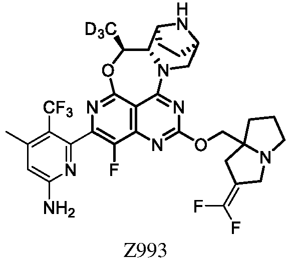 | 666.3 | 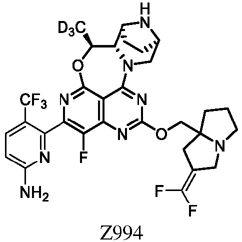 | 652.2 |
| 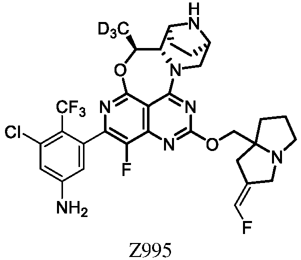 | 667.2 | 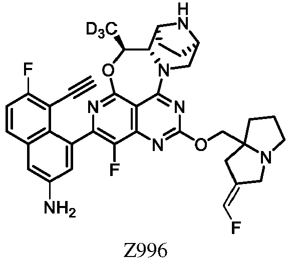 | 657.3 |
| 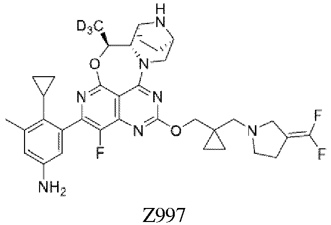 | 651.3 | 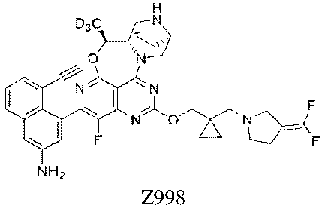 | 671.3 |
| 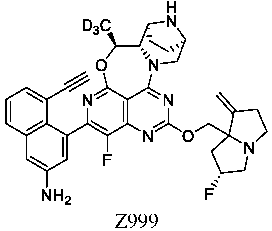 | 639.3 | 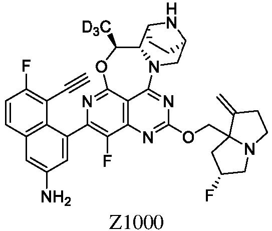 | 657.3 |
| 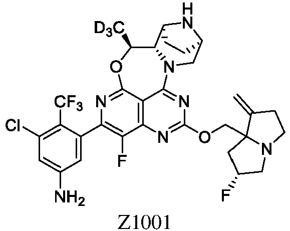 | 667.2 | 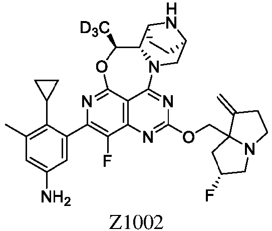 | 619.3 |
| 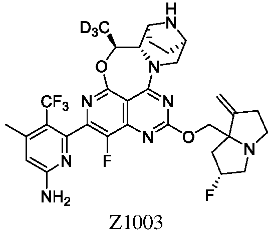 | 648.3 | 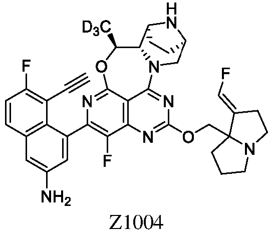 | 657.3 |
| 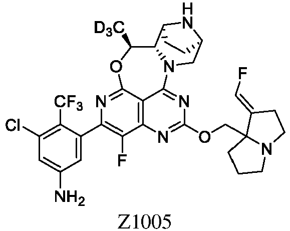 | 667.2 | 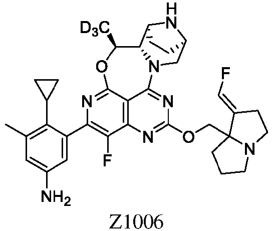 | 619.3 |
| 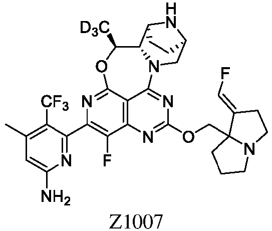 | 648.3 | 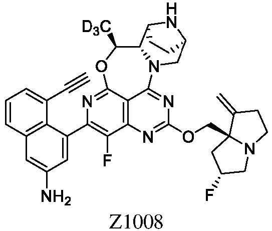 | 639.3 |
| 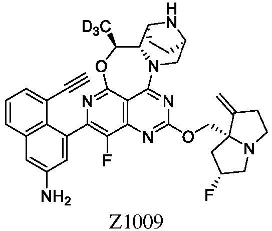 | 639.3 | 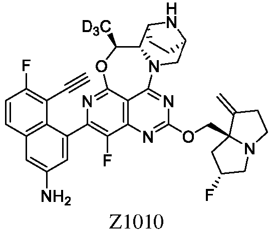 | 657.3 |
| 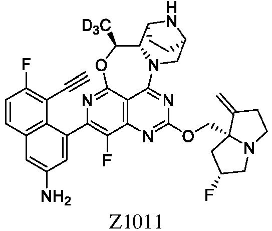 | 657.3 | 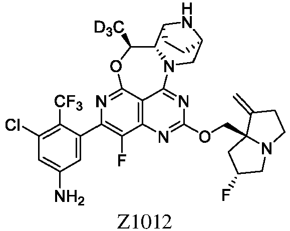 | 667.2 |
| 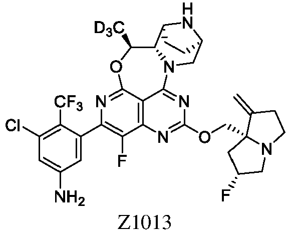 | 667.2 | 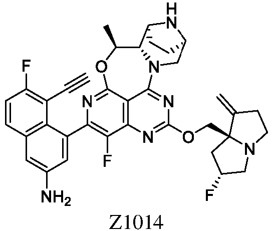 | 654.3 |
| 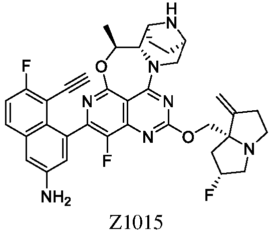 | 654.3 | 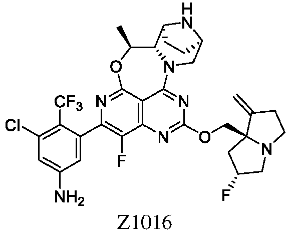 | 664.2 |
| 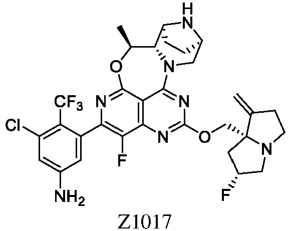 | 664.2 | 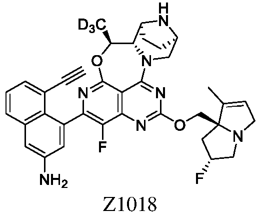 | 639.3 |
| 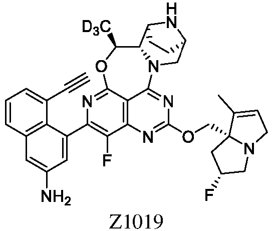 | 639.3 | 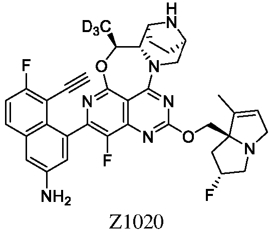 | 657.3 |
| 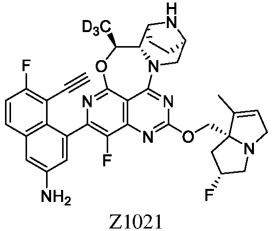 | 657.3 | 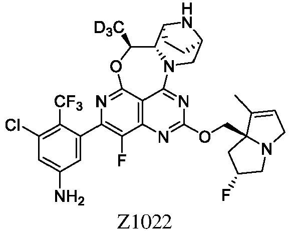 | 667.2 |
| 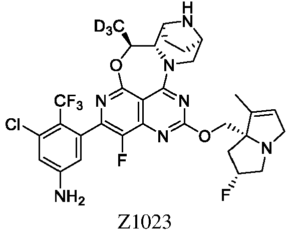 | 667.2 | 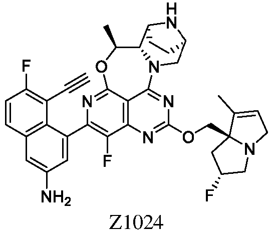 | 654.3 |
| 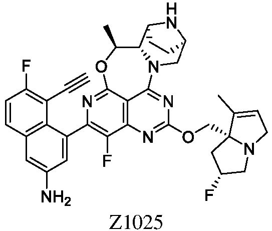 | 654.3 | 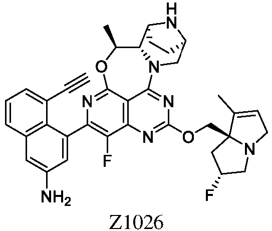 | 639.3 |
| 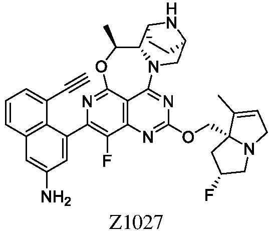 | 639.3 | 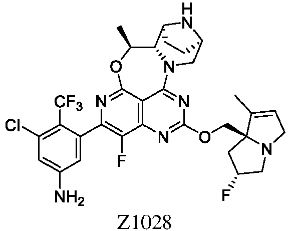 | 664.2 |
| 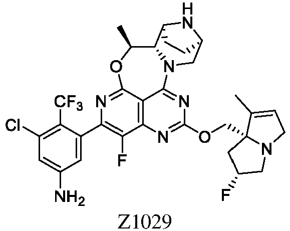 | 664.2 | 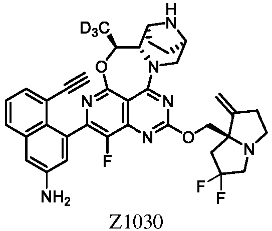 | 657.3 |
| 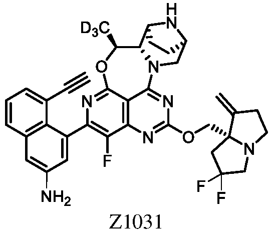 | 657.3 | 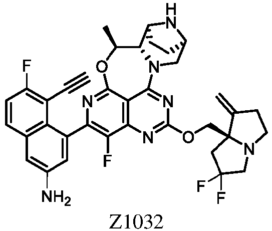 | 672.3 |
| 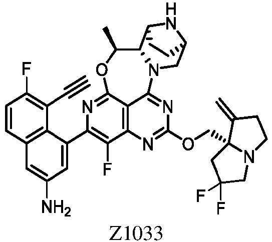 | 672.3 | 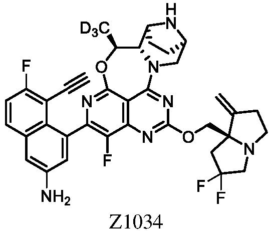 | 675.3 |
| 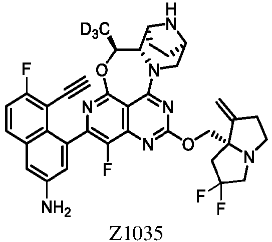 | 675.3 | 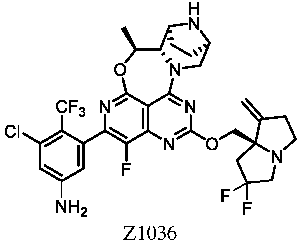 | 682.2 |
| 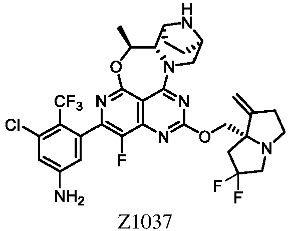 | 682.2 | 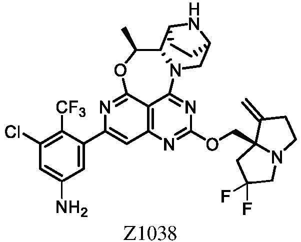 | 664.2 |
| 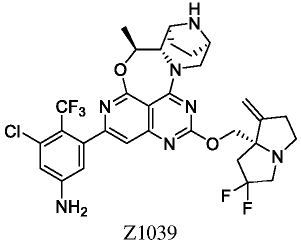 | 664.2 | 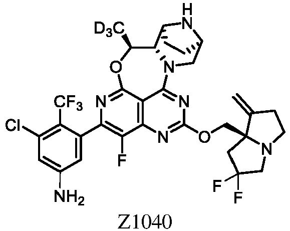 | 685.2 |
| 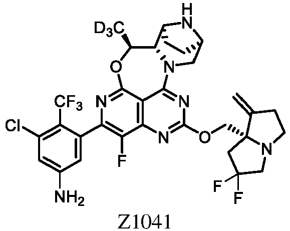 | 685.2 | 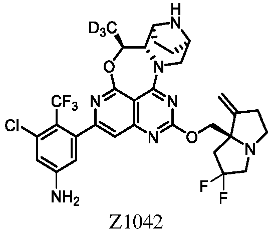 | 667.2 |
| 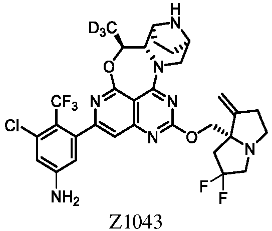 | 667.2 | 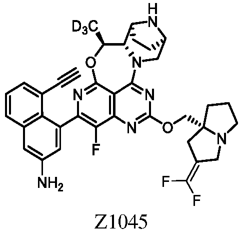 | 657.3 |
| 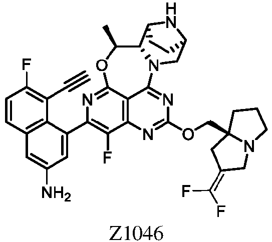 | 672.3 | 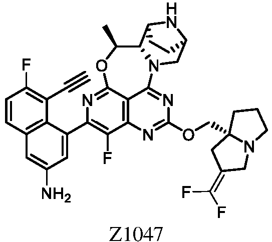 | 672.3 |
| 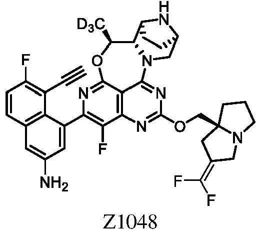 | 675.3 | 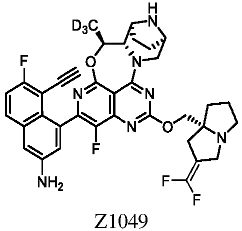 | 675.3 |
| 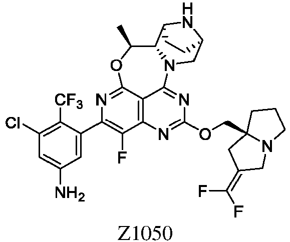 | 682.2 | 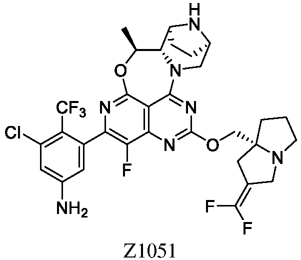 | 682.2 |
| 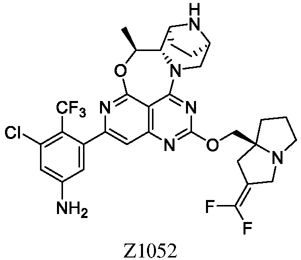 | 664.2 | 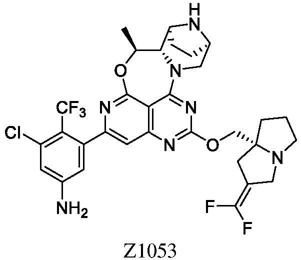 | 664.2 |
| 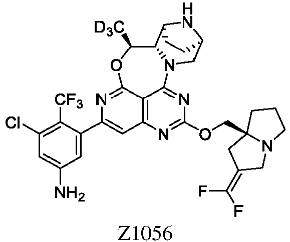 | 667.2 | 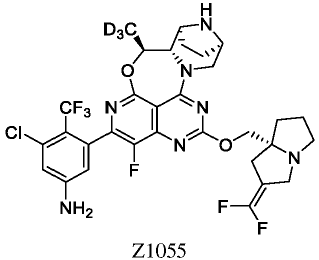 | 685.2 |
| 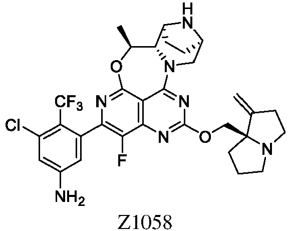 | 646.2 | 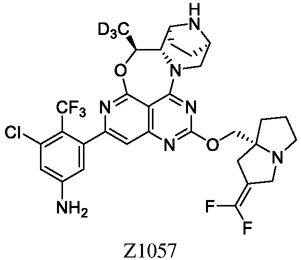 | 667.2 |
| 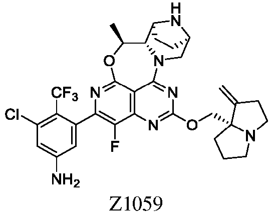 | 646.2 | 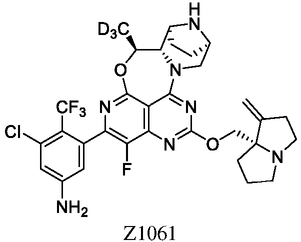 | 649.2 |
| 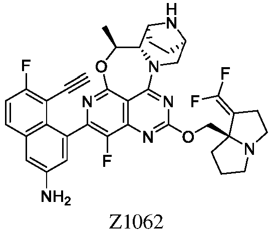 | 672.3 | 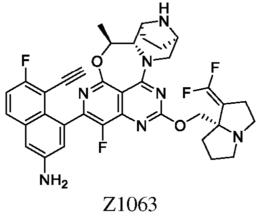 | 672.3 |
| 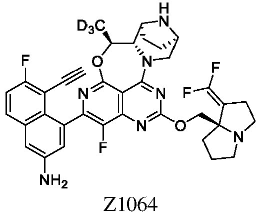 | 675.3 | 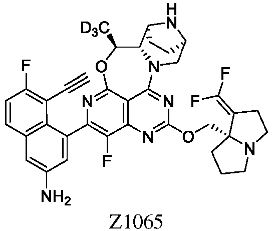 | 675.3 |
| 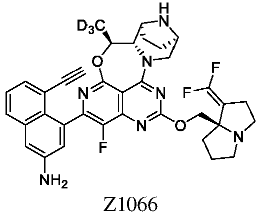 | 675.3 | 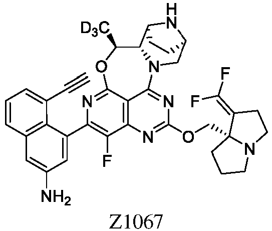 | 675.3 |
| 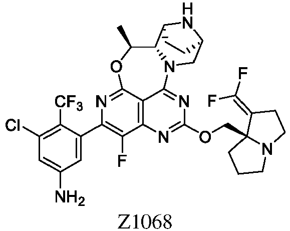 | 682.2 | 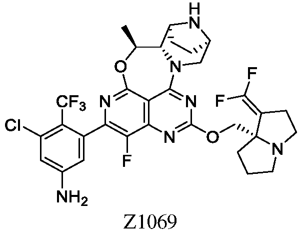 | 682.2 |
| 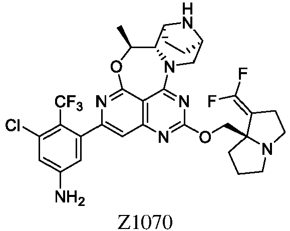 | 664.2 | 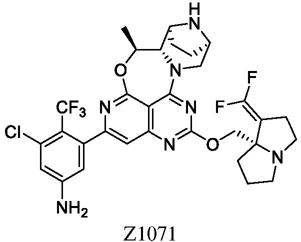 | 664.2 |
| 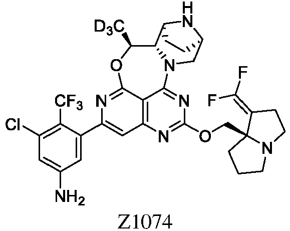 | 667.2 | 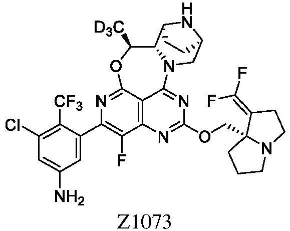 | 685.2 |
| 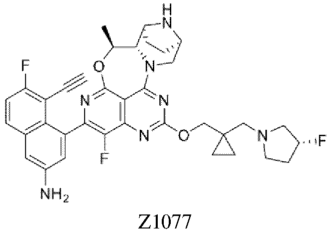 | 656.3 | 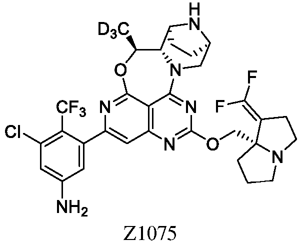 | 667.2 |
| 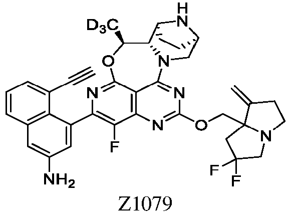 | 657.3 | 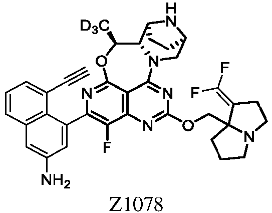 | 657.3 |
| 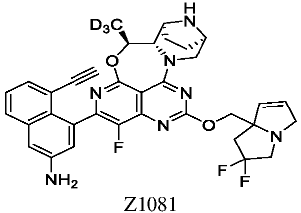 | 643.3 | 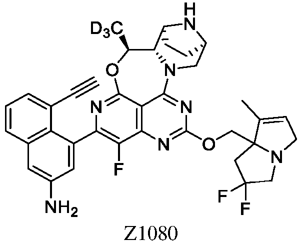 | 657.3 |
| 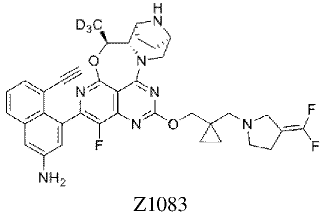 | 671.3 | 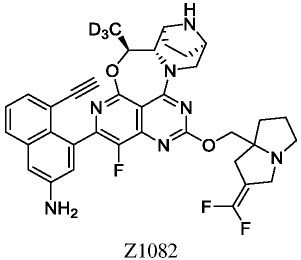 | 657.3 |
| 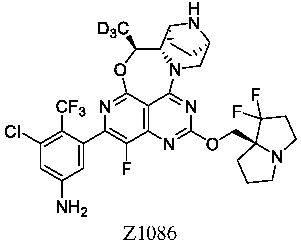 | 673.2 | 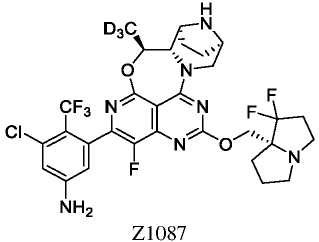 | 673.2 |
| 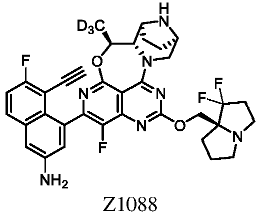 | 663.3 | 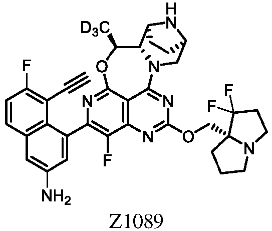 | 663.3 |
| 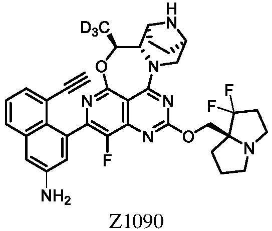 | 645.3 | 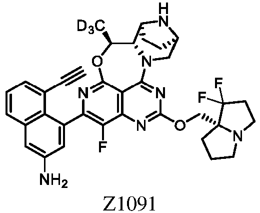 | 645.3 |
| 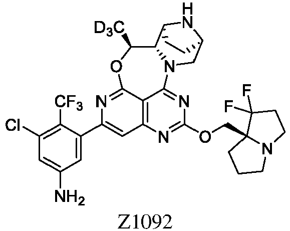 | 655.2 | 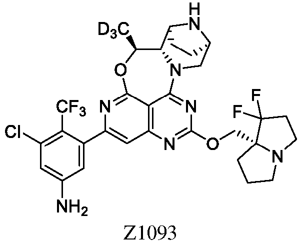 | 655.2 |
| 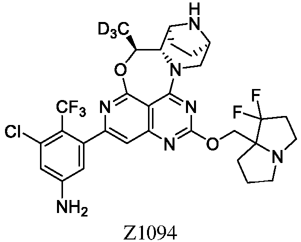 | 655.2 | 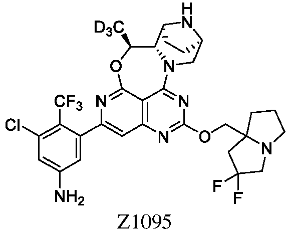 | 655.2 |
| 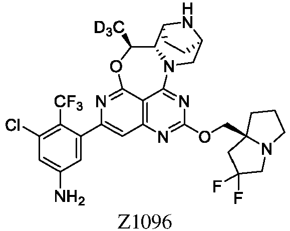 | 655.2 | 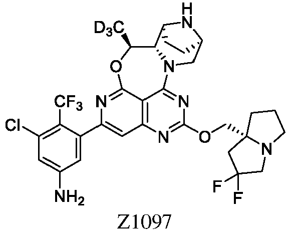 | 655.2 |
| 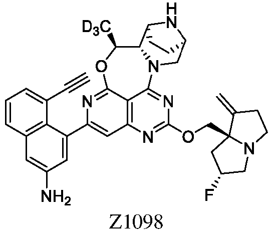 | 621.3 | 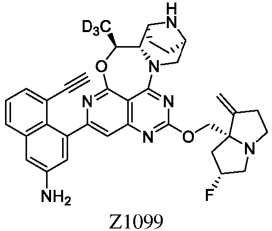 | 621.3 |
| 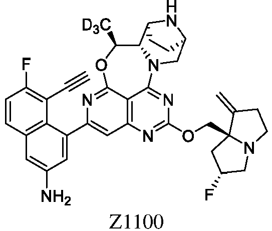 | 639.3 | 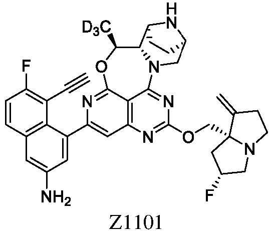 | 639.3 |
| 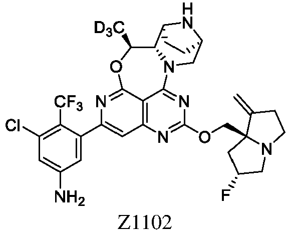 | 649.2 | 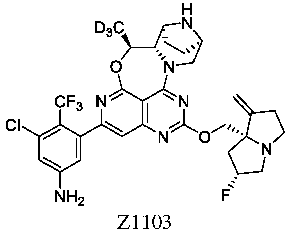 | 649.2 |
| 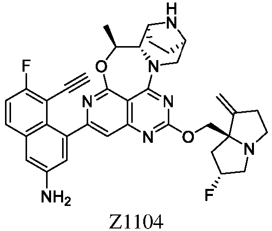 | 636.3 | 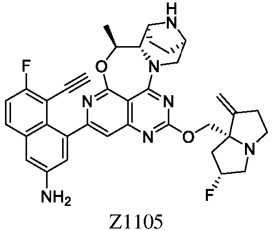 | 636.3 |
| 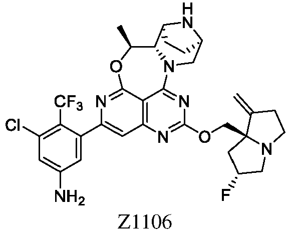 | 646.2 | 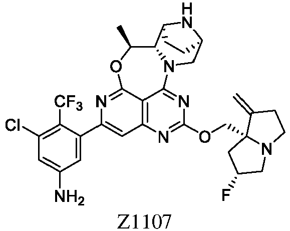 | 646.2 |
| 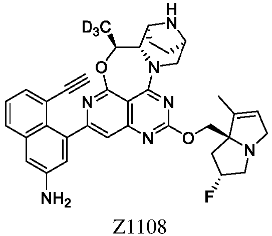 | 621.3 | 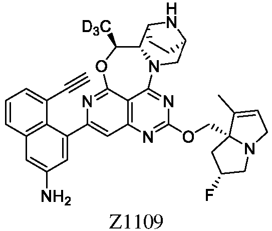 | 621.3 |
| 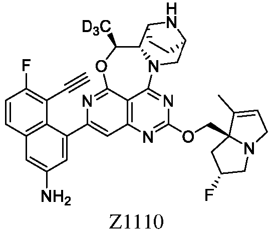 | 639.3 | 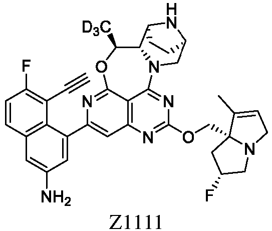 | 639.3 |
| 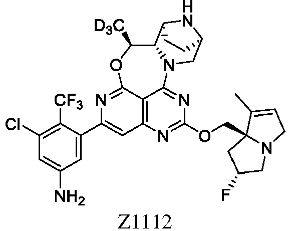 | 649.2 | 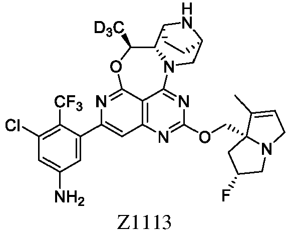 | 649.2 |
| 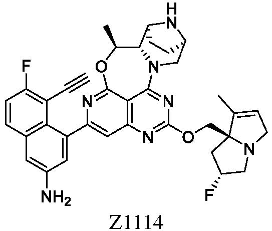 | 636.3 | 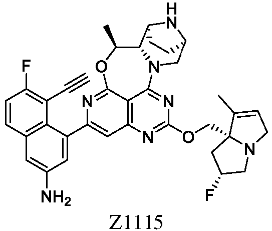 | 636.3 |
| 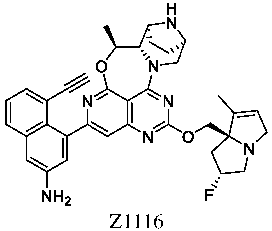 | 618.3 | 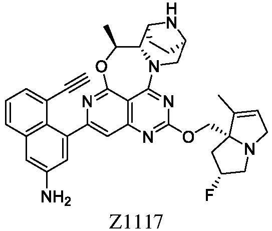 | 618.3 |
| 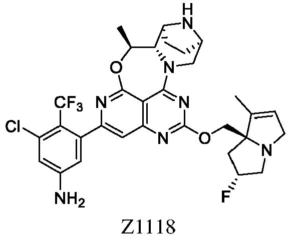 | 646.2 | 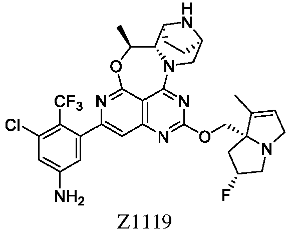 | 646.2 |
| 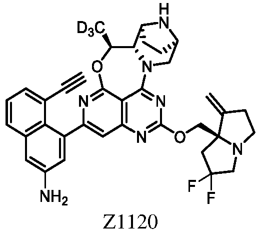 | 639.3 | 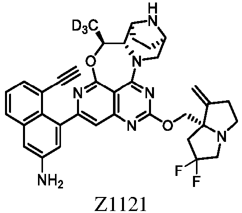 | 639.3 |
| 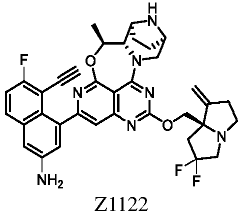 | 654.3 | 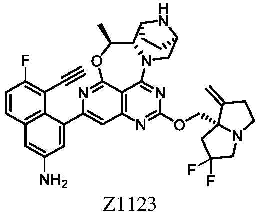 | 654.3 |
| 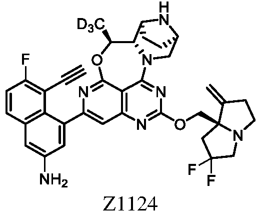 | 657.3 | 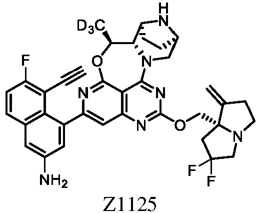 | 657.3 |
| 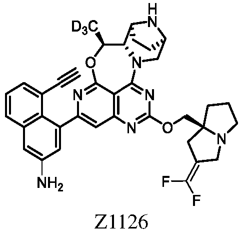 | 639.3 | 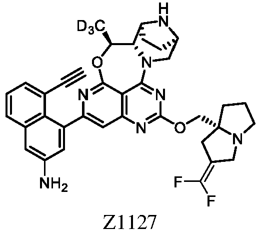 | 639.3 |
| 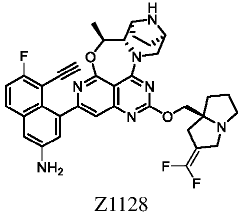 | 654.3 | 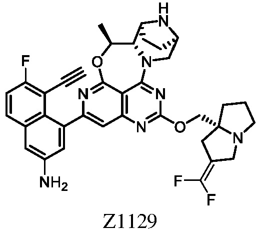 | 654.3 |
| 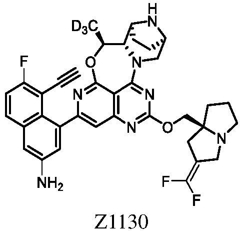 | 657.3 | 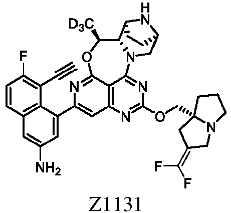 | 657.3 |
| 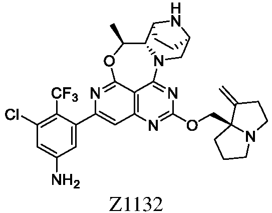 | 628.2 | 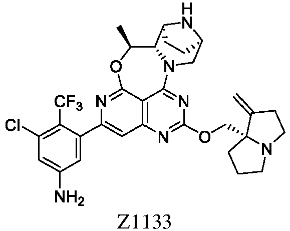 | 628.2 |
| 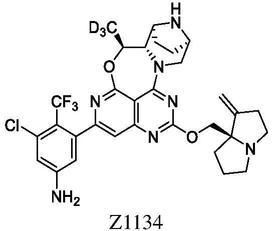 | 631.3 | 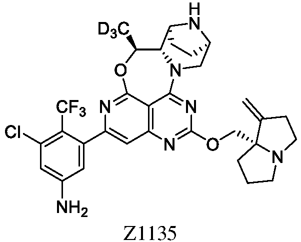 | 631.3 |
| 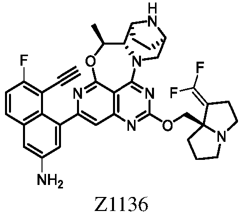 | 654.3 | 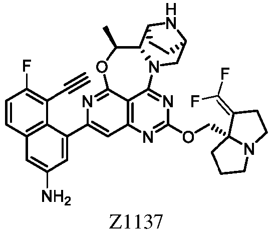 | 654.3 |
| 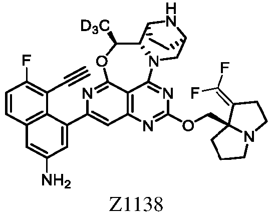 | 657.3 | 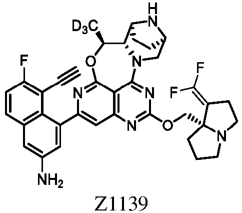 | 657.3 |
| 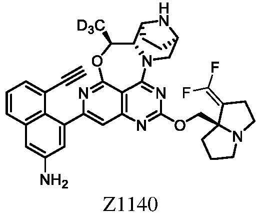 | 639.3 | 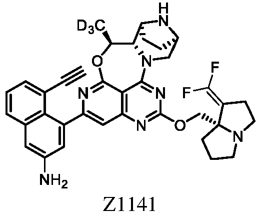 | 639.3 |
| 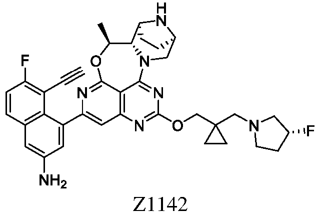 | 638.3 | 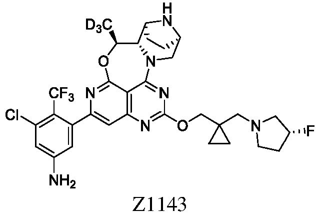 | 651.3 |
| 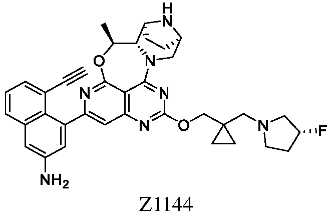 | 620.3 | 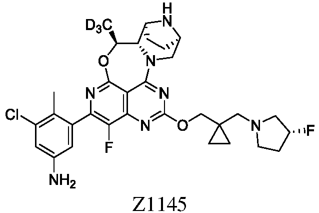 | 615.3 |
| 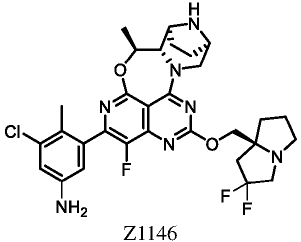 | 616.2 | 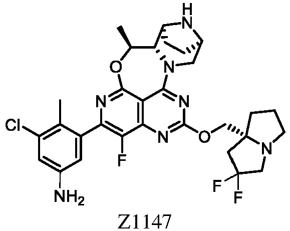 | 616.2 |
| 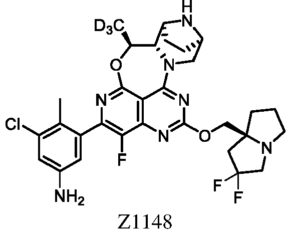 | 619.3 | 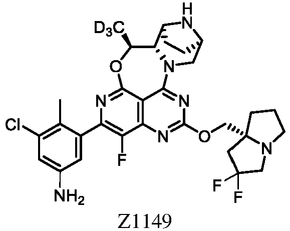 | 619.3 |
| 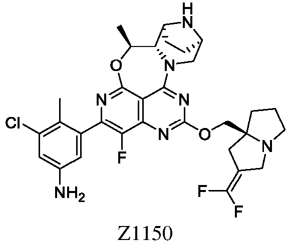 | 628.2 | 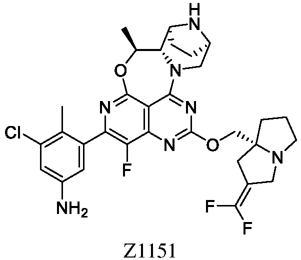 | 628.2 |
| 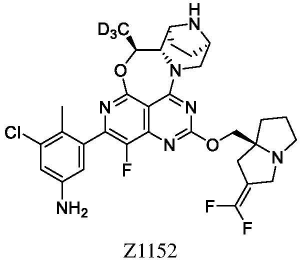 | 631.3 | 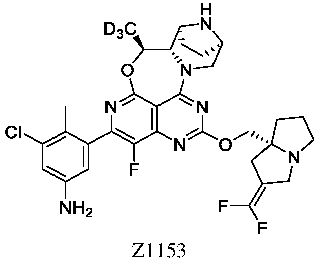 | 631.3 |
| 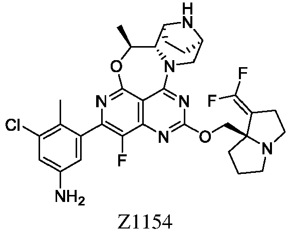 | 628.2 | 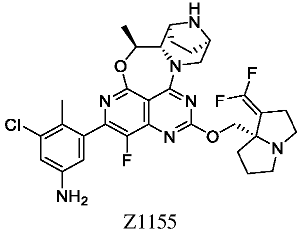 | 628.2 |
| 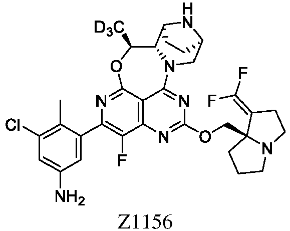 | 631.3 | 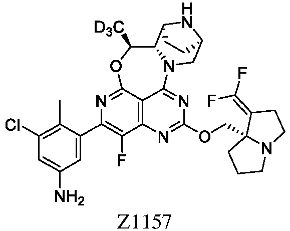 | 631.3 |
| 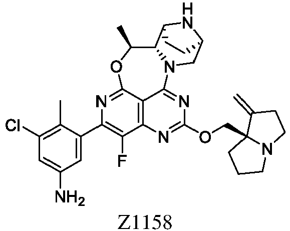 | 592.3 | 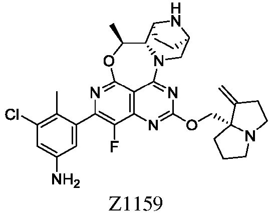 | 592.3 |
| 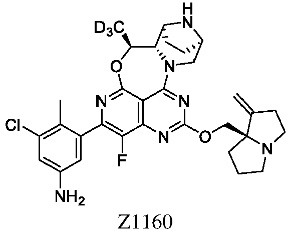 | 595.3 | 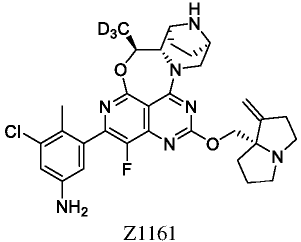 | 595.3 |
| 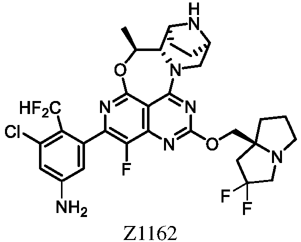 | 652.2 | 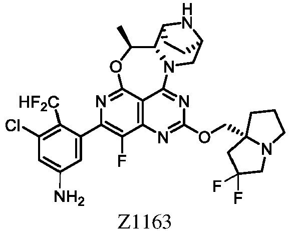 | 652.2 |
| 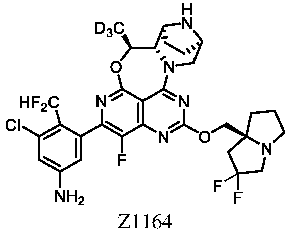 | 655.2 | 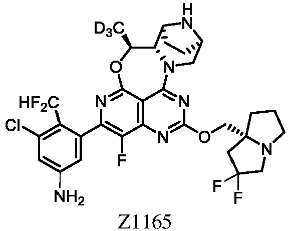 | 655.2 |
| 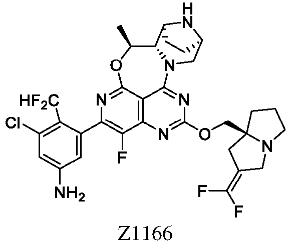 | 664.2 | 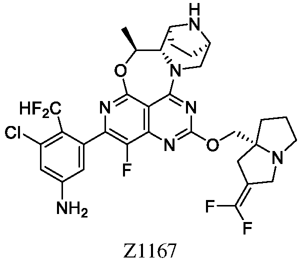 | 664.2 |
| 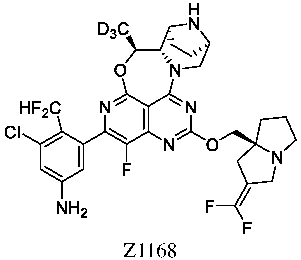 | 667.2 | 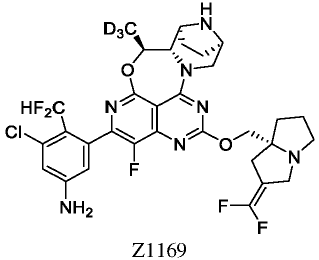 | 667.2 |
| 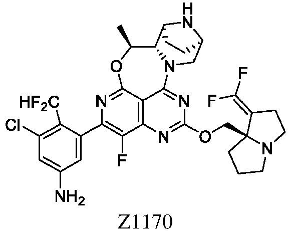 | 664.2 | 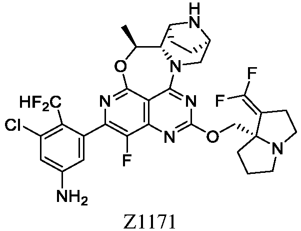 | 664.2 |
| 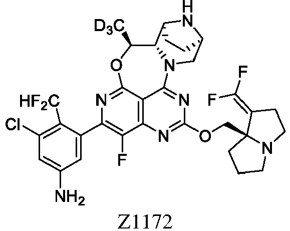 | 667.2 | 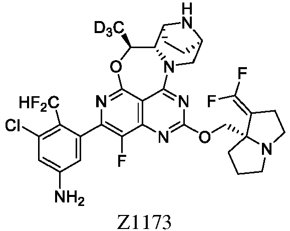 | 667.2 |
| 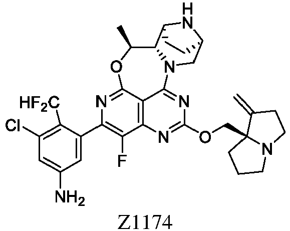 | 628.2 | 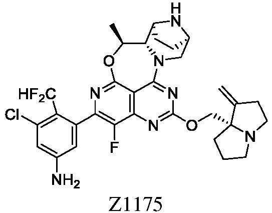 | 628.2 |
| 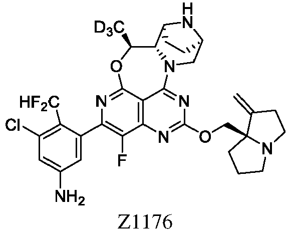 | 631.3 | 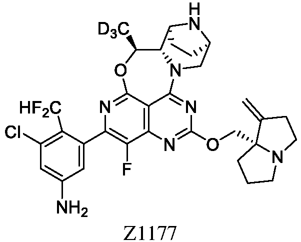 | 631.3 |
| 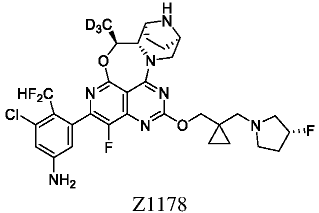 | 651.3 | 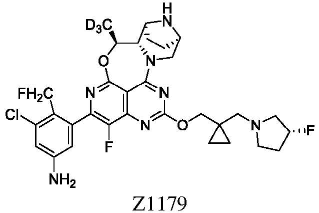 | 633.3 |
| 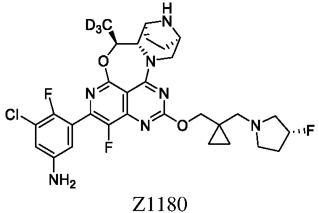 | 619.3 | 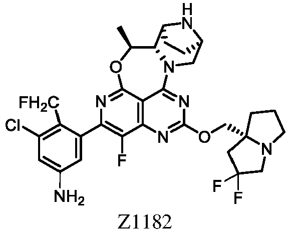 | 634.2 |
| 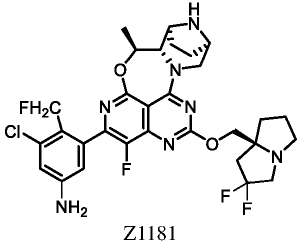 | 634.2 | 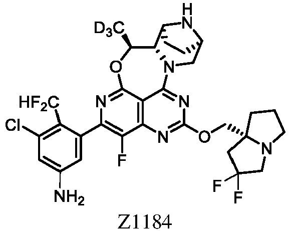 | 637.2 |
| 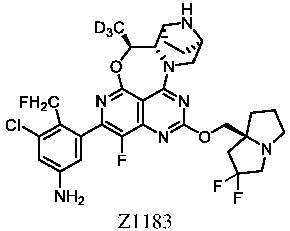 | 637.2 | 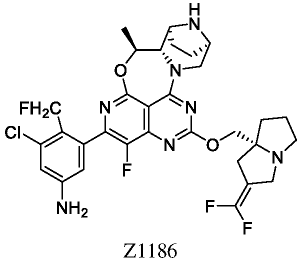 | 646.2 |
| 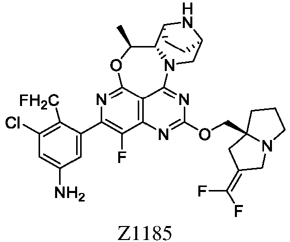 | 646.2 | 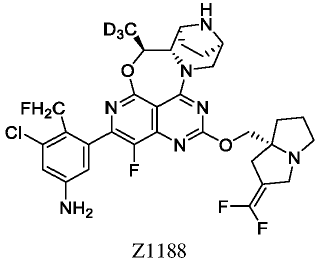 | 649.2 |
| 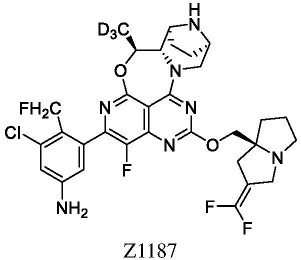 | 649.2 | 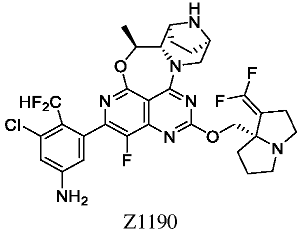 | 646.2 |
| 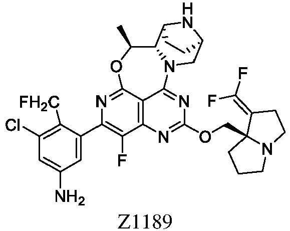 | 646.2 | 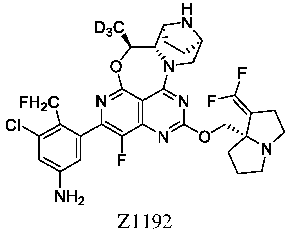 | 649.2 |
| 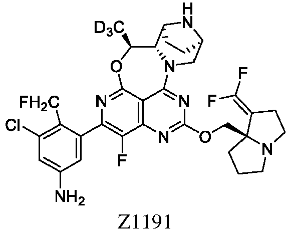 | 649.2 | 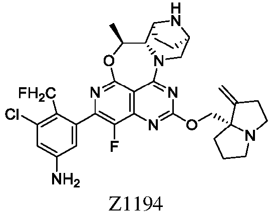 | 610.2 |
| 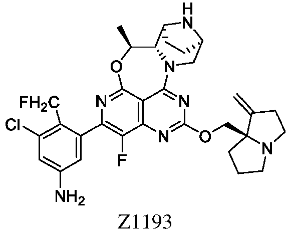 | 610.2 | 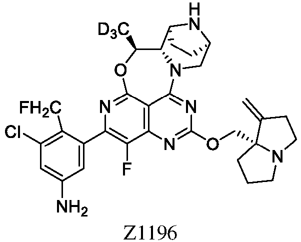 | 613.3 |
| 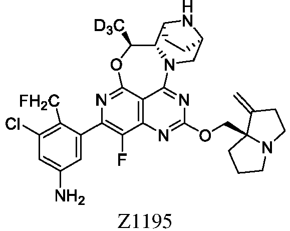 | 613.3 | 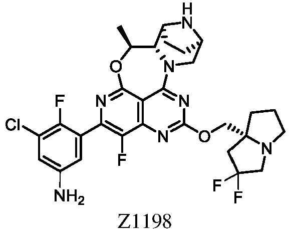 | 620.2 |
| 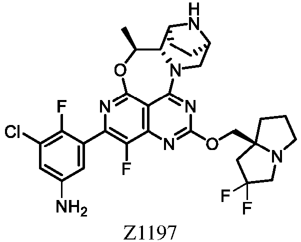 | 620.2 | 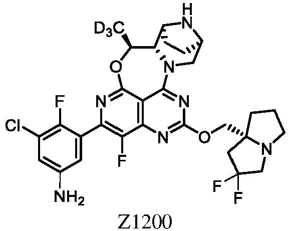 | 623.2 |
| 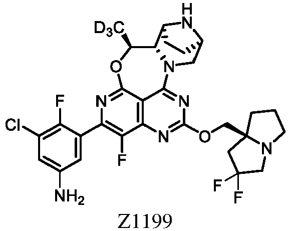 | 623.2 | 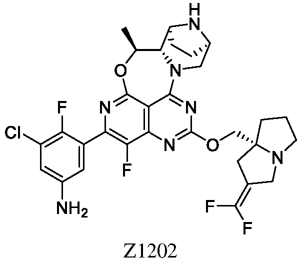 | 632.2 |
| 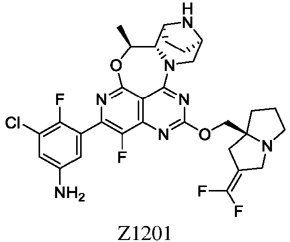 | 632.2 | 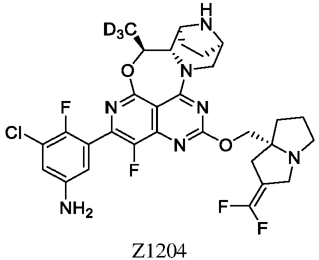 | 635.2 |
| 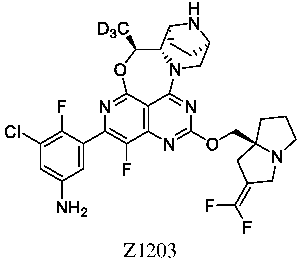 | 635.2 | 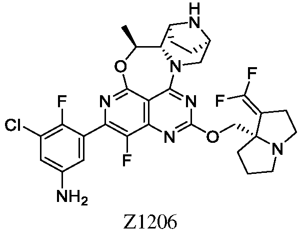 | 632.2 |
| 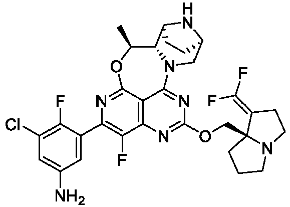 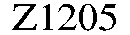 | 632.2 | 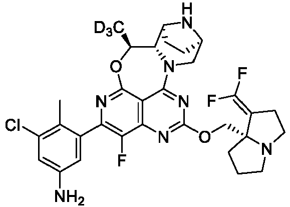 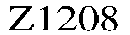 | 635.2 |
| 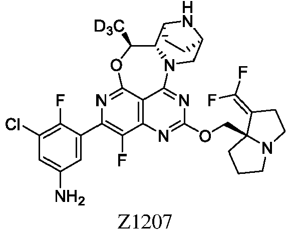 | 635.2 | 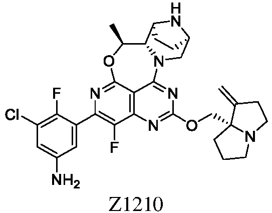 | 596.2 |
| 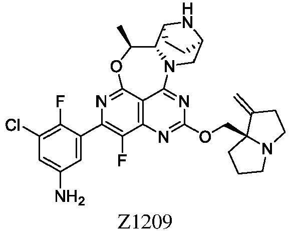 | 596.2 | 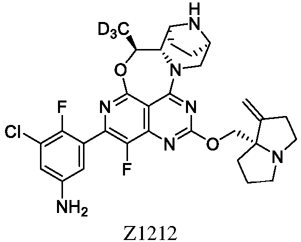 | 599.2 |
| 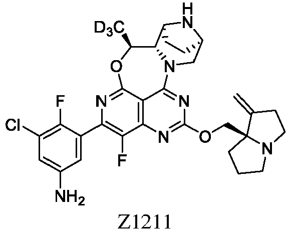 | 599.2 | 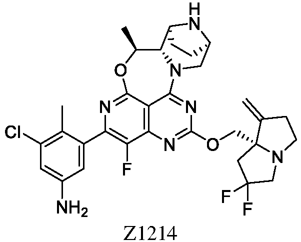 | 628.2 |
| 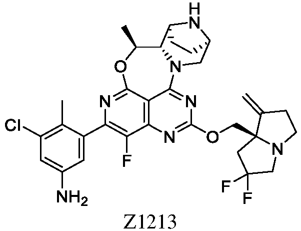 | 628.2 | 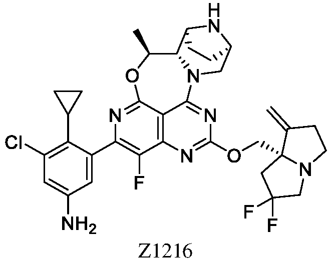 | 654.2 |
| 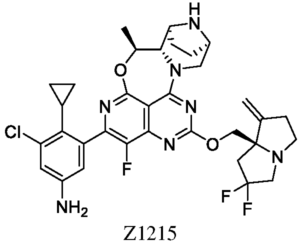 | 654.2 | 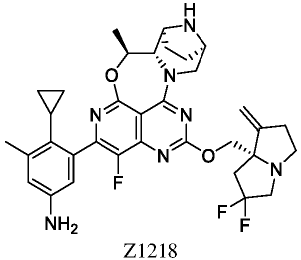 | 634.3 |
| 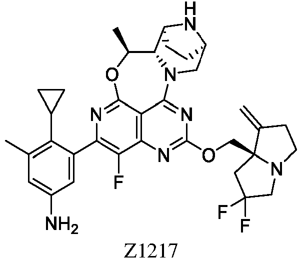 | 634.3 | 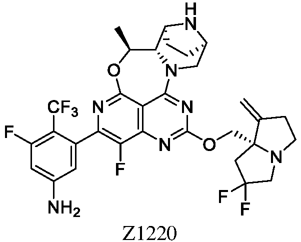 | 666.2 |
| 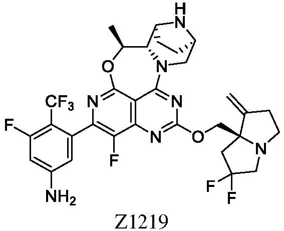 | 666.2 | 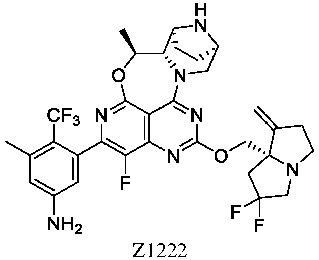 | 662.3 |
| 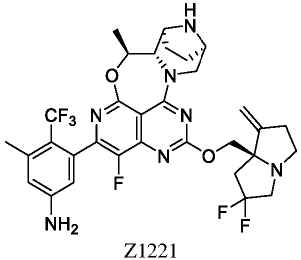 | 662.3 | 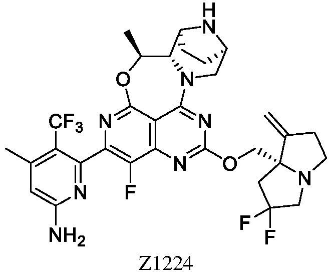 | 663.3 |
| 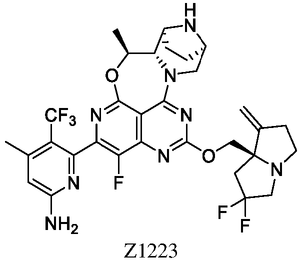 | 663.3 | 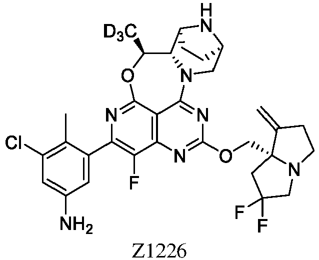 | 631.3 |
| 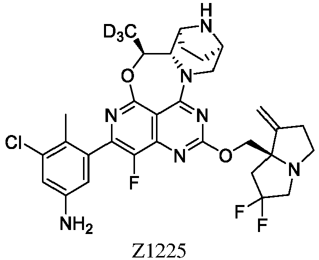 | 631.3 | 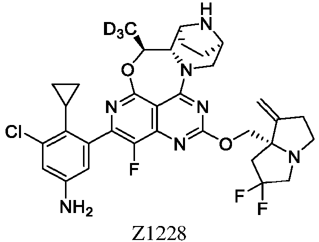 | 657.3 |
| 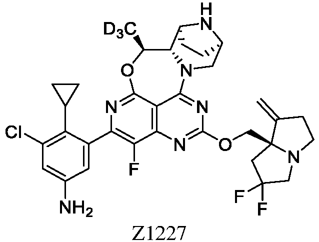 | 657.3 | 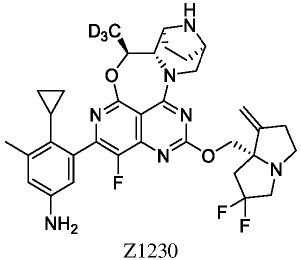 | 637.3 |
| 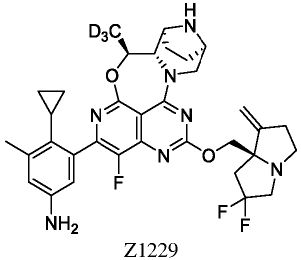 | 637.3 | 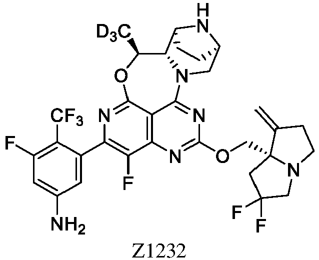 | 669.3 |
| 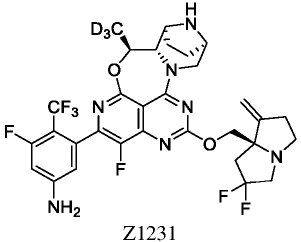 | 669.3 | 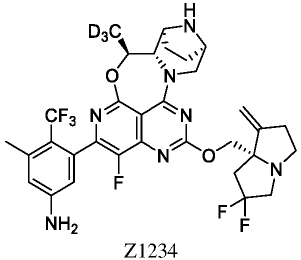 | 665.3 |
| 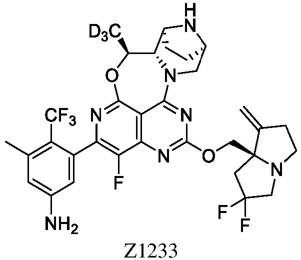 | 665.3 | 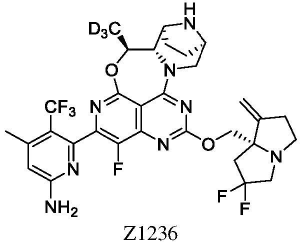 | 666.3 |
| 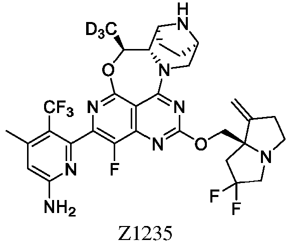 | 666.3 | 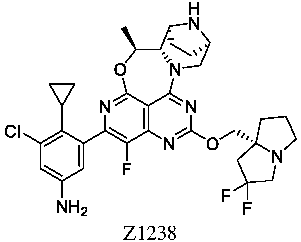 | 642.2 |
| 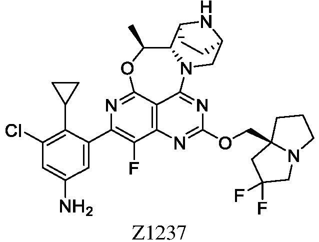 | 642.2 | 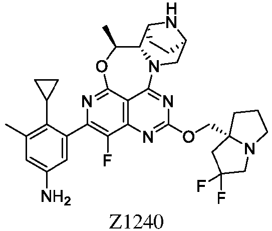 | 622.3 |
| 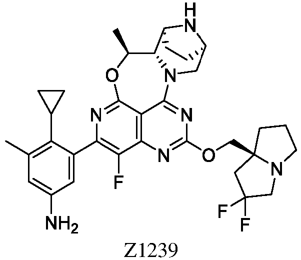 | 622.3 | 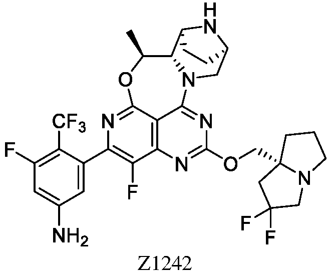 | 654.2 |
| 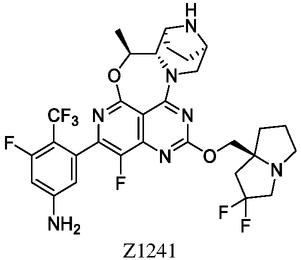 | 654.2 | 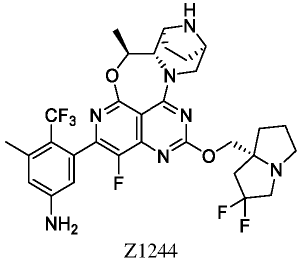 | 650.3 |
| 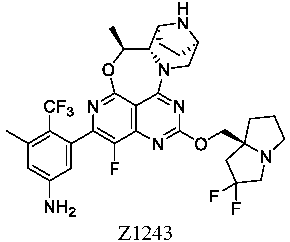 | 650.3 | 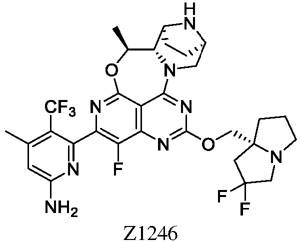 | 651.3 |
| 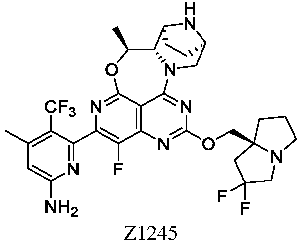 | 651.3 | 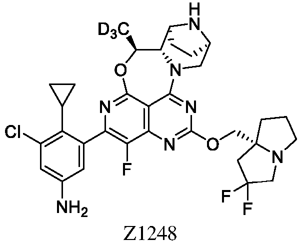 | 645.3 |
| 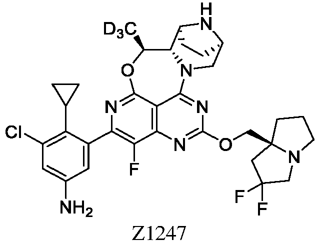 | 645.3 | 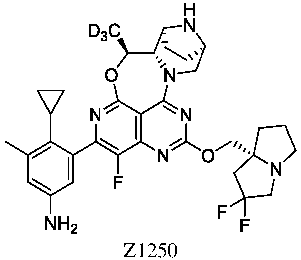 | 625.3 |
| 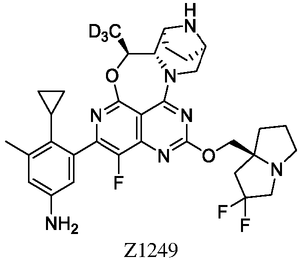 | 625.3 | 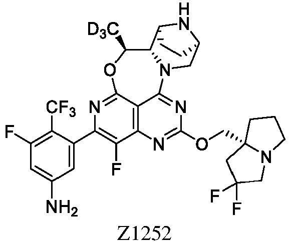 | 657.3 |
| 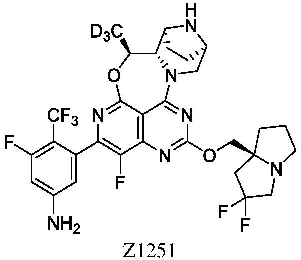 | 657.3 | 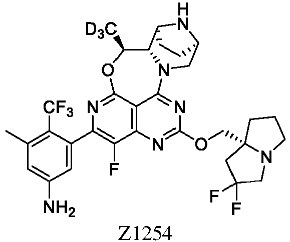 | 653.3 |
| 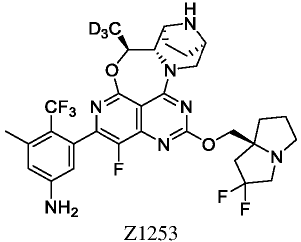 | 653.3 | 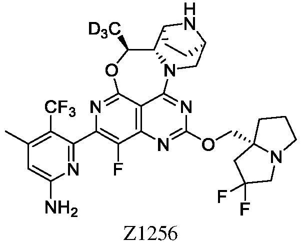 | 654.3 |
| 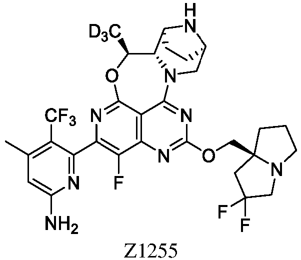 | 654.3 | 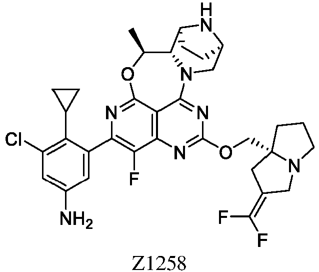 | 654.2 |
| 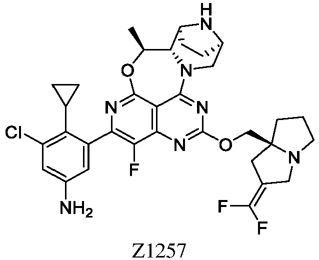 | 654.2 | 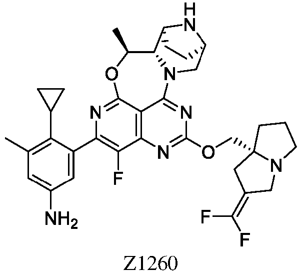 | 634.3 |
| 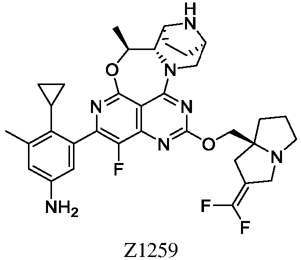 | 634.3 | 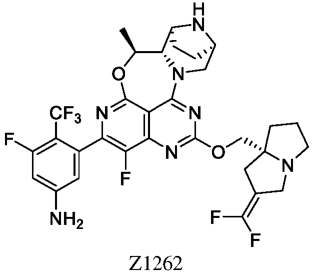 | 666.2 |
| 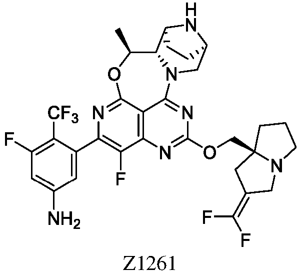 | 666.2 | 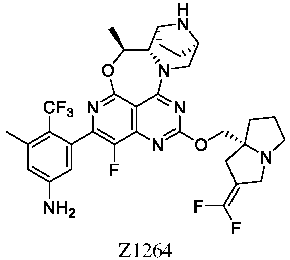 | 666.2 |
| 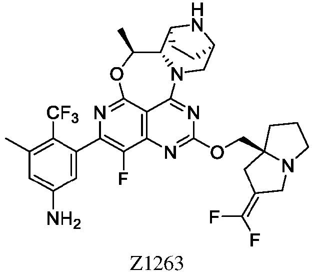 | 666.2 | 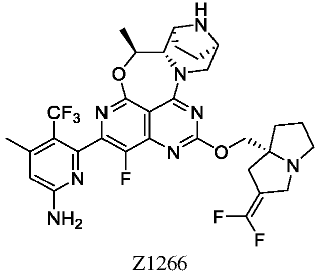 | 663.3 |
| 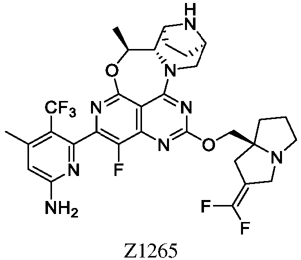 | 663.3 | 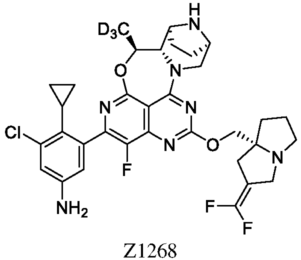 | 657.3 |
| 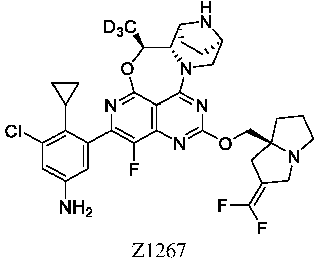 | 657.3 | 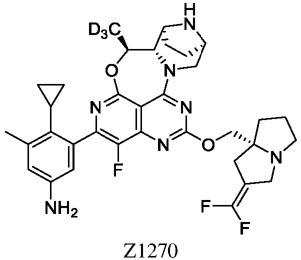 | 637.3 |
| 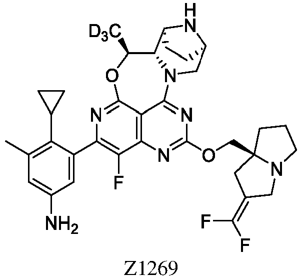 | 637.3 | 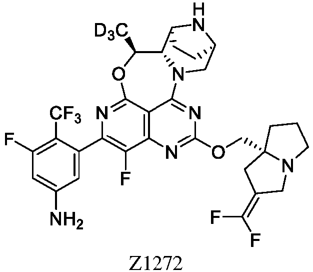 | 669.3 |
| 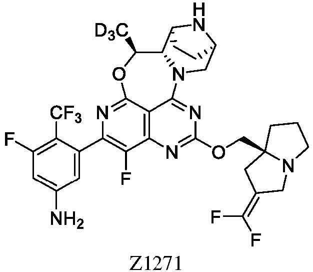 | 669.3 | 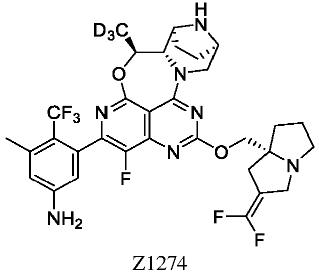 | 665.3 |
| 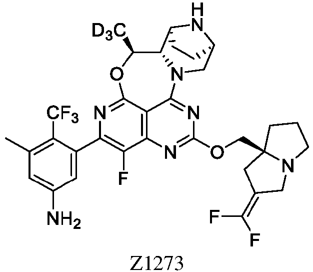 | 665.3 | 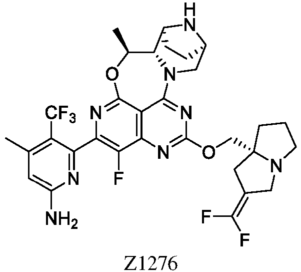 | 666.3 |
| 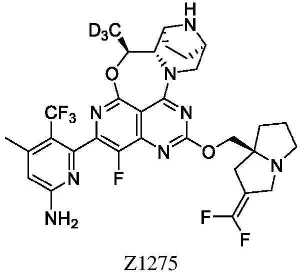 | 666.3 | 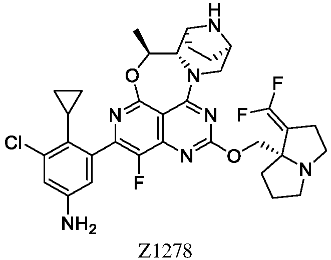 | 654.2 |
| 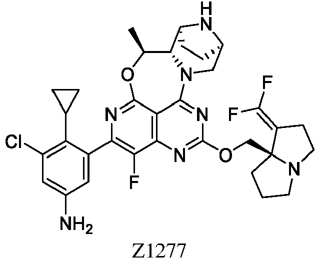 | 654.2 | 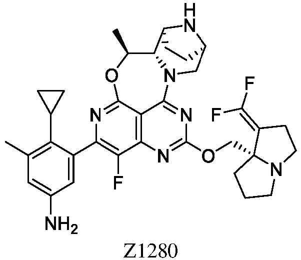 | 634.3 |
| 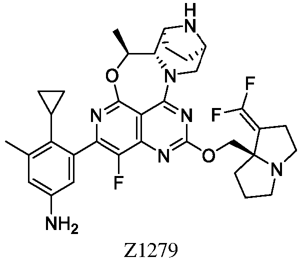 | 634.3 | 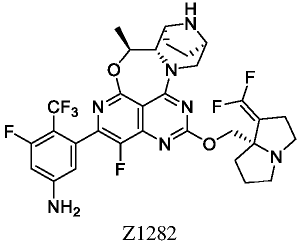 | 666.2 |
| 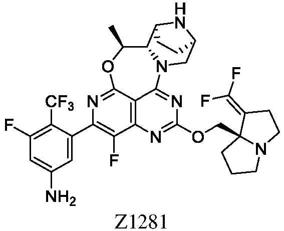 | 666.2 | 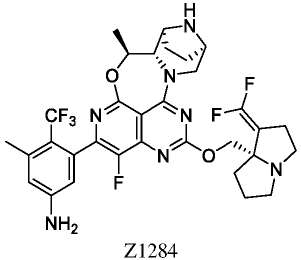 | 662.3 |
| 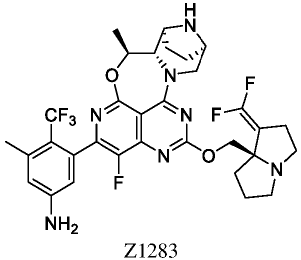 | 662.3 | 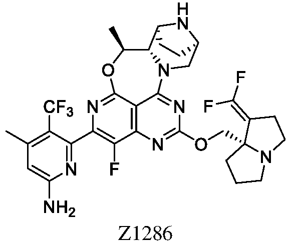 | 663.3 |
| 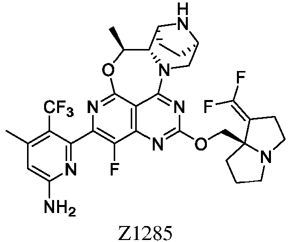 | 663.3 | 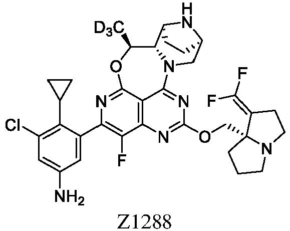 | 657.3 |
| 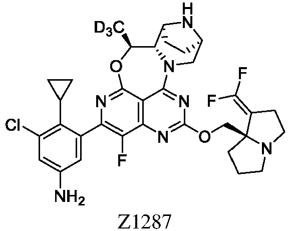 | 657.3 | 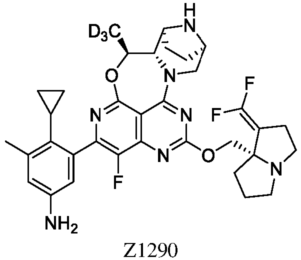 | 637.3 |
| 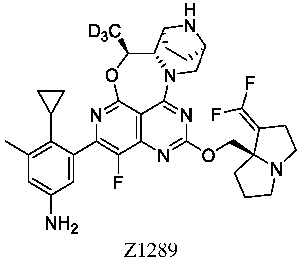 | 637.3 | 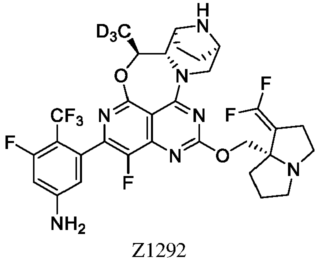 | 669.3 |
| 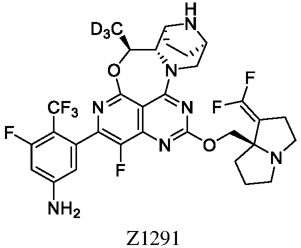 | 669.3 | 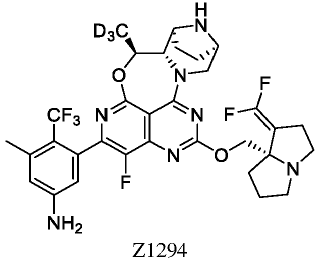 | 665.3 |
| 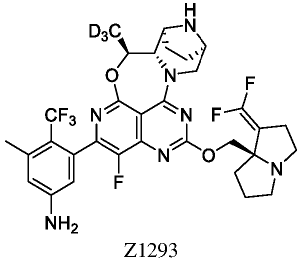 | 665.3 | 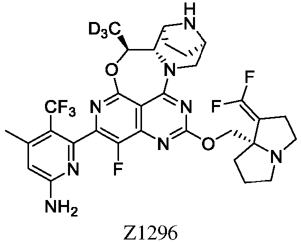 | 666.3 |
| 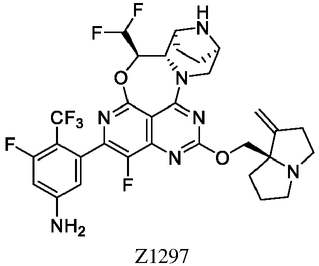 | 666.2 | 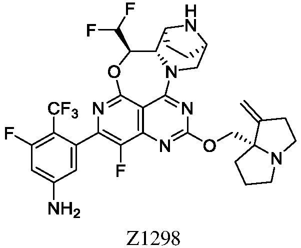 | 666.2 |
| 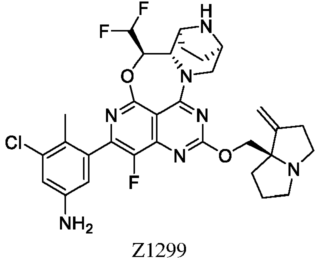 | 628.2 | 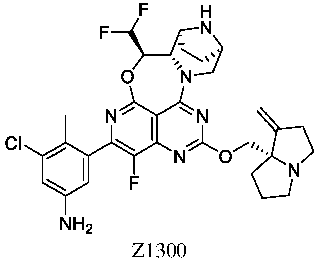 | 628.2 |
| 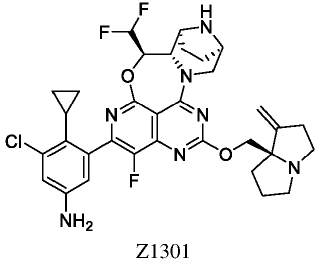 | 654.2 | 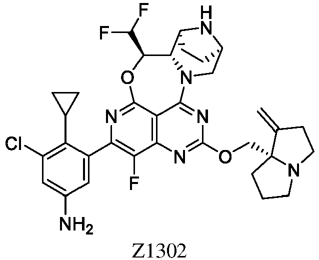 | 654.2 |
| 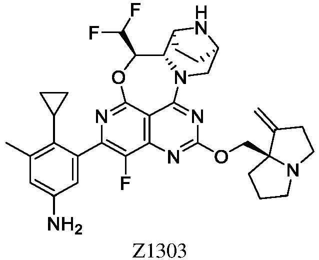 | 634.3 | 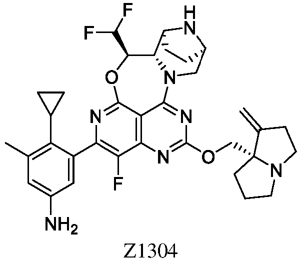 | 634.3 |
| 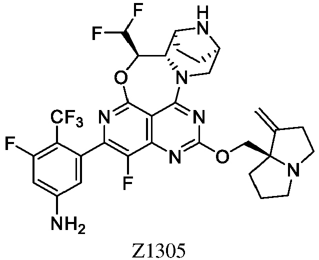 | 666.2 | 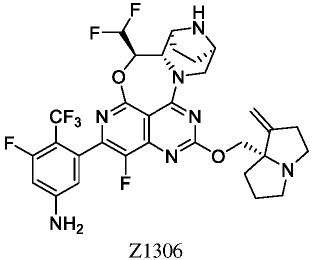 | 666.2 |
| 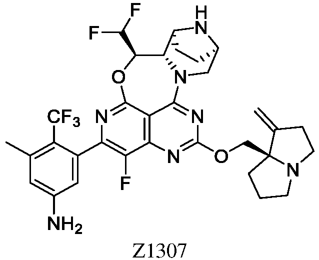 | 662.3 | 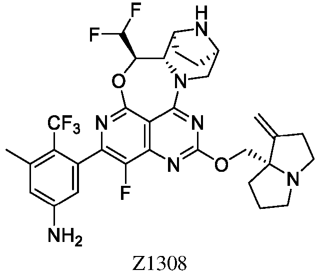 | 662.3 |
| 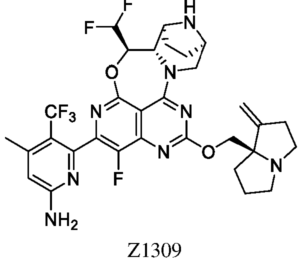 | 663.3 | 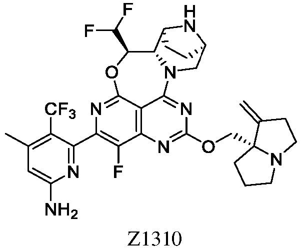 | 663.3 |
| 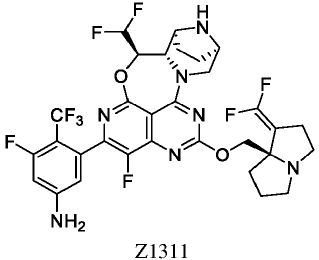 | 702.2 | 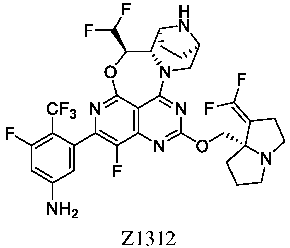 | 702.2 |
| 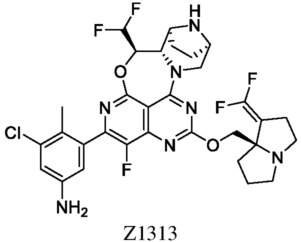 | 664.2 | 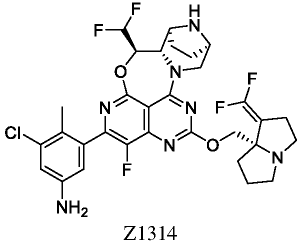 | 664.2 |
| 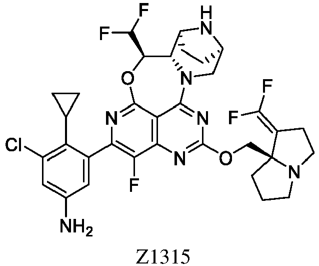 | 690.2 | 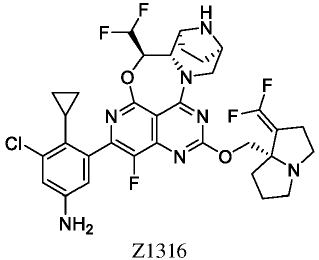 | 690.2 |
| 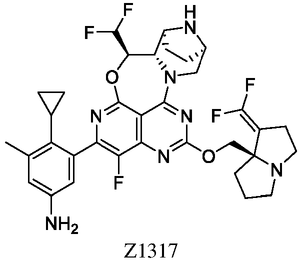 | 670.3 | 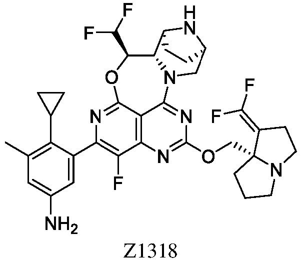 | 670.3 |
| 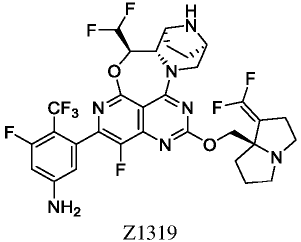 | 702.2 | 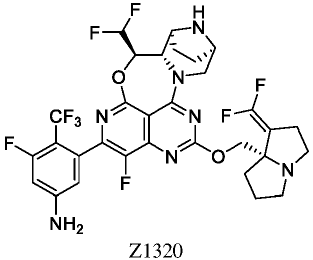 | 702.2 |
| 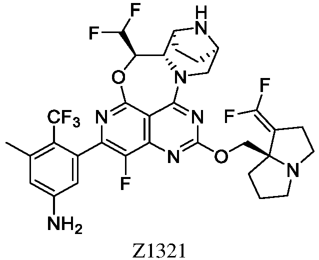 | 698.2 | 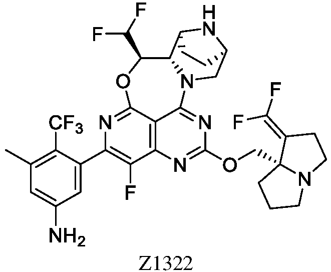 | 698.2 |
| 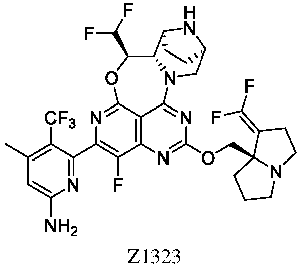 | 699.2 | 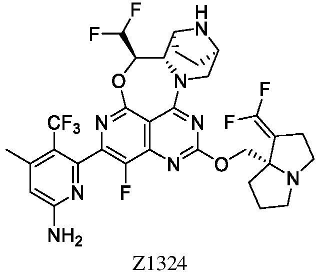 | 699.2 |
| 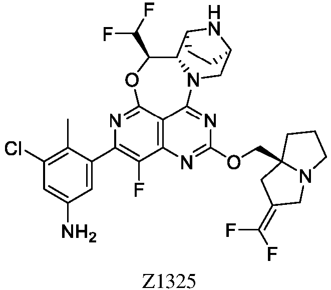 | 664.2 | 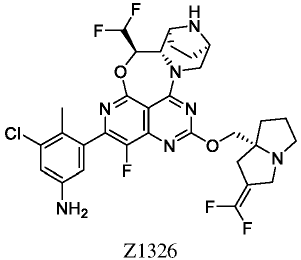 | 664.2 |
| 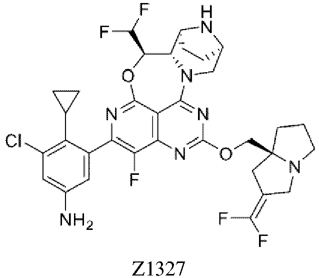 | 690.2 | 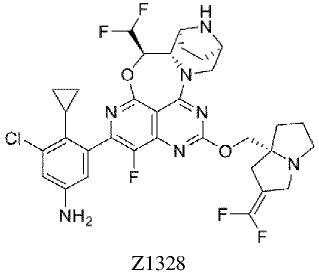 | 690.2 |
| 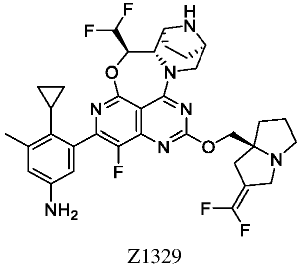 | 670.3 | 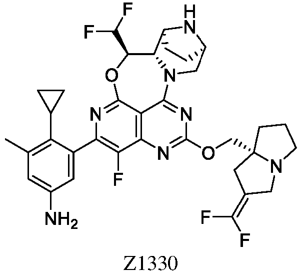 | 670.3 |
| 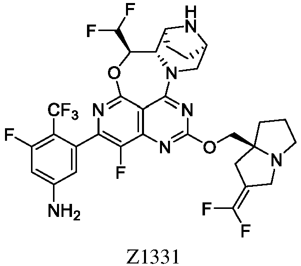 | 702.2 | 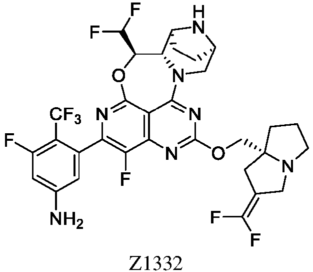 | 702.2 |
| 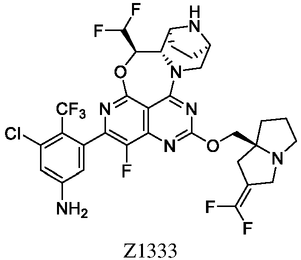 | 718.2 | 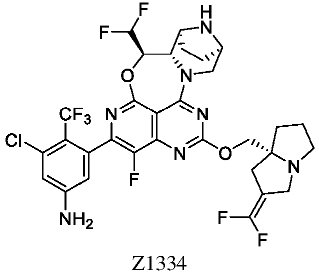 | 718.2 |
| 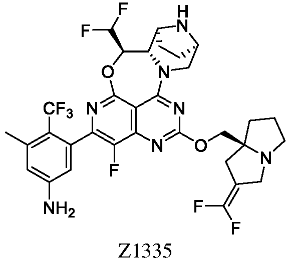 | 698.2 | 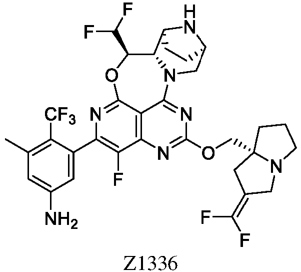 | 698.2 |
| 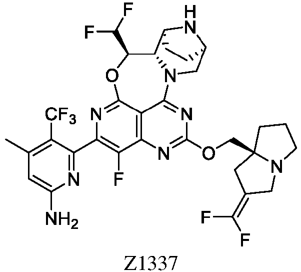 | 699.2 | 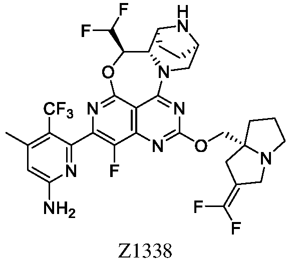 | 699.2 |
| 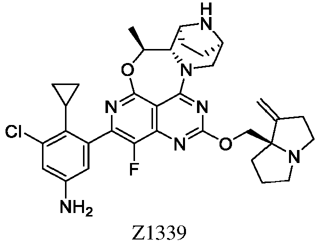 | 618.3 | 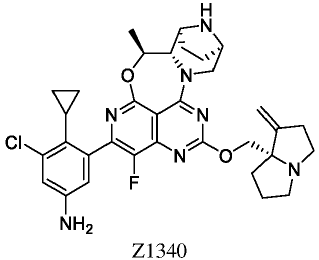 | 618.3 |
| 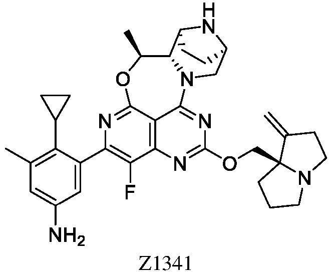 | 598.3 | 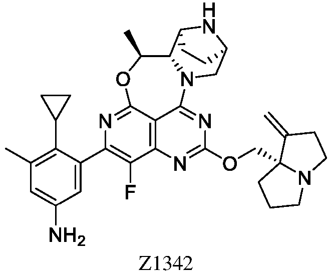 | 598.3 |
| 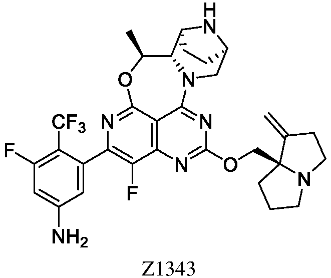 | 630.3 | 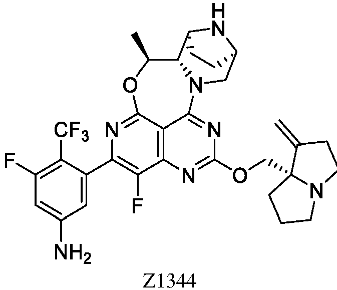 | 630.3 |
| 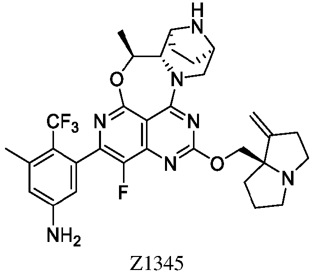 | 626.3 | 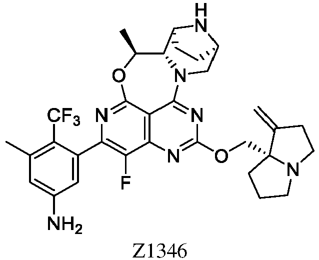 | 626.3 |
| 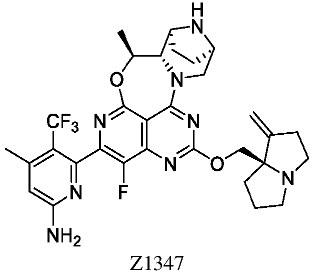 | 627.3 | 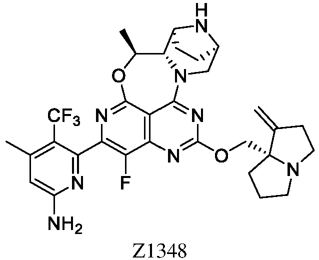 | 627.3 |
| 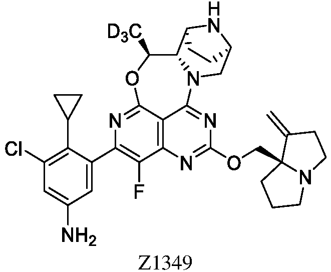 | 621.3 | 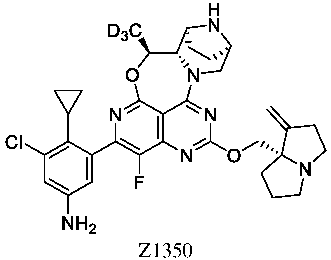 | 621.3 |
| 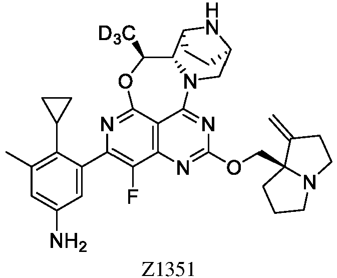 | 601.3 | 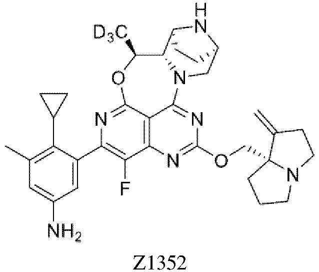 | 601.3 |
| 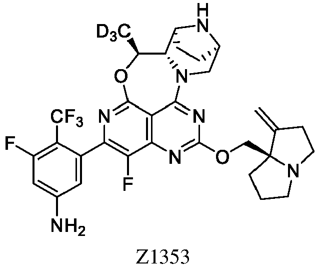 | 633.3 | 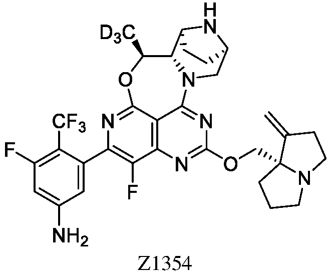 | 633.3 |
| 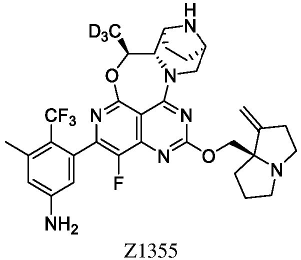 | 629.3 | 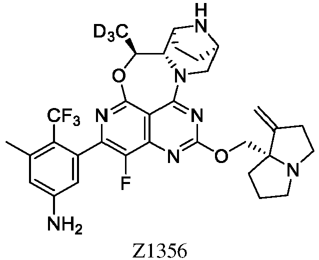 | 629.3 |
| 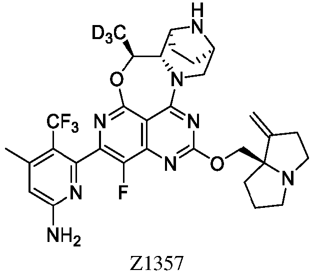 | 630.3 | 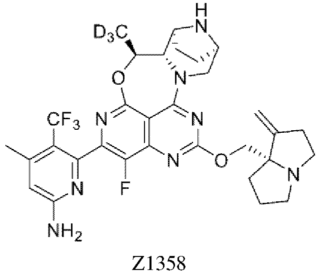 | 630.3 |
| 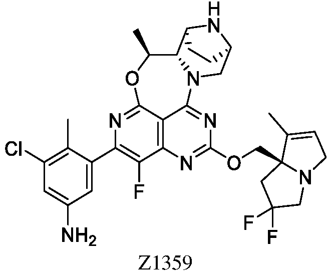 | 628.2 | 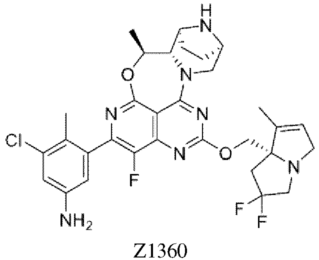 | 628.2 |
| 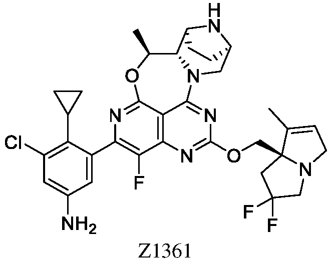 | 654.2 | 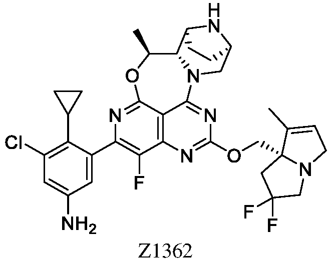 | 654.2 |
| 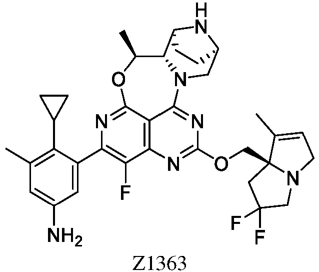 | 634.3 | 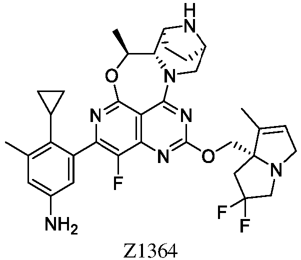 | 634.3 |
| 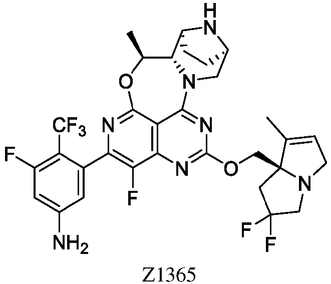 | 666.2 | 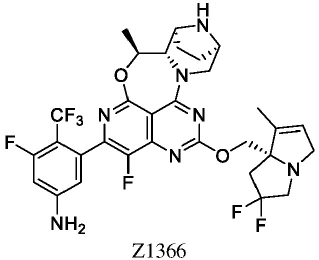 | 666.2 |
| 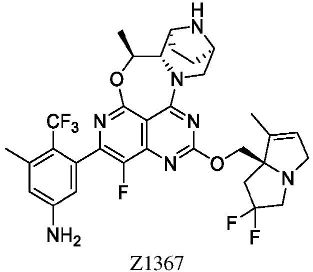 | 662.3 | 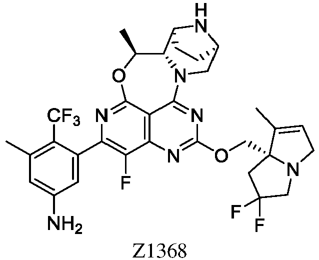 | 662.3 |
| 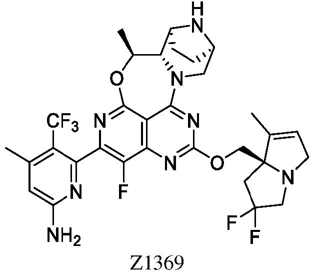 | 663.3 | 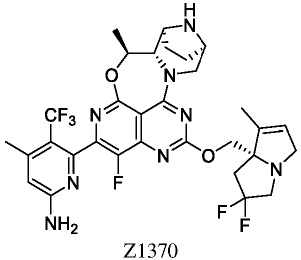 | 663.3 |
| 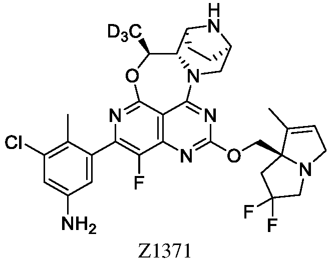 | 631.3 | 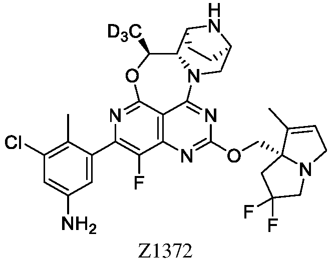 | 631.3 |
| 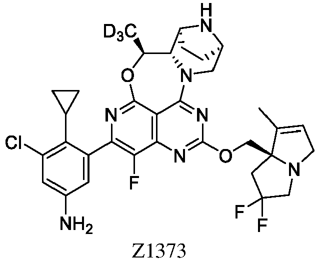 | 657.3 | 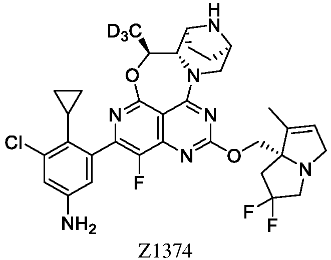 | 657.3 |
| 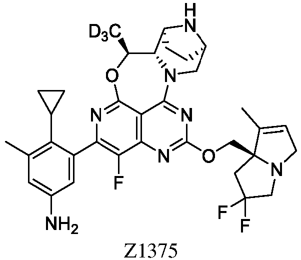 | 637.3 | 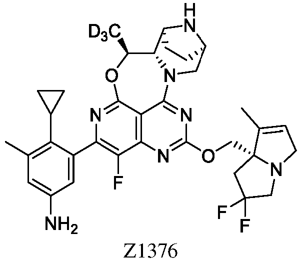 | 637.3 |
| 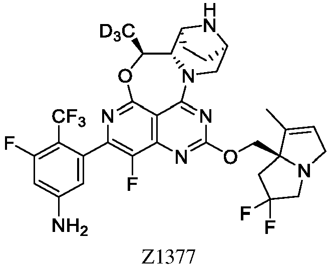 | 669.3 | 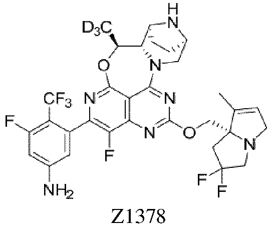 | 669.3 |
| 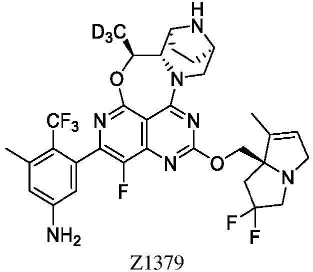 | 665.3 | 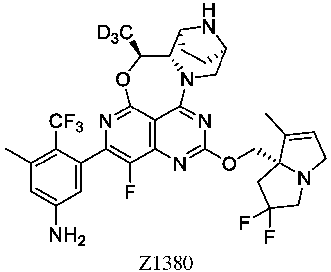 | 665.3 |
| 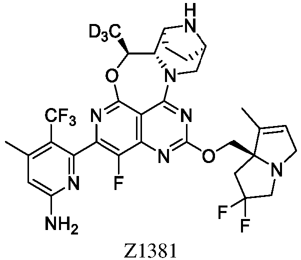 | 666.3 | 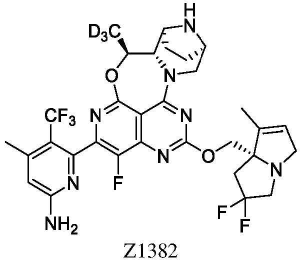 | 666.3 |
| 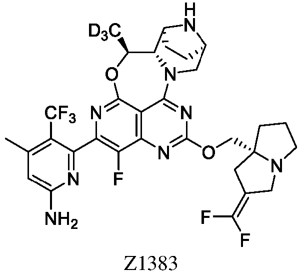 | 666.3 | | |

| | |
|---|---|
| Z748 | ¹H NMR (400 MHz, CD₃OD) δ 8.50-8.35 (m, 1H), 7.68 (t, J = 8.0 Hz, 1H), 7.42-7.35 (m, 1H), 7.32-7.25 (m, 1H), 7.20-7.13 (m, 1H), 7.05-6.96 (m, 1H), 5.54 (s, 1H), 5.43 (d, J = 12.0 Hz, 1H), 4.62-4.50 (m, 1H), 4.48 - 4.37 (m, 2H), 4.14 (d, J = 8.0 Hz, 1H), 4.02 - 3.83 (m, 2H), 3.79-3.68 (m, 1H), 3.52-3.38 (m, 2H), 3.28-3.20 (m, 2H), 3.14-3.05 (m, 1H), 2.96 (d, J = 4.0 Hz, 1H), 2.56 - 2.31 (m, 2H), 2.21-2.12 (m, 1H), 2.02 - 1.86 (m, 3H), 1.83 (s, 3H), 1.57 (t, J =8.0 Hz, 3H). |
| Z749 | ¹H NMR (400 MHz, DMSO-d₆) δ 8.26- 8.17 (m, 2 H), 7.22- 7.55 (m, 3 H), 5.46 (bs, 1 H), 5.19-5.15 (m, 1 H), 4.55- 4.49 (m, 1 H), 4.38- 3.76 (m, 5 H), 3.66- 3.65 (m, 1 H), 3.55- 3.52 (m, 1 H), 3.15- 3.07 (m, 5 H), 1.93- 1.60 (m, 11 H), 1.49- 1.45 (m, 3 H). |

The compounds shown in Table B or C were respectively prepared by changing some raw materials according to the preparative method in the examples above.

**Table B**

| Structure and No. | MS [M+H]⁺ | Structure and No. | MS [M+H]⁺ | Structure and No. | MS [M+H]⁺ |
|---|---|---|---|---|---|
| 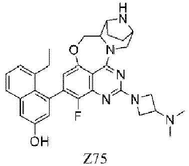 | 555.3 | 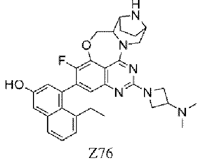 | 555.3 | 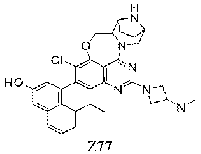 | 571.3 |
| 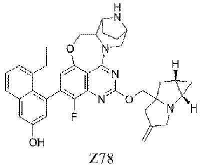 | 620.3 | 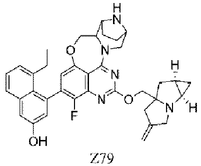 | 620.3 | 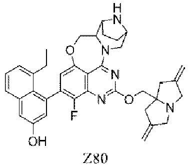 | 620.3 |
| 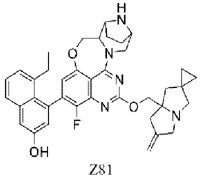 | 634.3 | 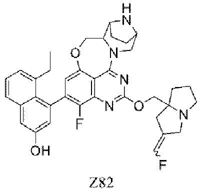 | 626.3 | 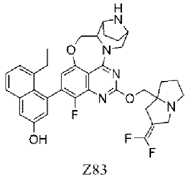 | 644.3 |
| 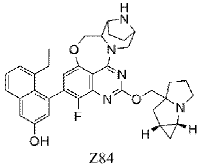 | 608.3 | 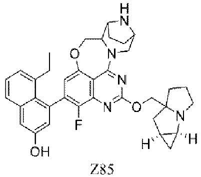 | 608.3 | 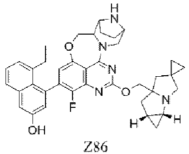 | 634.3 |
| 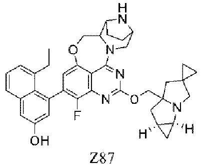 | 634.3 | 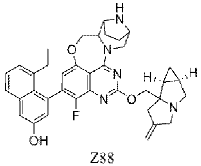 | 620.3 | 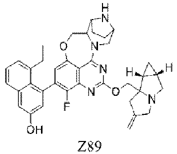 | 620.3 |
| 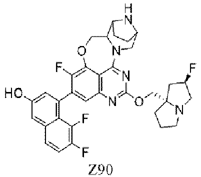 | 622.2 | 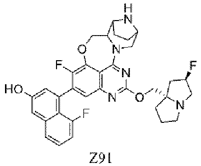 | 604.3 | 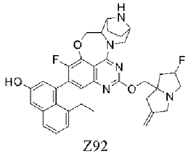 | 626.3 |
| 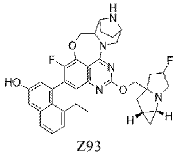 | 626.3 | 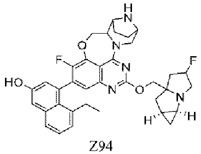 | 626.3 | 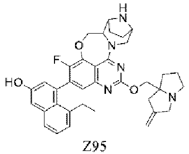 | 608.3 |
| 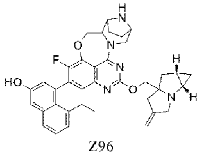 | 620.3 | 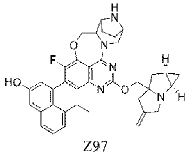 | 620.3 | 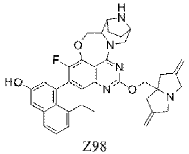 | 620.3 |
| 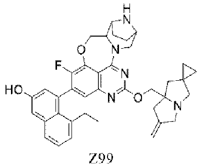 | 634.3 | 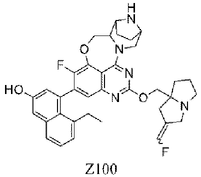 | 626.3 | 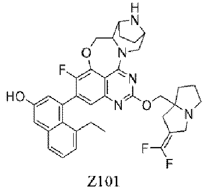 | 644.3 |
| 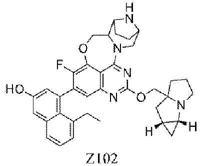 | 608.3 | 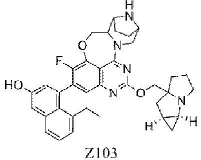 | 608.3 | 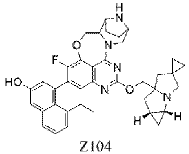 | 634.3 |
| 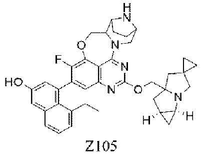 | 634.3 | 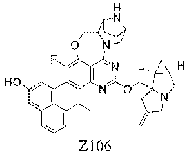 | 620.3 | 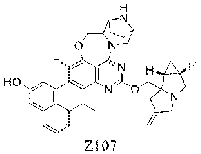 | 620.3 |
| 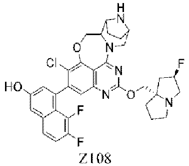 | 638.2 | 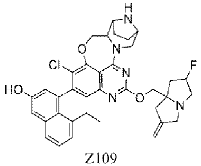 | 642.3 | 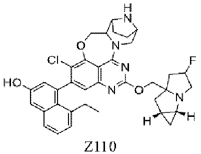 | 642.3 |
| 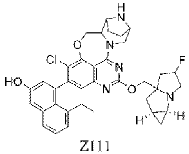 | 642.3 | 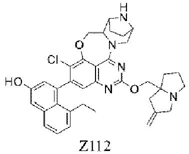 | 624.3 | 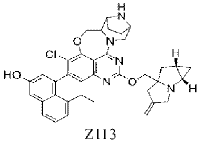 | 636.3 |
| 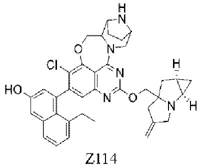 | 636.3 | 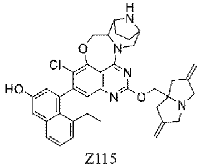 | 636.3 | 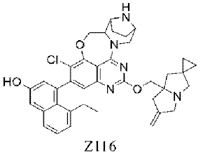 | 650.3 |
| 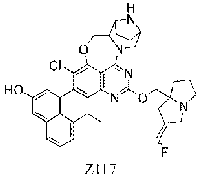 | 642.3 | 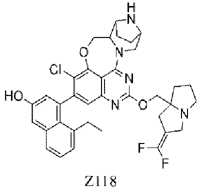 | 660.3 | 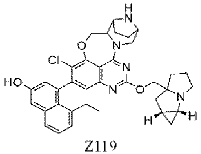 | 624.4 |
| 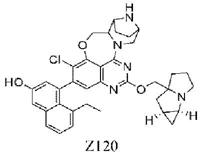 | 624.4 | 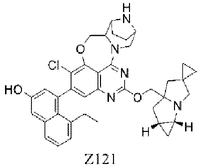 | 650.2 | 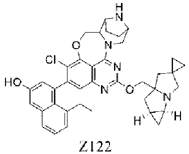 | 650.3 |
| 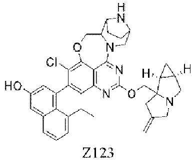 | 636.3 | 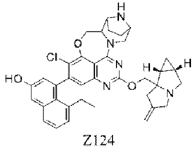 | 636.3 | 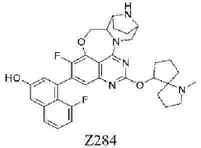 | 600.3 |
| 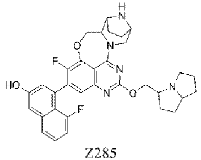 | 586.3 | 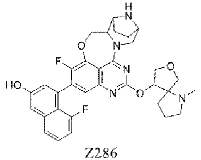 | 602.3 | 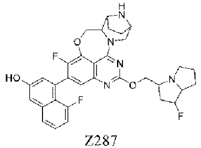 | 604.3 |
| 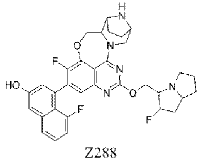 | 604.3 | 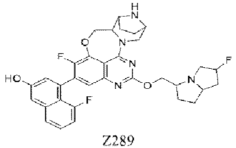 | 604.3 | 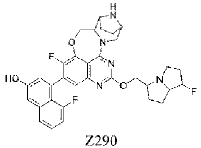 | 604.3 |
| 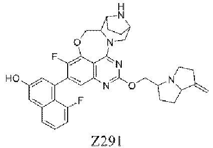 | 598.3 | 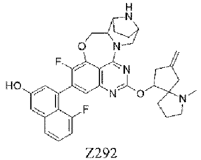 | 612.3 | 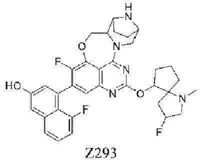 | 618.3 |
| 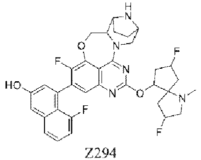 | 636.3 | 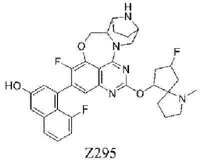 | 618.3 | 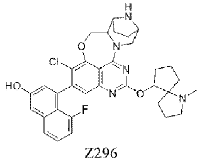 | 616.2 |
| 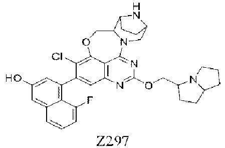 | 602.2 | 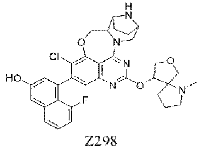 | 618.2 | 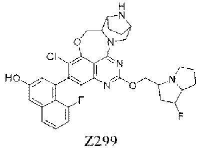 | 620.2 |
| 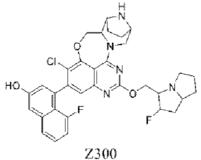 | 620.2 | 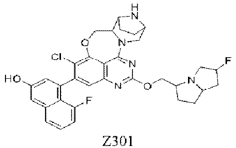 | 620.2 | 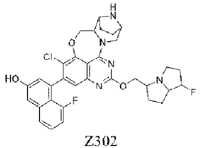 | 620.2 |
| 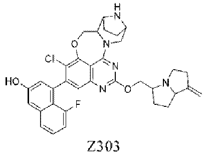 | 614.2 | 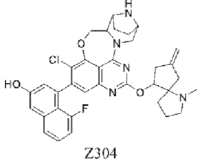 | 628.2 | 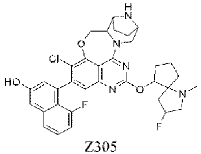 | 634.2 |
| 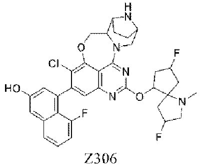 | 652.2 | 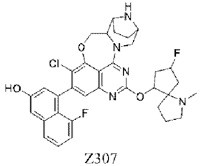 | 634.2 | 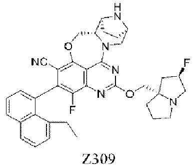 | 623.3 |
| 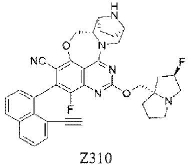 | 619.3 | 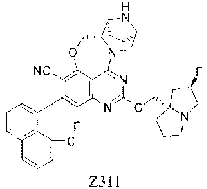 | 629.2 | 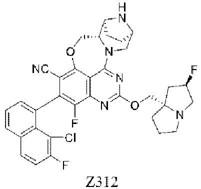 | 647.2 |
| 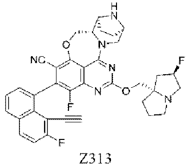 | 637.3 | 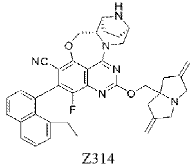 | 629.3 | 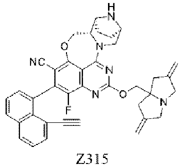 | 625.3 |
| 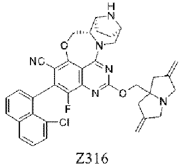 | 635.2 | 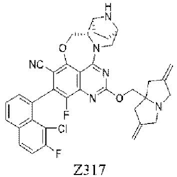 | 653.2 | 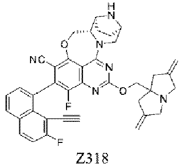 | 643.3 |
| 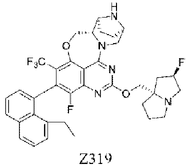 | 666.3 | 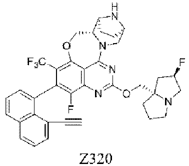 | 662.3 | 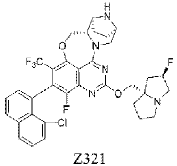 | 672.2 |
| 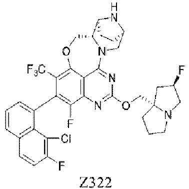 | 690.2 | 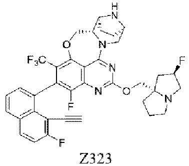 | 680.2 | 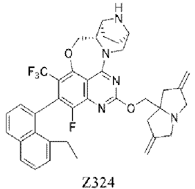 | 672.3 |
| 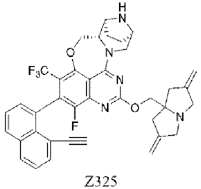 | 668.3 | 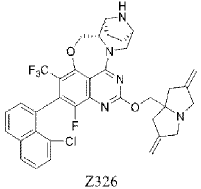 | 678.2 | 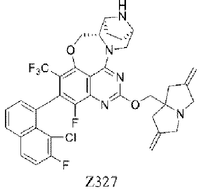 | 696.2 |
| 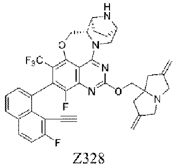 | 686.3 | 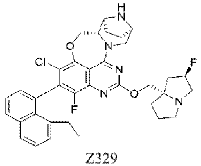 | 632.3 | 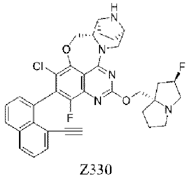 | 628.2 |
| 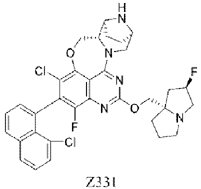 | 638.2 | 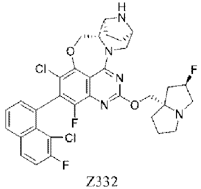 | 656.2 | 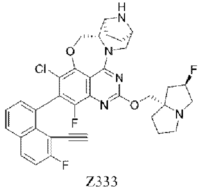 | 646.2 |
| 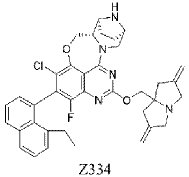 | 638.3 | 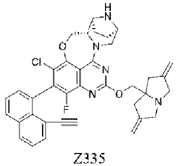 | 634.2 | 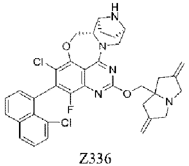 | 644.2 |
| 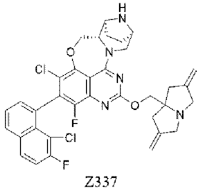 | 662.2 | 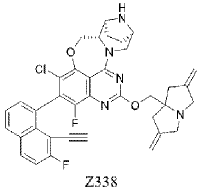 | 652.2 | 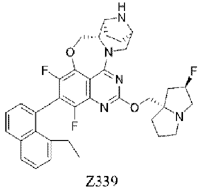 | 616.3 |
| 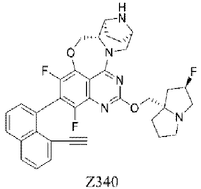 | 612.3 | 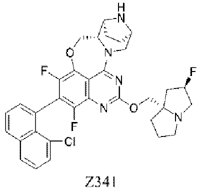 | 622.2 | 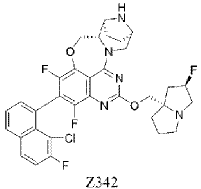 | 640.2 |
| 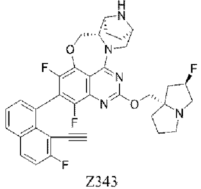 | 630.2 | 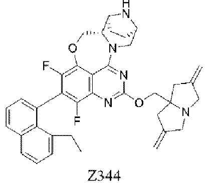 | 622.3 | 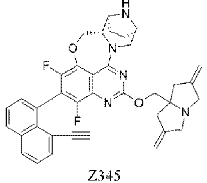 | 618.3 |
| 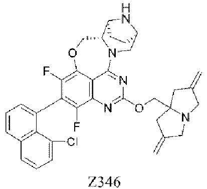 | 628.2 | 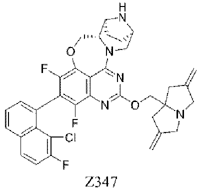 | 646.2 | 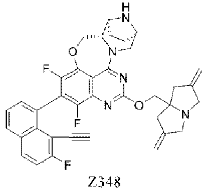 | 636.3 |
| 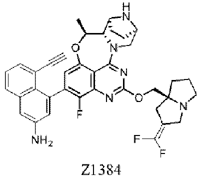 | 653.3 | 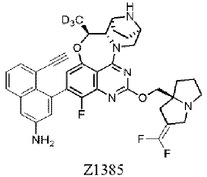 | 656.3 | 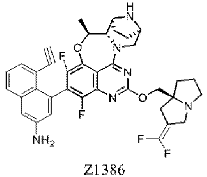 | 671.3 |
| 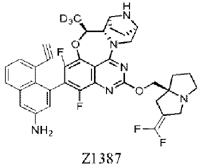 | 674.3 | 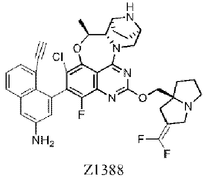 | 687.2 | 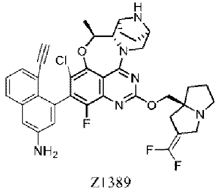 | 690.2 |

**Table C**

| Structure and No. | MS [M+H]⁺ | Structure and No. | MS [M+H]⁺ | Structure and No. | MS [M+H]⁺ |
|---|---|---|---|---|---|
| 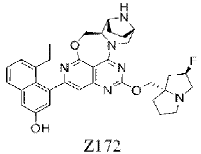 | 597.3 | 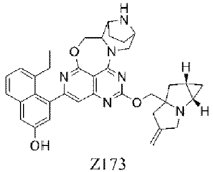 | 603.3 | 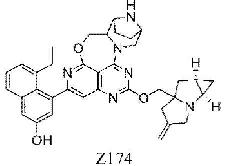 | 603.3 |
| 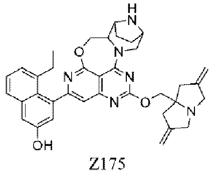 | 603.3 | 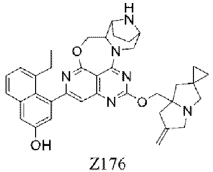 | 617.3 | 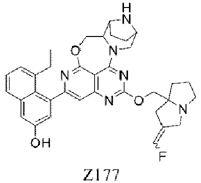 | 609.3 |
| 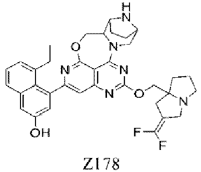 | 627.3 | 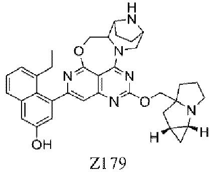 | 591.3 | 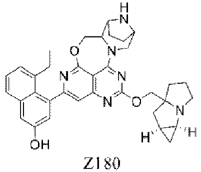 | 591.3 |
| 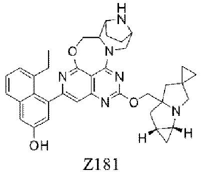 | 617.3 | 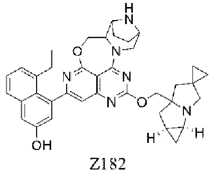 | 617.3 | 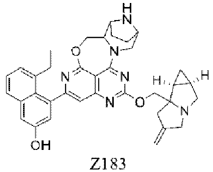 | 603.3 |
| 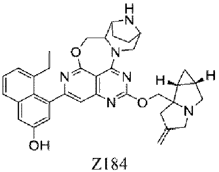 | 603.3 | 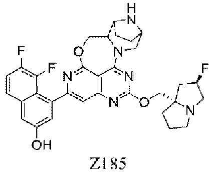 | 605.2 | 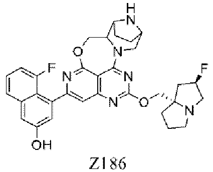 | 587.3 |
| 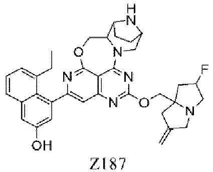 | 609.3 | 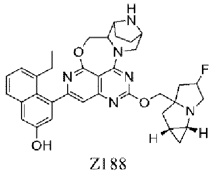 | 609.3 | 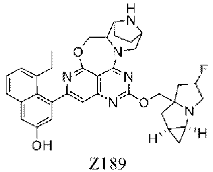 | 609.3 |
| 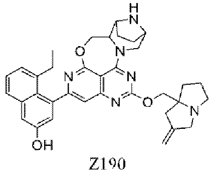 | 591.3 | 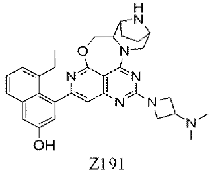 | 538.3 | 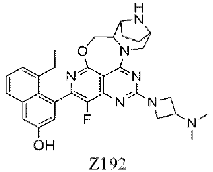 | 556.3 |
| 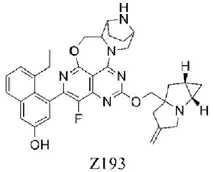 | 621.3 | 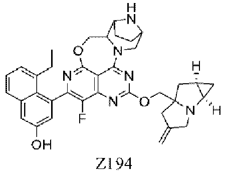 | 621.3 | 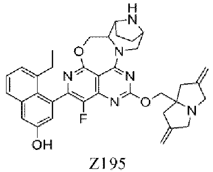 | 621.3 |
| 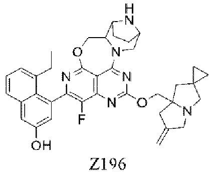 | 635.3 | 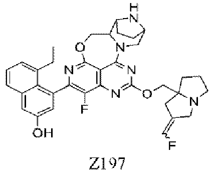 | 637.3 | 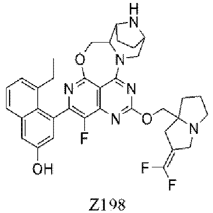 | 645.3 |
| 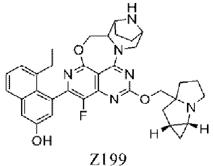 | 609.3 | 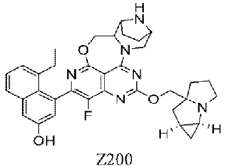 | 609.3 | 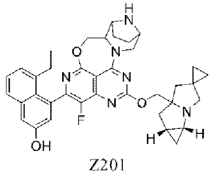 | 635.3 |
| 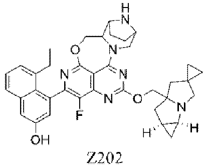 | 635.3 | 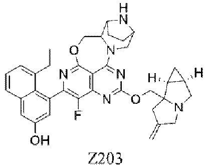 | 621.3 | 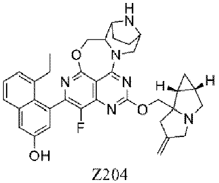 | 621.3 |
| 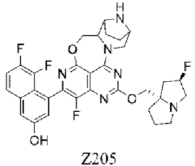 | 623.2 | 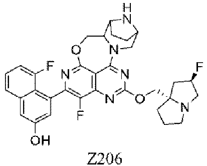 | 605.2 | 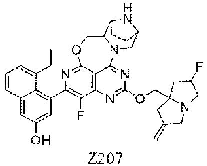 | 627.3 |
| 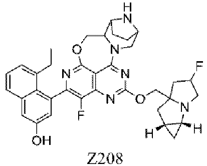 | 627.3 | 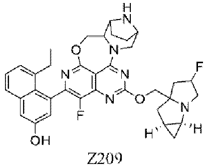 | 627.3 | 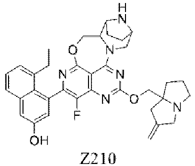 | 609.3 |
| 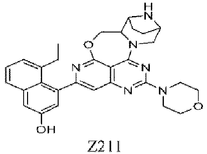 | *525.3* | 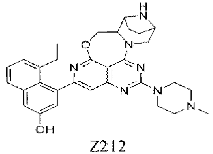 | 538.3 | 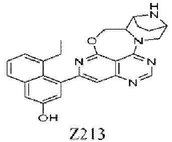 | 440.2 |
| 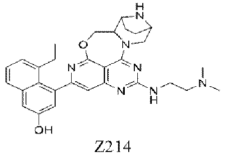 | 526.3 | 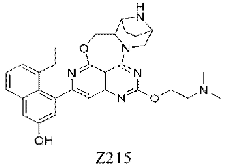 | 527.3 | 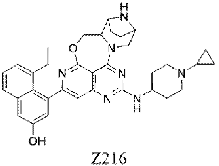 | 578.3 |
| 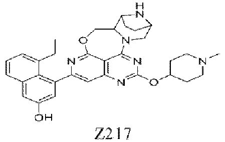 | 553.3 | 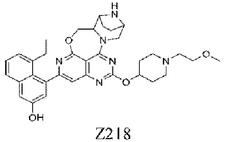 | 597.3 | 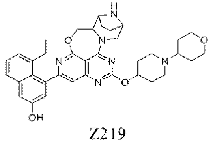 | 623.3 |
| 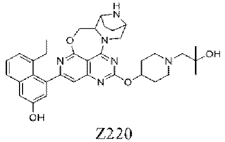 | 611.3 | 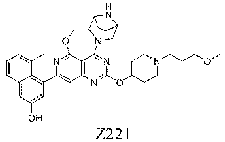 | 611.3 | 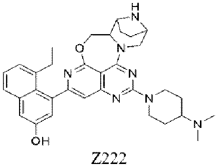 | 566.3 |
| 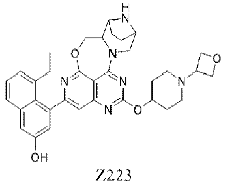 | 595.3 | 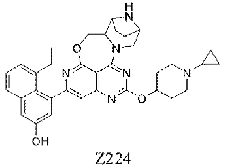 | 579.3 | 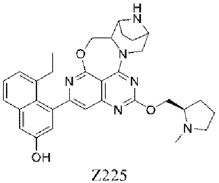 | 553.3 |
| 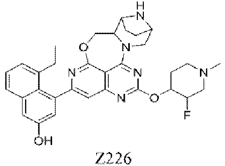 | 571.3 | 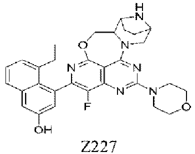 | 543.2 | 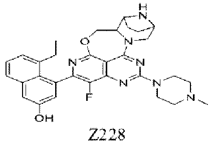 | 556.3 |
| 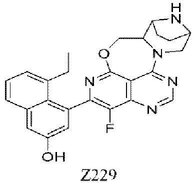 | 458.2 | 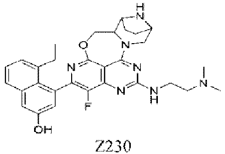 | 544.3 | 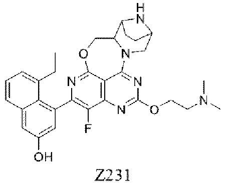 | 545.3 |
| 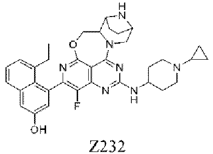 | 596.3 | 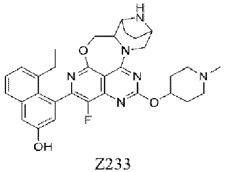 | 571.3 | 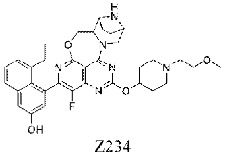 | 615.3 |
| 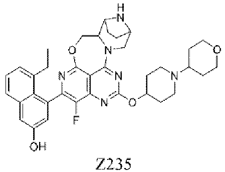 | 641.3 | 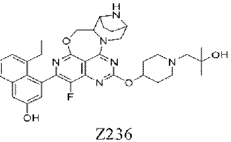 | 629.3 | 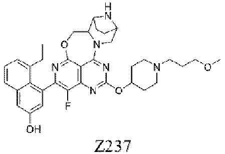 | 629.3 |
| 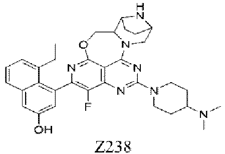 | 584.3 | 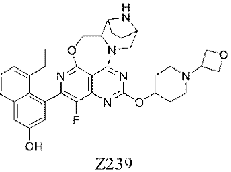 | 613.3 | 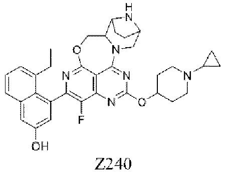 | 597.3 |
| 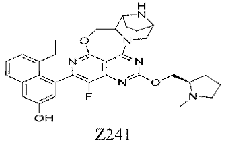 | 571.3 | 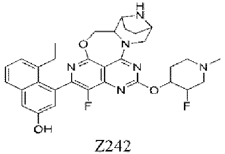 | 589.3 | 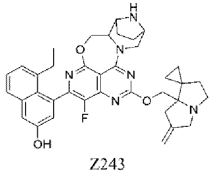 | 635.1 |
| 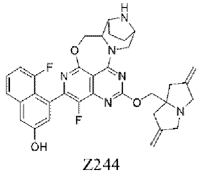 | 611.3 | 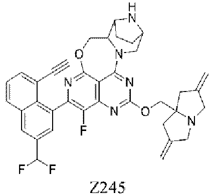 | 651.3 | 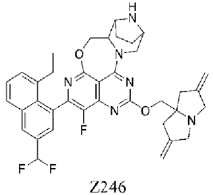 | 655.3 |
| 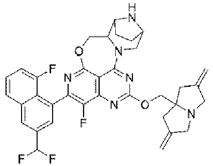 | 645.3 | 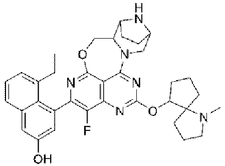 | 611.3 | 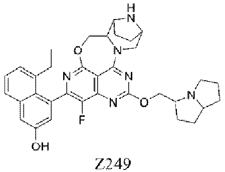 | 597.3 |
| 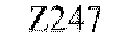 | | 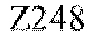 | | | |
| 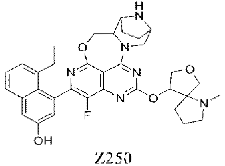 | 613.3 | 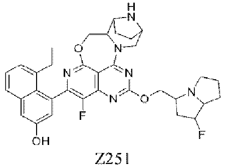 | 615.3 | 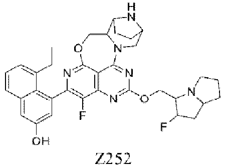 | 615.3 |
| 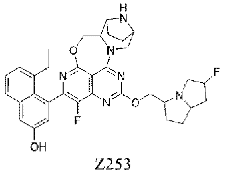 | 615.3 | 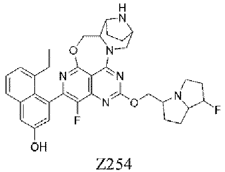 | 615.3 | 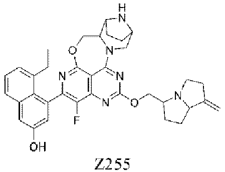 | 609.3 |
| 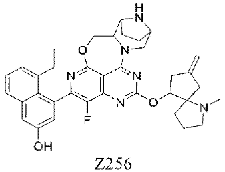 | 623.3 | 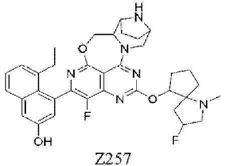 | 629.3 | 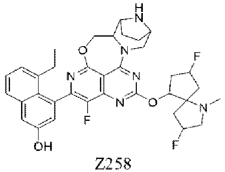 | 647.3 |
| 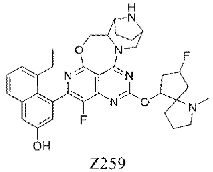 | 629.3 | 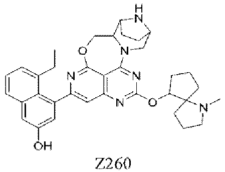 | 593.3 | 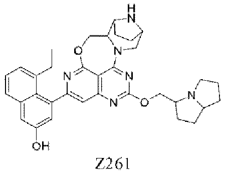 | 579.3 |
| 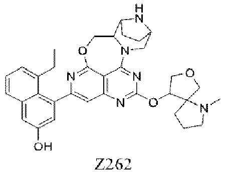 | 595.3 | 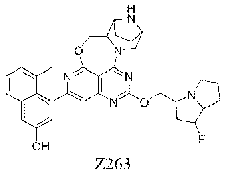 | 597.3 | 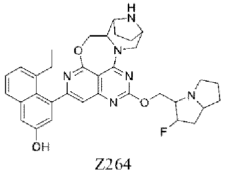 | 597.3 |
| 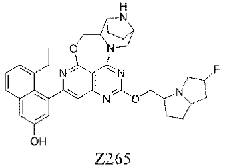 | 597.3 | 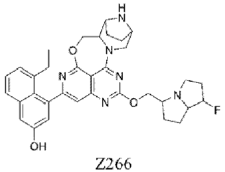 | 597.3 | 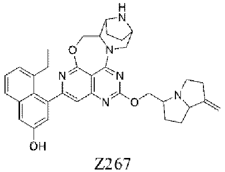 | 591.3 |
| 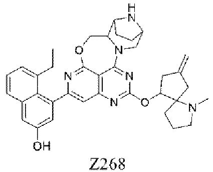 | 605.3 | 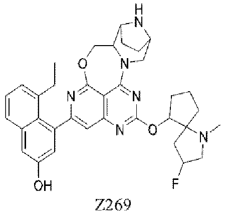 | 611.3 | 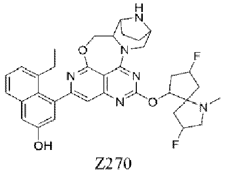 | 629.3 |
| 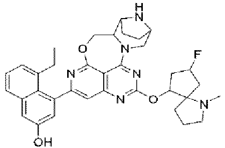 | 611.3 | 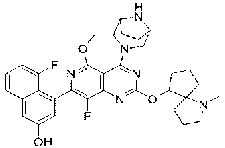 | 601.3 | 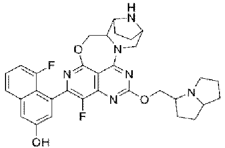 | 587.3 |
| 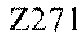 | | 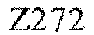 | | 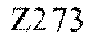 | |
| 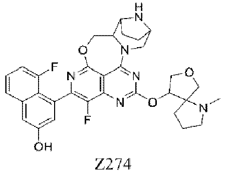 | 603.3 | 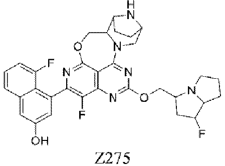 | 605.2 | 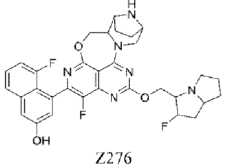 | 605.2 |
| 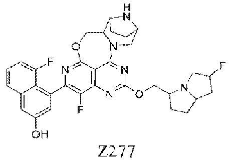 | 605.2 | 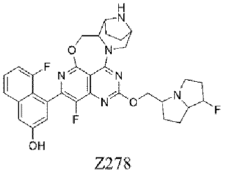 | 605.2 | 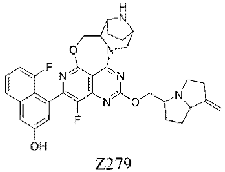 | 599.3 |
| 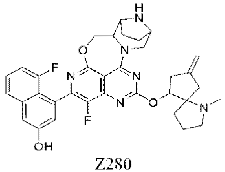 | 613.3 | 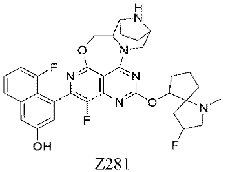 | 619.3 | 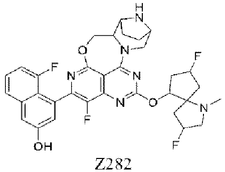 | 637.3 |
| 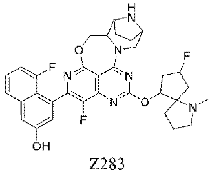 | 619.3 | 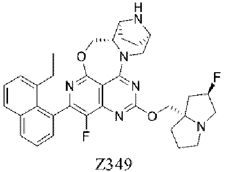 | 599.3 | 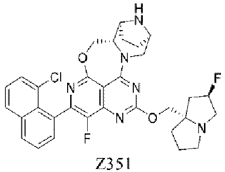 | 605.2 |
| 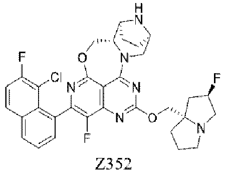 | 623.2 | 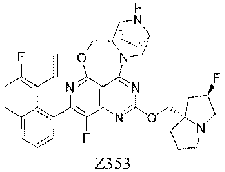 | 613.3 | 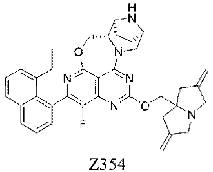 | 605.3 |
| 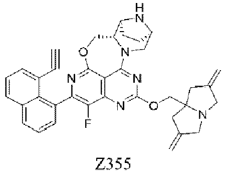 | 601.3 | 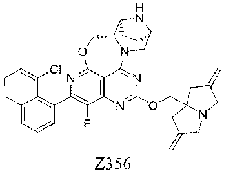 | 611.2 | 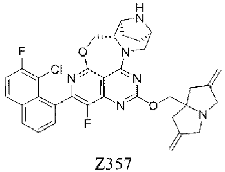 | 629.2 |
| 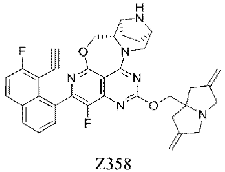 | 619.3 | 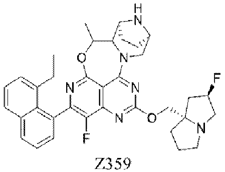 | 613.3 | 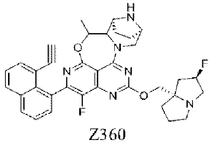 | 609.3 |
| 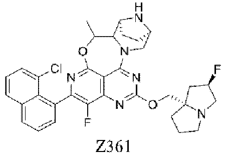 | 619.2 | 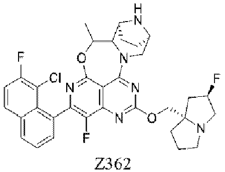 | 637.2 | 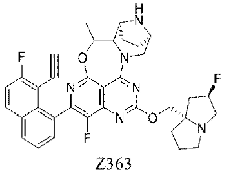 | 627.3 |
| 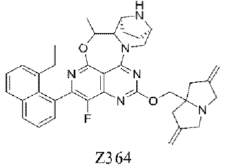 | 619.3 | 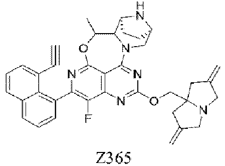 | 615.3 | 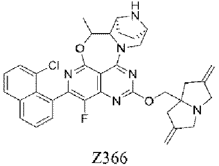 | 625.2 |
| 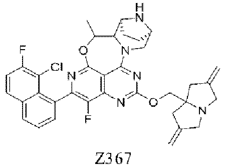 | 643.2 | 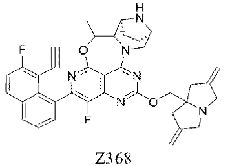 | 633.3 | 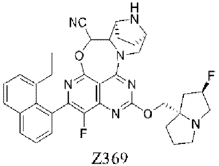 | 624.3 |
| 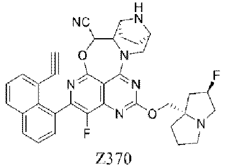 | 620.3 | 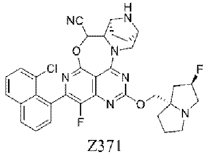 | 630.2 | 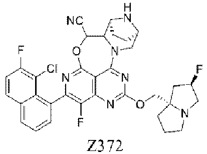 | 648.2 |
| 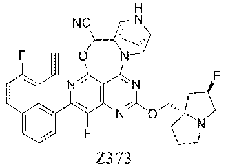 | 638.2 | 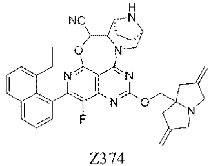 | 630.3 | 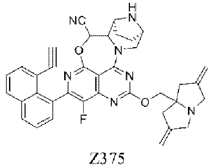 | 626.3 |
| 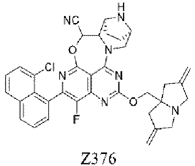 | 636.2 | 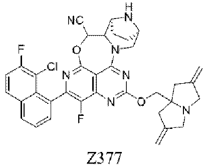 | 654.2 | 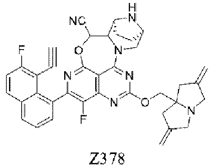 | 644.3 |
| 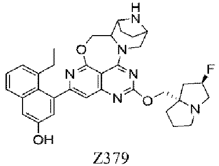 | 597.3 | 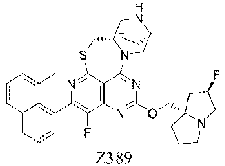 | 615.3 | 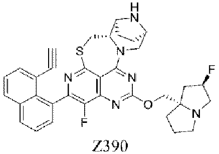 | 611.2 |
| 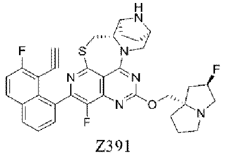 | 629.2 | 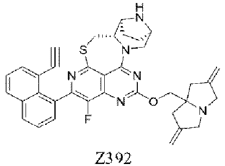 | 617.2 | 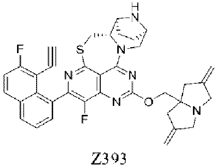 | 635.2 |
| 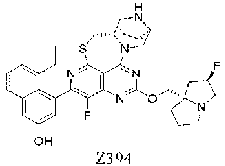 | 631.3 | 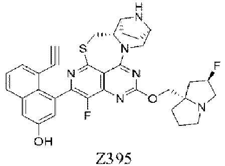 | 627.2 | 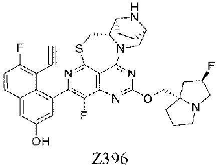 | 645.2 |
| 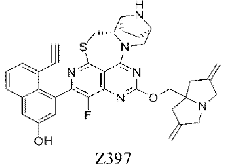 | 633.2 | 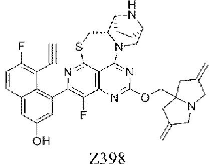 | 651.2 | 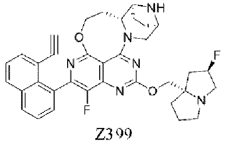 | 609.3 |
| 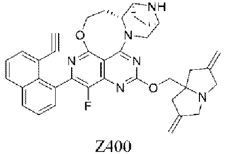 | 615.3 | 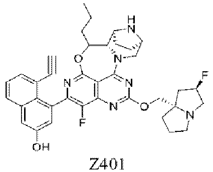 | 653.3 | 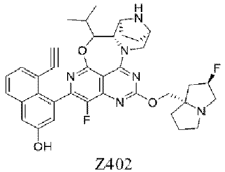 | 653.3 |
| 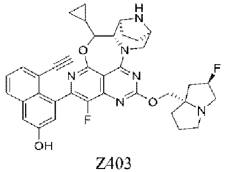 | 651.3 | 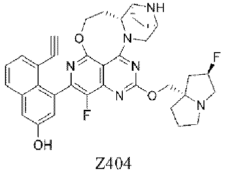 | 625.3 | 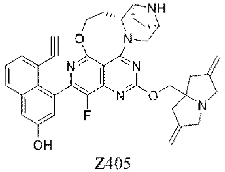 | 631.3 |
| 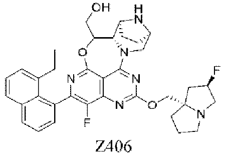 | 629.3 | 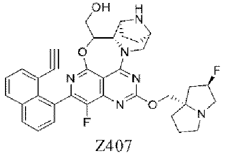 | 625.3 | 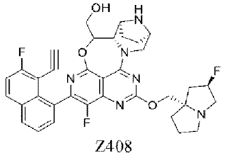 | 643.3 |
| 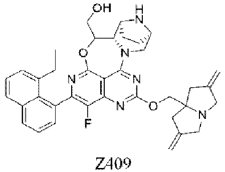 | 635.3 | 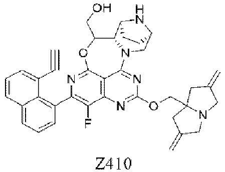 | 631.3 | 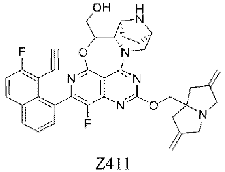 | 649.3 |
| 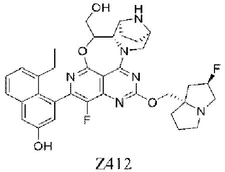 | 645.3 | 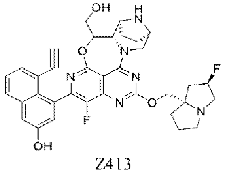 | 641.3 | 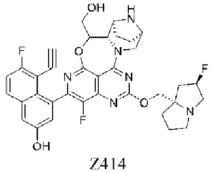 | 659.3 |
| 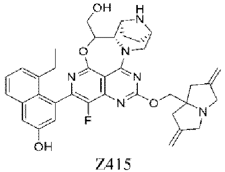 | 651.3 | 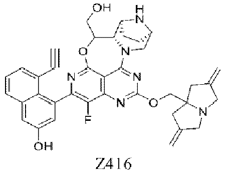 | 647.3 | 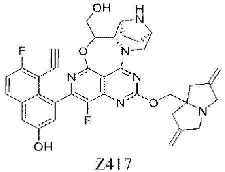 | 664.3 |
| 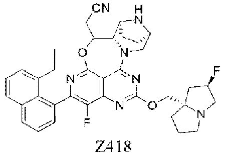 | 638.3 | 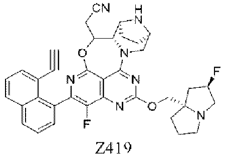 | 634.3 | 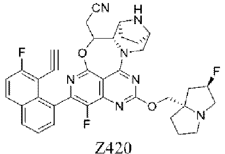 | 652.3 |
| 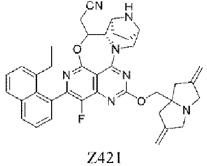 | 644.3 | 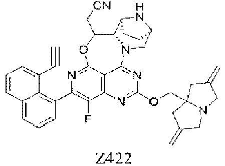 | 640.3 | 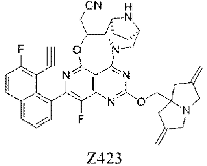 | 658.3 |
| 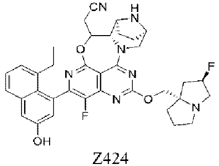 | 654.3 | 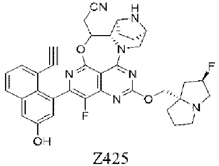 | 650.3 | 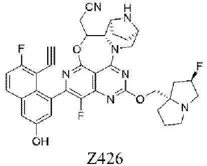 | 668.3 |
| 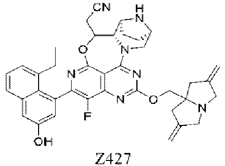 | 660.3 | 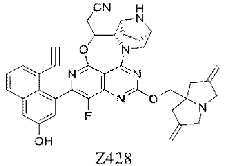 | 656.3 | 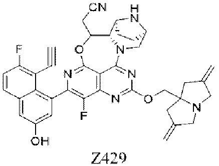 | 674.3 |
| 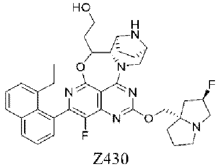 | 643.3 | 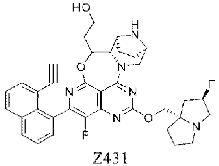 | 639.3 | 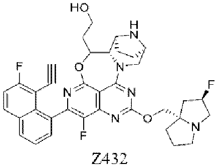 | 657.3 |
| 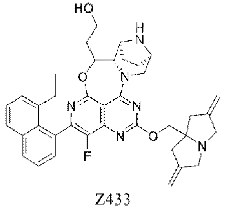 | 649.3 | 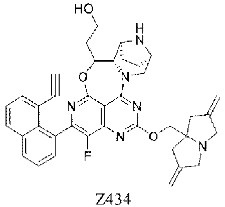 | 645.3 | 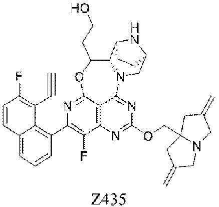 | 663.3 |
| 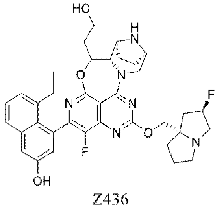 | 659.3 | 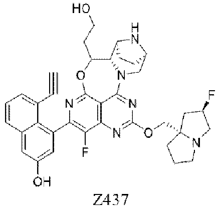 | 655.3 | 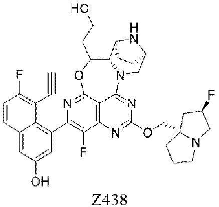 | 673.3 |
| 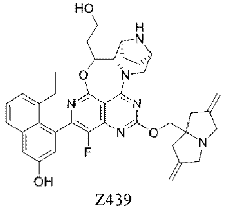 | 665.3 | 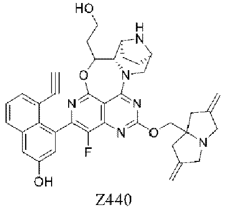 | 661.3 | 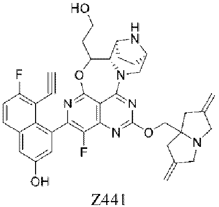 | 679.3 |
| 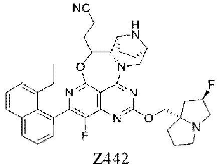 | 652.3 | 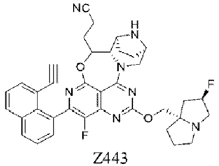 | 648.3 | 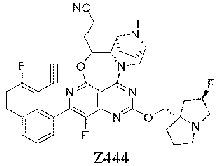 | 666.3 |
| 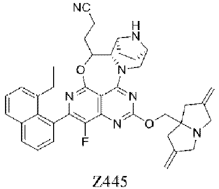 | 658.3 | 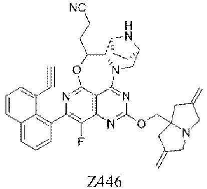 | 654.3 | 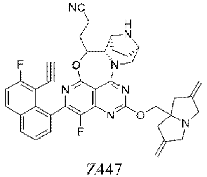 | 672.3 |
| 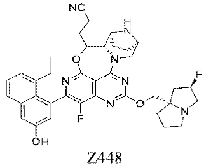 | 668.3 | 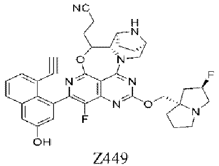 | 664.3 | 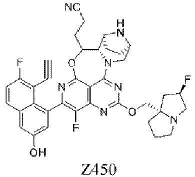 | 682.3 |
| 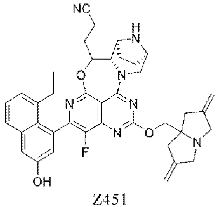 | 674.3 | 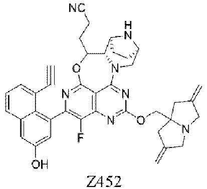 | 670.3 | 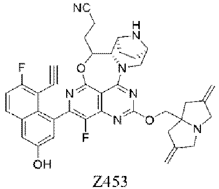 | 668.3 |
| 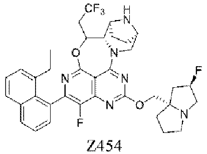 | 681.3 | 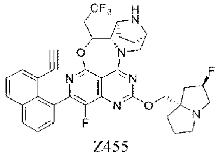 | 677.3 | 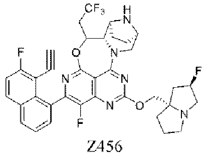 | 695.2 |
| 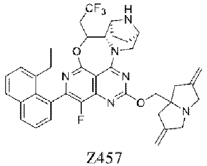 | 687.3 | 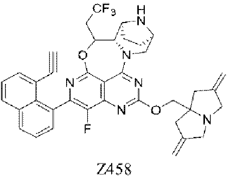 | 683.3 | 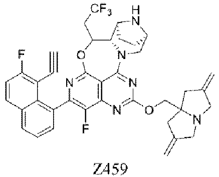 | 701.3 |
| 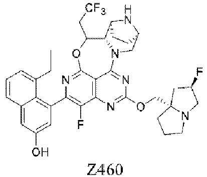 | 697.3 | 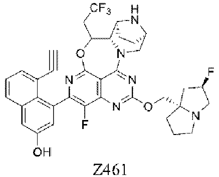 | 693.3 | 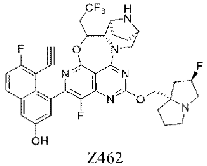 | 711.2 |
| 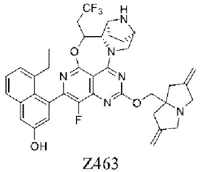 | 703.3 | 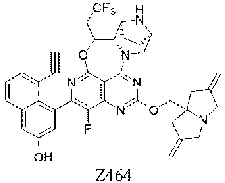 | 699.3 | 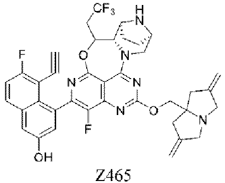 | 717.3 |
| 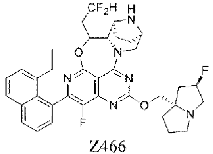 | 663.3 | 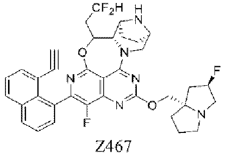 | 659.3 | 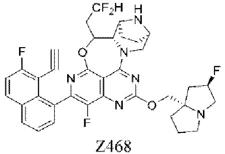 | 677.3 |
| 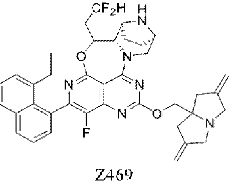 | 669.3 | 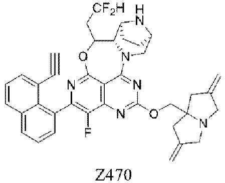 | 665.3 | 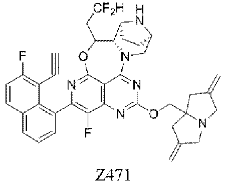 | 683.3 |
| 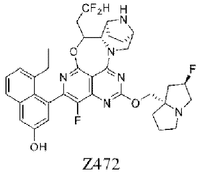 | 679.3 | 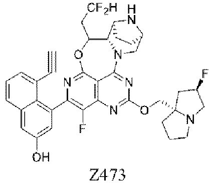 | 675.3 | 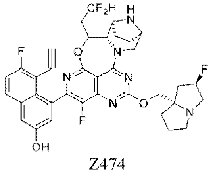 | 693.3 |
| 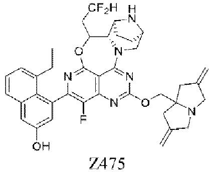 | 685.3 | 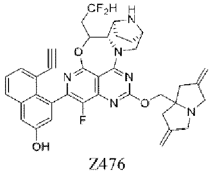 | 681.3 | 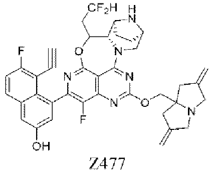 | 699.3 |
| 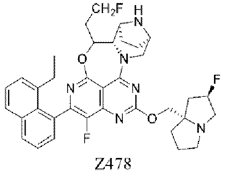 | 645.3 | 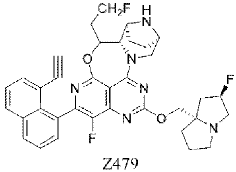 | 641.3 | 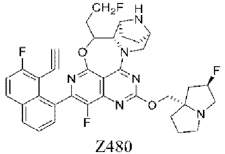 | 659.3 |
| 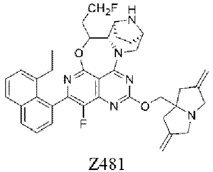 | 651.3 | 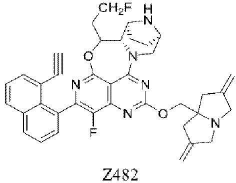 | 647.3 | 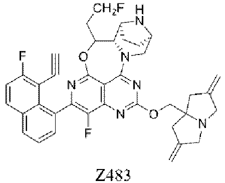 | 665.3 |
| 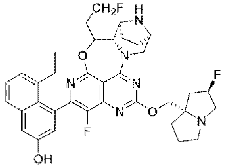 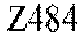 | 661.3 | 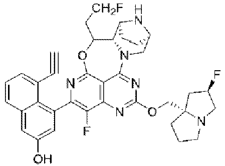 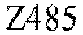 | 657.3 | 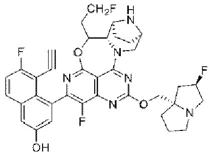 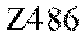 | 675.3 |
| 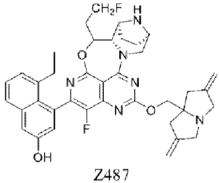 | 667.3 | 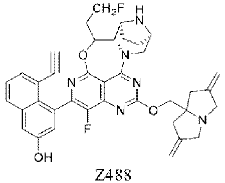 | 663.3 | 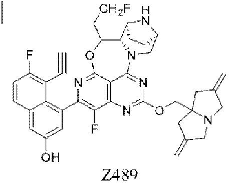 | 681.3 |
| 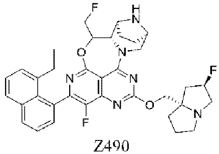 | 631.3 | 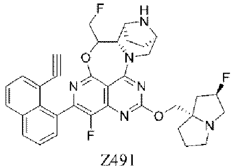 | 627.3 | 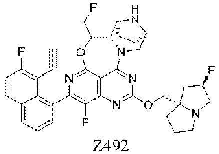 | 645.3 |
| 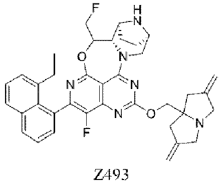 | 637.3 | 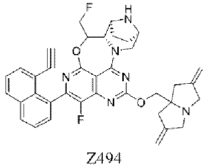 | 633.3 | 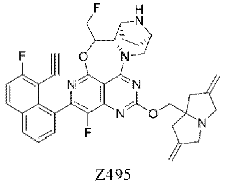 | 651.3 |
| 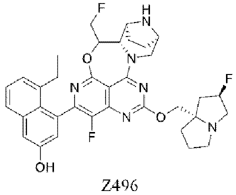 | 647.3 | 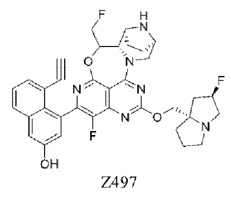 | 643.3 | 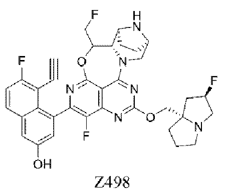 | 661.3 |
| 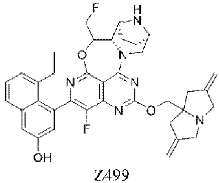 | 653.3 | 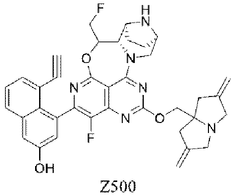 | 649.3 | 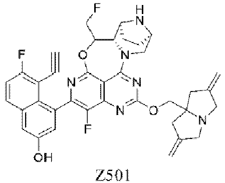 | 667.3 |
| 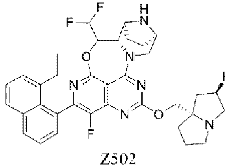 | 649.3 | 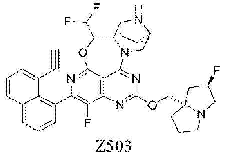 | 645.3 | 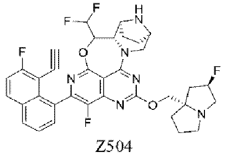 | 663.2 |
| 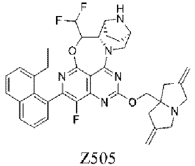 | 655.3 | 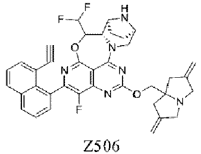 | 651.3 | 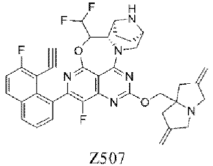 | 669.3 |
| 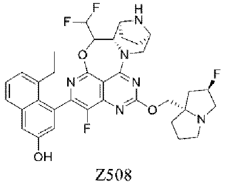 | 665.3 | 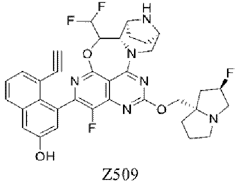 | 661.2 | 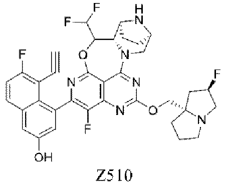 | 679.2 |
| 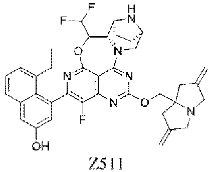 | 671.3 | 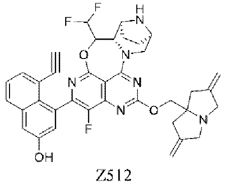 | 667.3 | 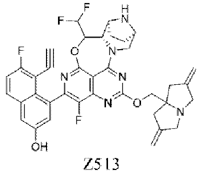 | 685.2 |
| 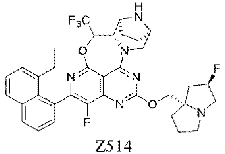 | 667.3 | 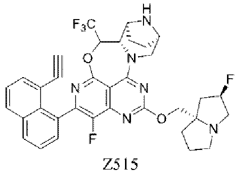 | 663.2 | 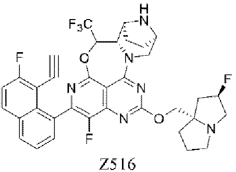 | 681.2 |
| 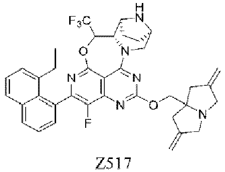 | 673.3 | 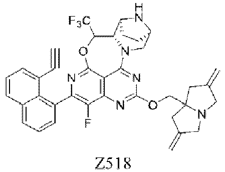 | 669.3 | 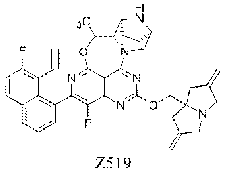 | 687.2 |
| 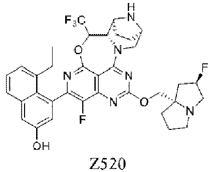 | 683.3 | 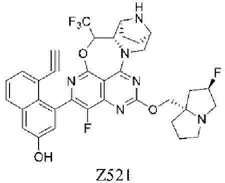 | 679.2 | 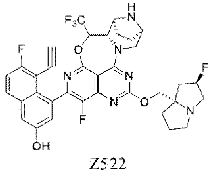 | 697.2 |
| 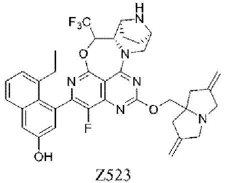 | 689.3 | 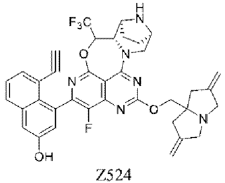 | 685.2 | 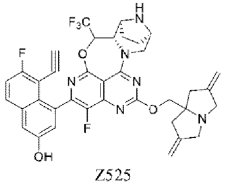 | 703.2 |
| 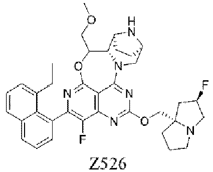 | 643.3 | 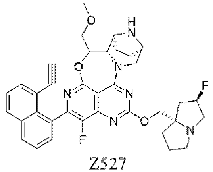 | 639.3 | 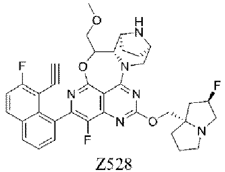 | 657.3 |
| 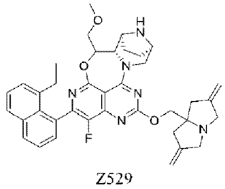 | 649.3 | 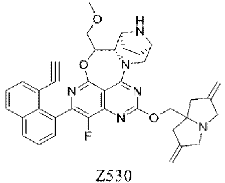 | 645.3 | 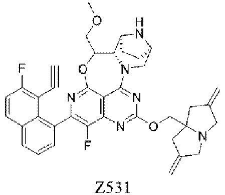 | 663.3 |
| 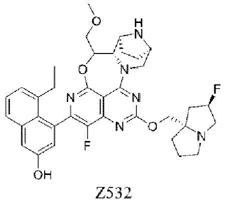 | 659.3 | 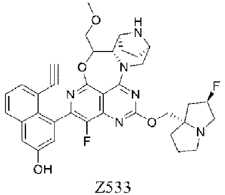 | 655.3 | 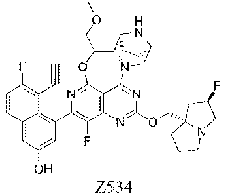 | 673.3 |
| 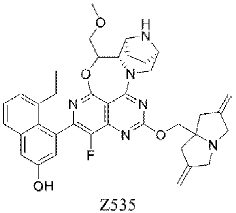 | 665.3 | 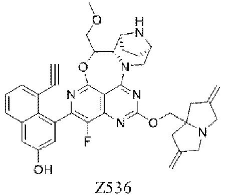 | 661.3 | 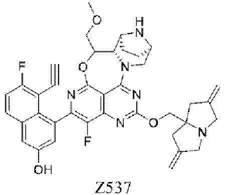 | 679.3 |
| 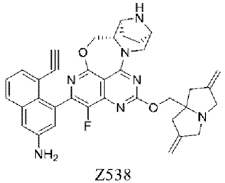 | 616.3 | 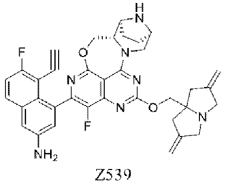 | 634.3 | 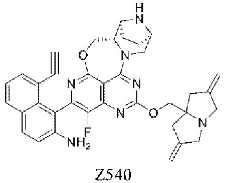 | 616.3 |
| 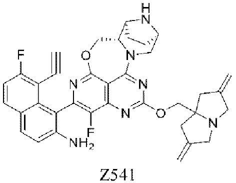 | 643.3 | 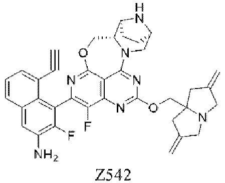 | 634.3 | 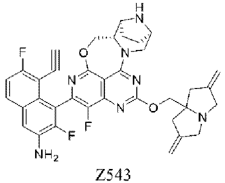 | 652.3 |
| 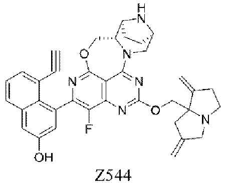 | 617.3 | 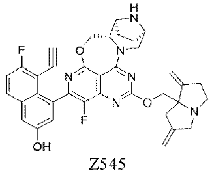 | 634.3 | 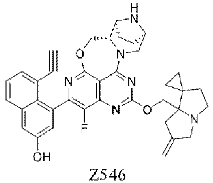 | 631.3 |
| 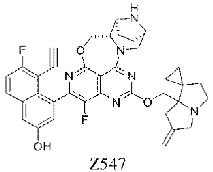 | 649.3 | 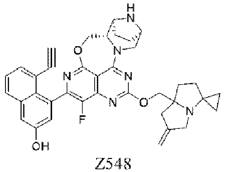 | 631.3 | 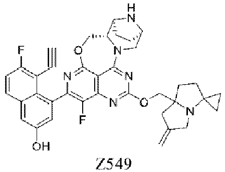 | 649.3 |
| 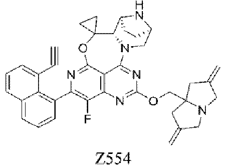 | 627.3 | 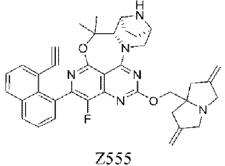 | 629.3 | 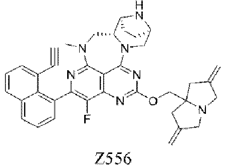 | 614.3 |
| 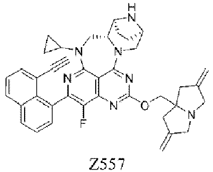 | 640.3 | 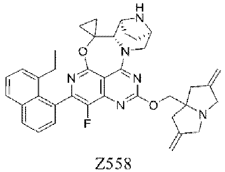 | 631.3 | 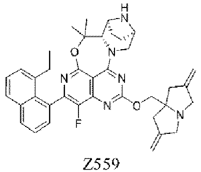 | 633.3 |
| 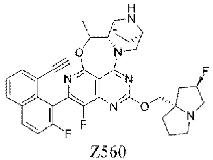 | 627.3 | 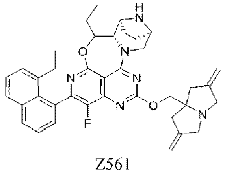 | 633.3 | 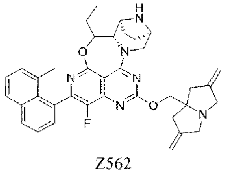 | 619.3 |
| 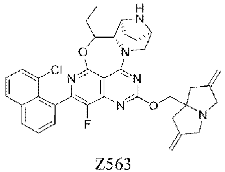 | 639.3 | 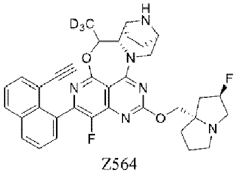 | 612.3 | 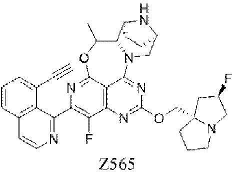 | 610.3 |
| 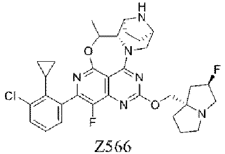 | 609.2 | 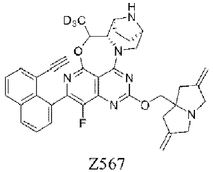 | 618.3 | 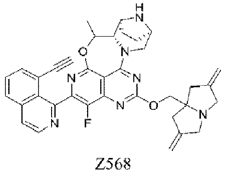 | 616.3 |
| 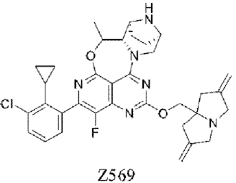 | 615.3 | 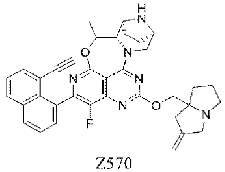 | 603.3 | 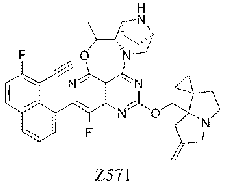 | 647.3 |
| 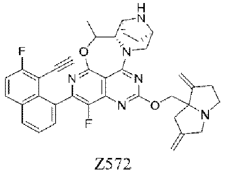 | 633.3 | 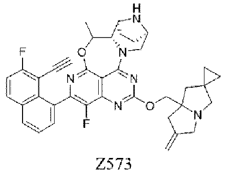 | 647.3 | 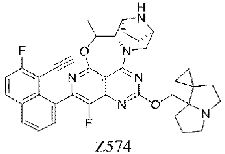 | 635.3 |
| 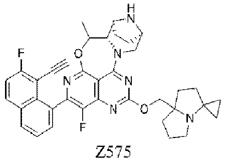 | 635.3 | 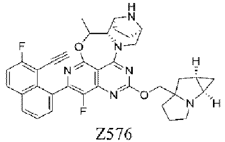 | 621.3 | 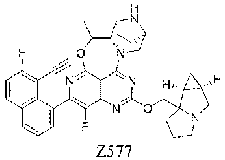 | 621.3 |
| 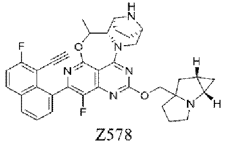 | 621.3 | 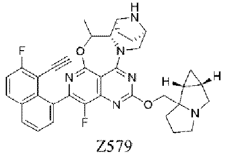 | 621.3 | 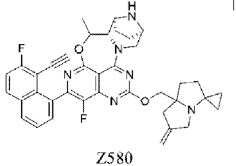 | 647.3 |
| 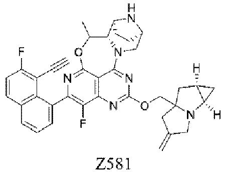 | 633.3 | 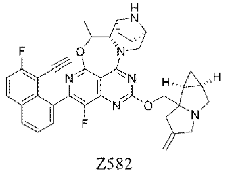 | 633.3 | 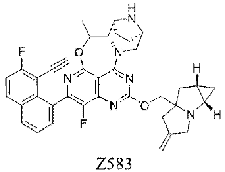 | 633.3 |
| 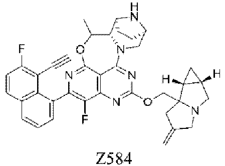 | 633.3 | 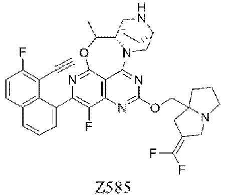 | 657.3 | 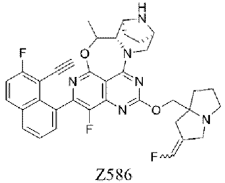 | 639.3 |
| 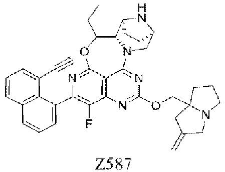 | 617.3 | 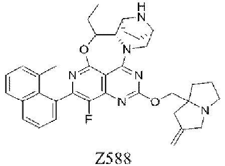 | 607.3 | 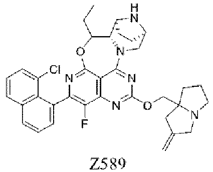 | 627.3 |
| 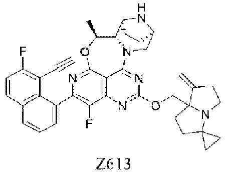 | 647.3 | 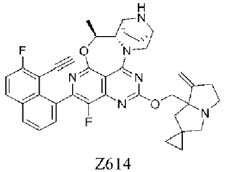 | 647.3 | 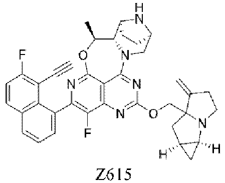 | 633.3 |
| 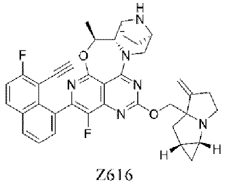 | 633.3 | 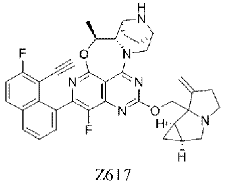 | 633.3 | 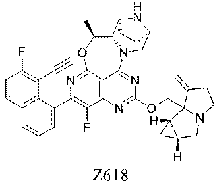 | 633.3 |
| 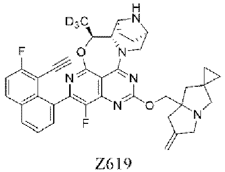 | 650.3 | 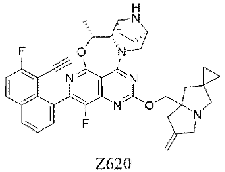 | 647.3 | 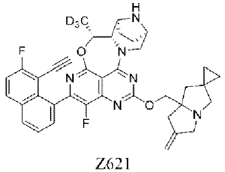 | 650.3 |
| 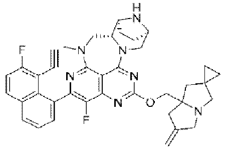 | 646.3 | 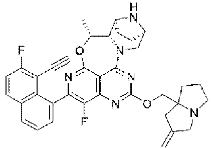 | 621.3 | 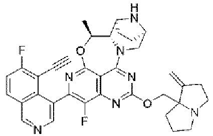 | 622.3 |
| 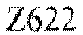 | | 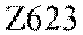 | | 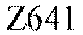 | |
| 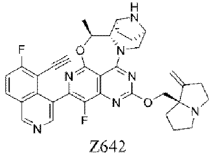 | 622.3 | 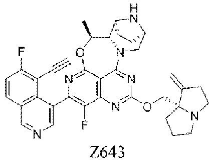 | 622.3 | 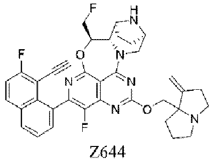 | 639.3 |
| 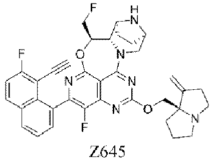 | 639.3 | 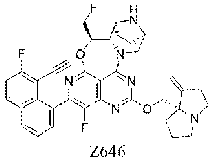 | 639.3 | 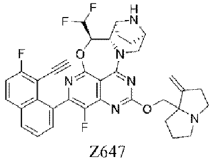 | 657.3 |
| 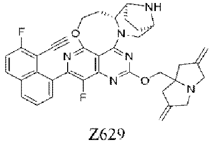 | 633.3 | 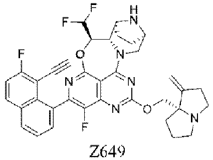 | 657.3 | 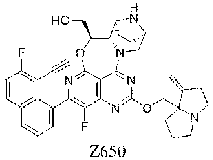 | 637.3 |
| 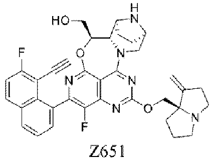 | 637.3 | 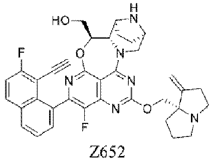 | 637.3 | 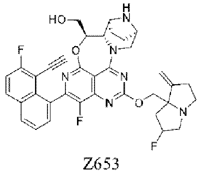 | 655.3 |
| 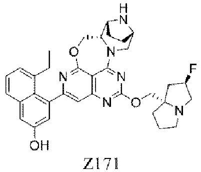 | 597.3 | 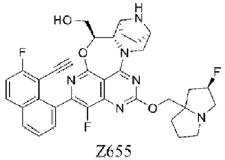 | 643.3 | 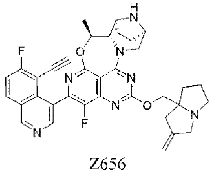 | 622.3 |
| 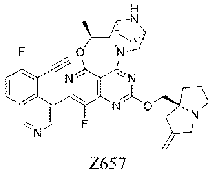 | 622.3 | 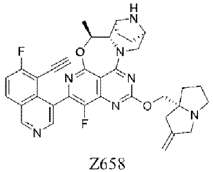 | 622.3 | 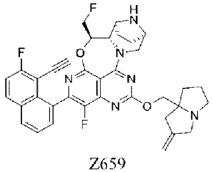 | 639.3 |
| 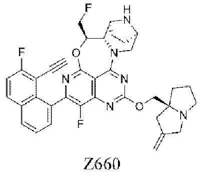 | 639.3 | 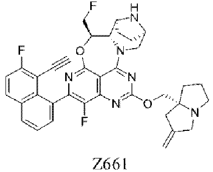 | *639.3* | 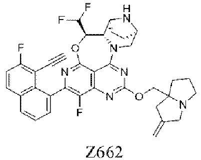 | 657.3 |
| 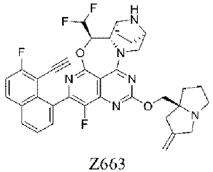 | 657.3 | 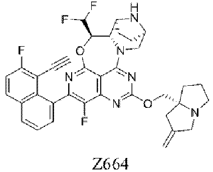 | 657.3 | 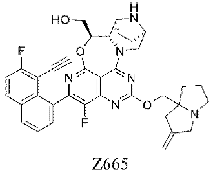 | 637.3 |
| 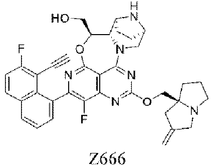 | 637.3 | 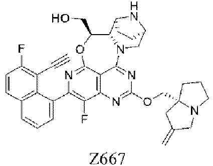 | 637.3 | 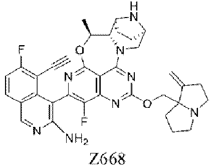 | 637.3 |
| 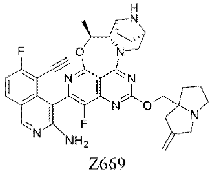 | 637.3 | 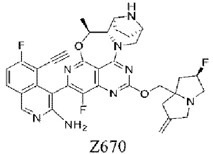 | 655.3 | 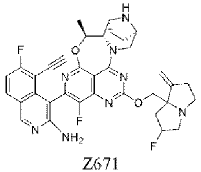 | 655.3 |
| 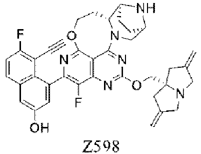 | 649.3 | 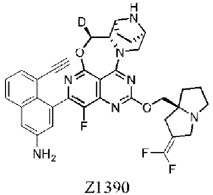 | 641.3 | 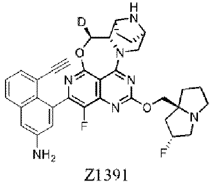 | 644.3 |
| 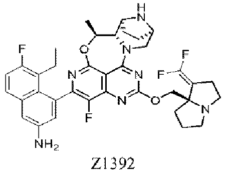 | 676.3 | 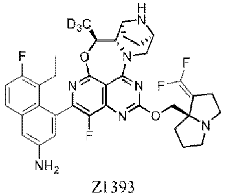 | 679.3 | 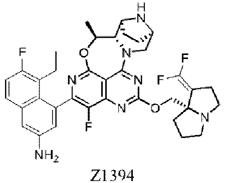 | *676.3* |
| 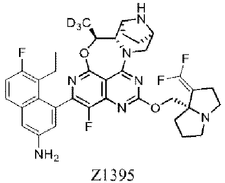 | 679.3 | 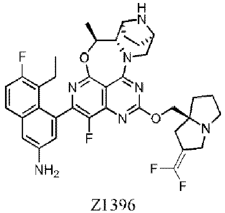 | 676.3 | 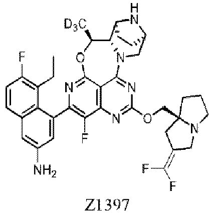 | 679.3 |
| 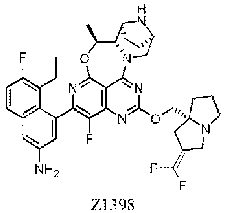 | 676.3 | 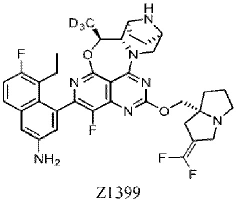 | 679.3 | 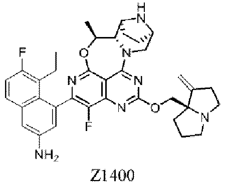 | 640.3 |
| 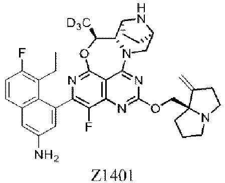 | 643.3 | 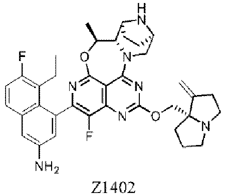 | 640.3 | 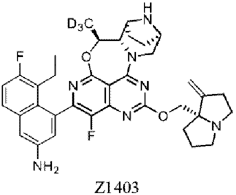 | 643.3 |
| 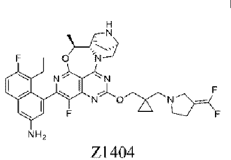 | 690.3 | 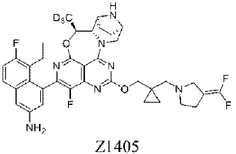 | 693.3 | 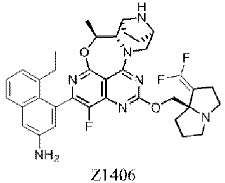 | 658.3 |
| 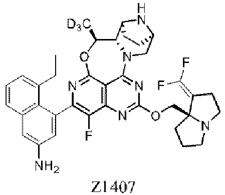 | 661.3 | 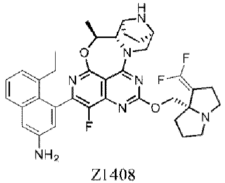 | 658.3 | 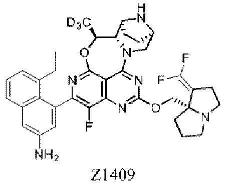 | 661.3 |
| 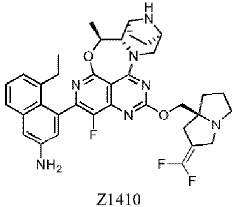 | 658.3 | 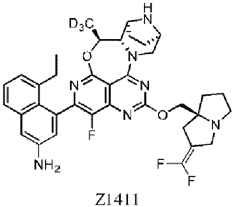 | 661.3 | 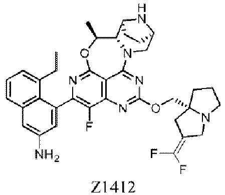 | 658.3 |
| 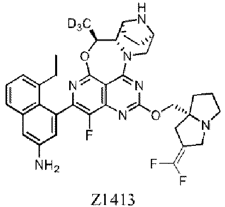 | 661.3 | 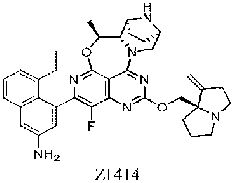 | 622.3 | 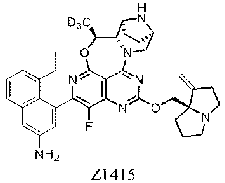 | 625.3 |
| 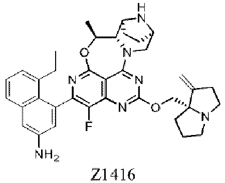 | 622.3 | 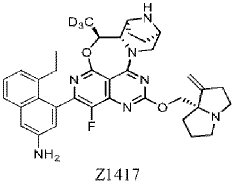 | 625.3 | 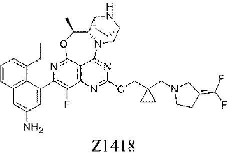 | 672.3 |
| 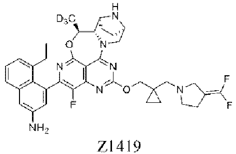 | 675.3 | 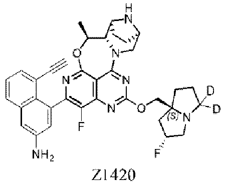 | 626.3 | 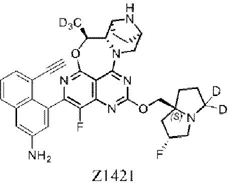 | 629.3 |
| 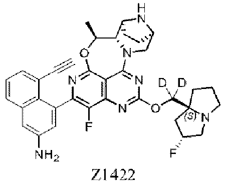 | 622.3 | 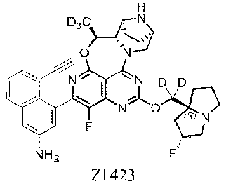 | 625.3 | | |

### Test Example 1: Cell assay for p-ERK

AGS was purchased from ATCC, Article No.: CRL-1739; F12K was purchased from Gibco, Article No.: 21127-022; FBS was purchased from Gibco, Article No.: 10099-141C; the trypsin (containing EDTA) was purchased from Gibco, Article No.: 25200-072; DMSO was purchased from VWRAMRESCO, Article No.: 0231-500ML; the 96-well cell culture plate was purchased from Corning, Article No.: 3599; the white-bottom opaque 96-well plate was purchased from Cisbio, Article No.: 66PL96025; Advance ERK phospho-T202/Y204 kit was purchased from Cisbio, Article No.: 64AERPEH; the cell counter was purchased from CHEMOMETEC, model: NC-200; the microplate reader was purchased from PerkinElmer, model: Envison.

AGS, an endogenous human gastric adenocarcinoma cell line with KRAS^{G12D} mutation, was cultured in F12K+10% FBS medium. On the first day of the experiment, cells in logarithmic growth period were digested with trypsin (containing EDTA), collected and counted, and about 20,000 cells/well were inoculated in a 96-well cell culture plate and cultured overnight in an incubator with 5% CO₂. 1000X compound stock solutions with gradient concentrations of 3.16 times were prepared with DMSO, and 200-fold diluted to 5X compound stock solutions with the above culture medium. The 5X compound stock solution was added to each cell culture well on the second day after cell inoculation, to give a final concentration of 1X and DMSO content of 0.1%. DMSO was used as experimental control. After adding the compound, the cells were continuously cultured for 3 hrs. Then, the culture medium in the well was removed, and 50 µl/well of cell lysis buffer (from Advanced ERK phospho-T202/Y204 kit, the same hereinafter) was added, and mixed uniformly. The cells were cultured for 30 min. Then 16 µl of the mixture was transferred to a white-bottom opaque 96-well plate, and 16 µl of the cell lysis buffer was added to an additional well and used as a blank. After that, 4 µl of p-ERK HTRF antibody mixed solution (from Advanced ERK Phos Pho-T202/Y204 Kit) was added to each well. After incubation for 4 hrs, the fluorescence value (RLU) of HTRF was read on a microplate reader. The inhibition rate of the compound on p-ERK level was calculated by the formula IR (%) = (RLU of control - RLU of compound) / (RLU of control - RLU of blank) x 100%. The compound concentration and the corresponding inhibition rate on p-ERK level in the cells were fitted by using Prism 8 four-parameter method, and the IC₅₀ value was calculated. The results show that the compound of the present invention has high inhibitory activity on p-ERK. The IC₅₀ values of some compounds are less than 1 µM, or the IC₅₀ values of some comounds are less than 500 nM, and even less than 100 nM or 50 nM. The results of some exemplary compounds are shown in Table 1 below.

**Table 1**

| No. | AGS p-ERK IC₅₀ (nM) | No. | AGS p-ERK IC₅₀ (nM) |
|---|---|---|---|
| Z2 | 10.65 | Z131-1 | 1.58 |
| Z384 | 0.65 | Z360-1 | 29.95 |
| Z140-1 | 2.85 | Z386 | 0.76 |
| Z350 | 46.98 | Z387 | 50.21 |
| Z380 | 0.78 | Z388 | 17.87 |
| Z381 | 435.88 | Z550 | 91.16 |
| Z552 | 0.67 | Z551 | 61.04 |
| Z553 | 0.48 | Z590 | 7.11 |
| Z593 | 28.97 | Z595 | 118.62 |
| Z597 | 48.52 | Z599 | 27.58 |
| Z600 | 18.29 | Z624 | 47.29 |
| Z625 | 81.86 | Z626-2 | 20.64 |
| Z601 | 10.49 | Z627-2 | 59.26 |
| Z628 | 45.48 | Z626-1 | 411.02 |
| Z605 | 553.75 | Z608 | 28.40 |
| Z604 | 95.01 | Z603 | 64.10 |
| Z602 | 34.12 | Z607 | 24.44 |
| Z630 | 728.71 | Z606 | 874.36 |
| Z631 | 91.90 | Z612 | 99.29 |
| Z611 | 19.57 | Z632 | 30.42 |
| Z610 | 17.24 | Z633 | 14.48 |
| Z635 | 49.97 | Z636 | 10.49 |
| Z637 | 48.75 | Z638 | 7.79 |
| Z639 | 33.44 | Z640 | 65.54 |
| Z609 | 49.23 | Z673 | 146.56 |
| Z674 | 10.70 | Z675 | 11.12 |
| Z676 | 69.99 | Z677 | 185.01 |
| Z678 | 12.44 | Z679 | 117.06 |
| Z680 | 142.56 | Z681 | 5.73 |
| Z682 | 82.05 | Z683 | 5.84 |
| Z684 | 57.52 | Z685 | 30.28 |
| Z686 | 446.58 | Z687 | 192.57 |
| Z688 | 8.77 | Z689 | 6.61 |
| Z691 | 14.23 | Z692 | 187.53 |
| Z693 | 9.23 | Z698 | 21.69 |
| Z654 | 135.88 | Z690 | 16.70 |
| Z695 | 4.71 | Z696 | 72.63 |
| Z699 | 0.44 | Z700 | 0.39 |
| Z701 | 39.48 | Z702 | 11.06 |
| Z703 | 1.05 | Z704 | 542.28 |
| Z705 | 194.79 | Z706 | 6.64 |
| Z707 | 0.95 | Z708 | 38.55 |
| Z709 | 12.24 | Z710 | 0.34 |
| Z711 | 13.30 | Z718 | 157.35 |
| Z712 | 28.18 | Z713 | 22.03 |
| Z714 | 621.69 | Z715 | 1.94 |
| Z716 | 81.84 | Z717 | 14.80 |
| Z719 | 1.16 | Z720 | 35.09 |
| Z721 | 37.51 | Z722 | 7.22 |
| Z723 | 6.96 | Z724 | 45.34 |
| Z725 | 42.33 | Z726 | 78.84 |
| Z727 | 5.01 | Z728 | 0.95 |
| Z729 | 212.17 | Z730 | 0.54 |
| Z731 | 1.60 | Z732 | 2.88 |
| Z733 | 76.04 | Z598 | 0.68 |
| Z629 | 50.02 | Z648 | 14.62 |
| Z734 | 3.42 | Z735 | 5.62 |
| Z737 | 15.32 | Z738 | 12.70 |
| Z739 | 2.63 | Z740 | 6.08 |
| Z741 | 4.06 | Z742 | 51.97 |
| Z743 | 0.84 | Z744 | 0.51 |
| Z745 | 0.84 | Z746 | 17.05 |
| Z747 | 0.37 | Z748 | 4.63 |
| Z749 | 1.91 | Z957 | 0.97 |
| Z959 | 1.59 | Z960 | 0.25 |
| Z961 | 0.21 | Z962 | 0.52 |
| Z963 | 0.39 | Z964 | 0.38 |
| Z1084 | 0.12 | Z1085 | 0.78 |
| Z958 | 29.17 | Z1060 | 0.72 |
| Z1044 | 0.34 | Z1054 | 0.43 |
| Z1295 | 13.86 | | |

### Test Example 2: Cell proliferation inhibition experiment

AsPC-1 was purchased from ATCC, Article No.: CRL-1682; AGS was purchased from ATCC, Article No.: CRL-1739; RPMI1640 was purchased from Gibco, Article No.: 11875-093 2235123; F12K was purchased from Gibco, Article No.: 21127-022; FBS was purchased from Gibco, Article No.: 10099-141C; the trypsin (containing EDTA) was purchased from Gibco, Article No.: 25200-072; CellTiter Glo-3D was purchased from Progema, Article No.: G9683; the 384-well spheroid plate was purchased from Corning, Article No.: 3830; the white-bottom opaque 384-well plate was purchased from Corning, Article No.: 3570; the cell counter was purchased from CHEMOMETEC, model: NC-200; the microplate reader was purchased from PerkinElmer, model: Envison;

AsPC-1, an endogenous human pancreatic cancer cell AsPC-1 with KRAS^{G12D} mutation, was cultured in RPMI-1640 medium containing 10%FBS. AGS, a human gastric cancer cell line with KRAS^{G12D} mutation, was cultured in F-12K medium containing 10% FBS. Cells in logarithmic growth period were digested with trypsin (containing EDTA), collected and counted. 800 AsPC-1 cells/well or 400 AGS wells/well were inoculated in a 384-well spheroid plate, and cultured overnight in an incubator with 5% CO₂ to establish a 3D cell model. 1000X compound stock solutions with gradient concentrations of 3.16 times were prepared with DMSO, and 100-fold diluted to 10X compound stock solutions with the culture medium. The 10X compound stock solution was added to each cell culture well on the second day after cell inoculation, to give a final concentration of 1X and DMSO content of 0.1%. DMSO was used as the control, and the culture medium was used as the blank. After the compound was added, the cells were cultured for 5 days. 30µl CellTiter-Glo working solution was added to each well, and mixed uniformly. The cells were incubated for 30 min, and allowed to stand at room temperature for 30 min. 40µl of the mixture was transferred to a white-bottom opaque 384-well plate, and the luminescence was read on a micropate reader. The inhibition rate on cell proliferation was calculated by a formula: IR (%) = (RLU of control - RLU of compound) / (RLU of control -RLU of blank) ×100%. The gradient concentrations of the compound and the corresponding inhibition rates were fitted by using XLFit four-parameter method, and the IC₅₀ value was calculated. The results show that the compound of the present invention has high inhibitory activity on KRAS^{G12D} mutant cells. The IC₅₀ values of some compounds are less than 1000nM; or the IC₅₀ values are less than 500nM, or even less than 100 nM or 50 nM. The results of some exemplary compounds are shown in Table 2 below (- means not tested).

**Table 2**

| No. | AGS IC₅₀ (nM) | AsPC-1 IC₅₀ (nM) | No. | AGS IC₅₀ (nM) | AsPC-1 IC₅₀ (nM) |
|---|---|---|---|---|---|
| Z2 | 10.35 | 127.31 | Z384 | 1.50 | 7.34 |
| Z388 | 53.1 | 191.2 | Z386 | 2.40 | 14.70 |
| Z380 | 2.68 | 26.16 | Z550 | 108.68 | 393.62 |
| Z360-1 | 27.8 | 36.4 | Z551 | 45.38 | 169.72 |
| Z552 | 0.53 | 5.77 | Z553 | 0.53 | 9.13 |
| Z590 | 17.31 | 113.54 | Z592 | 354.25 | - |
| Z387 | 73.28 | 201.91 | Z593 | 35.35 | 78.84 |
| Z595 | 99.01 | 157.29 | Z596 | 268.28 | 411.24 |
| Z599 | 10.76 | 22.31 | Z597 | 47.86 | 98.73 |
| Z624 | 88.23 | 194.07 | Z600 | 15.32 | 64.84 |
| Z626-2 | 28.19 | 56.58 | Z625 | 107.06 | 404.55 |
| Z627-2 | 83.42 | 286.81 | Z601 | 13.52 | 39.00 |
| Z626-1 | 502.15 | - | Z628 | 39.97 | 144.17 |
| Z602 | 26.54 | 90.36 | Z608 | 82.26 | 145.57 |
| Z606 | 907.6 | 802.34 | Z604 | 573.15 | 468.39 |
| Z612 | 65.41 | 124.21 | Z607 | 81.18 | 101.07 |
| Z632 | 26.16 | 53.83 | Z631 | 201.17 | 254.15 |
| Z633 | 59.01 | 56.73 | Z611 | 10.38 | 49.12 |
| Z635 | 223.59 | 218.24 | Z610 | 18.58 | 42.34 |
| Z637 | 67.59 | 111.81 | Z634 | - | 882.73 |
| Z639 | 112.61 | 93.66 | Z636 | 10.87 | 30.59 |
| Z638 | 19.71 | 32.01 | Z603 | 110.21 | 216.73 |
| Z674 | 108.26 | 475.07 | Z675 | 31.20 | 31.20 |
| Z676 | 266.67 | - | Z677 | 794.94 | 981.41 |
| Z678 | 100.5 | 73.48 | Z679 | 393.33 | 352.08 |
| Z680 | 385.92 | - | Z681 | 13.00 | 37.90 |
| Z682 | 145.71 | 236.94 | Z683 | 22.70 | 79.00 |
| Z684 | 340.62 | 326.14 | Z685 | 145.20 | 257.82 |
| Z698 | 18.76 | 49.29 | Z687 | 392.19 | 513.32 |
| Z688 | 19.96 | 39.19 | Z689 | 19.29 | 17.42 |
| Z691 | 54.60 | 87.94 | Z692 | 378.21 | 289.51 |
| Z693 | 27.29 | 31.12 | Z694 | 396.7 | 405.39 |
| Z654 | 725.87 | 388.84 | Z690 | 31.51 | 45.92 |
| Z695 | 36.14 | 77.67 | Z696 | 598.15 | 694.49 |
| Z699 | 1.98 | 2.37 | Z702 | 26.8 | 51.9 |
| Z700 | 1.91 | 2.14 | Z701 | 230.46 | 245.38 |
| Z703 | 2.64 | 1.78 | Z704 | - | 687.84 |
| Z706 | 25.99 | 37.87 | Z707 | 7.44 | 5.24 |
| Z708 | 175.87 | 940.69 | Z709 | 86.75 | 117.46 |
| Z710 | 4.26 | 3.72 | Z140-1 | 4.00 | 21.11 |
| Z131-1 | 2.53 | 32.8 | Z712 | 60.68 | 30.39 |
| Z713 | 73.54 | 55.59 | Z714 | 672.92 | 814.88 |
| Z715 | 3.44 | 3.83 | Z716 | 48.59 | 89.15 |
| Z717 | 29.11 | 50.18 | Z718 | 455.23 | 429.35 |
| Z719 | 1.03 | 3.00 | Z720 | 15.71 | 33.37 |
| Z721 | 43.60 | 105.26 | Z722 | 12.77 | 9.29 |
| Z723 | 15.10 | 55.40 | Z724 | 92.00 | 195.78 |
| Z725 | 62.70 | 105.80 | Z726 | 133.36 | 637.76 |
| Z727 | 9.30 | 12.35 | Z728 | 3.75 | 5.41 |
| Z729 | 584.08 | 757.02 | Z730 | 1.66 | 3.61 |
| Z731 | 5.70 | 15.76 | Z732 | 10.77 | 11.83 |
| Z733 | 296.69 | 267.29 | Z734 | 17.17 | 12.14 |
| Z735 | 35.23 | 18.14 | Z598 | 1.23 | 5.55 |
| Z629 | 51.69 | 166.87 | Z711 | 54.16 | 85.58 |
| Z648 | 39.13 | 53.51 | Z957 | 7.61 | 6.51 |
| Z736 | 389.33 | 254.45 | Z737 | 34.04 | 44.24 |
| Z738 | 44.18 | 29.89 | Z739 | 11.03 | 6.16 |
| Z740 | 17.96 | 25.98 | Z741 | 19.22 | 10.11 |
| Z742 | 156.35 | 312.27 | Z743 | 1.95 | 2.16 |
| Z744 | 2.86 | 1.99 | Z745 | 3.95 | 4.48 |
| Z748 | 23.14 | 12.88 | Z749 | 7.13 | 11.51 |
| Z959 | 7.27 | 7.21 | Z960 | 3.96 | 1.58 |
| Z961 | 2.78 | 1.11 | Z962 | 7.08 | 2.89 |
| Z963 | 0.21 | 1.69 | Z958 | 45.57 | 69.47 |
| Z964 | 0.51 | 1.58 | Z1084 | 0.38 | 1.23 |
| Z1085 | 0.88 | 3.04 | Z1060 | 0.19 | 1.63 |
| Z1044 | 0.53 | 0.92 | Z1054 | 0.37 | 1.29 |
| Z1072 | 0.50 | 2.26 | | | |

### Test Example 3: Pharmacokinetic evaluation test in mice

Test objective: Male CD-1 mice were used as test animals. The drug concentrations in plasma of mice at various times were determined by LC/MS/MS after intravenous and oral administration of the test compounds. The pharmacokinetic behavior of the test compounds in mice was studied and their pharmacokinetic characteristics were evaluated.

Test methods: test animals: 60 healthy adult male CD-1 mice, aged 6-8 weeks and weighing about 30 g, and divided into 10 groups, including IV groups (intravenous injection group, five groups), with 6 mice in each group; and PO groups (oral administration group, five groups), with 6 mice in each group. The mice were purchased from Zhejiang Vital River Laboratory Animal Technology Co., Ltd.

Preparation of drug solution: IV group: Taking 10 mL sample solution as an example, 2 mg sample was weighed, 0.5mL DMSO and 1mL Solutol HS 15 were sequentially added, and then 8.5mL 6% HP-β-CD was added, and stirred ultrasonically to obtain a clear solution of 0.2 mg/mL.

PO group 1: Taking 10 mL sample solution as an example, 10 mg sample was weighed, 0.5mL DMSO and 1mL Solutol HS 15 were sequentially added, and then 8.5mL 6% HP-β-CD was added, and stirred ultrasonically to obtain a 1 mg/mL solution.

PO group 2: A 3 mg/mL solution of the sample was prepared according to the preparation method for PO group 1.

Administration: After overnight fasting, the mice in the IV group were administered with a volume of 5 mL/kg at a dose of 1 mg/kg.

PO group 1: The mice were administered with a volume of 10 mL/kg at a dose of 10 mg/kg.

PO group 2: The mice were administered with a volume of 10 mL/kg at a dose of 30 mg/kg.

Experimental operations: Male CD-1 mice in the intravenous injection group and oral administration group were given the test compound, respectively. After that, 50 ul of blood was periodically collected into a K₂EDTA anticoagulation tube at 0.0833, 0.25, 0.5, 1, 2, 4, 6, 8 and 24 hrs, and the plasma was separated by centrifugation at 4°C and 8000 rpm for 15 min. The plasma was stored in dry ice at -80°C. 4 hrs after administration, the animals were allowed to free access to food. The contents of the test compounds in plasma of mice after intravenous and oral administration were determined by LC/MS/MS. The linear range of the method was 1-3000 ng/ml. The plasma samples were treated with acetonitrile to precipitate the protein and then analyzed.

The experimental results of the PO group are shown in Table 4 below.

**Table 4**

| Group | Z131-1 | Z140-1 | Z1084 | Z1054 |
|---|---|---|---|---|
| Dose (mpk) | 10 | 10 | 30 | 30 |
| AUCₗₐₛₜ (hr*ng/mL) | 40.4 | 42.1 | 2440 | 3252 |
| F (%) | 0.9 | 2.33 | 20.7 | 29.4 |

| | | | | |
|---|---|---|---|---|
| Note: AUC indicates the area under curve after oral administration; and F indicates the bioavailability. Conclusion: In the pharmacokinetic evaluation test of mice, the pharmacokinetic behaviors of Compounds Z131-1, Z140-1, Z1084 and Z1054 are different, such as AUC and oral bioavailability. | | | | |

Although the specific embodiments of the present invention have been described in detail, various modifications and substitutions can be made to the details of the technical solution of the present invention by those skilled in the art according to all the disclosed teachings, which are embraced in the protection scope of the present invention. The full scope of the present invention is defined by the appended claims and any equivalents thereof.

## Claims

1. A compound represented by Formula (Z1'), or a stereoisomer, a deuterated compound or a pharmaceutically acceptable salt thereof, 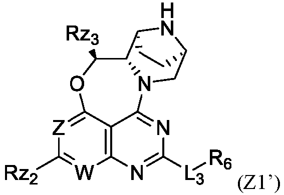 wherein
W is N or CRz1, where Rz1 is hydrogen, halogen, C1-C6 alkyl or C1-C6 alkoxy;
Z is N or CRz11, where Rz11 is hydrogen, halogen, C1-C6 alkyl or C1-C6 alkoxy;
Rz2 is substituted or unsubstituted phenyl, naphthyl, quinoline, isoquinoline, benzo [b]thiophene, benzo[d]thiazole, indole, indazole or pyridine, where the substitution means that 1, 2, 3, 4 or 5 hydrogen atoms on phenyl, naphthyl, quinoline, isoquinoline, benzo[b]thiophene, benzo[d]thiazole, indole, indazole or pyridine are each independently replaced by R¹;
Rz3 is hydrogen, deuterium, methyl, deuterated methyl or halomethyl or -CD₂F or -CDF₂;
R¹ is selected from the group consisting of: C1-C6 alkyl, halogen, hydroxyl, cyano, C1-C6 alkoxy, C1-C6 haloalkyl, C1-C6 haloalkoxy, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6hydroxyalkynyl, C1-C6 cyanoalkyl, triazolyl, - S-C1-C6 haloalkyl, C1-C6 hydroxylalkyl, -CH₂C(O)NR²⁷R²⁸, -C2-C6 alkynyl-NR²⁹R³⁰, C2-C6 deuterated alkynyl, (C1-C6 alkoxy)C1-C6 haloalkyl- or cycloalkyl, where the cycloalkyl is optionally substituted with halo or C1-C6 alkyl, where
R²¹ is H, C1-C6 alkyl, C1-C6 haloalkyl, C(O)C1-C6 alkyl or C(O)OC1-C6 alkyl; R²² is H, C1-C6 alkyl or C1-C6 haloalkyl; or R²¹ and R²², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²³ and R²⁴ are each independently H, C1-C6 alkyl, or C1-C6 haloalkyl; or R²³ and R²⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy; and
R²⁵ is hydroxy, cyano, heterocyclyl, NR²⁵¹R²⁵², C(O)NR²⁵³R²⁵⁴ or SO₂C1-C6 alkyl, where R²⁵¹, R²⁵², R²⁵³ and R²⁵⁴ are each independently H or C1-C6 alkyl; or R²⁵¹ and R²⁵², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy; or R²⁵³ and R²⁵⁴, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy and trifluoromethoxy;
R²⁶ is C1-C6 alkyl, C1-C6 haloalkyl, or NR²⁶¹R²⁶², where R²⁶¹ and R²⁶² are each independently H or C1-C6 alkyl, or R²⁶¹ and R²⁶², together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy;
R²⁷ and R²⁸ are each independently H, C1-C6 alkyl, or C1-C6 haloalkyl; or R²⁷ and R²⁸, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy; and
R²⁹ and R³⁰ are each independently H, C1-C6 alkyl, or C1-C6 haloalkyl; or R²⁹ and R³⁰, together with the nitrogen atom to which they are attached, form a 3- to 6-membered nitrogen-containing heterocyclic group, where the 3- to 6-membered nitrogen-containing heterocyclic group is optionally substituted with 1 or 2 groups selected from halogen, methyl, trifluoromethyl, methoxy, and trifluoromethoxy; and
-L₃-R⁶ is 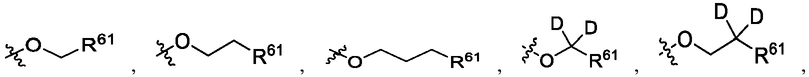 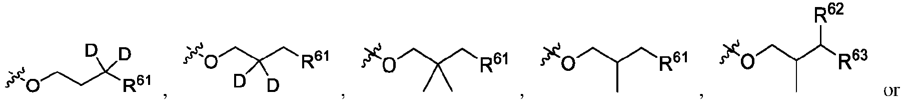 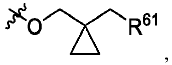
in which R⁶¹ is a 3- to 6-membered heterocyclyl or a 6- to 10-membered fused heterocyclyl, where
the 3- to 6-membered heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 3- to 6-membered heterocyclyl are optionally both replaced by =CR^{2a}R^{2b}, where R^{2a} and R^{2b} are each independently hydrogen, halogen, C1-C4 alkyl or C1-C4 haloalkyl;
the 6- to 10-membered fused heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 6- to 10-membered fused heterocyclyl are optionally both replaced by =CR^{2a}R^{2b}, where R^{2a} and R^{2b} are each independently hydrogen, halogen, C1-C4 alkyl or C1-C4 haloalkyl; or each pair of R^{2a} and R^{2b} independently form, together with the carbon atom to which they are attached, a C3-C6 monocyclic cycloalkyl or a 3- to 6-membered monocyclic heterocyclyl; and
the 3- to 6-membered heterocyclyl or the 6- to 10-membered fused heterocyclyl is further optionally substituted with 1 or 2 groups selected from C1-C4 alkyl, C1-C4 haloalkyl, and halo; and
R⁶² is hydroxyl or halo; and R⁶³ is C1-C4 alkyl, C1-C4 haloalkyl or halo.

2. The compound, or stereoisomer, deuterated compound or pharmaceutically acceptable salt thereof according to claim 1, wherein the compound represented by Formula (Z1') is a compound represented by Formula (Z1), Formula (Z2), or Formula (Z3): 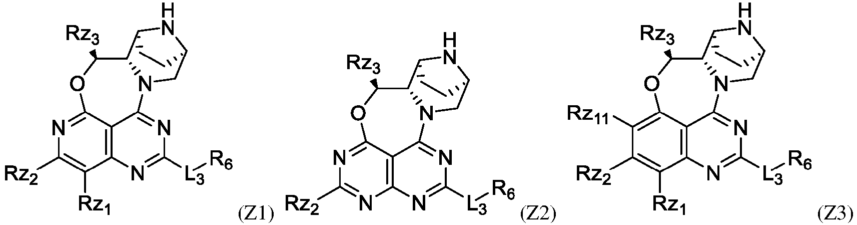 where in the formulas, Rz11, Rz1, Rz2, Rz3, L₃, and R₆ are each as defined in claim 1.

3. The compound, or stereoisomer, deuterated compound or pharmaceutically acceptable salt thereof according to claim 1, wherein the compound represented by Formula (Z1') is a compound represented by Formula (Z), where in the formula, Rz1 and Rz2 are each as defined in claim 1; 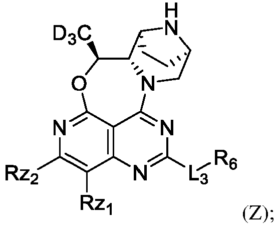 -L₃-R⁶ is 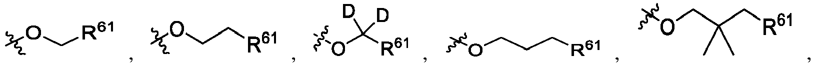 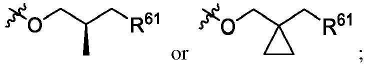 R⁶¹ is a 3- to 6-membered heterocyclyl or a 6- to 10-membered fused heterocyclyl; the 3- to 6-membered heterocyclyl is optionally substituted with 1 or 2 groups selected from C1-C4 alkyl, C1-C4 haloalkyl, and halo; and 2 hydrogen atoms on the same carbon atom in any 1 or 2 carbon atoms in the 6- to 10-membered fused heterocyclyl are optionally both replaced by deuterium or =CR^{2a}R^{2b}, where R^{2a} and R^{2b} are each independently hydrogen, halogen, C1-C4 alkyl or C1-C4 haloalkyl.

4. The compound, or stereoisomer, deuterated compound or pharmaceutically acceptable salt thereof according to claim 1, wherein the compound represented by Formula (Z1') is a compound represented by Formula (Z): 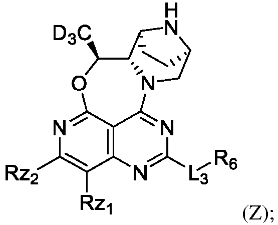 where in the formula, Rz1, Rz2, and R₆ are each as defined in claim 1; -L₃-R⁶ is 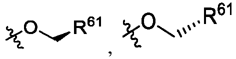 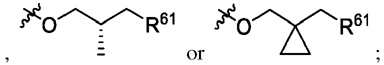 and R⁶¹ is as defined in claim 1.

5. The compound, or stereoisomer, deuterated compound or pharmaceutically acceptable salt thereof according to claim 1, wherein Rz1 is fluoro or hydrogen.

6. The compound, or stereoisomer, deuterated compound or pharmaceutically acceptable salt thereof according to claim 1, wherein R⁶¹ is selected from the group consisting of: 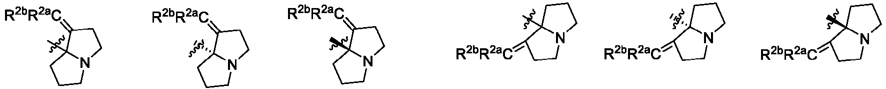 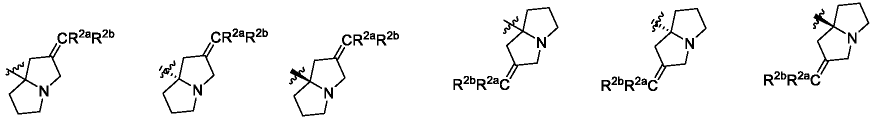 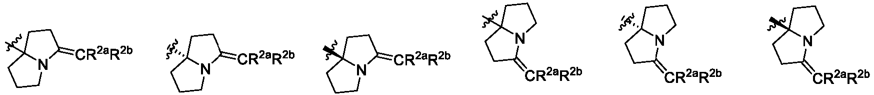 where in each formula, R^{2a} and R^{2b} are each independently hydrogen, halogen, C1-C4 alkyl or C1-C4 haloalkyl; or each pair of R^{2a}, R^{2b} independently form, together with the carbon atom to which they are attached, a C3-C6 monocyclic cycloalkyl or a 3- to 6-membered monocyclic heterocyclyl.

7. The compound, or stereoisomer, deuterated compound or pharmaceutically acceptable salt thereof according to claim 6, wherein in the substituents, 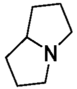 is optionally further substituted with 1 or 2 R^{6a}, in which R^{6a} is each independently hydrogen, deuterium or halo.

8. The compound, or stereoisomer, deuterated compound or pharmaceutically acceptable salt thereof according to claim 1, wherein R⁶¹ is a 6- to 10-membered fused heterocyclyl, in which the 6- to 10-membered fused heterocyclyl is 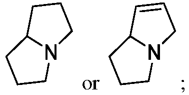 the 6- to 10-membered fused heterocyclyl is optionally substituted with 1 or 2 groups selected from deuterium, C1-C4 alkyl, C1-C4 haloalkyl, and halo; or 2 hydrogen atoms on the same carbon atom in any 1 or 2 carbon atoms in the 6- to 10-membered fused heterocyclyl are optionally both replaced by =CR^{2a}R^{2b}, where R^{2a} and R^{2b} are each independently hydrogen, halogen, C1-C4 alkyl or C1-C4 haloalkyl; and the 6- to 10-membered fused heterocyclyl is further optionally substituted with 1 or 2 groups selected from C1-C4 alkyl, C1-C4 haloalkyl, and halo.

9. The compound, or stereoisomer, deuterated compound or pharmaceutically acceptable salt thereof according to claim 1, wherein R⁶¹ is a 3- to 6-membered heterocyclyl, in which the 3- to 6-membered heterocyclyl is tetrahydropyrrolyl, piperidinyl, piperazinyl or morpholinyl; the 3- to 6-membered heterocyclyl is optionally substituted with 1 or 2 groups selected from C1-C4 alkyl, C1-C4 haloalkyl, and halo; or 2 hydrogen atoms on the same carbon atom in any 1 or 2 carbon atoms in the 3- to 6-membered heterocyclyl are optionally both replaced by =CR^{2a}R^{2b}, where R^{2a} and R^{2b} are each independently hydrogen, halogen, C1-C4 alkyl or C1-C4 haloalkyl; and the 3- to 6-membered heterocyclyl is further optionally substituted with 1 or 2 groups selected from C1-C4 alkyl, C1-C4 haloalkyl, and halo.

10. The compound according, or stereoisomer, deuterated compound or pharmaceutically acceptable salt thereof to claim 1, wherein R⁶¹ is selected from the group consisting of:

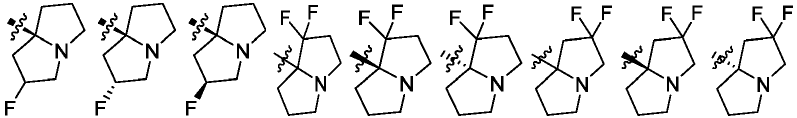

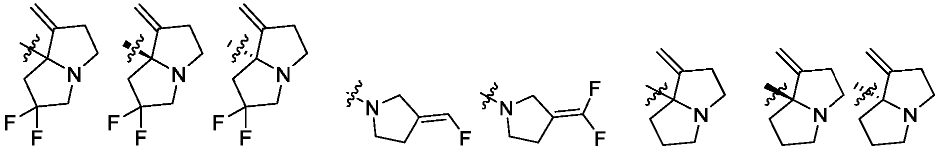

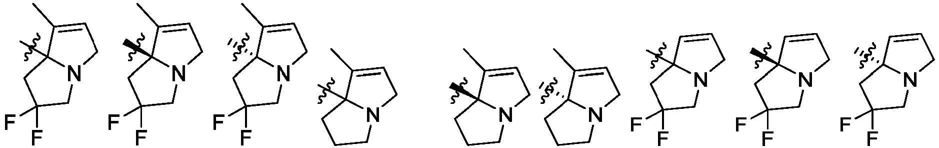

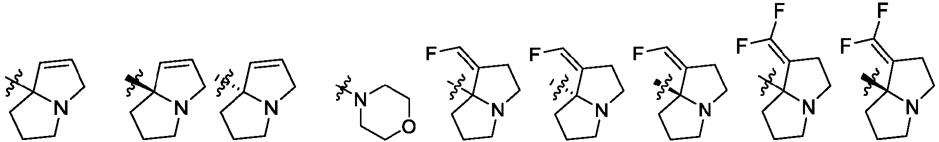

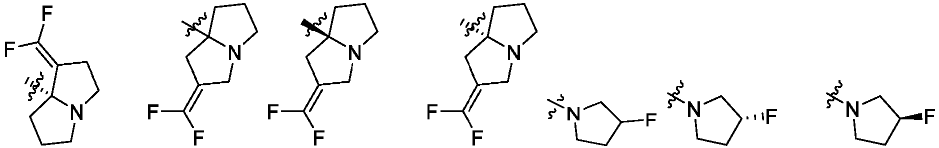

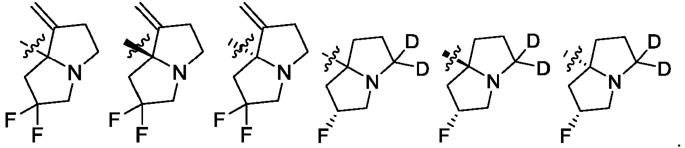

11. The compound, or stereoisomer, deuterated compound or pharmaceutically acceptable salt thereof according to claim 1, wherein Rz2 is selected from the group consisting of:

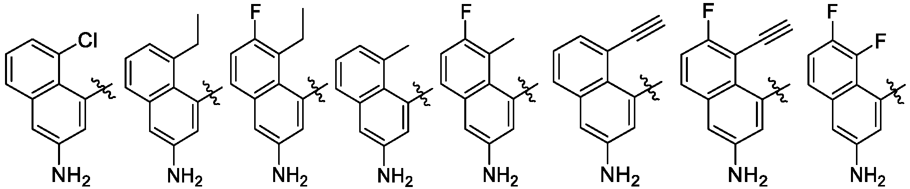

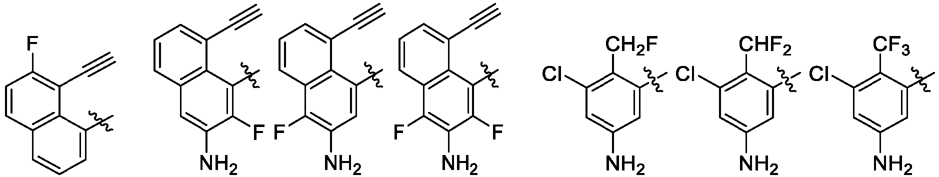

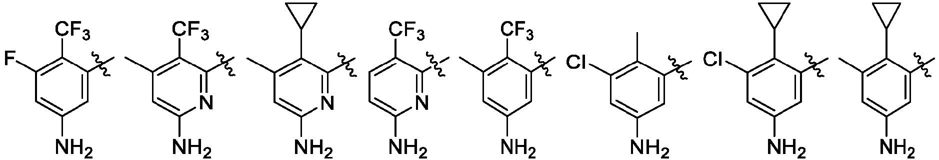

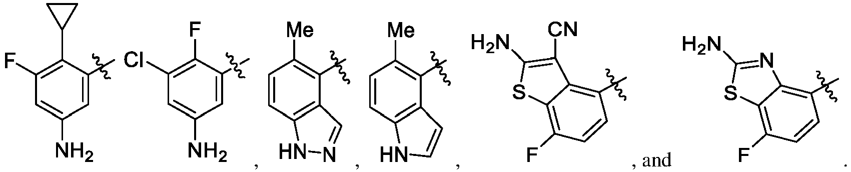

12. A compound, or a stereoisomer, a deuterated compound or a pharmaceutically acceptable salt thereof, wherein the compound is selected from Table A or from the group consisting of:

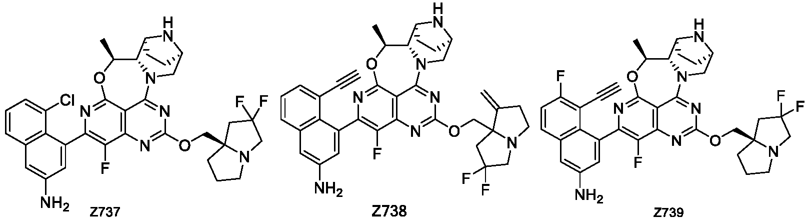

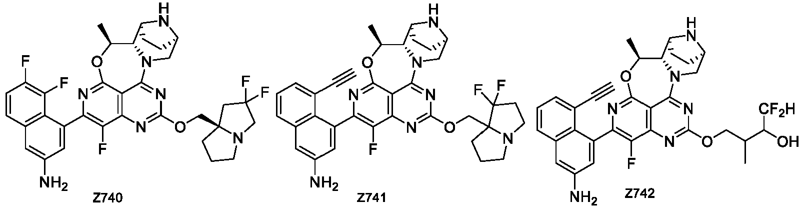

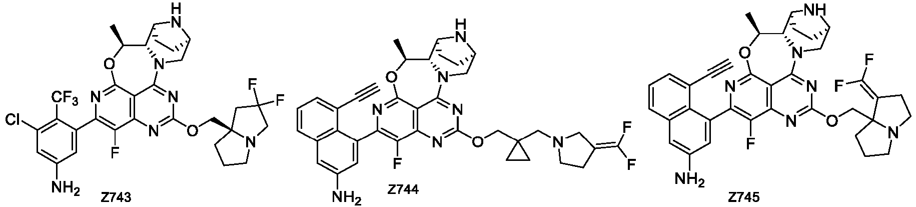

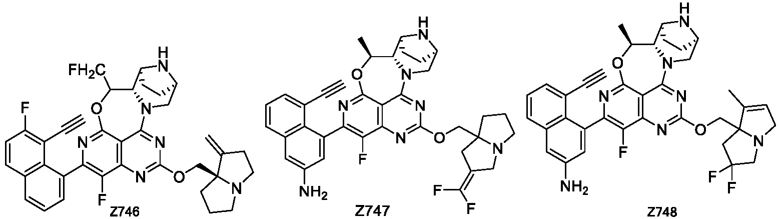

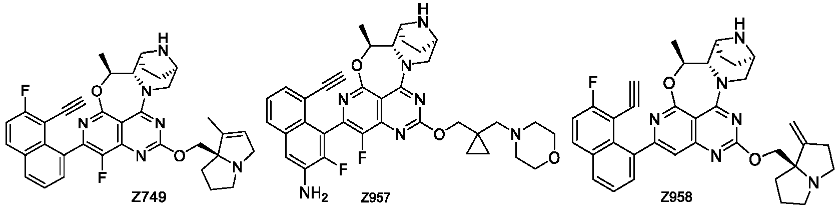

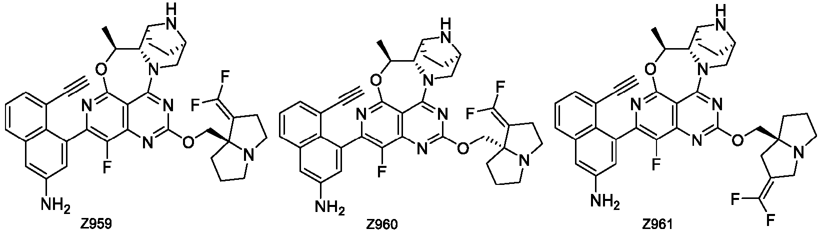

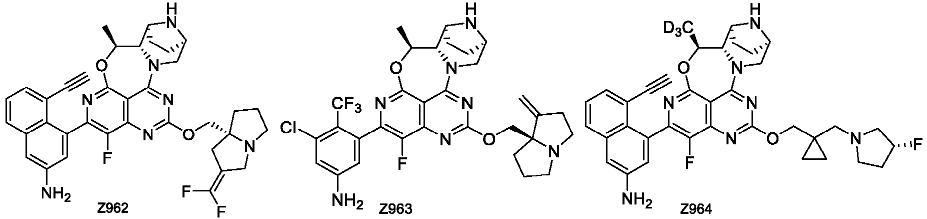

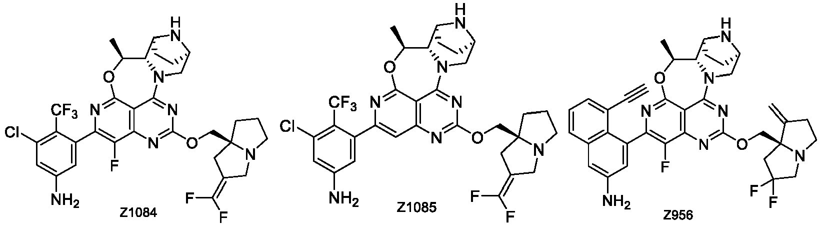

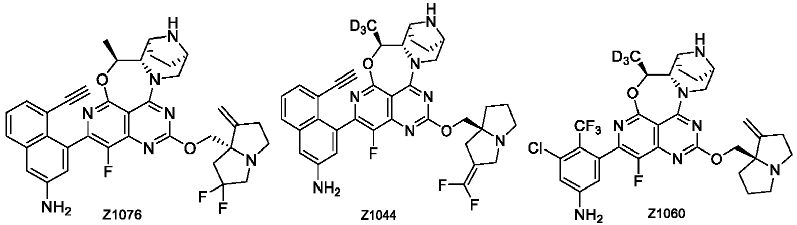

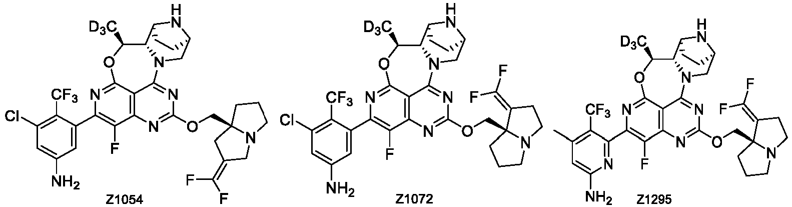

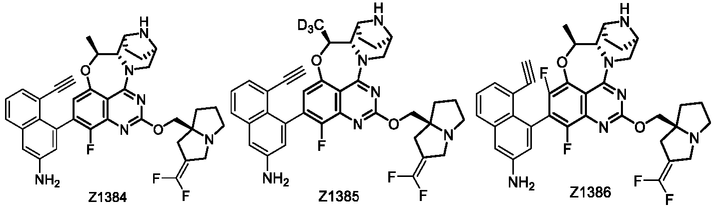

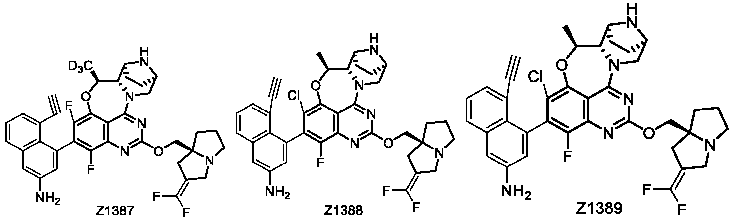

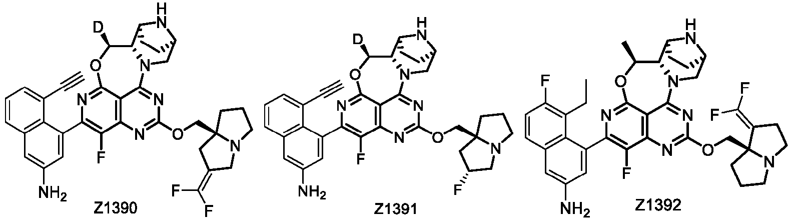

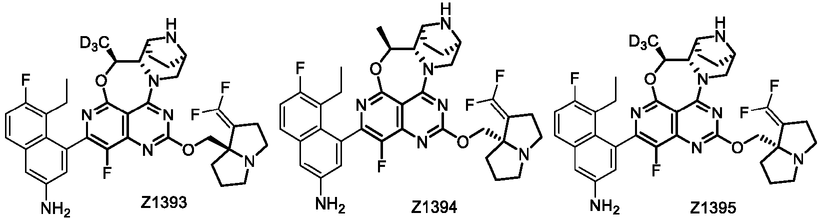

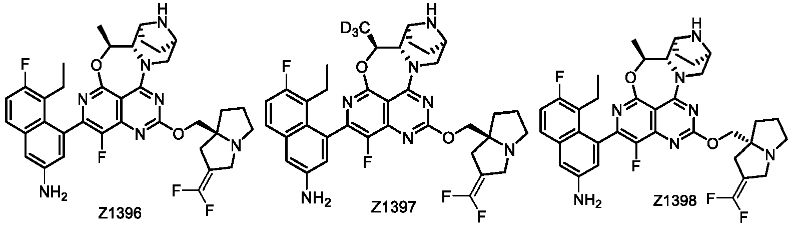

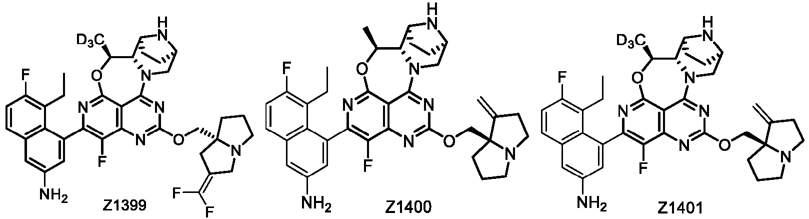

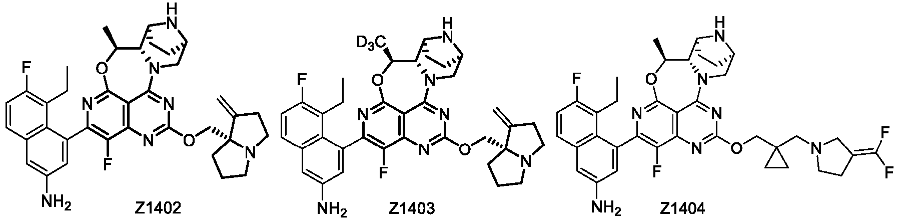

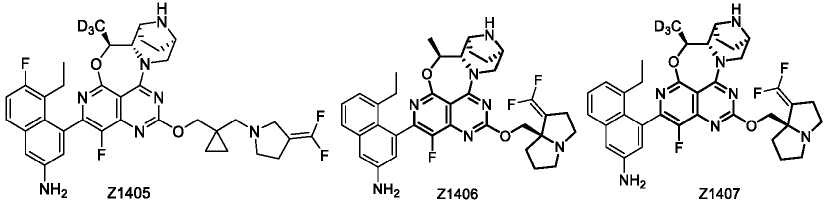

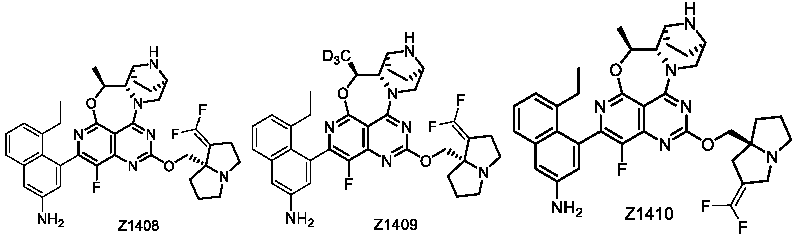

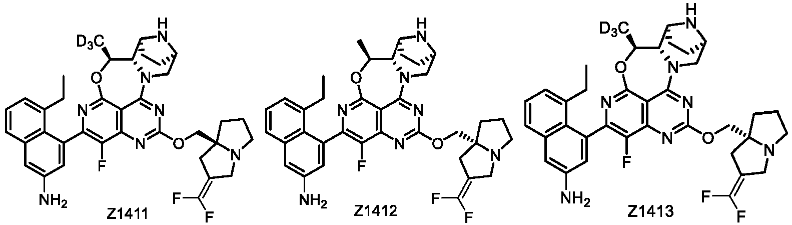

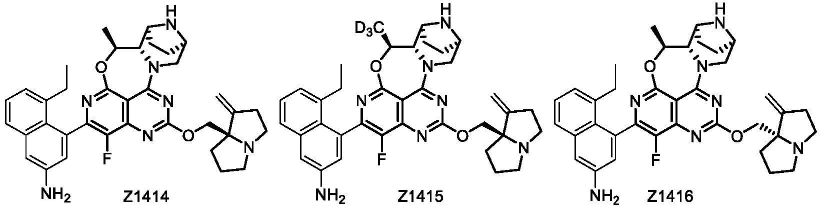

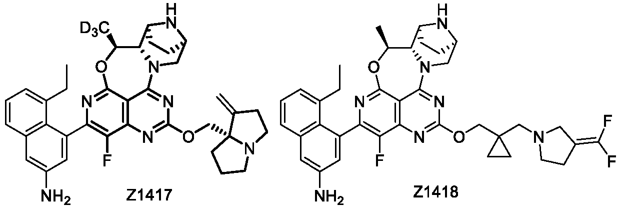

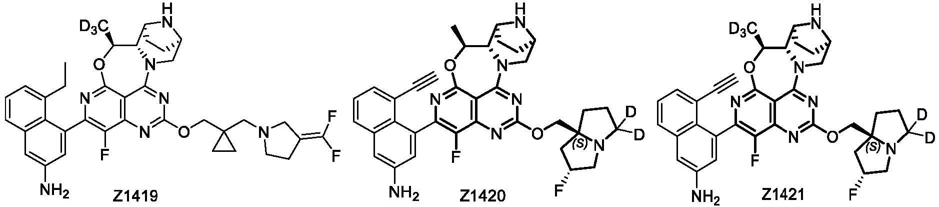

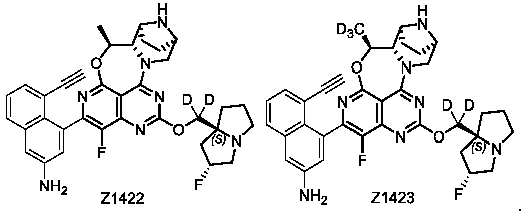

13. The compound, or stereoisomer, deuterated compound or pharmaceutically acceptable salt thereof according to claim 1 or 12, wherein the compound is in the form of a free base or a pharmaceutically acceptable salt.

14. A pharmaceutical composition, comprising a therapeutically effective amount of the compound or stereoisomer, deuterated compound, or pharmaceutically acceptable salt thereof according to any one of claims 1 to 12; and a pharmaceutically acceptable excipient.

15. Use of the compound or stereoisomer, deuterated compound, or pharmaceutically acceptable salt thereof according to any one of claims 1 to 12, or the pharmaceutical composition according to claim 13 in the preparation of drugs for preventing and/or treating diseases or disorders, wherein the diseases or disorders are KRAS G12D-related diseases or disorders.

16. The use according to claim 15, wherein the KRAS G12D-related diseases or disorders are cancers.
